# EUROPEAN PATENT APPLICATION

(11) **EP 4 494 706 A2**
(43) Date of publication of application: **22.01.2025**
(21) Application number: 24215260.1
(22) Date of filing: 23.10.2018
(51) Int. Cl.: A61P 27/02

(54) **ANTISENSE OLIGOMERS FOR TREATMENT OF NON-SENSE MEDIATED RNA DECAY BASED CONDITIONS AND DISEASES**

(30) Priority: 23.10.2017 US 201762575924 P; 04.05.2018 US 201862667200 P
(62) Divisional of application: 18871437.2
(71) Applicant: Stoke Therapeutics, Inc., Bedford, MA 01730 (US)
(72) Inventor: AZNAREZ, Isabel, Boston, MA 02130 (US); JING, Enxuan, West Roxbury, MA 02132 (US); KACH, Jacob, Jamaica Plain, MA 02130 (US); VENKATESH, Aditya, Malden, MA 02148 (US); SCHARNER, Juergen, Arlington, MA 02476 (US); TICHO, Baruch, Newton, MA 02459 (US); LIAU, Gene, Wayland, MA 01778 (US)
(74) Representative: Avidity IP

(57) **Abstract**

Alternative splicing events in genes can lead to non-productive mRNA transcripts which in turn can lead to aberrant protein expression, and therapeutic agents which can target the alternative splicing events in genes can modulate the expression level of functional proteins in patients and/or inhibit aberrant protein expression. Such therapeutic agents can be used to treat a condition or disease caused by protein deficiency.

## Description

### CROSS-REFERENCE

This application claims the benefit of U.S. Provisional Application No. 62/575,924, filed on October 23, 2017, and U.S. Provisional Application No. 62/667,200, filed on May 4, 2018, each of which is incorporated herein by reference in its entirety.

### BACKGROUND

Alternative splicing events in genes can lead to non-productive mRNA transcripts which in turn can lead to aberrant protein expression, and therapeutic agents which can target the alternative splicing events in genes can modulate the expression level of functional proteins in patients and/or inhibit aberrant protein expression. Such therapeutic agents can be used to treat a condition or disease caused by protein deficiency.

### SUMMARY

Described herein, in certain embodiments, is a method of modulating expression of a target protein, by a cell having an mRNA that comprises a non-sense mediated RNA decay-inducing exon (NMD exon) and encodes the target protein, the method comprising contacting a therapeutic agent to the cell, whereby the therapeutic agent modulates splicing of the NMD exon from the mRNA, thereby modulating level of processed mRNA encoding the target protein, and modulating the expression of the target protein in the cell, wherein the target protein is selected from the group consisting of: AKT3, CACNA1A, CBS, CD46, CFH, CHD2, CLN3, COL11A2, COL4A3, COL4A4, COL4A4, CR1, CRX, CYP2J2, DHDDS, DNAJC8, EIF2AK3, ERN1, GALE, GUCY2F, GUCY2F, HEXA, HEXA, MAPK3, MBD5, MBD5, MBD5, MUT, MYH14, MYO6, NF1, NF2, NIPBL, NR1H4, NSD1, NSD1, NSD1, NSD1, OPA1, OPA1, PCCA, PKP2, PPARA, PRPF3, PRPF3, SCN2A, SCN8A, SCN8A, SCN9A, SEMA3C, SEMA3D, SIRT3, STK11, STK11, SYNGAP1, TOPORS, and VCAN proteins.

Described herein, in certain embodiments, is a method of treating a disease or condition in a subject in need thereof by modulating expression of a target protein in a cell of the subject, comprising: contacting the cell of the subject with a therapeutic agent that modulates splicing of a non-sense mediated mRNA decay-inducing exon (NMD exon) from an mRNA in the cell, wherein the mRNA comprises the NMD exon and encodes the target protein, thereby modulating level of processed mRNA encoding the target protein, and modulating expression of the target protein in the cell of the subject, wherein the target protein is selected from the group consisting of: AKT3, CACNA1A, CBS, CD46, CFH, CHD2, CLN3, COL11A2, COL4A3, COL4A4, COL4A4, CR1, CRX, CYP2J2, DHDDS, DNAJC8, EIF2AK3, ERN1, GALE, GUCY2F, GUCY2F, HEXA, HEXA, MAPK3, MBD5, MBD5, MBD5, MUT, MYH14, MYO6, NF1, NF2, NIPBL, NR1H4, NSD1, NSD1, NSD1, NSD1, OPA1, OPA1, PCCA, PKP2, PPARA, PRPF3, PRPF3, SCN2A, SCN8A, SCN8A, SCN9A, SEMA3C, SEMA3D, SIRT3, STK11, STK11, SYNGAP1, TOPORS, and VCAN proteins.

In some embodiments, the therapeutic agent: (a) binds to a targeted portion of the mRNA encoding the target protein; (b) modulates binding of a factor involved in splicing of the NMD exon; or (c) a combination of (a) and (b).

In some embodiments, the therapeutic agent interferes with binding of the factor involved in splicing of the NMD exon to a region of the targeted portion. In some embodiments, the targeted portion is proximal to the NMD exon. In some embodiments, the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream of 5' end of the NMD exon. In some embodiments, the targeted portion is at least about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides, about 40 nucleotides, about 30 nucleotides, about 20 nucleotides, about 10 nucleotides, about 5 nucleotides, about 4 nucleotides, about 2 nucleotides, about 1 nucleotides upstream of 5' end of the NMD exon. In some embodiments, the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream of 3' end of the NMD exon. In some embodiments, the targeted portion is at least about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides, about 40 nucleotides, about 30 nucleotides, about 20 nucleotides, about 10 nucleotides, about 5 nucleotides, about 4 nucleotides, about 2 nucleotides, about 1 nucleotides downstream of 3' end of the NMD exon.

In some embodiments, the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream of genomic site selected from the group consisting of: GRCh38/ hg38: chrl 243564388; GRCh38/ hg38: chr19 13236618; GRCh38/ hg38: chr21 43060012; GRCh38/ hg38: chr1 207775610; GRCh38/ hg38: chr1 196675450; GRCh38/ hg38: chr15 92998149; GRCh38/ hg38: chr16 28479765; GRCh38/ hg38: chr6 33183698; GRCh38/ hg38: chr2 227296487; GRCh38/ hg38: chr2 227144833; GRCh38/ hg38: chr2 227015360; GRCh38/ hg38: chr1 207637688; GRCh38/ hg38: chr19 47835403; GRCh38/ hg38: chr1 59904516; GRCh38/ hg38: chr1 26442335; GRCh38/ hg38: chr1 28230252; GRCh38/ hg38: chr2 88582824; GRCh38/ hg38: chr17 64102804; GRCh38/ hg38: chr1 23798484; GRCh38/ hg38: chrX 109383446; GRCh38/ hg38: chrX 109439175; GRCh38/ hg38: chr15 72362466; GRCh38/ hg38: chr15 72345776; GRCh38/ hg38: chr16 30115645; GRCh38/ hg38: chr2 148460219; GRCh38/ hg38: chr2 148490695; GRCh38/ hg38: chr2 148505761; GRCh38/ hg38: chr6 49436597; GRCh38/ hg38: chr19 50230825; GRCh38/ hg38: chr6 75867431; GRCh38/ hg38: chr17 31249955; GRCh38/ hg38: chr22 29628658; GRCh38/ hg38: chr5 37048127; GRCh38/ hg38: chr12 100499841; GRCh38/ hg38: chr5 177169394; GRCh38/ hg38: chr5 177200761; GRCh38/ hg38: chr5 177247924; GRCh38/ hg38: chr5 177275947; GRCh38/ hg38: chr3 193628509; GRCh38/ hg38: chr3 193603500; GRCh38/ hg38: chr13 100305751; GRCh38/ hg38: chr12 32894778; GRCh38/ hg38: chr22 46203575; GRCh38/ hg38: chr1 150327557; GRCh38/ hg38: chr1 150330401; GRCh38/ hg38: chr2 165327155; GRCh38/ hg38: chr12 51688758; GRCh38/ hg38: chr12 51780202; GRCh38/ hg38: chr2 166304329; GRCh38/ hg38: chr7 80794957; GRCh38/ hg38: chr7 85059541; GRCh38/ hg38: chr11 226081; GRCh38/ hg38: chr19 1216268; GRCh38/ hg38: chr19 1221621; GRCh38/ hg38: chr6 33448789; GRCh38/ hg38: chr9 32551469; and GRCh38/ hg38: chr5 83544965.

In some embodiments, the targeted portion is about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream of genomic site selected from the group consisting of: GRCh38/ hg38: chr1 243564388; GRCh38/ hg38: chr19 13236618; GRCh38/ hg38: chr21 43060012; GRCh38/ hg38: chr1 207775610; GRCh38/ hg38: chr1 196675450; GRCh38/ hg38: chr15 92998149; GRCh38/ hg38: chr16 28479765; GRCh38/ hg38: chr6 33183698; GRCh38/ hg38: chr2 227296487; GRCh38/ hg38: chr2 227144833; GRCh38/ hg38: chr2 227015360; GRCh38/ hg38: chr1 207637688; GRCh38/ hg38: chr19 47835403; GRCh38/ hg38: chr1 59904516; GRCh38/ hg38: chr1 26442335; GRCh38/ hg38: chr1 28230252; GRCh38/ hg38: chr2 88582824; GRCh38/ hg38: chr17 64102804; GRCh38/ hg38: chr1 23798484; GRCh38/ hg38: chrX 109383446; GRCh38/ hg38: chrX 109439175; GRCh38/ hg38: chr15 72362466; GRCh38/ hg38: chr15 72345776; GRCh38/ hg38: chr16 30115645; GRCh38/ hg38: chr2 148460219; GRCh38/ hg38: chr2 148490695; GRCh38/ hg38: chr2 148505761; GRCh38/ hg38: chr6 49436597; GRCh38/ hg38: chr19 50230825; GRCh38/ hg38: chr6 75867431; GRCh38/ hg38: chr17 31249955; GRCh38/ hg38: chr22 29628658; GRCh38/ hg38: chr5 37048127; GRCh38/ hg38: chr12 100499841; GRCh38/ hg38: chr5 177169394; GRCh38/ hg38: chr5 177200761; GRCh38/ hg38: chr5 177247924; GRCh38/ hg38: chr5 177275947; GRCh38/ hg38: chr3 193628509; GRCh38/ hg38: chr3 193603500; GRCh38/ hg38: chr13 100305751; GRCh38/ hg38: chr12 32894778; GRCh38/ hg38: chr22 46203575; GRCh38/ hg38: chr1 150327557; GRCh38/ hg38: chr1 150330401; GRCh38/ hg38: chr2 165327155; GRCh38/ hg38: chr12 51688758; GRCh38/ hg38: chr12 51780202; GRCh38/ hg38: chr2 166304329; GRCh38/ hg38: chr7 80794957; GRCh38/ hg38: chr7 85059541; GRCh38/ hg38: chr11 226081; GRCh38/ hg38: chr19 1216268; GRCh38/ hg38: chr19 1221621; GRCh38/ hg38: chr6 33448789; GRCh38/ hg38: chr9 32551469; and GRCh38/ hg38: chr5 83544965.

In some embodiments, the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream of genomic site selected from the group consisting of: GRCh38/ hg38: chr1 243564285; GRCh38/ hg38: chr19 13236449; GRCh38/ hg38: chr21 43059730; GRCh38/ hg38: chr1 207775745; GRCh38/ hg38: chr1 196675529; GRCh38/ hg38: chr15 92998261; GRCh38/ hg38: chr16 28479644; GRCh38/ hg38: chr6 33183634; GRCh38/ hg38: chr2 227296526; GRCh38/ hg38: chr2 227144653; GRCh38/ hg38: chr2 227015283; GRCh38/ hg38: chr1 207637848; GRCh38/ hg38: chr19 47835579; GRCh38/ hg38: chr1 59904366; GRCh38/ hg38: chr1 26442372; GRCh38/ hg38: chr1 28230131; GRCh38/ hg38: chr2 88582755; GRCh38/ hg38: chr17 64102673; GRCh38/ hg38: chr1 23798311; GRCh38/ hg38: chrX 109383365; GRCh38/ hg38: chrX 109439038; GRCh38/ hg38: chr15 72362376; GRCh38/ hg38: chr15 72345677; GRCh38/ hg38: chr16 30115595; GRCh38/ hg38: chr2 148460304; GRCh38/ hg38: chr2 148490787; GRCh38/ hg38: chr2 148505830; GRCh38/ hg38: chr6 49436522; GRCh38/ hg38: chr19 50230999; GRCh38/ hg38: chr6 75867523; GRCh38/ hg38: chr17 31250125; GRCh38/ hg38: chr22 29628773; GRCh38/ hg38: chr5 37048354; GRCh38/ hg38: chr12 100500024; GRCh38/ hg38: chr5 177169559; GRCh38/ hg38: chr5 177200783; GRCh38/ hg38: chr5 177248079; GRCh38/ hg38: chr5 177276101; GRCh38/ hg38: chr3 193628616; GRCh38/ hg38: chr3 193603557; GRCh38/ hg38: chr13 100305834; GRCh38/ hg38: chr12 32894516; GRCh38/ hg38: chr22 46203752; GRCh38/ hg38: chr1 150327652; GRCh38/ hg38: chr1 150330498; GRCh38/ hg38: chr2 165327202; GRCh38/ hg38: chr12 51688849; GRCh38/ hg38: chr12 51780271; GRCh38/ hg38: chr2 166304238; GRCh38/ hg38: chr7 80794854; GRCh38/ hg38: chr7 85059498; GRCh38/ hg38: chr11 225673; GRCh38/ hg38: chr19 1216398; GRCh38/ hg38: chr19 1221846; GRCh38/ hg38: chr6 33448868; GRCh38/ hg38: chr9 32551365; and GRCh38/ hg38: chr5 83545070.

In some embodiments, the targeted portion is about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream of genomic site selected from the group consisting of: GRCh38/ hg38: chr1 243564285; GRCh38/ hg38: chr19 13236449; GRCh38/ hg38: chr21 43059730; GRCh38/ hg38: chr1 207775745; GRCh38/ hg38: chr1 196675529; GRCh38/ hg38: chr15 92998261; GRCh38/ hg38: chr16 28479644; GRCh38/ hg38: chr6 33183634; GRCh38/ hg38: chr2 227296526; GRCh38/ hg38: chr2 227144653; GRCh38/ hg38: chr2 227015283; GRCh38/ hg38: chr1 207637848; GRCh38/ hg38: chr19 47835579; GRCh38/ hg38: chr1 59904366; GRCh38/ hg38: chr1 26442372; GRCh38/ hg38: chr1 28230131; GRCh38/ hg38: chr2 88582755; GRCh38/ hg38: chr17 64102673; GRCh38/ hg38: chr1 23798311; GRCh38/ hg38: chrX 109383365; GRCh38/ hg38: chrX 109439038; GRCh38/ hg38: chr15 72362376; GRCh38/ hg38: chr15 72345677; GRCh38/ hg38: chr16 30115595; GRCh38/ hg38: chr2 148460304; GRCh38/ hg38: chr2 148490787; GRCh38/ hg38: chr2 148505830; GRCh38/ hg38: chr6 49436522; GRCh38/ hg38: chr19 50230999; GRCh38/ hg38: chr6 75867523; GRCh38/ hg38: chr17 31250125; GRCh38/ hg38: chr22 29628773; GRCh38/ hg38: chr5 37048354; GRCh38/ hg38: chr12 100500024; GRCh38/ hg38: chr5 177169559; GRCh38/ hg38: chr5 177200783; GRCh38/ hg38: chr5 177248079; GRCh38/ hg38: chr5 177276101; GRCh38/ hg38: chr3 193628616; GRCh38/ hg38: chr3 193603557; GRCh38/ hg38: chr13 100305834; GRCh38/ hg38: chr12 32894516; GRCh38/ hg38: chr22 46203752; GRCh38/ hg38: chr1 150327652; GRCh38/ hg38: chr1 150330498; GRCh38/ hg38: chr2 165327202; GRCh38/ hg38: chr12 51688849; GRCh38/ hg38: chr12 51780271; GRCh38/ hg38: chr2 166304238; GRCh38/ hg38: chr7 80794854; GRCh38/ hg38: chr7 85059498; GRCh38/ hg38: chr11 225673; GRCh38/ hg38: chr19 1216398; GRCh38/ hg38: chr19 1221846; GRCh38/ hg38: chr6 33448868; GRCh38/ hg38: chr9 32551365; and GRCh38/ hg38: chr5 83545070.

In some embodiments, the targeted portion is located in an intronic region between two canonical exonic regions of the mRNA encoding the target protein, and wherein the intronic region contains the NMD exon. In some embodiments, the targeted portion at least partially overlaps with the NMD exon. In some embodiments, the targeted portion at least partially overlaps with an intron upstream or downstream of the NMD exon. In some embodiments, the targeted portion comprises 5' NMD exon-intron junction or 3' NMD exon-intron junction. In some embodiments, the targeted portion is within the NMD exon. In some embodiments, the targeted portion comprises about 5, 6, 7, 8, 9, 10, 11, 12, 13, 14, 15, 16, 17, 18, 19, 20, 21, 22, 23, 24, 25, 26, 27, 28, 29, 30, or more consecutive nucleotides of the NMD exon.

In some embodiments, the mRNA encoding the target protein comprises a sequence with at least about 80%, 85%, 90%, 95%, 97%, or 100% sequence identity to a sequence selected from the group consisting of SEQ ID NOs: 135-191. In some embodiments, the mRNA encoding the target protein is encoded by a genetic sequence with at least about 80%, 85%, 90%, 95%, 97%, or 100% sequence identity to a sequence selected from the group consisting of SEQ ID NOs: 1-5, 12, 19-21, 25, 26, 28, 30, 33, 35, 38, 40, 41, 44, 45, 51, 53, 55-57, and 192-211. In some embodiments, the targeted portion of the mRNA comprises a sequence with at least 80%, 85%, 90%, 95%, 97%, or 100% sequence identity to a region comprising at least 8 contiguous nucleic acids of a sequence selected from the group consisting of SEQ ID NOs: 135-191. In some embodiments, the agent is an antisense oligomer (ASO) and wherein the ASO comprises a sequence that is at least about 80%, 85%, 90%, 95%, 97%, or 100% complementary to at least 8 contiguous nucleic acids of a sequence selected from the group consisting of SEQ ID NOs: 135-191.

In some embodiments, the targeted portion of the mRNA is within the non-sense mediated RNA decay-inducing exon selected from the group consisting of: GRCh38/ hg38: chr1 243564285 243564388 ; GRCh38/ hg38: chr19 13236449 13236618; GRCh38/ hg38: chr21 43059730 43060012; GRCh38/ hg38: chr1 207775610 207775745; GRCh38/ hg38: chr1 196675450 196675529; GRCh38/ hg38: chr15 92998149 92998261; GRCh38/ hg38: chr16 28479644 28479765; GRCh38/ hg38: chr6 33183634 33183698; GRCh38/ hg38: chr2 227296487 227296526; GRCh38/ hg38: chr2 227144653 227144833; GRCh38/ hg38: chr2 227015283 227015360; GRCh38/ hg38: chr1 207637688 207637848; GRCh38/ hg38: chr19 47835403 47835579; GRCh38/ hg38: chr1 59904366 59904516; GRCh38/ hg38: chr1 26442335 26442372; GRCh38/ hg38: chr1 28230131 28230252 ; GRCh38/ hg38: chr2 88582755 88582824; GRCh38/ hg38: chr17 64102673 64102804; GRCh38/ hg38: chr1 23798311 23798484; GRCh38/ hg38: chrX 109383365 109383446; GRCh38/ hg38: chrX 109439038 109439175; GRCh38/ hg38: chr15 72362376 72362466; GRCh38/ hg38: chr15 72345677 72345776; GRCh38/ hg38: chr16 30115595 30115645; GRCh38/ hg38: chr2 148460219 148460304; GRCh38/ hg38: chr2 148490695 148490787; GRCh38/ hg38: chr2 148505761 148505830; GRCh38/ hg38: chr6 49436522 49436597; GRCh38/ hg38: chr19 50230825 50230999; GRCh38/ hg38: chr6 75867431 75867523; GRCh38/ hg38: chr17 31249955 31250125; GRCh38/ hg38: chr22 29628658 29628773; GRCh38/ hg38: chr5 37048127 37048354; GRCh38/ hg38: chr12 100499841 100500024; GRCh38/ hg38: chr5 177169394 177169559; GRCh38/ hg38: chr5 177200761 177200783; GRCh38/ hg38: chr5 177247924 177248079; GRCh38/ hg38: chr5 177275947 177276101; GRCh38/ hg38: chr3 193628509 193628616; GRCh38/ hg38: chr3 193603500 193603557; GRCh38/ hg38: chr13 100305751 100305834; GRCh38/ hg38: chr12 32894516 32894778; GRCh38/ hg38: chr22 46203575 46203752; GRCh38/ hg38: chr1 150327557 150327652; GRCh38/ hg38: chr1 150330401 150330498; GRCh38/ hg38: chr2 165327155 165327202; GRCh38/ hg38: chr12 51688758 51688849; GRCh38/ hg38: chr12 51780202 51780271; GRCh38/ hg38: chr2 166304238 166304329; GRCh38/ hg38: chr7 80794854 80794957; GRCh38/ hg38: chr7 85059498 85059541; GRCh38/ hg38: chr11 225673 226081; GRCh38/ hg38: chr19 1216268 1216398; GRCh38/ hg38: chr19 1221621 1221846; GRCh38/ hg38: chr6 33448789 33448868; GRCh38/ hg38: chr9 32551365 32551469; and GRCh38/ hg38: chr5 83544965 83545070.

In some embodiments, the targeted portion of the mRNA is upstream or downstream of the non-sense mediated RNA decay-inducing exon selected from the group consisting of: GRCh38/ hg38: chr1 243564285 243564388 ; GRCh38/ hg38: chr19 13236449 13236618; GRCh38/ hg38: chr21 43059730 43060012; GRCh38/ hg38: chr1 207775610 207775745; GRCh38/ hg38: chr1 196675450 196675529; GRCh38/ hg38: chr15 92998149 92998261; GRCh38/ hg38: chr16 28479644 28479765; GRCh38/ hg38: chr6 33183634 33183698; GRCh38/ hg38: chr2 227296487 227296526; GRCh38/ hg38: chr2 227144653 227144833; GRCh38/ hg38: chr2 227015283 227015360; GRCh38/ hg38: chr1 207637688 207637848; GRCh38/ hg38: chr19 47835403 47835579; GRCh38/ hg38: chr1 59904366 59904516; GRCh38/ hg38: chr1 26442335 26442372; GRCh38/ hg38: chr1 28230131 28230252 ; GRCh38/ hg38: chr2 88582755 88582824; GRCh38/ hg38: chr17 64102673 64102804; GRCh38/ hg38: chr1 23798311 23798484; GRCh38/ hg38: chrX 109383365 109383446; GRCh38/ hg38: chrX 109439038 109439175; GRCh38/ hg38: chr15 72362376 72362466; GRCh38/ hg38: chr15 72345677 72345776; GRCh38/ hg38: chr16 30115595 30115645; GRCh38/ hg38: chr2 148460219 148460304; GRCh38/ hg38: chr2 148490695 148490787; GRCh38/ hg38: chr2 148505761 148505830; GRCh38/ hg38: chr6 49436522 49436597; GRCh38/ hg38: chr19 50230825 50230999; GRCh38/ hg38: chr6 75867431 75867523; GRCh38/ hg38: chr17 31249955 31250125; GRCh38/ hg38: chr22 29628658 29628773; GRCh38/ hg38: chr5 37048127 37048354; GRCh38/ hg38: chr12 100499841 100500024; GRCh38/ hg38: chr5 177169394 177169559; GRCh38/ hg38: chr5 177200761 177200783; GRCh38/ hg38: chr5 177247924 177248079; GRCh38/ hg38: chr5 177275947 177276101; GRCh38/ hg38: chr3 193628509 193628616; GRCh38/ hg38: chr3 193603500 193603557; GRCh38/ hg38: chr13 100305751 100305834; GRCh38/ hg38: chr12 32894516 32894778; GRCh38/ hg38: chr22 46203575 46203752; GRCh38/ hg38: chr1 150327557 150327652; GRCh38/ hg38: chr1 150330401 150330498; GRCh38/ hg38: chr2 165327155 165327202; GRCh38/ hg38: chr12 51688758 51688849; GRCh38/ hg38: chr12 51780202 51780271; GRCh38/ hg38: chr2 166304238 166304329; GRCh38/ hg38: chr7 80794854 80794957; GRCh38/ hg38: chr7 85059498 85059541; GRCh38/ hg38: chr11 225673 226081; GRCh38/ hg38: chr19 1216268 1216398; GRCh38/ hg38: chr19 1221621 1221846; GRCh38/ hg38: chr6 33448789 33448868; GRCh38/ hg38: chr9 32551365 32551469; and GRCh38/ hg38: chr5 83544965 83545070.

In some embodiments, the targeted portion of the mRNA comprises an exon-intron junction of exon selected from the group consisting of: GRCh38/ hg38: chr1 243564285 243564388; GRCh38/ hg38: chr19 13236449 13236618; GRCh38/ hg38: chr21 43059730 43060012; GRCh38/ hg38: chr1 207775610 207775745; GRCh38/ hg38: chr1 196675450 196675529; GRCh38/ hg38: chr15 92998149 92998261; GRCh38/ hg38: chr16 28479644 28479765; GRCh38/ hg38: chr6 33183634 33183698; GRCh38/ hg38: chr2 227296487 227296526; GRCh38/ hg38: chr2 227144653 227144833; GRCh38/ hg38: chr2 227015283 227015360; GRCh38/ hg38: chr1 207637688 207637848; GRCh38/ hg38: chr19 47835403 47835579; GRCh38/ hg38: chr1 59904366 59904516; GRCh38/ hg38: chr1 26442335 26442372; GRCh38/ hg38: chr1 28230131 28230252 ; GRCh38/ hg38: chr2 88582755 88582824; GRCh38/ hg38: chr17 64102673 64102804; GRCh38/ hg38: chr1 23798311 23798484; GRCh38/ hg38: chrX 109383365 109383446; GRCh38/ hg38: chrX 109439038 109439175; GRCh38/ hg38: chr15 72362376 72362466; GRCh38/ hg38: chr15 72345677 72345776; GRCh38/ hg38: chr16 30115595 30115645; GRCh38/ hg38: chr2 148460219 148460304; GRCh38/ hg38: chr2 148490695 148490787; GRCh38/ hg38: chr2 148505761 148505830; GRCh38/ hg38: chr6 49436522 49436597; GRCh38/ hg38: chr19 50230825 50230999; GRCh38/ hg38: chr6 75867431 75867523; GRCh38/ hg38: chr17 31249955 31250125; GRCh38/ hg38: chr22 29628658 29628773; GRCh38/ hg38: chr5 37048127 37048354; GRCh38/ hg38: chr12 100499841 100500024; GRCh38/ hg38: chr5 177169394 177169559; GRCh38/ hg38: chr5 177200761 177200783; GRCh38/ hg38: chr5 177247924 177248079; GRCh38/ hg38: chr5 177275947 177276101; GRCh38/ hg38: chr3 193628509 193628616; GRCh38/ hg38: chr3 193603500 193603557; GRCh38/ hg38: chr13 100305751 100305834; GRCh38/ hg38: chr12 32894516 32894778; GRCh38/ hg38: chr22 46203575 46203752; GRCh38/ hg38: chr1 150327557 150327652; GRCh38/ hg38: chr1 150330401 150330498; GRCh38/ hg38: chr2 165327155 165327202; GRCh38/ hg38: chr12 51688758 51688849; GRCh38/ hg38: chr12 51780202 51780271; GRCh38/ hg38: chr2 166304238 166304329; GRCh38/ hg38: chr7 80794854 80794957; GRCh38/ hg38: chr7 85059498 85059541; GRCh38/ hg38: chr11 225673 226081; GRCh38/ hg38: chr19 1216268 1216398; GRCh38/ hg38: chr19 1221621 1221846; GRCh38/ hg38: chr6 33448789 33448868; GRCh38/ hg38: chr9 32551365 32551469; and GRCh38/ hg38: chr5 83544965 83545070.

In some embodiments, the target protein produced is a full-length protein or a wild-type protein.

In some embodiments, the therapeutic agent promotes exclusion of the NMD exon from the processed mRNA encoding the target protein. In some embodiments, exclusion of the NMD exon from the processed mRNA encoding the target protein in the cell contacted with the therapeutic agent is increased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1. 1-fold, at least about 1.5-fold, at least about 2-fold, at least about 2.5-fold, at least about 3-fold, at least about 3.5-fold, at least about 4-fold, at least about 5-fold, or at least about 10-fold, compared to exclusion of the NMD exon from the processed mRNA encoding the target protein in a control cell. In some embodiments, the therapeutic agent increases the level of the processed mRNA encoding the target protein in the cell. In some embodiments, the level of the processed mRNA encoding the target protein produced in the cell contacted with the therapeutic agent is increased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the processed mRNA encoding the target protein in a control cell. In some embodiments, the therapeutic agent increases the expression of the target protein in the cell. In some embodiments, a level of the target protein produced in the cell contacted with the therapeutic agent is increased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the target protein produced in a control cell.

In some embodiments, the disease or condition is induced by a loss-of-function mutation in the target protein.

In some embodiments, the disease or condition is associated with haploinsufficiency of a gene encoding the target protein, and wherein the subject has a first allele encoding a functional target protein, and a second allele from which the target protein is not produced or produced at a reduced level, or a second allele encoding a nonfunctional target protein or a partially functional target protein. In some embodiments, the disease or condition is selected from the group consisting of: Sotos syndrome 1; Beckwith-Wiedemann syndrome; Migraine, familial hemiplegic, 1; Episodic ataxia, type 2; Epileptic encephalopathy, childhood-onset; Wagner syndrome 1; Optic atrophy type 1; Alport syndrome; Arrhythmogenic right ventricular dysplasia 9; Neurofibromatosis type 1; Epileptic encephalopathy, early infantile, 11; Seizures, benign familial infantile, 3; Cognitive impairment with or without cerebellar ataxia; Epileptic encephalopathy, early infantile, 13; Seizures, benign familial infantile, 5; Pathway (CNS); 16p11.2 deletion syndrome?; Mental retardation, autosomal dominant 1; Retinitis pigmentosa 18; Retinitis pigmentosa 31; Deafness, autosomal dominant 13; Cone-rod retinal dystrophy-2; Deafness, autosomal dominant 4A; Peripheral neuropathy, myopathy, hoarseness, and hearing loss ; Deafness, autosomal dominant 22; Neurofibromatosis type 2; Mental retardation, autosomal dominant 5; Epilepsy, generalized, with febrile seizures plus, type 7; and Febrile seizures, familial, 3B.

In some embodiments,the disease or condition is associated with an autosomal recessive mutation of a gene encoding the target protein, wherein the subject has a first allele encoding from which: (i) the target protein is not produced or produced at a reduced level compared to a wild-type allele; or (ii) the target protein produced is nonfunctional or partially functional compared to a wild-type allele, and a second allele from which: (iii) the target protein is produced at a reduced level compared to a wild-type allele and the target protein produced is at least partially functional compared to a wild-type allele; or (iv) the target protein produced is partially functional compared to a wild-type allele. In some embodiments, the disease or condition is selected from the group consisting of: Alport syndrome; Ceroid lipofuscinosis, neuronal, 3; Galactose epimerase deficiency; Homocystinuria, B6-responsive and nonresponsive types; Methyl Malonic Aciduria; Propionic acidemia; Retinitis pigmentosa 59; Tay-Sachs disease; Insensitivity to pain, congenital; and HSAN2D, autosomal recessive.

In some embodiments, the therapeutic agent promotes exclusion of the NMD exon from the processed mRNA encoding the target protein and increases the expression of the target protein in the cell. In some embodiments, the therapeutic agent inhibits exclusion of the NMD exon from the processed mRNA encoding the target protein. In some embodiments, exclusion of the NMD exon from the processed mRNA encoding the target protein in the cell contacted with the therapeutic agent is decreased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1. 1-fold, at least about 1.5-fold, at least about 2-fold, at least about 2.5-fold, at least about 3-fold, at least about 3.5-fold, at least about 4-fold, at least about 5-fold, or at least about 10-fold, compared to exclusion of the NMD exon from the processed mRNA encoding the target protein in a control cell. In some embodiments, the therapeutic agent decreases the level of the processed mRNA encoding the target protein in the cell. In some embodiments, the level of the processed mRNA encoding the target protein in the cell contacted with the therapeutic agent is decreased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1. 1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the processed mRNA encoding the target protein in a control cell.

In some embodiments, the therapeutic agent decreases the expression of the target protein in the cell. In some embodiments, a level of the target protein produced in the cell contacted with the therapeutic agent is decreased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1. 1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the target protein produced in a control cell.

In some embodiments, the disease or condition is induced by a gain-of-function mutation in the target protein. In some embodiments, the subject has an allele from which the target protein is produced at an increased level, or an allele encoding a mutant target protein that exhibits increased activity in the cell.

In some embodiments, the therapeutic agent inhibits exclusion of the NMD exon from the processed mRNA encoding the target protein and decreases the expression of the target protein in the cell. In some embodiments, the target protein comprises SCN8A. In some embodiments, the disease or condition comprises a central nervous system disease. In some embodiments, the disease or condition comprises epilepsy. In some embodiments, the disease or condition comprises Dravet syndrome.

In some embodiments, the therapeutic agent is an antisense oligomer (ASO) and wherein the antisense oligomer comprises a backbone modification comprising a phosphorothioate linkage or a phosphorodiamidate linkage. In some embodiments, the therapeutic agent is an antisense oligomer (ASO) and wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.

In some embodiments, the therapeutic agent is an antisense oligomer (ASO) and wherein the antisense oligomer comprises at least one modified sugar moiety. In some embodiments, each sugar moiety is a modified sugar moiety.

In some embodiments, the therapeutic agent is an antisense oligomer (ASO) and wherein the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.

In some embodiments, the therapeutic agent is an antisense oligomer (ASO) and wherein the antisense oligomer is at least 80%, at least 85%, at least 90%, at least 95%, at least 98%, at least 99%, or 100%, complementary to the targeted portion of the mRNA.

In some embodiments, the method further comprises assessing mRNA level or expression level of the target protein.

In some embodiments, the subject is a human. In some embodiments, the subject is a non-human animal. In some embodiments, the subject is a fetus, an embryo, or a child. In some embodiments, the cells are ex vivo. In some embodiments, the therapeutic agent is administered by intrathecal injection, intracerebroventricular injection, intraperitoneal injection, intramuscular injection, subcutaneous injection, intravitreal, or intravenous injection of the subject. In some embodiments, the method further comprises administering a second therapeutic agent to the subj ect.

In some embodiments, the second therapeutic agent is a small molecule. In some embodiments, the second therapeutic agent is an antisense oligomer. In some embodiments, the second therapeutic agent corrects intron retention.

In some embodiments, the disease or condition is selected from the group consisting of: 16p11.2 deletion syndrome; Alport syndrome; Arrhythmogenic right ventricular dysplasia 9; Ceroid lipofuscinosis, neuronal, 3; Cognitive impairment with or without cerebellar ataxia; Epileptic encephalopathy, early infantile, 13; Seizures, benign familial infantile, 5; Cone-rod retinal dystrophy-2; Cornelia de Lange; Deafness, autosomal dominant 13; Deafness, autosomal dominant 4A; Peripheral neuropathy, myopathy, hoarseness, and hearing loss ; Epilepsy, generalized, with febrile seizures plus, type 7; Febrile seizures, familial, 3B; Insensitivity to pain, congenital; HSAN2D, autosomal recessive; Epileptic encephalopathy, childhood-onset; Epileptic encephalopathy, early infantile, 11; Seizures, benign familial infantile, 3; Galactose epimerase deficiency; Homocystinuria, B6-responsive and nonresponsive types; Mental retardation, autosomal dominant 1; Mental retardation, autosomal dominant 5; Methyl Malonic Aciduria; Migraine, familial hemiplegic, 1; Episodic ataxia, type 2; NASH; Neurofibromatosis type 1; Neurofibromatosis type 2; Optic atrophy type 1; Propionic acidemia; Retinitis pigmentosa 18; Sotos syndrome 1; Beckwith-Wiedemann syndrome; Tay-Sachs disease; and Wagner syndrome 1.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:

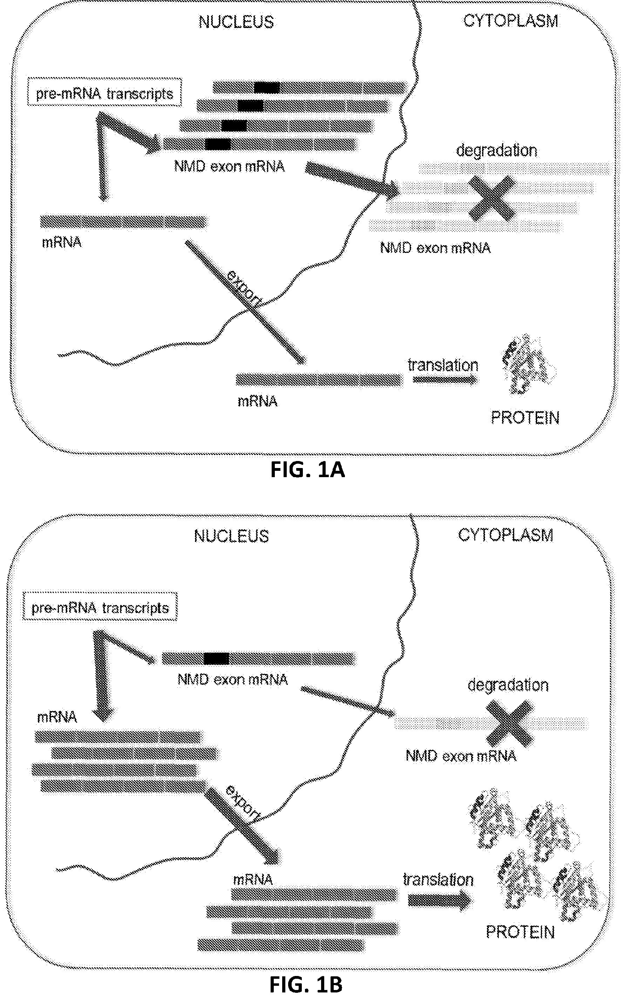

**FIG. 1** depicts a schematic representation of a target mRNA that contains a non-sense mediated mRNA decay-inducing exon (NMD exon mRNA) and therapeutic agent-mediated exclusion of the nonsense-mediated mRNA decay-inducing exon to increase expression of the full-length target protein or functional RNA. **FIG. 1A** shows a cell divided into nuclear and cytoplasmic compartments. In the nucleus, a pre-mRNA transcript of a target gene undergoes splicing to generate mRNA, and this mRNA is exported to the cytoplasm and translated into target protein. For this target gene, some fraction of the mRNA contains a nonsense-mediated mRNA decay-inducing exon (NMD exon mRNA) that is degraded in the cytoplasm, thus leading to no target protein production.
**FIG. 1B** shows an example of the same cell divided into nuclear and cytoplasmic compartments. Treatment with a therapeutic agent, such as an antisense oligomer (ASO), promotes the exclusion of the nonsense-mediated mRNA decay-inducing exon and results in an increase in mRNA, which is in turn translated into higher levels of target protein

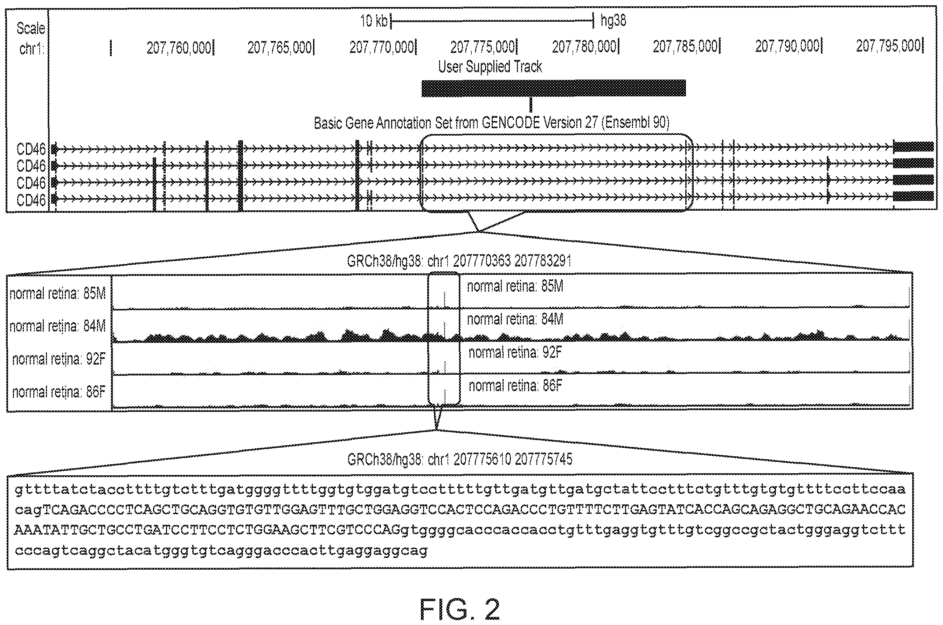

**FIG. 2** depicts identification of an exemplary nonsense-mediated mRNA decay (NMD)-inducing exon in the *CD46* gene. The identification of the NMD-inducing exon in the *CD46* gene using RNA sequencing is shown, visualized in the UCSC genome browser. The upper panel shows a graphic representation of the *CD46* gene to scale. Peaks corresponding to RNA sequencing reads were identified in intron GRCh38/hg38: chr1 207770363 207783291, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

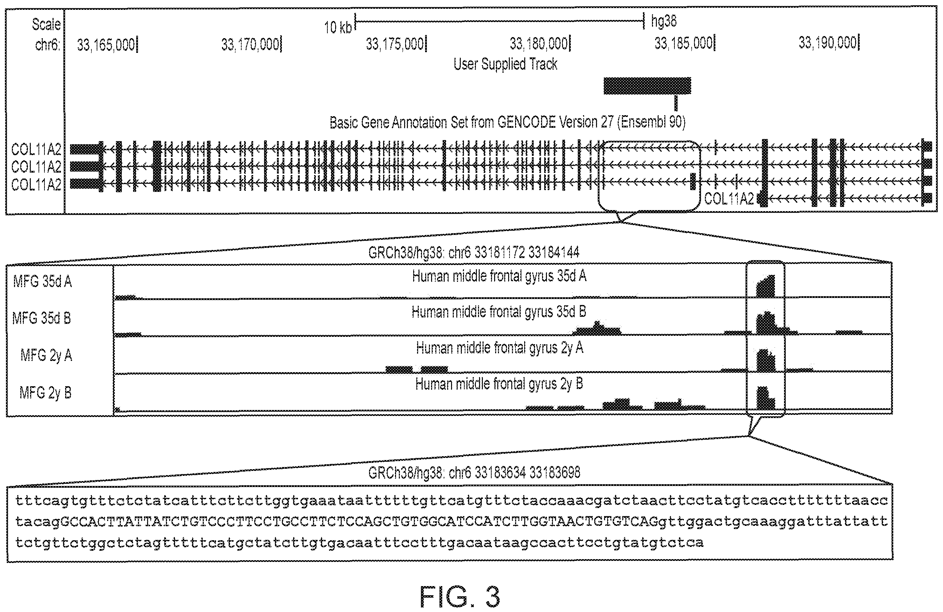

**FIG. 3** depicts identification of an exemplary nonsense-mediated mRNA decay (NMD)-inducing exon in the *COL11A2* gene. The identification of the NMD-inducing exon in the *COL11A2* gene using RNA sequencing is shown, visualized in the UCSC genome browser. The upper panel shows a graphic representation of the *COL11A2* gene to scale. Peaks corresponding to RNA sequencing reads were identified in intron GRCh38/hg38: chr6 33181172 33184144, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

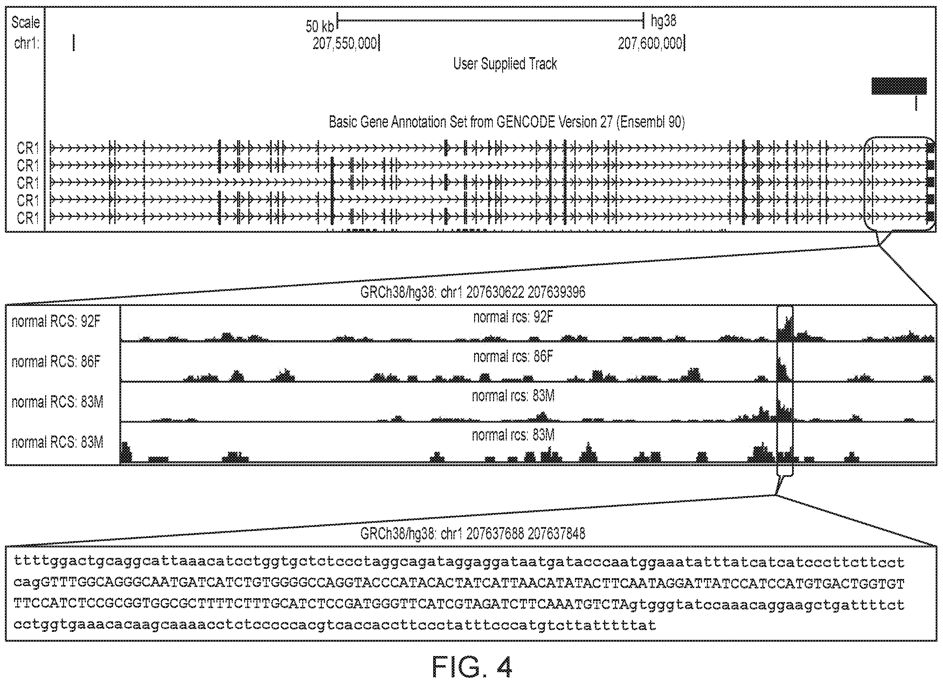

**FIG. 4** depicts identification of an exemplary nonsense-mediated mRNA decay (NMD)-inducing exon in the *CR1* gene. The identification of the NMD-inducing exon in the *CR1* gene using RNA sequencing is shown, visualized in the UCSC genome browser. The upper panel shows a graphic representation of the *CR1* gene to scale. Peaks corresponding to RNA sequencing reads were identified in intron GRCh38/hg38: chr1 207630622 207639396, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

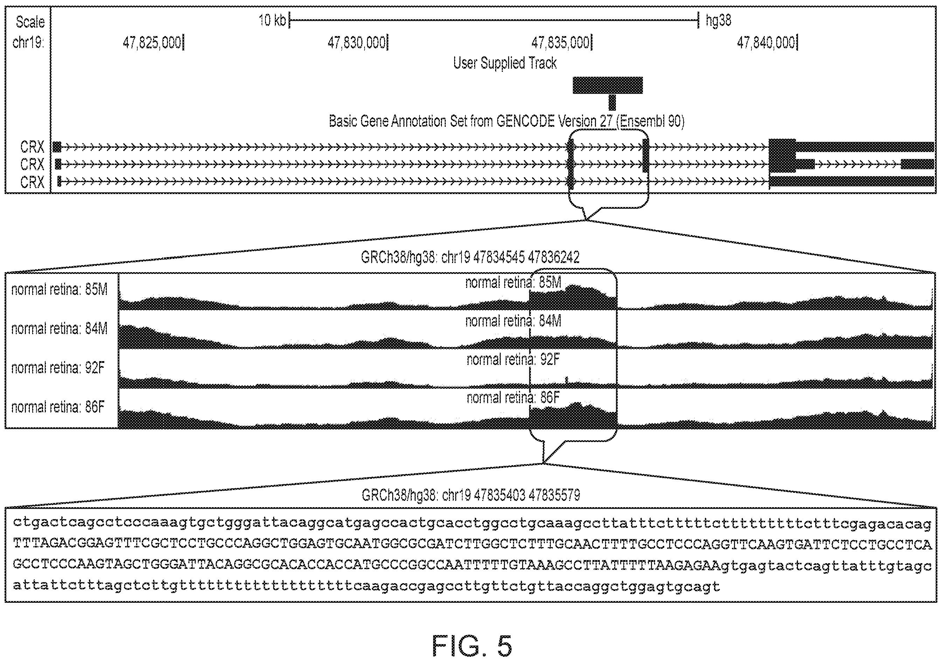

**FIG. 5** depicts identification of an exemplary nonsense-mediated mRNA decay (NMD)-inducing exon in the *CRX* gene. The identification of the NMD-inducing exon in the *CRX* gene using RNA sequencing is shown, visualized in the UCSC genome browser. The upper panel shows a graphic representation of the *CRX* gene to scale. Peaks corresponding to RNA sequencing reads were identified in intron GRCh38/hg38: chr19 47834545 47836242, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

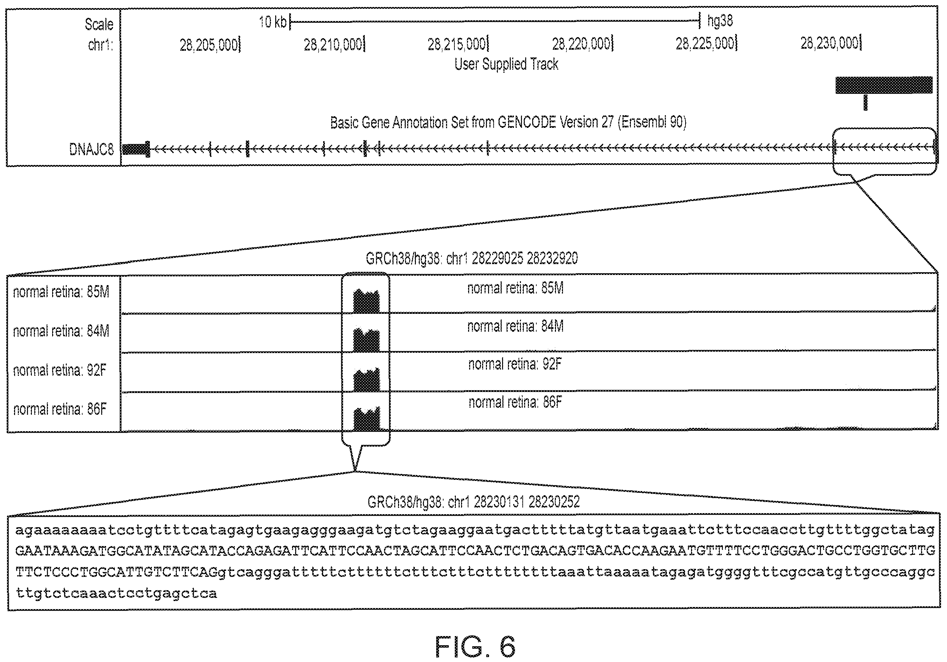

**FIG. 6** depicts identification of an exemplary nonsense-mediated mRNA decay (NMD)-inducing exon in the *DNAJC8* gene. The identification of the NMD-inducing exon in the *DNAJC8* gene using RNA sequencing is shown, visualized in the UCSC genome browser. The upper panel shows a graphic representation of the *DNAJC8* gene to scale. Peaks corresponding to RNA sequencing reads were identified in intron GRCh38/hg38: chr1 28229025 28232920, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

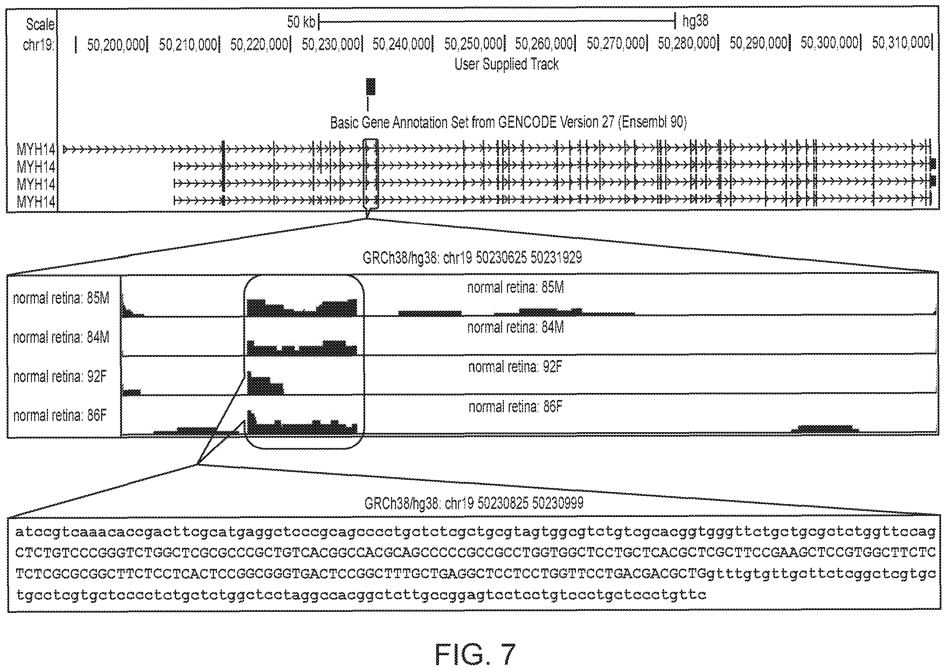

**FIG. 7** depicts identification of an exemplary nonsense-mediated mRNA decay (NMD)-inducing exon in the *MYH14* gene. The identification of the NMD-inducing exon in the *MYH14* gene using RNA sequencing is shown, visualized in the UCSC genome browser. The upper panel shows a graphic representation of the *MYH14* gene to scale. Peaks corresponding to RNA sequencing reads were identified in intron GRCh38/hg38: chr19 50230625 50231929, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

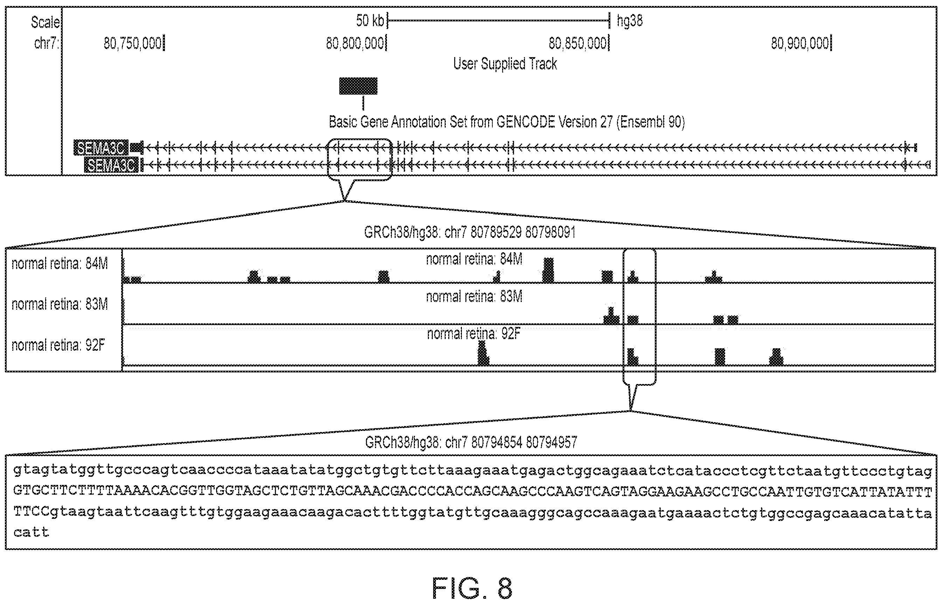

**FIG. 8** depicts identification of an exemplary nonsense-mediated mRNA decay (NMD)-inducing exon in the *SEMA3C* gene. The identification of the NMD-inducing exon in the *SEMA3C* gene using RNA sequencing is shown, visualized in the UCSC genome browser. The upper panel shows a graphic representation of the *SEMA3C* gene to scale. Peaks corresponding to RNA sequencing reads were identified in intron GRCh38/hg38: chr7 80789529 80798091, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

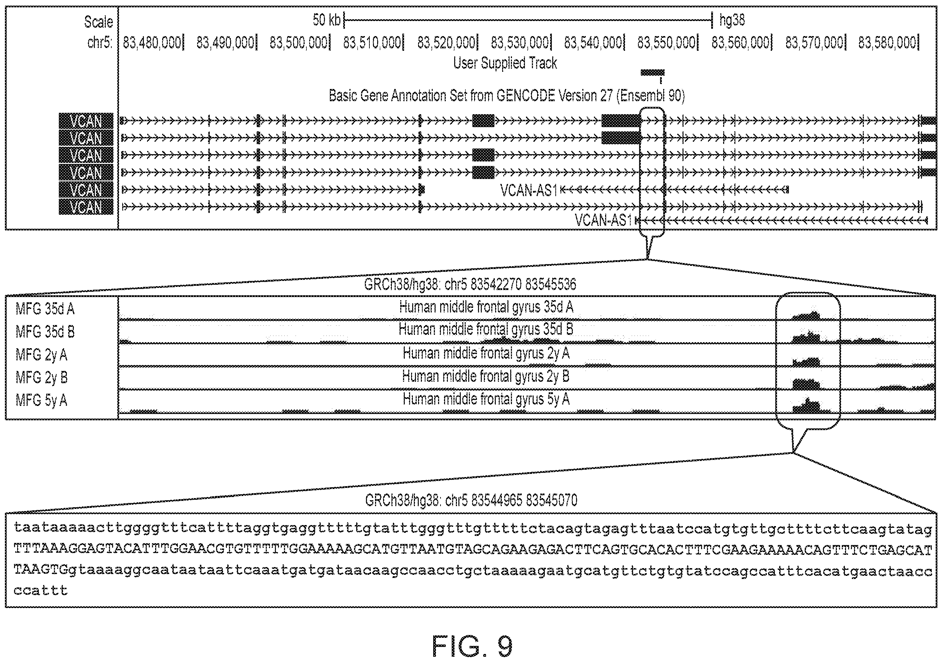

**FIG. 9** depicts identification of an exemplary nonsense-mediated mRNA decay (NMD)-inducing exon in the *VCAN* gene. The identification of the NMD-inducing exon in the *VCAN* gene using RNA sequencing is shown, visualized in the UCSC genome browser. The upper panel shows a graphic representation of the *VCAN* gene to scale. Peaks corresponding to RNA sequencing reads were identified in intron GRCh38/hg38: chr5 83542270 83545536, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

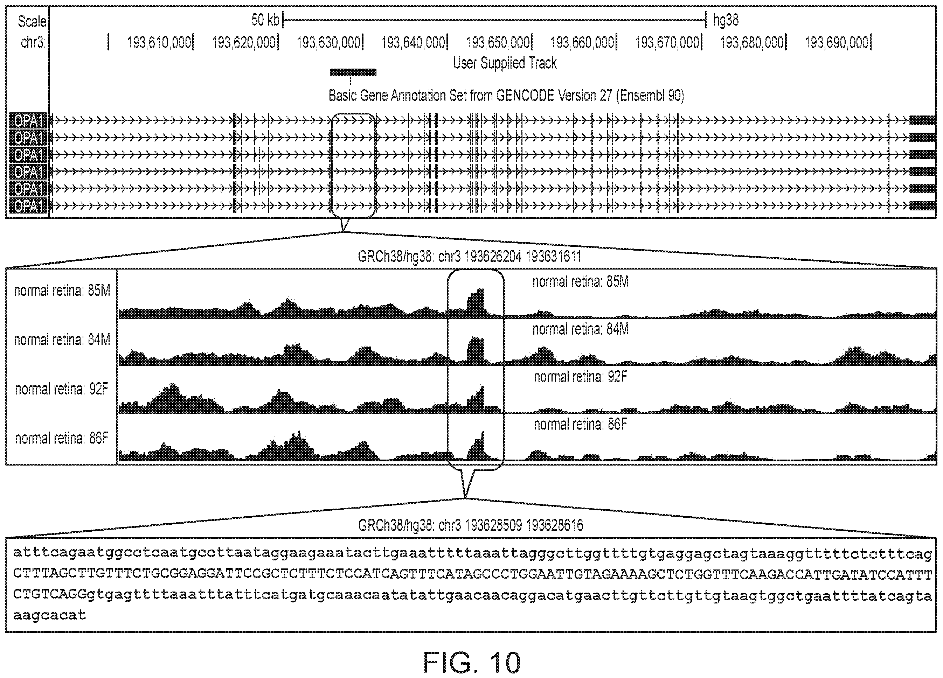

**FIG. 10** depicts identification of an exemplary nonsense-mediated mRNA decay (NMD)-inducing exon in the *OPAI* gene. The identification of the NMD-inducing exon in the *OPAI* gene using RNA sequencing is shown, visualized in the UCSC genome browser. The upper panel shows a graphic representation of the *OPAI* gene to scale. Peaks corresponding to RNA sequencing reads were identified in intron GRCh38/hg38: chr3 193626204 193631611, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

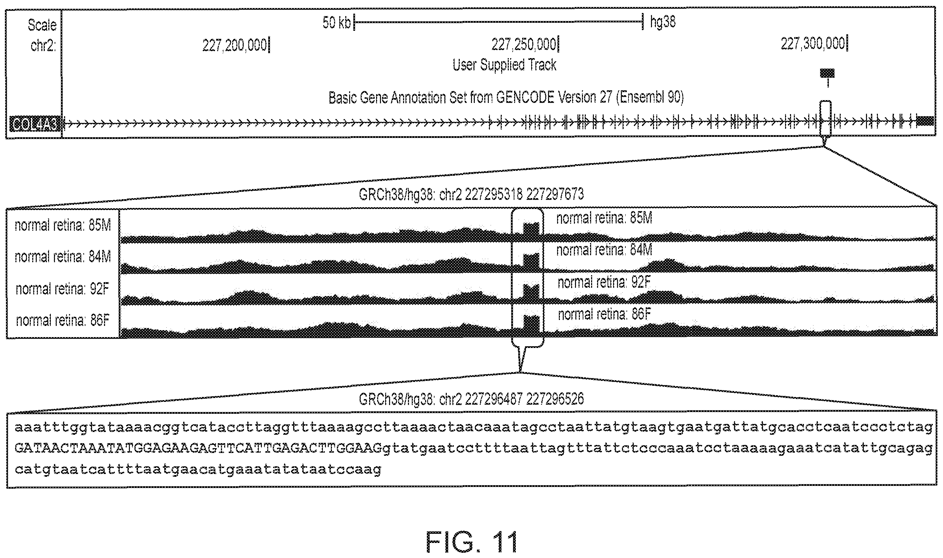

**FIG. 11** depicts identification of an exemplary nonsense-mediated mRNA decay (NMD)-inducing exon in the *COL4A3* gene. The identification of the NMD-inducing exon in the *COL4A3* gene using RNA sequencing is shown, visualized in the UCSC genome browser. The upper panel shows a graphic representation of the *COL4A3* gene to scale. Peaks corresponding to RNA sequencing reads were identified in intron GRCh38/hg38: chr2 227295318 227297673, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

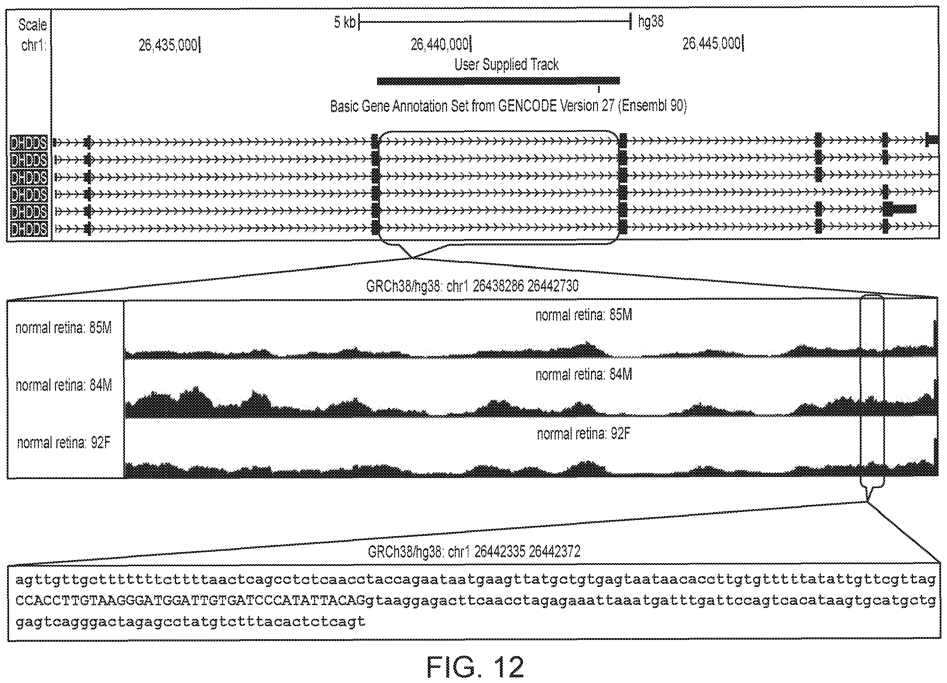

**FIG. 12** depicts identification of an exemplary nonsense-mediated mRNA decay (NMD)-inducing exon in the *DHDDS* gene. The identification of the NMD-inducing exon in the *DHDDS* gene using RNA sequencing is shown, visualized in the UCSC genome browser. The upper panel shows a graphic representation of the *DHDDS* gene to scale. Peaks corresponding to RNA sequencing reads were identified in intron GRCh38/hg38: chr1 26438286 26442730, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

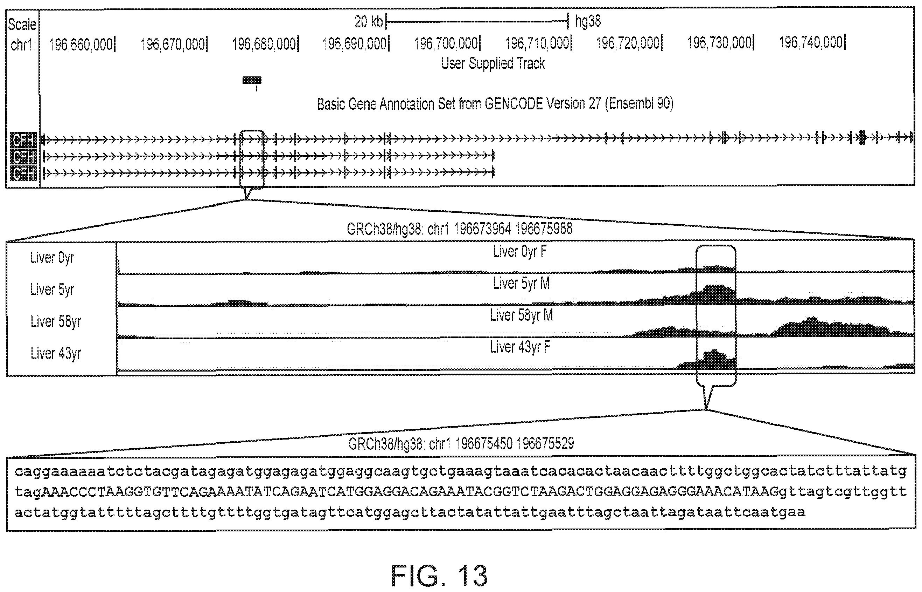

**FIG. 13** depicts identification of an exemplary nonsense-mediated mRNA decay (NMD)-inducing exon in the *CFH* gene. The identification of the NMD-inducing exon in the *CFH* gene using RNA sequencing is shown, visualized in the UCSC genome browser. The upper panel shows a graphic representation of the *CFH* gene to scale. Peaks corresponding to RNA sequencing reads were identified in intron GRCh38/hg38: chr1 196673964 196675988, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

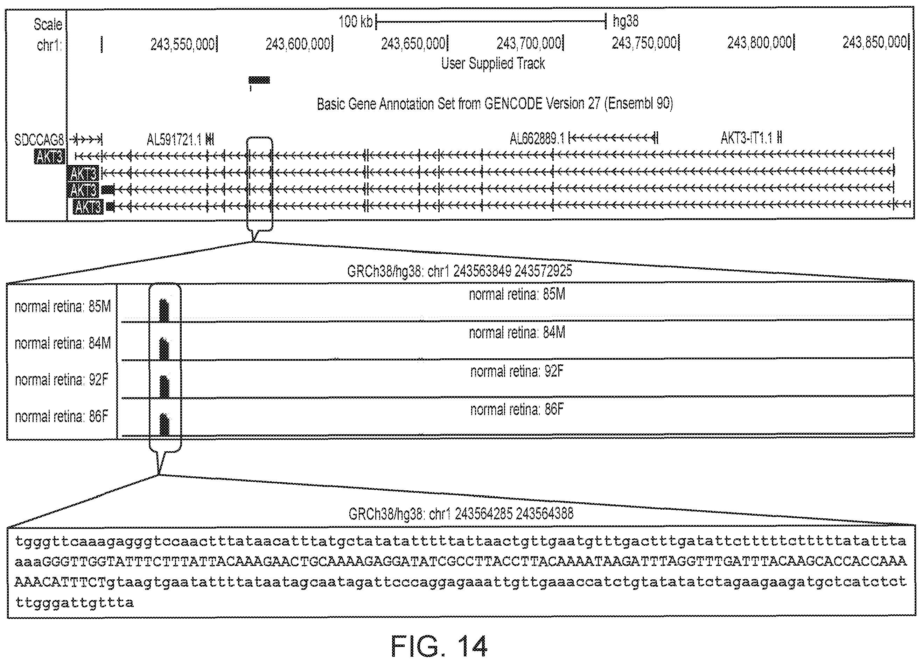

**FIG. 14** depicts identification of an exemplary nonsense-mediated mRNA decay (NMD)-inducing exon in the *AKT3* gene. The identification of the NMD-inducing exon in the *AKT3* gene using RNA sequencing is shown, visualized in the UCSC genome browser. The upper panel shows a graphic representation of the *AKT3* gene to scale. Peaks corresponding to RNA sequencing reads were identified in intron GRCh38/hg38: chr1 243563849 243572925, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

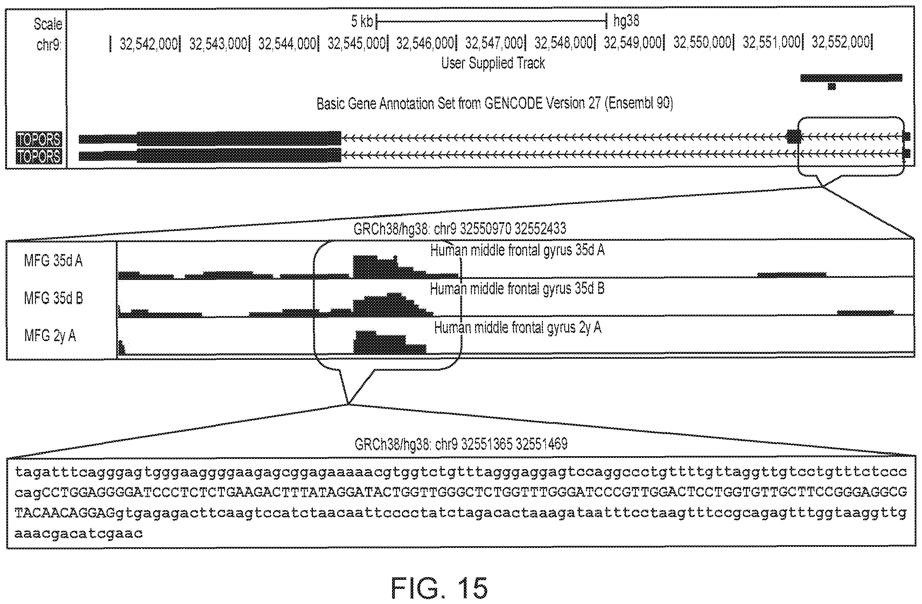

**FIG. 15** depicts identification of an exemplary nonsense-mediated mRNA decay (NMD)-inducing exon in the *TOPORS* gene. The identification of the NMD-inducing exon in the *TOPORS* gene using RNA sequencing is shown, visualized in the UCSC genome browser. The upper panel shows a graphic representation of the *TOPORS* gene to scale. Peaks corresponding to RNA sequencing reads were identified in intron GRCh38/hg38: chr9 32550970 32552433, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

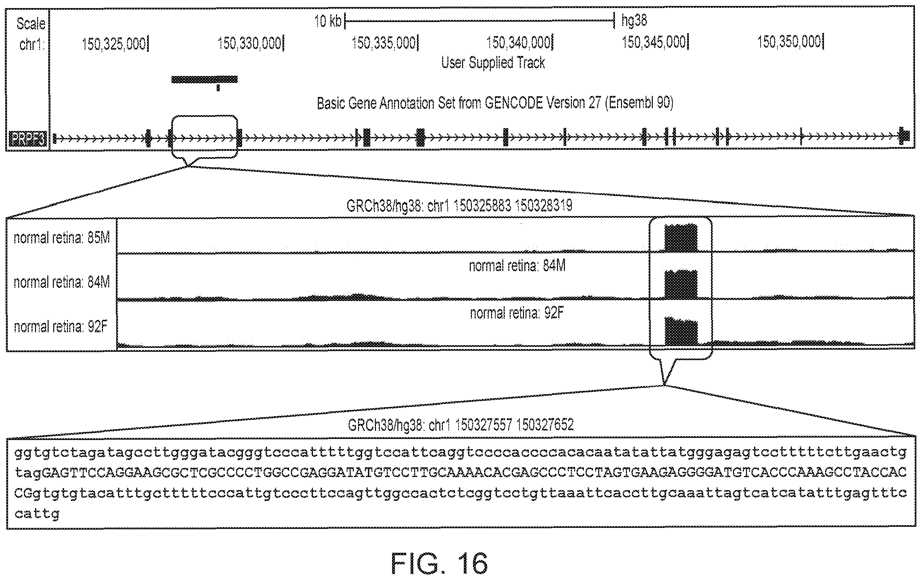

**FIG. 16** depicts identification of an exemplary nonsense-mediated mRNA decay (NMD)-inducing exon in the *PRPF3* gene. The identification of the NMD-inducing exon in the *PRPF3* gene using RNA sequencing is shown, visualized in the UCSC genome browser. The upper panel shows a graphic representation of the *PRPF3* gene to scale. Peaks corresponding to RNA sequencing reads were identified in intron GRCh38/hg38: chr1 150325883 150328319, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

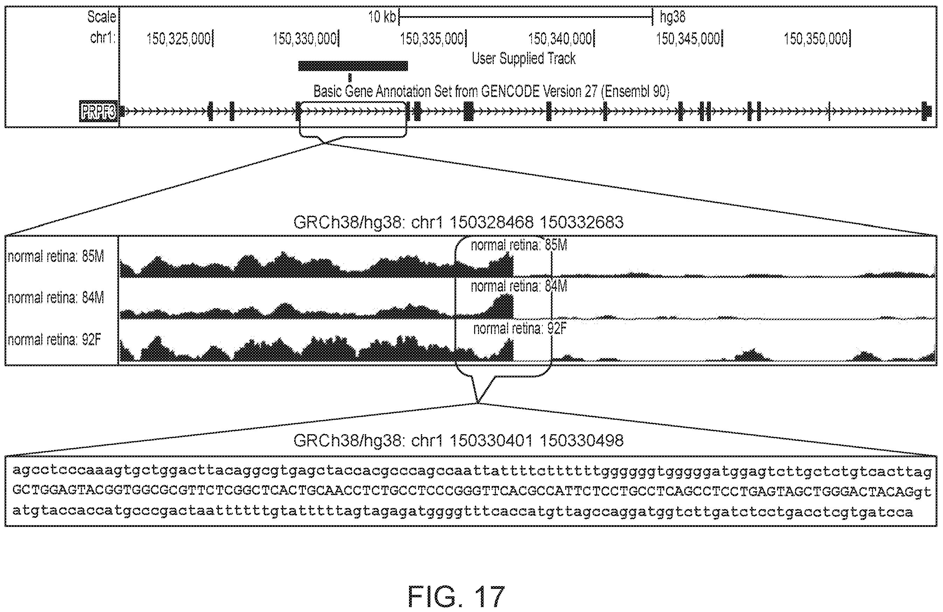

**FIG. 17** depicts identification of an exemplary nonsense-mediated mRNA decay (NMD)-inducing exon in the *PRPF3* gene. The identification of the NMD-inducing exon in the *PRPF3* gene using RNA sequencing is shown, visualized in the UCSC genome browser. The upper panel shows a graphic representation of the *PRPF3* gene to scale. Peaks corresponding to RNA sequencing reads were identified in intron GRCh38/hg38: chr1 150328468 150332683, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

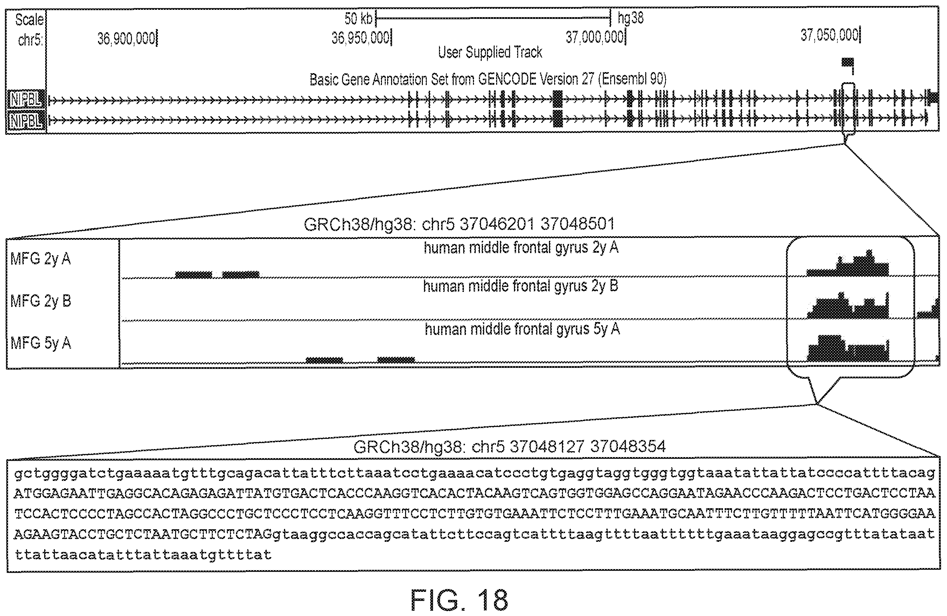

**FIG. 18** depicts identification of an exemplary nonsense-mediated mRNA decay (NMD)-inducing exon in the *NIPBL* gene. The identification of the NMD-inducing exon in the *NIPBL* gene using RNA sequencing is shown, visualized in the UCSC genome browser. The upper panel shows a graphic representation of the *NIPBL* gene to scale. Peaks corresponding to RNA sequencing reads were identified in intron GRCh38/hg38: chr5 37046201 37048501, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

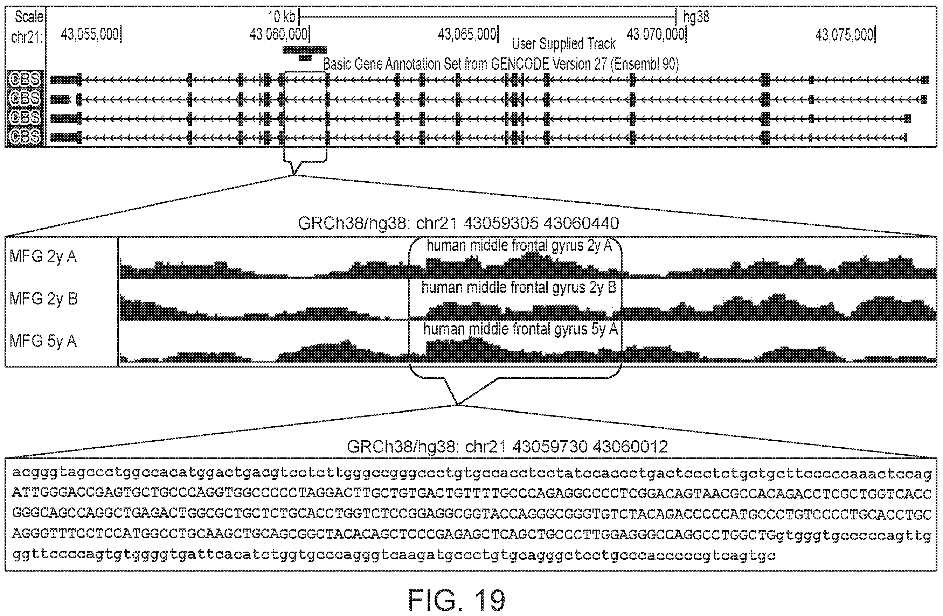

**FIG. 19** depicts identification of an exemplary nonsense-mediated mRNA decay (NMD)-inducing exon in the *CBS* gene. The identification of the NMD-inducing exon in the *CBS* gene using RNA sequencing is shown, visualized in the UCSC genome browser. The upper panel shows a graphic representation of the *CBS* gene to scale. Peaks corresponding to RNA sequencing reads were identified in intron GRCh38/hg38: chr21 43059305 43060440, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

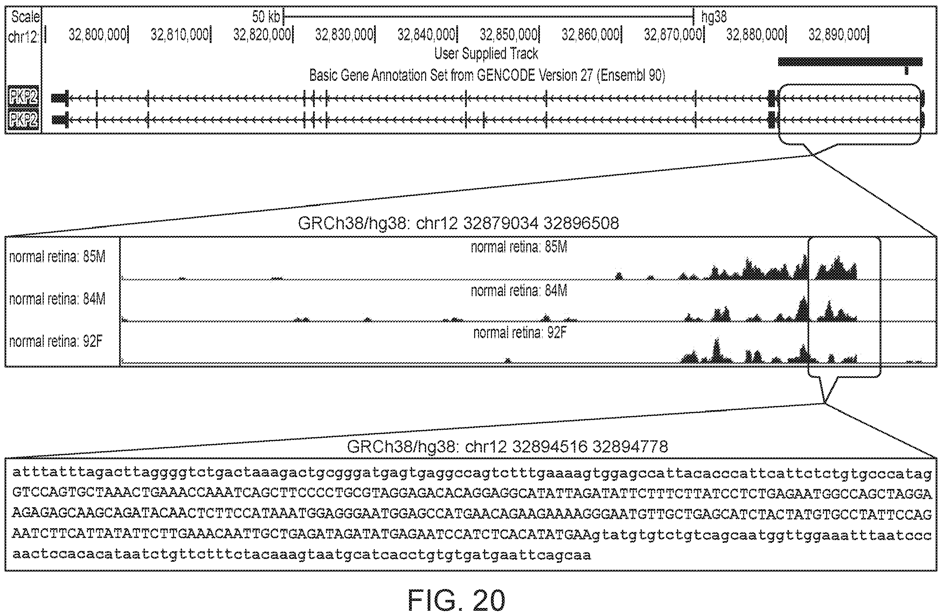

**FIG. 20** depicts identification of an exemplary nonsense-mediated mRNA decay (NMD)-inducing exon in the *PKP2* gene. The identification of the NMD-inducing exon in the *PKP2* gene using RNA sequencing is shown, visualized in the UCSC genome browser. The upper panel shows a graphic representation of the *PKP2* gene to scale. Peaks corresponding to RNA sequencing reads were identified in intron GRCh38/hg38: chr12 32879034 32896508, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

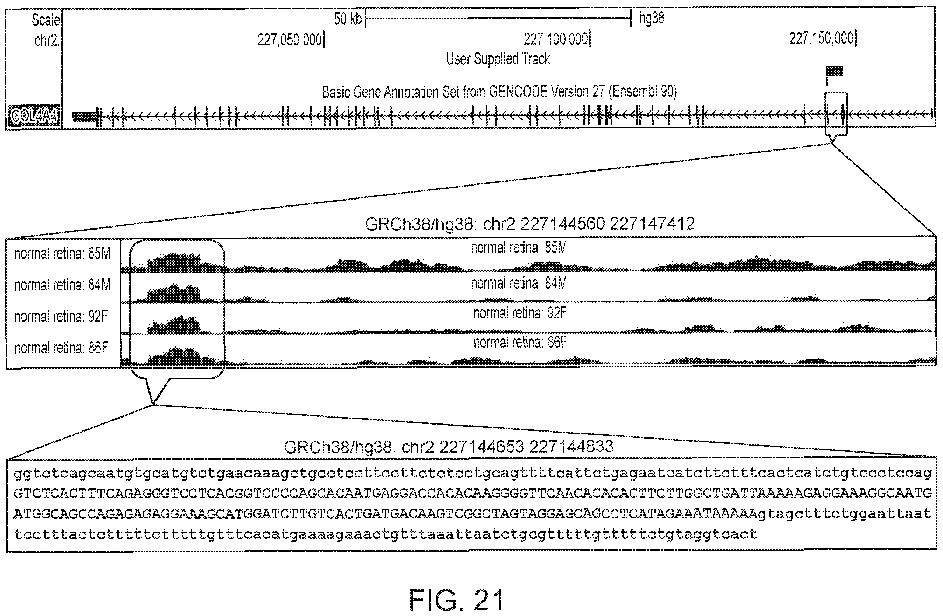

**FIG. 21** depicts identification of an exemplary nonsense-mediated mRNA decay (NMD)-inducing exon in the *COL4A4* gene. The identification of the NMD-inducing exon in the *COL4A4* gene using RNA sequencing is shown, visualized in the UCSC genome browser. The upper panel shows a graphic representation of the *COL4A4* gene to scale. Peaks corresponding to RNA sequencing reads were identified in intron GRCh38/hg38: chr2 227144560 227147412, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

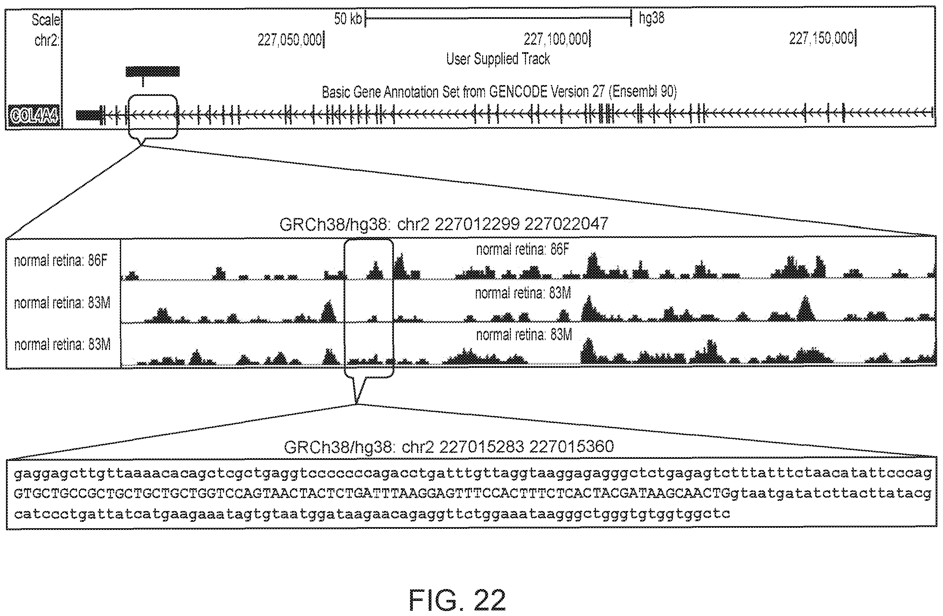

**FIG. 22** depicts identification of an exemplary nonsense-mediated mRNA decay (NMD)-inducing exon in the *COL4A4* gene. The identification of the NMD-inducing exon in the *COL4A4* gene using RNA sequencing is shown, visualized in the UCSC genome browser. The upper panel shows a graphic representation of the *COL4A4* gene to scale. Peaks corresponding to RNA sequencing reads were identified in intron GRCh38/hg38: chr2 227012299 227022047, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

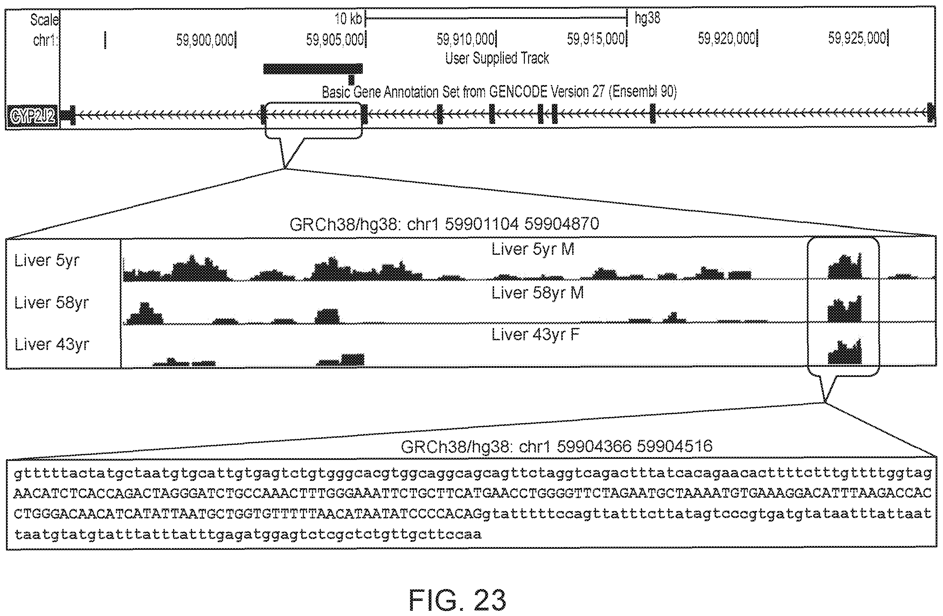

**FIG. 23** depicts identification of an exemplary nonsense-mediated mRNA decay (NMD)-inducing exon in the *CYP2J2* gene. The identification of the NMD-inducing exon in the *CYP2J2* gene using RNA sequencing is shown, visualized in the UCSC genome browser. The upper panel shows a graphic representation of the *CYP2J2* gene to scale. Peaks corresponding to RNA sequencing reads were identified in intron GRCh38/hg38: chr1 59901104 59904870, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

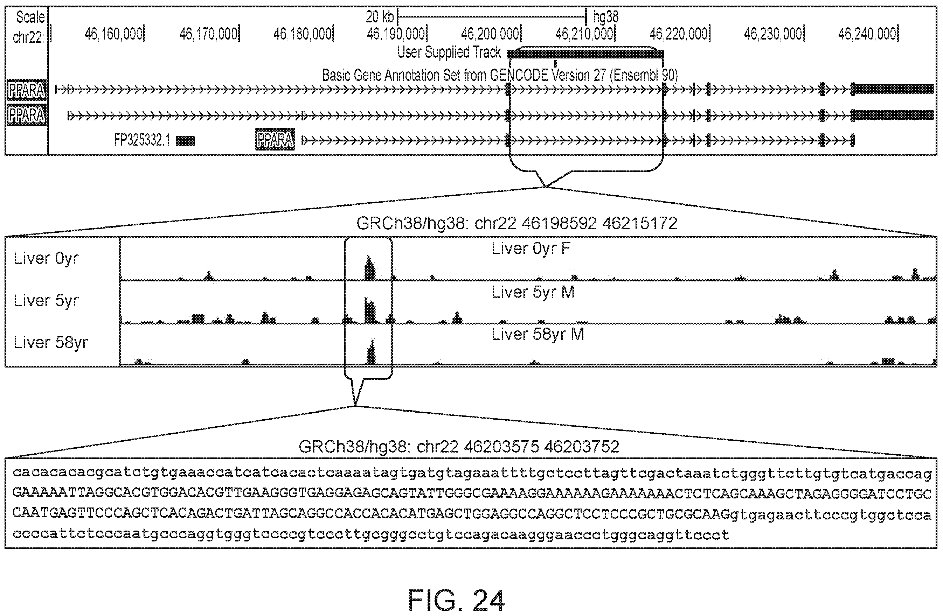

**FIG. 24** depicts identification of an exemplary nonsense-mediated mRNA decay (NMD)-inducing exon in the *PPARA* gene. The identification of the NMD-inducing exon in the *PPARA* gene using RNA sequencing is shown, visualized in the UCSC genome browser. The upper panel shows a graphic representation of the *PPARA* gene to scale. Peaks corresponding to RNA sequencing reads were identified in intron GRCh38/hg38: chr22 46198592 46215172, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

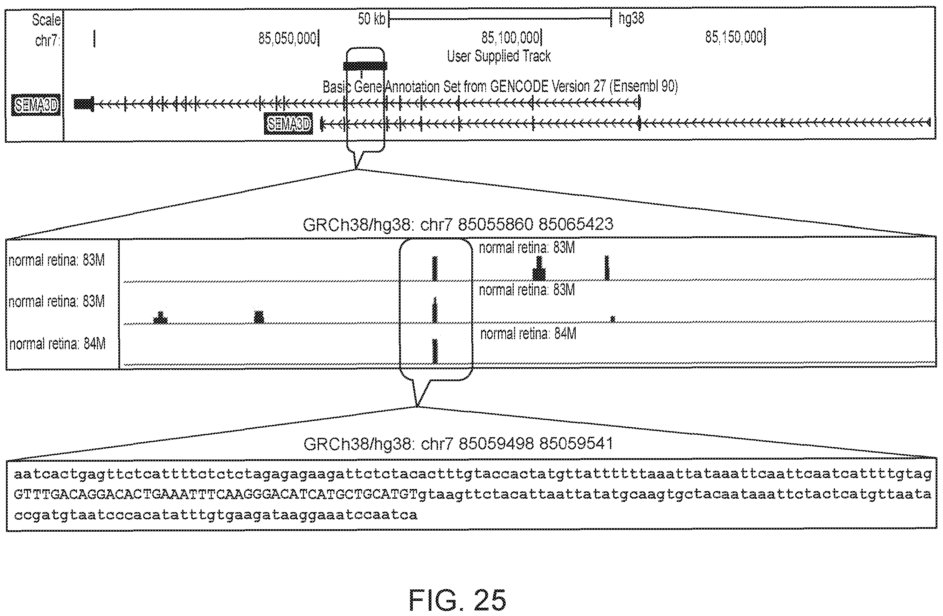

**FIG. 25** depicts identification of an exemplary nonsense-mediated mRNA decay (NMD)-inducing exon in the *SEMA3D* gene. The identification of the NMD-inducing exon in the *SEMA3D* gene using RNA sequencing is shown, visualized in the UCSC genome browser. The upper panel shows a graphic representation of the *SEMA3D* gene to scale. Peaks corresponding to RNA sequencing reads were identified in intron GRCh38/hg38: chr7 85055860 85065423, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

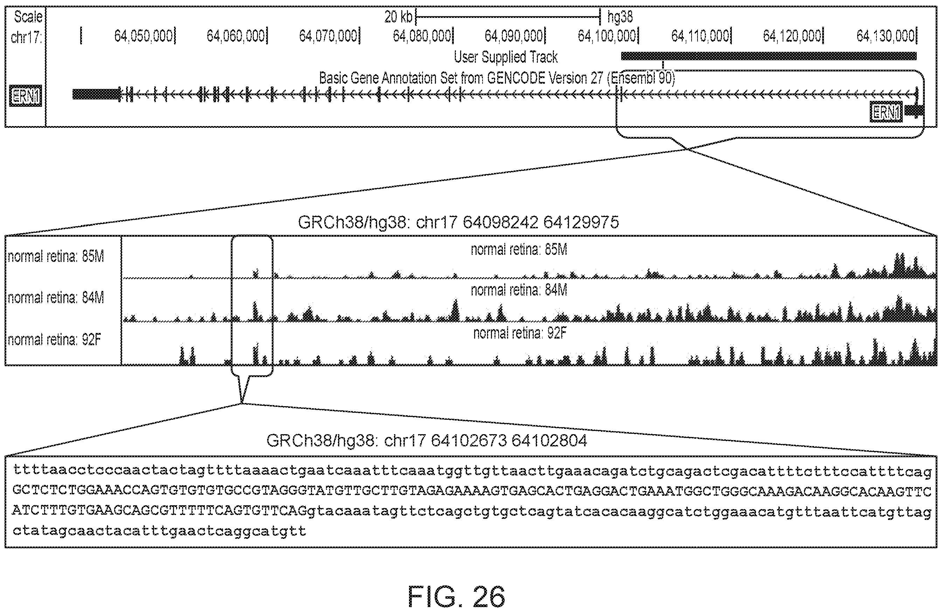

**FIG. 26** depicts identification of an exemplary nonsense-mediated mRNA decay (NMD)-inducing exon in the *ERN1* gene. The identification of the NMD-inducing exon in the *ERN1* gene using RNA sequencing is shown, visualized in the UCSC genome browser. The upper panel shows a graphic representation of the *ERN1* gene to scale. Peaks corresponding to RNA sequencing reads were identified in intron GRCh38/hg38: chr17 64098242 64129975, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

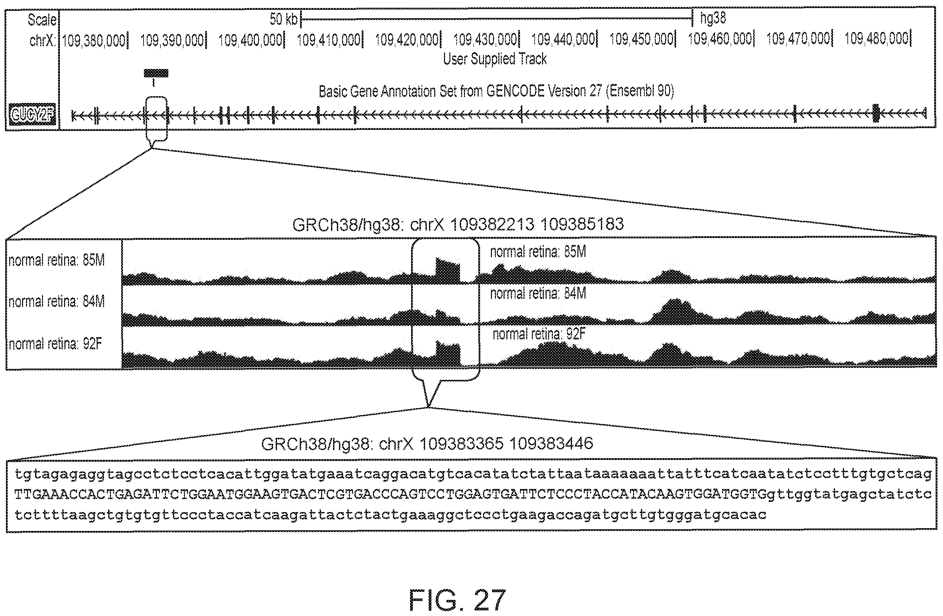

**FIG. 27** depicts identification of an exemplary nonsense-mediated mRNA decay (NMD)-inducing exon in the *GUCY2F* gene. The identification of the NMD-inducing exon in the *GUCY2F* gene using RNA sequencing is shown, visualized in the UCSC genome browser. The upper panel shows a graphic representation of the *GUCY2F* gene to scale. Peaks corresponding to RNA sequencing reads were identified in intron GRCh38/hg38: chrX 109382213 109385183, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

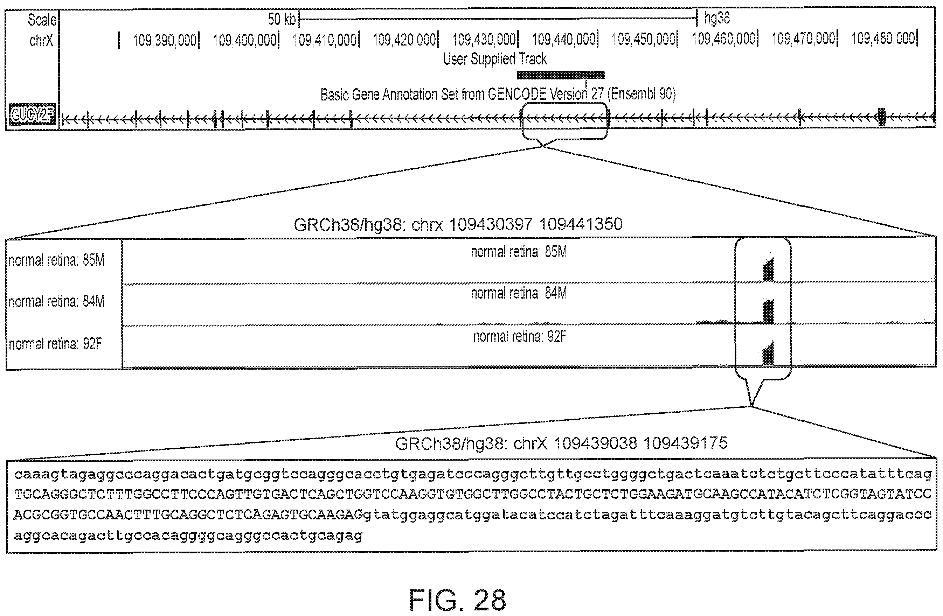

**FIG. 28** depicts identification of an exemplary nonsense-mediated mRNA decay (NMD)-inducing exon in the *GUCY2F* gene. The identification of the NMD-inducing exon in the *GUCY2F* gene using RNA sequencing is shown, visualized in the UCSC genome browser. The upper panel shows a graphic representation of the *GUCY2F* gene to scale. Peaks corresponding to RNA sequencing reads were identified in intron GRCh38/hg38: chrx 109430397 109441350, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

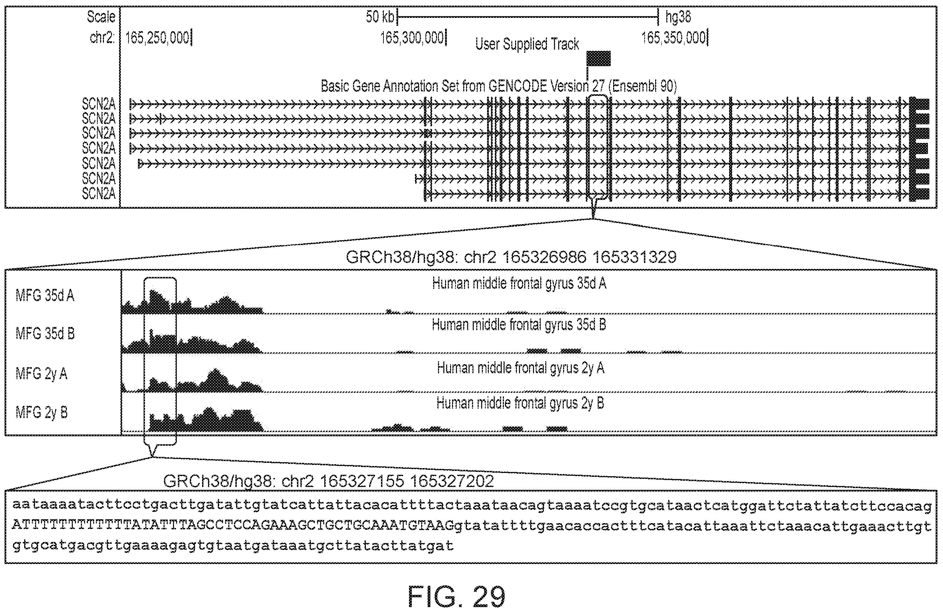

**FIG. 29** depicts identification of an exemplary nonsense-mediated mRNA decay (NMD)-inducing exon in the *SCN2A* gene. The identification of the NMD-inducing exon in the *SCN2A* gene using RNA sequencing is shown, visualized in the UCSC genome browser. The upper panel shows a graphic representation of the *SCN2A* gene to scale. Peaks corresponding to RNA sequencing reads were identified in intron GRCh38/hg38: chr2 165326986 165331329, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

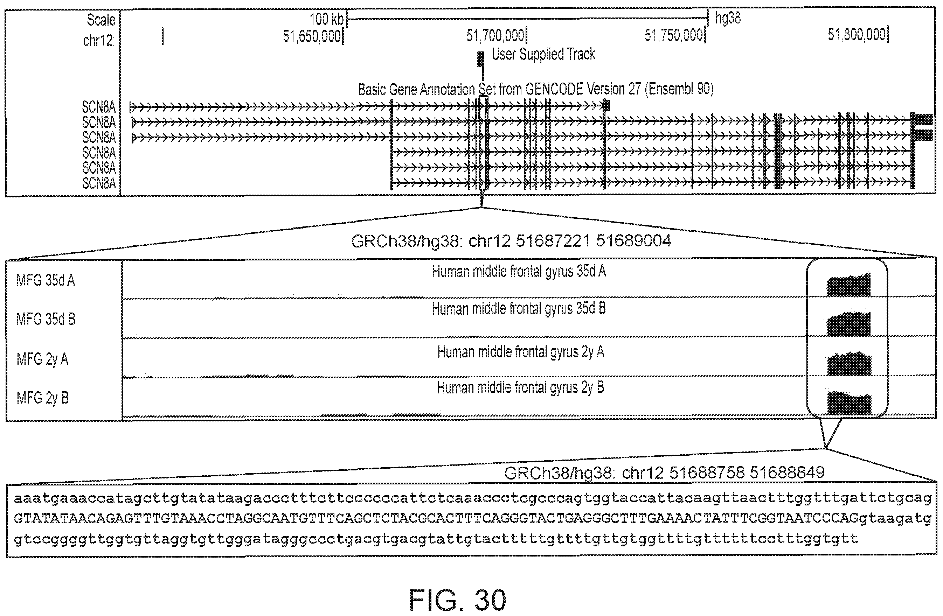

**FIG. 30** depicts identification of an exemplary nonsense-mediated mRNA decay (NMD)-inducing exon in the *SCN8A* gene. The identification of the NMD-inducing exon in the *SCN8A* gene using RNA sequencing is shown, visualized in the UCSC genome browser. The upper panel shows a graphic representation of the *SCN8A* gene to scale. Peaks corresponding to RNA sequencing reads were identified in intron GRCh38/hg38: chr12 51687221 51689004, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

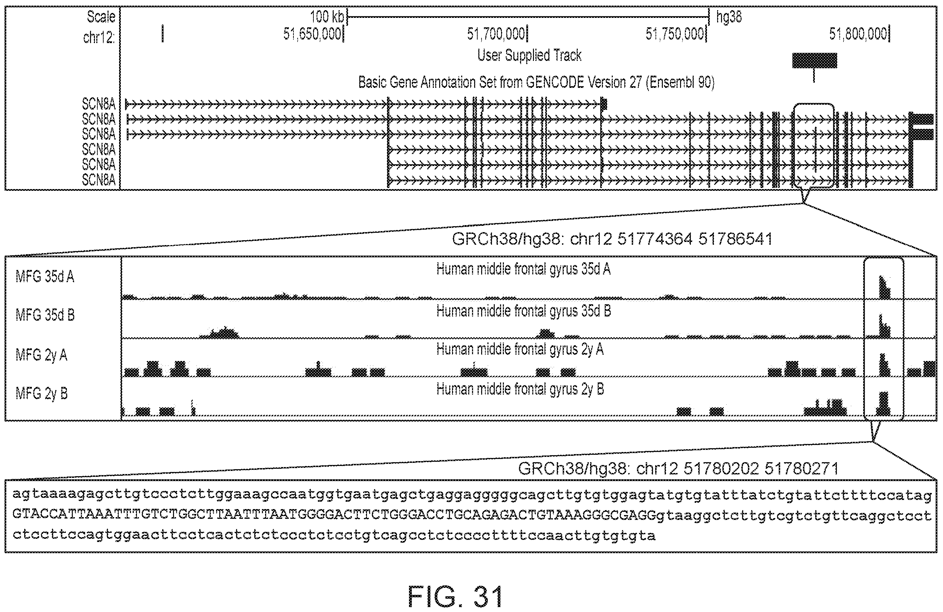

**FIG. 31** depicts identification of an exemplary nonsense-mediated mRNA decay (NMD)-inducing exon in the *SCN8A* gene. The identification of the NMD-inducing exon in the *SCN8A* gene using RNA sequencing is shown, visualized in the UCSC genome browser. The upper panel shows a graphic representation of the *SCN8A* gene to scale. Peaks corresponding to RNA sequencing reads were identified in intron GRCh38/hg38: chr12 51774364 51786541, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

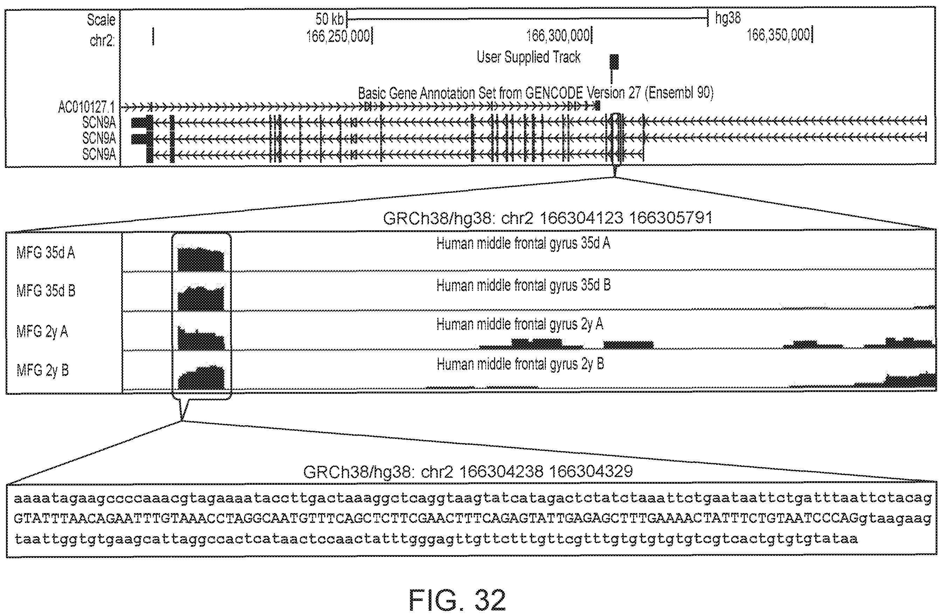

**FIG. 32** depicts identification of an exemplary nonsense-mediated mRNA decay (NMD)-inducing exon in the *SCN9A* gene. The identification of the NMD-inducing exon in the *SCN9A* gene using RNA sequencing is shown, visualized in the UCSC genome browser. The upper panel shows a graphic representation of the *SCN9A* gene to scale. Peaks corresponding to RNA sequencing reads were identified in intron GRCh38/hg38: chr2 166304123 166305791, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

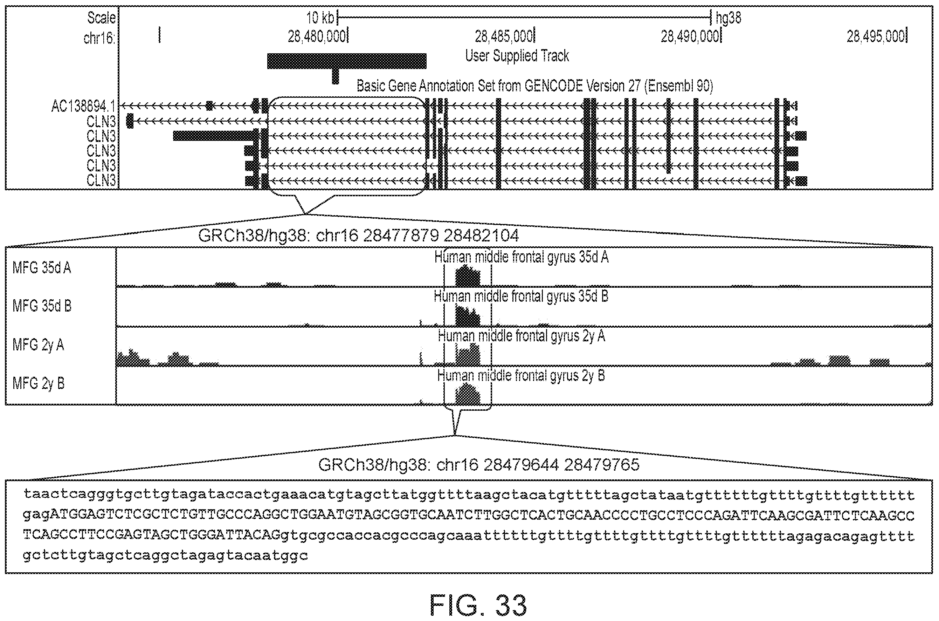

**FIG. 33** depicts identification of an exemplary nonsense-mediated mRNA decay (NMD)-inducing exon in the *CLN3* gene. The identification of the NMD-inducing exon in the *CLN3* gene using RNA sequencing is shown, visualized in the UCSC genome browser. The upper panel shows a graphic representation of the *CLN3* gene to scale. Peaks corresponding to RNA sequencing reads were identified in intron GRCh38/hg38: chr16 28477879 28482104, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

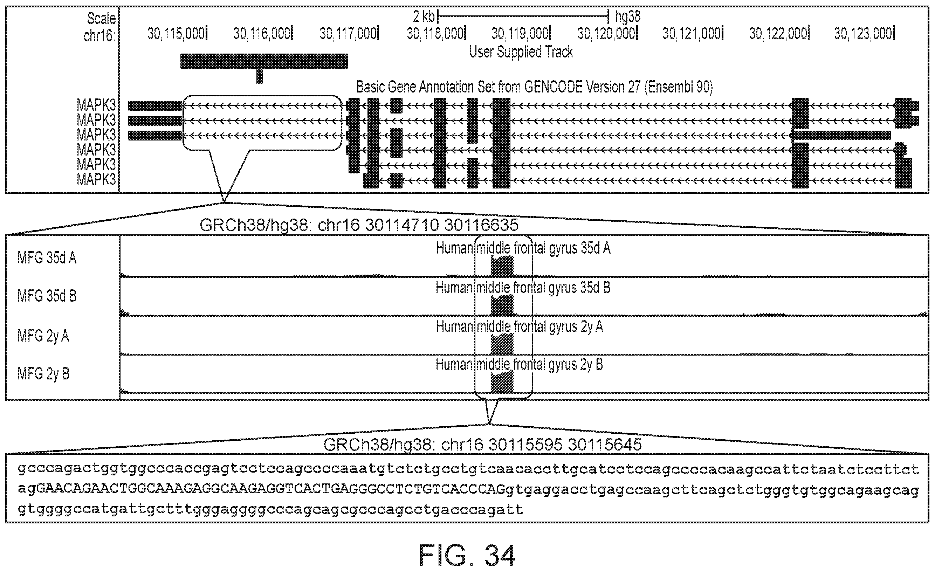

**FIG. 34** depicts identification of an exemplary nonsense-mediated mRNA decay (NMD)-inducing exon in the *MAPK3* gene. The identification of the NMD-inducing exon in the *MAPK3* gene using RNA sequencing is shown, visualized in the UCSC genome browser. The upper panel shows a graphic representation of the *MAPK3* gene to scale. Peaks corresponding to RNA sequencing reads were identified in intron GRCh38/hg38: chr16 30114710 30116635, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

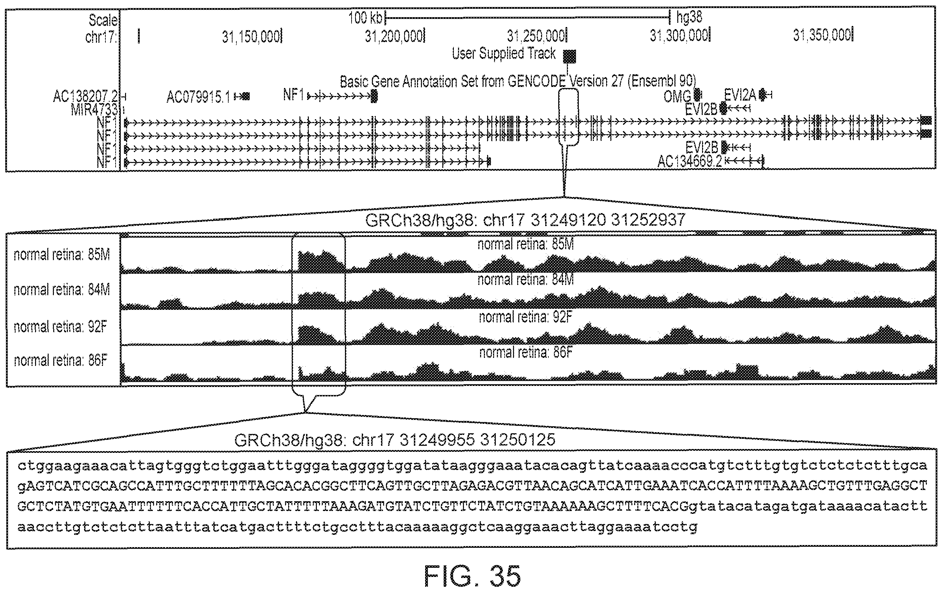

**FIG. 35** depicts identification of an exemplary nonsense-mediated mRNA decay (NMD)-inducing exon in the *NF1* gene. The identification of the NMD-inducing exon in the *NF1* gene using RNA sequencing is shown, visualized in the UCSC genome browser. The upper panel shows a graphic representation of the *NF1* gene to scale. Peaks corresponding to RNA sequencing reads were identified in intron GRCh38/hg38: chr17 31249120 31252937, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

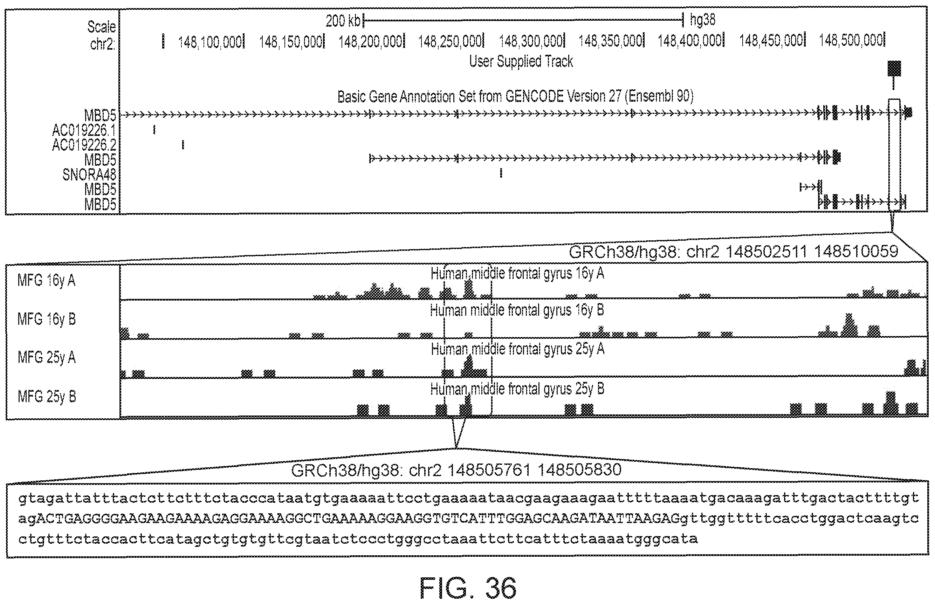

**FIG. 36** depicts identification of an exemplary nonsense-mediated mRNA decay (NMD)-inducing exon in the *MBD5* gene. The identification of the NMD-inducing exon in the *MBD5* gene using RNA sequencing is shown, visualized in the UCSC genome browser. The upper panel shows a graphic representation of the *MBD5* gene to scale. Peaks corresponding to RNA sequencing reads were identified in intron GRCh38/hg38: chr2 148502511 148510059, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

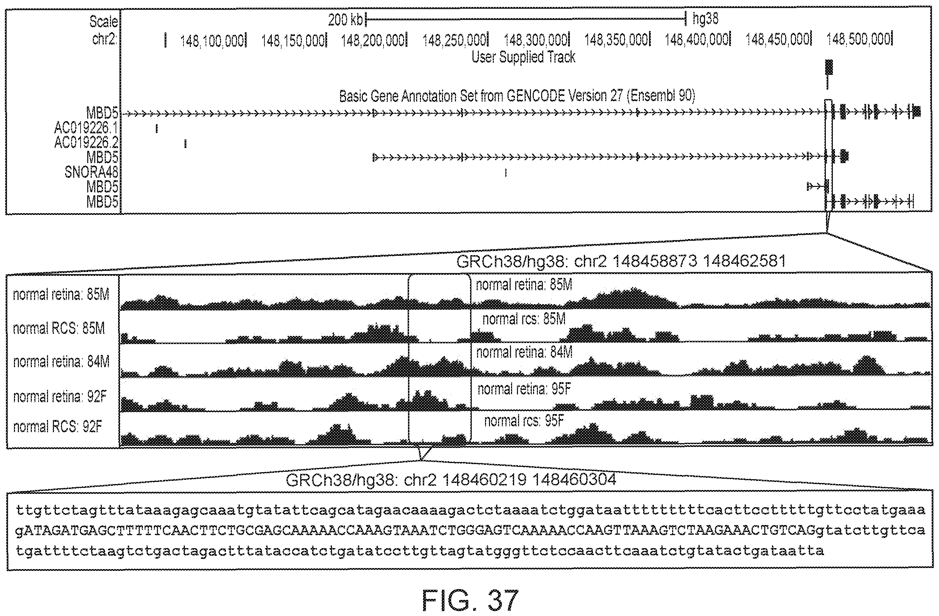

**FIG. 37** depicts identification of an exemplary nonsense-mediated mRNA decay (NMD)-inducing exon in the *MBD5* gene. The identification of the NMD-inducing exon in the *MBD5* gene using RNA sequencing is shown, visualized in the UCSC genome browser. The upper panel shows a graphic representation of the *MBD5* gene to scale. Peaks corresponding to RNA sequencing reads were identified in intron GRCh38/hg38: chr2 148458873 148462581, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

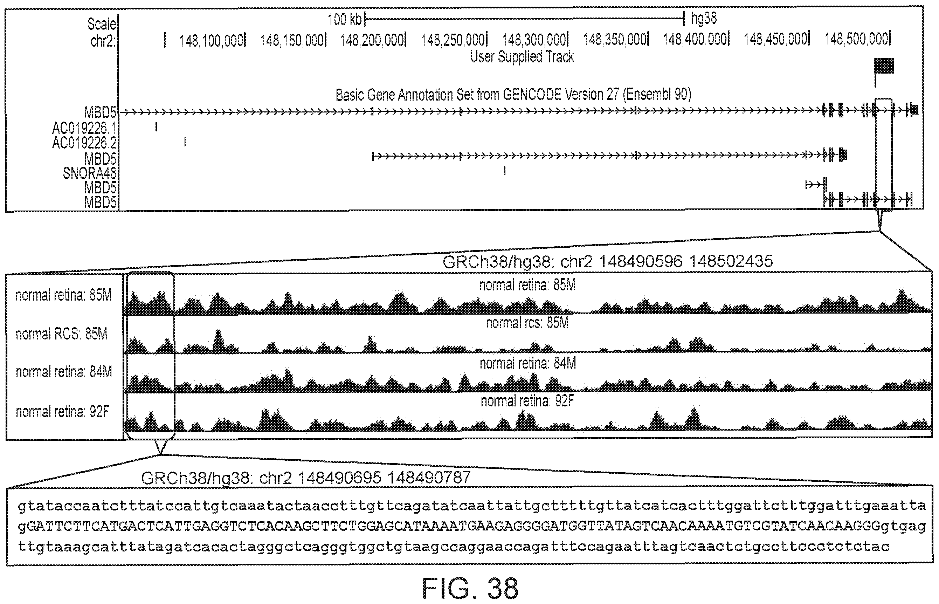

**FIG. 38** depicts identification of an exemplary nonsense-mediated mRNA decay (NMD)-inducing exon in the *MBD5* gene. The identification of the NMD-inducing exon in the *MBD5* gene using RNA sequencing is shown, visualized in the UCSC genome browser. The upper panel shows a graphic representation of the *MBD5* gene to scale. Peaks corresponding to RNA sequencing reads were identified in intron GRCh38/hg38: chr2 148490596 148502435, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

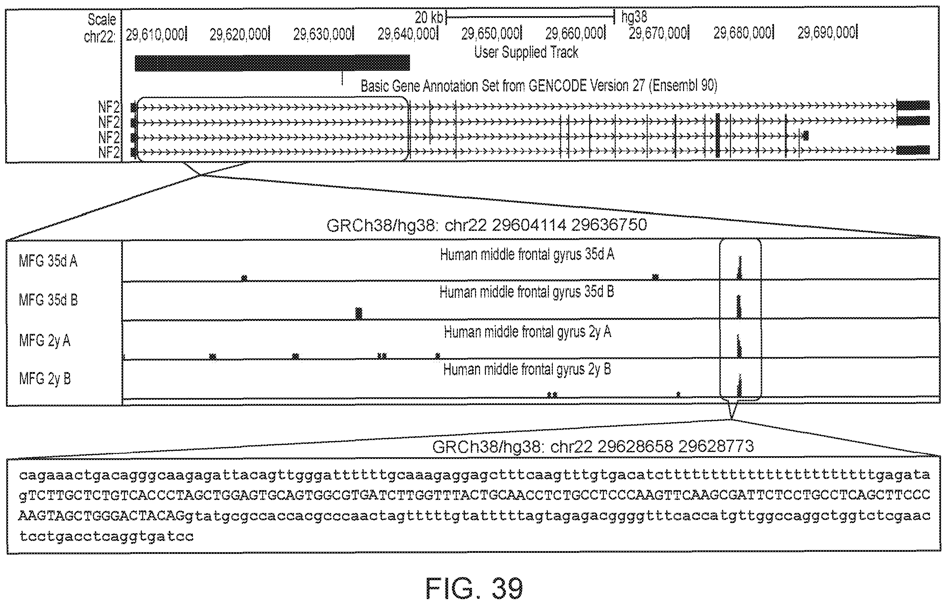

**FIG. 39** depicts identification of an exemplary nonsense-mediated mRNA decay (NMD)-inducing exon in the *NF2* gene. The identification of the NMD-inducing exon in the *NF2* gene using RNA sequencing is shown, visualized in the UCSC genome browser. The upper panel shows a graphic representation of the *NF2* gene to scale. Peaks corresponding to RNA sequencing reads were identified in intron GRCh38/hg38: chr22 29604114 29636750, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

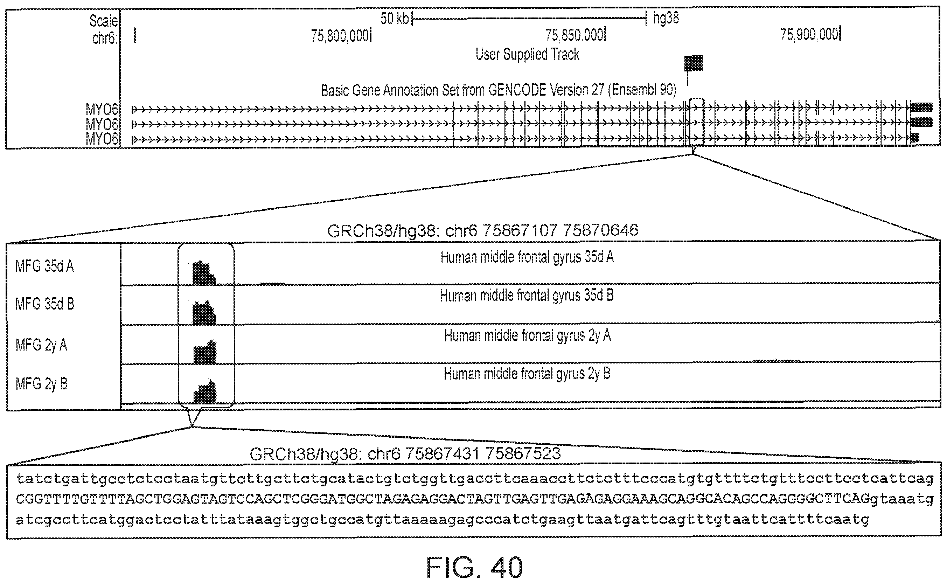

**FIG. 40** depicts identification of an exemplary nonsense-mediated mRNA decay (NMD)-inducing exon in the *MYO6* gene. The identification of the NMD-inducing exon in the *MYO6* gene using RNA sequencing is shown, visualized in the UCSC genome browser. The upper panel shows a graphic representation of the *MYO6* gene to scale. Peaks corresponding to RNA sequencing reads were identified in intron GRCh38/hg38: chr6 75867107 75870646, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

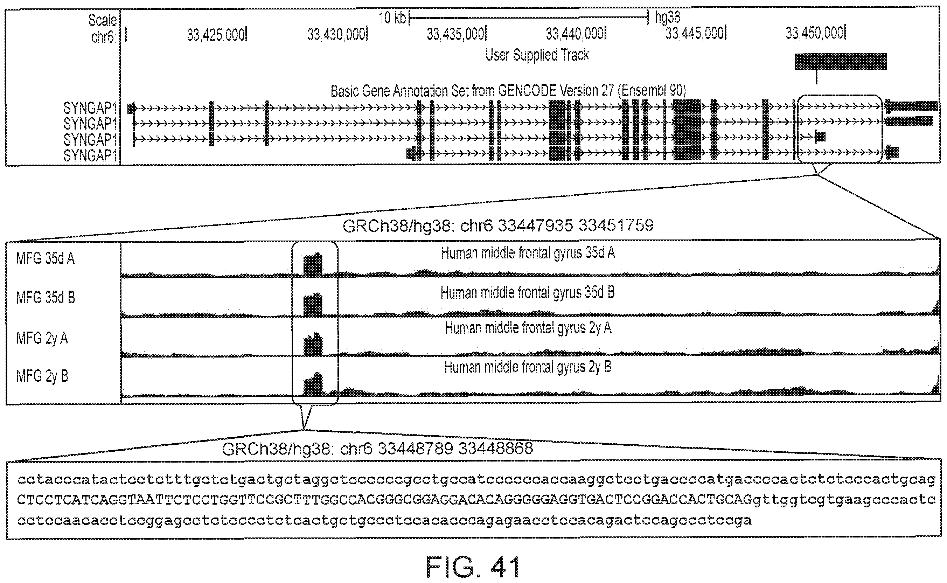

**FIG. 41** depicts identification of an exemplary nonsense-mediated mRNA decay (NMD)-inducing exon in the *SYNGAP1* gene. The identification of the NMD-inducing exon in the *SYNGAP1* gene using RNA sequencing is shown, visualized in the UCSC genome browser. The upper panel shows a graphic representation of the *SYNGAP1* gene to scale. Peaks corresponding to RNA sequencing reads were identified in intron GRCh38/hg38: chr6 33447935 33451759, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

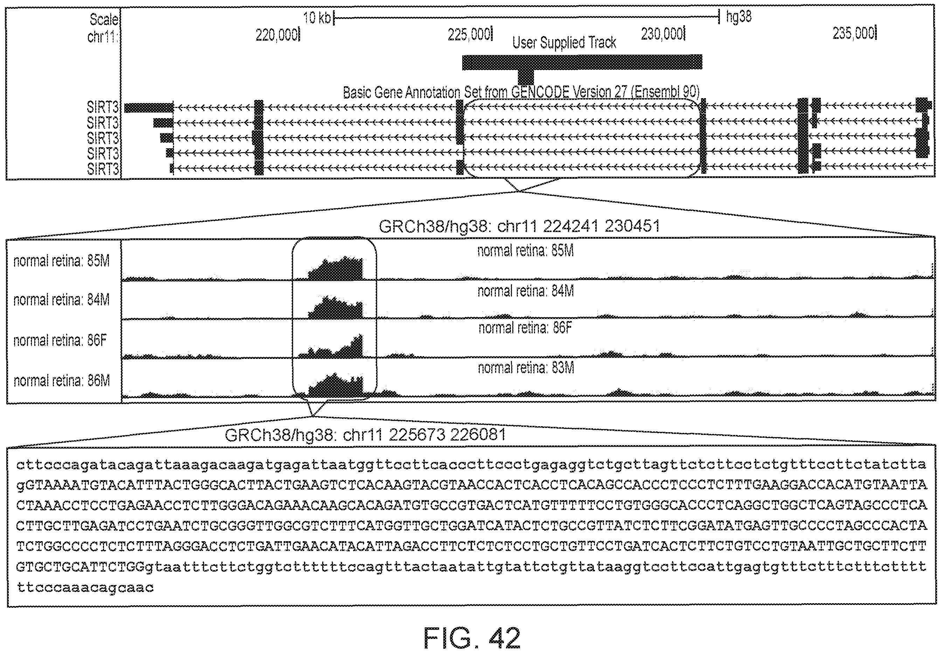

**FIG. 42** depicts identification of an exemplary nonsense-mediated mRNA decay (NMD)-inducing exon in the *SIRT3* gene. The identification of the NMD-inducing exon in the *SIRT3* gene using RNA sequencing is shown, visualized in the UCSC genome browser. The upper panel shows a graphic representation of the *SIRT3* gene to scale. Peaks corresponding to RNA sequencing reads were identified in intron GRCh38/hg38: chr11 224241 230451, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

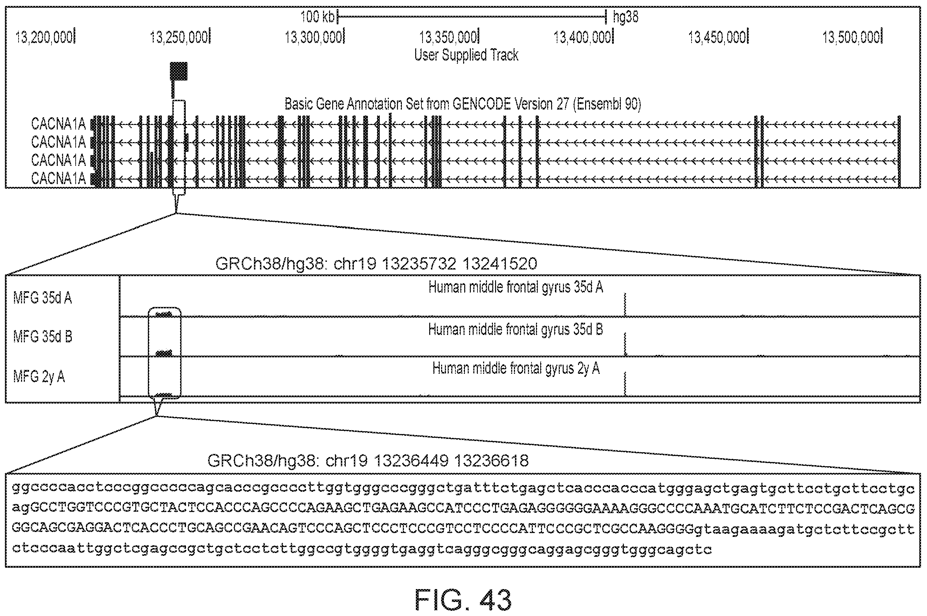

**FIG. 43** depicts identification of an exemplary nonsense-mediated mRNA decay (NMD)-inducing exon in the *CACNA1A* gene. The identification of the NMD-inducing exon in the *CACNA1A* gene using RNA sequencing is shown, visualized in the UCSC genome browser. The upper panel shows a graphic representation of the *CACNA1A* gene to scale. Peaks corresponding to RNA sequencing reads were identified in intron GRCh38/hg38: chr19 13235732 13241520, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

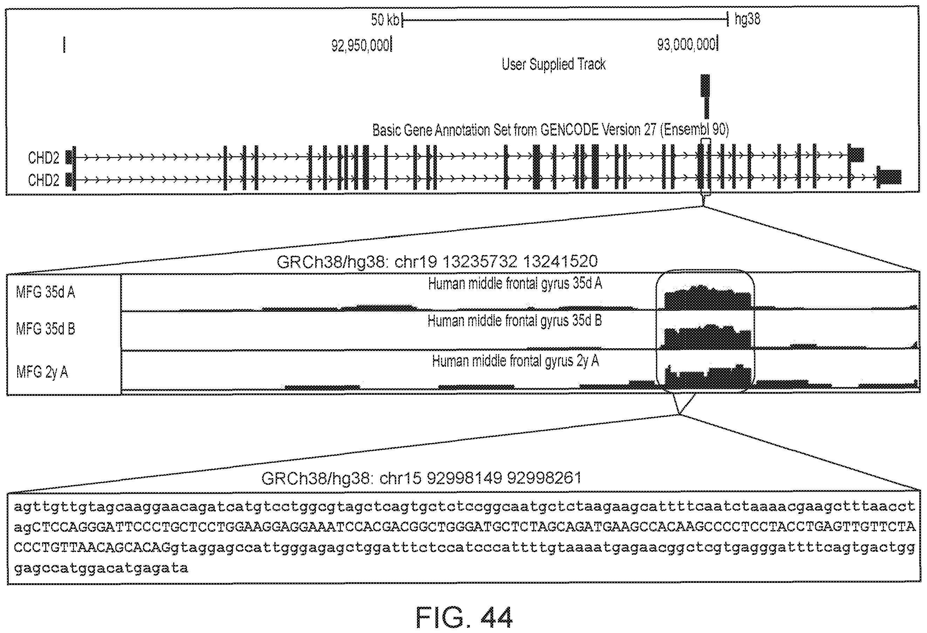

**FIG. 44** depicts identification of an exemplary nonsense-mediated mRNA decay (NMD)-inducing exon in the *CHD2* gene. The identification of the NMD-inducing exon in the *CHD2* gene using RNA sequencing is shown, visualized in the UCSC genome browser. The upper panel shows a graphic representation of the *CHD2* gene to scale. Peaks corresponding to RNA sequencing reads were identified in intron GRCh38/hg38: chr15 92997404 92998498, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

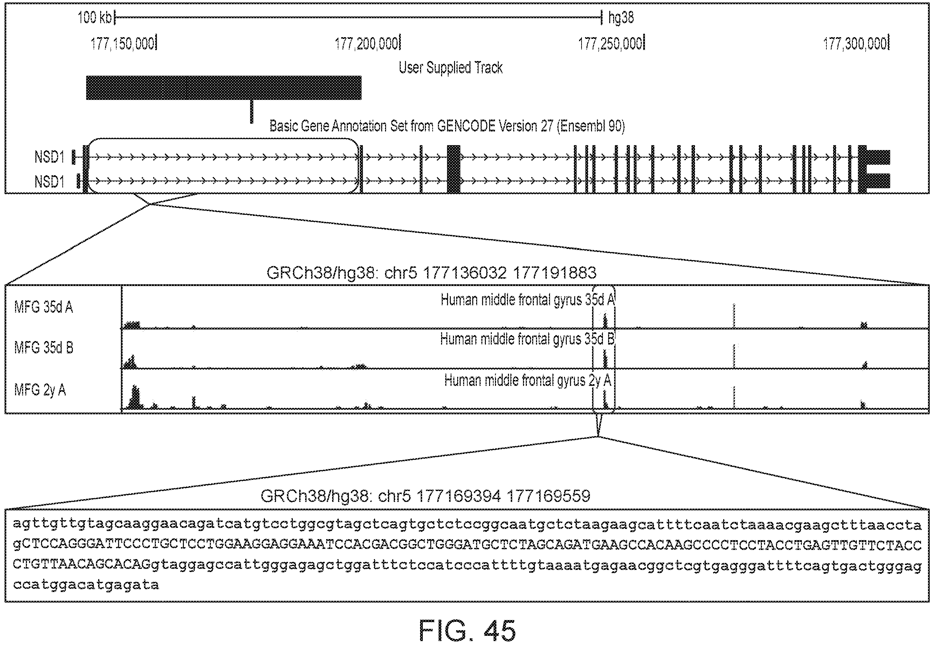

**FIG. 45** depicts identification of an exemplary nonsense-mediated mRNA decay (NMD)-inducing exon in the *NSD1* gene. The identification of the NMD-inducing exon in the *NSD1* gene using RNA sequencing is shown, visualized in the UCSC genome browser. The upper panel shows a graphic representation of the *NSD1* gene to scale. Peaks corresponding to RNA sequencing reads were identified in intron GRCh38/hg38: chr5 177136032 177191883, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

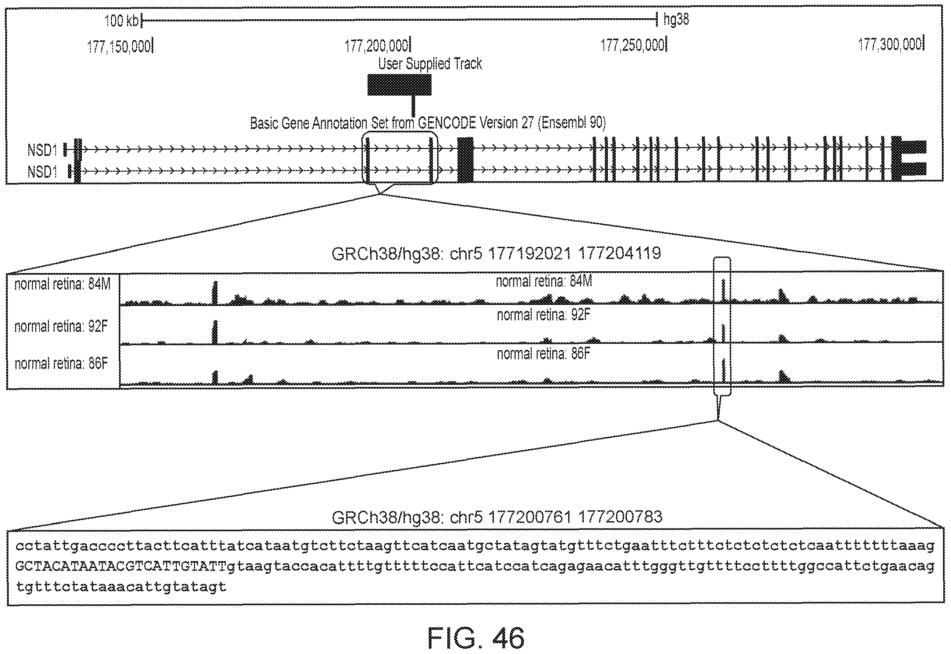

**FIG. 46** depicts identification of an exemplary nonsense-mediated mRNA decay (NMD)-inducing exon in the *NSD1* gene. The identification of the NMD-inducing exon in the *NSD1* gene using RNA sequencing is shown, visualized in the UCSC genome browser. The upper panel shows a graphic representation of the *NSD1* gene to scale. Peaks corresponding to RNA sequencing reads were identified in intron GRCh38/hg38: chr5 177192021 177204119, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

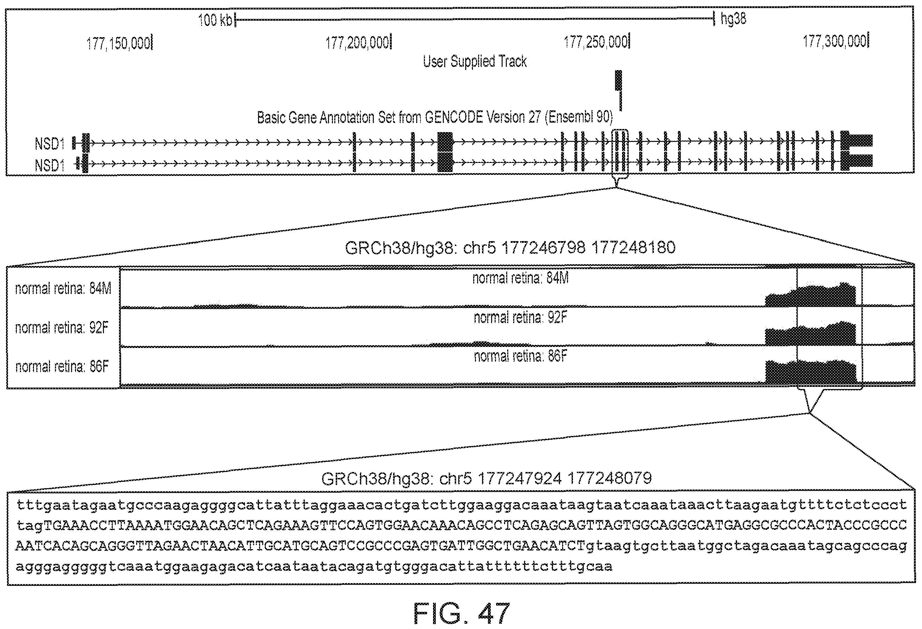

**FIG. 47** depicts identification of an exemplary nonsense-mediated mRNA decay (NMD)-inducing exon in the *NSD1* gene. The identification of the NMD-inducing exon in the *NSD1* gene using RNA sequencing is shown, visualized in the UCSC genome browser. The upper panel shows a graphic representation of the *NSD1* gene to scale. Peaks corresponding to RNA sequencing reads were identified in intron GRCh38/hg38: chr5 177246798 177248180, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

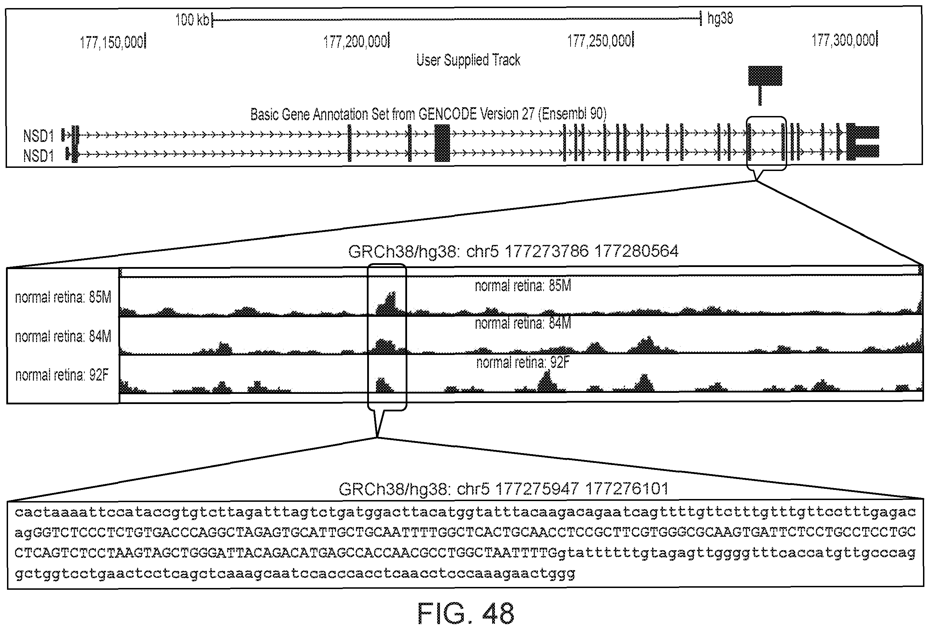

**FIG. 48** depicts identification of an exemplary nonsense-mediated mRNA decay (NMD)-inducing exon in the *NSD1* gene. The identification of the NMD-inducing exon in the *NSD1* gene using RNA sequencing is shown, visualized in the UCSC genome browser. The upper panel shows a graphic representation of the *NSD1* gene to scale. Peaks corresponding to RNA sequencing reads were identified in intron GRCh38/hg38: chr5 177273786 177280564, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

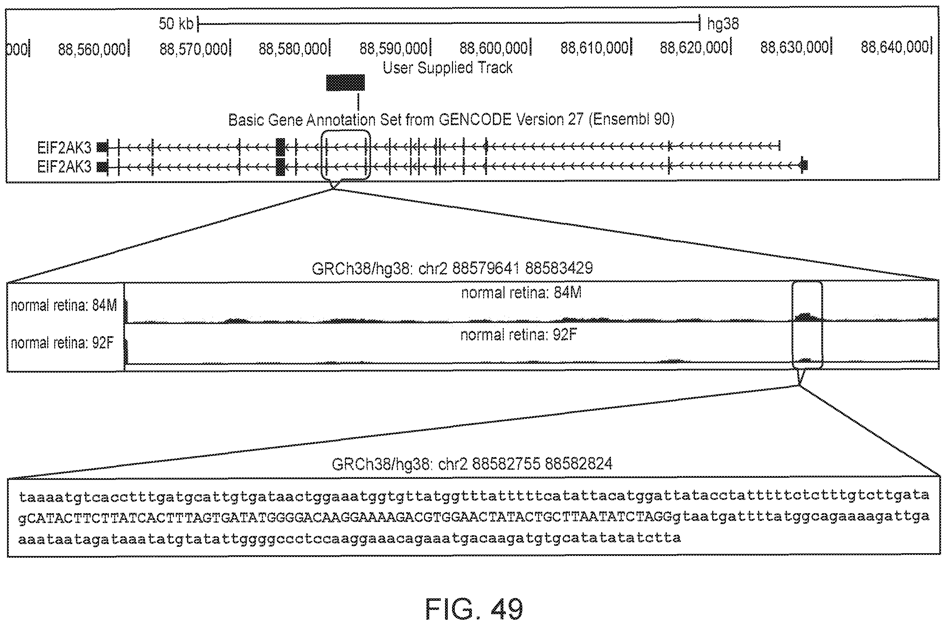

**FIG. 49** depicts identification of an exemplary nonsense-mediated mRNA decay (NMD)-inducing exon in the *EIF2AK3* gene. The identification of the NMD-inducing exon in the *EIF2AK3* gene using RNA sequencing is shown, visualized in the UCSC genome browser. The upper panel shows a graphic representation of the *EIF2AK3* gene to scale. Peaks corresponding to RNA sequencing reads were identified in intron GRCh38/hg38: chr2 88579641 88583429, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

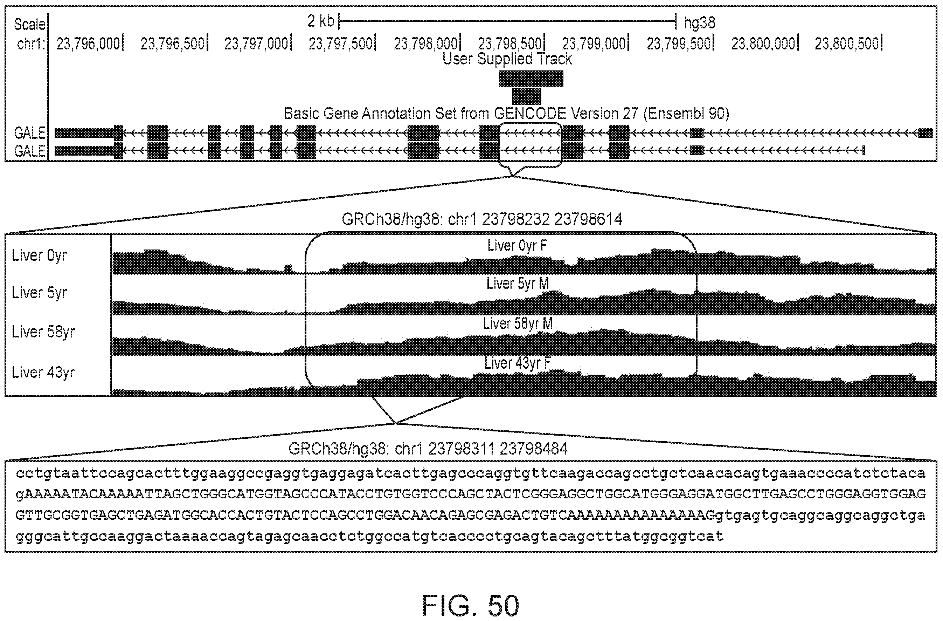

**FIG. 50** depicts identification of an exemplary nonsense-mediated mRNA decay (NMD)-inducing exon in the *GALE* gene. The identification of the NMD-inducing exon in the *GALE* gene using RNA sequencing is shown, visualized in the UCSC genome browser. The upper panel shows a graphic representation of the *GALE gene* to scale. Peaks corresponding to RNA sequencing reads were identified in intron GRCh38/hg38: chr1 23798232 23798614, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

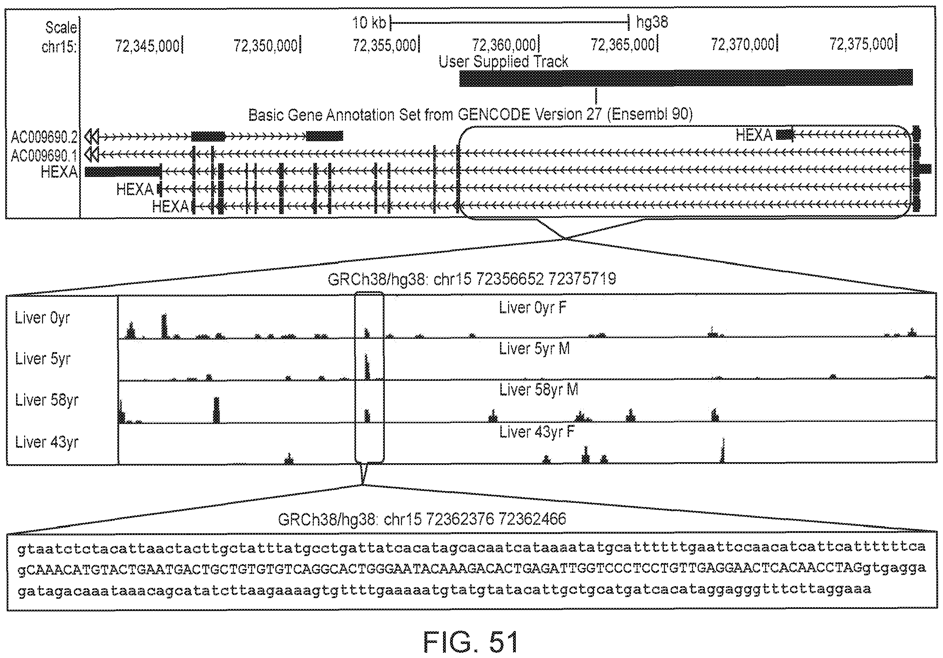

**FIG. 51** depicts identification of an exemplary nonsense-mediated mRNA decay (NMD)-inducing exon in the *HEXA* gene. The identification of the NMD-inducing exon in the *HEXA* gene using RNA sequencing is shown, visualized in the UCSC genome browser. The upper panel shows a graphic representation of the *HEXA* gene to scale. Peaks corresponding to RNA sequencing reads were identified in intron GRCh38/hg38: chr15 72356652 72375719, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

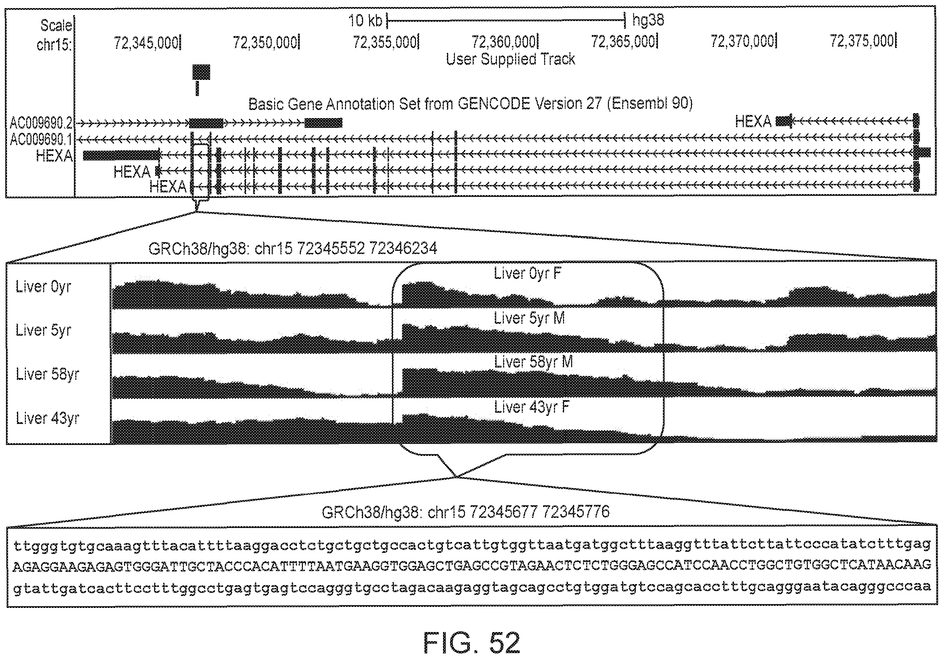

**FIG. 52** depicts identification of an exemplary nonsense-mediated mRNA decay (NMD)-inducing exon in the *HEXA* gene. The identification of the NMD-inducing exon in the *HEXA* gene using RNA sequencing is shown, visualized in the UCSC genome browser. The upper panel shows a graphic representation of the *HEXA* gene to scale. Peaks corresponding to RNA sequencing reads were identified in intron GRCh38/hg38: chr15 72345552 72346234, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

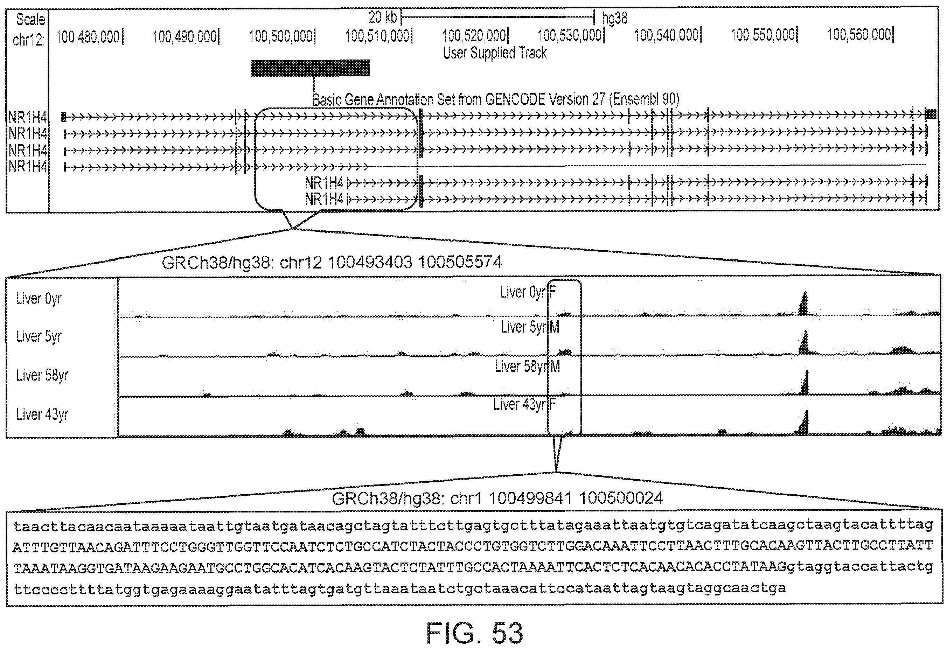

**FIG. 53** depicts identification of an exemplary nonsense-mediated mRNA decay (NMD)-inducing exon in the *NR1H4* gene. The identification of the NMD-inducing exon in the *NR1H4* gene using RNA sequencing is shown, visualized in the UCSC genome browser. The upper panel shows a graphic representation of the *NR1H4* gene to scale. Peaks corresponding to RNA sequencing reads were identified in intron GRCh38/hg38: chr12 100493403 100505574, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

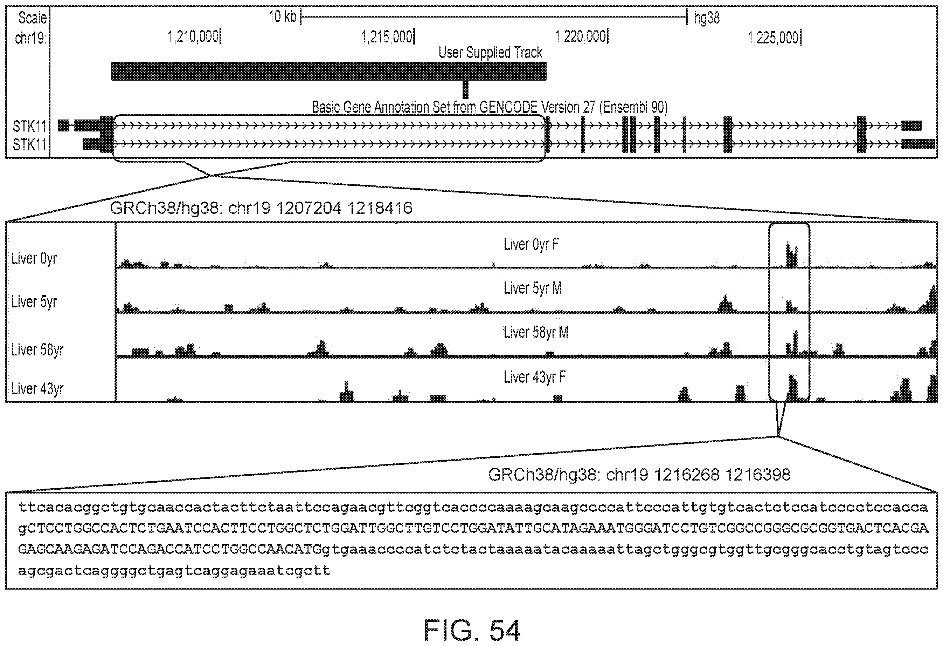

**FIG. 54** depicts identification of an exemplary nonsense-mediated mRNA decay (NMD)-inducing exon in the *STK11* gene. The identification of the NMD-inducing exon in the *STK11* gene using RNA sequencing is shown, visualized in the UCSC genome browser. The upper panel shows a graphic representation of the *STK11* gene to scale. Peaks corresponding to RNA sequencing reads were identified in intron GRCh38/hg38: chr19 1207204 1218416, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

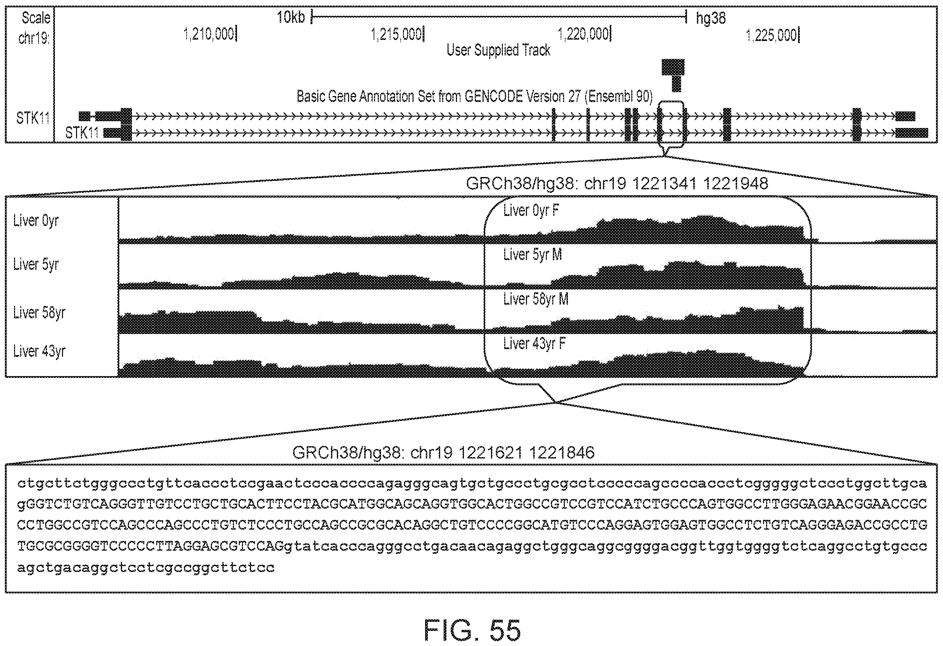

**FIG. 55** depicts identification of an exemplary nonsense-mediated mRNA decay (NMD)-inducing exon in the *STK11* gene. The identification of the NMD-inducing exon in the *STK11* gene using RNA sequencing is shown, visualized in the UCSC genome browser. The upper panel shows a graphic representation of the *STK11* gene to scale. Peaks corresponding to RNA sequencing reads were identified in intron GRCh38/hg38: chr19 1221341 1221948, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

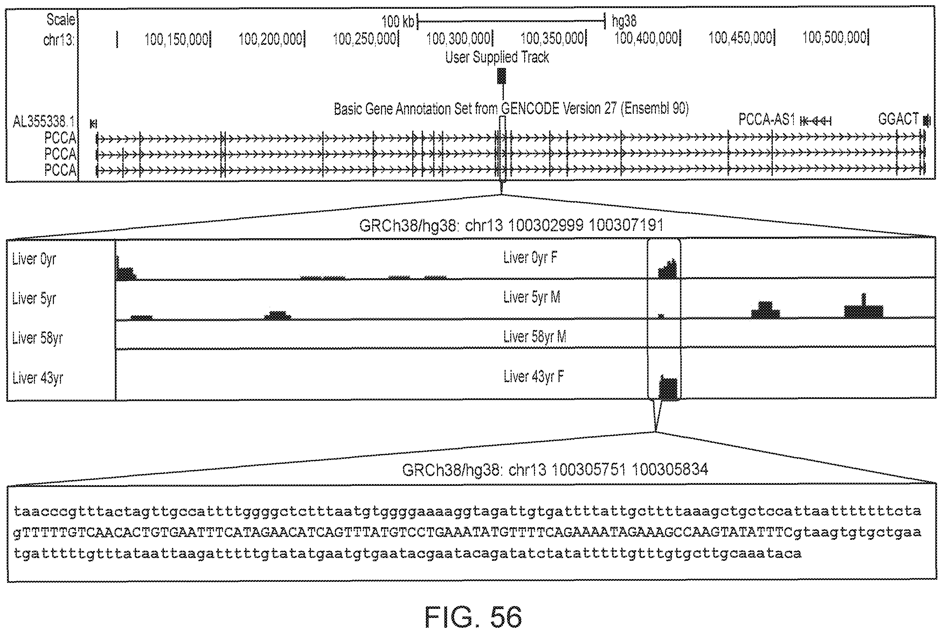

**FIG. 56** depicts identification of an exemplary nonsense-mediated mRNA decay (NMD)-inducing exon in the *PCCA* gene. The identification of the NMD-inducing exon in the *PCCA* gene using RNA sequencing is shown, visualized in the UCSC genome browser. The upper panel shows a graphic representation of the *PCCA* gene to scale. Peaks corresponding to RNA sequencing reads were identified in intron GRCh38/hg38: chr13 100302999 100307191, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

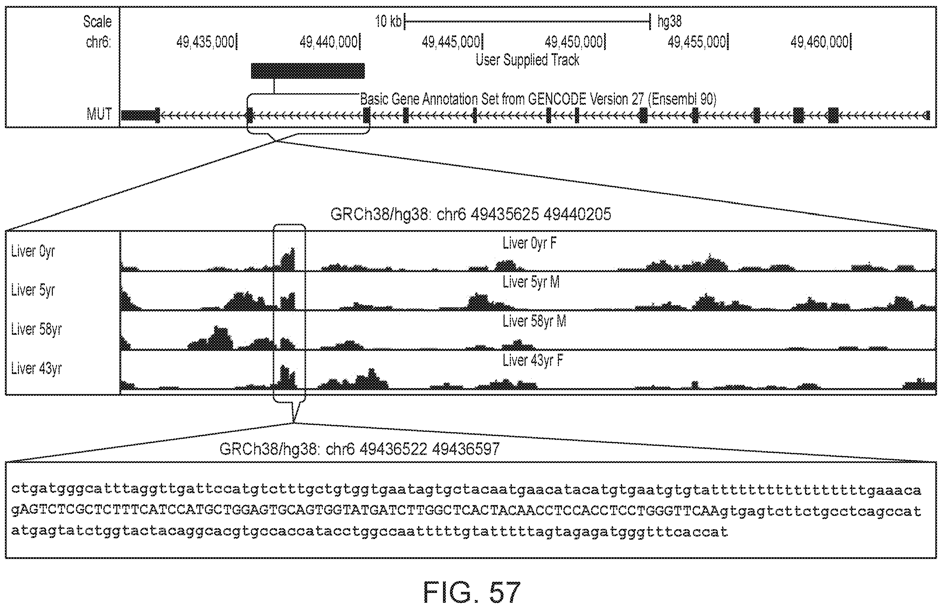

**FIG. 57** depicts identification of an exemplary nonsense-mediated mRNA decay (NMD)-inducing exon in the *MUT* gene. The identification of the NMD-inducing exon in the *MUT* gene using RNA sequencing is shown, visualized in the UCSC genome browser. The upper panel shows a graphic representation of the *MUT* gene to scale. Peaks corresponding to RNA sequencing reads were identified in intron GRCh38/hg38: chr6 49435625 49440205, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

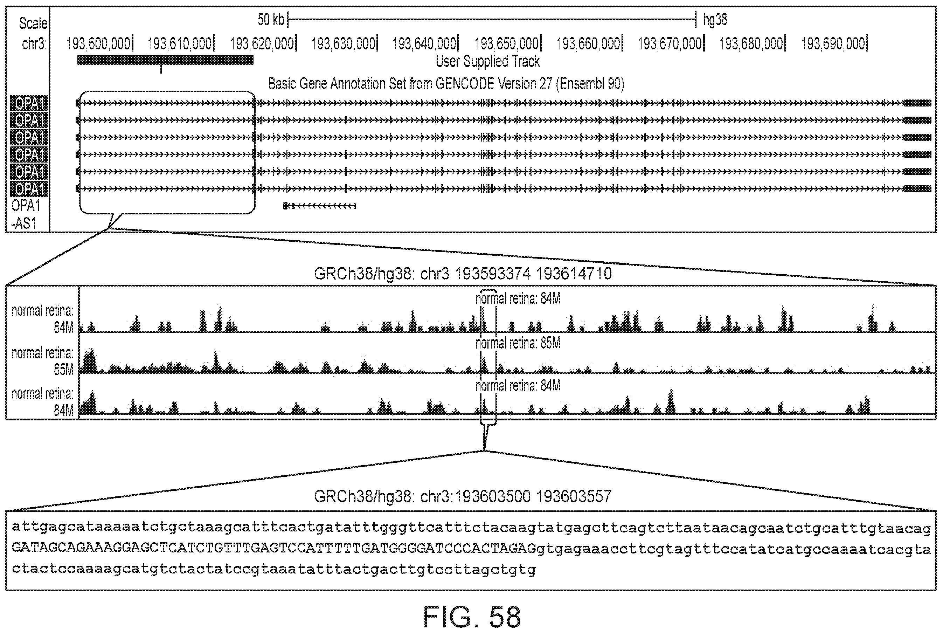

**FIG. 58** depicts identification of an exemplary nonsense-mediated mRNA decay (NMD)-inducing exon in the *OPA1* gene. The identification of the NMD-inducing exon in the *OPA1* gene using RNA sequencing is shown, visualized in the UCSC genome browser. The upper panel shows a graphic representation of the *OPA1* gene to scale. Peaks corresponding to RNA sequencing reads were identified in intron GRCh38/hg38: chr3 193593374 193614710, shown in the middle panel. Bioinformatic analysis identified an exon-like sequence (bottom panel, sequence highlighted in uppercase) flanked by 3' and 5' splice sites. Inclusion of this exon leads to the introduction of a premature termination codon rendering the transcript a target of NMD.

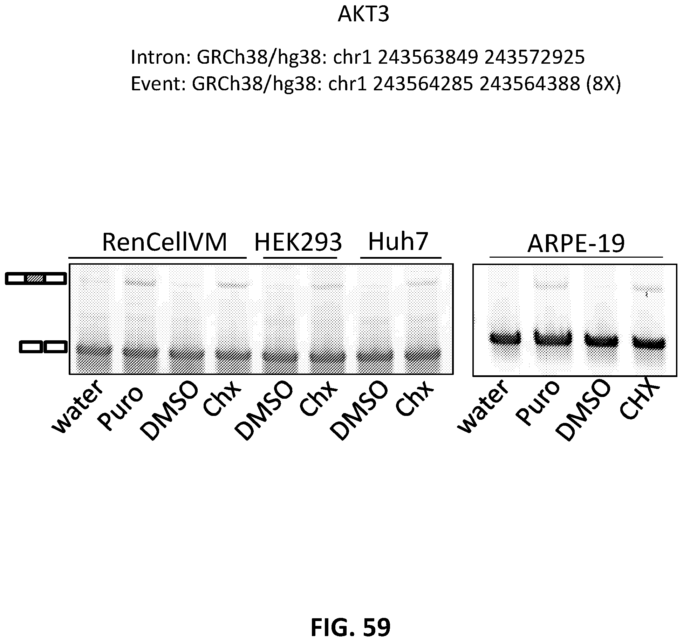

**FIG. 59** depicts confirmation of NMD-inducing exon via puromycin or cycloheximide treatment in various cell lines. RT-PCR analysis using total RNA from water-treated, DMSO-treated, puromycin-treated, or cycloheximide-treated cells confirmed the presence of a band corresponding to the NMD-inducing exon 8x (GRCh38/hg38: chr1 243564285 243564388) of *AKT3* gene.

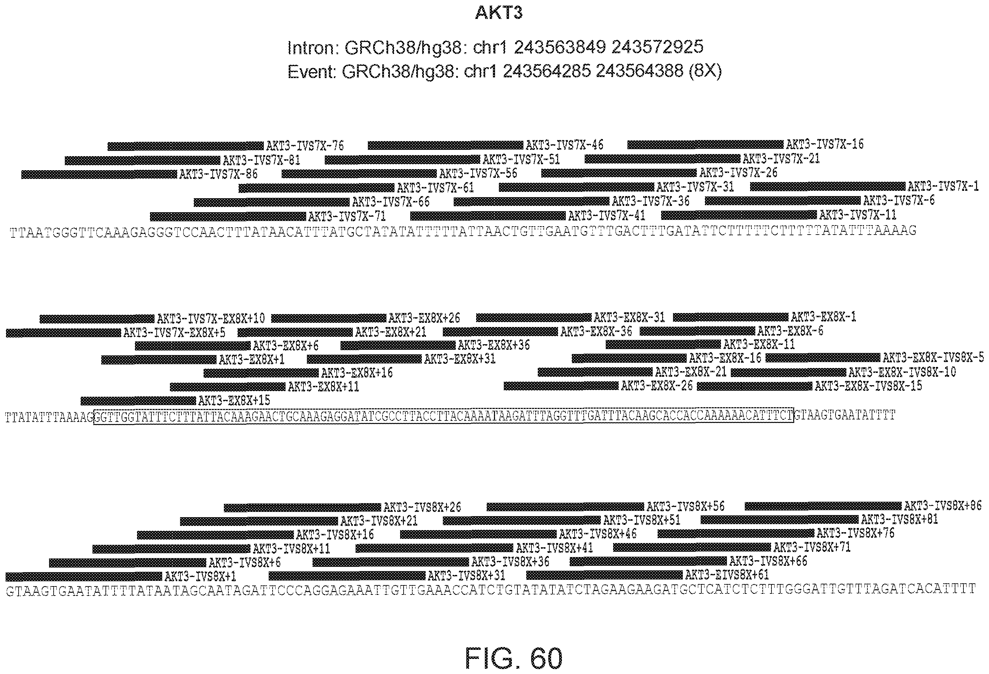

**FIG. 60** depicts an exemplary ASO walk around *AKT3* exon 8x (GRCh38/hg38: chr1 243564285 243564388) region. A graphic representation of an ASO walk performed for around *AKT3* exon 8x (GRCh38/hg38: chr1 243564285 243564388) region targeting sequences upstream of the 3' splice site, across the 3'splice site, exon 8x, across the 5' splice site, and downstream of the 5' splice site is shown. ASOs were designed to cover these regions by shifting 5 nucleotides at a time.

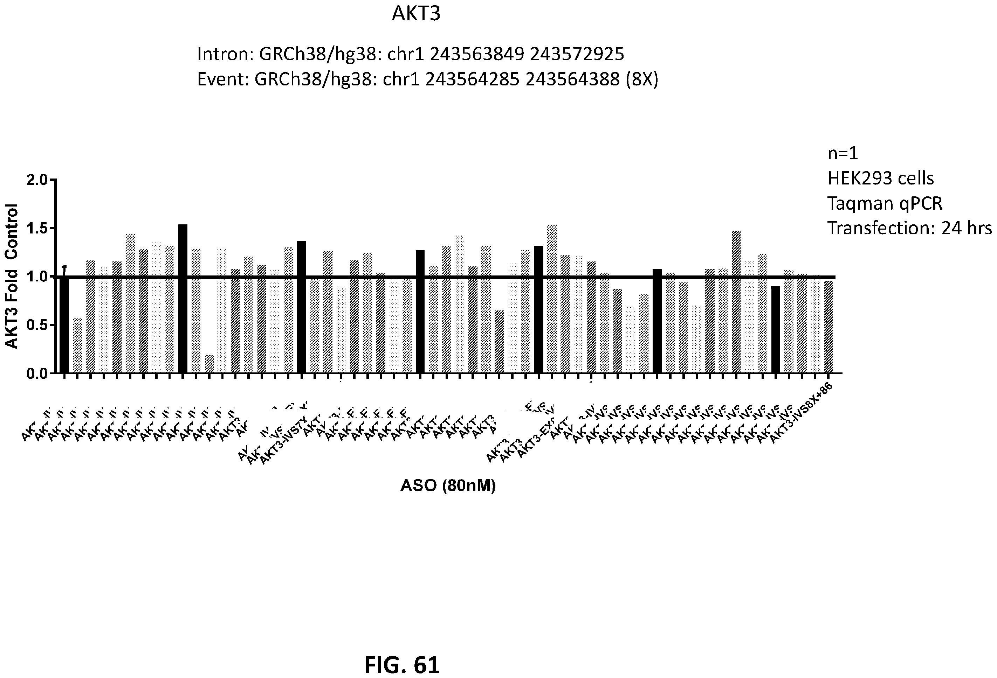

**FIG. 61** depicts *AKT3* exon 8x (GRCh38/hg38: chr1 243564285 243564388) region ASO walk evaluated by reverse transcription Taqman-qPCR. A graph of fold-change of the *AKT3* productive mRNA product relative to Sham is plotted.

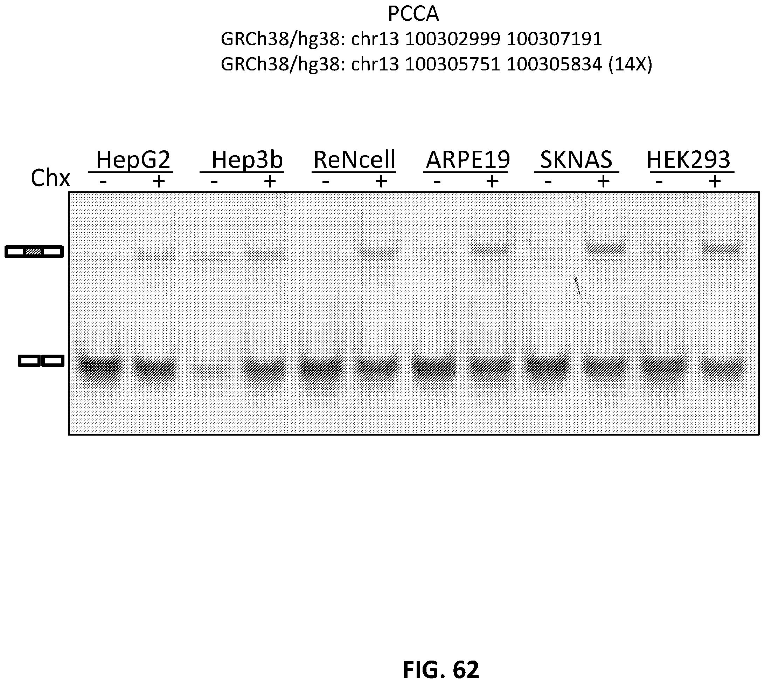

**FIG. 62** depicts confirmation of NMD-inducing exon via cycloheximide treatment in various cell lines. RT-PCR analysis using total RNA from DMSO-treated or cycloheximide-treated cells confirmed the presence of a band corresponding to the NMD-inducing exon 14x (GRCh38/hg38: chr13 100305751 100305834) of *PCCA* gene

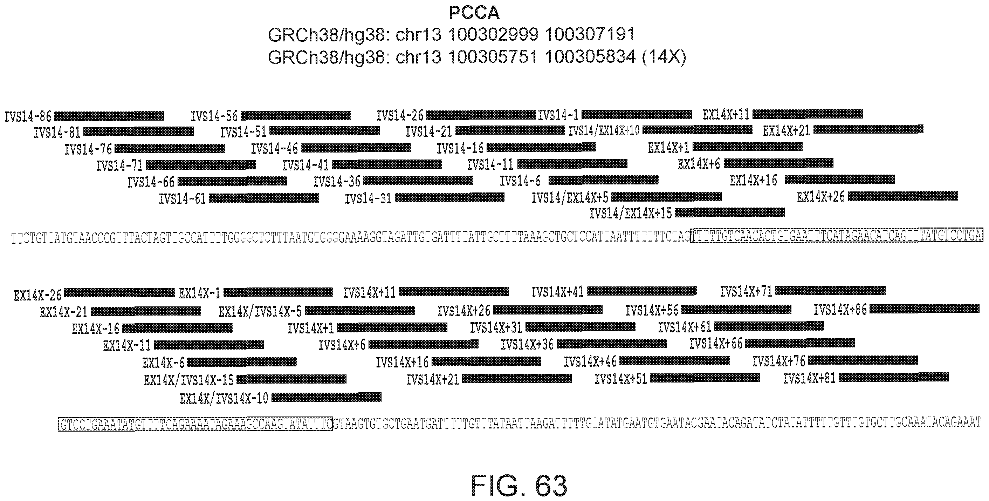

**FIG. 63** depicts an exemplary ASO walk around *PCCA* exon 14x (GRCh38/hg38: chr13 100305751 100305834) region. A graphic representation of an ASO walk performed for around *PCCA* exon 14x (GRCh38/hg38: chr13 100305751 100305834) region targeting sequences upstream of the 3' splice site, across the 3'splice site, exon 14x, across the 5' splice site, and downstream of the 5' splice site is shown. ASOs were designed to cover these regions by shifting 5 nucleotides at a time.

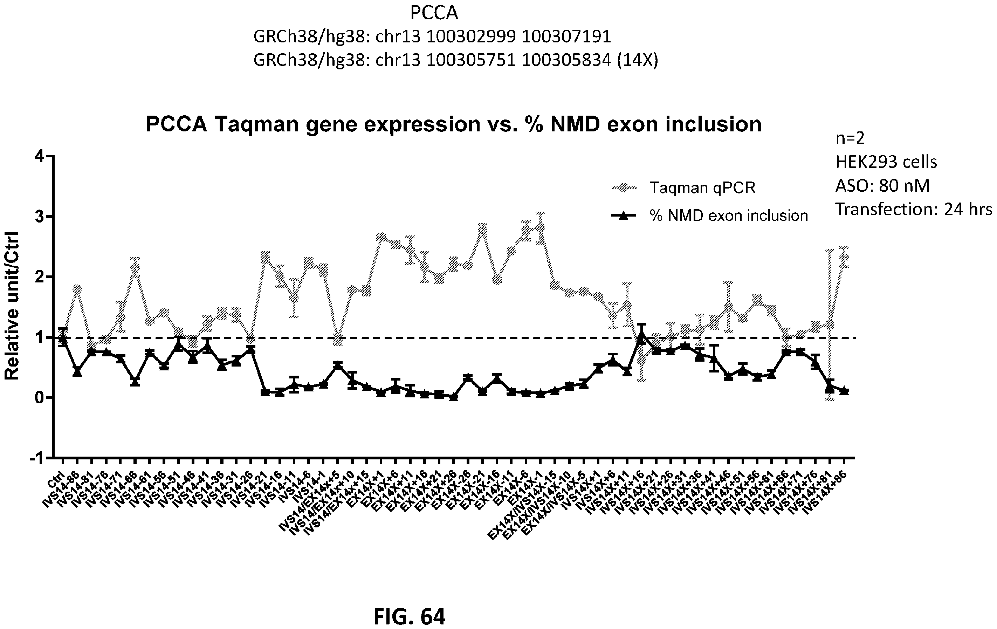

**FIG. 64** depicts *PCCA* exon 14x (GRCh38/hg38: chr13 100305751 100305834) region ASO walk evaluated by reverse transcription Taqman -qPCR and RT-PCR. A graph of fold-change of the *PCCA* productive mRNA product relative to Sham (grey) and percentage change in NMD exon inclusion (black) is plotted.

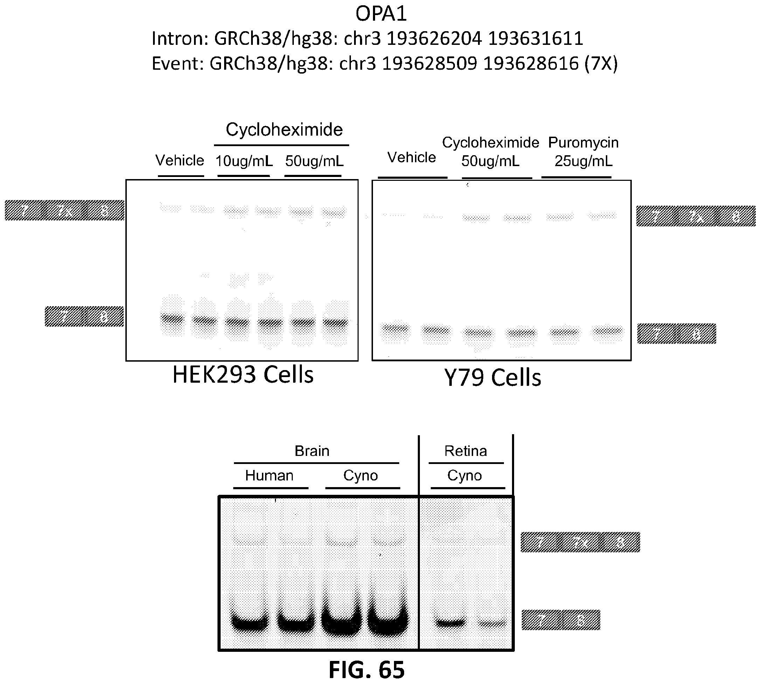

**FIG. 65** depicts confirmation of NMD-inducing exon via puromycin or cycloheximide treatment in various cell lines, as well as the confirmation of NMD-inducing exon in brain and retina samples. RT-PCR analysis using total RNA from water-treated, DMSO-treated, puromycin-treated, or cycloheximide-treated cells confirmed the presence of a band corresponding to the NMD-inducing exon 7x (GRCh38/hg38: chr3 193628509 193628616) of *OPA1* gene

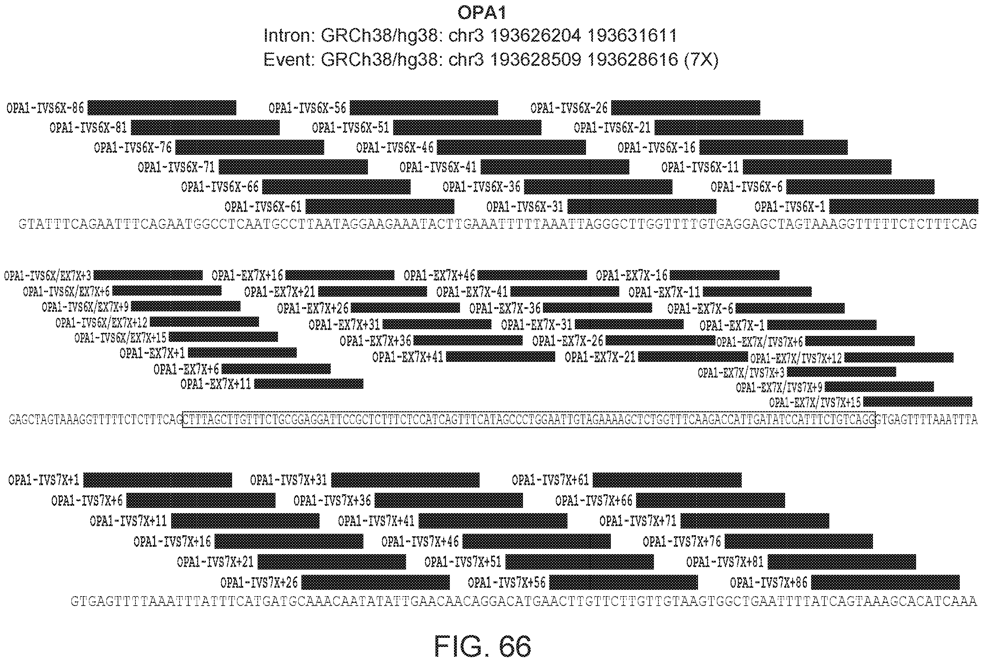

**FIG. 66** depicts an exemplary ASO walk around *OPA1* exon 7x (GRCh38/hg38: chr3 193628509 193628616) region. A graphic representation of an ASO walk performed for around *OPA1* exon 7x (GRCh38/hg38: chr3 193628509 193628616) region targeting sequences upstream of the 3' splice site, across the 3'splice site, exon 7x, across the 5' splice site, and downstream of the 5' splice site is shown. ASOs were designed to cover these regions by shifting 5 nucleotides at a time or 3 nucleotides across the splice site regions.

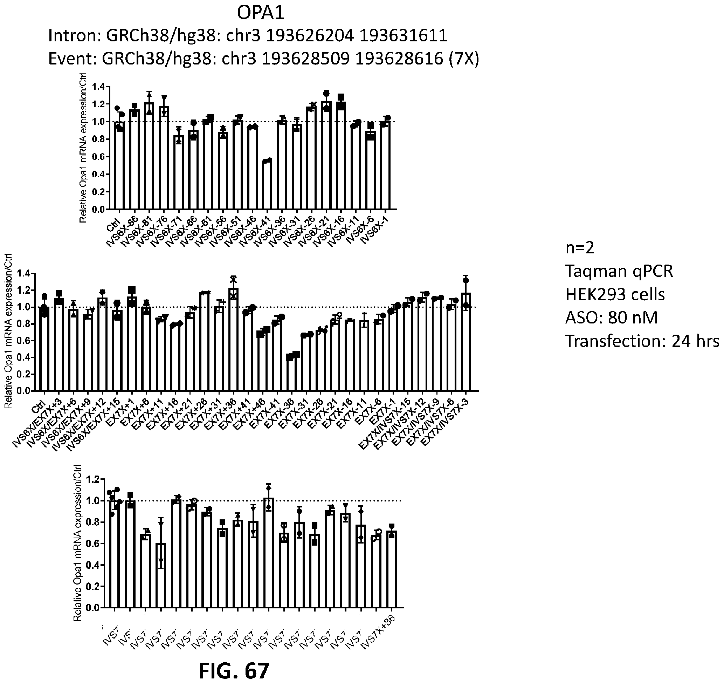

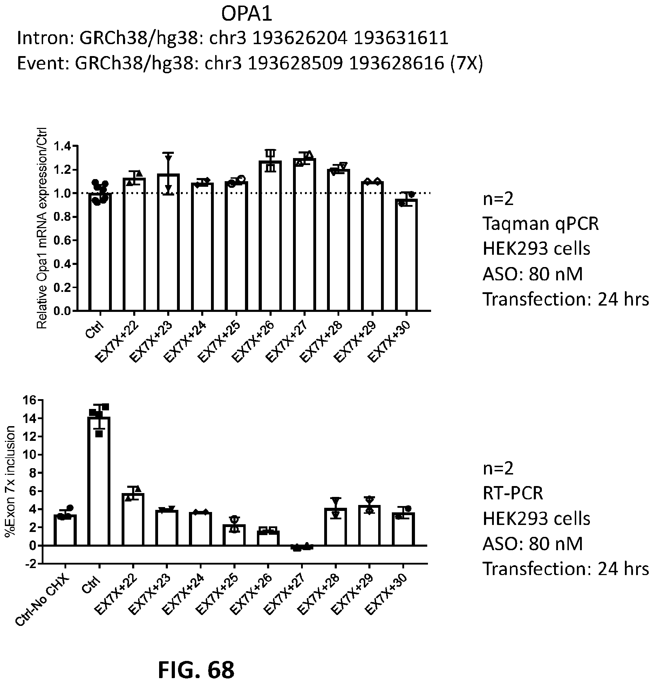

**FIGs. 67** and **68** depict *OPA1* exon 7x (GRCh38/hg38: chr3 193628509 193628616) region ASO walk evaluated by Taqman RT-qPCR. Graphs of fold-change of the *OPA1* productive mRNA product relative to Sham are plotted.

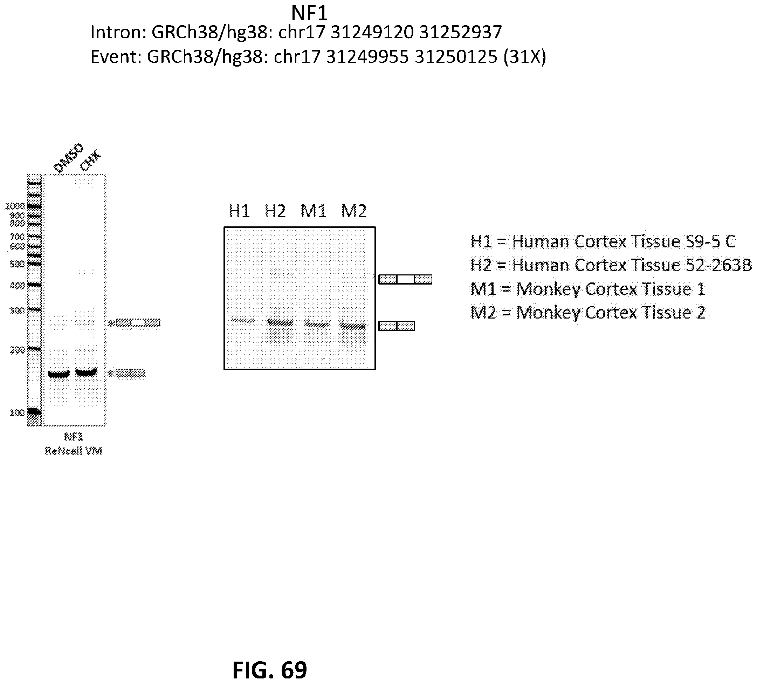

**FIG. 69** depicts confirmation of NMD-inducing exon via cycloheximide treatment in ReNCell VM and existence of NMD-inducing exon mRNA (*NF1*) in both human and monkey cortices. RT-PCR analysis using total RNA from DMSO-treated or cycloheximide-treated cells confirmed the presence of a band corresponding to the NMD-inducing exon 31x (GRCh38/hg38: chr17 31249955 31250125) of *NF1* gene

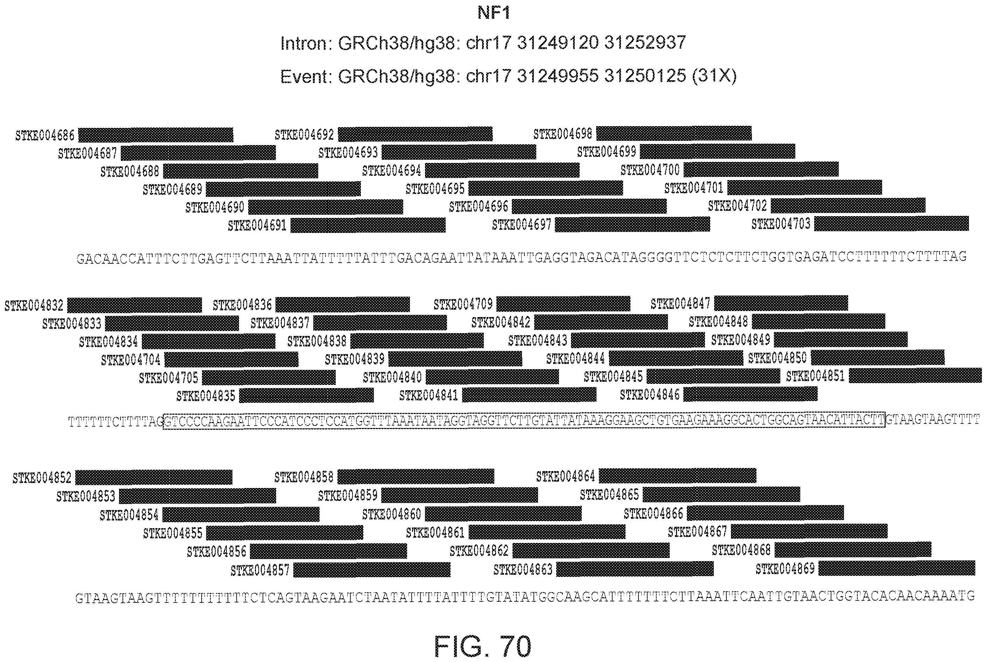

**FIG. 70** depicts an exemplary ASO walk around *NF1* exon 31x (GRCh38/hg38: chr17 31249955 31250125) region. A graphic representation of an ASO walk performed for around *NF1* exon 31x (GRCh38/hg38: chr17 31249955 31250125) region targeting sequences upstream of the 3' splice site, across the 3'splice site, exon 31x, across the 5' splice site, and downstream of the 5' splice site is shown. ASOs were designed to cover these regions by shifting 5 nucleotides at a time.

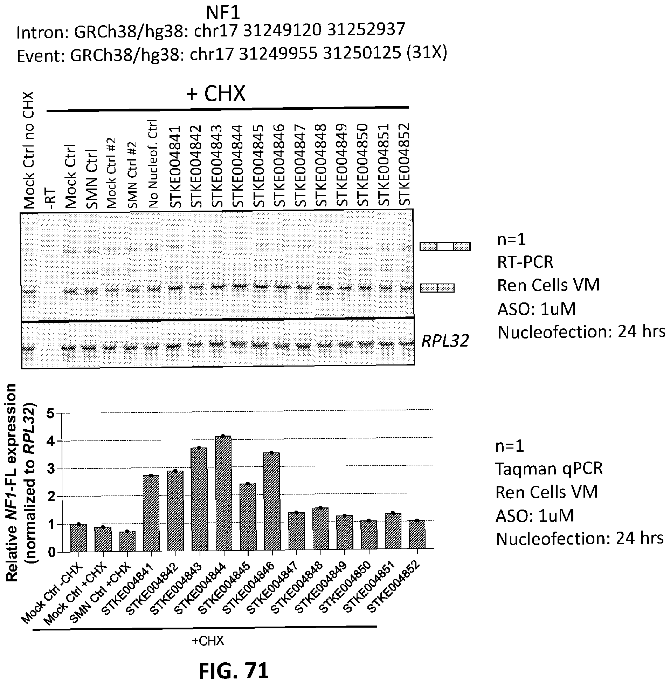

**FIG. 71** depicts *NF1* exon 31x (GRCh38/hg38: chr17 31249955 31250125) region ASO walk evaluated by RT-PCR (top) and RT-Taqman-qPCR (bottom). RT-PCR results indicating a decrease in exon 31x and a graph of fold-change of the *NF1* productive mRNA product relative to Sham are shown.

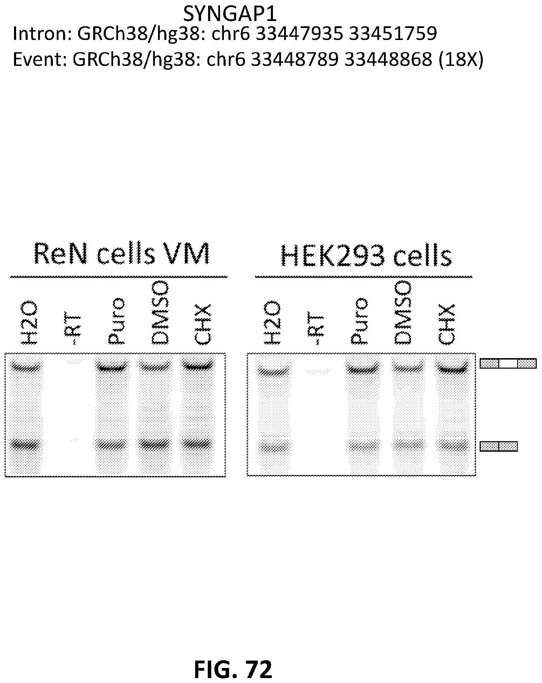

**FIG. 72** depicts confirmation of NMD-inducing exon via puromycin or cycloheximide treatment in various cell lines. RT-PCR analysis using total RNA from water-treated, DMSO-treated, puromycin-treated, or cycloheximide-treated cells confirmed the presence of a band corresponding to the NMD-inducing exon 18x (GRCh38/hg38: chr6 33448789 33448868) of *SYNGAP1* gene

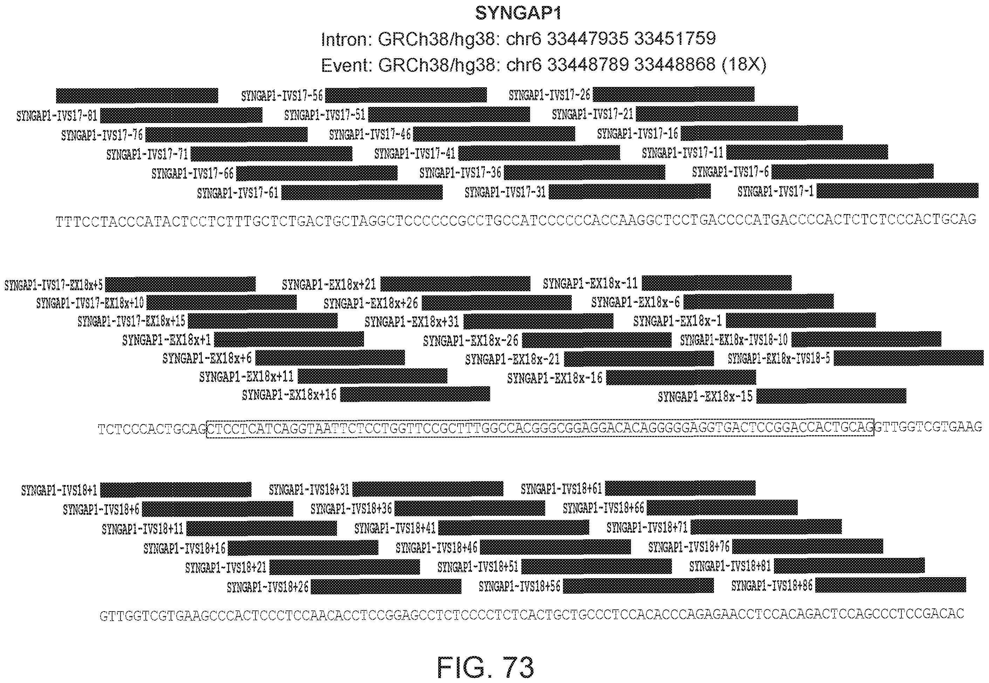

**FIG. 73** depicts an exemplary ASO walk around *SYNGAP1* exon 18x (GRCh38/hg38: chr6 33448789 33448868) region. A graphic representation of an ASO walk performed for around *SYNGAP1* exon 18x (GRCh38/hg38: chr6 33448789 33448868) region targeting sequences upstream of the 3' splice site, across the 3'splice site, exon 18x, across the 5' splice site, and downstream of the 5' splice site is shown. ASOs were designed to cover these regions by shifting 5 nucleotides at a time.

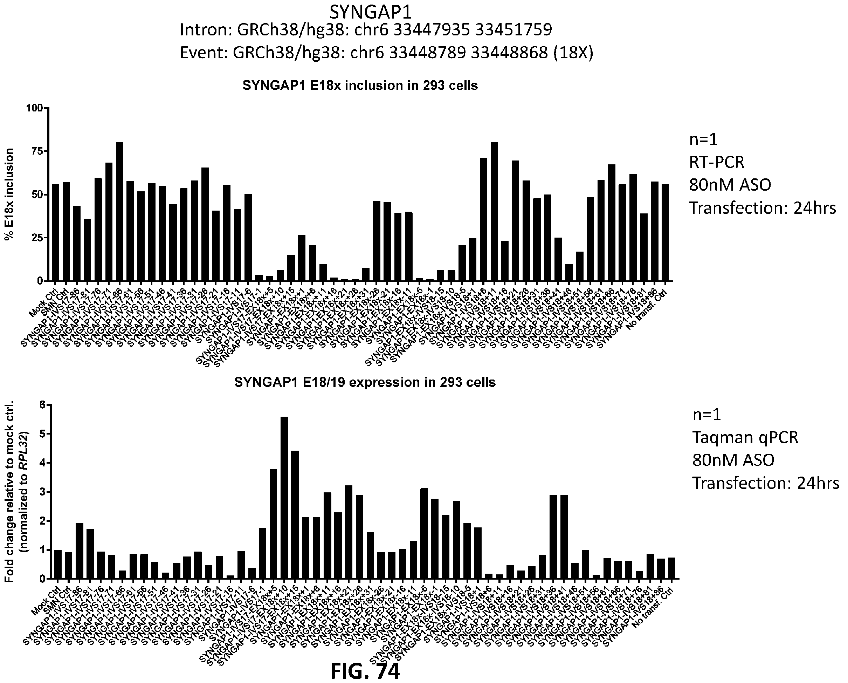

**FIG. 74** depicts *SYNGAP1* exon 18x (GRCh38/hg38: chr6 33448789 33448868) region ASO walk evaluated by RT-PCR (top) and Taqman-qPCR (bottom). Graphs of % exon 18x inclusion and fold-change of the *SYNGAP1* productive mRNA product relative to Sham are plotted (top and bottom, respectively).

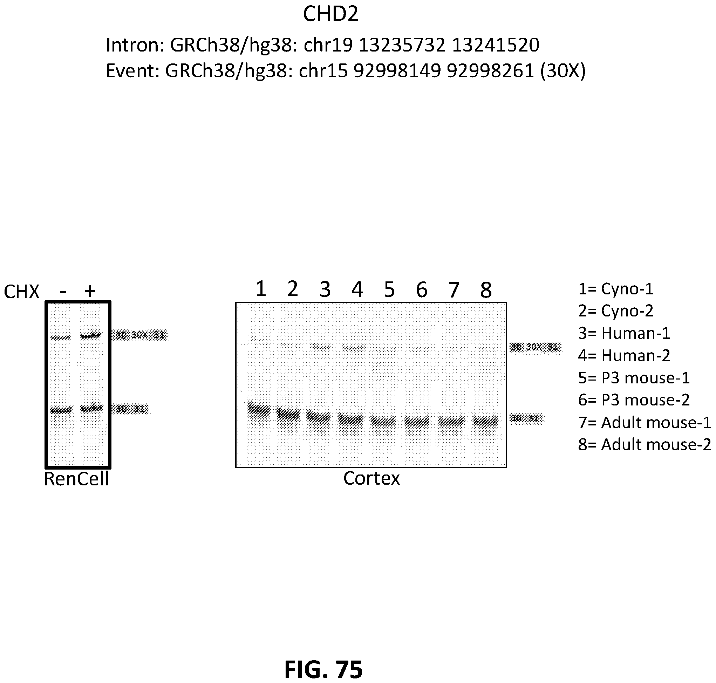

**FIG. 75** depicts confirmation of NMD-inducing exon via cycloheximide treatment. RT-PCR analysis using total RNA from DMSO-treated or cycloheximide-treated cells confirmed the presence of a band corresponding to the NMD-inducing exon 30x (GRCh38/hg38: chr15 92998149 92998261) *of CHD2* gene. Also shown is the RT-PCR analysis demonstrating the presence of mRNA containing NMD-inducing exon 30x in cortex samples from mouse, non-human primate and human.

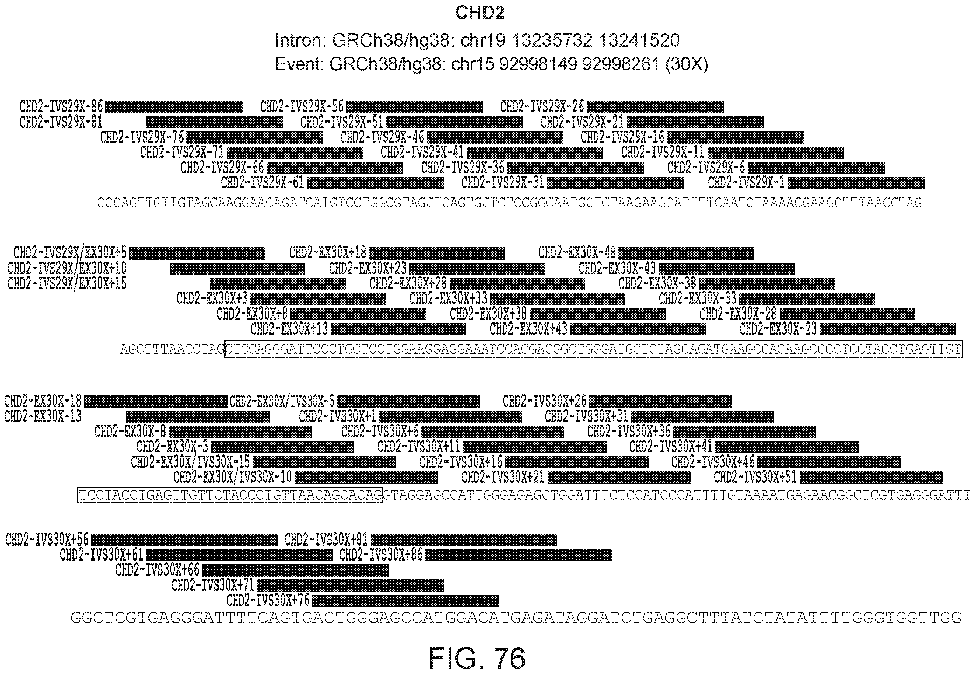

**FIG. 76** depicts an exemplary ASO walk around *CHD2* exon 30x (GRCh38/hg38: chr15 92998149 92998261) region. A graphic representation of an ASO walk performed for around *CHD2* exon 30x (GRCh38/hg38: chr15 92998149 92998261) region targeting sequences upstream of the 3' splice site, across the 3'splice site, exon 30x, across the 5' splice site, and downstream of the 5' splice site is shown. ASOs were designed to cover these regions by shifting 5 nucleotides at a time.

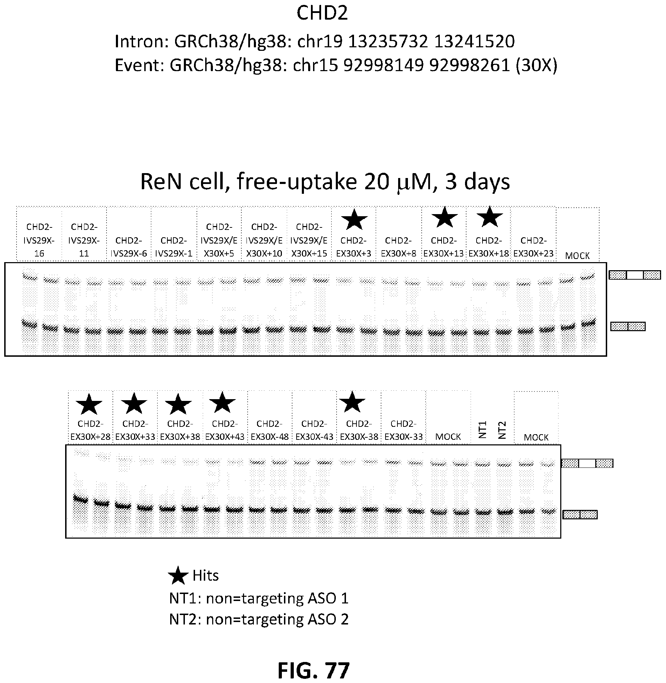

**FIG. 77** depicts *CHD2* exon 30x (GRCh38/hg38: chr15 92998149 92998261) region ASO walk evaluated by RT-PCR. RT-PCR results are shown demonstrating the changes in amount of mRNA containing NMD-inducing exon 30x.

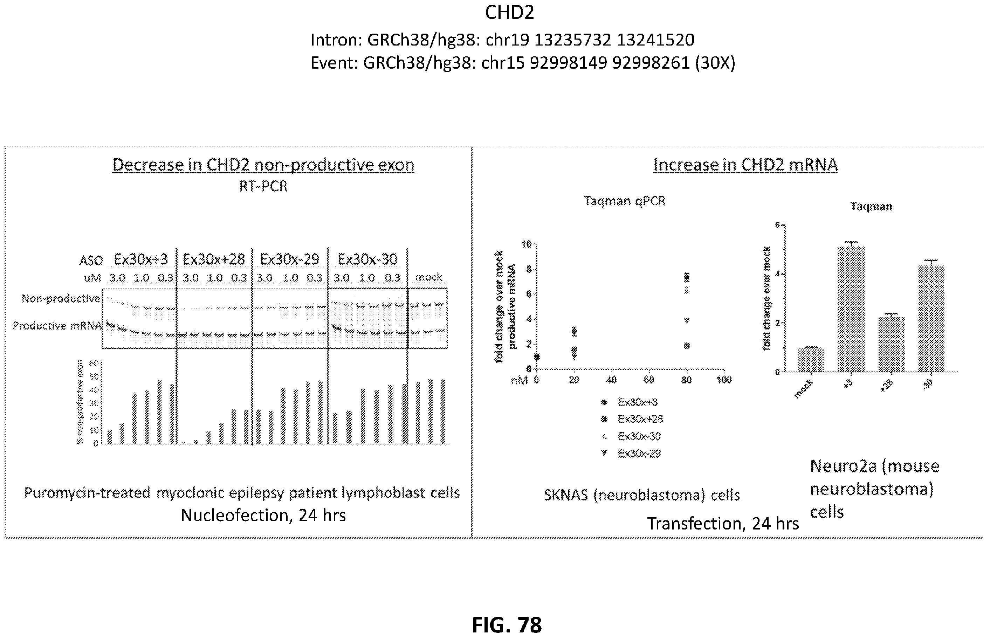

**FIG. 78** depicts changes induced by different ASOs in levels of *CHD2* non-productive exon (exon 30x (GRCh38/hg38: chr15 92998149 92998261)) and *CHD2* productive mRNA.

### DETAILED DESCRIPTION

Alternative splicing events in *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* gene can lead to non-productive mRNA transcripts which in turn can lead to aberrant protein expression, and therapeutic agents which can target the alternative splicing events in *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* gene can modulate the expression level of functional proteins in DS patients and/or inhibit aberrant protein expression. Such therapeutic agents can be used to treat a condition caused by ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein deficiency.

One of the alternative splicing events that can lead to non-productive mRNA transcripts is the inclusion of an extra exon in the mRNA transcript that can induce non-sense mediated mRNA decay. The present disclosure provides compositions and methods for modulating alternative splicing of *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* to increase the production of protein-coding mature mRNA, and thus, translated functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein. These compositions and methods include antisense oligomers (ASOs) that can cause exon skipping, e.g., pseudoexon skipping, and promote constitutive splicing of *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA. In various embodiments, functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein can be increased using the methods of the disclosure to treat a condition caused by ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein deficiency.

### Splicing and Nonsense-mediated mRNA Decay

Intervening sequences or introns are removed by a large and highly dynamic RNA-protein complex termed the spliceosome, which orchestrates complex interactions between primary transcripts, small nuclear RNAs (snRNAs) and a large number of proteins. Spliceosomes assemble ad hoc on each intron in an ordered manner, starting with recognition of the 5' splice site (5'ss) by U1 snRNA or the 3'splice site (3'ss) by the U2 pathway, which involves binding of the U2 auxiliary factor (U2AF) to the 3'ss region to facilitate U2 binding to the branch point sequence (BPS). U2AF is a stable heterodimer composed of a U2AF2-encoded 65-kD subunit (U2AF65), which binds the polypyrimidine tract (PPT), and a U2AF1-encoded 35-kD subunit (U2AF35), which interacts with highly conserved AG dinucleotides at 3'ss and stabilizes U2AF65 binding. In addition to the BPS/PPT unit and 3'ss/5'ss, accurate splicing requires auxiliary sequences or structures that activate or repress splice site recognition, known as intronic or exonic splicing enhancers or silencers. These elements allow genuine splice sites to be recognized among a vast excess of cryptic or pseudo-sites in the genome of higher eukaryotes, which have the same sequences but outnumber authentic sites by an order of magnitude. Although they often have a regulatory function, the exact mechanisms of their activation or repression are poorly understood.

The decision of whether to splice or not to splice can be typically modeled as a stochastic rather than deterministic process, such that even the most defined splicing signals can sometimes splice incorrectly. However, under normal conditions, pre-mRNA splicing proceeds at surprisingly high fidelity. This is attributed in part to the activity of adjacent cis-acting auxiliary exonic and intronic splicing regulatory elements (ESRs or ISRs). Typically, these functional elements are classified as either exonic or intronic splicing enhancers (ESEs or ISEs) or silencers (ESSs or ISSs) based on their ability to stimulate or inhibit splicing, respectively. Although there is now evidence that some auxiliary cis-acting elements may act by influencing the kinetics of spliceosome assembly, such as the arrangement of the complex between U1 snRNP and the 5'ss, it seems very likely that many elements function in concert with trans-acting RNA-binding proteins (RBPs). For example, the serine- and arginine-rich family of RBPs (SR proteins) is a conserved family of proteins that have a key role in defining exons. SR proteins promote exon recognition by recruiting components of the pre-spliceosome to adjacent splice sites or by antagonizing the effects of ESSs in the vicinity. The repressive effects of ESSs can be mediated by members of the heterogeneous nuclear ribonucleoprotein (hnRNP) family and can alter recruitment of core splicing factors to adjacent splice sites. In addition to their roles in splicing regulation, silencer elements are suggested to have a role in repression of pseudo-exons, sets of decoy intronic splice sites with the typical spacing of an exon but without a functional open reading frame. ESEs and ESSs, in cooperation with their cognate trans-acting RBPs, represent important components in a set of splicing controls that specify how, where and when mRNAs are assembled from their precursors.

The sequences marking the exon-intron boundaries are degenerate signals of varying strengths that can occur at high frequency within human genes. In multi-exon genes, different pairs of splice sites can be linked together in many different combinations, creating a diverse array of transcripts from a single gene. This is commonly referred to as alternative pre-mRNA splicing. Although most mRNA isoforms produced by alternative splicing can be exported from the nucleus and translated into functional polypeptides, different mRNA isoforms from a single gene can vary greatly in their translation efficiency. Those mRNA isoforms with premature termination codons (PTCs) at least 50 bp upstream of an exon junction complex are likely to be targeted for degradation by the nonsense-mediated mRNA decay (NMD) pathway. Mutations in traditional (BPS/PPT/3'ss/5'ss) and auxiliary splicing motifs can cause aberrant splicing, such as exon skipping or cryptic (or pseudo-) exon inclusion or splice-site activation, and contribute significantly to human morbidity and mortality. Both aberrant and alternative splicing patterns can be influenced by natural DNA variants in exons and introns.

Given that exon-intron boundaries can occur at any of the three positions of a codon, it is clear that only a subset of alternative splicing events can maintain the canonical open reading frame. For example, only exons that are evenly divisible by 3 can be skipped or included in the mRNA without any alteration of reading frame. Splicing events that do not have compatible phases will induce a frame-shift. Unless reversed by downstream events, frame-shifts can certainly lead to one or more PTCs, probably resulting in subsequent degradation by NMD. NMD is a translation-coupled mechanism that eliminates mRNAs containing PTCs. NMD can function as a surveillance pathway that exists in all eukaryotes. NMD can reduce errors in gene expression by eliminating mRNA transcripts that contain premature stop codons. Translation of these aberrant mRNAs could, in some cases, lead to deleterious gain-of-function or dominant-negative activity of the resulting proteins. NMD targets not only transcripts with PTCs but also a broad array of mRNA isoforms expressed from many endogenous genes, suggesting that NMD is a master regulator that drives both fine and coarse adjustments in steady-state RNA levels in the cell.

A NMD-inducing exon (NIE) is an exon or a pseudo-exon that is a region within an intron and can activate the NMD pathway if included in a mature RNA transcript. In constitutive splicing events, the intron containing an NIE is usually spliced out, but the intron or a portion thereof (e.g. NIE) may be retained during alternative or aberrant splicing events. Mature mRNA transcripts containing such an NIE may be non-productive due to frame shifts which induce the NMD pathway. Inclusion of a NIE in mature RNA transcripts may downregulate gene expression. mRNA transcripts containing an NIE may be referred to as "NIE containing mRNA" or "NMD exon mRNA" in the current disclosure.

Cryptic (or pseudo- splice sites) have the same splicing recognition sequences as genuine splice sites but are not used in splicing reactions. They outnumber genuine splice sites in the human genome by an order of a magnitude and are normally repressed by thus far poorly understood molecular mechanisms. Cryptic 5' splice sites have the consensus NNN/GUNNNN or NNN/GCNNNN where N is any nucleotide and / is the exon-intron boundary. Cryptic 3' splice sites have the consensus NAGN. Their activation is positively influenced by surrounding nucleotides that make them more similar to the optimal consensus of authentic splice sites, namely MAG/GURAGU and YAG/G, respectively, where M is C or A, R is G or A, and Y is C or U.

Splice sites and their regulatory sequences can be readily identified by a skilled person using suitable algorithms publicly available, listed for example in Kralovicova, J. and Vorechovsky, I. (2007) Global control of aberrant splice site activation by auxiliary splicing sequences: evidence for a gradient in exon and intron definition. Nucleic Acids Res., 35, 6399-6413, (http://www.ncbi.nlm.nih.gov/pmc/articles/PMC2095810/pdf/gkm680.pdf)

The cryptic splice sites or splicing regulatory sequences may compete for RNA-binding proteins, such as U2AF, with a splice site of the NIE. In some embodiments, an agent may bind to a cryptic splice site or splicing regulatory sequence to prevent binding of RNA-binding proteins and thereby favor binding of RNA-binding proteins to the NIE splice sites.

In some embodiments, the cryptic splice site may not comprise the 5' or 3' splice site of the NIE. In some embodiments, the cryptic splice site may be at least 10 nucleotides, at least 20 nucleotides, at least 50 nucleotides, at least 100 nucleotides or at least 200 nucleotides upstream of the NIE 5' splice site. In some embodiments, the cryptic splice site may be at least 10 nucleotides, at least 20 nucleotides, at least 50 nucleotides, at least 100 nucleotides, at least 200 nucleotides downstream of the NIE 3' splice site.

### Target Transcripts

In some embodiments, the methods of the present disclosure exploit the presence of NIE in the pre-mRNA transcribed from ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 genes. Splicing of the identified *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NRIH4, STK11, PPARA, CYP2J2, of SYNGAP1* NIE pre-mRNA species to produce functional mature *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* mRNA may be induced using a therapeutic agent such as an ASO that stimulates exon skipping of an NIE. Induction of exon skipping may result in inhibition of an NMD pathway. The resulting mature *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* mRNA can be translated normally without activating NMD pathway, thereby increasing the amount of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein in the patient's cells and alleviating symptoms of a condition or disease associated with ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 deficiency, such as Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; 16p11.2 deletion syndrome; Deafness, autosomal dominant 13; Cone-rod retinal dystrophy-2; Deafness, autosomal dominant 4A; Peripheral neuropathy, myopathy, hoarseness, and hearing loss; Deafness, autosomal dominant 22; Neurofibromatosis type 2; NASH; or Mental retardation, autosomal dominant 5.

In some embodiments, the diseases or conditions that can be treated or ameliorated using the method or composition disclosed herein are not directly associated with the target protein (gene) that the therapeutic agent targets. In some embodiments, a therapeutic agent provided herein can target a protein (gene) that is not directly associated with a disease or condition, but the modulation of expression of the target protein (gene) can treat or ameliorate the disease or condition. For instance, targeting genes like *CD46, CFH, CR1, DNAJC8, EIF2AK3, ERN1, GUCY2F, GUCY2F, SEMA3C, SEMA3D, SIRT3, or AKT3* by a therapeutic agent provided herein can treat or ameliorate eye diseases or conditions. In some embodiments, the targeting genes *CD46, CFH, CR1, DNAJC8, EIF2AK3, ERN1, GUCY2F, GUCY2F, SEMA3C, SEMA3D, SIRT3,* or *AKT3* are said to be indicated for Pathway (eye). In some embodiments, targeting gene like *SCN8A* can treat or ameliorate central nervous system diseases, e.g., epilepsy, e.g., Dravet syndrome. In some embodiments, such target genes like *SCN8A* are said to be indicated for Pathway (central nervous system) or Pathway (central nervous system, epilepsy).

In various embodiments, the present disclosure provides a therapeutic agent which can target *ABCB4, ASS1, ATP8Bl, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANKS, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERNI, GUCY2F, SIRT3, NR1H4, STK11, PPARA, CYP2J2, or SYNGAP1* mRNA transcripts to modulate splicing or protein expression level. The therapeutic agent can be a small molecule, polynucleotide, or polypeptide. In some embodiments, the therapeutic agent is an ASO. Various regions or sequences on the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA can be targeted by a therapeutic agent, such as an ASO. In some embodiments, the ASO targets a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANKS, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA transcript containing an NIE. In some embodiments, the ASO targets a sequence within an NIE of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA transcript. In some embodiments, the ASO targets a sequence upstream (or 5') from the 5' end of an NIE (3'ss) of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA transcript. In some embodiments, the ASO targets a sequence downstream (or 3') from the 3' end of an NIE (5'ss) of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* pre-mRNA transcript. In some embodiments, the ASO targets a sequence that is within an intron flanking on the 5' end of the NIE of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* pre-mRNA transcript. In some embodiments, the ASO targets a sequence that is within an intron flanking the 3' end of the NIE of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* pre-mRNA transcript. In some embodiments, the ASO targets a sequence comprising an NIE-intron boundary of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA transcript. An NIE-intron boundary can refer to the junction of an intron sequence and an NIE region. The intron sequence can flank the 5' end of the NIE, or the 3' end of the NIE. In some embodiments, the ASO targets a sequence within an exon of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* pre-mRNA transcript. In some embodiments, the ASO targets a sequence within an intron of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA transcript. In some embodiments, the ASO targets a sequence comprising both a portion of an intron and a portion of an exon of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA transcript.

In some embodiments, the ASO targets a sequence about 4 to about 300 nucleotides upstream (or 5') from the 5' end of the NIE. In some embodiments, the ASO targets a sequence about 1 to about 20 nucleotides, about 20 to about 50 nucleotides, about 50 to about 100 nucleotides, about 100 to about 150 nucleotides, about 150 to about 200 nucleotides, about 200 to about 250 nucleotides, or about 250 to about 300 nucleotides upstream (or 5') from the 5' end of the NIE region. In some embodiments, the ASO may target a sequence more than 300 nucleotides upstream from the 5' end of the NIE. In some embodiments, the ASO targets a sequence about 4 to about 300 nucleotides downstream (or 3') from the 3' end of the NIE. In some embodiments, the ASO targets a sequence about 1 to about 20 nucleotides, about 20 to about 50 nucleotides, about 50 to about 100 nucleotides, about 100 to about 150 nucleotides, about 150 to about 200 nucleotides, about 200 to about 250 nucleotides, or about 250 to about 300 nucleotides downstream from the 3' end of the NIE. In some embodiments, the ASO targets a sequence more than 300 nucleotides downstream from the 3' end of the NIE.

In some embodiments, the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA transcript is encoded by a genetic sequence with at least about 80%, 85%, 90%, 95%, 96%, 97%, 98%, 99% or 100% sequence identity to any one of SEQ ID NO. 1-59 or 192-211. In some embodiments, the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANKS, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE pre-mRNA transcript comprises a sequence with at least about 80%, 85%, 90%, 95%, 96%, 97%, 98%, 99% or 100% sequence identity to any one of SEQ ID NOs: 60-191.

In some embodiments, the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA transcript (or NMD exon mRNA) comprises a sequence with at least about 80%, 85%, 90%, 95%, 97%, or 100% sequence identity to any one of SEQ ID NOs: 60-191. In some embodiments, *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA transcript (or NMD exon mRNA) is encoded by a sequence with at least about 80%, 85%, 90%, 95%, 97%, or 100% sequence identity to any one of SEQ ID NOs: 60-191. In some embodiments, the targeted portion of the NMD exon mRNA comprises a sequence with at least 80%, 85%, 90%, 95%, 97%, or 100% sequence identity to a region comprising at least 8 contiguous nucleic acids of any one of SEQ ID NOs: 60-191.

In some embodiments, the ASO targets exon 8x of a ABCB4 NIE containing pre-mRNA comprising NIE exon 8, exon 9x of a ASS1 NIE containing pre-mRNA comprising NIE exon 9, exon 16x of a ATP8B1 NIE containing pre-mRNA comprising NIE exon 16, exon 1x of a BAG3 NIE containing pre-mRNA comprising NIE exon 1, exon 31x of a CACNA1A NIE containing pre-mRNA comprising NIE exon 31, exon 36x of a CACNA1A NIE containing pre-mRNA comprising NIE exon 36, exon 37x of a CACNA1A NIE containing pre-mRNA comprising NIE exon 37, exon 3x of a CBS NIE containing pre-mRNA comprising NIE exon 3, exon 12x of a CBS NIE containing pre-mRNA comprising NIE exon 12, exon 1x of a CD55 NIE containing pre-mRNA comprising NIE exon 1, exon 16x of a CDKL5 NIE containing pre-mRNA comprising NIE exon 16, exon 3x of CFH NIE containing pre-mRNA comprising NIE exon 3, exon 30x of a CHD2 NIE containing pre-mRNA comprising NIE exon 30, exon 4x of CHRNA7 NIE containing pre-mRNA comprising NIE exon 4, exon 1x of CISD2 NIE containing pre-mRNA comprising NIE exon 1, exon 15x of CLN3 NIE containing pre-mRNA comprising NIE exon 15, exon 11x of a COL4A3 NIE containing pre-mRNA comprising NIE exon 11, exon 41x of a COL4A3 NIE containing pre-mRNA comprising NIE exon 41, exon 22x of a COL4A4 NIE containing pre-mRNA comprising NIE exon 22, exon 44x of a COL4A4 NIE containing pre-mRNA comprising NIE exon 44, exon 20x of DEPDC5 NIE containing pre-mRNA comprising NIE exon 20, exon 2x of a DHDDS NIE containing pre-mRNA comprising NIE exon 2, exon 3x of a ELOVL4 NIE containing pre-mRNA comprising NIE exon 3, exon 5x of a FAH NIE containing pre-mRNA comprising NIE exon 5, exon 4x of FXN NIE containing pre-mRNA comprising NIE exon 4, exon 4x of a GALE NIE containing pre-mRNA comprising NIE exon 4, exon 3x of a GBE1 NIE containing pre-mRNA comprising NIE exon 3, exon 11x of GRIN2A NIE containing pre-mRNA comprising NIE exon 11, exon 1x of GRN NIE containing pre-mRNA comprising NIE exon 1, exon 2x of a HEXA NIE containing pre-mRNA comprising NIE exon 2, exon 2x of a KANSL1 NIE containing pre-mRNA comprising NIE exon 2, exon 1x of a KCNQ2 NIE containing pre-mRNA comprising NIE exon 1, exon 50x of a KMT2D NIE containing pre-mRNA comprising NIE exon 50, exon 8x of MAPK3 NIE containing pre-mRNA comprising NIE exon 8, exon 13x of MBD5 NIE containing pre-mRNA comprising NIE exon 13, exon 2x of a MECP2 NIE containing pre-mRNA comprising NIE exon 2, exon 11x of MUT NIE containing pre-mRNA comprising NIE exon 11, exon 31x of NF1 NIE containing pre-mRNA comprising NIE exon 31, exon 7x of a NIPBL NIE containing pre-mRNA comprising NIE exon 7, exon 38x of a NIPBL NIE containing pre-mRNA comprising NIE exon 38, exon 11x of a NSD1 NIE containing pre-mRNA comprising NIE exon 11, exon 6x of a OPA1 NIE containing pre-mRNA comprising NIE exon 6, exon 28x of a OPA1 NIE containing pre-mRNA comprising NIE exon 28, exon 1x of OPTN NIE containing pre-mRNA comprising NIE exon 1, exon 1x of PCCA NIE containing pre-mRNA comprising NIE exon 1, exon 5x of a PCCB NIE containing pre-mRNA comprising NIE exon 5, exon 6x of a PCCB NIE containing pre-mRNA comprising NIE exon 6, exon 4x of a PKP2 NIE containing pre-mRNA comprising NIE exon 4, exon 23x of a PLCB1 NIE containing pre-mRNA comprising NIE exon 23, exon 3x of PRPF3 NIE containing pre-mRNA comprising NIE exon 3, exon 9x of PRPF31 NIE containing pre-mRNA comprising NIE exon 9, exon 1x of a RAI1 NIE containing pre-mRNA comprising NIE exon 1, exon 5x of RBFOX2 NIE containing pre-mRNA comprising NIE exon 5, exon 13x of SCN2A NIE containing pre-mRNA comprising NIE exon 13, exon 6x of SCN3A NIE containing pre-mRNA comprising NIE exon 6, exon 7x of SCN3A NIE containing pre-mRNA comprising NIE exon 7, exon 4x of SCN8A NIE containing pre-mRNA comprising NIE exon 4, exon 6x of SCN8A NIE containing pre-mRNA comprising NIE exon 6, exon 20x of SCN8A NIE containing pre-mRNA comprising NIE exon 20, exon 6x of SCN9A NIE containing pre-mRNA comprising NIE exon 6, exon 24x of SHANK3 NIE containing pre-mRNA comprising NIE exon 24, exon 3x of a SLC25A13 NIE containing pre-mRNA comprising NIE exon 3, exon 6x of a SLC25A13 NIE containing pre-mRNA comprising NIE exon 6, exon 9x of a SLC25A13 NIE containing pre-mRNA comprising NIE exon 9, exon 11x of a SLC25A13 NIE containing pre-mRNA comprising NIE exon 11, exon 13x of a SLC25A13 NIE containing pre-mRNA comprising NIE exon 13, exon 1x of SLC6A1 NIE containing pre-mRNA comprising NIE exon 1, exon 12x of a SPTAN1 NIE containing pre-mRNA comprising NIE exon 12, exon 10x of a TEK NIE containing pre-mRNA comprising NIE exon 10, exon 15x of a TEK NIE containing pre-mRNA comprising NIE exon 15, exon 1x of TOPORS NIE containing pre-mRNA comprising NIE exon 1, exon 11x of a TSC2 NIE containing pre-mRNA comprising NIE exon 11, exon 30x of a TSC2 NIE containing pre-mRNA comprising NIE exon 30, exon 1x of UBE3A NIE containing pre-mRNA comprising NIE exon 1, or exon 7x of a VCAN NIE containing pre-mRNA comprising NIE exon 7. In some embodiments, the ASO targets exon (GRCh38/ hg38: chrl 243564285 243564388) of AKT3; exon (GRCh38/ hg38: chr19 13236449 13236618) of CACNA1A; exon (GRCh38/ hg38: chr21 43059730 43060012) of CBS; exon (GRCh38/ hg38: chr1 207775610 207775745) of CD46; exon (GRCh38/ hg38: chr1 196675450 196675529) of CFH; exon (GRCh38/ hg38: chr15 92998149 92998261) of CHD2; exon (GRCh38/ hg38: chr16 28479644 28479765) of CLN3; exon (GRCh38/ hg38: chr6 33183634 33183698) of COL11A2; exon (GRCh38/ hg38: chr2 227296487 227296526) of COL4A3; exon (GRCh38/ hg38: chr2 227144653 227144833) of COL4A4; exon (GRCh38/ hg38: chr2 227015283 227015360) of COL4A4; exon (GRCh38/ hg38: chr1 207637688 207637848) of CR1; exon (GRCh38/ hg38: chr19 47835403 47835579) of CRX; exon (GRCh38/ hg38: chr1 59904366 59904516) of CYP2J2; exon (GRCh38/ hg38: chr1 26442335 26442372) of DHDDS; exon (GRCh38/ hg38: chr1 28230131 28230252 ) of DNAJC8; exon (GRCh38/ hg38: chr2 88582755 88582824) of EIF2AK3; exon (GRCh38/ hg38: chr17 64102673 64102804) of ERN1; exon (GRCh38/ hg38: chr1 23798311 23798484) of GALE; exon (GRCh38/ hg38: chrX 109383365 109383446) of GUCY2F; exon (GRCh38/ hg38: chrX 109439038 109439175) of GUCY2F; exon (GRCh38/ hg38: chr15 72362376 72362466) of HEXA; exon (GRCh38/ hg38: chr15 72345677 72345776) of HEXA; exon (GRCh38/ hg38: chr16 30115595 30115645) of MAPK3; exon (GRCh38/ hg38: chr2 148460219 148460304) of MBDS; exon (GRCh38/ hg38: chr2 148490695 148490787) of MBD5; exon (GRCh38/ hg38: chr2 148505761 148505830) of MBD5; exon (GRCh38/ hg38: chr6 49436522 49436597) of MUT; exon (GRCh38/ hg38: chr19 50230825 50230999) of MYH14; exon (GRCh38/ hg38: chr6 75867431 75867523) of MYO6; exon (GRCh38/ hg38: chr17 31249955 31250125) of NF1; exon (GRCh38/ hg38: chr22 29628658 29628773) of NF2; exon (GRCh38/ hg38: chr5 37048127 37048354) of NIPBL; exon (GRCh38/ hg38: chr12 100499841 100500024) of NR1H4; exon (GRCh38/ hg38: chr5 177169394 177169559) of NSD1; exon (GRCh38/ hg38: chr5 177200761 177200783) of NSD1; exon (GRCh38/ hg38: chr5 177247924 177248079) of NSD1; exon (GRCh38/ hg38: chr5 177275947 177276101) of NSD1; exon (GRCh38/ hg38: chr3 193628509 193628616) of OPA1; exon (GRCh38/ hg38: chr3 193603500 193603557) of OPA1; exon (GRCh38/ hg38: chr13 100305751 100305834) of PCCA; exon (GRCh38/ hg38: chr12 32894516 32894778) of PKP2; exon (GRCh38/ hg38: chr22 46203575 46203752) of PPARA; exon (GRCh38/ hg38: chr1 150327557 150327652) of PRPF3; exon (GRCh38/ hg38: chr1 150330401 150330498) of PRPF3; exon (GRCh38/ hg38: chr2 165327155 165327202) of SCN2A; exon (GRCh38/ hg38: chr12 51688758 51688849) of SCN8A; exon (GRCh38/ hg38: chr12 51780202 51780271) of SCN8A; exon (GRCh38/ hg38: chr2 166304238 166304329) of SCN9A; exon (GRCh38/ hg38: chr7 80794854 80794957) of SEMA3C; exon (GRCh38/ hg38: chr7 85059498 85059541) of SEMA3D; exon (GRCh38/ hg38: chr11 225673 226081) of SIRT3; exon (GRCh38/ hg38: chr19 1216268 1216398) of STK11; exon (GRCh38/ hg38: chr19 1221621 1221846) of STK11; exon (GRCh38/ hg38: chr6 33448789 33448868) of SYNGAP1; exon (GRCh38/ hg38: chr9 32551365 32551469) of TOPORS; exon (GRCh38/ hg38: chr5 83544965 83545070) of VCAN.

In some embodiments, the ASO targets a sequence about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream (or 5') from the 5' end of exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAR, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN. In some embodiments, the ASO targets a sequence about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream (or 5') from GRCh38/ hg38: chrl 243564388 of AKT3; GRCh38/ hg38: chr19 13236618 of CACNA1A; GRCh38/ hg38: chr21 43060012 of CBS; GRCh38/ hg38: chrl 207775610 of CD46; GRCh38/ hg38: chrl 196675450 of CFH; GRCh38/ hg38: chr15 92998149 of CHD2; GRCh38/ hg38: chr16 28479765 of CLN3; GRCh38/ hg38: chr6 33183698 of COL11A2; GRCh38/ hg38: chr2 227296487 of COL4A3; GRCh38/ hg38: chr2 227144833 of COL4A4; GRCh38/ hg38: chr2 227015360 of COL4A4; GRCh38/ hg38: chrl 207637688 of CR1; GRCh38/ hg38: chr19 47835403 of CRX; GRCh38/ hg38: chr1 59904516 of CYP2J2; GRCh38/ hg38: chr1 26442335 of DHDDS; GRCh38/ hg38: chr1 28230252 of DNAJC8; GRCh38/ hg38: chr2 88582824 of EIF2AK3; GRCh38/ hg38: chr17 64102804 of ERN1; GRCh38/ hg38: chr1 23798484 of GALE; GRCh38/ hg38: chrX 109383446 of GUCY2F; GRCh38/ hg38: chrX 109439175 of GUCY2F; GRCh38/ hg38: chr15 72362466 of HEXA; GRCh38/ hg38: chr15 72345776 of HEXA; GRCh38/ hg38: chr16 30115645 ofMAPK3; GRCh38/ hg38: chr2 148460219 ofMBD5; GRCh38/ hg38: chr2 148490695 ofMBD5; GRCh38/ hg38: chr2 148505761 ofMBD5; GRCh38/ hg38: chr6 49436597 of MUT; GRCh38/ hg38: chr19 50230825 of MYH14; GRCh38/ hg38: chr6 75867431 of MYO6; GRCh38/ hg38: chr17 31249955 of NF1; GRCh38/ hg38: chr22 29628658 of NF2; GRCh38/ hg38: chr5 37048127 of NIPBL; GRCh38/ hg38: chr12 100499841 of NR1H4; GRCh38/ hg38: chr5 177169394 of NSD1; GRCh38/ hg38: chr5 177200761 of NSD1; GRCh38/ hg38: chr5 177247924 of NSD1; GRCh38/hg38: chr5 177275947 of NSD1; GRCh38/hg38: chr3 193628509 of OPA1; GRCh38/ hg38: chr3 193603500 of OPA1; GRCh38/ hg38: chr13 100305751 ofPCCA; GRCh38/ hg38: chr12 32894778 of PKP2; GRCh38/ hg38: chr22 46203575 ofPPARA; GRCh38/ hg38: chr1 150327557 of PRPF3; GRCh38/ hg38: chr1 150330401 of PRPF3; GRCh38/ hg38: chr2 165327155 of SCN2A; GRCh38/ hg38: chr12 51688758 of SCN8A; GRCh38/ hg38: chr12 51780202 of SCN8A; GRCh38/ hg38: chr2 166304329 of SCN9A; GRCh38/ hg38: chr7 80794957 of SEMA3C; GRCh38/ hg38: chr7 85059541 of SEMA3D; GRCh38/ hg38: chr11 226081 of SIRT3; GRCh38/ hg38: chr19 1216268 of STK11; GRCh38/ hg38: chr19 1221621 of STK11; GRCh38/ hg38: chr6 33448789 of SYNGAP1; GRCh38/ hg38: chr9 32551469 of TOPORS; or GRCh38/ hg38: chr5 83544965 of VCAN.

In some embodiments, the ASO targets a sequence at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream (or 5') from the 5' end of exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAR, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBDS, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN. In some embodiments, the ASO targets a sequence at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream (or 5') from GRCh38/ hg38: chr1 243564388 of AKT3; GRCh38/ hg38: chr19 13236618 of CACNA1A; GRCh38/ hg38: chr21 43060012 of CBS; GRCh38/ hg38: chr1 207775610 of CD46; GRCh38/ hg38: chr1 196675450 of CFH; GRCh38/ hg38: chr15 92998149 of CHD2; GRCh38/ hg38: chr16 28479765 of CLN3; GRCh38/ hg38: chr6 33183698 of COL11A2; GRCh38/ hg38: chr2 227296487 of COL4A3; GRCh38/ hg38: chr2 227144833 of COL4A4; GRCh38/ hg38: chr2 227015360 of COL4A4; GRCh38/ hg38: chr1 207637688 of CR1; GRCh38/ hg38: chr19 47835403 of CRX; GRCh38/ hg38: chr1 59904516 of CYP2J2; GRCh38/ hg38: chr1 26442335 of DHDDS; GRCh38/ hg38: chr1 28230252 of DNAJC8; GRCh38/ hg38: chr2 88582824 of EIF2AK3; GRCh38/ hg38: chr17 64102804 of ERN1; GRCh38/ hg38: chr1 23798484 of GALE; GRCh38/ hg38: chrX 109383446 of GUCY2F; GRCh38/ hg38: chrX 109439175 of GUCY2F; GRCh38/ hg38: chr15 72362466 of HEXA; GRCh38/ hg38: chr15 72345776 of HEXA; GRCh38/ hg38: chr16 30115645 of MAPK3; GRCh38/ hg38: chr2 148460219 ofMBD5; GRCh38/ hg38: chr2 148490695 ofMBD5; GRCh38/ hg38: chr2 148505761 of MBDS; GRCh38/ hg38: chr6 49436597 of MUT; GRCh38/ hg38: chr19 50230825 of MYH14; GRCh38/ hg38: chr6 75867431 of MYO6; GRCh38/ hg38: chr17 31249955 of NF1; GRCh38/ hg38: chr22 29628658 of NF2; GRCh38/ hg38: chr5 37048127 of NIPBL; GRCh38/ hg38: chr12 100499841 of NR1H4; GRCh38/ hg38: chr5 177169394 of NSD1; GRCh38/ hg38: chr5 177200761 of NSD 1; GRCh38/ hg38: chr5 177247924 of NSD1; GRCh38/ hg38: chr5 177275947 of NSD1; GRCh38/ hg38: chr3 193628509 of OPA1; GRCh38/ hg38: chr3 193603500 of OPA1; GRCh38/ hg38: chr13 100305751 of PCCA; GRCh38/ hg38: chr12 32894778 of PKP2; GRCh38/ hg38: chr22 46203575 ofPPARA; GRCh38/ hg38: chr1 150327557 of PRPF3; GRCh38/ hg38: chr1 150330401 of PRPF3; GRCh38/ hg38: chr2 165327155 of SCN2A; GRCh38/ hg38: chr12 51688758 of SCN8A; GRCh38/ hg38: chr12 51780202 of SCN8A; GRCh38/ hg38: chr2 166304329 of SCN9A; GRCh38/ hg38: chr7 80794957 of SEMA3C; GRCh38/ hg38: chr7 85059541 of SEMA3D; GRCh38/ hg38: chr11 226081 of SIRT3; GRCh38/ hg38: chr19 1216268 of STK11; GRCh38/ hg38: chr19 1221621 of STK11; GRCh38/ hg38: chr6 33448789 of SYNGAP1; GRCh38/ hg38: chr9 32551469 of TOPORS; or GRCh38/ hg38: chr5 83544965 of VCAN.

In some embodiments, the ASO targets a sequence about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream (or 3') from the 3' end of exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAR, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBDS, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN. In some embodiments, the ASO targets a sequence about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream (or 3') from GRCh38/ hg38: chrl 243564285 of AKT3; GRCh38/ hg38: chr19 13236449 of CACNA1A; GRCh38/ hg38: chr21 43059730 of CBS; GRCh38/ hg38: chr1 207775745 of CD46; GRCh38/ hg38: chr1 196675529 of CFH; GRCh38/ hg38: chr15 92998261 of CHD2; GRCh38/ hg38: chr16 28479644 of CLN3; GRCh38/ hg38: chr6 33183634 of COL11A2; GRCh38/ hg38: chr2 227296526 of COL4A3; GRCh38/ hg38: chr2 227144653 of COL4A4; GRCh38/ hg38: chr2 227015283 of COL4A4; GRCh38/ hg38: chr1 207637848 of CR1; GRCh38/ hg38: chr19 47835579 of CRX; GRCh38/ hg38: chr1 59904366 of CYP2J2; GRCh38/ hg38: chr1 26442372 of DHDDS; GRCh38/ hg38: chr1 28230131 of DNAJC8; GRCh38/ hg38: chr2 88582755 of EIF2AK3; GRCh38/ hg38: chr17 64102673 of ERN1; GRCh38/ hg38: chr1 23798311 of GALE; GRCh38/ hg38: chrX 109383365 of GUCY2F; GRCh38/ hg38: chrX 109439038 of GUCY2F; GRCh38/ hg38: chr15 72362376 of HEXA; GRCh38/ hg38: chr15 72345677 of HEXA; GRCh38/ hg38: chr16 30115595 of MAPK3; GRCh38/ hg38: chr2 148460304 of MBD5; GRCh38/ hg38: chr2 148490787 ofMBD5; GRCh38/ hg38: chr2 148505830 of MBD5; GRCh38/ hg38: chr6 49436522 of MUT; GRCh38/ hg38: chr19 50230999 of MYH14; GRCh38/ hg38: chr6 75867523 of MYO6; GRCh38/ hg38: chr17 31250125 of NF1; GRCh38/ hg38: chr22 29628773 of NF2; GRCh38/ hg38: chr5 37048354 of NIPBL; GRCh38/ hg38: chr12 100500024 of NR1H4; GRCh38/ hg38: chr5 177169559 of NSD1; GRCh38/ hg38: chr5 177200783 of NSD1; GRCh38/ hg38: chr5 177248079 of NSD1; GRCh38/ hg38: chr5 177276101 of NSD1; GRCh38/ hg38: chr3 193628616 of OPA1; GRCh38/ hg38: chr3 193603557 of OPA1; GRCh38/ hg38: chr13 100305834 of PCCA; GRCh38/ hg38: chr12 32894516 of PKP2; GRCh38/ hg38: chr22 46203752 ofPPARA; GRCh38/ hg38: chr1 150327652 of PRPF3; GRCh38/ hg38: chr1 150330498 of PRPF3; GRCh38/ hg38: chr2 165327202 of SCN2A; GRCh38/ hg38: chr12 51688849 of SCN8A; GRCh38/ hg38: chr12 51780271 of SCN8A; GRCh38/ hg38: chr2 166304238 of SCN9A; GRCh38/ hg38: chr7 80794854 of SEMA3C; GRCh38/ hg38: chr7 85059498 of SEMA3D; GRCh38/ hg38: chr11 225673 of SIRT3; GRCh38/ hg38: chr19 1216398 of STK11; GRCh38/ hg38: chr19 1221846 of STK11; GRCh38/ hg38: chr6 33448868 of SYNGAP1; GRCh38/ hg38: chr9 32551365 of TOPORS; or GRCh38/ hg38: chr5 83545070 of VCAN.

In some embodiments, the ASO targets a sequence at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream (or 3') from the 3' end of exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 1 1x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN. In some embodiments, the ASO targets a sequence at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream (or 3') from GRCh38/ hg38: chr1 243564285 of AKT3; GRCh38/ hg38: chr19 13236449 of CACNA1A; GRCh38/ hg38: chr21 43059730 of CBS; GRCh38/ hg38: chr1 207775745 of CD46; GRCh38/ hg38: chr1 196675529 of CFH; GRCh38/ hg38: chr15 92998261 of CHD2; GRCh38/ hg38: chr16 28479644 of CLN3; GRCh38/ hg38: chr6 33183634 of COL11A2; GRCh38/ hg38: chr2 227296526 of COL4A3; GRCh38/ hg38: chr2 227144653 of COL4A4; GRCh38/ hg38: chr2 227015283 of COL4A4; GRCh38/ hg38: chr1 207637848 of CR1; GRCh38/ hg38: chr19 47835579 of CRX; GRCh38/ hg38: chr1 59904366 of CYP2J2; GRCh38/ hg38: chr1 26442372 of DHDDS; GRCh38/ hg38: chr1 28230131 of DNAJC8; GRCh38/ hg38: chr2 88582755 of EIF2AK3; GRCh38/ hg38: chr17 64102673 of ERN1; GRCh38/ hg38: chr1 23798311 of GALE; GRCh38/ hg38: chrX 109383365 of GUCY2F; GRCh38/ hg38: chrX 109439038 of GUCY2F; GRCh38/ hg38: chr15 72362376 of HEXA; GRCh38/ hg38: chr15 72345677 of HEXA; GRCh38/ hg38: chr16 30115595 of MAPK3; GRCh38/ hg38: chr2 148460304 of MBD5; GRCh38/ hg38: chr2 148490787 of MBD5; GRCh38/ hg38: chr2 148505830 ofMBD5; GRCh38/ hg38: chr6 49436522 of MUT; GRCh38/ hg38: chr19 50230999 of MYH14; GRCh38/ hg38: chr6 75867523 of MYO6; GRCh38/ hg38: chr17 31250125 of NF1; GRCh38/ hg38: chr22 29628773 of NF2; GRCh38/ hg38: chr5 37048354 of NIPBL; GRCh38/ hg38: chr12 100500024 of NR1H4; GRCh38/ hg38: chr5 177169559 of NSD1; GRCh38/ hg38: chr5 177200783 of NSD1; GRCh38/ hg38: chr5 177248079 of NSD1; GRCh38/ hg38: chr5 177276101 of NSD1; GRCh38/ hg38: chr3 193628616 of OPA1; GRCh38/ hg38: chr3 193603557 of OPA1; GRCh38/ hg38: chr13 100305834 of PCCA; GRCh38/ hg38: chr12 32894516 of PKP2; GRCh38/ hg38: chr22 46203752 ofPPARA; GRCh38/ hg38: chr1 150327652 of PRPF3; GRCh38/ hg38: chr1 150330498 of PRPF3; GRCh38/ hg38: chr2 165327202 of SCN2A; GRCh38/ hg38: chr12 51688849 of SCN8A; GRCh38/ hg38: chr12 51780271 of SCN8A; GRCh38/ hg38: chr2 166304238 of SCN9A; GRCh38/ hg38: chr7 80794854 of SEMA3C; GRCh38/ hg38: chr7 85059498 of SEMA3D; GRCh38/ hg38: chr11 225673 of SIRT3; GRCh38/ hg38: chr19 1216398 of STK11; GRCh38/ hg38: chr19 1221846 of STK11; GRCh38/ hg38: chr6 33448868 of SYNGAP1; GRCh38/ hg38: chr9 32551365 of TOPORS; or GRCh38/ hg38: chr5 83545070 of VCAN.

In some embodiments, the ASO has a sequence complementary to the targeted portion of the NMD exon mRNA according to any one of SEQ ID NOs: 60-191.

In some embodiments, the ASO targets a sequence upstream from the 5' end of an NIE. For example, ASOs targeting a sequence upstream from the 5' end of an NIE (e.g. exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN) comprises a sequence that is at least about 80%, 85%, 90%, 95%, 97%, or 100% complimentary to at least 8 contiguous nucleic acids of any one of SEQ ID NOs: 60-134. For example, ASOs targeting a sequence upstream from the 5' end of an NIE (e.g., exon (GRCh38/ hg38: chr1 243564285 243564388) of AKT3; exon (GRCh38/ hg38: chr19 13236449 13236618) of CACNA1A; exon (GRCh38/ hg38: chr21 43059730 43060012) of CBS; exon (GRCh38/ hg38: chr1 207775610 207775745) of CD46; exon (GRCh38/ hg38: chr1 196675450 196675529) of CFH; exon (GRCh38/ hg38: chr15 92998149 92998261) of CHD2; exon (GRCh38/ hg38: chr16 28479644 28479765) of CLN3; exon (GRCh38/ hg38: chr6 33183634 33183698) of COL11A2; exon (GRCh38/ hg38: chr2 227296487 227296526) of COL4A3; exon (GRCh38/ hg38: chr2 227144653 227144833) of COL4A4; exon (GRCh38/ hg38: chr2 227015283 227015360) of COL4A4; exon (GRCh38/ hg38: chr1 207637688 207637848) of CR1; exon (GRCh38/ hg38: chr19 47835403 47835579) of CRX; exon (GRCh38/ hg38: chr1 59904366 59904516) of CYP2J2; exon (GRCh38/ hg38: chr1 26442335 26442372) of DHDDS; exon (GRCh38/ hg38: chr1 28230131 28230252 ) of DNAJC8; exon (GRCh38/ hg38: chr2 88582755 88582824) of EIF2AK3; exon (GRCh38/ hg38: chr17 64102673 64102804) of ERN1; exon (GRCh38/ hg38: chr1 23798311 23798484) of GALE; exon (GRCh38/ hg38: chrX 109383365 109383446) of GUCY2F; exon (GRCh38/ hg38: chrX 109439038 109439175) of GUCY2F; exon (GRCh38/ hg38: chr15 72362376 72362466) of HEXA; exon (GRCh38/ hg38: chr15 72345677 72345776) of HEXA; exon (GRCh38/ hg38: chr16 30115595 30115645) of MAPK3; exon (GRCh38/ hg38: chr2 148460219 148460304) of MBD5; exon (GRCh38/ hg38: chr2 148490695 148490787) of MBD5; exon (GRCh38/ hg38: chr2 148505761 148505830) of MBD5; exon (GRCh38/ hg38: chr6 49436522 49436597) of MUT; exon (GRCh38/ hg38: chr19 50230825 50230999) of MYH14; exon (GRCh38/ hg38: chr6 75867431 75867523) of MYO6; exon (GRCh38/ hg38: chr17 31249955 31250125) of NF1; exon (GRCh38/ hg38: chr22 29628658 29628773) of NF2; exon (GRCh38/ hg38: chr5 37048127 37048354) of NIPBL; exon (GRCh38/ hg38: chr12 100499841 100500024) of NR1H4; exon (GRCh38/ hg38: chr5 177169394 177169559) of NSD1; exon (GRCh38/ hg38: chr5 177200761 177200783) of NSD1; exon (GRCh38/ hg38: chr5 177247924 177248079) of NSD1; exon (GRCh38/ hg38: chr5 177275947 177276101) of NSD1; exon (GRCh38/ hg38: chr3 193628509 193628616) of OPA1; exon (GRCh38/ hg38: chr3 193603500 193603557) of OPA1; exon (GRCh38/ hg38: chr13 100305751 100305834) of PCCA; exon (GRCh38/ hg38: chr12 32894516 32894778) of PKP2; exon (GRCh38/ hg38: chr22 46203575 46203752) of PPARA; exon (GRCh38/ hg38: chr1 150327557 150327652) of PRPF3; exon (GRCh38/ hg38: chr1 150330401 150330498) of PRPF3; exon (GRCh38/ hg38: chr2 165327155 165327202) of SCN2A; exon (GRCh38/ hg38: chr12 51688758 51688849) of SCN8A; exon (GRCh38/ hg38: chr12 51780202 51780271) of SCN8A; exon (GRCh38/ hg38: chr2 166304238 166304329) of SCN9A; exon (GRCh38/ hg38: chr7 80794854 80794957) of SEMA3C; exon (GRCh38/ hg38: chr7 85059498 85059541) of SEMA3D; exon (GRCh38/ hg38: chr11 225673 226081) of SIRT3; exon (GRCh38/ hg38: chr19 1216268 1216398) of STK11; exon (GRCh38/ hg38: chr19 1221621 1221846) of STK11; exon (GRCh38/ hg38: chr6 33448789 33448868) of SYNGAP1; exon (GRCh38/ hg38: chr9 32551365 32551469) of TOPORS; exon (GRCh38/ hg38: chr5 83544965 83545070) of VCAN) can comprise a sequence with at least 80%, 85%, 90%, 95%, 97%, or 100% sequence identity to any one of SEQ ID NOs: 135-191.

In some embodiments, the ASOs target a sequence containing an exon-intron boundary (or junction). For example, ASOs targeting a sequence containing an exon-intron boundary can comprise a sequence that is at least about 80%, 85%, 90%, 95%, 97%, or 100% complimentary to at least 8 contiguous nucleic acids of any one of SEQ ID NOs: 60-191. In some embodiments, the ASOs target a sequence downstream from the 3' end of an NIE. For example, ASOs targeting a sequence downstream from the 3' end of an NIE (e.g. exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN) can comprise a sequence with at least 80%, 85%, 90%, 95%, 97%, or 100% sequence identity to any one of SEQ ID NOs: 60-134. For example, ASOs targeting a sequence downstream from the 3' end of an NIE (e.g., exon (GRCh38/ hg38: chr1 243564285 243564388) of AKT3; exon (GRCh38/ hg38: chr19 13236449 13236618) of CACNA1A; exon (GRCh38/ hg38: chr21 43059730 43060012) of CBS; exon (GRCh38/ hg38: chr1 207775610 207775745) of CD46; exon (GRCh38/ hg38: chr1 196675450 196675529) of CFH; exon (GRCh38/ hg38: chr15 92998149 92998261) of CHD2; exon (GRCh38/ hg38: chr16 28479644 28479765) of CLN3; exon (GRCh38/ hg38: chr6 33183634 33183698) of COL11A2; exon (GRCh38/ hg38: chr2 227296487 227296526) of COL4A3; exon (GRCh38/ hg38: chr2 227144653 227144833) of COL4A4; exon (GRCh38/ hg38: chr2 227015283 227015360) of COL4A4; exon (GRCh38/ hg38: chr1 207637688 207637848) of CR1; exon (GRCh38/ hg38: chr19 47835403 47835579) of CRX; exon (GRCh38/ hg38: chr1 59904366 59904516) of CYP2J2; exon (GRCh38/ hg38: chr1 26442335 26442372) of DHDDS; exon (GRCh38/ hg38: chr1 28230131 28230252 ) of DNAJC8; exon (GRCh38/ hg38: chr2 88582755 88582824) of EIF2AK3; exon (GRCh38/ hg38: chr17 64102673 64102804) of ERN1; exon (GRCh38/ hg38: chr1 23798311 23798484) of GALE; exon (GRCh38/ hg38: chrX 109383365 109383446) of GUCY2F; exon (GRCh38/ hg38: chrX 109439038 109439175) of GUCY2F; exon (GRCh38/ hg38: chr15 72362376 72362466) of HEXA; exon (GRCh38/ hg38: chr15 72345677 72345776) of HEXA; exon (GRCh38/ hg38: chr16 30115595 30115645) of MAPK3; exon (GRCh38/ hg38: chr2 148460219 148460304) of MBD5; exon (GRCh38/ hg38: chr2 148490695 148490787) of MBD5; exon (GRCh38/ hg38: chr2 148505761 148505830) of MBD5; exon (GRCh38/ hg38: chr6 49436522 49436597) of MUT; exon (GRCh38/ hg38: chr19 50230825 50230999) of MYH14; exon (GRCh38/ hg38: chr6 75867431 75867523) of MYO6; exon (GRCh38/ hg38: chr17 31249955 31250125) of NF1; exon (GRCh38/ hg38: chr22 29628658 29628773) of NF2; exon (GRCh38/ hg38: chr5 37048127 37048354) of NIPBL; exon (GRCh38/ hg38: chr12 100499841 100500024) of NR1H4; exon (GRCh38/ hg38: chr5 177169394 177169559) of NSD1; exon (GRCh38/ hg38: chr5 177200761 177200783) of NSD1; exon (GRCh38/ hg38: chr5 177247924 177248079) of NSD1; exon (GRCh38/ hg38: chr5 177275947 177276101) of NSD1; exon (GRCh38/ hg38: chr3 193628509 193628616) of OPA1; exon (GRCh38/ hg38: chr3 193603500 193603557) of OPA1; exon (GRCh38/ hg38: chr13 100305751 100305834) of PCCA; exon (GRCh38/ hg38: chr12 32894516 32894778) of PKP2; exon (GRCh38/ hg38: chr22 46203575 46203752) of PPARA; exon (GRCh38/ hg38: chr1 150327557 150327652) of PRPF3; exon (GRCh38/ hg38: chr1 150330401 150330498) of PRPF3; exon (GRCh38/ hg38: chr2 165327155 165327202) of SCN2A; exon (GRCh38/ hg38: chr12 51688758 51688849) of SCN8A; exon (GRCh38/ hg38: chr12 51780202 51780271) of SCN8A; exon (GRCh38/ hg38: chr2 166304238 166304329) of SCN9A; exon (GRCh38/ hg38: chr7 80794854 80794957) of SEMA3C; exon (GRCh38/ hg38: chr7 85059498 85059541) of SEMA3D; exon (GRCh38/ hg38: chr11 225673 226081) of SIRT3; exon (GRCh38/ hg38: chr19 1216268 1216398) of STK11; exon (GRCh38/ hg38: chr19 1221621 1221846) of STK11; exon (GRCh38/ hg38: chr6 33448789 33448868) of SYNGAP1; exon (GRCh38/ hg38: chr9 32551365 32551469) of TOPORS; exon (GRCh38/ hg38: chr5 83544965 83545070) of VCAN) can comprise a sequence with at least 80%, 85%, 90%, 95%, 97%, or 100% sequence identity to any one of SEQ ID NOs: 135-191. In some embodiments, ASOs target a sequence within an NIE.

In some embodiments, the ASO targets exon 8x of a ABCB4 NIE containing pre-mRNA comprising NIE exon 8, exon 9x of a ASS1 NIE containing pre-mRNA comprising NIE exon 9, exon 16x of a ATP8B1 NIE containing pre-mRNA comprising NIE exon 16, exon 1x of a BAG3 NIE containing pre-mRNA comprising NIE exon 1, exon 31x of a CACNA1A NIE containing pre-mRNA comprising NIE exon 31, exon 36x of a CACNA1A NIE containing pre-mRNA comprising NIE exon 36, exon 37x of a CACNA1A NIE containing pre-mRNA comprising NIE exon 37, exon 3x of a CBS NIE containing pre-mRNA comprising NIE exon 3, exon 12x of a CBS NIE containing pre-mRNA comprising NIE exon 12, exon 1x of a CD55 NIE containing pre-mRNA comprising NIE exon 1, exon 16x of a CDKL5 NIE containing pre-mRNA comprising NIE exon 16, exon 3x of CFH NIE containing pre-mRNA comprising NIE exon 3, exon 30x of a CHD2 NIE containing pre-mRNA comprising NIE exon 30, exon 4x of CHRNA7 NIE containing pre-mRNA comprising NIE exon 4, exon 1x of CISD2 NIE containing pre-mRNA comprising NIE exon 1, exon 15x of CLN3 NIE containing pre-mRNA comprising NIE exon 15, exon 11x of a COL4A3 NIE containing pre-mRNA comprising NIE exon 11, exon 41x of a COL4A3 NIE containing pre-mRNA comprising NIE exon 41, exon 22x of a COL4A4 NIE containing pre-mRNA comprising NIE exon 22, exon 44x of a COL4A4 NIE containing pre-mRNA comprising NIE exon 44, exon 20x of DEPDC5 NIE containing pre-mRNA comprising NIE exon 20, exon 2x of a DHDDS NIE containing pre-mRNA comprising NIE exon 2, exon 3x of a ELOVL4 NIE containing pre-mRNA comprising NIE exon 3, exon 5x of a FAH NIE containing pre-mRNA comprising NIE exon 5, exon 4x of FXN NIE containing pre-mRNA comprising NIE exon 4, exon 4x of a GALE NIE containing pre-mRNA comprising NIE exon 4, exon 3x of a GBE1 NIE containing pre-mRNA comprising NIE exon 3, exon 11x of GRIN2A NIE containing pre-mRNA comprising NIE exon 11, exon 1x of GRN NIE containing pre-mRNA comprising NIE exon 1, exon 2x of a HEXA NIE containing pre-mRNA comprising NIE exon 2, exon 2x of a KANSL1 NIE containing pre-mRNA comprising NIE exon 2, exon 1x of a KCNQ2 NIE containing pre-mRNA comprising NIE exon 1, exon 50x of a KMT2D NIE containing pre-mRNA comprising NIE exon 50, exon 8x of MAPK3 NIE containing pre-mRNA comprising NIE exon 8, exon 13x of MBD5 NIE containing pre-mRNA comprising NIE exon 13, exon 2x of a MECP2 NIE containing pre-mRNA comprising NIE exon 2, exon 11x of MUT NIE containing pre-mRNA comprising NIE exon 11, exon 31x of NF1 NIE containing pre-mRNA comprising NIE exon 31, exon 7x of a NIPBL NIE containing pre-mRNA comprising NIE exon 7, exon 38x of a NIPBL NIE containing pre-mRNA comprising NIE exon 38, exon 11x of a NSD1 NIE containing pre-mRNA comprising NIE exon 11, exon 6x of a OPA1 NIE containing pre-mRNA comprising NIE exon 6, exon 28x of a OPA1 NIE containing pre-mRNA comprising NIE exon 28, exon 1x of OPTN NIE containing pre-mRNA comprising NIE exon 1, exon 1x of PCCA NIE containing pre-mRNA comprising NIE exon 1, exon 5x of a PCCB NIE containing pre-mRNA comprising NIE exon 5, exon 6x of a PCCB NIE containing pre-mRNA comprising NIE exon 6, exon 4x of a PKP2 NIE containing pre-mRNA comprising NIE exon 4, exon 23x of a PLCB1 NIE containing pre-mRNA comprising NIE exon 23, exon 3x of PRPF3 NIE containing pre-mRNA comprising NIE exon 3, exon 9x of PRPF31 NIE containing pre-mRNA comprising NIE exon 9, exon 1x of a RAI1 NIE containing pre-mRNA comprising NIE exon 1, exon 5x of RBFOX2 NIE containing pre-mRNA comprising NIE exon 5, exon 13x of SCN2A NIE containing pre-mRNA comprising NIE exon 13, exon 6x of SCN3A NIE containing pre-mRNA comprising NIE exon 6, exon 7x of SCN3A NIE containing pre-mRNA comprising NIE exon 7, exon 4x of SCN8A NIE containing pre-mRNA comprising NIE exon 4, exon 6x of SCN8A NIE containing pre-mRNA comprising NIE exon 6, exon 20x of SCN8A NIE containing pre-mRNA comprising NIE exon 20, exon 6x of SCN9A NIE containing pre-mRNA comprising NIE exon 6, exon 24x of SHANK3 NIE containing pre-mRNA comprising NIE exon 24, exon 3x of a SLC25A13 NIE containing pre-mRNA comprising NIE exon 3, exon 6x of a SLC25A13 NIE containing pre-mRNA comprising NIE exon 6, exon 9x of a SLC25A13 NIE containing pre-mRNA comprising NIE exon 9, exon 11x of a SLC25A13 NIE containing pre-mRNA comprising NIE exon 11, exon 13x of a SLC25A13 NIE containing pre-mRNA comprising NIE exon 13, exon 1x of SLC6A1 NIE containing pre-mRNA comprising NIE exon 1, exon 12x of a SPTAN1 NIE containing pre-mRNA comprising NIE exon 12, exon 10x of a TEK NIE containing pre-mRNA comprising NIE exon 10, exon 15x of a TEK NIE containing pre-mRNA comprising NIE exon 15, exon 1x of TOPORS NIE containing pre-mRNA comprising NIE exon 1, exon 11x of a TSC2 NIE containing pre-mRNA comprising NIE exon 11, exon 30x of a TSC2 NIE containing pre-mRNA comprising NIE exon 30, exon 1x of UBE3A NIE containing pre-mRNA comprising NIE exon 1, or exon 7x of a VCAN NIE containing pre-mRNA comprising NIE exon 7. In some embodiments, the ASO targets a sequence downstream (or 3') from the 5' end of the exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN pre-mRNA. In some embodiments, the ASO targets an exon 20x sequence upstream (or 5') from the 3' end of the exon 8x of ABCB4, exon 9x of ASS1, exon 16x of ATP8B 1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN pre-mRNA.

In some embodiments, the targeted portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA is in intron 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33,34, 35, 36, 37, 38, 39, 40, 41, 42, 43, 44, 45, 46, 47, 48, 49, or 50. In some embodiments, hybridization of an ASO to the targeted portion of the NIE pre-mRNA results in exon skipping of at least one of NIE within intron 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33,34, 35, 36, 37, 38, 39, 40, 41, 42, 43, 44, 45, 46, 47, 48, 49, or 50, and subsequently increases ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP 1 protein production. In some embodiments, the targeted portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, or VCAN* NIE containing pre-mRNA is in intron 8 of ABCB4, intron 9 of ASS1, intron 16 of ATP8B1, intron 1 of BAG3, intron 31 of CACNA1A, intron 36 of CACNA1A, intron 37 of CACNA1A, intron 3 of CBS, intron 12 of CBS, intron 1 of CD55, intron 16 of CDKL5, intron 3 of CFH, intron 30 of CHD2, intron 4 of CHRNA7, intron 1 of CISD2, intron 15 of CLN3, intron 11 of COL4A3, intron 41 of COL4A3, intron 22 of COL4A4, intron 44 of COL4A4, intron 20 of DEPDC5, intron 2 of DHDDS, intron 3 of ELOVL4, intron 5 of FAH, intron 4 of FN, intron 4 of GALE, intron 3 of GBE1, intron 11 of GRIN2A, intron 1 of GRN, intron 2 of HEA, intron 2 of KANSL1, intron 50 of KMT2D, intron 8 of MAPK3, intron 13 of MBD5, intron 2 of MECP2, intron 11 of MUT, intron 31 of NF1, intron 7 of NIPBL, intron 38 of NIPBL, intron 11 of NSD1, intron 6 of OPA1, intron 28 of OPA1, intron 1 of OPTN, intron 1 of PCCA, intron 5 of PCCB, intron 6 of PCCB, intron 4 of PKP2, intron 23 of PLCB1, intron 3 of PRPF3, intron 9 of PRPF31, intron 1 of RAI1, intron 5 of RBFO2, intron 13 of SCN2A, intron 6 of SCN3A, intron 7 of SCN3A, intron 4 of SCN8A, intron 6 of SCN8A, intron 20 of SCN8A, intron 6 of SCN9A, intron 24 of SHANK3, intron 3 of SLC25A13, intron 6 of SLC25A13, intron 9 of SLC25A13, intron 11 of SLC25A13, intron 13 of SLC25A13, intron 1 of SLC6A1, intron 12 of SPTAN1, intron 10 of TEK, intron 15 of TEK, intron 1 of TOPORS, intron 11 of TSC2, intron 30 of TSC2, intron 1 of UBE3A, or intron 7 of VCAN. In some embodiments, the targeted portion of the *AKT3, CACNA1A, CBS, CD46, CFH, CHD2, CLN3, COL11A2, COL4A3, COL4A4, COL4A4, CR1, CRX, CYP2J2, DHDDS, DNAJC8, EIF2AK3, ERN1, GALE, GUCY2F, GUCY2F, HEXA, HEXA, MAPK3, MBD5, MBD5, MBD5, MUT, MYH14, MYO6, NF1, NF2, NIPBL, NR1H4, NSD1, NSD1, NSD1, NSD1, OPA1, OPA1, PCCA, PKP2, PPARA, PRPF3, PRPF3, SCN2A, SCN8A, SCN8A, SCN9A, SEMA3C, SEMA3D, SIRT3, STK11, STK11, SYNGAP1, TOPORS,* or *VCAN* NIE containing pre-mRNA is intron (GRCh38/ hg38: chr1 243563849 243572925) of AKT3; intron (GRCh38/ hg38: chr19 13235731 13241520) of CACNA1A; intron (GRCh38/ hg38: chr21 43059304 43060440) of CBS; intron (GRCh38/ hg38: chr1 207770363 207783291) of CD46; intron (GRCh38/ hg38: chr1 196673963 196675988) of CFH; intron (GRCh38/ hg38: chr15 92997404 92998498) of CHD2; intron (GRCh38/ hg38: chr16 28477878 28482104) of CLN3; intron (GRCh38/ hg38: chr6 33181172 33184144) of COL11A2; intron (GRCh38/ hg38: chr2 227295317 227297673) of COL4A3; intron (GRCh38/ hg38: chr2 227144559 227147412) of COL4A4; intron (GRCh38/ hg38: chr2 227012299 227022047) of COL4A4; intron (GRCh38/ hg38: chr1 207630622 207639396) of CR1; intron (GRCh38/ hg38: chr19 47834544 47836242) of CRX; intron (GRCh38/ hg38: chr1 59901104 59904870) of CYP2J2; intron (GRCh38/ hg38: chr1 26438285 26442730) of DHDDS; intron (GRCh38/ hg38: chr1 28229025 28232920) of DNAJC8; intron (GRCh38/ hg38: chr2 88579641 88583429) of EIF2AK3; intron (GRCh38/ hg38: chr17 64098242 64129975) of ERN1; intron (GRCh38/ hg38: chr1 23798231 23798614) of GALE; intron (GRCh38/ hg38: chrx 109382213 109385183) of GUCY2F; intron (GRCh38/ hg38: chrx 109430397 109441350) of GUCY2F; intron (GRCh38/ hg38: chr15 72356651 72375719) of HEXA; intron (GRCh38/ hg38: chr15 72345552 72346234) of HEXA; intron (GRCh38/ hg38: chr16 30114709 30116635) of MAPK3; intron (GRCh38/ hg38: chr2 148458872 148462581) of MBD5; intron (GRCh38/ hg38: chr2 148490595 148502435) of MBD5; intron (GRCh38/ hg38: chr2 148502510 148510059) ofMBD5; intron (GRCh38/ hg38: chr6 49435625 49440205) of MUT; intron (GRCh38/ hg38: chr19 50230624 50231929) of MYH14; intron (GRCh38/ hg38: chr6 75867106 75870646) of MYO6; intron (GRCh38/ hg38: chr17 31249120 31252937) of NF1; intron (GRCh38/ hg38: chr22 29604113 29636750) of NF2; intron (GRCh38/ hg38: chr5 37046200 37048501) of NIPBL; intron (GRCh38/ hg38: chr12 100493403 100505574) of NR1H4; intron (GRCh38/ hg38: chr5 177136031 177191883) of NSD1; intron (GRCh38/ hg38: chr5 177192020 177204119) of NSD1; intron (GRCh38/ hg38: chr5 177246797 177248180) of NSD1; intron (GRCh38/ hg38: chr5 177273785 177280564) of NSD1; intron (GRCh38/ hg38: chr3 193626203 193631611) of OPA1; intron (GRCh38/ hg38: chr3 193593374 193614710) of OPA1; intron (GRCh38/ hg38: chr13 100302999 100307191) of PCCA; intron (GRCh38/ hg38: chr12 32879033 32896508) of PKP2; intron (GRCh38/ hg38: chr22 46198592 46215172) of PPARA; intron (GRCh38/ hg38: chr1 150325882 150328319) of PRPF3; intron (GRCh38/ hg38: chr1 150328467 150332683) of PRPF3; intron (GRCh38/ hg38: chr2 165326985 165331329) of SCN2A; intron (GRCh38/ hg38: chr12 51687220 51689004) of SCN8A; intron (GRCh38/ hg38: chr12 51774363 51786541) of SCN8A; intron (GRCh38/ hg38: chr2 166304122 166305791) of SCN9A; intron (GRCh38/ hg38: chr7 80789529 80798091) of SEMA3C; intron (GRCh38/ hg38: chr7 85055860 85065423) of SEMA3D; intron (GRCh38/ hg38: chr11 224241 230451) of SIRT3; intron (GRCh38/ hg38: chr19 1207204 1218416) of STK11; intron (GRCh38/ hg38: chr19 1221341 1221948) of STK11; intron (GRCh38/ hg38: chr6 33447934 33451759) of SYNGAP1; intron (GRCh38/ hg38: chr9 32550969 32552433) of TOPORS; or intron (GRCh38/ hg38: chr5 83542269 83545536) of VCAN.

In some embodiments, the methods and compositions of the present disclosure are used to increase the expression of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 by inducing exon skipping of a pseudo-exon of an *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA,* KANSL1, *KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA. In some embodiments, the pseudo-exon is a sequence within any of introns 1-50. In some embodiments, the pseudo-exon is a sequence within any of introns 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33,34, 35, 36, 37, 38, 39, 40, 41, 42, 43, 44, 45, 46, 47, 48, 49, or 50. In some embodiments, the pseudo-exon can be any *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1*, *KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* intron or a portion thereof. In some embodiments, the pseudo-exon is within intron 8 of ABCB4, intron 9 of ASS1, intron 16 of ATP8B1, intron 1 of BAG3, intron 31 of CACNA1A, intron 36 of CACNA1A, intron 37 of CACNA1A, intron 3 of CBS, intron 12 of CBS, intron 1 of CD55, intron 16 of CDKL5, intron 3 of CFH, intron 30 of CHD2, intron 4 of CHRNA7, intron 1 of CISD2, intron 15 of CLN3, intron 11 of COL4A3, intron 41 of COL4A3, intron 22 of COL4A4, intron 44 of COL4A4, intron 20 of DEPDC5, intron 2 of DHDDS, intron 3 of ELOVL4, intron 5 of FAH, intron 4 of FN, intron 4 of GALE, intron 3 of GBE1, intron 11 of GRIN2A, intron 1 of GRN, intron 2 of HEA, intron 2 of KANSL1, intron 50 of KMT2D, intron 8 of MAPK3, intron 13 of MBD5, intron 2 of MECP2, intron 11 of MUT, intron 31 of NF1, intron 7 of NIPBL, intron 38 of NIPBL, intron 11 of NSD1, intron 6 of OPA1, intron 28 of OPA1, intron 1 of OPTN, intron 1 of PCCA, intron 5 of PCCB, intron 6 of PCCB, intron 4 of PKP2, intron 23 of PLCB1, intron 3 of PRPF3, intron 9 of PRPF31, intron 1 of RAI1, intron 5 of RBFO2, intron 13 of SCN2A, intron 6 of SCN3A, intron 7 of SCN3A, intron 4 of SCN8A, intron 6 of SCN8A, intron 20 of SCN8A, intron 6 of SCN9A, intron 24 of SHANK3, intron 3 of SLC25A13, intron 6 of SLC25A13, intron 9 of SLC25A13, intron 11 of SLC25A13, intron 13 of SLC25A13, intron 1 of SLC6A1, intron 12 of SPTAN1, intron 10 of TEK, intron 15 of TEK, intron 1 of TOPORS, intron 11 of TSC2, intron 30 of TSC2, intron 1 of UBE3A, or intron 7 of VCAN. In some embodiments, the pseudo-exon is within intron (GRCh38/ hg38: chr1 243563849 243572925) of AKT3; intron (GRCh38/ hg38: chr19 13235731 13241520) of CACNA1A; intron (GRCh38/ hg38: chr21 43059304 43060440) of CBS; intron (GRCh38/ hg38: chr1 207770363 207783291) of CD46; intron (GRCh38/ hg38: chr1 196673963 196675988) of CFH; intron (GRCh38/ hg38: chr15 92997404 92998498) of CHD2; intron (GRCh38/ hg38: chr16 28477878 28482104) of CLN3; intron (GRCh38/ hg38: chr6 33181172 33184144) of COL11A2; intron (GRCh38/ hg38: chr2 227295317 227297673) of COL4A3; intron (GRCh38/ hg38: chr2 227144559 227147412) of COL4A4; intron (GRCh38/ hg38: chr2 227012299 227022047) of COL4A4; intron (GRCh38/ hg38: chr1 207630622 207639396) of CR1; intron (GRCh38/ hg38: chr19 47834544 47836242) of CRX; intron (GRCh38/ hg38: chr1 59901104 59904870) of CYP2J2; intron (GRCh38/ hg38: chr1 26438285 26442730) of DHDDS; intron (GRCh38/ hg38: chr1 28229025 28232920) of DNAJC8; intron (GRCh38/ hg38: chr2 88579641 88583429) of EIF2AK3; intron (GRCh38/ hg38: chr17 64098242 64129975) of ERN1; intron (GRCh38/ hg38: chr1 23798231 23798614) of GALE; intron (GRCh38/ hg38: chrx 109382213 109385183) of GUCY2F; intron (GRCh38/ hg38: chrx 109430397 109441350) of GUCY2F; intron (GRCh38/ hg38: chr15 72356651 72375719) of HEXA; intron (GRCh38/ hg38: chr15 72345552 72346234) of HEXA; intron (GRCh38/ hg38: chr16 30114709 30116635) of MAPK3; intron (GRCh38/ hg38: chr2 148458872 148462581) of MBD5; intron (GRCh38/ hg38: chr2 148490595 148502435) of MBD5; intron (GRCh38/ hg38: chr2 148502510 148510059) of MBD5; intron (GRCh38/ hg38: chr6 49435625 49440205) of MUT; intron (GRCh38/ hg38: chr19 50230624 50231929) of MYH14; intron (GRCh38/ hg38: chr6 75867106 75870646) of MYO6; intron (GRCh38/ hg38: chr17 31249120 31252937) of NF1; intron (GRCh38/ hg38: chr22 29604113 29636750) of NF2; intron (GRCh38/ hg38: chr5 37046200 37048501) of NIPBL; intron (GRCh38/ hg38: chr12 100493403 100505574) of NR1H4; intron (GRCh38/ hg38: chr5 177136031 177191883) of NSD1; intron (GRCh38/ hg38: chr5 177192020 177204119) of NSD1; intron (GRCh38/ hg38: chr5 177246797 177248180) of NSD1; intron (GRCh38/ hg38: chr5 177273785 177280564) of NSD1; intron (GRCh38/ hg38: chr3 193626203 193631611) of OPA1; intron (GRCh38/ hg38: chr3 193593374 193614710) of OPA1; intron (GRCh38/ hg38: chr13 100302999 100307191) of PCCA; intron (GRCh38/ hg38: chr12 32879033 32896508) of PKP2; intron (GRCh38/ hg38: chr22 46198592 46215172) of PPARA; intron (GRCh38/ hg38: chr1 150325882 150328319) of PRPF3; intron (GRCh38/ hg38: chr1 150328467 150332683) of PRPF3; intron (GRCh38/ hg38: chr2 165326985 165331329) of SCN2A; intron (GRCh38/ hg38: chr12 51687220 51689004) of SCN8A; intron (GRCh38/ hg38: chr12 51774363 51786541) of SCN8A; intron (GRCh38/ hg38: chr2 166304122 166305791) of SCN9A; intron (GRCh38/ hg38: chr7 80789529 80798091) of SEMA3C; intron (GRCh38/ hg38: chr7 85055860 85065423) of SEMA3D; intron (GRCh38/ hg38: chr11 224241 230451) of SIRT3; intron (GRCh38/ hg38: chr19 1207204 1218416) of STK11; intron (GRCh38/ hg38: chr19 1221341 1221948) of STK11; intron (GRCh38/ hg38: chr6 33447934 33451759) of SYNGAP1; intron (GRCh38/ hg38: chr9 32550969 32552433) of TOPORS; or intron (GRCh38/ hg38: chr5 83542269 83545536) of VCAN.

### Protein Expression

In some embodiments, the methods described herein are used to increase the production of a functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein or RNA. As used herein, the term "functional" refers to the amount of activity or function of a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein or RNA that is necessary to eliminate any one or more symptoms of a treated condition or disease, e.g., Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; 16p11.2 deletion syndrome; Deafness, autosomal dominant 13; Cone-rod retinal dystrophy-2; Deafness, autosomal dominant 4A; Peripheral neuropathy, myopathy, hoarseness, and hearing loss; Deafness, autosomal dominant 22; Neurofibromatosis type 2; NASH; or Mental retardation, autosomal dominant 5. In some embodiments, the methods are used to increase the production of a partially functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein or RNA. As used herein, the term "partially functional" refers to any amount of activity or function of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein or RNA that is less than the amount of activity or function that is necessary to eliminate or prevent any one or more symptoms of a disease or condition. In some embodiments, a partially functional protein or RNA will have at least 10%, at least 20%, at least 30%, at least 40%, at least 50%, at least 60%, at least 70%, at least 75%, at least 80%, at least 85%, at least 90%, or at least 95% less activity relative to the fully functional protein or RNA.

In some embodiments, the method is a method of increasing the expression of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP 1 protein by cells of a subject having a NIE containing pre-mRNA encoding the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, wherein the subject has Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; 16p11.2 deletion syndrome; Deafness, autosomal dominant 13; Cone-rod retinal dystrophy-2; Deafness, autosomal dominant 4A; Peripheral neuropathy, myopathy, hoarseness, and hearing loss; Deafness, autosomal dominant 22; Neurofibromatosis type 2; NASH; or Mental retardation, autosomal dominant 5 caused by a deficient amount of activity of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, and wherein the deficient amount of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is caused by haploinsufficiency of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein. In such an embodiment, the subject has a first allele encoding a functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, and a second allele from which the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is not produced. In another such embodiment, the subject has a first allele encoding a functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, and a second allele encoding a nonfunctional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein. In another such embodiment, the subject has a first allele encoding a functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, and a second allele encoding a partially functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein. In any of these embodiments, the antisense oligomer binds to a targeted portion of the NIE containing pre-mRNA transcribed from the second allele, thereby inducing exon skipping of the pseudo-exon from the pre-mRNA, and causing an increase in the level of mature mRNA encoding functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, and an increase in the expression of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein in the cells of the subject.

In some embodiments, the method is a method of increasing the expression of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP 1 protein by cells of a subject having a NIE containing pre-mRNA encoding the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, wherein the subject has Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; 16p11.2 deletion syndrome; Deafness, autosomal dominant 13; Cone-rod retinal dystrophy-2; Deafness, autosomal dominant 4A; Peripheral neuropathy, myopathy, hoarseness, and hearing loss; Deafness, autosomal dominant 22; Neurofibromatosis type 2; NASH; or Mental retardation, autosomal dominant 5 caused by a deficient amount of activity of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, and wherein the deficient amount of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is caused by autosomal recessive inheritance.

In some embodiments, the method is a method of increasing the expression of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP 1 protein by cells of a subject having a NIE containing pre-mRNA encoding the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, wherein the subject has Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; 16p11.2 deletion syndrome; Deafness, autosomal dominant 13; Cone-rod retinal dystrophy-2; Deafness, autosomal dominant 4A; Peripheral neuropathy, myopathy, hoarseness, and hearing loss; Deafness, autosomal dominant 22; Neurofibromatosis type 2; NASH; or Mental retardation, autosomal dominant 5 caused by a deficient amount of activity of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, and wherein the deficient amount of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is caused by autosomal dominant inheritance.

In some embodiments, the method is a method of increasing the expression of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP 1 protein by cells of a subject having a NIE containing pre-mRNA encoding the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, wherein the subject has Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; 16p11.2 deletion syndrome; Deafness, autosomal dominant 13; Cone-rod retinal dystrophy-2; Deafness, autosomal dominant 4A; Peripheral neuropathy, myopathy, hoarseness, and hearing loss; Deafness, autosomal dominant 22; Neurofibromatosis type 2; NASH; or Mental retardation, autosomal dominant 5 caused by a deficient amount of activity of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, and wherein the deficient amount of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is caused by X-linked dominant inheritance.

In related embodiments, the method is a method of using an ASO to increase the expression of a protein or functional RNA. In some embodiments, an ASO may be used to increase the expression of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein in cells of a subject having a NIE containing pre-mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, wherein the subject has a deficiency, e.g., Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; 16p11.2 deletion syndrome; Deafness, autosomal dominant 13; Cone-rod retinal dystrophy-2; Deafness, autosomal dominant 4A; Peripheral neuropathy, myopathy, hoarseness, and hearing loss; Deafness, autosomal dominant 22; Neurofibromatosis type 2; NASH; or Mental retardation, autosomal dominant 5, in the amount or function of a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein.

In some embodiments, the NIE containing pre-mRNA transcript that encodes the protein that is causative of the disease or condition is targeted by the ASOs described herein. In some embodiments, a NIE containing pre-mRNA transcript that encodes a protein that is not causative of the disease is targeted by the ASOs. For example, a disease that is the result of a mutation or deficiency of a first protein in a particular pathway may be ameliorated by targeting a NIE containing pre-mRNA that encodes a second protein, thereby increasing production of the second protein. In some embodiments, the function of the second protein is able to compensate for the mutation or deficiency of the first protein (which is causative of the disease or condition).

In some embodiments, the subject has:
(a) a first mutant allele from which
   (i) the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is produced at a reduced level compared to production from a wild-type allele,
   (ii) the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is produced in a form having reduced function compared to an equivalent wild-type protein, or
   (iii) the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein or functional RNA is not produced; and
(b) a second mutant allele from which
   (i) the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is produced at a reduced level compared to production from a wild-type allele,
   (ii) the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is produced in a form having reduced function compared to an equivalent wild-type protein, or
   (iii) the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is not produced, and
   wherein the NIE containing pre-mRNA is transcribed from the first allele and/or the second allele. In these embodiments, the ASO binds to a targeted portion of the NIE containing pre-mRNA transcribed from the first allele or the second allele, thereby inducing exon skipping of the pseudo-exon from the NIE containing pre-mRNA, and causing an increase in the level of mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein and an increase in the expression of the target protein or functional RNA in the cells of the subject. In these embodiments, the target protein or functional RNA having an increase in expression level resulting from the exon skipping of the pseudo-exon from the NIE containing pre-mRNA may be either in a form having reduced function compared to the equivalent wild-type protein (partially-functional), or having full function compared to the equivalent wild-type protein (fully-functional).

In some embodiments, the level of mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein is increased 1.1 to 10-fold, when compared to the amount of mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein that is produced in a control cell, e.g., one that is not treated with the antisense oligomer or one that is treated with an antisense oligomer that does not bind to the targeted portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* NIE containing pre-mRNA.

In some embodiments, a subject treated using the methods of the present disclosure expresses a partially functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein from one allele, wherein the partially functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein may be caused by a frameshift mutation, a nonsense mutation, a missense mutation, or a partial gene deletion. In some embodiments, a subject treated using the methods of the disclosure expresses a nonfunctional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein from one allele, wherein the nonfunctional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein may be caused by a frameshift mutation, a nonsense mutation, a missense mutation, a partial gene deletion, in one allele. In some embodiments, a subject treated using the methods of the disclosure has *a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1*, *KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* whole gene deletion, in one allele.

### Exon Inclusion

As used herein, a "NIE containing pre-mRNA" is a pre-mRNA transcript that contains at least one pseudo-exon. Alternative or aberrant splicing can result in inclusion of the at least one pseudo-exon in the mature mRNA transcripts. The terms "mature mRNA," and "fully-spliced mRNA," are used interchangeably herein to describe a fully processed mRNA. Inclusion of the at least one pseudo-exon can be non-productive mRNA and lead to NMD of the mature mRNA. NIE containing mature mRNA may sometimes lead to aberrant protein expression.

In some embodiments, the included pseudo-exon is the most abundant pseudo-exon in a population of NIE containing pre-mRNAs transcribed from the gene encoding the target protein in a cell. In some embodiments, the included pseudo-exon is the most abundant pseudo-exon in a population of NIE containing pre-mRNAs transcribed from the gene encoding the target protein in a cell, wherein the population of NIE containing pre-mRNAs comprises two or more included pseudo-exons. In some embodiments, an antisense oligomer targeted to the most abundant pseudo-exon in the population of NIE containing pre-mRNAs encoding the target protein induces exon skipping of one or two or more pseudo-exons in the population, including the pseudo-exon to which the antisense oligomer is targeted or binds. In some embodiments, the targeted region is in a pseudo-exon that is the most abundant pseudo-exon in a NIE containing pre-mRNA encoding the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein.

The degree of exon inclusion can be expressed as percent exon inclusion, e.g., the percentage of transcripts in which a given pseudo-exon is included. In brief, percent exon inclusion can be calculated as the percentage of the amount of RNA transcripts with the exon inclusion, over the sum of the average of the amount of RNA transcripts with exon inclusion plus the average of the amount of RNA transcripts with exon exclusion.

In some embodiments, an included pseudo-exon is an exon that is identified as an included pseudo-exon based on a determination of at least about 5%, at least about 10%, at least about 15%, at least about 20%, at least about 25%, at least about 30%, at least about 35%, at least about 40%, at least about 45%, or at least about 50%, inclusion. In embodiments, a included pseudo-exon is an exon that is identified as a included pseudo-exon based on a determination of about 5% to about 100%, about 5% to about 95%, about 5% to about 90%, about 5% to about 85%, about 5% to about 80%, about 5% to about 75%, about 5% to about 70%, about 5% to about 65%, about 5% to about 60%, about 5% to about 55%, about 5% to about 50%, about 5% to about 45%, about 5% to about 40%, about 5% to about 35%, about 5% to about 30%, about 5% to about 25%, about 5% to about 20%, about 5% to about 15%, about 10% to about 100%, about 10% to about 95%, about 10% to about 90%, about 10% to about 85%, about 10% to about 80%, about 10% to about 75%, about 10% to about 70%, about 10% to about 65%, about 10% to about 60%, about 10% to about 55%, about 10% to about 50%, about 10% to about 45%, about 10% to about 40%, about 10% to about 35%, about 10% to about 30%, about 10% to about 25%, about 10% to about 20%, about 15% to about 100%, about 15% to about 95%, about 15% to about 90%, about 15% to about 85%, about 15% to about 80%, about 15% to about 75%, about 15% to about 70%, about 15% to about 65%, about 15% to about 60%, about 15% to about 55%, about 15% to about 50%, about 15% to about 45%, about 15% to about 40%, about 15% to about 35%, about 15% to about 30%, about 15% to about 25%, about 20% to about 100%, about 20% to about 95%, about 20% to about 90%, about 20% to about 85%, about 20% to about 80%, about 20% to about 75%, about 20% to about 70%, about 20% to about 65%, about 20% to about 60%, about 20% to about 55%, about 20% to about 50%, about 20% to about 45%, about 20% to about 40%, about 20% to about 35%, about 20% to about 30%, about 25% to about 100%, about 25% to about 95%, about 25% to about 90%, about 25% to about 85%, about 25% to about 80%, about 25% to about 75%, about 25% to about 70%, about 25% to about 65%, about 25% to about 60%, about 25% to about 55%, about 25% to about 50%, about 25% to about 45%, about 25% to about 40%, or about 25% to about 35%, inclusion. ENCODE data (described by, *e.g*., Tilgner, et al., 2012, "Deep sequencing of subcellular RNA fractions shows splicing to be predominantly co-transcriptional in the human genome but inefficient for lncRNAs," Genome Research 22(9): 1616-25) can be used to aid in identifying exon inclusion.

In some embodiments, contacting cells with an ASO that is complementary to a targeted portion of a*ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1*, *KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* pre-mRNA transcript results in an increase in the amount of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein produced by at least 10, 20, 30, 40, 50, 60, 80, 100, 150, 200, 250, 300, 350, 400, 450, 500, or 1000%, compared to the amount of the protein produced by a cell in the absence of the ASO/absence of treatment. In some embodiments, the total amount of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein produced by the cell to which the antisense oligomer is contacted is increased about 20% to about 300%, about 50% to about 300%, about 100% to about 300%, about 150% to about 300%, about 20% to about 50%, about 20% to about 100%, about 20% to about 150%, about 20% to about 200%, about 20% to about 250%, about 50% to about 100%, about 50% to about 150%, about 50% to about 200%, about 50% to about 250%, about 100% to about 150%, about 100% to about 200%, about 100% to about 250%, about 150% to about 200%, about 150% to about 250%, about 200% to about 250%, at least about 10%, at least about 20%, at least about 50%, at least about 100%, at least about 150%, at least about 200%, at least about 250%, or at least about 300%, compared to the amount of target protein produced by a control compound. In some embodiments, the total amount of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein produced by the cell to which the antisense oligomer is contacted is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1. 1-fold, at least about 1.5-fold, at least about 2-fold, at least about 2.5-fold, at least about 3-fold, at least about 3.5-fold, at least about 4-fold, at least about 5-fold, or at least about 10-fold, compared to the amount of target protein produced by a control compound. A control compound can be, for example, an oligonucleotide that is not complementary to a targeted portion of the pre-mRNA.

In some embodiments, contacting cells with an ASO that is complementary to a targeted portion of a*ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1*, *KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* pre-mRNA transcript results in an increase in the amount of mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1, including the mature mRNA encoding the target protein. In some embodiments, the amount of mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, or the mature mRNA encoding the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, is increased by at least 10, 20, 30, 40, 50, 60, 80, 100, 150, 200, 250, 300, 350, 400, 450, 500, or 1000%, compared to the amount of the protein produced by a cell in the absence of the ASO/absence of treatment. In some embodiments, the total amount of the mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, or the mature mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1*,* NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein produced in the cell to which the antisense oligomer is contacted is increased about 20% to about 300%, about 50% to about 300%, about 100% to about 300%, about 150% to about 300%, about 20% to about 50%, about 20% to about 100%, about 20% to about 150%, about 20% to about 200%, about 20% to about 250%, about 50% to about 100%, about 50% to about 150%, about 50% to about 200%, about 50% to about 250%, about 100% to about 150%, about 100% to about 200%, about 100% to about 250%, about 150% to about 200%, about 150% to about 250%, about 200% to about 250%, at least about 10%, at least about 20%, at least about 50%, at least about 100%, at least about 150%, at least about 200%, at least about 250%, or at least about 300%, compared to the amount of mature RNA produced in an untreated cell, *e*.*g*., an untreated cell or a cell treated with a control compound. In some embodiments, the total amount of the mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, or the mature mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein produced in the cell to which the antisense oligomer is contacted is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1. 1-fold, at least about 1.5-fold, at least about 2-fold, at least about 2.5-fold, at least about 3-fold, at least about 3.5-fold, at least about 4-fold, at least about 5-fold, or at least about 10-fold compared to the amount of mature RNA produced in an untreated cell, e.g., an untreated cell or a cell treated with a control compound. A control compound can be, for example, an oligonucleotide that is not complementary to a targeted portion of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1*,* NIPBL, NSD1, OPA1, OPTN, PCCA, PCCB, PKP2, PLCB1*,* PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 NIE containing pre-mRNA.

The NIE can be in any length. In some embodiments, the NIE comprises a full sequence of an intron, in which case, it can be referred to as intron retention. In some embodiments, the NIE can be a portion of the intron. In some embodiments, the NIE can be a 5' end portion of an intron including a 5'ss sequence. In some embodiments, the NIE can be a 3' end portion of an intron including a 3'ss sequence. In some embodiments, the NIE can be a portion within an intron without inclusion of a 5'ss sequence. In some embodiments, the NIE can be a portion within an intron without inclusion of a 3'ss sequence. In some embodiments, the NIE can be a portion within an intron without inclusion of either a 5'ss or a 3'ss sequence. In some embodiments, the NIE can be from 5 nucleotides to 10 nucleotides in length, from 10 nucleotides to 15 nucleotides in length, from 15 nucleotides to 20 nucleotides in length, from 20 nucleotides to 25 nucleotides in length, from 25 nucleotides to 30 nucleotides in length, from 30 nucleotides to 35 nucleotides in length, from 35 nucleotides to 40 nucleotides in length, from 40 nucleotides to 45 nucleotides in length, from 45 nucleotides to 50 nucleotides in length, from 50 nucleotides to 55 nucleotides in length, from 55 nucleotides to 60 nucleotides in length, from 60 nucleotides to 65 nucleotides in length, from 65 nucleotides to 70 nucleotides in length, from 70 nucleotides to 75 nucleotides in length, from 75 nucleotides to 80 nucleotides in length, from 80 nucleotides to 85 nucleotides in length, from 85 nucleotides to 90 nucleotides in length, from 90 nucleotides to 95 nucleotides in length, or from 95 nucleotides to 100 nucleotides in length. In some embodiments, the NIE can be at least 10 nucleotides, at least 20 nucleotides, at least 30 nucleotides, at least 40 nucleotides, at least 50 nucleotides, at least 60 nucleoids, at least 70 nucleotides, at least 80 nucleotides in length, at least 90 nucleotides, or at least 100 nucleotides in length. In some embodiments, the NIE can be from 100 to 200 nucleotides in length, from 200 to 300 nucleotides in length, from 300 to 400 nucleotides in length, from 400 to 500 nucleotides in length, from 500 to 600 nucleotides in length, from 600 to 700 nucleotides in length, from 700 to 800 nucleotides in length, from 800 to 900 nucleotides in length, from 900 to 1,000 nucleotides in length. In some embodiments, the NIE may be longer than 1,000 nucleotides in length.

Inclusion of a pseudo-exon can lead to a frameshift and the introduction of a premature termination codon (PIC) in the mature mRNA transcript rendering the transcript a target of NMD. Mature mRNA transcript containing NIE can be non-productive mRNA transcript which does not lead to protein expression. The PIC can be present in any position downstream of an NIE. In some embodiments, the PIC can be present in any exon downstream of an NIE. In some embodiments, the PIC can be present within the NIE. For example, inclusion of exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB 1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN in an mRNA transcript encoded by the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN gene can induce a PIC in the mRNA transcript. For example, inclusion of exon (GRCh38/ hg38: chr1 243564285 243564388) of AKT3; exon (GRCh38/ hg38: chr19 13236449 13236618) of CACNA1A; exon (GRCh38/ hg38: chr21 43059730 43060012) of CBS; exon (GRCh38/ hg38: chr1 207775610 207775745) of CD46; exon (GRCh38/ hg38: chr1 196675450 196675529) of CFH; exon (GRCh38/ hg38: chr15 92998149 92998261) of CHD2; exon (GRCh38/ hg38: chr16 28479644 28479765) of CLN3; exon (GRCh38/ hg38: chr6 33183634 33183698) of COL11A2; exon (GRCh38/ hg38: chr2 227296487 227296526) of COL4A3; exon (GRCh38/ hg38: chr2 227144653 227144833) of COL4A4; exon (GRCh38/ hg38: chr2 227015283 227015360) of COL4A4; exon (GRCh38/ hg38: chr1 207637688 207637848) of CR1; exon (GRCh38/ hg38: chr19 47835403 47835579) of CRX; exon (GRCh38/ hg38: chr1 59904366 59904516) of CYP2J2; exon (GRCh38/ hg38: chr1 26442335 26442372) of DHDDS; exon (GRCh38/ hg38: chr1 28230131 28230252 ) of DNAJC8; exon (GRCh38/ hg38: chr2 88582755 88582824) of EIF2AK3; exon (GRCh38/ hg38: chr17 64102673 64102804) of ERN1; exon (GRCh38/ hg38: chr1 23798311 23798484) of GALE; exon (GRCh38/ hg38: chrX 109383365 109383446) of GUCY2F; exon (GRCh38/ hg38: chrX 109439038 109439175) of GUCY2F; exon (GRCh38/ hg38: chr15 72362376 72362466) of HEXA; exon (GRCh38/ hg38: chr15 72345677 72345776) of HEXA; exon (GRCh38/ hg38: chr16 30115595 30115645) of MAPK3; exon (GRCh38/ hg38: chr2 148460219 148460304) of MBD5; exon (GRCh38/ hg38: chr2 148490695 148490787) of MBD5; exon (GRCh38/ hg38: chr2 148505761 148505830) of MBD5; exon (GRCh38/ hg38: chr6 49436522 49436597) of MUT; exon (GRCh38/ hg38: chr19 50230825 50230999) of MYH14; exon (GRCh38/ hg38: chr6 75867431 75867523) of MYO6; exon (GRCh38/ hg38: chr17 31249955 31250125) of NF1; exon (GRCh38/ hg38: chr22 29628658 29628773) of NF2; exon (GRCh38/ hg38: chr5 37048127 37048354) of NIPBL; exon (GRCh38/ hg38: chr12 100499841 100500024) of NR1H4; exon (GRCh38/ hg38: chr5 177169394 177169559) of NSD1; exon (GRCh38/ hg38: chr5 177200761 177200783) of NSD1; exon (GRCh38/ hg38: chr5 177247924 177248079) of NSD1; exon (GRCh38/ hg38: chr5 177275947 177276101) of NSD1; exon (GRCh38/ hg38: chr3 193628509 193628616) of OPA1; exon (GRCh38/ hg38: chr3 193603500 193603557) of OPA1; exon (GRCh38/ hg38: chr13 100305751 100305834) of PCCA; exon (GRCh38/ hg38: chr12 32894516 32894778) of PKP2; exon (GRCh38/ hg38: chr22 46203575 46203752) of PPARA; exon (GRCh38/ hg38: chr1 150327557 150327652) of PRPF3; exon (GRCh38/ hg38: chr1 150330401 150330498) of PRPF3; exon (GRCh38/ hg38: chr2 165327155 165327202) of SCN2A; exon (GRCh38/ hg38: chr12 51688758 51688849) of SCN8A; exon (GRCh38/ hg38: chr12 51780202 51780271) of SCN8A; exon (GRCh38/ hg38: chr2 166304238 166304329) of SCN9A; exon (GRCh38/ hg38: chr7 80794854 80794957) of SEMA3C; exon (GRCh38/ hg38: chr7 85059498 85059541) of SEMA3D; exon (GRCh38/ hg38: chr11 225673 226081) of SIRT3; exon (GRCh38/ hg38: chr19 1216268 1216398) of STK11; exon (GRCh38/ hg38: chr19 1221621 1221846) of STK11; exon (GRCh38/ hg38: chr6 33448789 33448868) of SYNGAP1; exon (GRCh38/ hg38: chr9 32551365 32551469) of TOPORS; exon (GRCh38/ hg38: chr5 83544965 83545070) of VCAN in an mRNA transcript encoded by the AKT3, CACNA1A, CBS, CD46, CFH, CHD2, CLN3, COL11A2, COL4A3, COL4A4, COL4A4, CR1, CRX, CYP2J2, DHDDS, DNAJC8, EIF2AK3, ERN1, GALE, GUCY2F, GUCY2F, HEXA, HEXA, MAPK3, MBD5, MBD5, MBD5, MUT, MYH14, MYO6, NF1, NF2, NIPBL, NR1H4, NSD1, NSD1, NSD1, NSD1, OPA1, OPA1, PCCA, PKP2, PPARA, PRPF3, PRPF3, SCN2A, SCN8A, SCN8A, SCN9A, SEMA3C, SEMA3D, SIRT3, STK11, STK11, SYNGAP1, TOPORS, or VCAN.

### Therapeutic Agents

In various embodiments of the present disclosure, compositions and methods comprising a therapeutic agent are provided to modulate protein expression level of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1. In some embodiments, provided herein are compositions and methods to modulate alternative splicing of *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1*, *KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA. In some embodiments, provided herein are compositions and methods to induce exon skipping in the splicing of *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1*, *KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANKS, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA, e.g., to induce skipping of a pseudo-exon during splicing of *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1*, *KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* pre-mRNA. In other embodiments, therapeutic agents may be used to induce the inclusion of an exon in order to decrease the protein expression level.

A therapeutic agent disclosed herein can be a NIE repressor agent. A therapeutic agent may comprise a polynucleic acid polymer. According to one aspect of the present disclosure, provided herein is a method of treatment or prevention of a condition or disease associated with a functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein deficiency, comprising administering a NIE repressor agent to a subject to increase levels of functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein, wherein the agent binds to a region of the pre-mRNA transcript to decrease inclusion of the NIE in the mature transcript. For example, provided herein is a method of treatment or prevention of a condition associated with a functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein deficiency, comprising administering a NIE repressor agent to a subject to increase levels of functional ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, wherein the agent binds to a region of an intron containing an NIE (e.g., exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN) of the pre-mRNA transcript or to a NIE-activating regulatory sequence in the same intron. For example, provided herein is a method of treatment or prevention of a condition associated with a functional AKT3, CACNA1A, CBS, CD46, CFH, CHD2, CLN3, COL11A2, COL4A3, COL4A4, COL4A4, CR1, CRX, CYP2J2, DHDDS, DNAJC8, EIF2AK3, ERN1, GALE, GUCY2F, GUCY2F, HEXA, HEXA, MAPK3, MBD5, MBD5, MBD5, MUT, MYH14, MYO6, NF1, NF2, NIPBL, NR1H4, NSD1, NSD1, NSD1, NSD1, OPA1, OPA1, PCCA, PKP2, PPARA, PRPF3, PRPF3, SCN2A, SCN8A, SCN8A, SCN9A, SEMA3C, SEMA3D, SIRT3, STK11, STK11, SYNGAP1, TOPORS, or VCAN protein deficiency, comprising administering a NIE repressor agent to a subject to increase levels of functional AKT3, CACNA1A, CBS, CD46, CFH, CHD2, CLN3, COL11A2, COL4A3, COL4A4, COL4A4, CR1, CRX, CYP2J2, DHDDS, DNAJC8, EIF2AK3, ERN1, GALE, GUCY2F, GUCY2F, HEXA, HEXA, MAPK3, MBD5, MBD5, MBD5, MUT, MYH14, MYO6, NF1, NF2, NIPBL, NR1H4, NSD1, NSD1, NSD1, NSD1, OPA1, OPA1, PCCA, PKP2, PPARA, PRPF3, PRPF3, SCN2A, SCN8A, SCN8A, SCN9A, SEMA3C, SEMA3D, SIRT3, STK11, STK11, SYNGAP1, TOPORS, or VCAN protein, wherein the agent binds to a region of an intron containing an NIE (e.g., exon (GRCh38/ hg38: chr1 243564285 243564388) of AKT3; exon (GRCh38/ hg38: chr19 13236449 13236618) of CACNA1A; exon (GRCh38/ hg38: chr21 43059730 43060012) of CBS; exon (GRCh38/ hg38: chr1 207775610 207775745) of CD46; exon (GRCh38/ hg38: chr1 196675450 196675529) of CFH; exon (GRCh38/ hg38: chr15 92998149 92998261) of CHD2; exon (GRCh38/ hg38: chr16 28479644 28479765) of CLN3; exon (GRCh38/ hg38: chr6 33183634 33183698) of COL11A2; exon (GRCh38/ hg38: chr2 227296487 227296526) of COL4A3; exon (GRCh38/ hg38: chr2 227144653 227144833) of COL4A4; exon (GRCh38/ hg38: chr2 227015283 227015360) of COL4A4; exon (GRCh38/ hg38: chr1 207637688 207637848) of CR1; exon (GRCh38/ hg38: chr19 47835403 47835579) of CRX; exon (GRCh38/ hg38: chr1 59904366 59904516) of CYP2J2; exon (GRCh38/ hg38: chr1 26442335 26442372) of DHDDS; exon (GRCh38/ hg38: chr1 28230131 28230252 ) of DNAJC8; exon (GRCh38/ hg38: chr2 88582755 88582824) of EIF2AK3; exon (GRCh38/ hg38: chr17 64102673 64102804) of ERN1; exon (GRCh38/ hg38: chr1 23798311 23798484) of GALE; exon (GRCh38/ hg38: chrX 109383365 109383446) of GUCY2F; exon (GRCh38/ hg38: chrX 109439038 109439175) of GUCY2F; exon (GRCh38/ hg38: chr15 72362376 72362466) of HEXA; exon (GRCh38/ hg38: chr15 72345677 72345776) of HEXA; exon (GRCh38/ hg38: chr16 30115595 30115645) of MAPK3; exon (GRCh38/ hg38: chr2 148460219 148460304) of MBD5; exon (GRCh38/ hg38: chr2 148490695 148490787) of MBD5; exon (GRCh38/ hg38: chr2 148505761 148505830) of MBD5; exon (GRCh38/ hg38: chr6 49436522 49436597) of MUT; exon (GRCh38/ hg38: chr19 50230825 50230999) of MYH14; exon (GRCh38/ hg38: chr6 75867431 75867523) of MYO6; exon (GRCh38/ hg38: chr17 31249955 31250125) of NF1; exon (GRCh38/ hg38: chr22 29628658 29628773) of NF2; exon (GRCh38/ hg38: chr5 37048127 37048354) of NIPBL; exon (GRCh38/ hg38: chr12 100499841 100500024) of NR1H4; exon (GRCh38/ hg38: chr5 177169394 177169559) of NSD1; exon (GRCh38/ hg38: chr5 177200761 177200783) of NSD1; exon (GRCh38/ hg38: chr5 177247924 177248079) of NSD1; exon (GRCh38/ hg38: chr5 177275947 177276101) of NSD1; exon (GRCh38/ hg38: chr3 193628509 193628616) of OPA1; exon (GRCh38/ hg38: chr3 193603500 193603557) of OPA1; exon (GRCh38/ hg38: chr13 100305751 100305834) of PCCA; exon (GRCh38/ hg38: chr12 32894516 32894778) of PKP2; exon (GRCh38/ hg38: chr22 46203575 46203752) of PPARA; exon (GRCh38/ hg38: chr1 150327557 150327652) of PRPF3; exon (GRCh38/ hg38: chr1 150330401 150330498) of PRPF3; exon (GRCh38/ hg38: chr2 165327155 165327202) of SCN2A; exon (GRCh38/ hg38: chr12 51688758 51688849) of SCN8A; exon (GRCh38/ hg38: chr12 51780202 51780271) of SCN8A; exon (GRCh38/ hg38: chr2 166304238 166304329) of SCN9A; exon (GRCh38/ hg38: chr7 80794854 80794957) of SEMA3C; exon (GRCh38/ hg38: chr7 85059498 85059541) of SEMA3D; exon (GRCh38/ hg38: chr11 225673 226081) of SIRT3; exon (GRCh38/ hg38: chr19 1216268 1216398) of STK11; exon (GRCh38/ hg38: chr19 1221621 1221846) of STK11; exon (GRCh38/ hg38: chr6 33448789 33448868) of SYNGAP1; exon (GRCh38/ hg38: chr9 32551365 32551469) of TOPORS; exon (GRCh38/ hg38: chr5 83544965 83545070) of VCAN) of the pre-mRNA transcript or to a NIE-activating regulatory sequence in the same intron.

Where reference is made to reducing NIE inclusion in the mature mRNA, the reduction may be complete, e.g., 100%, or may be partial. The reduction may be clinically significant. The reduction/correction may be relative to the level of NIE inclusion in the subject without treatment, or relative to the amount of NIE inclusion in a population of similar subjects. The reduction/correction may be at least 10% less NIE inclusion relative to the average subject, or the subject prior to treatment. The reduction may be at least 20% less NIE inclusion relative to an average subject, or the subject prior to treatment. The reduction may be at least 40% less NIE inclusion relative to an average subject, or the subject prior to treatment. The reduction may be at least 50% less NIE inclusion relative to an average subject, or the subject prior to treatment. The reduction may be at least 60% less NIE inclusion relative to an average subject, or the subject prior to treatment. The reduction may be at least 80% less NIE inclusion relative to an average subject, or the subject prior to treatment. The reduction may be at least 90% less NIE inclusion relative to an average subject, or the subject prior to treatment.

Where reference is made to increasing active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein levels, the increase may be clinically significant. The increase may be relative to the level of active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein in the subject without treatment, or relative to the amount of active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein in a population of similar subjects. The increase may be at least 10% more active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein relative to the average subject, or the subject prior to treatment. The increase may be at least 20% more active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein relative to the average subject, or the subject prior to treatment. The increase may be at least 40% more active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein relative to the average subject, or the subject prior to treatment. The increase may be at least 50% more active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein relative to the average subject, or the subject prior to treatment. The increase may be at least 80% more active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein relative to the average subject, or the subject prior to treatment. The increase may be at least 100% more active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein relative to the average subject, or the subject prior to treatment. The increase may be at least 200% more active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein relative to the average subject, or the subject prior to treatment The increase may be at least 500% more active ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1 protein relative to the average subject, or the subject prior to treatment.

In embodiments wherein the NIE repressor agent comprises a polynucleic acid polymer, the polynucleic acid polymer may be about 50 nucleotides in length. The polynucleic acid polymer may be about 45 nucleotides in length. The polynucleic acid polymer may be about 40 nucleotides in length. The polynucleic acid polymer may be about 35 nucleotides in length. The polynucleic acid polymer may be about 30 nucleotides in length. The polynucleic acid polymer may be about 24 nucleotides in length. The polynucleic acid polymer may be about 25 nucleotides in length. The polynucleic acid polymer may be about 20 nucleotides in length. The polynucleic acid polymer may be about 19 nucleotides in length. The polynucleic acid polymer may be about 18 nucleotides in length. The polynucleic acid polymer may be about 17 nucleotides in length. The polynucleic acid polymer may be about 16 nucleotides in length. The polynucleic acid polymer may be about 15 nucleotides in length. The polynucleic acid polymer may be about 14 nucleotides in length. The polynucleic acid polymer may be about 13 nucleotides in length. The polynucleic acid polymer may be about 12 nucleotides in length. The polynucleic acid polymer may be about 11 nucleotides in length. The polynucleic acid polymer may be about 10 nucleotides in length. The polynucleic acid polymer may be between about 10 and about 50 nucleotides in length. The polynucleic acid polymer may be between about 10 and about 45 nucleotides in length. The polynucleic acid polymer may be between about 10 and about 40 nucleotides in length. The polynucleic acid polymer may be between about 10 and about 35 nucleotides in length. The polynucleic acid polymer may be between about 10 and about 30 nucleotides in length. The polynucleic acid polymer may be between about 10 and about 25 nucleotides in length. The polynucleic acid polymer may be between about 10 and about 20 nucleotides in length. The polynucleic acid polymer may be between about 15 and about 25 nucleotides in length. The polynucleic acid polymer may be between about 15 and about 30 nucleotides in length. The polynucleic acid polymer may be between about 12 and about 30 nucleotides in length.

The sequence of the polynucleic acid polymer may be at least 50%, 55%, 60%, 65%, 70%, 75%, 80%, 85%, 90%, 91%, 92%, 93%, 94%, 95%, 96%, 97%, 98%, 99%, or 99.5% complementary to a target sequence of an mRNA transcript, e.g., a partially processed mRNA transcript. The sequence of the polynucleic acid polymer may be 100% complementary to a target sequence of a pre-mRNA transcript.

The sequence of the polynucleic acid polymer may have 4 or fewer mismatches to a target sequence of the pre-mRNA transcript. The sequence of the polynucleic acid polymer may have 3 or fewer mismatches to a target sequence of the pre-mRNA transcript. The sequence of the polynucleic acid polymer may have 2 or fewer mismatches to a target sequence of the pre-mRNA transcript. The sequence of the polynucleic acid polymer may have 1 or fewer mismatches to a target sequence of the pre-mRNA transcript. The sequence of the polynucleic acid polymer may have no mismatches to a target sequence of the pre-mRNA transcript.

The polynucleic acid polymer may specifically hybridize to a target sequence of the pre-mRNA transcript. For example, the polynucleic acid polymer may have 91%, 92%, 93%, 94%, 95%, 96%, 97%, 98%, 99%, 99.5% or 100% sequence complementarity to a target sequence of the pre-mRNA transcript. The hybridization may be under high stringent hybridization conditions.

The polynucleic acid polymer comprising a sequence with at least 50%, 55%, 60%, 65%, 70%, 75%, 80%, 85%, 90%, 91%, 92%, 93%, 94%, 95%, 96%, 97%, 98%, 99%, or 99.5% sequence identity to a sequence selected from the group consisting of SEQ ID NOs: 60-191. The polynucleic acid polymer may comprise a sequence with 100% sequence identity to a sequence selected from the group consisting of SEQ ID NOs: 60-191.

Where reference is made to a polynucleic acid polymer sequence, the skilled person will understand that one or more substitutions may be tolerated, optionally two substitutions may be tolerated in the sequence, such that it maintains the ability to hybridize to the target sequence; or where the substitution is in a target sequence, the ability to be recognized as the target sequence. References to sequence identity may be determined by BLAST sequence alignment using standard/default parameters. For example, the sequence may have 99% identity and still function according to the present disclosure. In other embodiments, the sequence may have 98% identity and still function according to the present disclosure. In another embodiment, the sequence may have 95% identity and still function according to the present disclosure. In another embodiment, the sequence may have 90% identity and still function according to the present disclosure.

### Antisense Oligomers

Provided herein is a composition comprising an antisense oligomer that induces exon skipping by binding to a targeted portion of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1*, *KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1*, *RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA. As used herein, the terms "ASO" and "antisense oligomer" are used interchangeably and refer to an oligomer such as a polynucleotide, comprising nucleobases that hybridizes to a target nucleic acid (*e.g*., *a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* NIE containing pre-mRNA) sequence by Watson-Crick base pairing or wobble base pairing (G-U). The ASO may have exact sequence complementary to the target sequence or near complementarity (*e.g.*, sufficient complementarity to bind the target sequence and enhancing splicing at a splice site). ASOs are designed so that they bind (hybridize) to a target nucleic acid (*e.g*., a targeted portion of a pre-mRNA transcript) and remain hybridized under physiological conditions. Typically, if they hybridize to a site other than the intended (targeted) nucleic acid sequence, they hybridize to a limited number of sequences that are not a target nucleic acid (to a few sites other than a target nucleic acid). Design of an ASO can take into consideration the occurrence of the nucleic acid sequence of the targeted portion of the pre-mRNA transcript or a sufficiently similar nucleic acid sequence in other locations in the genome or cellular pre-mRNA or transcriptome, such that the likelihood the ASO will bind other sites and cause "off-target" effects is limited. Any antisense oligomers known in the art, for example in PCT Application No. PCT/US2014/054151, published as WO 2015/035091, titled "Reducing Nonsense-Mediated mRNA Decay," incorporated by reference herein, can be used to practice the methods described herein.

In some embodiments, ASOs "specifically hybridize" to or are "specific" to a target nucleic acid or a targeted portion of a NIE containing pre-mRNA. Typically such hybridization occurs with a Tₘ substantially greater than 37 °C, preferably at least 50 °C, and typically between 60 °C to approximately 90 °C. Such hybridization preferably corresponds to stringent hybridization conditions. At a given ionic strength and pH, the Tₘ is the temperature at which 50% of a target sequence hybridizes to a complementary oligonucleotide.

Oligomers, such as oligonucleotides, are "complementary" to one another when hybridization occurs in an antiparallel configuration between two single-stranded polynucleotides. A double-stranded polynucleotide can be "complementary" to another polynucleotide, if hybridization can occur between one of the strands of the first polynucleotide and the second. Complementarity (the degree to which one polynucleotide is complementary with another) is quantifiable in terms of the proportion (*e.g*., the percentage) of bases in opposing strands that are expected to form hydrogen bonds with each other, according to generally accepted base-pairing rules. The sequence of an antisense oligomer (ASO) need not be 100% complementary to that of its target nucleic acid to hybridize. In certain embodiments, ASOs can comprise at least 70%, at least 75%, at least 80%, at least 85%, at least 90%, at least 95%, at least 96%, at least 97%, at least 98%, or at least 99% sequence complementarity to a target region within the target nucleic acid sequence to which they are targeted. For example, an ASO in which 18 of 20 nucleobases of the oligomeric compound are complementary to a target region, and would therefore specifically hybridize, would represent 90 percent complementarity. In this example, the remaining non-complementary nucleobases may be clustered together or interspersed with complementary nucleobases and need not be contiguous to each other or to complementary nucleobases. Percent complementarity of an ASO with a region of a target nucleic acid can be determined routinely using BLAST programs (basic local alignment search tools) and PowerBLAST programs known in the art (Altschul, et al., J. Mol. Biol., 1990, 215, 403-410; Zhang and Madden, Genome Res., 1997, 7, 649-656).

An ASO need not hybridize to all nucleobases in a target sequence and the nucleobases to which it does hybridize may be contiguous or noncontiguous. ASOs may hybridize over one or more segments of a pre-mRNA transcript, such that intervening or adjacent segments are not involved in the hybridization event (*e.g*., a loop structure or hairpin structure may be formed). In certain embodiments, an ASO hybridizes to noncontiguous nucleobases in a target pre-mRNA transcript. For example, an ASO can hybridize to nucleobases in a pre-mRNA transcript that are separated by one or more nucleobase(s) to which the ASO does not hybridize.

The ASOs described herein comprise nucleobases that are complementary to nucleobases present in a target portion of a NIE containing pre-mRNA. The term ASO embodies oligonucleotides and any other oligomeric molecule that comprises nucleobases capable of hybridizing to a complementary nucleobase on a target mRNA but does not comprise a sugar moiety, such as a peptide nucleic acid (PNA). The ASOs may comprise naturally-occurring nucleotides, nucleotide analogs, modified nucleotides, or any combination of two or three of the preceding. The term "naturally occurring nucleotides" includes deoxyribonucleotides and ribonucleotides. The term "modified nucleotides" includes nucleotides with modified or substituted sugar groups and/or having a modified backbone. In some embodiments, all of the nucleotides of the ASO are modified nucleotides. Chemical modifications of ASOs or components of ASOs that are compatible with the methods and compositions described herein will be evident to one of skill in the art and can be found, for example, in U.S. Patent No. 8,258,109 B2, U.S. Patent No. 5,656,612, U.S. Patent Publication No. 2012/0190728, and Dias and Stein, Mol. Cancer Ther. 2002, 347-355, herein incorporated by reference in their entirety.

One or more nucleobases of an ASO may be any naturally occurring, unmodified nucleobase such as adenine, guanine, cytosine, thymine and uracil, or any synthetic or modified nucleobase that is sufficiently similar to an unmodified nucleobase such that it is capable of hydrogen bonding with a nucleobase present on a target pre-mRNA. Examples of modified nucleobases include, without limitation, hypoxanthine, xanthine, 7-methylguanine, 5, 6-dihydrouracil, 5-methylcytosine, and 5-hydroxymethoylcytosine.

The ASOs described herein also comprise a backbone structure that connects the components of an oligomer. The term "backbone structure" and "oligomer linkages" may be used interchangeably and refer to the connection between monomers of the ASO. In naturally occurring oligonucleotides, the backbone comprises a 3'-5' phosphodiester linkage connecting sugar moieties of the oligomer. The backbone structure or oligomer linkages of the ASOs described herein may include (but are not limited to) phosphorothioate, phosphorodithioate, phosphoroselenoate, phosphorodiselenoate, phosphoroanilothioate, phosphoraniladate, phosphoramidate, and the like. See, *e.g*., LaPlanche, et al., Nucleic Acids Res. 14:9081 (1986); Stec, et al., J. Am. Chem. Soc. 106:6077 (1984), Stein, et al., Nucleic Acids Res. 16:3209 (1988), Zon, et al., Anti-Cancer Drug Design 6:539 (1991); Zon, et al., Oligonucleotides and Analogues: A Practical Approach, pp. 87-108 (F. Eckstein, Ed., Oxford University Press, Oxford England (1991)); Stec, et al., U.S. Pat. No. 5,151,510; Uhlmann and Peyman, Chemical Reviews 90:543 (1990). In some embodiments, the backbone structure of the ASO does not contain phosphorous but rather contains peptide bonds, for example in a peptide nucleic acid (PNA), or linking groups including carbamate, amides, and linear and cyclic hydrocarbon groups. In some embodiments, the backbone modification is a phosphothioate linkage. In some embodiments, the backbone modification is a phosphoramidate linkage.

In some embodiments, the stereochemistry at each of the phosphorus internucleotide linkages of the ASO backbone is random. In some embodiments, the stereochemistry at each of the phosphorus internucleotide linkages of the ASO backbone is controlled and is not random. For example, U.S. Pat. App. Pub. No. 2014/0194610, "Methods for the Synthesis of Functionalized Nucleic Acids," incorporated herein by reference, describes methods for independently selecting the handedness of chirality at each phosphorous atom in a nucleic acid oligomer. In some embodiments, an ASO used in the methods of the disclosure, including, but not limited to, any of the ASOs set forth herein in Tables 5 and 6, comprises an ASO having phosphorus internucleotide linkages that are not random. In some embodiments, a composition used in the methods of the disclosure comprises a pure diastereomeric ASO. In some embodiments, a composition used in the methods of the disclosure comprises an ASO that has diastereomeric purity of at least about 90%, at least about 91%, at least about 92%, at least about 93%, at least about 94%, at least about 95%, at least about 96%, at least about 97%, at least about 98%, at least about 99%, about 100%, about 90% to about 100%, about 91% to about 100%, about 92% to about 100%, about 93% to about 100%, about 94% to about 100%, about 95% to about 100%, about 96% to about 100%, about 97% to about 100%, about 98% to about 100% , or about 99% to about 100%.

In some embodiments, the ASO has a nonrandom mixture of Rp and Sp configurations at its phosphorus internucleotide linkages. For example, it has been suggested that a mix of Rp and Sp is required in antisense oligonucleotides to achieve a balance between good activity and nuclease stability (Wan, et al., 2014, "Synthesis, biophysical properties and biological activity of second generation antisense oligonucleotides containing chiral phosphorothioate linkages," Nucleic Acids Research 42(22): 13456-13468, incorporated herein by reference). In some embodiments, an ASO used in the methods of the disclosure, including, but not limited to, any of the ASOs set forth herein in SEQ ID NOs: 60-191, comprises about 5-100% Rp, at least about 5% Rp, at least about 10% Rp, at least about 15% Rp, at least about 20% Rp, at least about 25% Rp, at least about 30% Rp, at least about 35% Rp, at least about 40% Rp, at least about 45% Rp, at least about 50% Rp, at least about 55% Rp, at least about 60% Rp, at least about 65% Rp, at least about 70% Rp, at least about 75% Rp, at least about 80% Rp, at least about 85% Rp, at least about 90% Rp, or at least about 95% Rp, with the remainder Sp, or about 100% Rp. In some embodiments, an ASO used in the methods of the disclosure, including, but not limited to, any of the ASOs set forth herein comprise a sequence with at least about 80%, 85%, 90%, 95%, 97%, or 100% sequence identity to a region comprising at least 8 contiguous nucleic acids of any one of SEQ ID NOs: 60-191, comprises about 10% to about 100% Rp, about 15% to about 100% Rp, about 20% to about 100% Rp, about 25% to about 100% Rp, about 30% to about 100% Rp, about 35% to about 100% Rp, about 40% to about 100% Rp, about 45% to about 100% Rp, about 50% to about 100% Rp, about 55% to about 100% Rp, about 60% to about 100% Rp, about 65% to about 100% Rp, about 70% to about 100% Rp, about 75% to about 100% Rp, about 80% to about 100% Rp, about 85% to about 100% Rp, about 90% to about 100% Rp, or about 95% to about 100% Rp, about 20% to about 80% Rp, about 25% to about 75% Rp, about 30% to about 70% Rp, about 40% to about 60% Rp, or about 45% to about 55% Rp, with the remainder Sp.

In some embodiments, an ASO used in the methods of the disclosure, including, but not limited to, any of the ASOs set forth herein comprise a sequence with at least about 80%, 85%, 90%, 95%, 97%, or 100% sequence identity to a region comprising at least 8 contiguous nucleic acids of any one of SEQ ID NOs: 60-191, comprises about 5-100% Sp, at least about 5% Sp, at least about 10% Sp, at least about 15% Sp, at least about 20% Sp, at least about 25% Sp, at least about 30% Sp, at least about 35% Sp, at least about 40% Sp, at least about 45% Sp, at least about 50% Sp, at least about 55% Sp, at least about 60% Sp, at least about 65% Sp, at least about 70% Sp, at least about 75% Sp, at least about 80% Sp, at least about 85% Sp, at least about 90% Sp, or at least about 95% Sp, with the remainder Rp, or about 100% Sp. In embodiments, an ASO used in the methods of the disclosure, including, but not limited to, any of the ASOs set forth herein comprise a sequence with at least about 80%, 85%, 90%, 95%, 97%, or 100% sequence identity to a region comprising at least 8 contiguous nucleic acids of any one of SEQ ID NOs: 60-191, comprises about 10% to about 100% Sp, about 15% to about 100% Sp, about 20% to about 100% Sp, about 25% to about 100% Sp, about 30% to about 100% Sp, about 35% to about 100% Sp, about 40% to about 100% Sp, about 45% to about 100% Sp, about 50% to about 100% Sp, about 55% to about 100% Sp, about 60% to about 100% Sp, about 65% to about 100% Sp, about 70% to about 100% Sp, about 75% to about 100% Sp, about 80% to about 100% Sp, about 85% to about 100% Sp, about 90% to about 100% Sp, or about 95% to about 100% Sp, about 20% to about 80% Sp, about 25% to about 75% Sp, about 30% to about 70% Sp, about 40% to about 60% Sp, or about 45% to about 55% Sp, with the remainder Rp.

Any of the ASOs described herein may contain a sugar moiety that comprises ribose or deoxyribose, as present in naturally occurring nucleotides, or a modified sugar moiety or sugar analog, including a morpholine ring. Non-limiting examples of modified sugar moieties include 2' substitutions such as 2'-O-methyl (2'-O-Me), 2'-O-methoxyethyl (2'MOE), 2'-O-aminoethyl, 2'F; N3'->P5' phosphoramidate, 2'dimethylaminooxyethoxy, 2'dimethylaminoethoxyethoxy, 2'-guanidinidium, 2'-O-guanidinium ethyl, carbamate modified sugars, and bicyclic modified sugars. In some embodiments, the sugar moiety modification is selected from 2'-O-Me, 2'F, and 2'MOE. In some embodiments, the sugar moiety modification is an extra bridge bond, such as in a locked nucleic acid (LNA). In some embodiments the sugar analog contains a morpholine ring, such as phosphorodiamidate morpholino (PMO). In some embodiments, the sugar moiety comprises a ribofuransyl or 2'deoxyribofuransyl modification. In some embodiments, the sugar moiety comprises 2'4'-constrained 2'O-methyloxyethyl (cMOE) modifications. In some embodiments, the sugar moiety comprises cEt 2', 4' constrained 2'-O ethyl BNA modifications. In some embodiments, the sugar moiety comprises tricycloDNA (tcDNA) modifications. In some embodiments, the sugar moiety comprises ethylene nucleic acid (ENA) modifications. In some embodiments, the sugar moiety comprises MCE modifications. Modifications are known in the art and described in the literature, *e.g*., by Jarver, et al., 2014, "A Chemical View of Oligonucleotides for Exon Skipping and Related Drug Applications," Nucleic Acid Therapeutics 24(1): 37-47, incorporated by reference for this purpose herein.

In some embodiments, each monomer of the ASO is modified in the same way, for example each linkage of the backbone of the ASO comprises a phosphorothioate linkage or each ribose sugar moiety comprises a 2'O-methyl modification. Such modifications that are present on each of the monomer components of an ASO are referred to as "uniform modifications." In some examples, a combination of different modifications may be desired, for example, an ASO may comprise a combination of phosphorodiamidate linkages and sugar moieties comprising morpholine rings (morpholinos). Combinations of different modifications to an ASO are referred to as "mixed modifications" or "mixed chemistries."

In some embodiments, the ASO comprises one or more backbone modifications. In some embodiments, the ASO comprises one or more sugar moiety modification. In some embodiments, the ASO comprises one or more backbone modifications and one or more sugar moiety modifications. In some embodiments, the ASO comprises a 2'MOE modification and a phosphorothioate backbone. In some embodiments, the ASO comprises a phosphorodiamidate morpholino (PMO). In some embodiments, the ASO comprises a peptide nucleic acid (PNA). Any of the ASOs or any component of an ASO (*e.g*., a nucleobase, sugar moiety, backbone) described herein may be modified in order to achieve desired properties or activities of the ASO or reduce undesired properties or activities of the ASO. For example, an ASO or one or more components of any ASO may be modified to enhance binding affinity to a target sequence on a pre-mRNA transcript; reduce binding to any non-target sequence; reduce degradation by cellular nucleases (*i.e*., RNase H); improve uptake of the ASO into a cell and/or into the nucleus of a cell; alter the pharmacokinetics or pharmacodynamics of the ASO; and/or modulate the half-life of the ASO.

In some embodiments, the ASOs are comprised of 2'-O-(2-methoxyethyl) (MOE) phosphorothioate-modified nucleotides. ASOs comprised of such nucleotides are especially well-suited to the methods disclosed herein; oligomers having such modifications have been shown to have significantly enhanced resistance to nuclease degradation and increased bioavailability, making them suitable, for example, for oral delivery in some embodiments described herein. See *e.g.,* Geary, et al., J Pharmacol Exp Ther. 2001; 296(3):890-7; Geary, et al., J Pharmacol Exp Ther. 2001; 296(3):898-904.

Methods of synthesizing ASOs will be known to one of skill in the art. Alternatively or in addition, ASOs may be obtained from a commercial source.

Unless specified otherwise, the left-hand end of single-stranded nucleic acid (*e.g*., pre-mRNA transcript, oligonucleotide, ASO, etc.) sequences is the 5' end and the left-hand direction of single or double-stranded nucleic acid sequences is referred to as the 5' direction. Similarly, the right-hand end or direction of a nucleic acid sequence (single or double stranded) is the 3' end or direction. Generally, a region or sequence that is 5' to a reference point in a nucleic acid is referred to as "upstream," and a region or sequence that is 3' to a reference point in a nucleic acid is referred to as "downstream." Generally, the 5' direction or end of an mRNA is where the initiation or start codon is located, while the 3' end or direction is where the termination codon is located. In some aspects, nucleotides that are upstream of a reference point in a nucleic acid may be designated by a negative number, while nucleotides that are downstream of a reference point may be designated by a positive number. For example, a reference point (*e.g*., an exon-exon junction in mRNA) may be designated as the "zero" site, and a nucleotide that is directly adjacent and upstream of the reference point is designated "minus one," *e.g*., "-1," while a nucleotide that is directly adjacent and downstream of the reference point is designated "plus one," *e.g.,* "+1."

In some embodiments, the ASOs are complementary to (and bind to) a targeted portion of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANKS, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is downstream (in the 3' direction) of the 5' splice site (or 3' end of the NIE) of the included exon in a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA (*e.g*., the direction designated by positive numbers relative to the 5' splice site). In some embodiments, the ASOs are complementary to a targeted portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is within the region about +1 to about +500 relative to the 5' splice site (or 3' end) of the included exon. In some embodiments, the ASOs may be complementary to a targeted portion of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is within the region between nucleotides +6 and +40,000 relative to the 5' splice site (or 3' end) of the included exon. In some aspects, the ASOs are complementary to a targeted portion that is within the region about +1 to about +40,000, about +1 to about +30,000, about +1 to about +20,000, about +1 to about +15,000, about +1 to about +10,000, about +1 to about +5,000, about +1 to about +4,000, about +1 to about +3,000, about +1 to about +2,000, about +1 to about +1,000, about +1 to about +500, about +1 to about +490, about +1 to about +480, about +1 to about +470, about +1 to about +460, about +1 to about +450, about +1 to about +440, about +1 to about +430, about +1 to about +420, about +1 to about +410, about +1 to about +400, about +1 to about +390, about +1 to about +380, about +1 to about +370, about +1 to about +360, about +1 to about +350, about +1 to about +340, about +1 to about +330, about +1 to about +320, about +1 to about +310, about +1 to about +300, about +1 to about +290, about +1 to about +280, about +1 to about +270, about +1 to about +260, about +1 to about +250, about +1 to about +240, about +1 to about +230, about +1 to about +220, about +1 to about +210, about +1 to about +200, about +1 to about +190, about +1 to about +180, about +1 to about +170, about +1 to about +160, about +1 to about +150, about +1 to about +140, about +1 to about +130, about +1 to about +120, about +1 to about +110, about +1 to about +100, about +1 to about +90, about +1 to about +80, about +1 to about +70, about +1 to about +60, about +1 to about +50, about +1 to about +40, about +1 to about +30, or about +1 to about +20 relative to 5' splice site (or 3' end) of the included exon. In some aspects, the ASOs are complementary to a targeted portion that is within the region from about +1 to about +100, from about +100 to about +200, from about +200 to about +300, from about +300 to about +400, or from about +400 to about +500 relative to 5' splice site (or 3' end) of the included exon.

In some embodiments, the ASOs are complementary to (and bind to) a targeted portion of a*ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANKS, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is upstream (in the 5' direction) of the 5' splice site (or 3' end) of the included exon in *a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA (*e.g*., the direction designated by negative numbers relative to the 5' splice site). In some embodiments, the ASOs are complementary to a targeted portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is within the region about -4 to about -270 relative to the 5' splice site (or 3'end) of the included exon. In some embodiments, the ASOs may be complementary to a targeted portion of *a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANKS, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is within the region between nucleotides -1 and - 40,000 relative to the 5' splice site (or 3' end) of the included exon. In some aspects, the ASOs are complementary to a targeted portion that is within the region about -1 to about -40,000, about -1 to about -30,000, about -1 to about -20,000, about -1 to about -15,000, about -1 to about - 10,000, about -1 to about -5,000, about -1 to about -4,000, about -1 to about -3,000, about -1 to about -2,000, about -1 to about -1,000, about -1 to about -500, about -1 to about -490, about -1 to about -480, about -1 to about -470, about -1 to about -460, about -1 to about -450, about -1 to about -440, about -1 to about -430, about -1 to about -420, about -1 to about -410, about -1 to about -400, about -1 to about -390, about -1 to about -380, about -1 to about -370, about -1 to about -360, about -1 to about -350, about -1 to about -340, about -1 to about -330, about -1 to about -320, about -1 to about -310, about -1 to about -300, about -1 to about -290, about -1 to about -280, about -1 to about -270, about -1 to about -260, about -1 to about -250, about -1 to about -240, about -1 to about -230, about -1 to about -220, about -1 to about -210, about -1 to about -200, about -1 to about -190, about -1 to about -180, about -1 to about -170, about -1 to about -160, about -1 to about -150, about -1 to about -140, about -1 to about -130, about -1 to about -120, about -1 to about -110, about -1 to about -100, about -1 to about -90, about -1 to about -80, about -1 to about -70, about -1 to about -60, about -1 to about -50, about -1 to about - 40, about -1 to about -30, or about -1 to about -20 relative to 5' splice site (or 3' end) of the included exon.

In some embodiments, the ASOs are complementary to a targeted region of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is upstream (in the 5' direction) of the 3' splice site (or 5' end) of the included exon in a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* NIE containing pre-mRNA (*e.g*., in the direction designated by negative numbers). In some embodiments, the ASOs are complementary to a targeted portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is within the region about -1 to about -500 relative to the 3' splice site (or 5' end) of the included exon. In some embodiments, the ASOs are complementary to a targeted portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is within the region -1 to -40,000 relative to the 3' splice site of the included exon. In some aspects, the ASOs are complementary to a targeted portion that is within the region about -1 to about - 40,000, about -1 to about -30,000, -1 to about -20,000, about -1 to about -15,000, about -1 to about -10,000, about -1 to about -5,000, about -1 to about -4,000, about -1 to about -3,000, about -1 to about -2,000, about -1 to about -1,000, about -1 to about -500, about -1 to about -490, about -1 to about -480, about -1 to about -470, about -1 to about -460, about -1 to about -450, about -1 to about -440, about -1 to about -430, about -1 to about -420, about -1 to about -410, about -1 to about -400, about -1 to about -390, about -1 to about -380, about -1 to about -370, about -1 to about -360, about -1 to about -350, about -1 to about -340, about -1 to about -330, about -1 to about -320, about -1 to about -310, about -1 to about -300, about -1 to about -290, about -1 to about -280, about -1 to about -270, about -1 to about -260, about -1 to about -250, about -1 to about -240, about -1 to about -230, about -1 to about -220, about -1 to about -210, about -1 to about -200, about -1 to about -190, about -1 to about -180, about -1 to about -170, about -1 to about -160, about -1 to about -150, about -1 to about -140, about -1 to about -130, about -1 to about -120, about -1 to about -110, about -1 to about -100, about -1 to about -90, about -1 to about -80, about -1 to about -70, about -1 to about -60, about -1 to about -50, about -1 to about -40, about -1 to about -30, or about -1 to about -20 relative to 3' splice site of the included exon. In some aspects, the ASOs are complementary to a targeted portion that is within the region from about -1 to about -100, from about -100 to about -200, from about -200 to about -300, from about -300 to about -400, or from about -400 to about -500 relative to 3' splice site of the included exon.

In some embodiments, the ASOs are complementary to a targeted region of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is downstream (in the 3' direction) of the 3' splice site (5' end) of the included exon in *a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* NIE containing pre-mRNA (e.g., in the direction designated by positive numbers). In some embodiments, the ASOs are complementary to a targeted portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANKS, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA that is within the region of about +1 to about +40,000 relative to the 3' splice site of the included exon. In some aspects, the ASOs are complementary to a targeted portion that is within the region about +1 to about +40,000, about +1 to about +30,000, about +1 to about +20,000, about +1 to about +15,000, about +1 to about +10,000, about +1 to about +5,000, about +1 to about +4,000, about +1 to about +3,000, about +1 to about +2,000, about +1 to about +1,000, about +1 to about +500, about +1 to about +490, about +1 to about +480, about +1 to about +470, about +1 to about +460, about +1 to about +450, about +1 to about +440, about +1 to about +430, about +1 to about +420, about +1 to about +410, about +1 to about +400, about +1 to about +390, about +1 to about +380, about +1 to about +370, about +1 to about +360, about +1 to about +350, about +1 to about +340, about +1 to about +330, about +1 to about +320, about +1 to about +310, about +1 to about +300, about +1 to about +290, about +1 to about +280, about +1 to about +270, about +1 to about +260, about +1 to about +250, about +1 to about +240, about +1 to about +230, about +1 to about +220, about +1 to about +210, about +1 to about +200, about +1 to about +190, about +1 to about +180, about +1 to about +170, about +1 to about +160, about +1 to about +150, about +1 to about +140, about +1 to about +130, about +1 to about +120, about +1 to about +110, about +1 to about +100, about +1 to about +90, about +1 to about +80, about +1 to about +70, about +1 to about +60, about +1 to about +50, about +1 to about +40, about +1 to about +30, or about +1 to about +20, or about +1 to about +10 relative to 3' splice site of the included exon.

In some embodiments, the targeted portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA is within the region +100 relative to the 5' splice site (3' end) of the included exon to -100 relative to the 3' splice site (5' end) of the included exon. In some embodiments, the targeted portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANKS, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA is within the NIE. In some embodiments, the target portion of the *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* NIE containing pre-mRNA comprises a pseudo-exon and intron boundary.

The ASOs may be of any length suitable for specific binding and effective enhancement of splicing. In some embodiments, the ASOs consist of 8 to 50 nucleobases. For example, the ASO may be 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, or 50 nucleobases in length. In some embodiments, the ASOs consist of more than 50 nucleobases. In some embodiments, the ASO is from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, 12 to 15 nucleobases, 13 to 50 nucleobases, 13 to 40 nucleobases, 13 to 35 nucleobases, 13 to 30 nucleobases, 13 to 25 nucleobases, 13 to 20 nucleobases, 14 to 50 nucleobases, 14 to 40 nucleobases, 14 to 35 nucleobases, 14 to 30 nucleobases, 14 to 25 nucleobases, 14 to 20 nucleobases, 15 to 50 nucleobases, 15 to 40 nucleobases, 15 to 35 nucleobases, 15 to 30 nucleobases, 15 to 25 nucleobases, 15 to 20 nucleobases, 20 to 50 nucleobases, 20 to 40 nucleobases, 20 to 35 nucleobases, 20 to 30 nucleobases, 20 to 25 nucleobases, 25 to 50 nucleobases, 25 to 40 nucleobases, 25 to 35 nucleobases, or 25 to 30 nucleobases in length. In some embodiments, the ASOs are 18 nucleotides in length. In some embodiments, the ASOs are 15 nucleotides in length. In some embodiments, the ASOs are 25 nucleotides in length.

In some embodiments, two or more ASOs with different chemistries but complementary to the same targeted portion of the NIE containing pre-mRNA are used. In some embodiments, two or more ASOs that are complementary to different targeted portions of the NIE containing pre-mRNA are used.

In some embodiments, the antisense oligonucleotides of the disclosure are chemically linked to one or more moieties or conjugates, e.g., a targeting moiety or other conjugate that enhances the activity or cellular uptake of the oligonucleotide. Such moieties include, but are not limited to, a lipid moiety, *e.g*., as a cholesterol moiety, a cholesteryl moiety, an aliphatic chain, *e.g*., dodecandiol or undecyl residues, a polyamine or a polyethylene glycol chain, or adamantane acetic acid. Oligonucleotides comprising lipophilic moieties and preparation methods have been described in the published literature. In embodiments, the antisense oligonucleotide is conjugated with a moiety including, but not limited to, an abasic nucleotide, a polyether, a polyamine, a polyamide, a peptides, a carbohydrate, *e.g*., N-acetylgalactosamine (GalNAc), N-Ac-Glucosamine (GluNAc), or mannose (*e.g*., mannose-6-phosphate), a lipid, or a polyhydrocarbon compound. Conjugates can be linked to one or more of any nucleotides comprising the antisense oligonucleotide at any of several positions on the sugar, base or phosphate group, as understood in the art and described in the literature, *e.g*., using a linker. Linkers can include a bivalent or trivalent branched linker. In embodiments, the conjugate is attached to the 3' end of the antisense oligonucleotide. Methods of preparing oligonucleotide conjugates are described, *e.g*., in U.S. Pat. No. 8,450,467, "Carbohydrate conjugates as delivery agents for oligonucleotides," incorporated by reference herein.

In some embodiments, the nucleic acid to be targeted by an ASO is a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA expressed in a cell, such as a eukaryotic cell. In some embodiments, the term "cell" may refer to a population of cells. In some embodiments, the cell is in a subject. In some embodiments, the cell is isolated from a subject. In some embodiments, the cell is *ex vivo.* In some embodiments, the cell is a condition or disease-relevant cell or a cell line. In some embodiments, the cell is *in vitro* (*e.g.,* in cell culture).

### Pharmaceutical Compositions

Pharmaceutical compositions or formulations comprising the agent, e.g., antisense oligonucleotide, of the described compositions and for use in any of the described methods can be prepared according to conventional techniques well known in the pharmaceutical industry and described in the published literature. In embodiments, a pharmaceutical composition or formulation for treating a subject comprises an effective amount of any antisense oligomer as described herein, or a pharmaceutically acceptable salt, solvate, hydrate or ester thereof. The pharmaceutical formulation comprising an antisense oligomer may further comprise a pharmaceutically acceptable excipient, diluent or carrier.

Pharmaceutically acceptable salts are suitable for use in contact with the tissues of humans and lower animals without undue toxicity, irritation, allergic response, etc., and are commensurate with a reasonable benefit/risk ratio. (See, *e.g.,* S. M. Berge, et al., J. Pharmaceutical Sciences, 66: 1-19 (1977), incorporated herein by reference for this purpose. The salts can be prepared in situ during the final isolation and purification of the compounds, or separately by reacting the free base form with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other documented methodologies such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

In some embodiments, the compositions are formulated into any of many possible dosage forms such as, but not limited to, tablets, capsules, gel capsules, liquid syrups, soft gels, suppositories, and enemas. In embodiments, the compositions are formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers. In embodiments, a pharmaceutical formulation or composition of the present disclosure includes, but is not limited to, a solution, emulsion, microemulsion, foam or liposome-containing formulation (*e*.*g.*, cationic or noncationic liposomes).

The pharmaceutical composition or formulation described herein may comprise one or more penetration enhancers, carriers, excipients or other active or inactive ingredients as appropriate and well known to those of skill in the art or described in the published literature. In embodiments, liposomes also include sterically stabilized liposomes, *e.g*., liposomes comprising one or more specialized lipids. These specialized lipids result in liposomes with enhanced circulation lifetimes. In embodiments, a sterically stabilized liposome comprises one or more glycolipids or is derivatized with one or more hydrophilic polymers, such as a polyethylene glycol (PEG) moiety. In some embodiments, a surfactant is included in the pharmaceutical formulation or compositions. The use of surfactants in drug products, formulations and emulsions is well known in the art. In embodiments, the present disclosure employs a penetration enhancer to effect the efficient delivery of the antisense oligonucleotide, *e.g*., to aid diffusion across cell membranes and /or enhance the permeability of a lipophilic drug. In some embodiments, the penetration enhancers are a surfactant, fatty acid, bile salt, chelating agent, or non-chelating nonsurfactant.

In some embodiments, the pharmaceutical formulation comprises multiple antisense oligonucleotides. In embodiments, the antisense oligonucleotide is administered in combination with another drug or therapeutic agent.

### Combination Therapies

In some embodiments, the ASOs disclosed in the present disclosure can be used in combination with one or more additional therapeutic agents. In some embodiments, the one or more additional therapeutic agents can comprise a small molecule. For example, the one or more additional therapeutic agents can comprise a small molecule described in WO2016128343A1, WO2017053982A1, WO2016196386A1, WO201428459A1, WO201524876A2, WO2013119916A2, and WO2014209841A2, which are incorporated by reference herein in their entirety. In some embodiments, the one or more additional therapeutic agents comprise an ASO that can be used to correct intron retention.

### Treatment of Subjects

Any of the compositions provided herein may be administered to an individual. "Individual" may be used interchangeably with "subject" or "patient." An individual may be a mammal, for example a human or animal such as a non-human primate, a rodent, a rabbit, a rat, a mouse, a horse, a donkey, a goat, a cat, a dog, a cow, a pig, or a sheep. In embodiments, the individual is a human. In embodiments, the individual is a fetus, an embryo, or a child. In other embodiments, the individual may be another eukaryotic organism, such as a plant. In some embodiments, the compositions provided herein are administered to a cell *ex vivo.*

In some embodiments, the compositions provided herein are administered to an individual as a method of treating a disease or disorder. In some embodiments, the individual has a genetic disease, such as any of the diseases described herein. In some embodiments, the individual is at risk of having a disease, such as any of the diseases described herein. In some embodiments, the individual is at increased risk of having a disease or disorder caused by insufficient amount of a protein or insufficient activity of a protein. If an individual is "at an increased risk" of having a disease or disorder caused insufficient amount of a protein or insufficient activity of a protein, the method involves preventative or prophylactic treatment. For example, an individual may be at an increased risk of having such a disease or disorder because of family history of the disease. Typically, individuals at an increased risk of having such a disease or disorder benefit from prophylactic treatment (*e.g*., by preventing or delaying the onset or progression of the disease or disorder). In embodiments, a fetus is treated in utero, *e.g*., by administering the ASO composition to the fetus directly or indirectly (*e.g*., via the mother).

Suitable routes for administration of ASOs of the present disclosure may vary depending on cell type to which delivery of the ASOs is desired. Multiple tissues and organs are affected by Dravet syndrome, with the brain being the most significantly affected tissue. The ASOs of the present disclosure may be administered to patients parenterally, for example, by intrathecal injection, intracerebroventricular injection, intraperitoneal injection, intramuscular injection, subcutaneous injection, or intravenous injection.

In embodiments, the antisense oligonucleotide is administered with one or more agents capable of promoting penetration of the subject antisense oligonucleotide across the blood-brain barrier by any method known in the art. For example, delivery of agents by administration of an adenovirus vector to motor neurons in muscle tissue is described in U.S. Pat. No. 6,632,427, "Adenoviral-vector-mediated gene transfer into medullary motor neurons," incorporated herein by reference. Delivery of vectors directly to the brain, *e.g*., the striatum, the thalamus, the hippocampus, or the substantia nigra, is described, *e.g*., in U.S. Pat. No. 6,756,523, "Adenovirus vectors for the transfer of foreign genes into cells of the central nervous system particularly in brain," incorporated herein by reference.

In some embodiments, the antisense oligonucleotides are linked or conjugated with agents that provide desirable pharmaceutical or pharmacodynamic properties. In embodiments, the antisense oligonucleotide is coupled to a substance, known in the art to promote penetration or transport across the blood-brain barrier, *e.g*., an antibody to the transferrin receptor. In embodiments, the antisense oligonucleotide is linked with a viral vector, *e.g*., to render the antisense compound more effective or increase transport across the blood-brain barrier. In embodiments, osmotic blood brain barrier disruption is assisted by infusion of sugars, *e.g*., meso erythritol, xylitol, D(+) galactose, D(+) lactose, D(+) xylose, dulcitol, myo-inositol, L(-) fructose, D(-) mannitol, D(+) glucose, D(+) arabinose, D(-) arabinose, cellobiose, D(+) maltose, D(+) raffinose, L(+) rhamnose, D(+) melibiose, D(-) ribose, adonitol, D(+) arabitol, L(-) arabitol, D(+) fucose, L(-) fucose, D(-) lyxose, L(+) lyxose, and L(-) lyxose, or amino acids, *e.g*., glutamine, lysine, arginine, asparagine, aspartic acid, cysteine, glutamic acid, glycine, histidine, leucine, methionine, phenylalanine, proline, serine, threonine, tyrosine, valine, and taurine. Methods and materials for enhancing blood brain barrier penetration are described, *e.g*., in U.S. Pat. No. 9,193,969, "Compositions and methods for selective delivery of oligonucleotide molecules to specific neuron types," U.S. Pat. No. 4,866,042, "Method for the delivery of genetic material across the blood brain barrier," U.S. Pat. No. 6,294,520, "Material for passage through the blood-brain barrier," and U.S. Pat. No. 6,936,589, "Parenteral delivery systems," each incorporated herein by reference.

In some embodiments, an ASO of the disclosure is coupled to a dopamine reuptake inhibitor (DRI), a selective serotonin reuptake inhibitor (SSRI), a noradrenaline reuptake inhibitor (NRI), a norepinephrine-dopamine reuptake inhibitor (NDRI), and a serotonin-norepinephrine-dopamine reuptake inhibitor (SNDRI), using methods described in, *e.g*., U.S. Pat. No. 9,193,969, incorporated herein by reference.

In some embodiments, subjects treated using the methods and compositions are evaluated for improvement in condition using any methods known and described in the art.

### Methods of Identifying Additional ASOs that Induce Exon Skipping

Also within the scope of the present disclosure are methods for identifying or determining ASOs that induce exon skipping of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, or SYNGAP1* NIE containing pre-mRNA. For example, a method can comprise identifying or determining ASOs that induce pseudo-exon skipping of a *ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, VCAN, AKT3, CD46, COL11A2, CR1, CRX, DNAJC8, MYH14, MYO6, NF2, SEMA3C, SEMA3D, EIF2AK3, ERN1, GUCY2F, SIRT3, NR1H4, STK11, PPARA, CYP2J2, of SYNGAP1* NIE containing pre-mRNA. ASOs that specifically hybridize to different nucleotides within the target region of the pre-mRNA may be screened to identify or determine ASOs that improve the rate and/or extent of splicing of the target intron. In some embodiments, the ASO may block or interfere with the binding site(s) of a splicing repressor(s)/silencer. Any method known in the art may be used to identify (determine) an ASO that when hybridized to the target region of the exon results in the desired effect (*e.g*., pseudo-exon skipping, protein or functional RNA production). These methods also can be used for identifying ASOs that induce exon skipping of the included exon by binding to a targeted region in an intron flanking the included exon, or in a non-included exon. An example of a method that may be used is provided below.

A round of screening, referred to as an ASO "walk" may be performed using ASOs that have been designed to hybridize to a target region of a pre-mRNA. For example, the ASOs used in the ASO walk can be tiled every 5 nucleotides from approximately 100 nucleotides upstream of the 3' splice site of the included exon (*e.g*., a portion of sequence of the exon located upstream of the target/included exon) to approximately 100 nucleotides downstream of the 3' splice site of the target/included exon and/or from approximately 100 nucleotides upstream of the 5' splice site of the included exon to approximately 100 nucleotides downstream of the 5' splice site of the target/included exon (*e.g*., a portion of sequence of the exon located downstream of the target/included exon). For example, a first ASO of 15 nucleotides in length may be designed to specifically hybridize to nucleotides +6 to +20 relative to the 3' splice site of the target/included exon. A second ASO may be designed to specifically hybridize to nucleotides +11 to +25 relative to the 3' splice site of the target/included exon. ASOs are designed as such spanning the target region of the pre-mRNA. In embodiments, the ASOs can be tiled more closely, *e.g*., every 1, 2, 3, or 4 nucleotides. Further, the ASOs can be tiled from 100 nucleotides downstream of the 5' splice site, to 100 nucleotides upstream of the 3' splice site. In some embodiments, the ASOs can be tiled from about 1,160 nucleotides upstream of the 3' splice site, to about 500 nucleotides downstream of the 5' splice site. In some embodiments, the ASOs can be tiled from about 500 nucleotides upstream of the 3' splice site, to about 1,920 nucleotides downstream of the 3' splice site.

One or more ASOs, or a control ASO (an ASO with a scrambled sequence, sequence that is not expected to hybridize to the target region) are delivered, for example by transfection, into a disease-relevant cell line that expresses the target pre-mRNA (*e.g*., a NIE containing pre-mRNA described herein). The exon skipping effects of each of the ASOs may be assessed by any method known in the art, for example by reverse transcriptase (RT)-PCR using primers that span the splice junction, as described in **Example 4.** A reduction or absence of a longer RT-PCR product produced using the primers spanning the region containing the included exon (e.g. including the flanking exons of the NIE) in ASO-treated cells as compared to in control ASO-treated cells indicates that splicing of the target NIE has been enhanced. In some embodiments, the exon skipping efficiency (or the splicing efficiency to splice the intron containing the NIE), the ratio of spliced to unspliced pre-mRNA, the rate of splicing, or the extent of splicing may be improved using the ASOs described herein. The amount of protein or functional RNA that is encoded by the target pre-mRNA can also be assessed to determine whether each ASO achieved the desired effect (*e.g*., enhanced functional protein production). Any method known in the art for assessing and/or quantifying protein production, such as Western blotting, flow cytometry, immunofluorescence microscopy, and ELISA, can be used.

A second round of screening, referred to as an ASO "micro-walk" may be performed using ASOs that have been designed to hybridize to a target region of a pre-mRNA. The ASOs used in the ASO micro-walk are tiled every 1 nucleotide to further refine the nucleotide acid sequence of the pre-mRNA that when hybridized with an ASO results in exon skipping (or enhanced splicing of NIE).

Regions defined by ASOs that promote splicing of the target intron are explored in greater detail by means of an ASO "micro-walk", involving ASOs spaced in 1-nt steps, as well as longer ASOs, typically 18-25 nt.

As described for the ASO walk above, the ASO micro-walk is performed by delivering one or more ASOs, or a control ASO (an ASO with a scrambled sequence, sequence that is not expected to hybridize to the target region), for example by transfection, into a disease-relevant cell line that expresses the target pre-mRNA. The splicing-inducing effects of each of the ASOs may be assessed by any method known in the art, for example by reverse transcriptase (RT)-PCR using primers that span the NIE, as described herein (see, *e.g*., **Example 4**). A reduction or absence of a longer RT-PCR product produced using the primers spanning the NIE in ASO-treated cells as compared to in control ASO-treated cells indicates that exon skipping (or splicing of the target intron containing an NIE) has been enhanced. In some embodiments, the exon skipping efficiency (or the splicing efficiency to splice the intron containing the NIE), the ratio of spliced to unspliced pre-mRNA, the rate of splicing, or the extent of splicing may be improved using the ASOs described herein. The amount of protein or functional RNA that is encoded by the target pre-mRNA can also be assessed to determine whether each ASO achieved the desired effect (*e.g*., enhanced functional protein production). Any method known in the art for assessing and/or quantifying protein production, such as Western blotting, flow cytometry, immunofluorescence microscopy, and ELISA, can be used.

ASOs that when hybridized to a region of a pre-mRNA result in exon skipping (or enhanced splicing of the intron containing a NIE) and increased protein production may be tested *in vivo* using animal models, for example transgenic mouse models in which the full-length human gene has been knocked-in or in humanized mouse models of disease. Suitable routes for administration of ASOs may vary depending on the disease and/or the cell types to which delivery of the ASOs is desired. ASOs may be administered, for example, by intrathecal injection, intracerebroventricular injection, intraperitoneal injection, intramuscular injection, subcutaneous injection, or intravenous injection. Following administration, the cells, tissues, and/or organs of the model animals may be assessed to determine the effect of the ASO treatment by for example evaluating splicing (e.g., efficiency, rate, extent) and protein production by methods known in the art and described herein. The animal models may also be any phenotypic or behavioral indication of the disease or disease severity.

Also within the scope of the present disclosure is a method to identify or validate an NMD-inducing exon in the presence of an NMD inhibitor, for example, cycloheximide. An exemplary method is provided in **Example 2.**

### SPECIFIC EMBODIMENTS (A)

Embodiment A1. A method of treating Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Pathway (eye); Pathway (central nervous system, epilepsy); Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; or 16p11.2 deletion syndrome in a subject in need thereof, by increasing the expression of a target protein or functional RNA by a cell of the subject, wherein the cell has an mRNA that contains a non-sense mediated RNA decay-inducing exon (NMD exon mRNA), and wherein the NMD exon mRNA encodes the target protein or functional RNA, the method comprising contacting the cell of the subject with a therapeutic agent that binds to a targeted portion of the NMD exon mRNA encoding the target protein or functional RNA, whereby the non-sense mediated RNA decay-inducing exon is excluded from the NMD exon mRNA encoding the target protein or functional RNA, thereby increasing the level of mRNA encoding the target protein or functional RNA, and increasing the expression of the target protein or functional RNA in the cell of the subj ect.

Embodiment A2. The method of embodiment A1, wherein the target protein is ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN.

Embodiment A3. A method of increasing expression of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein by a cell having an mRNA that contains a non-sense mediated RNA decay-inducing exon (NMD exon mRNA) and encodes ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, the method comprising contacting the cell an agent that binds to a targeted portion of the NMD exon mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, whereby the non-sense mediated RNA decay-inducing exon is excluded from the NMD exon mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, thereby increasing the level of mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, and increasing the expression of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein in the cell.

Embodiment A4. The method of any one of embodiments A1 to A3, wherein the non-sense mediated RNA decay-inducing exon is spliced out from the NMD exon mRNA encoding the target protein or functional RNA.

Embodiment A5. The method of any one of embodiments A1 to A4, wherein the target protein does not comprise an amino acid sequence encoded by the non-sense mediated RNA decay-inducing exon.

Embodiment A6. The method of any one of embodiments A1 to A5, wherein the target protein is a full-length target protein.

Embodiment A7. The method of any one of embodiments A1 to A6, wherein the agent is an antisense oligomer (ASO) complementary to the targeted portion of the NMD exon mRNA.

Embodiment A8. The method of any one of embodiments A1 to A7, wherein the mRNA is pre-mRNA.

Embodiment A9. The method of any one of embodiments A1 to A8, wherein the contacting comprises contacting the therapeutic agent to the mRNA, wherein the mRNA is in a nucleus of the cell.

Embodiment A10. The method of any one of embodiments A1 to A9, wherein the target protein or the functional RNA corrects a deficiency in the target protein or functional RNA in the subject.

Embodiment A11. The method of any one of embodiments A1 to A10, wherein the cells are in or from a subject with a condition caused by a deficient amount or activity of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein.

Embodiment A12. The method of any one of embodiments A1 to A11, wherein the deficient amount of the target protein is caused by haploinsufficiency of the target protein, wherein the subject has a first allele encoding a functional target protein, and a second allele from which the target protein is not produced, or a second allele encoding a nonfunctional target protein, and wherein the antisense oligomer binds to a targeted portion of a NMD exon mRNA transcribed from the first allele.

Embodiment A13. The method of any one of embodiments A1 to A11, wherein the subject has a condition caused by a disorder resulting from a deficiency in the amount or function of the target protein, wherein the subject has
(a) a first mutant allele from which
   (i) the target protein is produced at a reduced level compared to production from a wild-type allele,
   (ii)the target protein is produced in a form having reduced function compared to an equivalent wild-type protein, or
   (iii) the target protein is not produced, and
(b) a second mutant allele from which
   (i) the target protein is produced at a reduced level compared to production from a wild-type allele,
   (ii)the target protein is produced in a form having reduced function compared to an equivalent wild-type protein, or
   (iii) the target protein is not produced, and
wherein when the subject has a first mutant allele (a)(iii)., the second mutant allele is (b)(i) or (b)(ii) and wherein when the subject has a second mutant allele (b)(iii), the first mutant allele is (a)(i) or (a)(ii), and wherein the NMD exon mRNA is transcribed from either the first mutant allele that is (a)(i) or (a)(ii), and/or the second allele that is (b)(i) or (b)(ii).

Embodiment A14. The method of embodiment A13, wherein the target protein is produced in a form having reduced function compared to the equivalent wild-type protein.

Embodiment A15. The method of embodiment A13, wherein the target protein is produced in a form that is fully-functional compared to the equivalent wild-type protein.

Embodiment A16. The method of any one of embodiments A1 to A15, wherein the targeted portion of the NMD exon mRNA is within the non-sense mediated RNA decay-inducing exon.

Embodiment A17. The method of any one of embodiments A1 to A15, wherein the targeted portion of the NMD exon mRNA is either upstream or downstream of the non-sense mediated RNA decay-inducing exon.

Embodiment A18. The method of any one of embodiments A1 to A17, wherein the NMD exon mRNA comprises a sequence with at least about 80%, 85%, 90%, 95%, 97%, or 100% sequence identity to any one of SEQ ID NOs: 60-134.

Embodiment A19. The method of any one of embodiments A1 to A17, wherein the NMD exon mRNA is encoded by a genetic sequence with at least about 80%, 85%, 90%, 95%, 97%, or 100% sequence identity to SEQ ID NOs: 1-59.

Embodiment A20. The method of any one of embodiments A1 to A17, wherein the targeted portion of the NMD exon mRNA comprises a sequence with at least 80%, 85%, 90%, 95%, 97%, or 100% sequence identity to a region comprising at least 8 contiguous nucleic acids of SEQ ID NO: SEQ ID NOs: 60-134.

Embodiment A21. The method of any one of embodiments A1 to A20, wherein the agent is an antisense oligomer (ASO) and wherein the ASO comprises a sequence that is at least about 80%, 85%, 90%, 95%, 97%, or 100% complementary to at least 8 contiguous nucleic acids of SEQ ID NOs: 60-134.

Embodiment A22. The method of any one of embodiments A1 to A15, wherein the targeted portion of the NMD exon mRNA is within the non-sense mediated RNA decay-inducing exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 1 1x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN.

Embodiment A23. The method of any one of embodiments A1 to A15, wherein the targeted portion of the NMD exon mRNA is upstream or downstream of the non-sense mediated RNA decay-inducing exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN.

Embodiment A24. The method of any one of embodiments A1 to A15, wherein the targeted portion of the NMD exon mRNA comprises an exon-intron junction exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN.

Embodiment A25. The method of any one of embodiments A1 to A24, wherein the target protein produced is full-length protein, or wild-type protein.

Embodiment A26. The method of any one of embodiments A1 to A25, wherein the total amount of the mRNA encoding the target protein or functional RNA produced in the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1. 1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of the mRNA encoding the target protein or functional RNA produced in a control cell.

Embodiment A27. The method of any one of embodiments A1 to A25, wherein the total amount of the mRNA encoding the target protein or functional RNA produced in the cell contacted with the antisense oligomer is increased about 20% to about 300%, about 50% to about 300%, about 100% to about 300%, about 150% to about 300%, about 20% to about 50%, about 20% to about 100%, about 20% to about 150%, about 20% to about 200%, about 20% to about 250%, about 50% to about 100%, about 50% to about 150%, about 50% to about 200%, about 50% to about 250%, about 100% to about 150%, about 100% to about 200%, about 100% to about 250%, about 150% to about 200%, about 150% to about 250%, about 200% to about 250%, at least about 10%, at least about 20%, at least about 50%, at least about 100%, at least about 150%, at least about 200%, at least about 250%, or at least about 300%, compared to the total amount of the mRNA encoding the target protein or functional RNA produced in a control cell.

Embodiment A28. The method of one any of embodiments A1 to A25, wherein the total amount of target protein produced by the cell contacted with the antisense oligomer is increased about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to the total amount of target protein produced by a control cell.

Embodiment A29. The method of one any of embodiments A1 to A25, wherein the total amount of target protein produced by the cell contacted with the antisense oligomer is increased about 20% to about 300%, about 50% to about 300%, about 100% to about 300%, about 150% to about 300%, about 20% to about 50%, about 20% to about 100%, about 20% to about 150%, about 20% to about 200%, about 20% to about 250%, about 50% to about 100%, about 50% to about 150%, about 50% to about 200%, about 50% to about 250%, about 100% to about 150%, about 100% to about 200%, about 100% to about 250%, about 150% to about 200%, about 150% to about 250%, about 200% to about 250%, at least about 10%, at least about 20%, at least about 50%, at least about 100%, at least about 150%, at least about 200%, at least about 250%, or at least about 300%, compared to the total amount of target protein produced by a control cell.

Embodiment A30. The method of any one of embodiments A1 to 29, wherein the agent is an antisense oligomer (ASO) and wherein the antisense oligomer comprises a backbone modification comprising a phosphorothioate linkage or a phosphorodiamidate linkage.

Embodiment A31. The method of any one of embodiments A1 to A30, wherein the agent is an antisense oligomer (ASO) and wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.

Embodiment A32. The method of any one of embodiments A1 to A31, wherein the agent is an antisense oligomer (ASO) and wherein the antisense oligomer comprises at least one modified sugar moiety.

Embodiment A33. The method of embodiment A32, wherein each sugar moiety is a modified sugar moiety.

Embodiment A34. The method of any one of embodiments A1 to A33, wherein the agent is an antisense oligomer (ASO) and wherein the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.

Embodiment A35. The method of any one of embodiments A1 to A34, wherein the agent is an antisense oligomer (ASO) and wherein the antisense oligomer is at least 80%, at least 85%, at least 90%, at least 95%, at least 98%, at least 99%, or 100%, complementary to the targeted portion of the NMD exon mRNA encoding the protein.

Embodiment A36. The method of any one of embodiments A1 to A35, wherein the method further comprises assessing ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA or protein expression.

Embodiment A37. The method of any one of embodiments A1 to A36, wherein Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Pathway (eye); Pathway (central nervous system, epilepsy); Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; or 16p11.2 deletion syndrome is treated and wherein the antisense oligomer binds to a targeted portion of a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN NMD exon mRNA, wherein the targeted portion is within a sequence selected from SEQ ID NOs: 60-134.

Embodiment A38. The method of any one of embodiments A1 to A37, wherein the subject is a human.

Embodiment A39. The method of any one of embodiments A1 to A38, wherein the subject is a non-human animal.

Embodiment A40. The method of any one of embodiments A1 to A39, wherein the subject is a fetus, an embryo, or a child.

Embodiment A41. The method of any one of embodiments A1 to A40, wherein the cells are ex vivo.

Embodiment A42. The method of any one of embodiments A1 to A41, wherein the therapeutic agent is administered by intrathecal injection, intracerebroventricular injection, intraperitoneal injection, intramuscular injection, subcutaneous injection, or intravenous injection of the subject.

Embodiment A43. The method of any of embodiments A1 to A42, wherein the method further comprises administering a second therapeutic agent to the subject.

Embodiment A44. The method of embodiment A43, wherein the second therapeutic agent is a small molecule.

Embodiment A45. The method of embodiment A43, wherein the second therapeutic agent is an ASO.

Embodiment A46. The method of any one of embodiments A43 to A45, wherein the second therapeutic agent corrects intron retention.

Embodiment A47. An antisense oligomer as used in a method of any of embodiments A1 to A46.

Embodiment A48. An antisense oligomer comprising a sequence with at least about 80%, 85%, 90%, 95%, 97%, or 100% sequence identity to a region comprising at least 8 contiguous nucleic acids of SEQ ID NOs: 60-134.

Embodiment A49. A pharmaceutical composition comprising the antisense oligomer of embodiment A47 or A48 and an excipient.

Embodiment A50. A method of treating a subject in need thereof, comprising administering the pharmaceutical composition of embodiment A49 to the subject, wherein the administering is by intrathecal injection, intracerebroventricular injection, intraperitoneal injection, intramuscular injection, subcutaneous injection, or intravenous injection.

Embodiment A51. A composition comprising a therapeutic agent for use in a method of increasing expression of a target protein or a functional RNA by cells to treat Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Pathway (eye); Pathway (central nervous system, epilepsy); Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; or 16p11.2 deletion syndrome in a subject in need thereof, associated with a deficient protein or deficient functional RNA, wherein the deficient protein or deficient functional RNA is deficient in amount or activity in the subject, wherein the target protein is:
(a) the deficient protein; or
(b) a compensating protein which functionally augments or replaces the deficient protein or in the subject;

and wherein the functional RNA is:
   (c) the deficient RNA; or
   (d) a compensating functional RNA which functionally augments or replaces the deficient functional RNA in the subject;
wherein the therapeutic agent enhances exclusion of the non-sense mediated RNA decay-inducing exon from the NMD exon mRNA encoding the target protein or functional RNA, thereby increasing production or activity of the target protein or the functional RNA in the subj ect.

Embodiment A52. A composition comprising a therapeutic agent for use in a method of treating a condition associated with ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein in a subject in need thereof, the method comprising the step of increasing expression of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein by cells of the subject, wherein the cells have an mRNA that contains a non-sense mediated RNA decay-inducing exon (NMD exon mRNA) and encodes ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, the method comprising contacting the cells with the therapeutic agent, whereby the non-sense mediated RNA decay-inducing exon is excluded from the NMD exon mRNA that encodes ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, thereby increasing the level of mRNA encoding ABCB4, ASS1, ATP8B 1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, and increasing the expression of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein in the cells of the subject.

Embodiment A53. The composition of embodiment A52, wherein the condition is a disease or disorder.

Embodiment A54. The composition of embodiment A53, wherein the disease or disorder is Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Pathway (eye); Pathway (central nervous system, epilepsy); Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; or 16p11.2 deletion syndrome.

Embodiment A55. The composition of any one of embodiments A52 to 54, wherein the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein and NMD exon mRNA are encoded by the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN gene.

Embodiment A56. The composition of any one of embodiments A51 to A55, wherein the non-sense mediated RNA decay-inducing exon is spliced out from the NMD exon mRNA encoding the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein.

Embodiment A57. The composition of any one of embodiments A51 to A56, wherein the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein does not comprise an amino acid sequence encoded by the non-sense mediated RNA decay-inducing exon.

Embodiment A58. The composition of any one of embodiments A51 to A57, wherein the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein is a full-length ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein.

Embodiment A59. The composition of any one of embodiments A51 to A58, wherein the therapeutic agent is an antisense oligomer (ASO) complementary to the targeted portion of the NMD exon mRNA.

Embodiment A60. The composition of any of embodiments A51 to A59, wherein the therapeutic agent is an antisense oligomer (ASO) and wherein the antisense oligomer targets a portion of the NMD exon mRNA that is within the non-sense mediated RNA decay-inducing exon.

Embodiment A61. The composition of any of embodiments A51 to A59, wherein the therapeutic agent is an antisense oligomer (ASO) and wherein the antisense oligomer targets a portion of the NMD exon mRNA that is upstream or downstream of the non-sense mediated RNA decay-inducing exon.

Embodiment A62. The composition of any one of embodiments A51 to A61, wherein the target protein is ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN.

Embodiment A63. The composition of embodiment A62, wherein the NMD exon mRNA comprises a sequence with at least about 80%, 85%, 90%, 95%, 97%, or 100% sequence identity to any one of SEQ ID NOs: 60-134.

Embodiment A64. The composition of embodiment A62, wherein the NMD exon mRNA is encoded by a genetic sequence with at least about 80%, 85%, 90%, 95%, 97%, or 100% sequence identity to SEQ ID NO: 1-59.

Embodiment A65. The composition of embodiment A62, wherein the targeted portion of the NMD exon mRNA comprises a sequence with at least 80%, 85%, 90%, 95%, 97%, or 100% sequence identity to a region comprising at least 8 contiguous nucleic acids of SEQ ID NO: 60-134.

Embodiment A66. The composition of any one of embodiments A62 to A65, wherein the targeted portion of the NMD exon mRNA is within the non-sense mediated RNA decay-inducing exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBDS, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN.

Embodiment A67. The composition of any one of embodiments A62 to A65, wherein the targeted portion of the NMD exon mRNA is upstream or downstream of the non-sense mediated RNA decay-inducing exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN.

Embodiment A68. The composition of any one of embodiments A62 to A65, wherein the targeted portion of the NMD exon mRNA comprises an exon-intron junction of exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN.

Embodiment A69. The composition of any one of embodiments A62 to A68, wherein the therapeutic agent is an antisense oligomer (ASO) and wherein the ASO comprises a sequence that is at least about 80%, 85%, 90%, 95%, 97%, or 100% complementary to a region comprising at least 8 contiguous nucleic acids of SEQ ID NOs: 60-134.

Embodiment A70. The composition of any one of embodiments A51 to A69, wherein the mRNA encoding the target protein or functional RNA is a full-length mature mRNA, or a wild-type mature mRNA.

Embodiment A71. The composition of any one of embodiments A51 to A70, wherein the target protein produced is full-length protein, or wild-type protein.

Embodiment A72. The composition of any one of embodiments A51 to A71, wherein the therapeutic agent is an antisense oligomer (ASO) and wherein the antisense oligomer comprises a backbone modification comprising a phosphorothioate linkage or a phosphorodiamidate linkage.

Embodiment A73. The composition of any of embodiments A51 to A72, wherein the therapeutic agent is an antisense oligomer (ASO) and wherein said antisense oligomer is an antisense oligonucleotide.

Embodiment A74. The composition of any of embodiments A51 to A73, wherein the therapeutic agent is an antisense oligomer (ASO) and wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.

Embodiment A75. The composition of any of embodiments A51 to A74, wherein the therapeutic agent is an antisense oligomer (ASO) and wherein the antisense oligomer comprises at least one modified sugar moiety.

Embodiment A76. The composition of embodiment A75, wherein each sugar moiety is a modified sugar moiety.

Embodiment A77. The composition of any of embodiments A51 to A76, wherein the therapeutic agent is an antisense oligomer (ASO) and wherein the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.

Embodiment A78. A pharmaceutical composition comprising the therapeutic agent of any of the compositions of embodiments A51 to A77, and an excipient.

Embodiment A79. A method of treating a subject in need thereof, comprising administering the pharmaceutical composition of embodiment A78 to the subject, wherein the administering is by intrathecal injection, intracerebroventricular injection, intraperitoneal injection, intramuscular injection, subcutaneous injection, or intravenous injection.

Embodiment A80. The method of any of embodiments A51 to A79, wherein the method further comprises administering a second therapeutic agent to the subject.

Embodiment A81. The method of embodiment A80, wherein the second therapeutic agent is a small molecule.

Embodiment A82. The method of embodiment A80, wherein the second therapeutic agent is an ASO.

Embodiment A83. The method of any one of embodiments A80 to A82, wherein the second therapeutic agent corrects intron retention.

Embodiment A84. A pharmaceutical composition comprising: an antisense oligomer that hybridizes to a target sequence of a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCANmRNA transcript, wherein the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA transcript comprises a non-sense mediated RNA decay-inducing exon, wherein the antisense oligomer induces exclusion of the non-sense mediated RNA decay-inducing exon from the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA transcript; and a pharmaceutical acceptable excipient.

Embodiment A85. The pharmaceutical composition of embodiment A84, wherein the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA transcript is a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN NMD exon mRNA transcript.

Embodiment A86. The pharmaceutical composition of embodiment A84 or A85, wherein the targeted portion of the NMD exon mRNA is within the non-sense mediated RNA decay-inducing exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBDS, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN.

Embodiment A87. The pharmaceutical composition of embodiment A84 or A85, wherein the targeted portion of the NMD exon mRNA is upstream or downstream of the non-sense mediated RNA decay-inducing exon 8x of ABCB4, exon 9x of ASS1, exon 16x of ATP8B1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN.

Embodiment A88. The pharmaceutical composition of embodiment A84 or A85, wherein the targeted portion of the NMD exon mRNA comprises an exon-intron junction exon 8x of ABCB4, exon 9x of ASS1, exon 16x of ATP8B 1, exon 1x of BAG3, exon 31x of CACNA1A, exon 36x of CACNA1A, exon 37x of CACNA1A, exon 3x of CBS, exon 12x of CBS, exon 1x of CD55, exon 16x of CDKL5, exon 3x of CFH, exon 30x of CHD2, exon 4x of CHRNA7, exon 1x of CISD2, exon 15x of CLN3, exon 11x of COL4A3, exon 41x of COL4A3, exon 22x of COL4A4, exon 44x of COL4A4, exon 20x of DEPDC5, exon 2x of DHDDS, exon 3x of ELOVL4, exon 5x of FAH, exon 4x of FXN, exon 4x of GALE, exon 3x of GBE1, exon 11x of GRIN2A, exon 1x of GRN, exon 2x of HEXA, exon 2x of KANSL1, exon 1x of KCNQ2, exon 50x of KMT2D, exon 8x of MAPK3, exon 13x of MBD5, exon 2x of MECP2, exon 11x of MUT, exon 31x of NF1, exon 7x of NIPBL, exon 38x of NIPBL, exon 11x of NSD1, exon 6x of OPA1, exon 28x of OPA1, exon 1x of OPTN, exon 1x of PCCA, exon 5x of PCCB, exon 6x of PCCB, exon 4x of PKP2, exon 23x of PLCB1, exon 3x of PRPF3, exon 9x of PRPF31, exon 1x of RAI1, exon 5x of RBFOX2, exon 13x of SCN2A, exon 6x of SCN3A, exon 7x of SCN3A, exon 4x of SCN8A, exon 6x of SCN8A, exon 20x of SCN8A, exon 6x of SCN9A, exon 24x of SHANK3, exon 3x of SLC25A13, exon 6x of SLC25A13, exon 9x of SLC25A13, exon 11x of SLC25A13, exon 13x of SLC25A13, exon 1x of SLC6A1, exon 12x of SPTAN1, exon 10x of TEK, exon 15x of TEK, exon 1x of TOPORS, exon 11x of TSC2, exon 30x of TSC2, exon 1x of UBE3A, or exon 7x of VCAN.

Embodiment A89. The pharmaceutical composition of any one of embodiments A84 to A88, wherein the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN NMD exon mRNA transcript is encoded by a genetic sequence with at least about 80%, 85%, 90%, 95%, 96%, 97%, 98%, 99% or 100% sequence identity to any one of SEQ ID NOs: 1-59.

Embodiment A90. The pharmaceutical composition of embodiment A84 or A88, wherein the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN NMD exon mRNA transcript comprises a sequence with at least about 80%, 85%, 90%, 95%, 96%, 97%, 98%, 99% or 100% sequence identity to any one of SEQ ID NOs: 60-134.

Embodiment A91. The pharmaceutical composition of embodiment A84, wherein the antisense oligomer comprises a backbone modification comprising a phosphorothioate linkage or a phosphorodiamidate linkage.

Embodiment A92. The pharmaceutical composition of embodiment A84, wherein the antisense oligomer is an antisense oligonucleotide.

Embodiment A93. The pharmaceutical composition of embodiment A84, wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.

Embodiment A94. The pharmaceutical composition of embodiment A84, wherein the antisense oligomer comprises at least one modified sugar moiety.

Embodiment A95. The pharmaceutical composition of embodiment A84, wherein the antisense oligomer comprises from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.

Embodiment A96. The pharmaceutical composition of embodiment A84 or A85, wherein the antisense oligomer is at least 80%, at least 85%, at least 90%, at least 95%, at least 98%, at least 99%, or is 100% complementary to a targeted portion of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN NMD exon mRNA transcript.

Embodiment A97. The pharmaceutical composition of embodiment A84 or A85, wherein the targeted portion of the ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN NMD exon mRNA transcript is within a sequence selected from SEQ ID NOs: 60-134.

Embodiment A98. The pharmaceutical composition of embodiment A84, wherein the antisense oligomer comprises a nucleotide sequence that is at least about 80%, 85%, 90%, 91%, 92%, 93%, 94%, 95%, 96%, 97%, 98%, or 99% sequence identity to a region comprising at least 8 contiguous nucleic acids of SEQ ID NOs: 60-134.

Embodiment A99. The pharmaceutical composition of embodiment A84, wherein the antisense oligomer comprises a nucleotide sequence that is identical a region comprising at least 8 contiguous nucleic acids of SEQ ID NOs: 60-134.

Embodiment A100. The pharmaceutical composition of any one of the embodiments A84 to A99, wherein the pharmaceutical composition is formulated for intrathecal injection, intracerebroventricular injection, intraperitoneal injection, intramuscular injection, subcutaneous injection, or intravenous injection.

Embodiment A101. The method of any of embodiments A84 to A100, wherein the method further comprises administering a second therapeutic agent to the subject.

Embodiment A102. The method of embodiment A101, wherein the second therapeutic agent is a small molecule.

Embodiment A103. The method of embodiment A101, wherein the second therapeutic agent is an ASO.

Embodiment A104. The method of any one of embodiments A101 to A103, wherein the second therapeutic agent corrects intron retention.

Embodiment A105. A method of inducing processing of a deficient ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA transcript to facilitate removal of a non-sense mediated RNA decay-inducing exon to produce a fully processed ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA transcript that encodes a functional form of a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, the method comprising:
(a) contacting an antisense oligomer to a target cell of a subject;
(b) hybridizing the antisense oligomer to the deficient ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA transcript, wherein the deficient ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA transcript is capable of encoding the functional form of a ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein and comprises at least one non-sense mediated RNA decay-inducing exon;
(c) removing the at least one non-sense mediated RNA decay-inducing exon from the deficient ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA transcript to produce the fully processed ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA transcript that encodes the functional form of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein; and
(d) translating the functional form of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein from the fully processed ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN mRNA transcript.

Embodiment A106. A method of treating a subject having a condition caused by a deficient amount or activity of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein comprising administering to the subject an antisense oligomer comprising a nucleotide sequence with at least about 80%, 85%, 90%, 91%, 92%, 93%, 94%, 95%, 96%, 97%, 98%, or 99% sequence identity to a region comprising at least 8 contiguous nucleic acids of SEQ ID NOs: 60-134.

Embodiment A107. A method of treating Alport syndrome; Amyotrophic lateral sclerosis (ALS); Angelman syndrome; Aphasia, primary progressive; Arrhythmogenic right ventricular dysplasia 9; Autism spectrum disorder; Cardiomyopathy, dilated, 1HH; Myopathy, myofibrillar 6; Ceriod lipofuscinosis, neuronal, 3; Cholestasis, intrahepatic, of pregnancy, 3; Cholestasis, progressive familial intrahepatic 1; Citrullinemia Type II; Citrullinemia, Type 1; Cognitive impairment with or without cerebral ataxia; Cornelia de Lange; Early-onset epileptic encephalopathy; Epilepsy-aphasia spectrum; Epilepsy, generalized, with febrile seizures plus, type 7; Epileptic encephalopathy, childhood-onset; Epileptic encephalopathy, early infantile, 11; Epileptic encephalopathy, early infantile, 12; Epileptic encephalopathy, early infantile, 13; Epileptic encephalopathy, early infantile, 2; Episodic ataxia, type 2; Familial focal epilepsy; Febrile seizures, familial, 3B; Friedreich ataxia; Friedreich ataxia with retained reflexes; Galactose epimerase deficiency; Glaucoma 3, primary congenital, E; Glycogen storage disease IV; GRN-related frontotemporal dementia; Homocystinuria, B6-responsive and nonresponsive types; HSAN2D, autosomal recessive; Insensitivity to pain, congenital; Kabuki syndrome ; Koolen-De Vries syndrome; Mental retardation, autosomal dominant 1; Methyl malonic aciduria; Migraine, Myoclonic-atonic epilepsy; familial hemiplegic, 1; Neurofibromatosis type 1; Opioid addiction; Optic atrophy type 1; Pathway (eye); Pathway (central nervous system, epilepsy); Phelan-McDermid syndrome; Propionicacidemia; Primary open angle glaucoma; Propionic academia; Retinitis pigmentosa 11; Retinitis pigmentosa 18; Retinitis pigmentosa 31; Retinitis pigmentosa 59; Rett syndrome; Seizures, benign familial infantile, 3; Seizures, benign familial infantile, 5; Smith-Magenis syndrome; Sotos syndrome 1; Beckwith-Wiedemann syndrome; Stargardt disease 3; Tay-Sachs disease; Tuberous sclerosis; Tyrosinemia, type I; Wagner syndrome 1; West syndrome; Wolfram syndrome 2/NAFLD; 15q13.3 microdeletion; or 16p11.2 deletion syndrome in a subject in need thereof, by increasing the expression of a target protein or functional RNA by a cell of the subject, wherein the cell has an mRNA that contains a non-sense mediated RNA decay-inducing exon (NMD exon mRNA), and wherein the NMD exon mRNA encodes the target protein or functional RNA, the method comprising contacting the cell of the subject with a therapeutic agent that modulates splicing of the NMD exon mRNA encoding the target protein or functional RNA, whereby the non-sense mediated RNA decay-inducing exon is excluded from the NMD exon mRNA encoding the target protein or functional RNA, thereby increasing the level of mRNA encoding the target protein or functional RNA, and increasing the expression of the target protein or functional RNA in the cell of the subject.

Embodiment A108. A method of increasing expression of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein by a cell having an mRNA that contains a non-sense mediated RNA decay-inducing exon (NMD exon mRNA) and encodes ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, the method comprising contacting the cell with an agent that modulates splicing of the NMD exon mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, whereby the non-sense mediated RNA decay-inducing exon is excluded from the NMD exon mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, thereby increasing the level of mRNA encoding ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein, and increasing the expression of ABCB4, ASS1, ATP8B1, BAG3, CACNA1A, CBS, CD55, CDKL5, CFH, CHD2, CHRNA7, CISD2, CLN3, COL4A3, COL4A4, DEPDC5, DHDDS, ELOVL4, FAH, FXN, GALE, GBE1, GRIN2A, GRN, HEXA, KANSL1, KCNQ2, KMT2D, MAPK3, MBD5, MECP2, MUT, NF1, NIPBL, NSD1, OPA1, OPTN, PCCA, PCCB, PKP2, PLCB1, PRPF3, PRPF31, RAI1, RBFOX2, SCN2A, SCN3A, SCN8A, SCN9A, SHANK3, SLC25A13, SLC6A1, SPTAN1, TEK, TOPORS, TSC2, UBE3A, or VCAN protein in the cell.

Embodiment A109. The method of embodiment A107 or A108, wherein the agent
(a) binds to a targeted portion of the NMD exon mRNA encoding the target protein or functional RNA;
(b) binds to one or more components of a spliceosome; or
(c) a combination of (a) and (b).

### FURTHER SPECIFIC EMBODIMENTS

Embodiment 1. A method of modulating expression of a target protein, by a cell having an mRNA that comprises a non-sense mediated RNA decay-inducing exon (NMD exon) and encodes the target protein, the method comprising contacting a therapeutic agent to the cell, whereby the therapeutic agent modulates splicing of the NMD exon from the mRNA, thereby modulating level of processed mRNA encoding the target protein, and modulating the expression of the target protein in the cell, wherein the target protein is selected from the group consisting of: AKT3, CACNA1A, CBS, CD46, CFH, CHD2, CLN3, COL11A2, COL4A3, COL4A4, COL4A4, CR1, CRX, CYP2J2, DHDDS, DNAJC8, EIF2AK3, ERN1, GALE, GUCY2F, GUCY2F, HEXA, HEXA, MAPK3, MBD5, MBD5, MBD5, MUT, MYH14, MYO6, NF1, NF2, NIPBL, NR1H4, NSD1, NSD1, NSD1, NSD1, OPA1, OPA1, PCCA, PKP2, PPARA, PRPF3, PRPF3, SCN2A, SCN8A, SCN8A, SCN9A, SEMA3C, SEMA3D, SIRT3, STK11, STK11, SYNGAP1, TOPORS, and VCAN proteins.

Embodiment 2. A method of treating a disease or condition in a subject in need thereof by modulating expression of a target protein in a cell of the subject, comprising: contacting the cell of the subject with a therapeutic agent that modulates splicing of a non-sense mediated mRNA decay-inducing exon (NMD exon) from an mRNA in the cell, wherein the mRNA comprises the NMD exon and encodes the target protein, thereby modulating level of processed mRNA encoding the target protein, and modulating expression of the target protein in the cell of the subject, wherein the target protein is selected from the group consisting of: AKT3, CACNA1A, CBS, CD46, CFH, CHD2, CLN3, COL11A2, COL4A3, COL4A4, COL4A4, CR1, CRX, CYP2J2, DHDDS, DNAJC8, EIF2AK3, ERN1, GALE, GUCY2F, GUCY2F, HEXA, HEXA, MAPK3, MBD5, MBD5, MBD5, MUT, MYH14, MYO6, NF1, NF2, NIPBL, NR1H4, NSD1, NSD1, NSD1, NSD1, OPA1, OPA1, PCCA, PKP2, PPARA, PRPF3, PRPF3, SCN2A, SCN8A, SCN8A, SCN9A, SEMA3C, SEMA3D, SIRT3, STK11, STK11, SYNGAP1, TOPORS, and VCAN proteins.

Embodiment 3. The method of embodiment 1 or 2, wherein the therapeutic agent
(a) binds to a targeted portion of the mRNA encoding the target protein;
(b) modulates binding of a factor involved in splicing of the NMD exon; or
(c) a combination of (a) and (b).

Embodiment 4. The method of embodiment 3, wherein the therapeutic agent interferes with binding of the factor involved in splicing of the NMD exon to a region of the targeted portion.

Embodiment 5. The method of embodiment 3 or 4, wherein the targeted portion is proximal to the NMD exon.

Embodiment 6. The method of any one of embodiments 3 to 5, wherein the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream of 5' end of the NMD exon.

Embodiment 7. The method of any one of embodiments 3 to 6, wherein the targeted portion is at least about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides, about 40 nucleotides, about 30 nucleotides, about 20 nucleotides, about 10 nucleotides, about 5 nucleotides, about 4 nucleotides, about 2 nucleotides, about 1 nucleotides upstream of 5' end of the NMD exon.

Embodiment 8. The method of any one of embodiments 3 to 5, wherein the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream of 3' end of the NMD exon.

Embodiment 9. The method of any one of embodiments 3 to 5 or 8, wherein the targeted portion is at least about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides, about 40 nucleotides, about 30 nucleotides, about 20 nucleotides, about 10 nucleotides, about 5 nucleotides, about 4 nucleotides, about 2 nucleotides, about 1 nucleotides downstream of 3' end of the NMD exon.

Embodiment 10. The method of any one of embodiments 3 to 5, wherein the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream of genomic site selected from the group consisting of: GRCh38/ hg38: chr1 243564388; GRCh38/ hg38: chr19 13236618; GRCh38/ hg38: chr21 43060012; GRCh38/ hg38: chr1 207775610; GRCh38/ hg38: chr1 196675450; GRCh38/ hg38: chr15 92998149; GRCh38/ hg38: chr16 28479765; GRCh38/ hg38: chr6 33183698; GRCh38/ hg38: chr2 227296487; GRCh38/ hg38: chr2 227144833; GRCh38/ hg38: chr2 227015360; GRCh38/ hg38: chr1 207637688; GRCh38/ hg38: chr19 47835403; GRCh38/ hg38: chr1 59904516; GRCh38/ hg38: chr1 26442335; GRCh38/ hg38: chr1 28230252; GRCh38/ hg38: chr2 88582824; GRCh38/ hg38: chr17 64102804; GRCh38/ hg38: chr1 23798484; GRCh38/ hg38: chrX 109383446; GRCh38/ hg38: chrX 109439175; GRCh38/ hg38: chr15 72362466; GRCh38/ hg38: chr15 72345776; GRCh38/ hg38: chr16 30115645; GRCh38/ hg38: chr2 148460219; GRCh38/ hg38: chr2 148490695; GRCh38/ hg38: chr2 148505761; GRCh38/ hg38: chr6 49436597; GRCh38/ hg38: chr19 50230825; GRCh38/ hg38: chr6 75867431; GRCh38/ hg38: chr17 31249955; GRCh38/ hg38: chr22 29628658; GRCh38/ hg38: chr5 37048127; GRCh38/ hg38: chr12 100499841; GRCh38/ hg38: chr5 177169394; GRCh3 8/ hg38: chr5 177200761; GRCh3 8/ hg38: chr5 177247924; GRCh38/ hg38: chr5 177275947; GRCh38/ hg38: chr3 193628509; GRCh38/ hg38: chr3 193603500; GRCh38/ hg38: chr13 100305751; GRCh38/ hg38: chr12 32894778; GRCh38/ hg38: chr22 46203575; GRCh38/ hg38: chr1 150327557; GRCh38/ hg38: chr1 150330401; GRCh38/ hg38: chr2 165327155; GRCh38/ hg38: chr12 51688758; GRCh38/ hg38: chr12 51780202; GRCh38/ hg38: chr2 166304329; GRCh38/ hg38: chr7 80794957; GRCh38/ hg38: chr7 85059541; GRCh38/ hg38: chr11 226081; GRCh38/ hg38: chr19 1216268; GRCh38/ hg38: chr19 1221621; GRCh38/ hg38: chr6 33448789; GRCh38/ hg38: chr9 32551469; and GRCh38/ hg38: chr5 83544965.

Embodiment 11. The method of any one of embodiments 3 to 5 or 10, wherein the targeted portion is about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream of genomic site selected from the group consisting of: GRCh38/ hg38: chr1 243564388; GRCh38/ hg38: chr19 13236618; GRCh38/ hg38: chr21 43060012; GRCh38/ hg38: chr1 207775610; GRCh38/ hg38: chr1 196675450; GRCh38/ hg38: chr15 92998149; GRCh38/ hg38: chr16 28479765; GRCh38/ hg38: chr6 33183698; GRCh38/ hg38: chr2 227296487; GRCh38/ hg38: chr2 227144833; GRCh38/ hg38: chr2 227015360; GRCh38/ hg38: chr1 207637688; GRCh38/ hg38: chr19 47835403; GRCh38/ hg38: chr1 59904516; GRCh38/ hg38: chr1 26442335; GRCh38/ hg38: chr1 28230252; GRCh38/ hg38: chr2 88582824; GRCh38/ hg38: chr17 64102804; GRCh38/ hg38: chr1 23798484; GRCh38/ hg38: chrX 109383446; GRCh38/ hg38: chrX 109439175; GRCh38/ hg38: chr15 72362466; GRCh38/ hg38: chr15 72345776; GRCh38/ hg38: chr16 30115645; GRCh38/ hg38: chr2 148460219; GRCh38/ hg38: chr2 148490695; GRCh38/ hg38: chr2 148505761; GRCh38/ hg38: chr6 49436597; GRCh38/ hg38: chr19 50230825; GRCh38/ hg38: chr6 75867431; GRCh38/ hg38: chr17 31249955; GRCh38/ hg38: chr22 29628658; GRCh38/ hg38: chr5 37048127; GRCh38/ hg38: chr12 100499841; GRCh38/ hg38: chr5 177169394; GRCh38/ hg38: chr5 177200761; GRCh38/ hg38: chr5 177247924; GRCh38/ hg38: chr5 177275947; GRCh38/ hg38: chr3 193628509; GRCh38/ hg38: chr3 193603500; GRCh38/ hg38: chr13 100305751; GRCh38/ hg38: chr12 32894778; GRCh38/ hg38: chr22 46203575; GRCh38/ hg38: chr1 150327557; GRCh38/ hg38: chr1 150330401; GRCh38/ hg38: chr2 165327155; GRCh38/ hg38: chr12 51688758; GRCh38/ hg38: chr12 51780202; GRCh38/ hg38: chr2 166304329; GRCh38/ hg38: chr7 80794957; GRCh38/ hg38: chr7 85059541; GRCh38/ hg38: chr11 226081; GRCh38/ hg38: chr19 1216268; GRCh38/ hg38: chr19 1221621; GRCh38/ hg38: chr6 33448789; GRCh38/ hg38: chr9 32551469; and GRCh38/ hg38: chr5 83544965.

Embodiment 12. The method of any one of embodiments 3 to 5, wherein the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream of genomic site selected from the group consisting of: GRCh38/ hg38: chr1 243564285; GRCh38/ hg38: chr19 13236449; GRCh38/ hg38: chr21 43059730; GRCh38/ hg38: chr1 207775745; GRCh38/ hg38: chr1 196675529; GRCh38/ hg38: chr15 92998261; GRCh38/ hg38: chr16 28479644; GRCh38/ hg38: chr6 33183634; GRCh38/ hg38: chr2 227296526; GRCh38/ hg38: chr2 227144653; GRCh38/ hg38: chr2 227015283; GRCh38/ hg38: chr1 207637848; GRCh38/ hg38: chr19 47835579; GRCh38/ hg38: chr1 59904366; GRCh38/ hg38: chr1 26442372; GRCh38/ hg38: chr1 28230131; GRCh38/ hg38: chr2 88582755; GRCh38/ hg38: chr17 64102673; GRCh38/ hg38: chr1 23798311; GRCh38/ hg38: chrX 109383365; GRCh38/ hg38: chrX 109439038; GRCh38/ hg38: chr15 72362376; GRCh38/ hg38: chr15 72345677; GRCh38/ hg38: chr16 30115595; GRCh38/ hg38: chr2 148460304; GRCh38/ hg38: chr2 148490787; GRCh38/ hg38: chr2 148505830; GRCh38/ hg38: chr6 49436522; GRCh38/ hg38: chr19 50230999; GRCh38/ hg38: chr6 75867523; GRCh38/ hg38: chr17 31250125; GRCh38/ hg38: chr22 29628773; GRCh38/ hg38: chr5 37048354; GRCh38/ hg38: chr12 100500024; GRCh38/ hg38: chr5 177169559; GRCh38/ hg38: chr5 177200783; GRCh38/ hg38: chr5 177248079; GRCh38/ hg38: chr5 177276101; GRCh38/ hg38: chr3 193628616; GRCh38/ hg38: chr3 193603557; GRCh38/ hg38: chr13 100305834; GRCh38/ hg38: chr12 32894516; GRCh38/ hg38: chr22 46203752; GRCh38/ hg38: chr1 150327652; GRCh38/ hg38: chr1 150330498; GRCh38/ hg38: chr2 165327202; GRCh38/ hg38: chr12 51688849; GRCh38/ hg38: chr12 51780271; GRCh38/ hg38: chr2 166304238; GRCh38/ hg38: chr7 80794854; GRCh38/ hg38: chr7 85059498; GRCh38/ hg38: chr11 225673; GRCh38/ hg38: chr19 1216398; GRCh38/ hg38: chr19 1221846; GRCh38/ hg38: chr6 33448868; GRCh38/ hg38: chr9 32551365; and GRCh38/ hg38: chr5 83545070.

Embodiment 13. The method of any one of embodiments 3 to 5 or 12, wherein the targeted portion is about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream of genomic site selected from the group consisting of: GRCh38/ hg38: chr1 243564285; GRCh38/ hg38: chr19 13236449; GRCh38/ hg38: chr21 43059730; GRCh38/ hg38: chr1 207775745; GRCh38/ hg38: chr1 196675529; GRCh38/ hg38: chr15 92998261; GRCh38/ hg38: chr16 28479644; GRCh38/ hg38: chr6 33183634; GRCh38/ hg38: chr2 227296526; GRCh38/ hg38: chr2 227144653; GRCh38/ hg38: chr2 227015283; GRCh38/ hg38: chr1 207637848; GRCh38/ hg38: chr19 47835579; GRCh38/ hg38: chr1 59904366; GRCh38/ hg38: chr1 26442372; GRCh38/ hg38: chr1 28230131; GRCh38/ hg38: chr2 88582755; GRCh38/ hg38: chr17 64102673; GRCh38/ hg38: chr1 23798311; GRCh38/ hg38: chrX 109383365; GRCh38/ hg38: chrX 109439038; GRCh38/ hg38: chr15 72362376; GRCh38/ hg38: chr15 72345677; GRCh38/ hg38: chr16 30115595; GRCh38/ hg38: chr2 148460304; GRCh38/ hg38: chr2 148490787; GRCh38/ hg38: chr2 148505830; GRCh38/ hg38: chr6 49436522; GRCh38/ hg38: chr19 50230999; GRCh38/ hg38: chr6 75867523; GRCh38/ hg38: chr17 31250125; GRCh38/ hg38: chr22 29628773; GRCh38/ hg38: chr5 37048354; GRCh38/ hg38: chr12 100500024; GRCh38/ hg38: chr5 177169559; GRCh38/ hg38: chr5 177200783; GRCh38/ hg38: chr5 177248079; GRCh38/ hg38: chr5 177276101; GRCh38/ hg38: chr3 193628616; GRCh38/ hg38: chr3 193603557; GRCh38/ hg38: chr13 100305834; GRCh38/ hg38: chr12 32894516; GRCh38/ hg38: chr22 46203752; GRCh38/ hg38: chr1 150327652; GRCh38/ hg38: chr1 150330498; GRCh38/ hg38: chr2 165327202; GRCh38/ hg38: chr12 51688849; GRCh38/ hg38: chr12 51780271; GRCh38/ hg38: chr2 166304238; GRCh38/ hg38: chr7 80794854; GRCh38/ hg38: chr7 85059498; GRCh38/ hg38: chr11 225673; GRCh38/ hg38: chr19 1216398; GRCh38/ hg38: chr19 1221846; GRCh38/ hg38: chr6 33448868; GRCh38/ hg38: chr9 32551365; and GRCh38/ hg38: chr5 83545070.

Embodiment 14. The method of any one of embodiments 3 to 13, wherein the targeted portion is located in an intronic region between two canonical exonic regions of the mRNA encoding the target protein, and wherein the intronic region contains the NMD exon.

Embodiment 15. The method of any one of embodiments 3 to 14, wherein the targeted portion at least partially overlaps with the NMD exon.

Embodiment 16. The method of any one of embodiments 3 to 15, wherein the targeted portion at least partially overlaps with an intron upstream or downstream of the NMD exon.

Embodiment 17. The method of any one of embodiments 3 to 16, wherein the targeted portion comprises 5' NMD exon-intron junction or 3' NMD exon-intron junction.

Embodiment 18. The method of any one of embodiments 3 to 16, wherein the targeted portion is within the NMD exon.

Embodiment 19. The method of any one of embodiments 1 to 18, wherein the targeted portion comprises about 5, 6, 7, 8, 9, 10, 11, 12, 13, 14, 15, 16, 17, 18, 19, 20, 21, 22, 23, 24, 25, 26, 27, 28, 29, 30, or more consecutive nucleotides of the NMD exon.

Embodiment 20. The method of any one of embodiments 1 to 19, wherein the mRNA encoding the target protein comprises a sequence with at least about 80%, 85%, 90%, 95%, 97%, or 100% sequence identity to a sequence selected from the group consisting of SEQ ID NOs: 135-191 .

Embodiment 21. The method of any one of embodiments 1 to 20, wherein the mRNA encoding the target protein is encoded by a genetic sequence with at least about 80%, 85%, 90%, 95%, 97%, or 100% sequence identity to a sequence selected from the group consisting of SEQ ID NOs: 1-5, 12, 19-21, 25, 26, 28, 30, 33, 35, 38, 40, 41, 44, 45, 51, 53, 55-57, and 192-211.

Embodiment 22. The method of any one of embodiments 3 to 21, wherein the targeted portion of the mRNA comprises a sequence with at least 80%, 85%, 90%, 95%, 97%, or 100% sequence identity to a region comprising at least 8 contiguous nucleic acids of a sequence selected from the group consisting of SEQ ID NOs: 135-191 .

Embodiment 23. The method of any one of embodiments 1 to 22, wherein the agent is an antisense oligomer (ASO) and wherein the ASO comprises a sequence that is at least about 80%, 85%, 90%, 95%, 97%, or 100% complementary to at least 8 contiguous nucleic acids of a sequence selected from the group consisting of SEQ ID NOs: 135-191.

Embodiment 24. The method of any one of embodiments 3 to 23, wherein the targeted portion of the mRNA is within the non-sense mediated RNA decay-inducing exon selected from the group consisting of: GRCh38/ hg38: chr1 243564285 243564388 ; GRCh38/ hg38: chr19 13236449 13236618; GRCh38/ hg38: chr21 43059730 43060012; GRCh38/ hg38: chr1 207775610 207775745; GRCh38/ hg38: chr1 196675450 196675529; GRCh38/ hg38: chr15 92998149 92998261; GRCh38/ hg38: chr16 28479644 28479765; GRCh38/ hg38: chr6 33183634 33183698; GRCh38/ hg38: chr2 227296487 227296526; GRCh38/ hg38: chr2 227144653 227144833; GRCh38/ hg38: chr2 227015283 227015360; GRCh38/ hg38: chr1 207637688 207637848; GRCh38/ hg38: chr19 47835403 47835579; GRCh38/ hg38: chr1 59904366 59904516; GRCh38/ hg38: chr1 26442335 26442372; GRCh38/ hg38: chr1 28230131 28230252 ; GRCh38/ hg38: chr2 88582755 88582824; GRCh38/ hg38: chr17 64102673 64102804; GRCh38/ hg38: chr1 23798311 23798484; GRCh38/ hg38: chrX 109383365 109383446; GRCh38/ hg38: chrX 109439038 109439175; GRCh38/ hg38: chr15 72362376 72362466; GRCh38/ hg38: chr15 72345677 72345776; GRCh38/ hg38: chr16 30115595 30115645; GRCh38/ hg38: chr2 148460219 148460304; GRCh38/ hg38: chr2 148490695 148490787; GRCh38/ hg38: chr2 148505761 148505830; GRCh38/ hg38: chr6 49436522 49436597; GRCh38/ hg38: chr19 50230825 50230999; GRCh38/ hg38: chr6 75867431 75867523; GRCh38/ hg38: chr17 31249955 31250125; GRCh38/ hg38: chr22 29628658 29628773; GRCh38/ hg38: chr5 37048127 37048354; GRCh38/ hg38: chr12 100499841 100500024; GRCh38/ hg38: chr5 177169394 177169559; GRCh38/ hg38: chr5 177200761 177200783; GRCh38/ hg38: chr5 177247924 177248079; GRCh38/ hg38: chr5 177275947 177276101; GRCh38/ hg38: chr3 193628509 193628616; GRCh38/ hg38: chr3 193603500 193603557; GRCh38/ hg38: chr13 100305751 100305834; GRCh38/ hg38: chr12 32894516 32894778; GRCh38/ hg38: chr22 46203575 46203752; GRCh38/ hg38: chr1 150327557 150327652; GRCh38/ hg38: chr1 150330401 150330498; GRCh38/ hg38: chr2 165327155 165327202; GRCh38/ hg38: chr12 51688758 51688849; GRCh38/ hg38: chr12 51780202 51780271; GRCh38/ hg38: chr2 166304238 166304329; GRCh38/ hg38: chr7 80794854 80794957; GRCh38/ hg38: chr7 85059498 85059541; GRCh38/ hg38: chr11 225673 226081; GRCh38/ hg38: chr19 1216268 1216398; GRCh38/ hg38: chr19 1221621 1221846; GRCh38/ hg38: chr6 33448789 33448868; GRCh38/ hg38: chr9 32551365 32551469; and GRCh38/ hg38: chr5 83544965 83545070.

Embodiment 25. The method of any one of embodiments 3 to 23, wherein the targeted portion of the mRNA is upstream or downstream of the non-sense mediated RNA decay-inducing exon selected from the group consisting of: GRCh38/ hg38: chr1 243564285 243564388 ; GRCh38/ hg38: chr19 13236449 13236618; GRCh38/ hg38: chr21 43059730 43060012; GRCh38/ hg38: chr1 207775610 207775745; GRCh38/ hg38: chr1 196675450 196675529; GRCh38/ hg38: chr15 92998149 92998261; GRCh38/ hg38: chr16 28479644 28479765; GRCh38/ hg38: chr6 33183634 33183698; GRCh38/ hg38: chr2 227296487 227296526; GRCh38/ hg38: chr2 227144653 227144833; GRCh38/ hg38: chr2 227015283 227015360; GRCh38/ hg38: chr1 207637688 207637848; GRCh38/ hg38: chr19 47835403 47835579; GRCh38/ hg38: chr1 59904366 59904516; GRCh38/ hg38: chr1 26442335 26442372; GRCh38/ hg38: chr1 28230131 28230252 ; GRCh38/ hg38: chr2 88582755 88582824; GRCh38/ hg38: chr17 64102673 64102804; GRCh38/ hg38: chr1 23798311 23798484; GRCh38/ hg38: chrX 109383365 109383446; GRCh38/ hg38: chrX 109439038 109439175; GRCh38/ hg38: chr15 72362376 72362466; GRCh38/ hg38: chr15 72345677 72345776; GRCh38/ hg38: chr16 30115595 30115645; GRCh38/ hg38: chr2 148460219 148460304; GRCh38/ hg38: chr2 148490695 148490787; GRCh38/ hg38: chr2 148505761 148505830; GRCh38/ hg38: chr6 49436522 49436597; GRCh38/ hg38: chr19 50230825 50230999; GRCh38/ hg38: chr6 75867431 75867523; GRCh38/ hg38: chr17 31249955 31250125; GRCh38/ hg38: chr22 29628658 29628773; GRCh38/ hg38: chr5 37048127 37048354; GRCh38/ hg38: chr12 100499841 100500024; GRCh38/ hg38: chr5 177169394 177169559; GRCh38/ hg38: chr5 177200761 177200783; GRCh38/ hg38: chr5 177247924 177248079; GRCh38/ hg38: chr5 177275947 177276101; GRCh38/ hg38: chr3 193628509 193628616; GRCh38/ hg38: chr3 193603500 193603557; GRCh38/ hg38: chr13 100305751 100305834; GRCh38/ hg38: chr12 32894516 32894778; GRCh38/ hg38: chr22 46203575 46203752; GRCh38/ hg38: chr1 150327557 150327652; GRCh38/ hg38: chr1 150330401 150330498; GRCh38/ hg38: chr2 165327155 165327202; GRCh38/ hg38: chr12 51688758 51688849; GRCh38/ hg38: chr12 51780202 51780271; GRCh38/ hg38: chr2 166304238 166304329; GRCh38/ hg38: chr7 80794854 80794957; GRCh38/ hg38: chr7 85059498 85059541; GRCh38/ hg38: chr11 225673 226081; GRCh38/ hg38: chr19 1216268 1216398; GRCh38/ hg38: chr19 1221621 1221846; GRCh38/ hg38: chr6 33448789 33448868; GRCh38/ hg38: chr9 32551365 32551469; and GRCh38/ hg38: chr5 83544965 83545070.

Embodiment 26. The method of any one of embodiments 3 to 23, wherein the targeted portion of the mRNA comprises an exon-intron junction of exon selected from the group consisting of: GRCh38/ hg38: chr1 243564285 243564388; GRCh38/ hg38: chr19 13236449 13236618; GRCh38/ hg38: chr21 43059730 43060012; GRCh38/ hg38: chr1 207775610 207775745; GRCh38/ hg38: chr1 196675450 196675529; GRCh38/ hg38: chr15 92998149 92998261; GRCh38/ hg38: chr16 28479644 28479765; GRCh38/ hg38: chr6 33183634 33183698; GRCh38/ hg38: chr2 227296487 227296526; GRCh38/ hg38: chr2 227144653 227144833; GRCh38/ hg38: chr2 227015283 227015360; GRCh38/ hg38: chr1 207637688 207637848; GRCh38/ hg38: chr19 47835403 47835579; GRCh38/ hg38: chr1 59904366 59904516; GRCh38/ hg38: chr1 26442335 26442372; GRCh38/hg38: chr1 28230131 28230252 ; GRCh38/ hg38: chr2 88582755 88582824; GRCh38/ hg38: chr17 64102673 64102804; GRCh38/ hg38: chr1 23798311 23798484; GRCh38/ hg38: chrX 109383365 109383446; GRCh38/ hg38: chrX 109439038 109439175; GRCh38/ hg38: chr15 72362376 72362466; GRCh38/ hg38: chr15 72345677 72345776; GRCh38/ hg38: chr16 30115595 30115645; GRCh38/ hg38: chr2 148460219 148460304; GRCh38/ hg38: chr2 148490695 148490787; GRCh38/ hg38: chr2 148505761 148505830; GRCh38/ hg38: chr6 49436522 49436597; GRCh38/ hg38: chr19 50230825 50230999; GRCh38/ hg38: chr6 75867431 75867523; GRCh38/ hg38: chr17 31249955 31250125; GRCh38/ hg38: chr22 29628658 29628773; GRCh38/ hg38: chr5 37048127 37048354; GRCh38/ hg38: chr12 100499841 100500024; GRCh38/ hg38: chr5 177169394 177169559; GRCh38/ hg38: chr5 177200761 177200783; GRCh38/ hg38: chr5 177247924 177248079; GRCh38/ hg38: chr5 177275947 177276101; GRCh38/ hg38: chr3 193628509 193628616; GRCh38/ hg38: chr3 193603500 193603557; GRCh38/ hg38: chr13 100305751 100305834; GRCh38/ hg38: chr12 32894516 32894778; GRCh38/ hg38: chr22 46203575 46203752; GRCh38/ hg38: chr1 150327557 150327652; GRCh38/ hg38: chr1 150330401 150330498; GRCh38/ hg38: chr2 165327155 165327202; GRCh38/ hg38: chr12 51688758 51688849; GRCh38/ hg38: chr12 51780202 51780271; GRCh38/ hg38: chr2 166304238 166304329; GRCh38/ hg38: chr7 80794854 80794957; GRCh38/ hg38: chr7 85059498 85059541; GRCh38/ hg38: chr11 225673 226081; GRCh38/ hg38: chr19 1216268 1216398; GRCh38/ hg38: chr19 1221621 1221846; GRCh38/ hg38: chr6 33448789 33448868; GRCh38/ hg38: chr9 32551365 32551469; and GRCh38/ hg38: chr5 83544965 83545070.

Embodiment 27. The method of any one of embodiments 1 to 26, wherein the target protein produced is a full-length protein or a wild-type protein.

Embodiment 28. The method of any one of embodiments 1 to 27, wherein the therapeutic agent promotes exclusion of the NMD exon from the processed mRNA encoding the target protein.

Embodiment 29. The method of embodiment 28, wherein exclusion of the NMD exon from the processed mRNA encoding the target protein in the cell contacted with the therapeutic agent is increased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to exclusion of the NMD exon from the processed mRNA encoding the target protein in a control cell.

Embodiment 30. The method of embodiment 28 or 29, wherein the therapeutic agent increases the level of the processed mRNA encoding the target protein in the cell.

Embodiment 31. The method of any one of embodiments 28 to 30, wherein the level of the processed mRNA encoding the target protein produced in the cell contacted with the therapeutic agent is increased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the processed mRNA encoding the target protein in a control cell.

Embodiment 32. The method of any one of embodiments 28 to 31, wherein the therapeutic agent increases the expression of the target protein in the cell.

Embodiment 33. The method of any one of embodiments 28 to 32, wherein a level of the target protein produced in the cell contacted with the therapeutic agent is increased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the target protein produced in a control cell.

Embodiment 34. The method of any one of embodiments 2 to 33, wherein the disease or condition is induced by a loss-of-function mutation in the target protein.

Embodiment 35. The method of embodiment 34, wherein the disease or condition is associated with haploinsufficiency of a gene encoding the target protein, and wherein the subject has a first allele encoding a functional target protein, and a second allele from which the target protein is not produced or produced at a reduced level, or a second allele encoding a nonfunctional target protein or a partially functional target protein.

Embodiment 36. The method of any one of embodiments 2 to 35, wherein the disease or condition is selected from the group consisting of: Sotos syndrome 1; Beckwith-Wiedemann syndrome; Migraine, familial hemiplegic, 1; Episodic ataxia, type 2; Epileptic encephalopathy, childhood-onset; Wagner syndrome 1; Optic atrophy type 1; Alport syndrome; Arrhythmogenic right ventricular dysplasia 9; Neurofibromatosis type 1; Epileptic encephalopathy, early infantile, 11; Seizures, benign familial infantile, 3; Cognitive impairment with or without cerebellar ataxia; Epileptic encephalopathy, early infantile, 13; Seizures, benign familial infantile, 5; Pathway (CNS); 16p11.2 deletion syndrome?; Mental retardation, autosomal dominant 1; Retinitis pigmentosa 18; Retinitis pigmentosa 31; Deafness, autosomal dominant 13; Cone-rod retinal dystrophy-2; Deafness, autosomal dominant 4A; Peripheral neuropathy, myopathy, hoarseness, and hearing loss ; Deafness, autosomal dominant 22; Neurofibromatosis type 2; Mental retardation, autosomal dominant 5; Epilepsy, generalized, with febrile seizures plus, type 7; and Febrile seizures, familial, 3B.

Embodiment 37. The method of any one of embodiments 2 to 36, wherein the disease or condition is associated with an autosomal recessive mutation of a gene encoding the target protein, wherein the subject has a first allele encoding from which:
(i) the target protein is not produced or produced at a reduced level compared to a wild-type allele; or
(ii) the target protein produced is nonfunctional or partially functional compared to a wild-type allele, and
a second allele from which:
(iii) the target protein is produced at a reduced level compared to a wild-type allele and the target protein produced is at least partially functional compared to a wild-type allele; or
(iv) the target protein produced is partially functional compared to a wild-type allele.

Embodiment 38. The method of embodiment 37, wherein the disease or condition is selected from the group consisting of: Alport syndrome; Ceroid lipofuscinosis, neuronal, 3; Galactose epimerase deficiency; Homocystinuria, B6-responsive and nonresponsive types; Methyl Malonic Aciduria; Propionic acidemia; Retinitis pigmentosa 59; Tay-Sachs disease; Insensitivity to pain, congenital; and HSAN2D, autosomal recessive.

Embodiment 39. The method of any one of embodiments 34 to 39, wherein the therapeutic agent promotes exclusion of the NMD exon from the processed mRNA encoding the target protein and increases the expression of the target protein in the cell.

Embodiment 40. The method of any one of embodiments 1 to 27, wherein the therapeutic agent inhibits exclusion of the NMD exon from the processed mRNA encoding the target protein.

Embodiment 41. The method of embodiment 40, wherein exclusion of the NMD exon from the processed mRNA encoding the target protein in the cell contacted with the therapeutic agent is decreased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to exclusion of the NMD exon from the processed mRNA encoding the target protein in a control cell.

Embodiment 42. The method of embodiment 40 or 41, wherein the therapeutic agent decreases the level of the processed mRNA encoding the target protein in the cell.

Embodiment 43. The method of any one of embodiments 40 to 42, wherein the level of the processed mRNA encoding the target protein in the cell contacted with the therapeutic agent is decreased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the processed mRNA encoding the target protein in a control cell.

Embodiment 44. The method of any one of embodiments 40 to 43, wherein the therapeutic agent decreases the expression of the target protein in the cell.

Embodiment 45. The method of any one of embodiments 40 to 44, wherein a level of the target protein produced in the cell contacted with the therapeutic agent is decreased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the target protein produced in a control cell.

Embodiment 46. The method of any one of embodiments 2 to 27 or 40 to 45, wherein the disease or condition is induced by a gain-of-function mutation in the target protein

Embodiment 47. The method of embodiment 46, wherein the subject has an allele from which the target protein is produced at an increased level, or an allele encoding a mutant target protein that exhibits increased activity in the cell.

Embodiment 48. The method of embodiment 46 or 47, wherein the therapeutic agent inhibits exclusion of the NMD exon from the processed mRNA encoding the target protein and decreases the expression of the target protein in the cell.

Embodiment 49. The method of embodiment 40, wherein the target protein comprises SCN8A.

Embodiment 50. The method of embodiment 49, wherein the disease or condition comprises a central nervous system disease.

Embodiment 51. The method of embodiment 50, wherein the disease or condition comprises epilepsy.

Embodiment 52. The method of embodiment 51, wherein the disease or condition comprises Dravet syndrome.

Embodiment 53. The method of any one of embodiments 1 to 52, wherein the therapeutic agent is an antisense oligomer (ASO) and wherein the antisense oligomer comprises a backbone modification comprising a phosphorothioate linkage or a phosphorodiamidate linkage.

Embodiment 54. The method of any one of embodiments 1 to 53, wherein the therapeutic agent is an antisense oligomer (ASO) and wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.

Embodiment 55. The method of any one of embodiments 1 to 54, wherein the therapeutic agent is an antisense oligomer (ASO) and wherein the antisense oligomer comprises at least one modified sugar moiety.

Embodiment 56. The method of embodiment 55, wherein each sugar moiety is a modified sugar moiety.

Embodiment 57. The method of any one of embodiments 1 to 56, wherein the therapeutic agent is an antisense oligomer (ASO) and wherein the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.

Embodiment 58. The method of any one of embodiments 3 to 57, wherein the therapeutic agent is an antisense oligomer (ASO) and wherein the antisense oligomer is at least 80%, at least 85%, at least 90%, at least 95%, at least 98%, at least 99%, or 100%, complementary to the targeted portion of the mRNA.

Embodiment 59. The method of any one of embodiments 1 to 58, wherein the method further comprises assessing mRNA level or expression level of the target protein.

Embodiment 60. The method of any one of embodiments 1 to 59, wherein the subject is a human.

Embodiment 61. The method of any one of embodiments 1 to 59, wherein the subject is a non-human animal.

Embodiment 62. The method of any one of embodiments 2 to 60, wherein the subject is a fetus, an embryo, or a child.

Embodiment 63. The method of any one of embodiments 1 to 62, wherein the cells are ex vivo.

Embodiment 64. The method of any one of embodiments 2 to 62, wherein the therapeutic agent is administered by intrathecal injection, intracerebroventricular injection, intraperitoneal injection, intramuscular injection, subcutaneous injection, intravitreal, or intravenous injection of the subject.

Embodiment 65. The method of any one of embodiments 2 to 62 or 64, wherein the method further comprises administering a second therapeutic agent to the subject.

Embodiment 66. The method of any one of embodiments 1 to 65, wherein the second therapeutic agent is a small molecule.

Embodiment 67. The method of any one of embodiments 1 to 65, wherein the second therapeutic agent is an antisense oligomer.

Embodiment 68. The method of any one of embodiments 1 to 67, wherein the second therapeutic agent corrects intron retention.

Embodiment 69. The method of any one of embodiments 2 to 68, wherein the disease or condition is selected from the group consisting of: 16p11.2 deletion syndrome; Alport syndrome; Arrhythmogenic right ventricular dysplasia 9; Ceroid lipofuscinosis, neuronal, 3; Cognitive impairment with or without cerebellar ataxia; Epileptic encephalopathy, early infantile, 13; Seizures, benign familial infantile, 5; Cone-rod retinal dystrophy-2; Cornelia de Lange; Deafness, autosomal dominant 13; Deafness, autosomal dominant 4A; Peripheral neuropathy, myopathy, hoarseness, and hearing loss ; Epilepsy, generalized, with febrile seizures plus, type 7; Febrile seizures, familial, 3B; Insensitivity to pain, congenital; HSAN2D, autosomal recessive; Epileptic encephalopathy, childhood-onset; Epileptic encephalopathy, early infantile, 11; Seizures, benign familial infantile, 3; Galactose epimerase deficiency; Homocystinuria, B6-responsive and nonresponsive types; Mental retardation, autosomal dominant 1; Mental retardation, autosomal dominant 5; Methyl Malonic Aciduria; Migraine, familial hemiplegic, 1; Episodic ataxia, type 2; NASH; Neurofibromatosis type 1; Neurofibromatosis type 2; Optic atrophy type 1; Propionic acidemia; Retinitis pigmentosa 18; Sotos syndrome 1; Beckwith-Wiedemann syndrome; Tay-Sachs disease; and Wagner syndrome 1.

### EXAMPLES

The present disclosure will be more specifically illustrated by the following Examples. However, it should be understood that the present disclosure is not limited by these examples in any manner.

### Example 1: Identification of NMD-inducing Exon Inclusion Events in Transcripts by RNAseq using Next Generation Sequencing

Whole transcriptome shotgun sequencing is carried out using next generation sequencing to reveal a snapshot of transcripts produced by the genes described herein to identify NIE inclusion events. For this purpose, polyA+ RNA from nuclear and cytoplasmic fractions of human cells is isolated and cDNA libraries are constructed using Illumina's TruSeq Stranded mRNA library Prep Kit. The libraries are pair-end sequenced resulting in 100-nucleotide reads that are mapped to the human genome (Feb. 2009, GRCh37/hg19 assembly). **FIGs. 2-58** depict identification of different exemplary nonsense-mediated mRNA decay (NMD)-inducing exons in various genes.

Exemplary genes and intron sequences are summarized in **Table 1** and **Table 2** (SEQ ID NOs indicate the corresponding nucleotide sequences represented by the Gene ID Nos). The sequence for each intron is summarized in **Table 3** and **Table 4.**

**Table 1. List of exemplary target gene sequences.**

| **Gene Symbol** | **Gene ID No.** | **SEQ ID No.** | **Disease** | **OMIM** | **Genetics** | **Introns** |
|---|---|---|---|---|---|---|
| NIPBL | 71175 | 1 | Cornelia de Lange | 122470 | Haploinsufficien t | ENST00000282516.12:38 |
| | | | | | | ENST00000282516.12:7 |
| NSD1 | 18193 | 2 | Sotos syndrome 1; Beckwith-Wiedemann syndrome | 117550; 130650 | Haploinsufficien t | ENST00000354179.8:11 |
| CACN A1A | 12286 | 3 | Migraine, familial hemiplegic, 1; Episodic ataxia, type 2 | 141500; 108500 | Haploinsufficien t | ENST00000360228.10:31 |
| | | | | | | ENST00000360228.10:36 |
| | | | | | | ENST00000637769.1:37 |
| CHD2 | 24405 9 | 4 | Epileptic encephalopathy, childhood-onset | 615369 | Haploinsufficien t | ENST00000394196.8:30 |
| VCAN | 13003 | 5 | Wagner syndrome 1 | 143200 | Haploinsufficien t | ENST00000265077.7:7 |
| MECP2 | 4204 | 6 | Rett syndrome | 312750 | X-linked dominant | ENST00000303391.10:2 |
| TSC2 | 7249 | 7 | Tuberous sclerosis | 613254 | Haploinsufficien t | ENST00000219476.7:11 |
| | | | | | | ENST00000219476.7:30 |
| KMT2 D | 8085 | 8 | Kabuki syndrome | 147920 | Haploinsufficien t | ENST00000301067.11:50 |
| KANSL 1 | 28405 8 | 9 | Koolen-De Vries syndrome | 610443 | Haploinsufficien t | ENST00000574590.6:2 |
| RAI1 | 19377 | 10 | Smith-Magenis syndrome | 182290 | Haploinsufficien t | ENST00000353383.5:1 |
| ELOVL 4 | 6785 | 11 | Stargardt disease 3 | 600110 | Haploinsufficien t | ENST00000369816.4:3 |
| OPA1 | 4976 | 12 | Optic atrophy type 1 | 165500 | Haploinsufficien t | ENST00000361908.7:6 |
| | | | | | | ENST00000361908.7:28 |
| TEK | 7010 | 13 | Glaucoma 3, primary congenital, E | 617272 | Haploinsufficien t | ENST00000380036.8: 10 |
| | | | | | | ENST00000380036.8:15 |
| CDKL5 | 6292 | 14 | Epileptic encephalopathy, early infantile, 2 | 300672 | X-linked dominant | ENST00000379996.7:16 |
| SLC25 A13 | 10165 | 15 | Citrullinemia Type II | 603471 | Autosomal recessive | ENST00000265631.9:3 |
| | | | | | | ENST00000265631.9:6 |
| | | | | | | ENST00000265631.9:9 |
| | | | | | | ENST00000265631.9:11 |
| | | | | | | ENST00000265631.9:13 |
| ABCB4 | 5244 | 16 | Cholestasis, intrahepatic, of pregnancy, 3 | 614972 | Autosomal recessive, autosomal dominant | ENST00000358400.7:8 |
| ATP8B 1 | 5205 | 17 | Cholestasis, progressive familial intrahepatic 1 | 211600 | Autosomal recessive | ENST00000283684.8:16 |
| PCCB | 5096 | 18 | Propionicacidemia | 606054 | Autosomal recessive | ENST00000251654.8:5 |
| | | | | | | ENST00000251654.8:6 |
| COL4A 3 | 1285 | 19 | Alport syndrome | 104200 | Haploinsufficien t | ENST00000396578.7:11 |
| | | | | | | ENST00000396578.7:41 |
| COL4A 4 | 1286 | 20 | Alport syndrome | 203780 | Autosomal recessive | ENST00000396625.3:22 |
| | | | | | | ENST00000396625.3:44 |
| PKP2 | 5318 | 21 | Arrhythmogenic right ventricular dysplasia 9 | 609040 | Haploinsufficien t | ENST00000340811.8:4 |
| BAG3 | 9531 | 22 | Cardiomyopathy, dilated, 1HH; Myopathy, myofibrillar 6 | 613881; 612954 | Haploinsufficien t | ENST00000369085.7:1 |
| SPTAN 1 | 6709 | 23 | West syndrome | 613477 | Haploinsufficien t | ENST00000372739.5:12 |
| ASS1 | 445 | 24 | Citrullinemia, type 1 | 215700 | Autosomal recessive | ENST00000372393.7:9 |
| CBS | 875 | 25 | Homocystinuria, B6-responsive and nonresponsive types | 236200 | Autosomal recessive | ENST00000352178.9:12 |
| | | | | | | ENST00000352178.9:3 |
| DHDD S | 79947 | 26 | Retinitis pigmentosa 59 | 613861 | Autosomal recessive | ENST00000236342.11:2 |
| FAH | 2184 | 27 | Tyrosinemia, type I | 276700 | Autosomal recessive | ENST00000407106.5:5 |
| GALE | 2582 | 28 | Galactose epimerase deficiency | 230350 | Autosomal recessive | ENST00000617979.4:4 |
| GBE1 | 2632 | 29 | Glycogen storage disease IV | 232500 | Autosomal recessive | ENST00000429644.6:3 |
| HEXA | 3073 | 30 | Tay-Sachs disease | 272800 | Autosomal recessive | ENST00000268097.9:2 |
| PLCB1 | 23236 | 31 | Epileptic encephalopathy, early infantile, 12 | 613722 | Autosomal recessive | ENST00000378641.7:23 |
| CD55 | 1604 | 32 | Pathway (eye) | N/A | N/A | ENST00000367064.7:1 |
| CFH | 3075 | 33 | Pathway (eye) | N/A | N/A | ENST00000367429.8:3 |
| DEPDC 5 | 9681 | 34 | Familial focal epilepsy | 604364 | Haploinsufficien t | ENST00000400246.5:20 |
| NF1 | 4763 | 35 | Neurofibromatosis type 1 | 162200 | Haploinsufficien t | ENST00000358273.8:31 |
| OPTN | 10133 | 36 | ALS; Primary open angle glaucoma | 613435; 137760; 606657 | Haploinsufficien t | ENST00000378757.6:1 |
| RBFOX 2 | 23543 | 37 | Pathway (CNS, epilepsy) | 612149 | Haploinsufficien t | ENST00000449924.6:5 |
| SCN2A | 6326 | 38 | Epileptic encephalopathy, early infantile, 11; Seizures, benign familial infantile, 3 | 613721 | Haploinsufficien t | ENST00000375437.6:13 |
| SCN3A | 6328 | 39 | Pathway (CNS, epilepsy) | 182391 | N/A | ENST00000283254.11:6 |
| | | | | | | ENST00000360093.7:7 |
| SCN8A | 6334 | 40 | Cognitive impairment with or without cerebellar ataxia; Epileptic encephalopathy, early infantile, 13; Seizures, benign familial infantile, 5; Pathway (CNS) | 614306; 614558; 617080; N/A | Haploinsufficien t | ENST00000627620.2:4 |
| | | | | | | ENST00000354534.10:6 |
| | | | | | | ENST00000354534.10:20 |
| SCN9A | 6335 | 41 | Epilepsy, generalized, with febrile seizures plus, type 7; Febrile seizures, familial, 3B; Insensitivity to pain, congenital; HSAN2D, autosomal recessive | 613863; 613863; 243000; 243000 | Haploinsufficien t; haploinsufficien t; autosomal recessive, autosomal recessive | ENST00000409672.5:6 |
| SLC6A 1 | 6529 | 42 | Myoclonic-atonic epilepsy | 616421 | Haploinsufficien t | ENST00000287766.8:1 |
| GRN | 2896 | 43 | GRN-related frontotemporal demensia; Aphasia, primary progressive | 607485; 607485 | Haploinsufficien t | ENST00000053867.7:1 |
| CLN3 | 1201 | 44 | Ceroid lipofuscinosis, neuronal, 3 | 204200 | Autosomal recessive | ENST00000569430.6:15 |
| MAPK 3 | 5595 | 45 | 16p11.2 deletion syndrome | 601795 | Haploinsufficien t | ENST00000263025.8:8 |
| FXN | 2395 | 46 | Friedreich ataxia; Friedreich ataxia with retained reflexes | 229300 | Autosomal recessive | ENST00000396364.7:4 |
| SHAN K3 | 85358 | 47 | Phelan-McDermid syndrome; Autism spectrum disorder | 606232 | Haploinsufficien t | ENST00000262795.5:24 |
| UBE3A | 7337 | 48 | Angelman syndrome | 105830 | Haploinsufficien t | ENST00000625778.2:1 |
| GRIN2 A | 2903 | 49 | Epilepsy-aphasia spectrum; Opioid addiction | 245570 | Haploinsufficien t | ENST00000562109.5:11 |
| CHRN A7 | 1139 | 50 | 15q13.3 microdeletion | 612001 | Haploinsufficien t | ENST00000636603.1:4 |
| MBD5 | 55777 | 51 | Mental retardation, autosomal dominant 1 | 156200 | Haploinsufficien t | ENST00000407073.5:13 |
| MECP2 | 4204 | 52 | Rett syndrome | 312750 | N/A | ENST00000453960.6:1 |
| PRPF3 | 9129 | 53 | Retinitis pigmentosa 18 | 601414 | Haploinsufficien t | ENST00000324862.6:3 |
| PRPF31 | 26121 | 54 | Retinitis pigmentosa 11 | 600138 | Haploinsufficien t | ENST00000321030.8:9 |
| TOPOR S | 10210 | 55 | Retinitis pigmentosa 31 | 609923 | Haploinsufficien t | ENST00000360538.6:1 |
| PCCA | 5095 | 56 | Propionic academia | 606054 | Autosomal recessive | ENST00000376285.5:14 |
| MUT | 4594 | 57 | Methyl Malonic Aciduria | 251000 | Autosomal recessive | ENST00000274813.3:11 |
| CISD2 | 49385 6 | 58 | Wolfram syndrome2 /NAFLD | 604928 | Autosomal recessive | ENST00000273986.8:1 |
| KCNQ2 | 3785 | 59 | Early-onset epileptic encephalopathy | 613720 | Haploinsufficien t | ENST00000626839.2:1 |

**Table 2. List of exemplary target gene sequences.**

| **Gene Symbol** | **Gene ID No.** | **SEQ ID No.** | **Disease** | **OMIM** | **Genetics** | **Introns** |
|---|---|---|---|---|---|---|
| NIPBL | 71175 | 1 | Cornelia de Lange | 122470 | Haploinsufficient | GRCh38/ hg38: chr5 37046200 37048501 |
| NSD1 | 18193 | 2 | Sotos syndrome 1; Beckwith-Wiedemann syndrome | 117550 | Haploinsufficient | GRCh38/ hg38: chr5 177136031 177191883 |
| | | | | | | GRCh38/ hg38: chr5 177192020 177204119 |
| | | | | | | GRCh38/ hg38: chr5 177246797 177248180 GRCh38/ hg38: chr5 177273785 177280564 |
| CACNA1A | 12286 | 3 | Migraine, familial hemiplegic, 1; Episodic ataxia, type 2 | 141500; 108500 | Haploinsufficient | GRCh38/ hg38: chr19 13235731 13241520 |
| CHD2 | 244059 | 4 | Epileptic encephalopathy, childhood-onset | 615369 | Haploinsufficient | GRCh38/ hg38: chr15 92997404 92998498 |
| VCAN | 13003 | 5 | Wagner syndrome 1 | 143200 | Haploinsufficient | GRCh38/ hg38: chr5 83542269 83545536 |
| OPA1 | 4976 | 12 | Optic atrophy type 1 | 165500 | Haploinsufficient | GRCh38/ hg38: chr3 193626203 193631611 |
| | | | | | | GRCh38/ hg38: chr3 193593374 193614710 |
| COL4A3 | 1285 | 19 | Alport syndrome | 104200 | Haploinsufficient | GRCh38/ hg38: chr2 227295317 227297673 |
| COL4A4 | 1286 | 20 | Alport syndrome | 203780 | Autosomal recessive | GRCh38/ hg38: chr2 227144559 227147412 |
| | | | | | | GRCh38/ hg38: chr2 227012299 227022047 |
| PKP2 | 5318 | 21 | Arrhythmogenic right ventricular dysplasia 9 | 609040 | Haploinsufficient | GRCh38/ hg38: chr12 32879033 32896508 |
| CBS | 875 | 25 | Homocystinuria, B6-responsive and nonresponsive types | 236200 | Autosomal recessive | GRCh38/ hg38: chr21 43059304 43060440 |
| DHDDS | 79947 | 26 | Retinitis pigmentosa 59 | 613861 | Autosomal recessive | GRCh38/ hg38: chr1 26438285 26442730 |
| GALE | 2582 | 28 | Galactose epimerase deficiency | 230350 | Autosomal recessive | GRCh38/ hg38: chr1 23798231 23798614 |
| HEXA | 3073 | 30 | Tay-Sachs disease | 272800 | Autosomal recessive | GRCh38/ hg38: chr15 72356651 72375719 |
| | | | | | | GRCh38/ hg38: chr15 72345552 72346234 |
| CFH | 3075 | 33 | Pathway (eye) | N/A | N/A | GRCh38/ hg38: chr1 196673963 196675988 |
| NF1 | 4763 | 35 | Neurofibromatosis type 1 | 162200 | Haploinsufficient | GRCh38/ hg38: chr17 31249120 31252937 |
| SCN2A | 6326 | 38 | Epileptic encephalopathy, early infantile, 11; Seizures, benign familial infantile, 3 | 613721 | Haploinsufficient | GRCh38/ hg38: chr2 165326985 165331329 |
| SCN8A | 6334 | 40 | Cognitive impairment with or without cerebellar ataxia; Epileptic encephalopathy, early infantile, 13; Seizures, benign familial infantile, 5; Pathway (CNS) | 614306; 614558; 617080; N/A | Haploinsufficient | GRCh38/ hg38: chr12 51687220 51689004 |
| | | | | | | GRCh38/ hg38: chr12 51774363 51786541 |
| SCN9A | 6335 | 41 | Epilepsy, generalized, with febrile seizures plus, type 7; Febrile seizures, familial, 3B; Insensitivity to pain, congenital; HSAN2D, autosomal recessive | 613863; 613863; 243000; 243000 | Haploinsufficient; haploinsufficient; autosomal recessive, autosomal recessive | GRCh38/ hg38: chr2 166304122 166305791 |
| CLN3 | 1201 | 44 | Ceroid lipofuscinosis, neuronal, 3 | 204200 | Autosomal recessive | GRCh38/ hg38: chr16 28477878 28482104 |
| MAPK3 | 5595 | 45 | 16p11.2 deletion syndrome | 601795 | Haploinsufficient | GRCh38/ hg38: chr16 30114709 30116635 |
| MBD5 | 55777 | 51 | | 156200 | Haploinsufficient | GRCh38/ hg38: chr2 |
| | | | Mental retardation, autosomal dominant 1 | | | 148458872 148462581 |
| | | | | | | GRCh38/ hg38: chr2 148490595 148502435 |
| | | | | | | GRCh38/ hg38: chr2 148502510 148510059 |
| PRPF3 | 9129 | 53 | Retinitis pigmentosa 18 | 601414 | Haploinsufficient | GRCh38/ hg38: chr1 150328467 150332683 |
| | | | | | | GRCh38/ hg38: chr1 150325882 150328319 |
| TOPORS | 10210 | 55 | Retinitis pigmentosa 31 | 609923 | Haploinsufficient | GRCh38/ hg38: chr9 32550969 32552433 |
| PCCA | 5095 | 56 | Propionic acidemia | 606054 | Autosomal recessive | GRCh38/ hg38: chr13 100302999 100307191 |
| MUT | 4594 | 57 | Methyl Malonic Aciduria | 251000 | Autosomal recessive | GRCh38/ hg38: chr6 49435625 49440205 |
| AKT3 | 393 | 192 | Pathway (EYE) | N/A | N/A | GRCh38/ hg38: chr1 243563849 243572925 |
| CD46 | 6953 | 193 | Pathway (EYE) | N/A | N/A | GRCh38/ hg38: chr1 207770363 207783291 |
| COL11A2 | 2187 | 194 | Deafness, autosomal dominant 13 | 601868 | Haploinsufficient | GRCh38/ hg38: chr6 33181172 33184144 |
| CR1 | 2334 | 195 | Pathway (EYE) | N/A | N/A | GRCh38/ hg38: chr1 207630622 207639396 |
| CRX | 2383 | 196 | Cone-rod retinal dystrophy-2 | 120970 | Haploinsufficient | GRCh38/ hg38: chr19 47834544 47836242 |
| DNAJC8 | 15470 | 197 | Pathway (EYE) | N/A | N/A | GRCh38/ hg38: chr1 28229025 28232920 |
| MYH14 | 23212 | 198 | Deafness, autosomal dominant 4A; Peripheral neuropathy, myopathy, hoarseness, and hearing loss | 600652; 600652 | Haploinsufficient | GRCh38/ hg38: chr19 50230624 50231929 |
| MYO6 | 7605 | 199 | Deafness, autosomal dominant 22 | 606346 | Haploinsufficient | GRCh38/ hg38: chr6 75867106 75870646 |
| NF2 | 7773 | 200 | Neurofibromatosis type 2 | 101000 | Haploinsufficient | GRCh38/ hg38: chr22 29604113 29636750 |
| SEMA3C | 10725 | 201 | Pathway (EYE) | N/A | N/A | GRCh38/ hg38: chr7 80789529 80798091 |
| SEMA3D | 10726 | 202 | Pathway (EYE) | N/A | N/A | GRCh38/ hg38: chr7 85055860 85065423 |
| EIF2AK3 | 3255 | 203 | Pathway (EYE) | N/A | N/A | GRCh38/ hg38: chr2 88579641 88583429 |
| ERN1 | 3449 | 204 | Pathway (EYE) | N/A | N/A | GRCh38/ hg38: chr17 64098242 64129975 |
| GUCY2F | 4691 | 205 | Pathway (EYE) | N/A | N/A | GRCh38/ hg38: chrx 109382213 109385183 |
| | | | | | | GRCh38/ hg38: chrx 109430397 109441350 |
| SIRT3 | 14931 | 206 | Pathway (EYE) | N/A | N/A | GRCh38/ hg38: chr11 224241 230451 |
| NR1H4 | 7967 | 207 | NASH | N/A | N/A | GRCh38/ hg38: chr12 100493403 100505574 |
| STK11 | 11389 | 208 | NASH | N/A | N/A | GRCh38/ hg38: chr19 1207204 1218416 |
| | | | | | | GRCh38/ hg38: chr19 1221341 1221948 |
| PPARA | 9232 | 209 | NASH | N/A | N/A | GRCh38/ hg38: chr22 46198592 46215172 |
| CYP2J2 | 2634 | 210 | NASH | N/A | N/A | GRCh38/ hg38: chr1 59901104 59904870 |
| SYNGAP1 | 11497 | 211 | Mental retardation, autosomal dominant 5 | 612621 | Haploinsufficient | GRCh38/ hg38: chr6 33447934 33451759 |

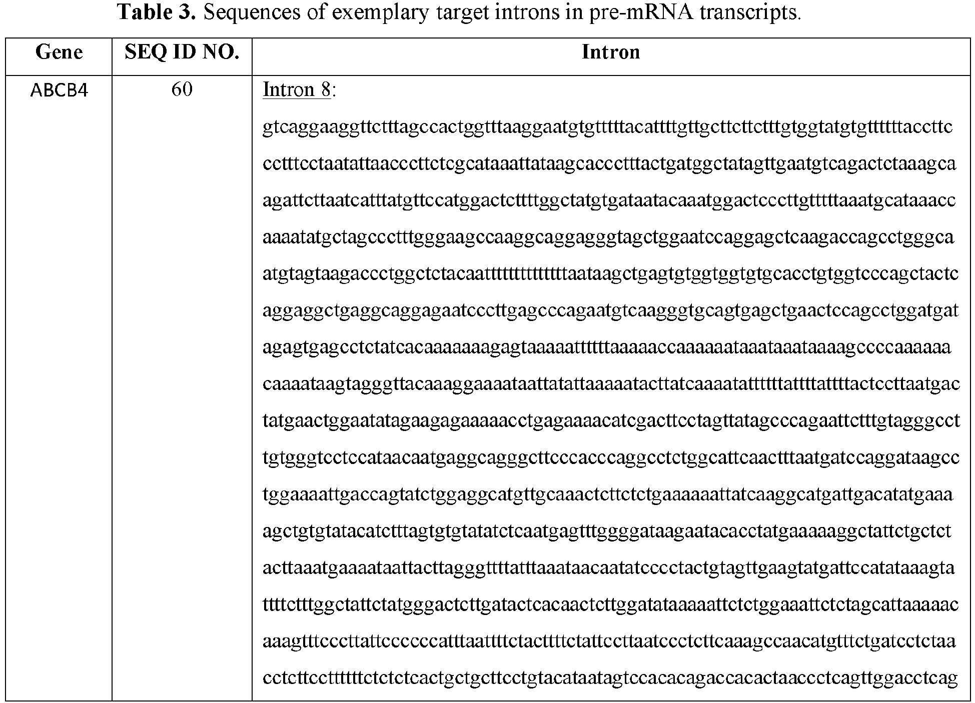

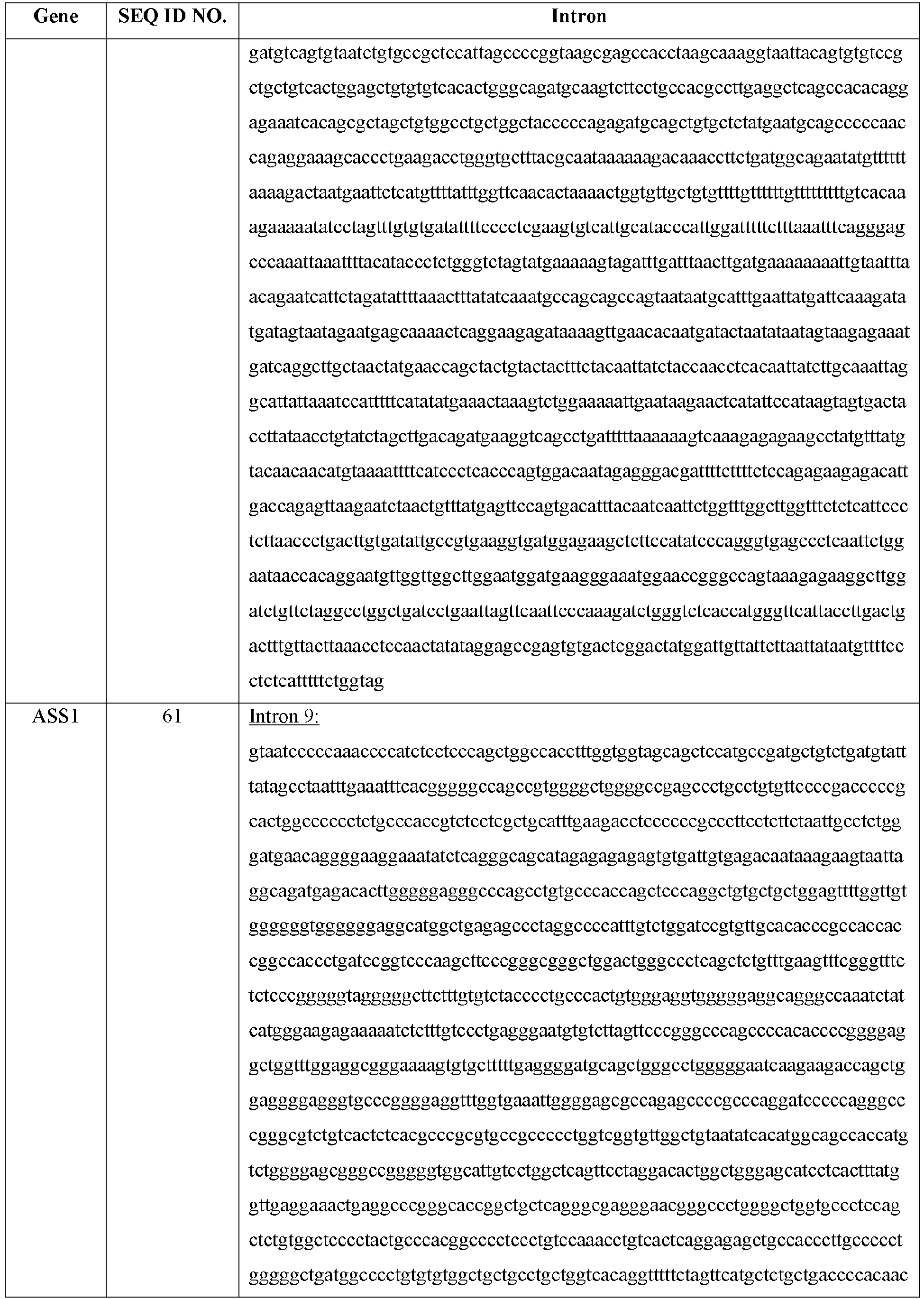

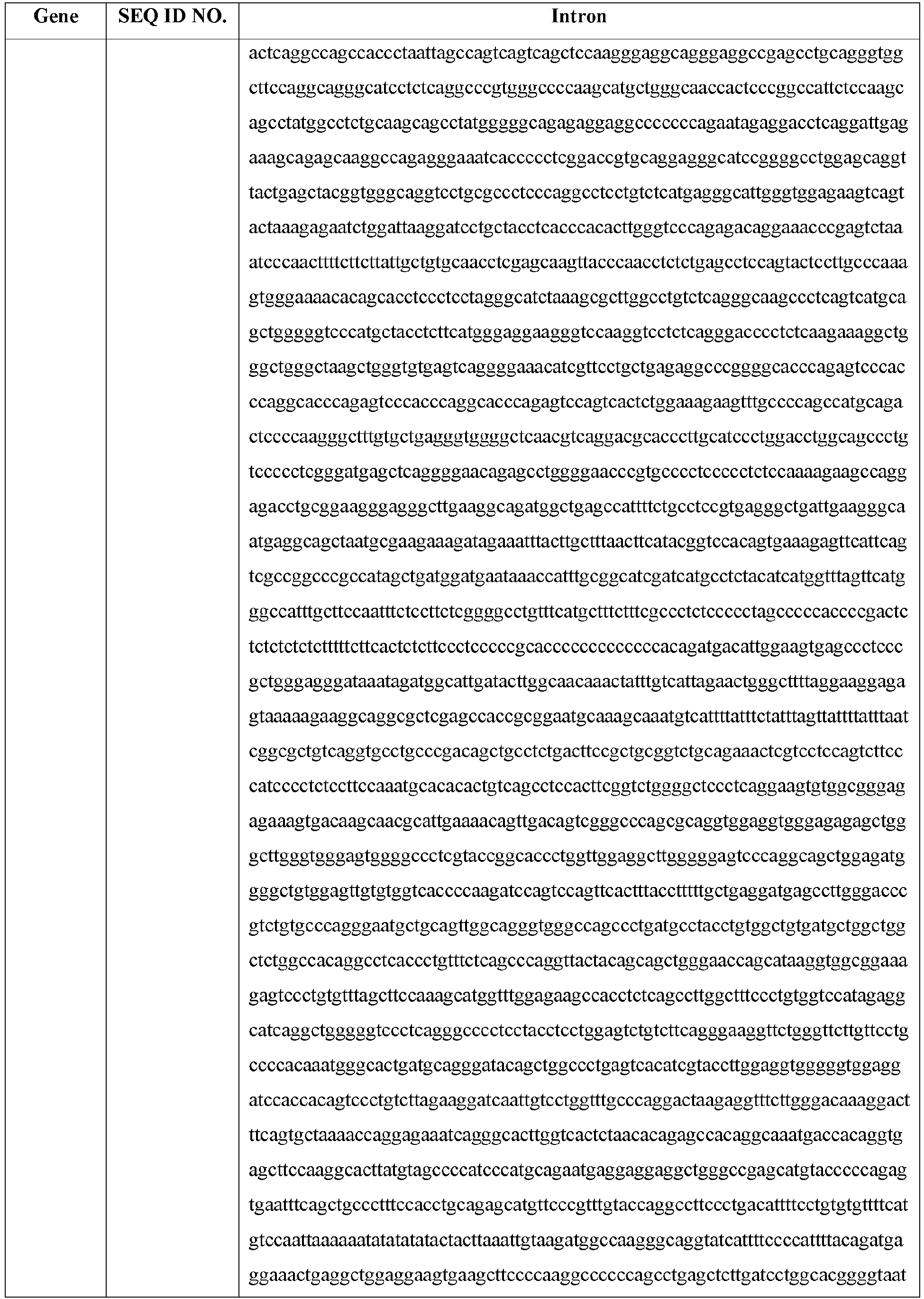

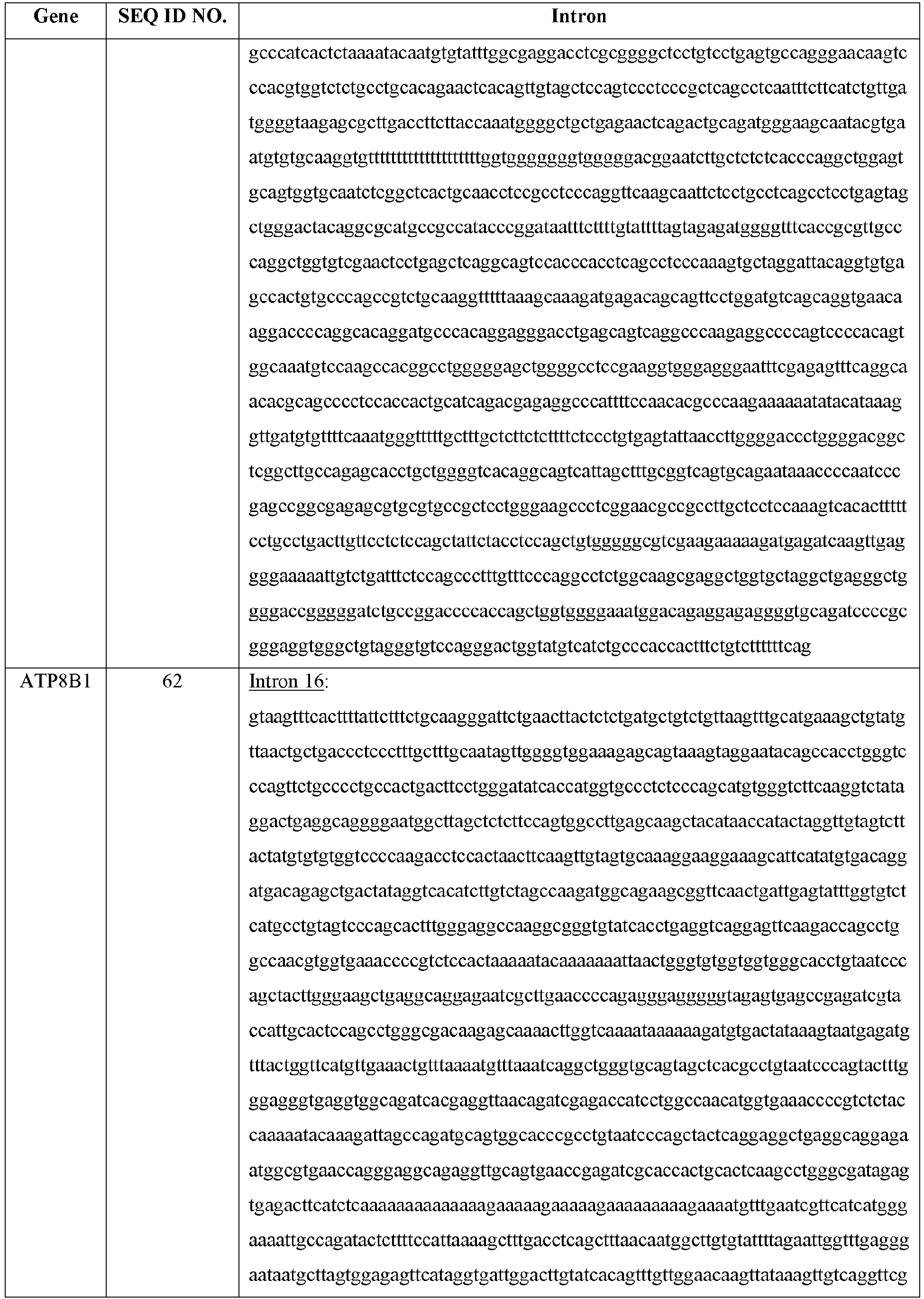

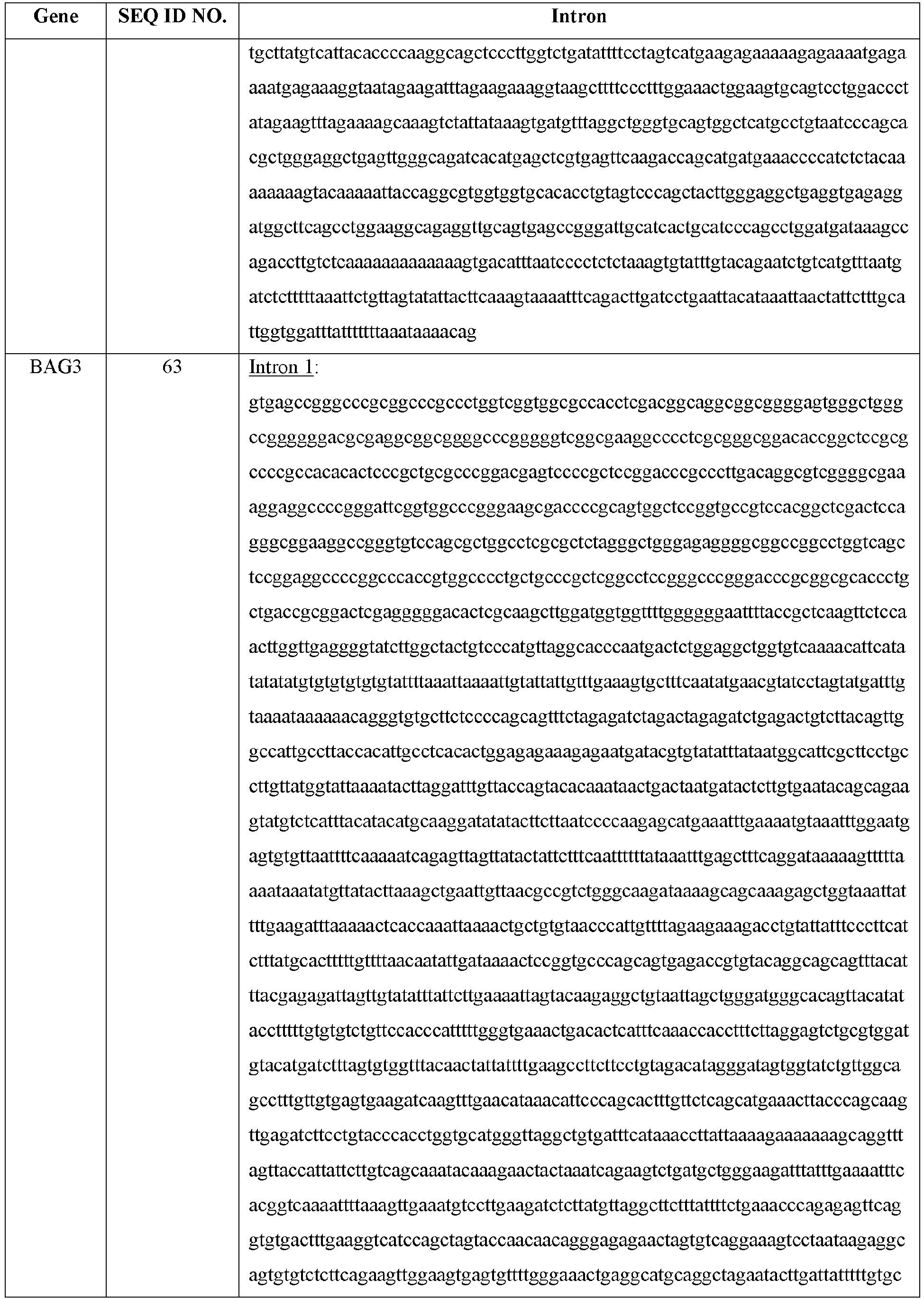

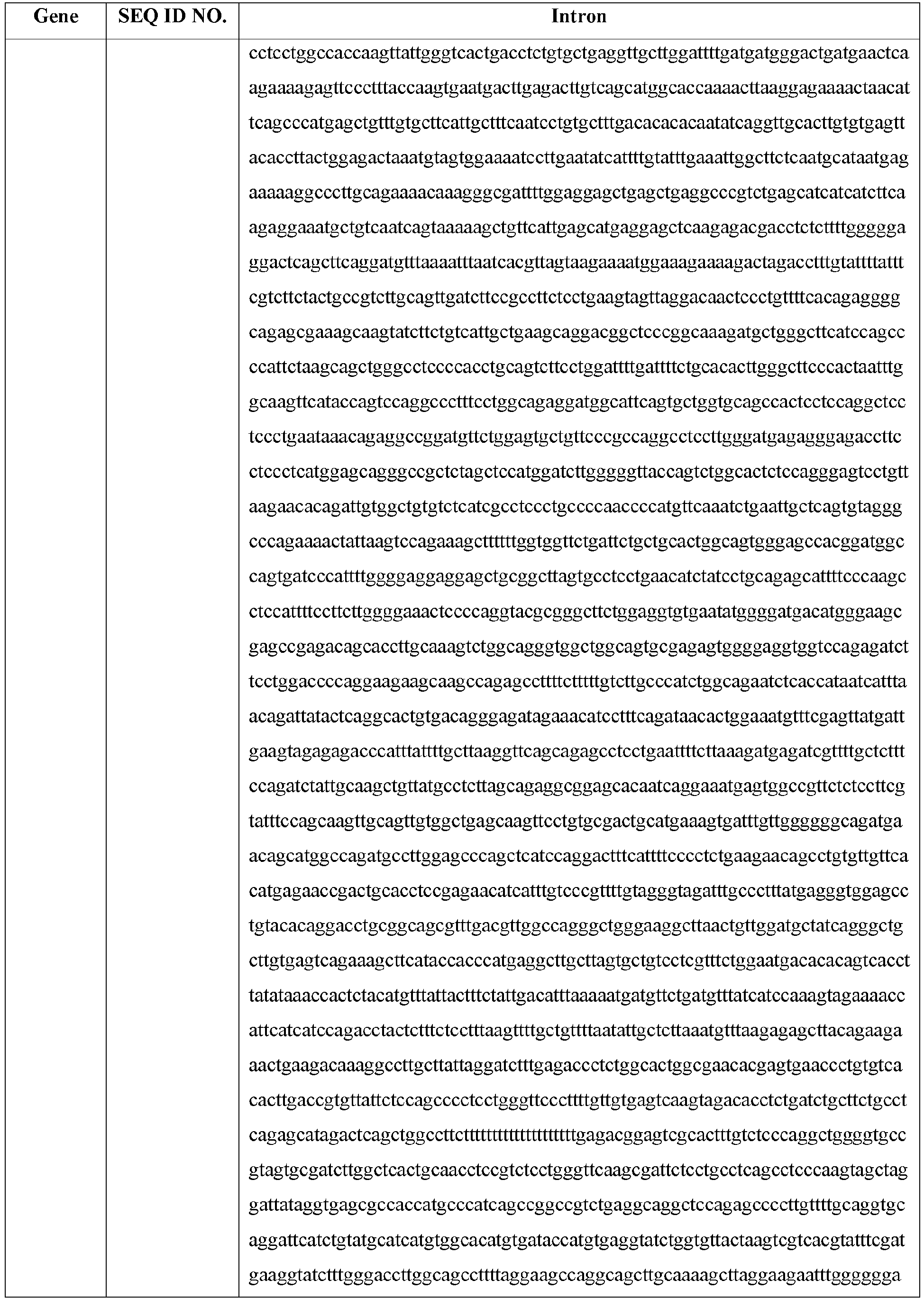

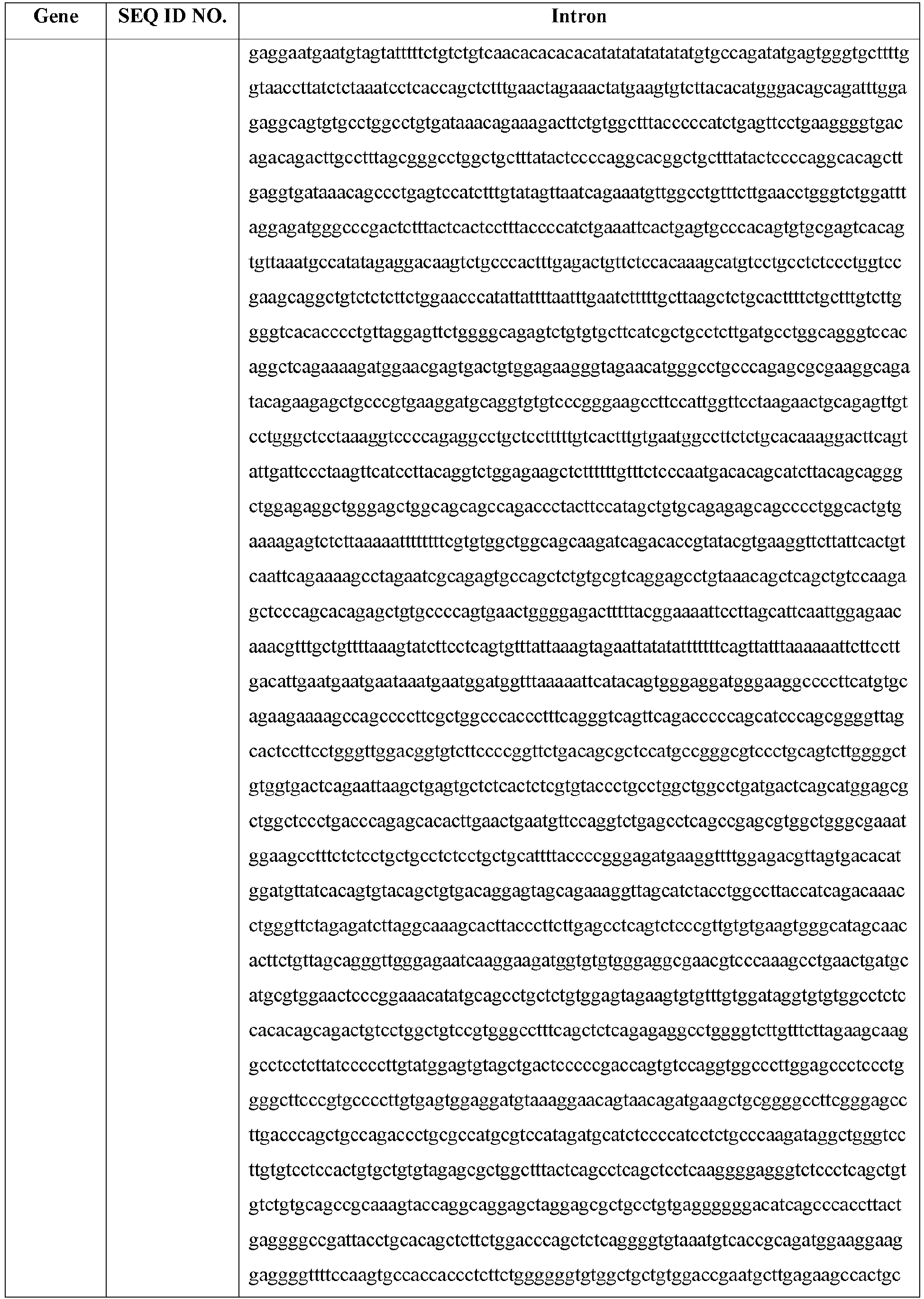

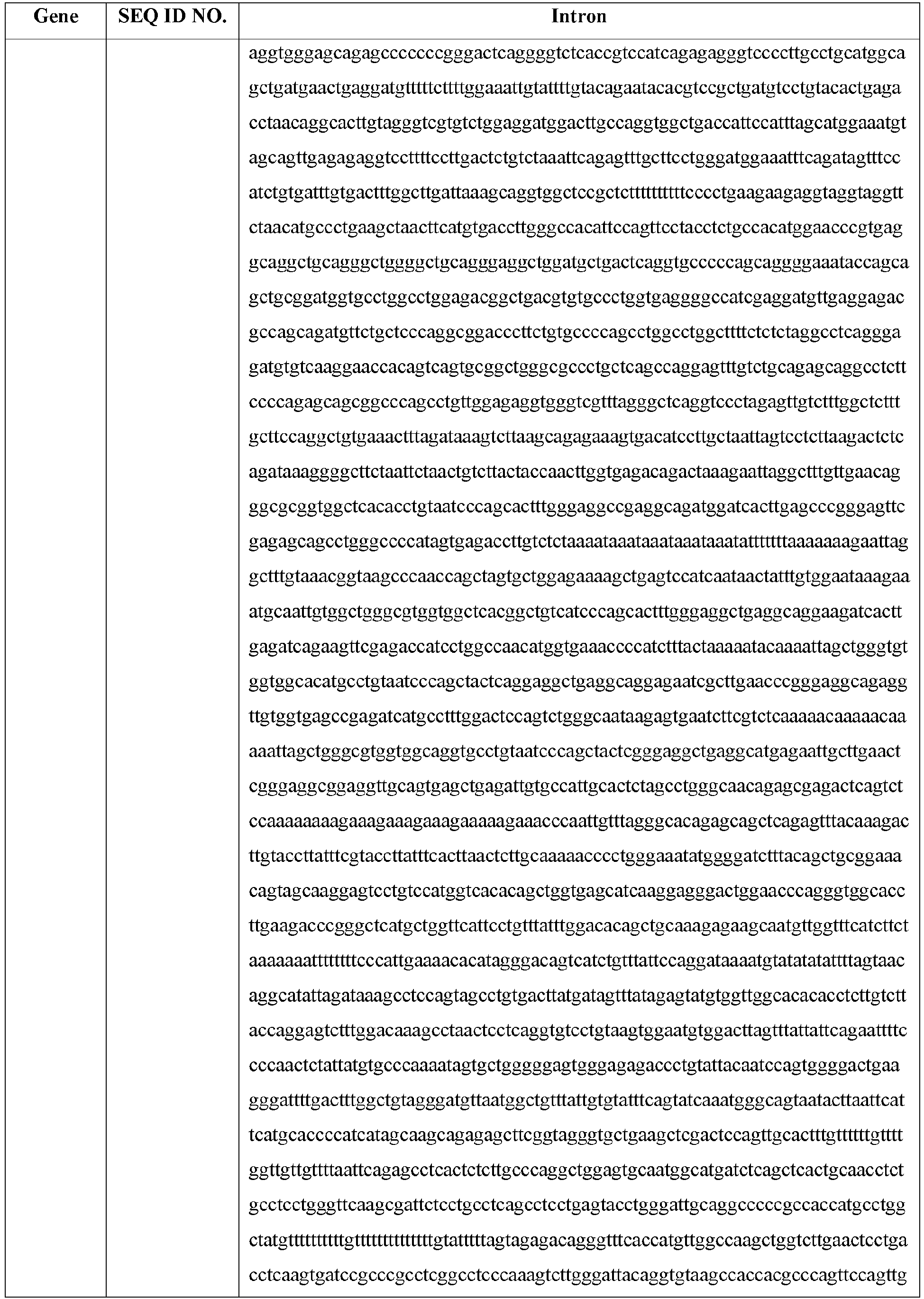

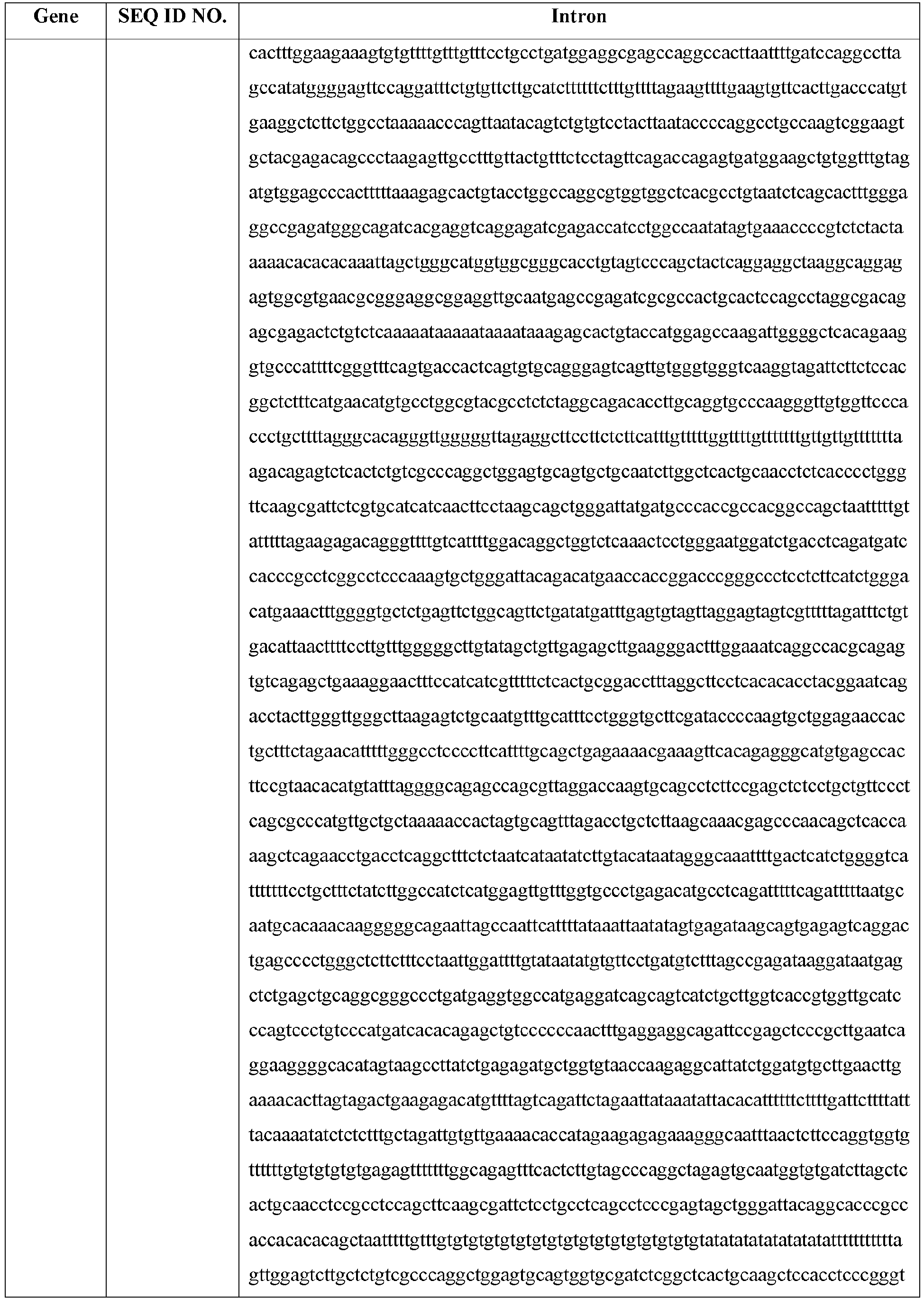

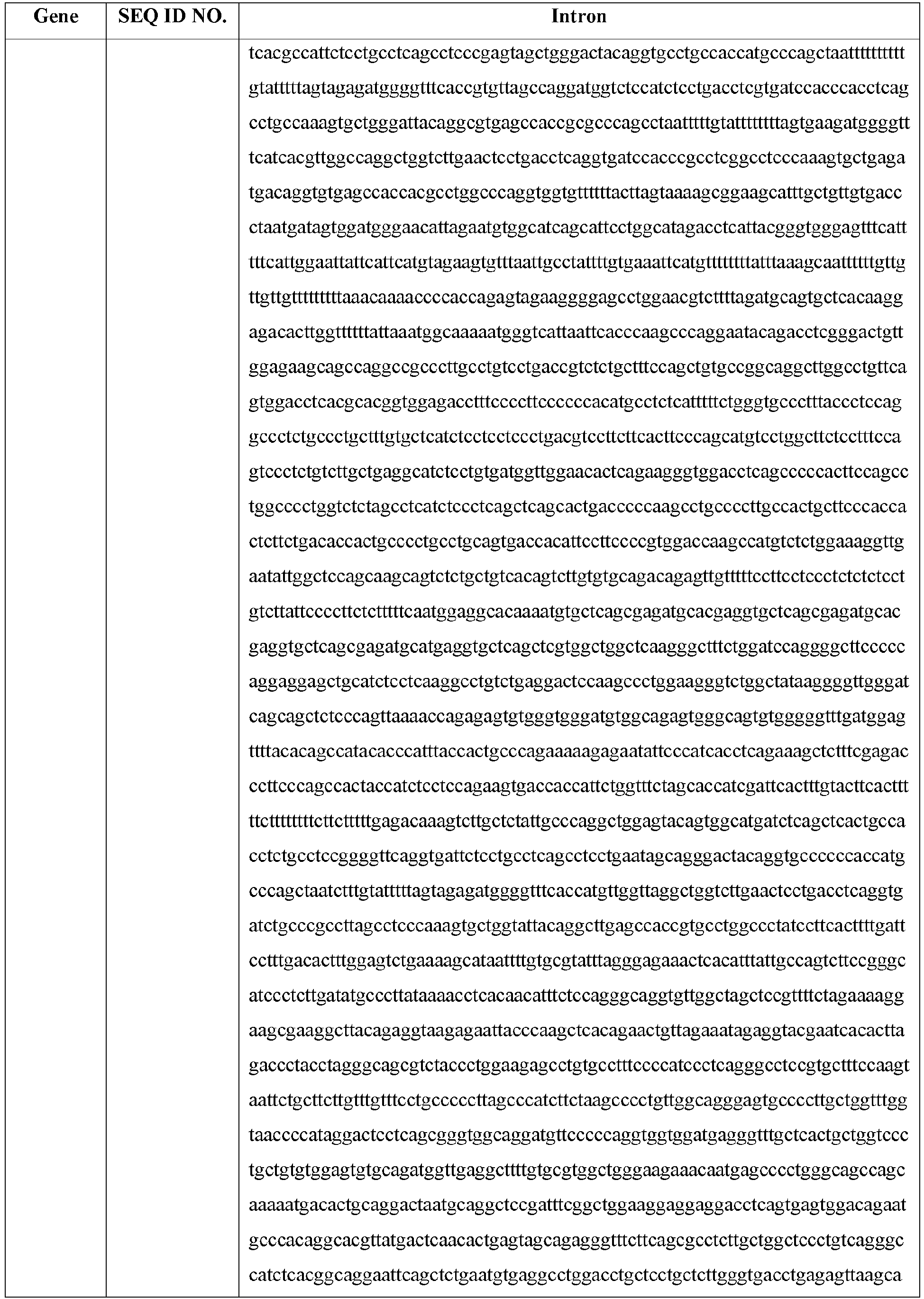

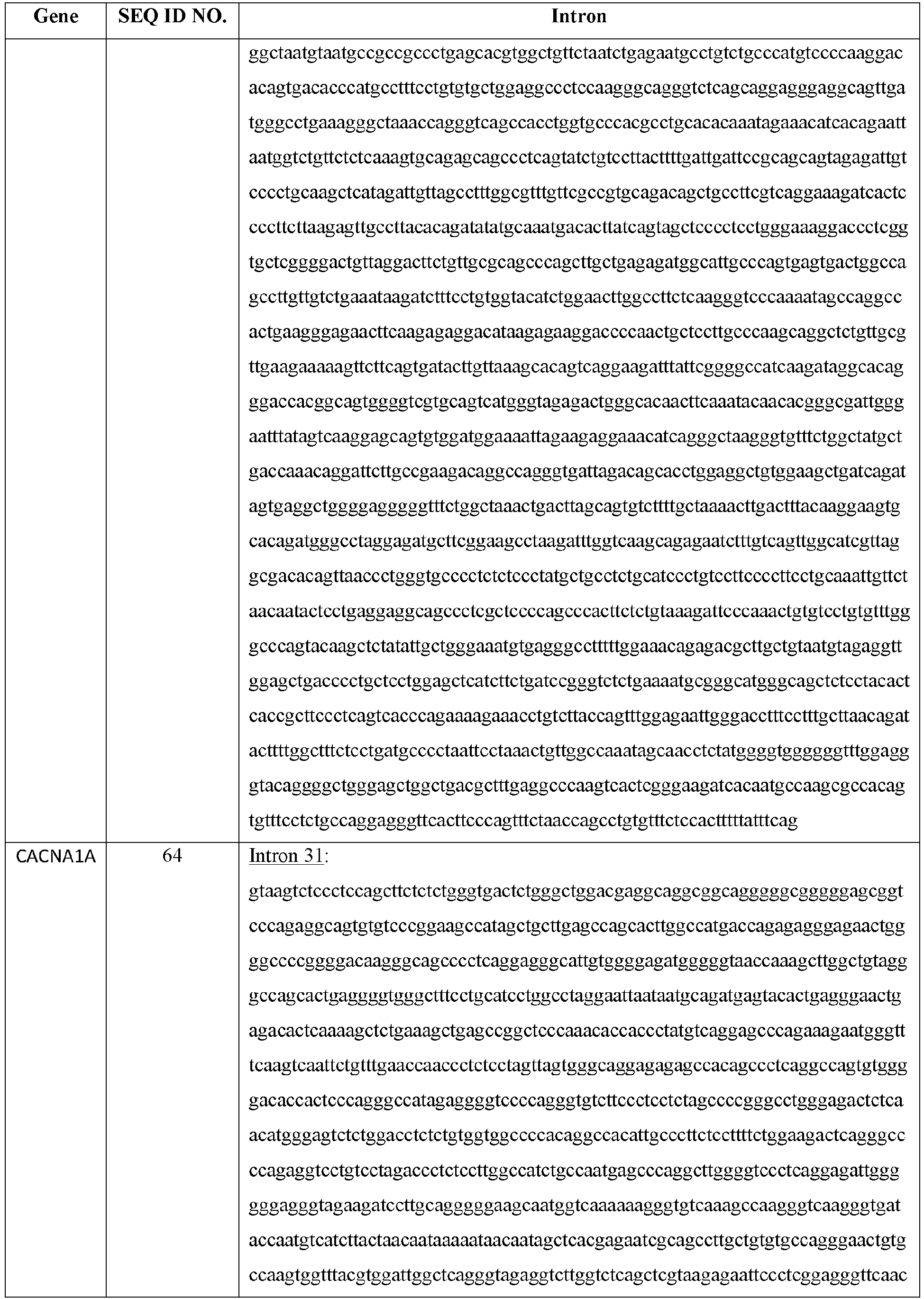

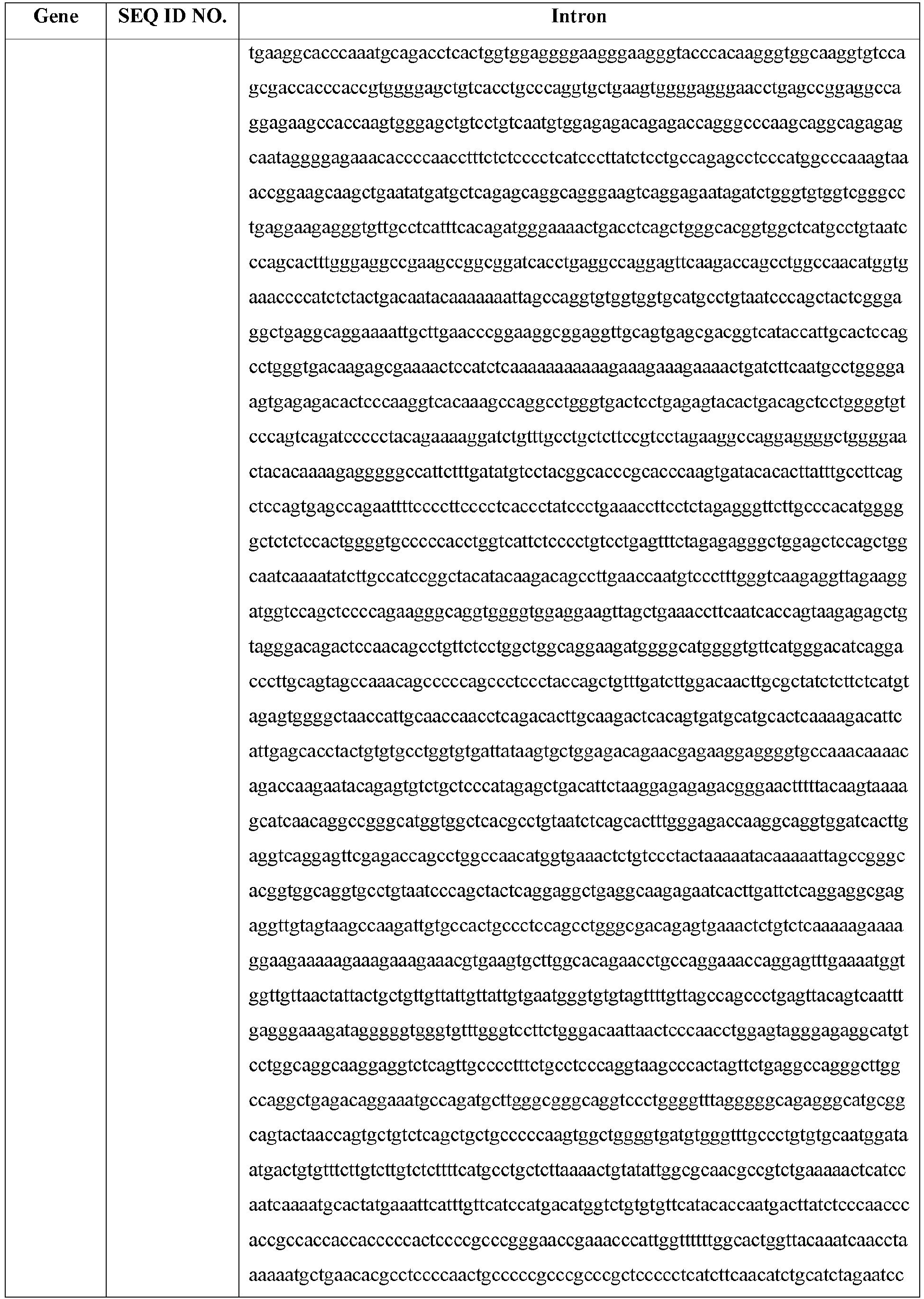

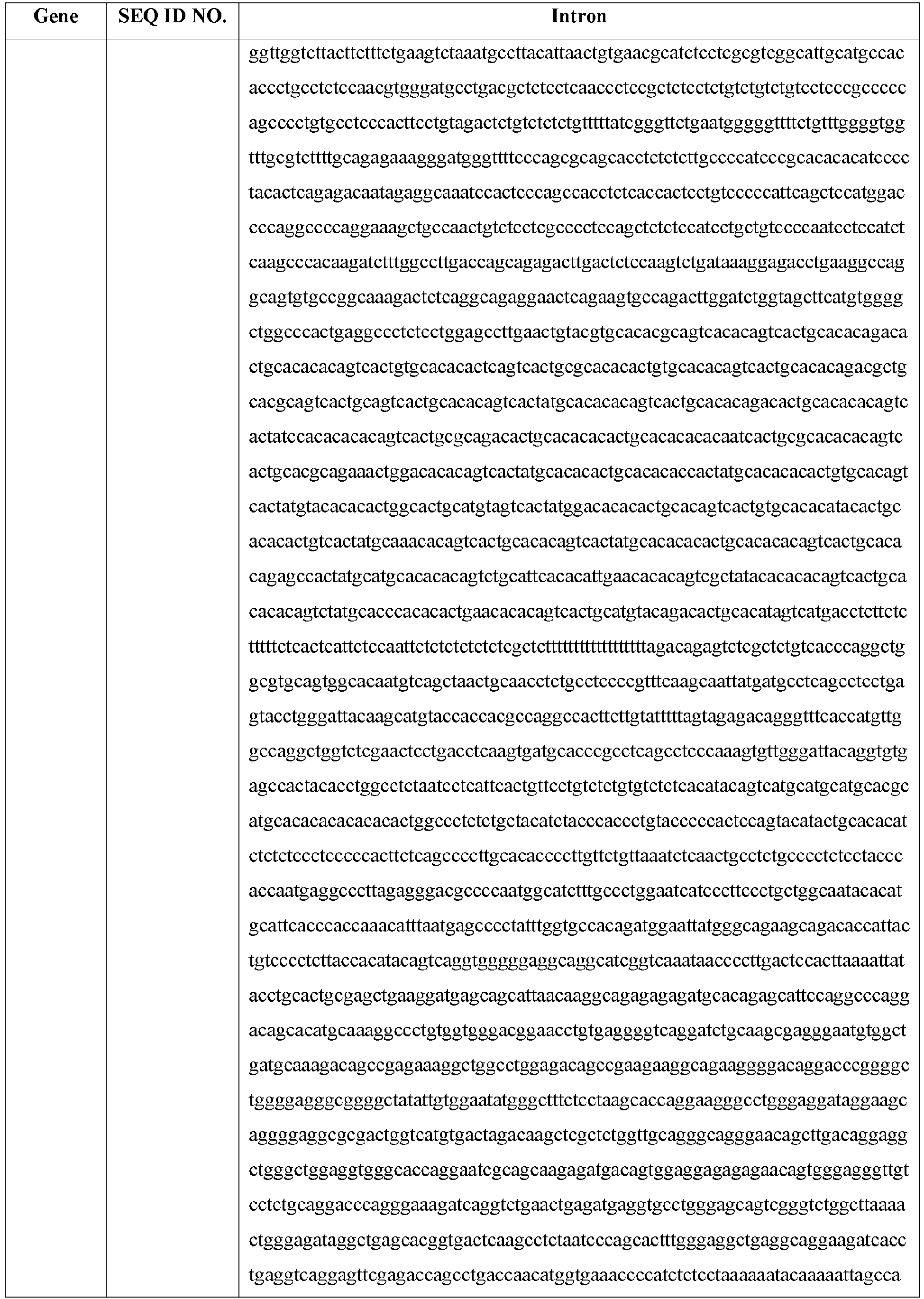

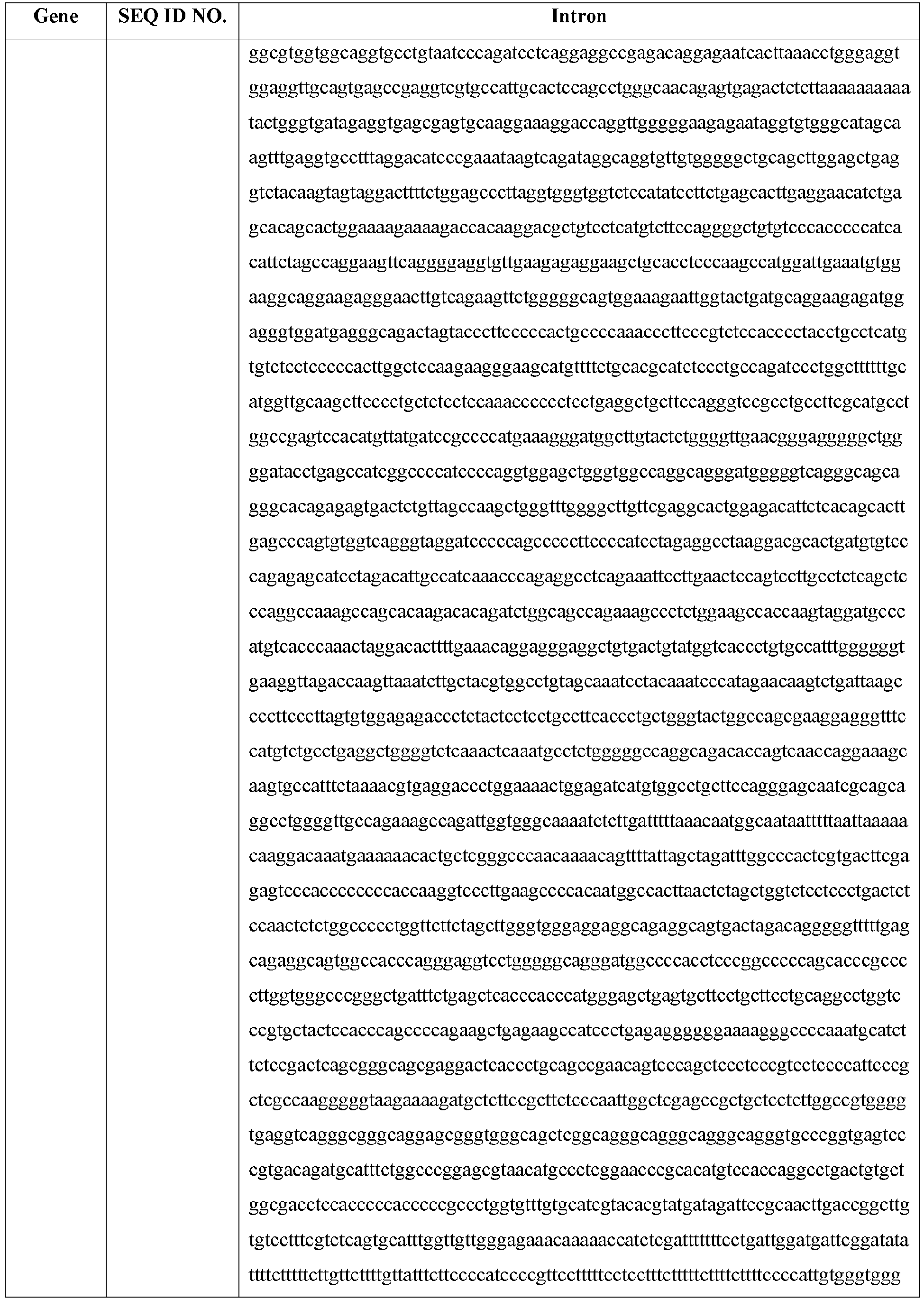

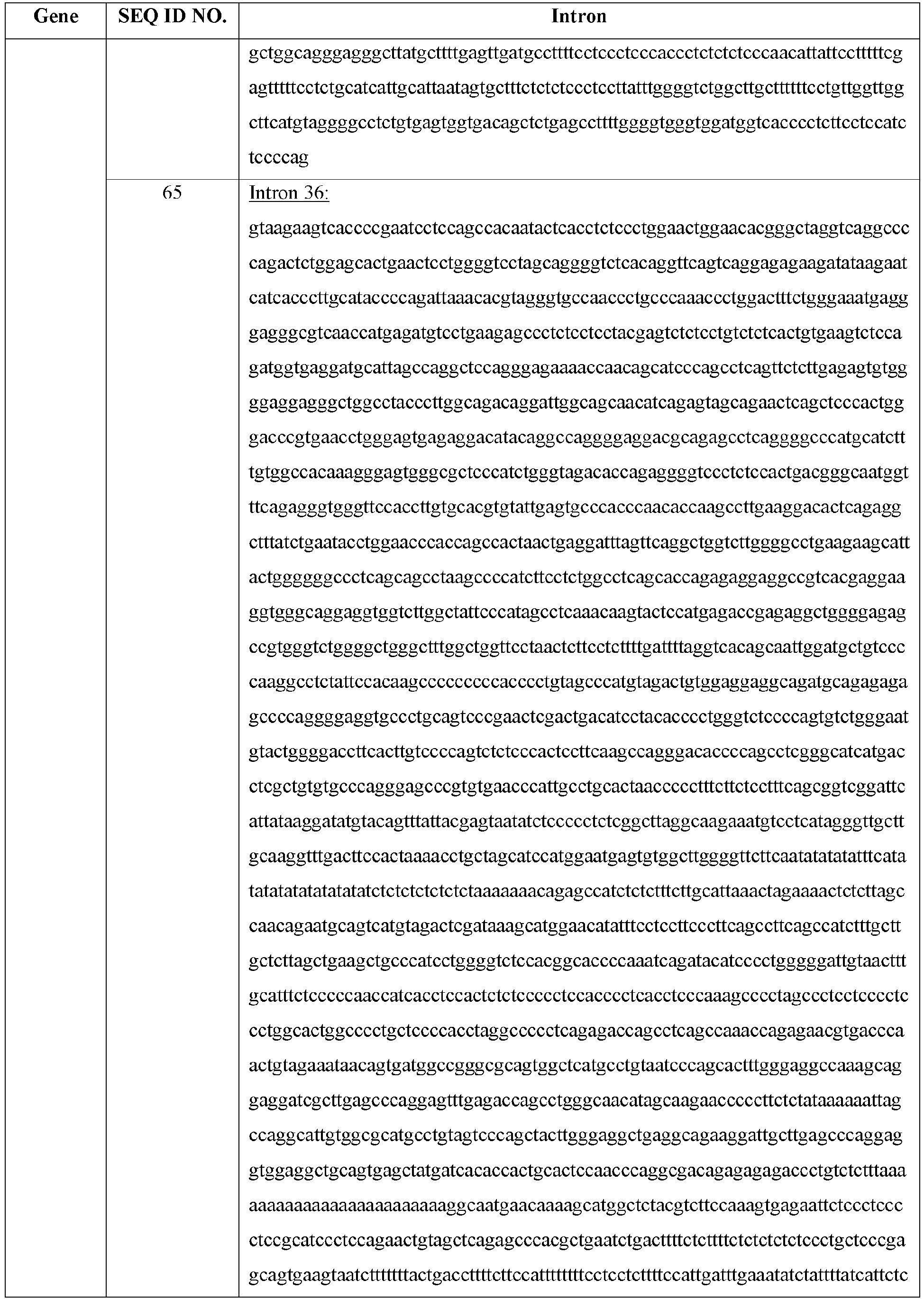

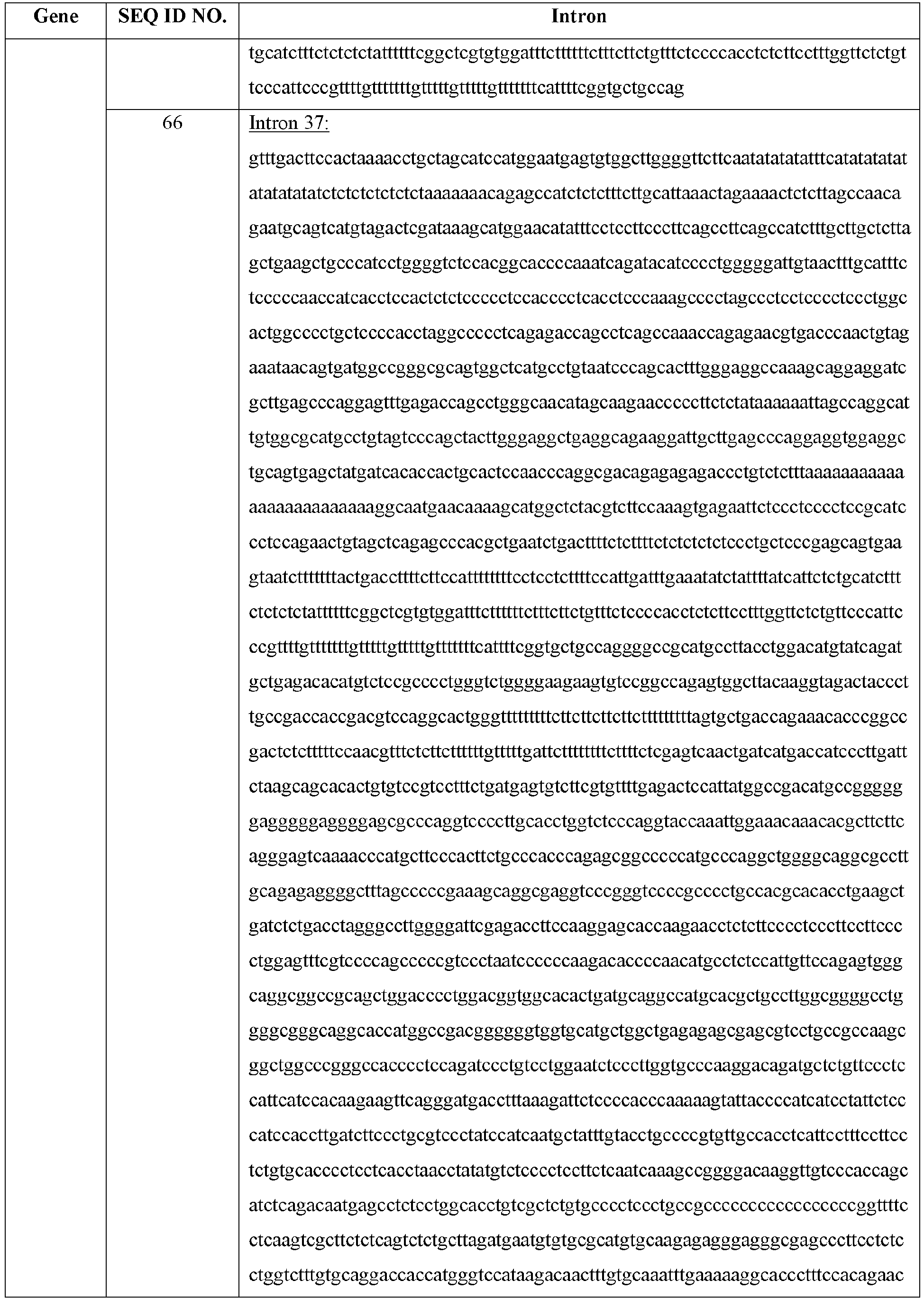

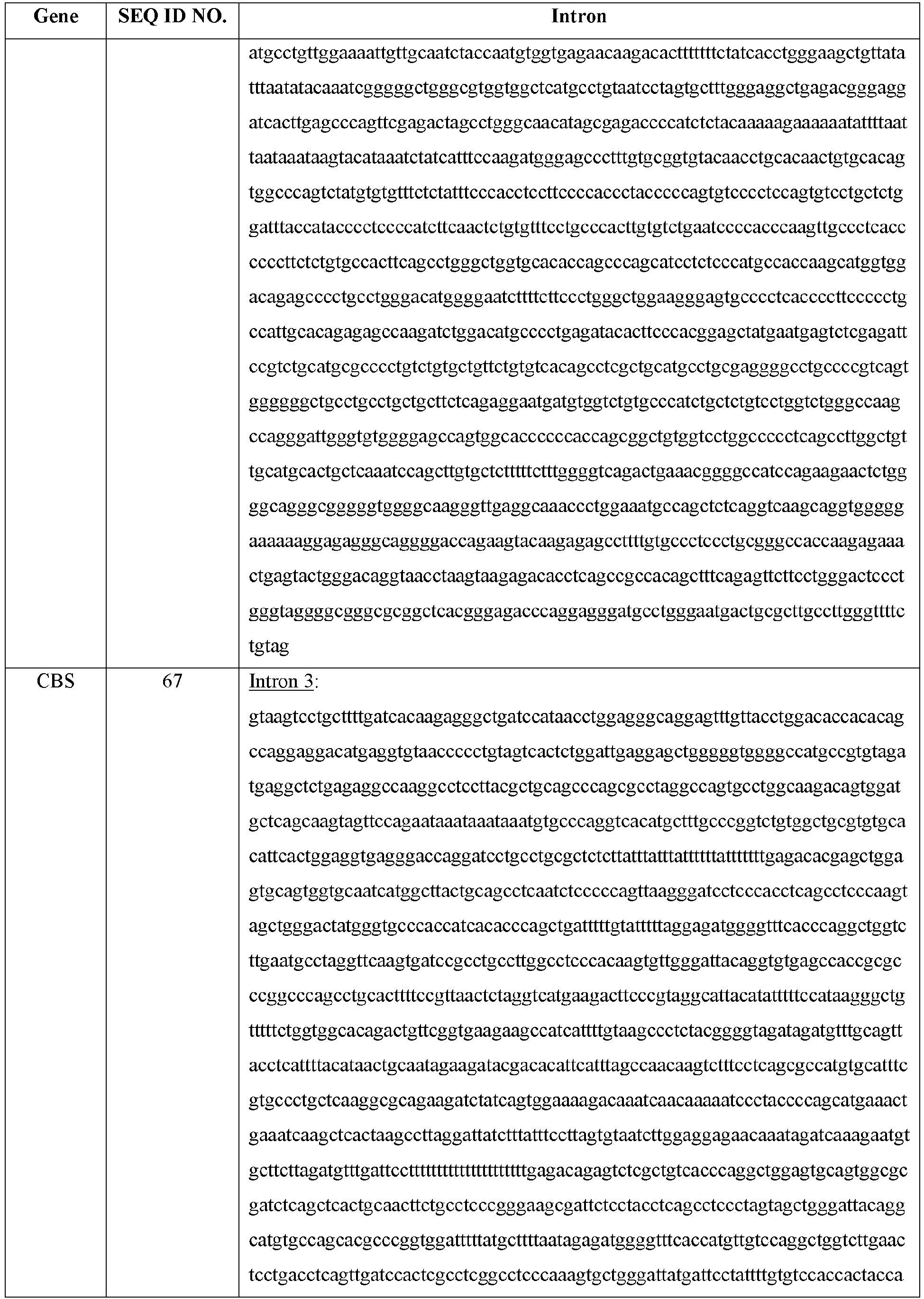

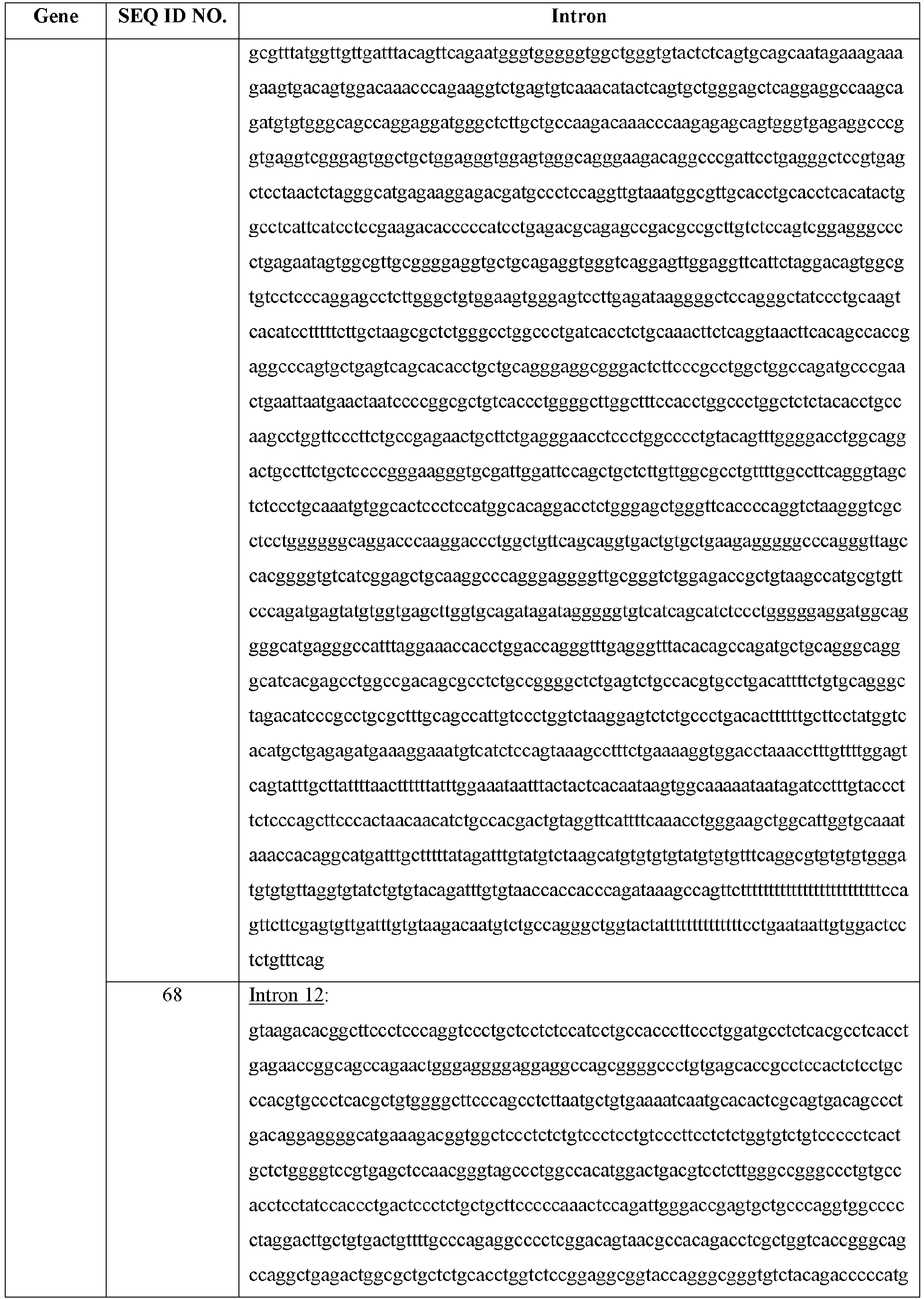

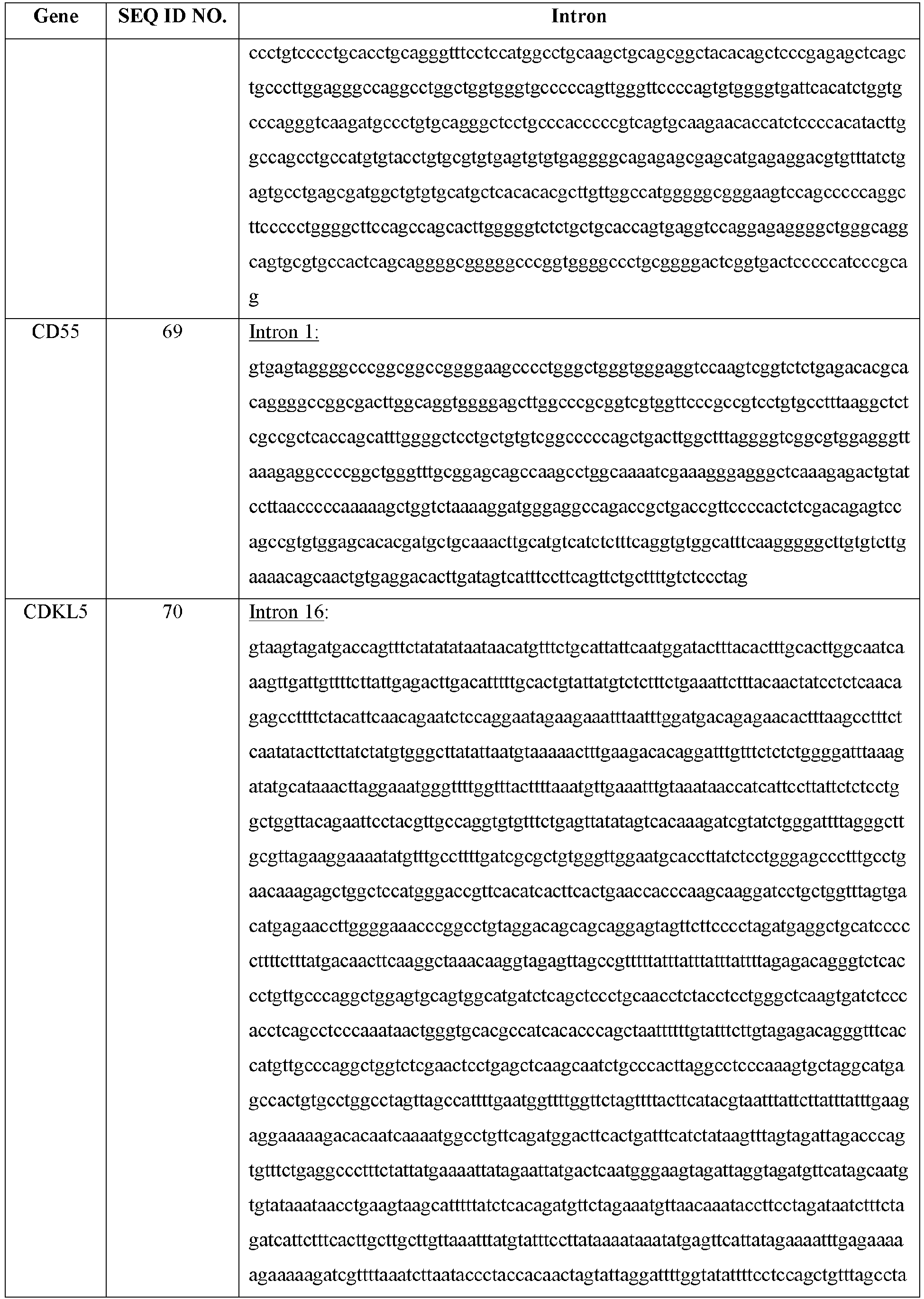

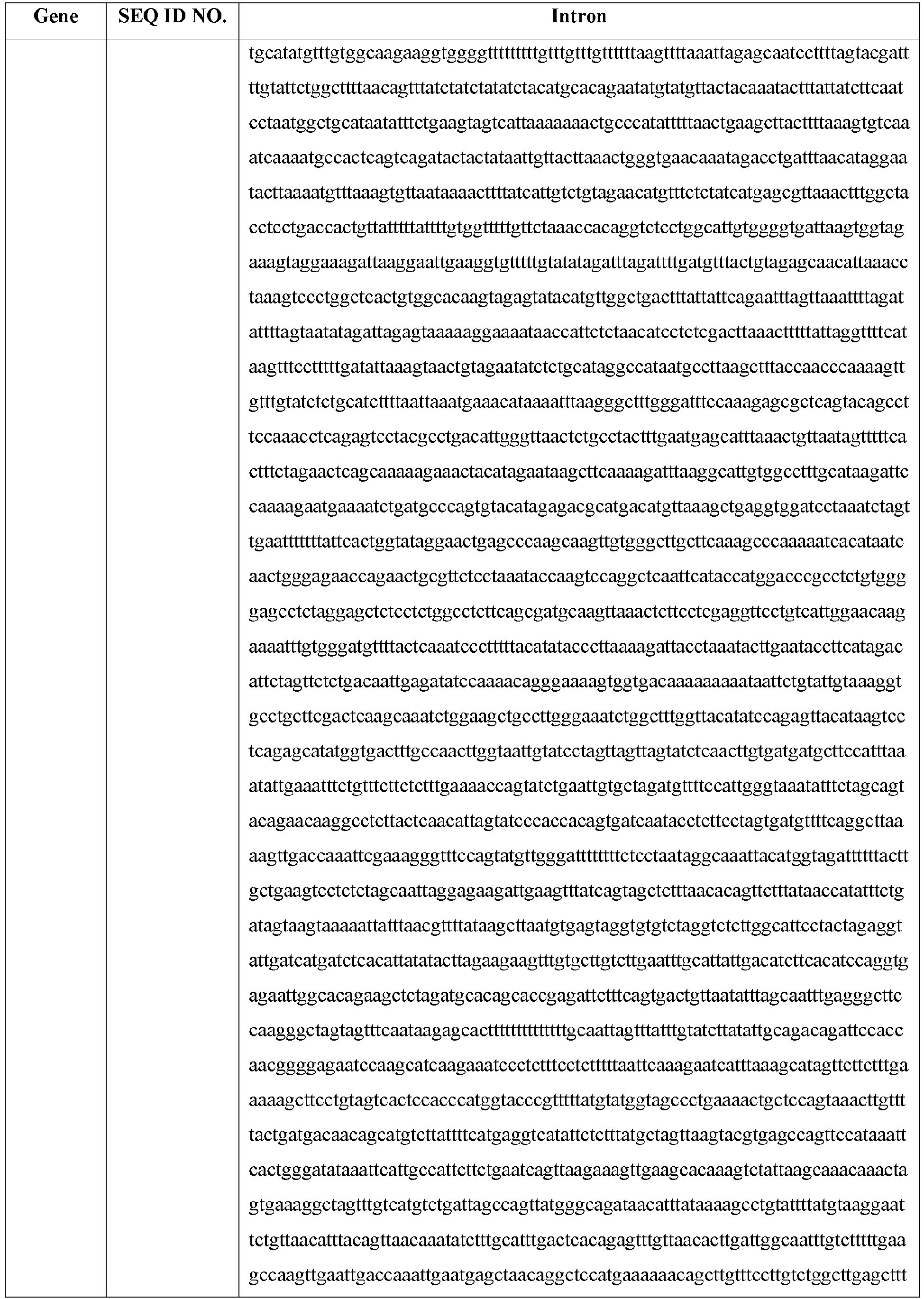

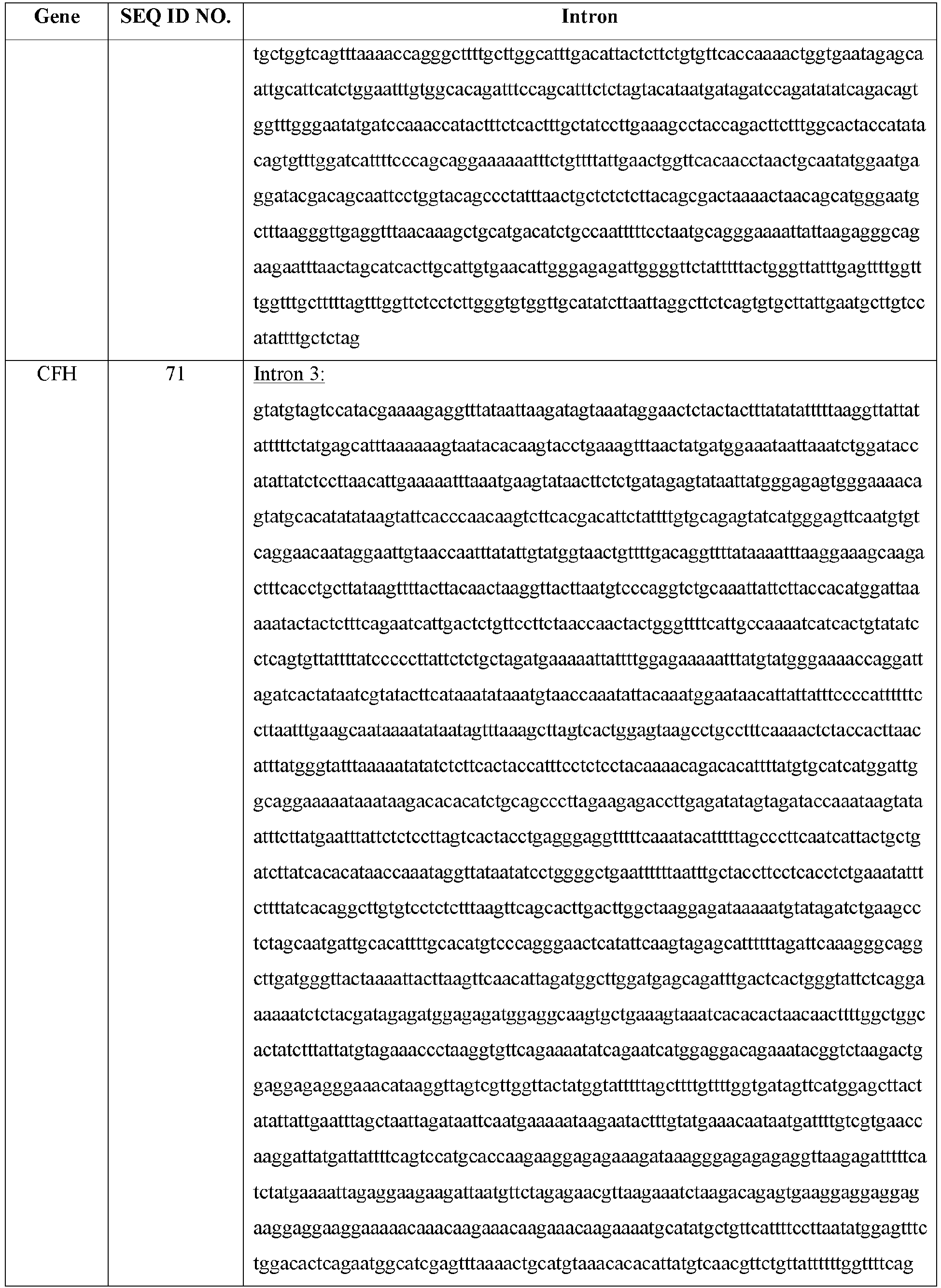

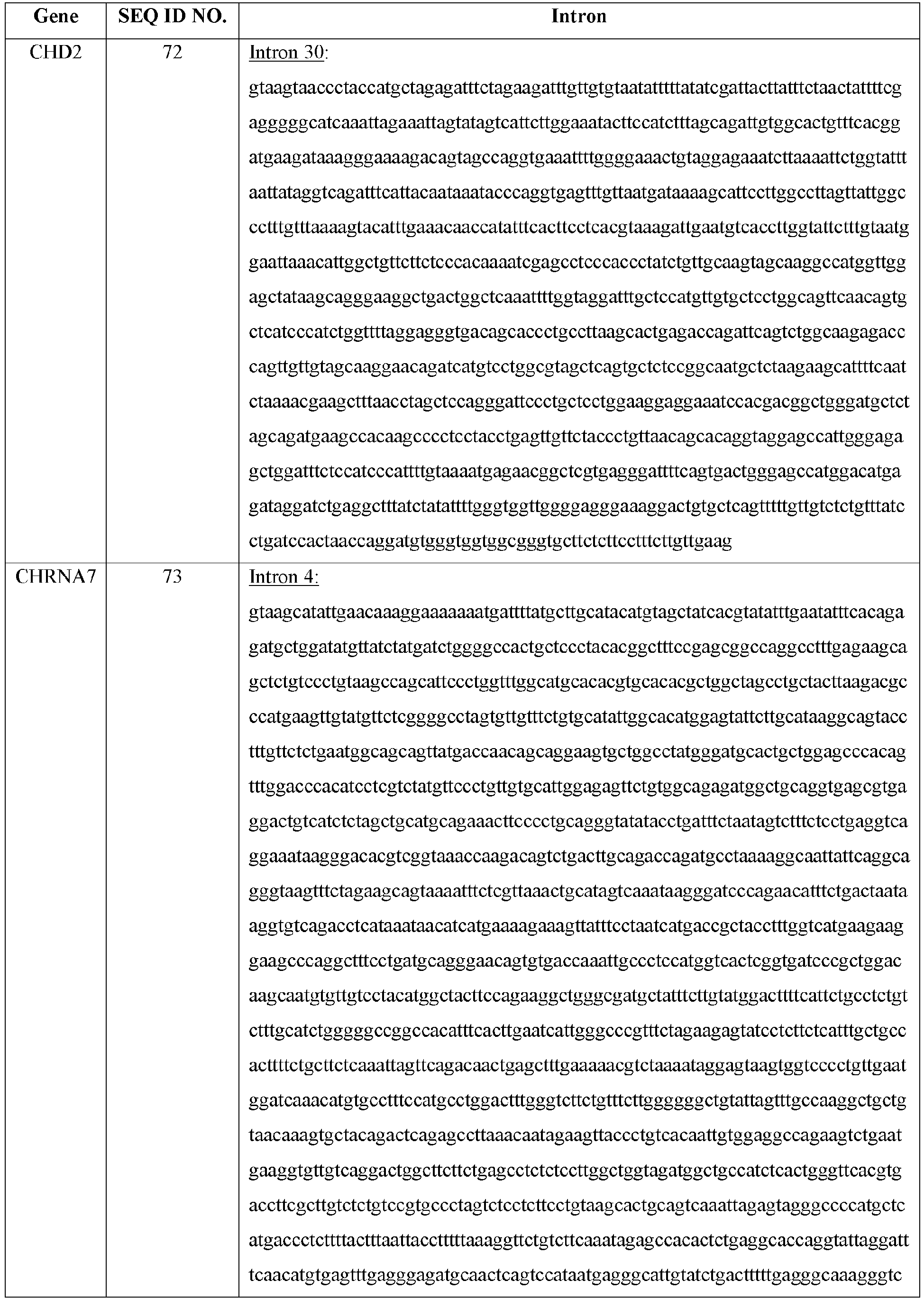

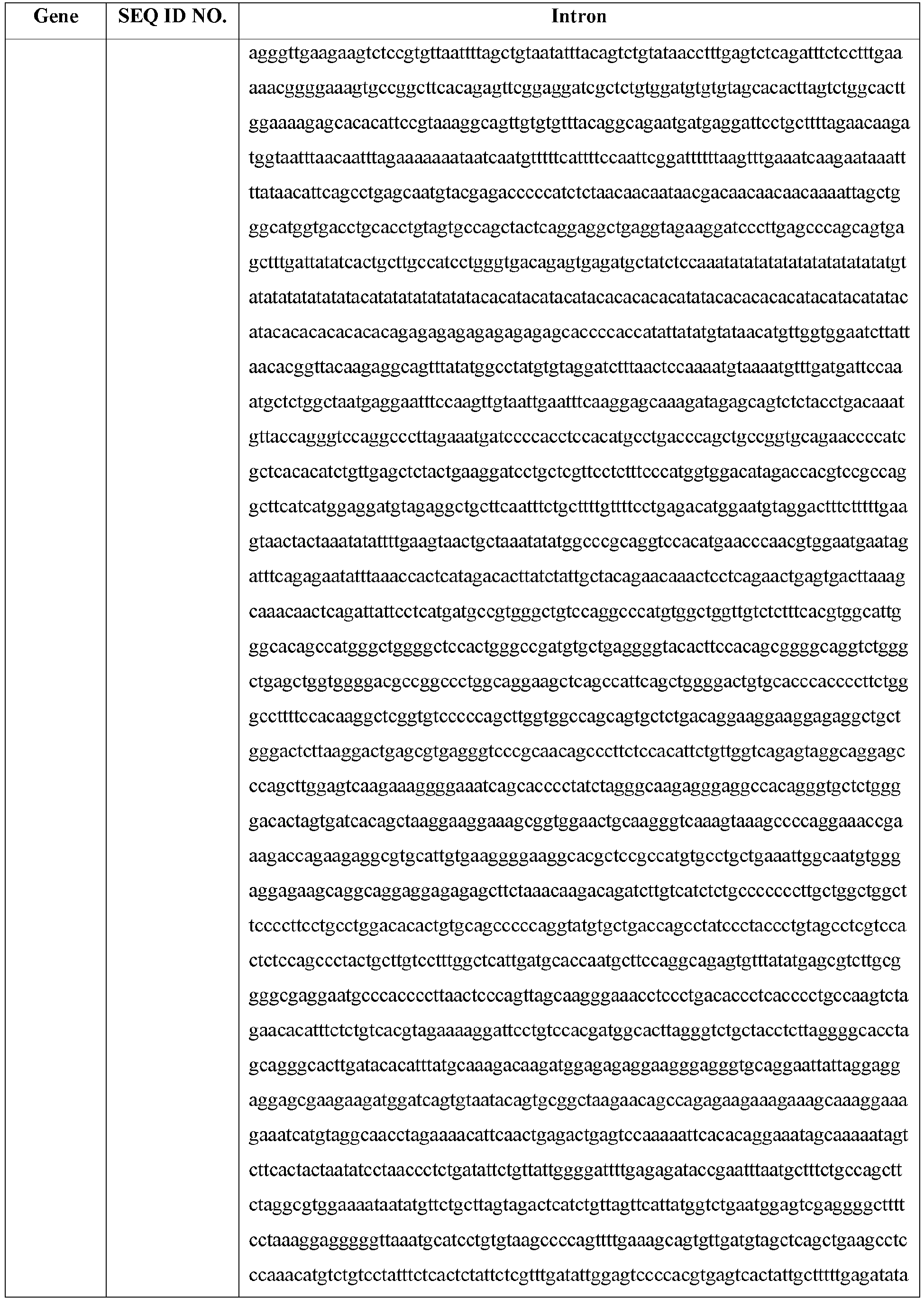

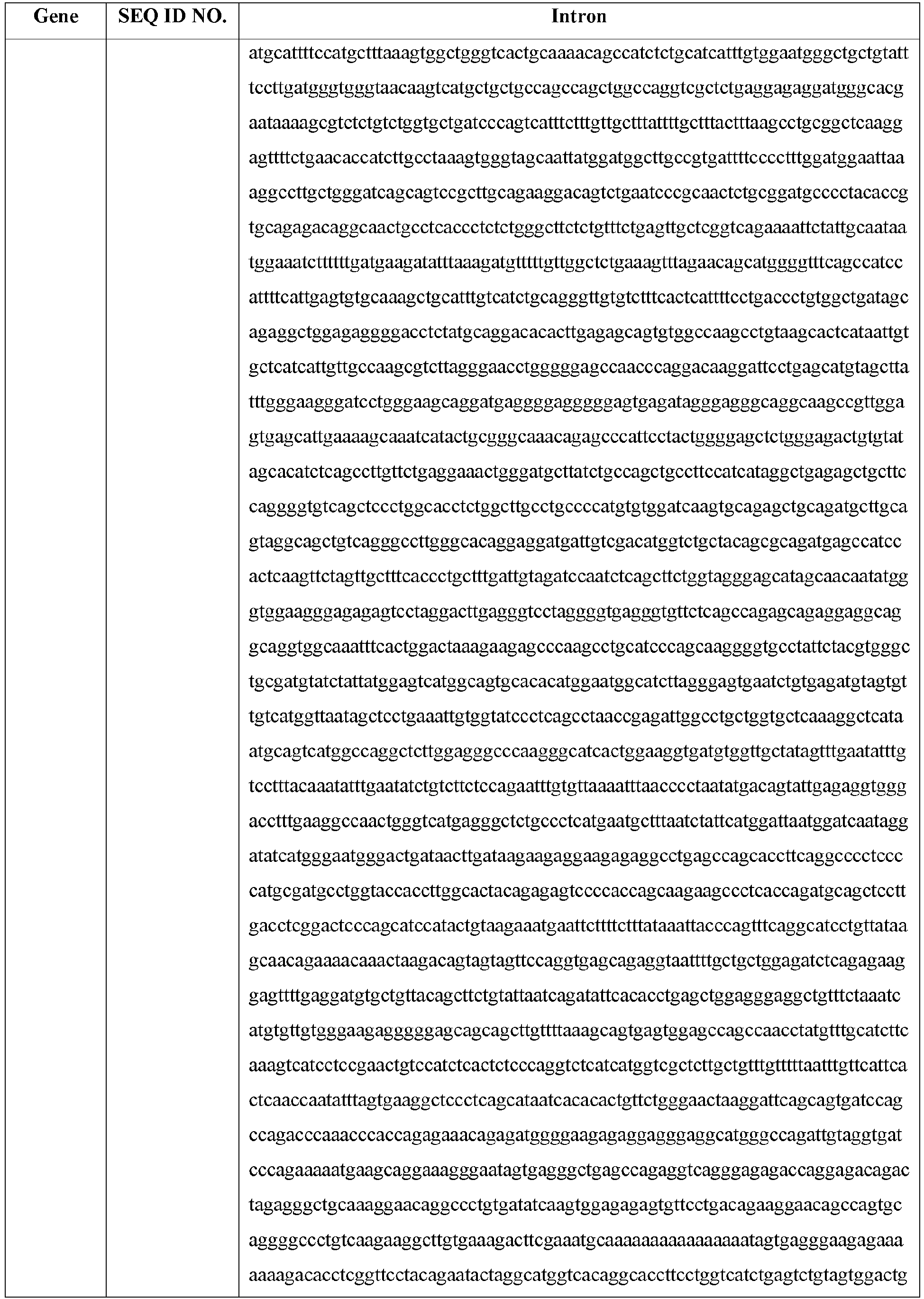

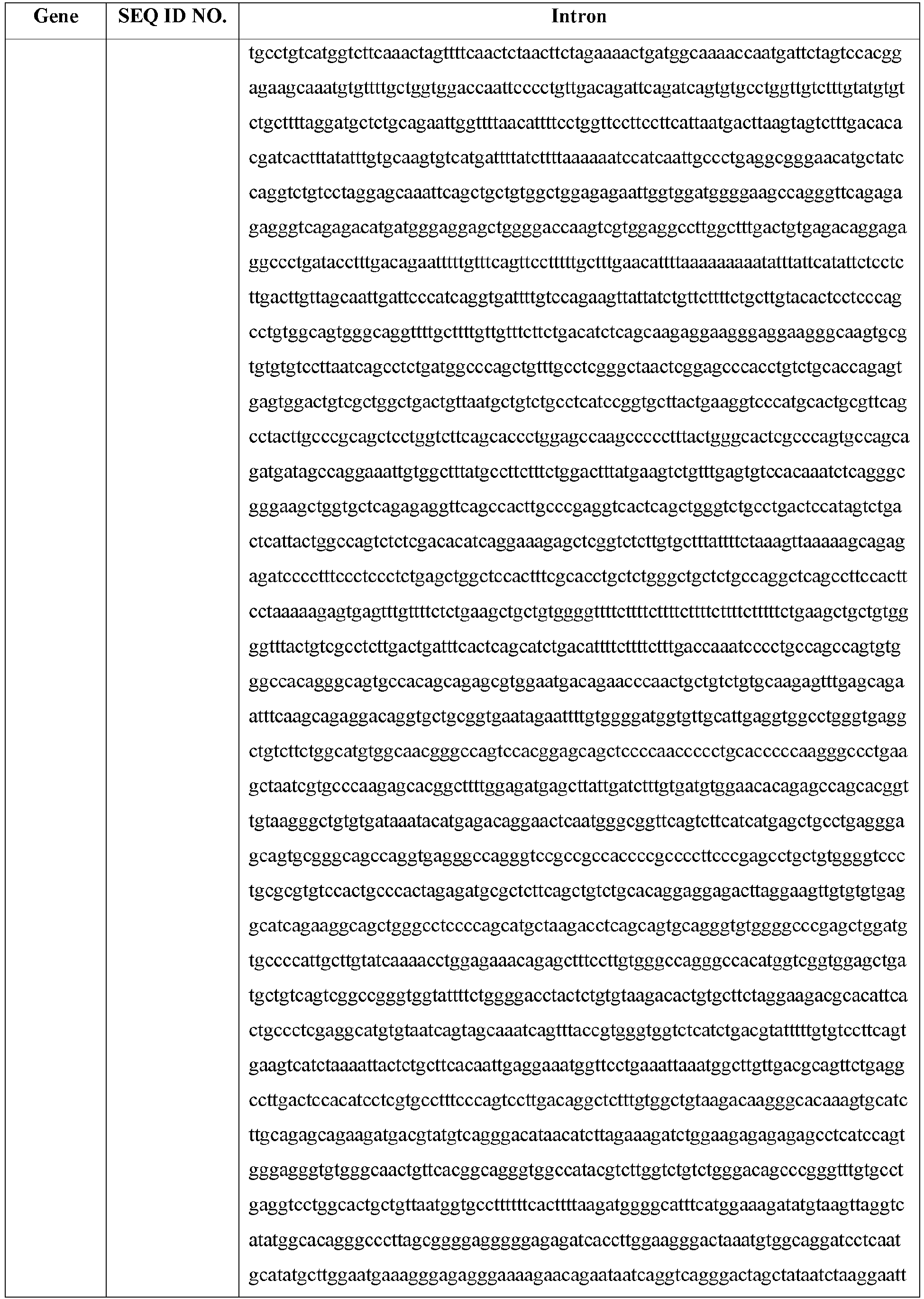

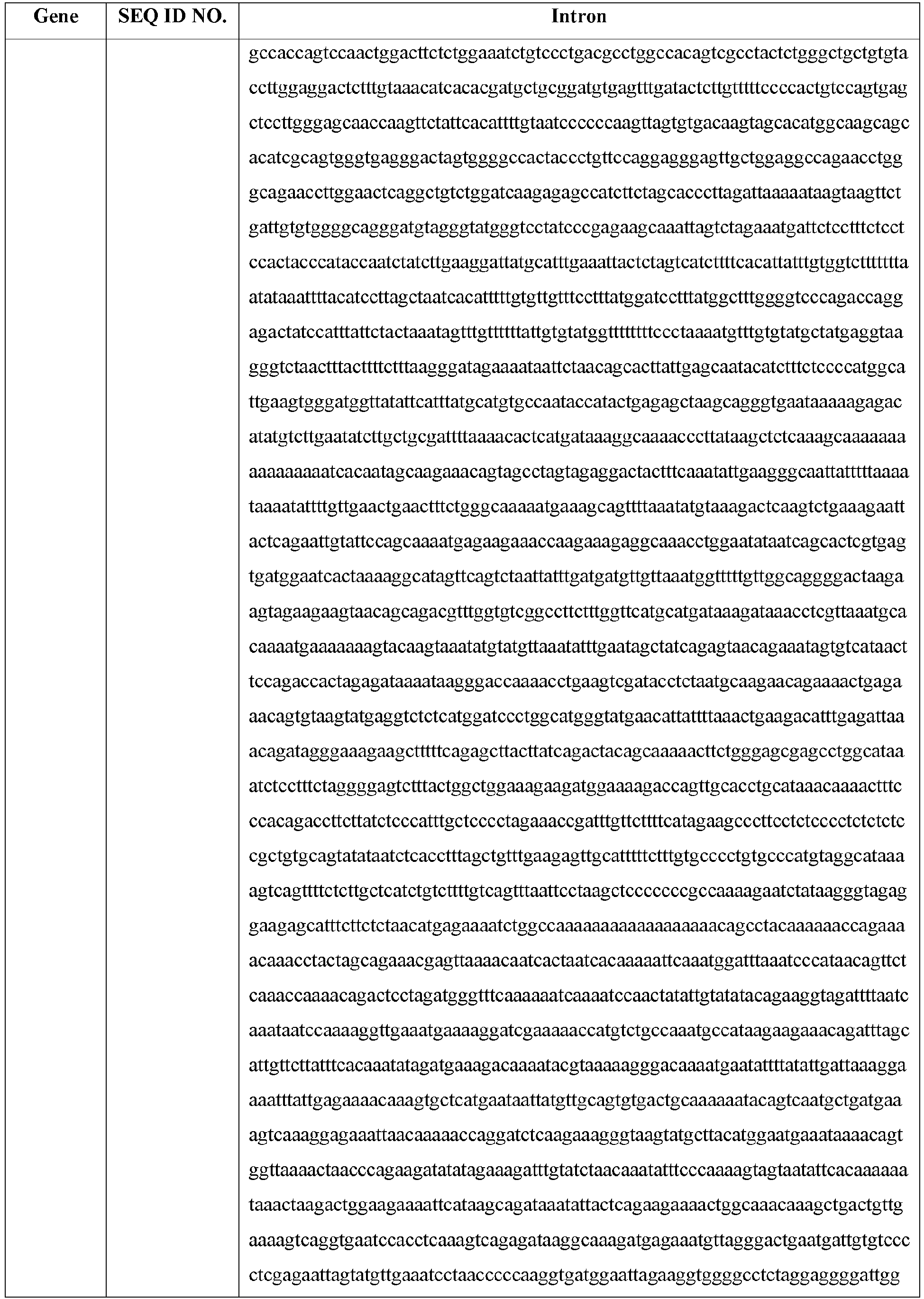

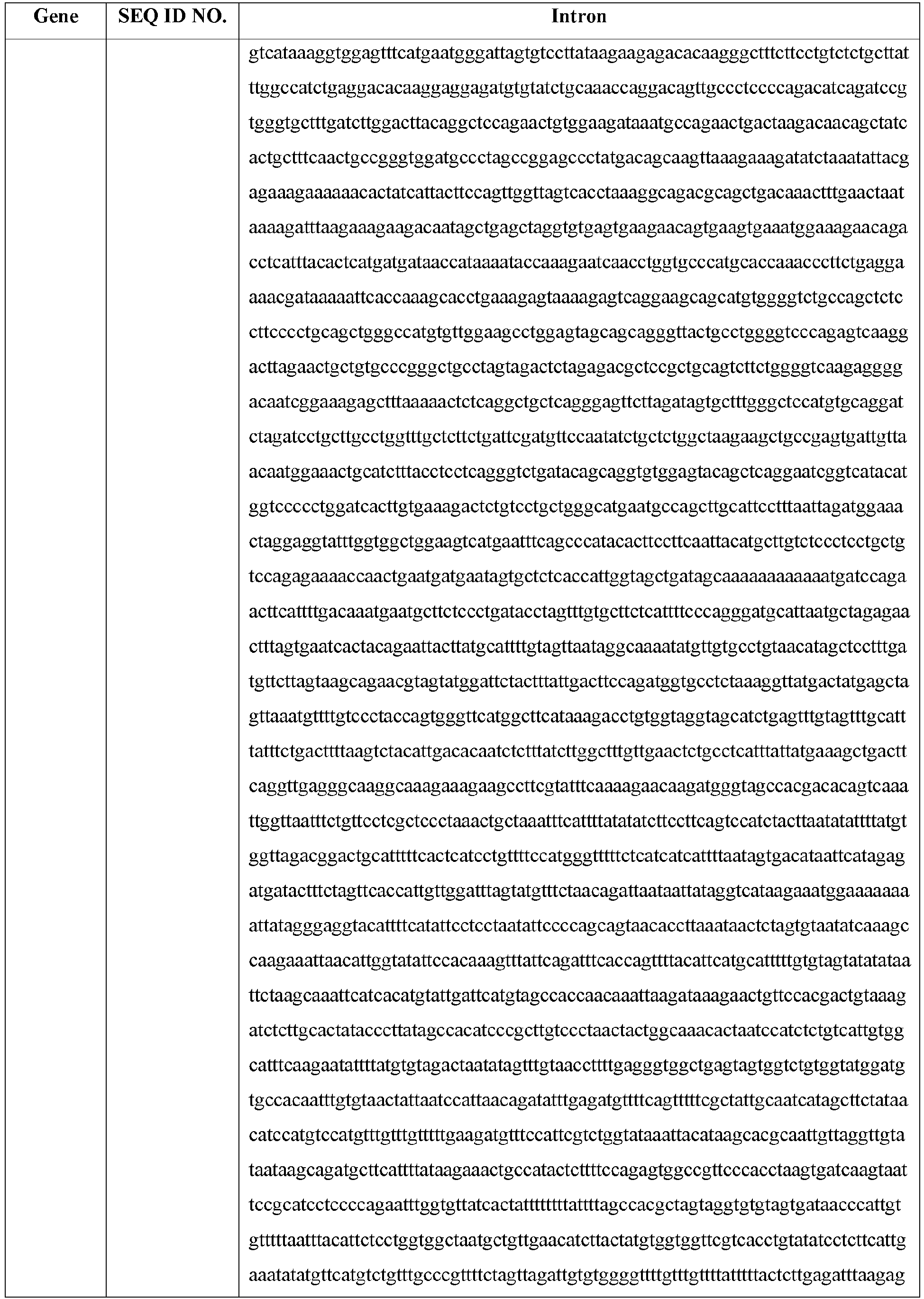

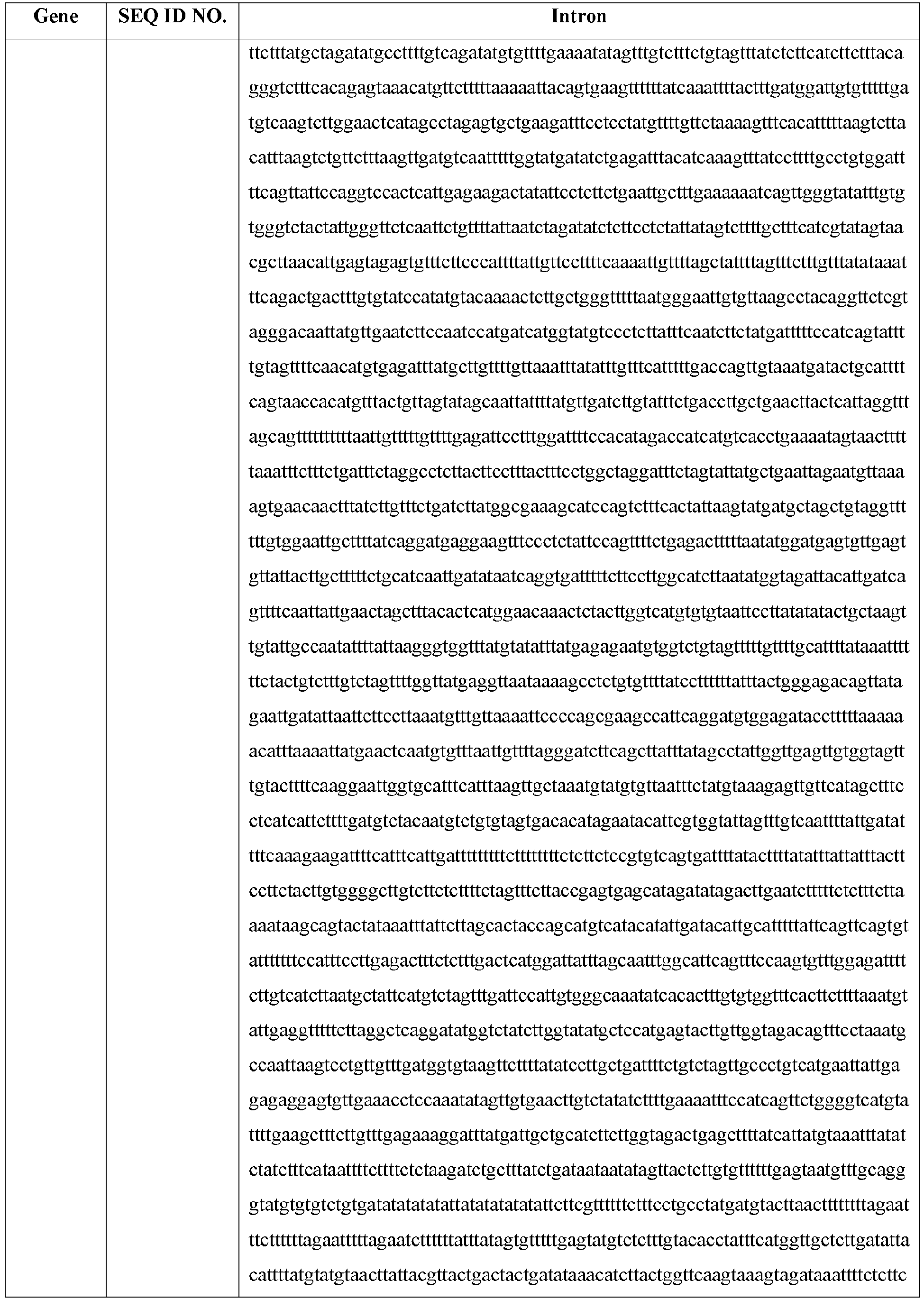

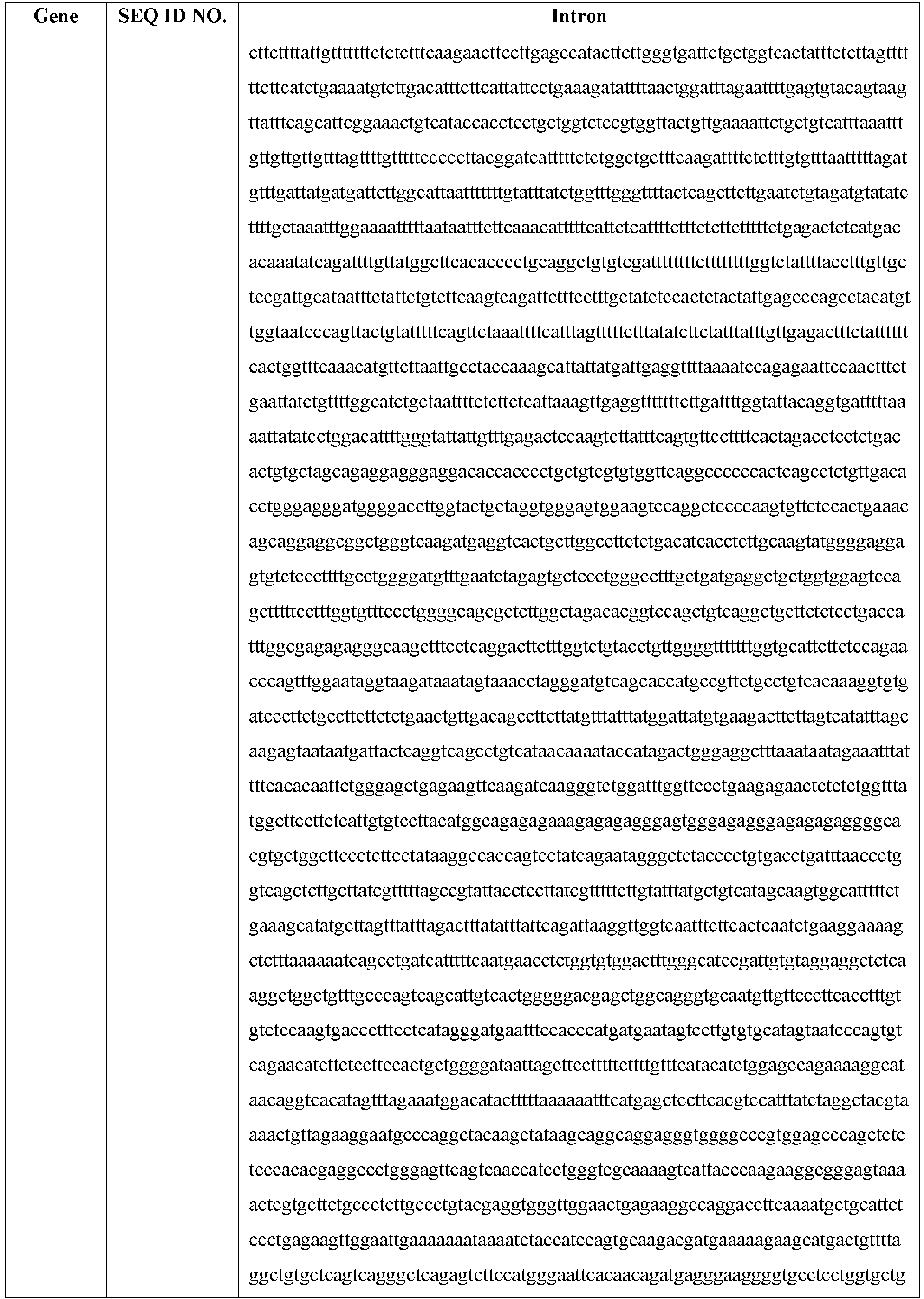

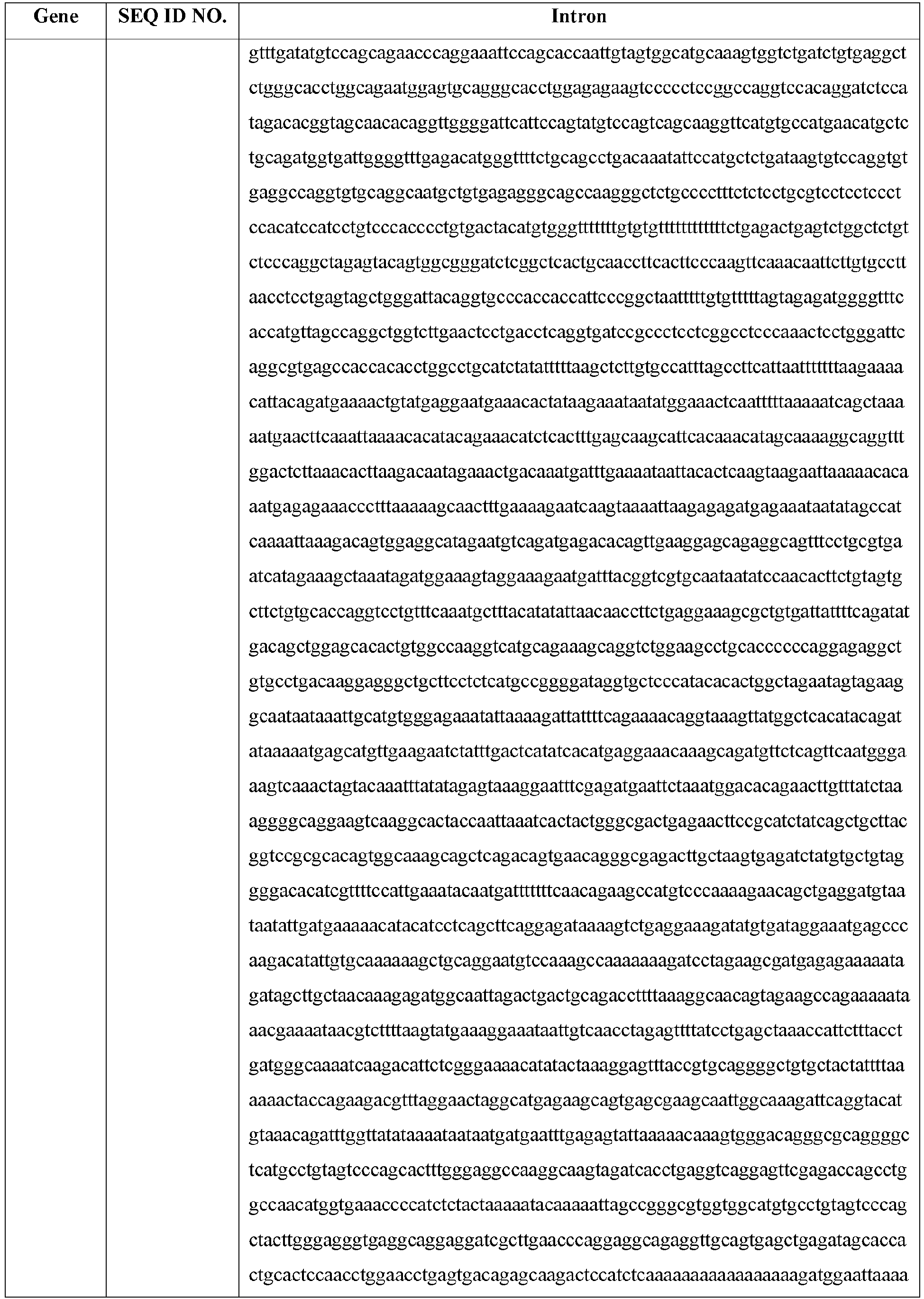

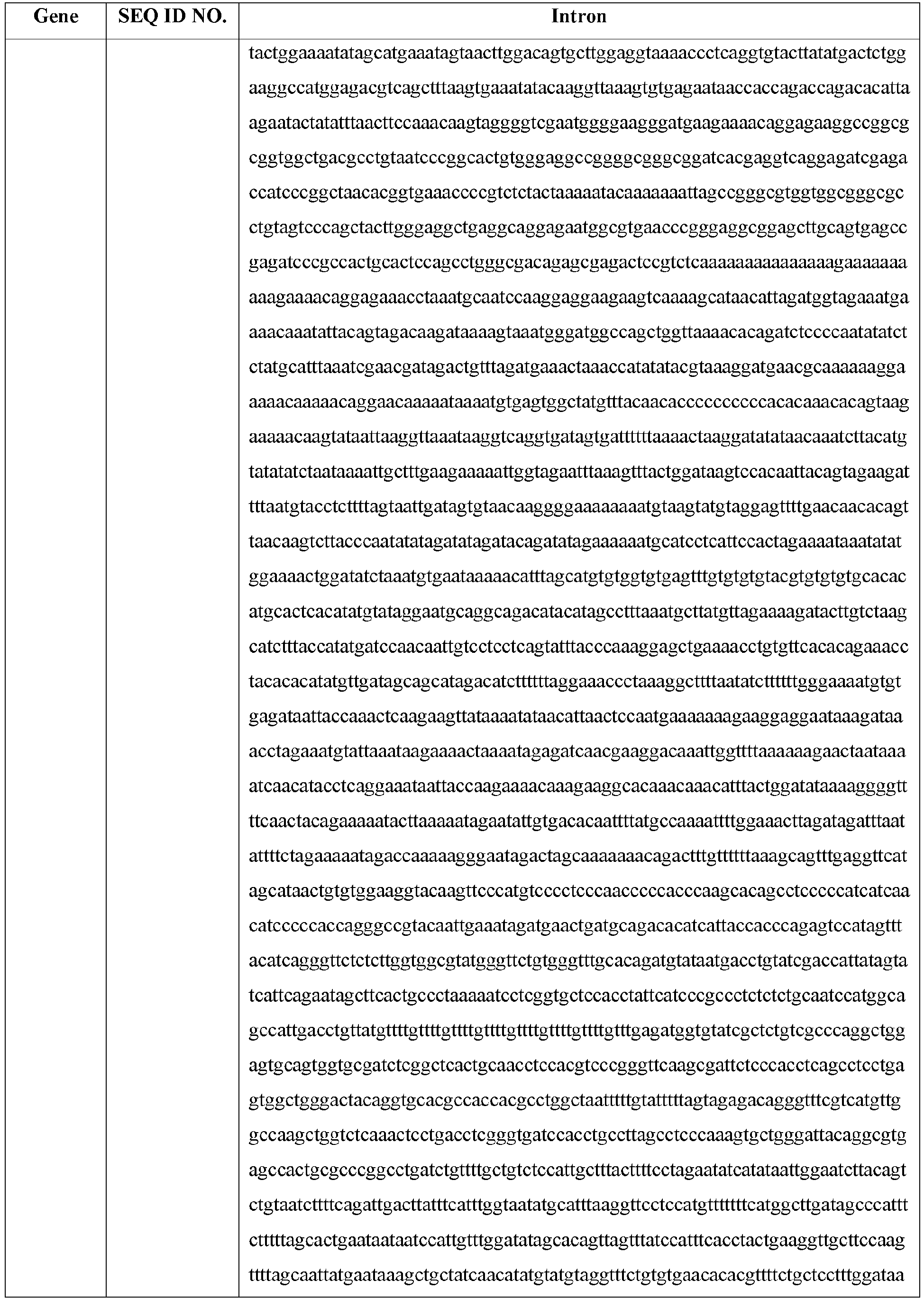

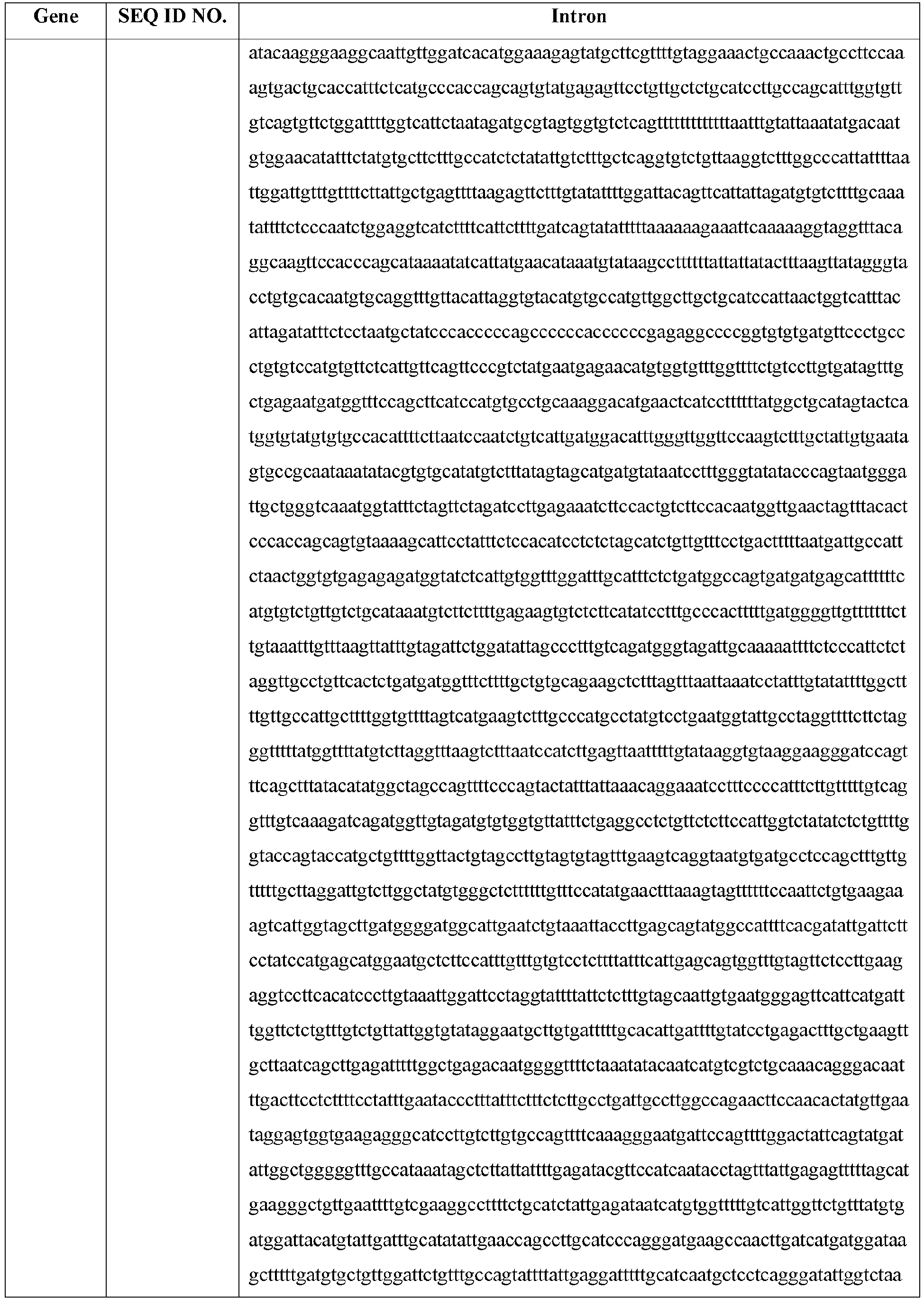

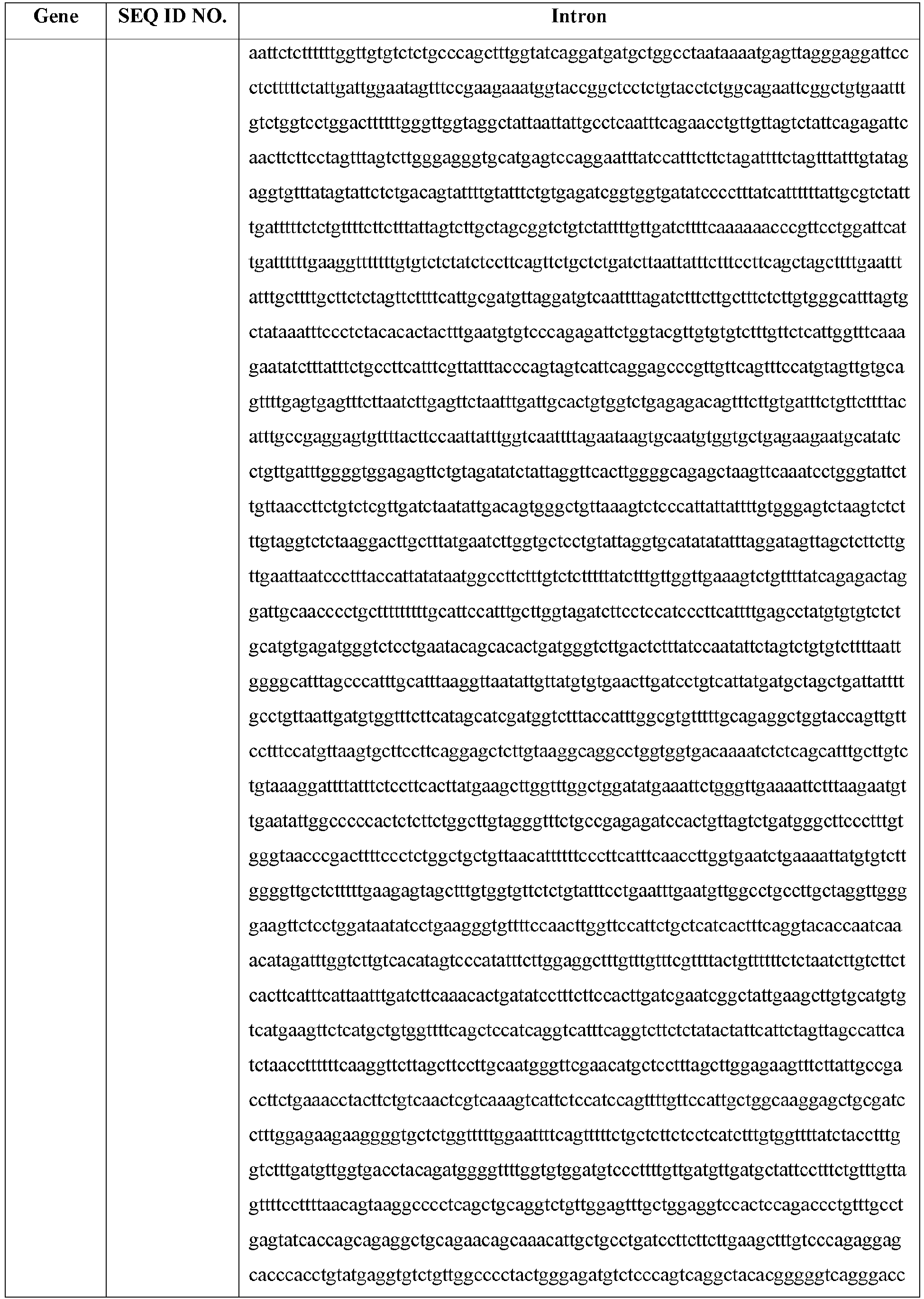

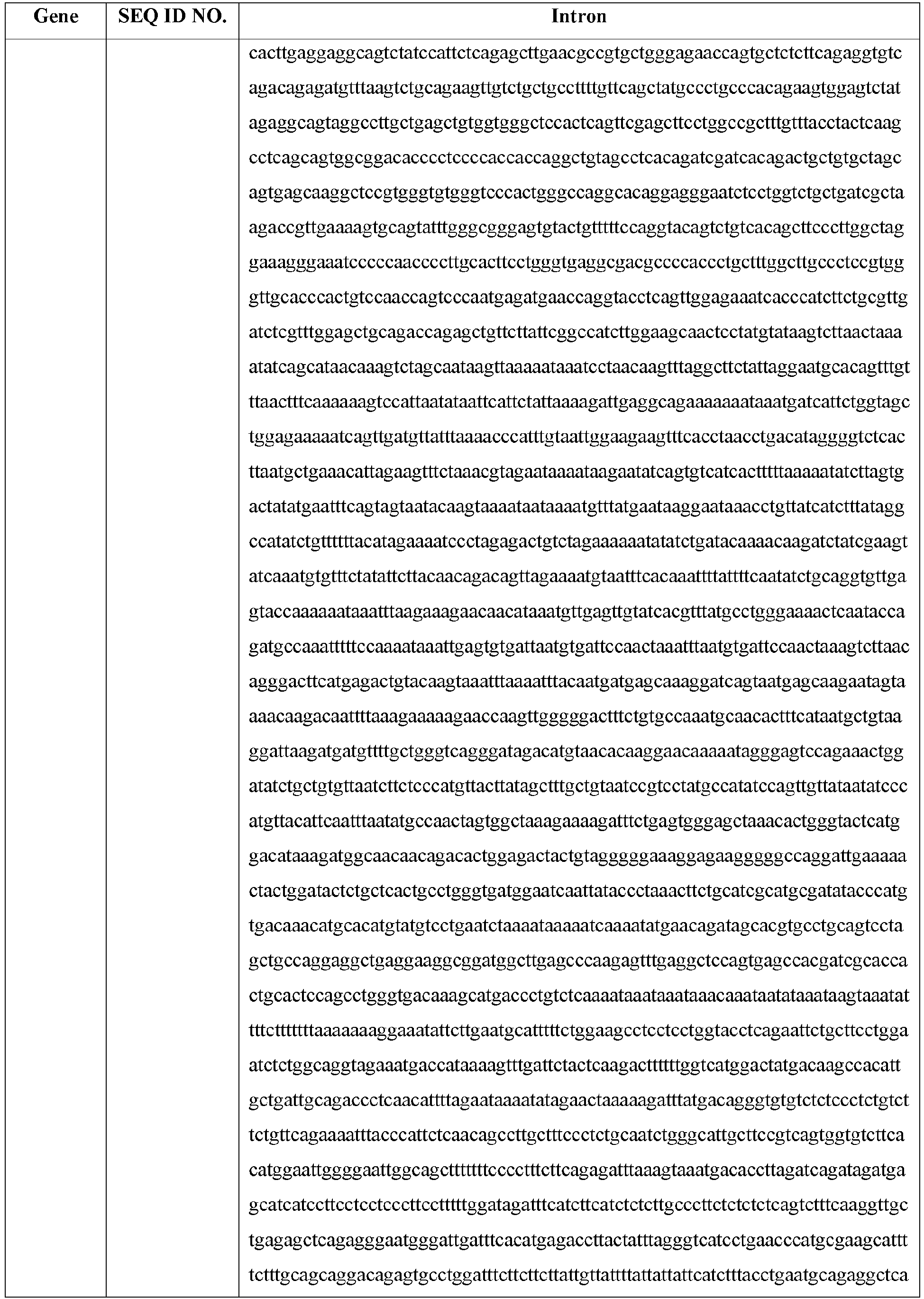

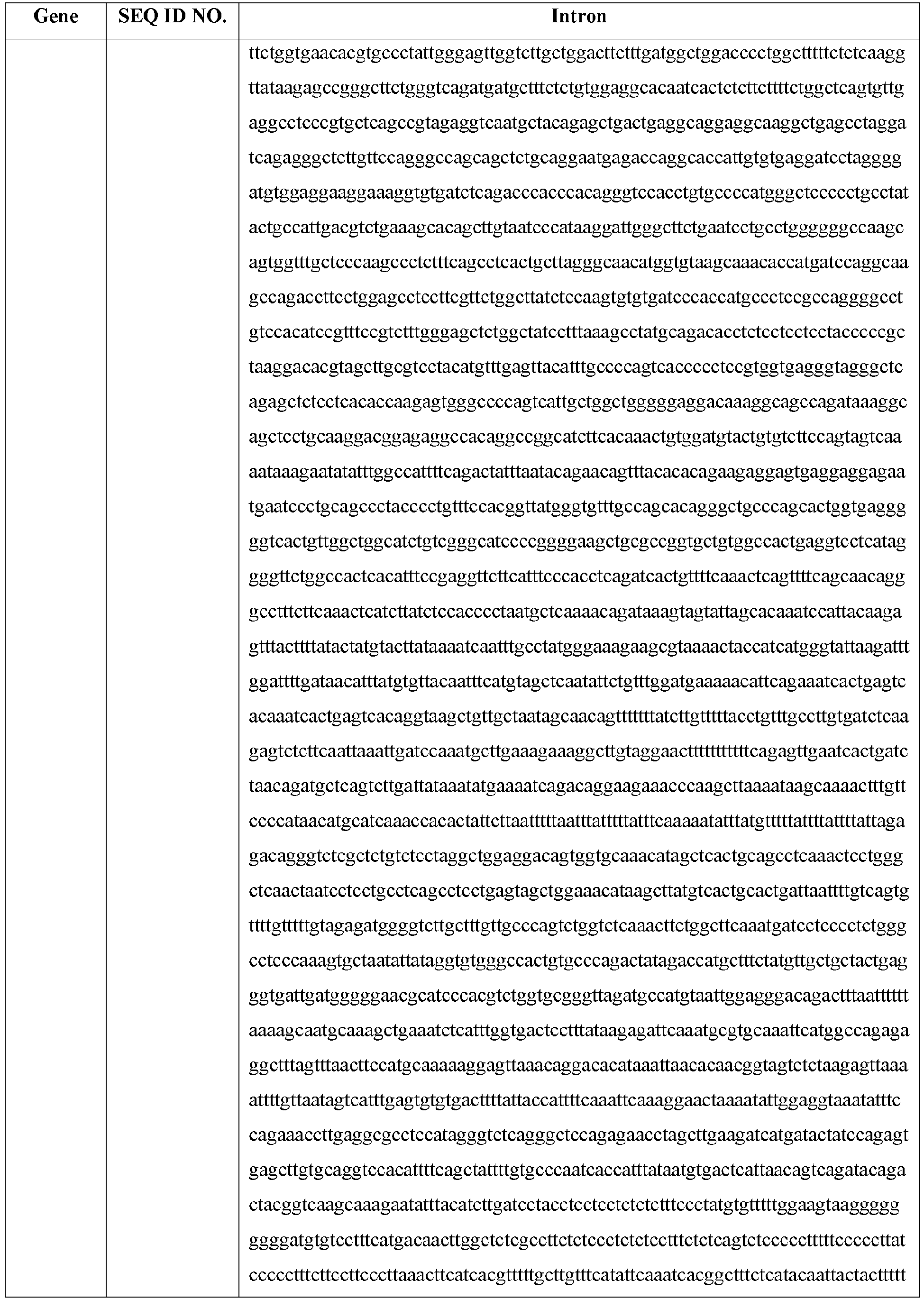

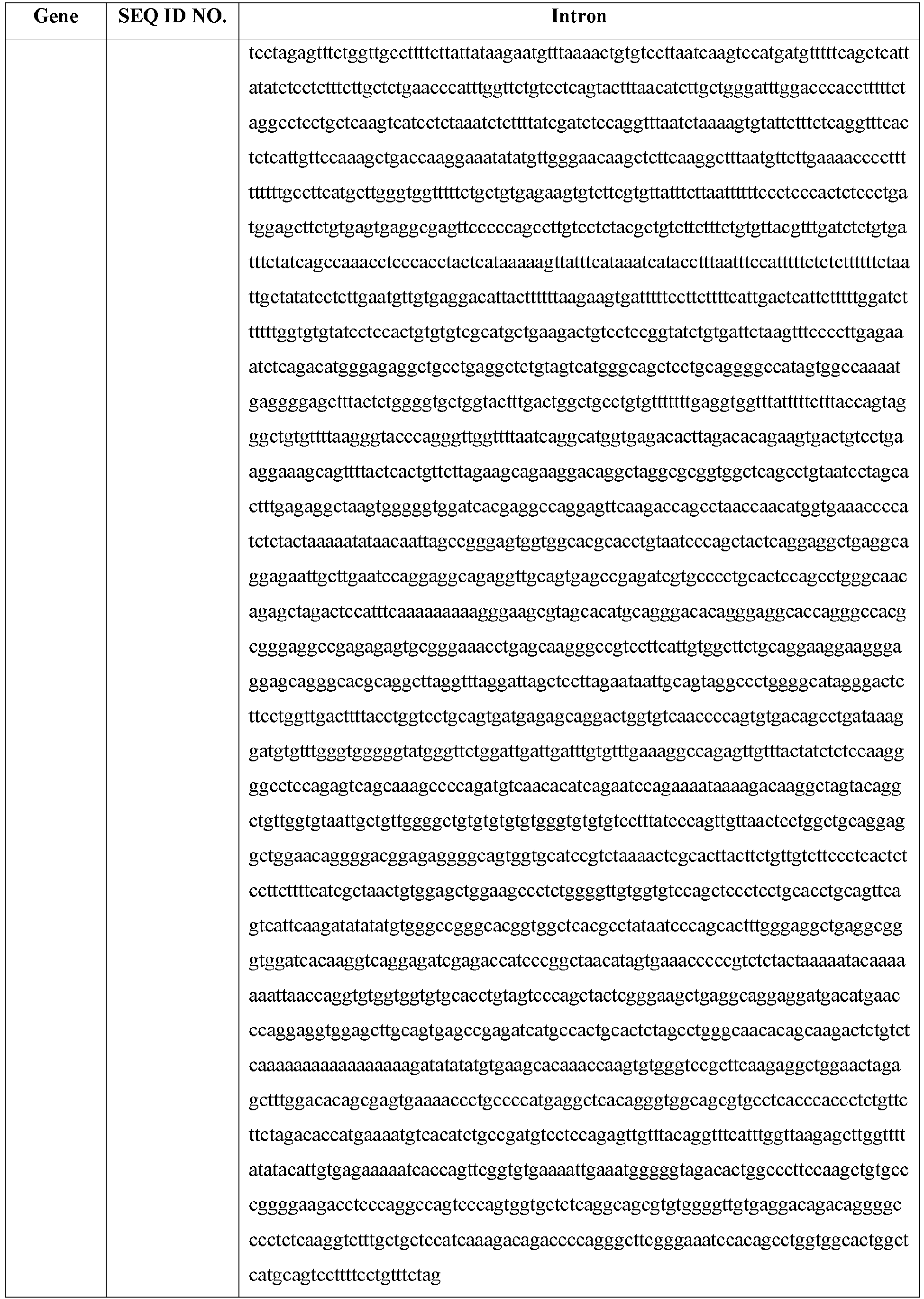

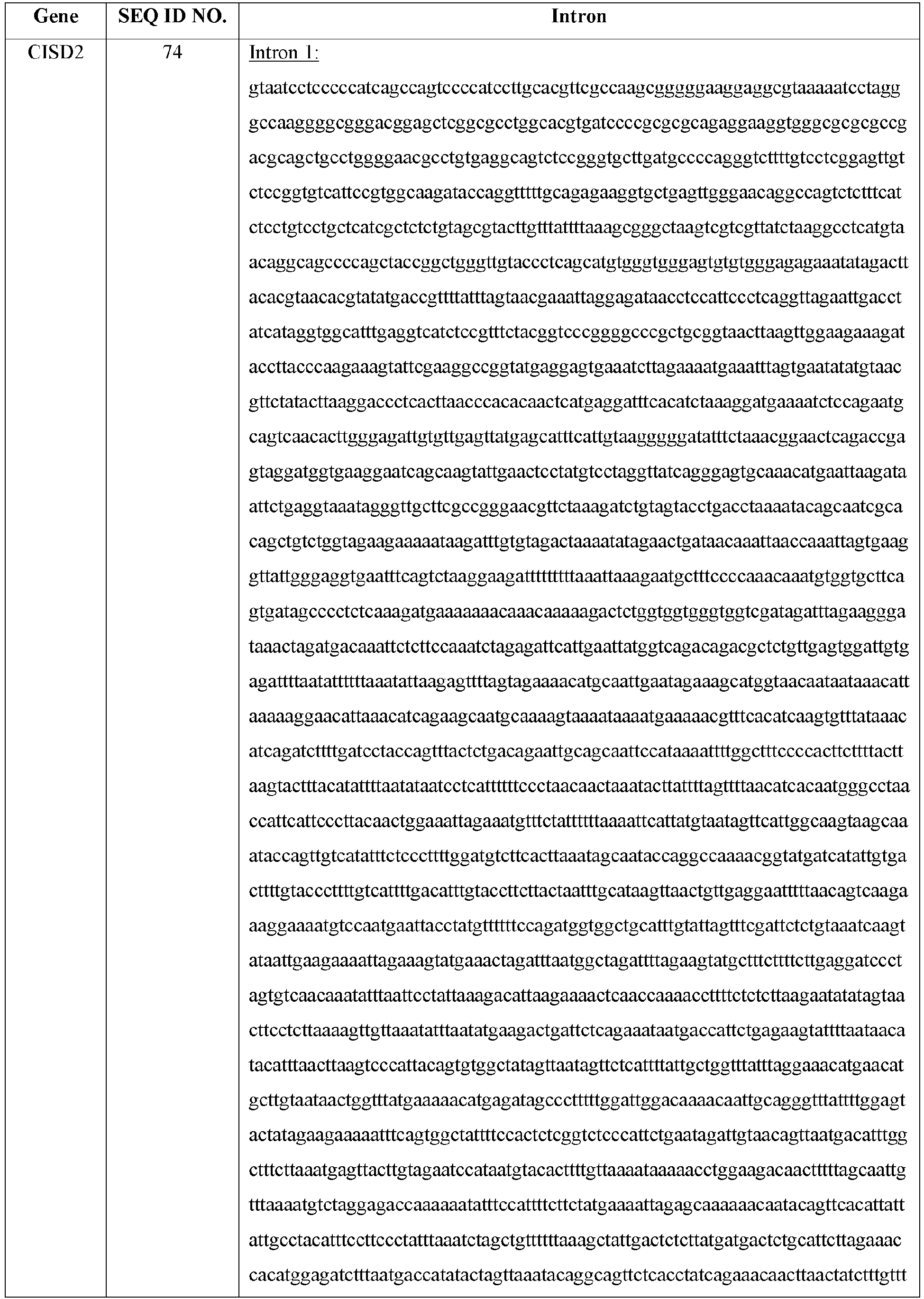

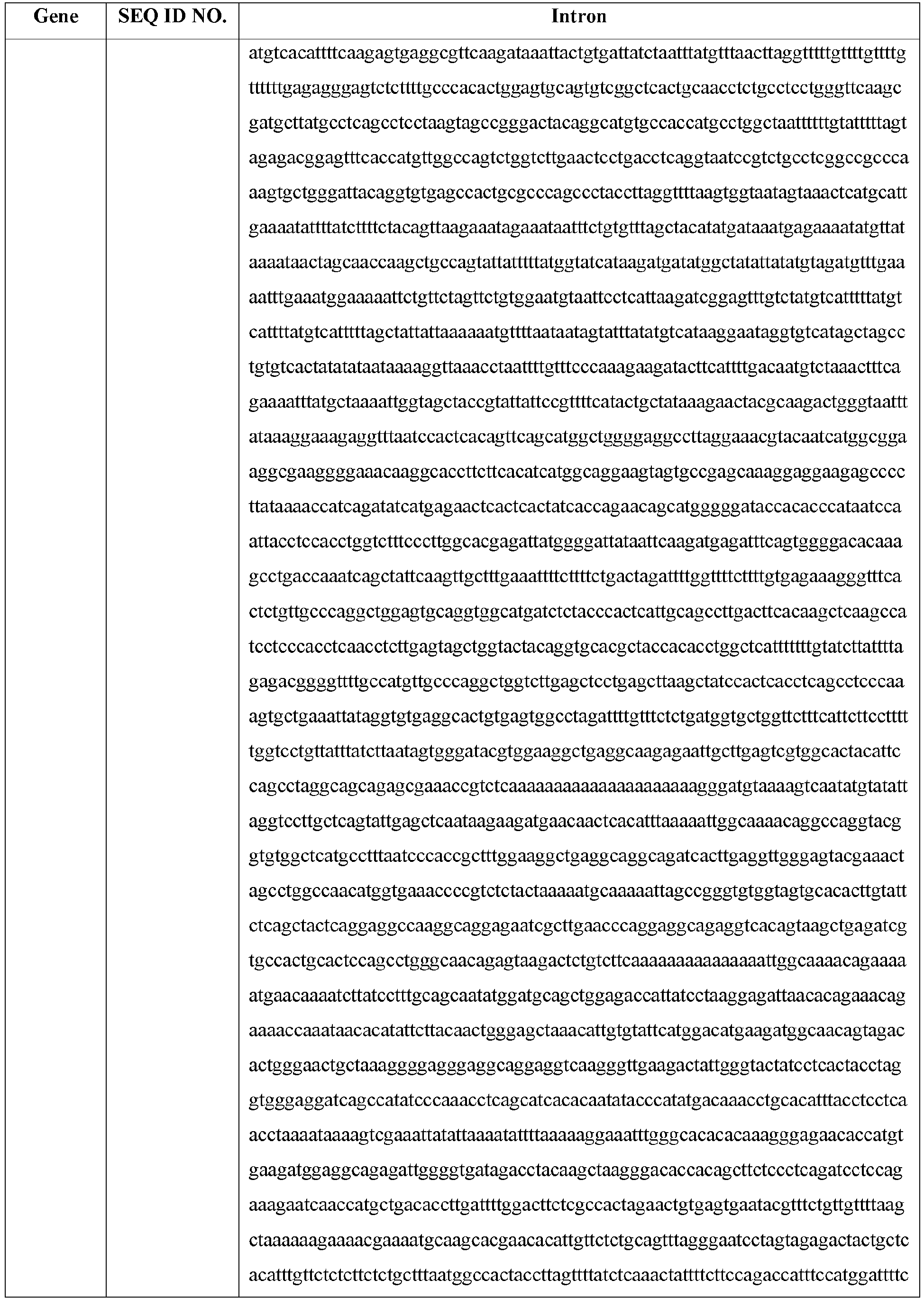

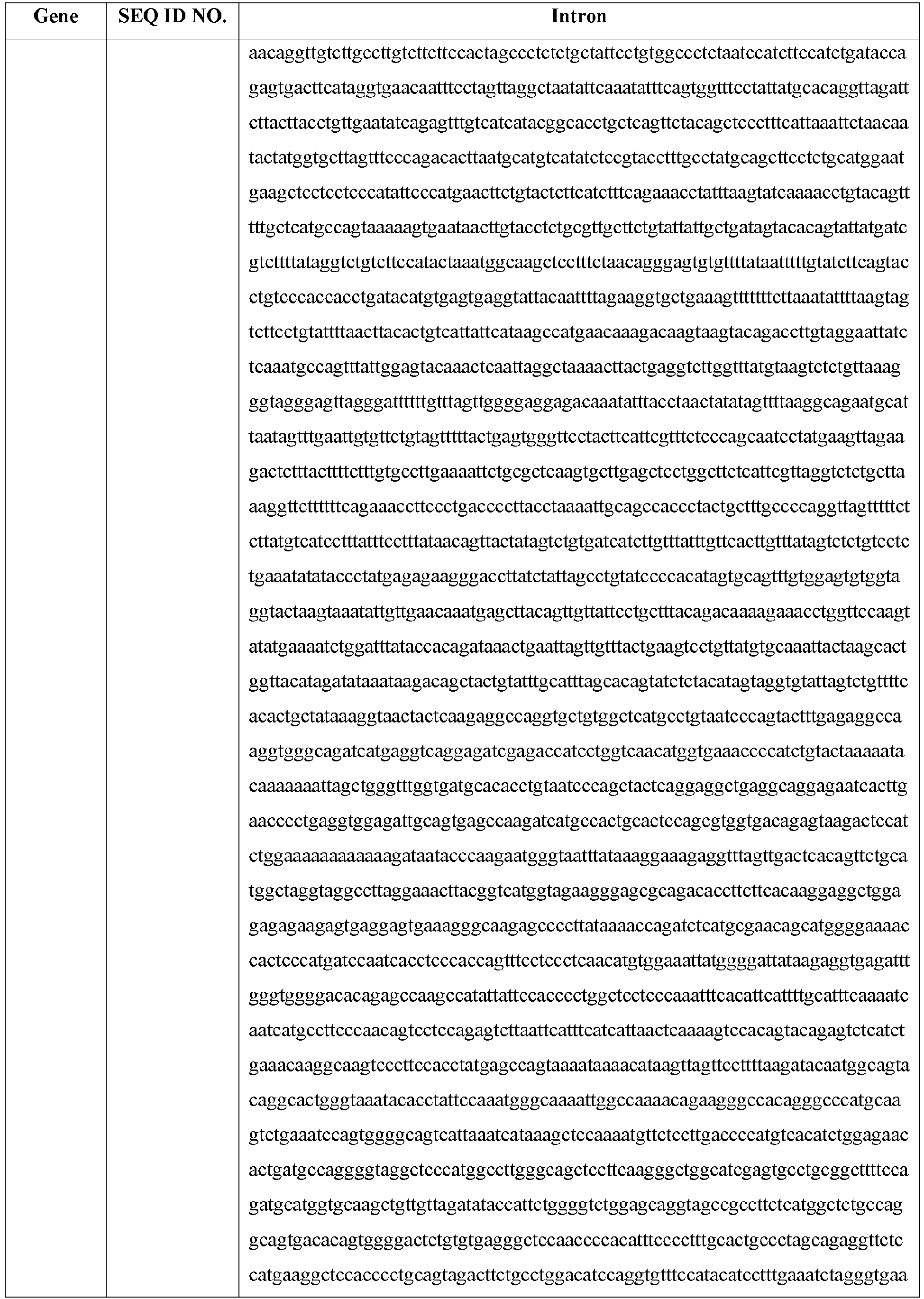

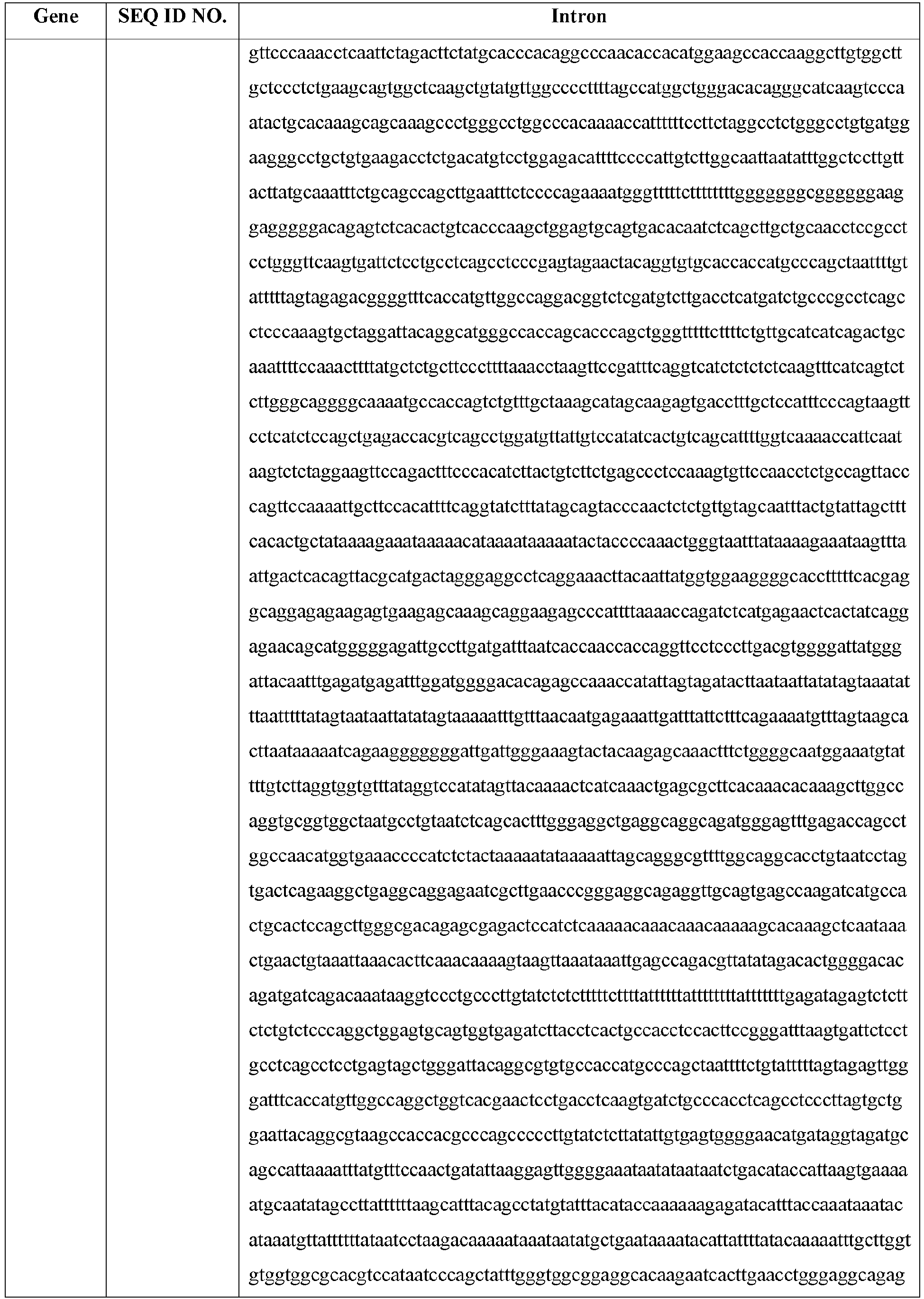

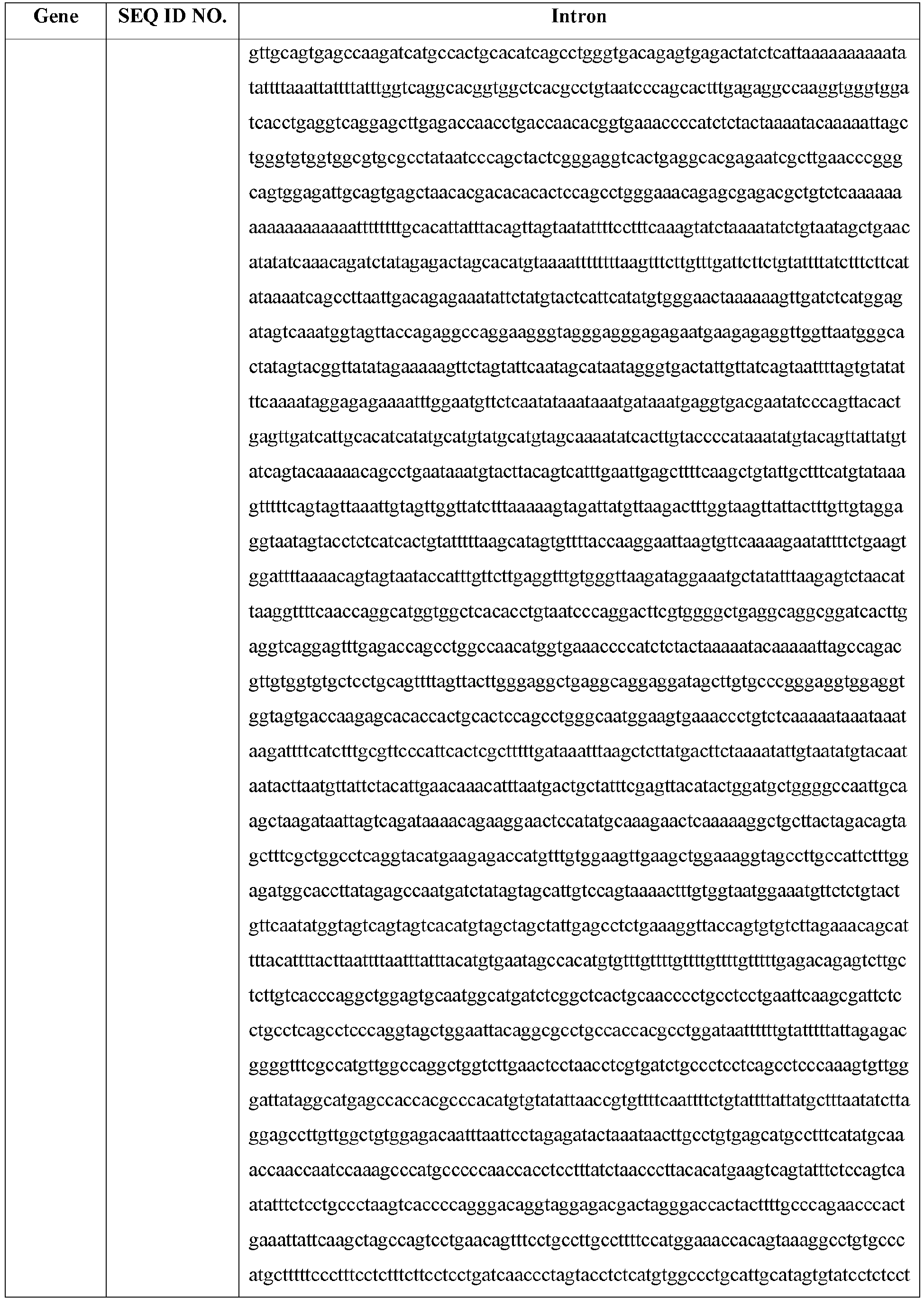

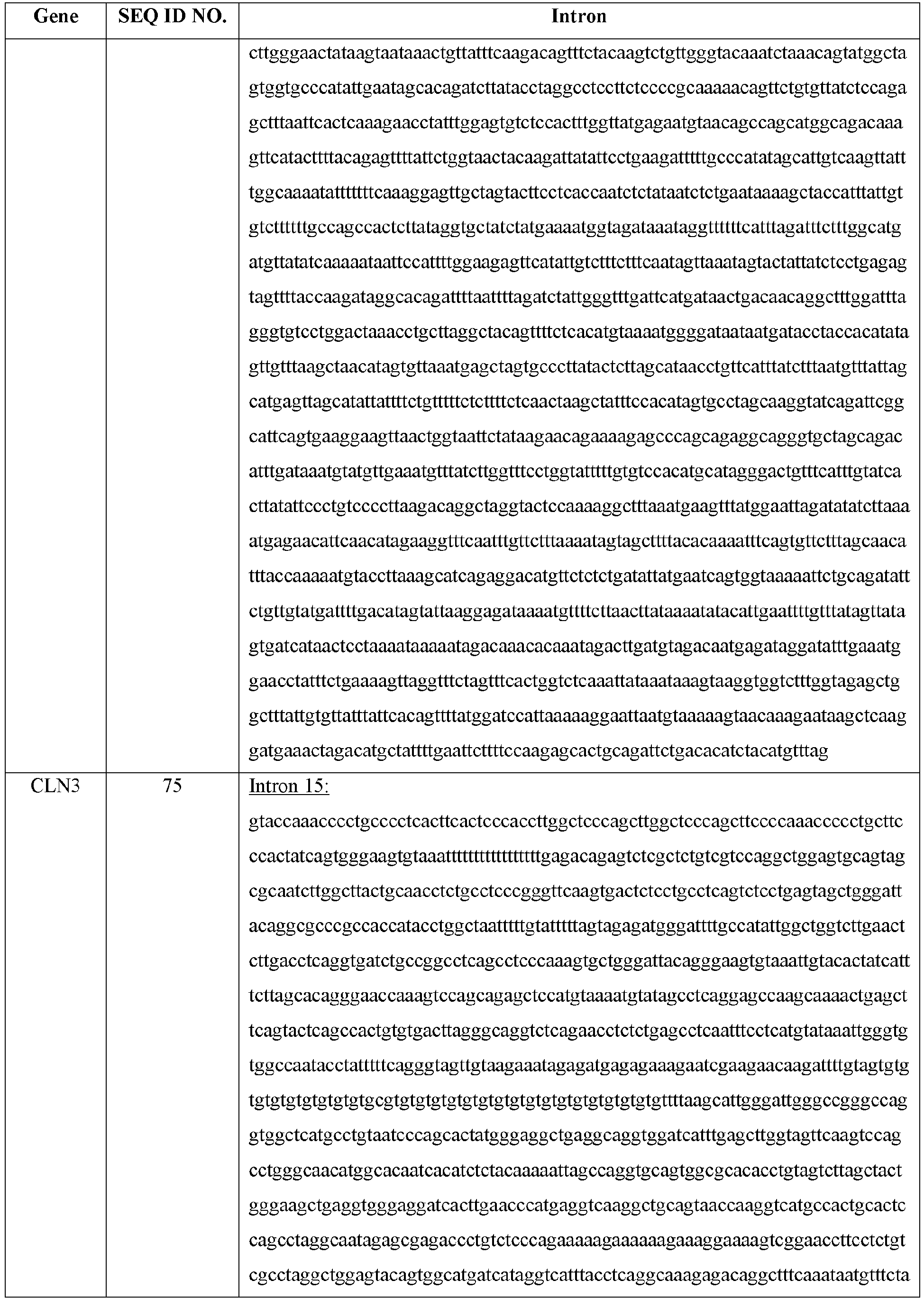

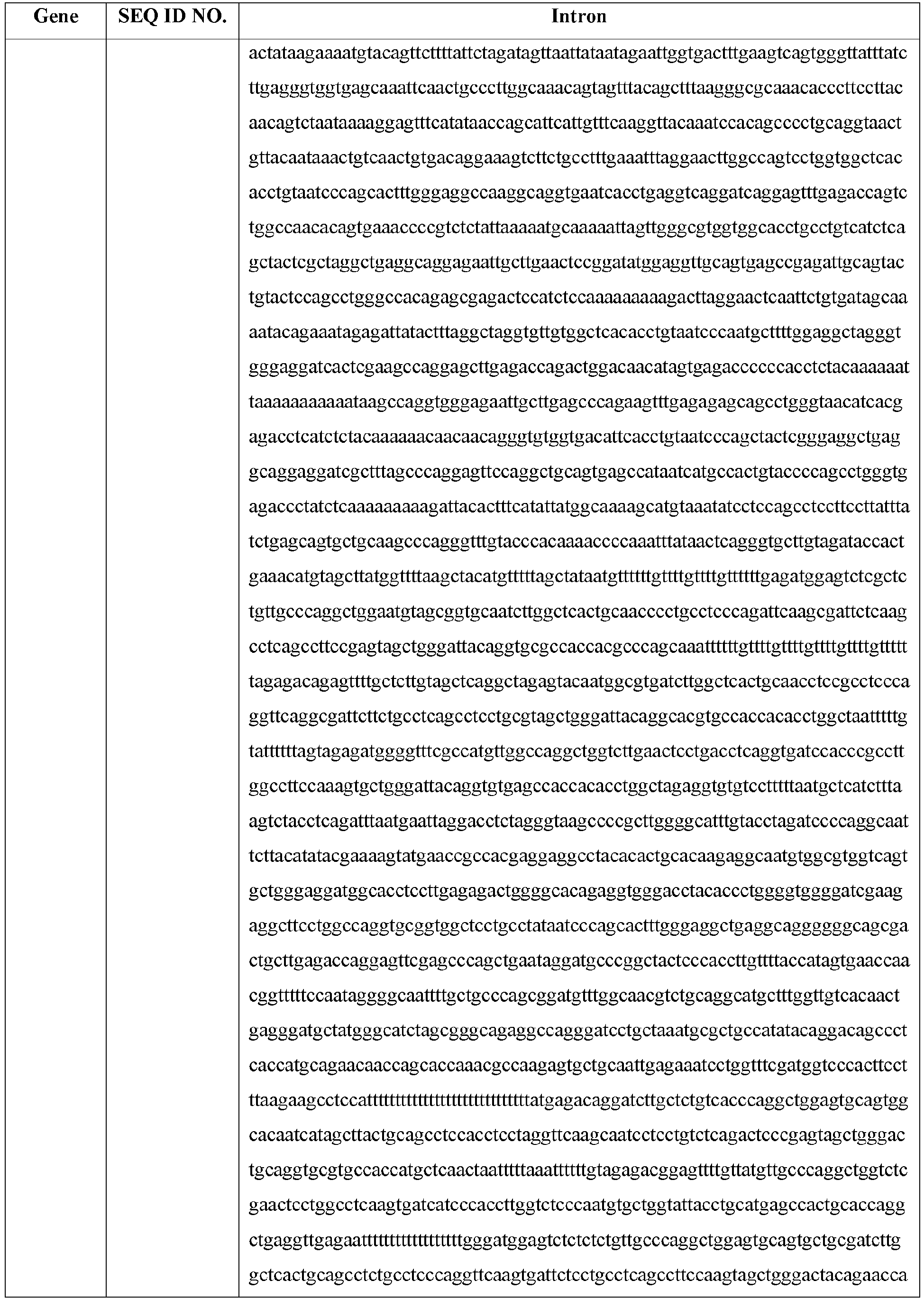

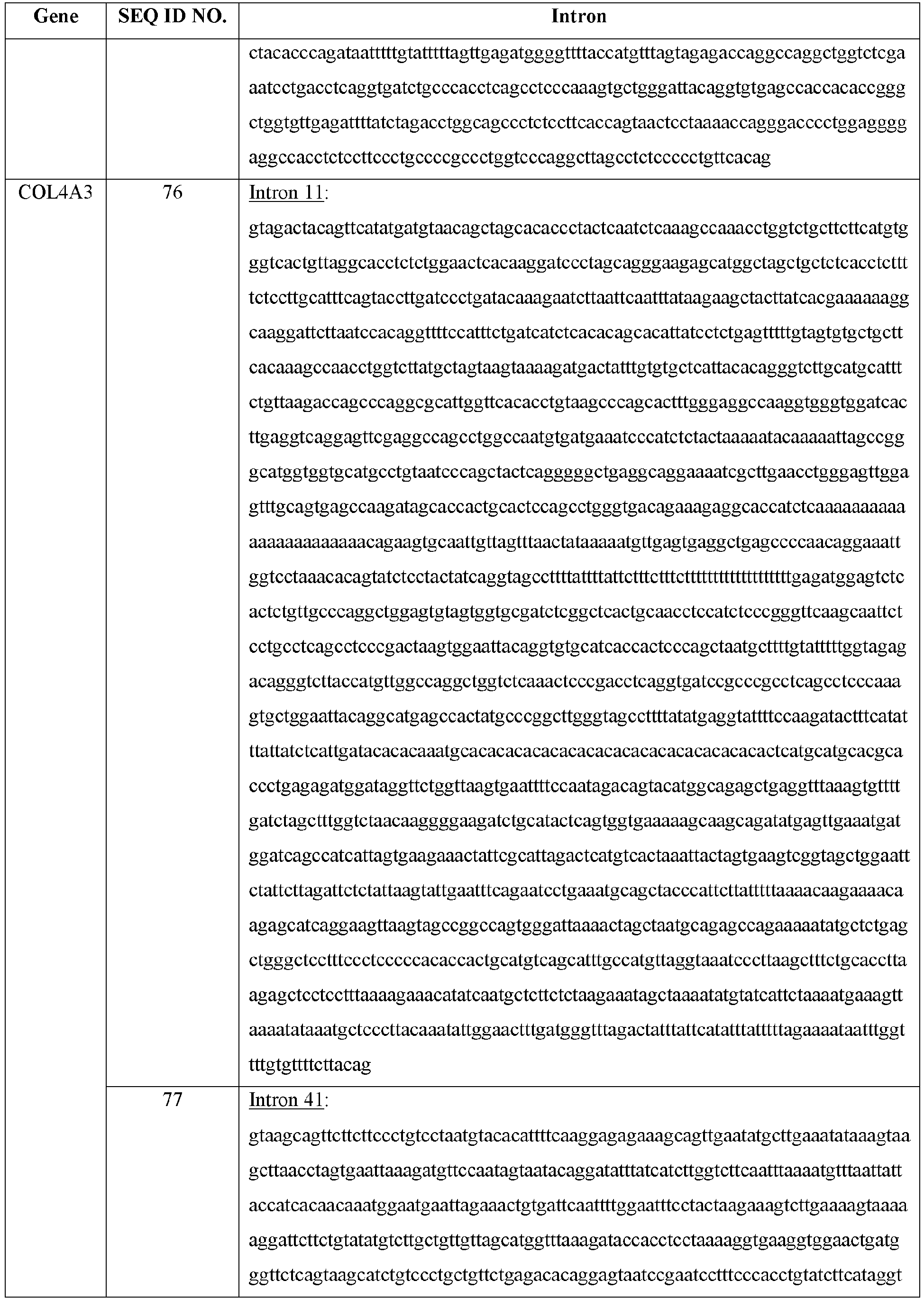

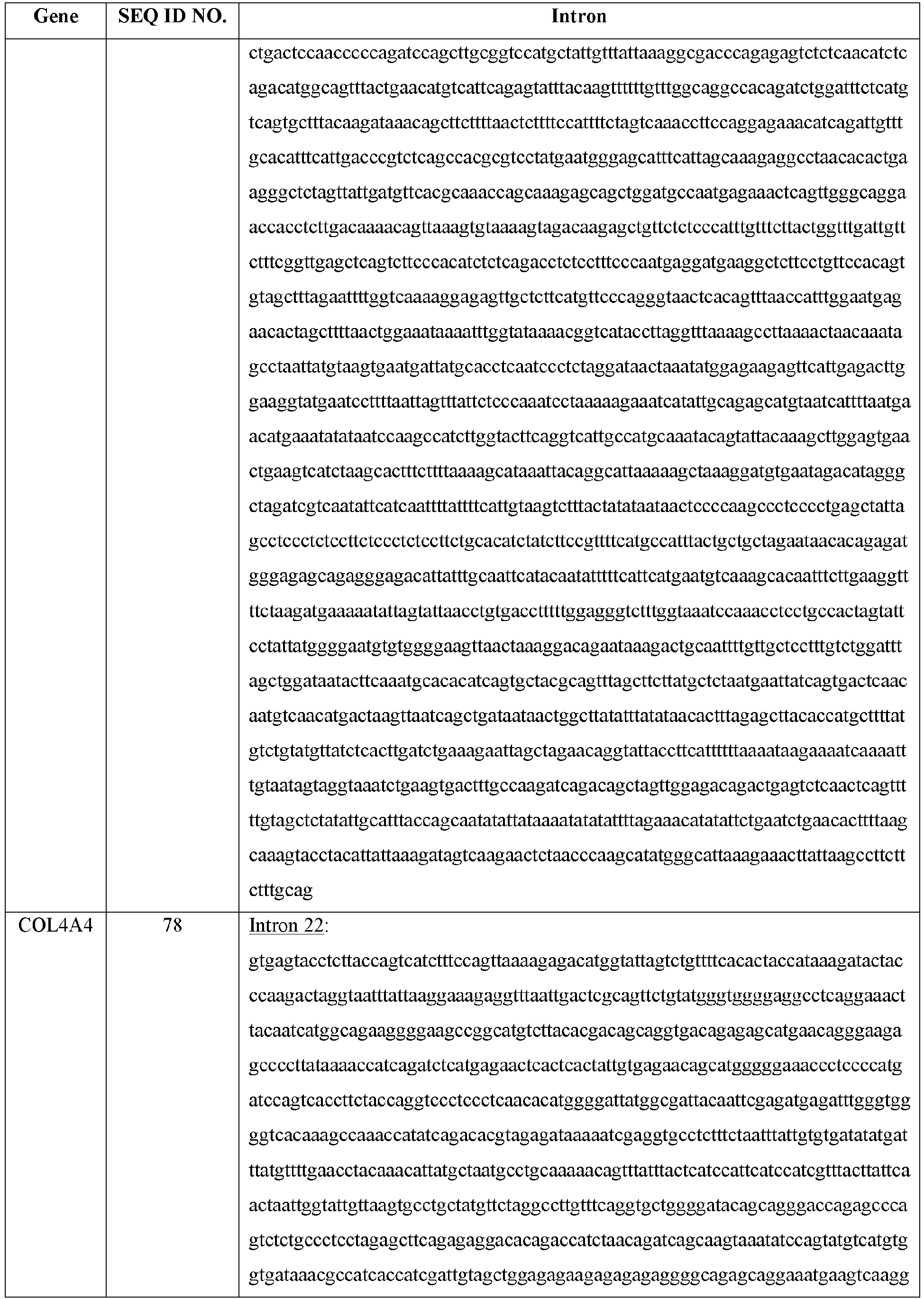

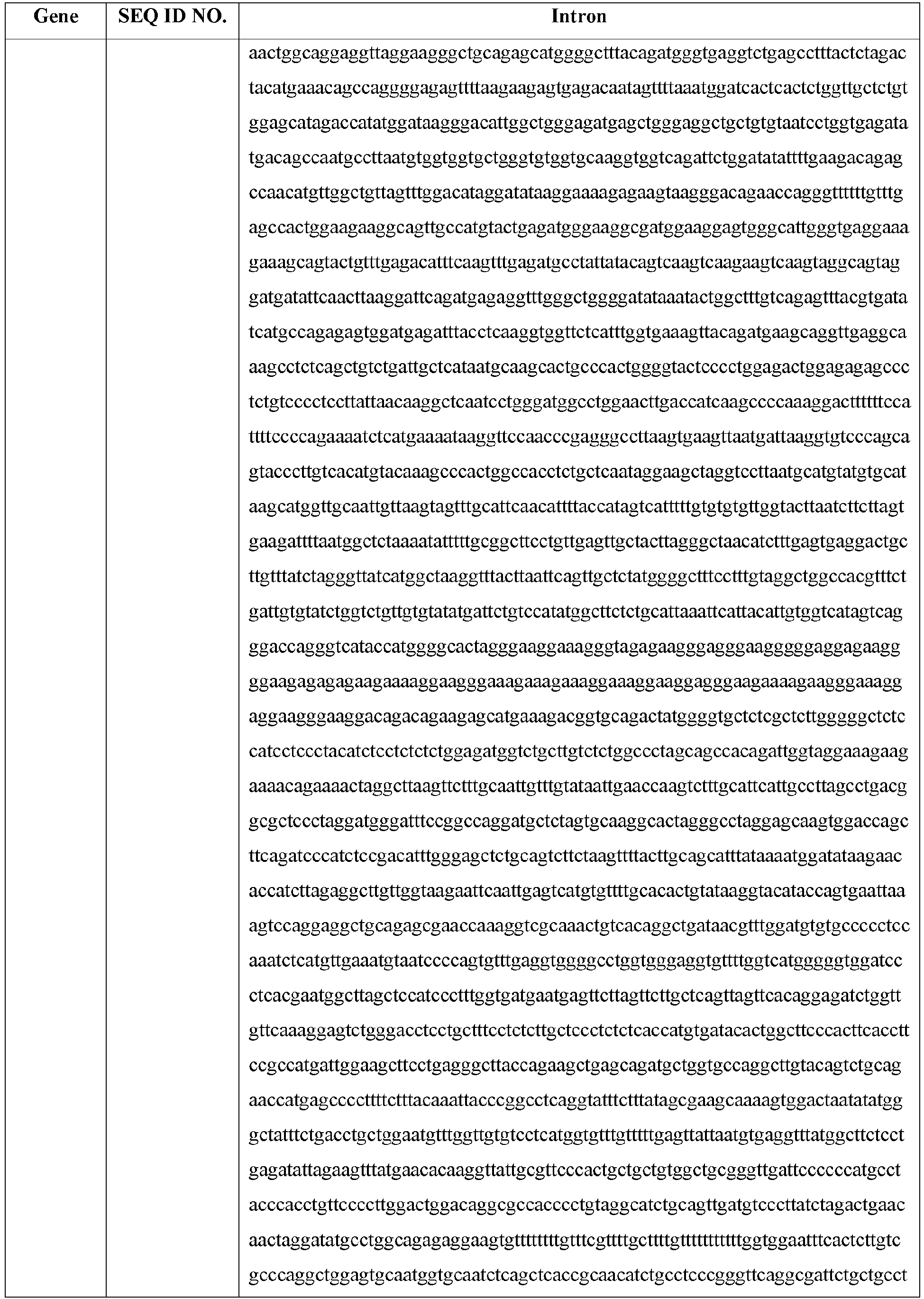

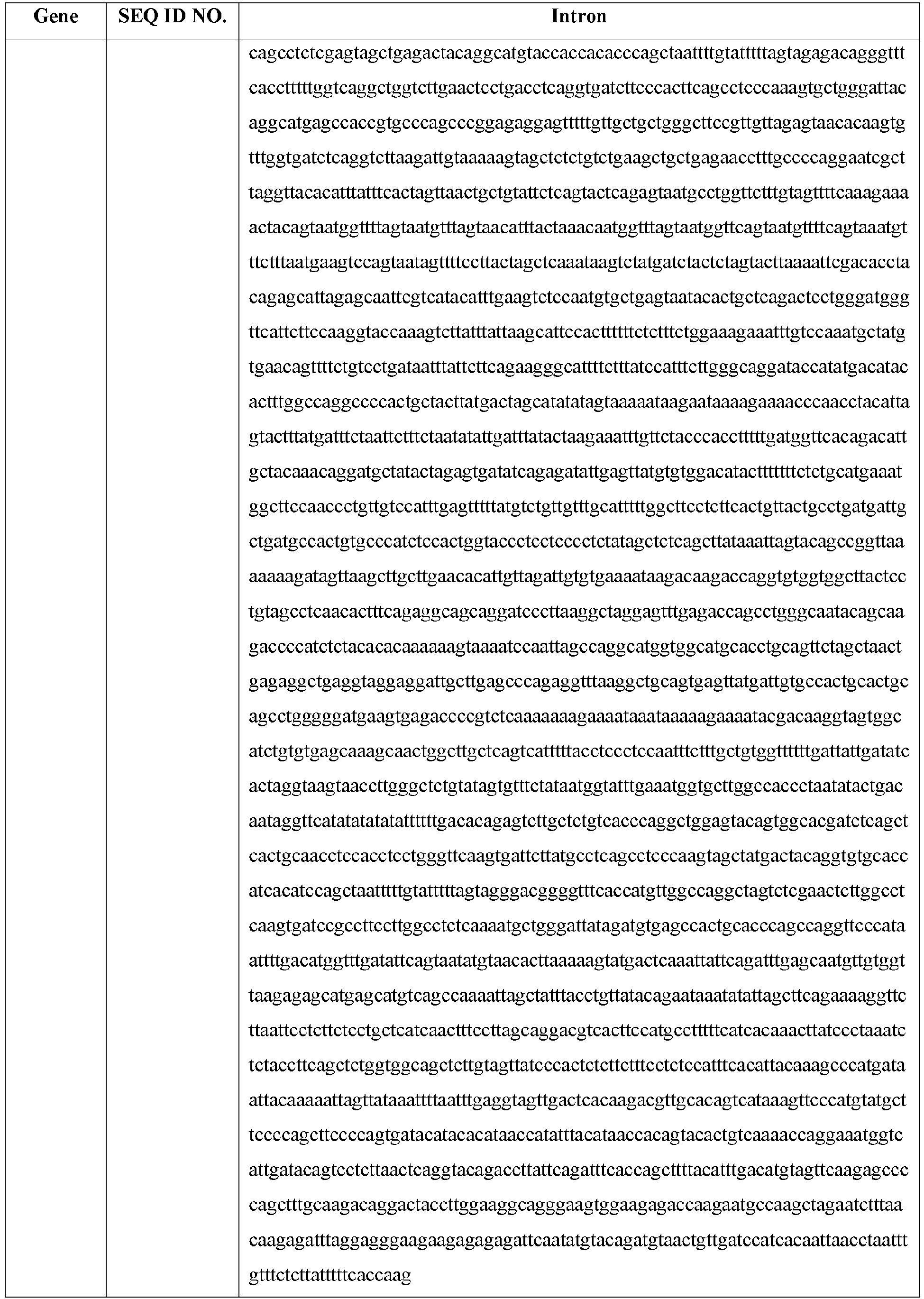

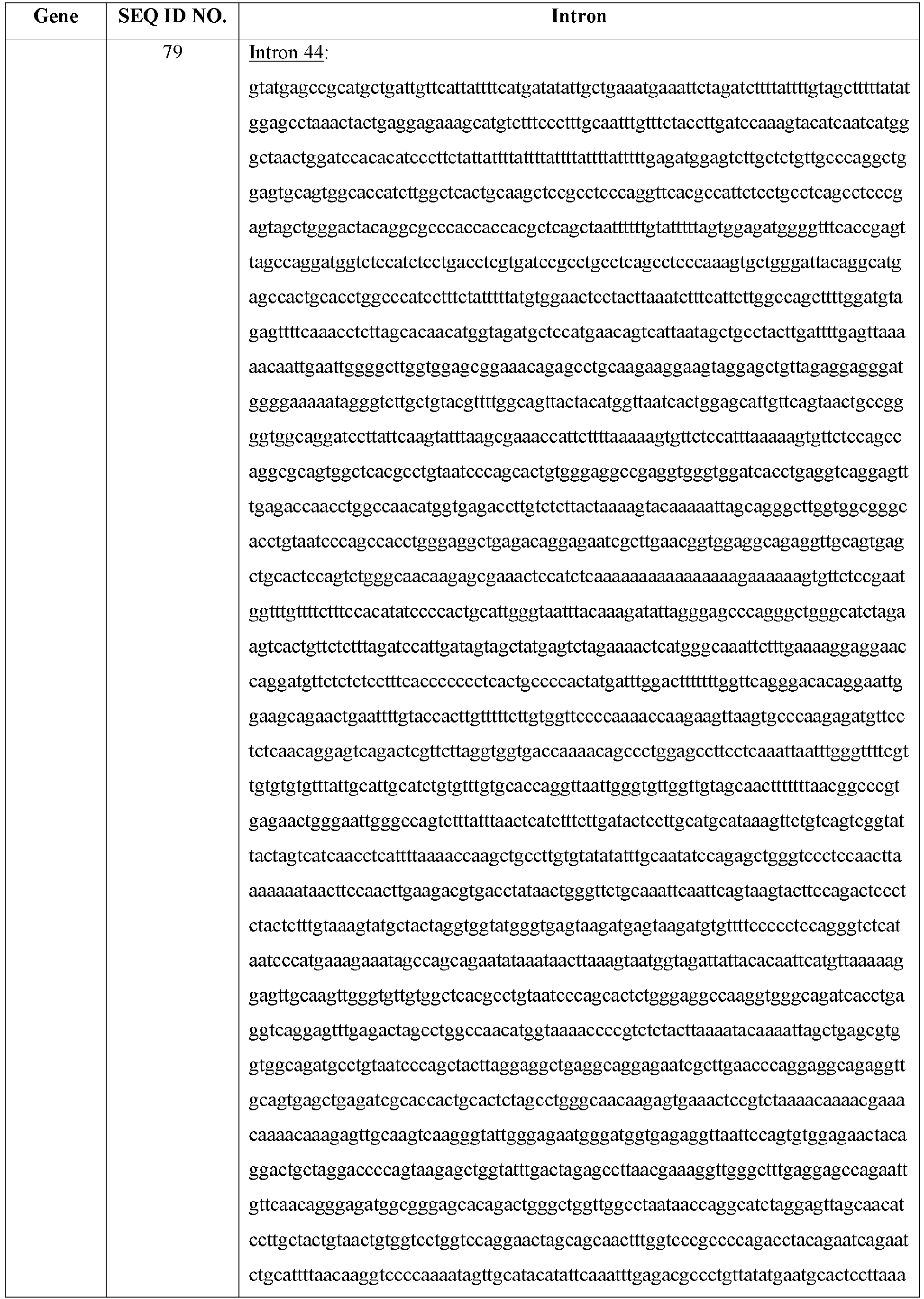

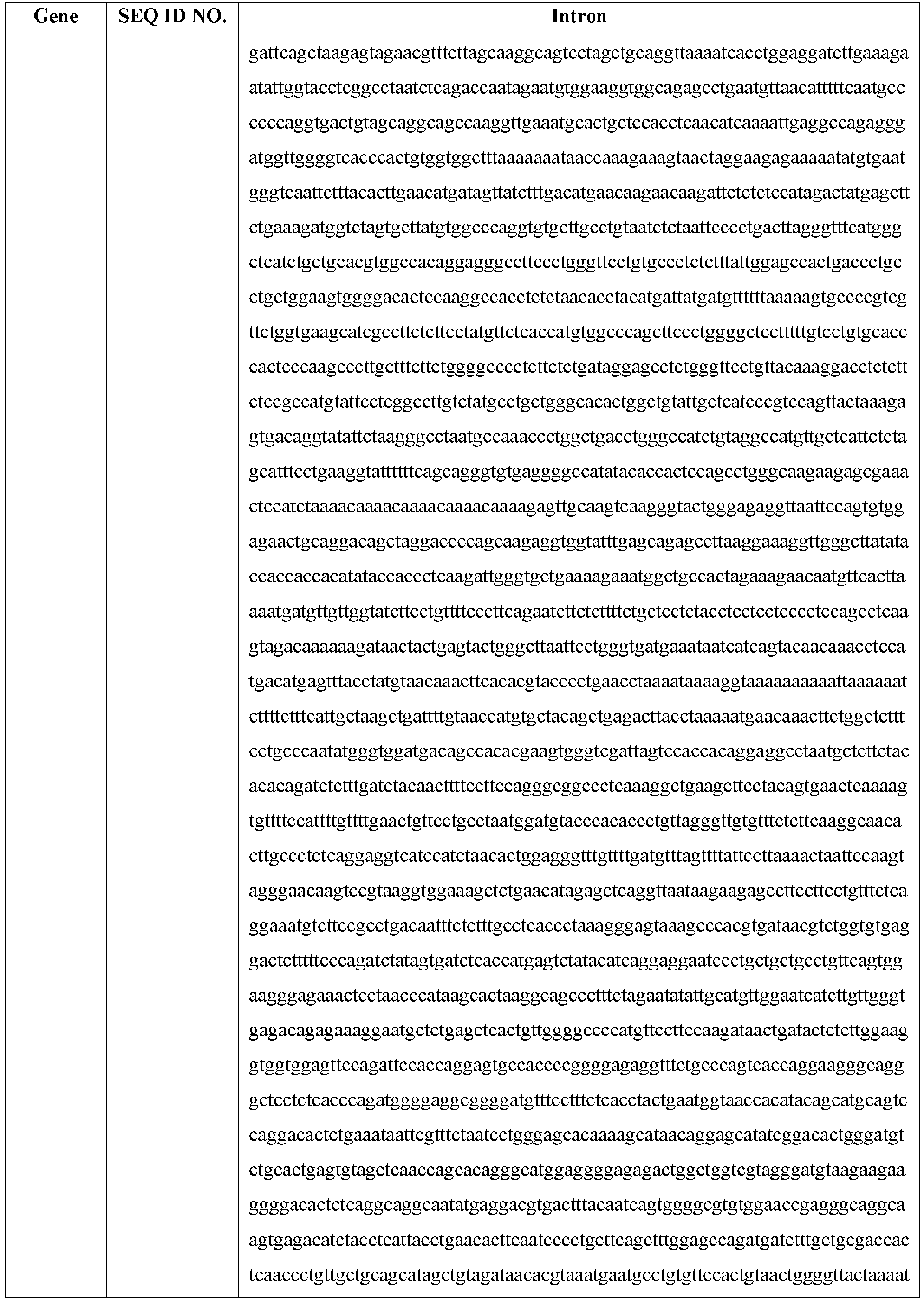

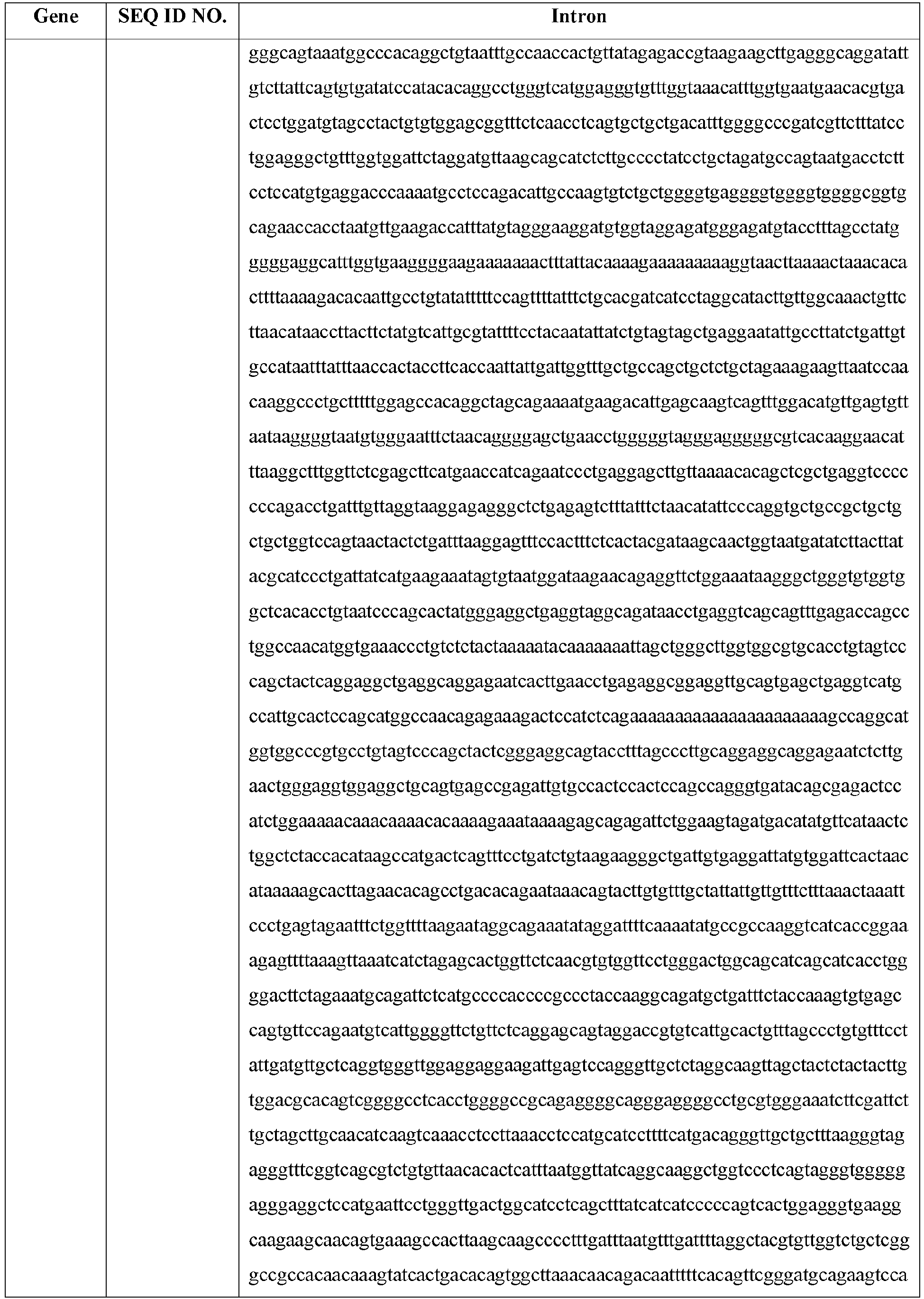

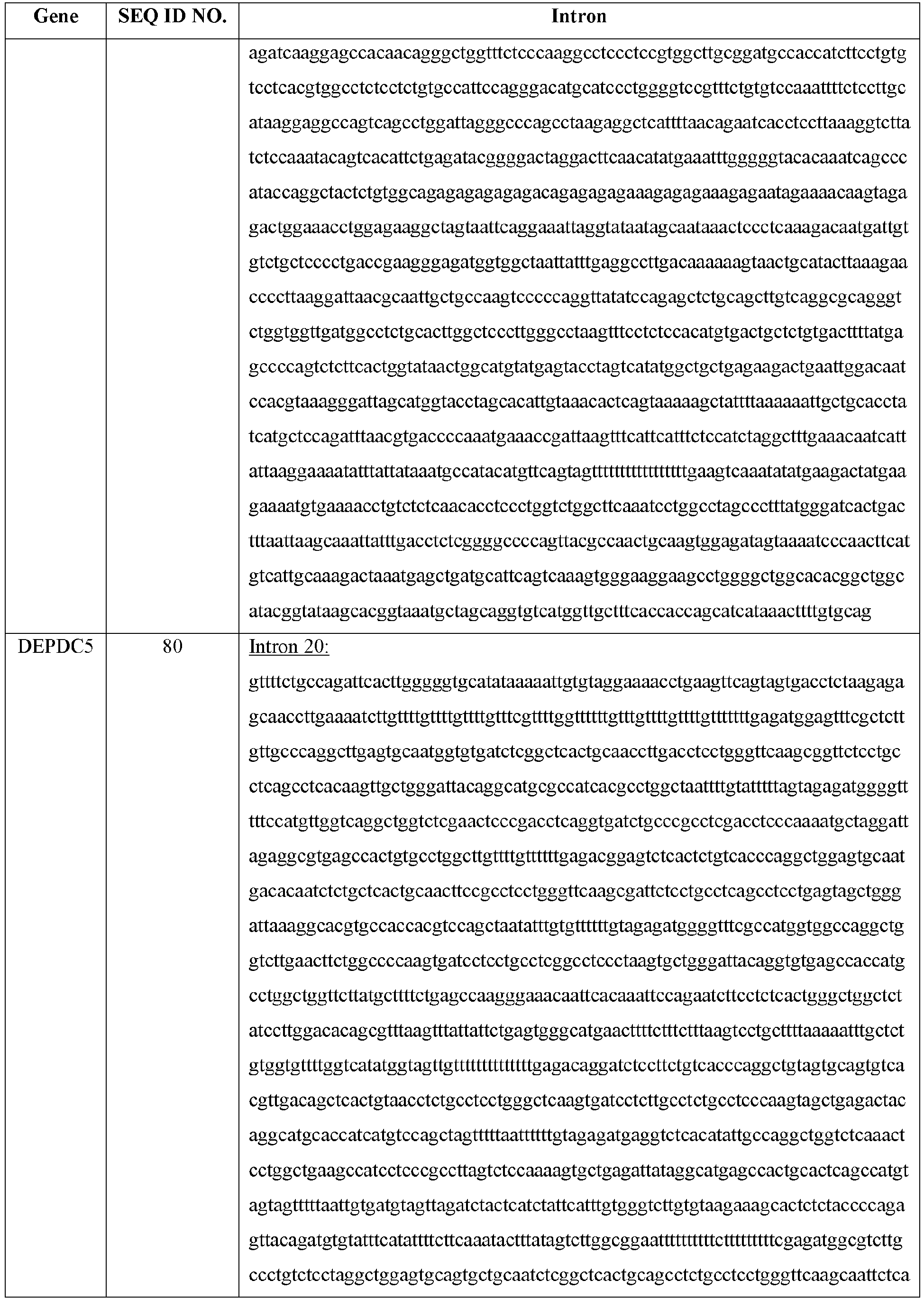

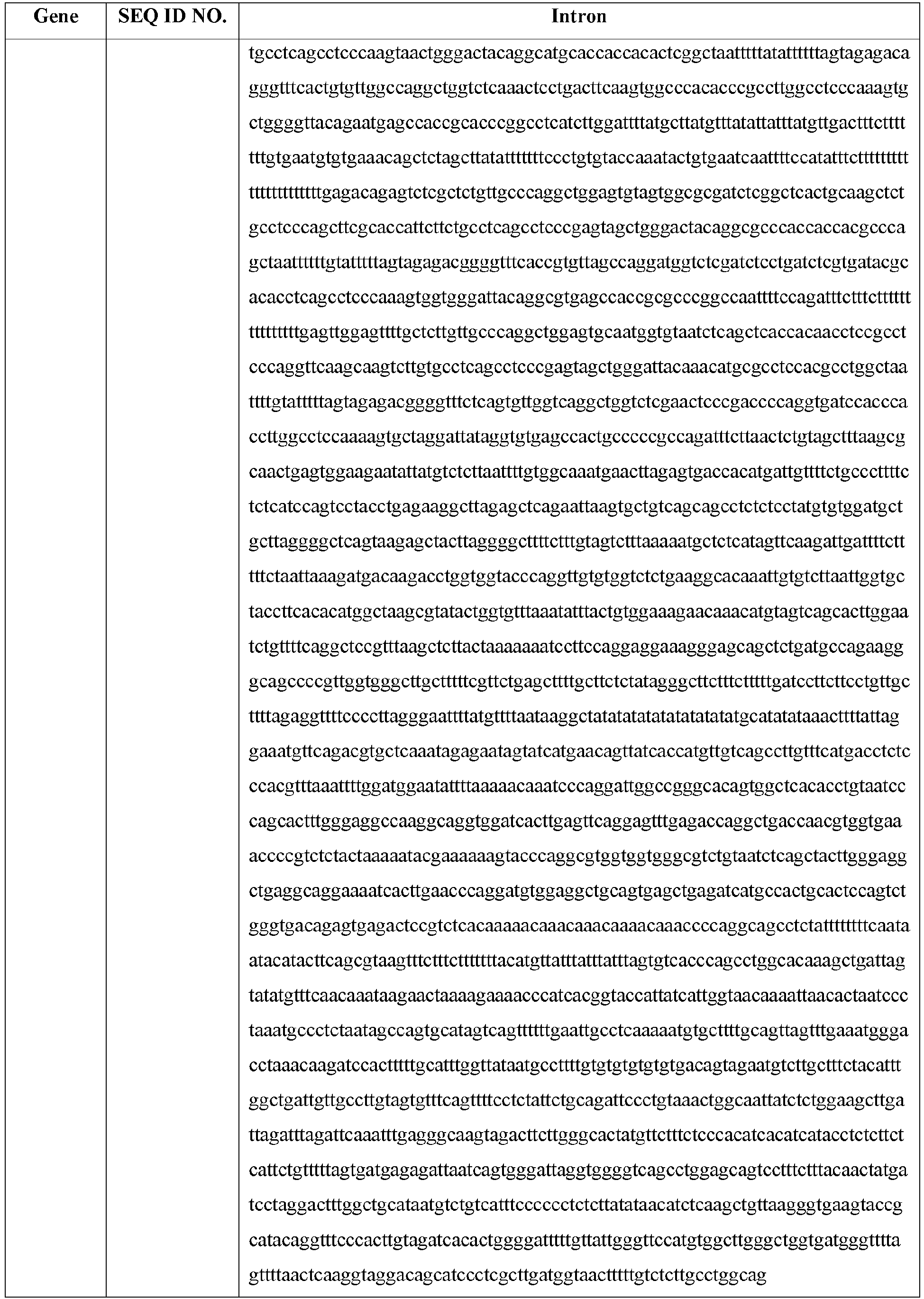

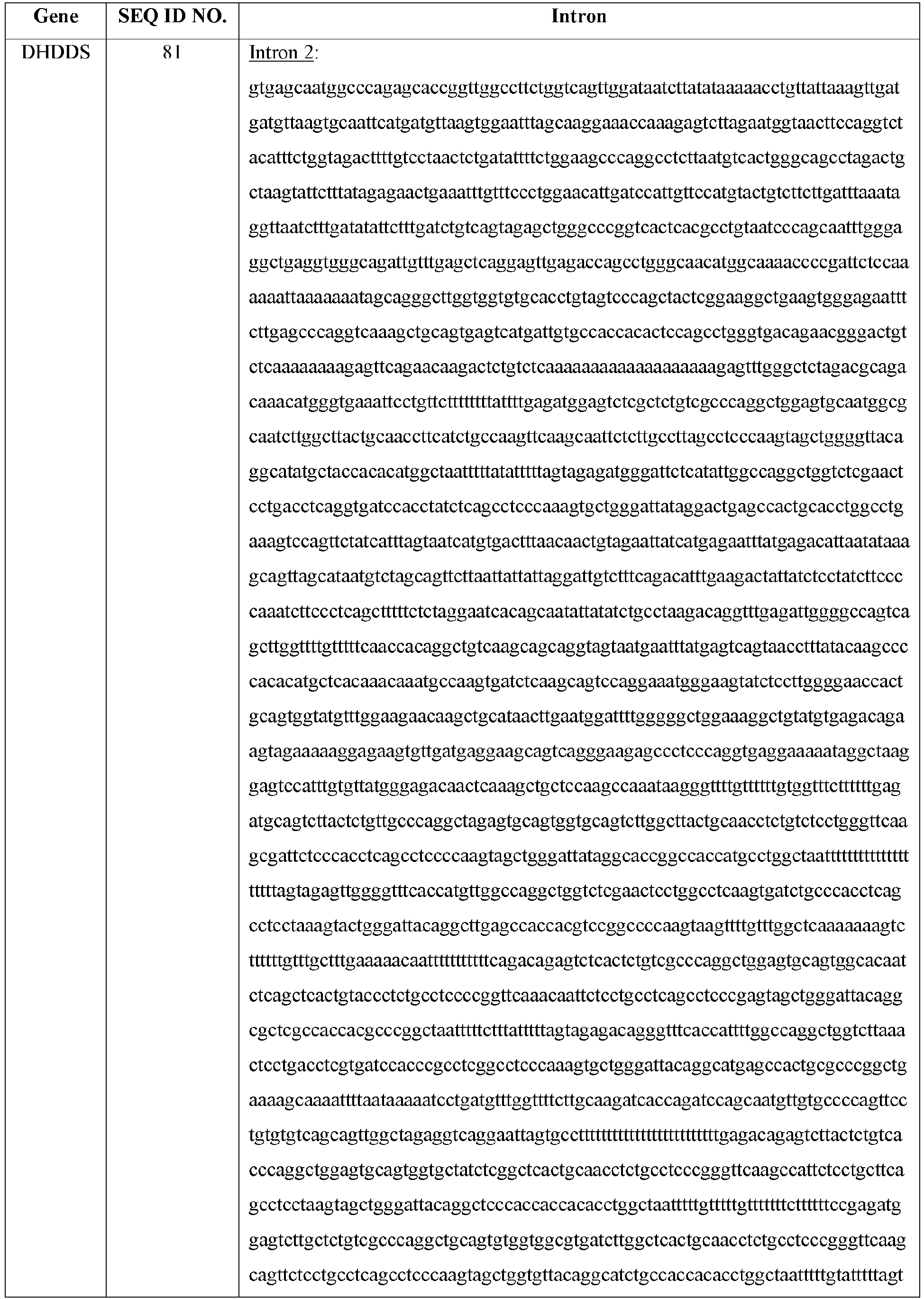

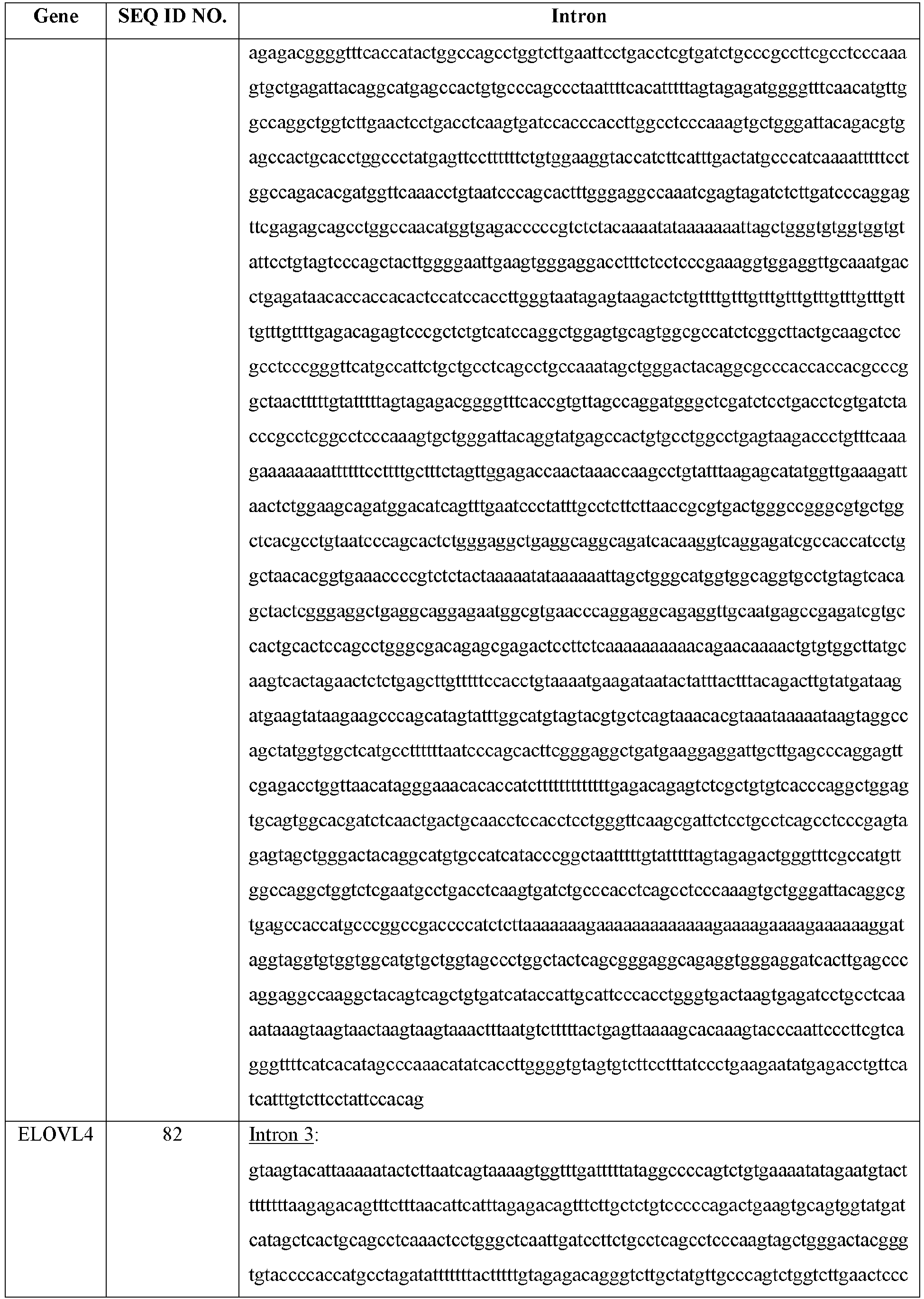

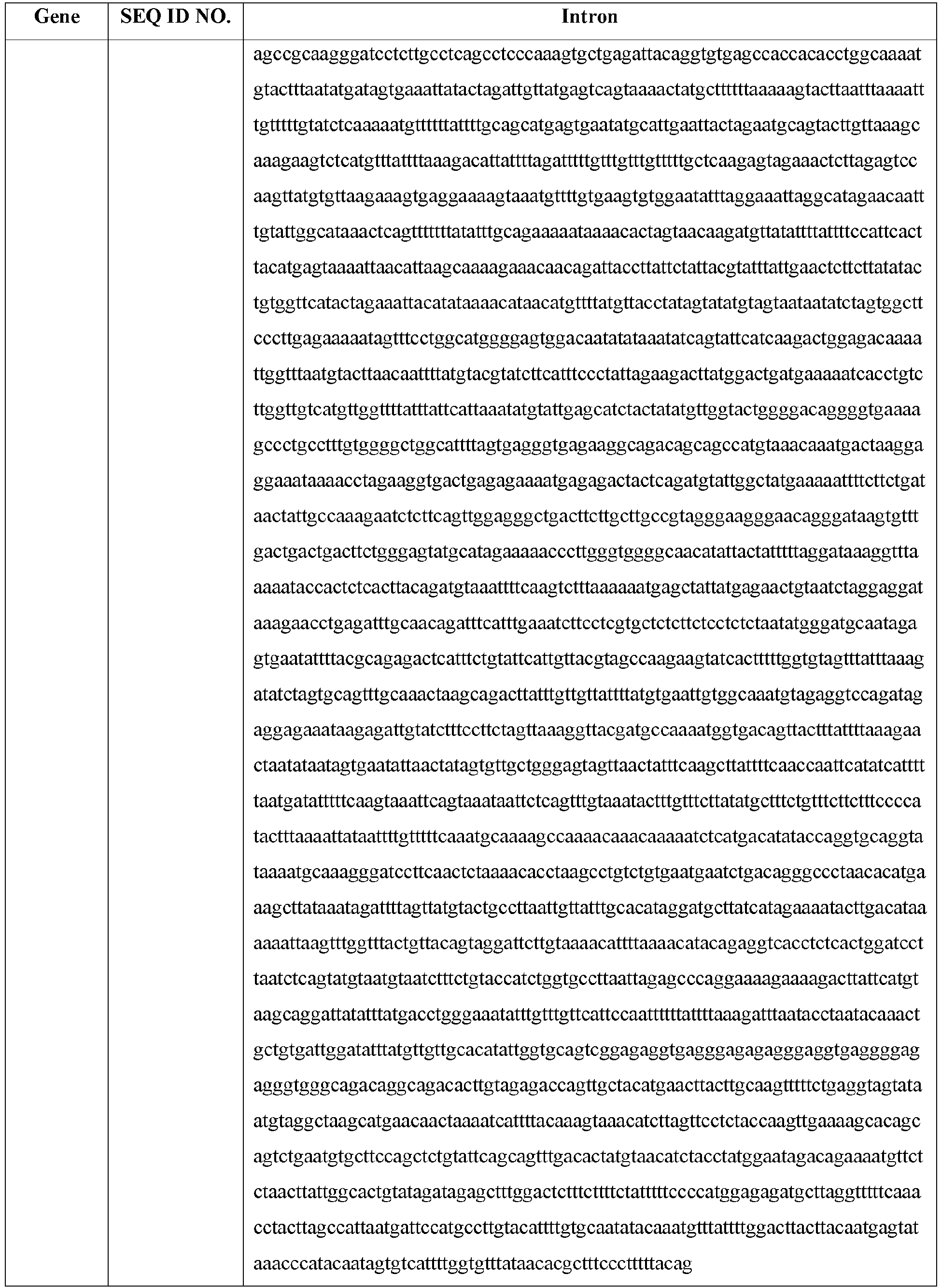

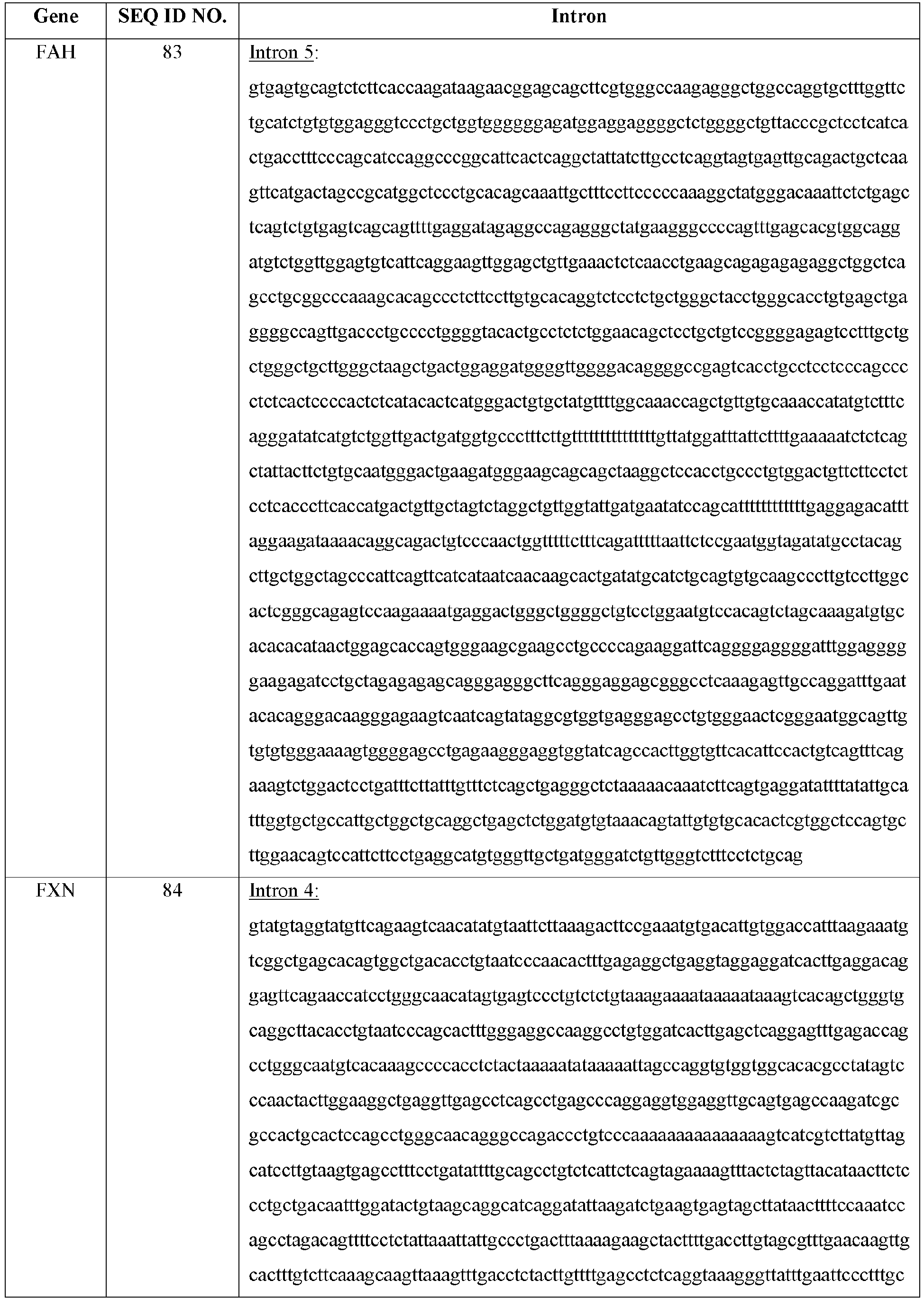

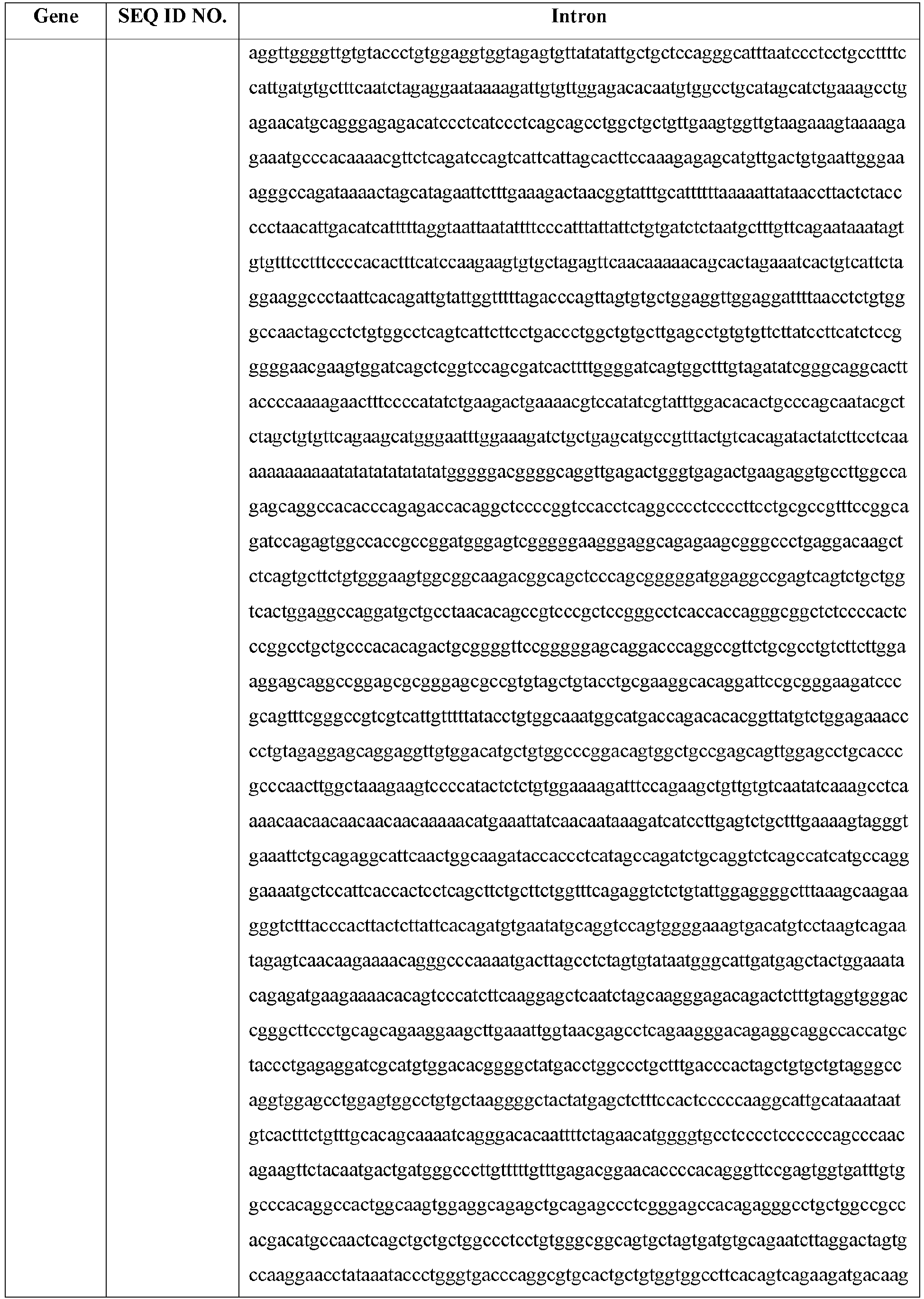

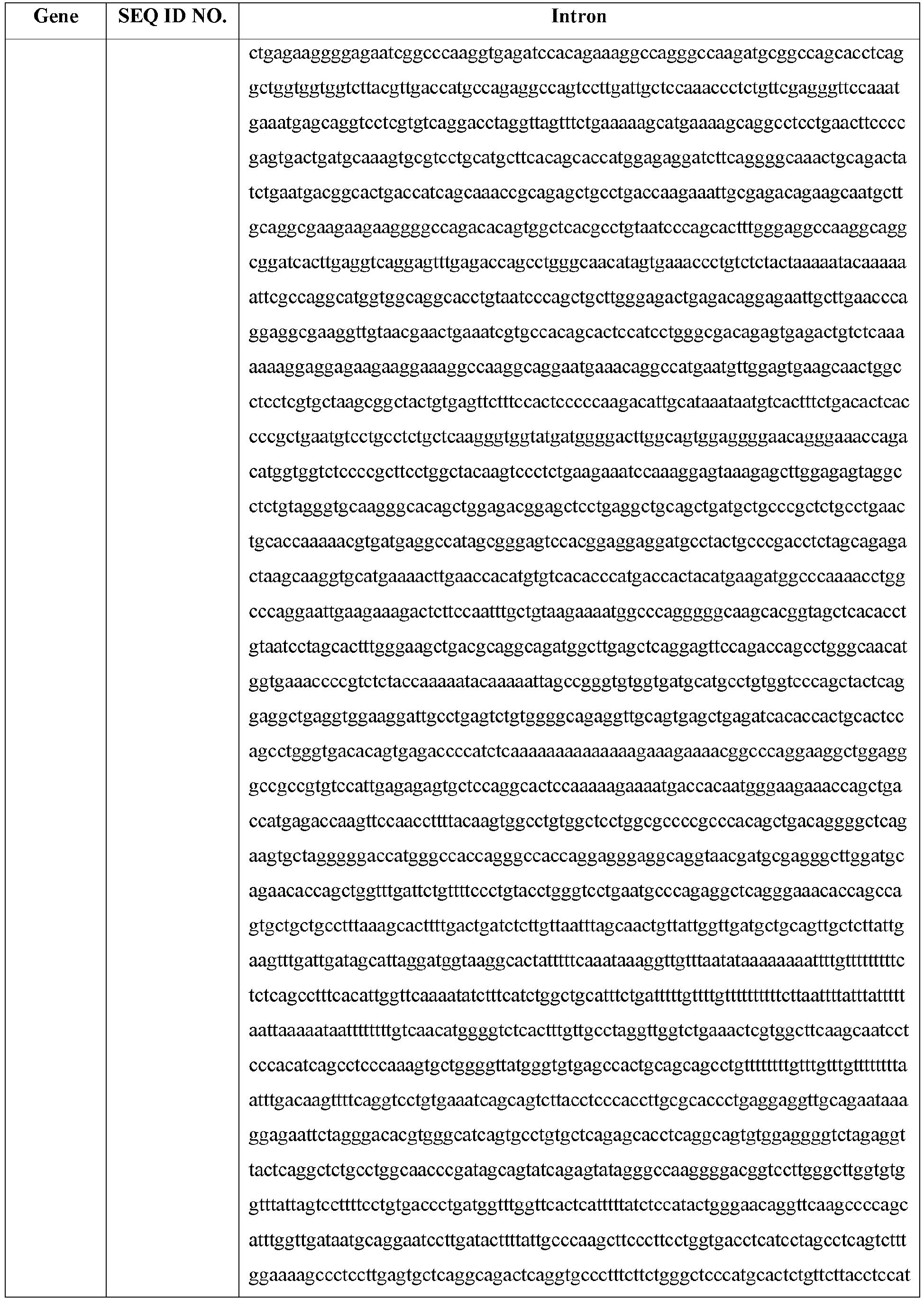

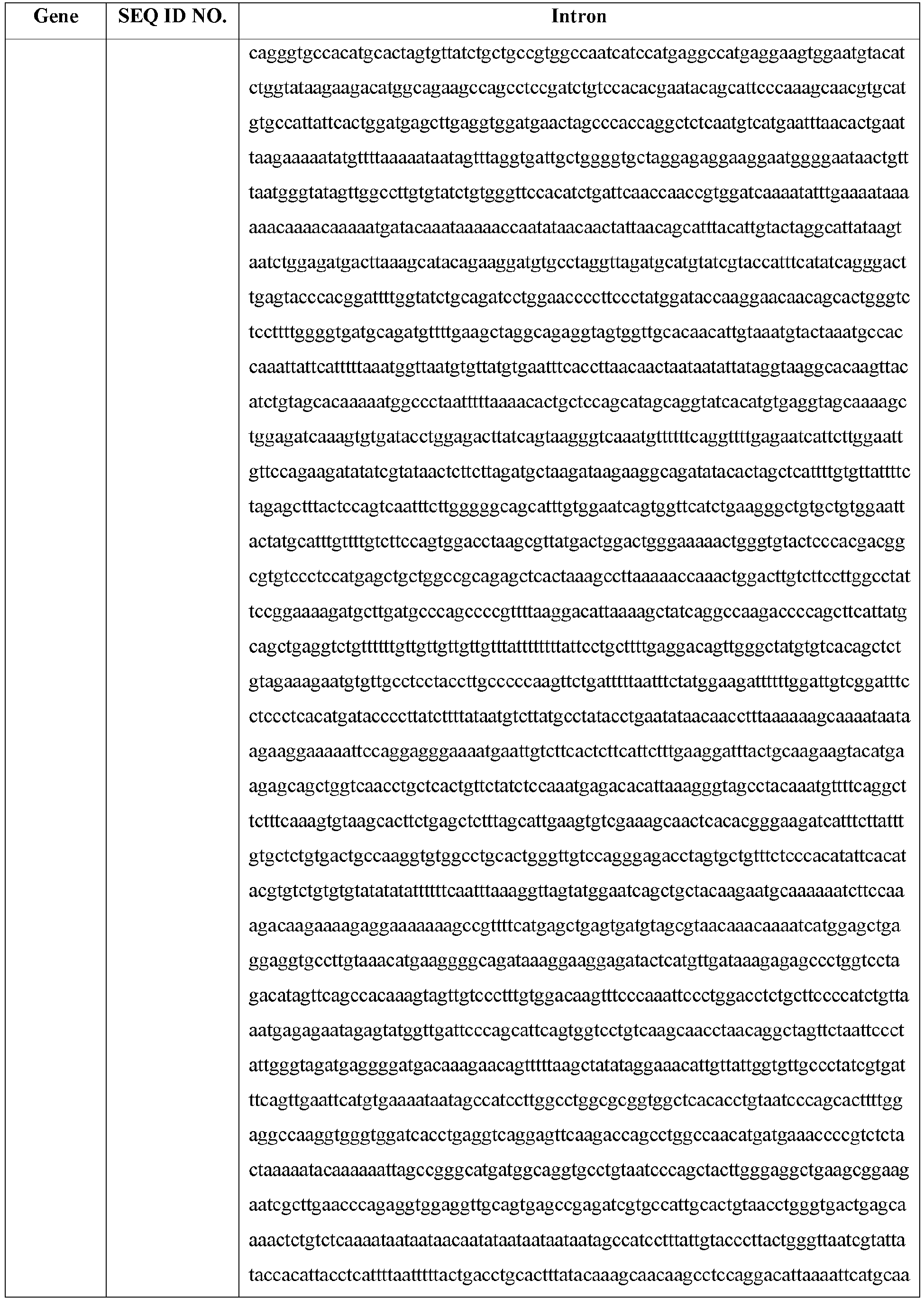

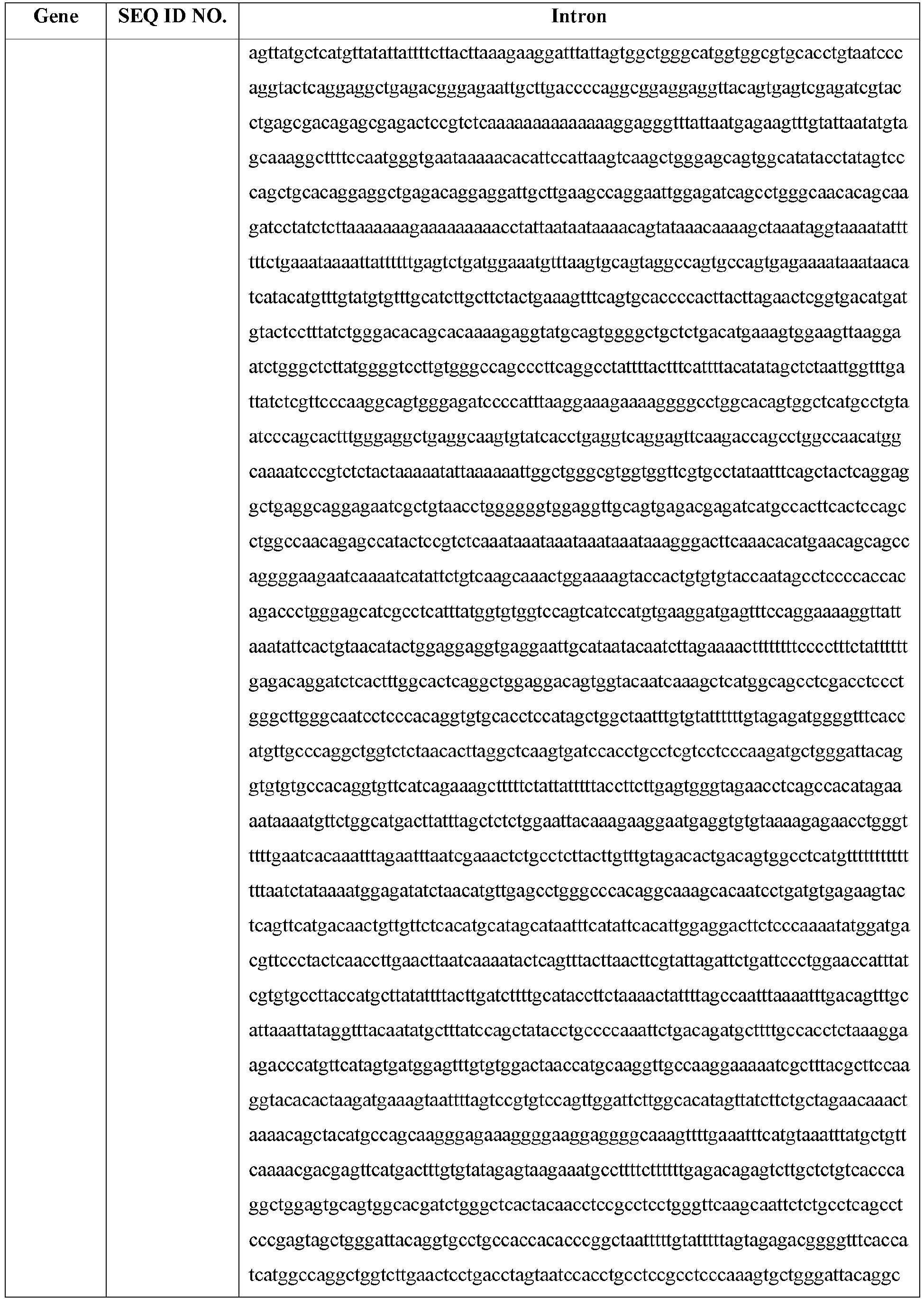

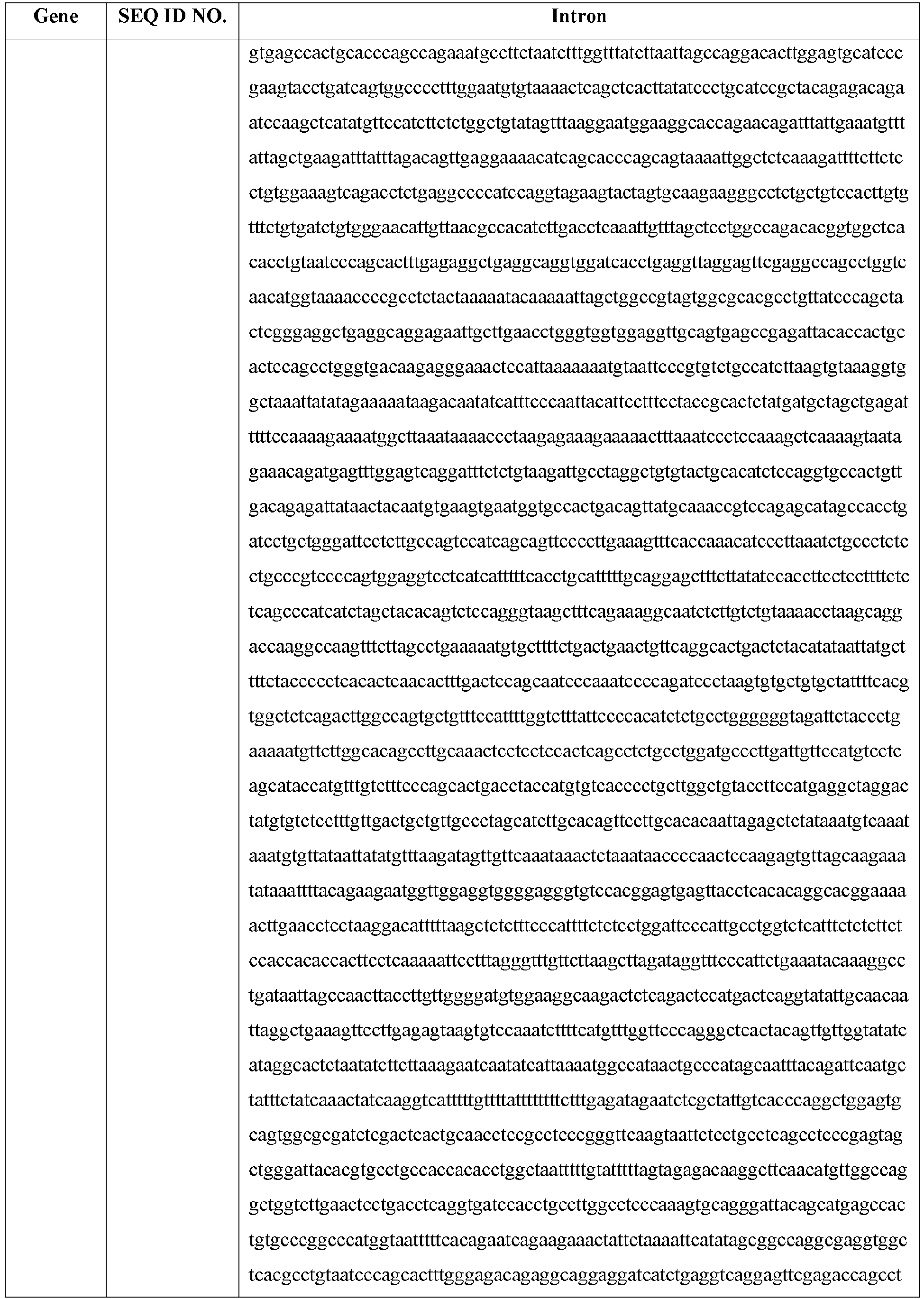

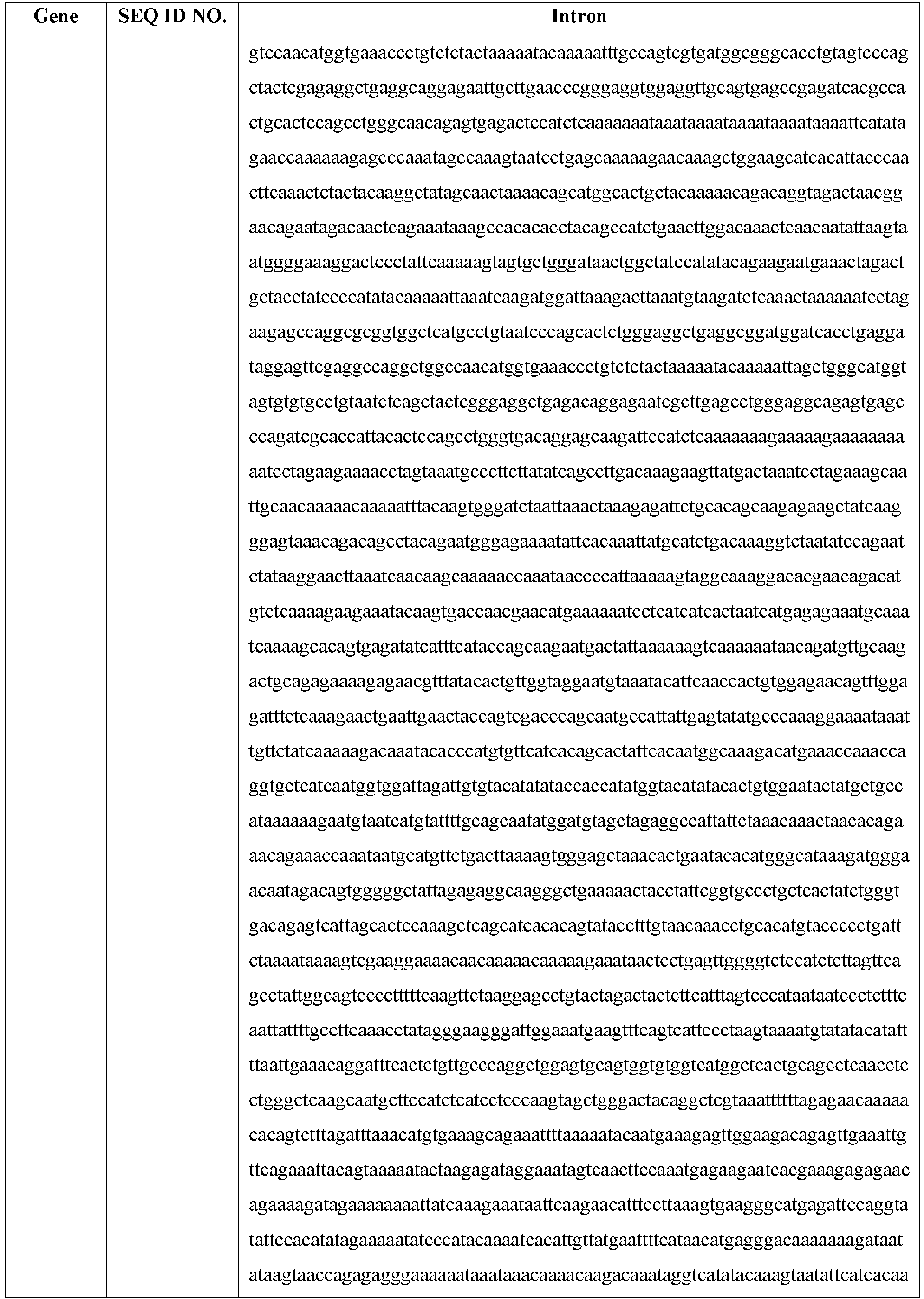

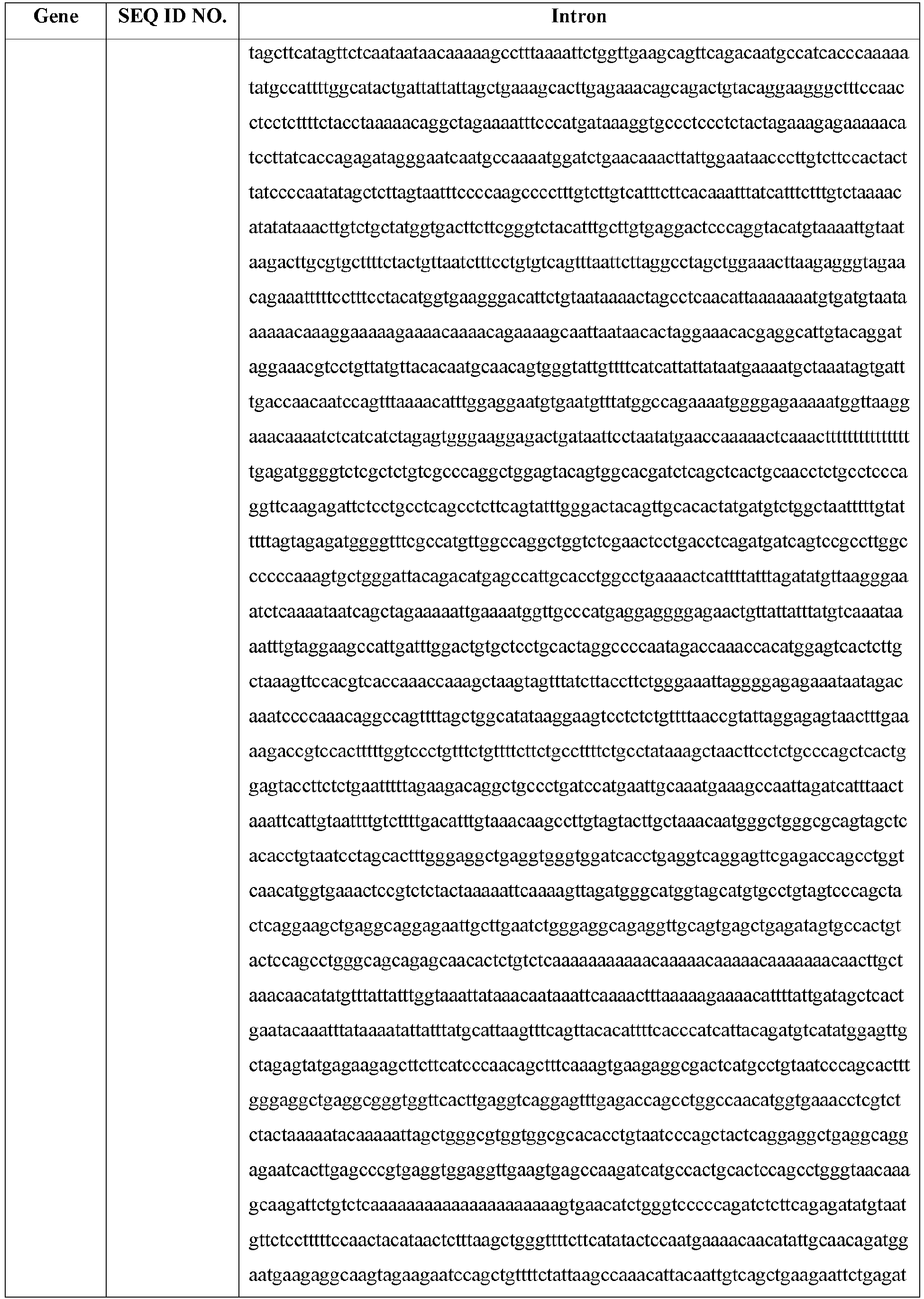

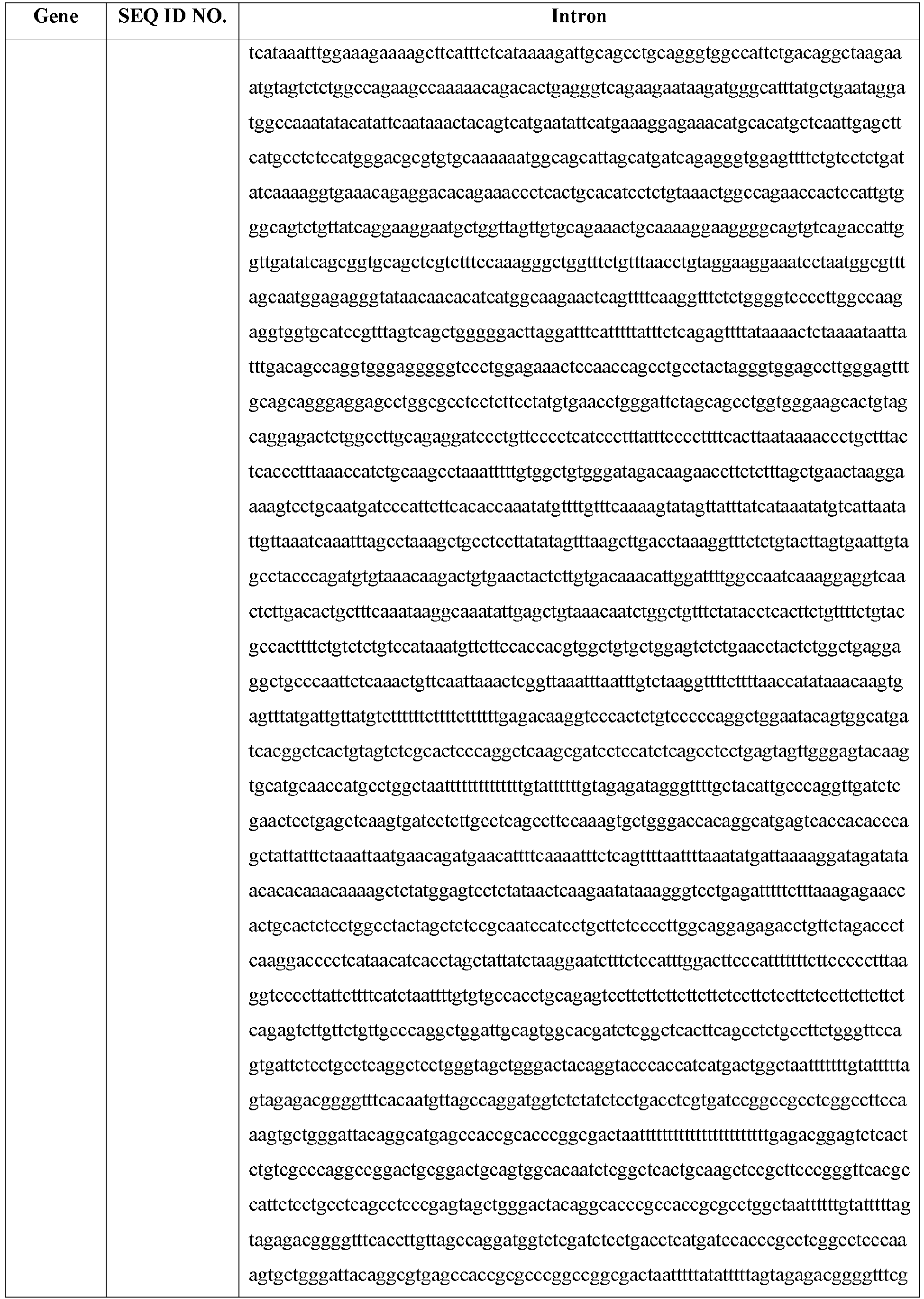

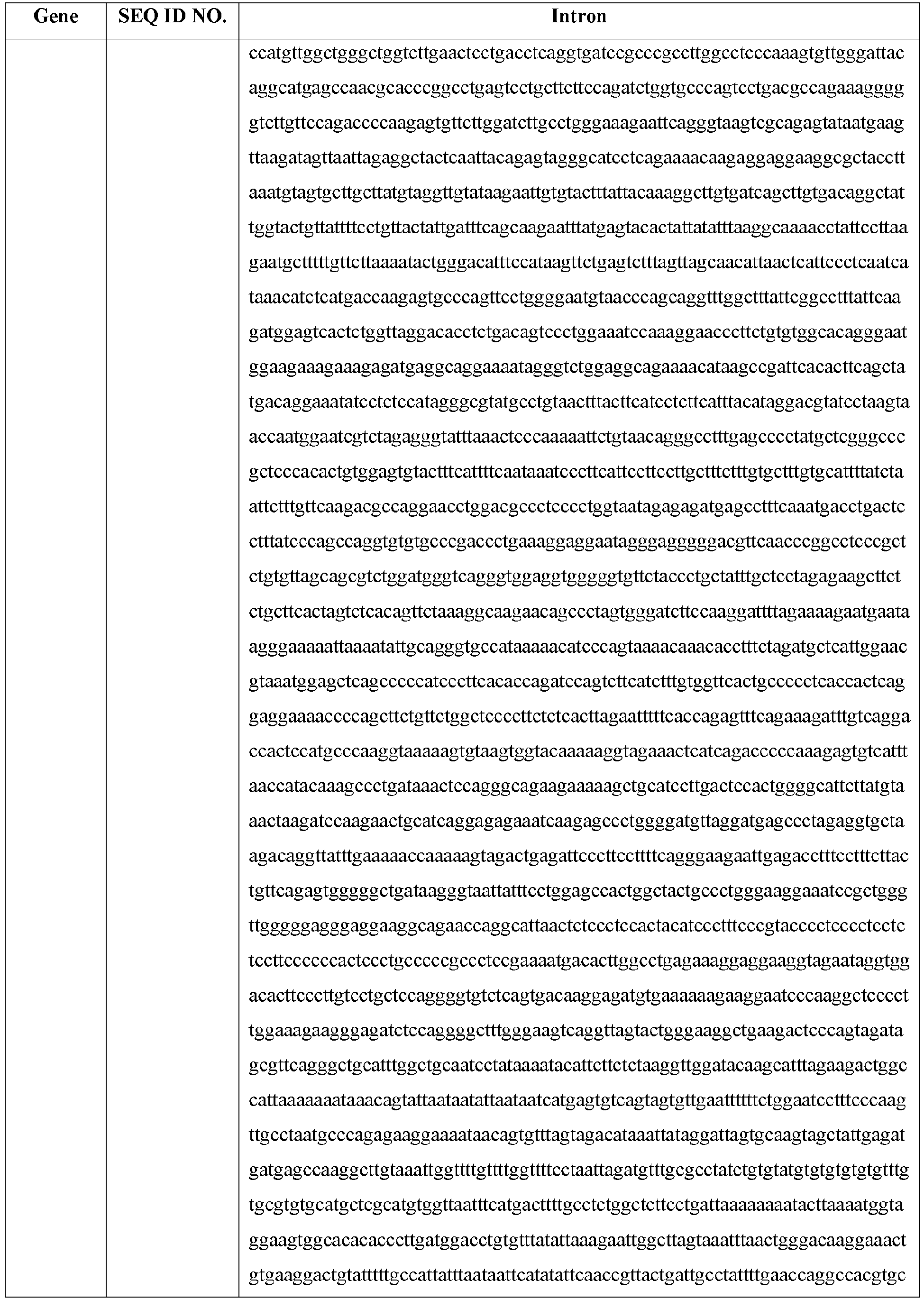

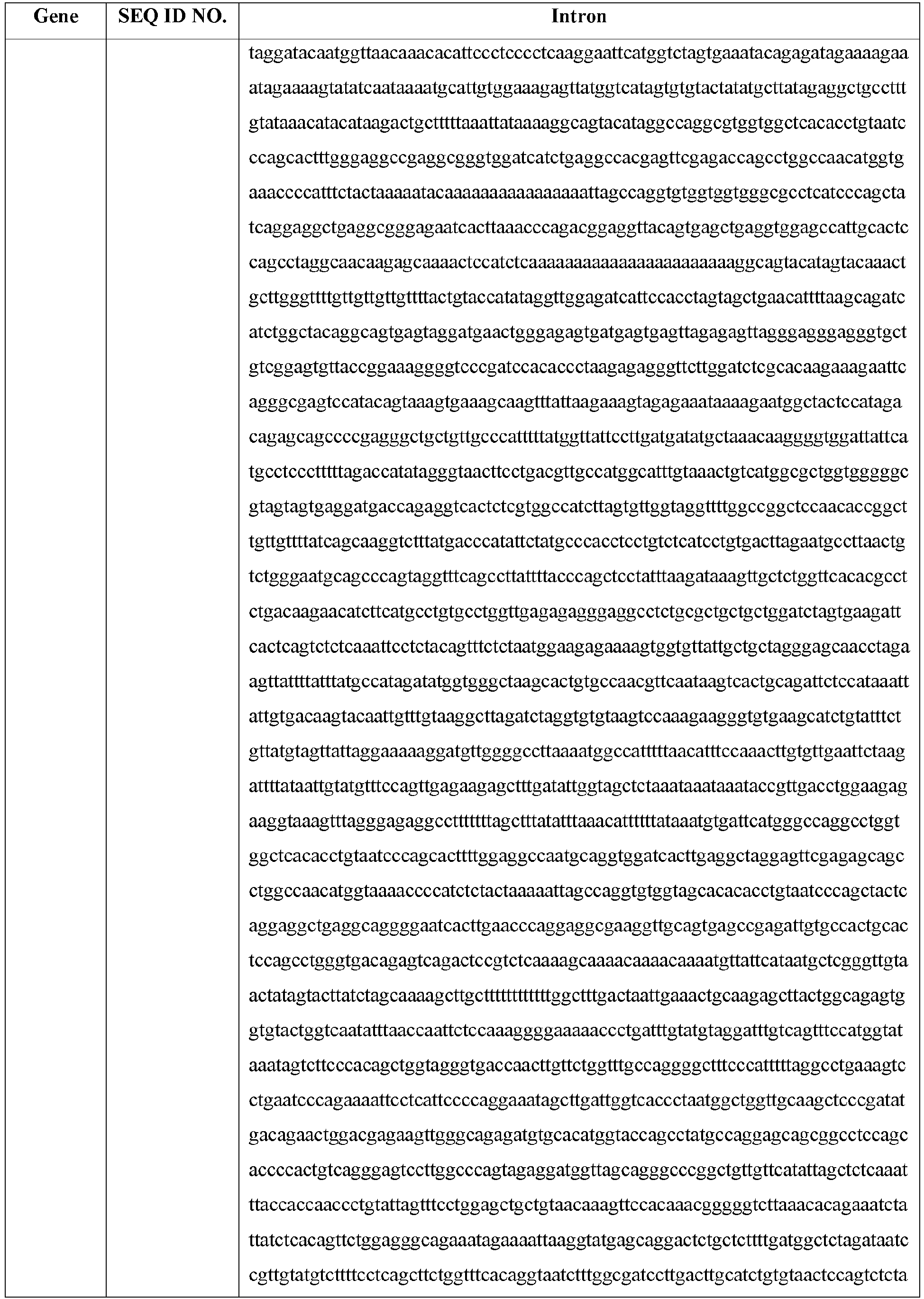

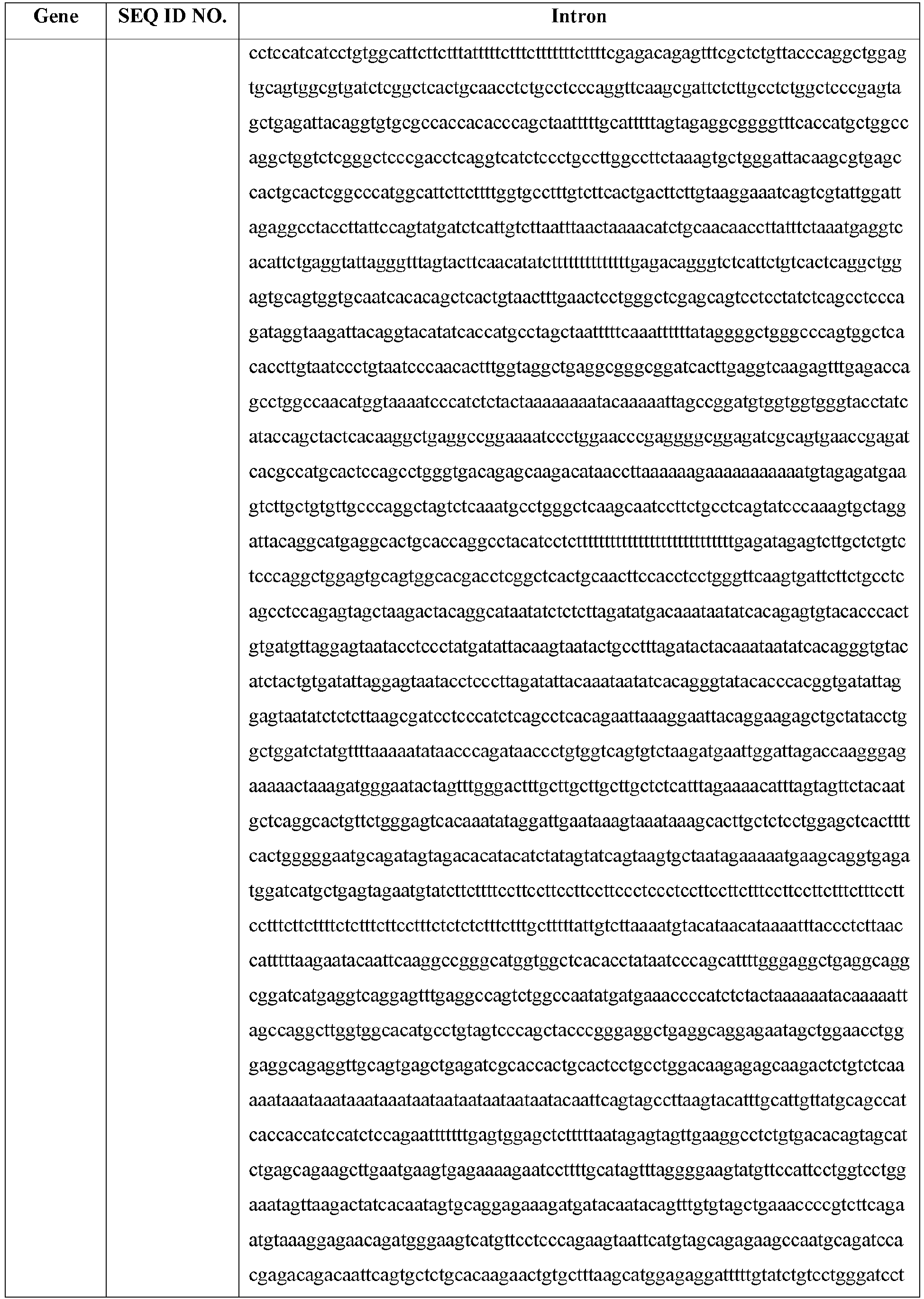

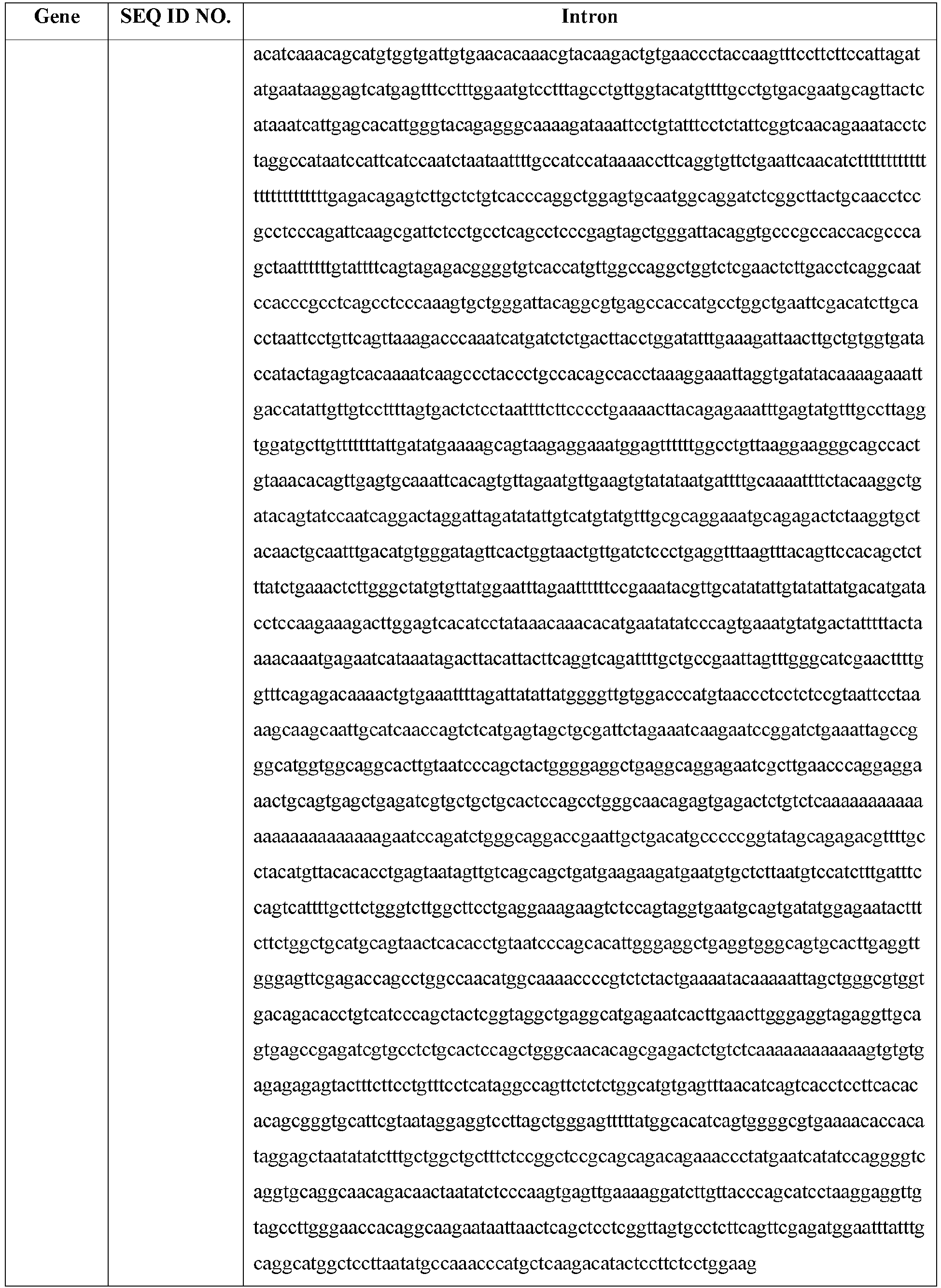

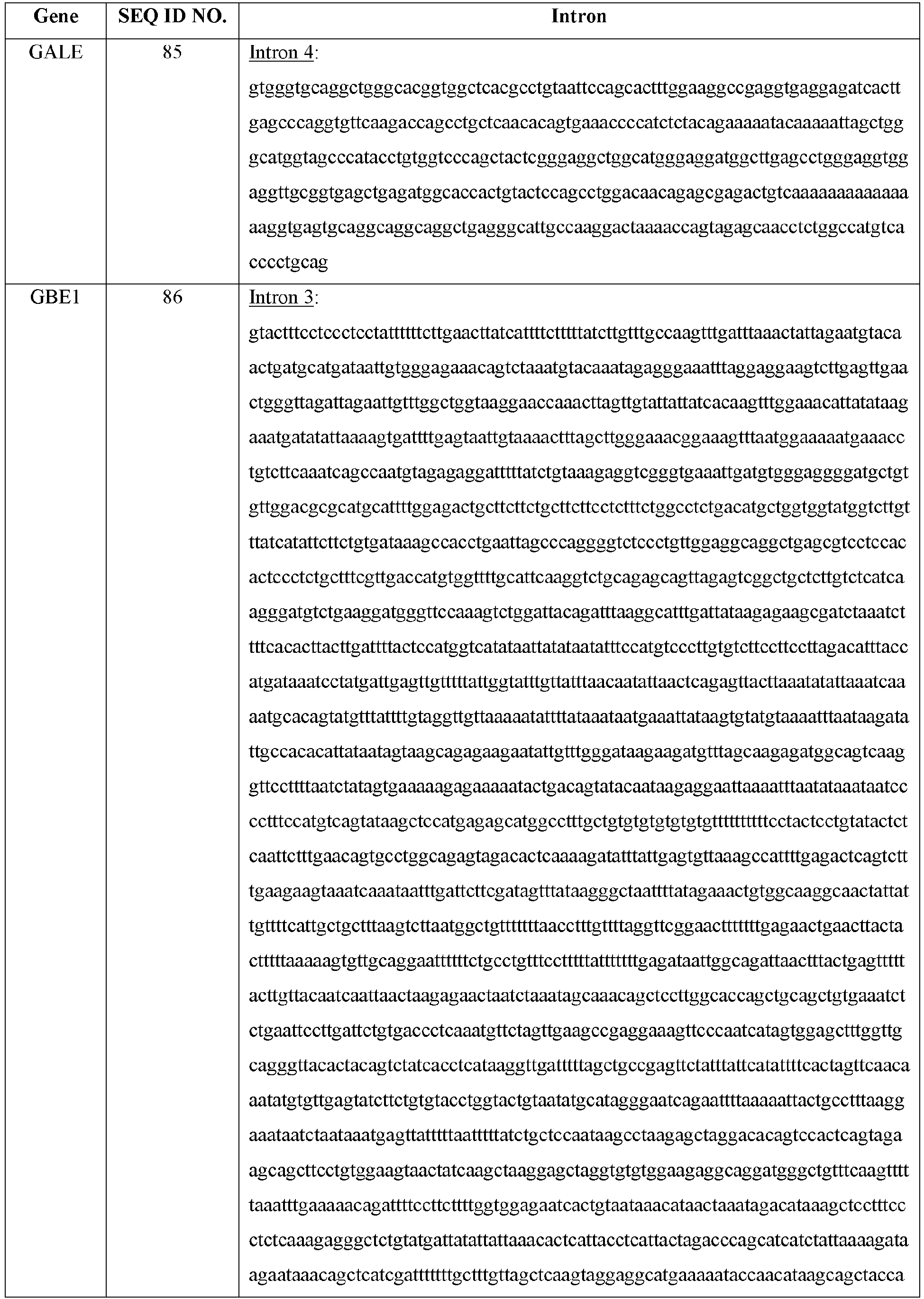

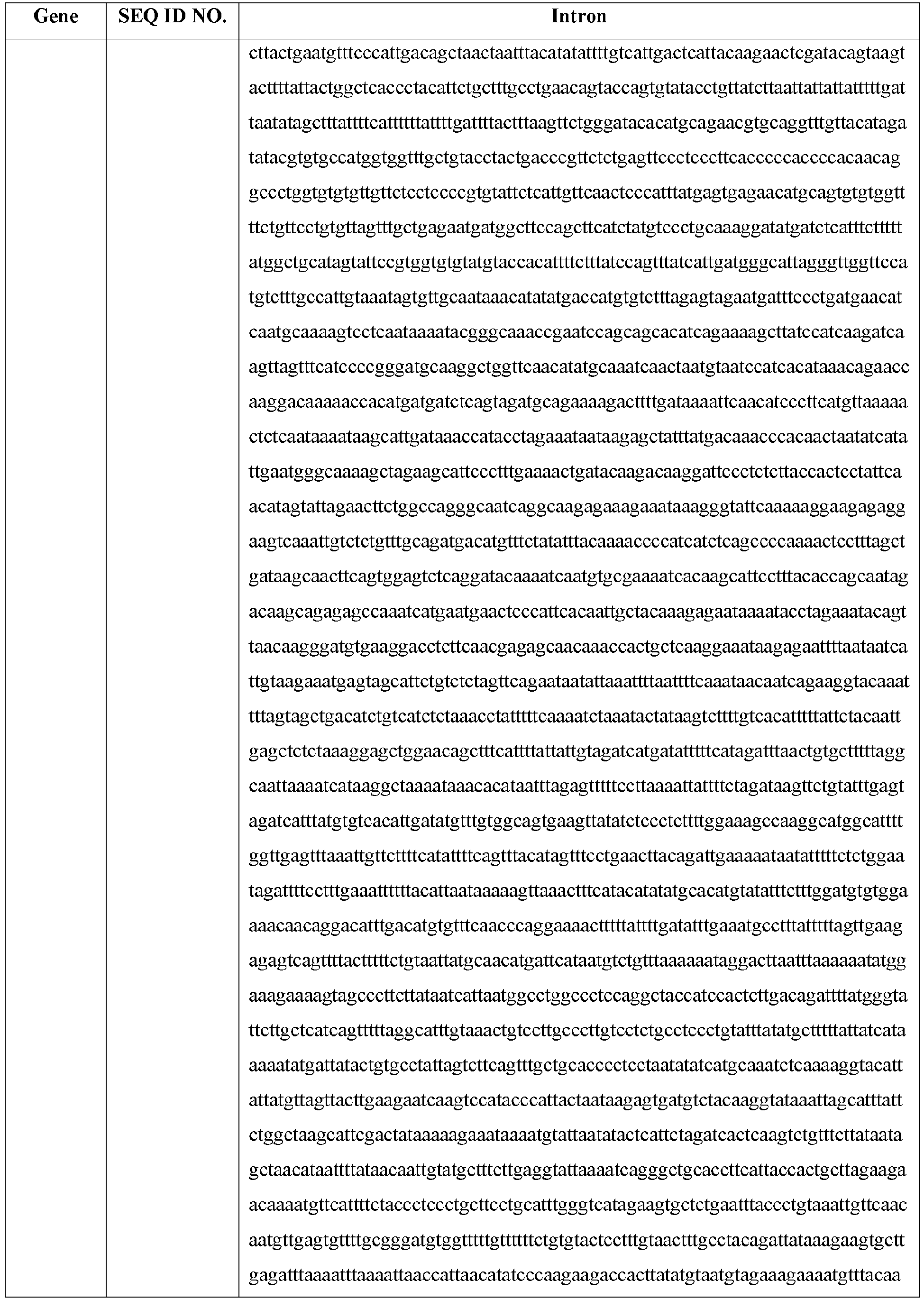

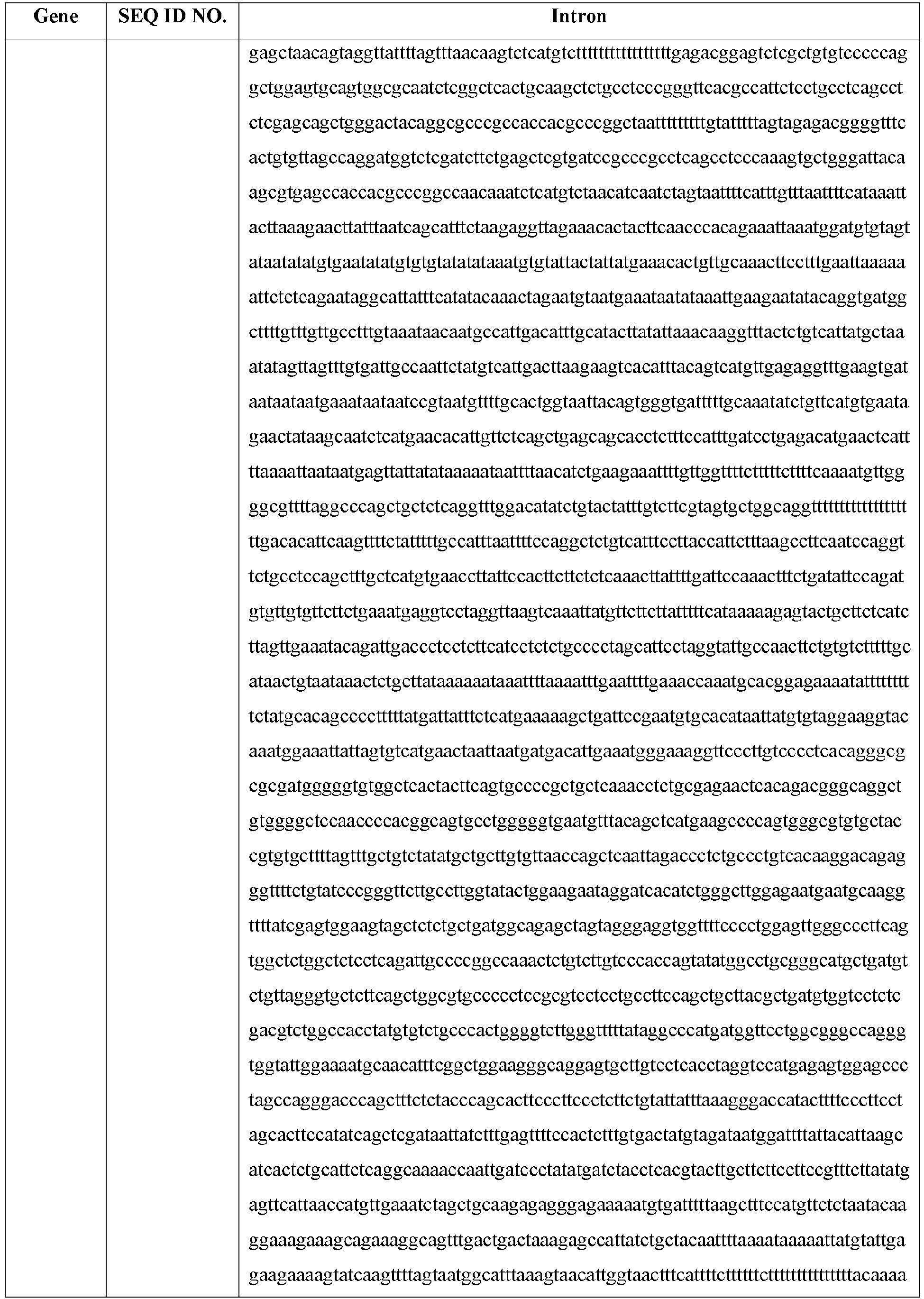

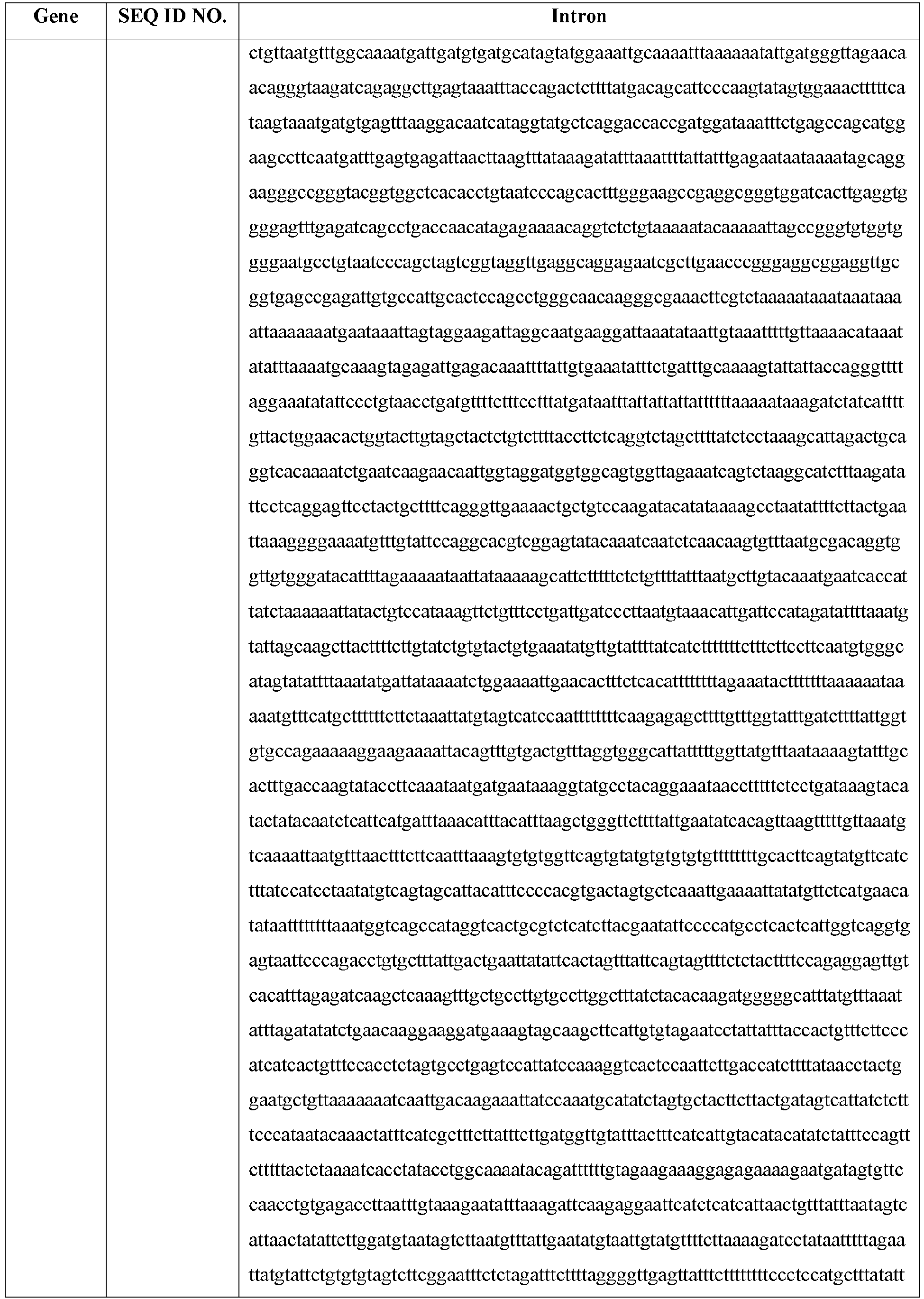

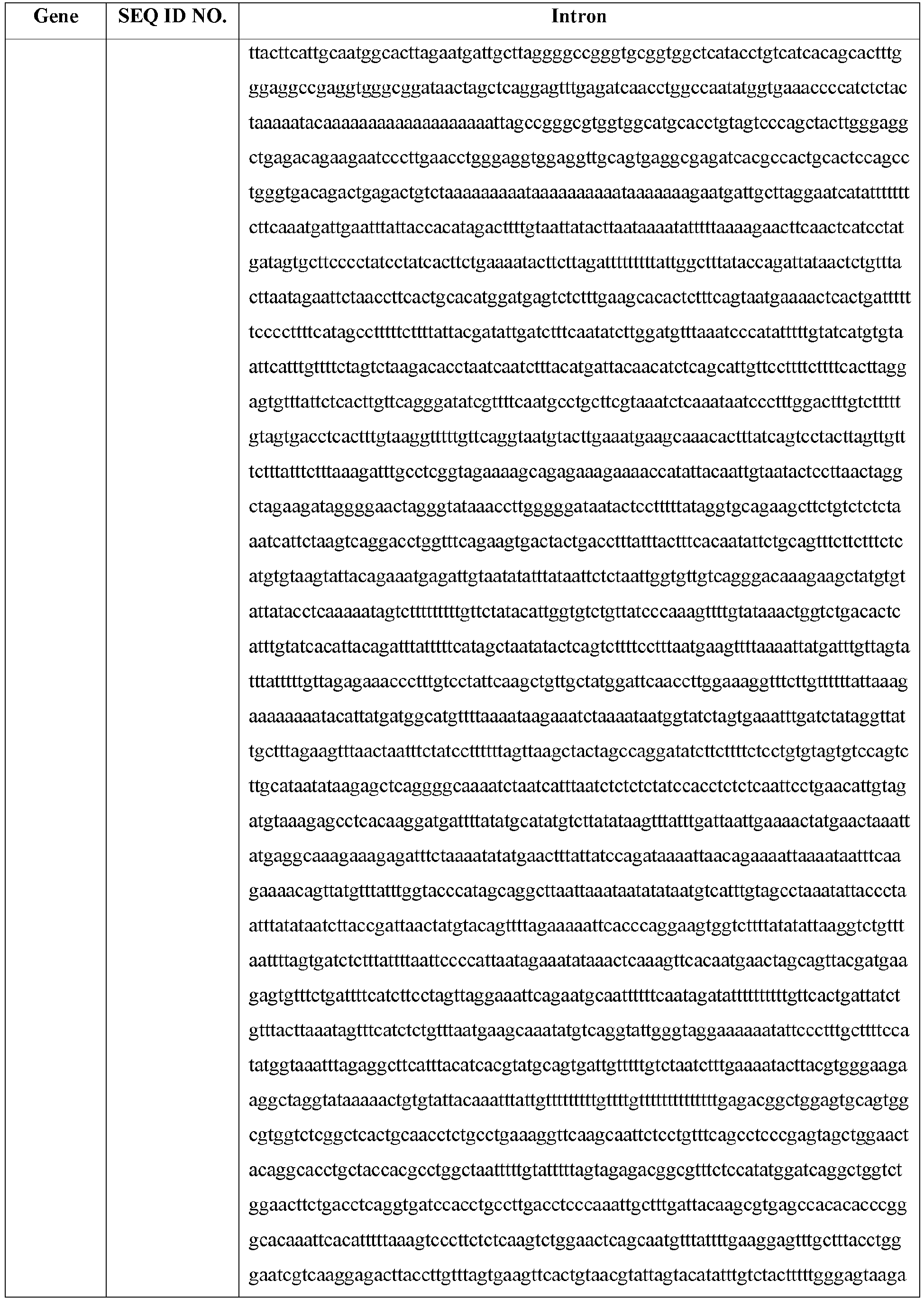

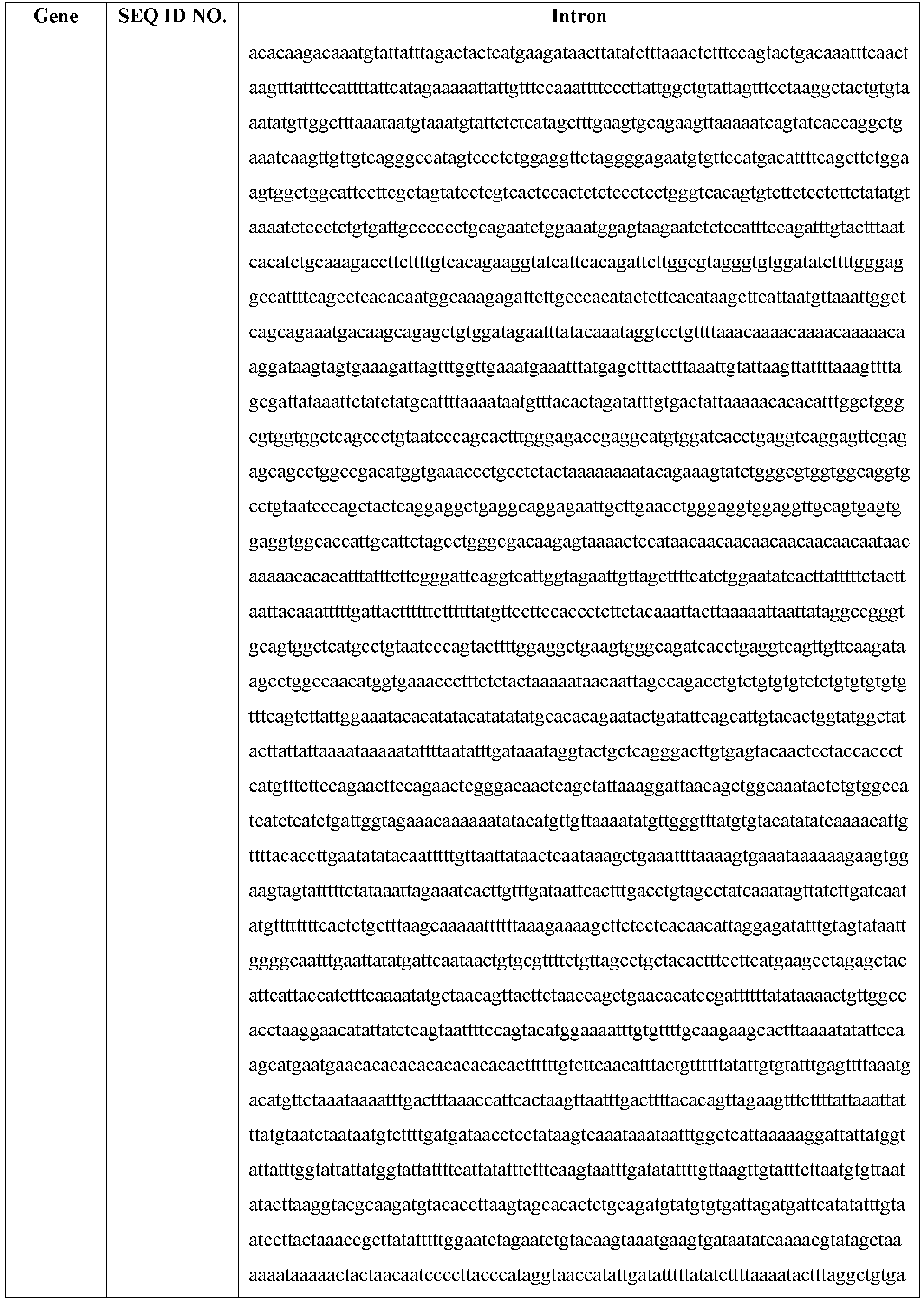

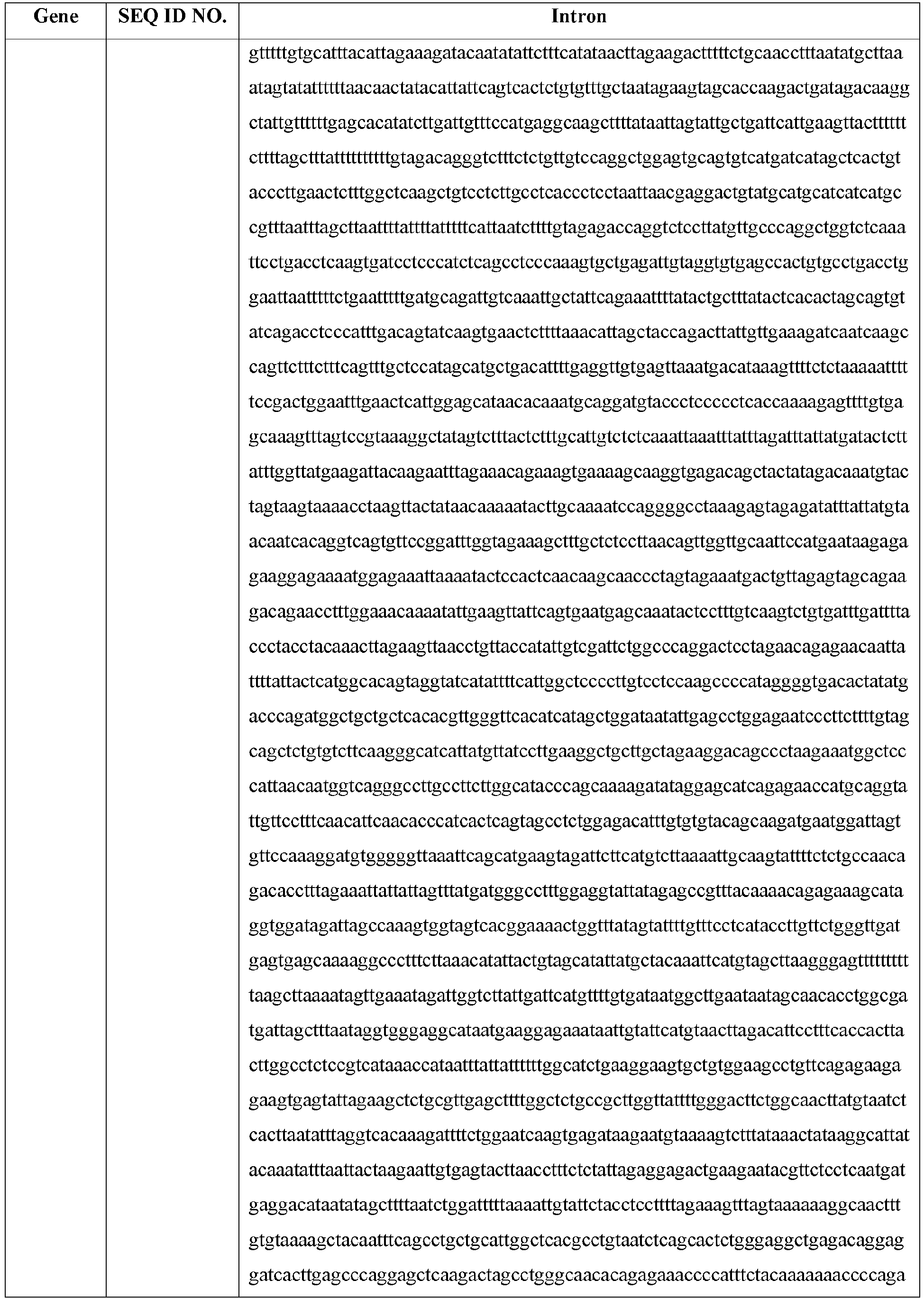

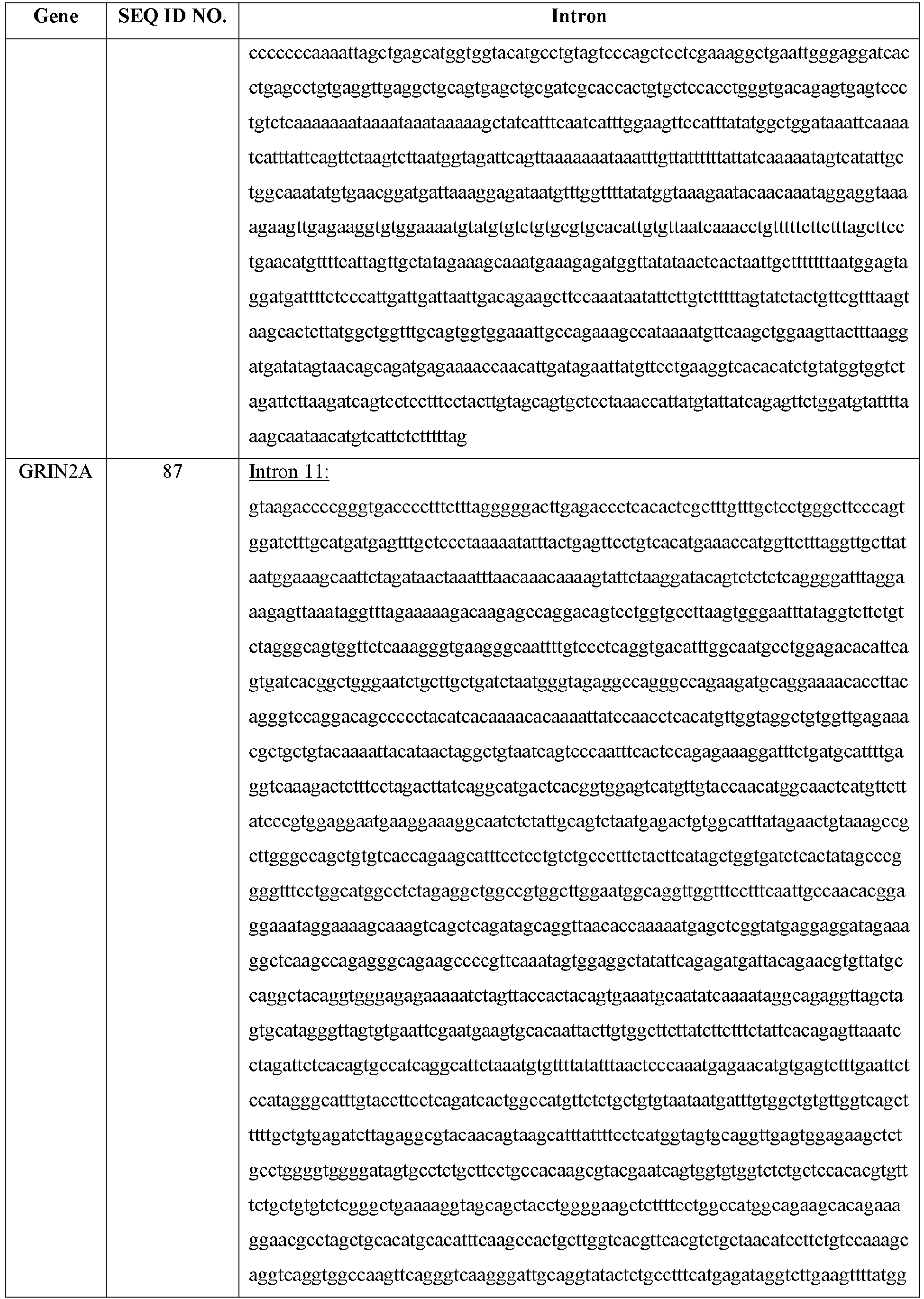

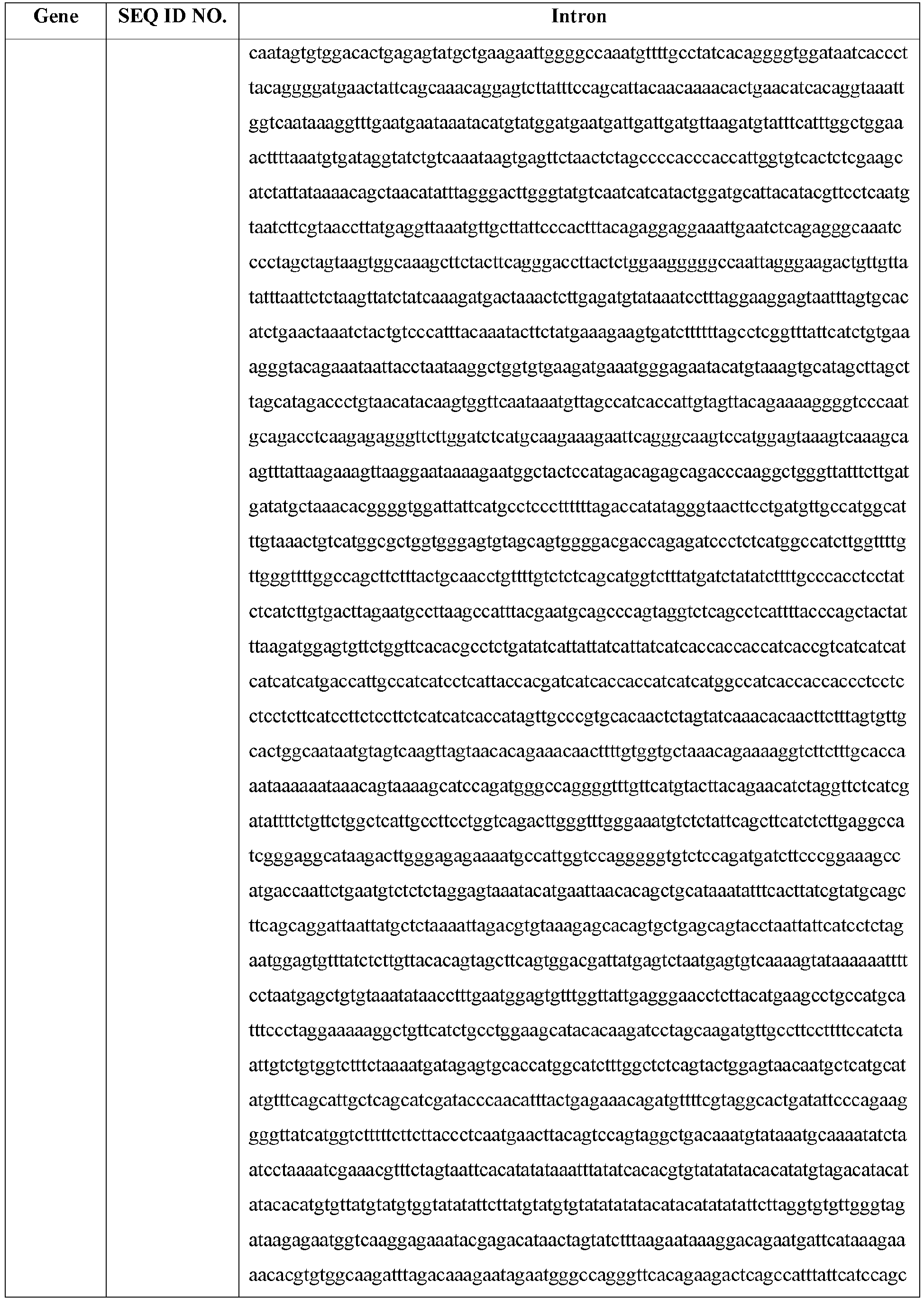

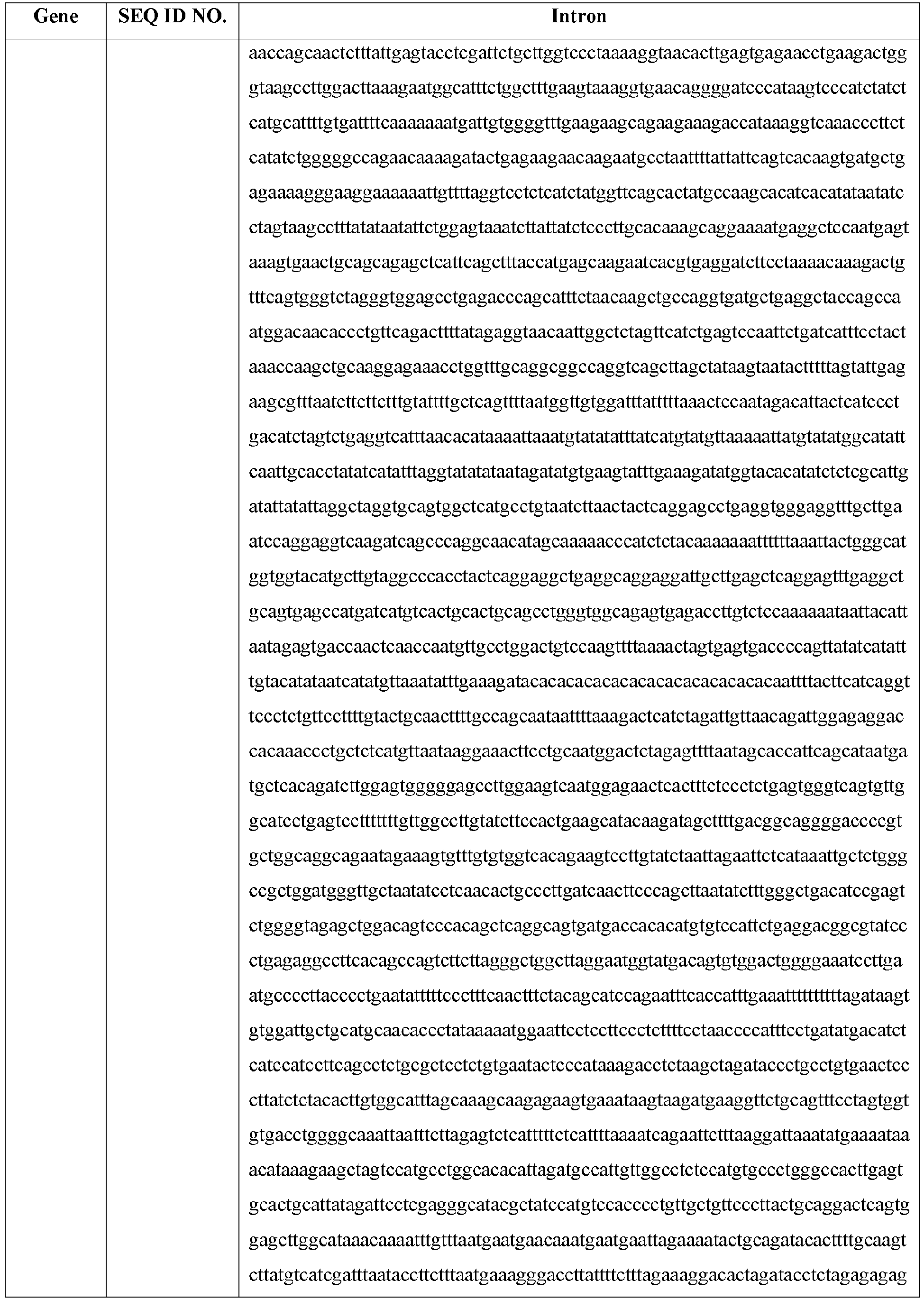

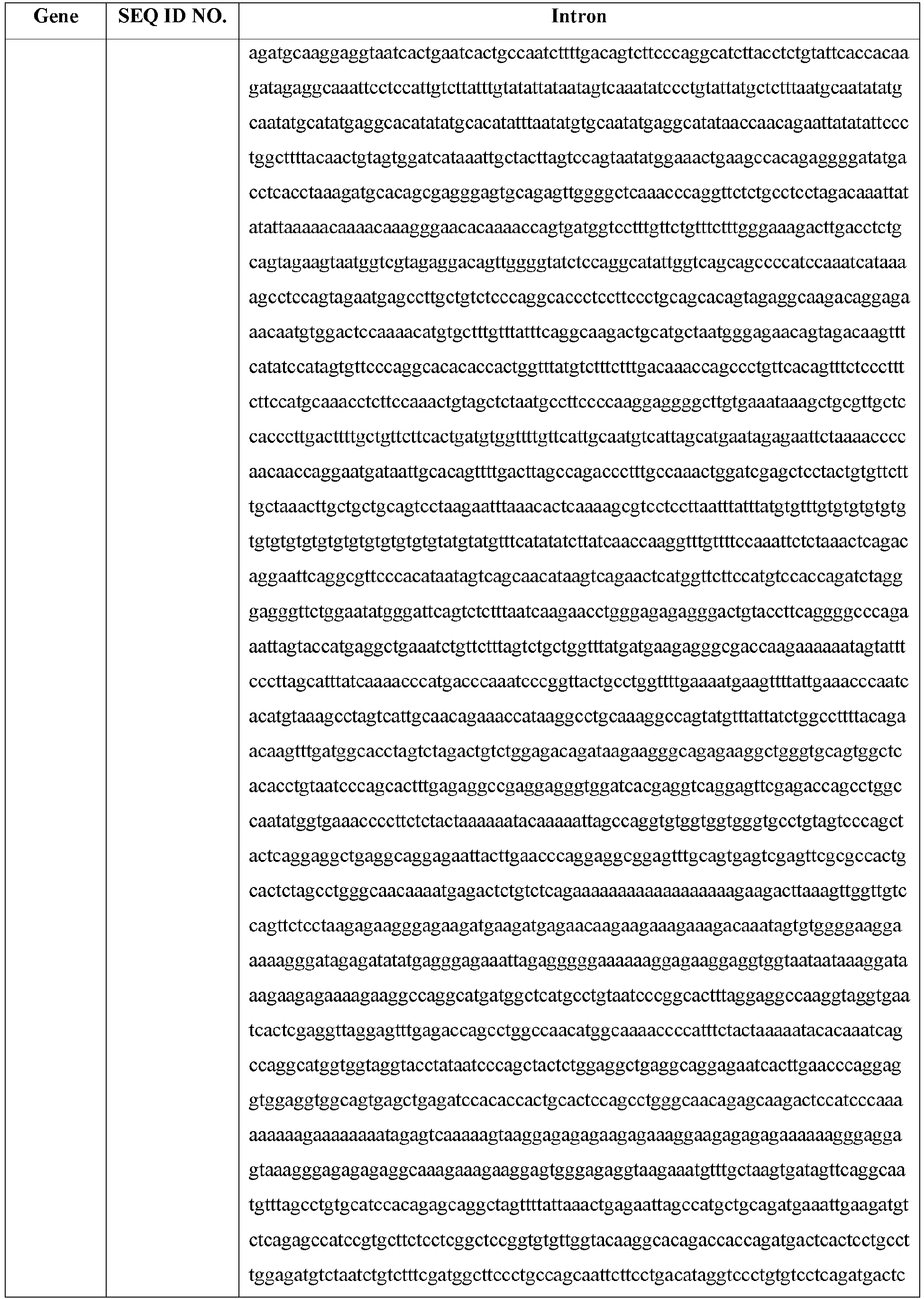

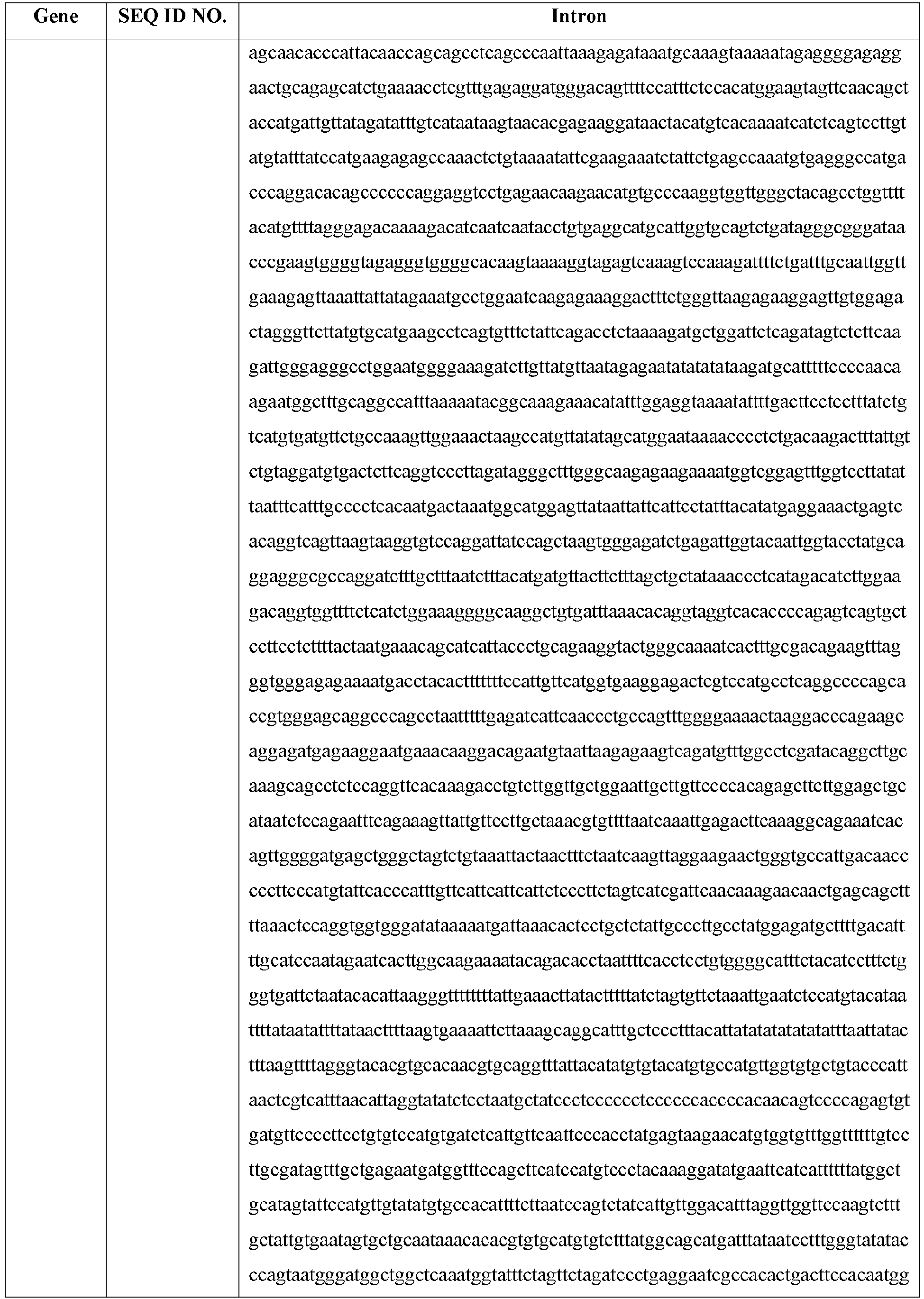

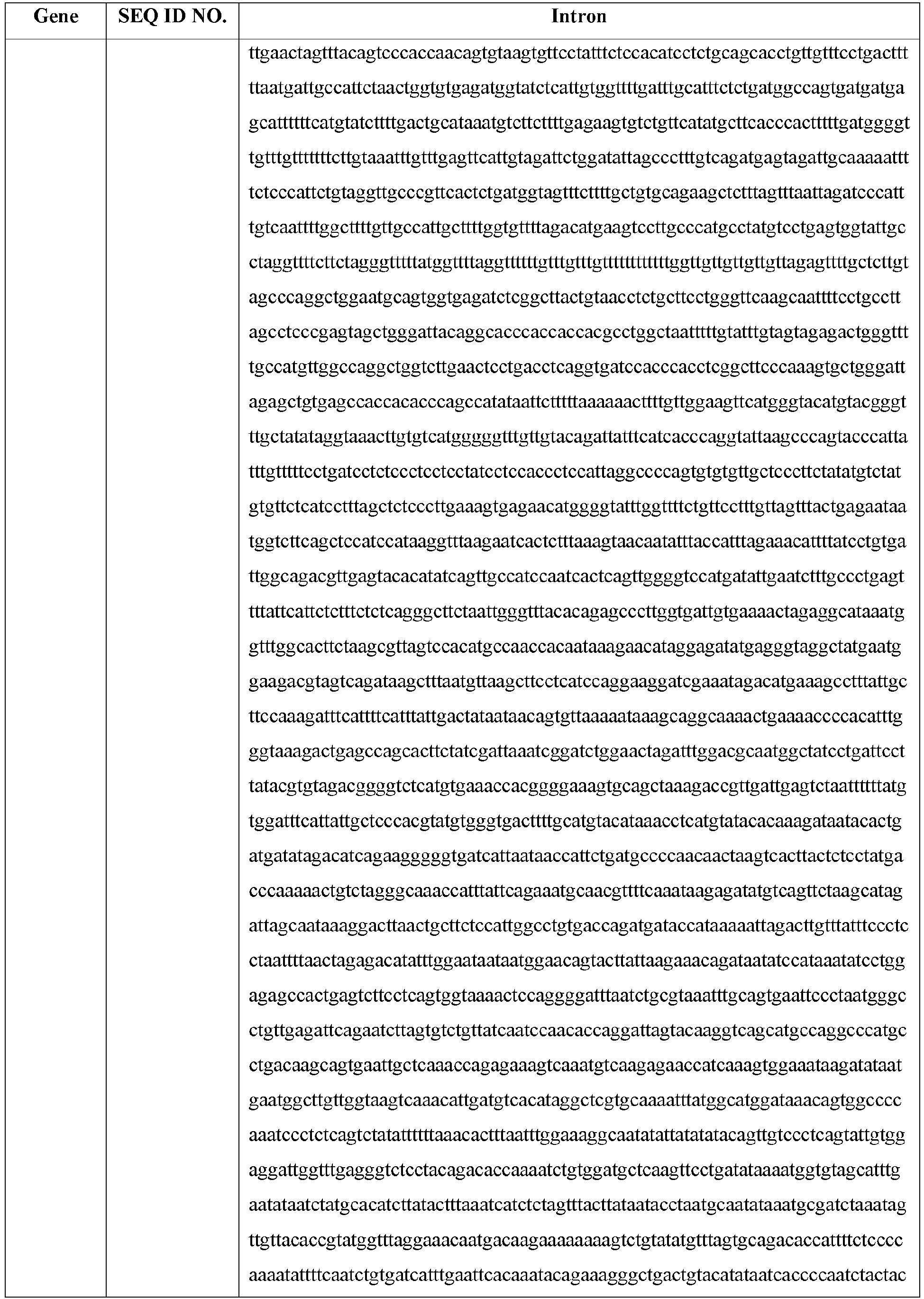

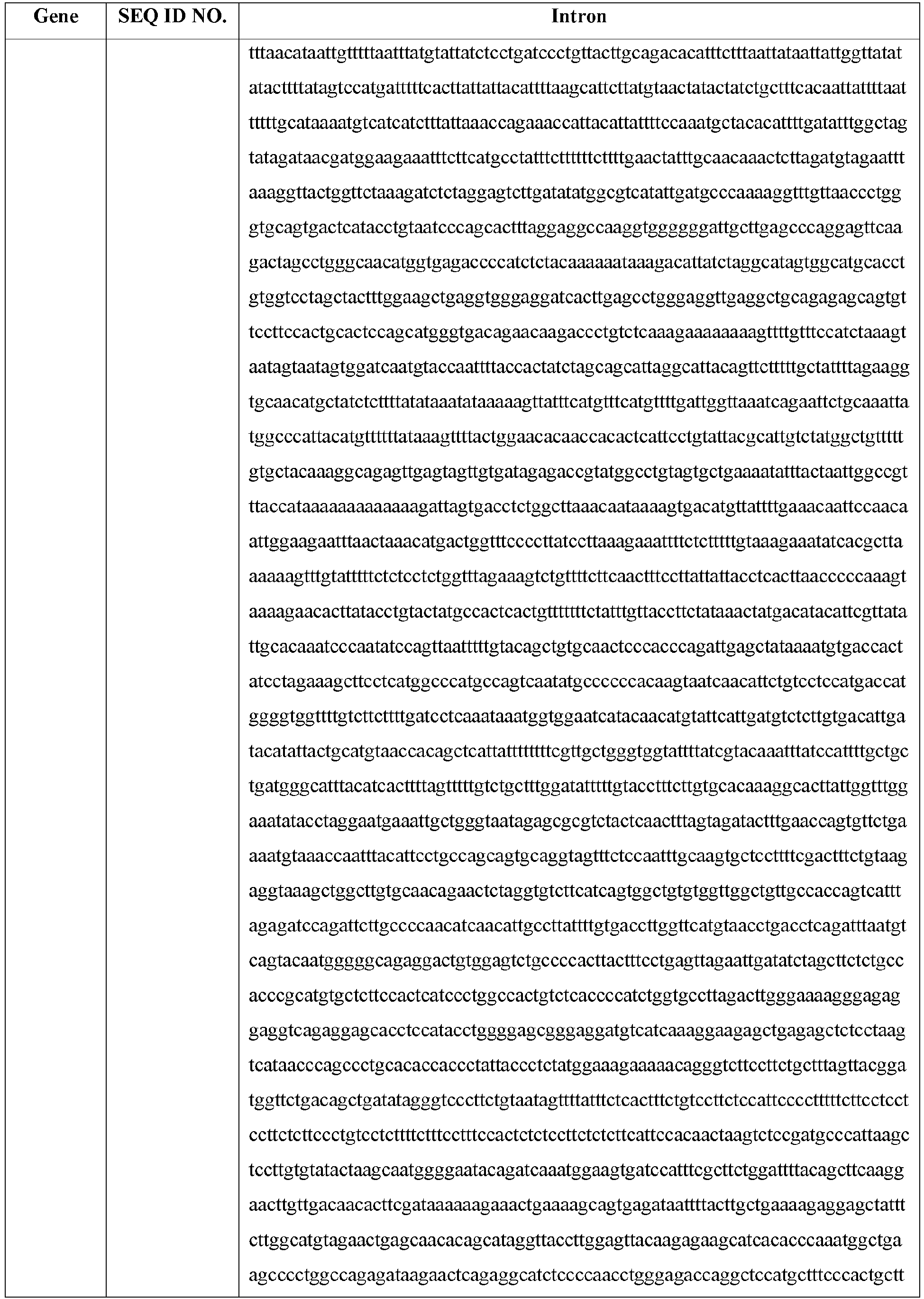

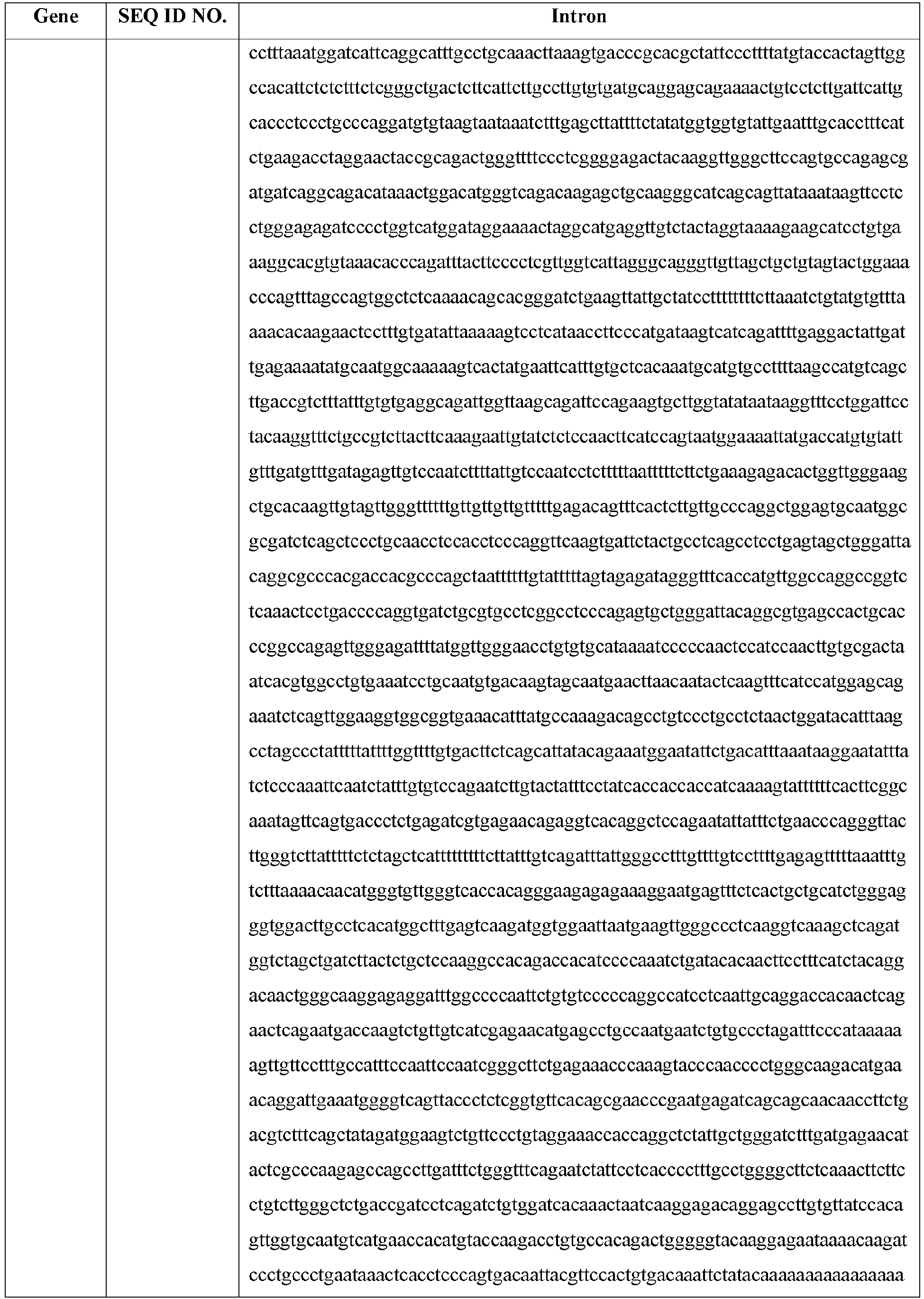

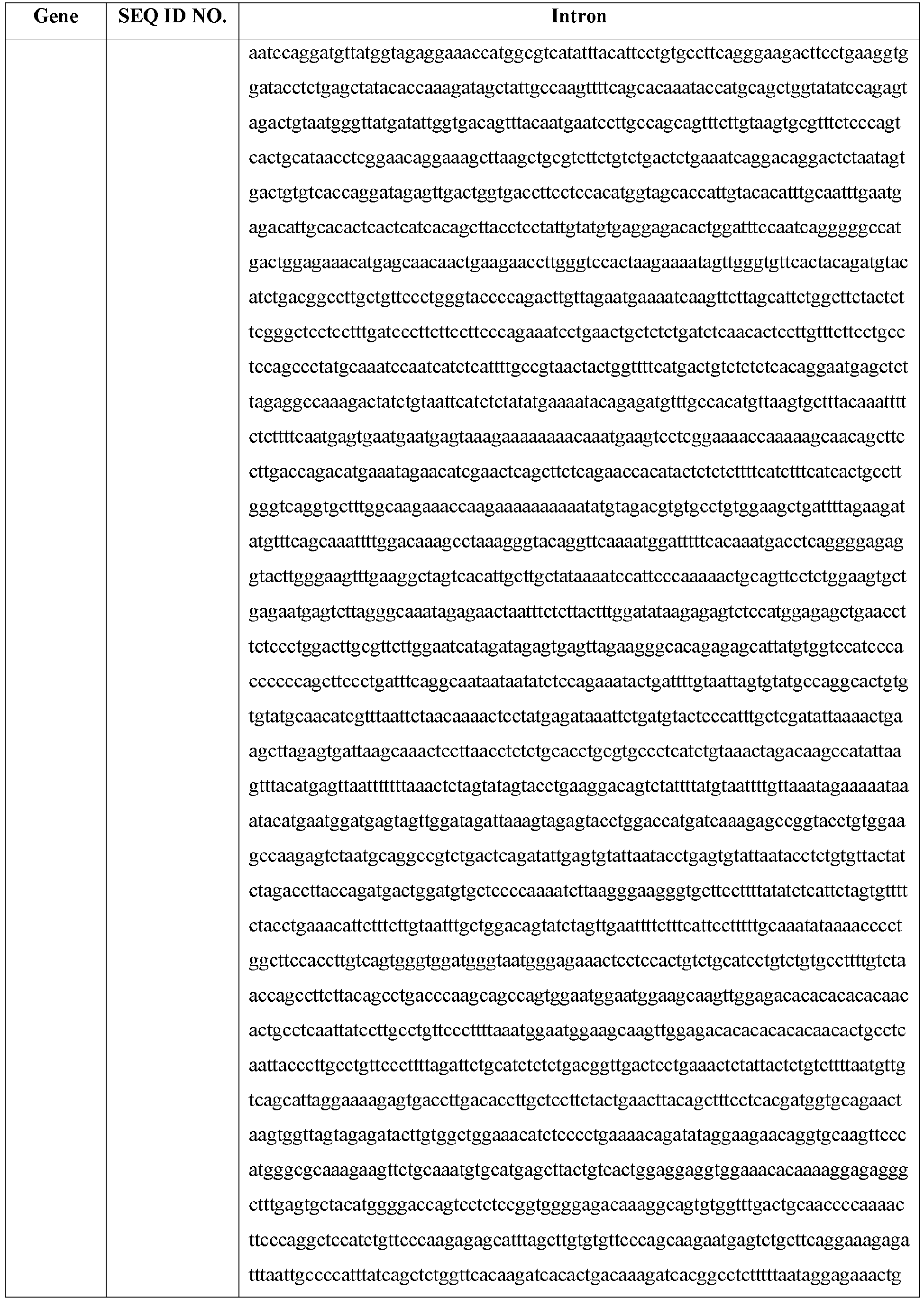

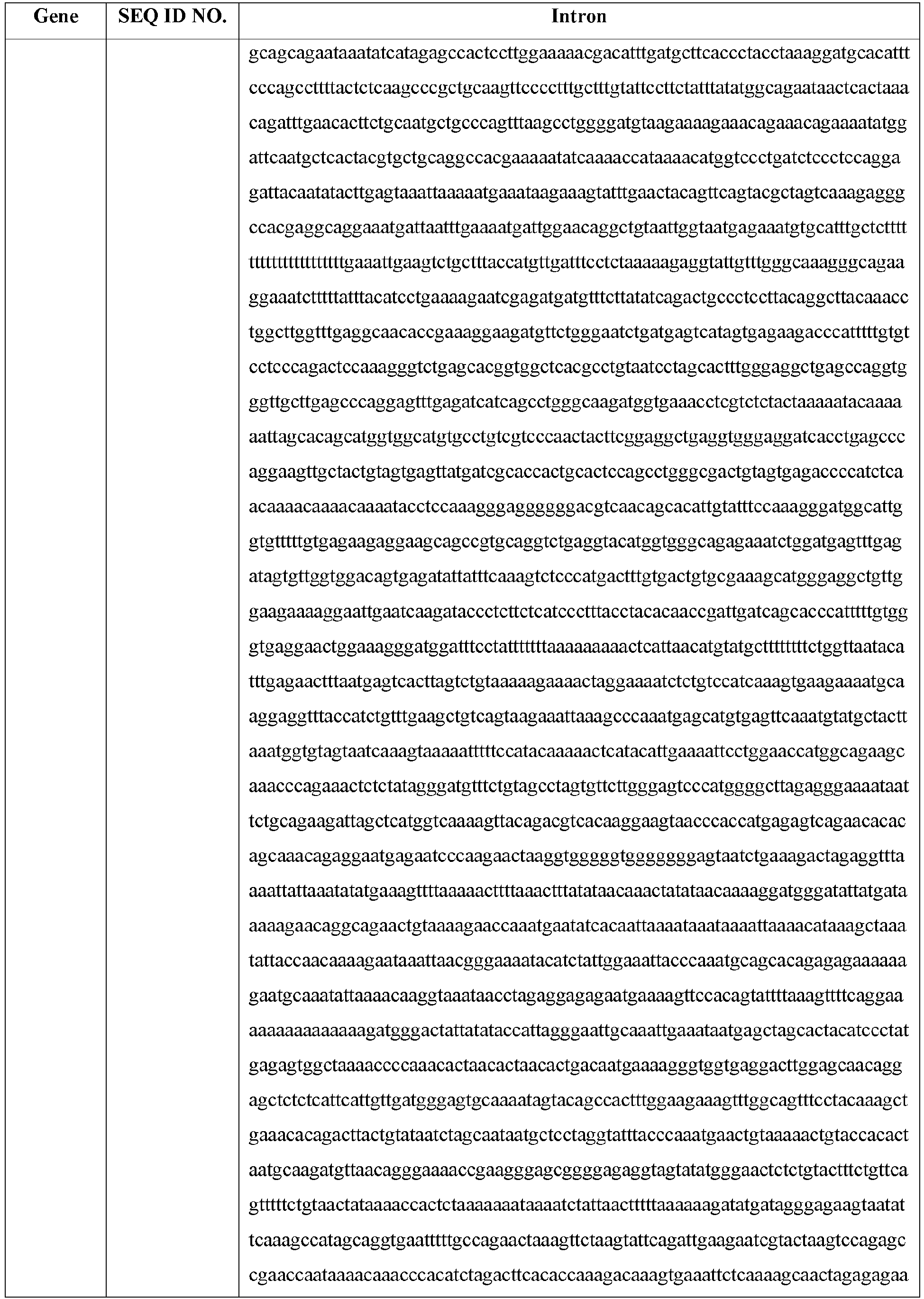

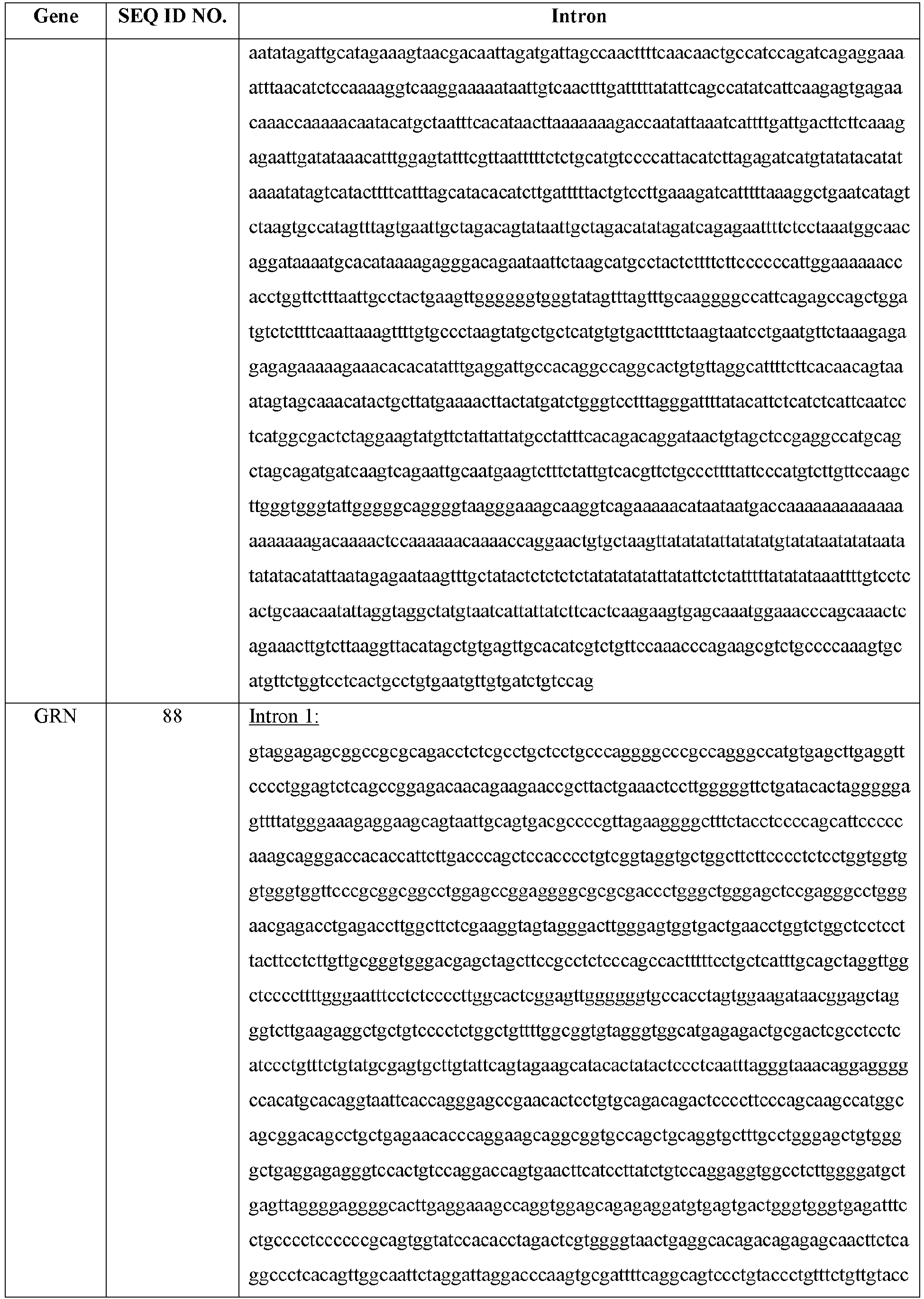

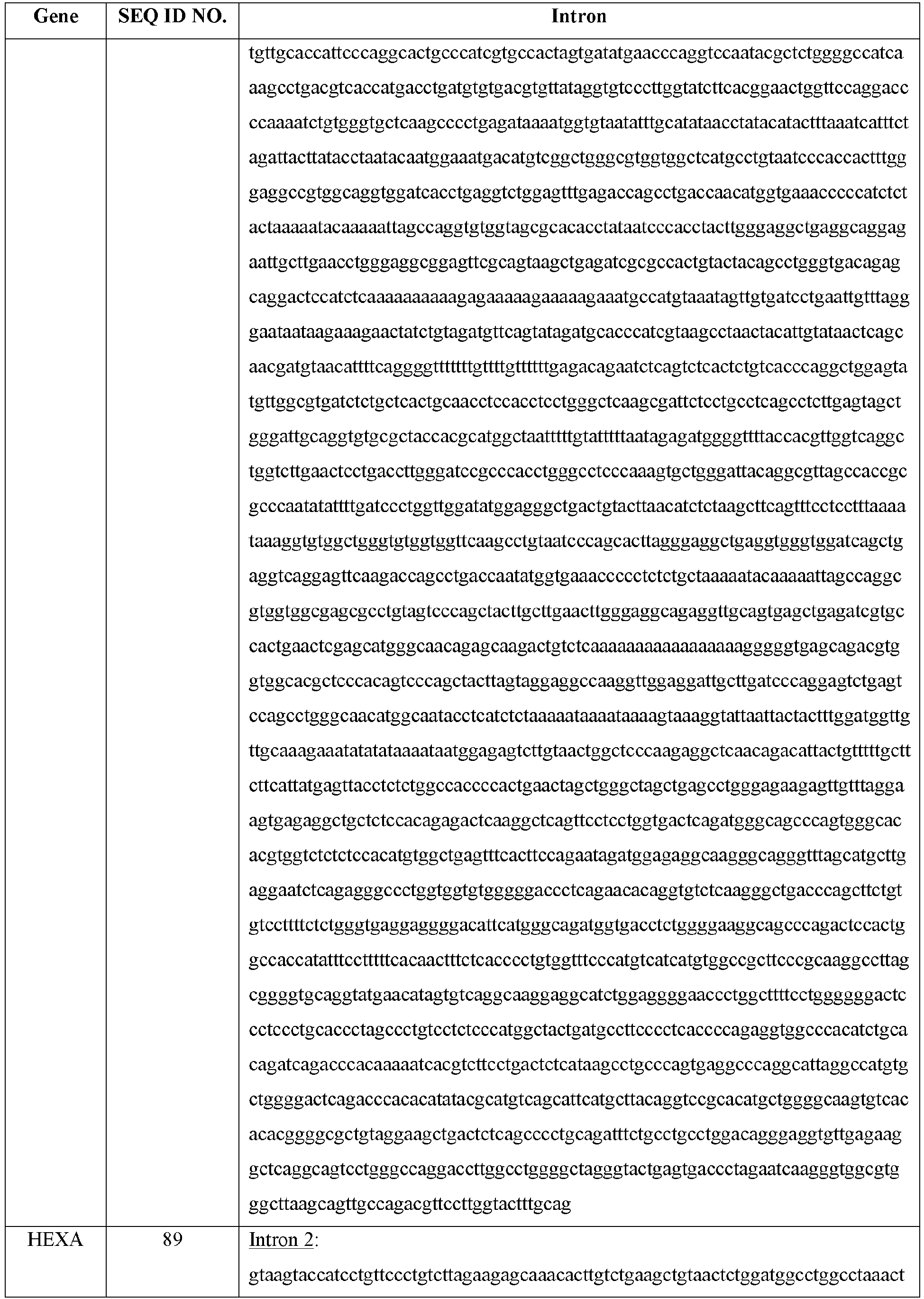

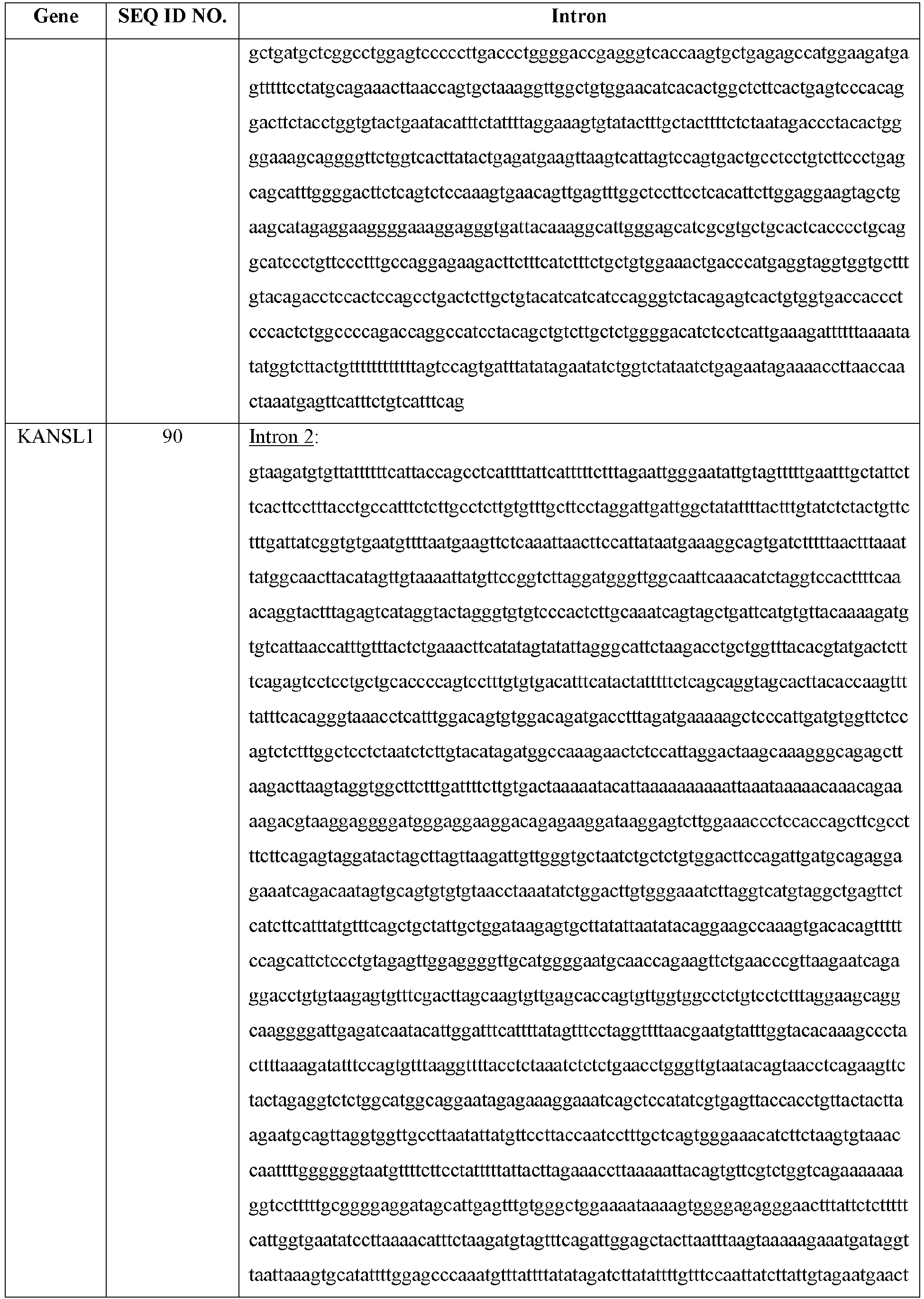

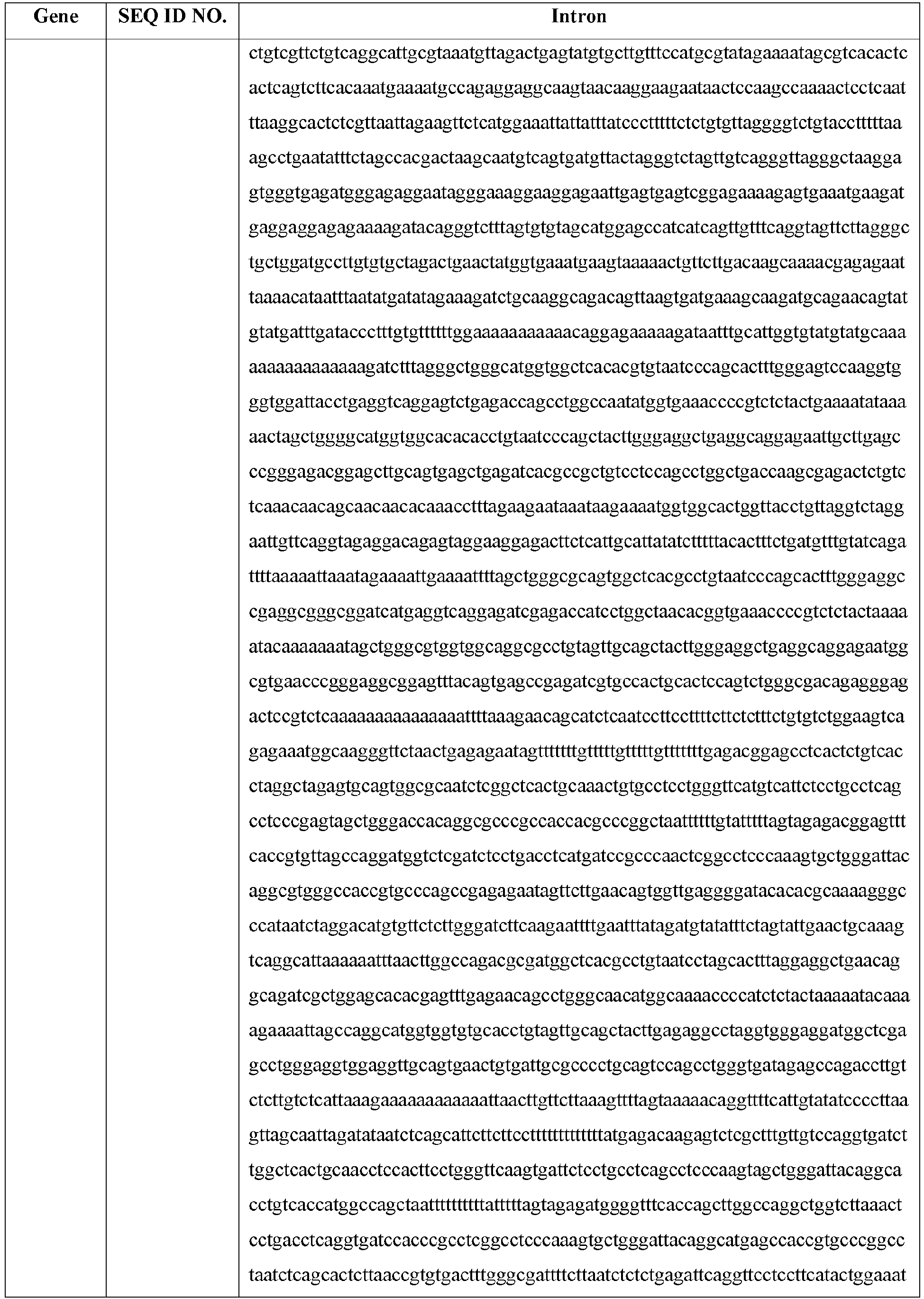

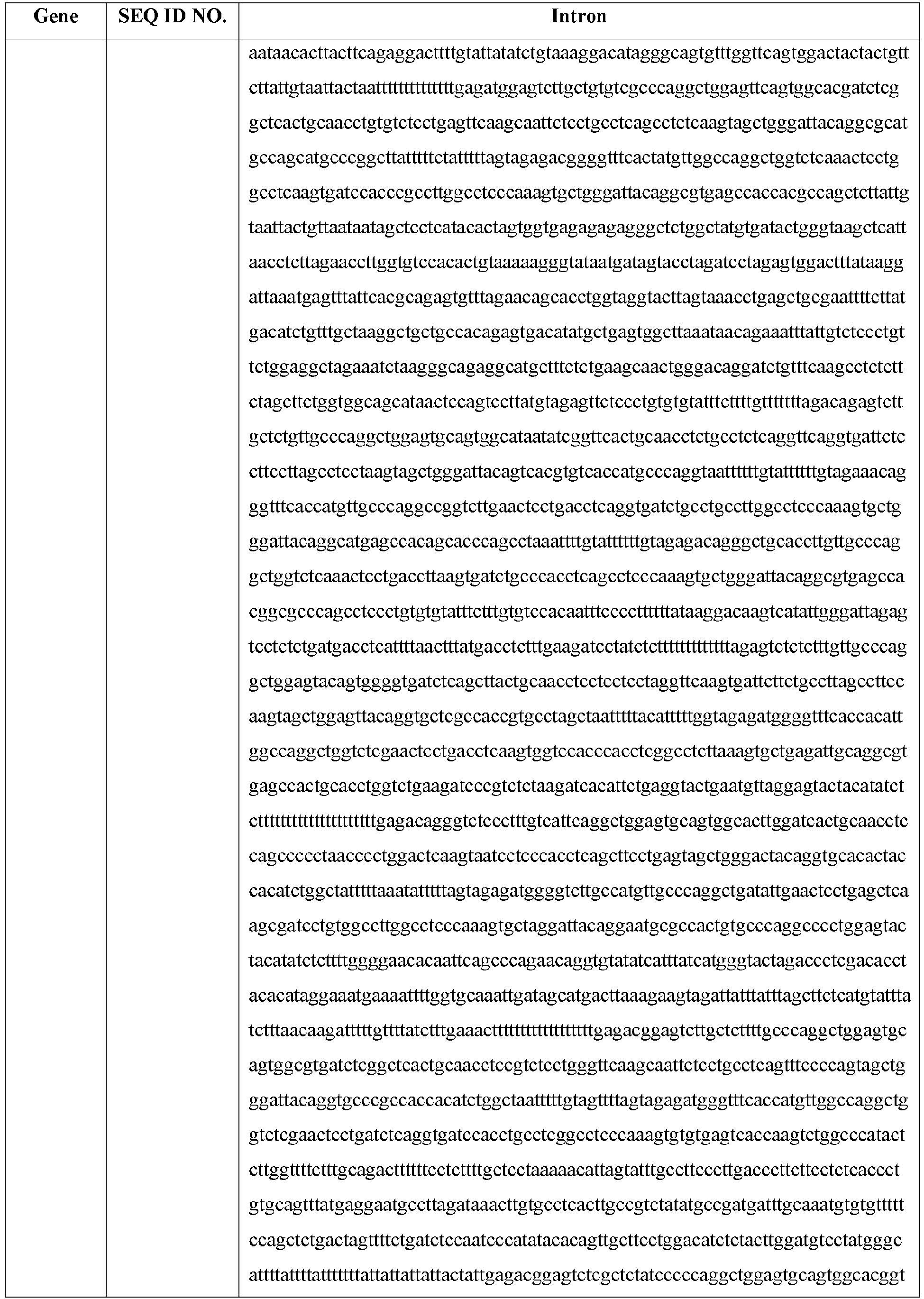

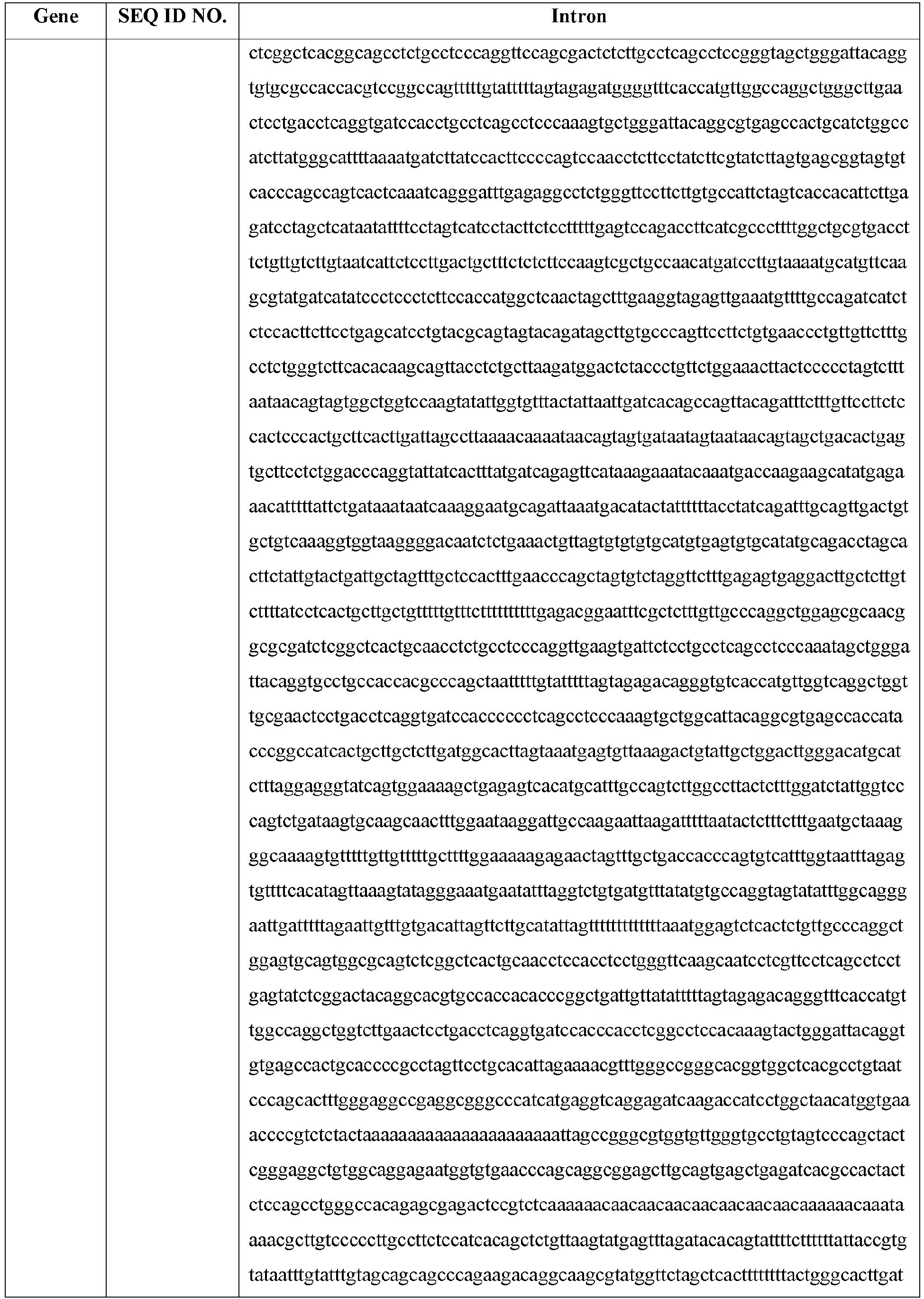

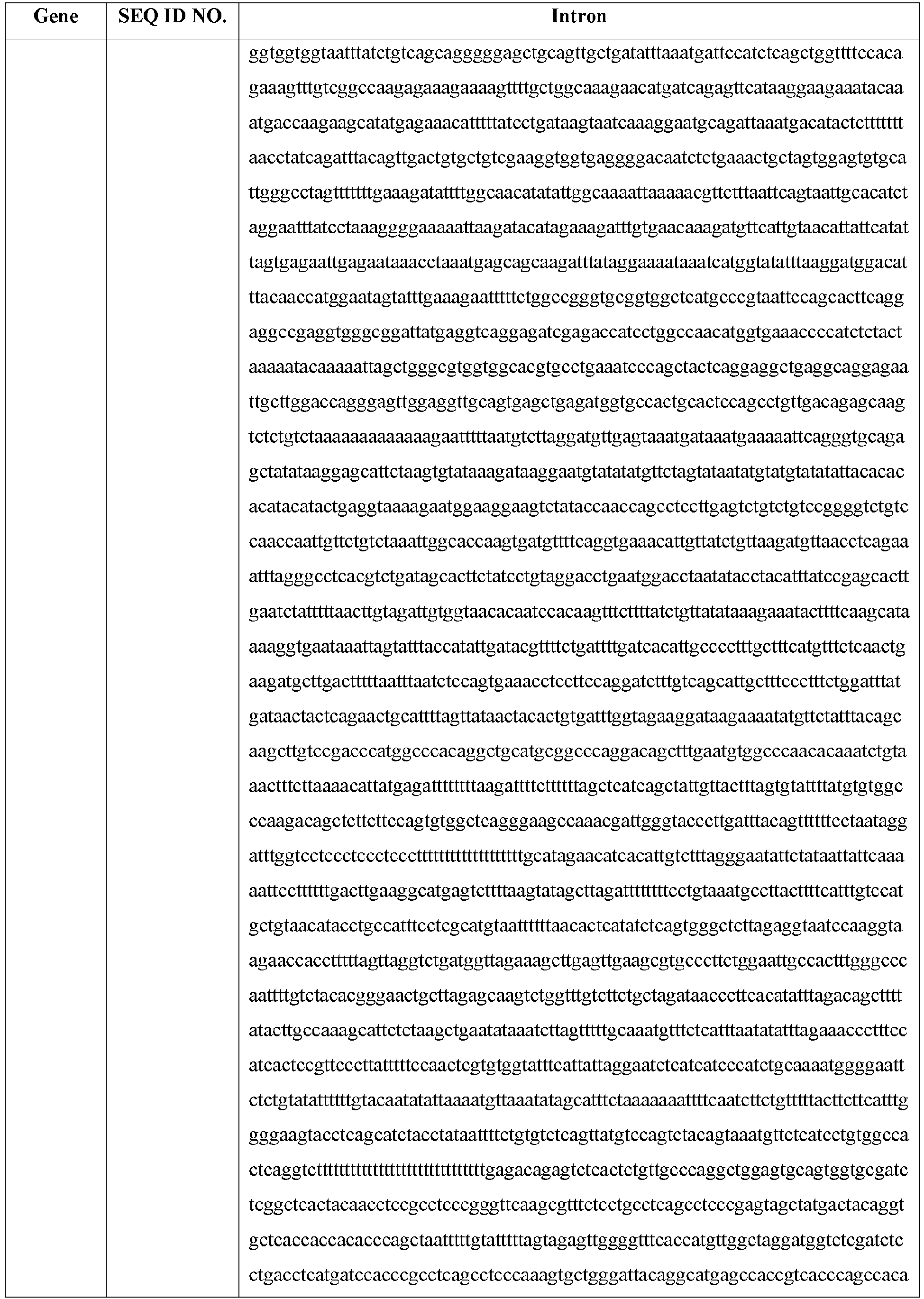

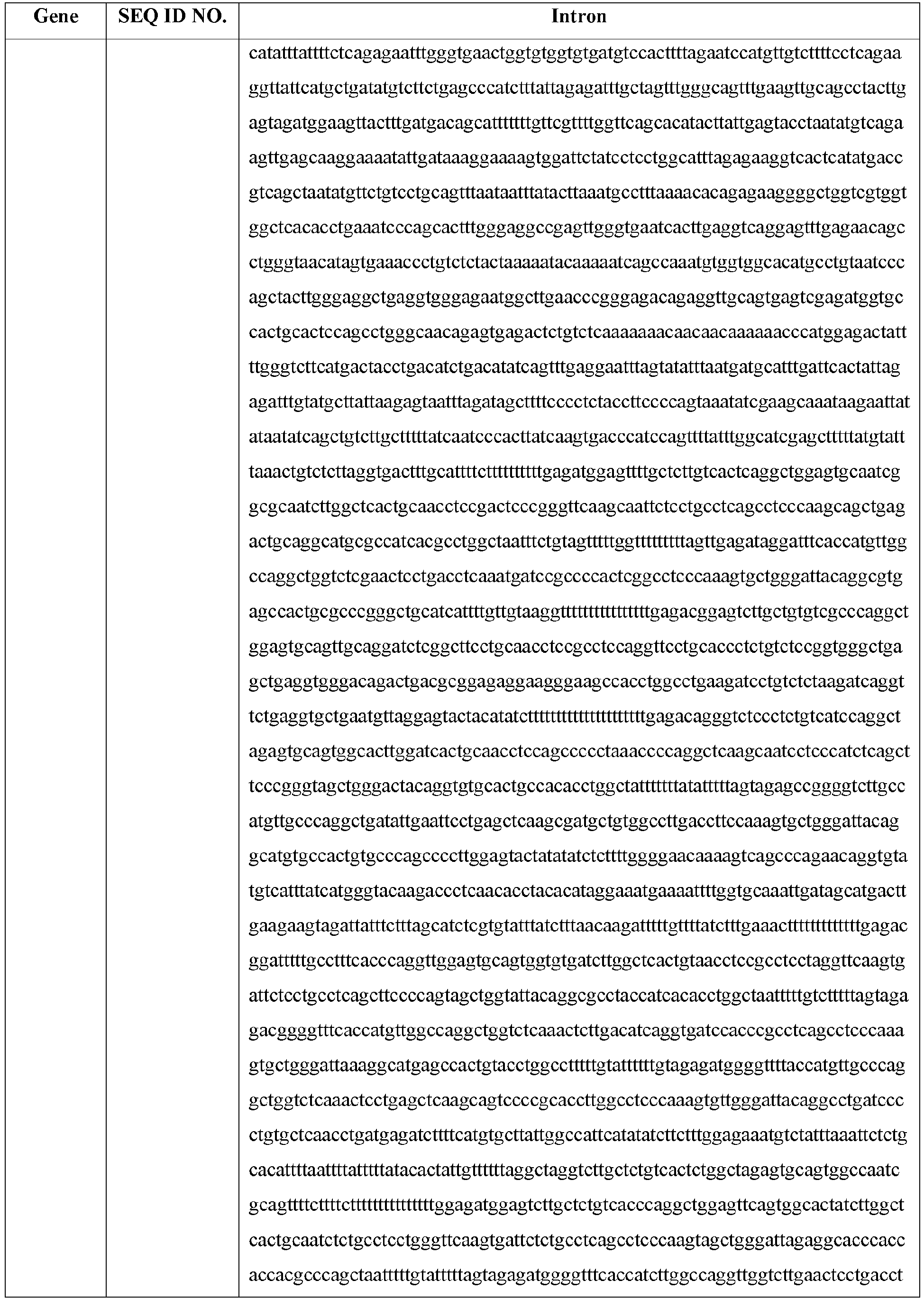

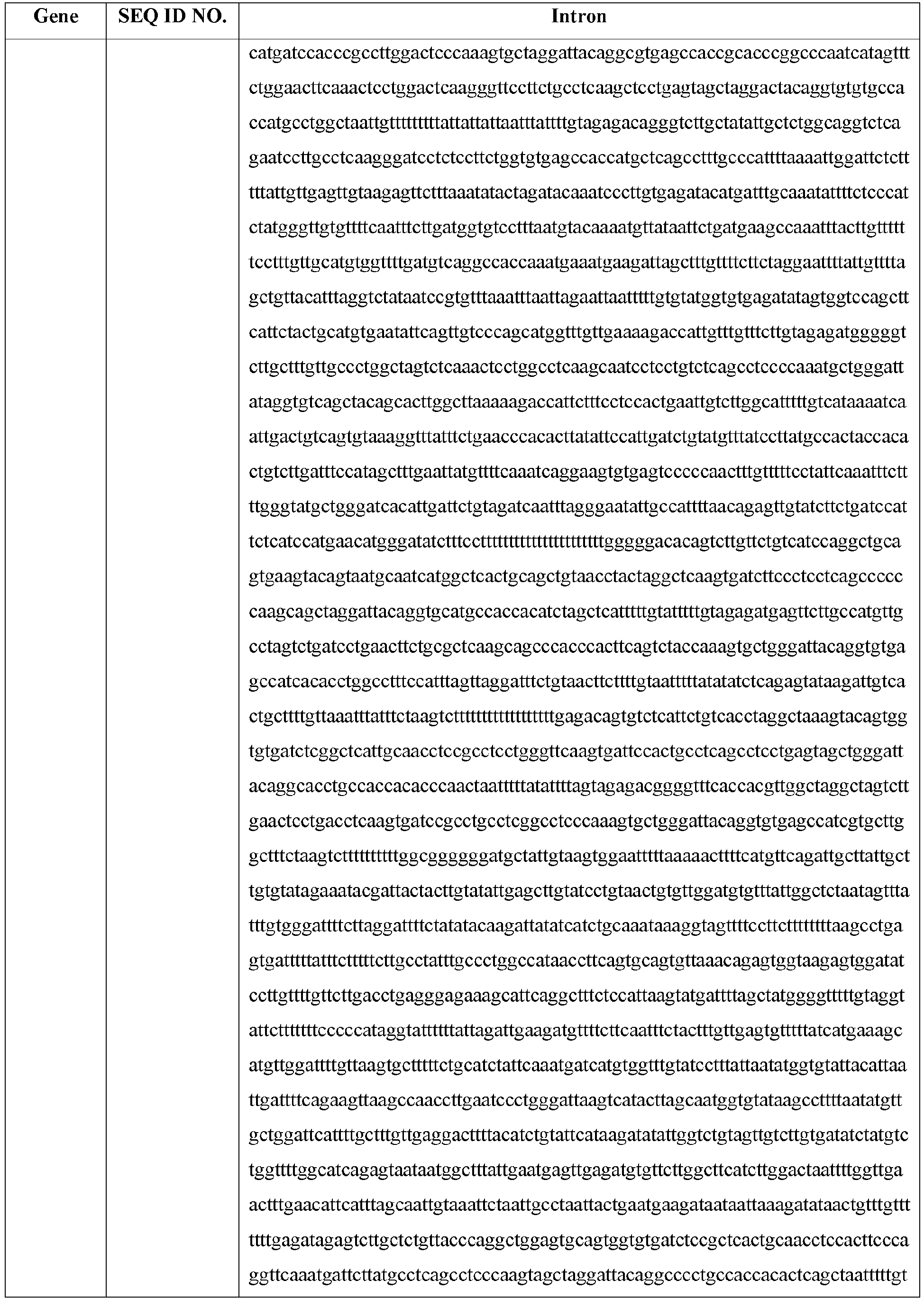

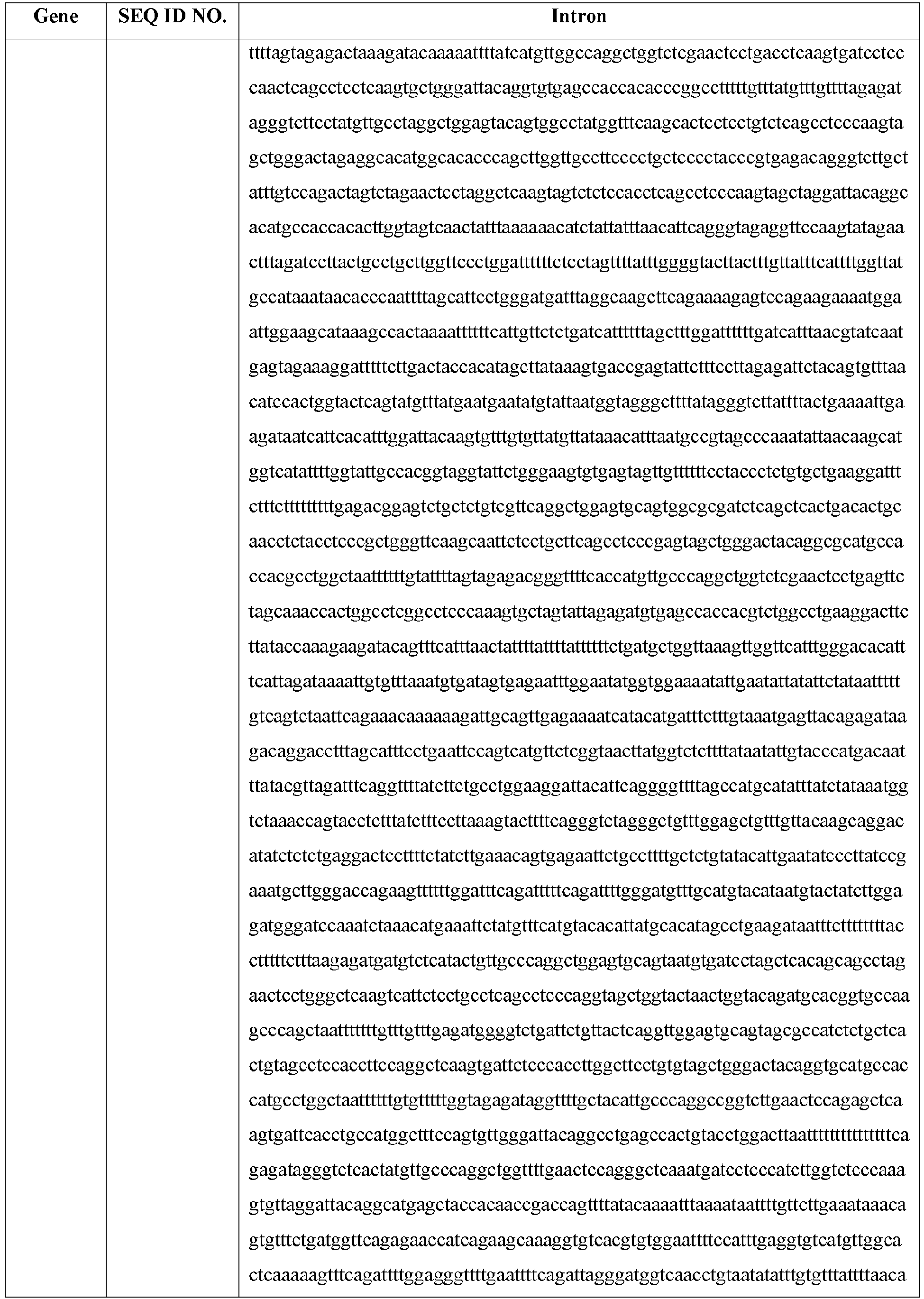

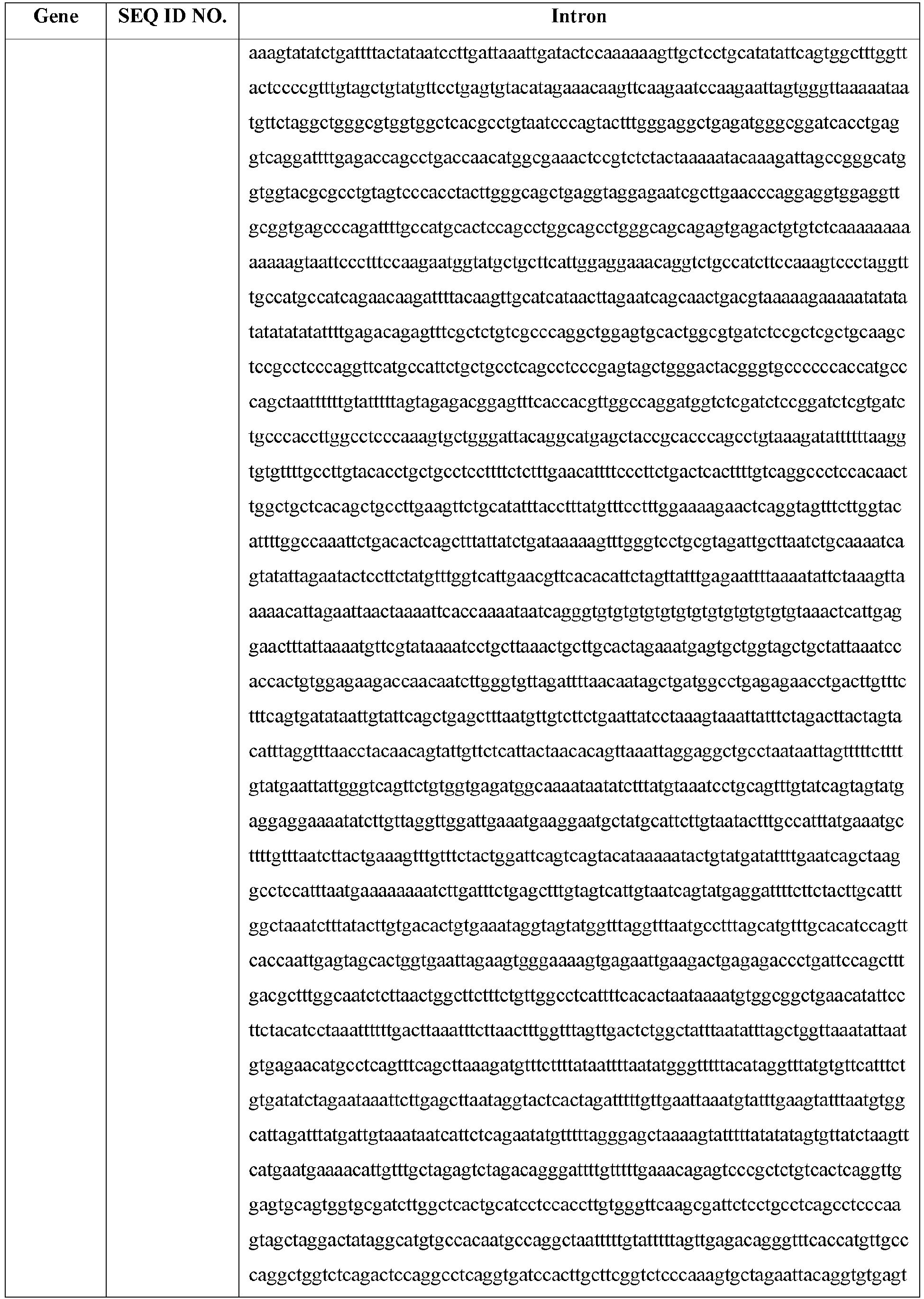

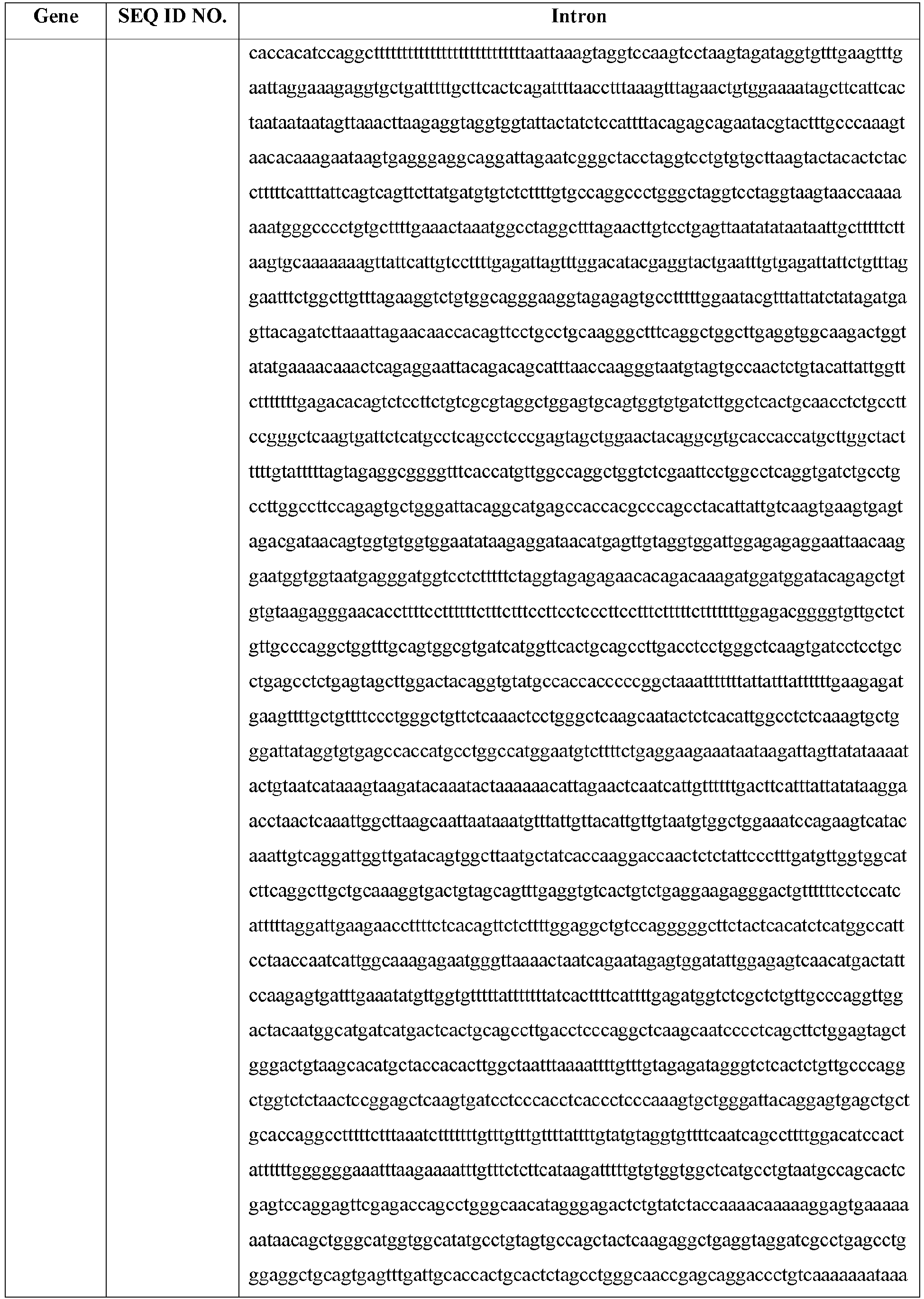

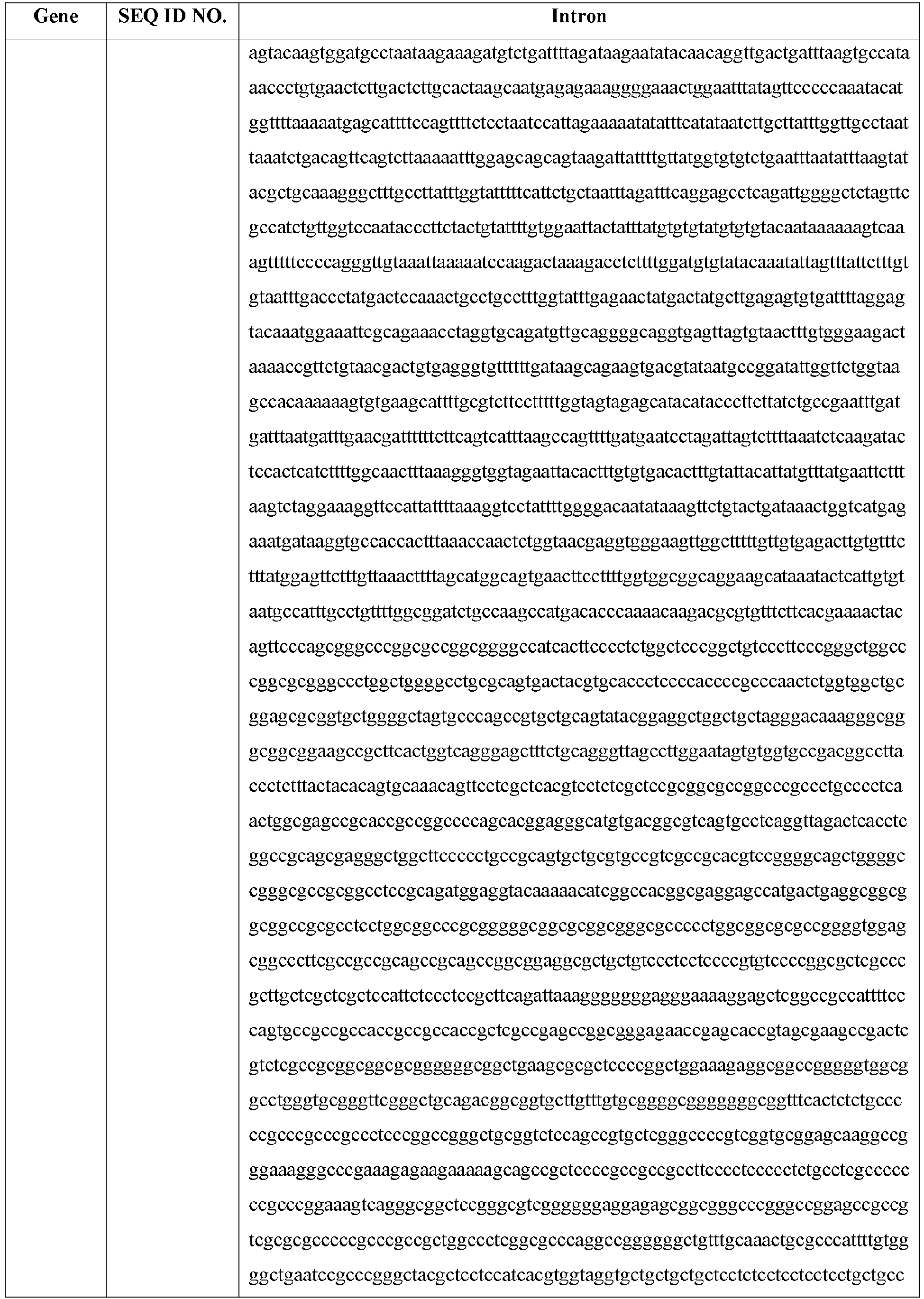

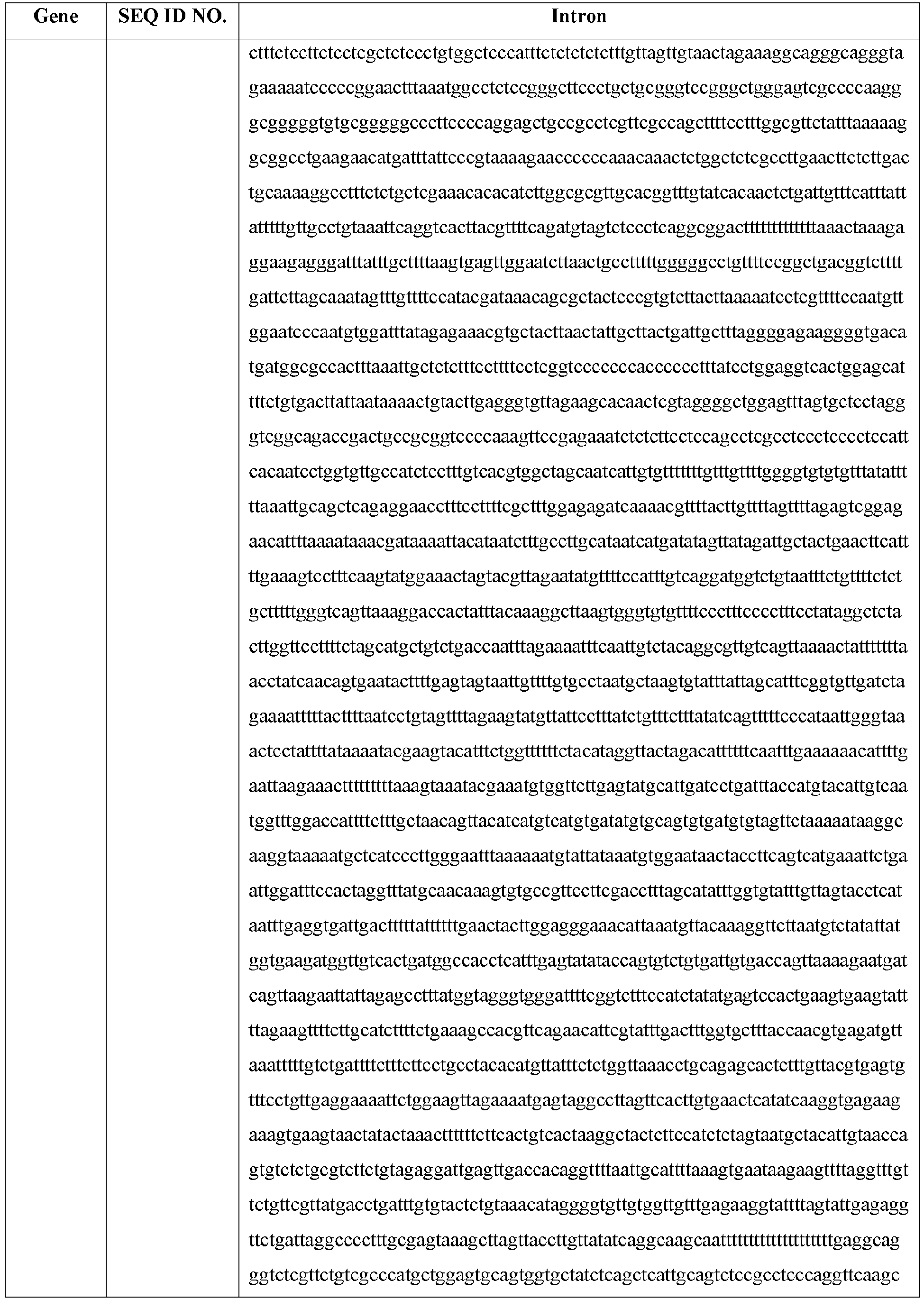

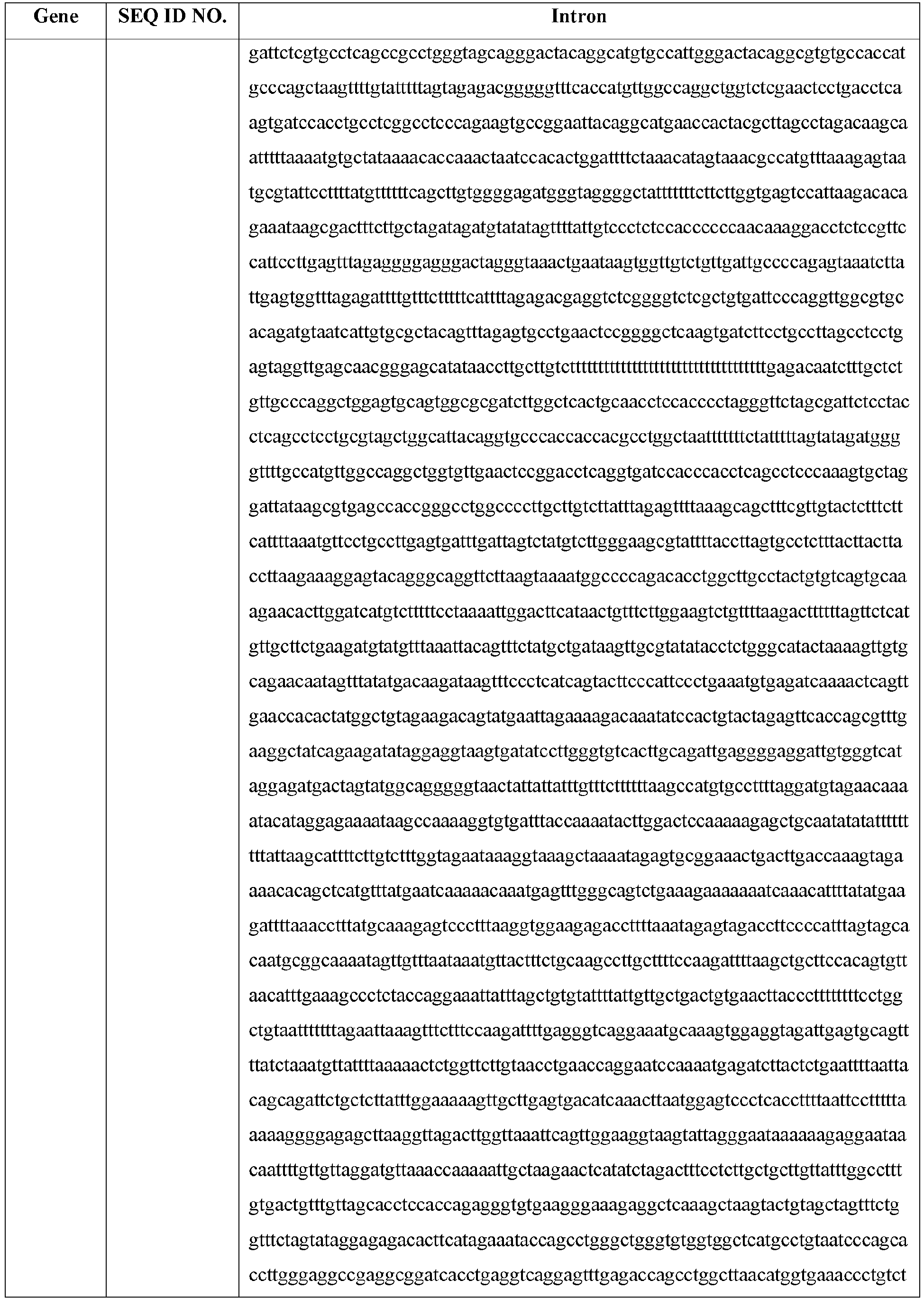

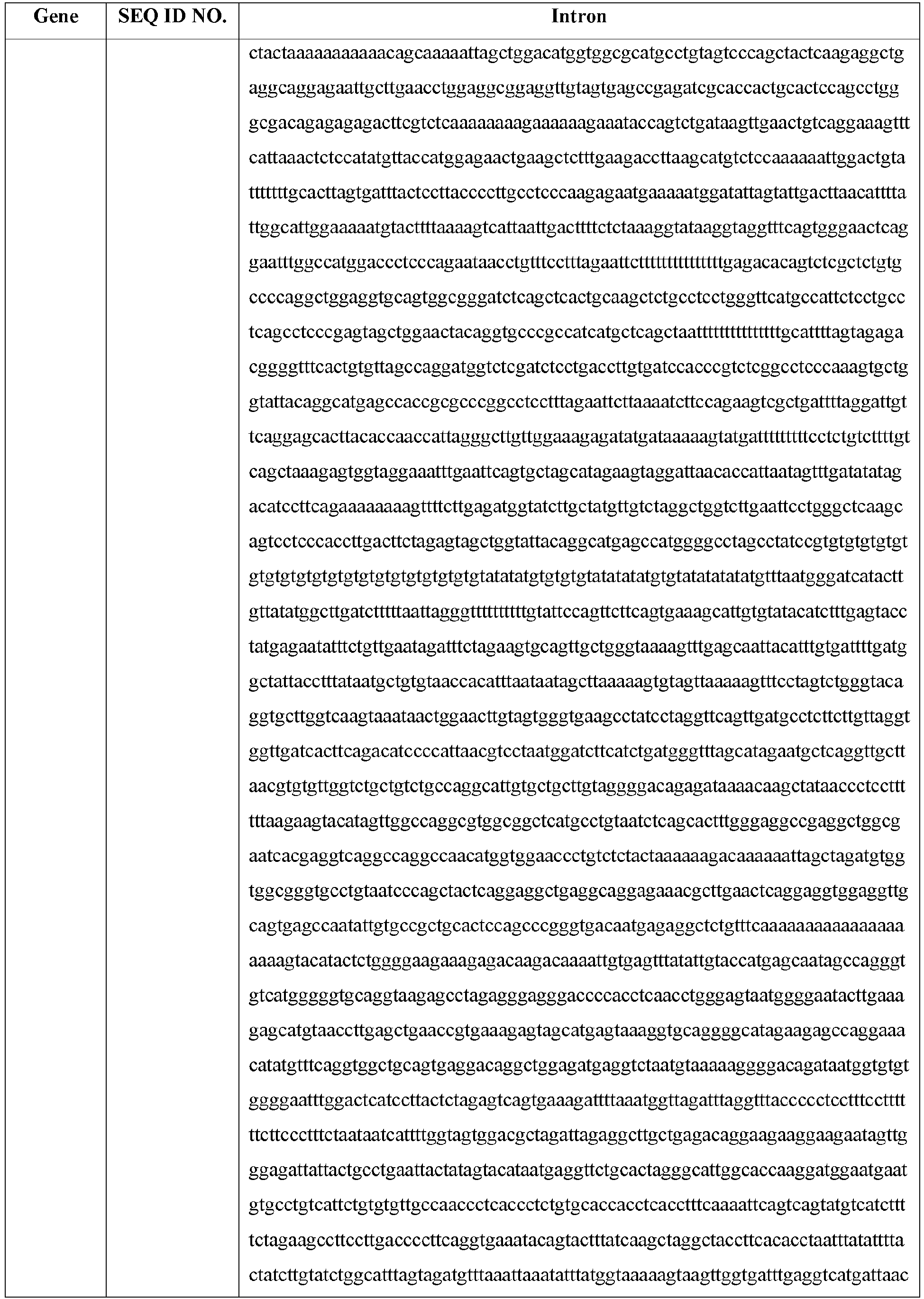

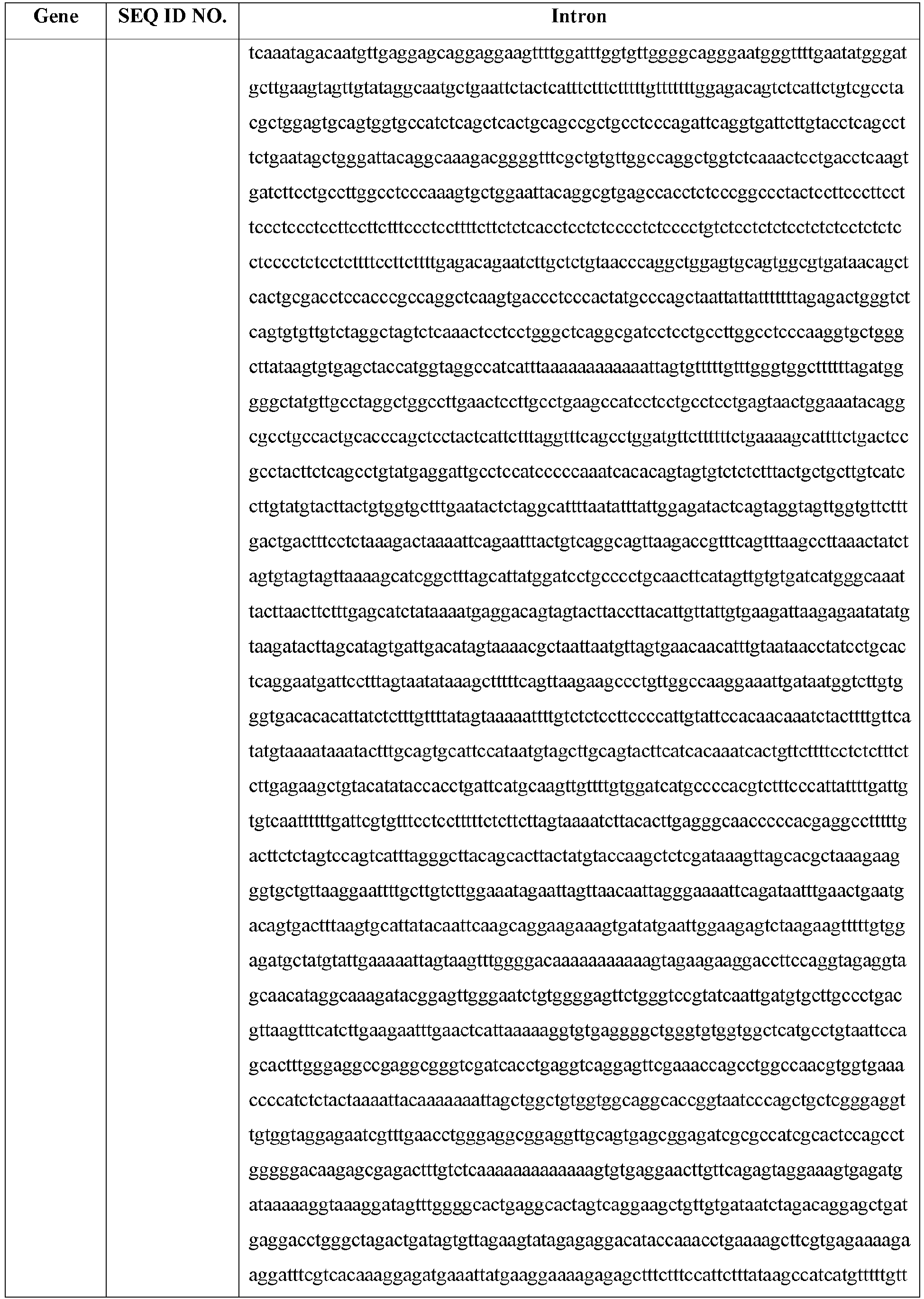

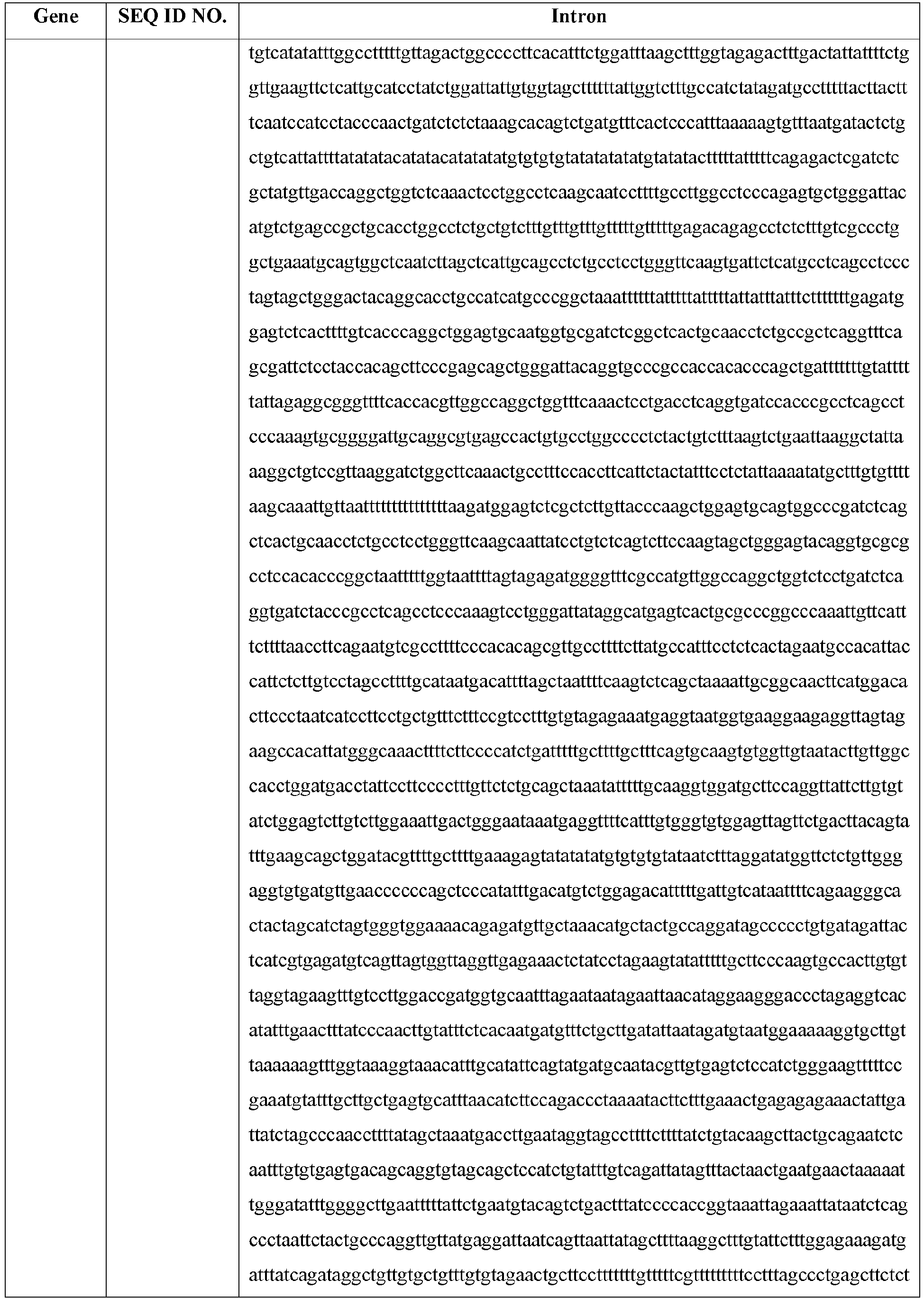

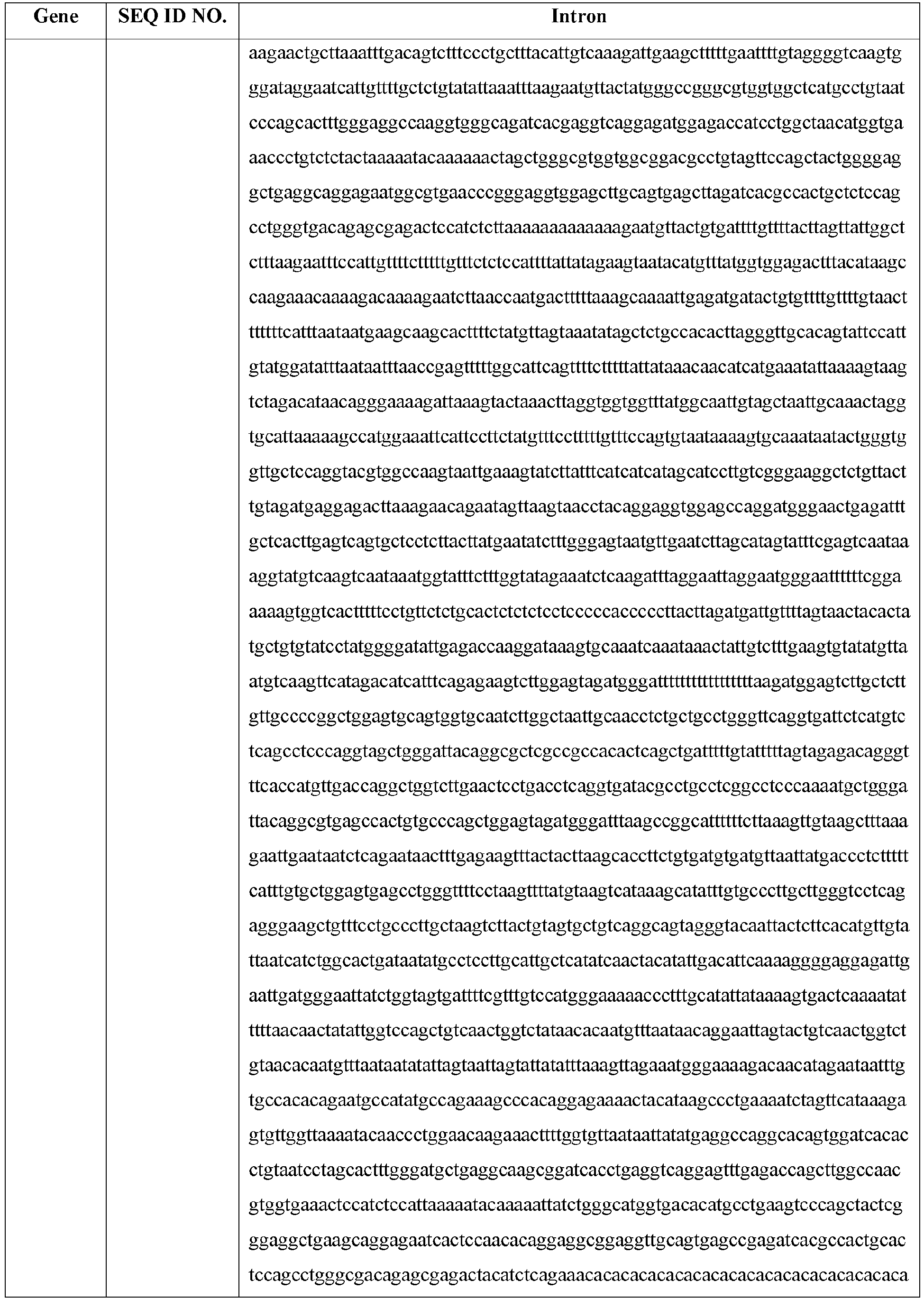

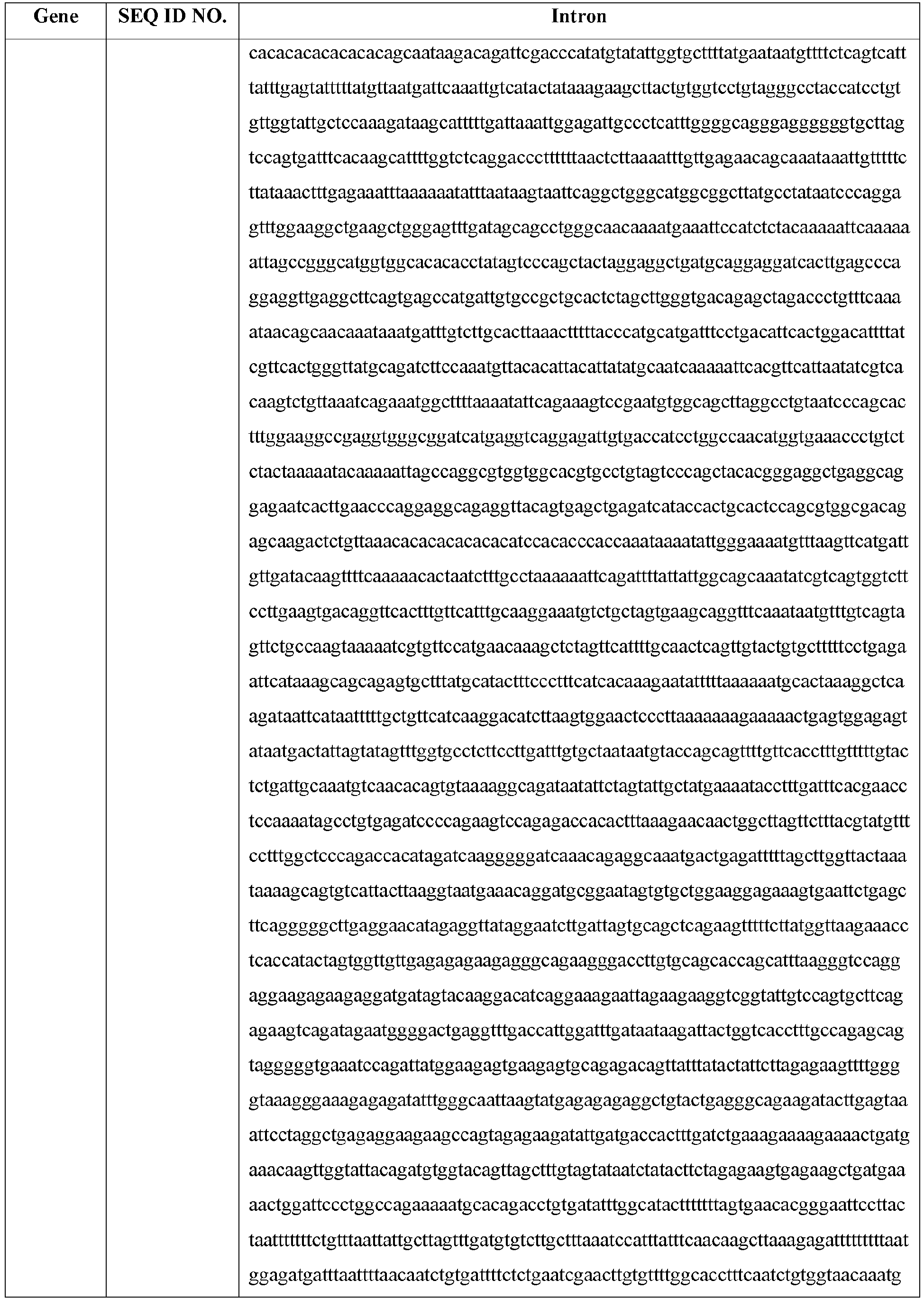

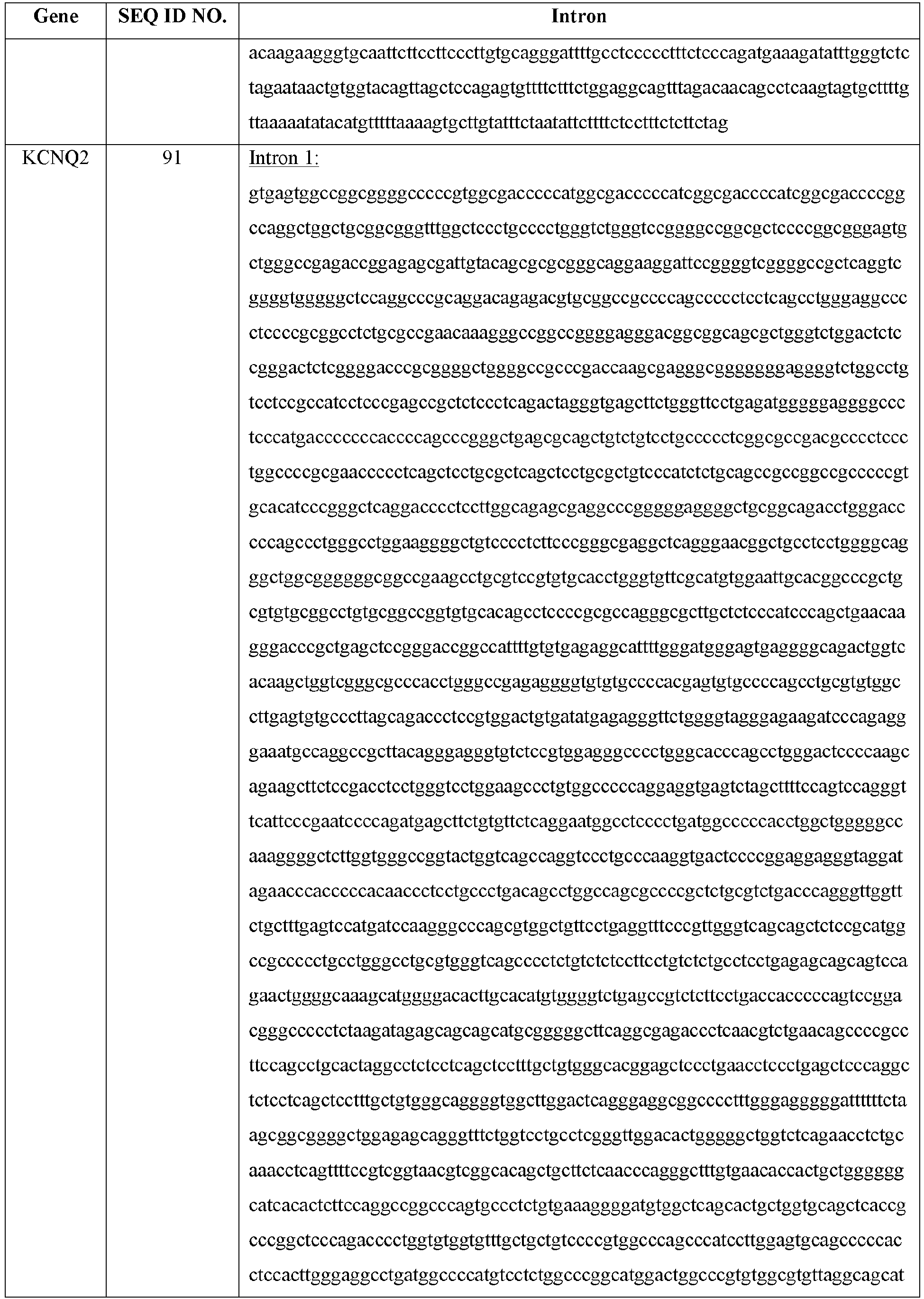

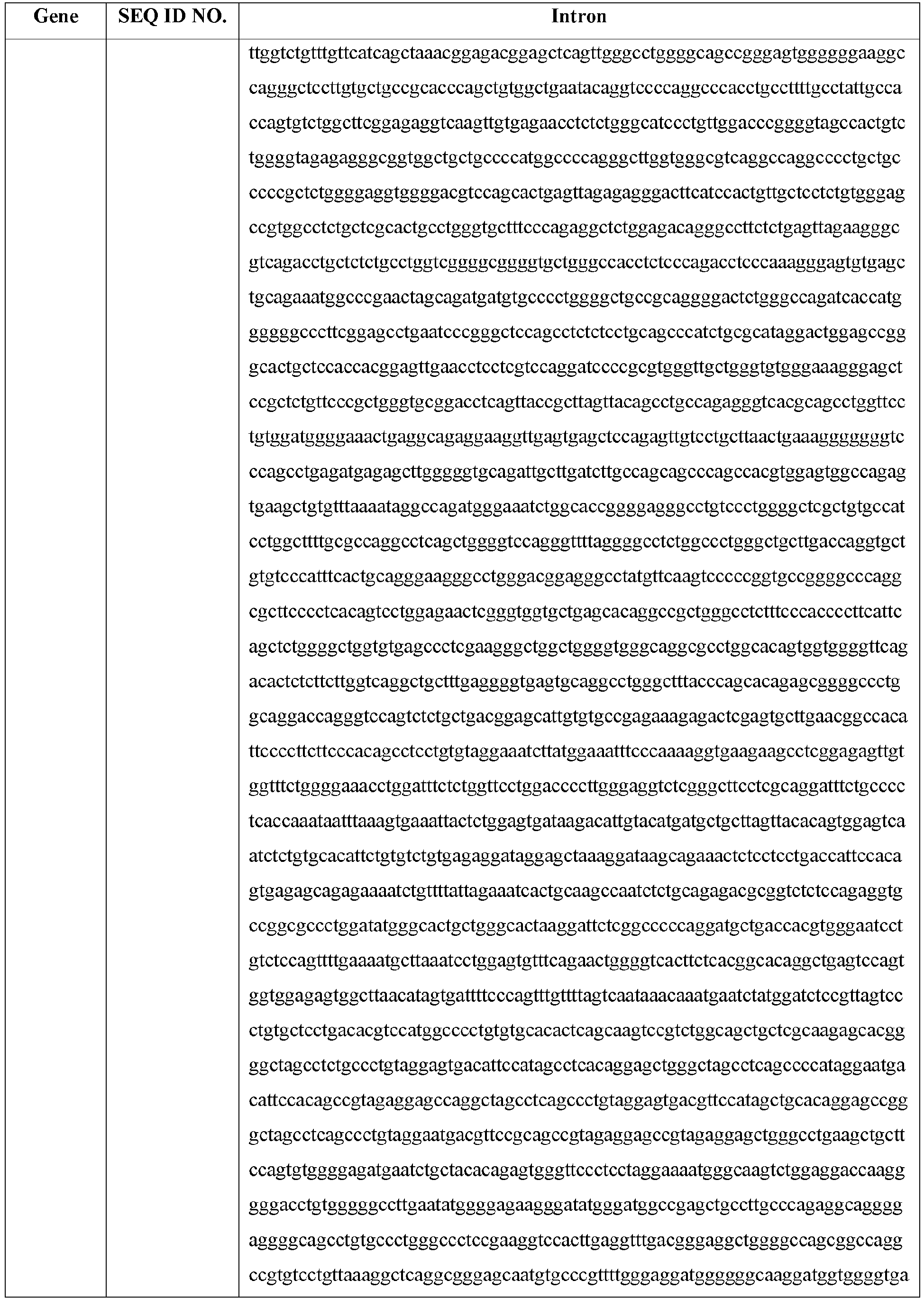

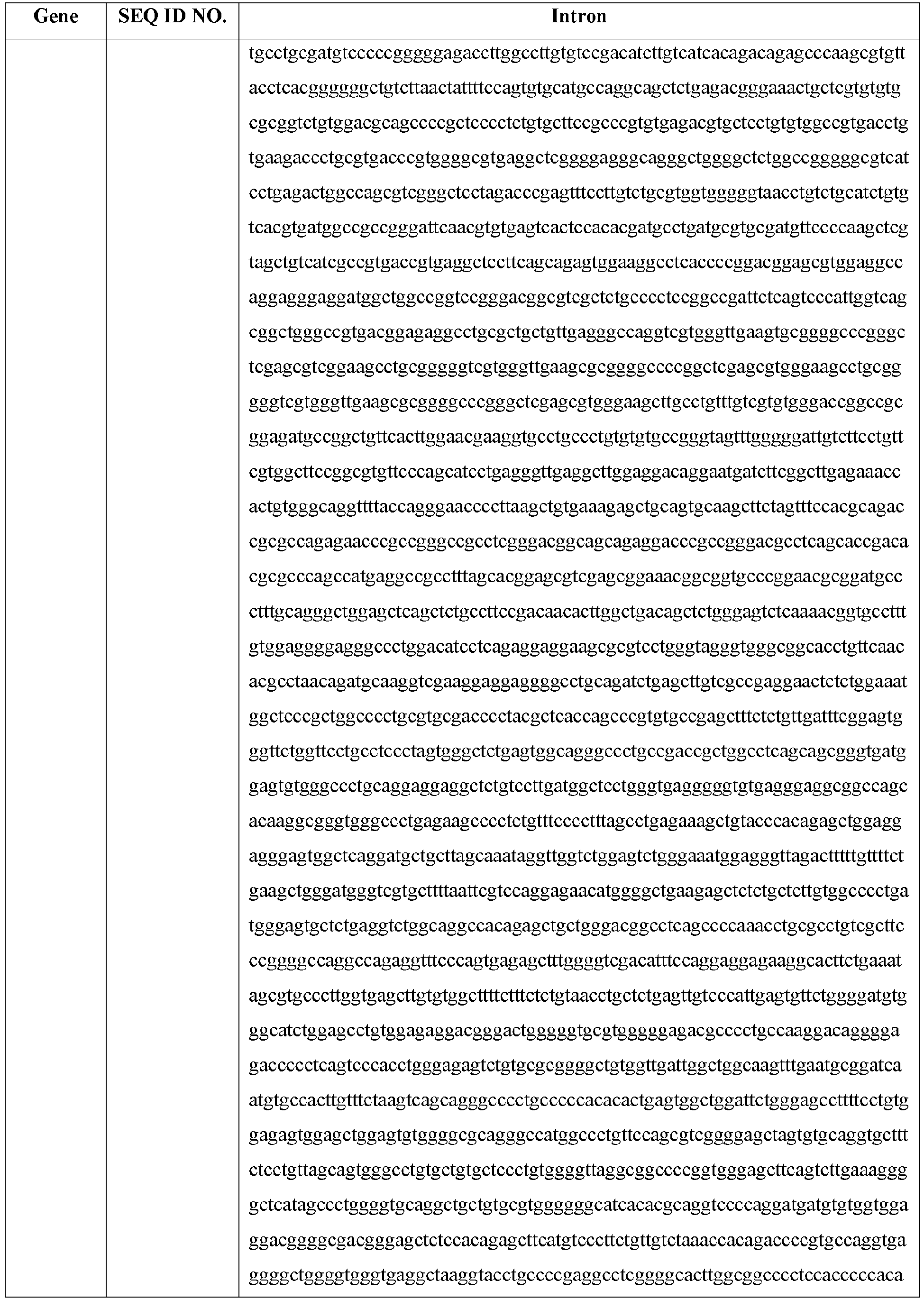

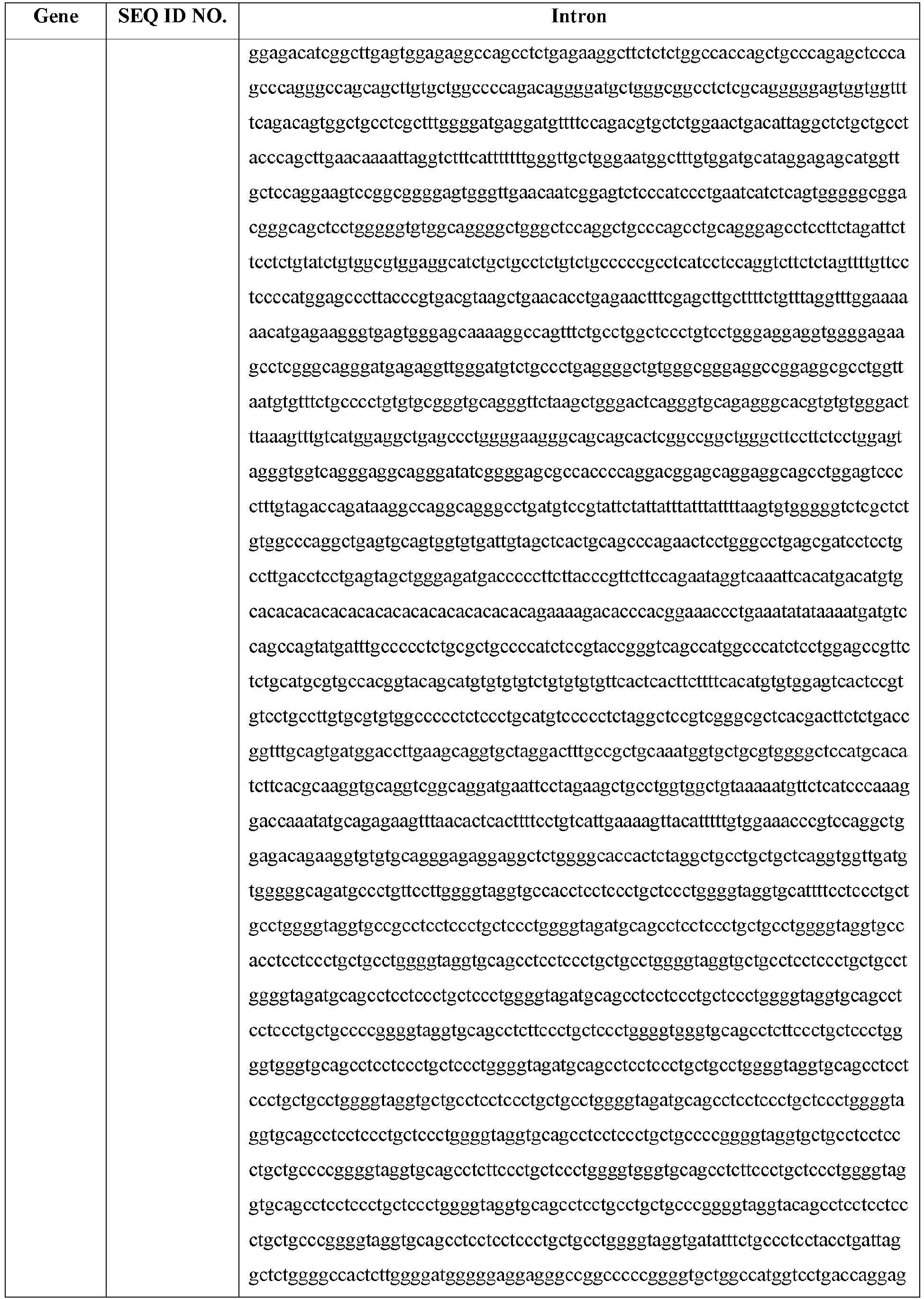

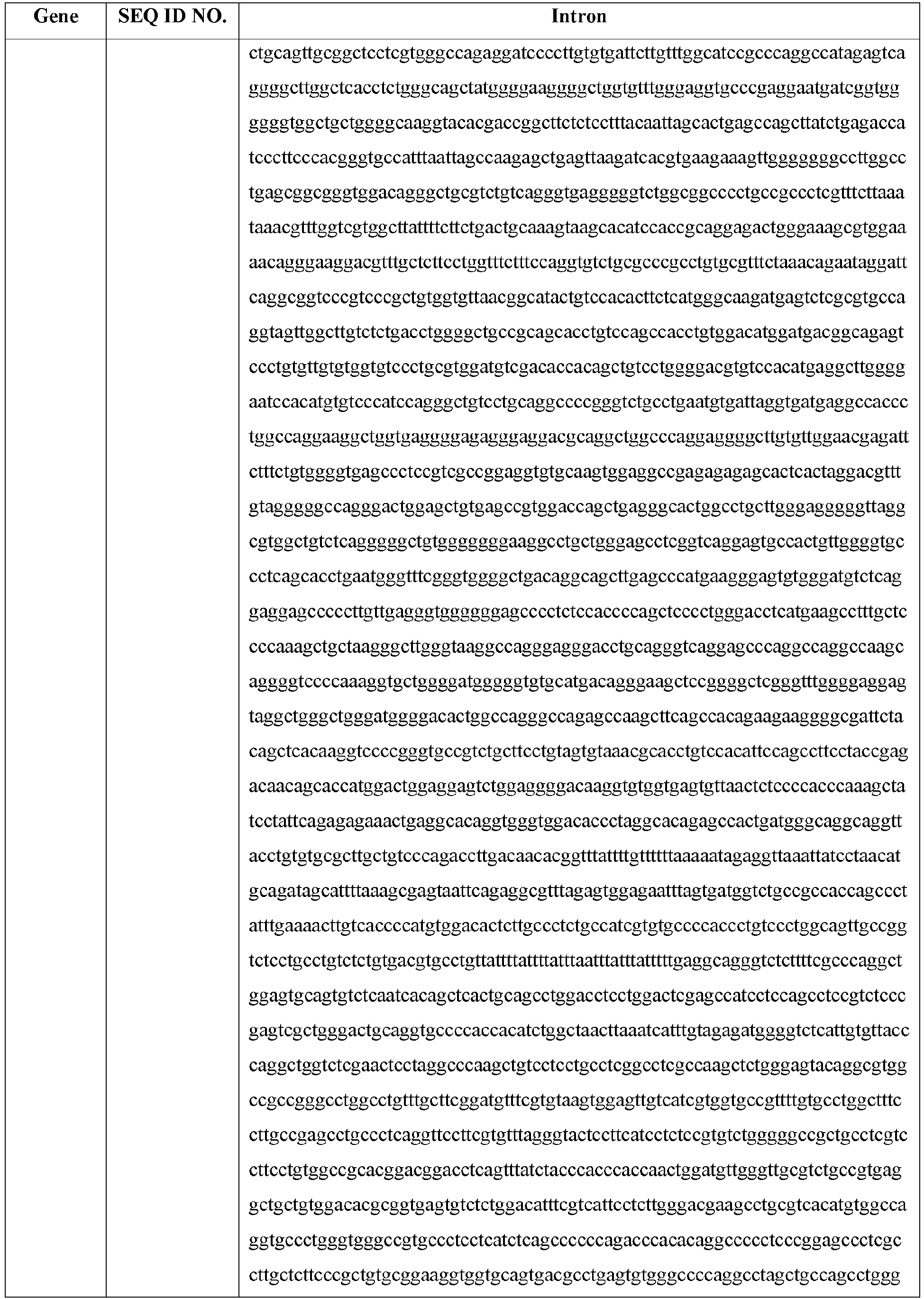

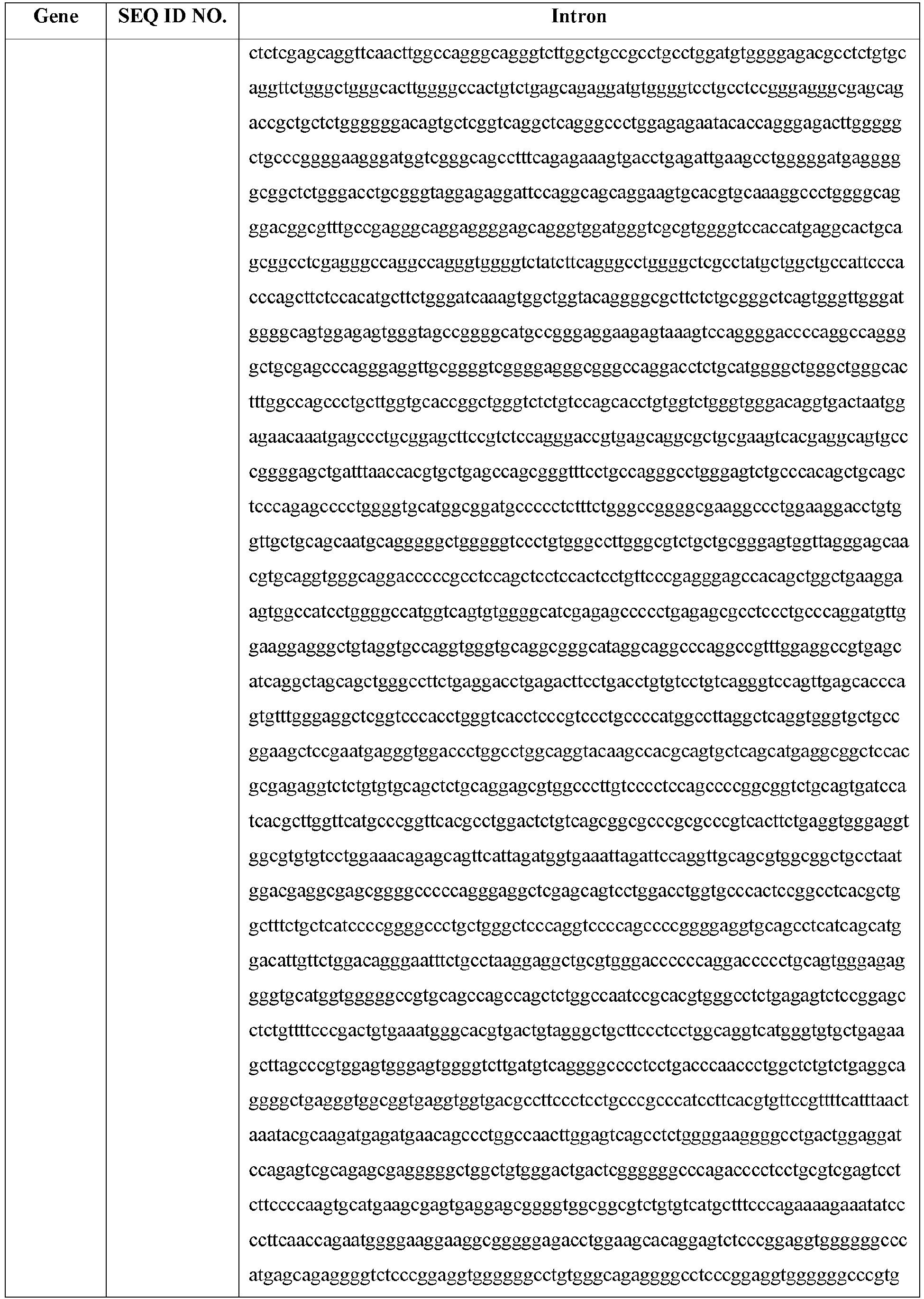

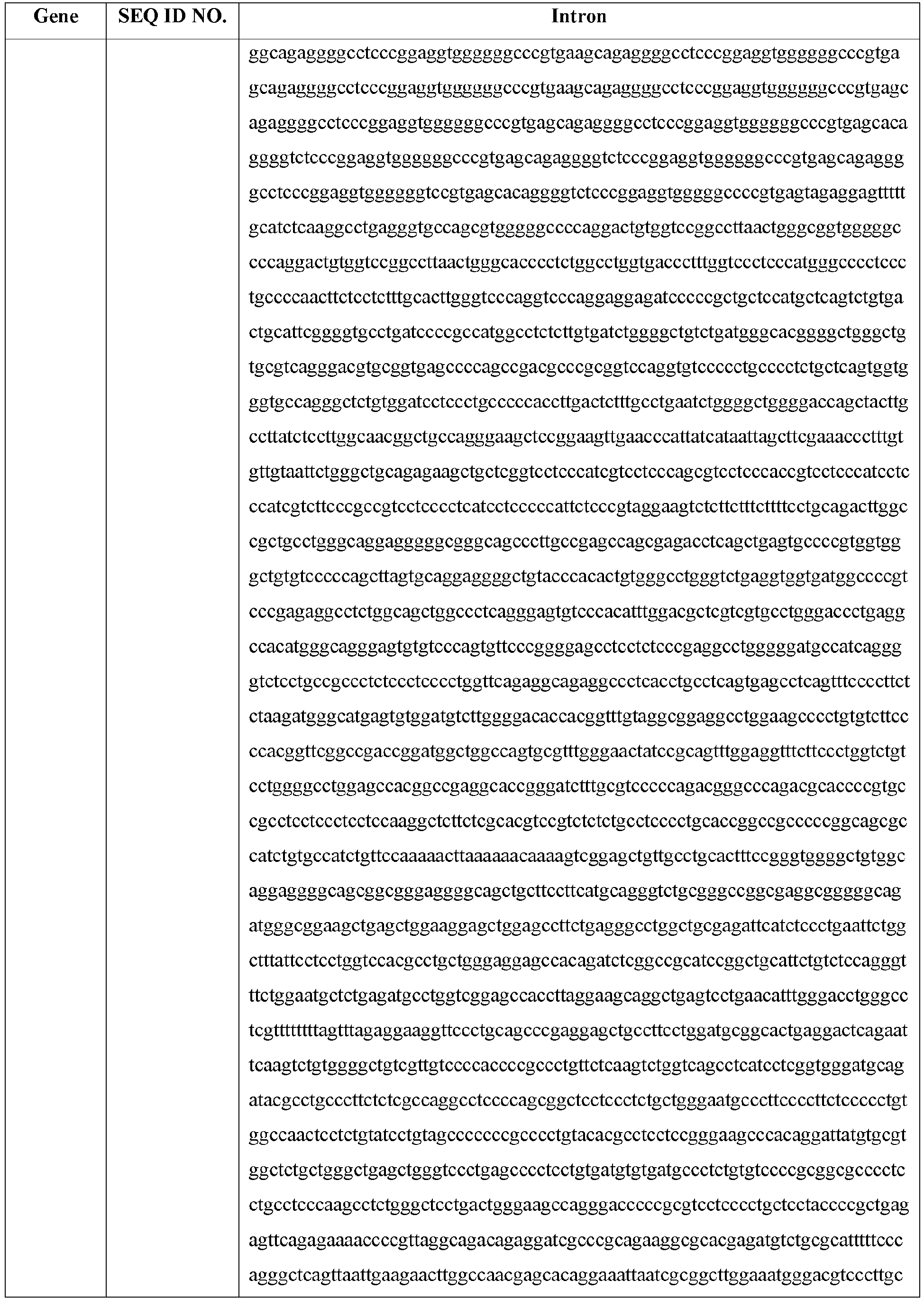

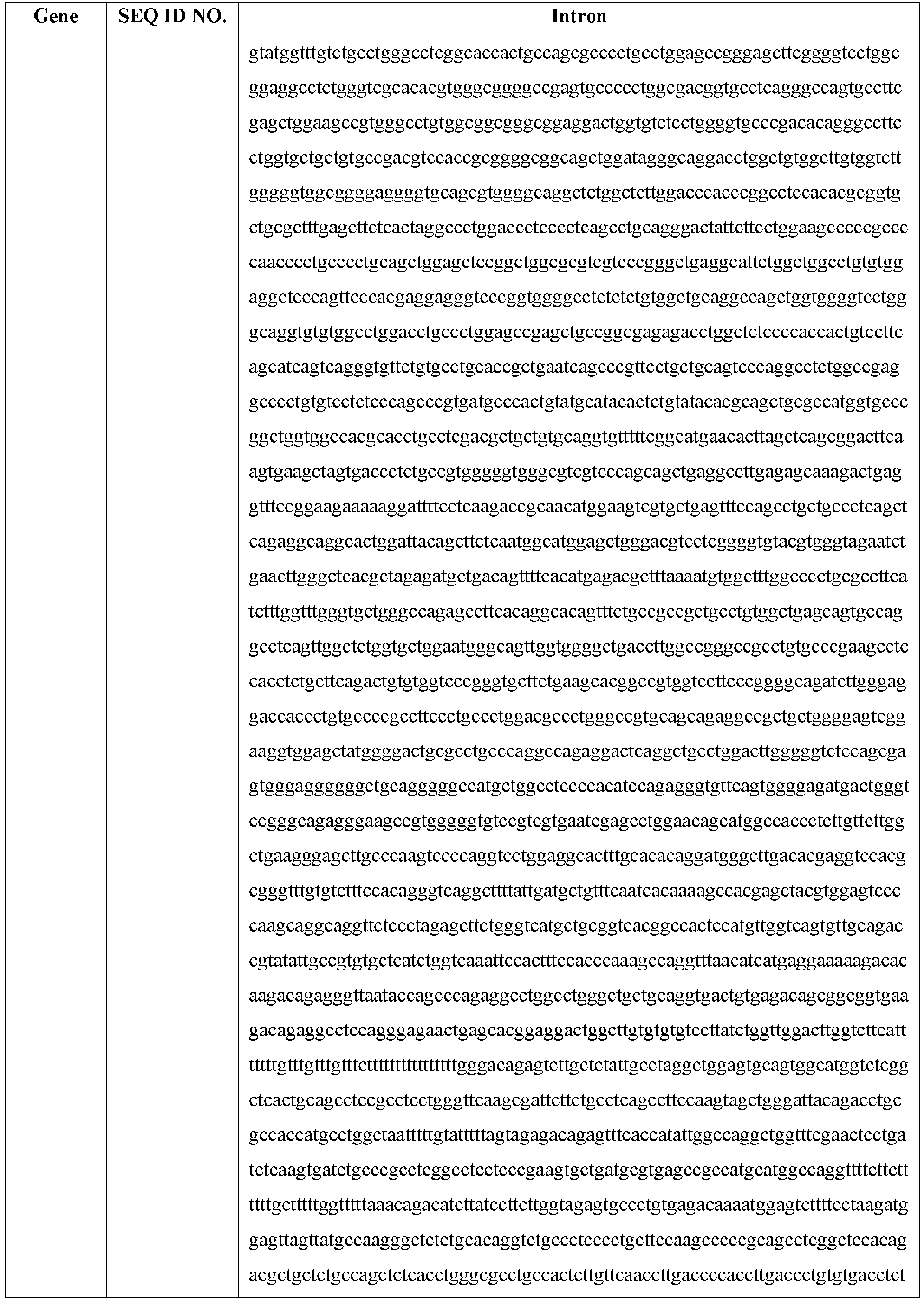

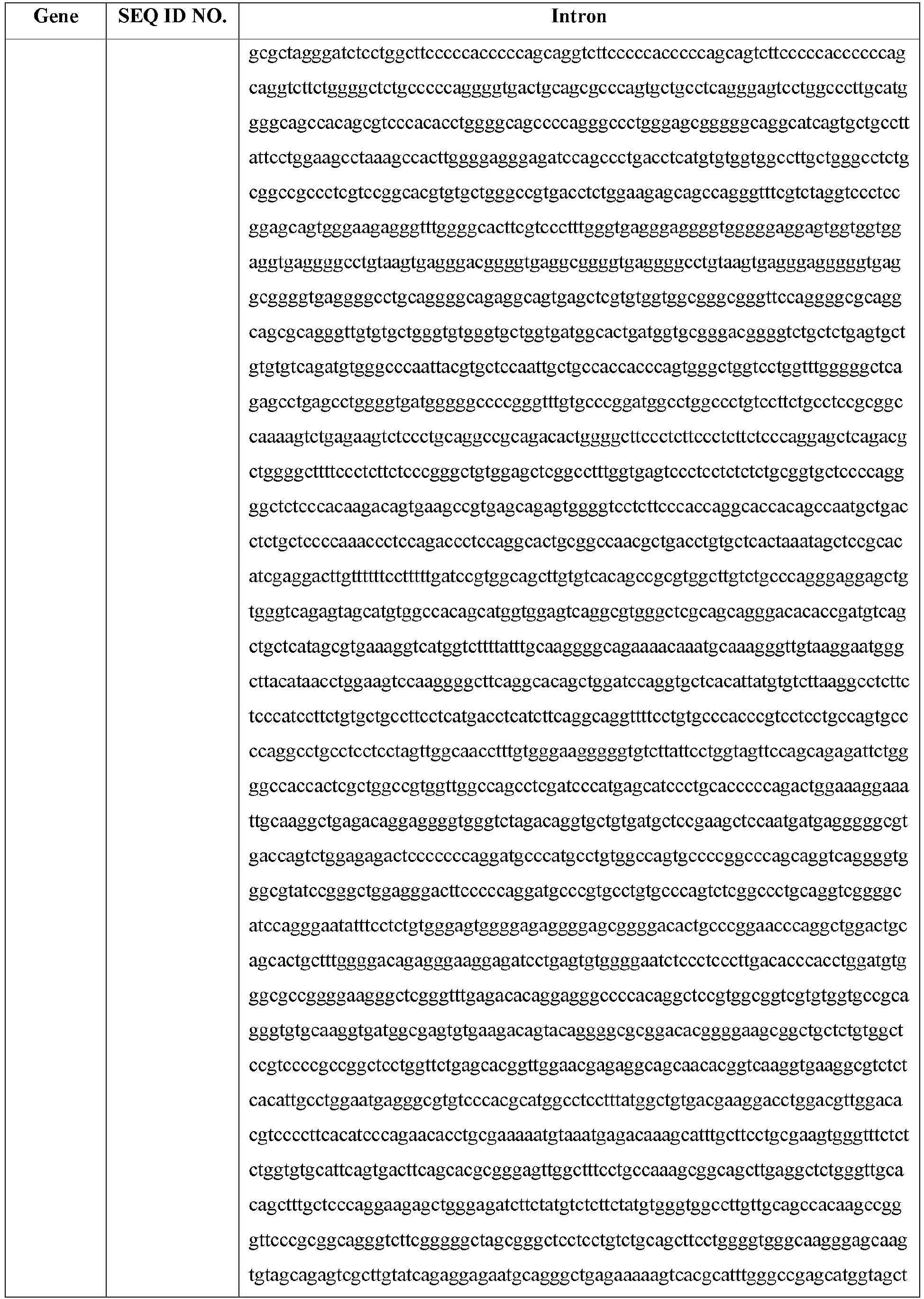

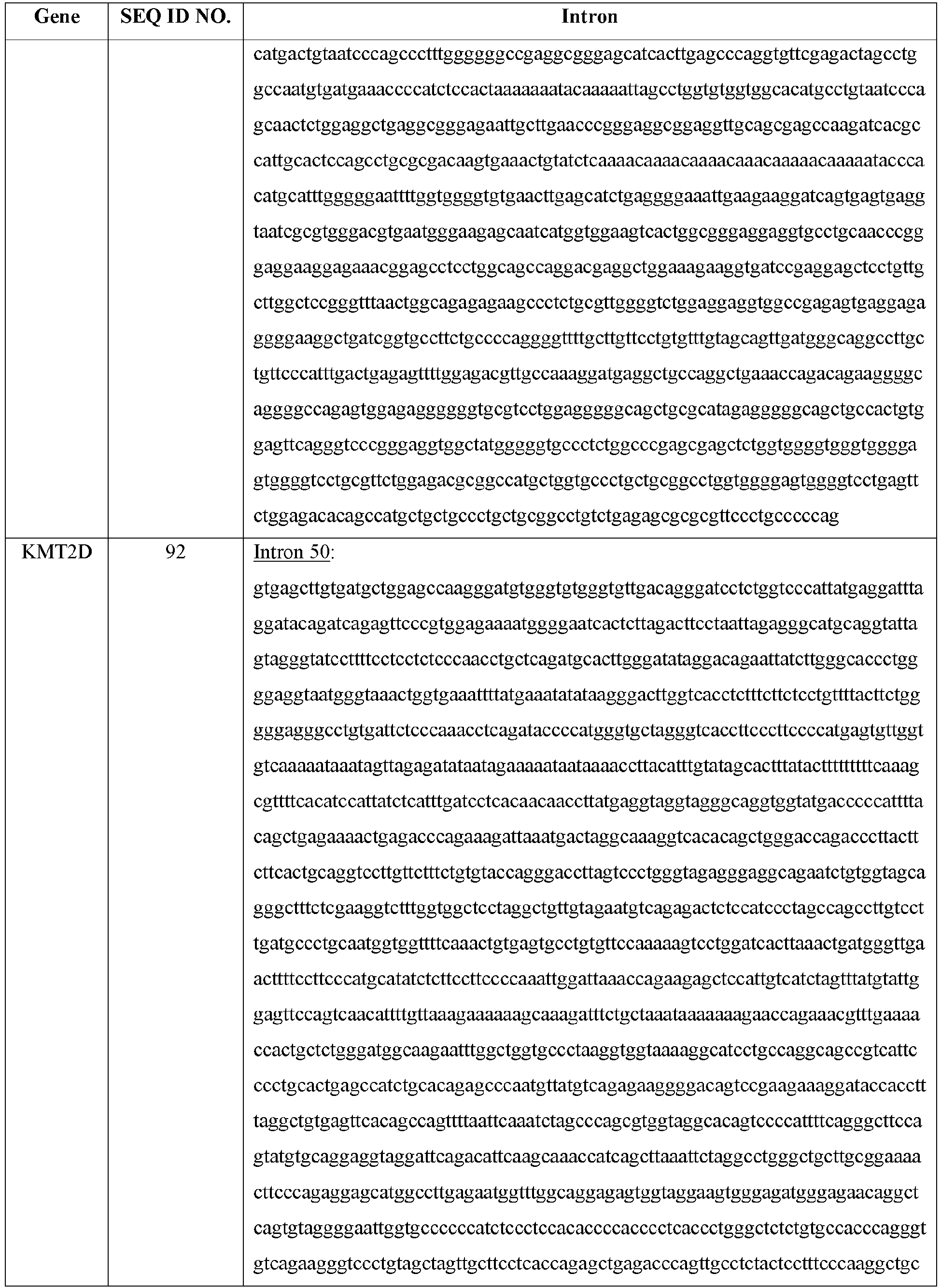

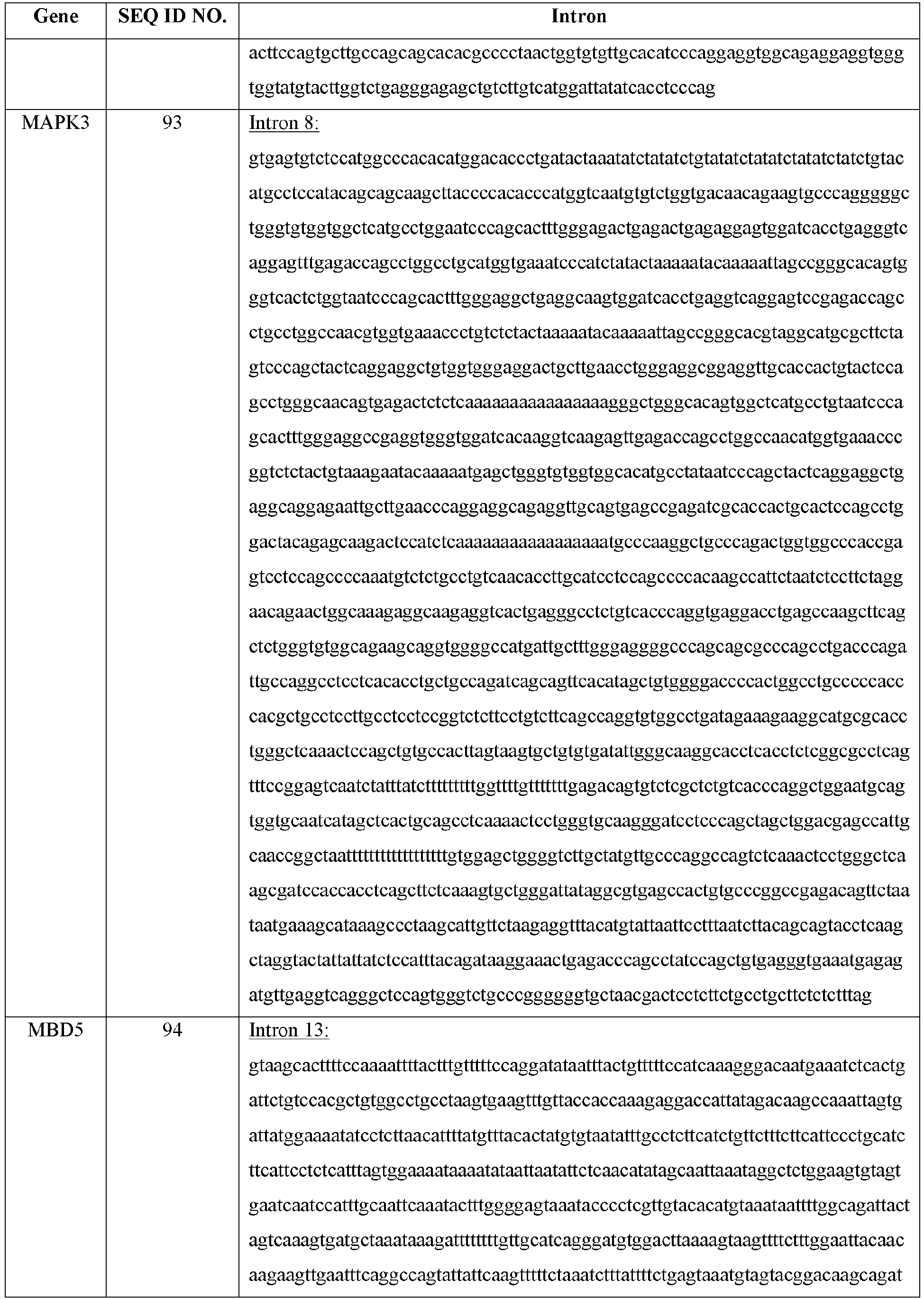

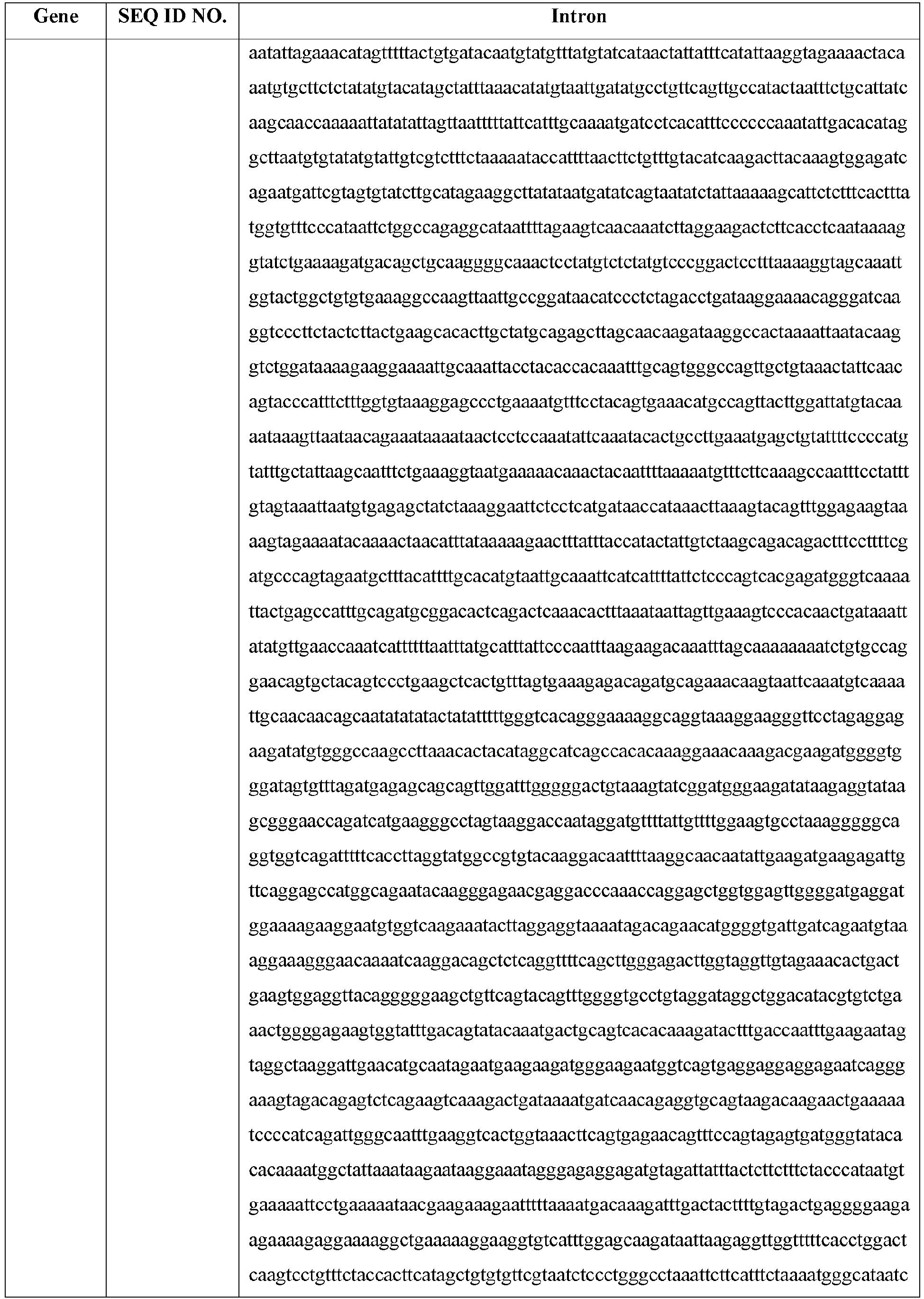

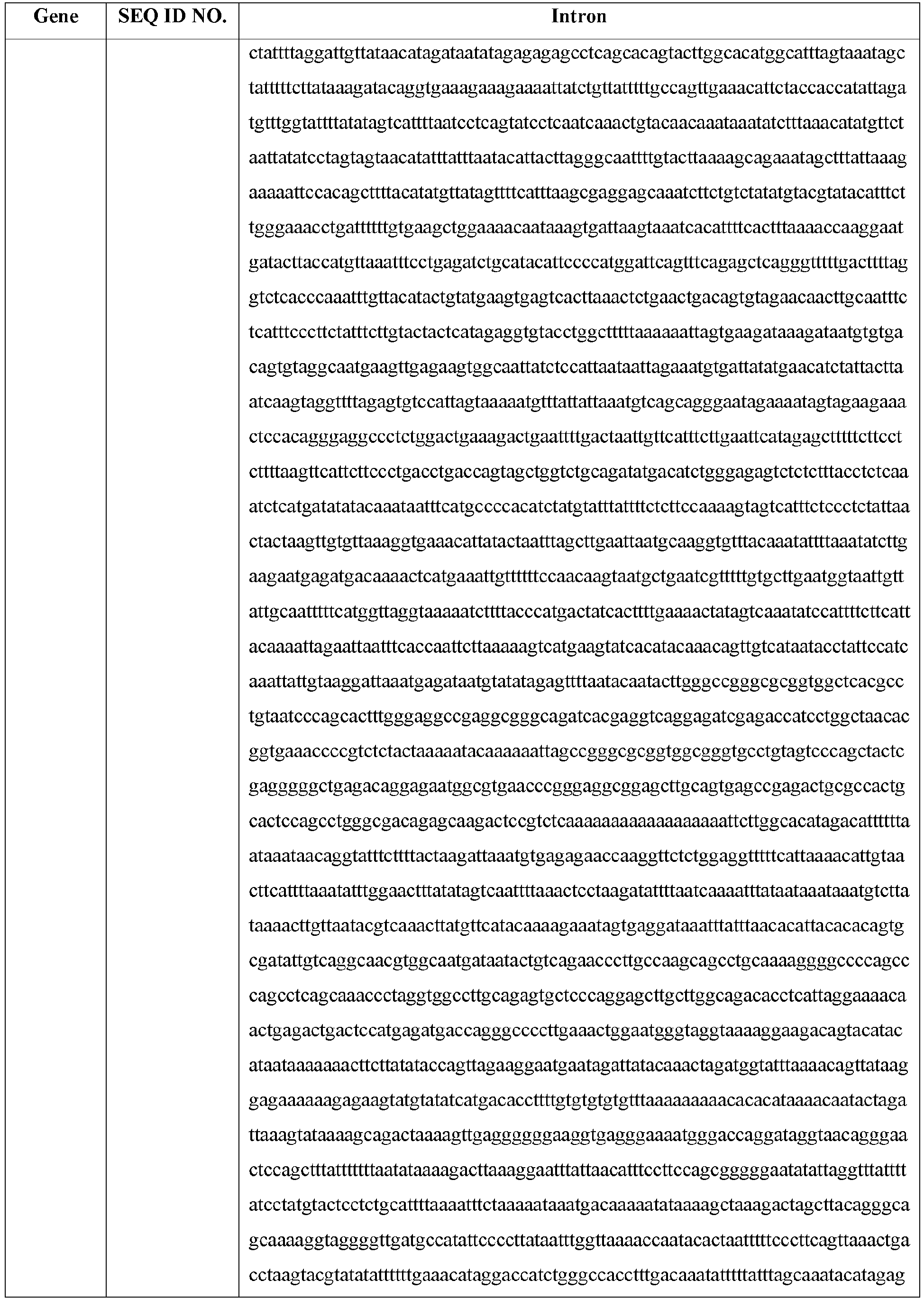

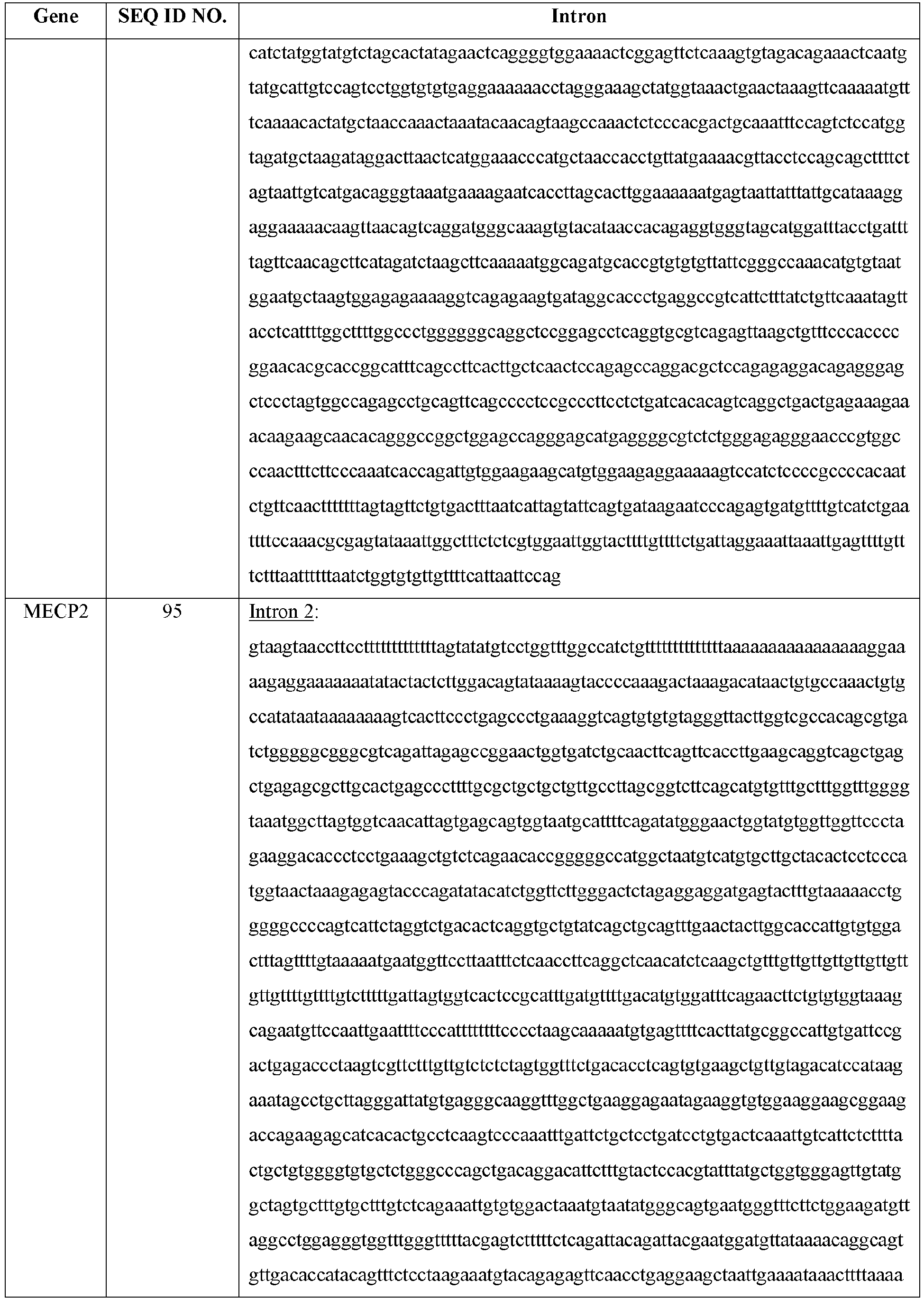

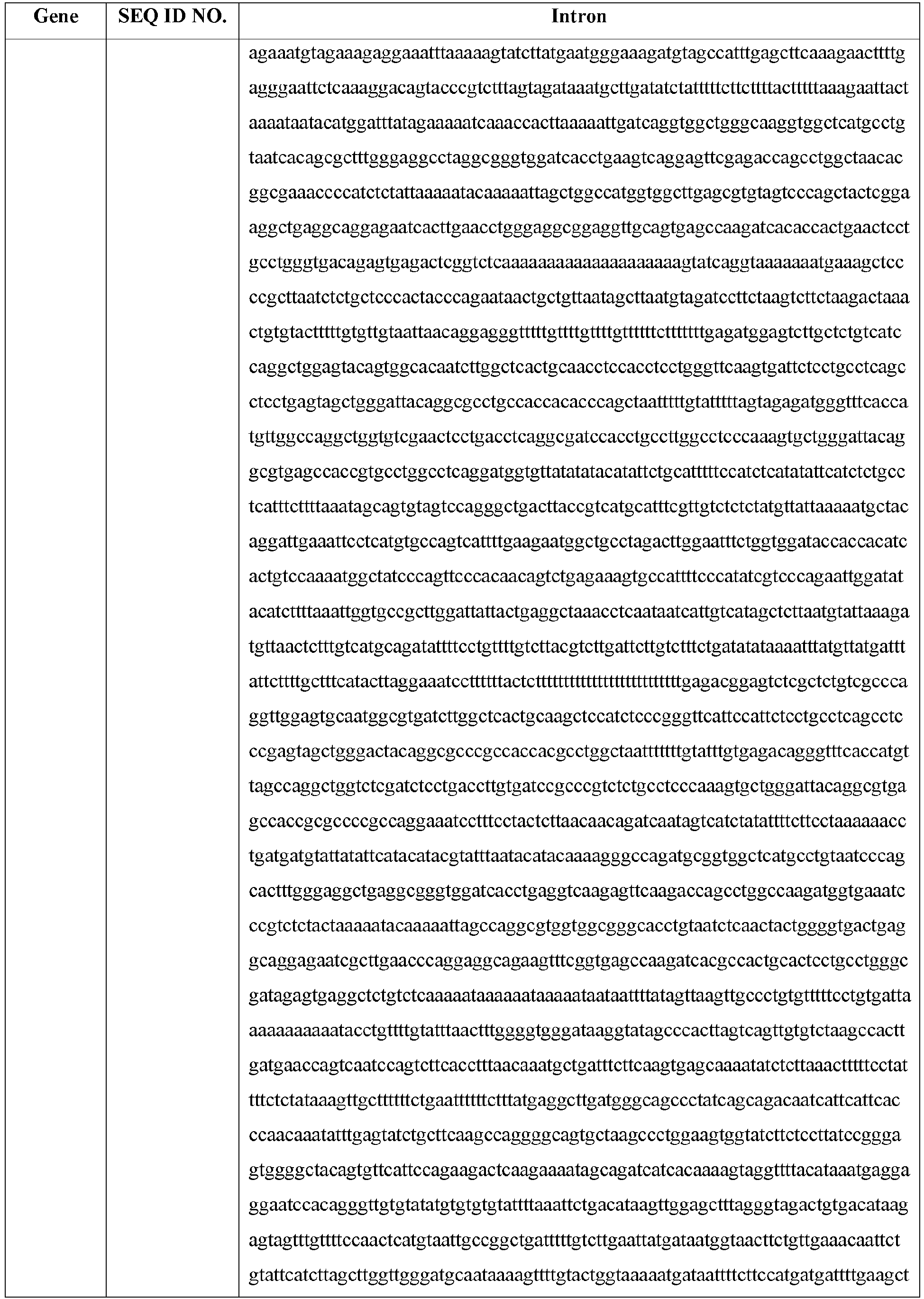

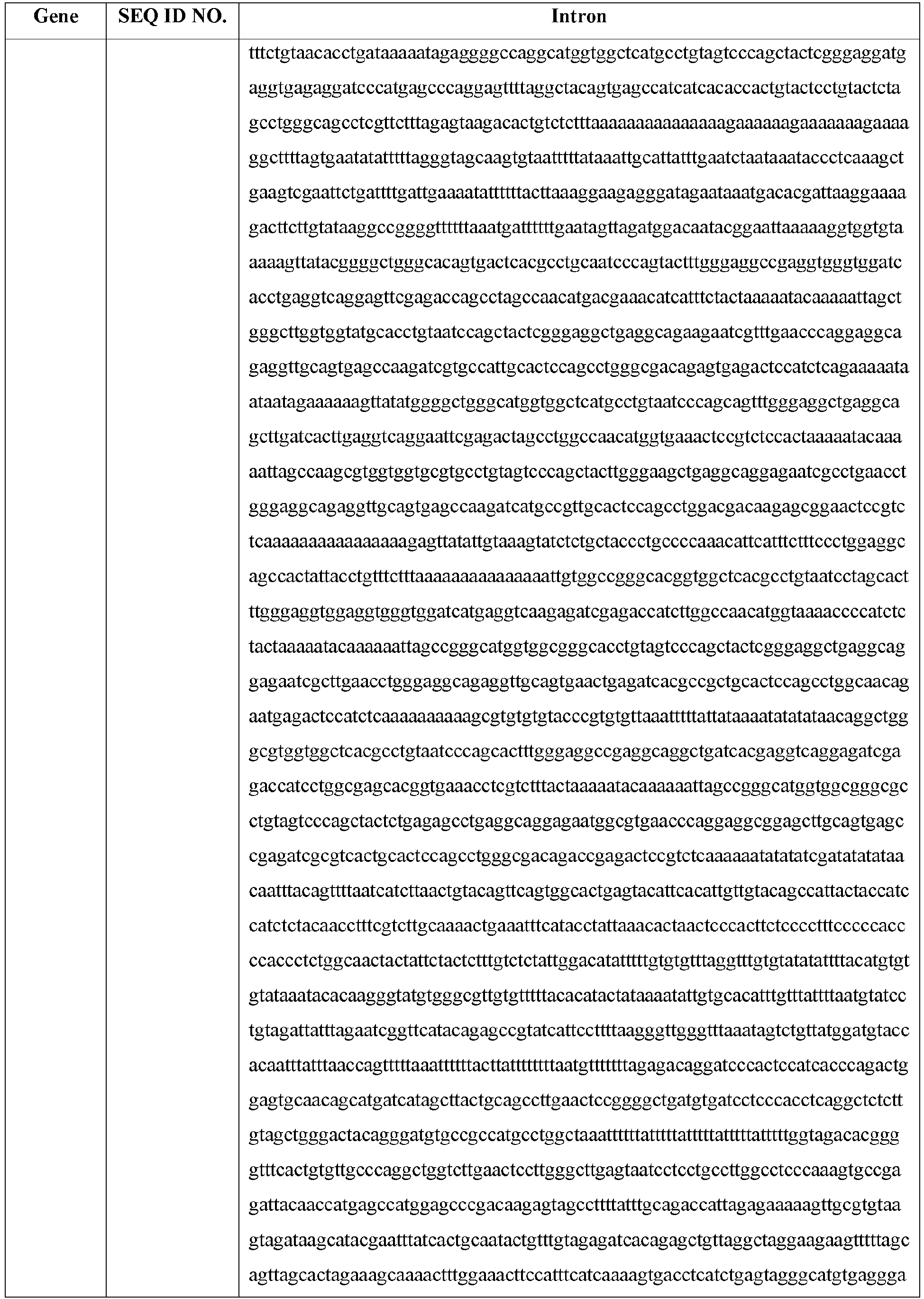

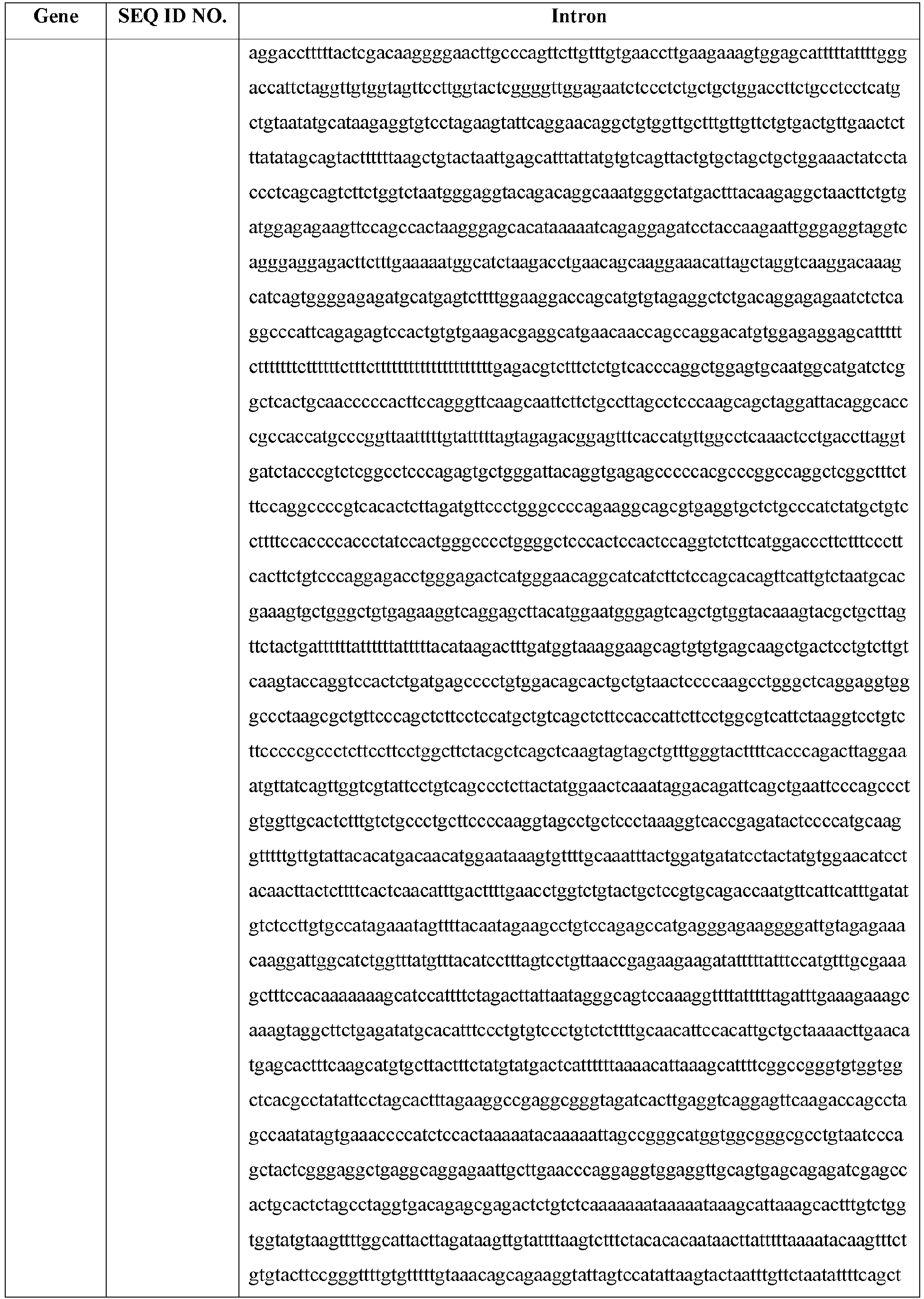

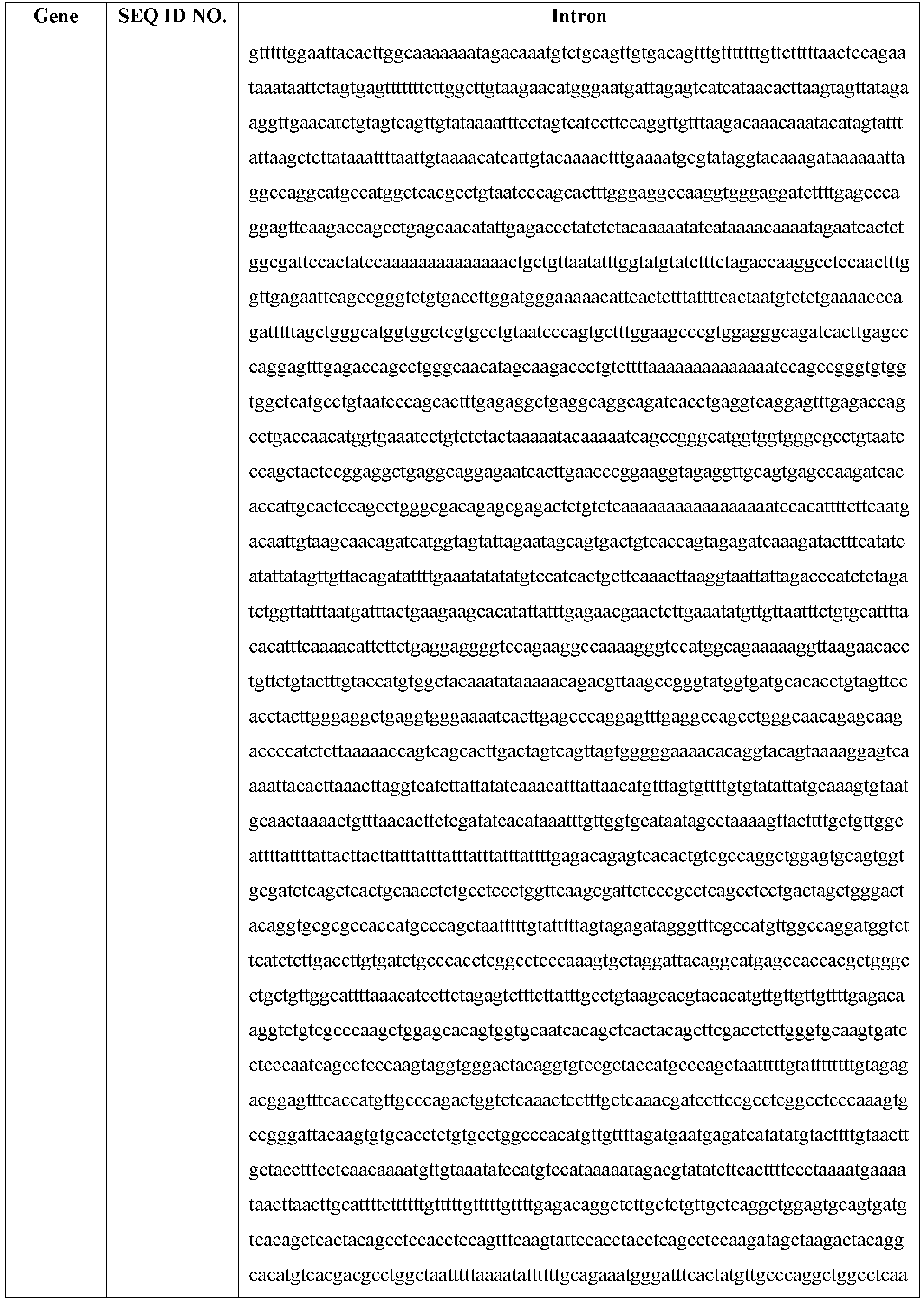

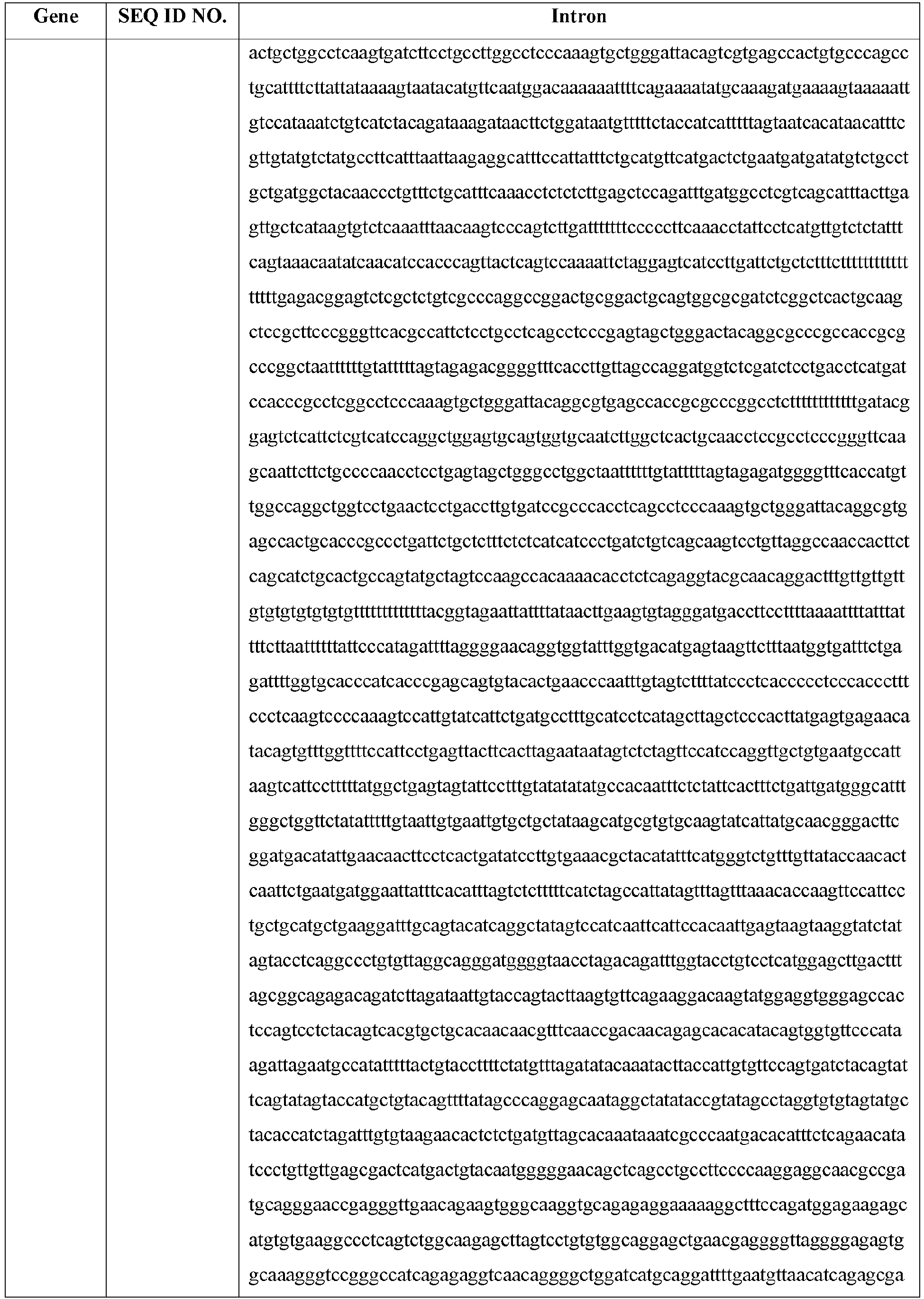

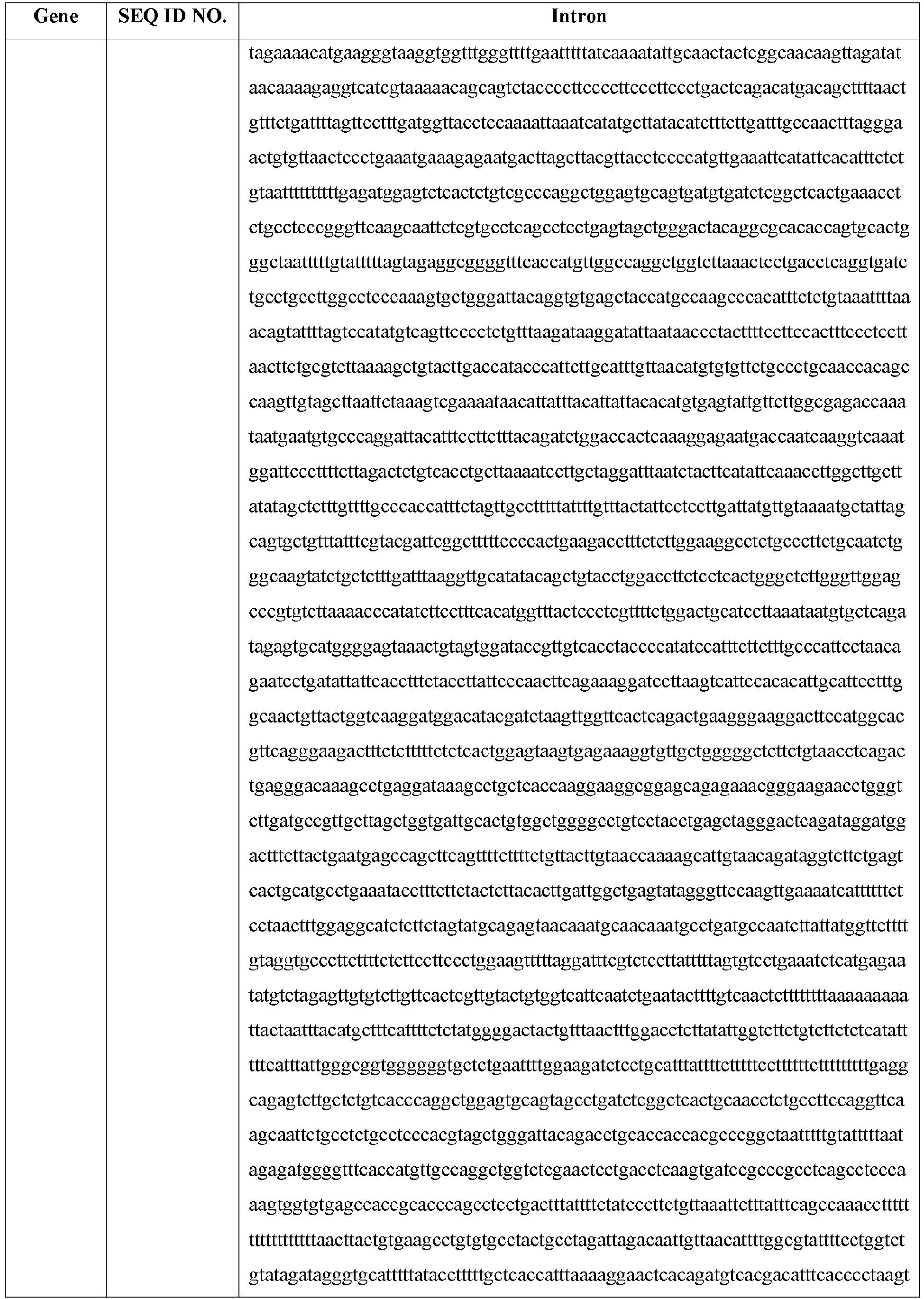

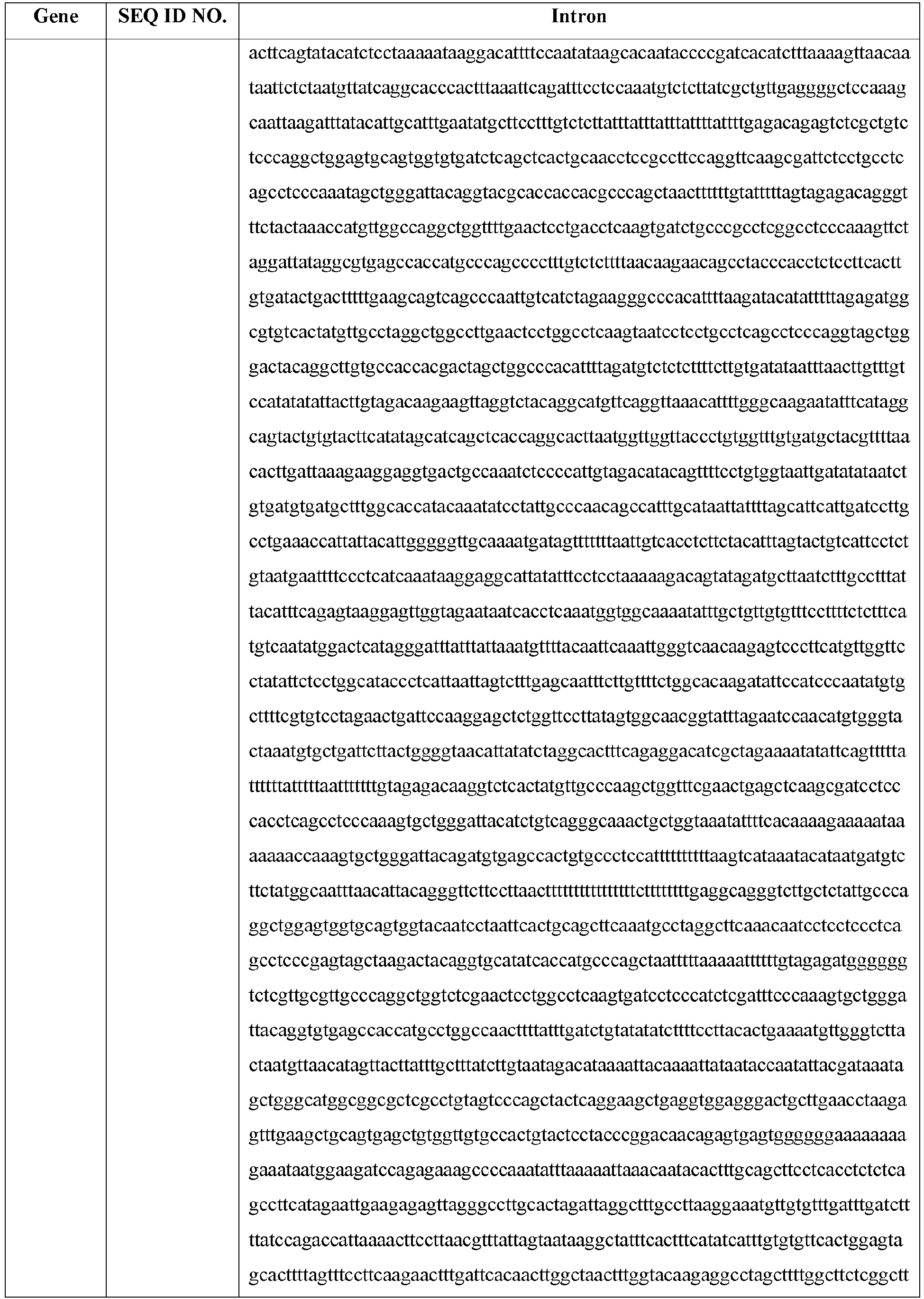

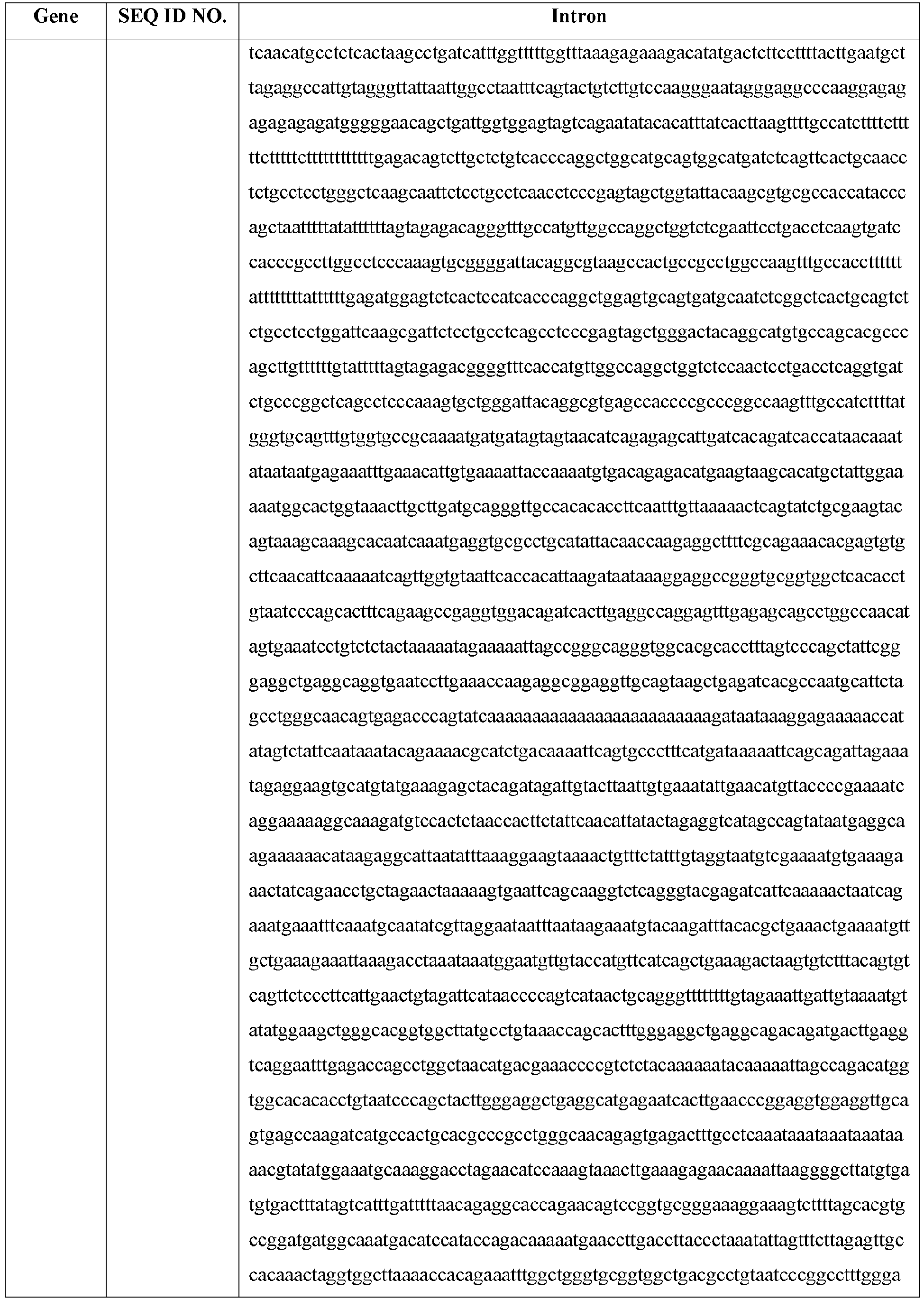

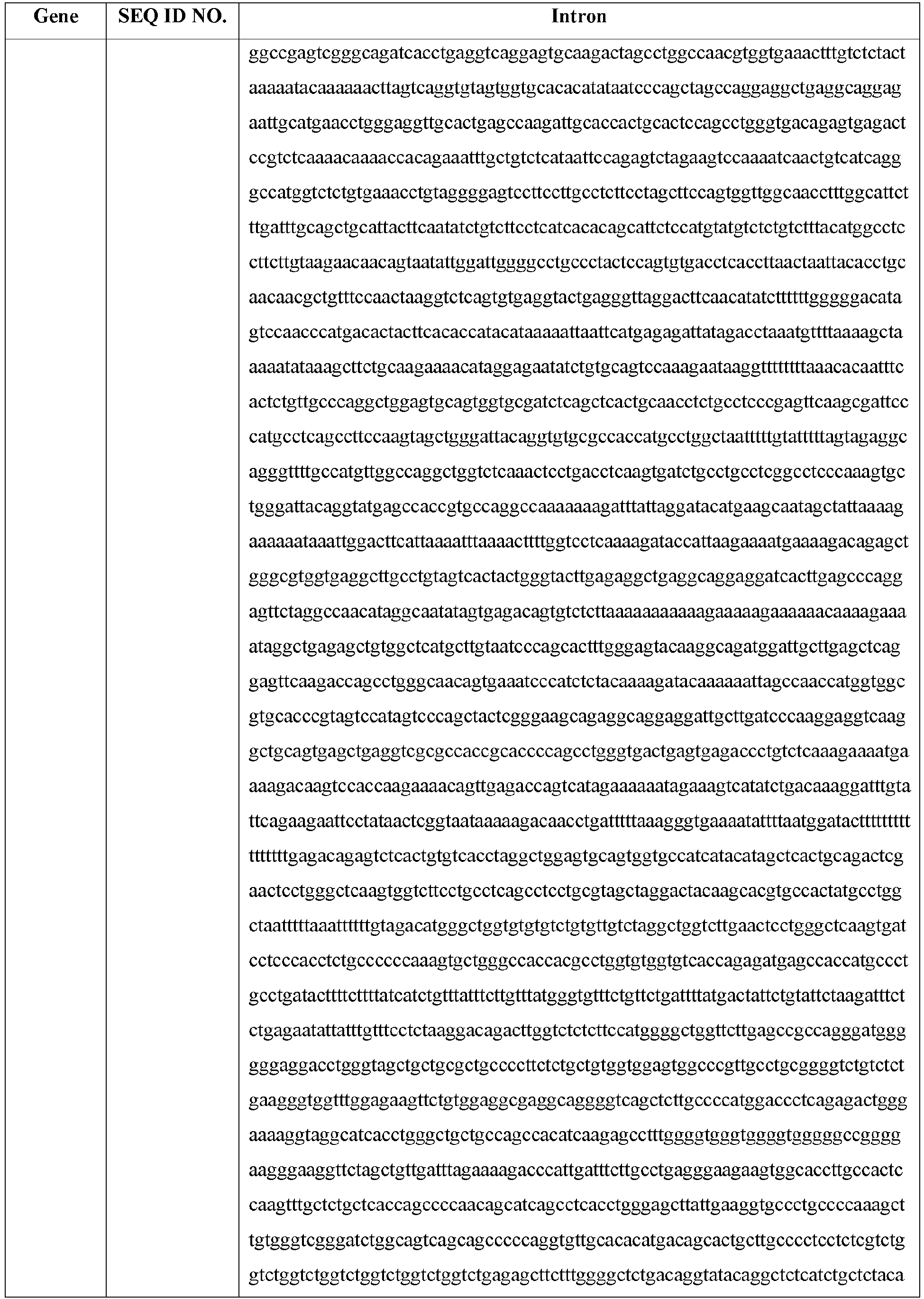

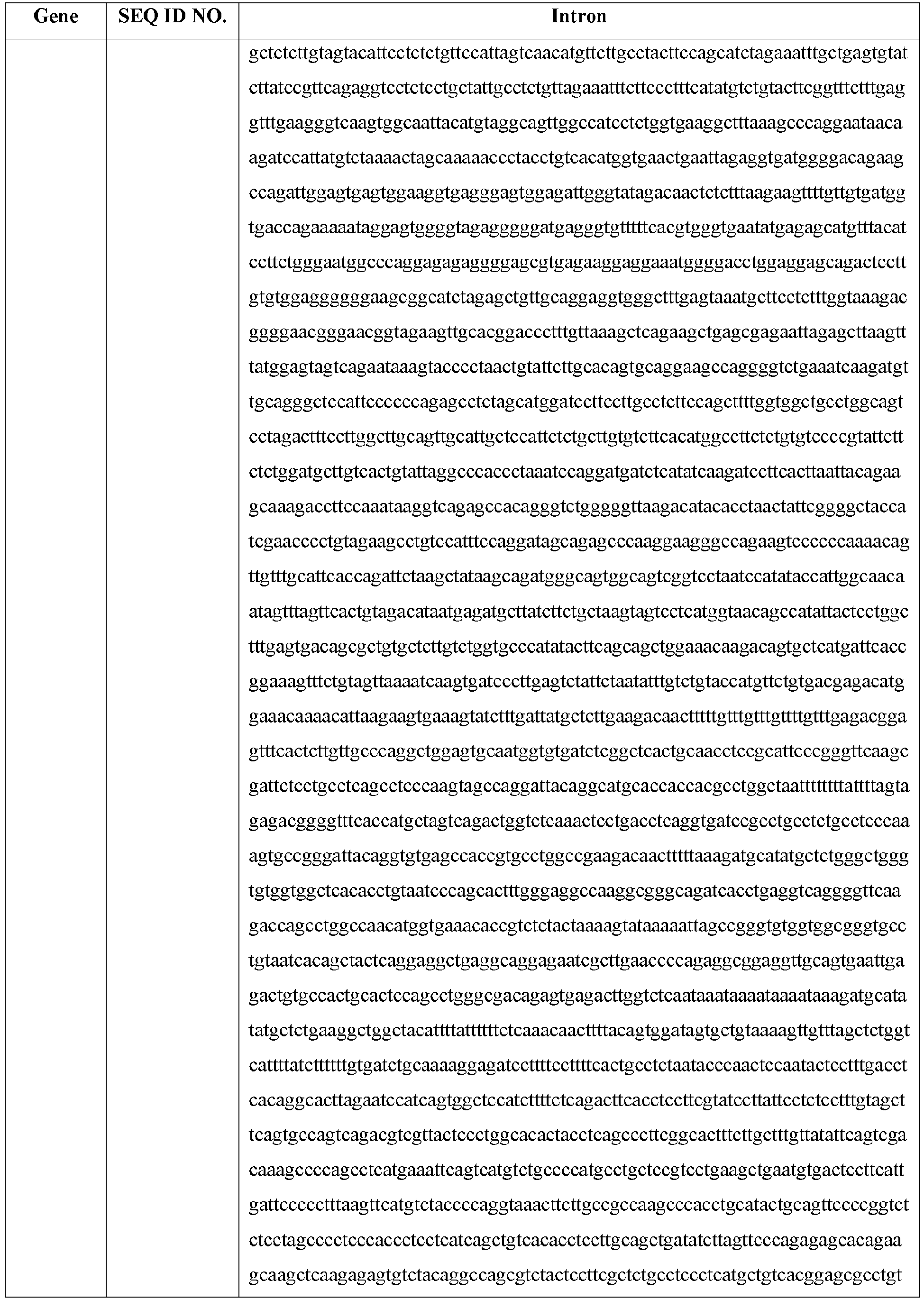

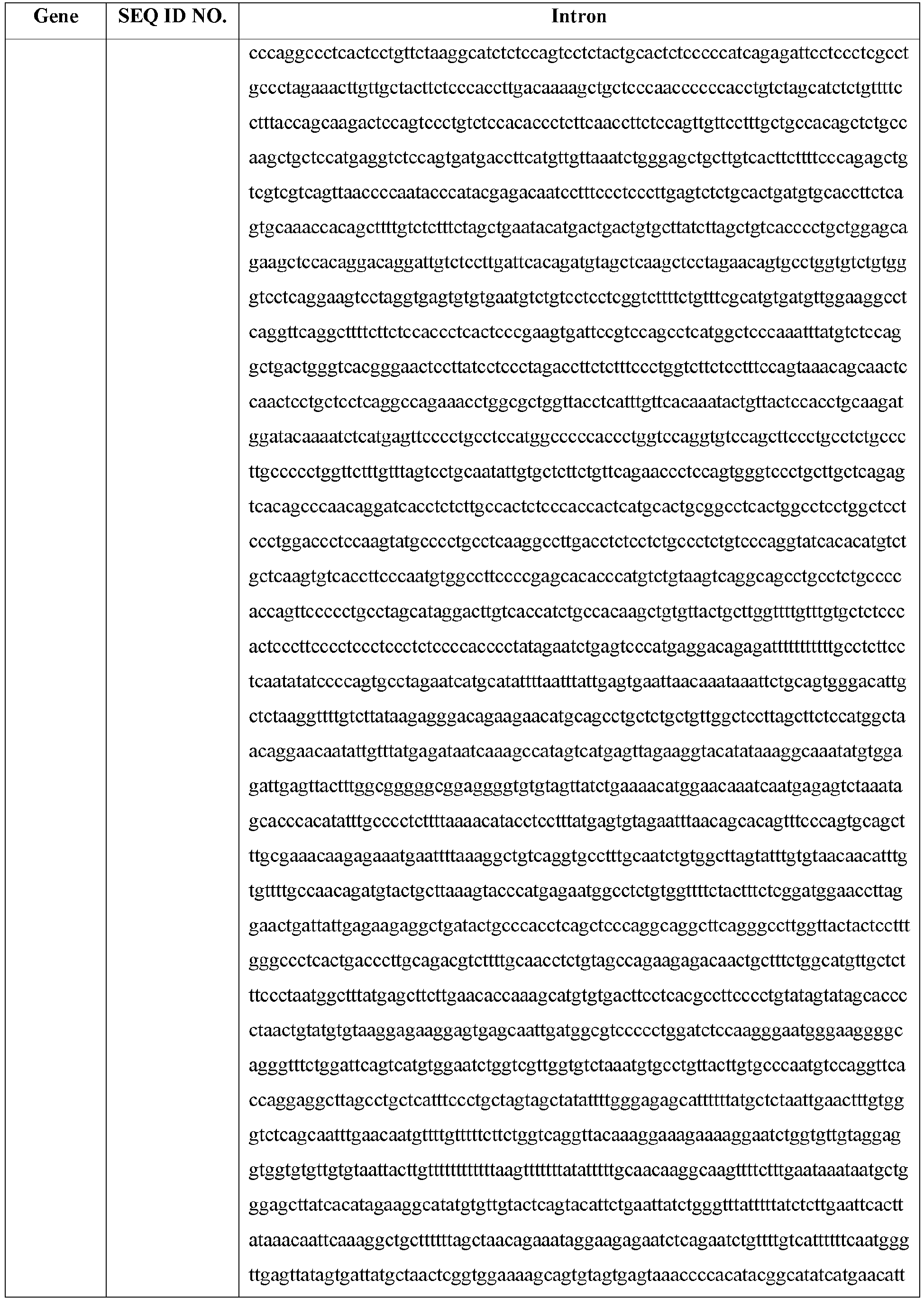

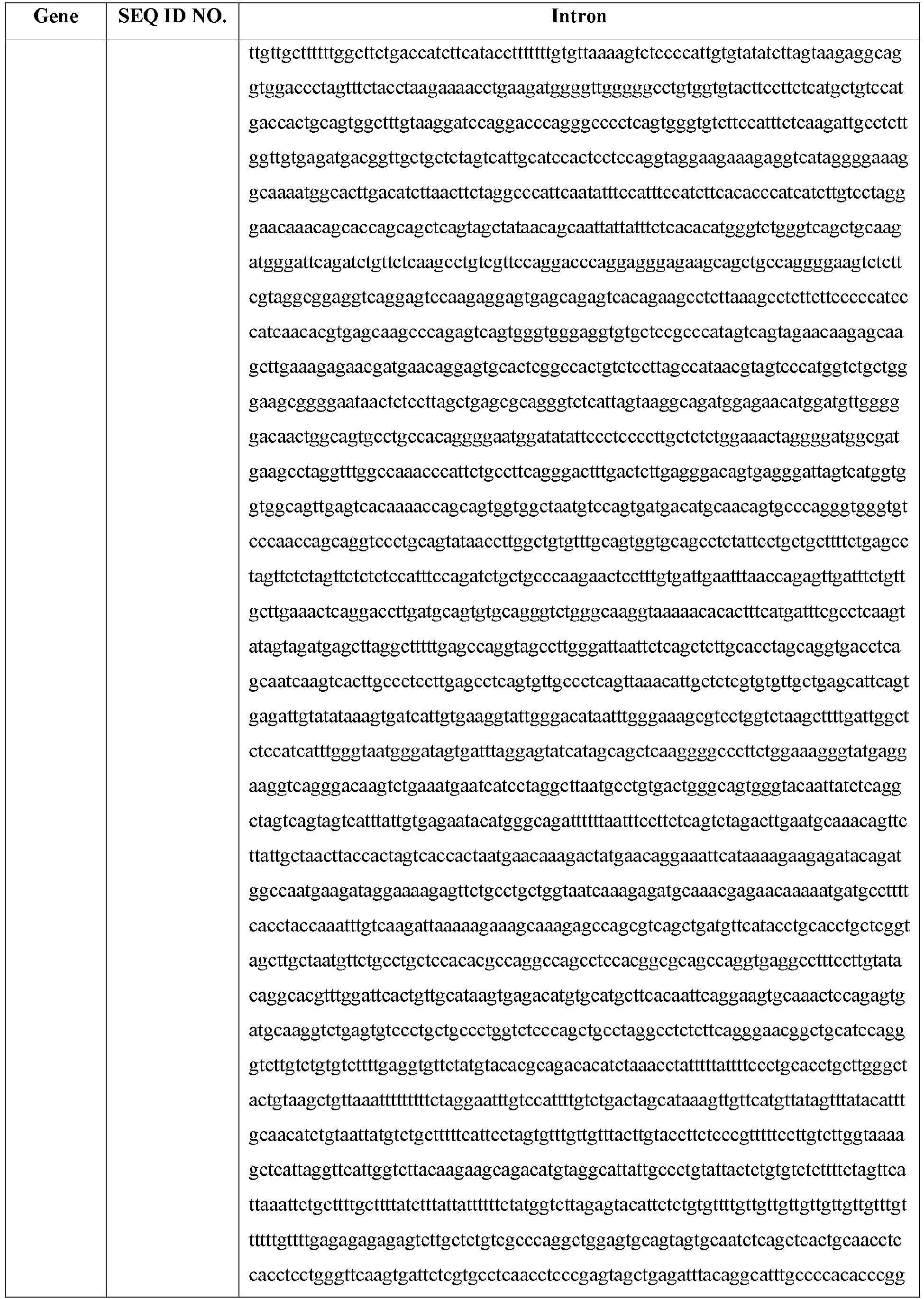

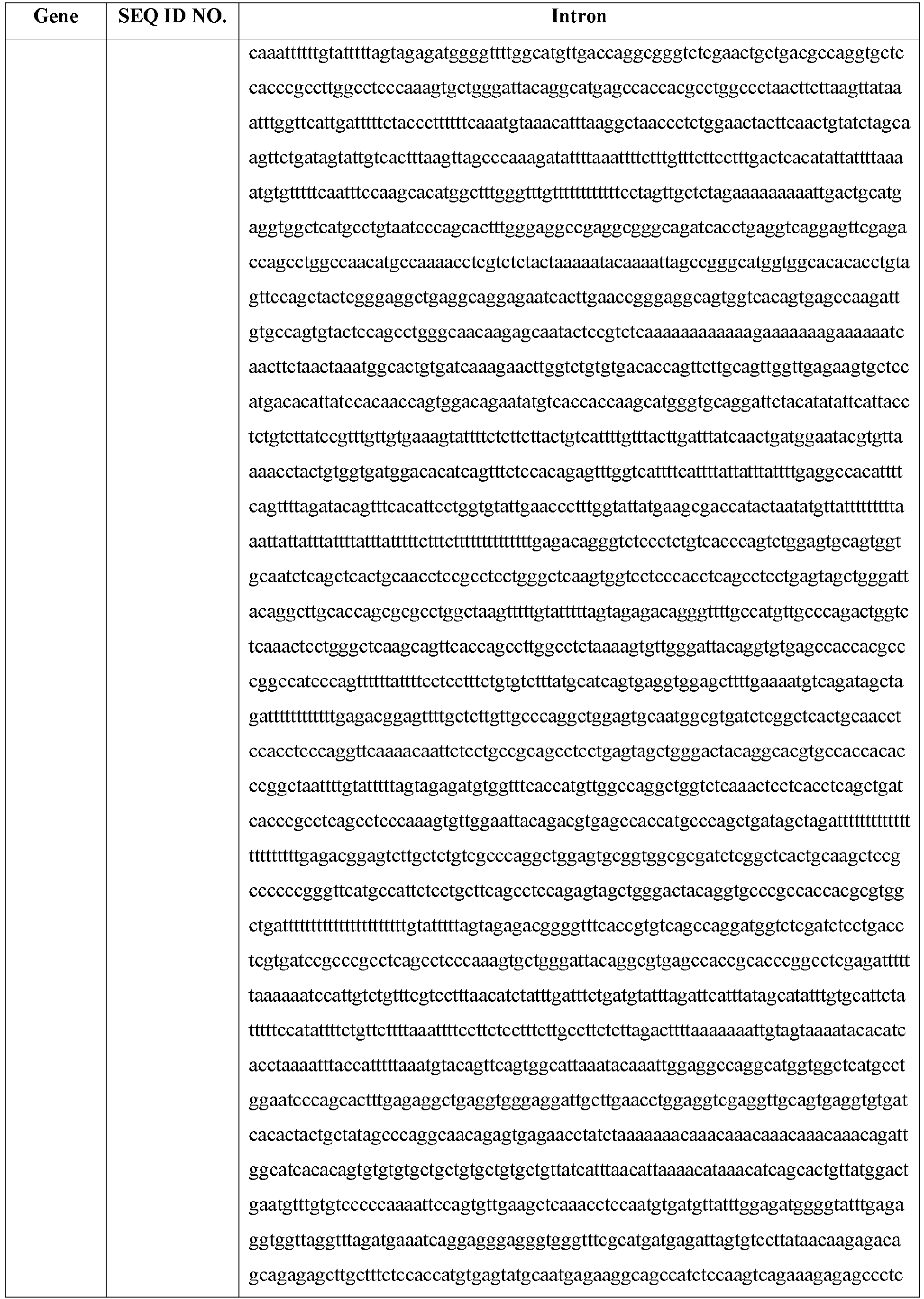

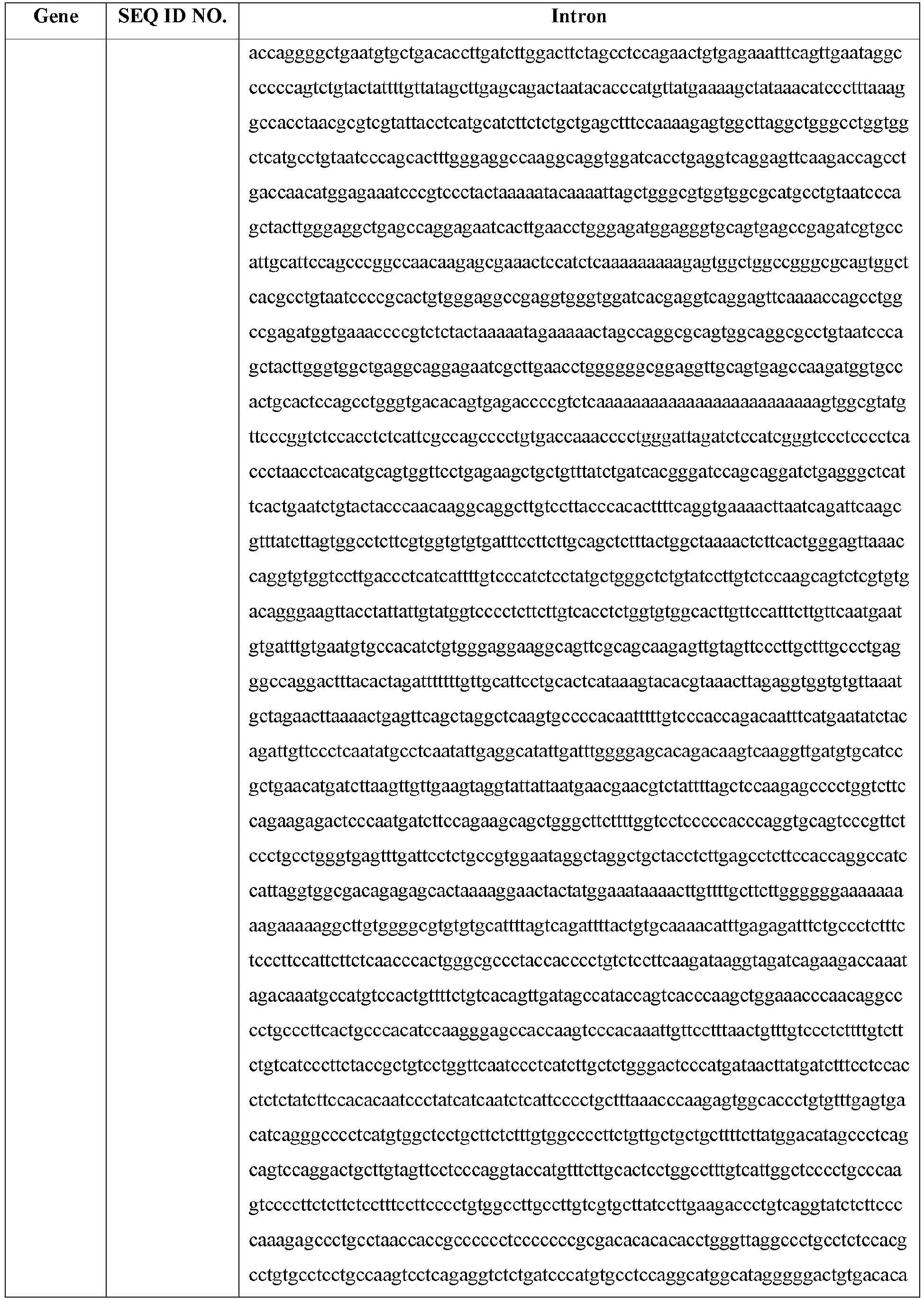

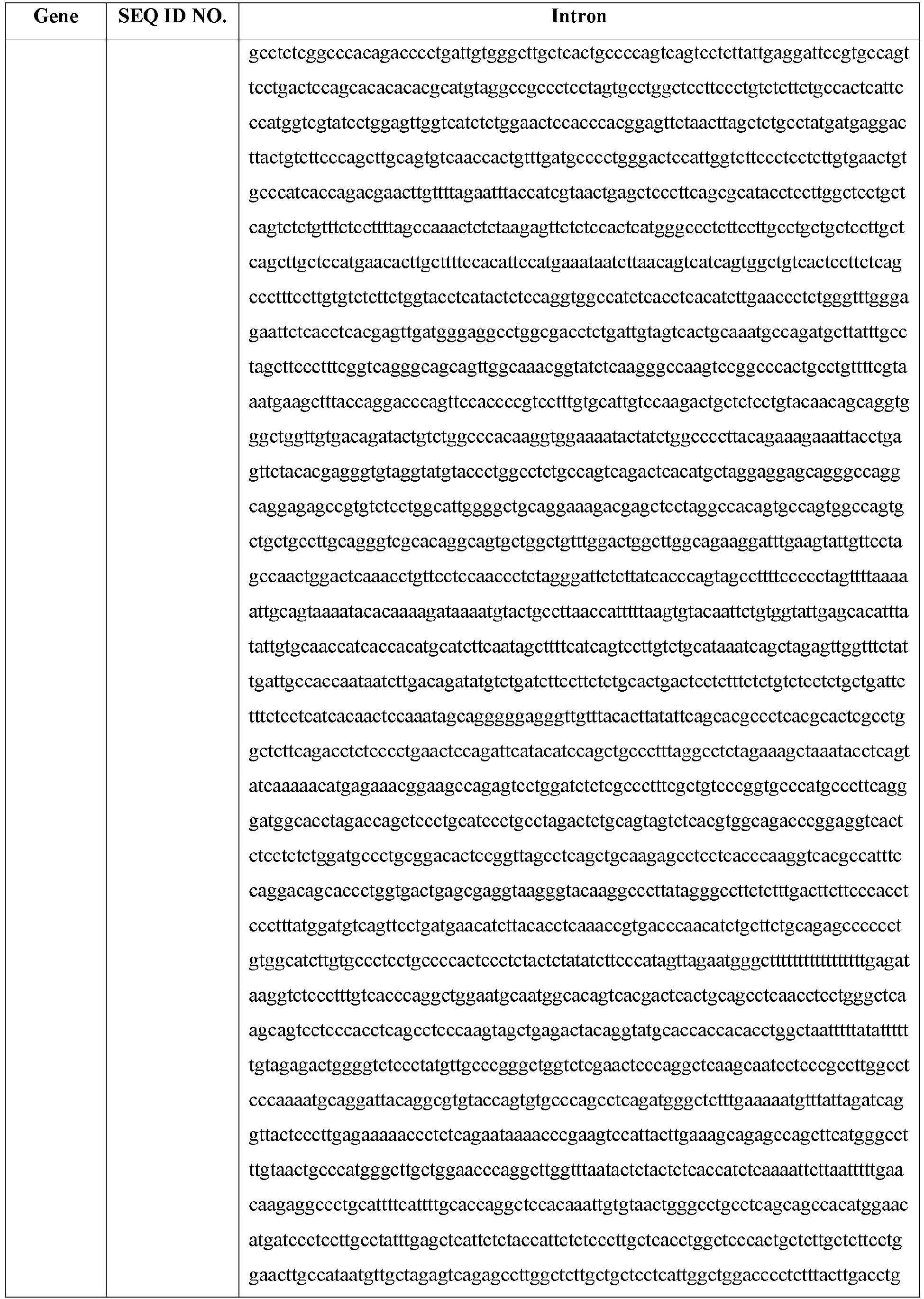

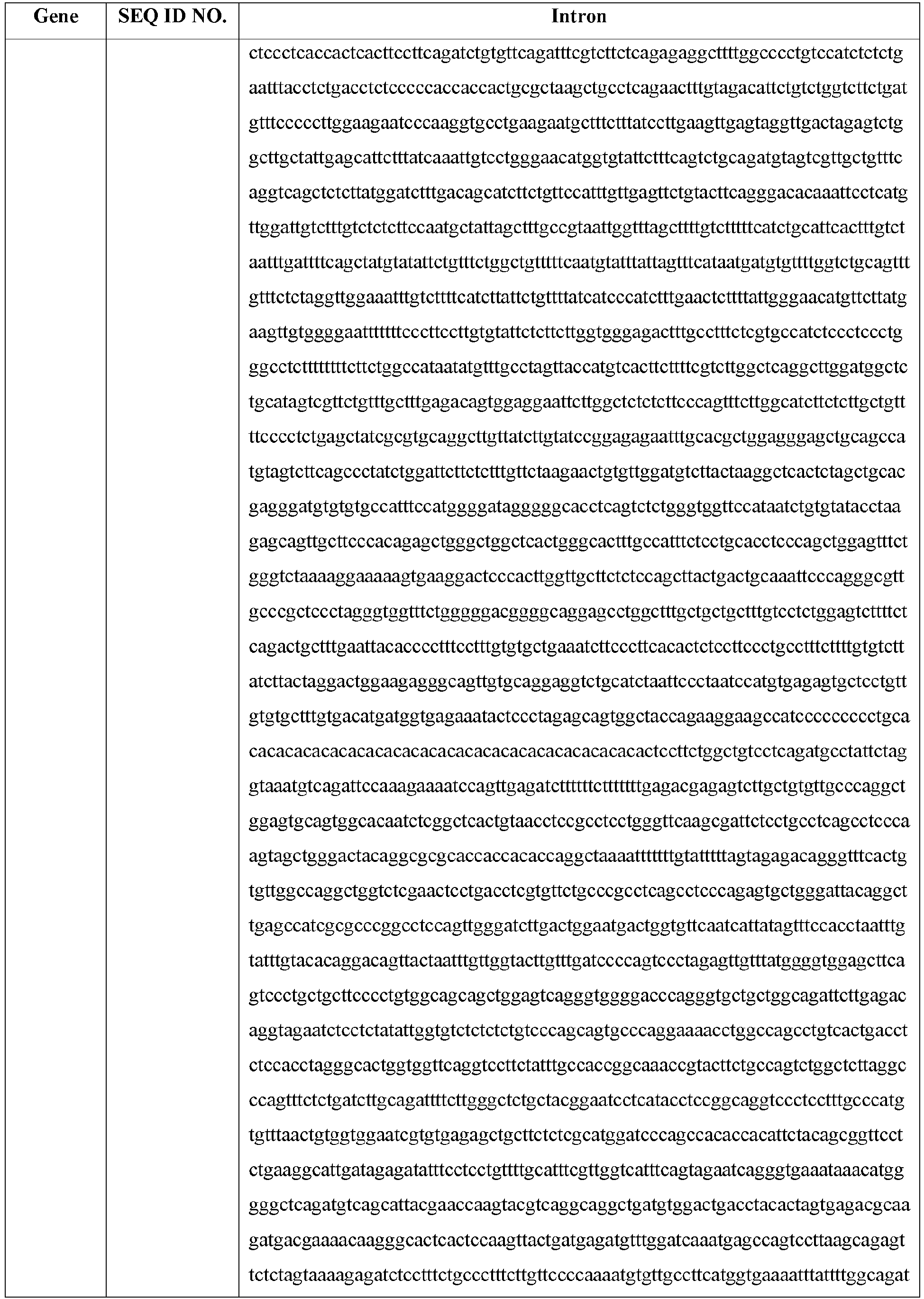

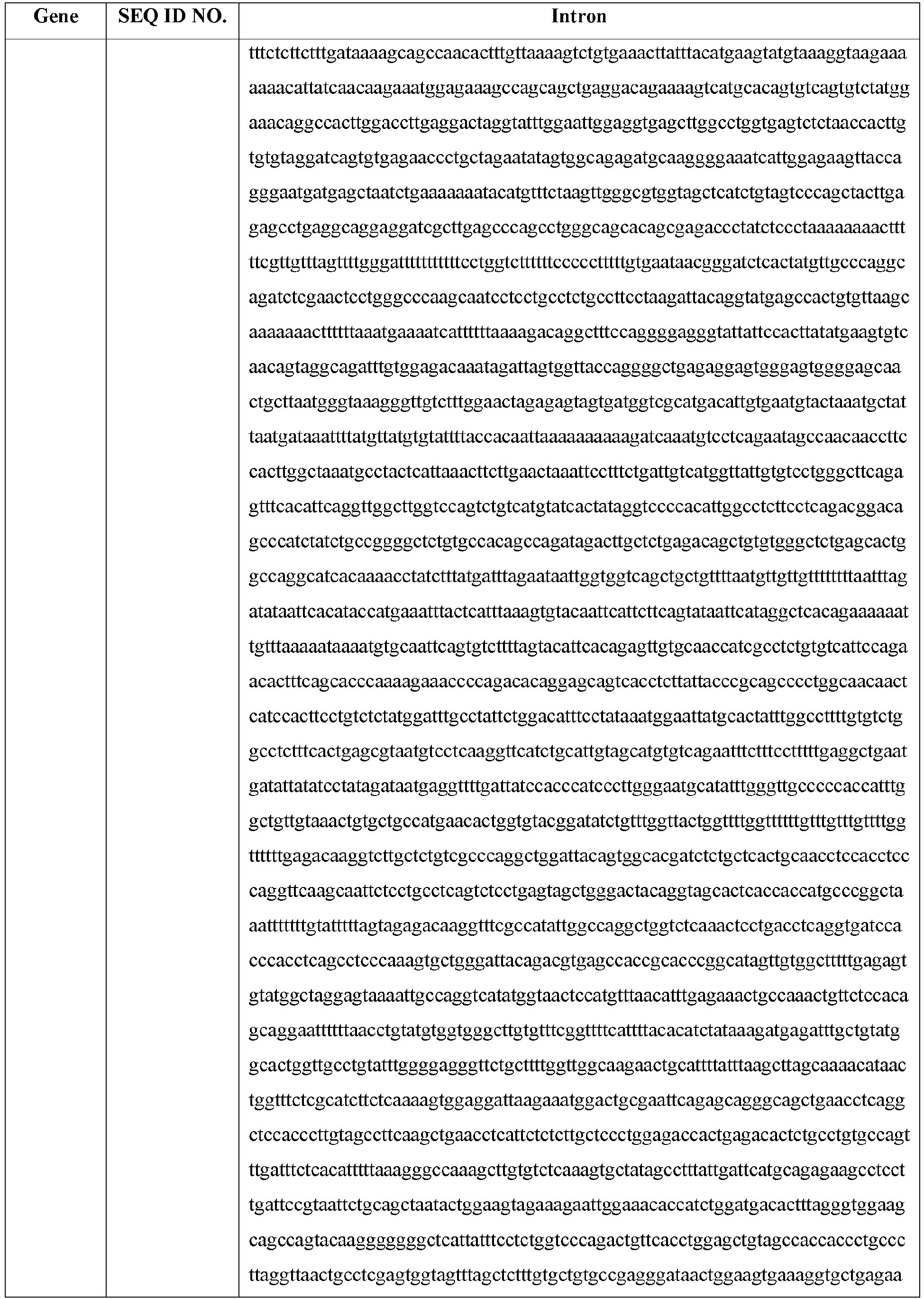

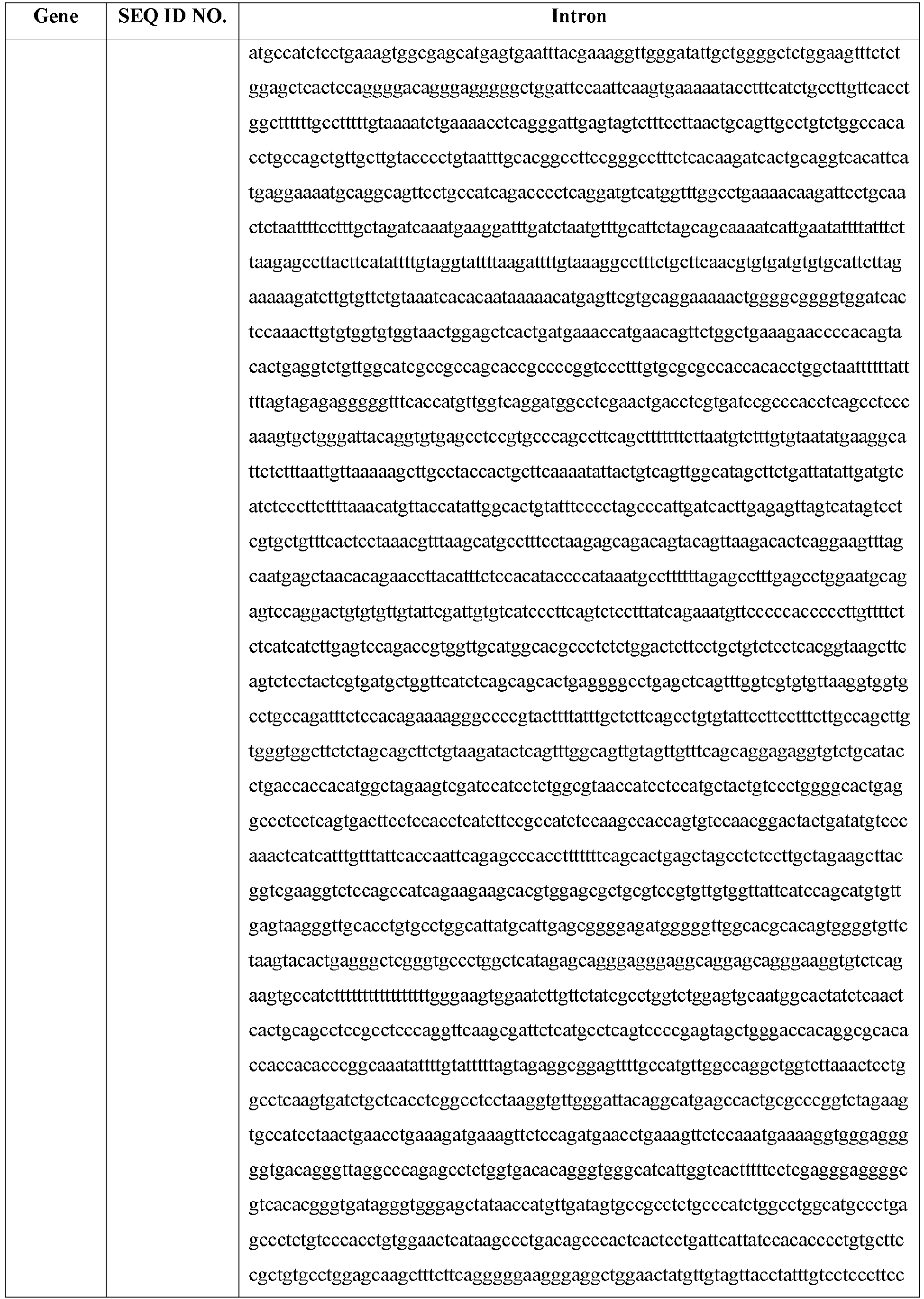

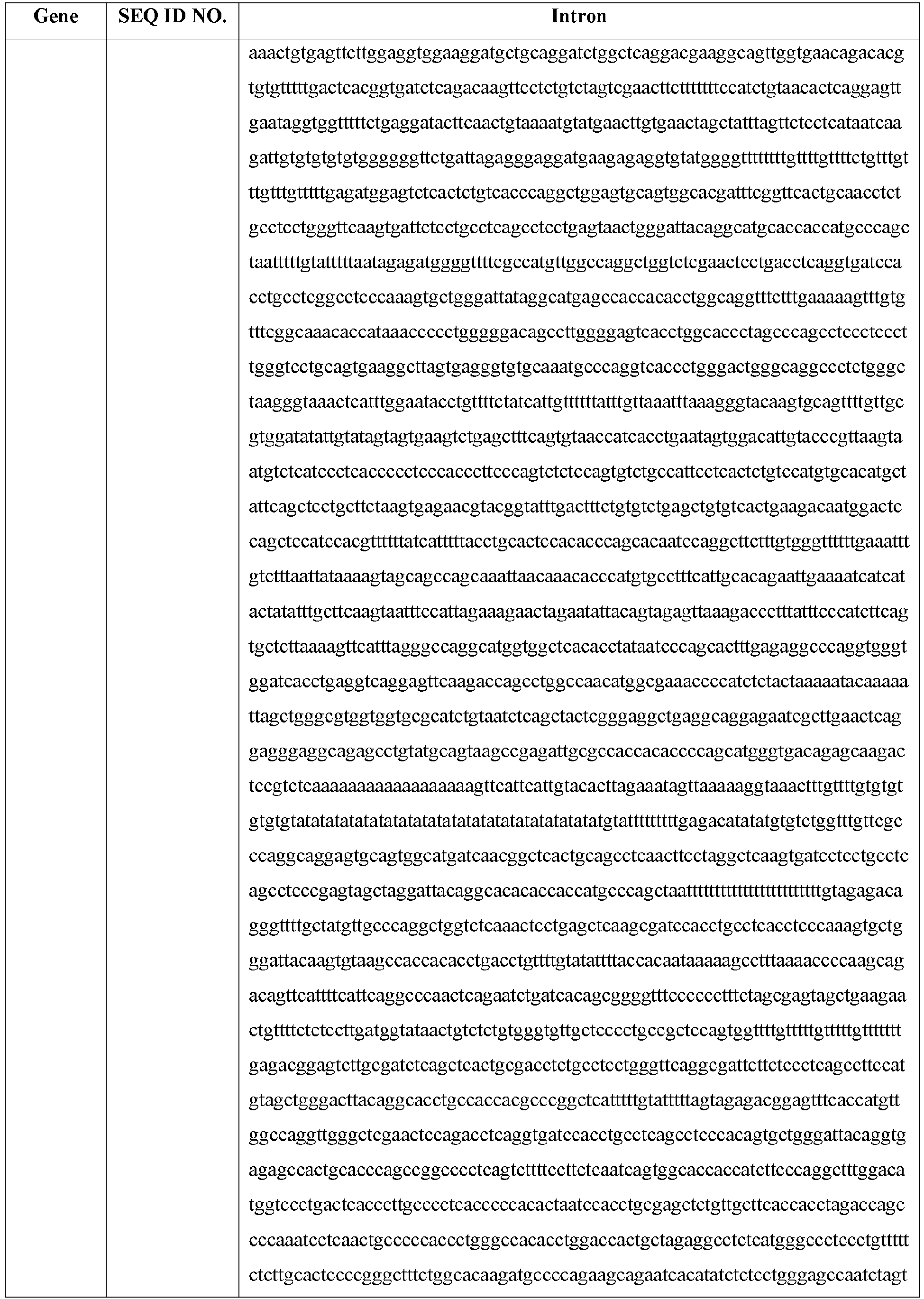

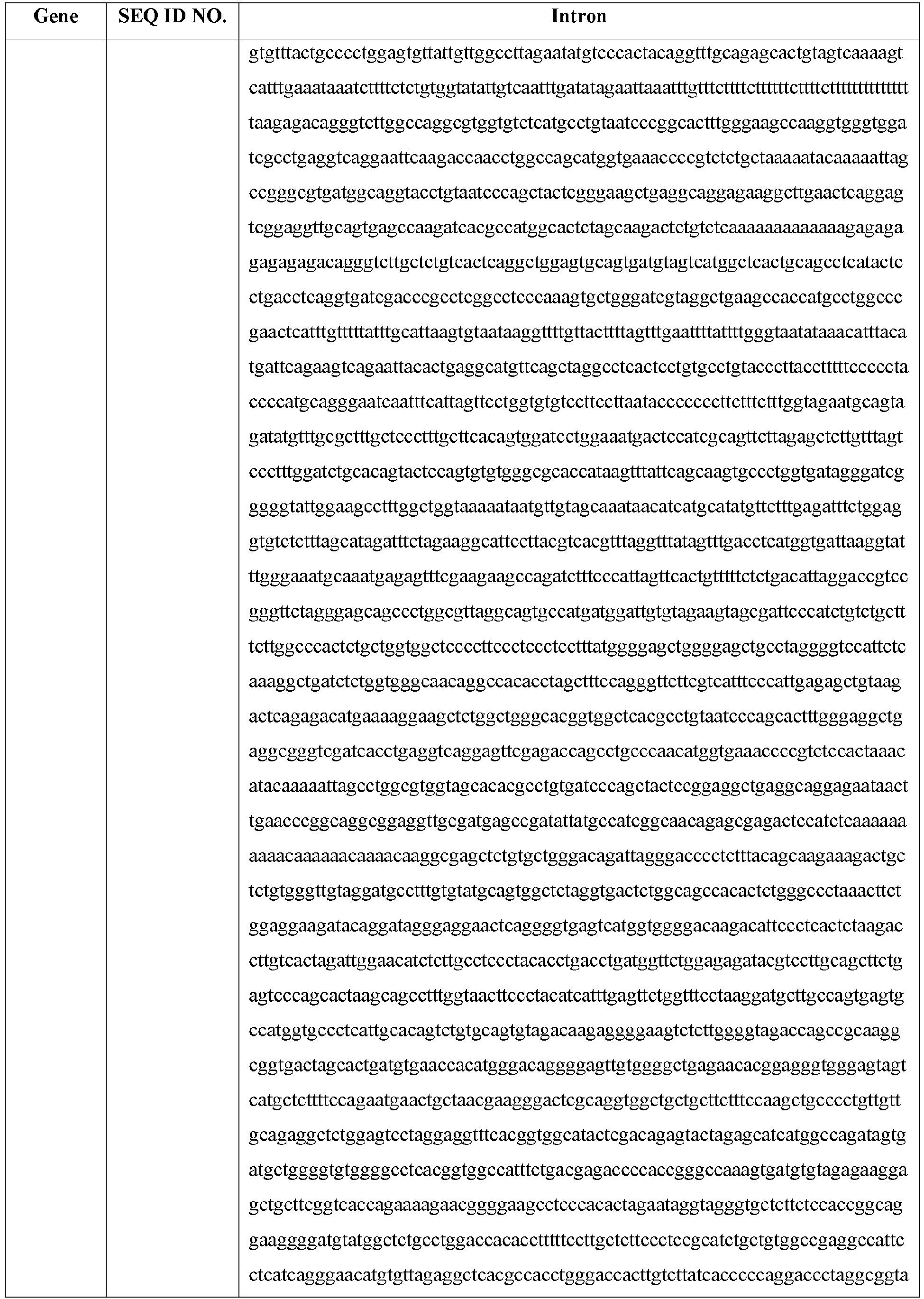

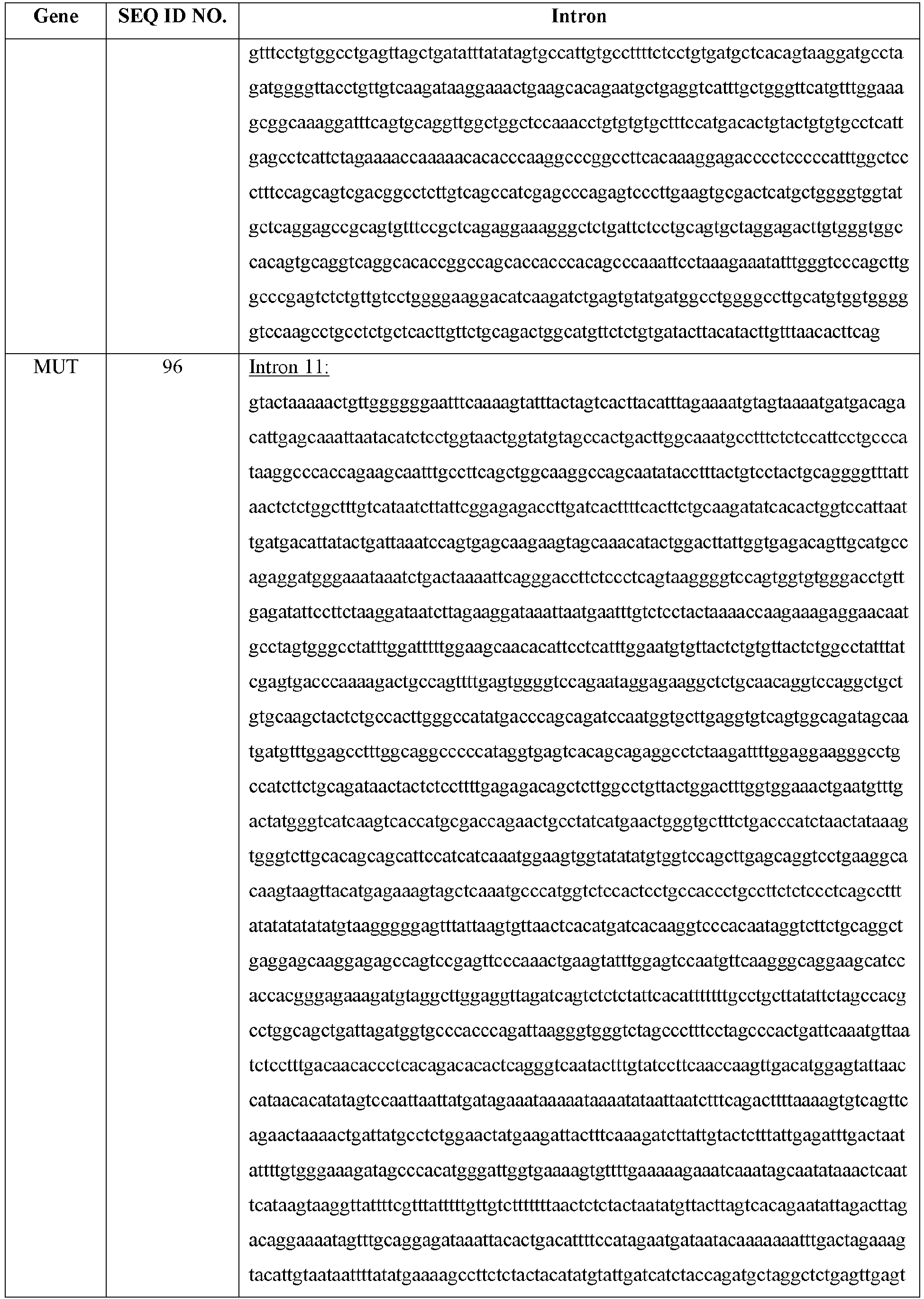

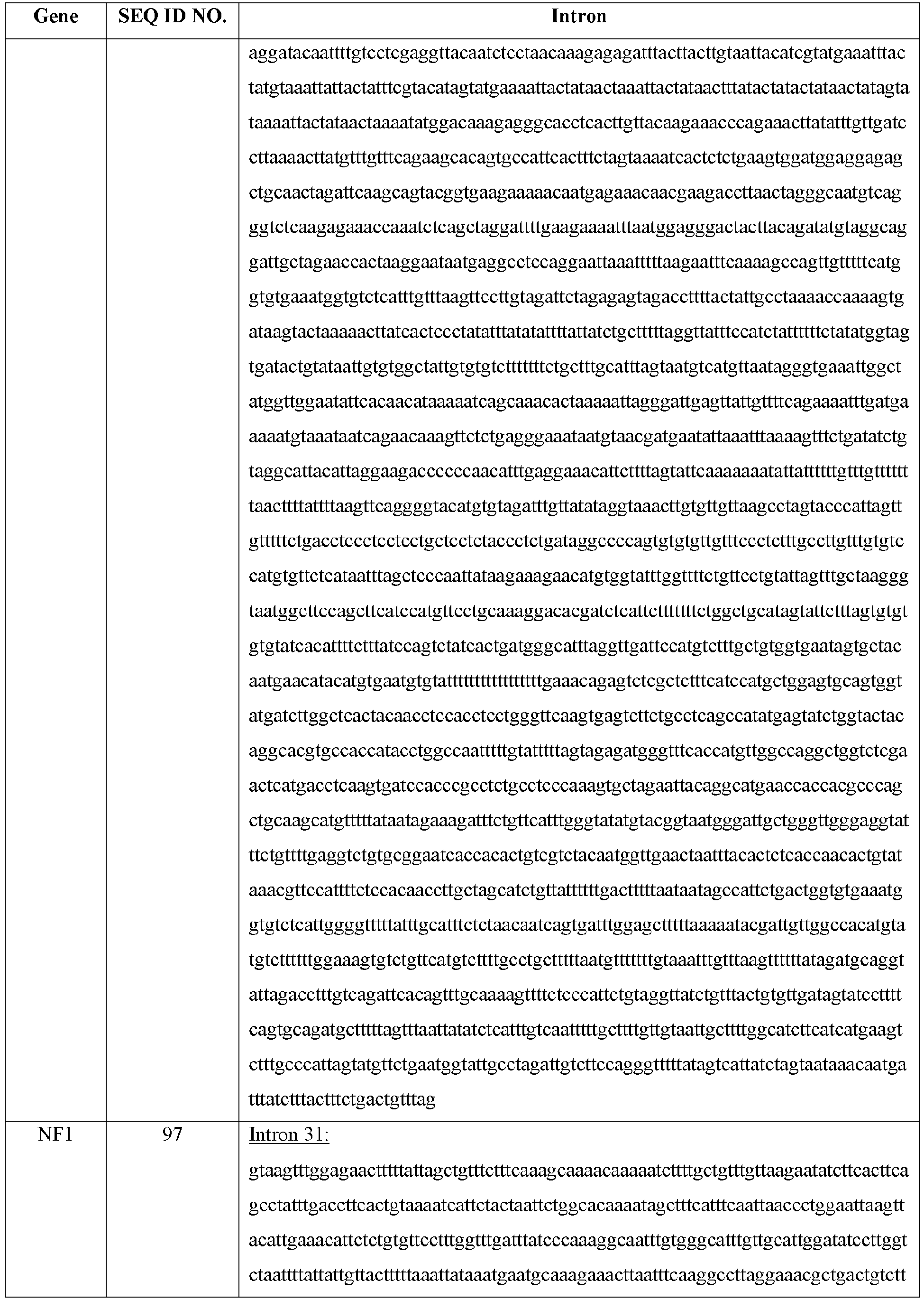

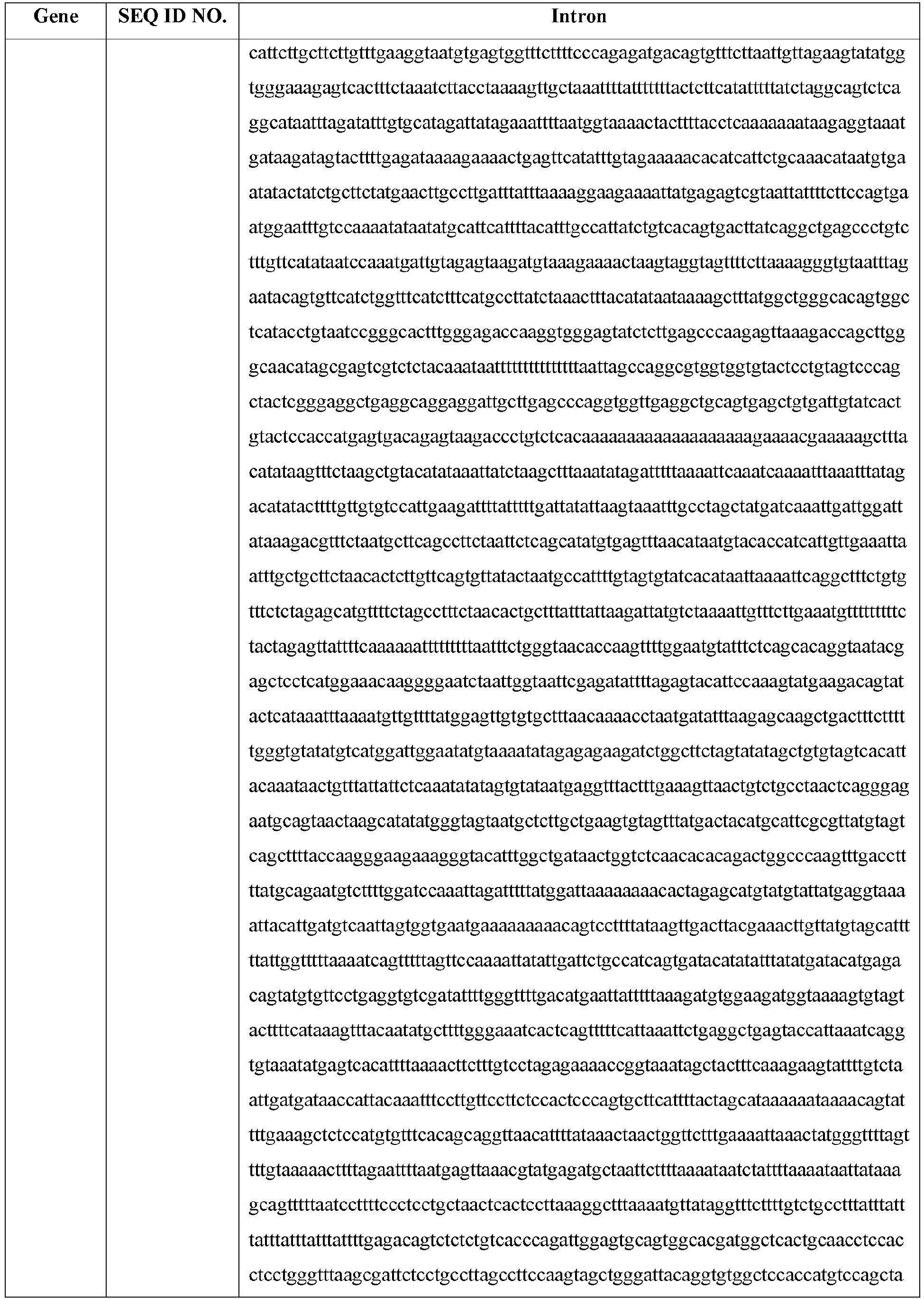

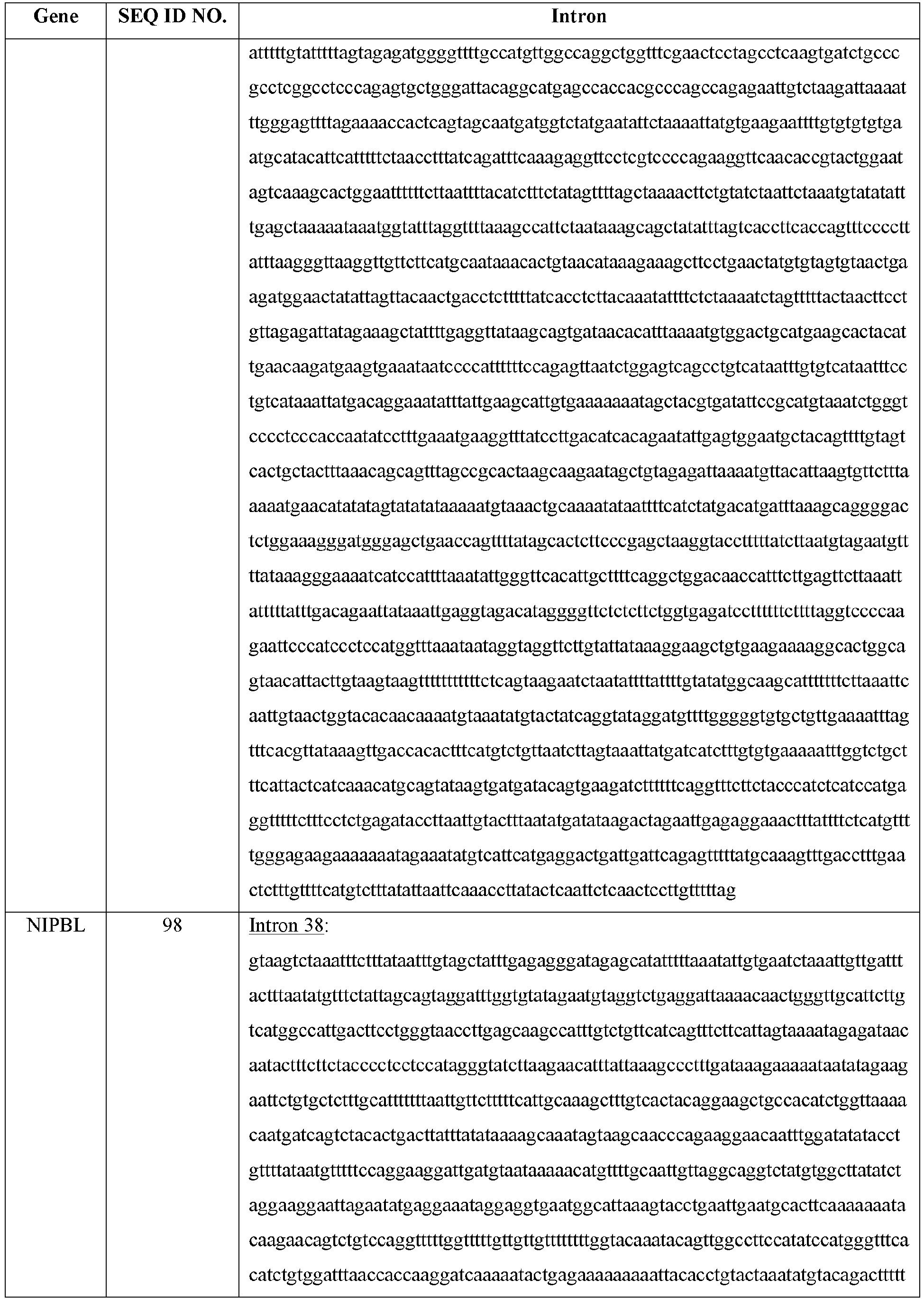

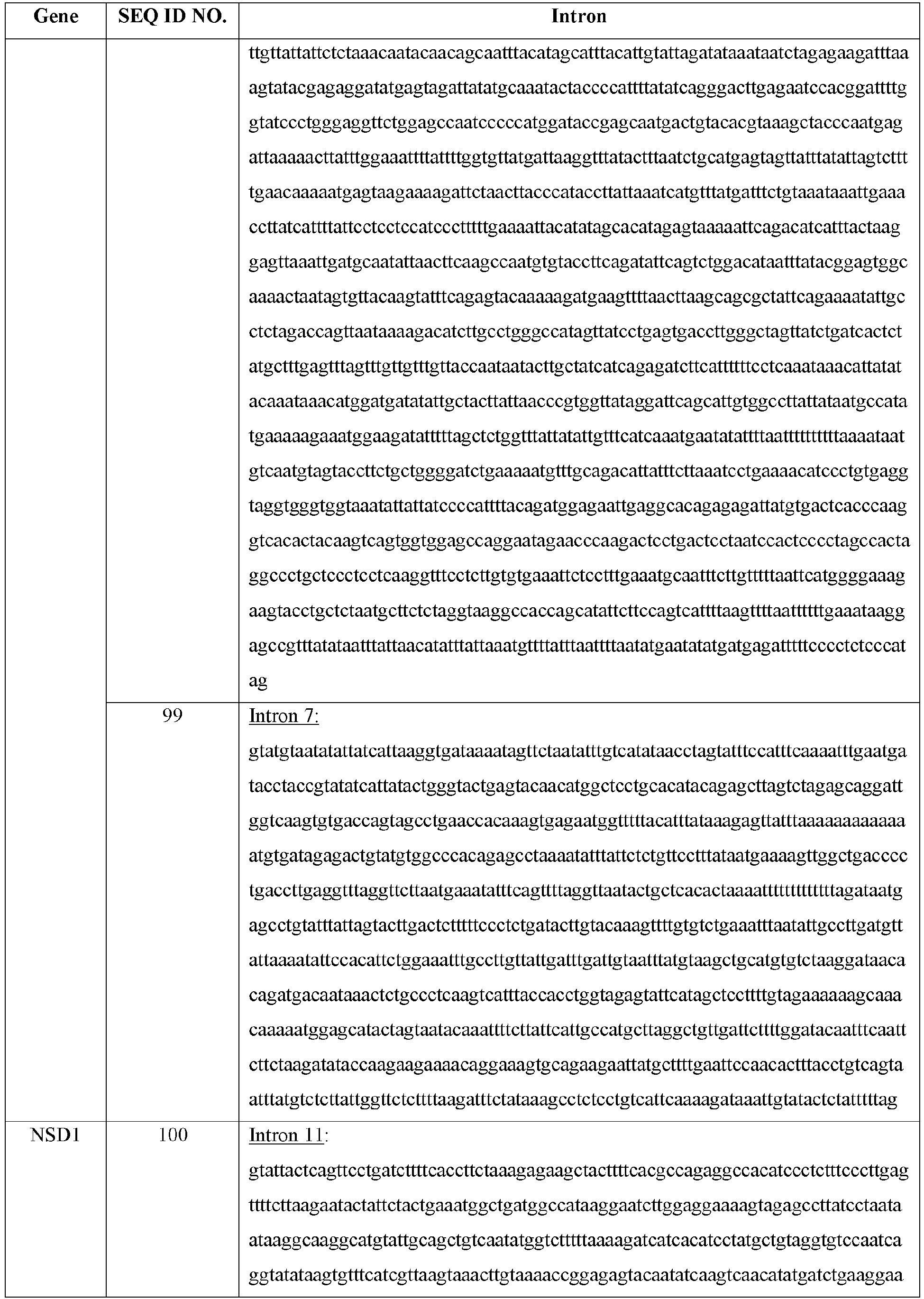

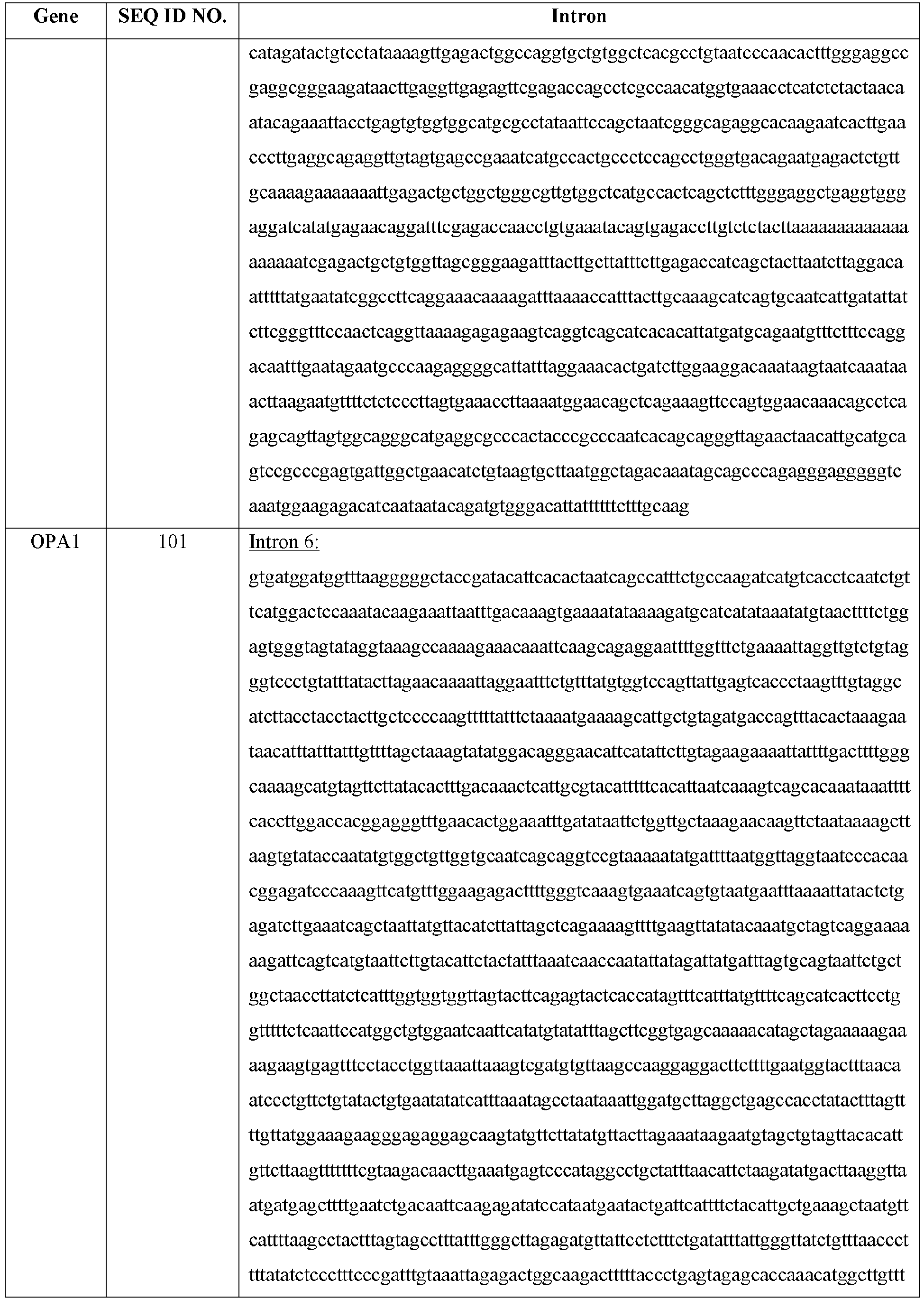

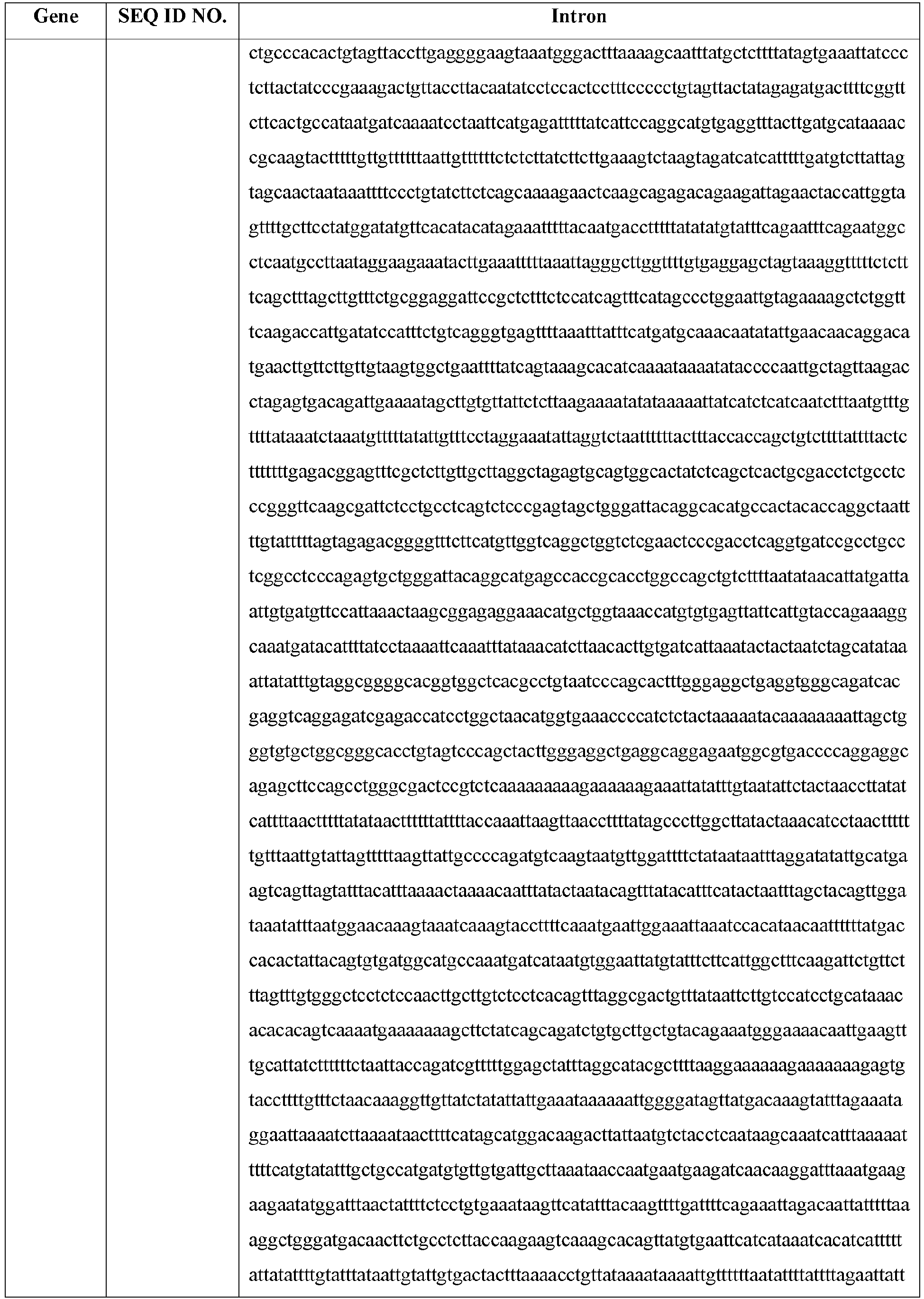

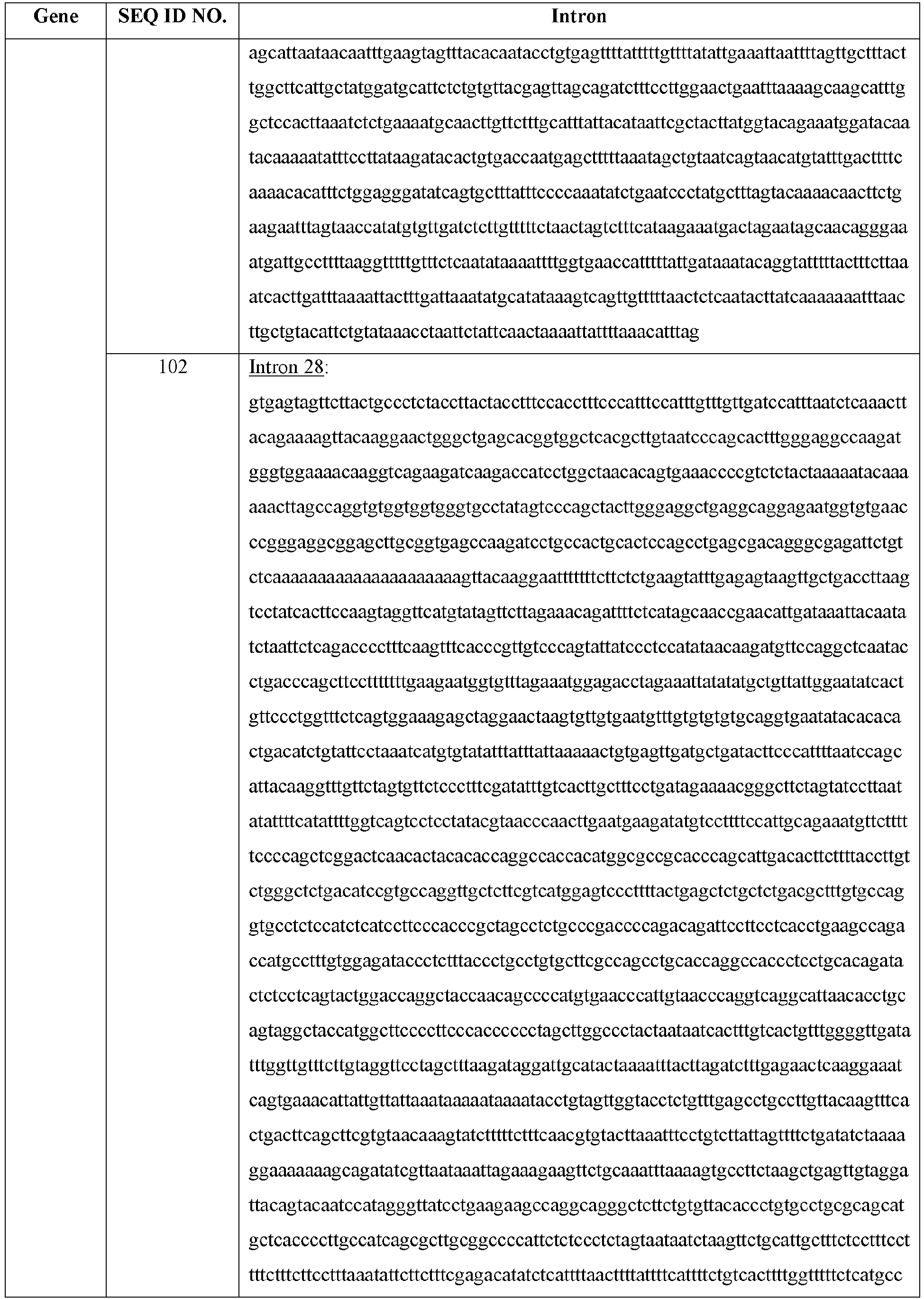

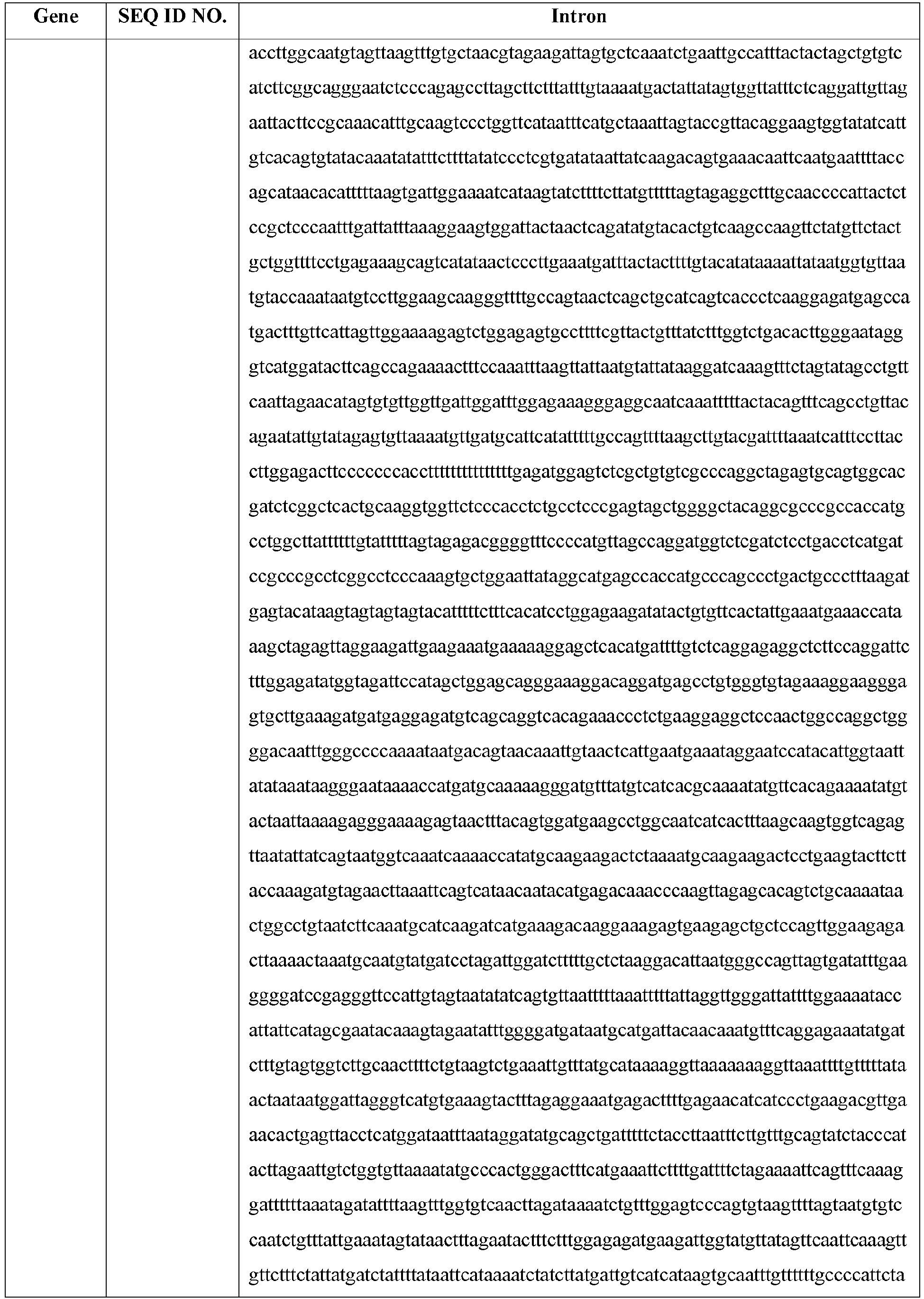

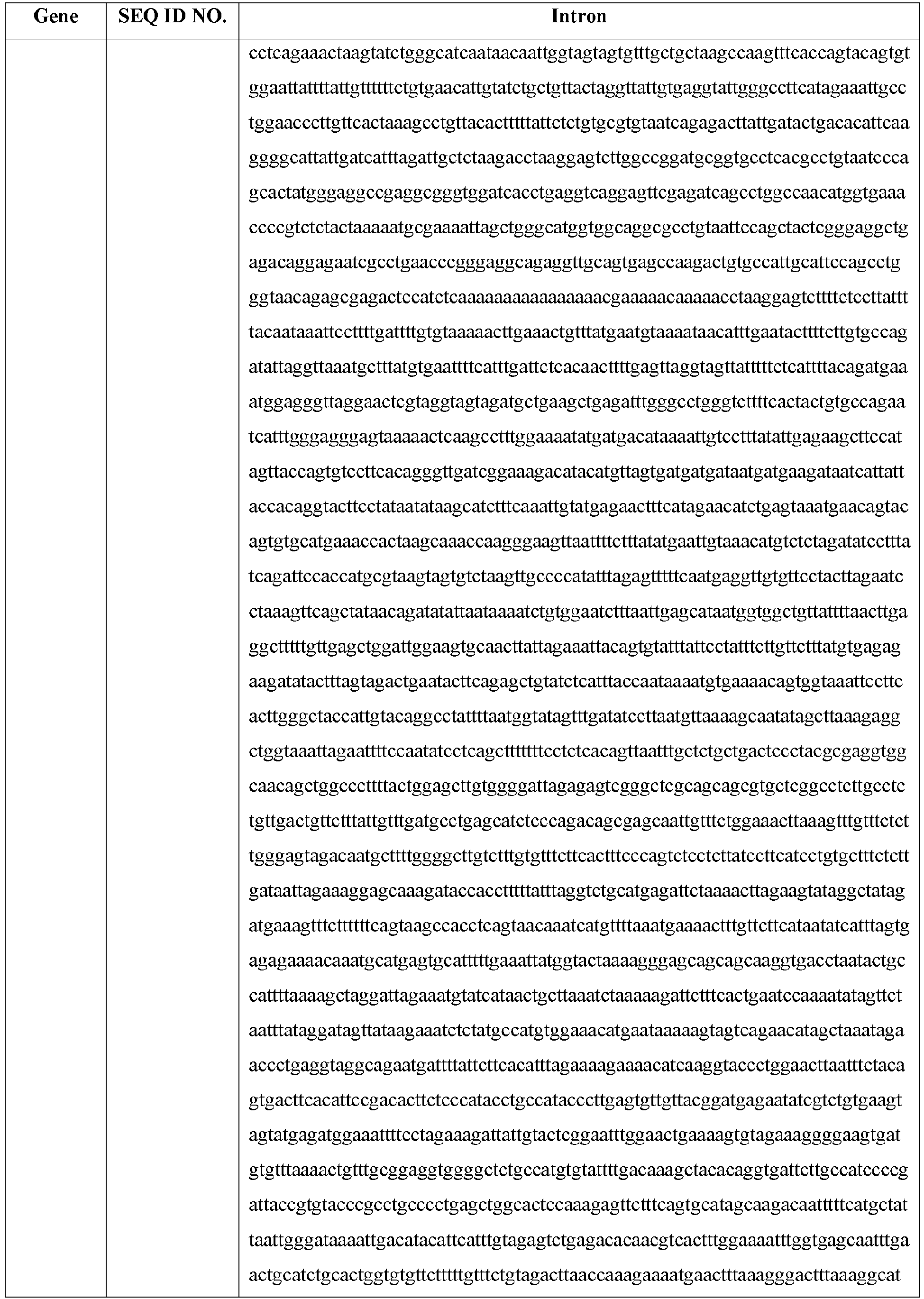

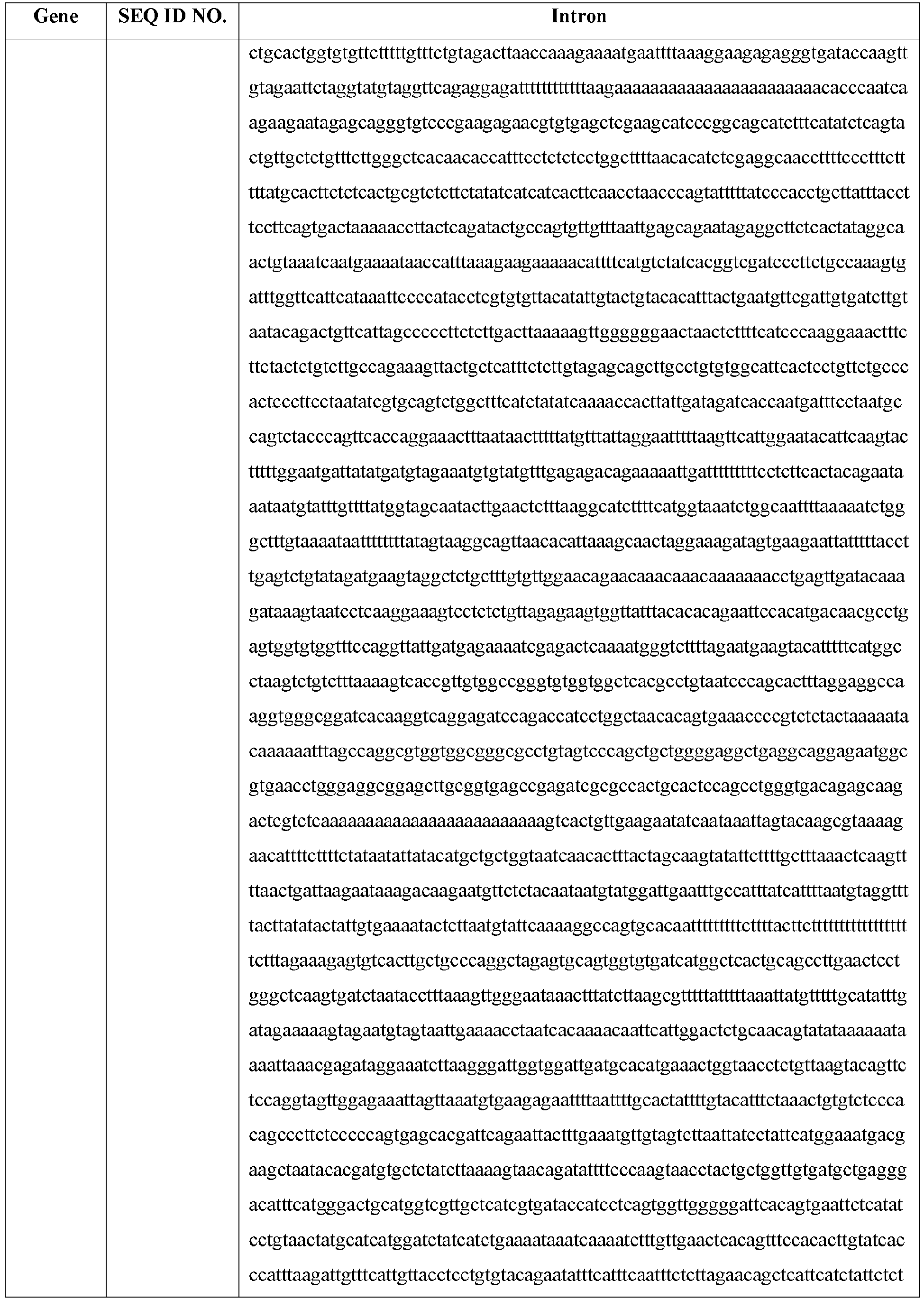

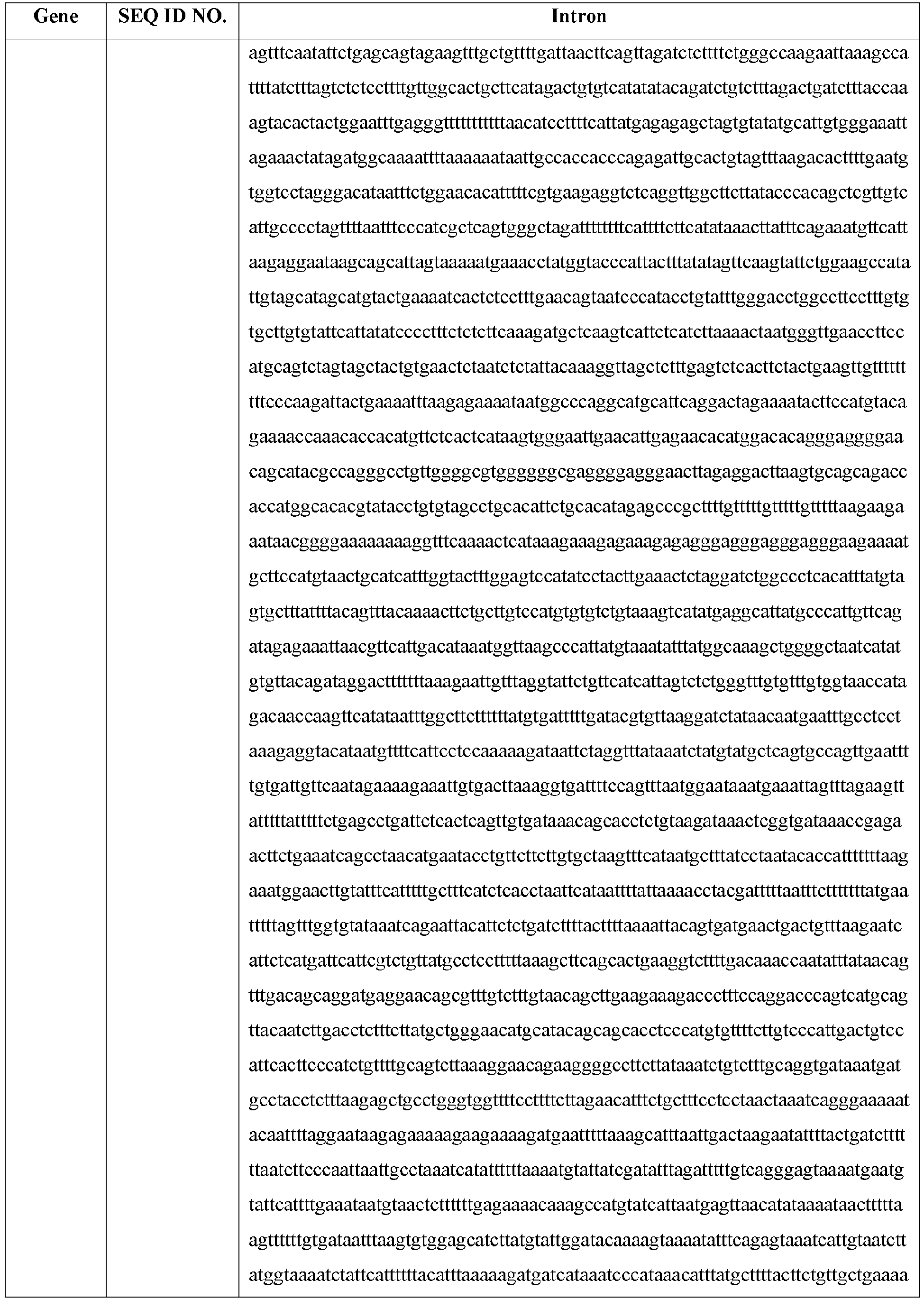

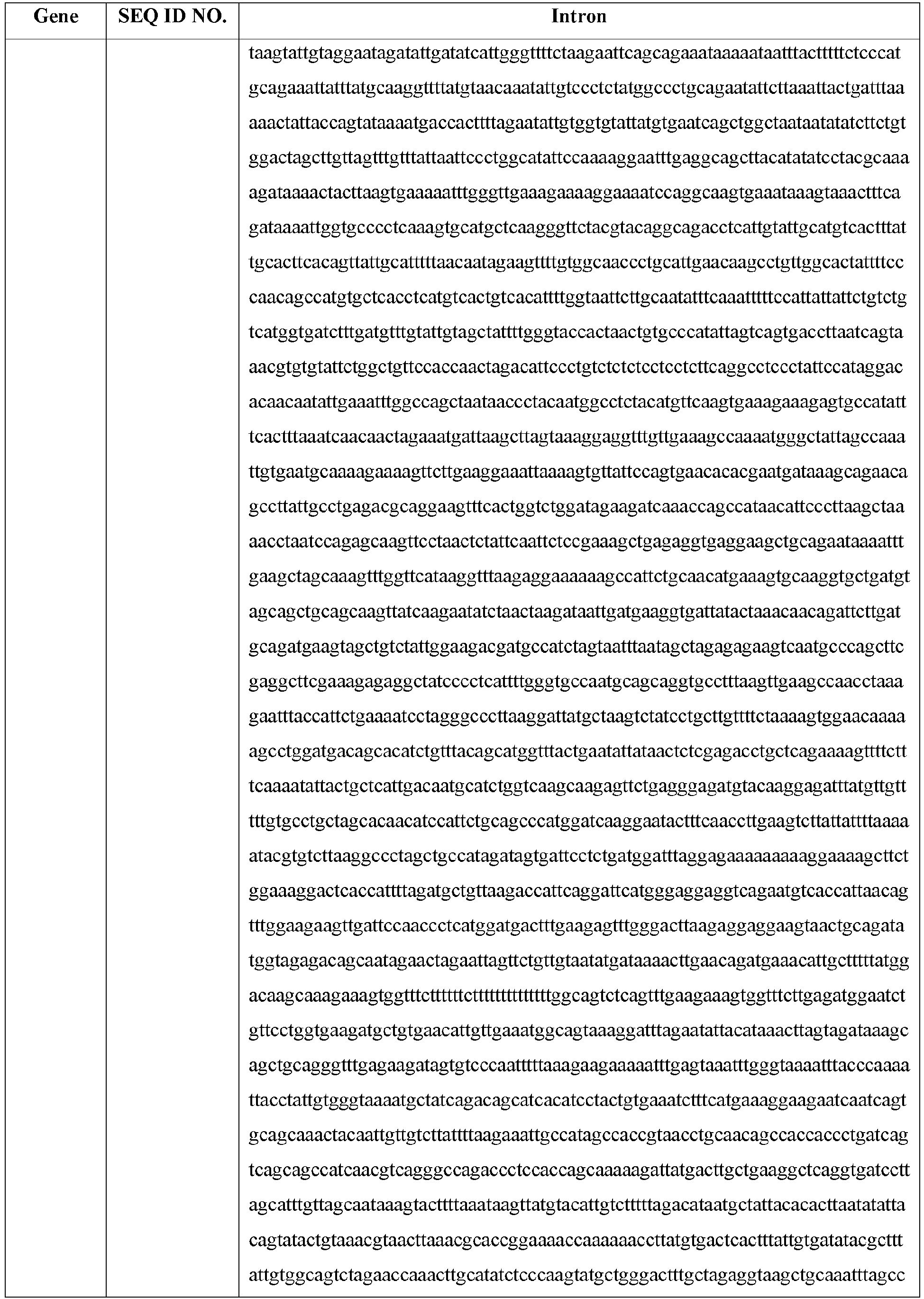

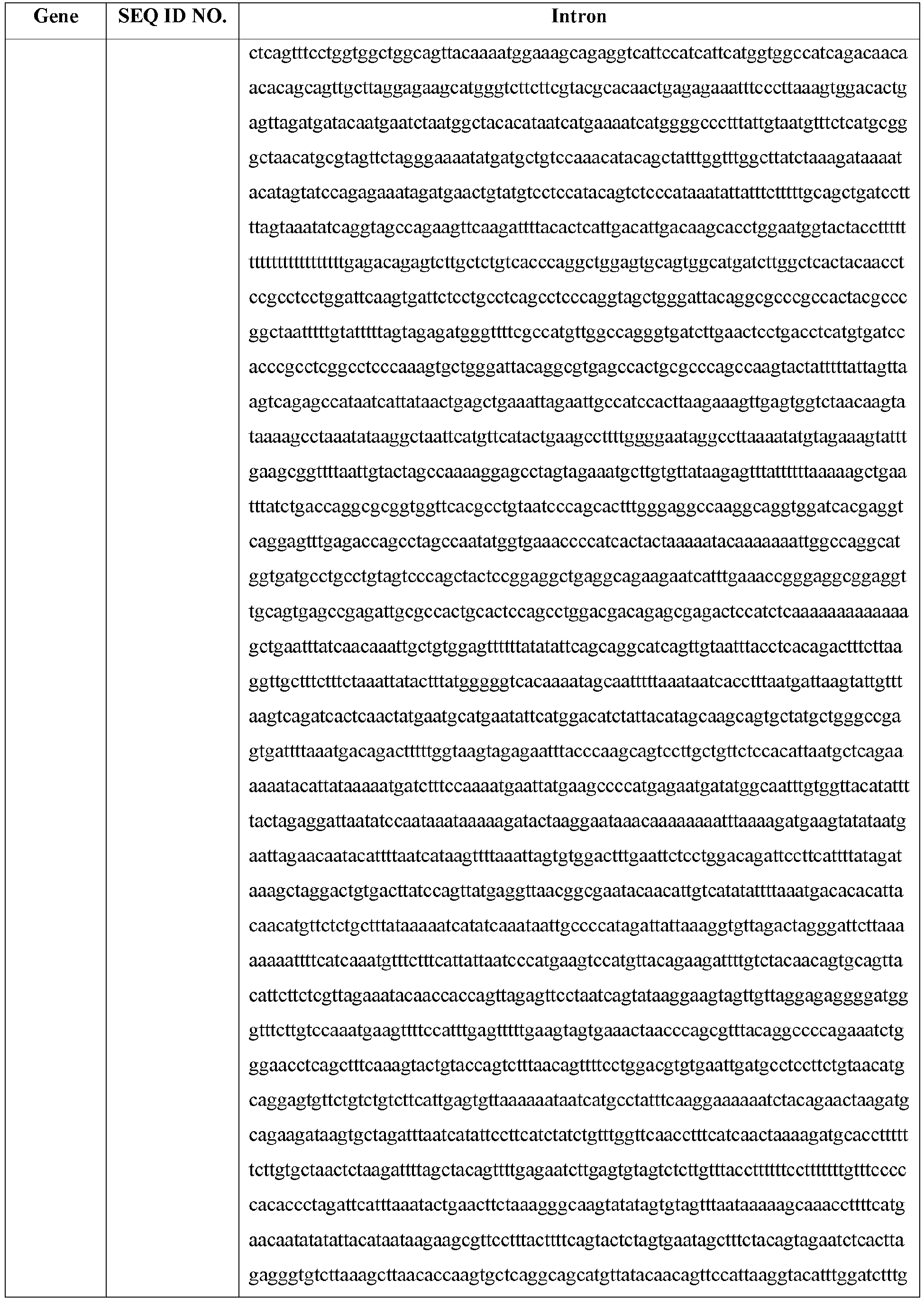

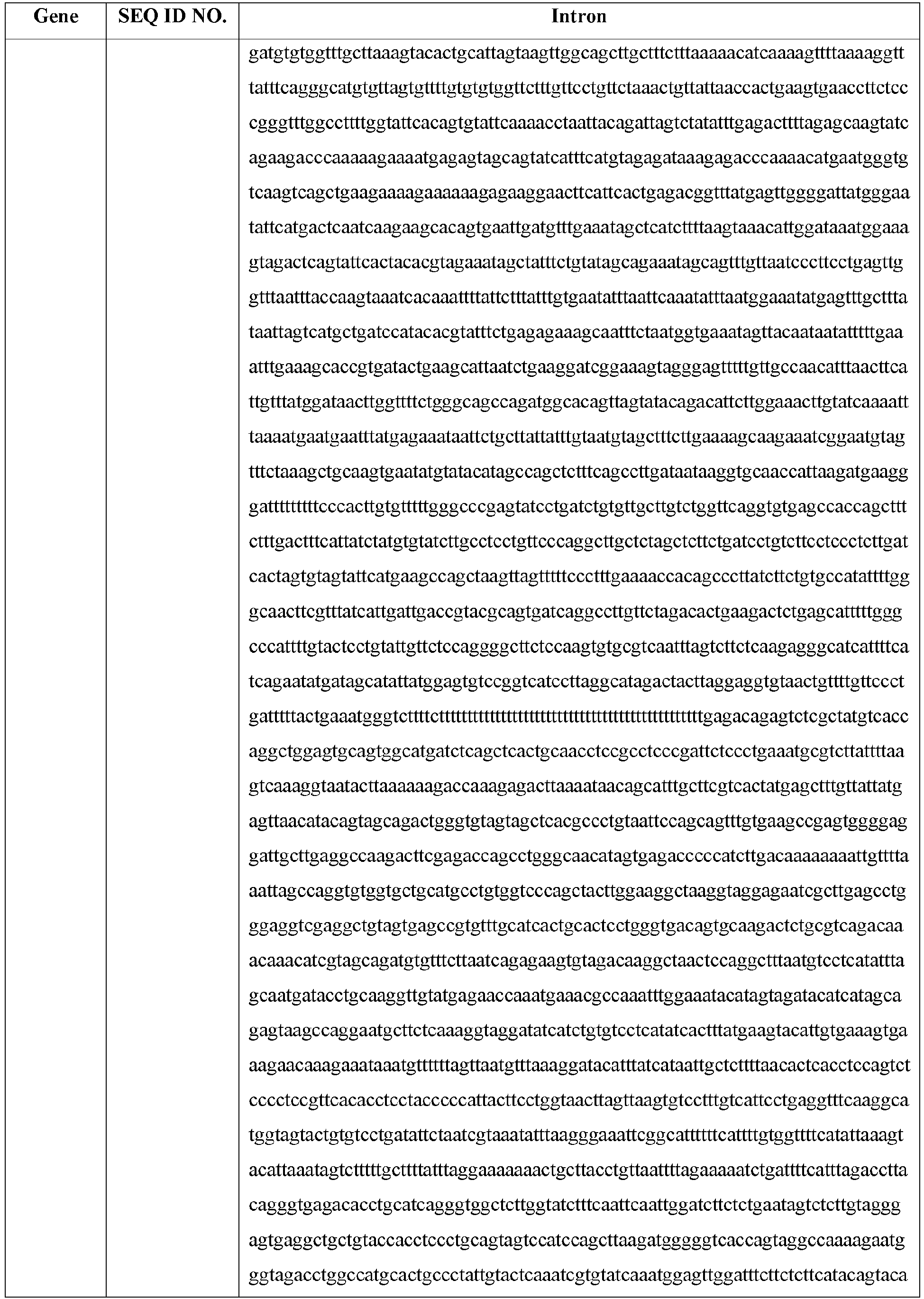

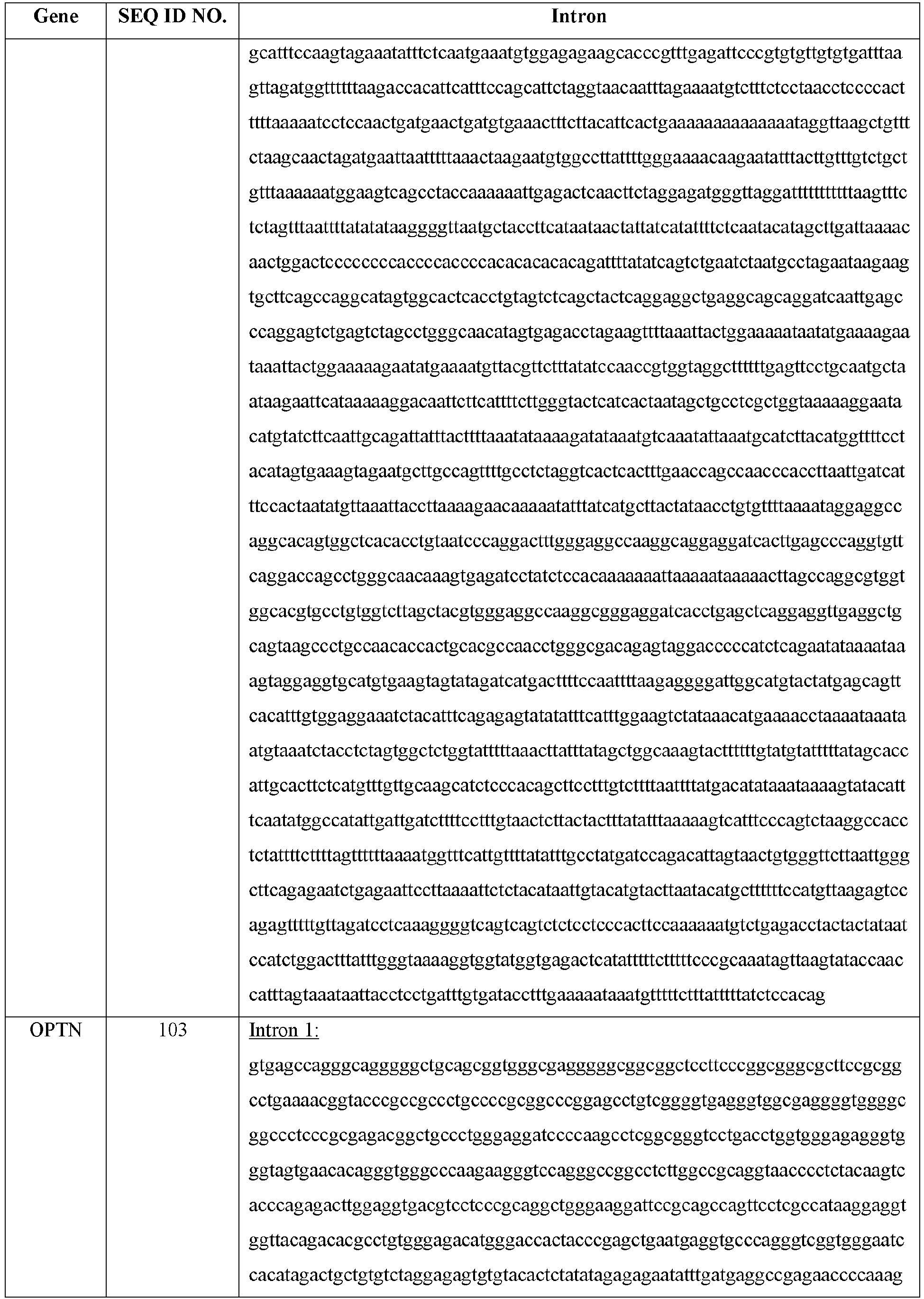

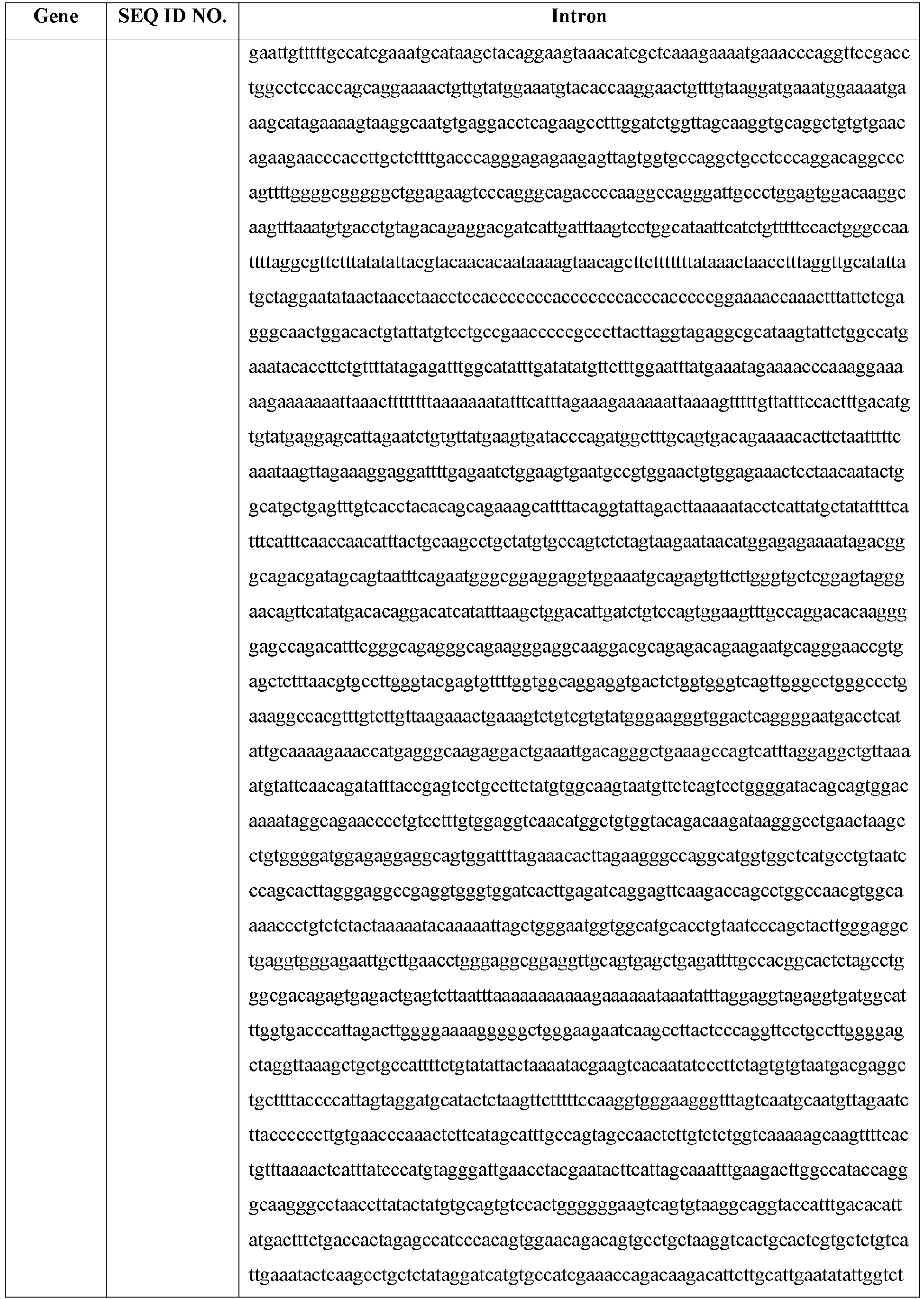

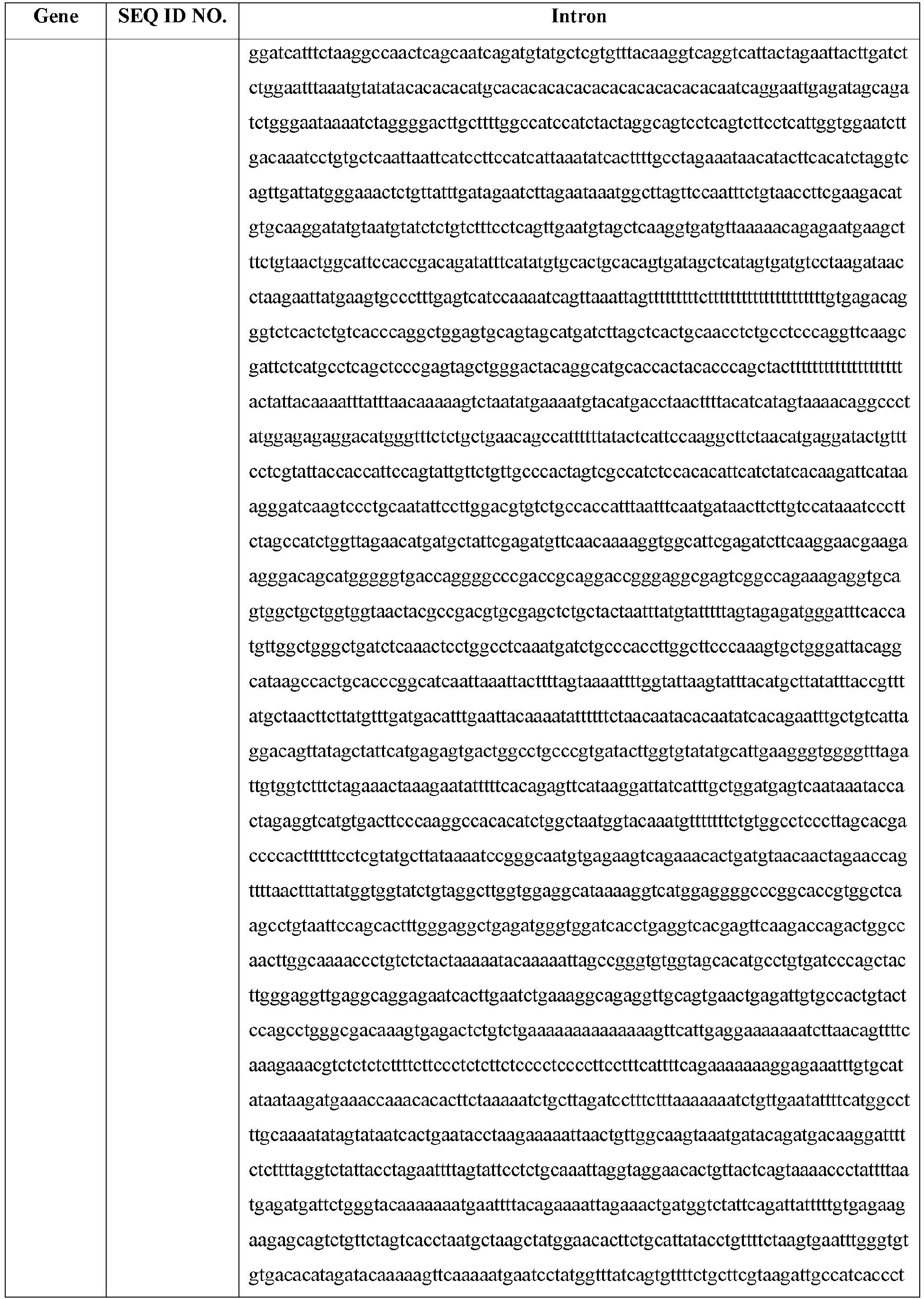

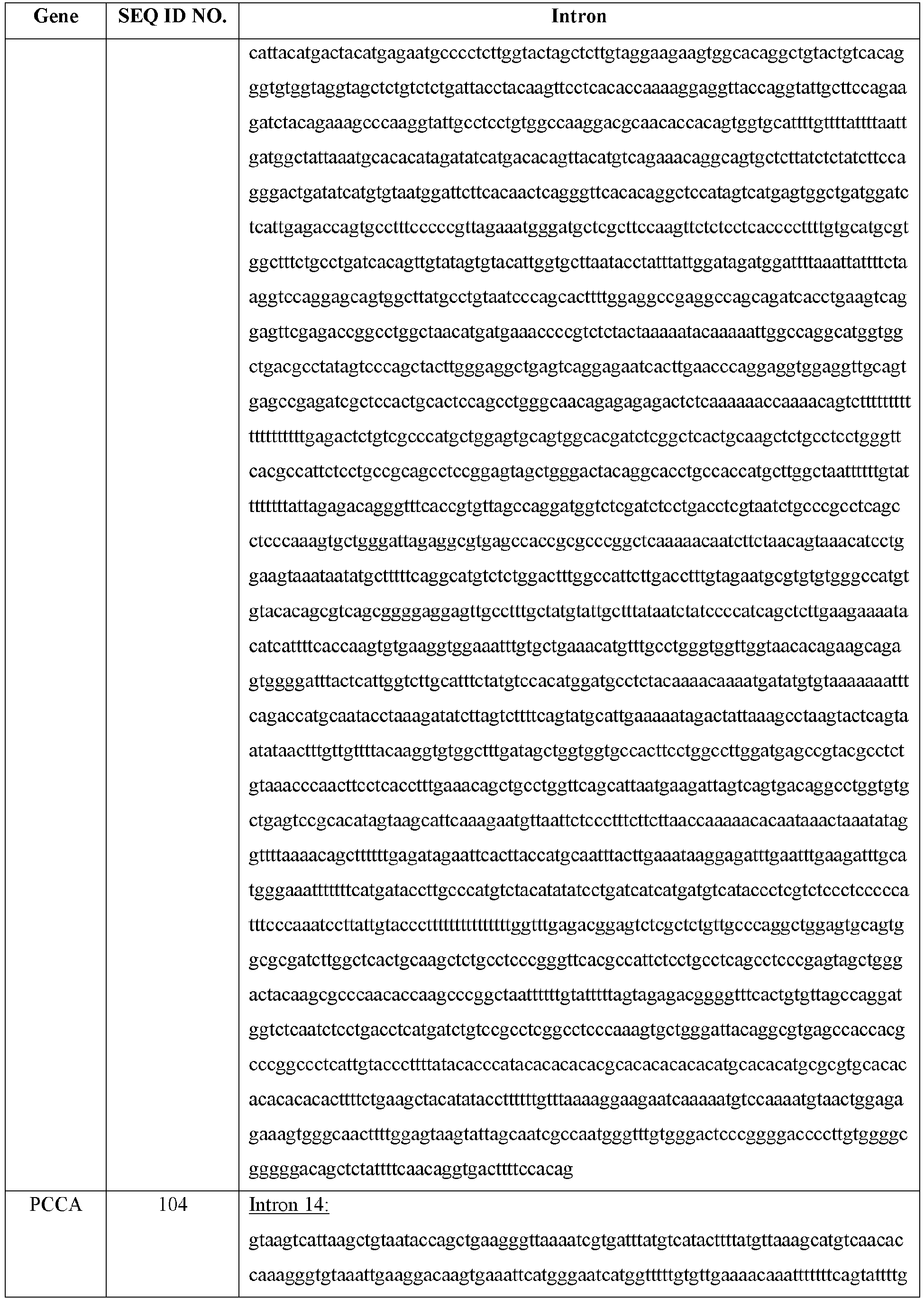

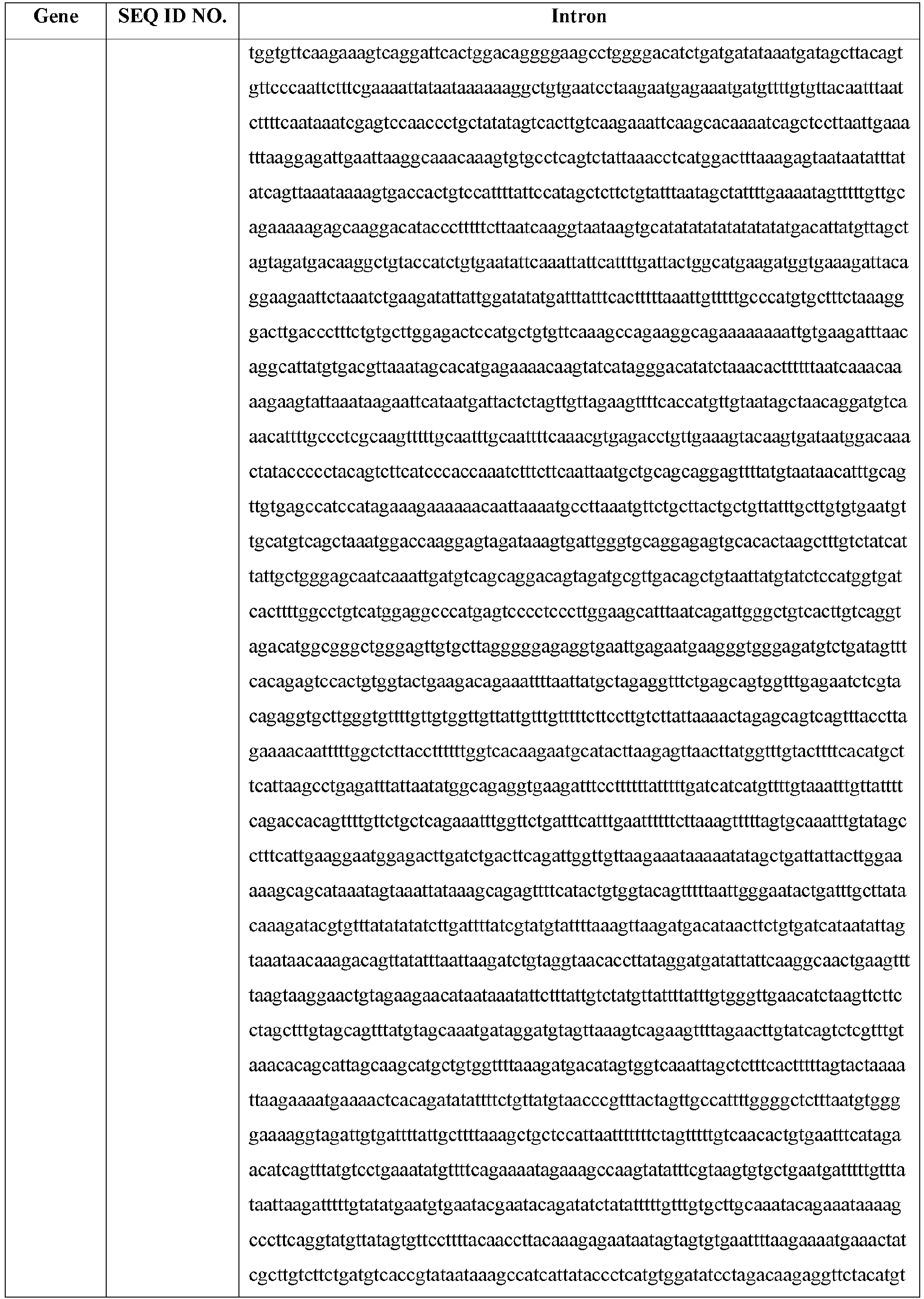

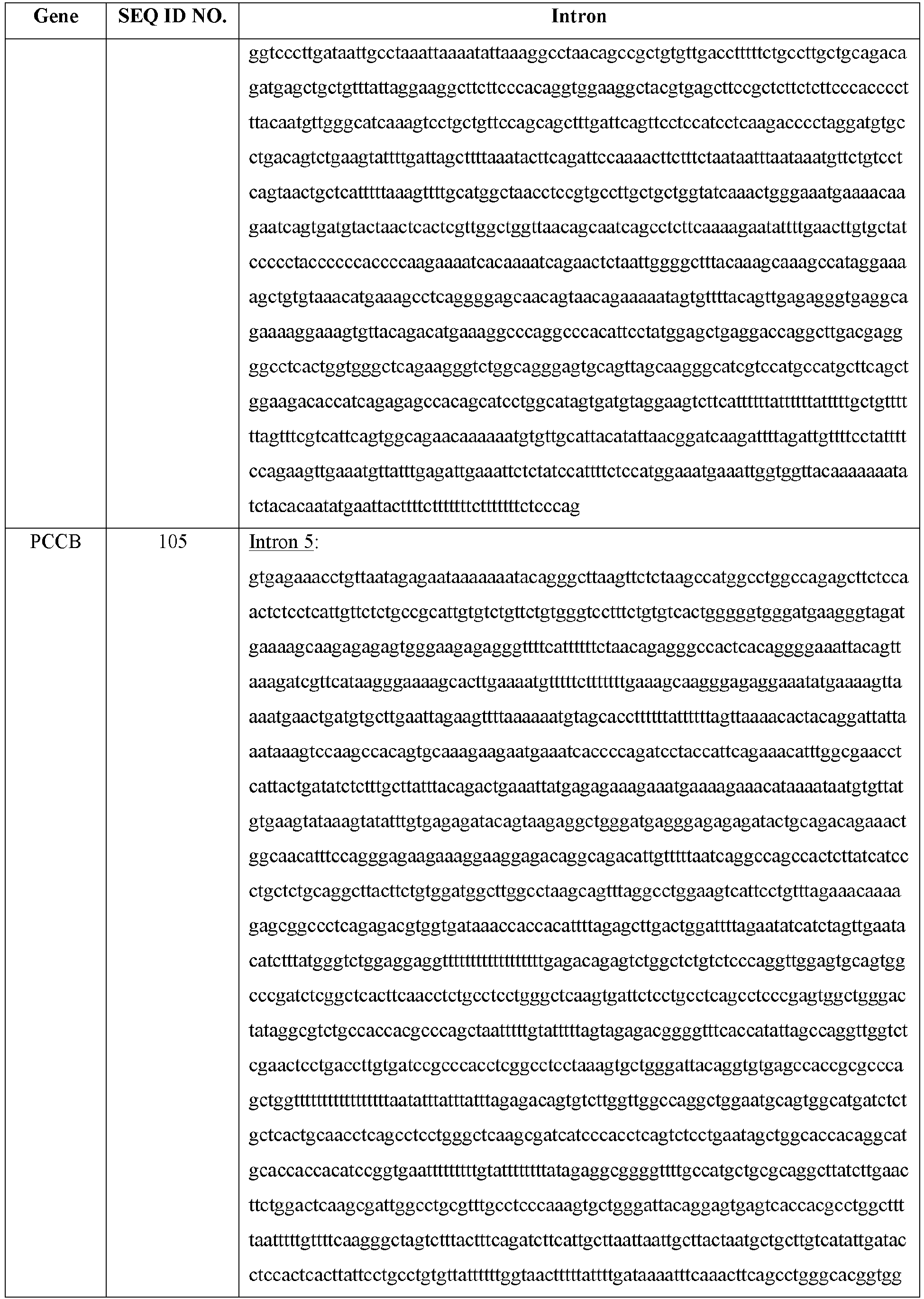

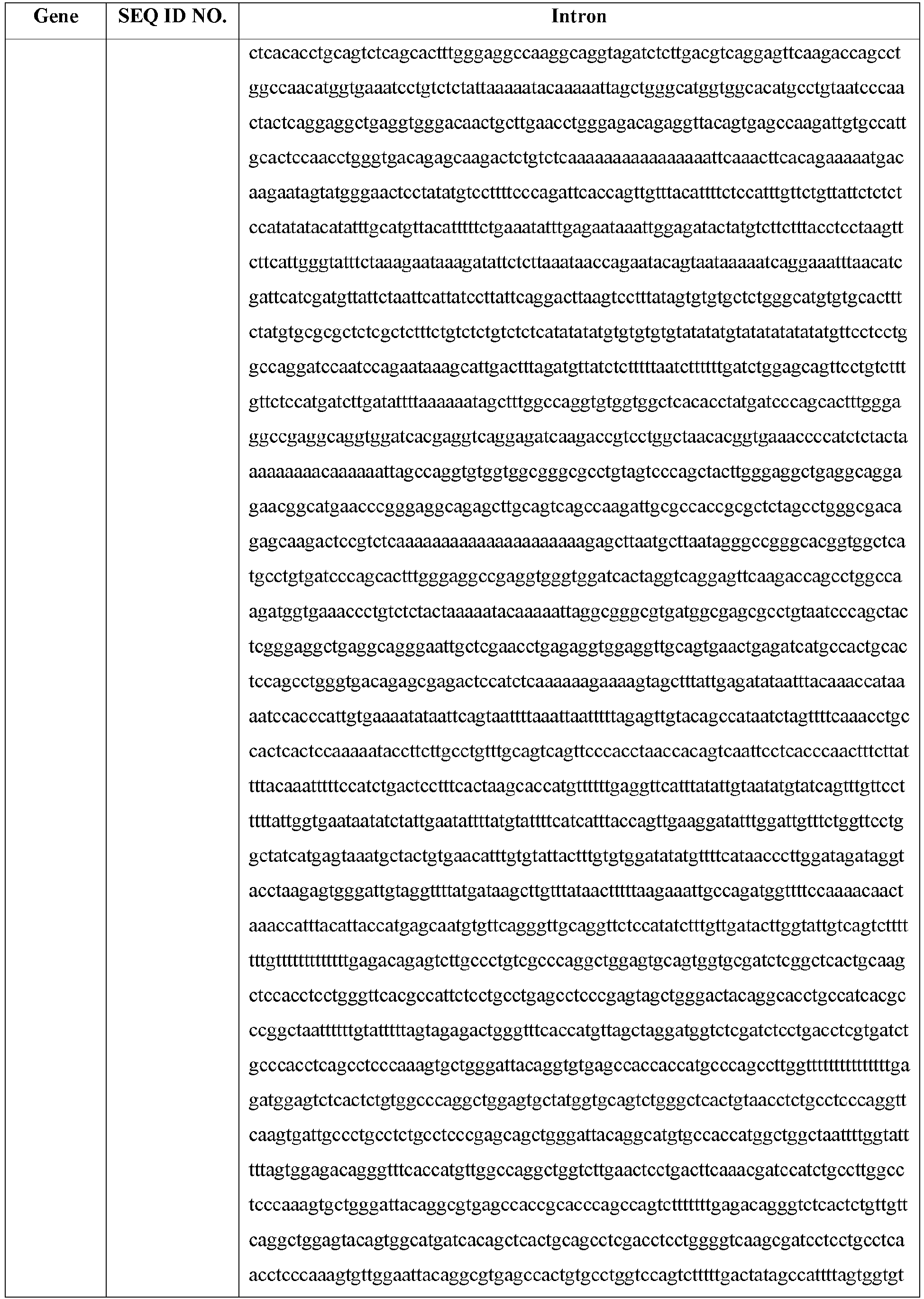

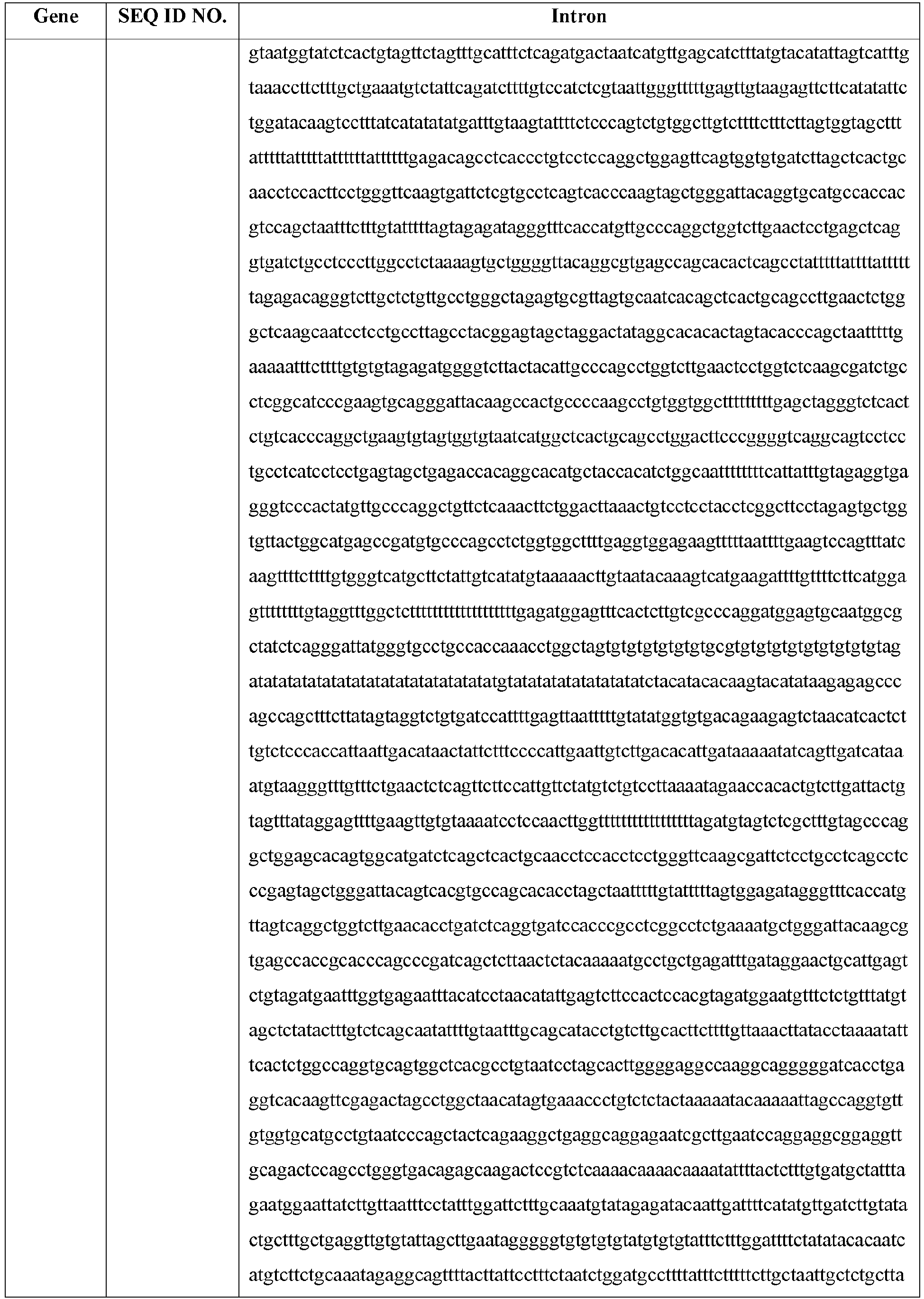

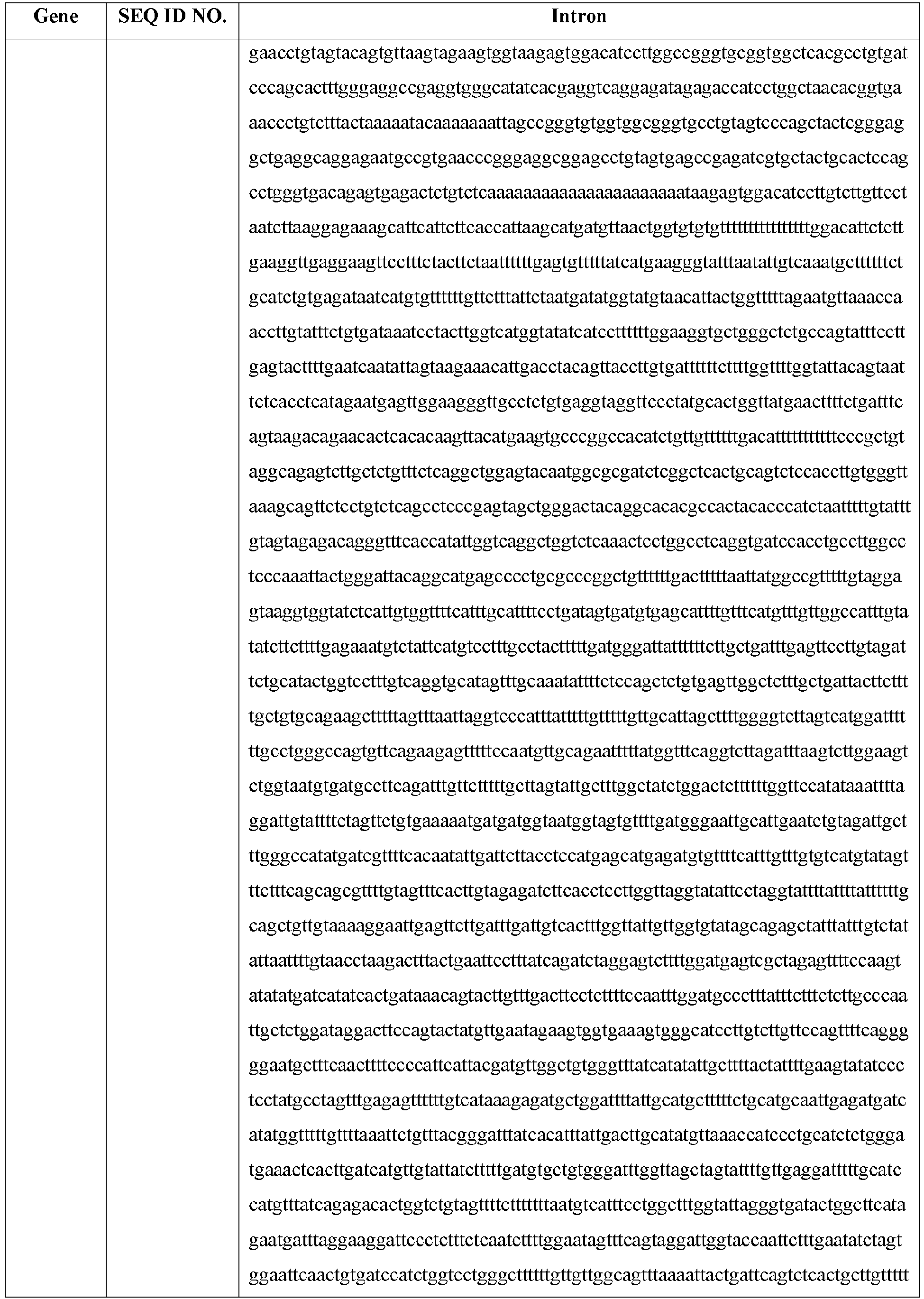

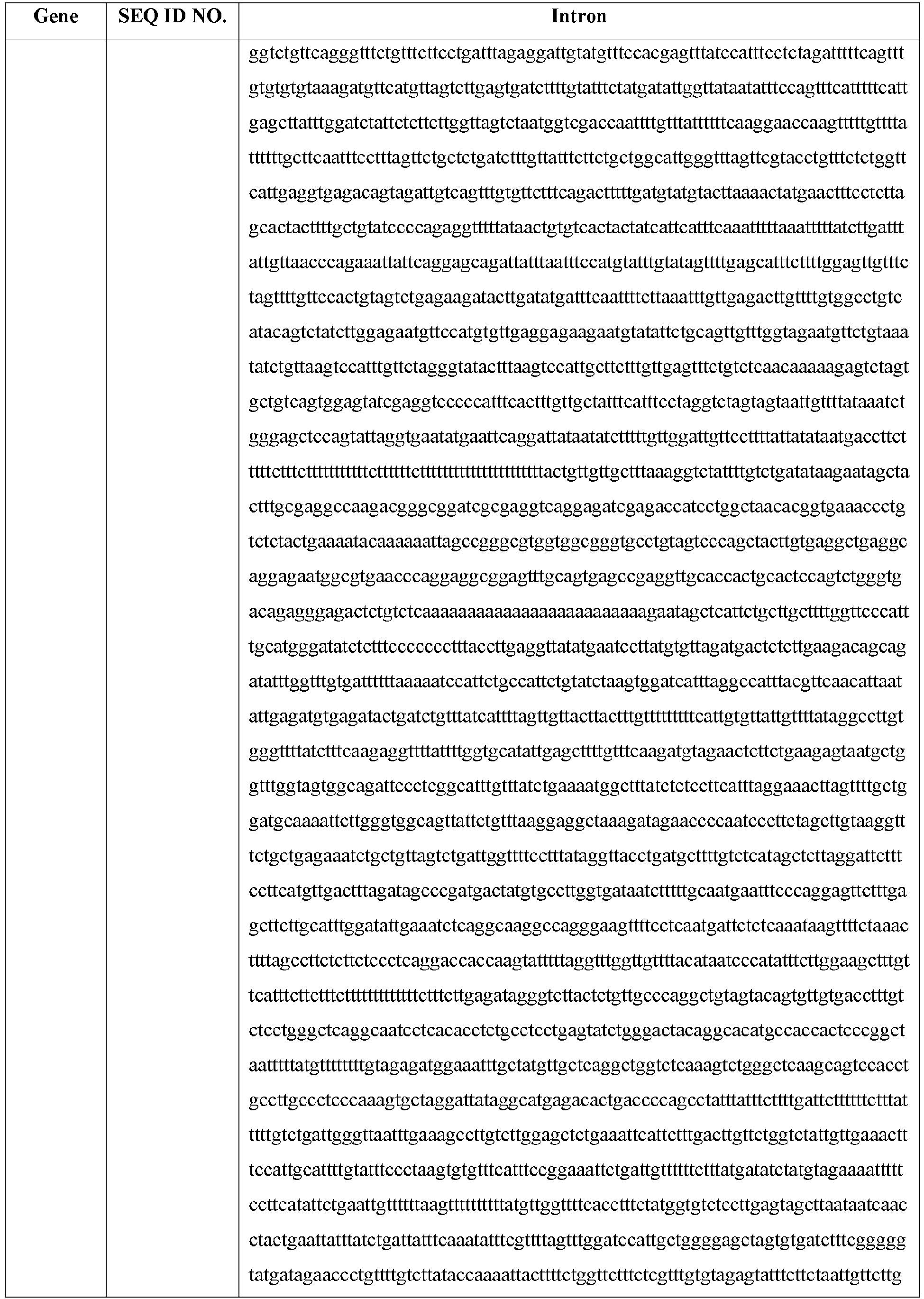

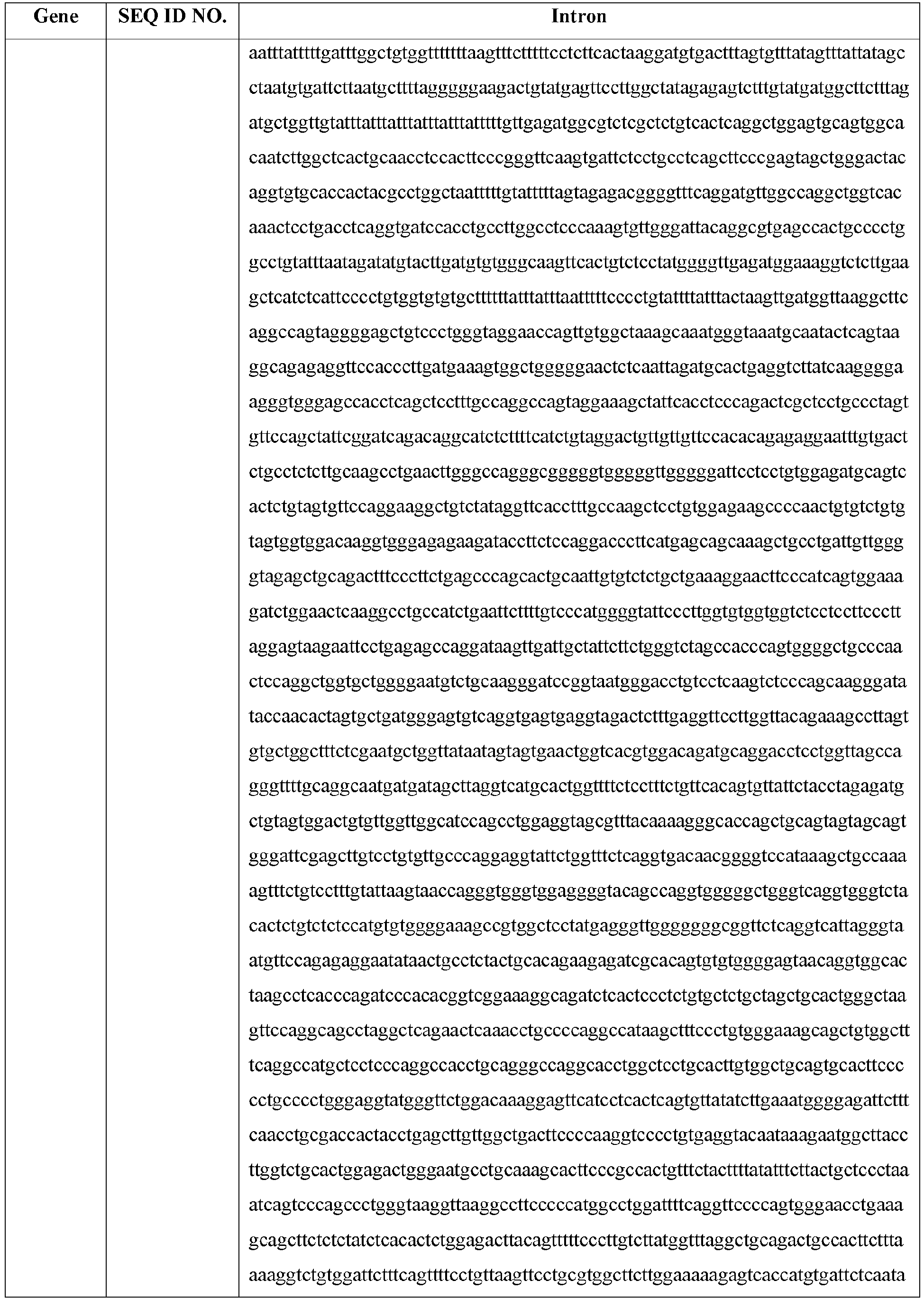

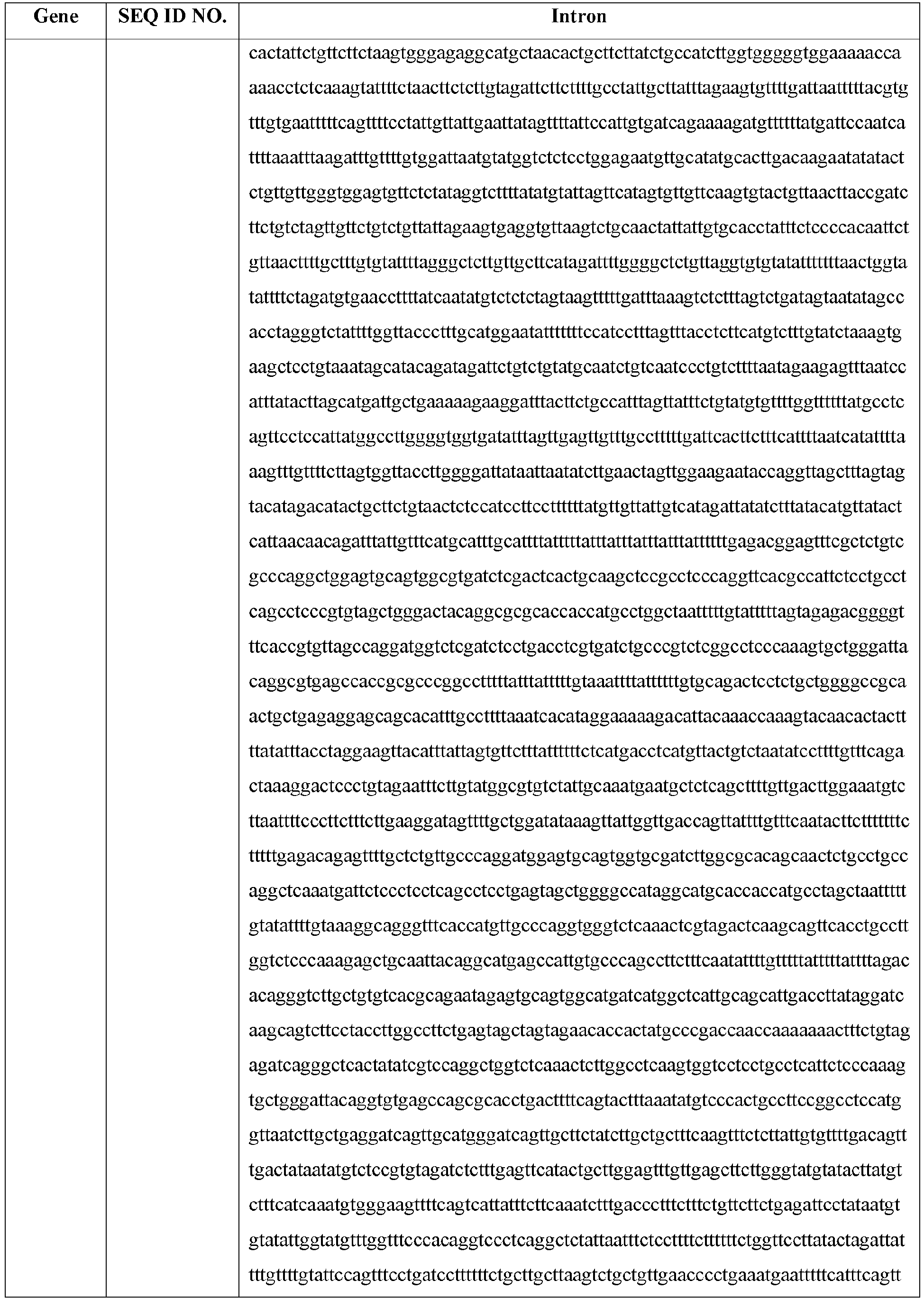

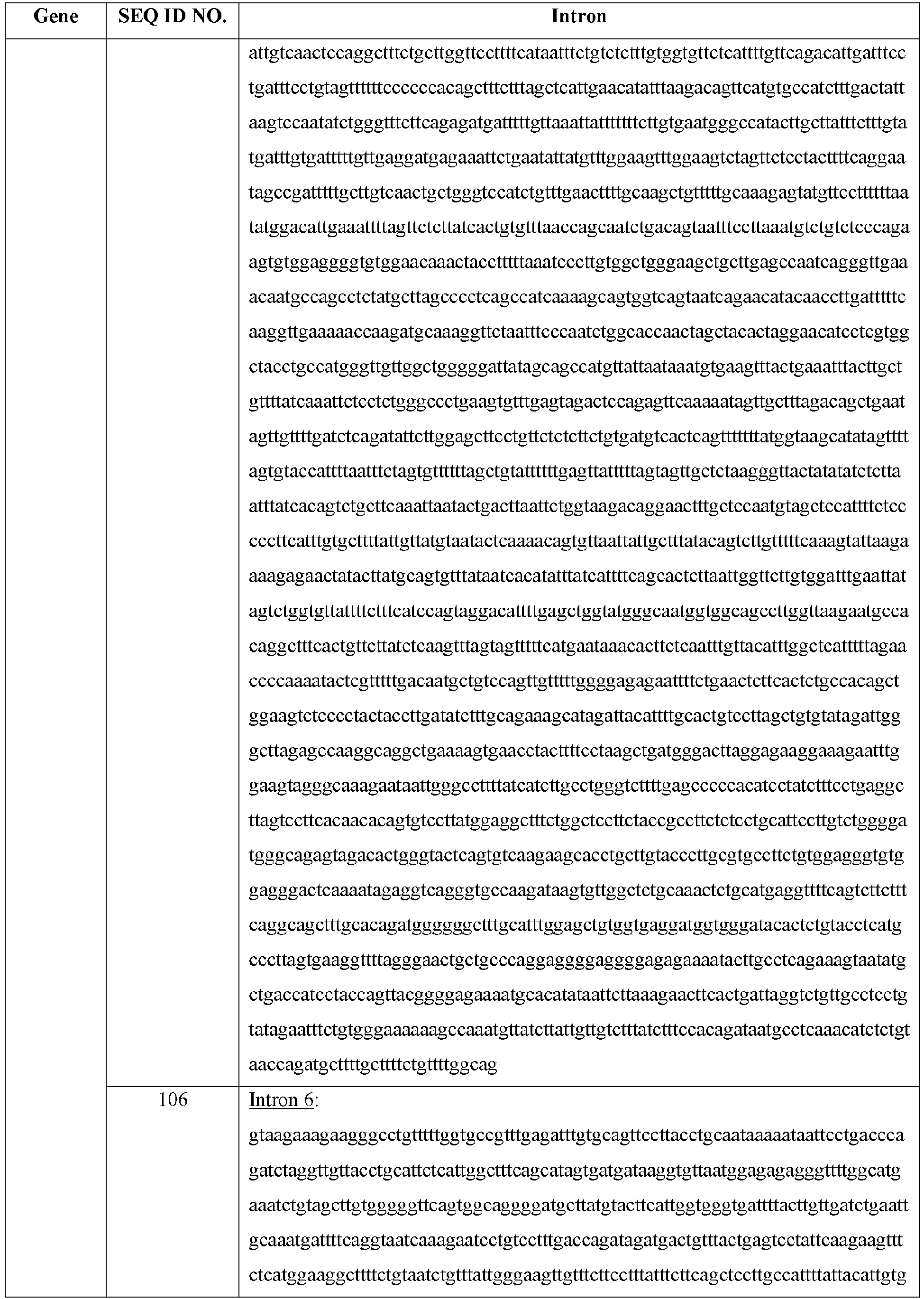

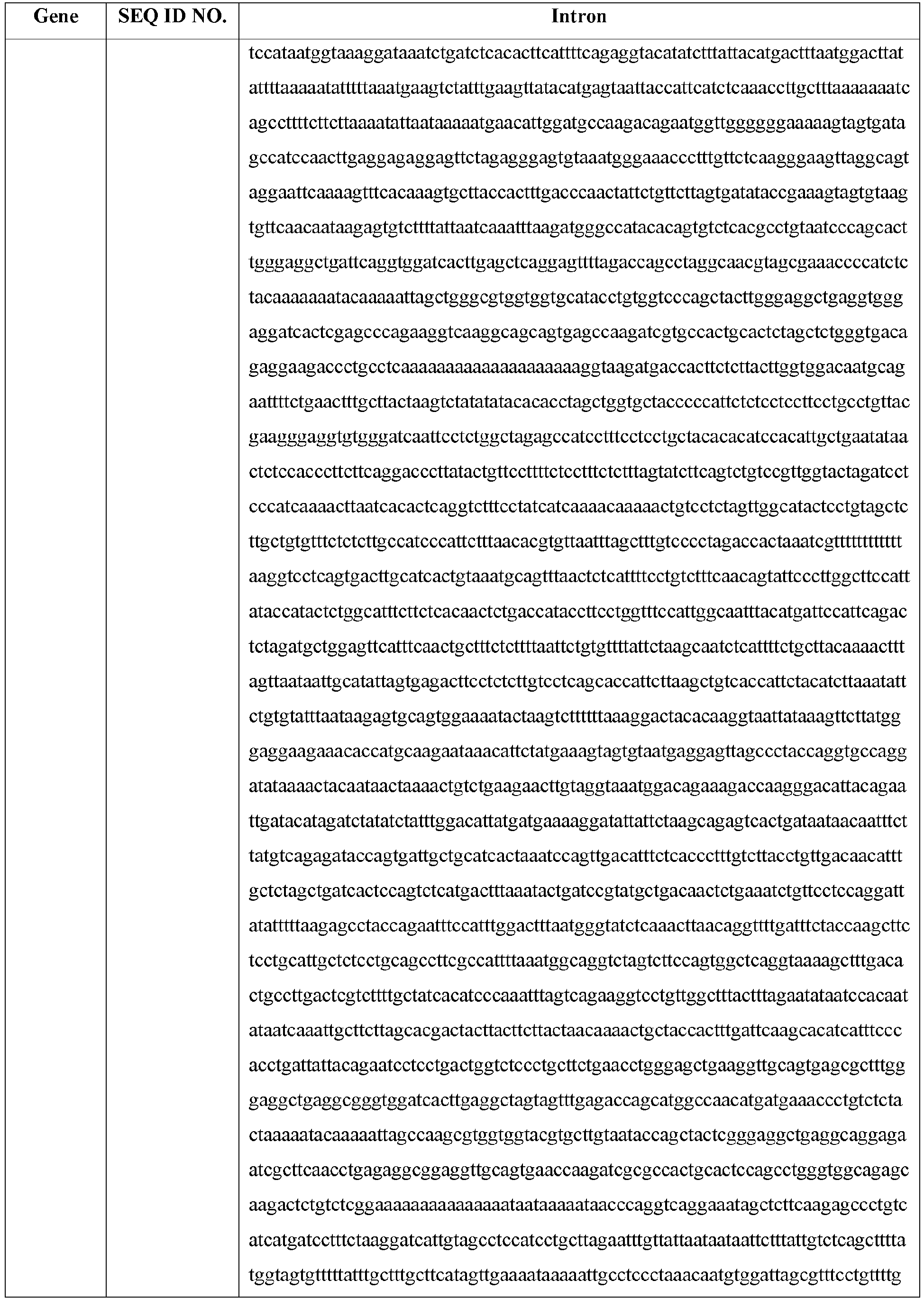

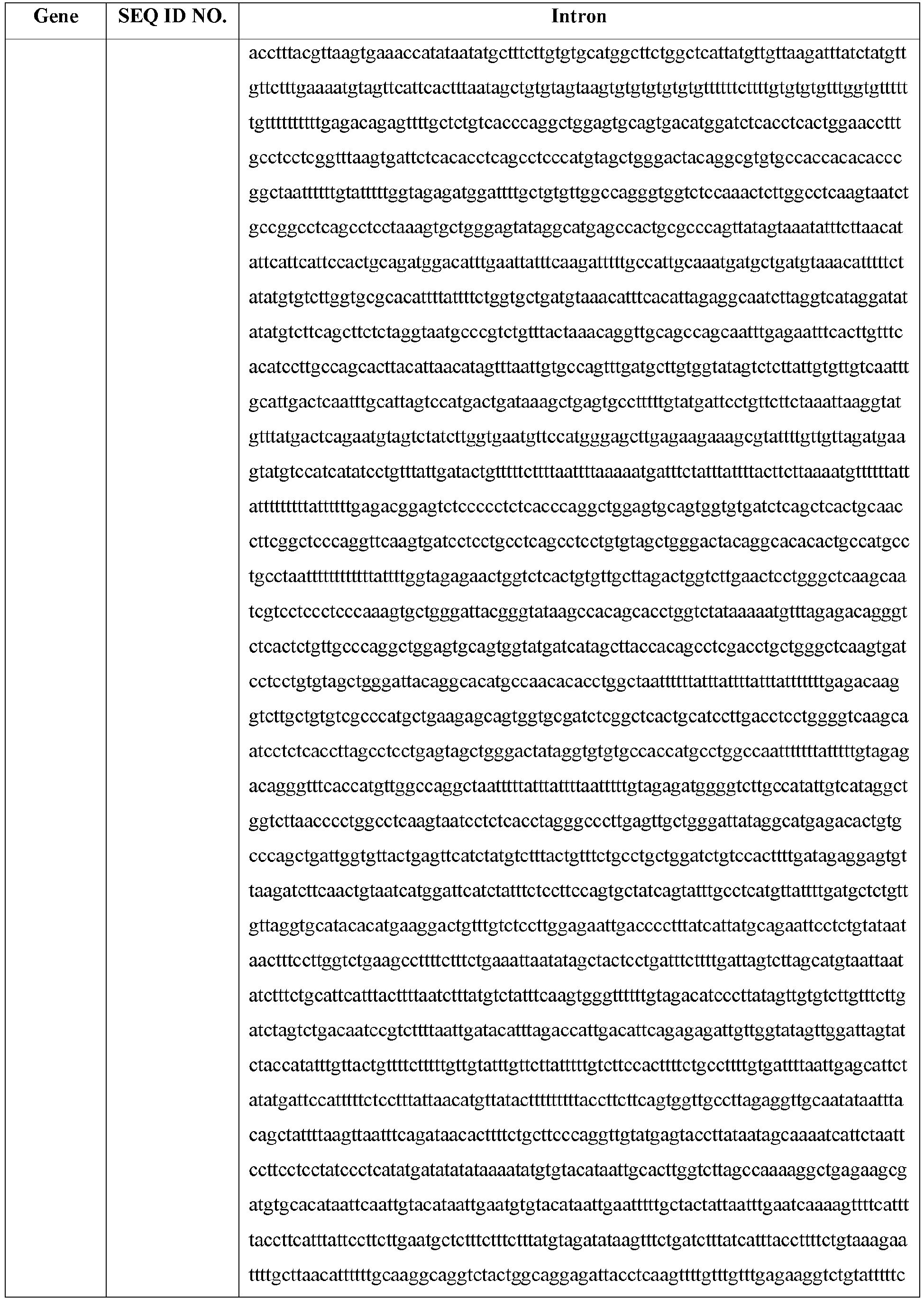

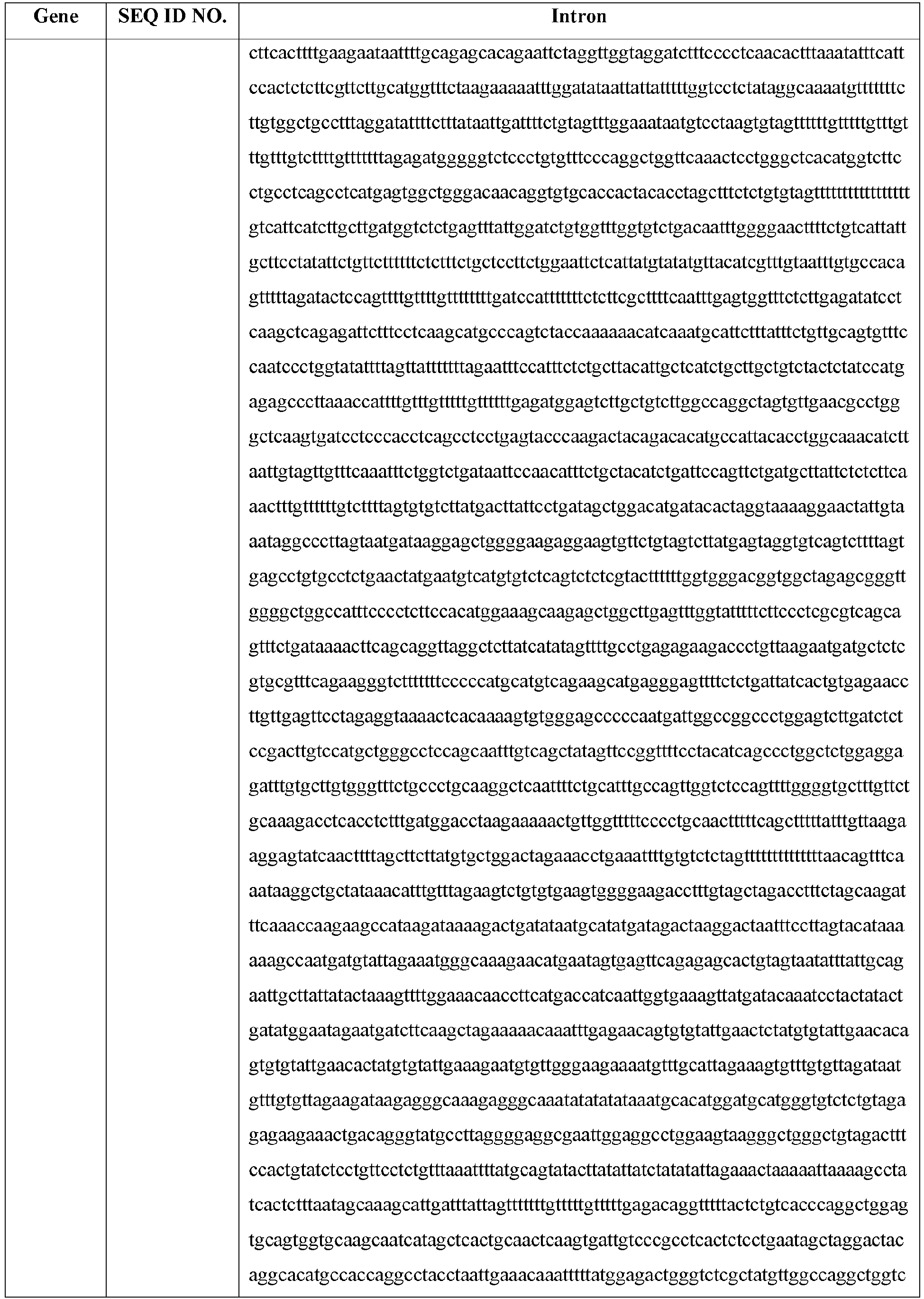

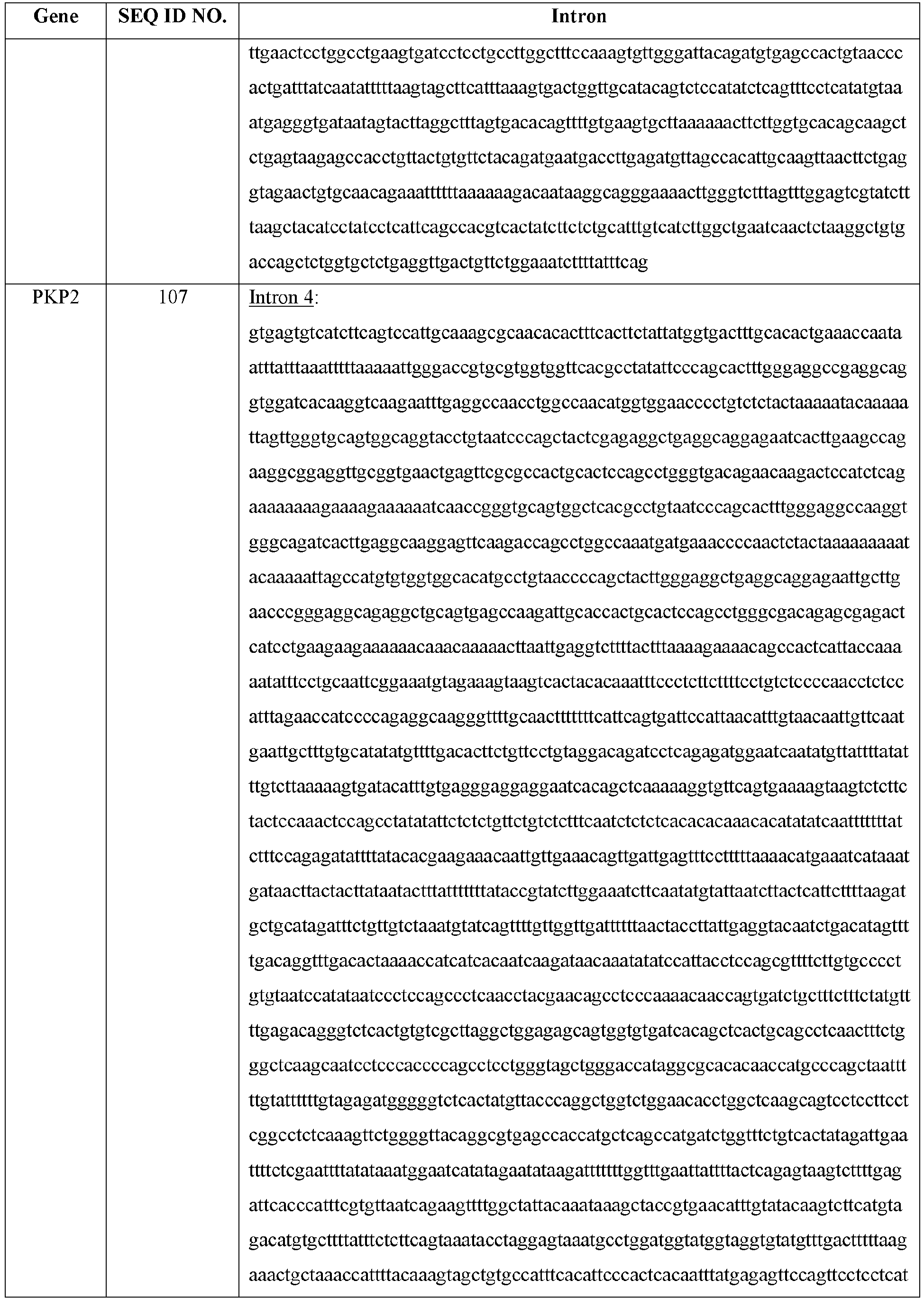

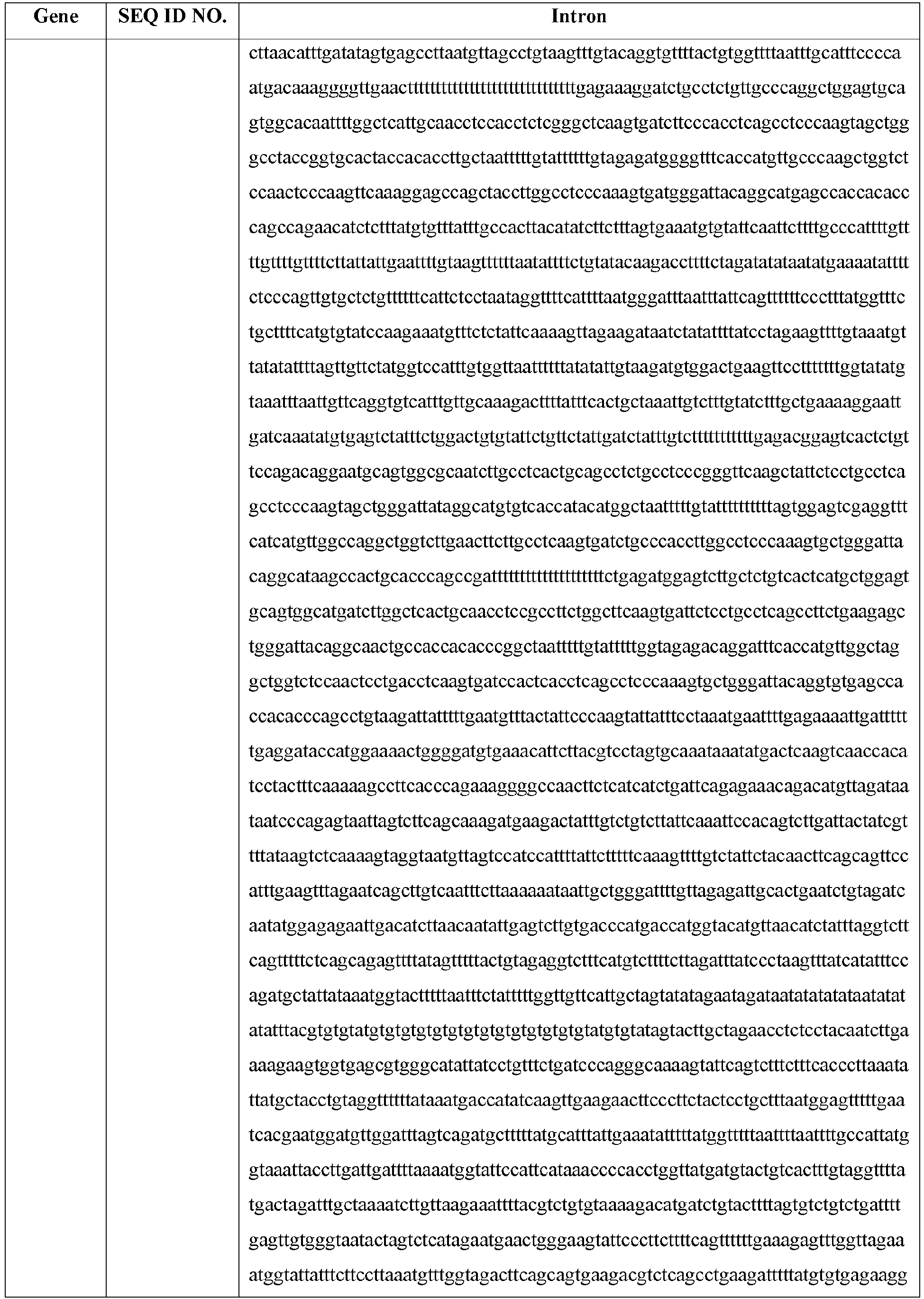

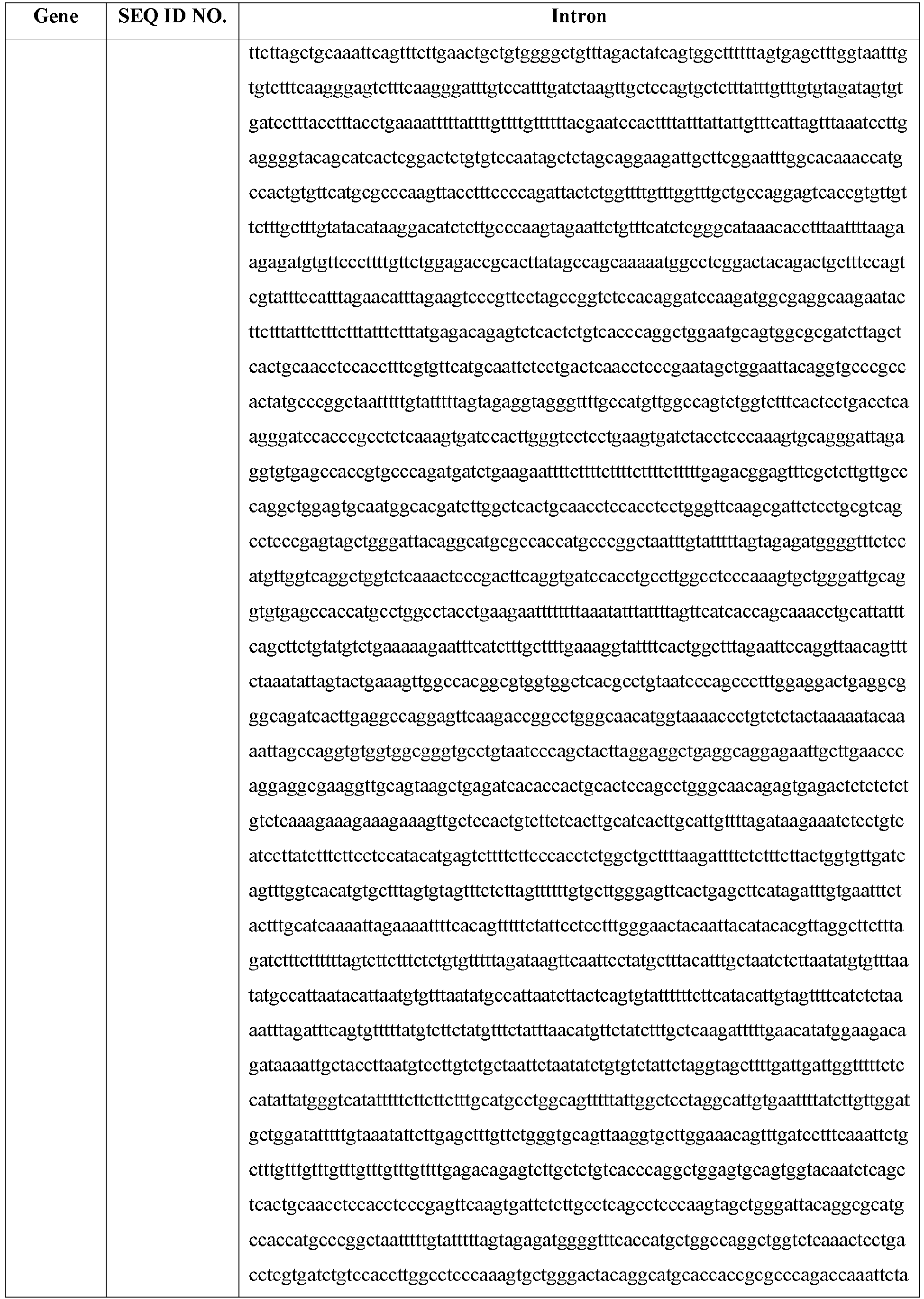

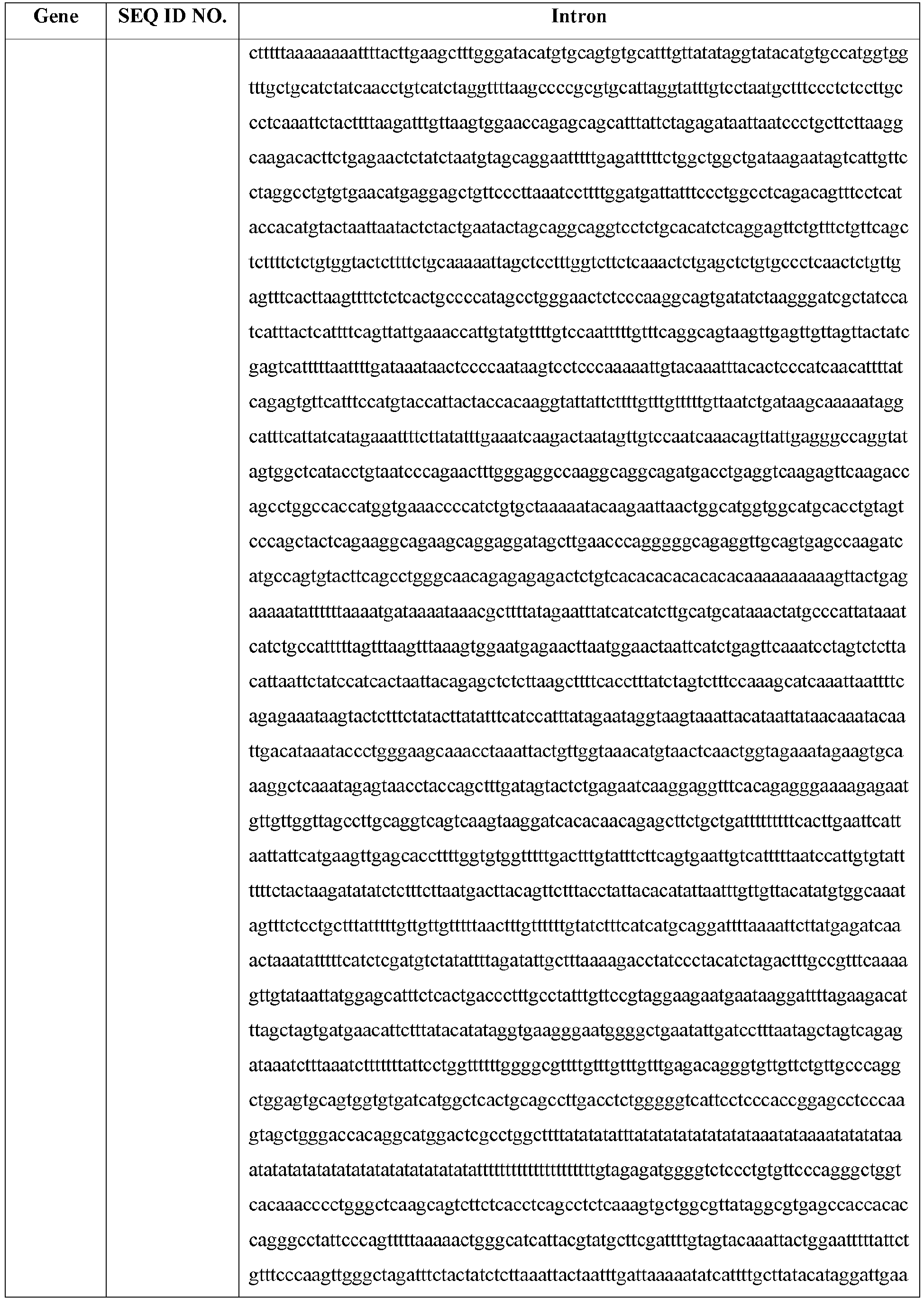

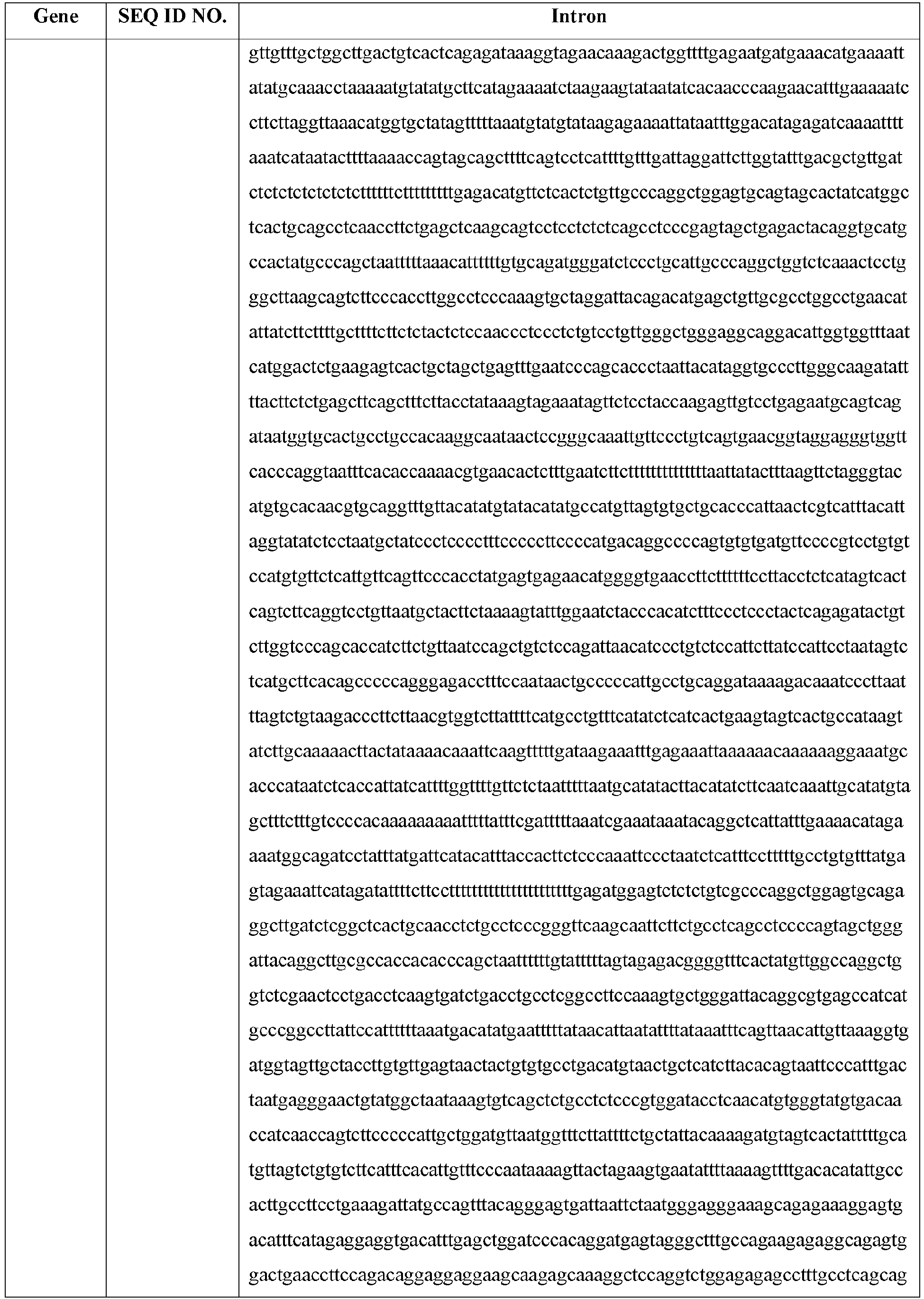

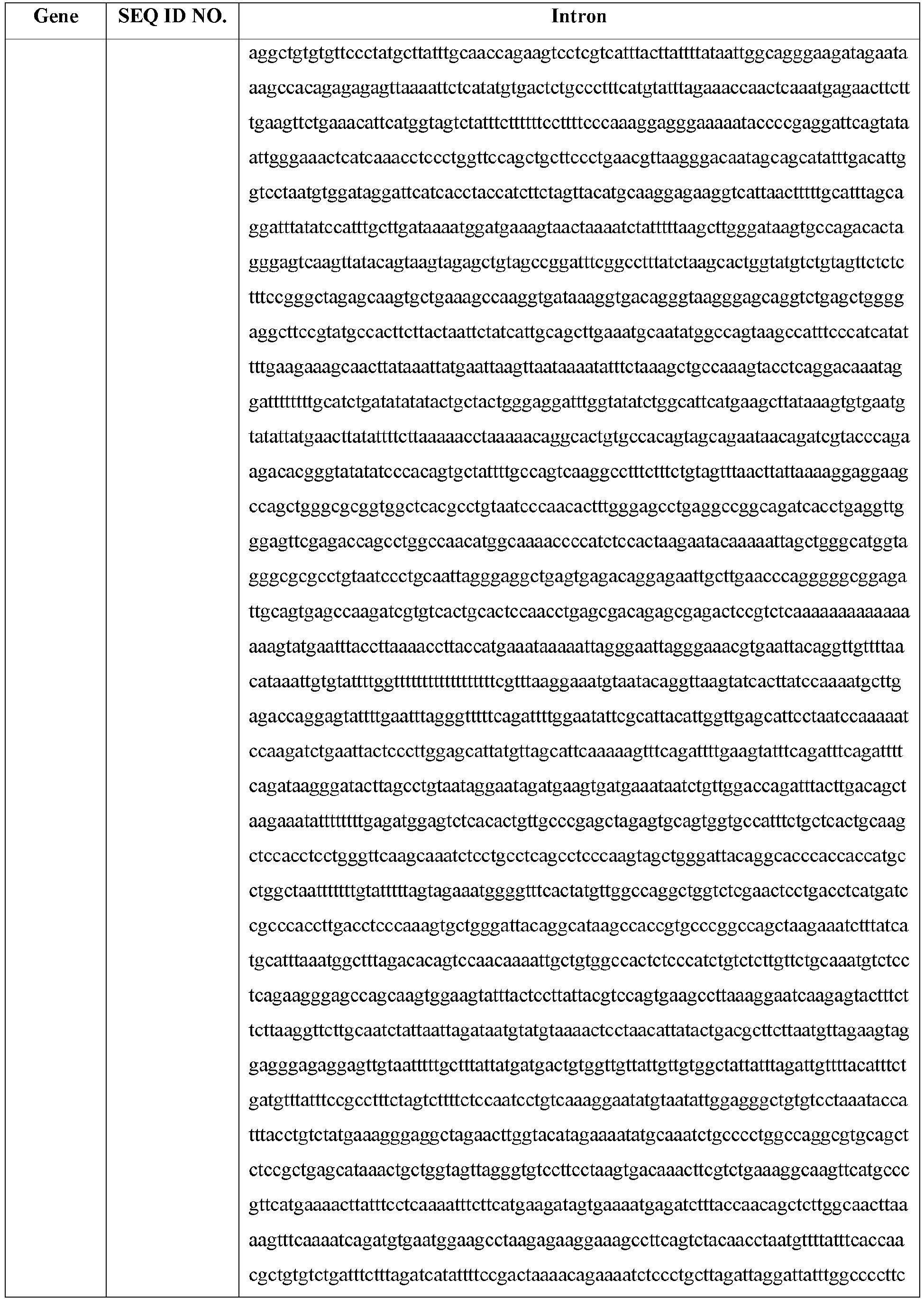

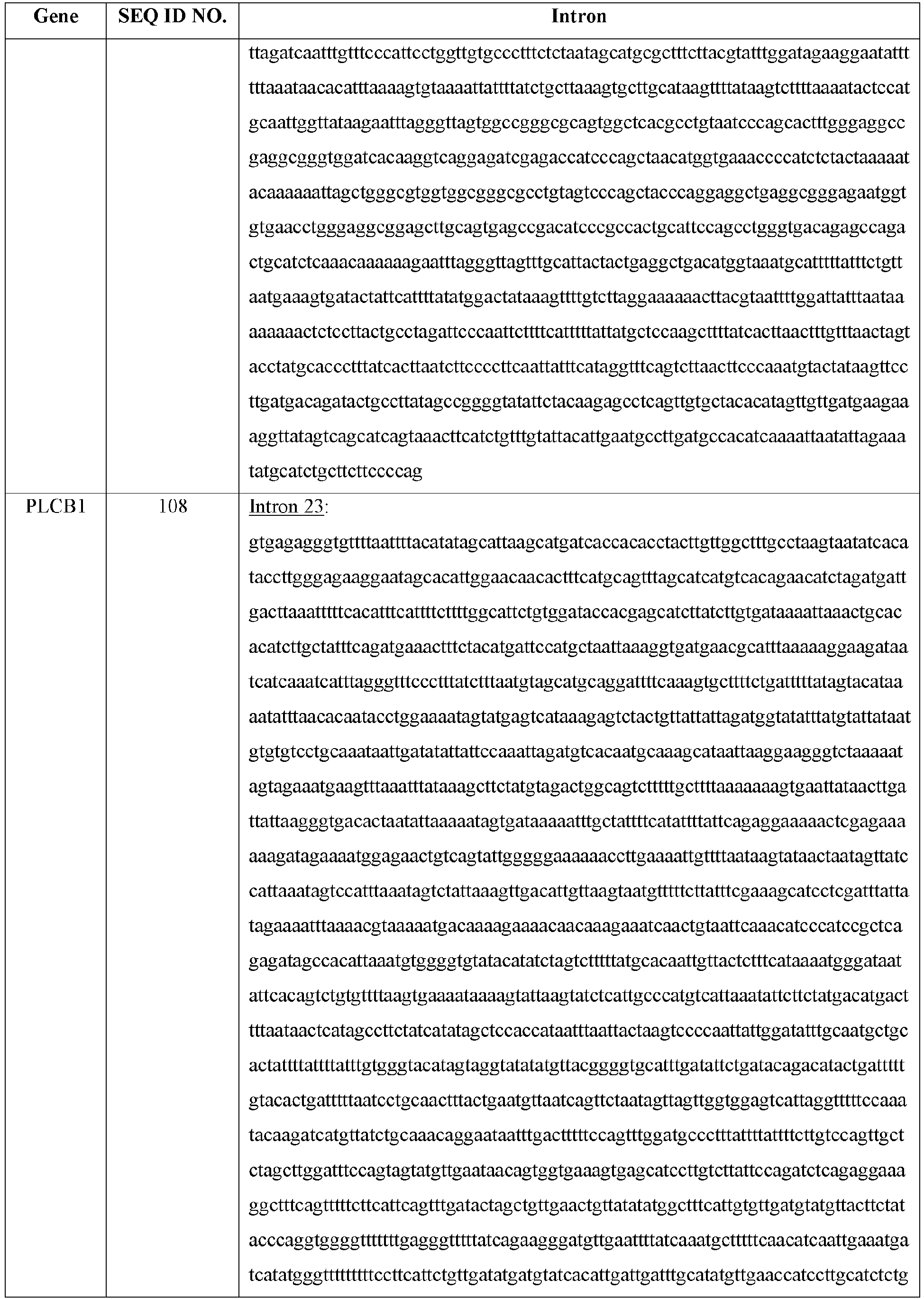

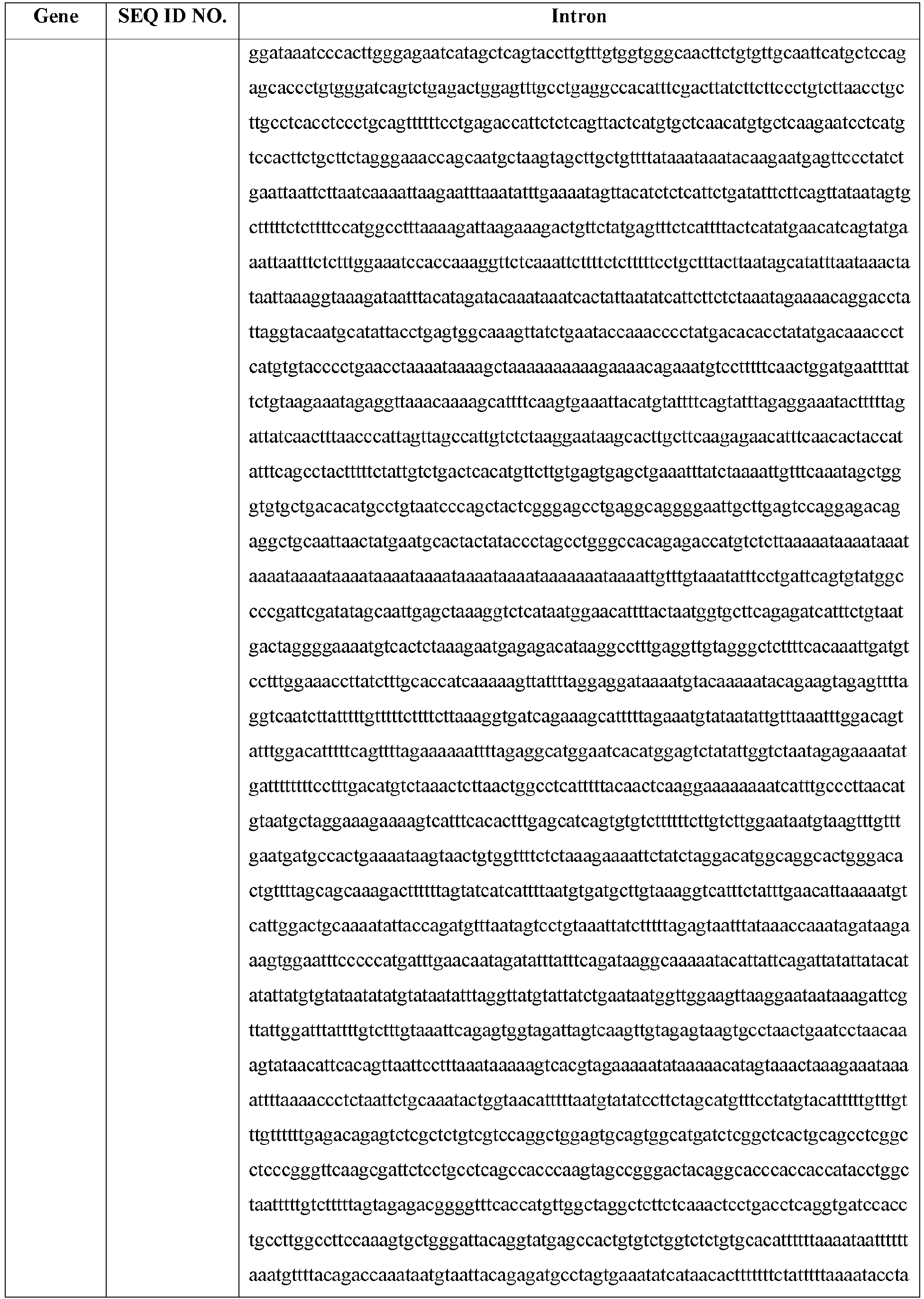

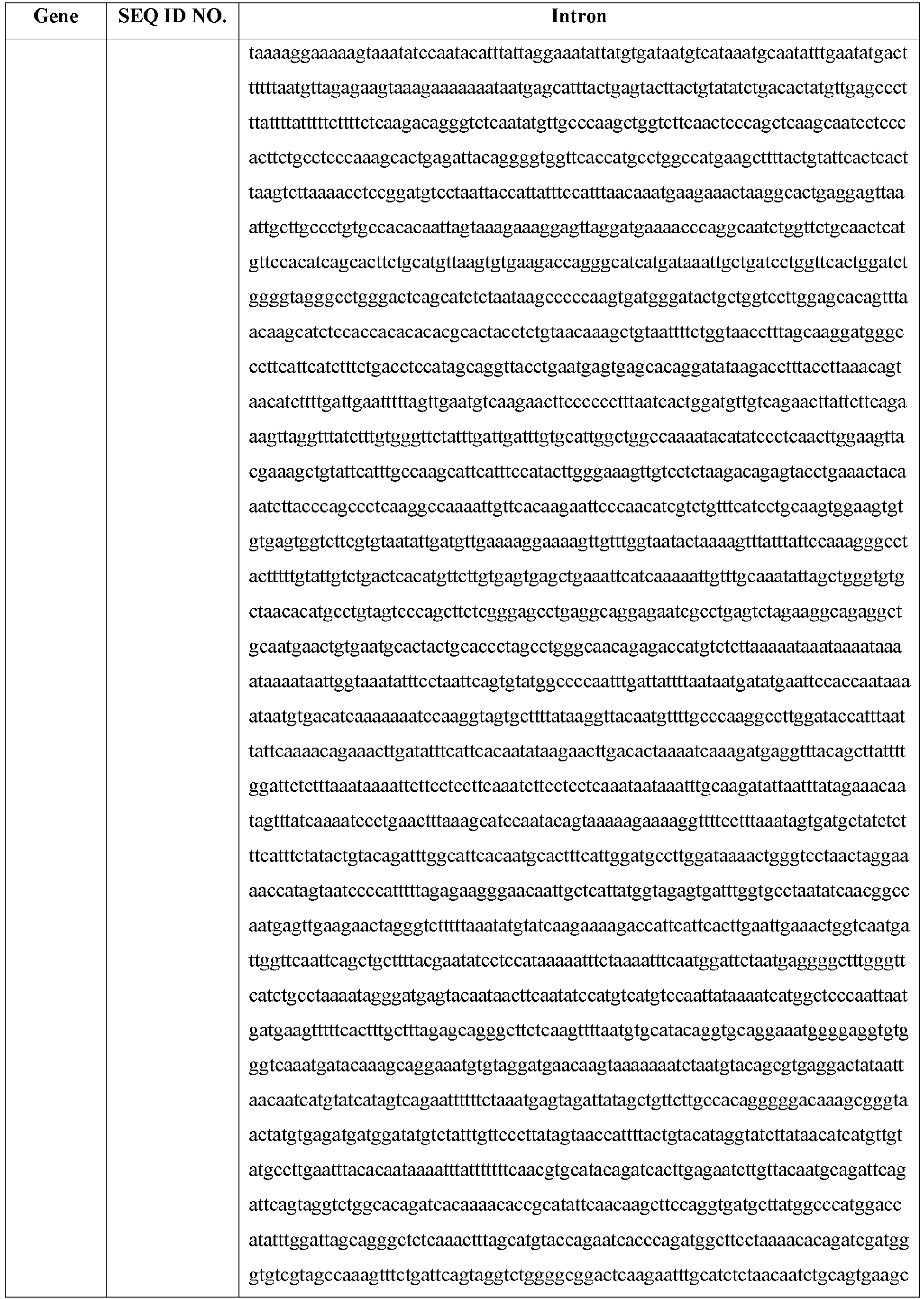

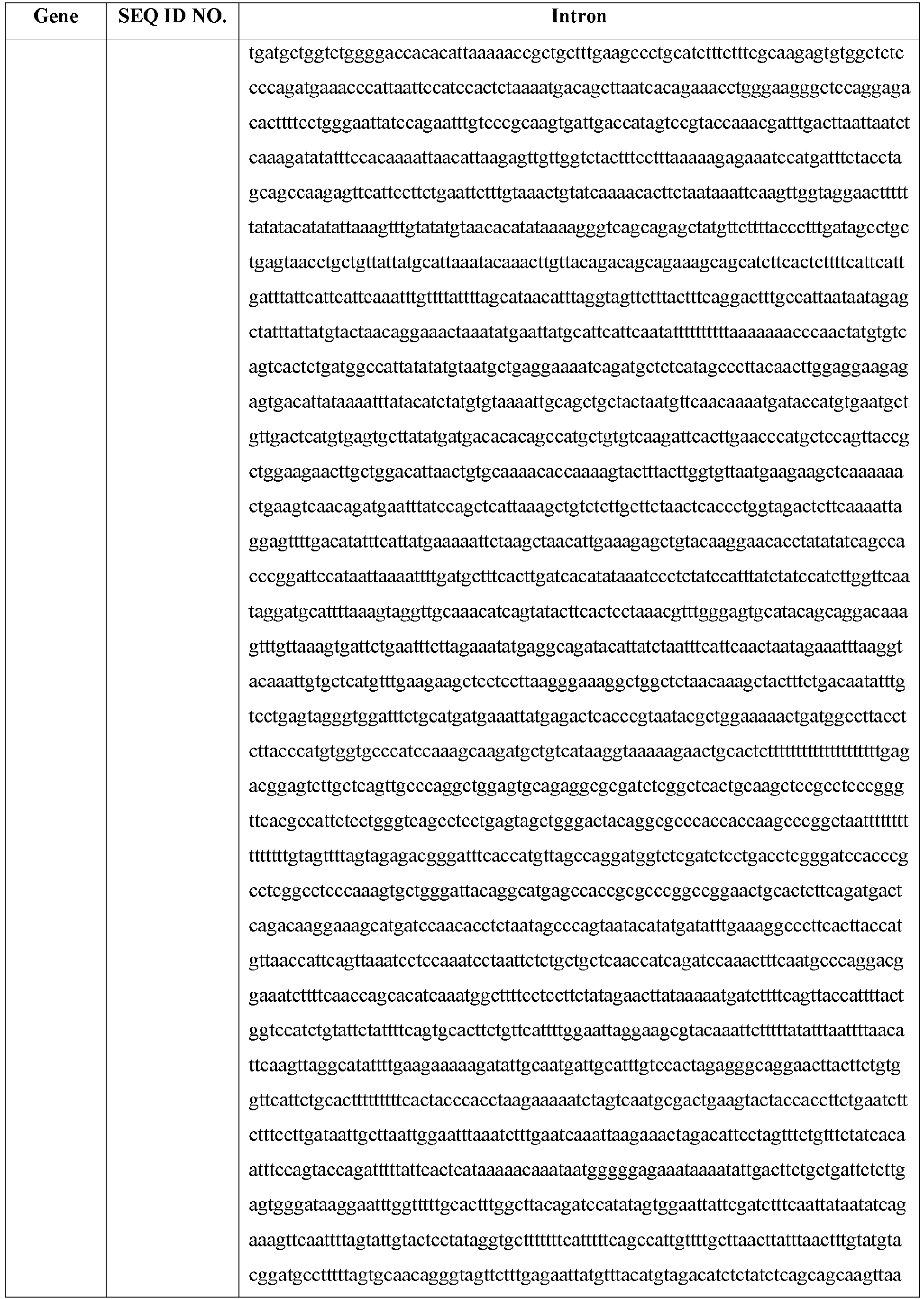

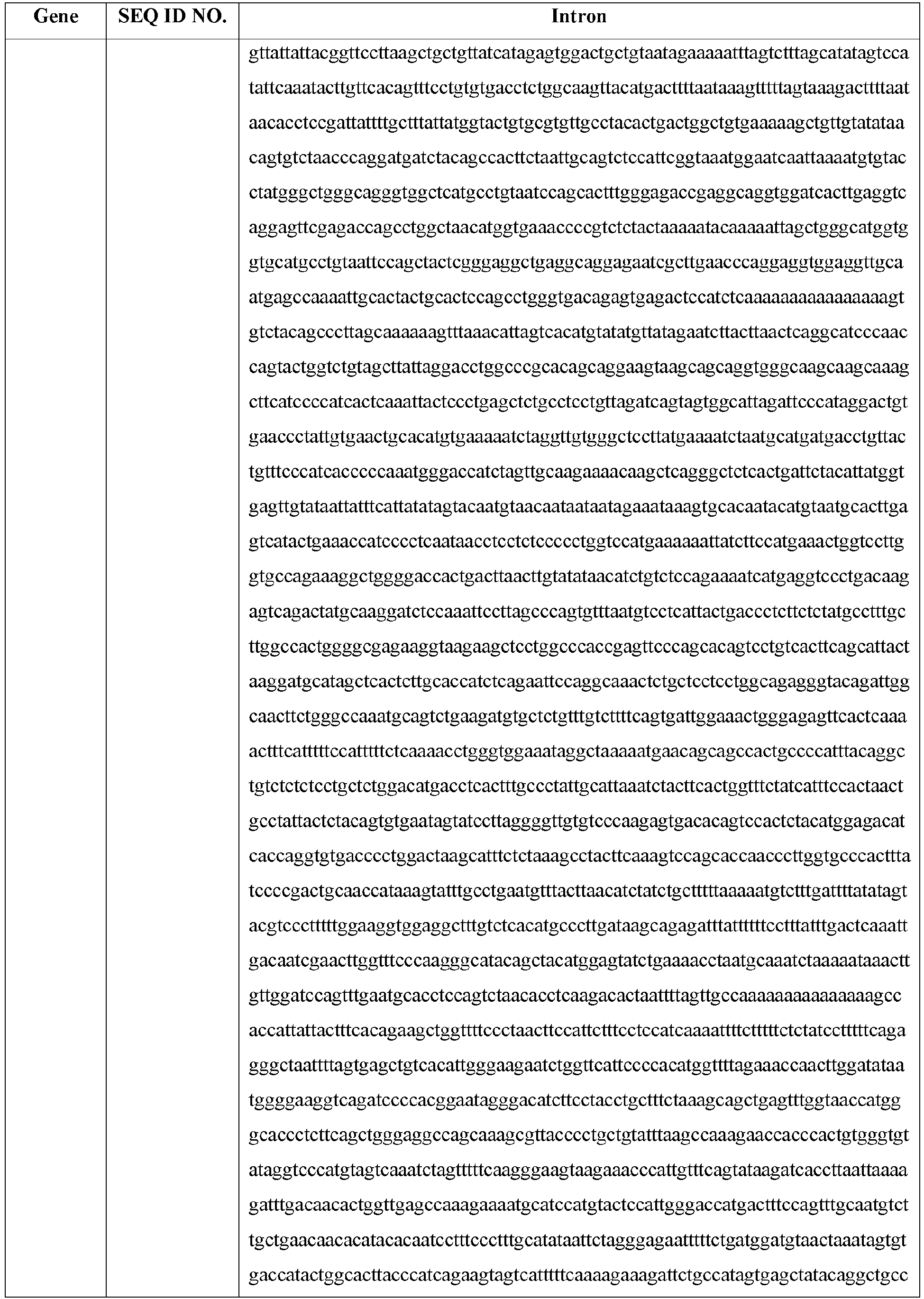

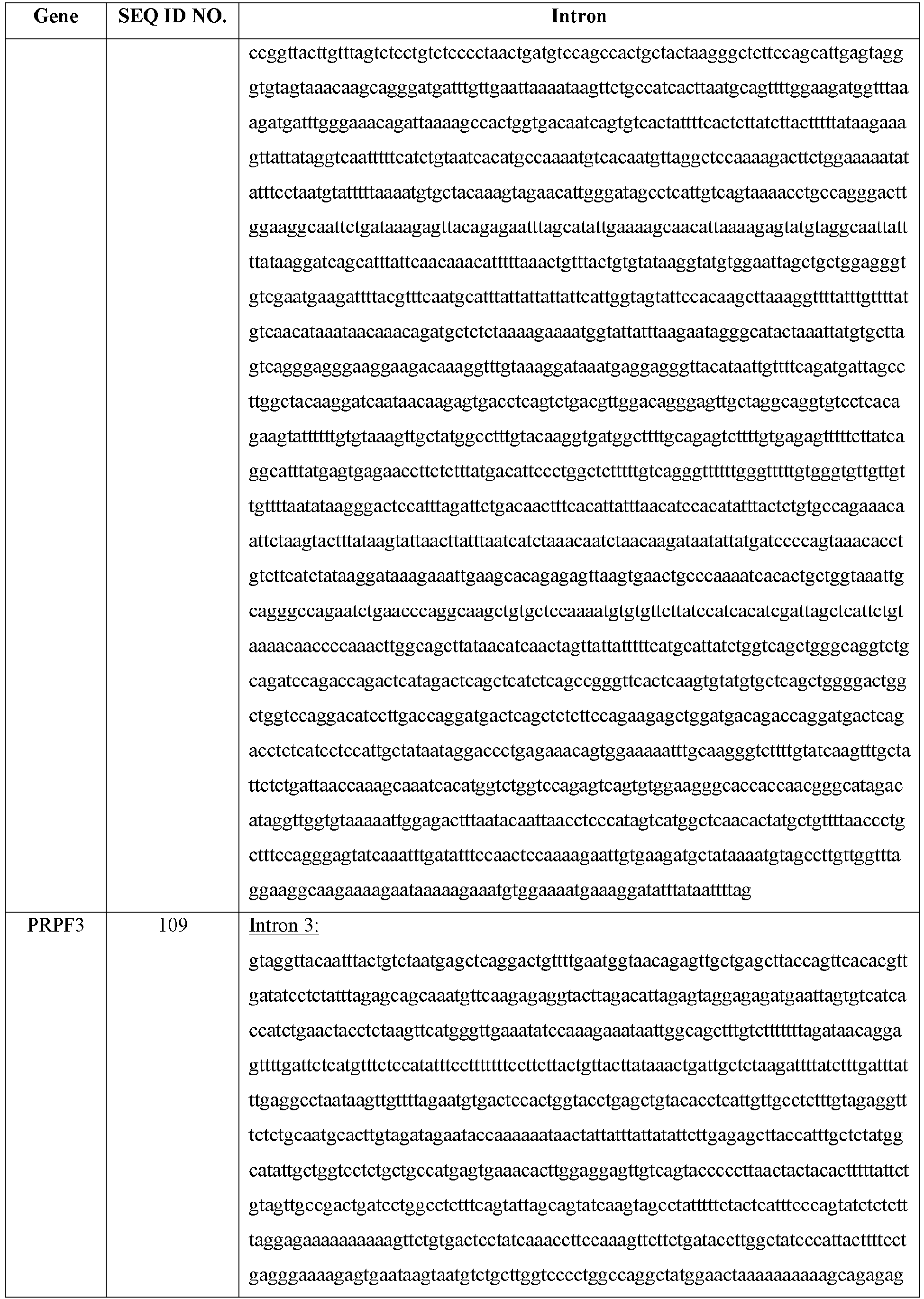

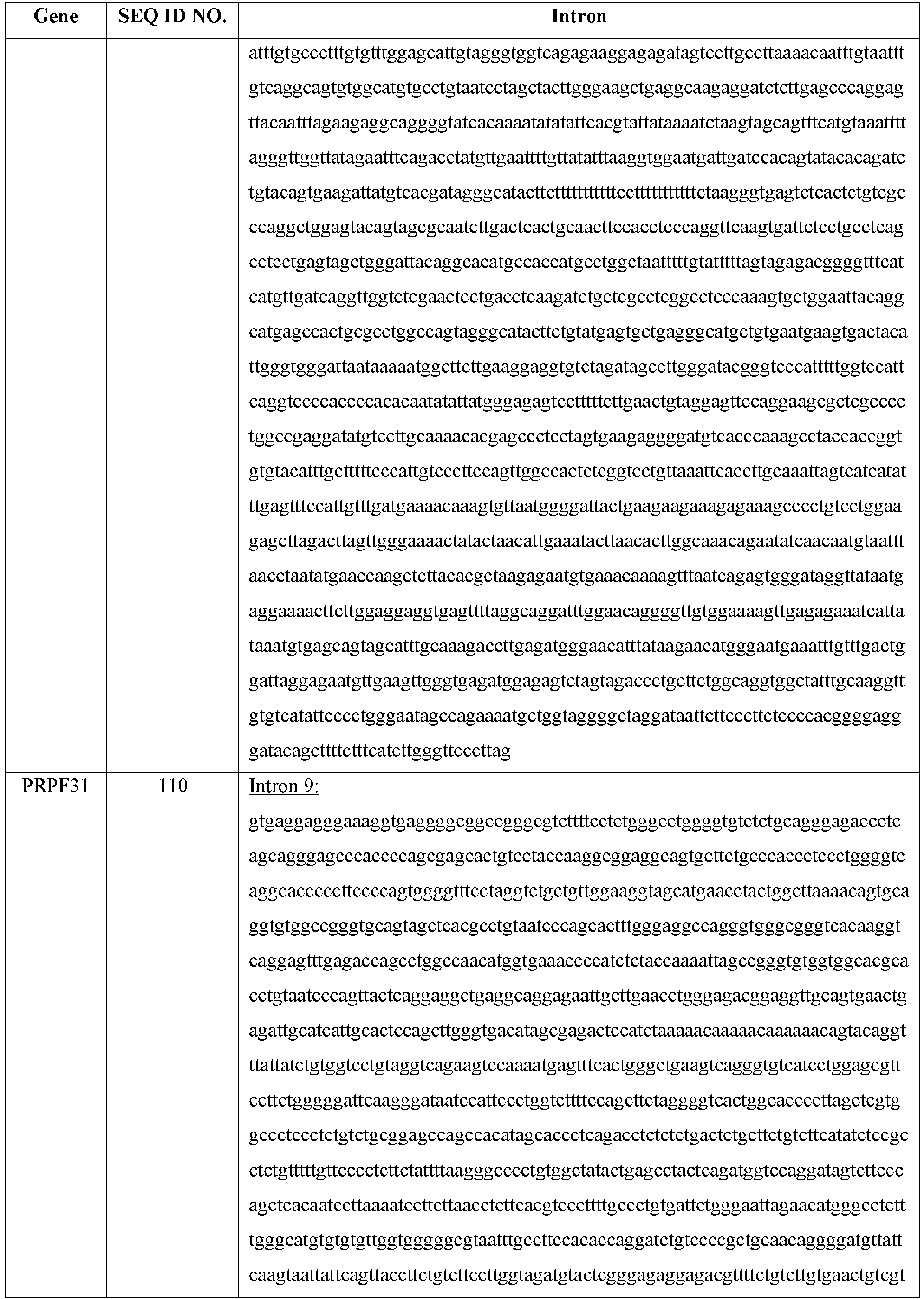

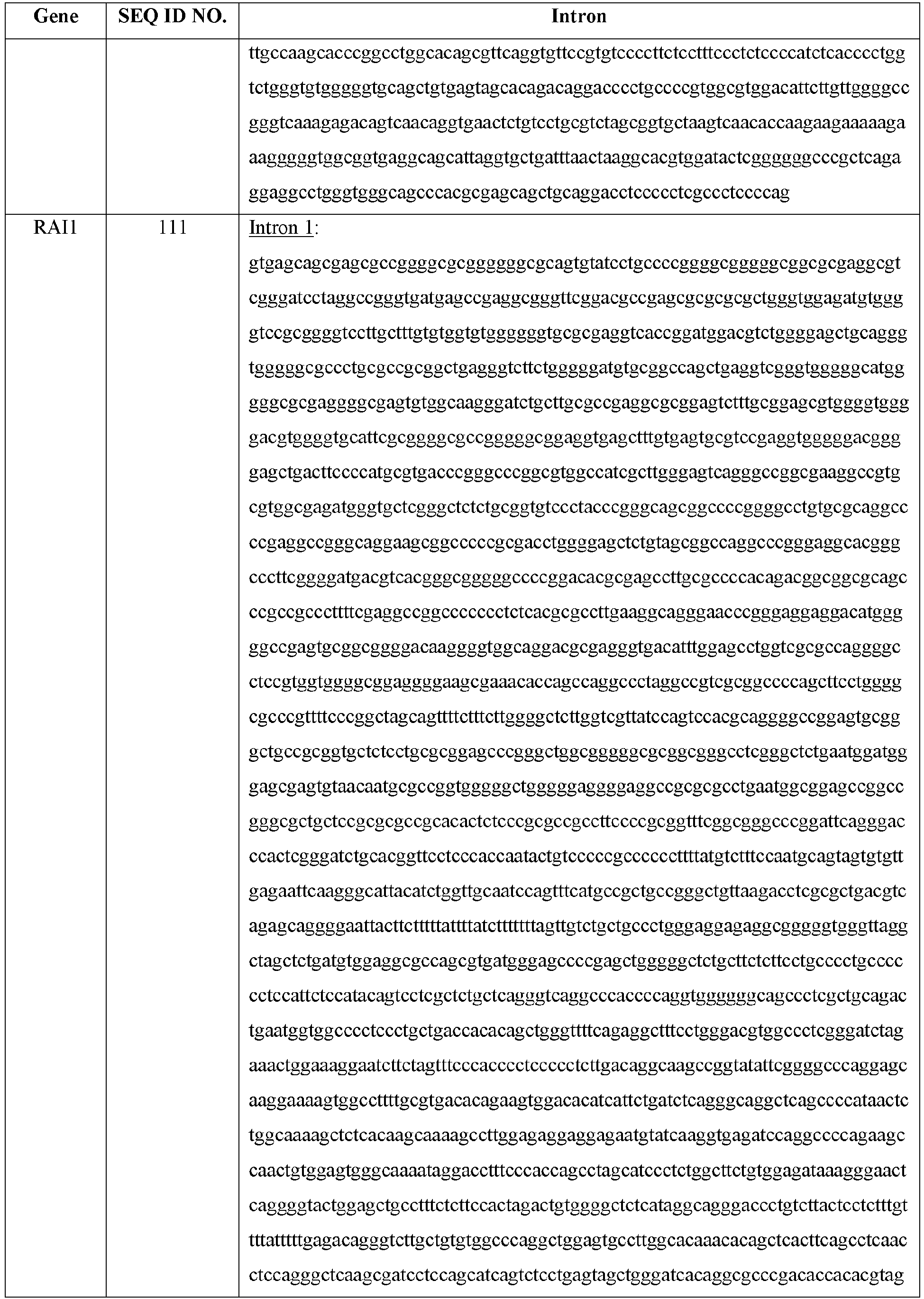

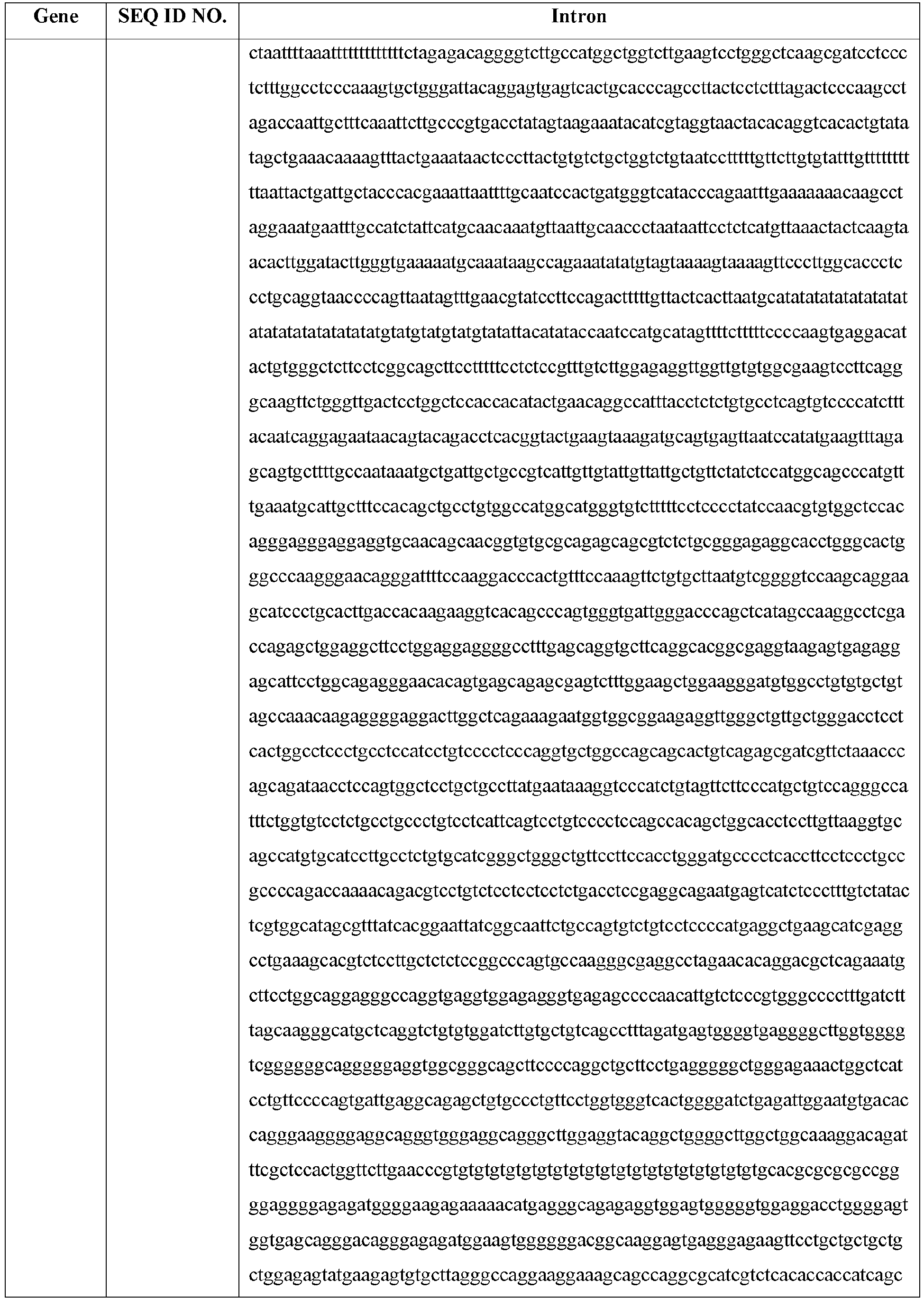

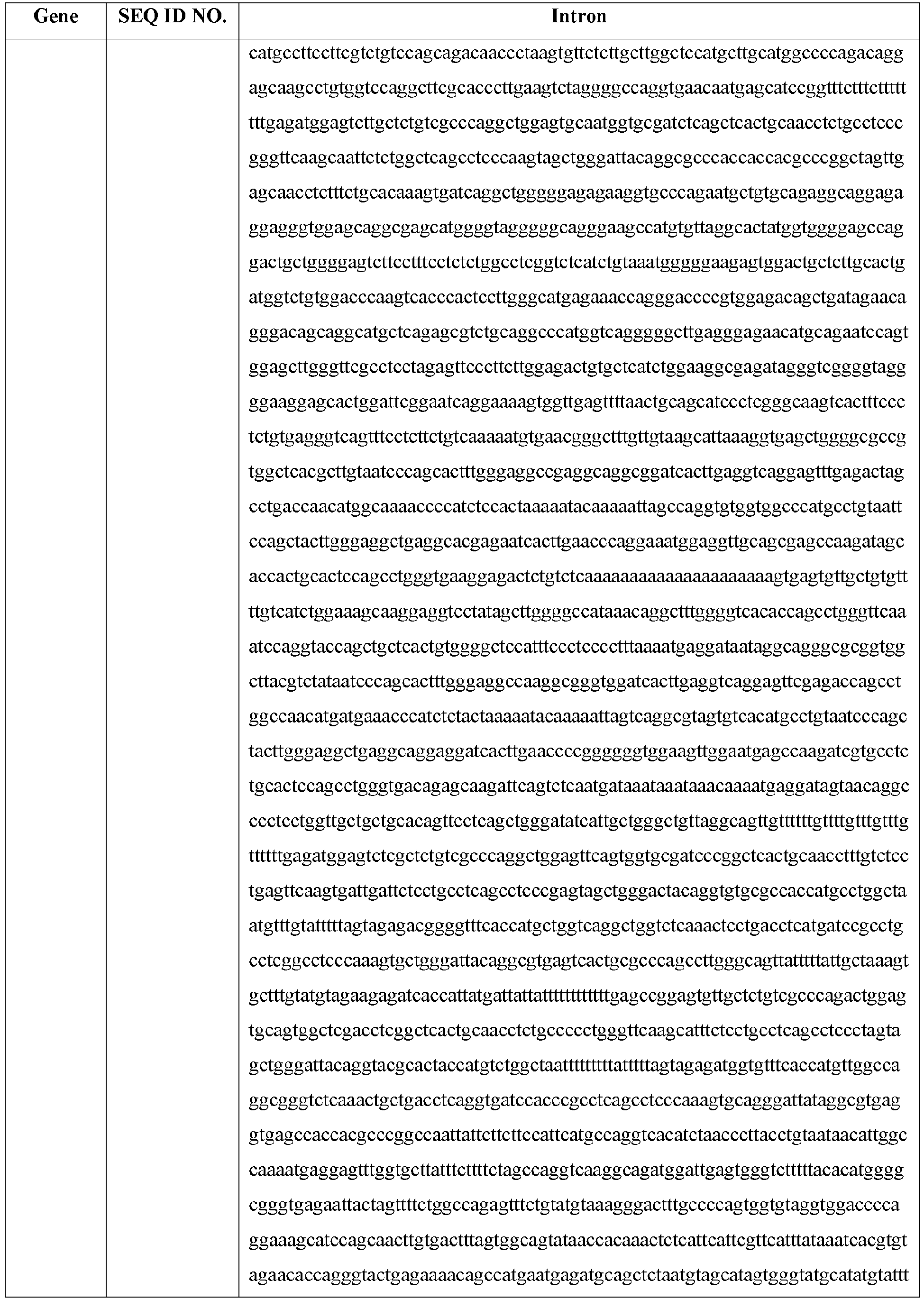

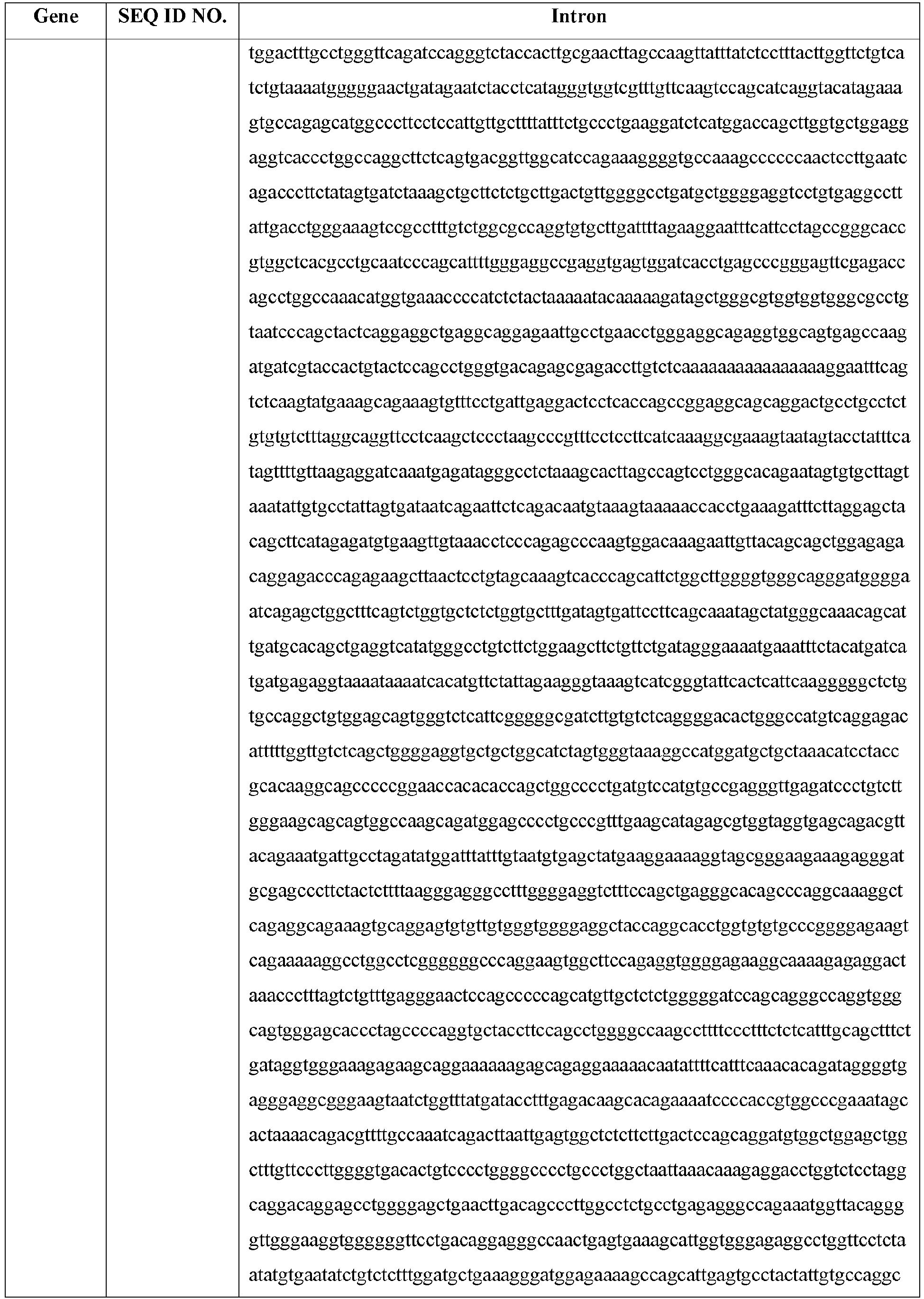

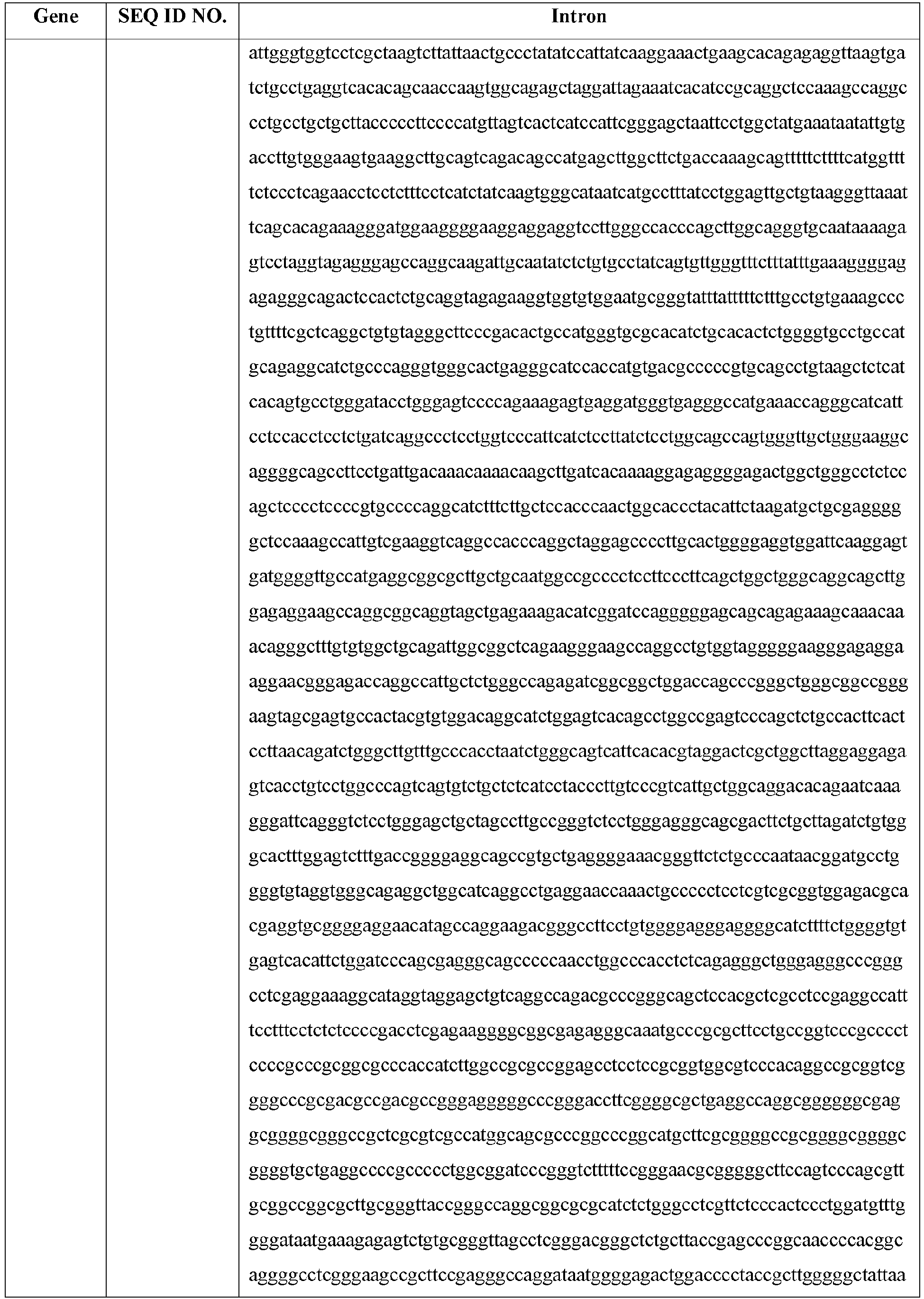

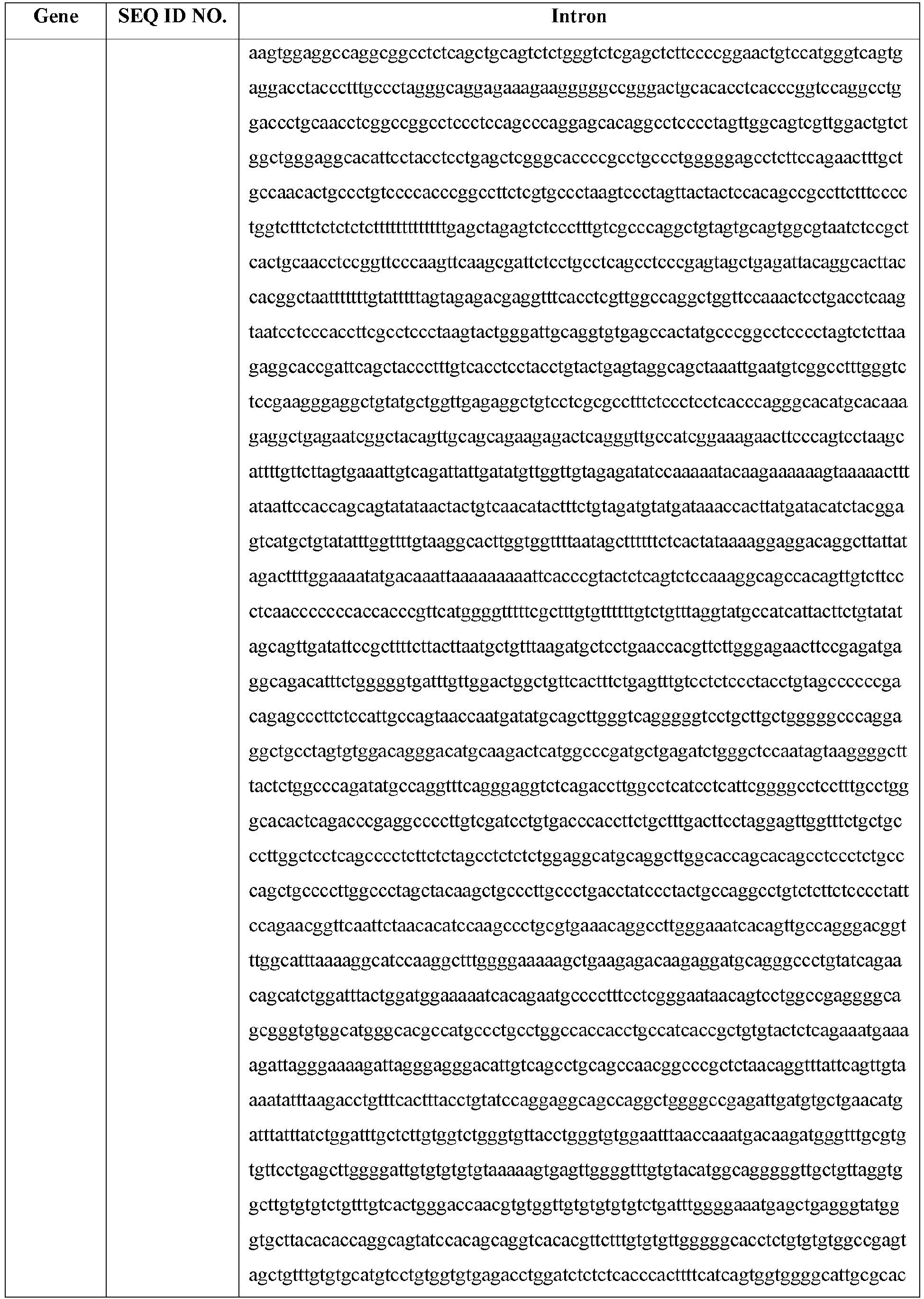

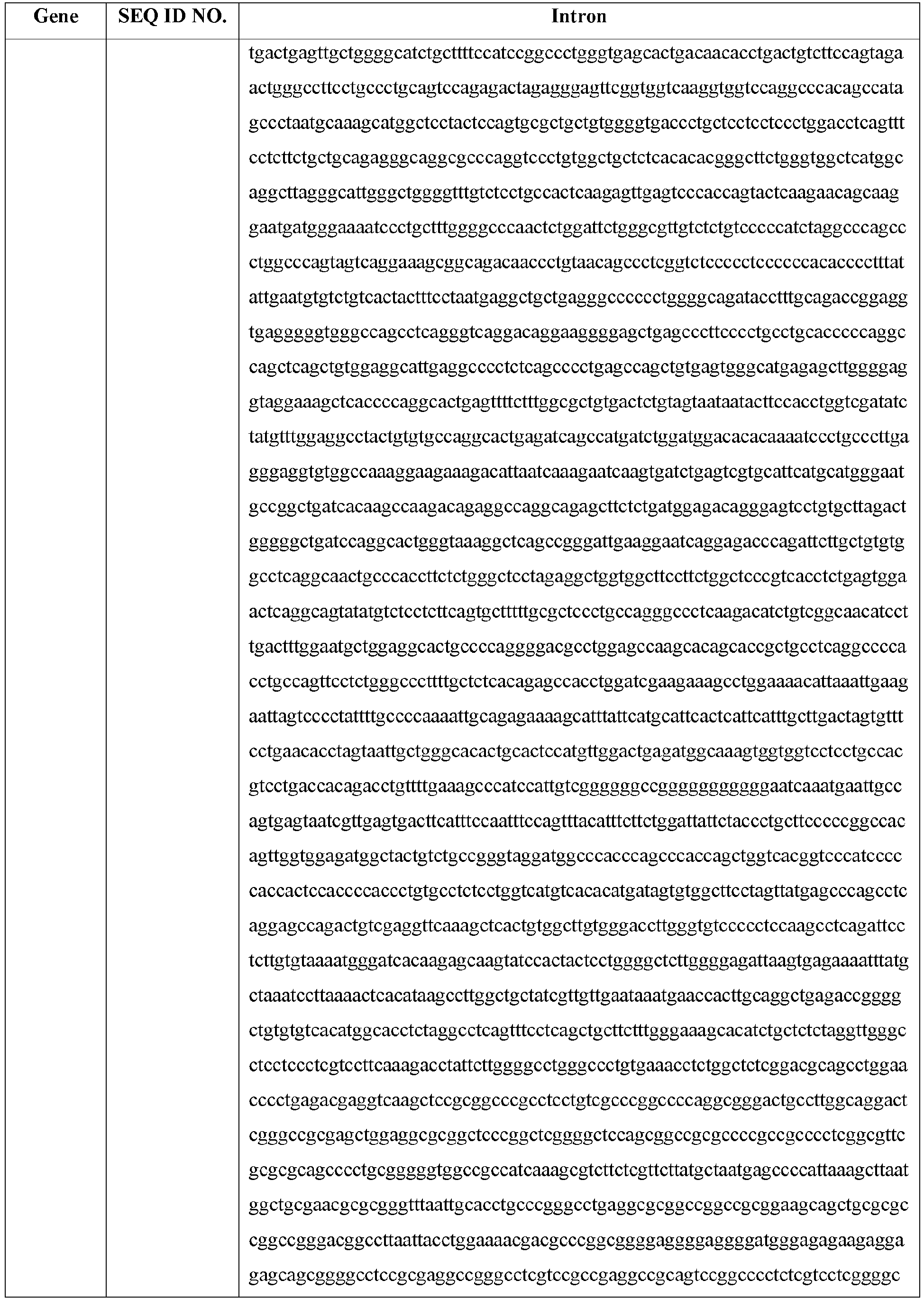

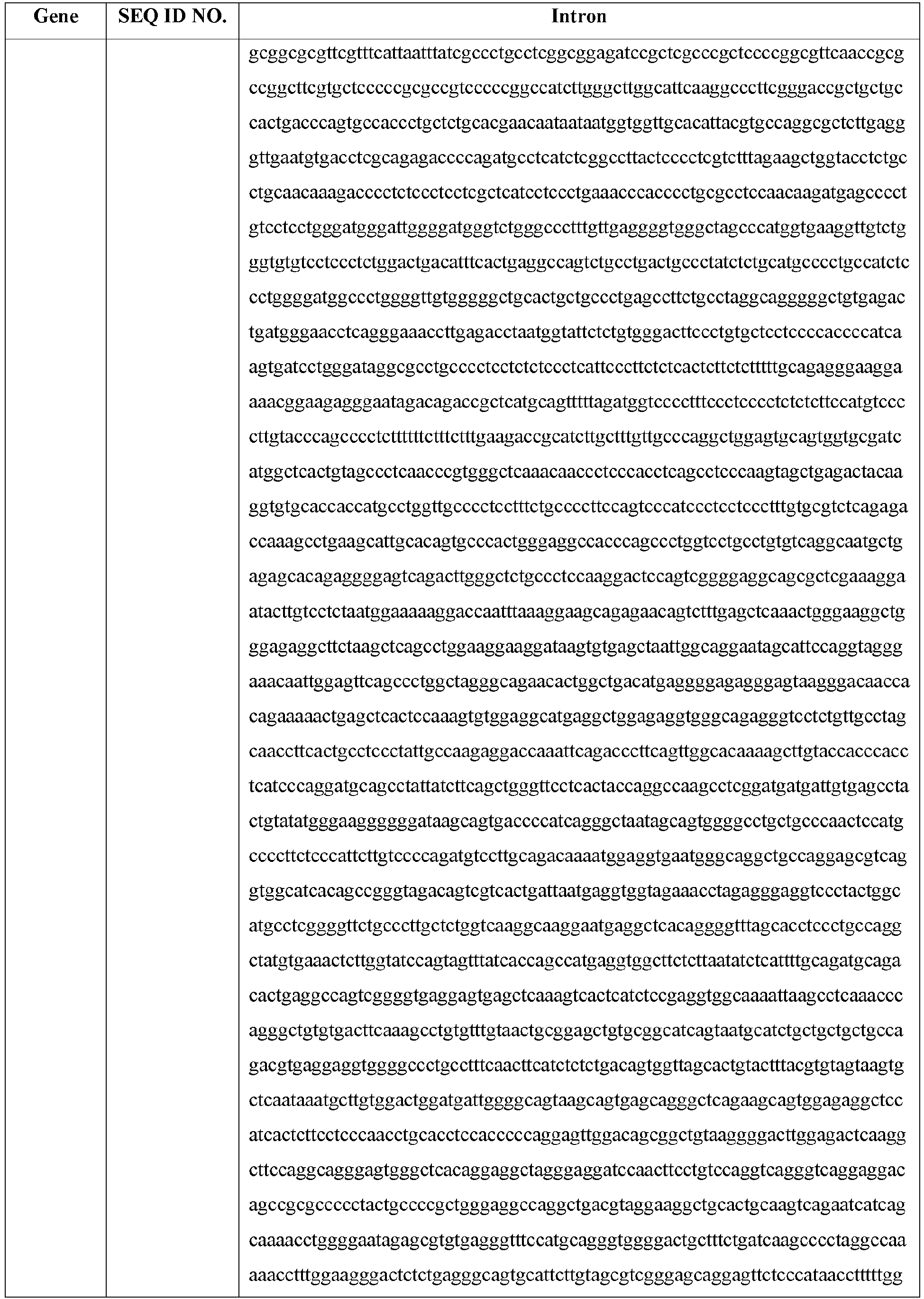

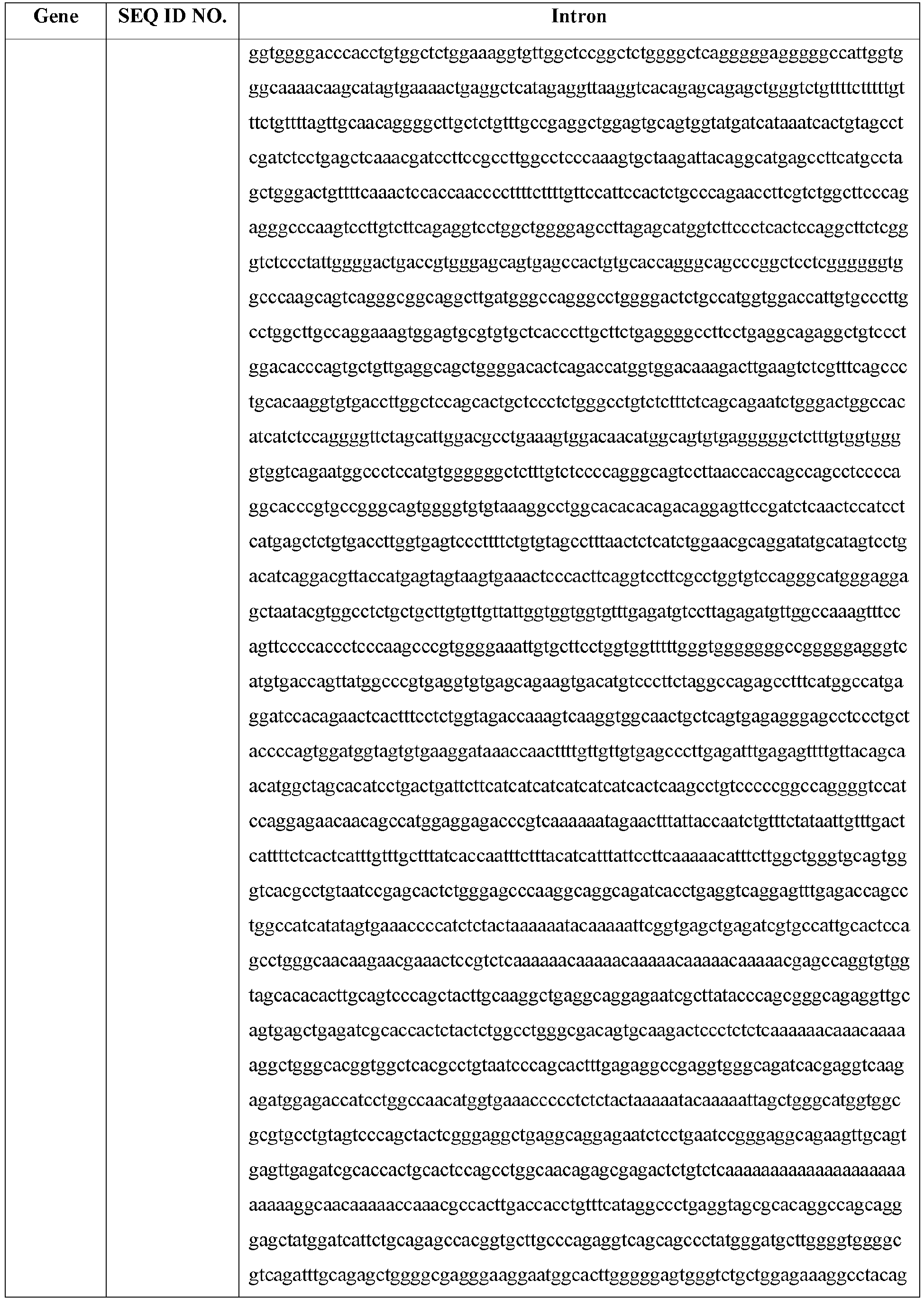

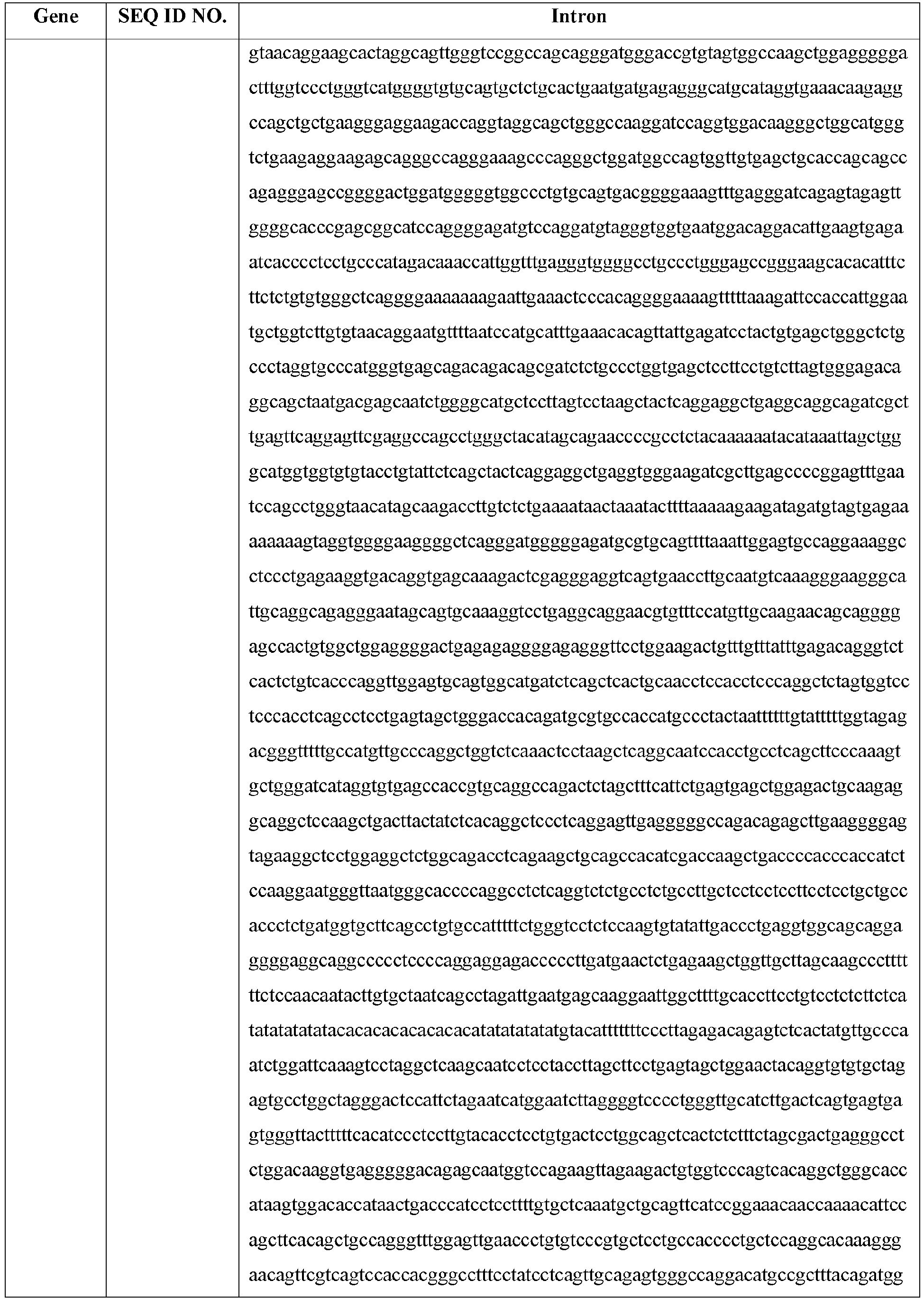

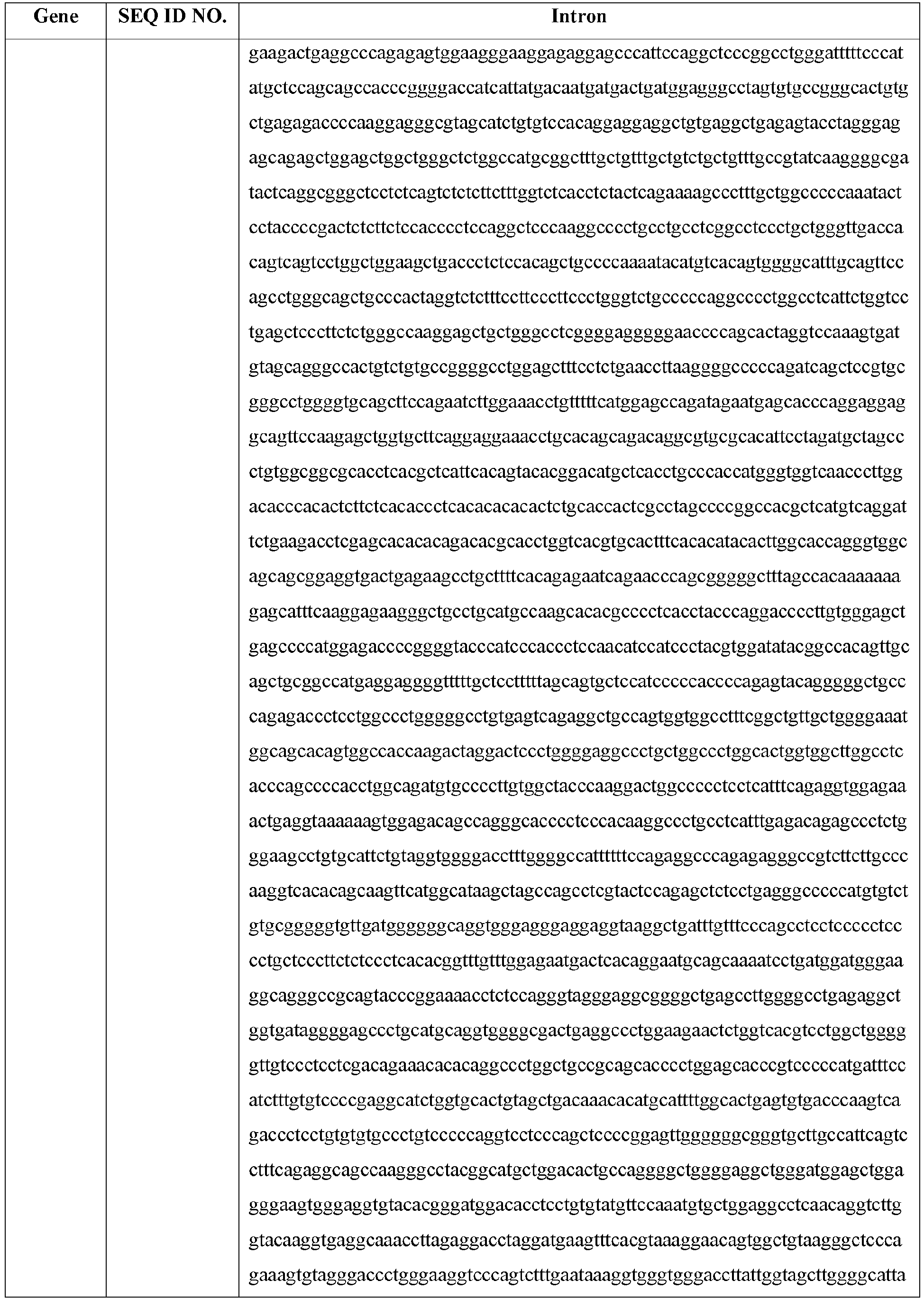

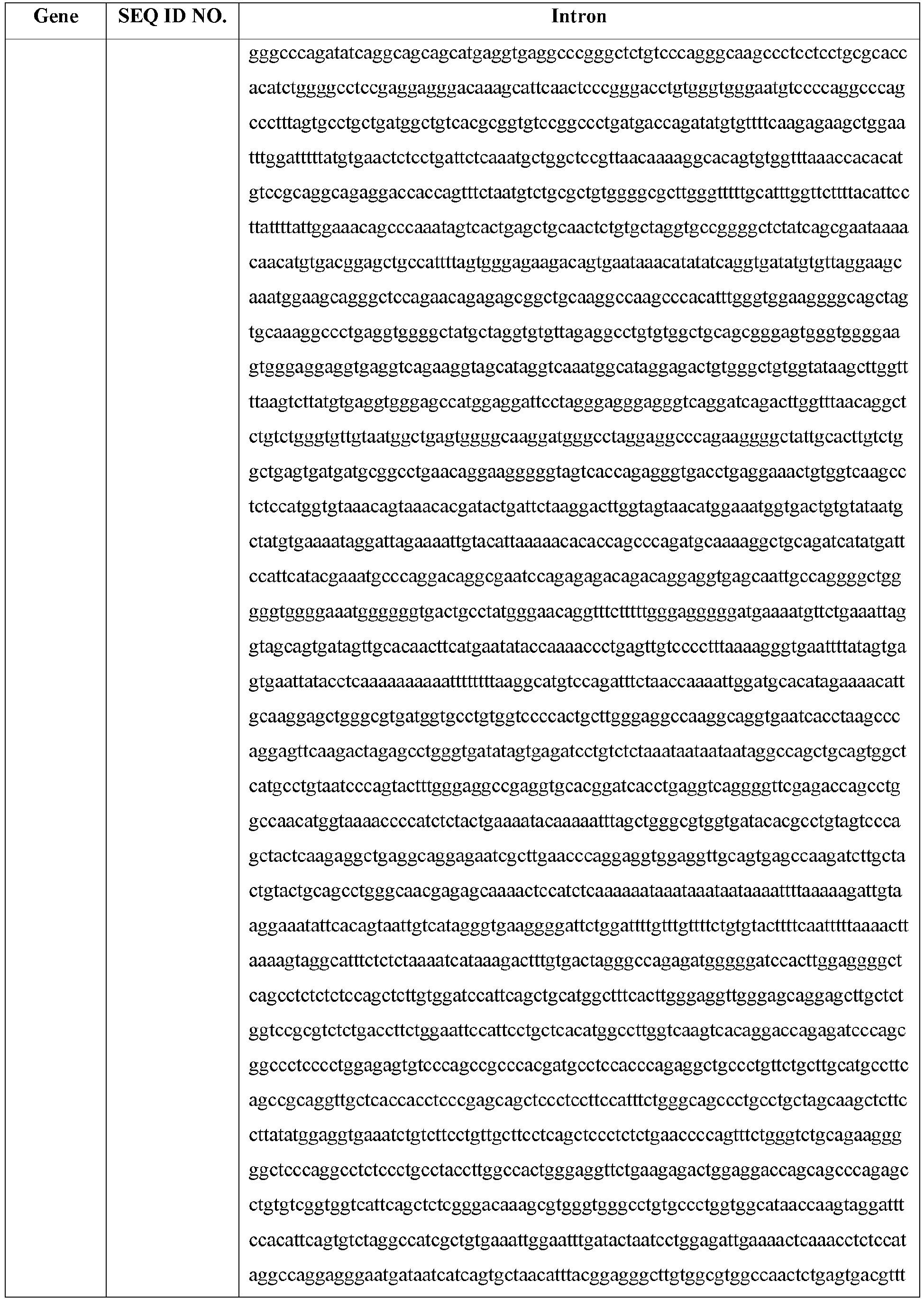

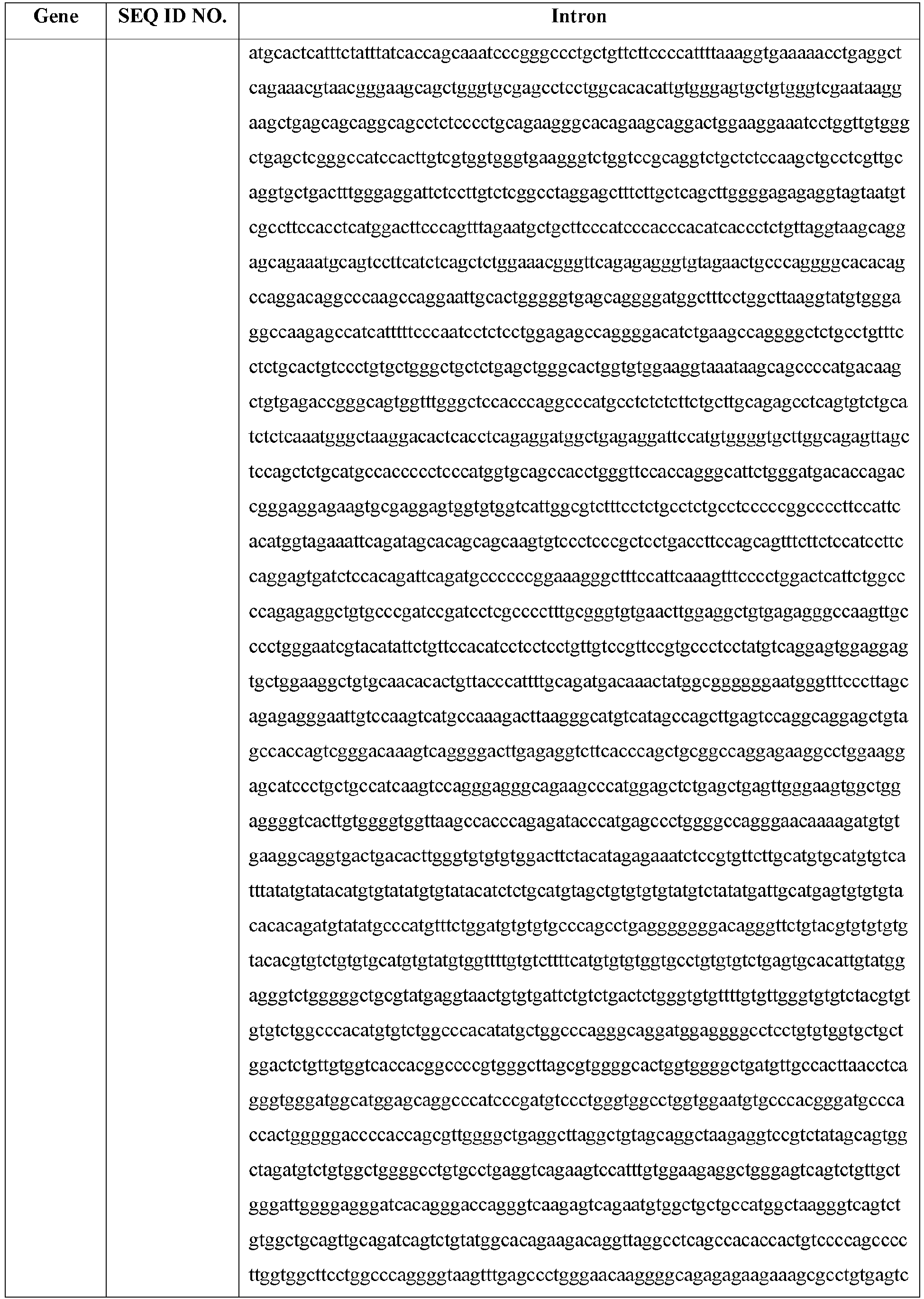

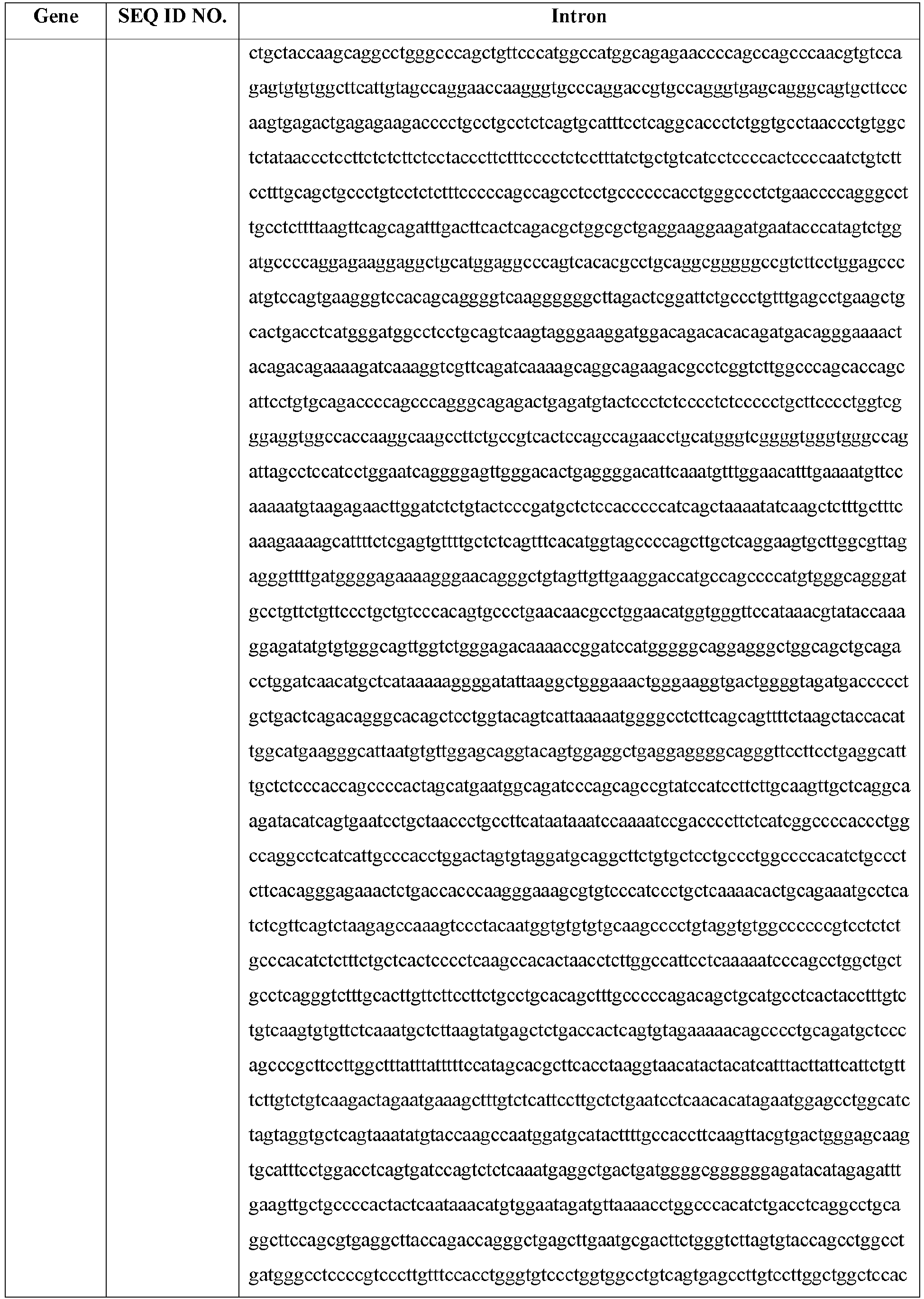

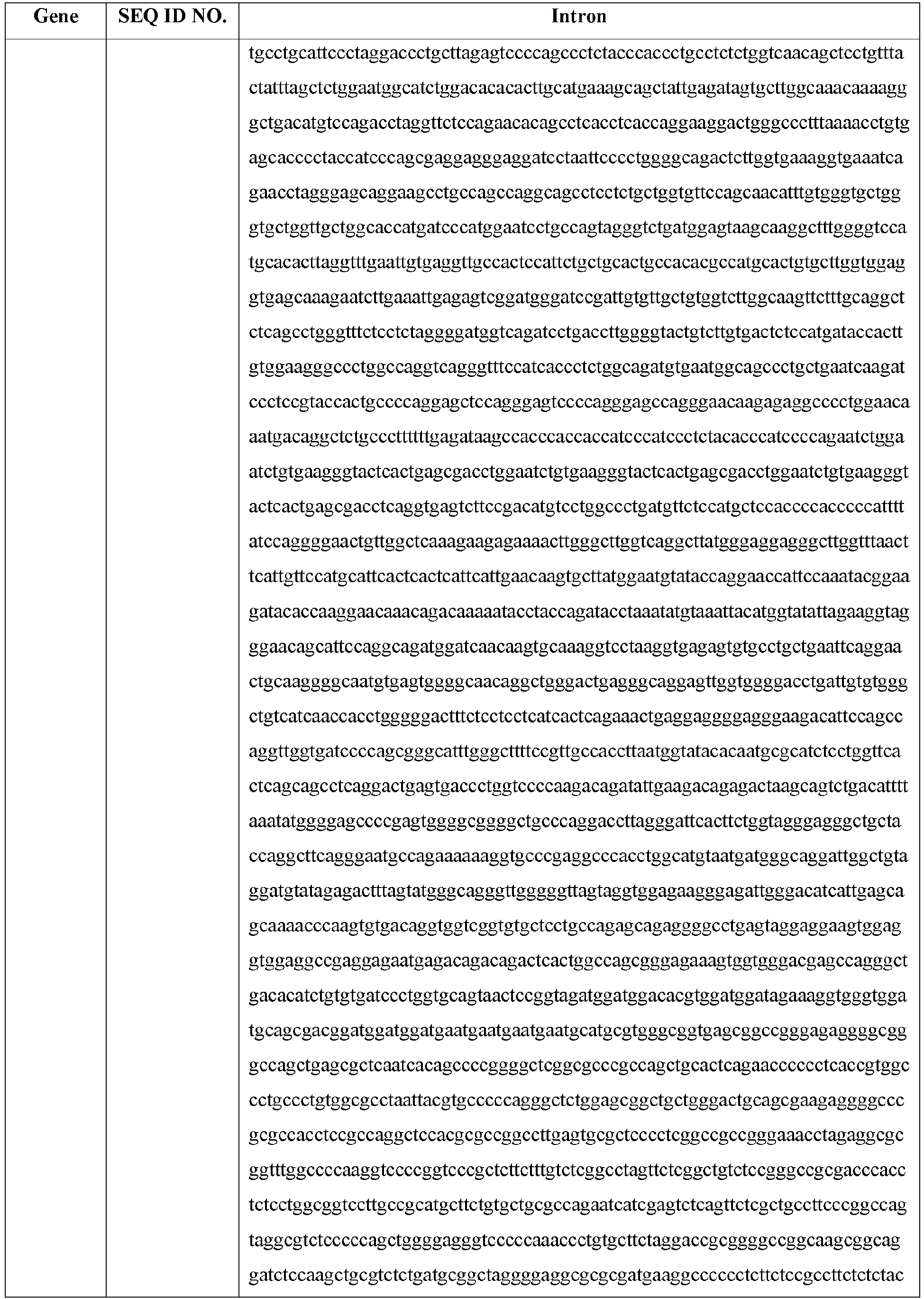

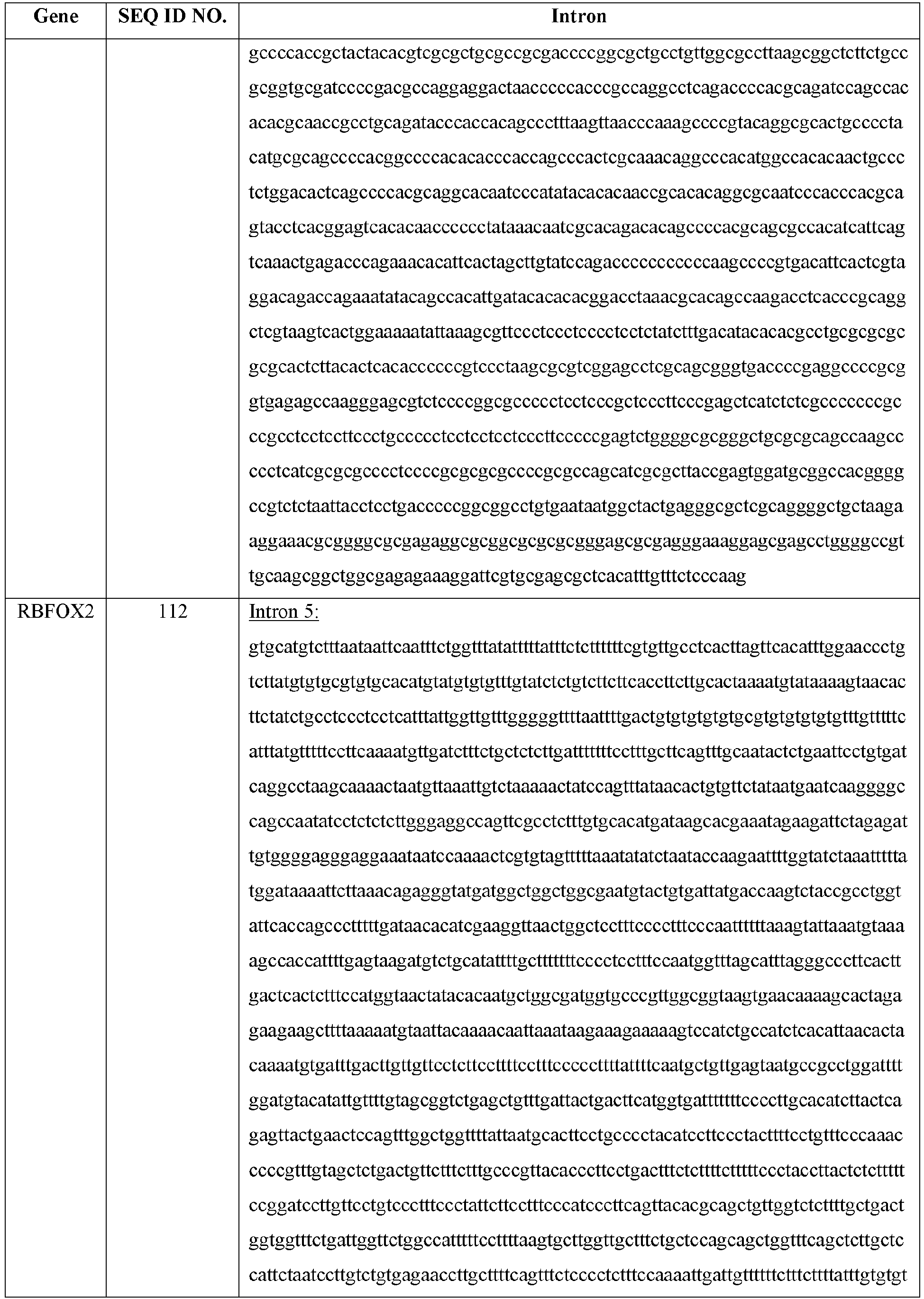

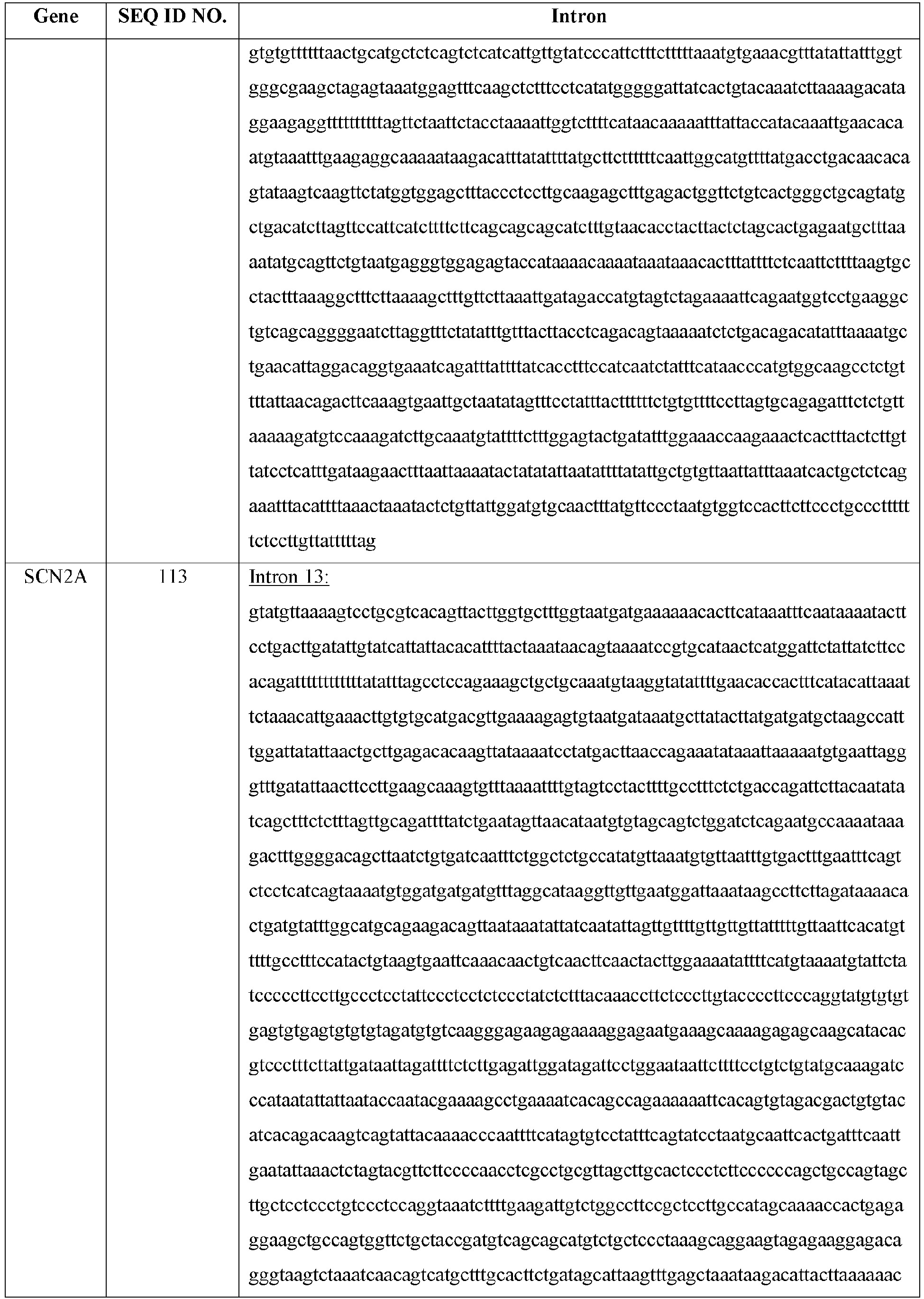

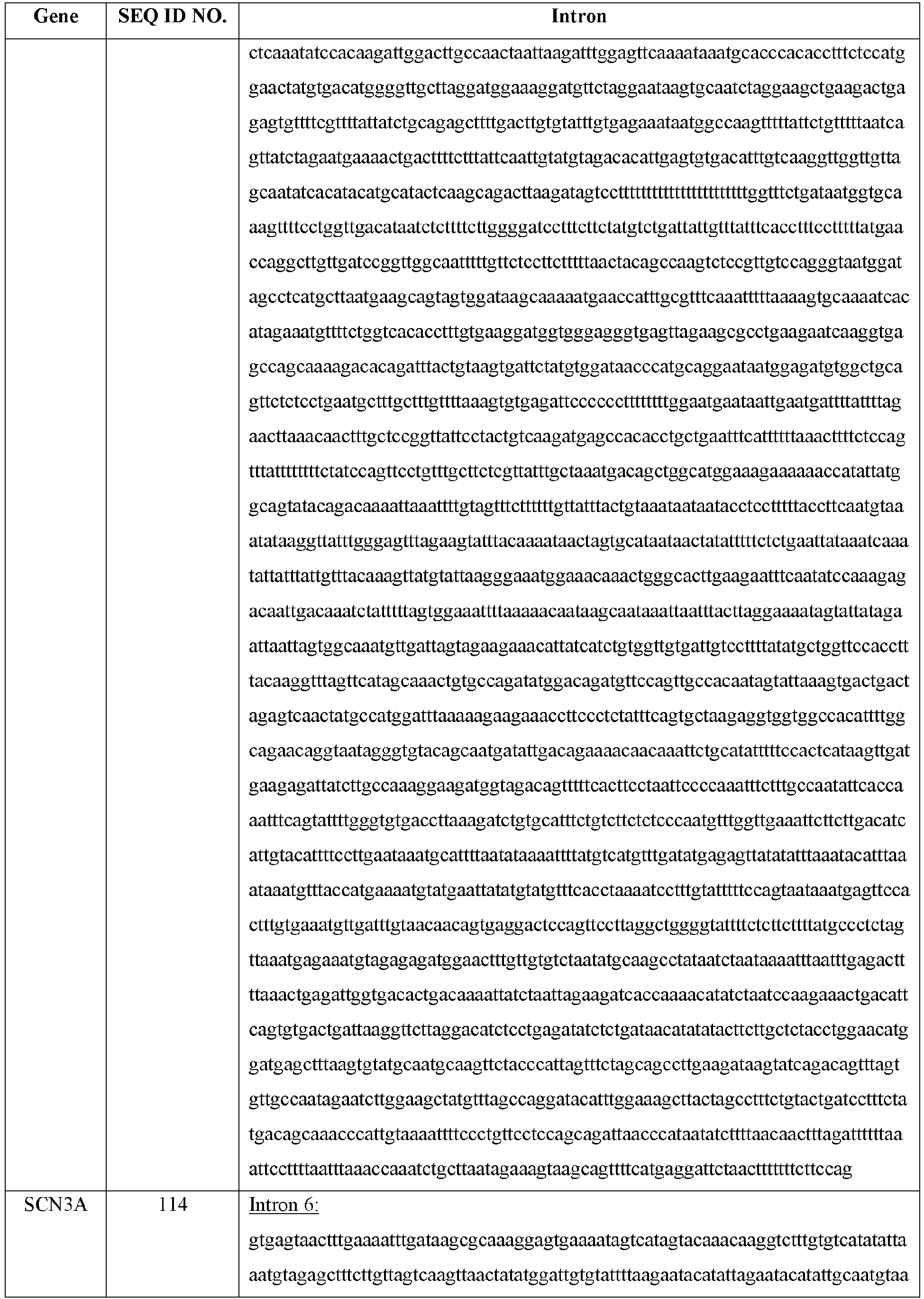

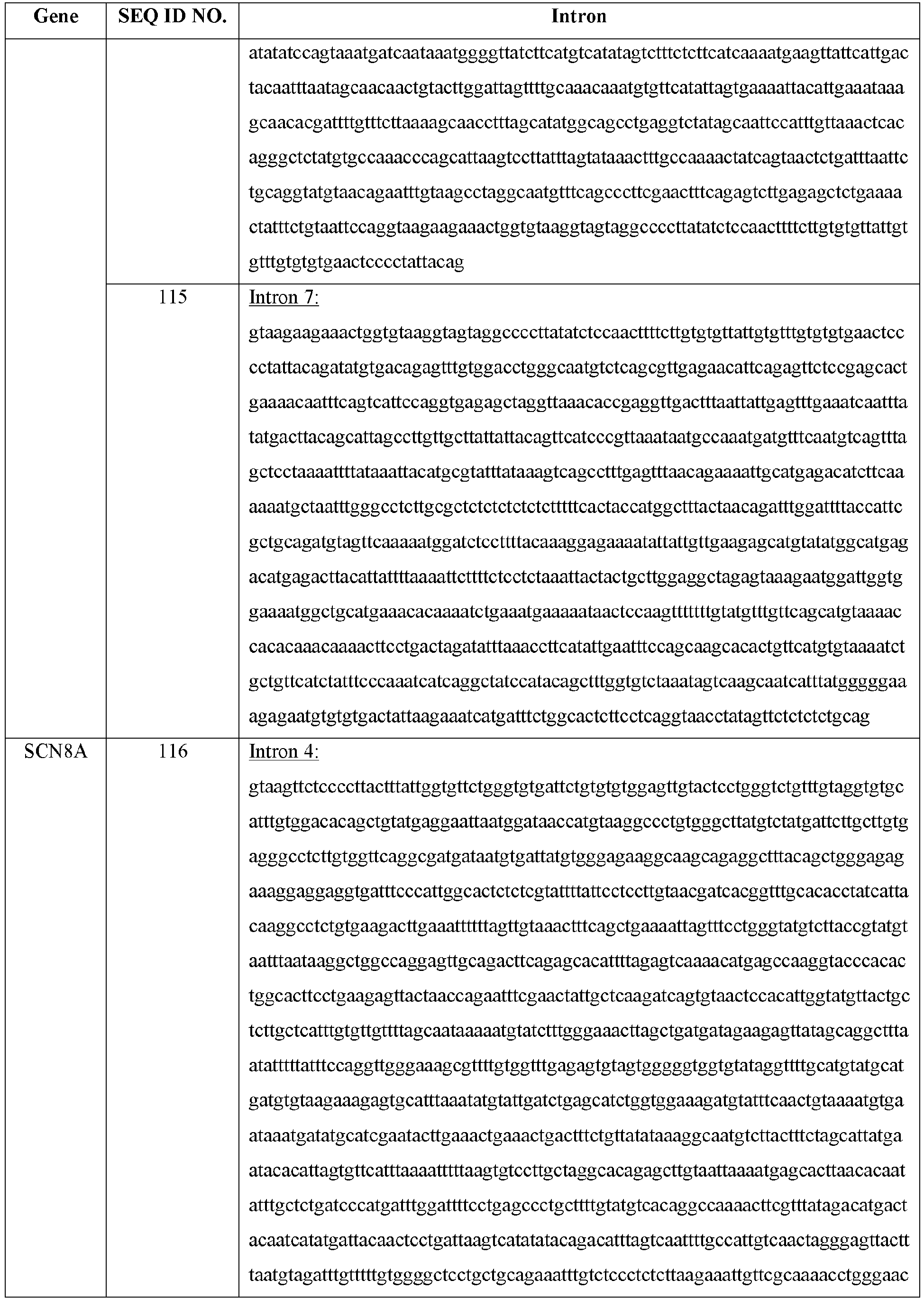

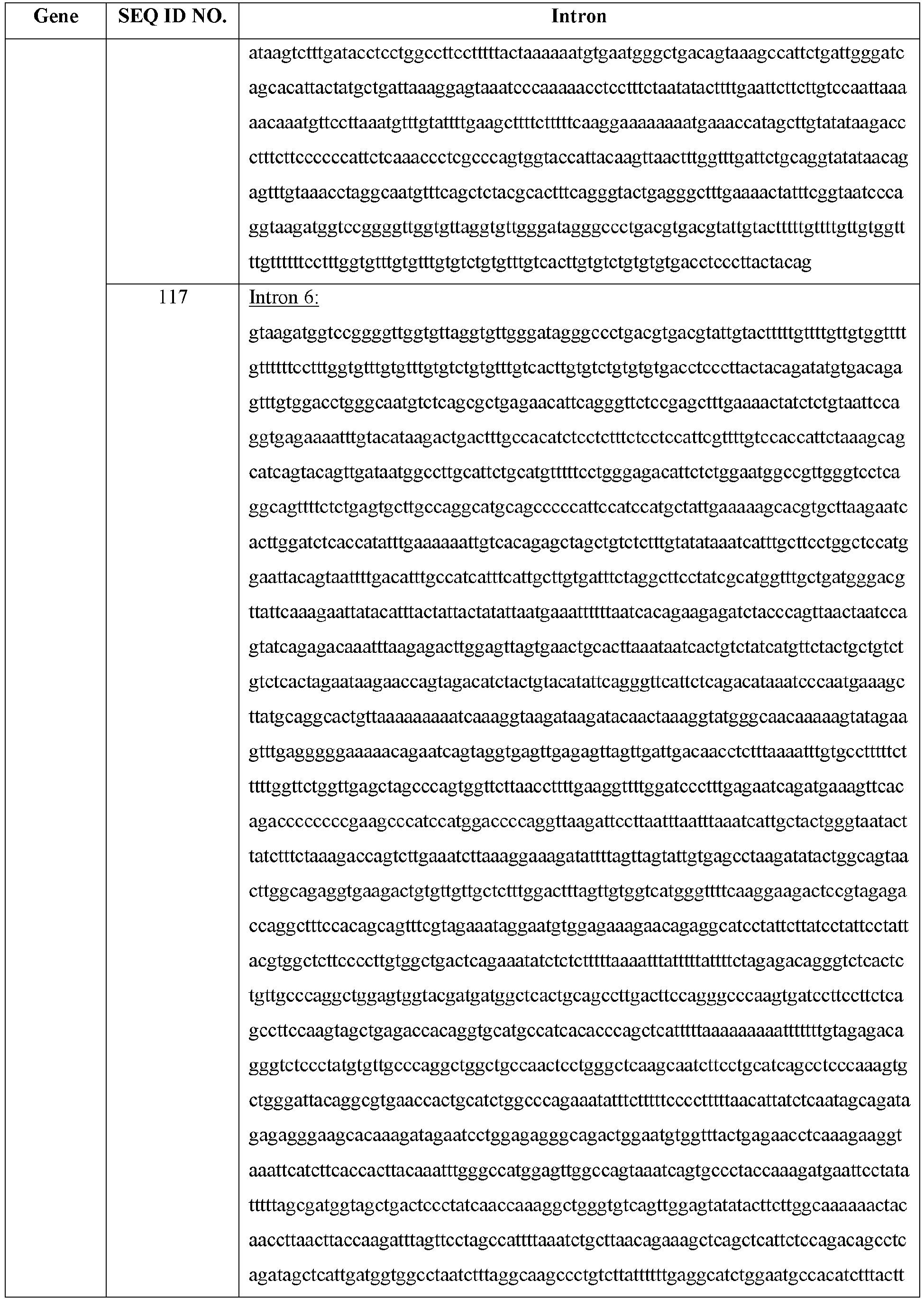

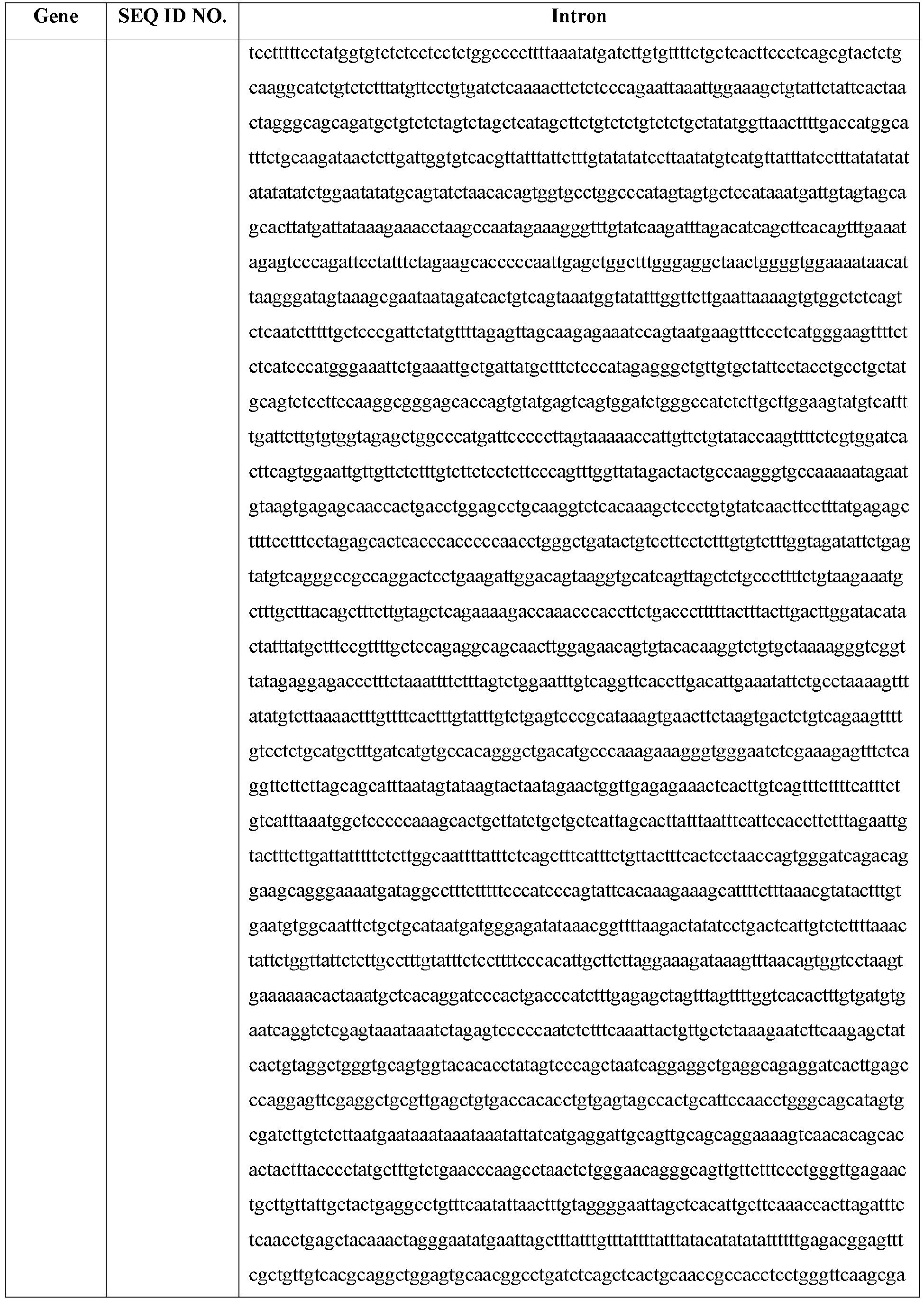

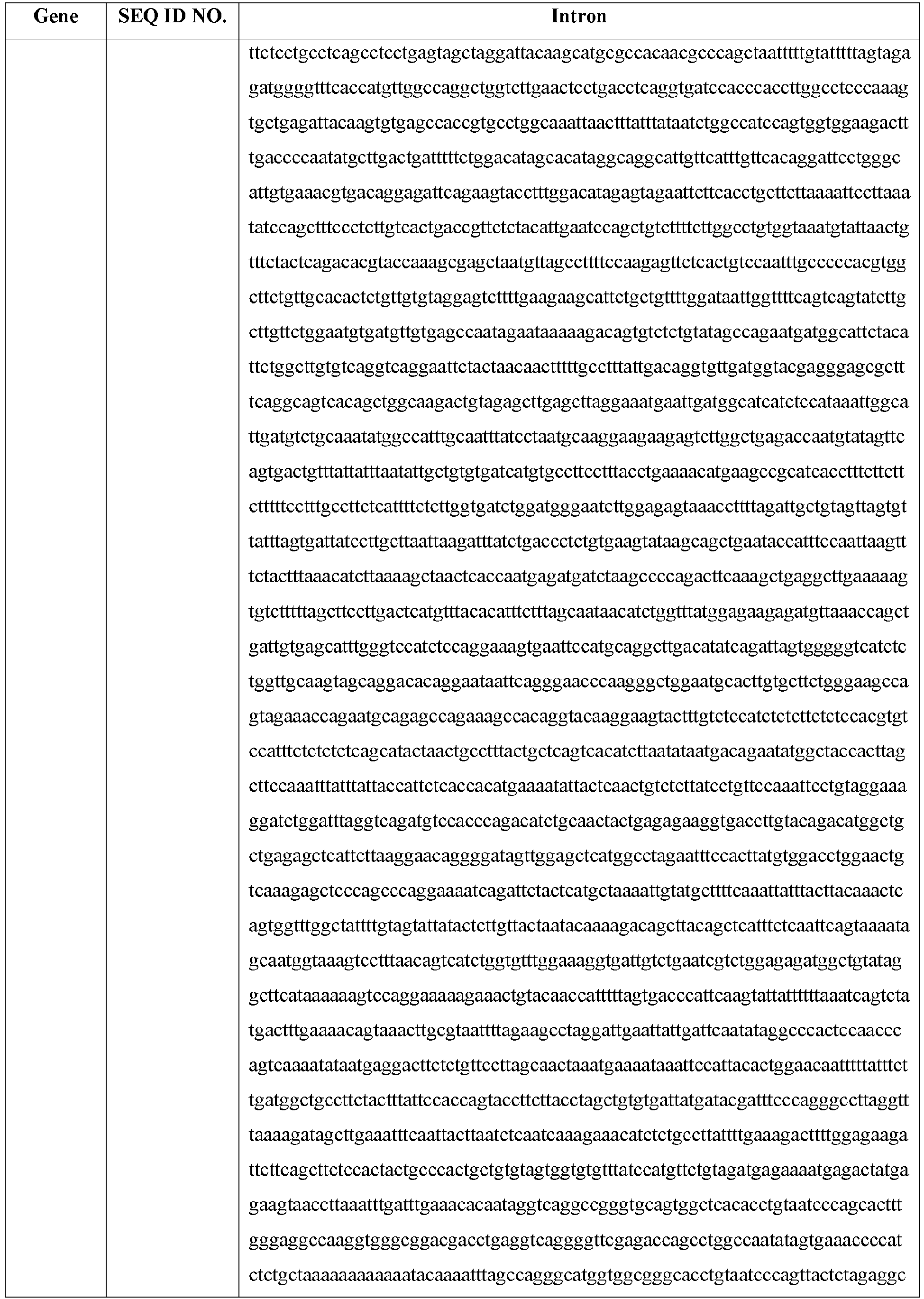

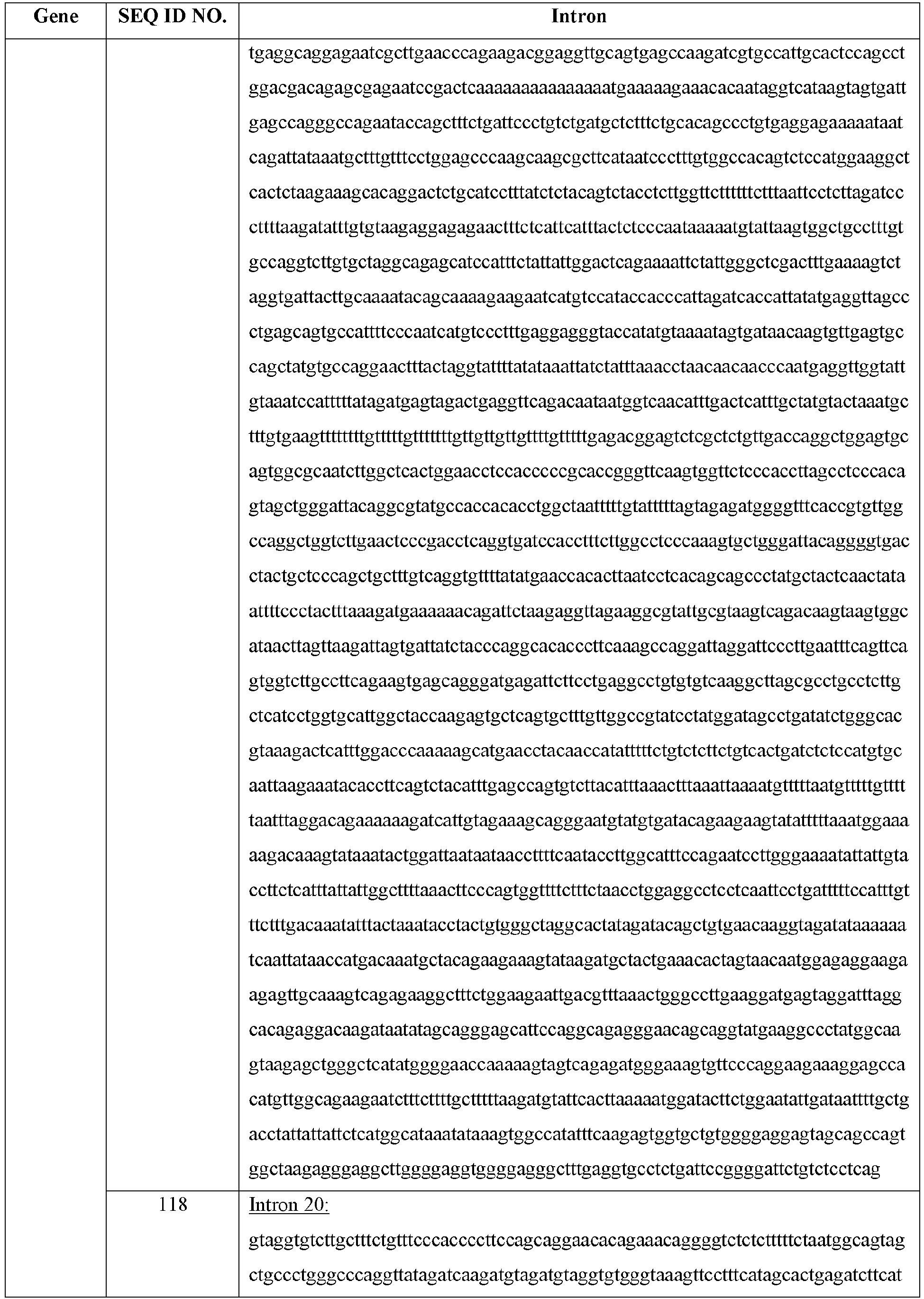

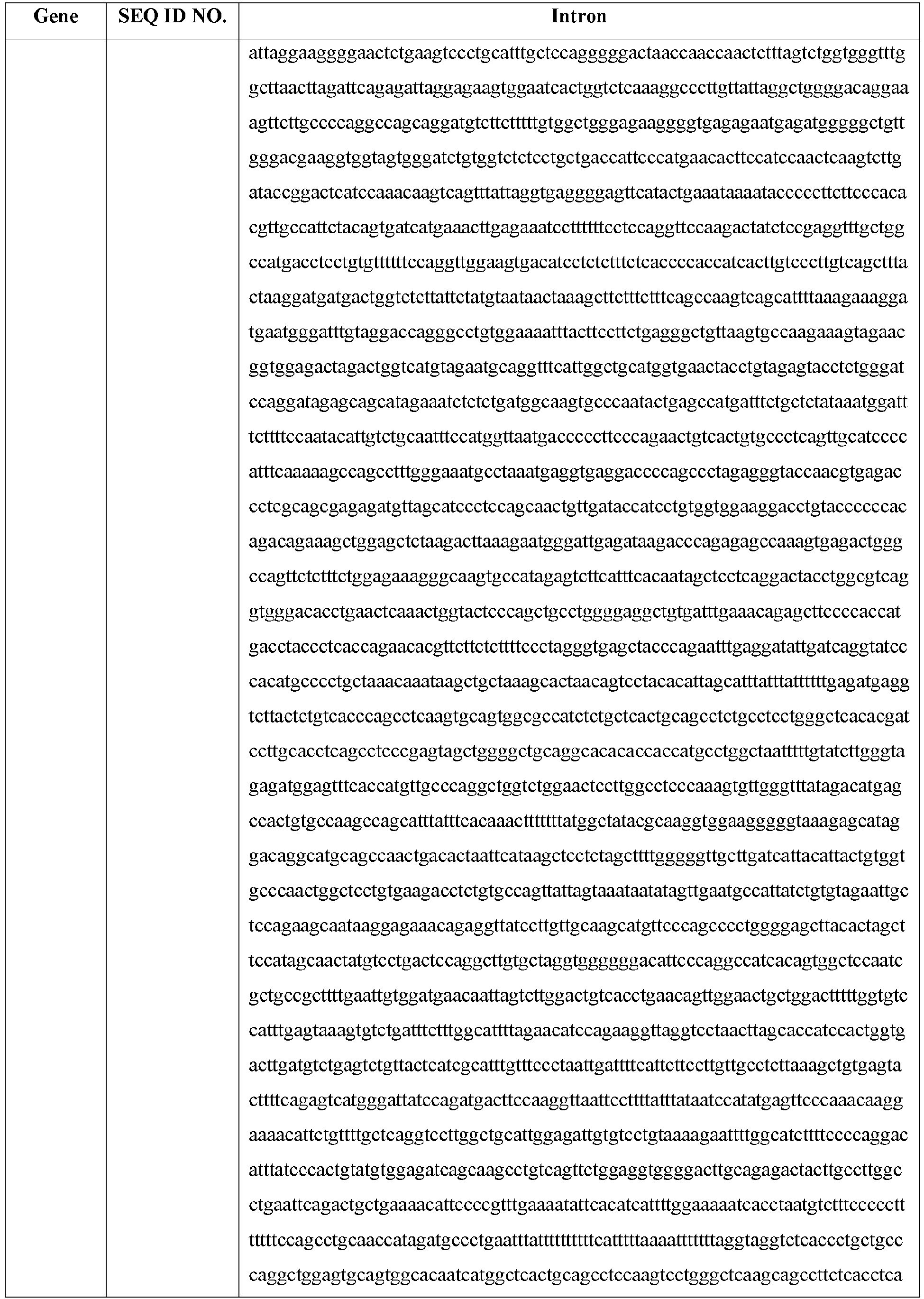

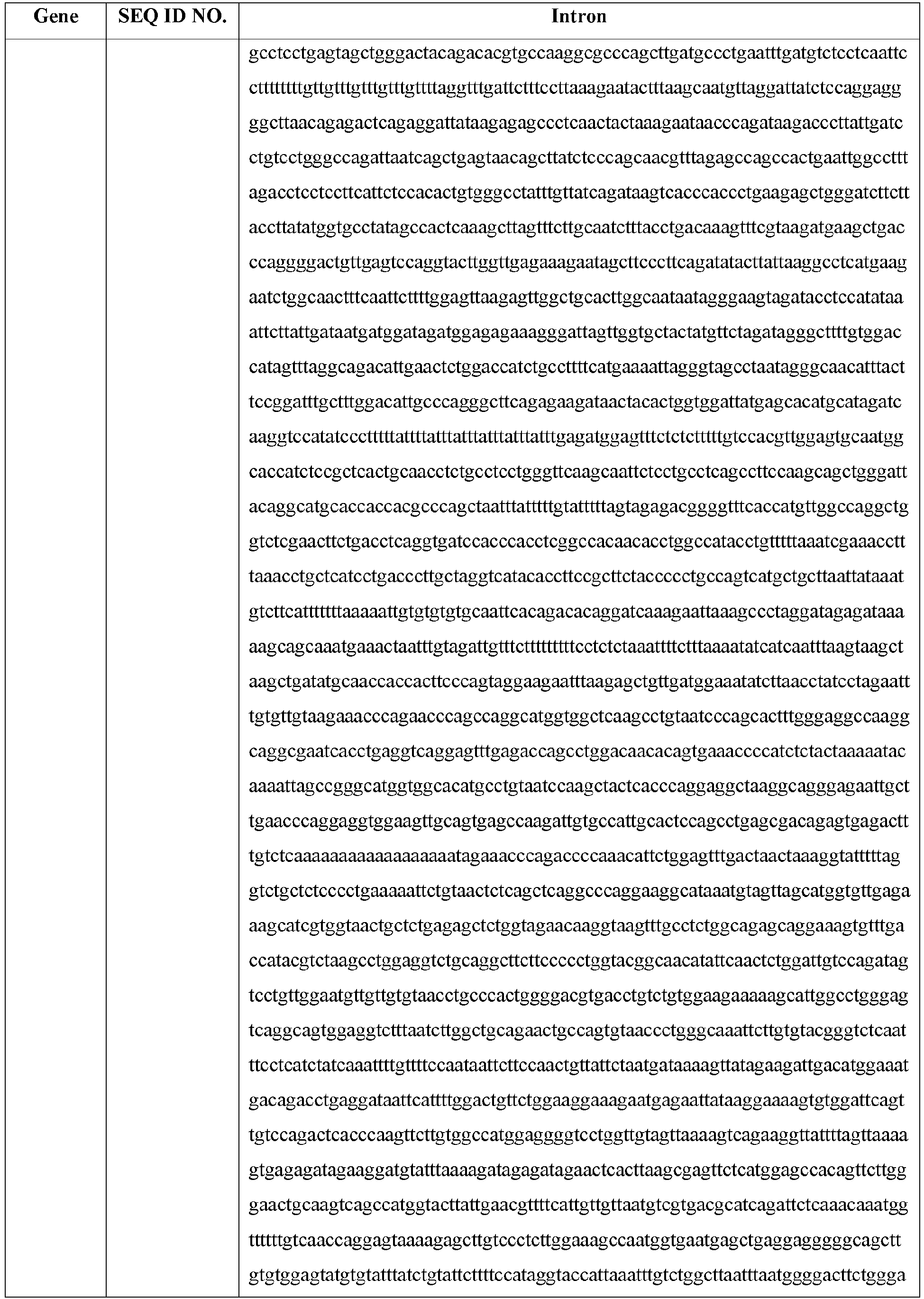

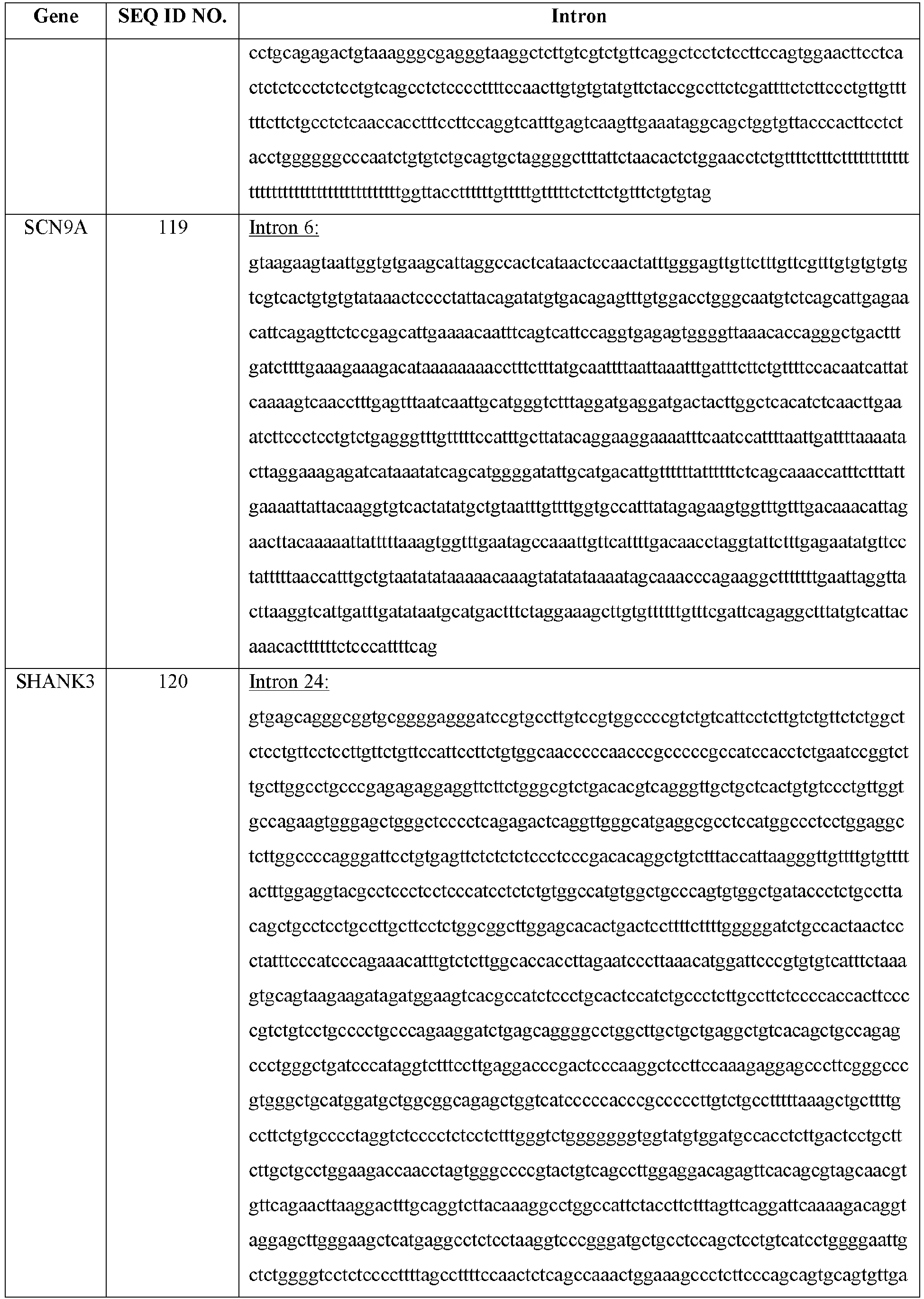

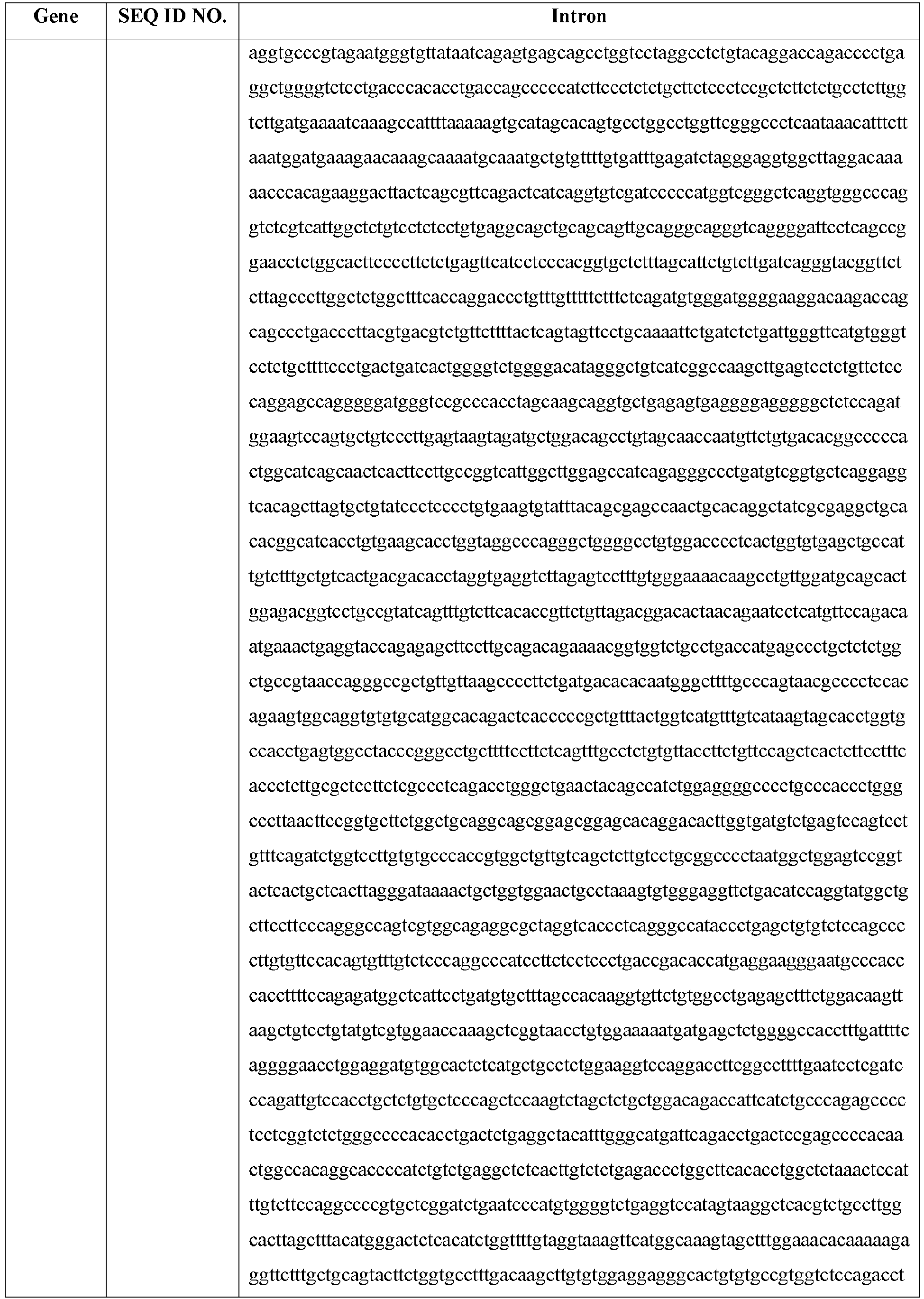

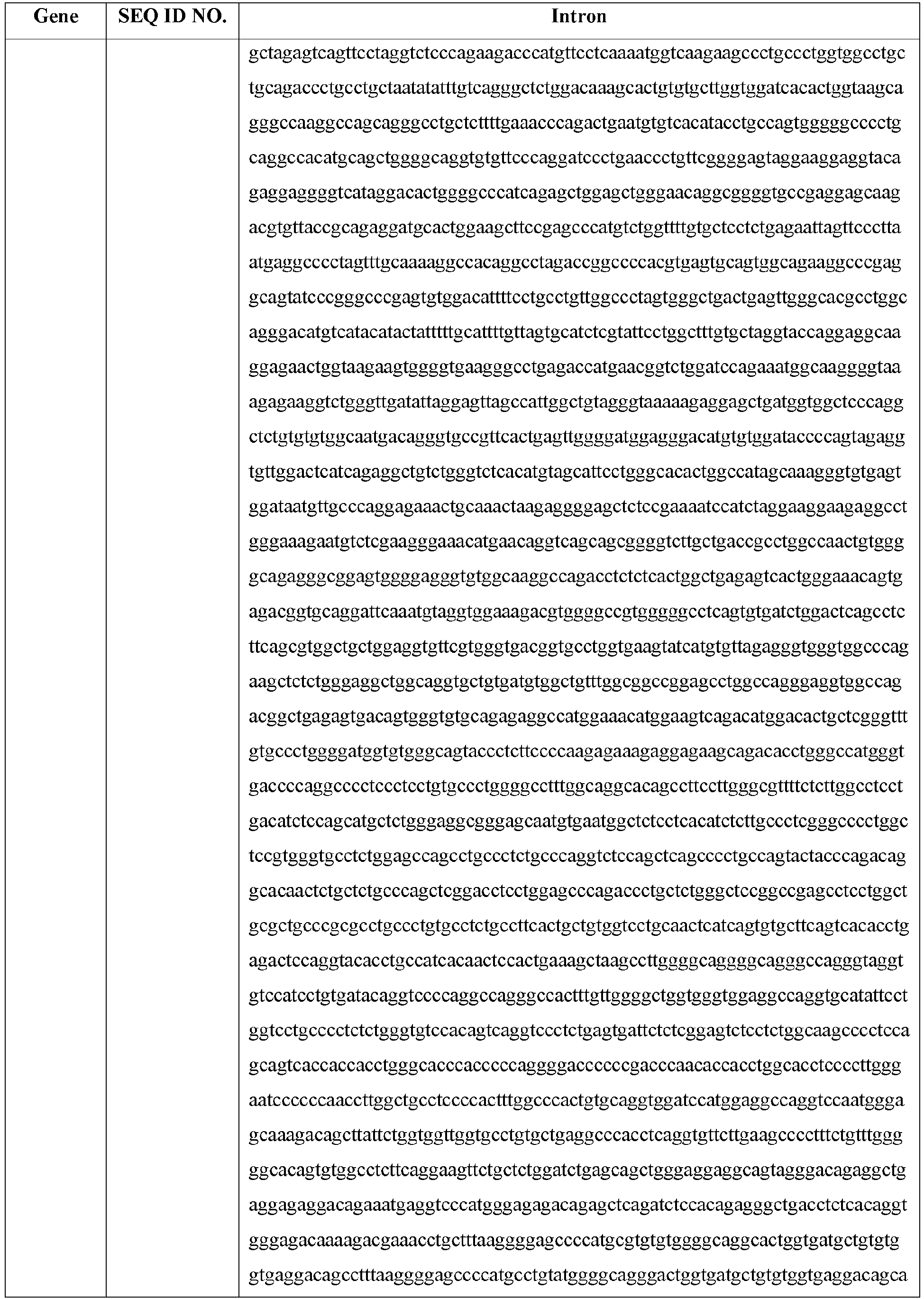

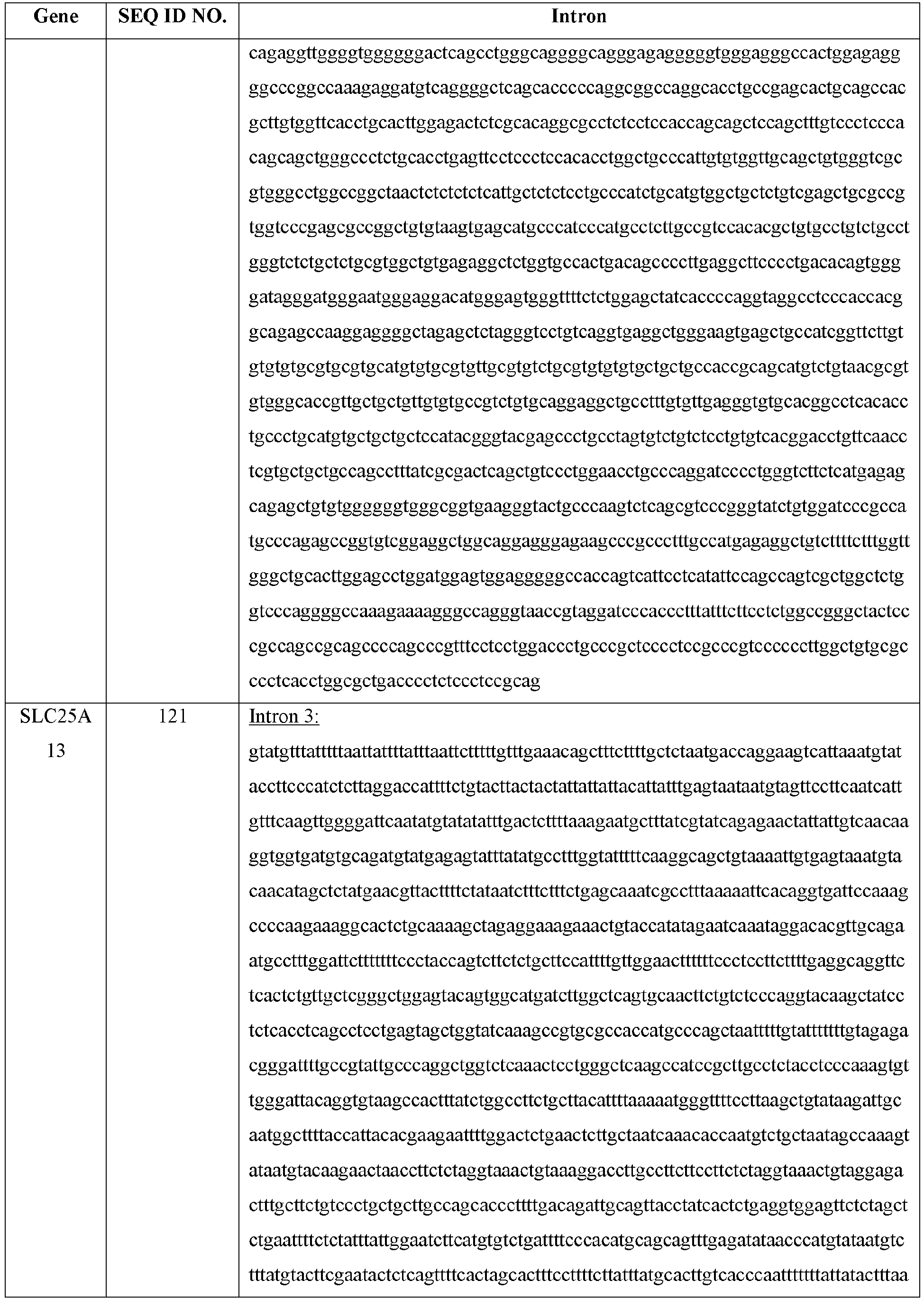

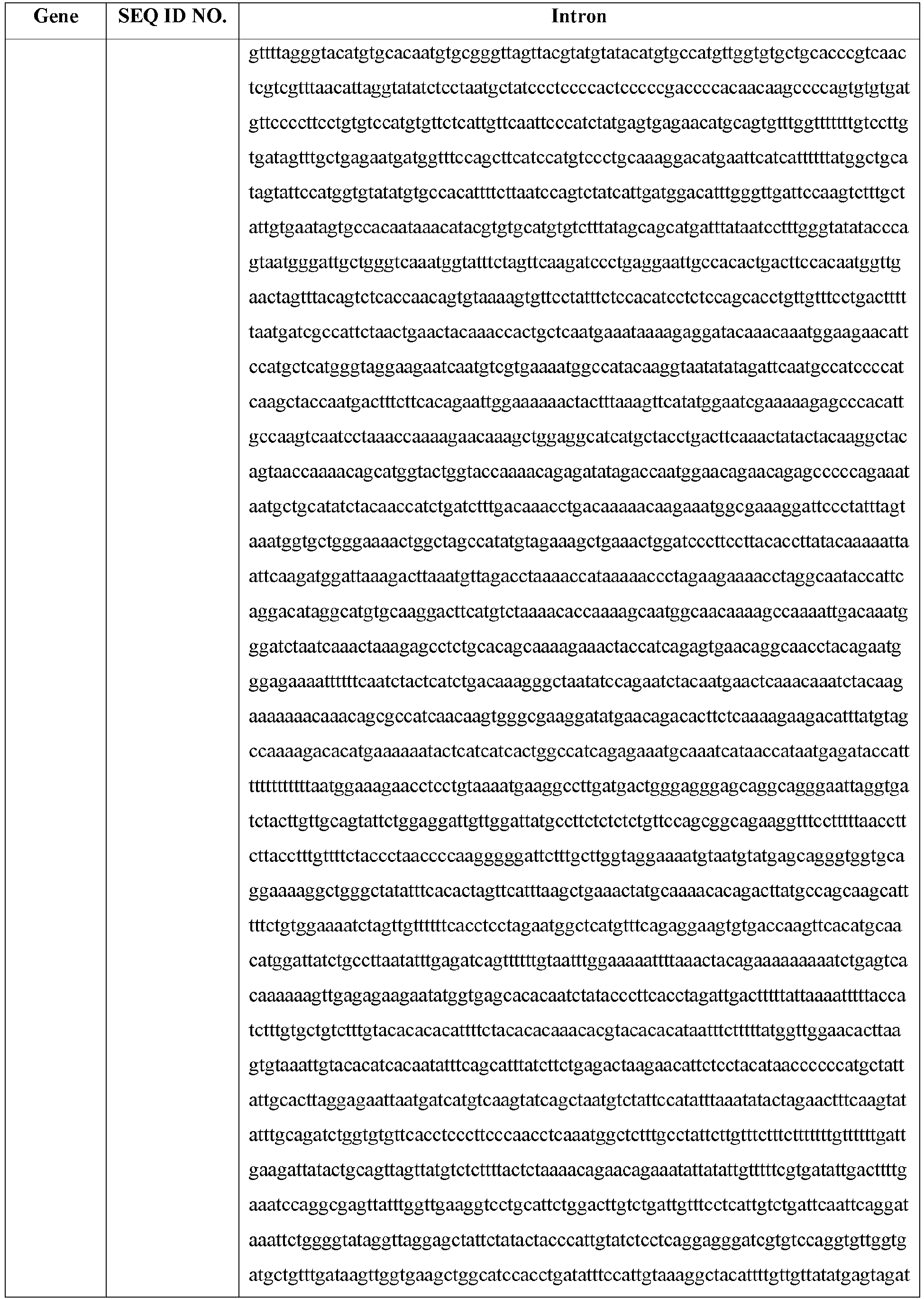

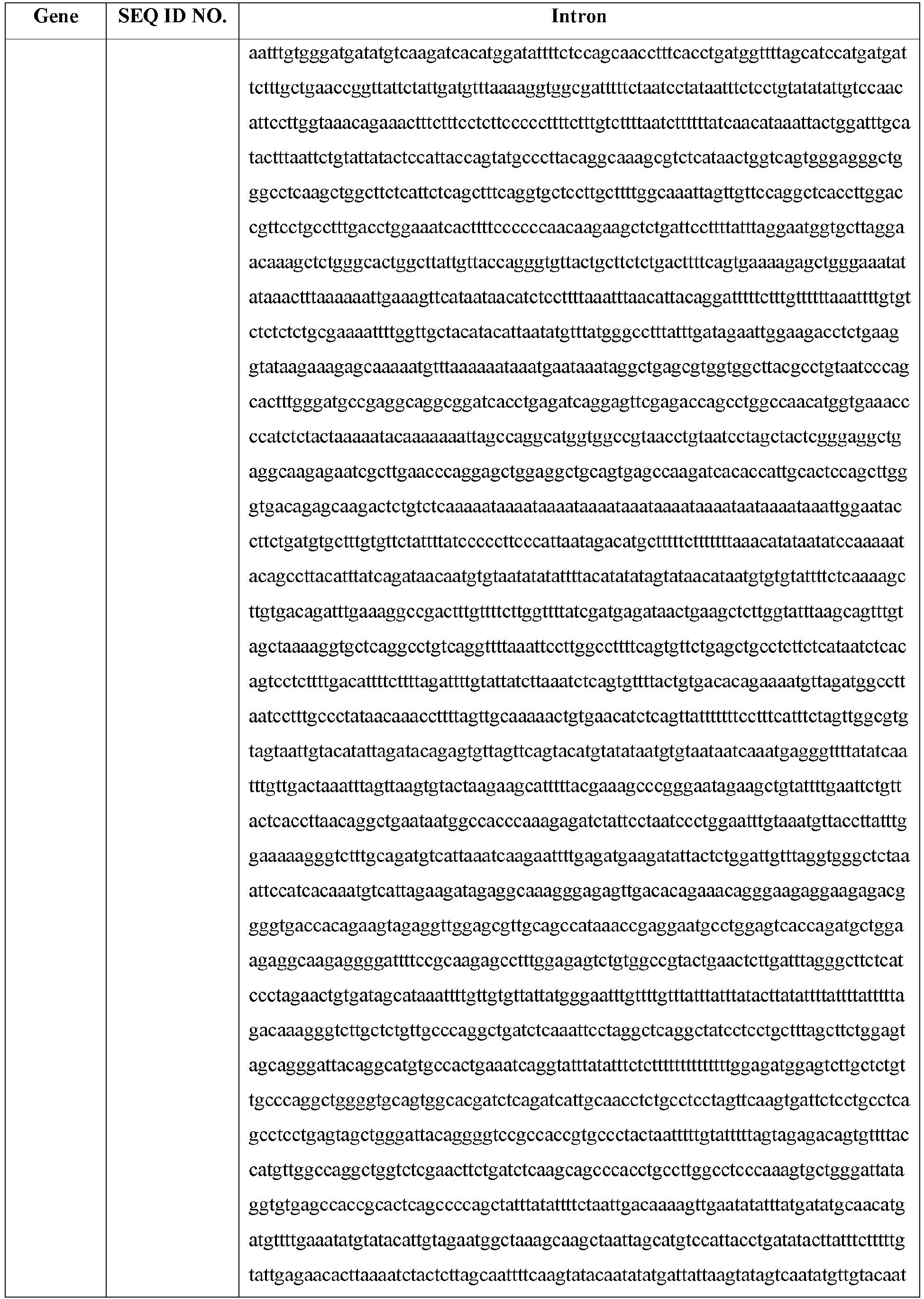

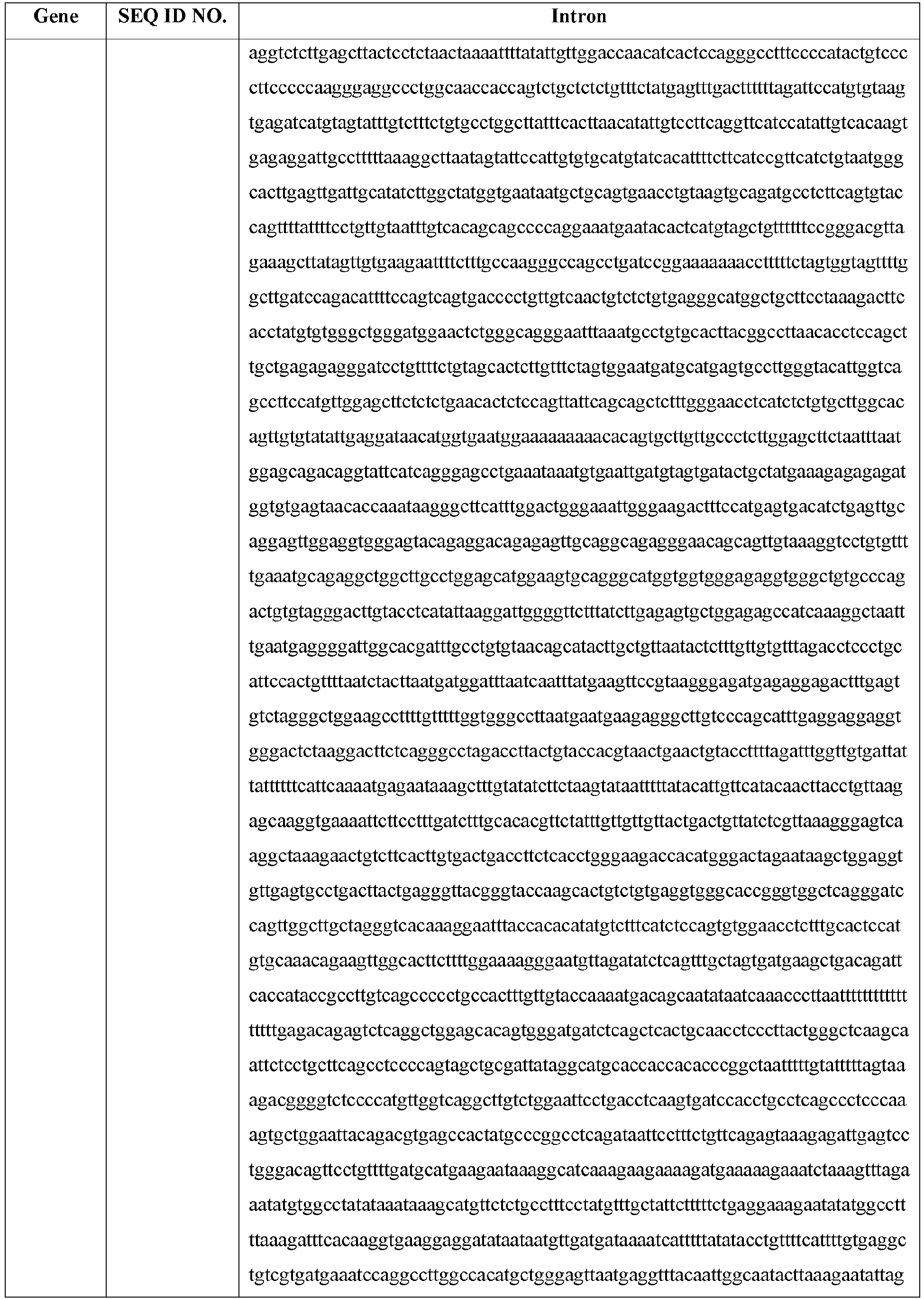

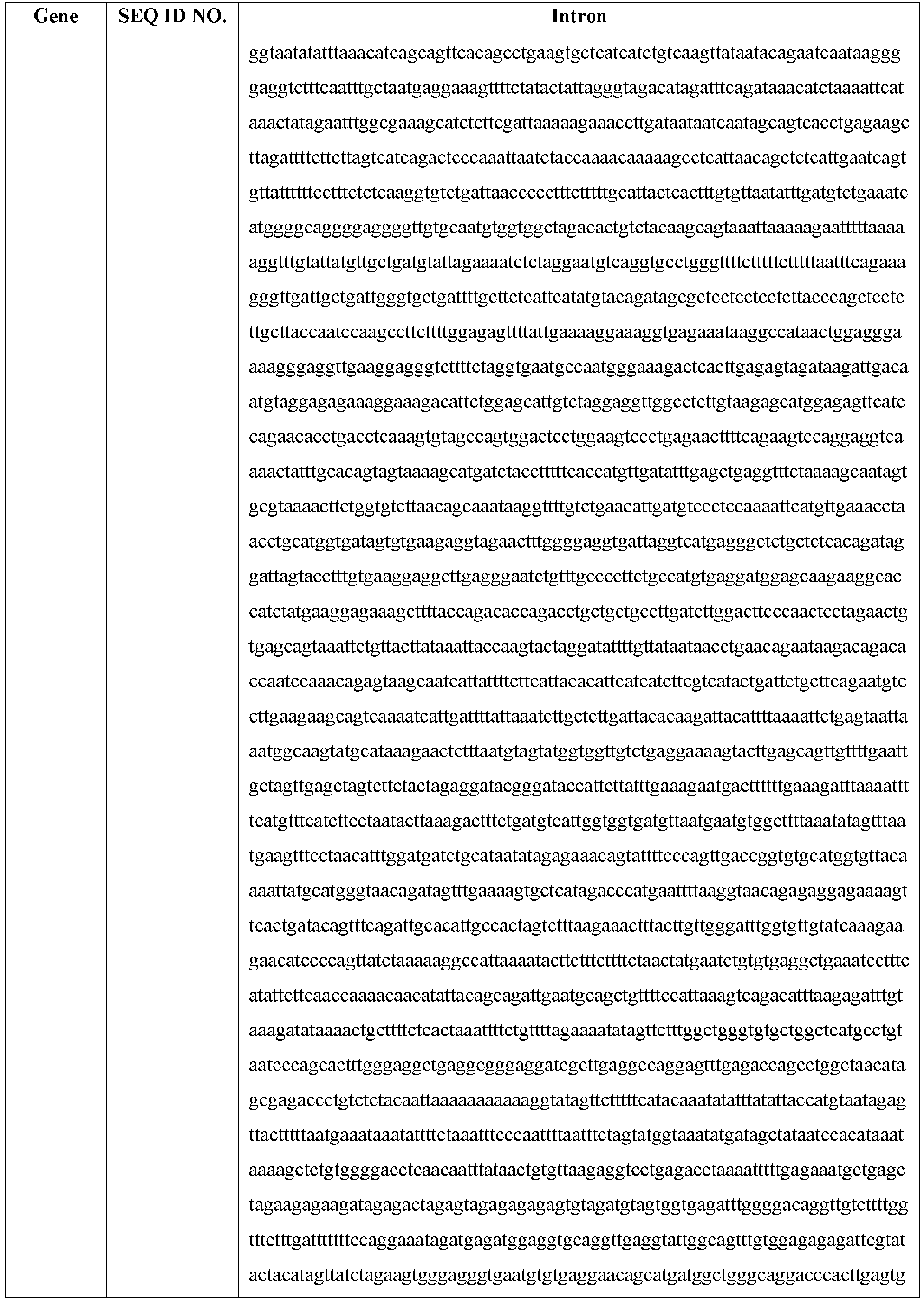

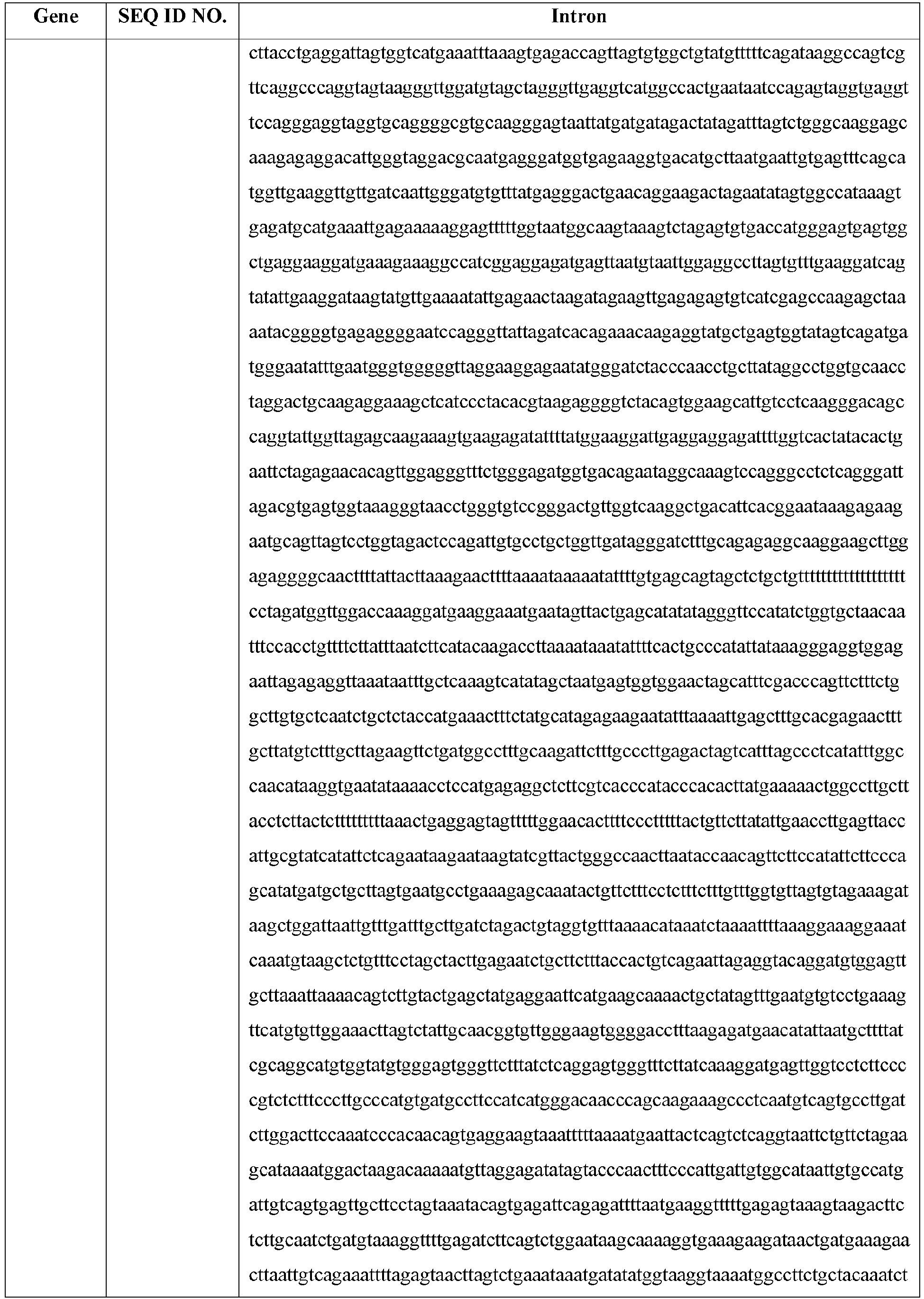

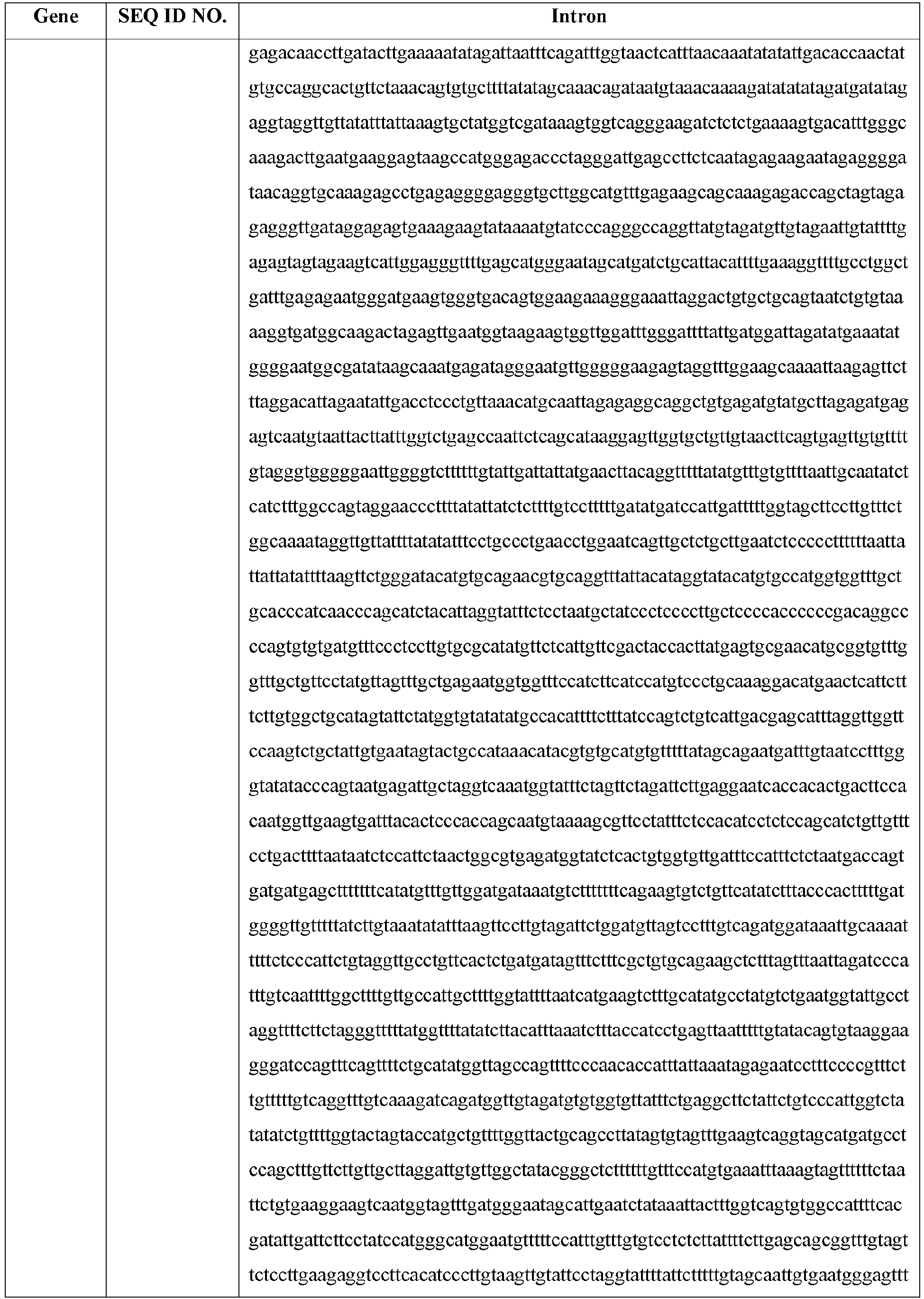

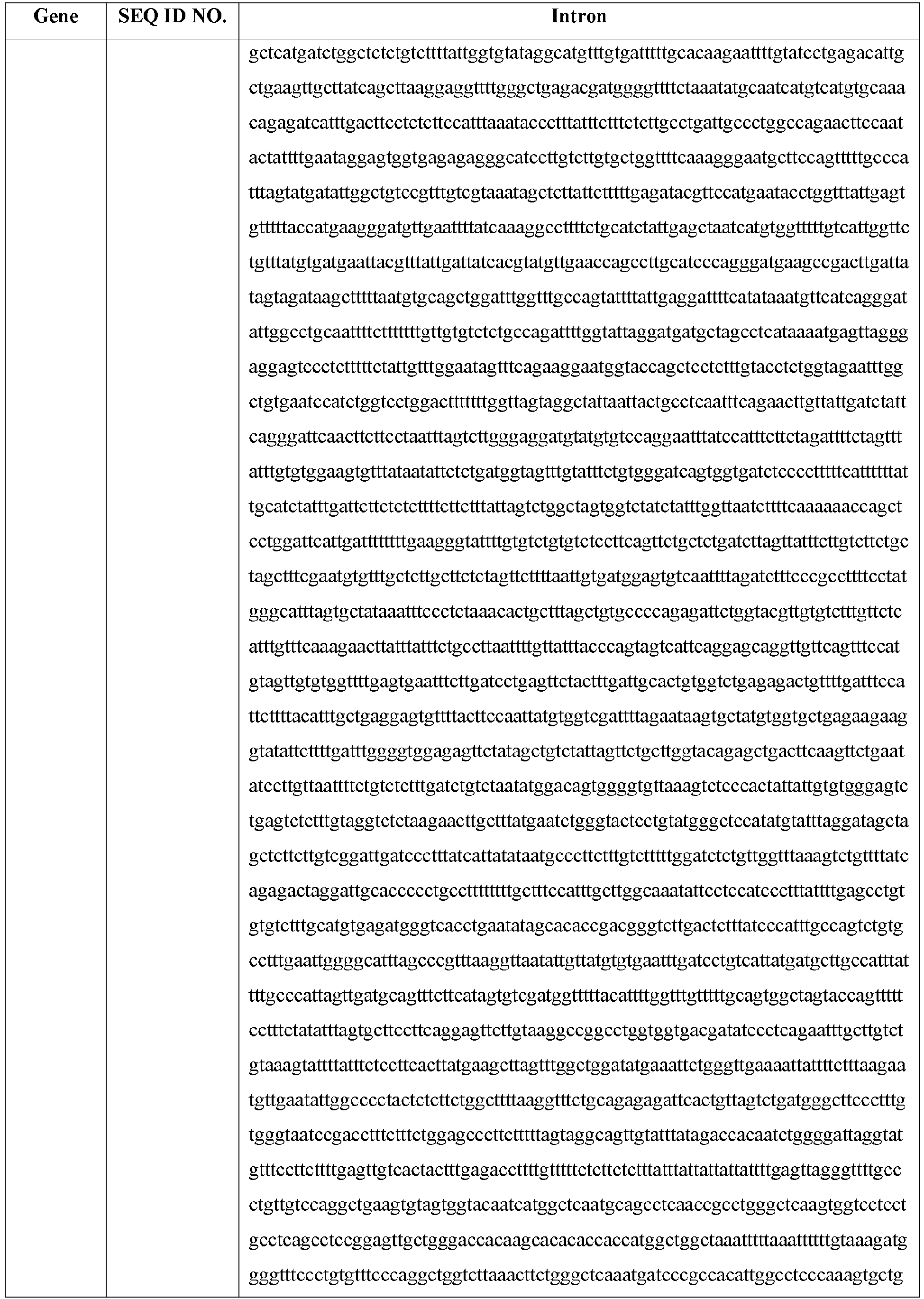

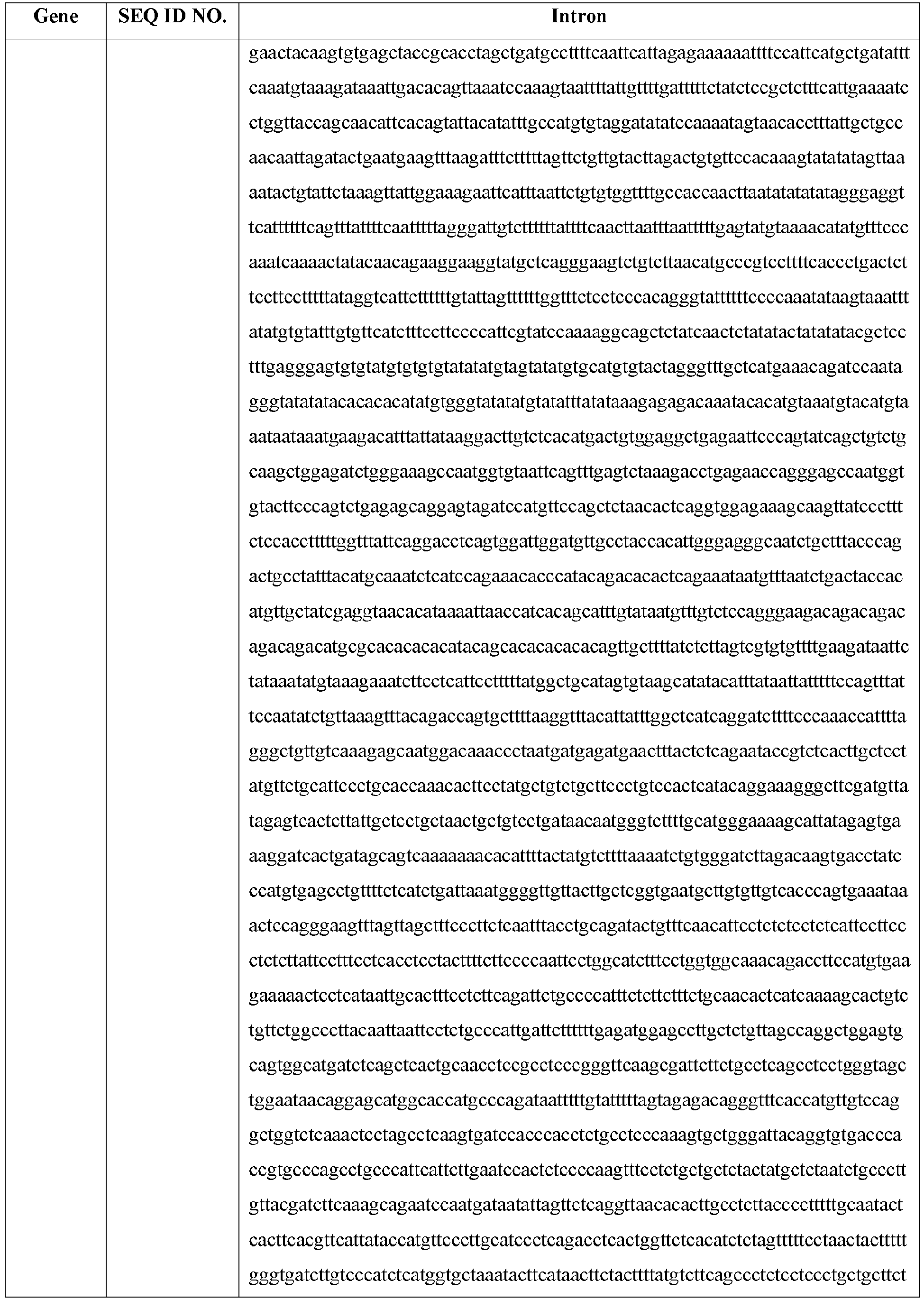

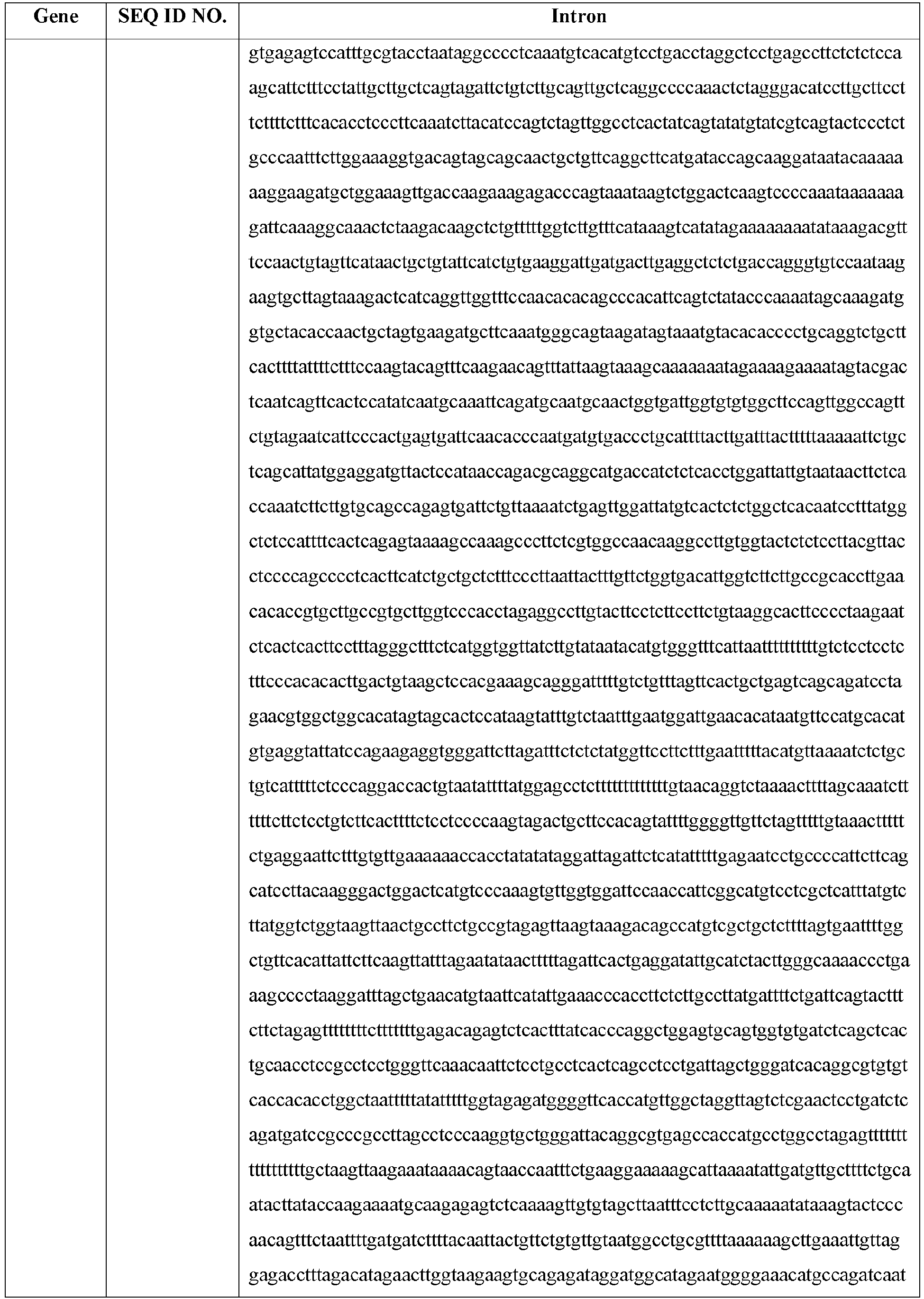

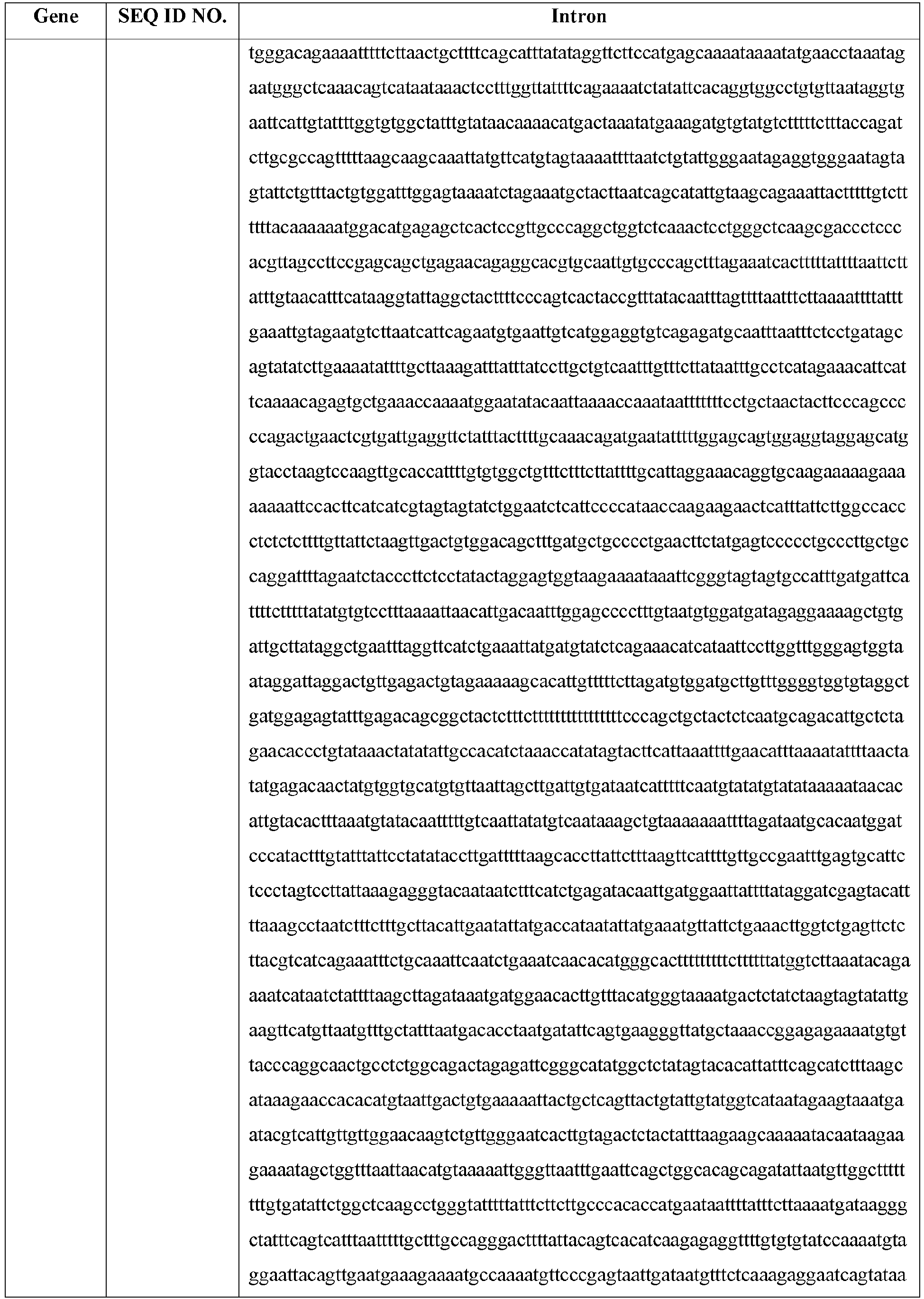

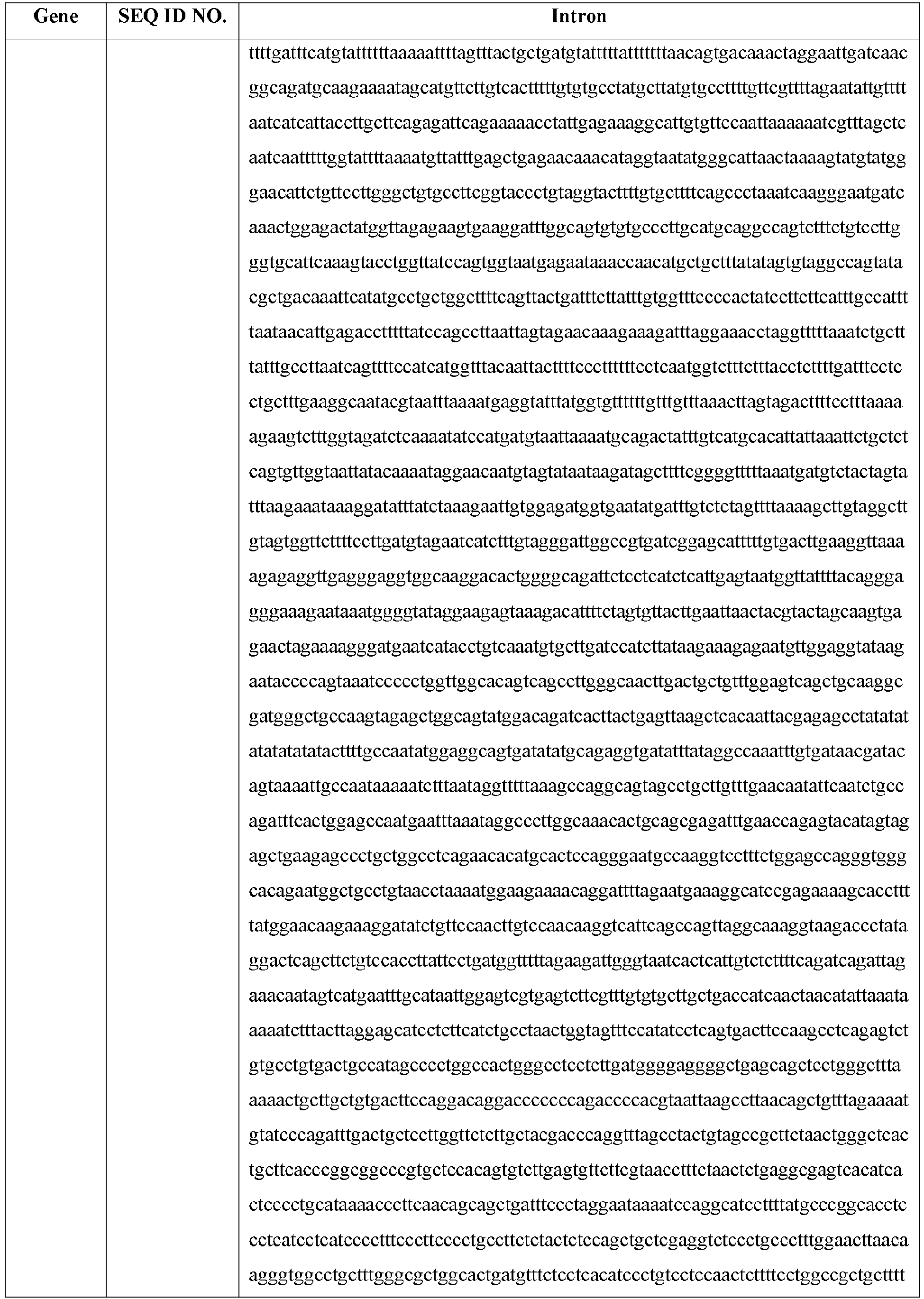

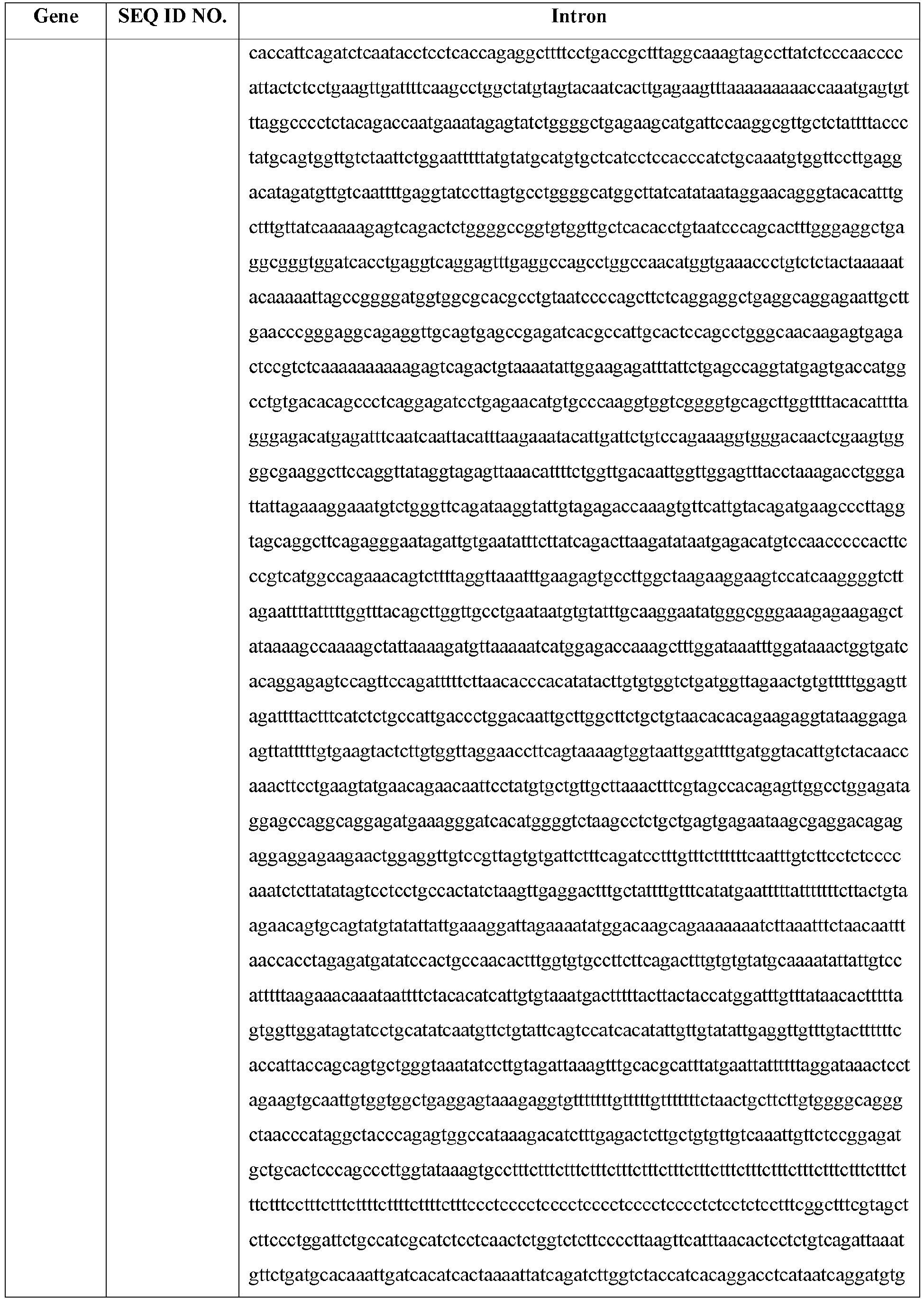

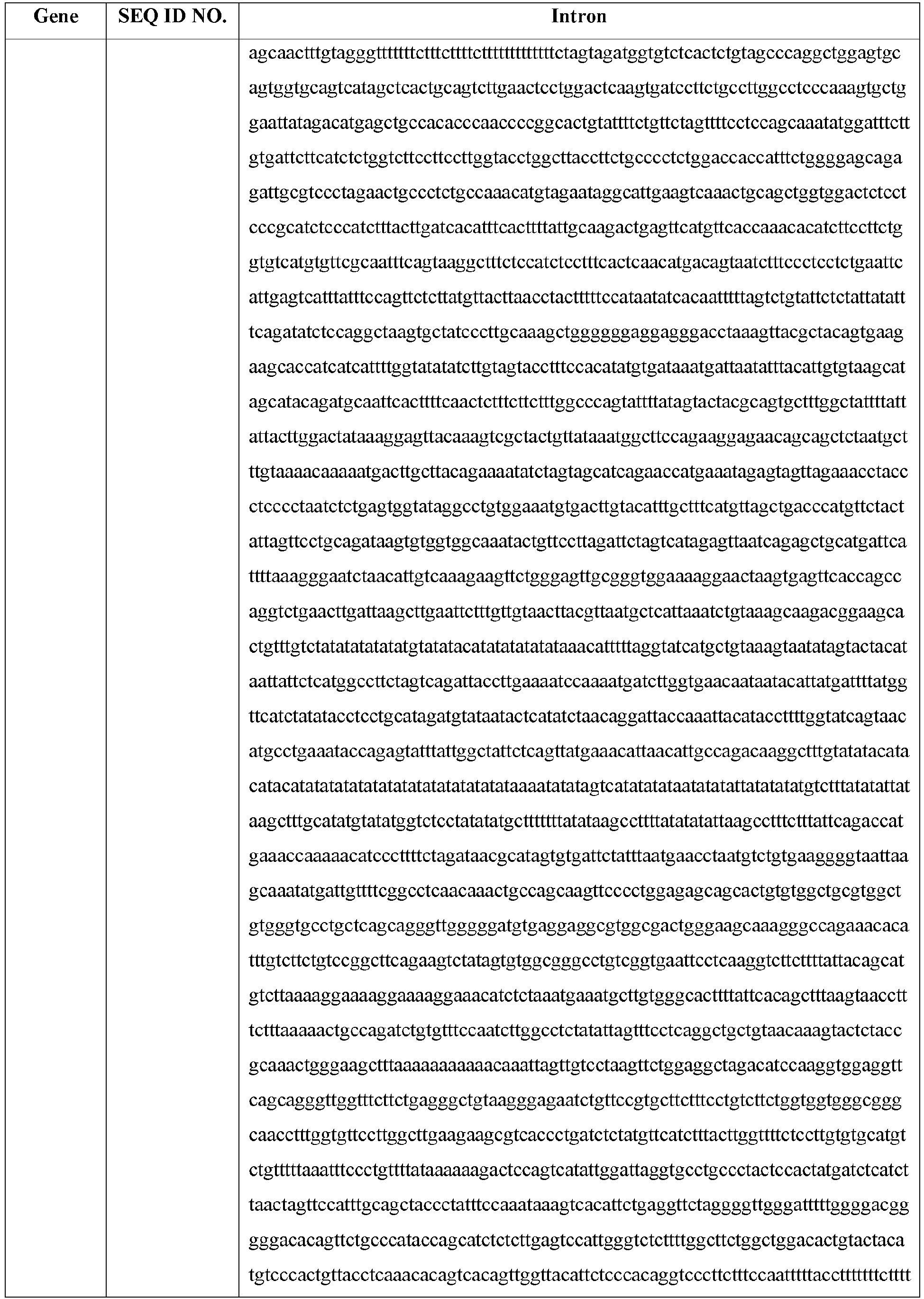

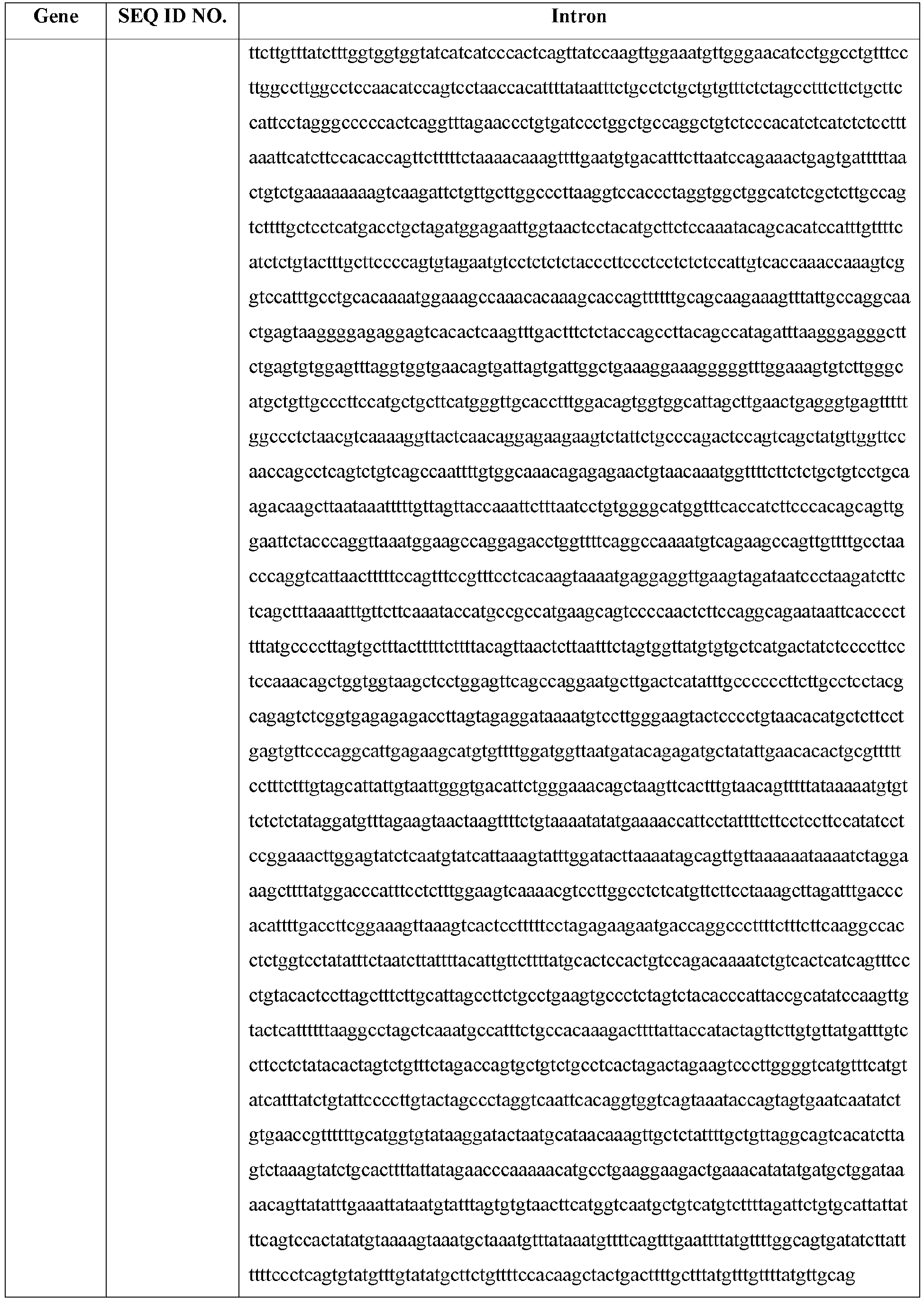

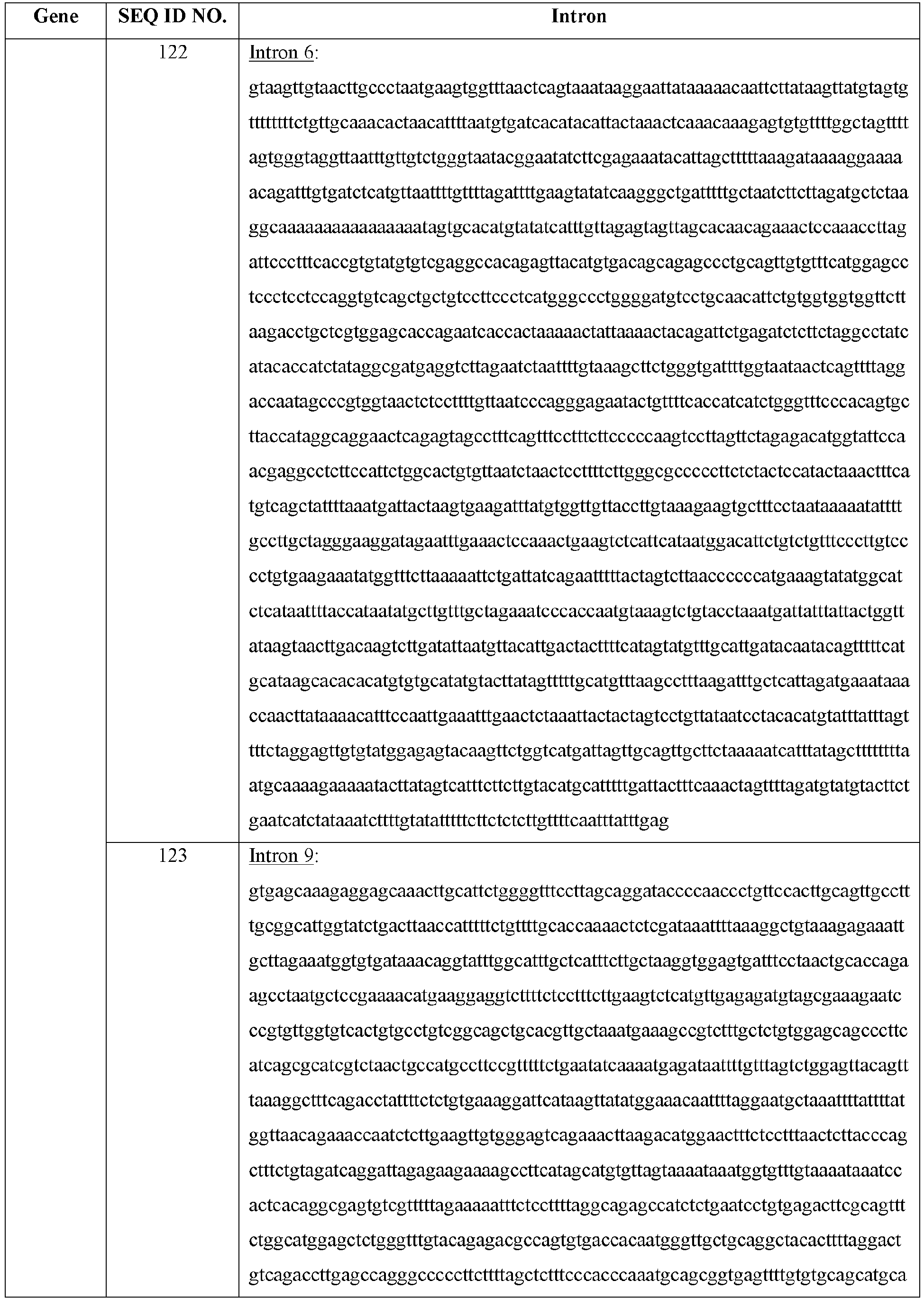

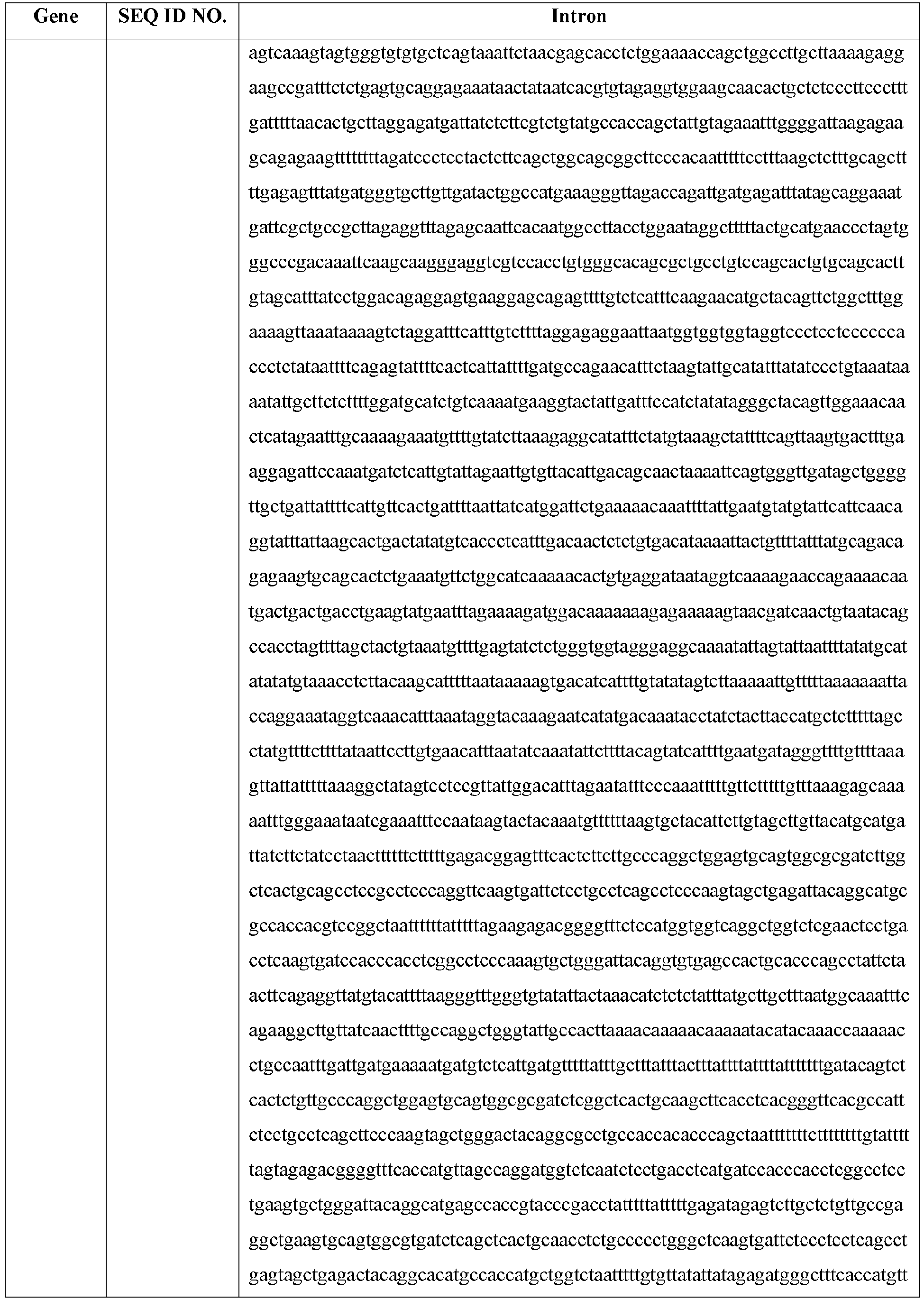

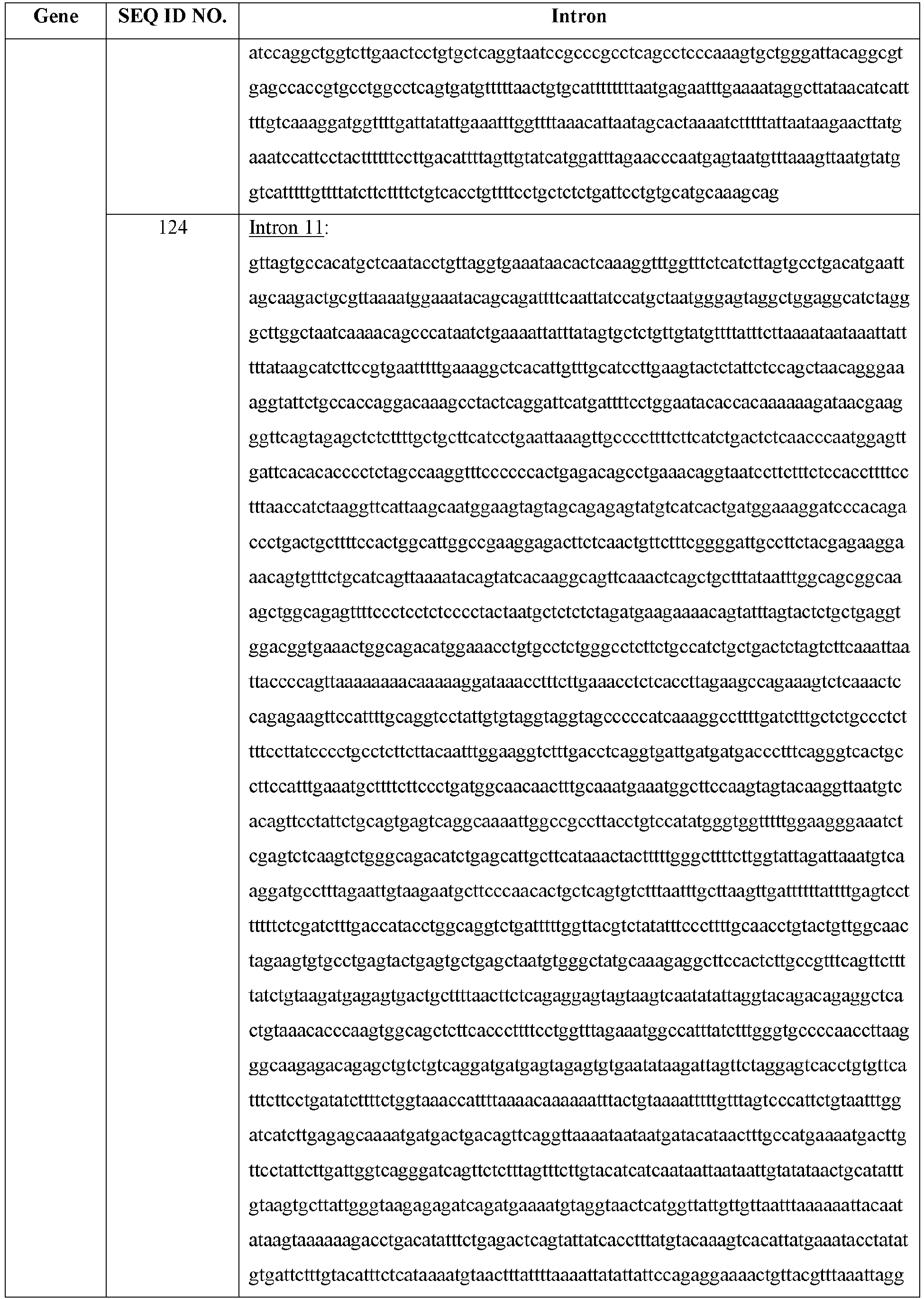

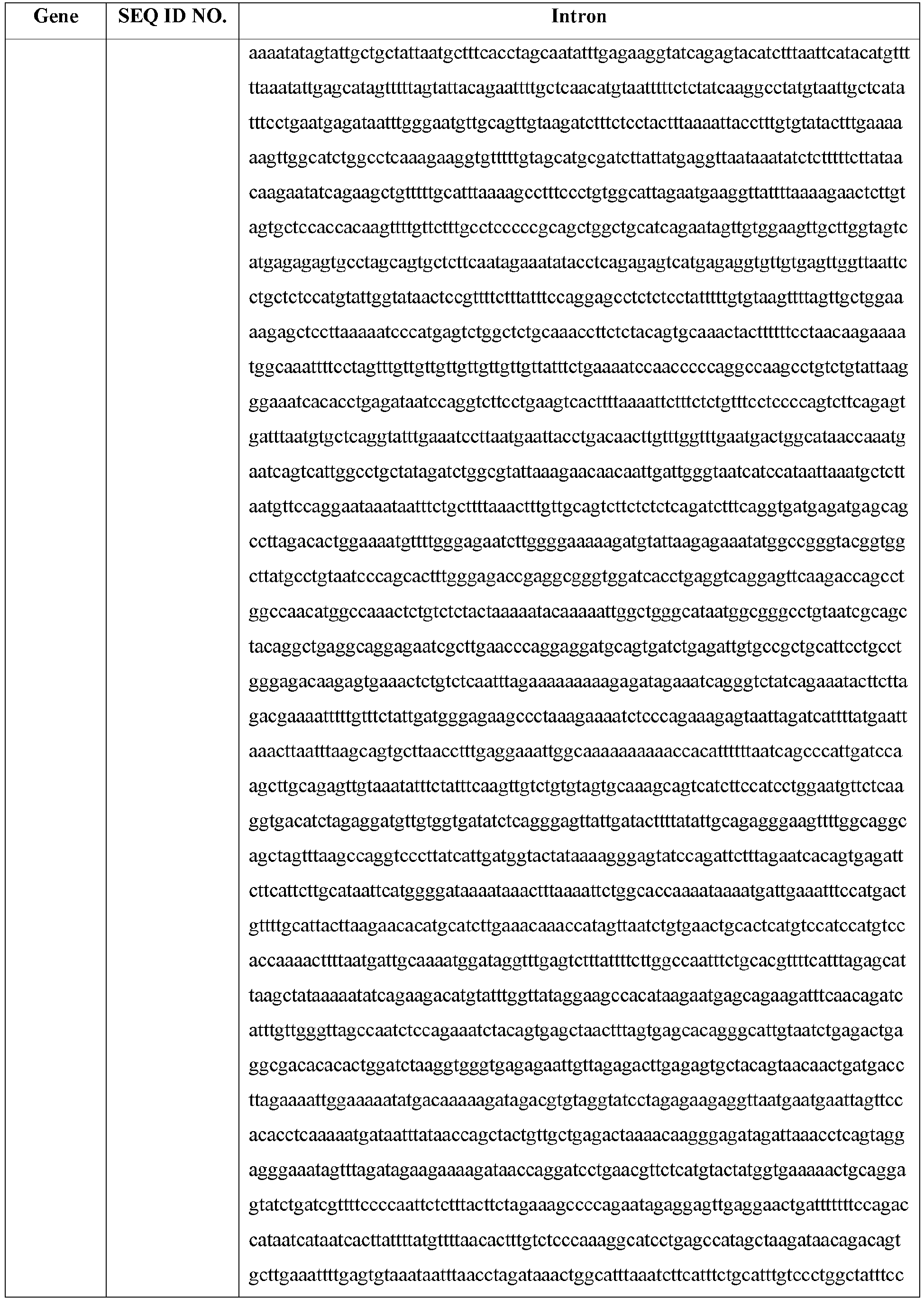

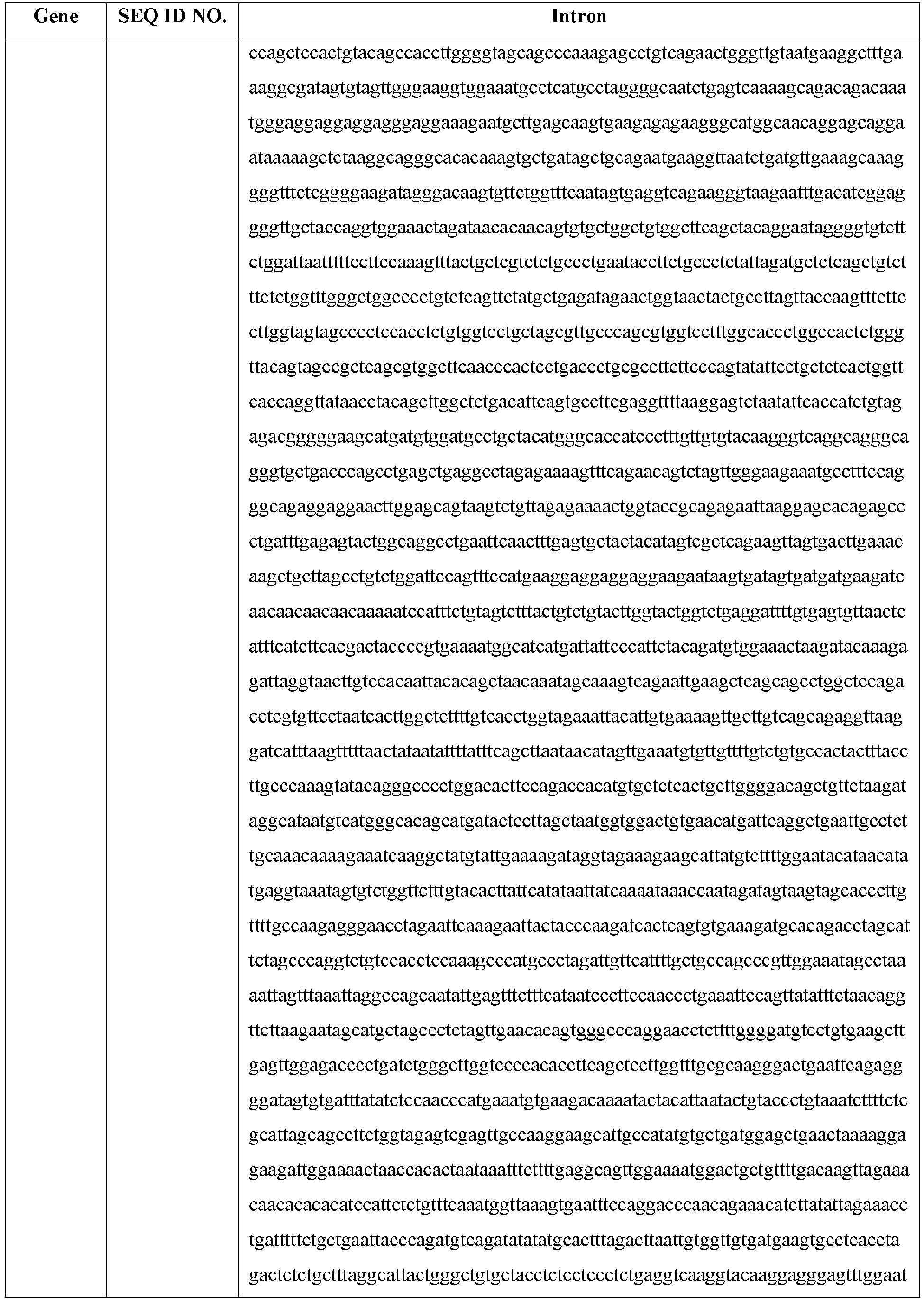

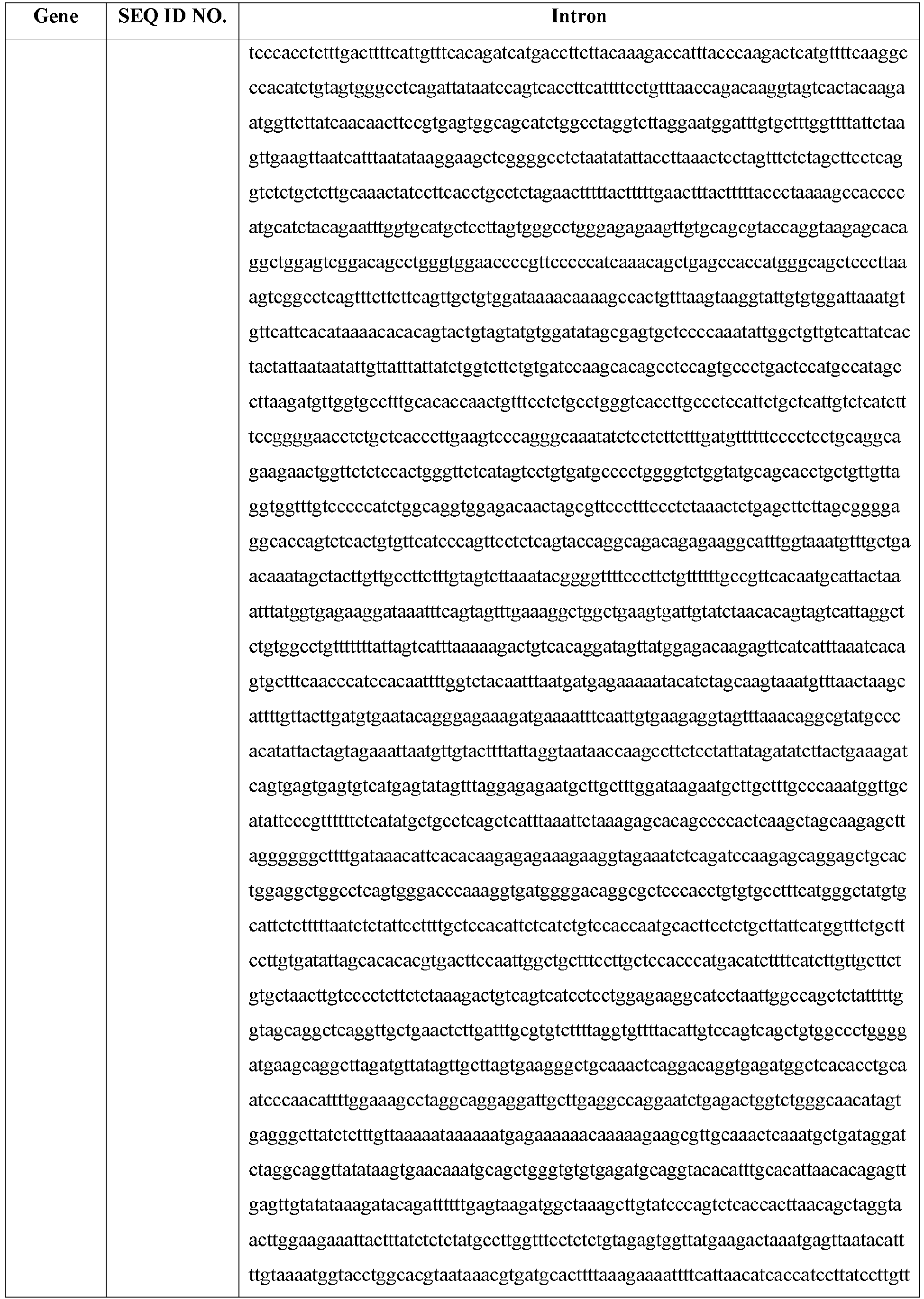

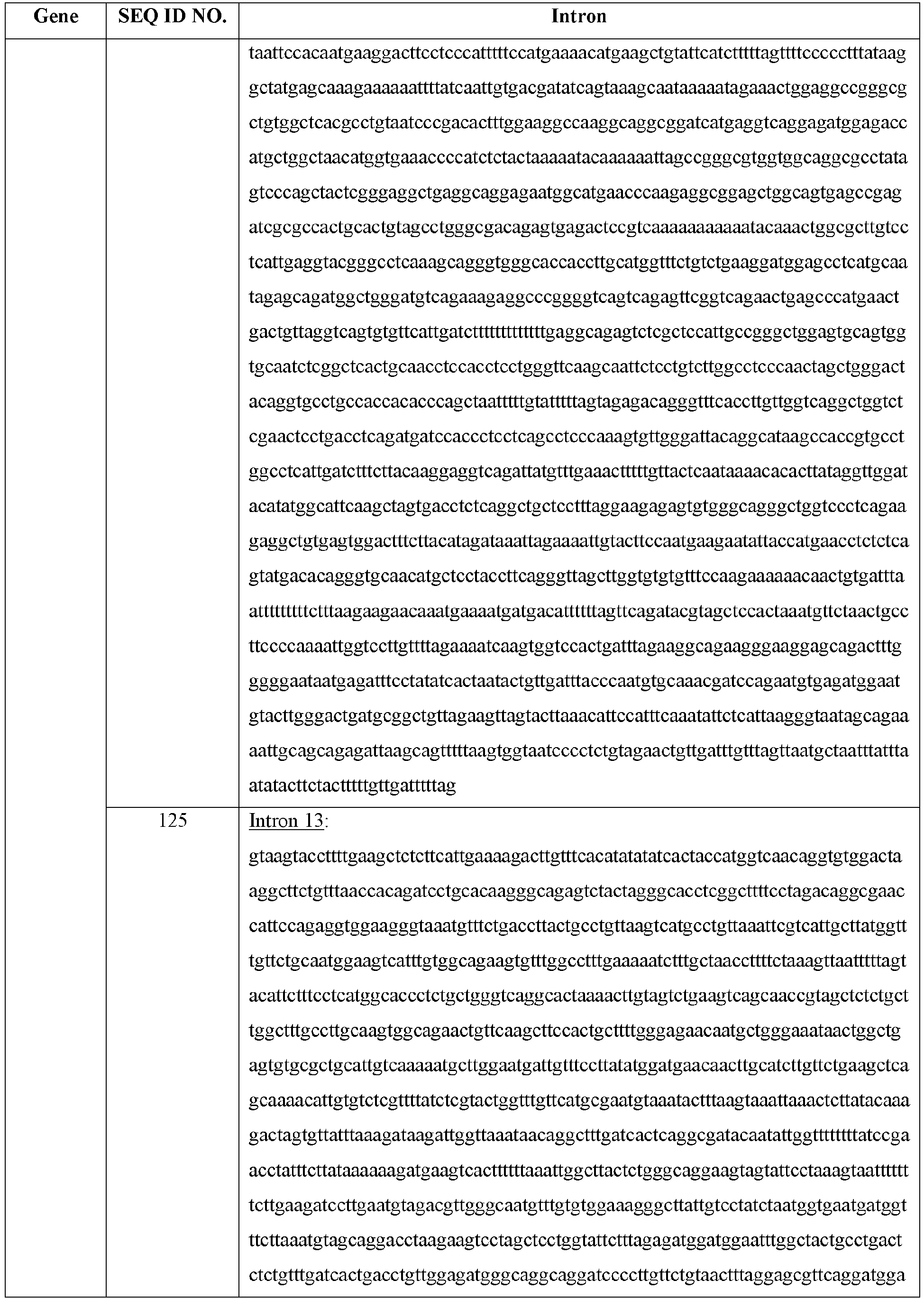

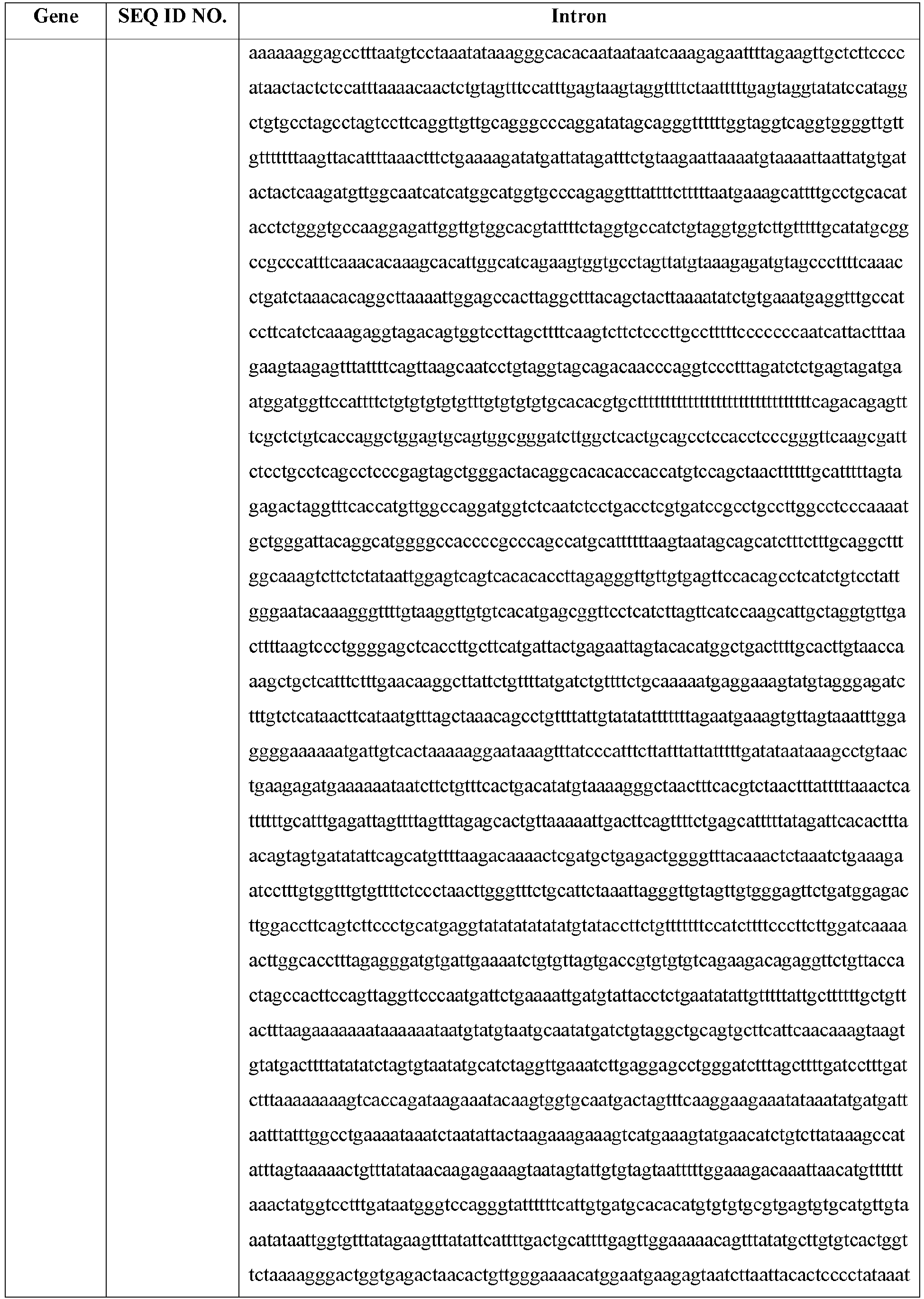

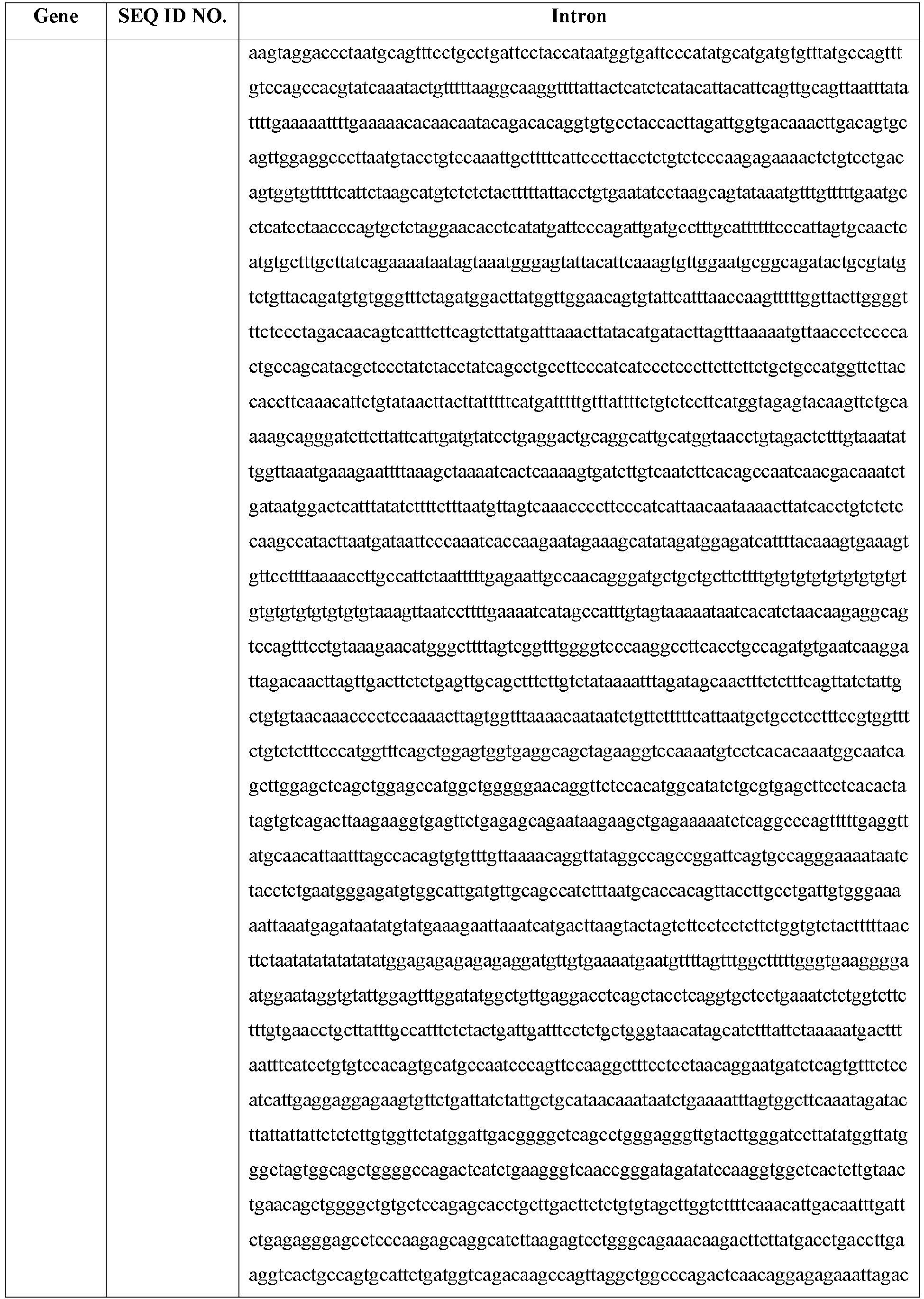

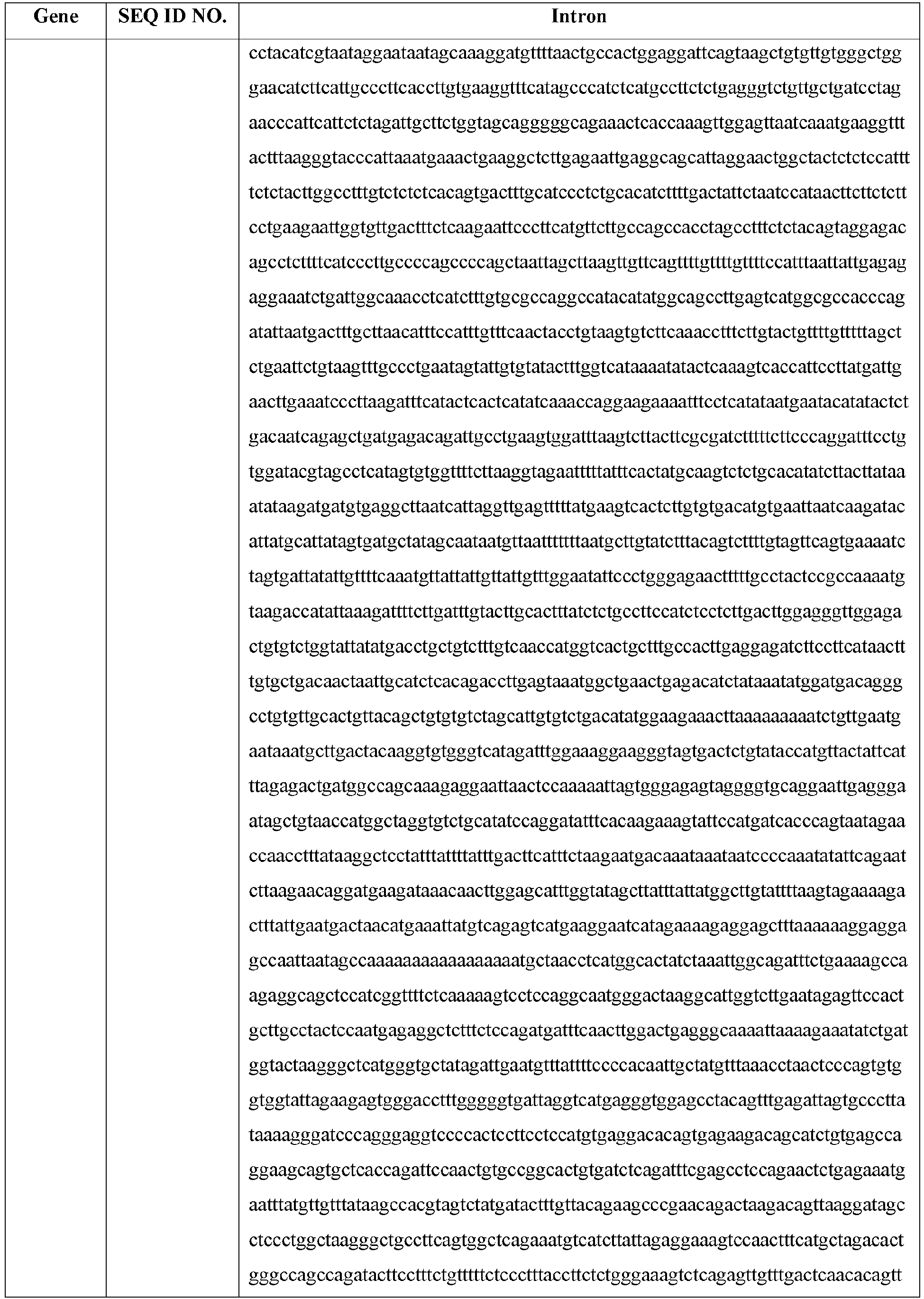

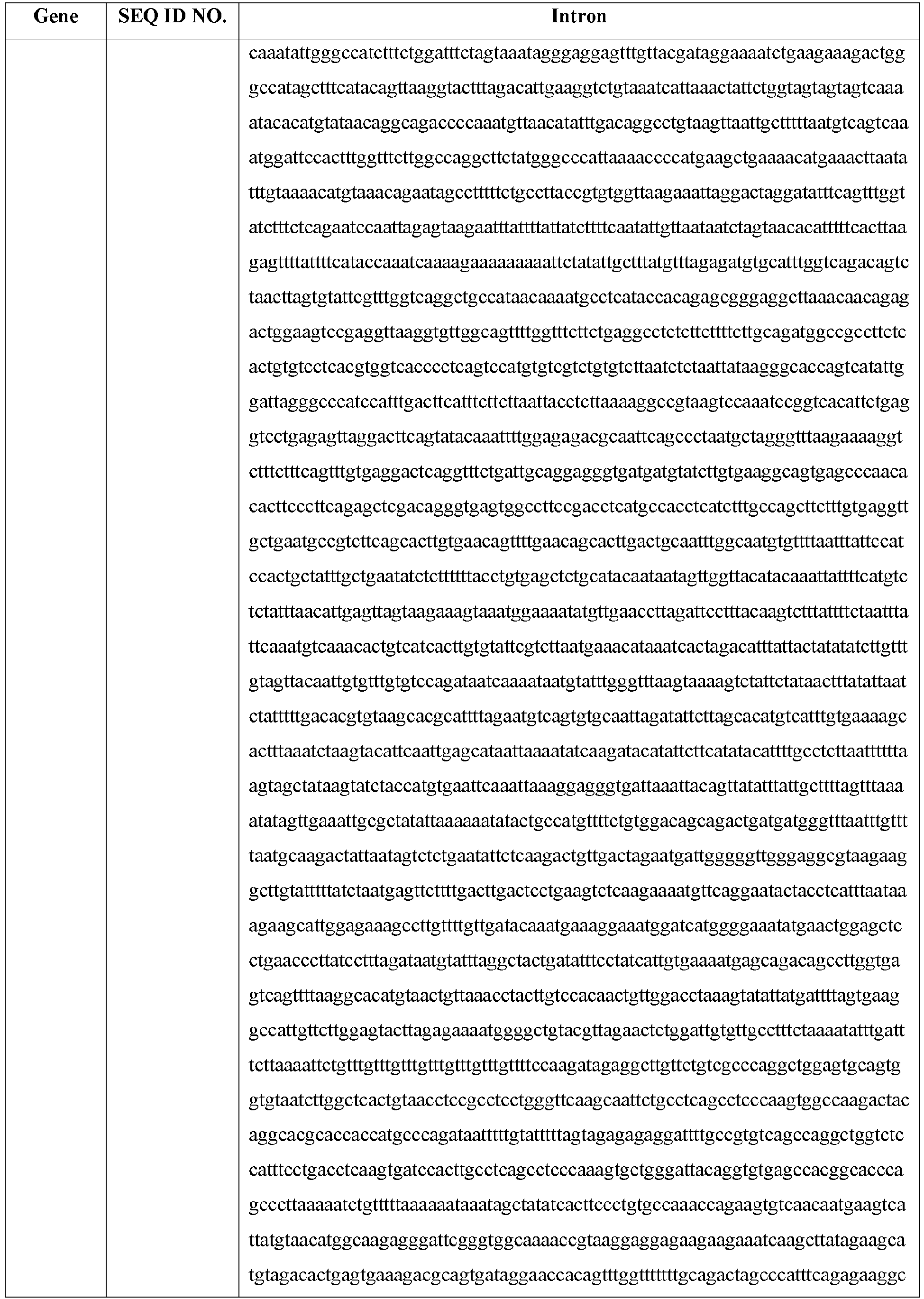

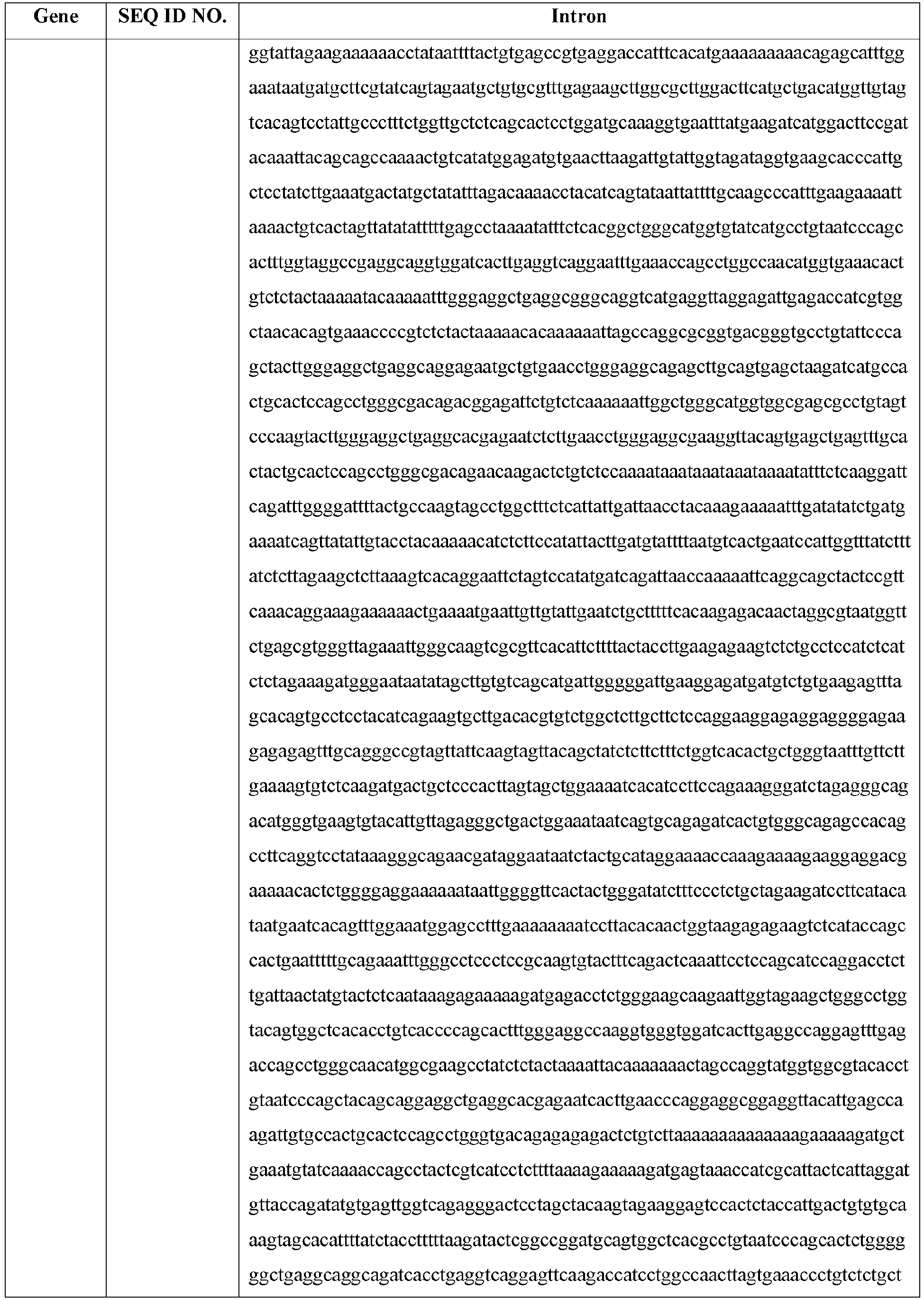

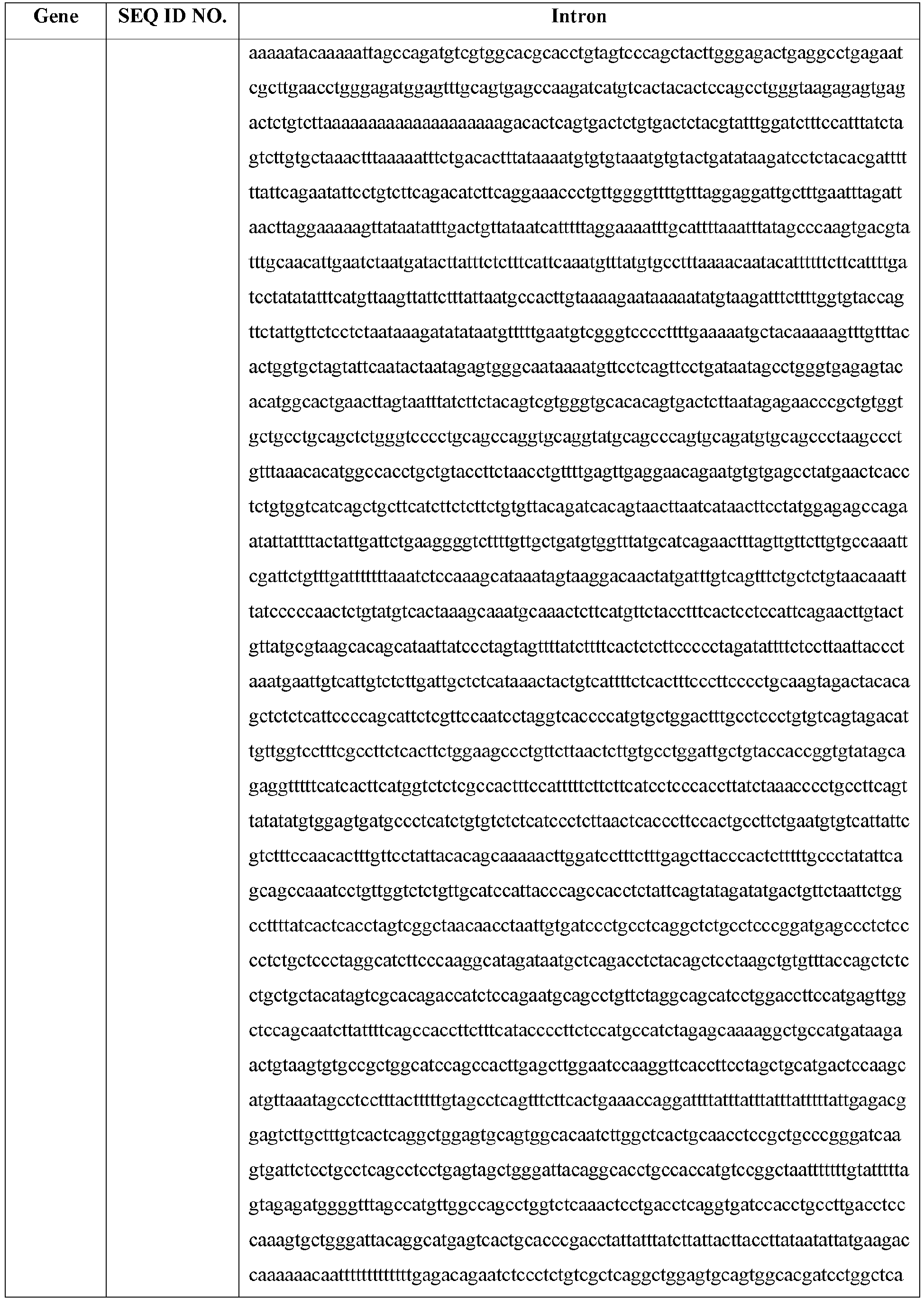

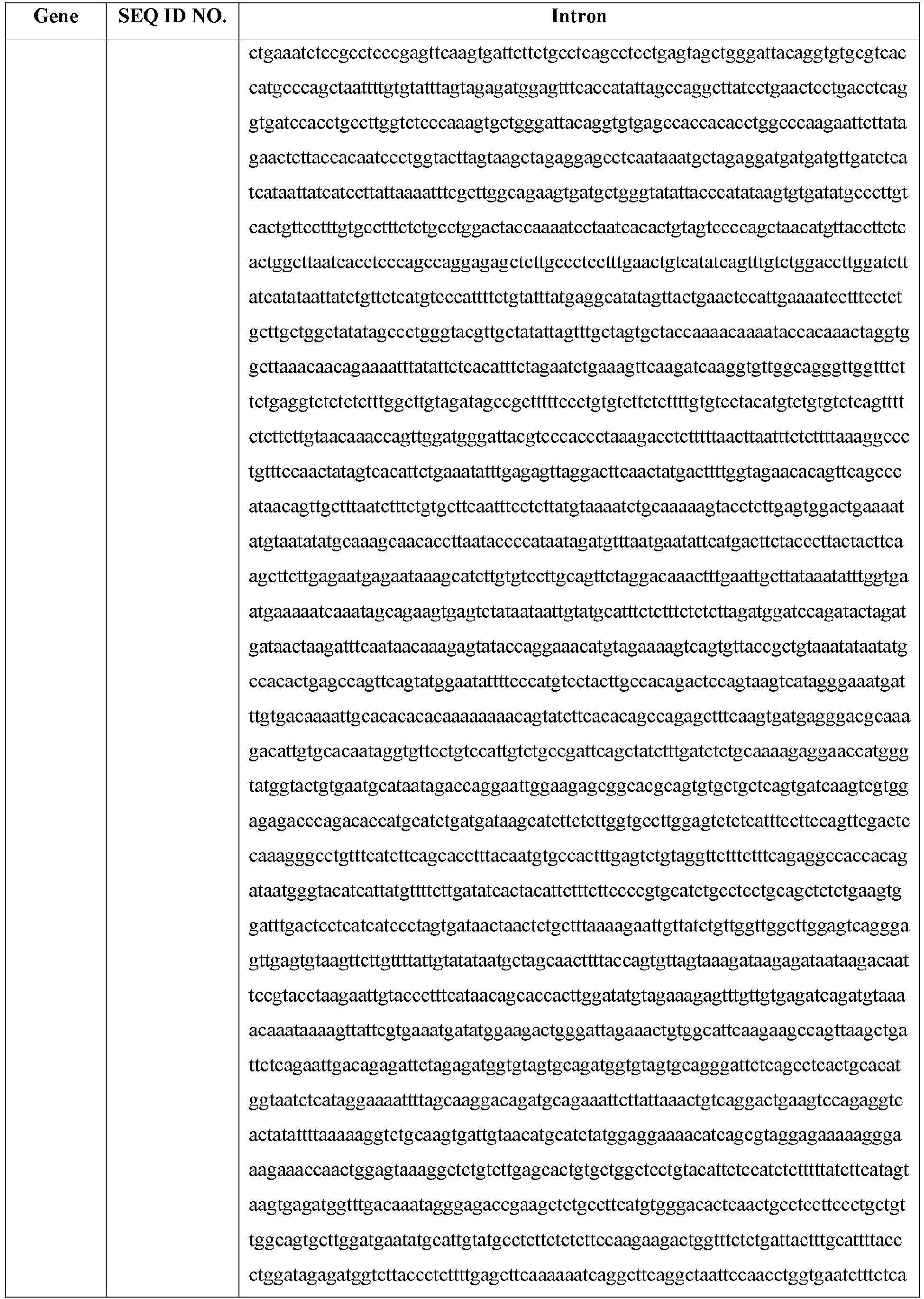

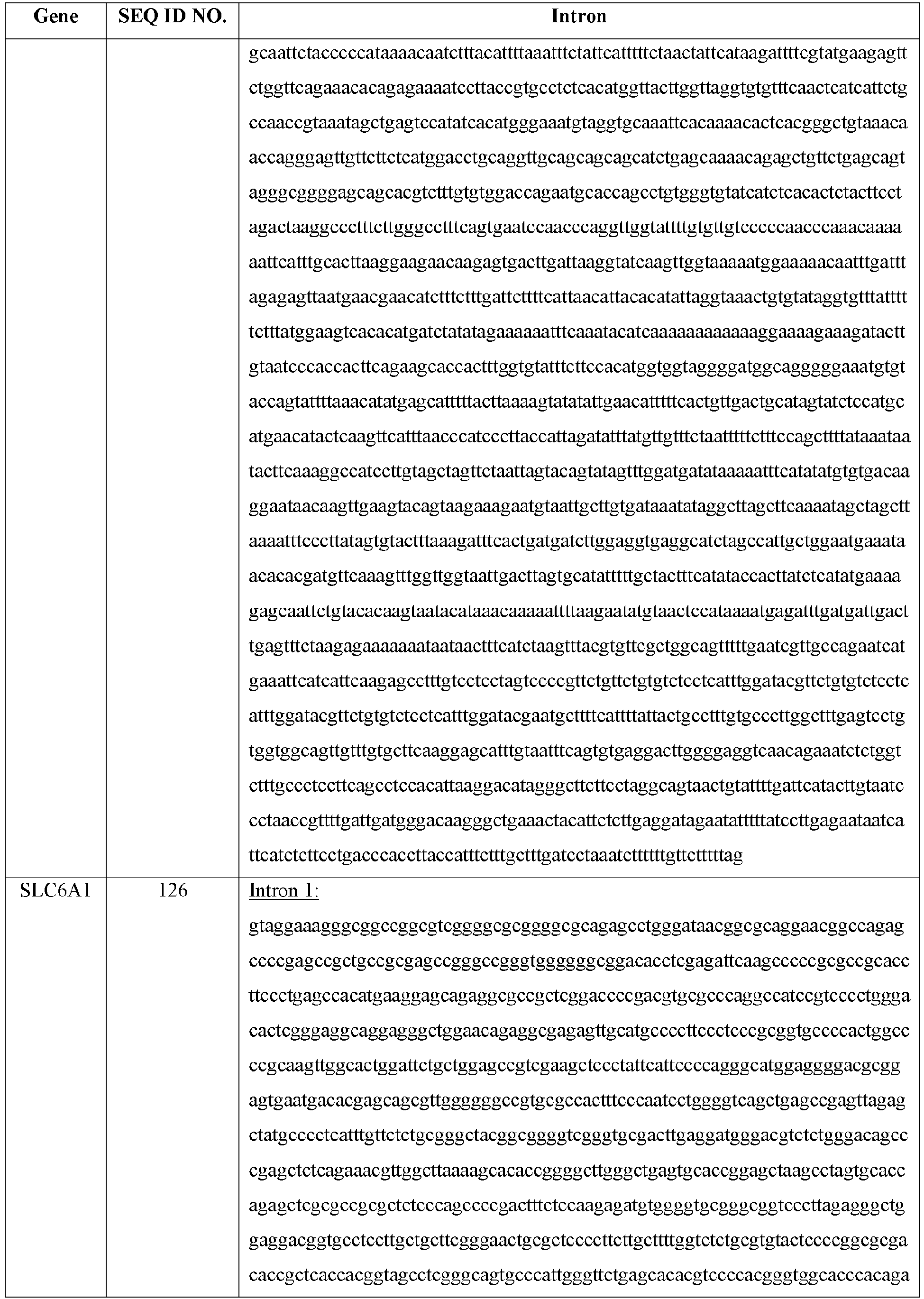

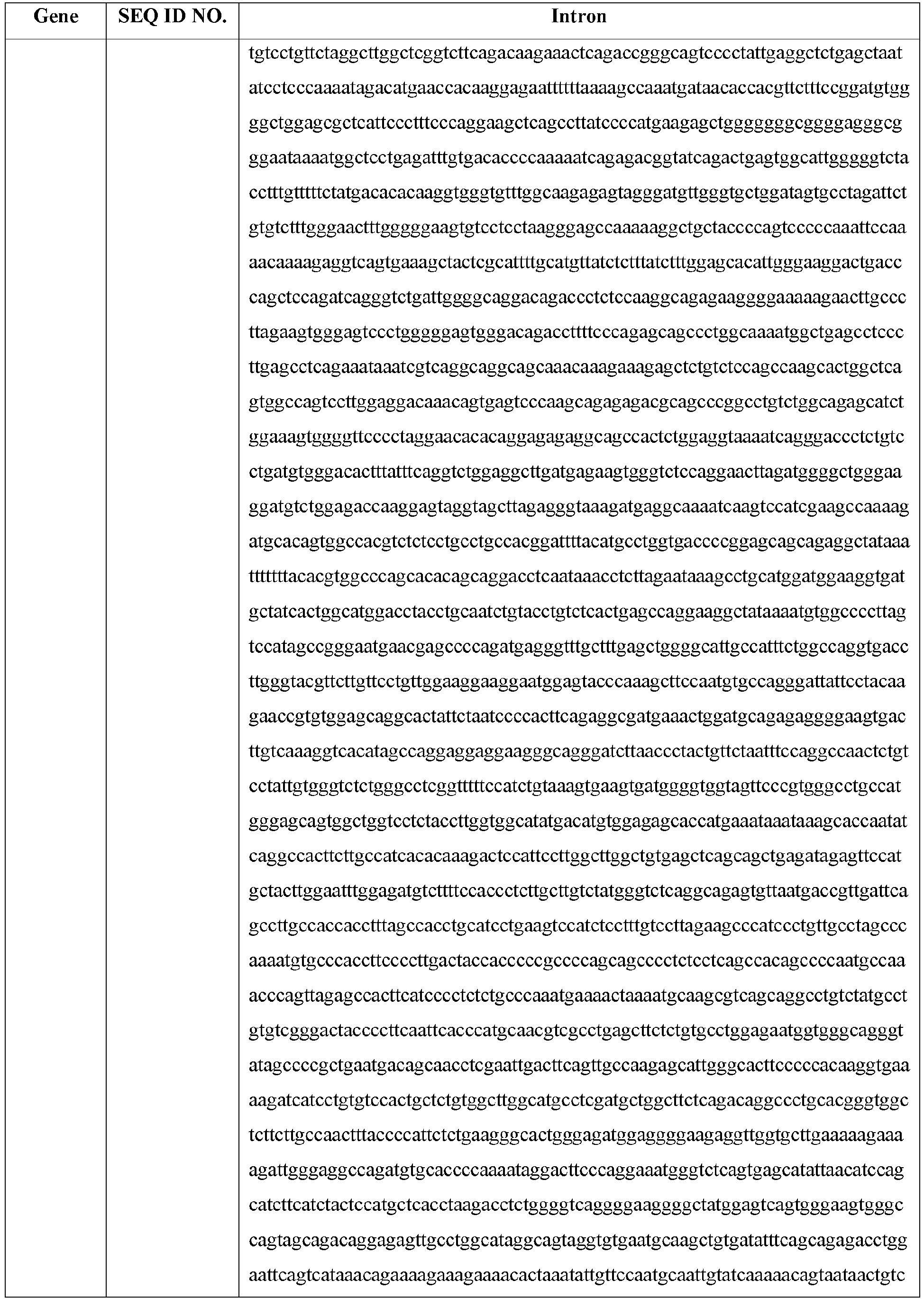

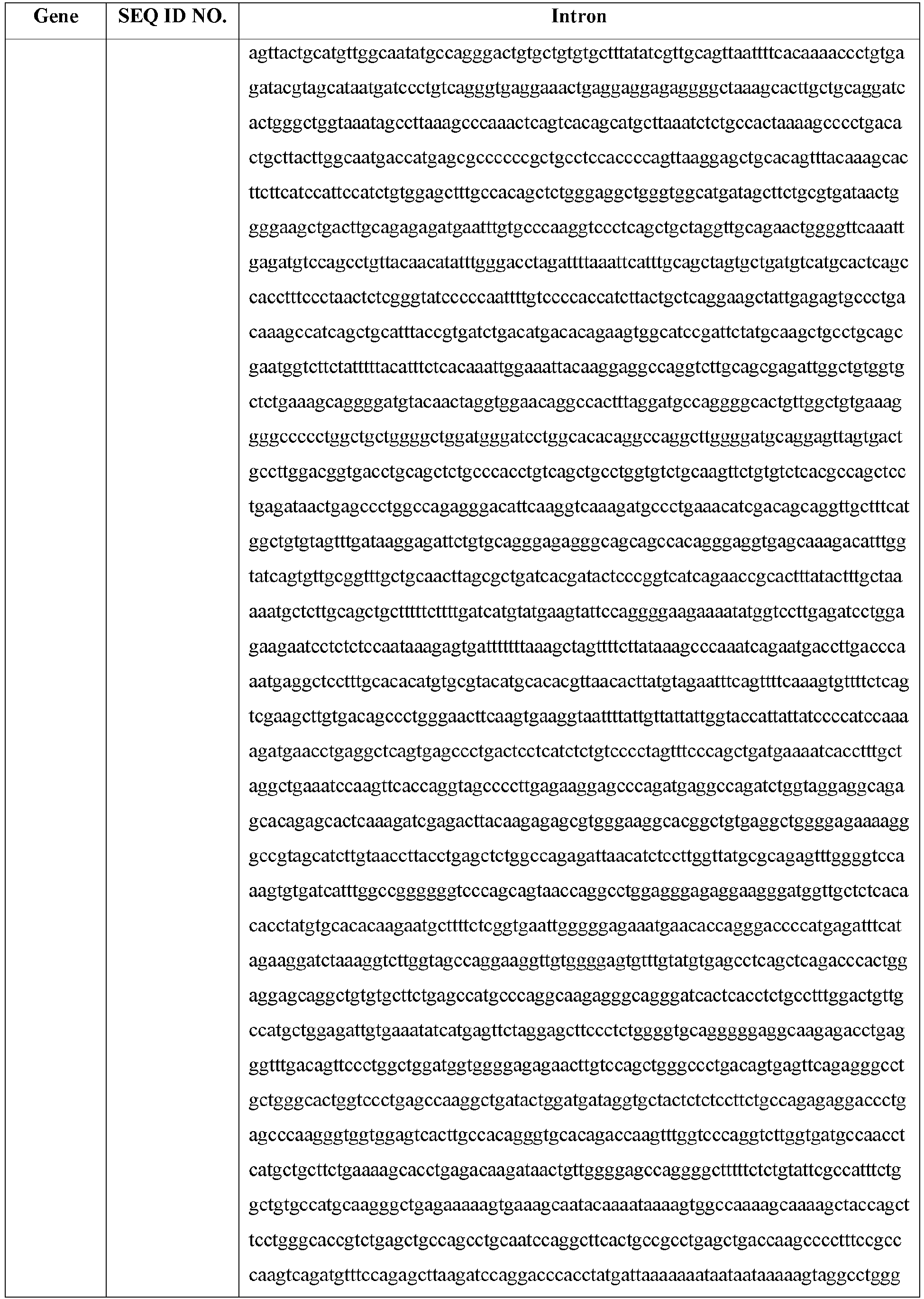

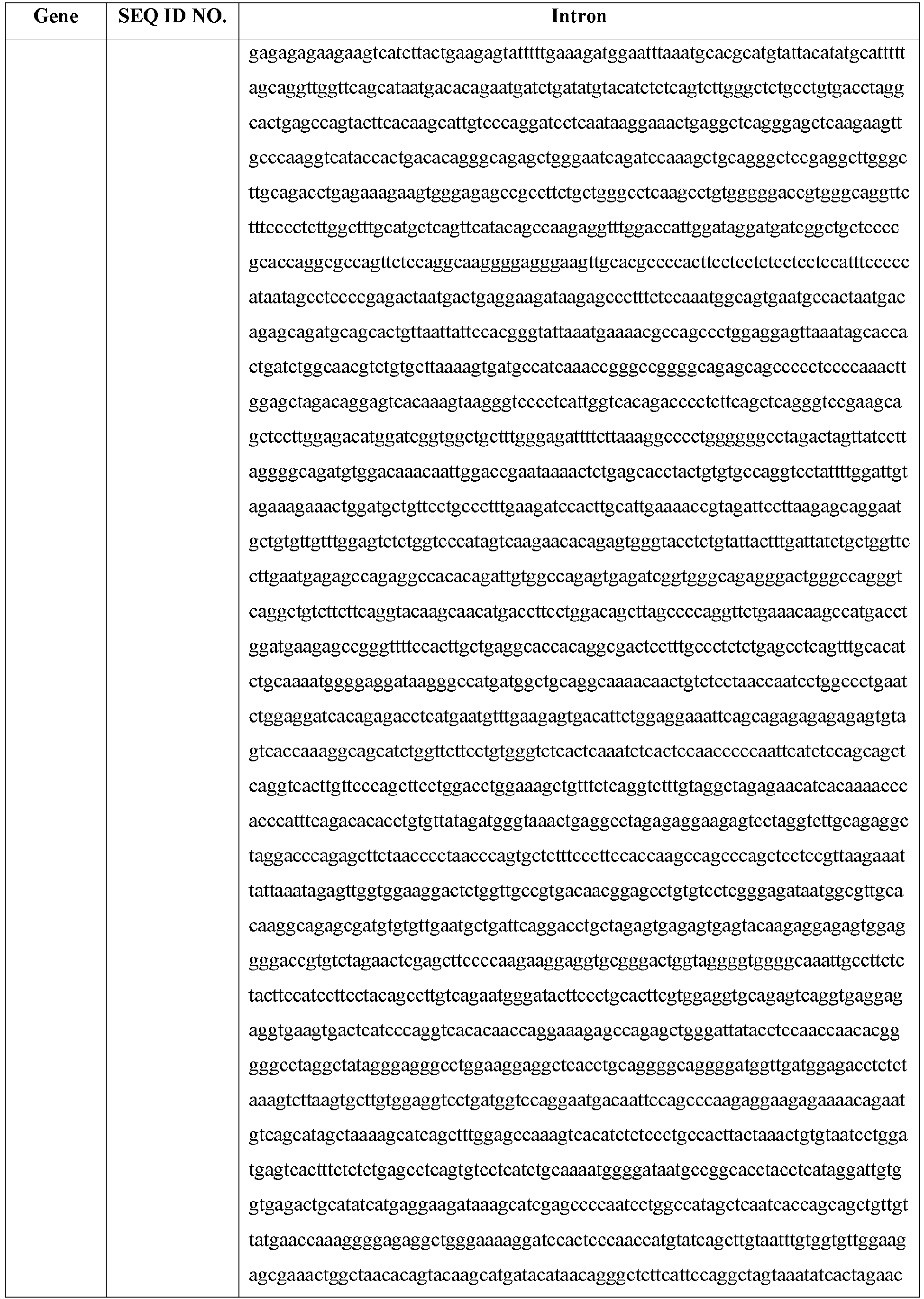

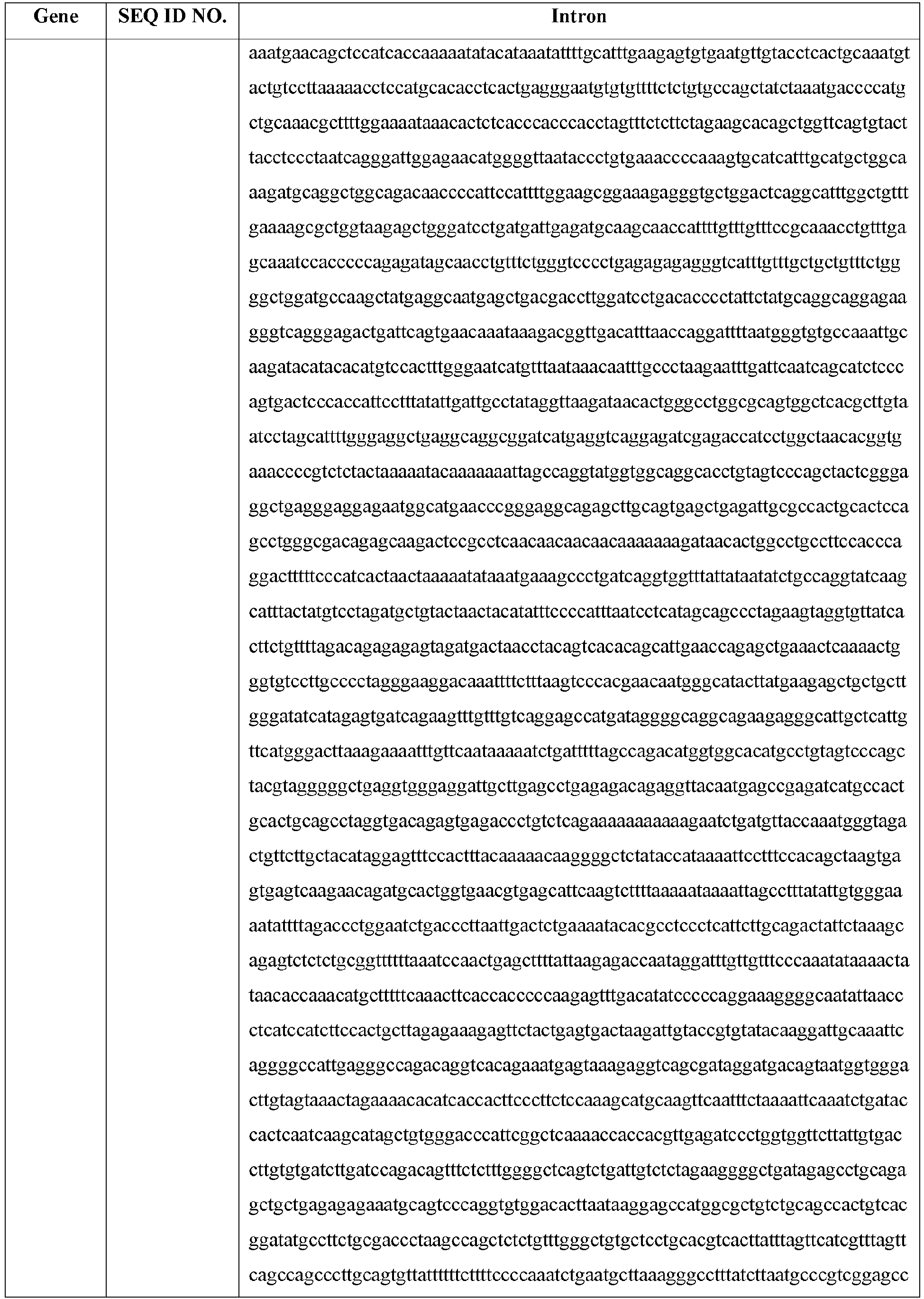

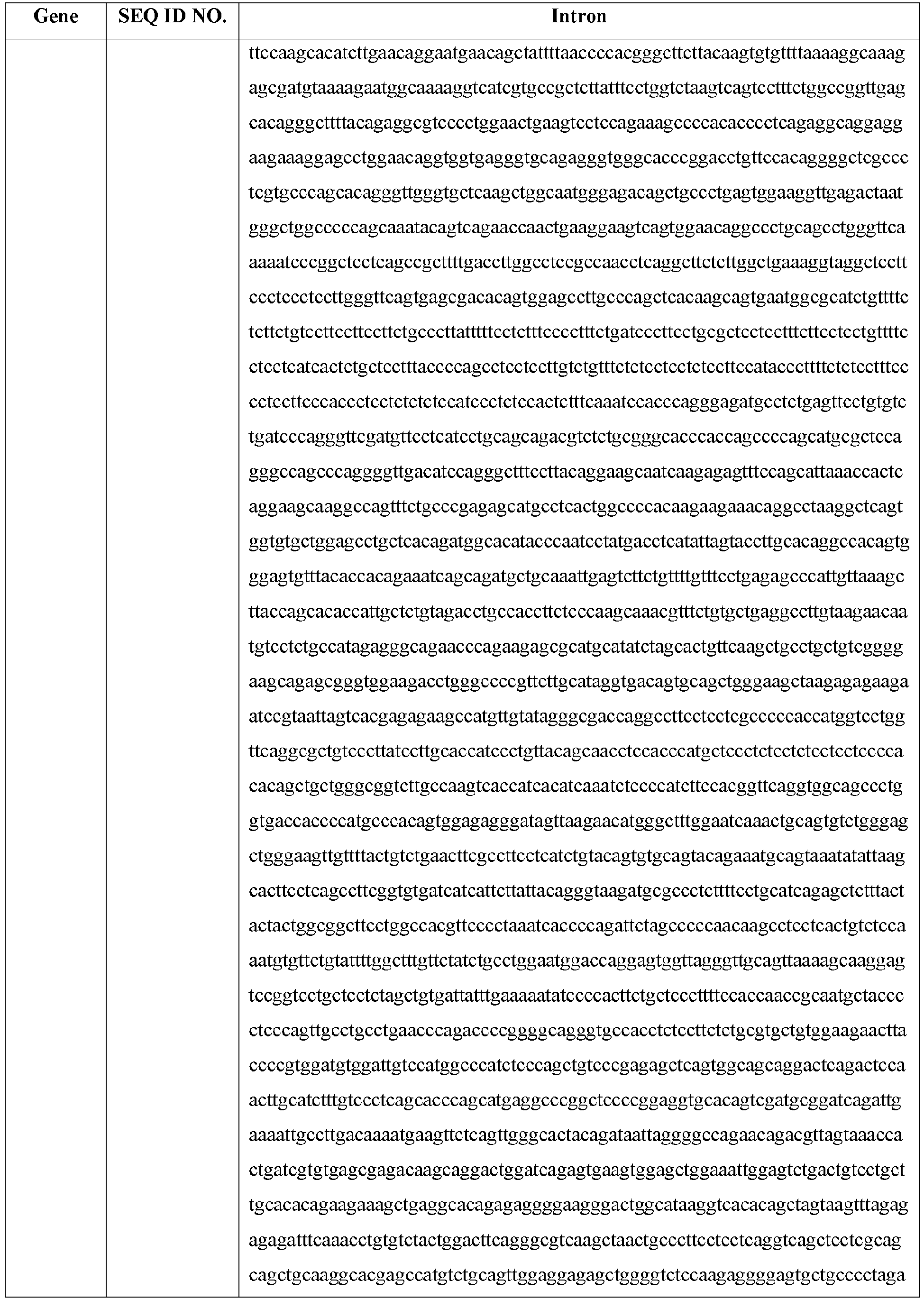

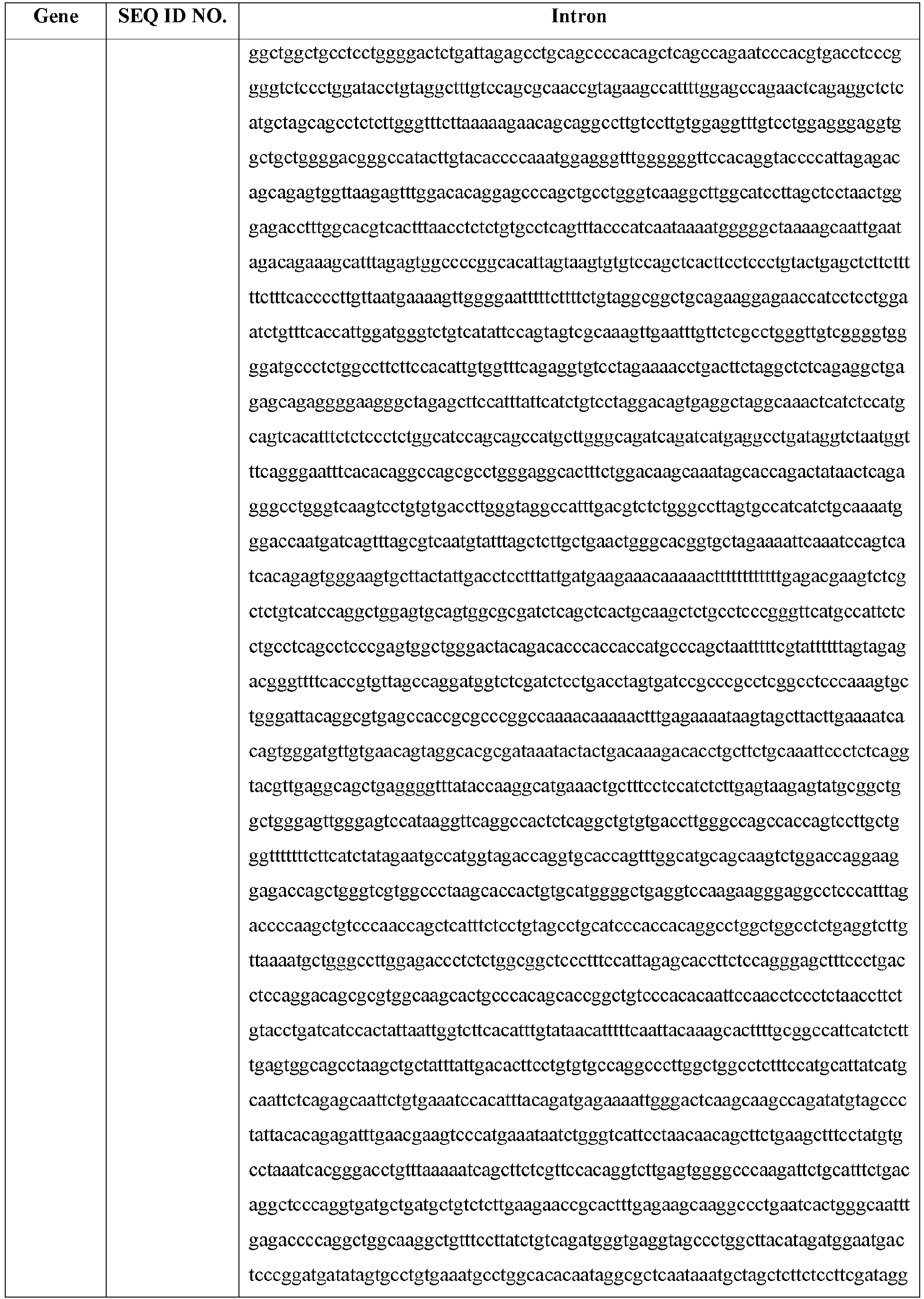

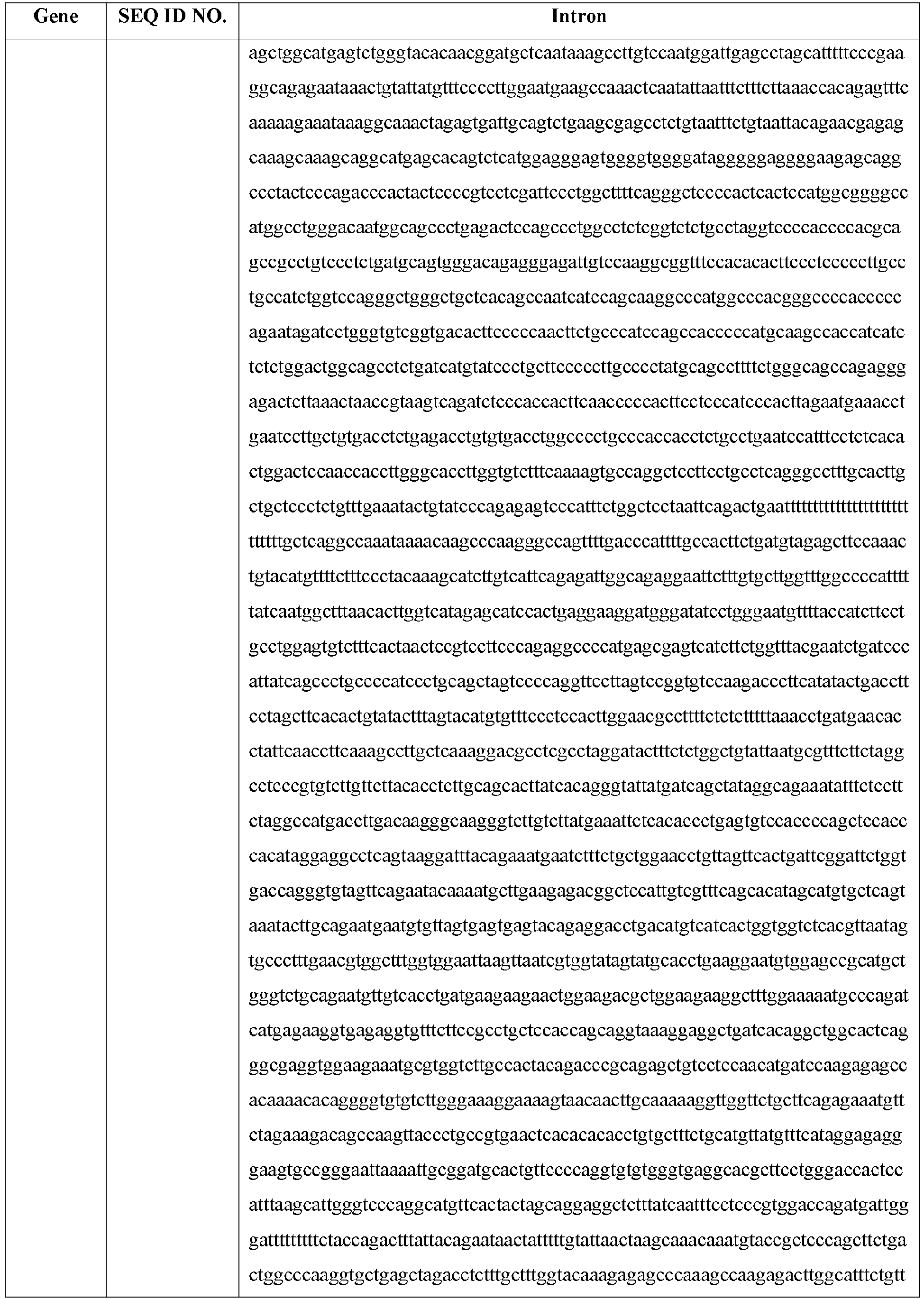

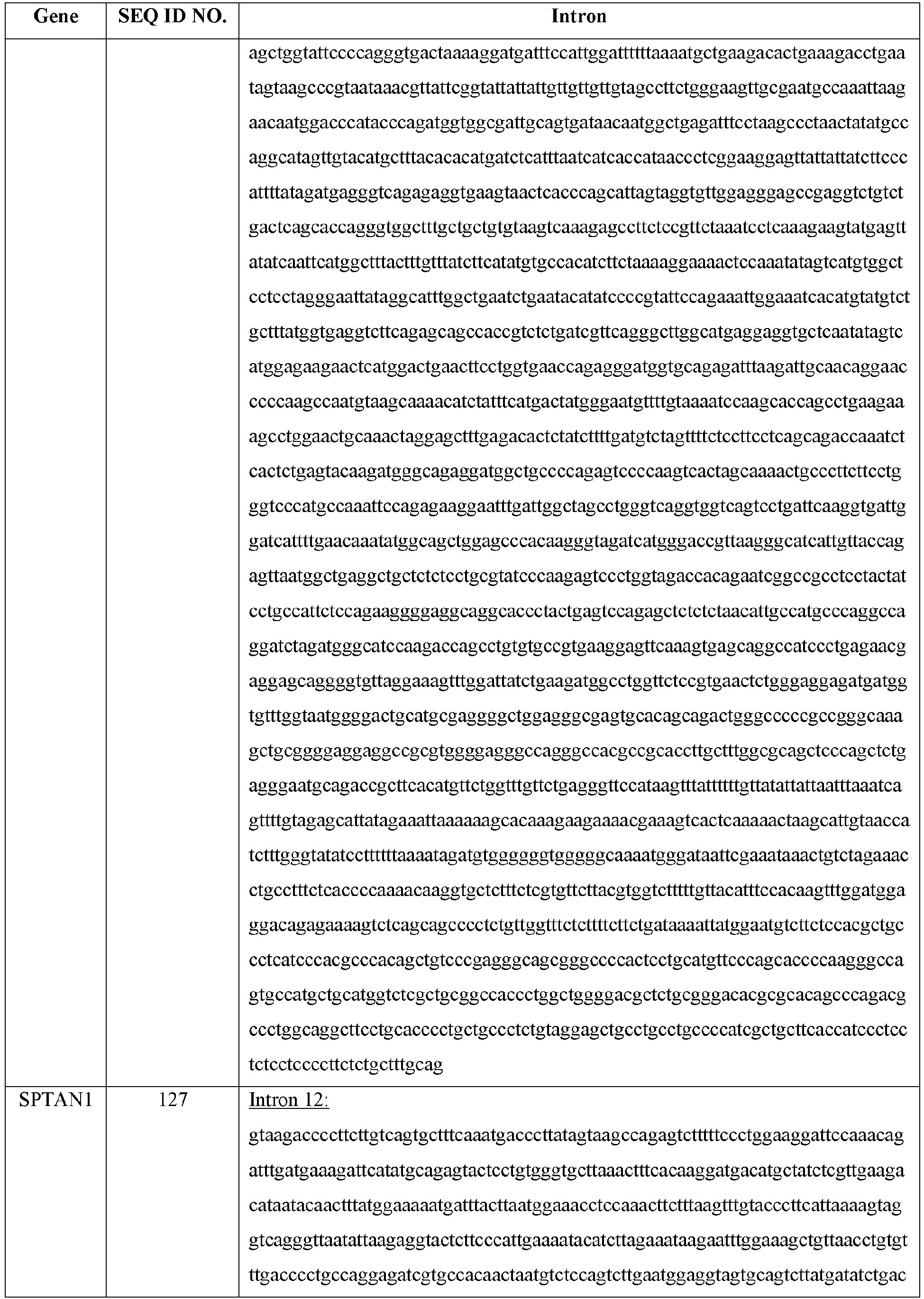

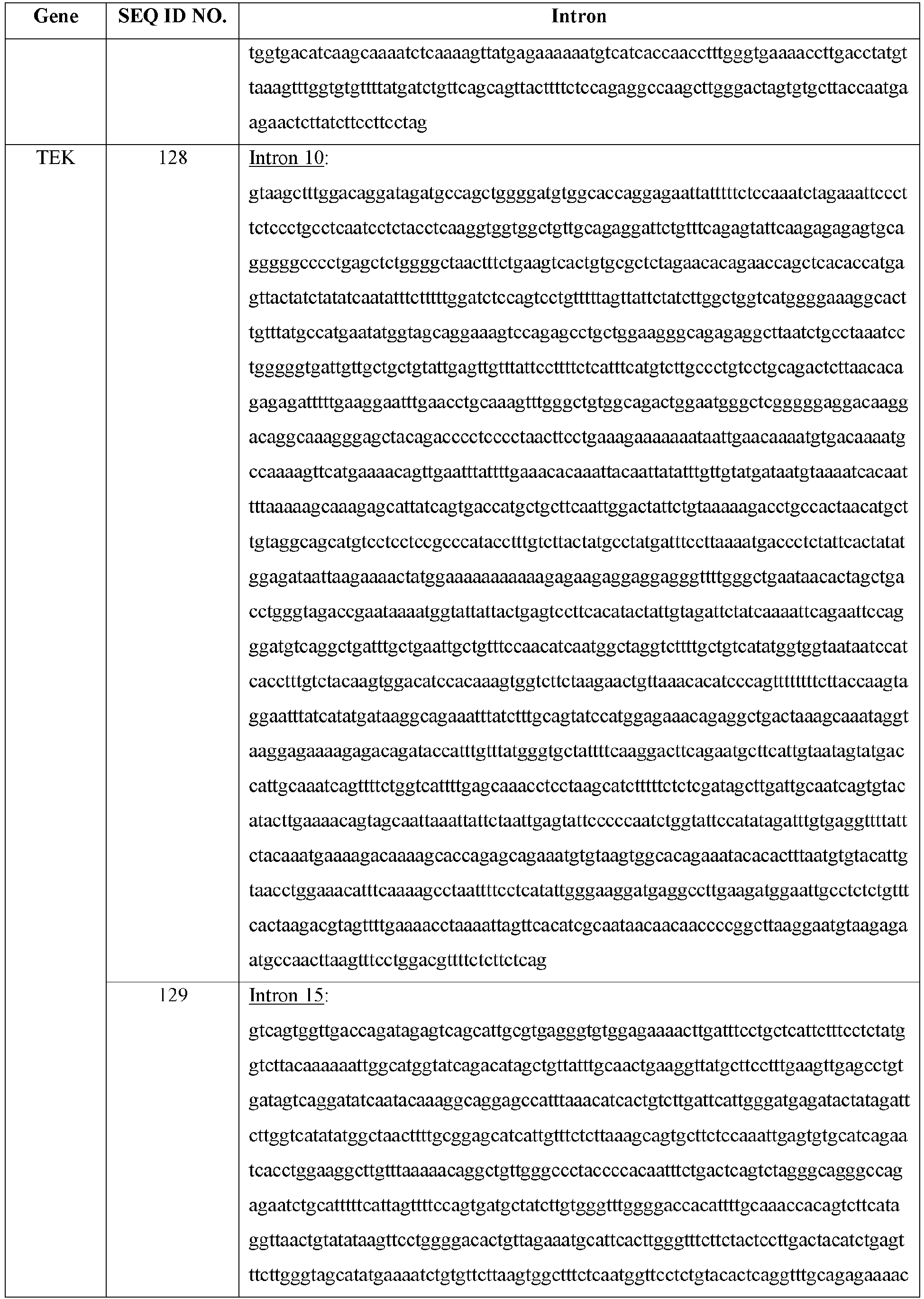

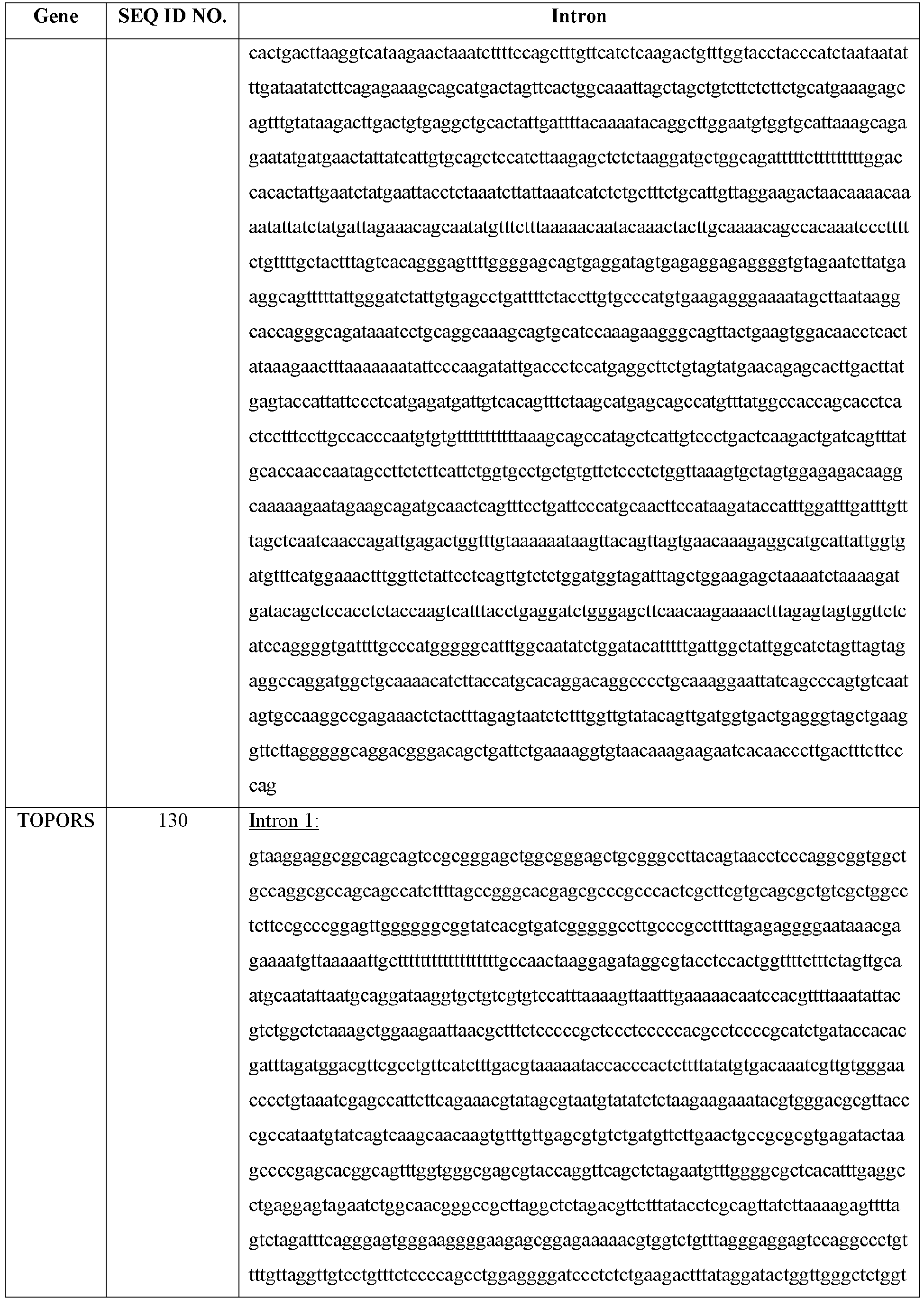

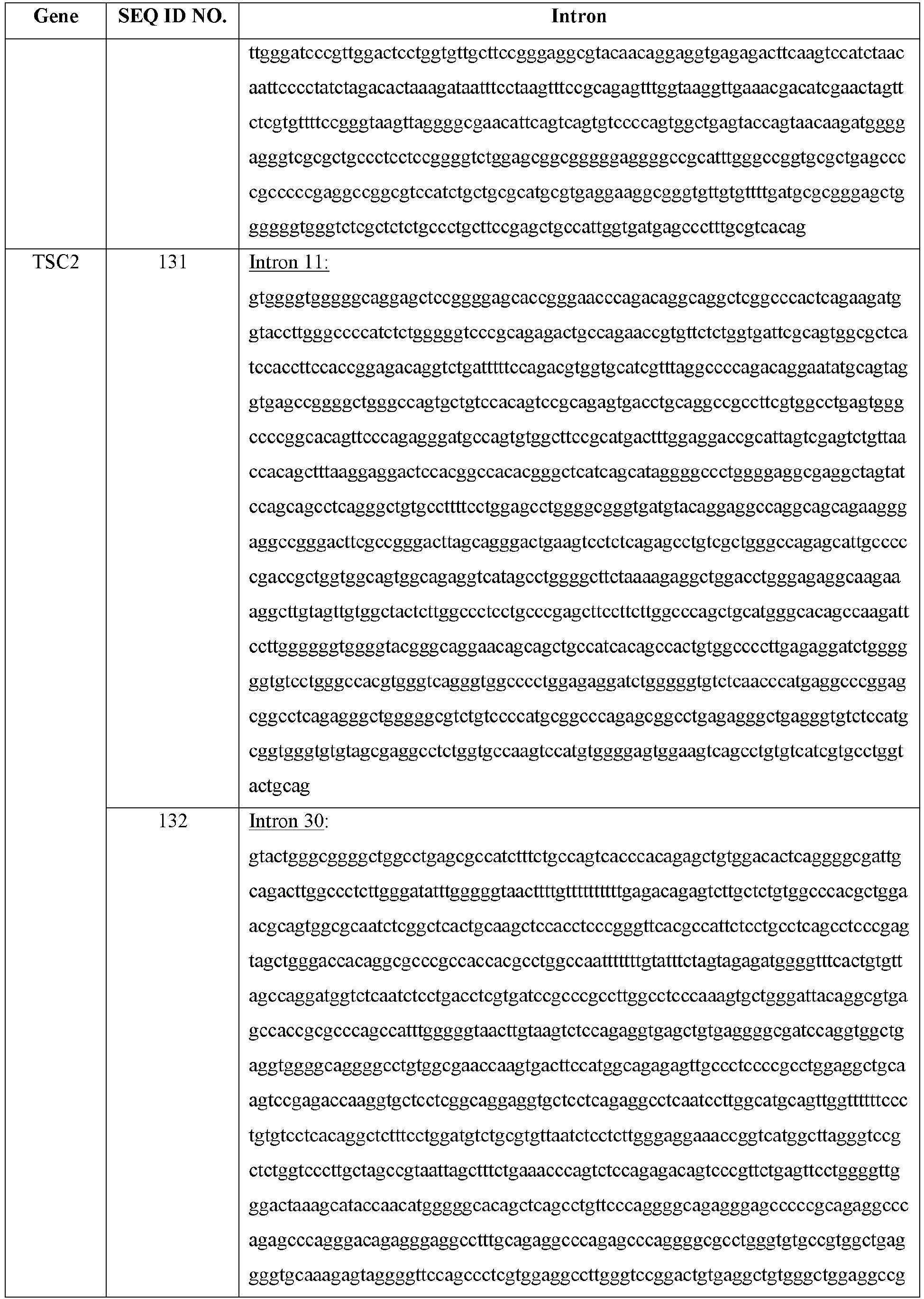

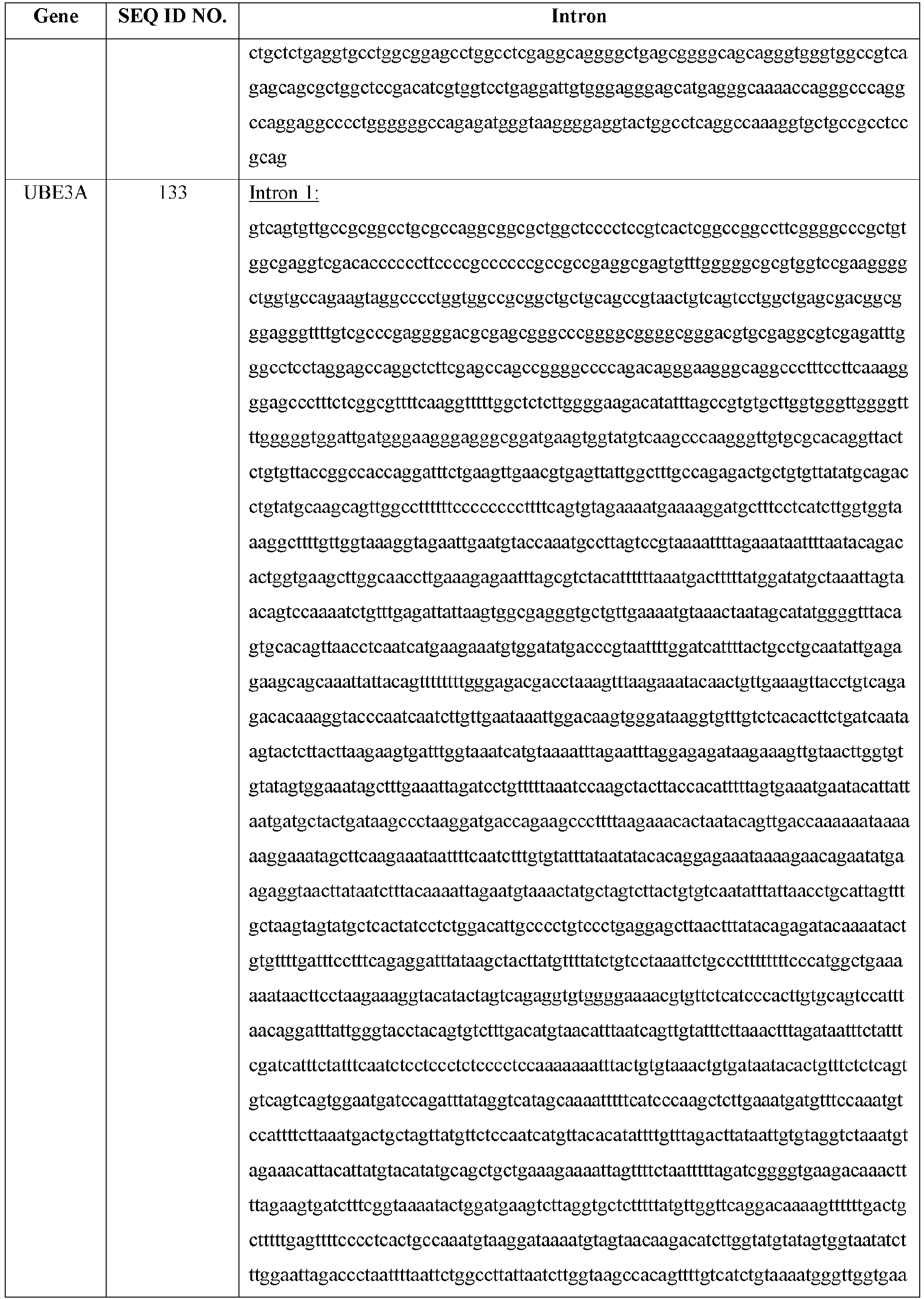

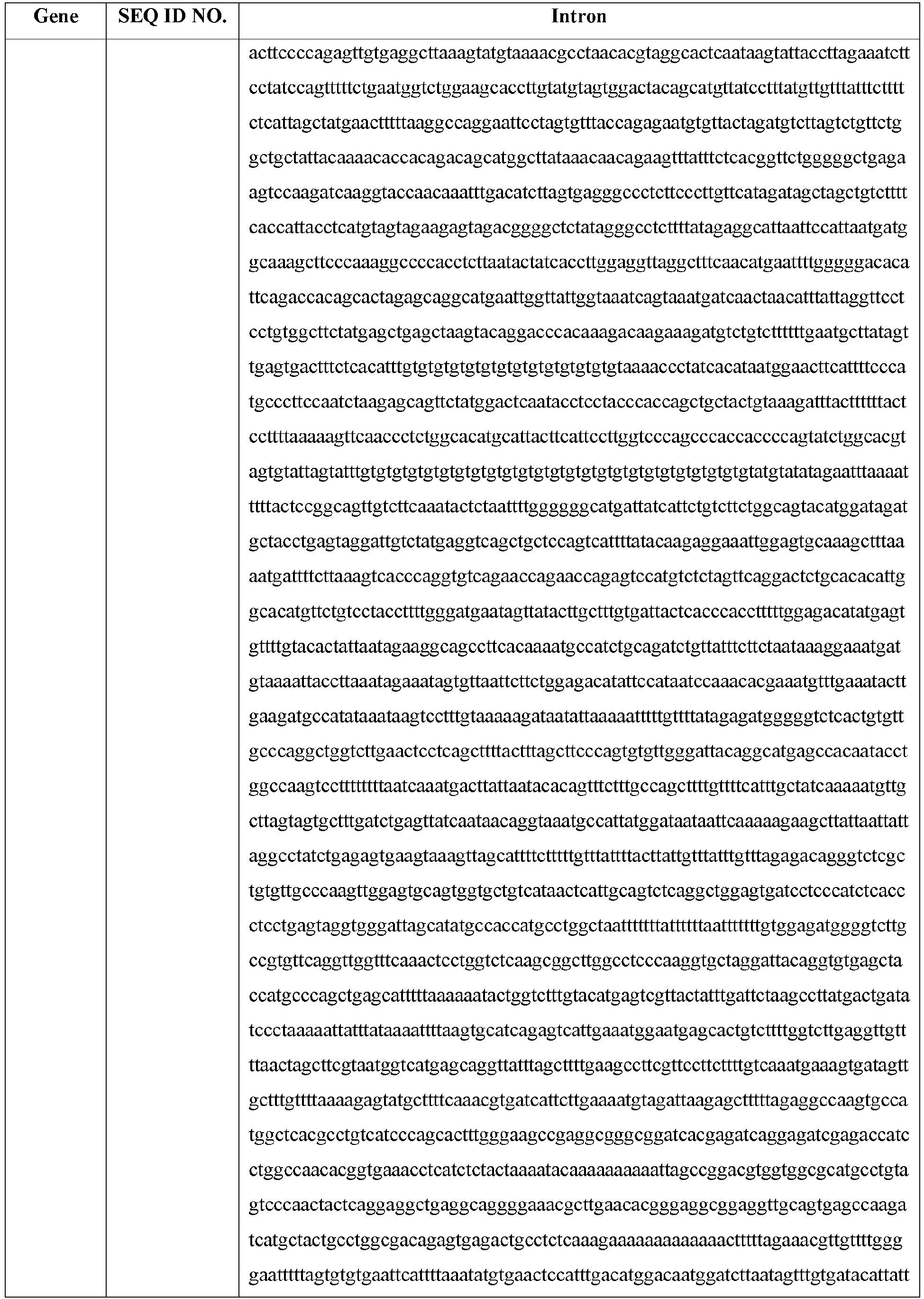

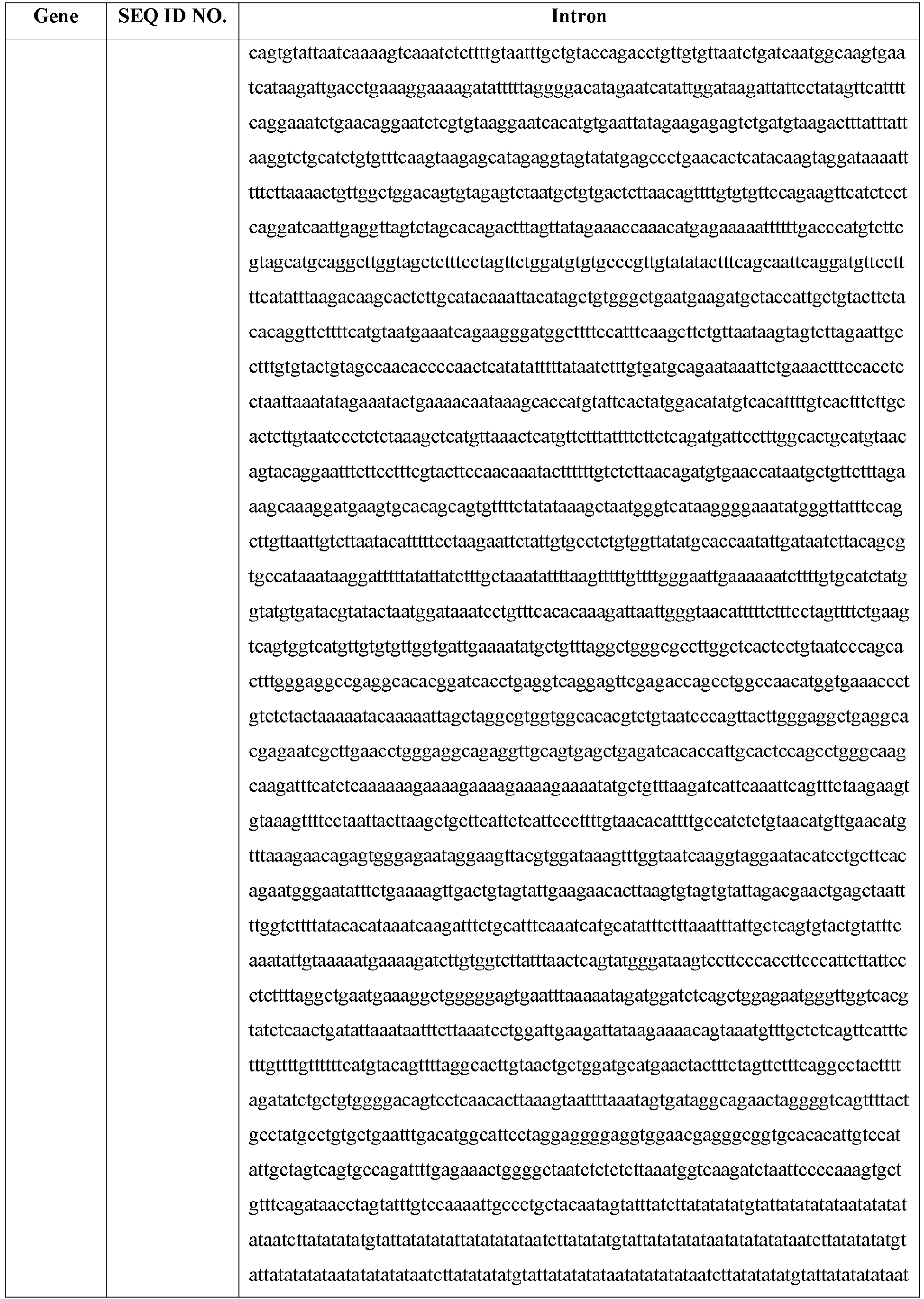

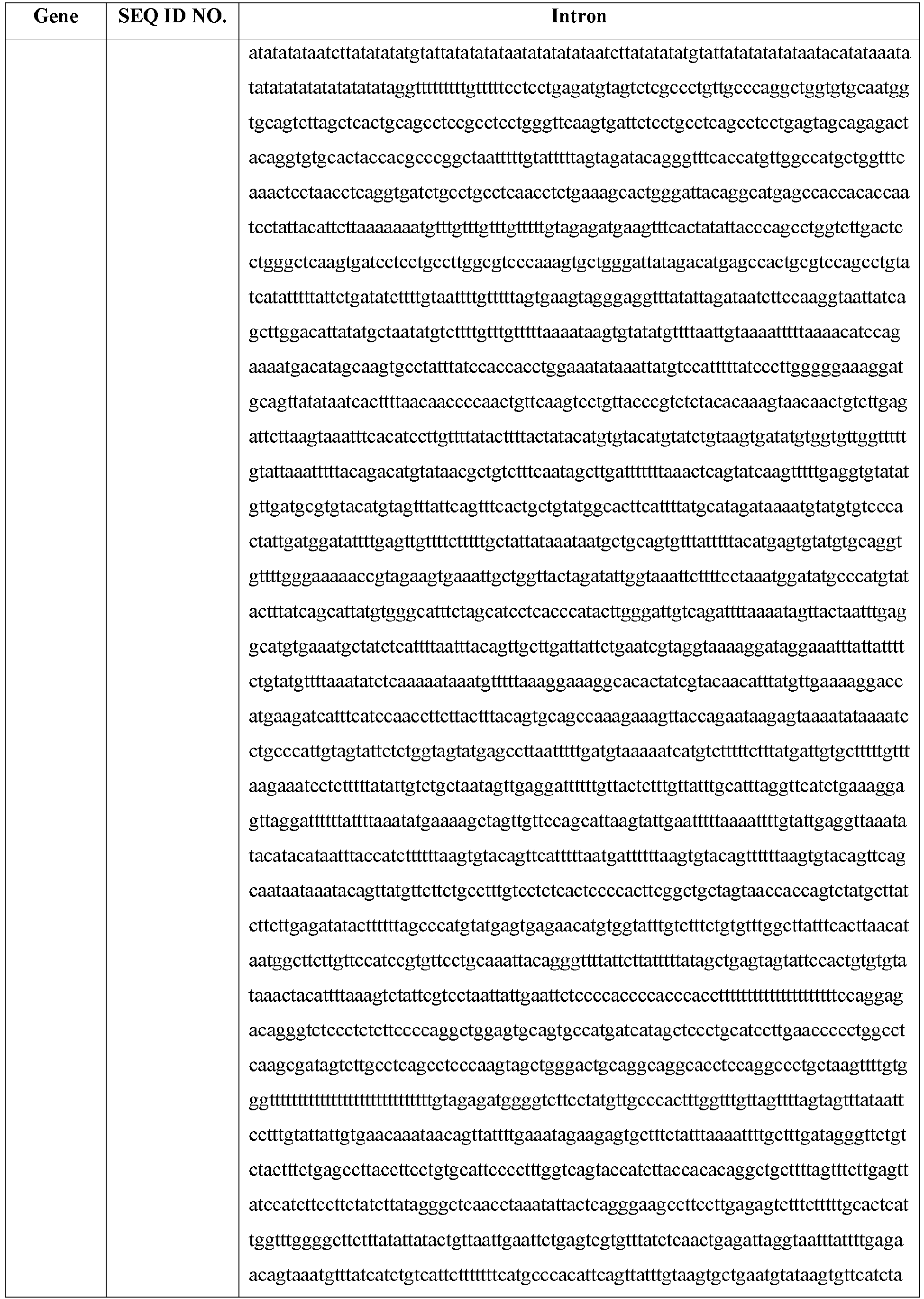

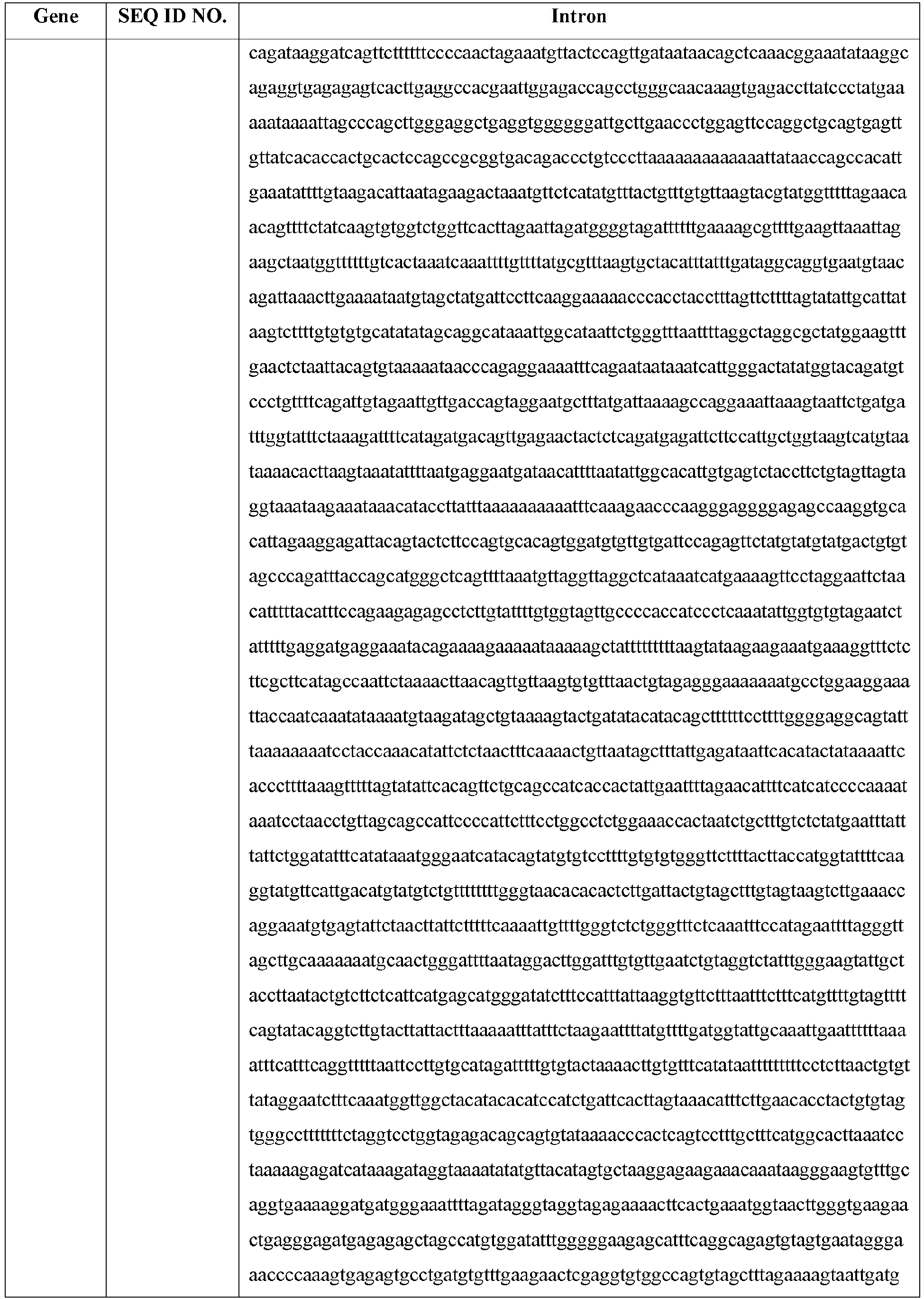

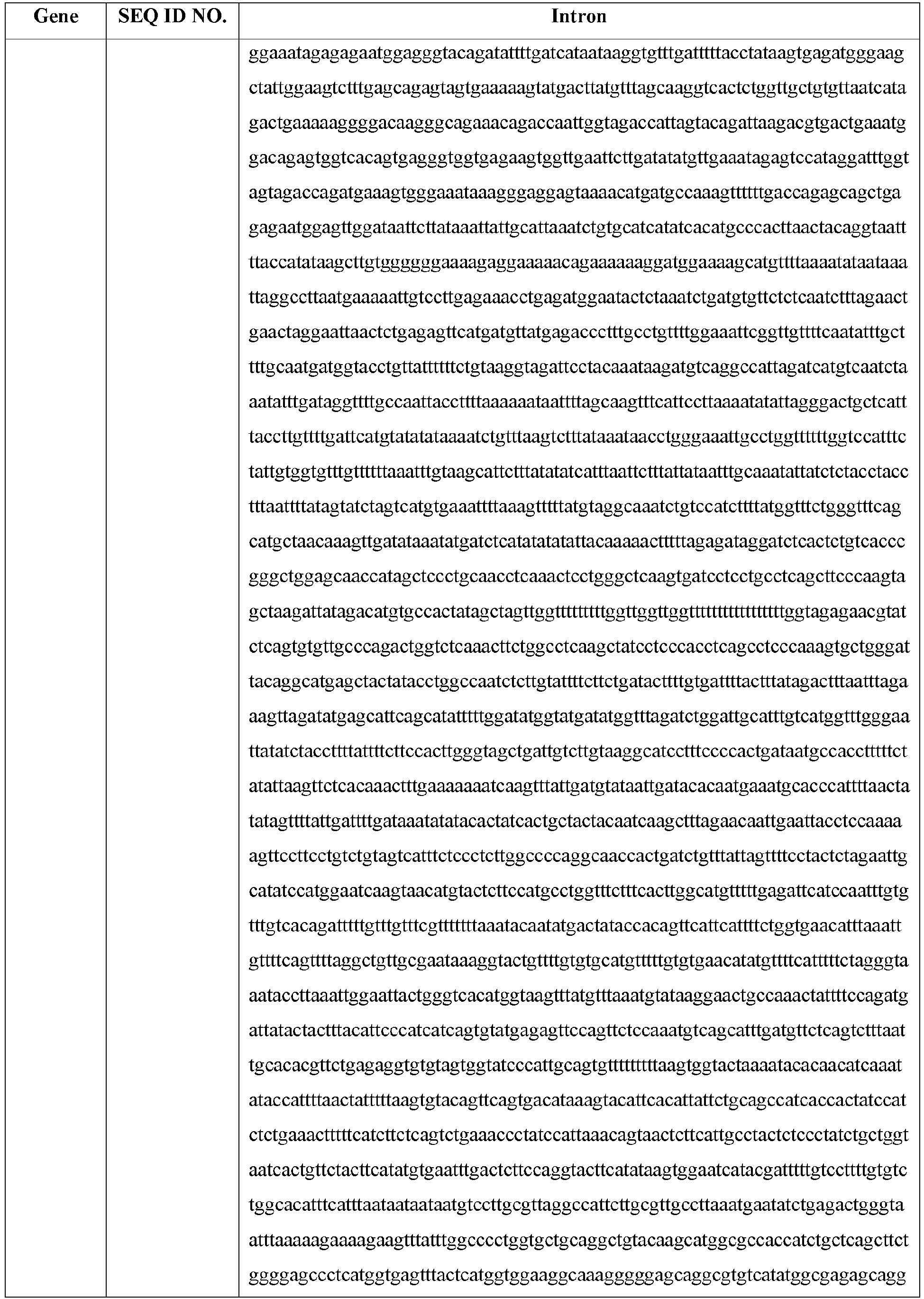

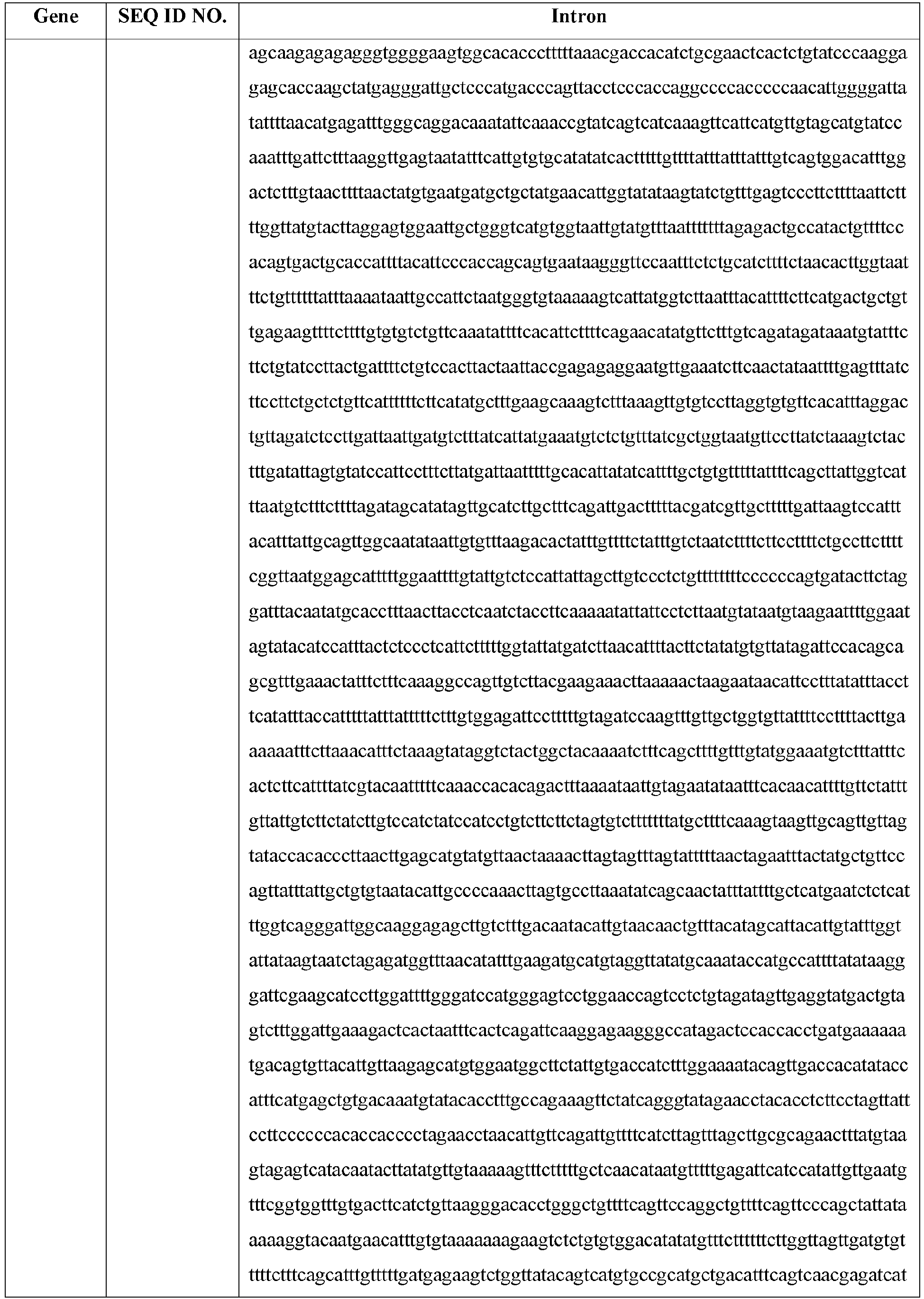

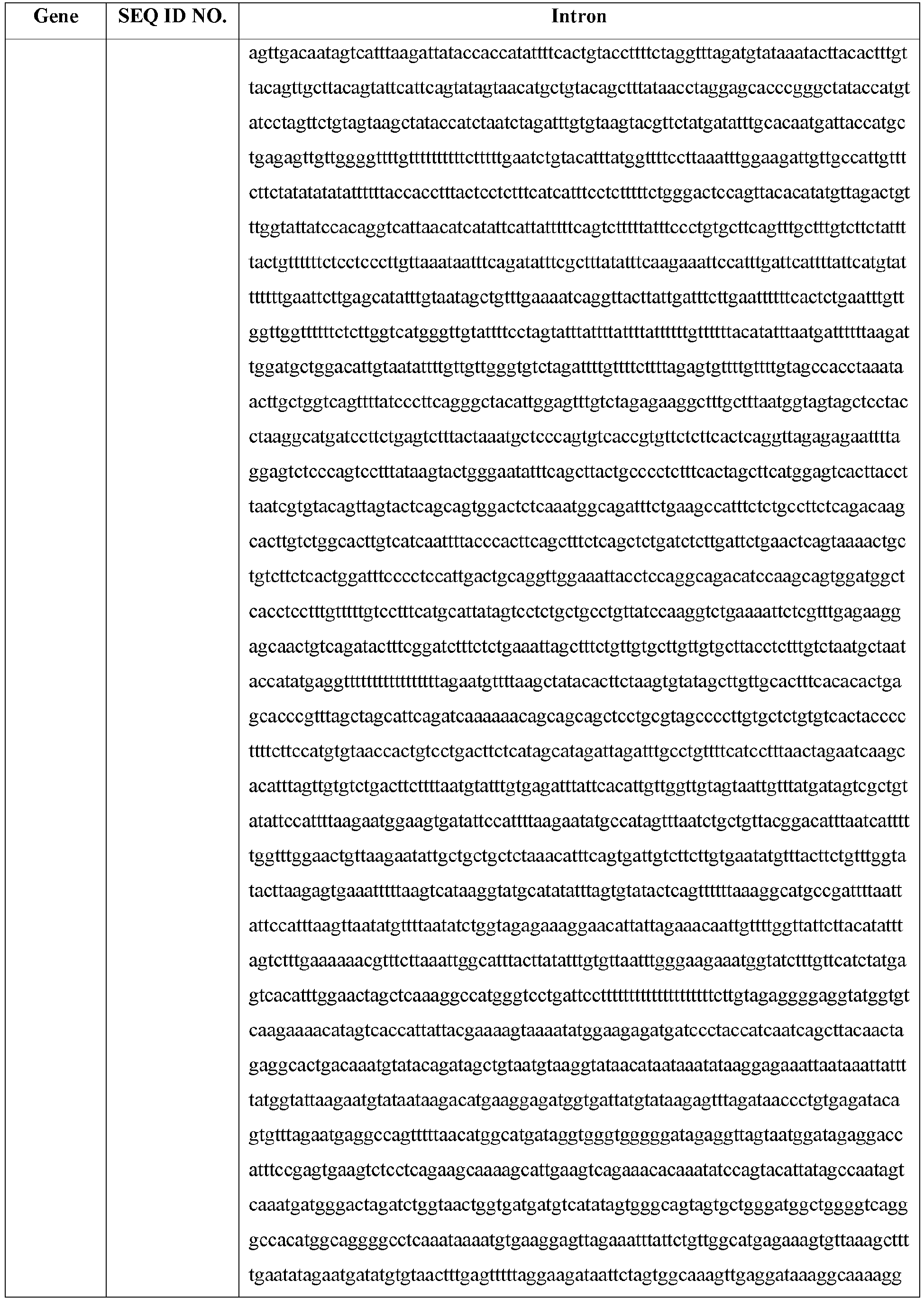

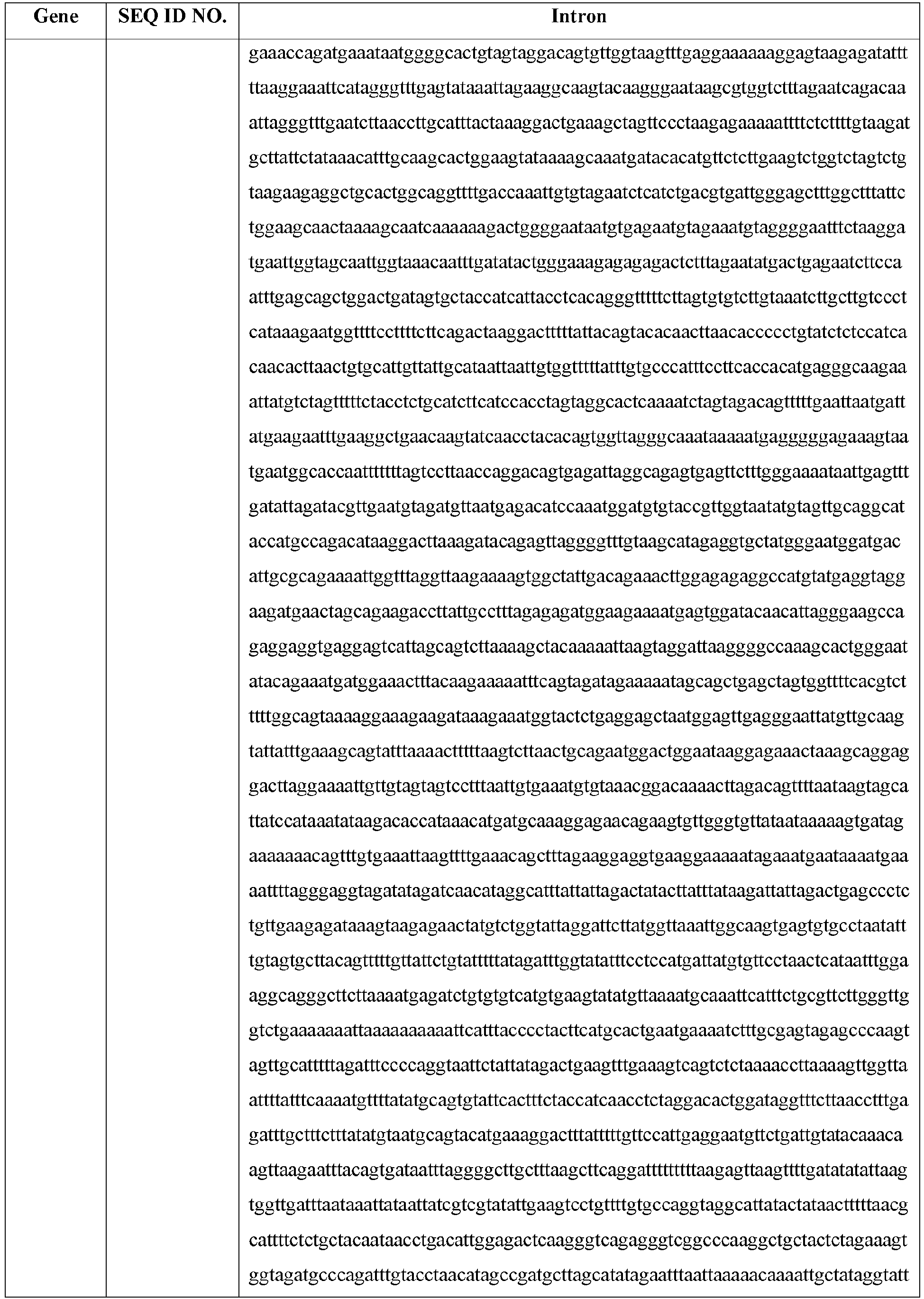

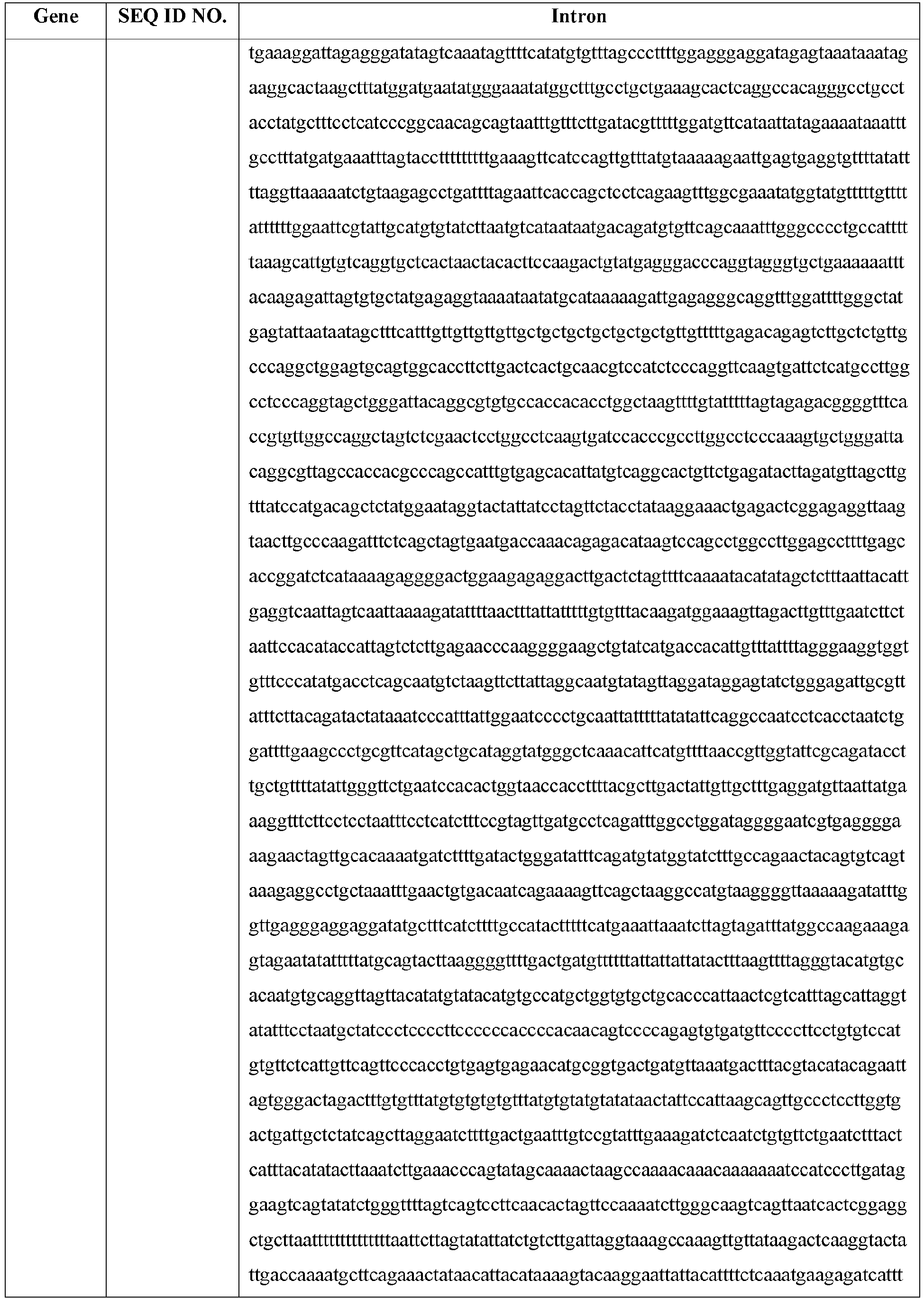

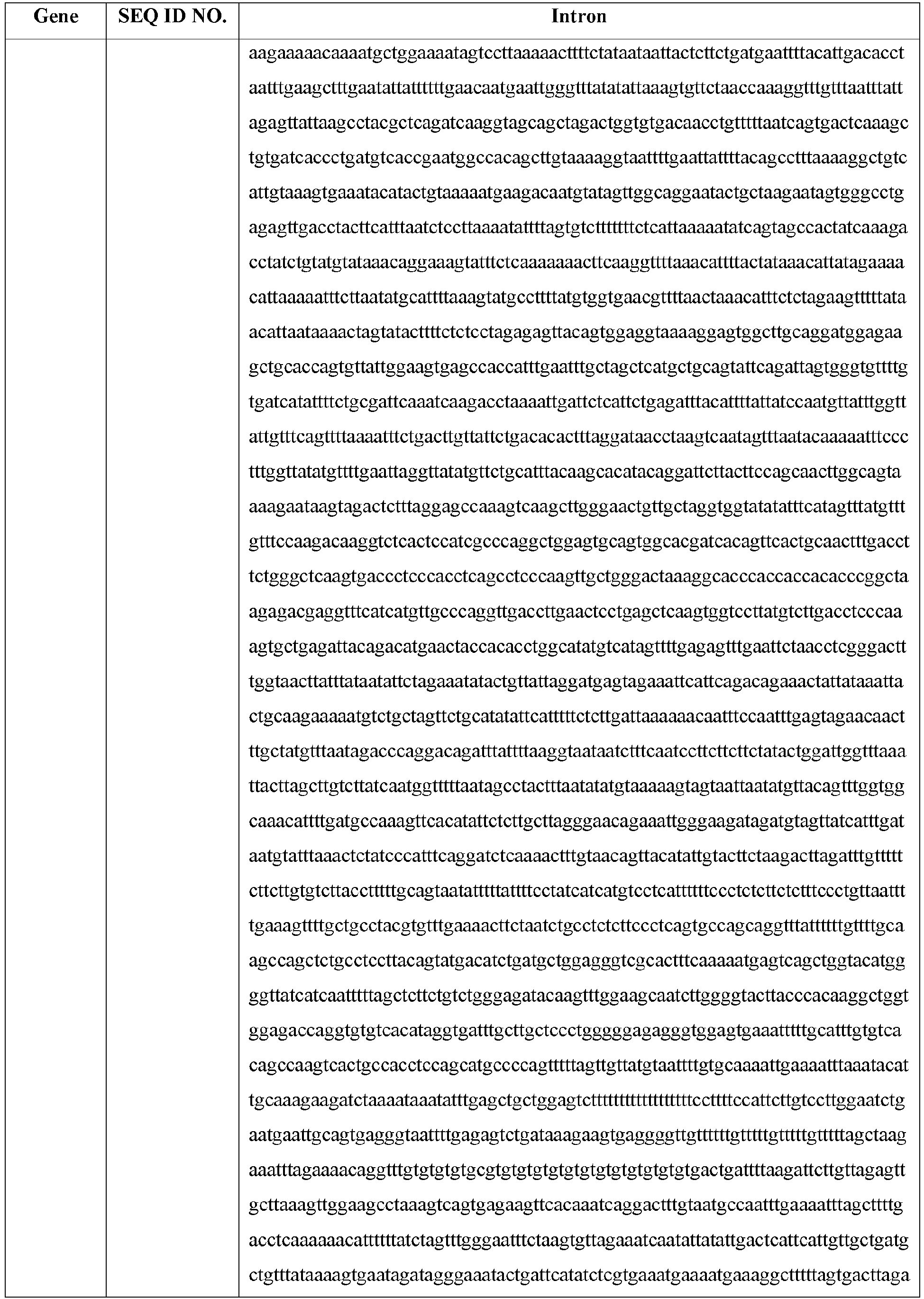

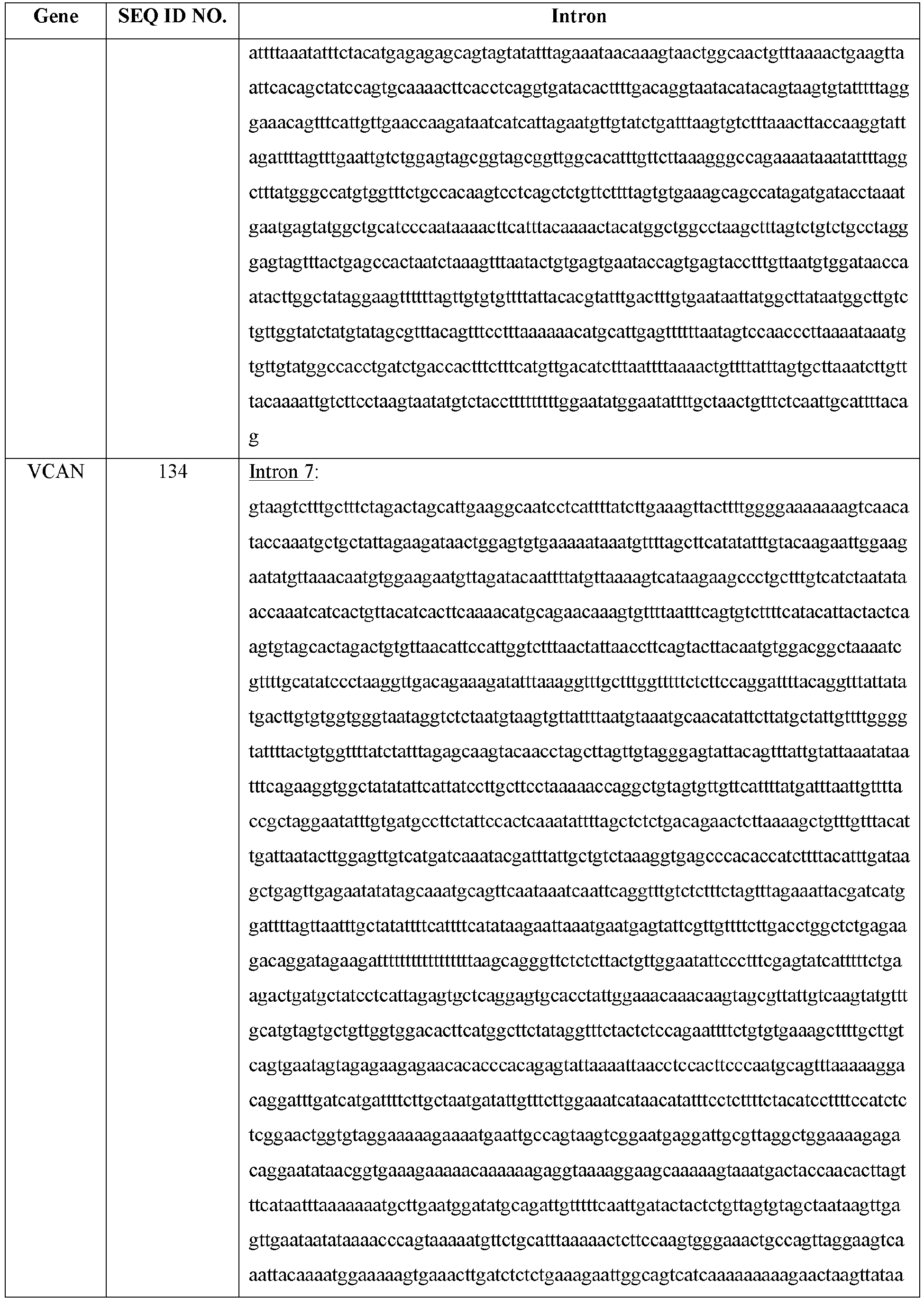

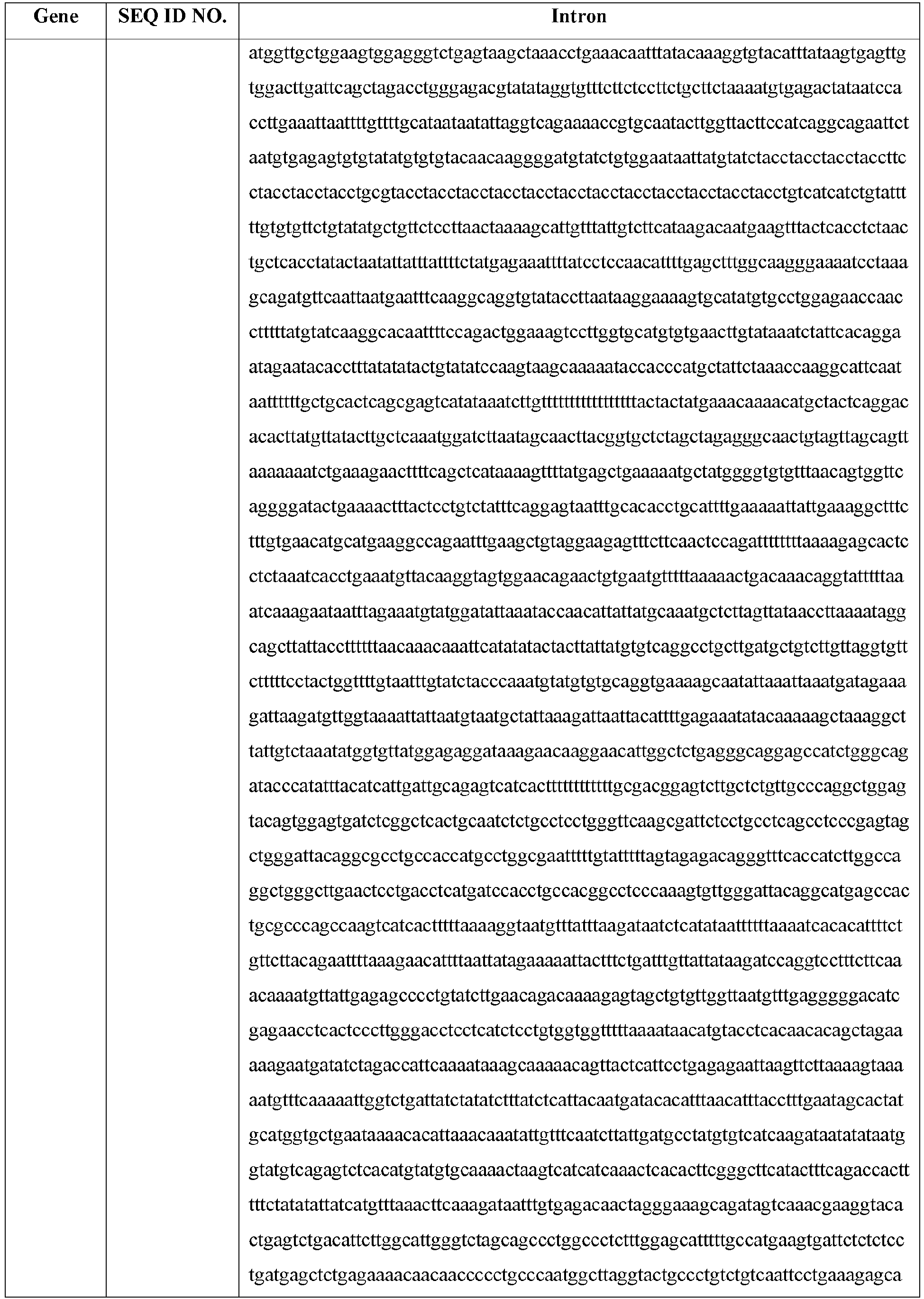

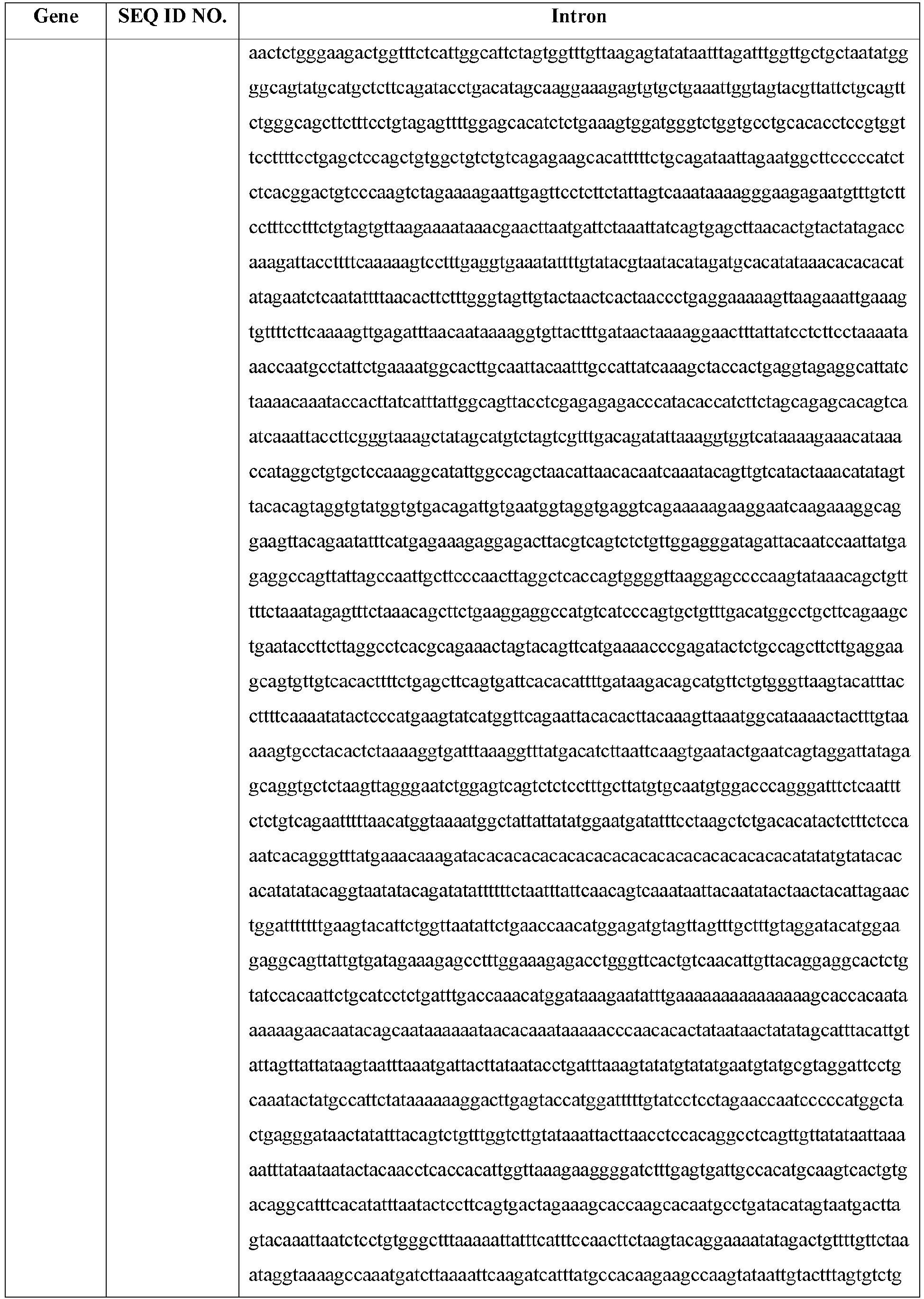

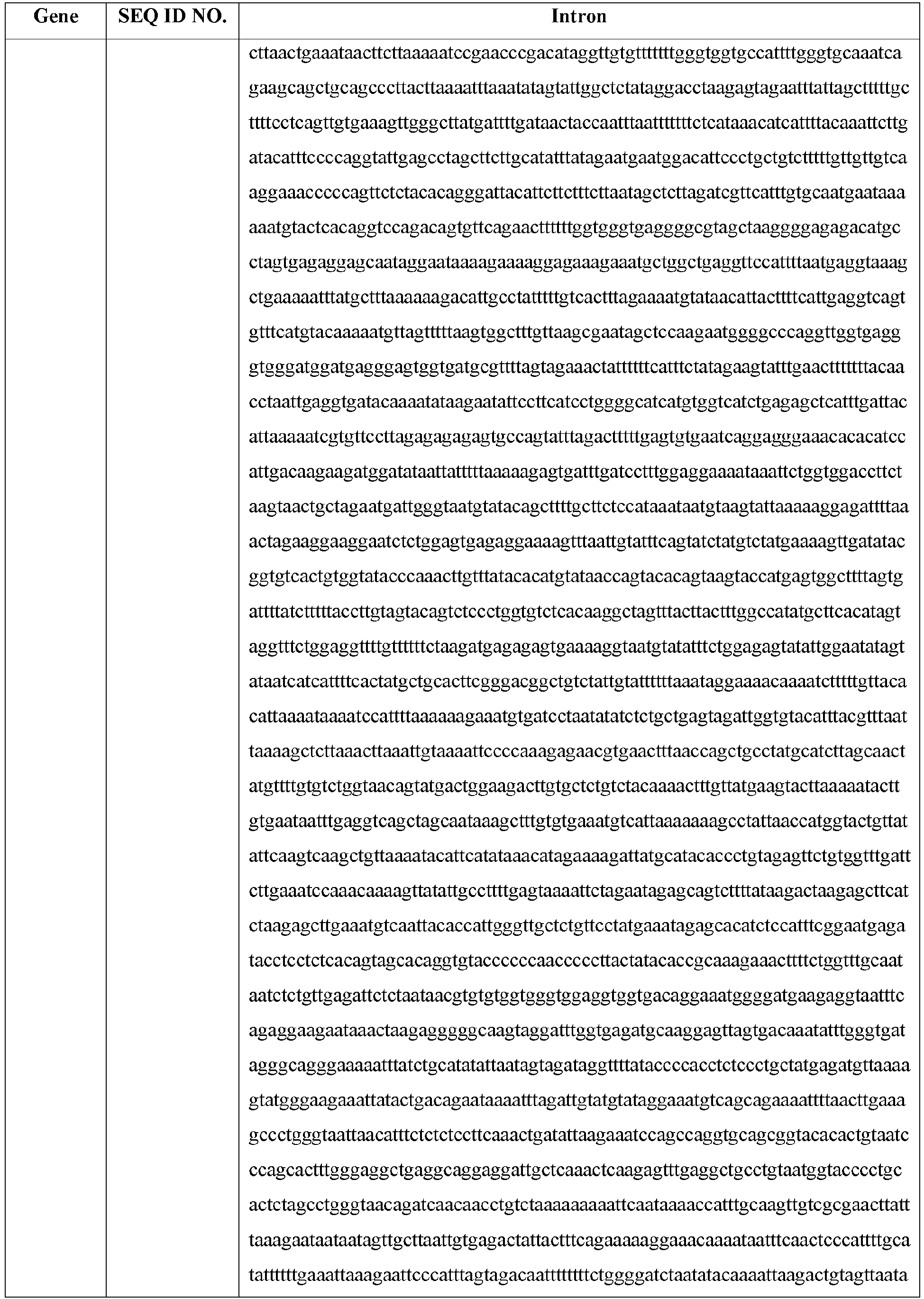

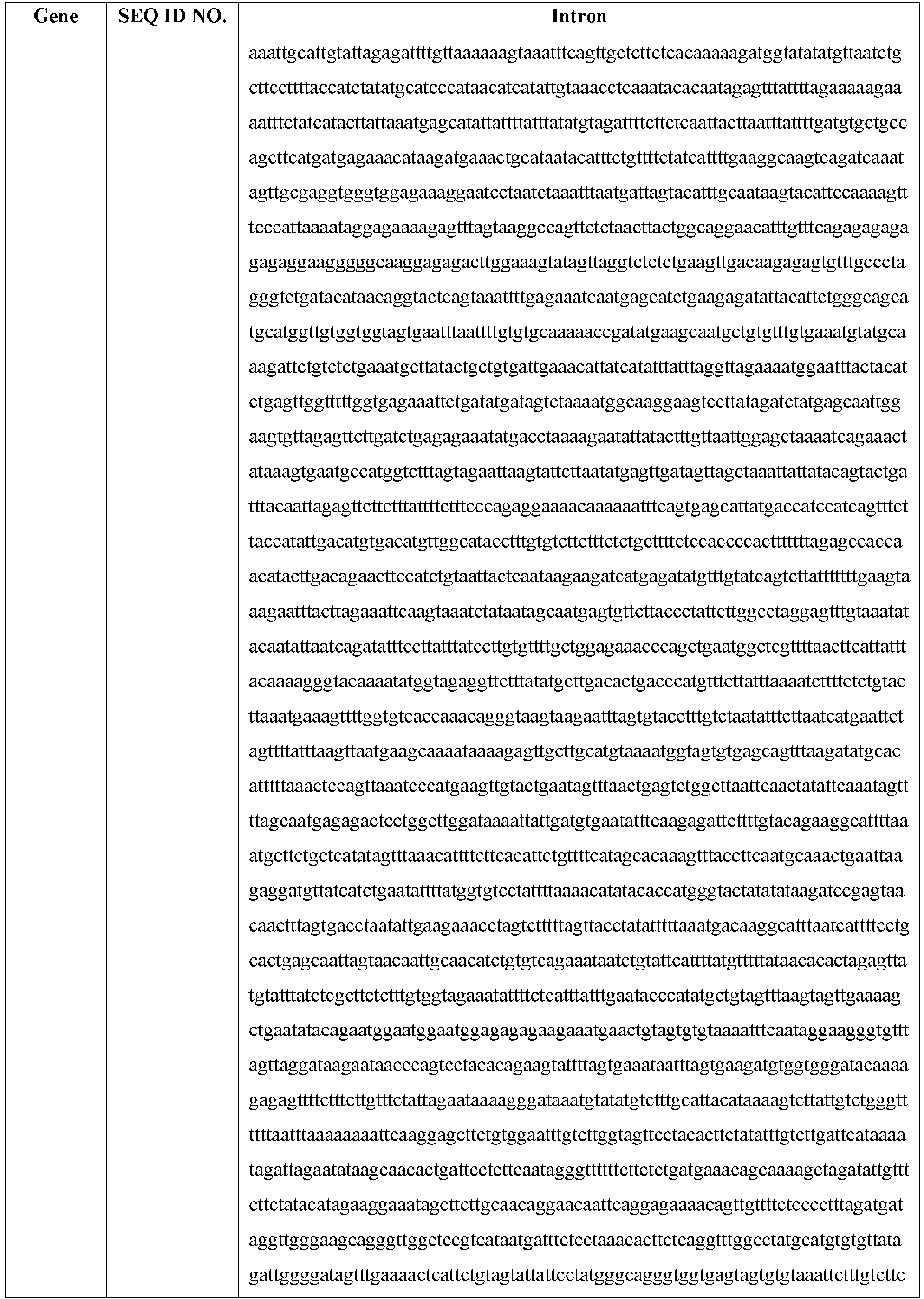

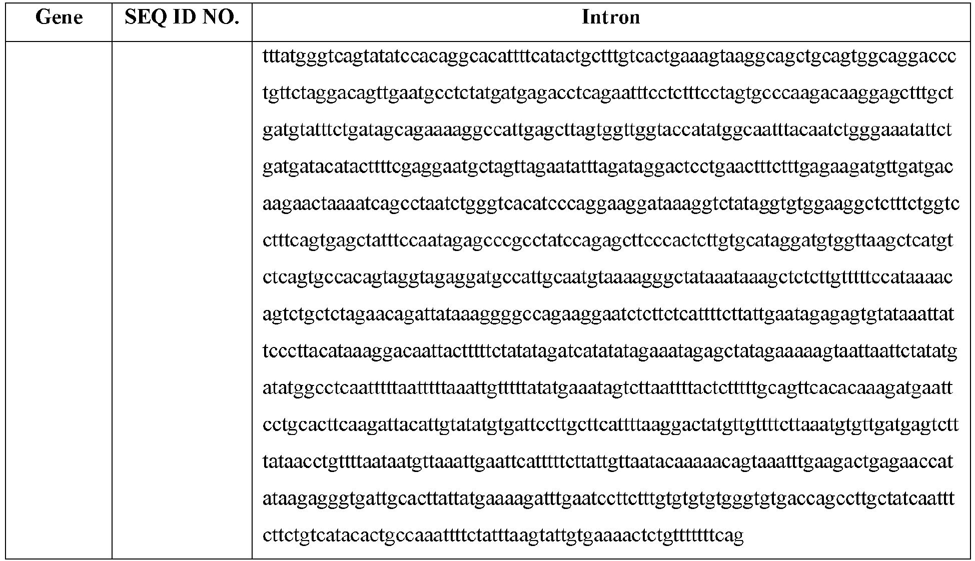

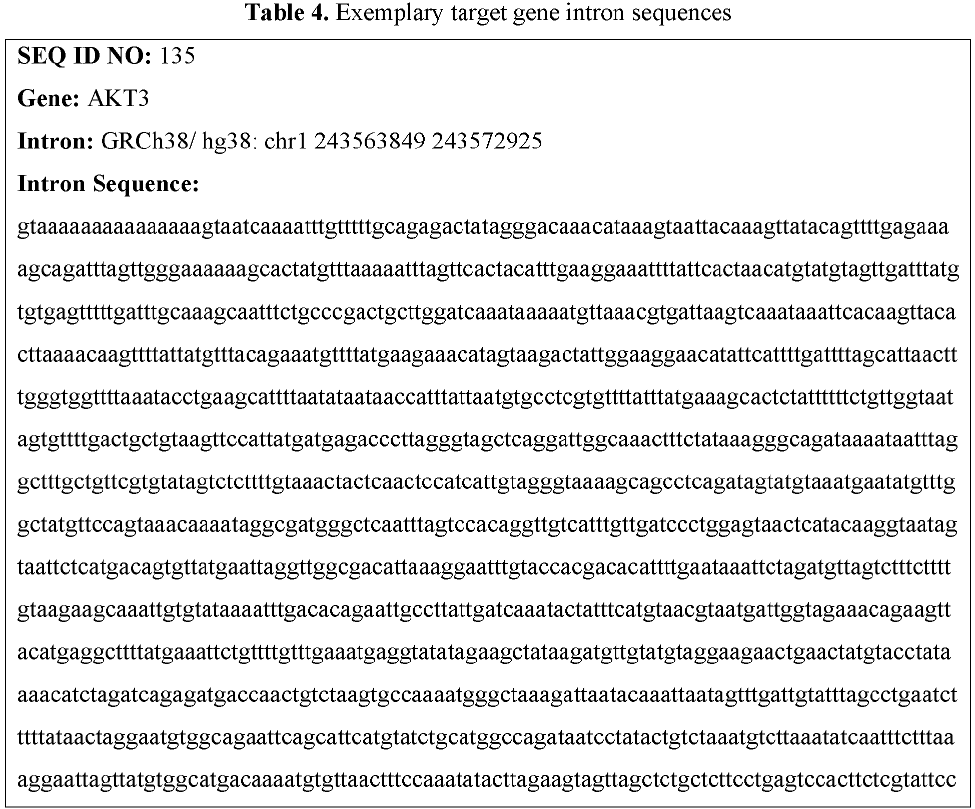

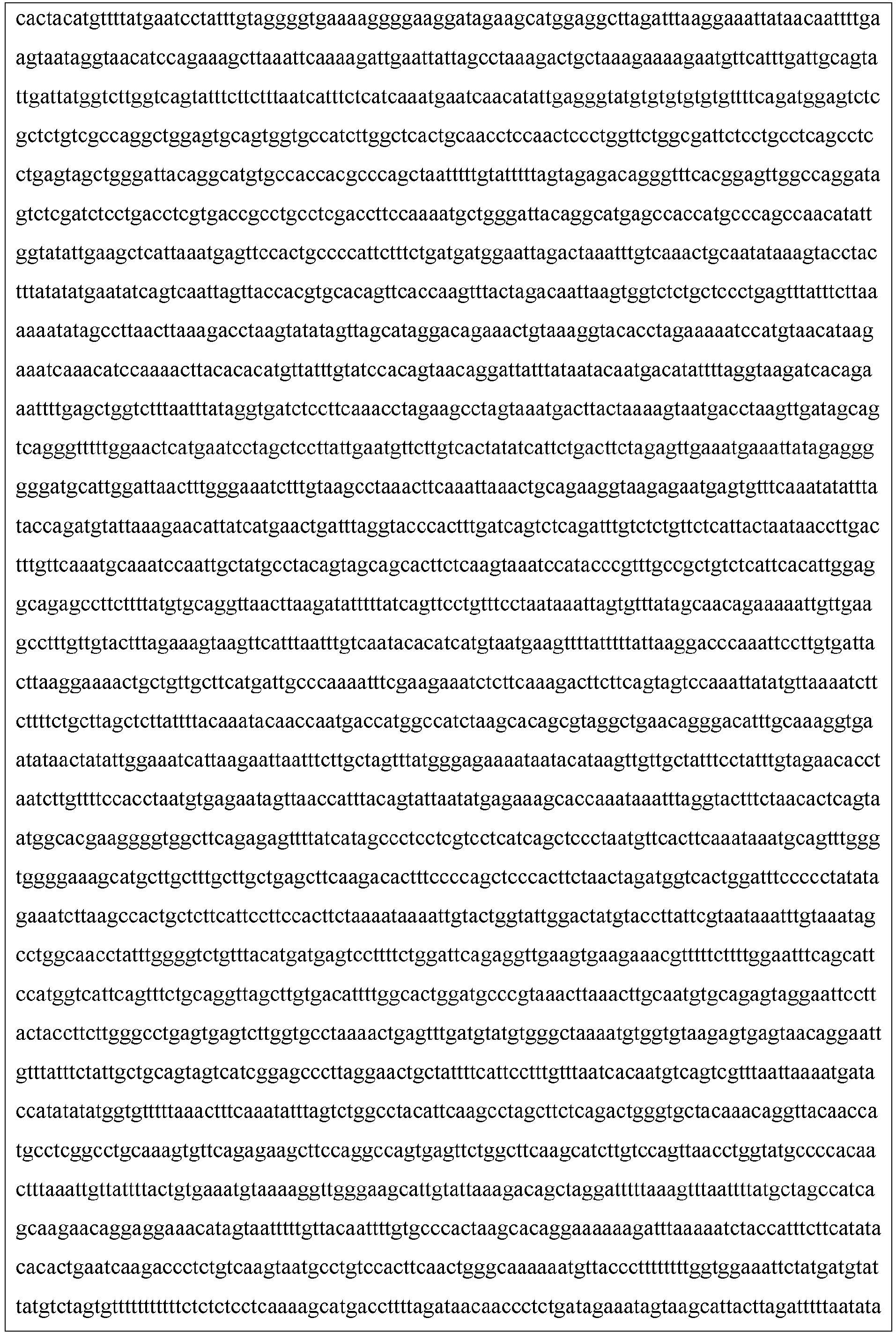

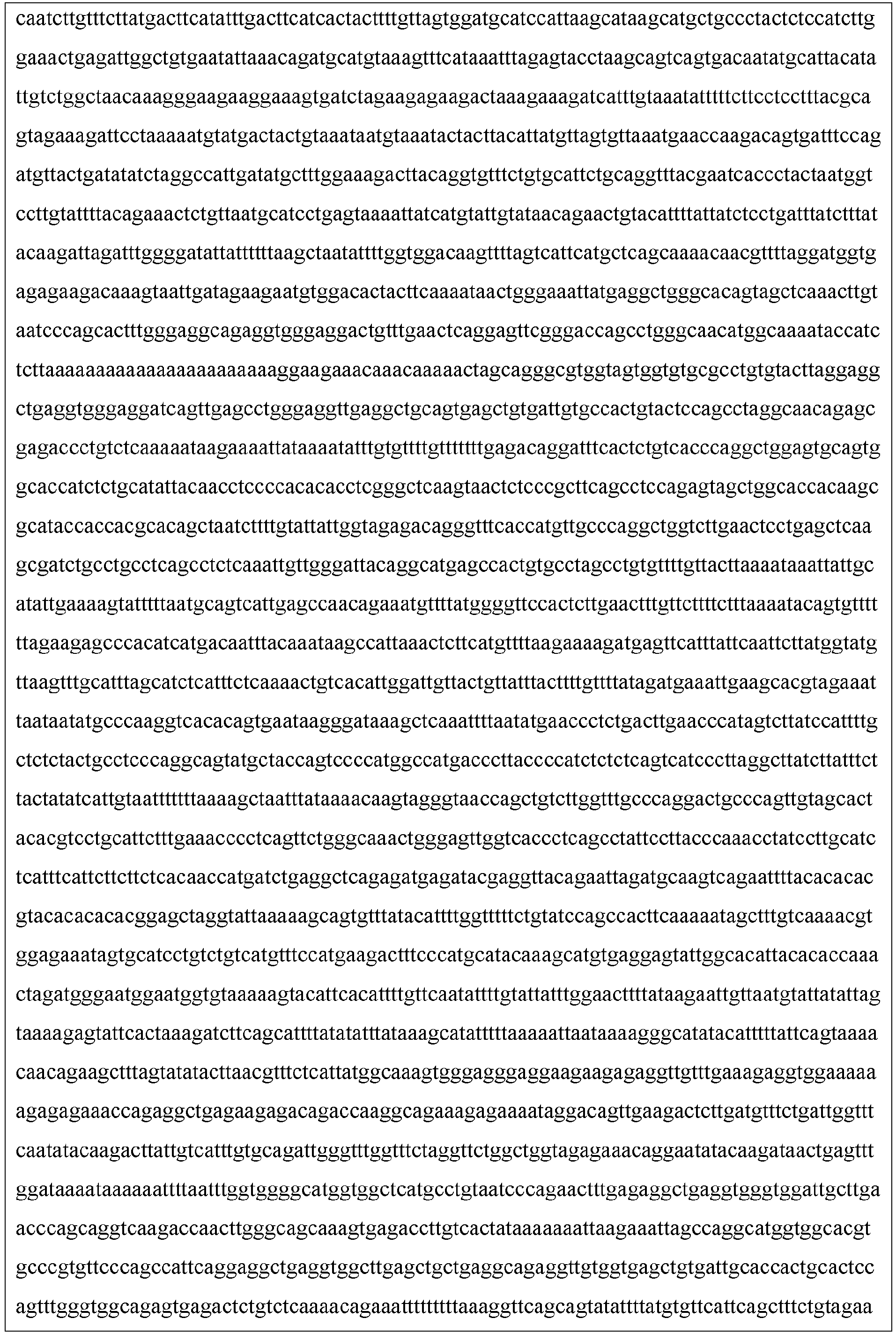

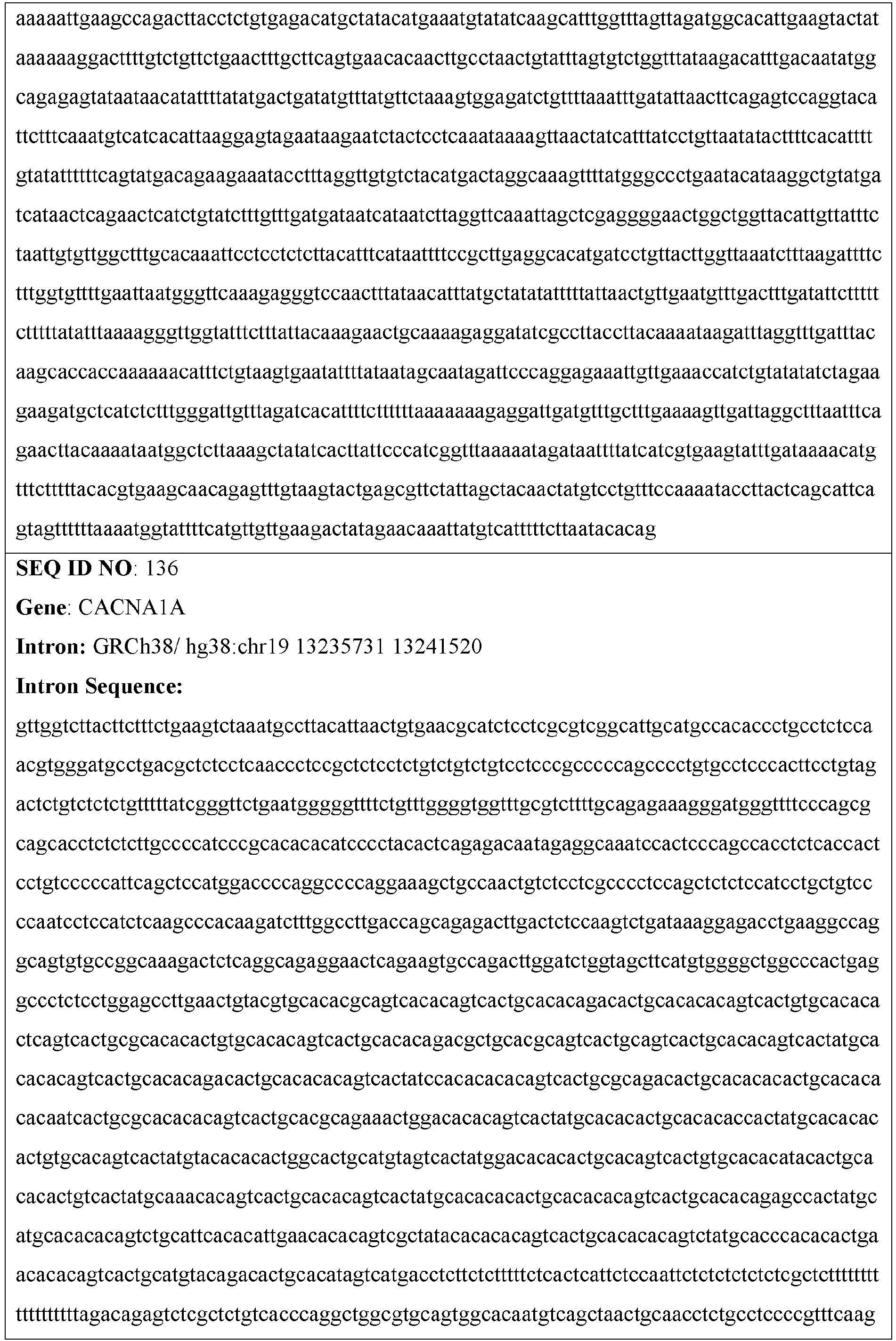

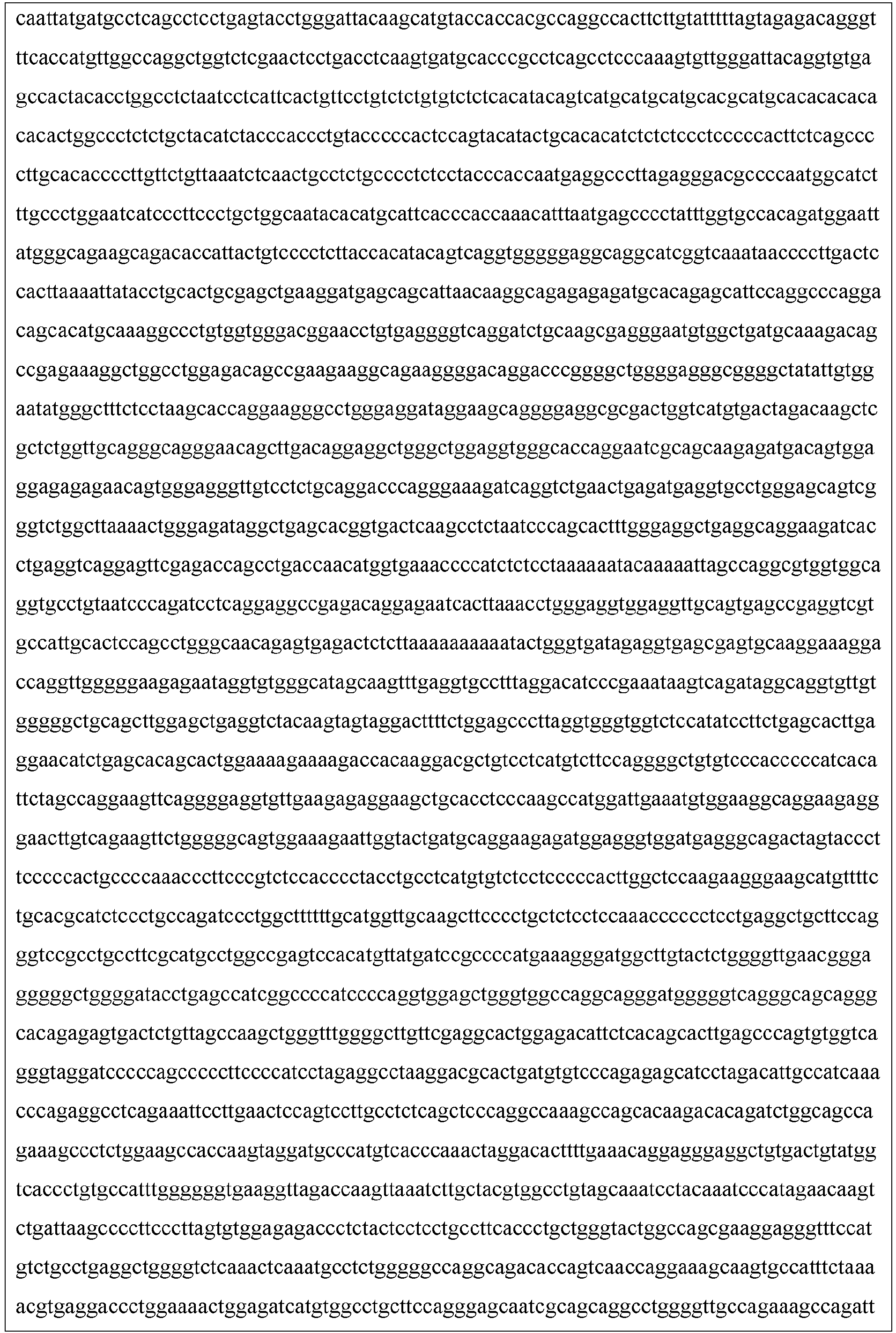

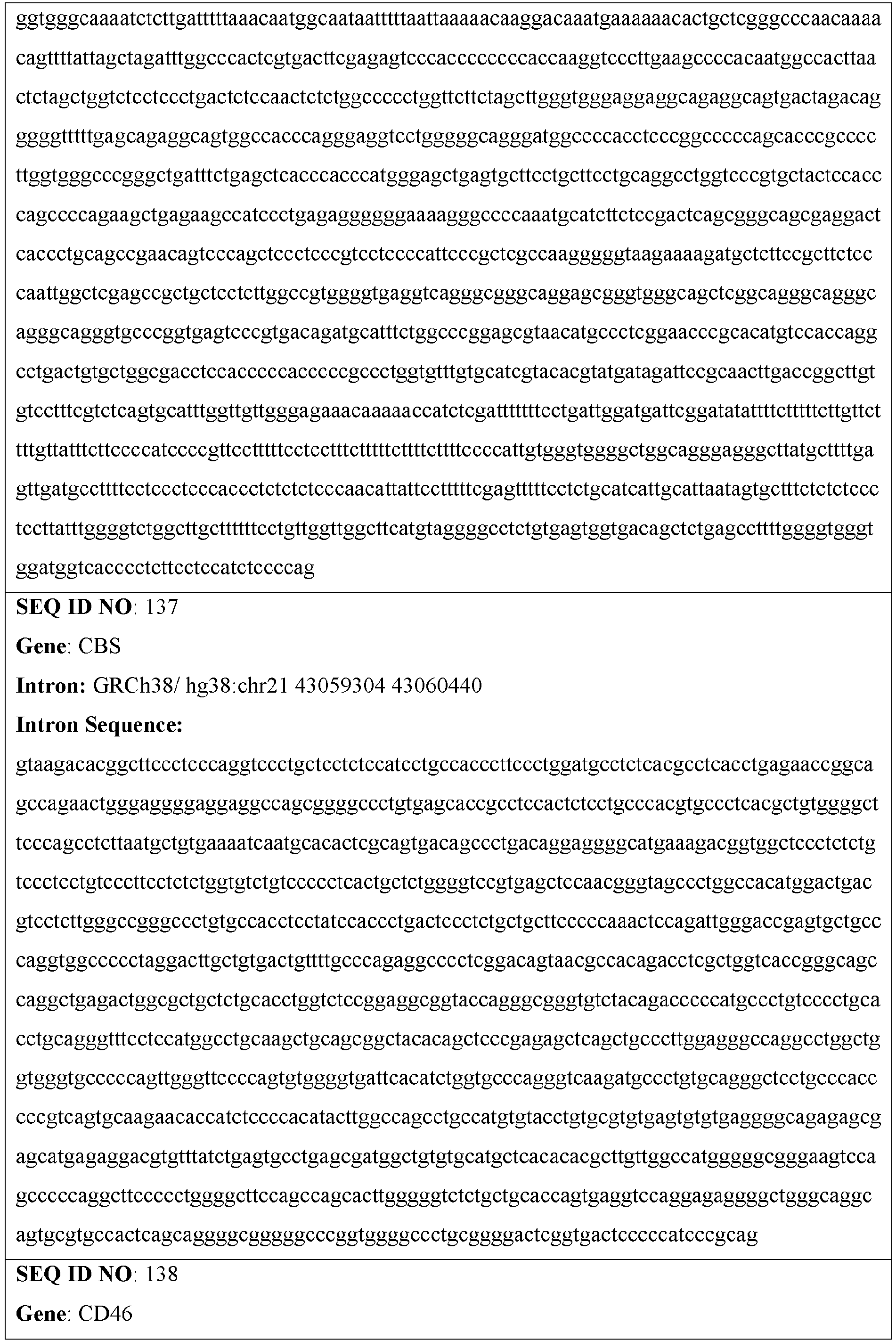

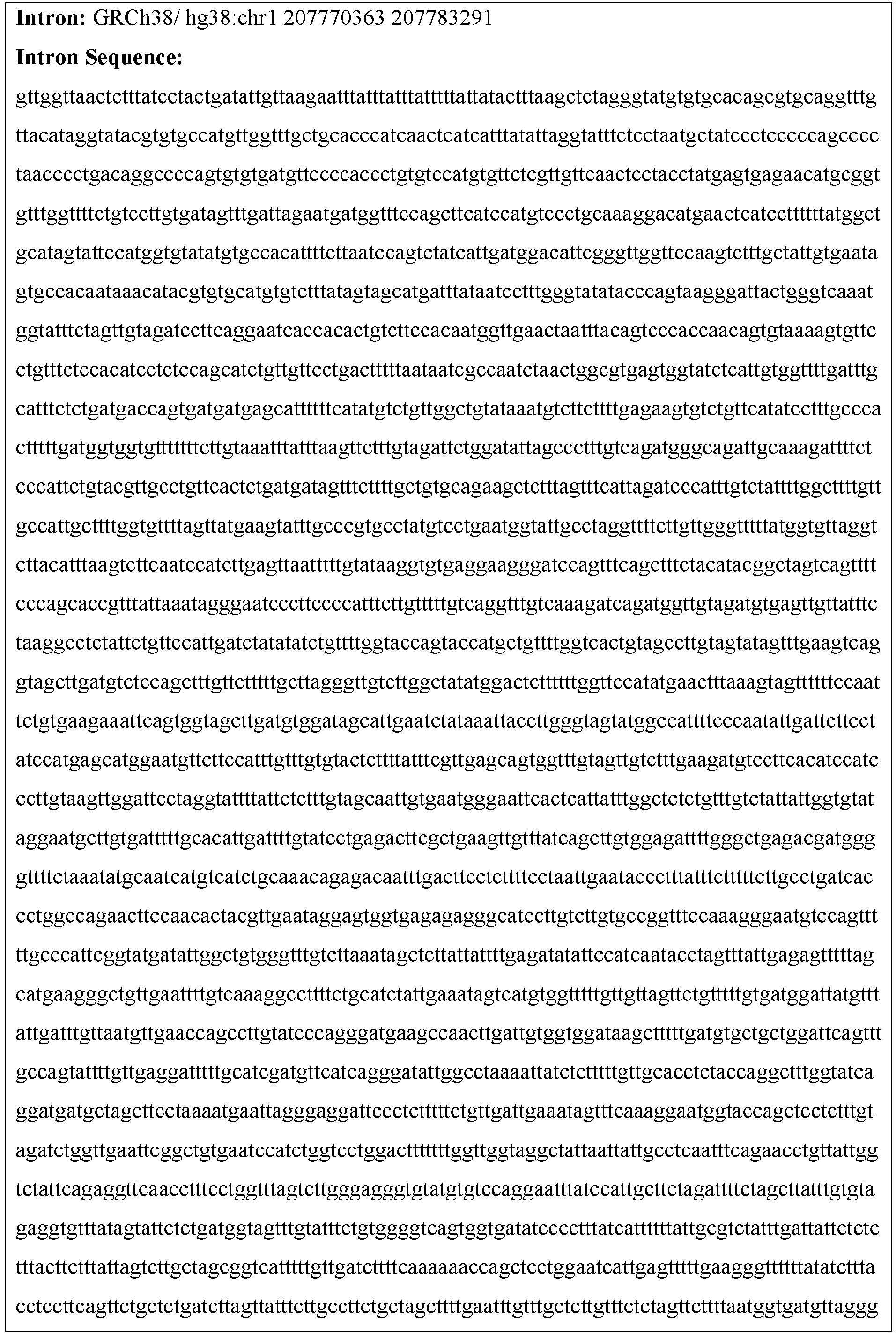

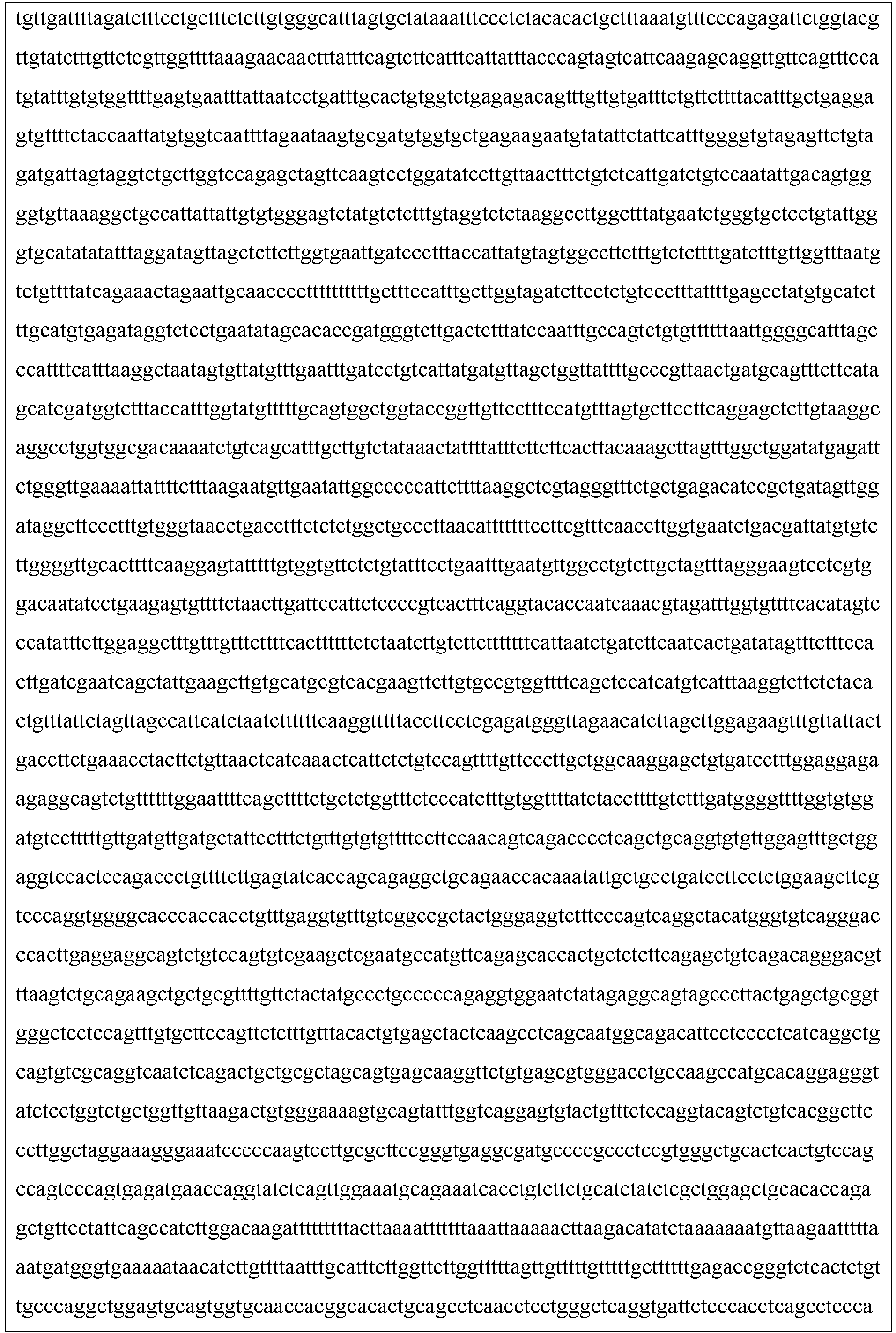

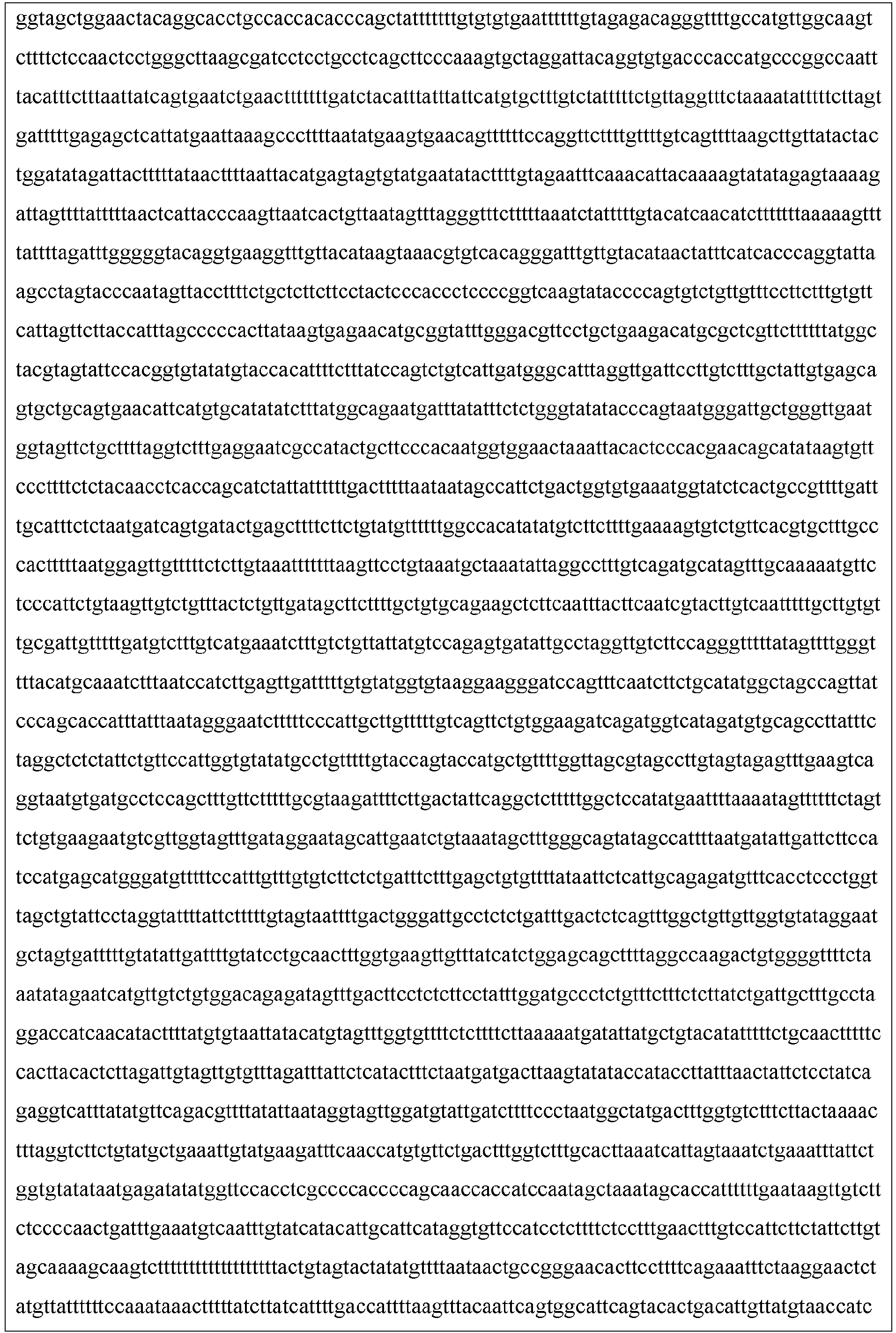

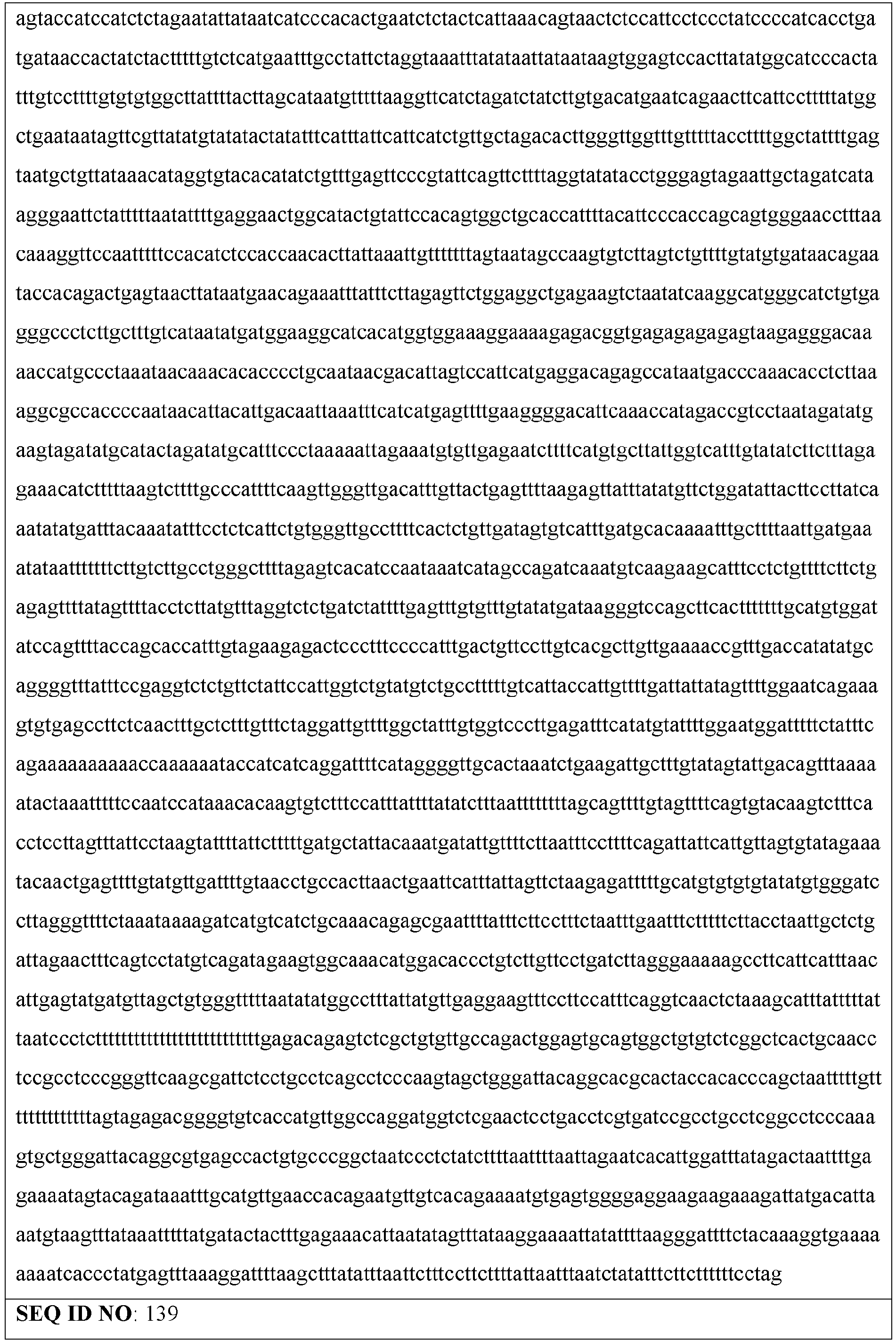

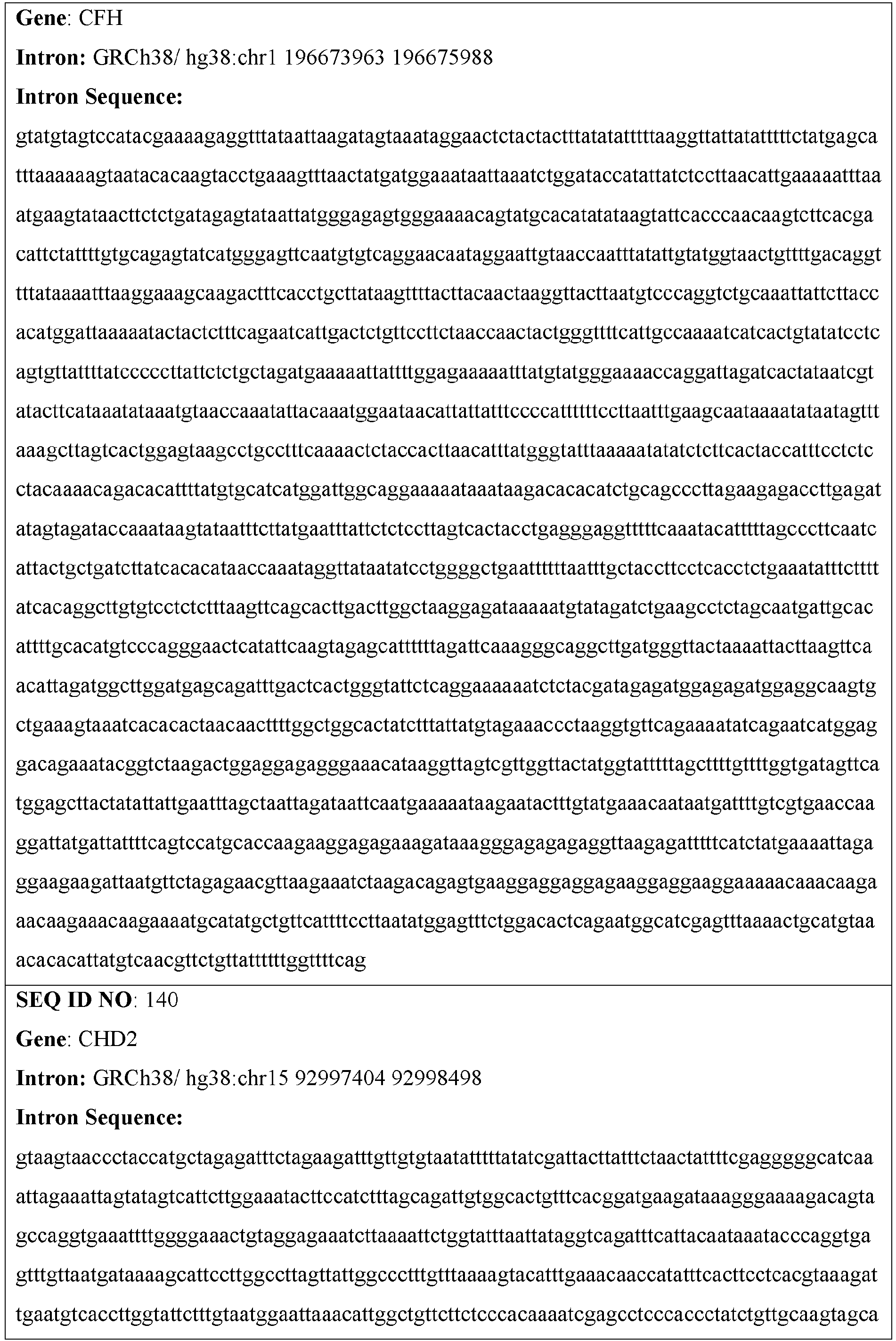

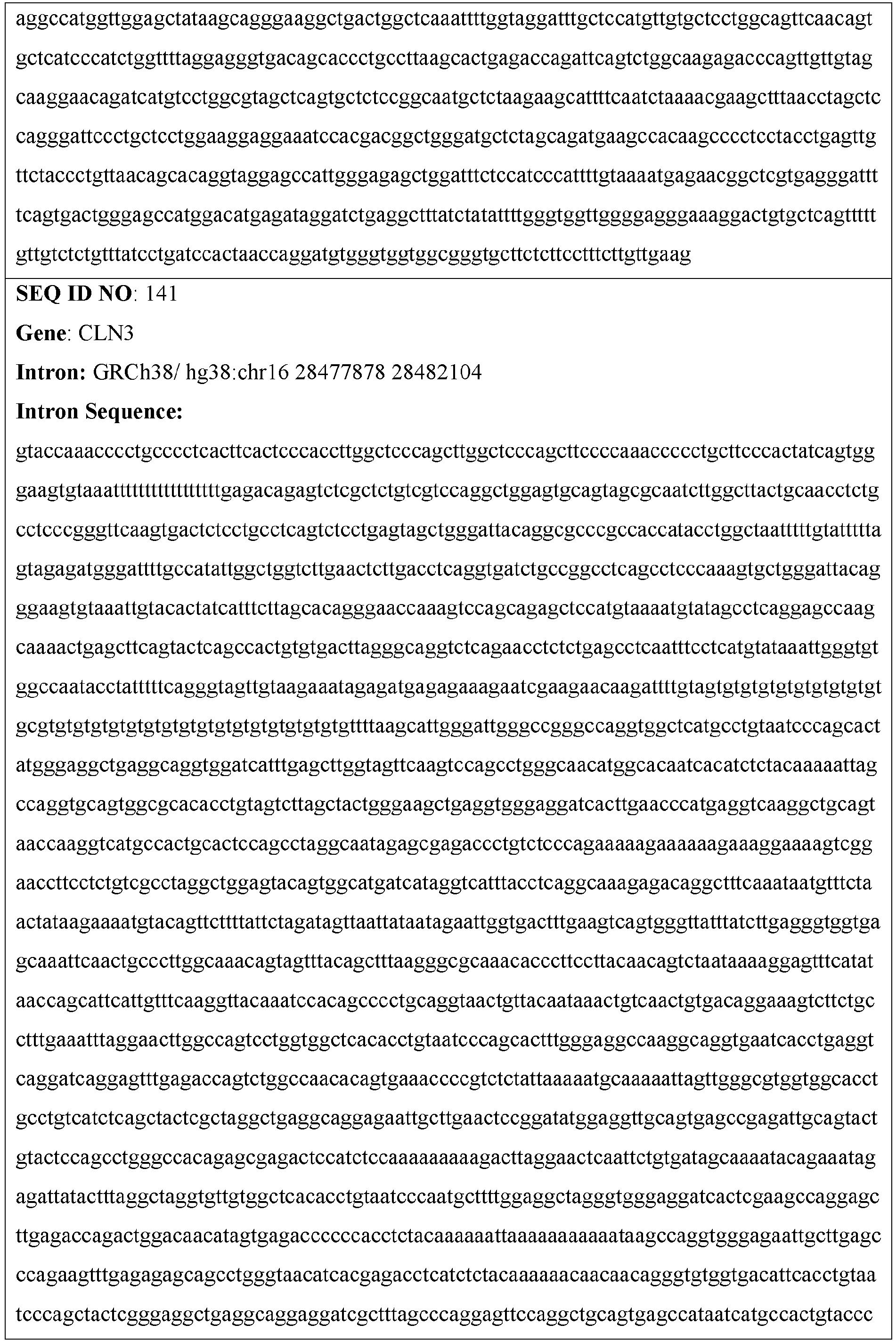

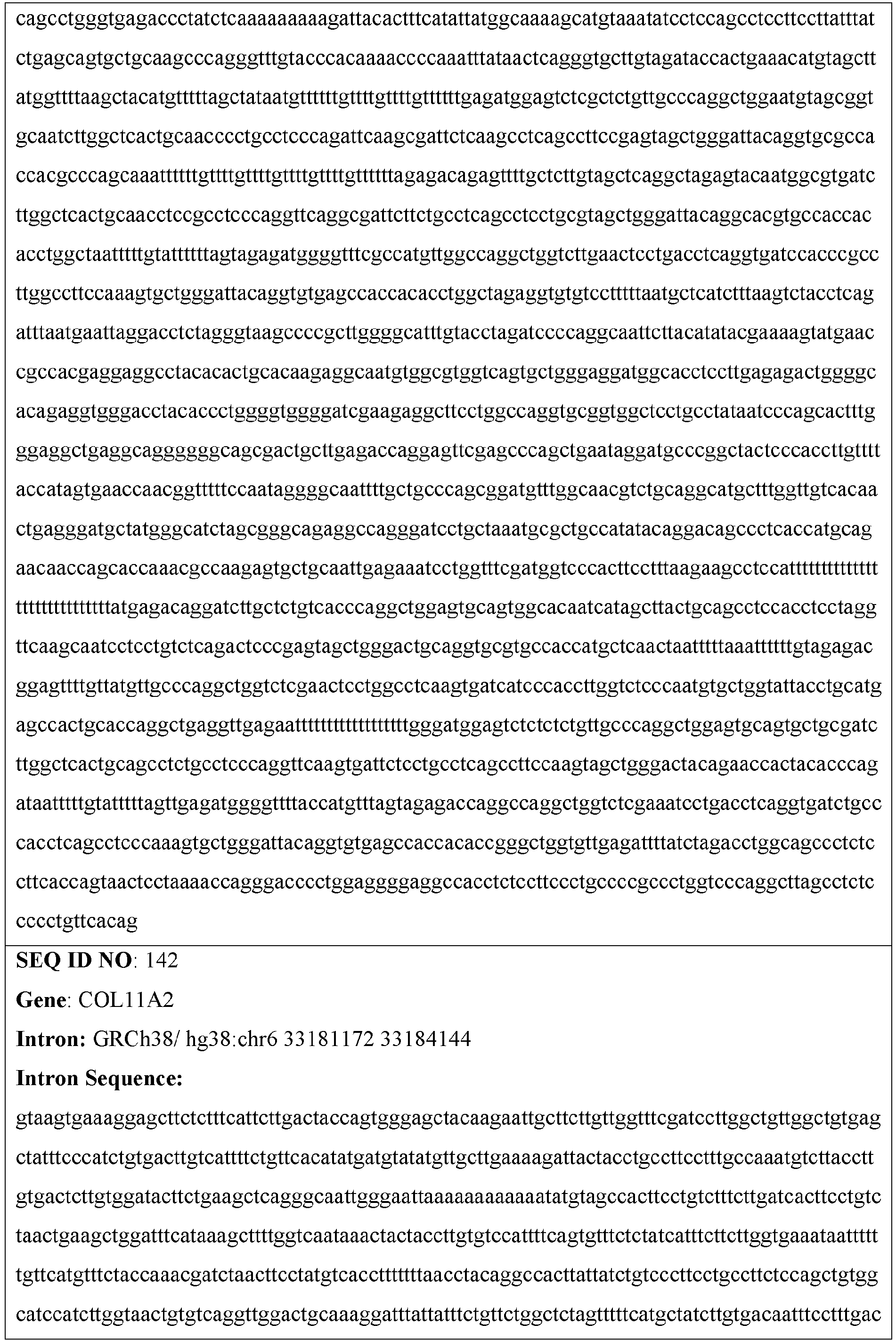

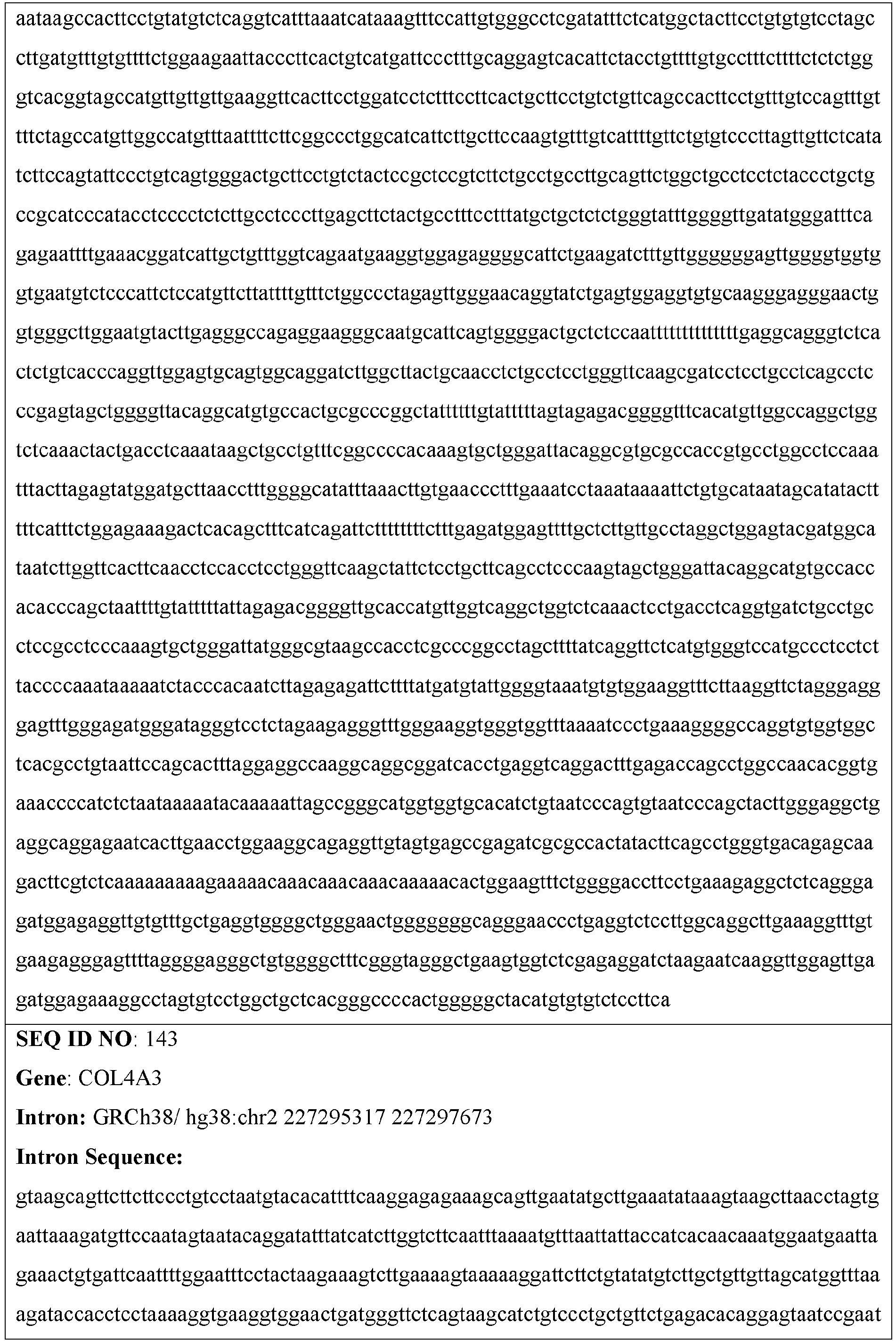

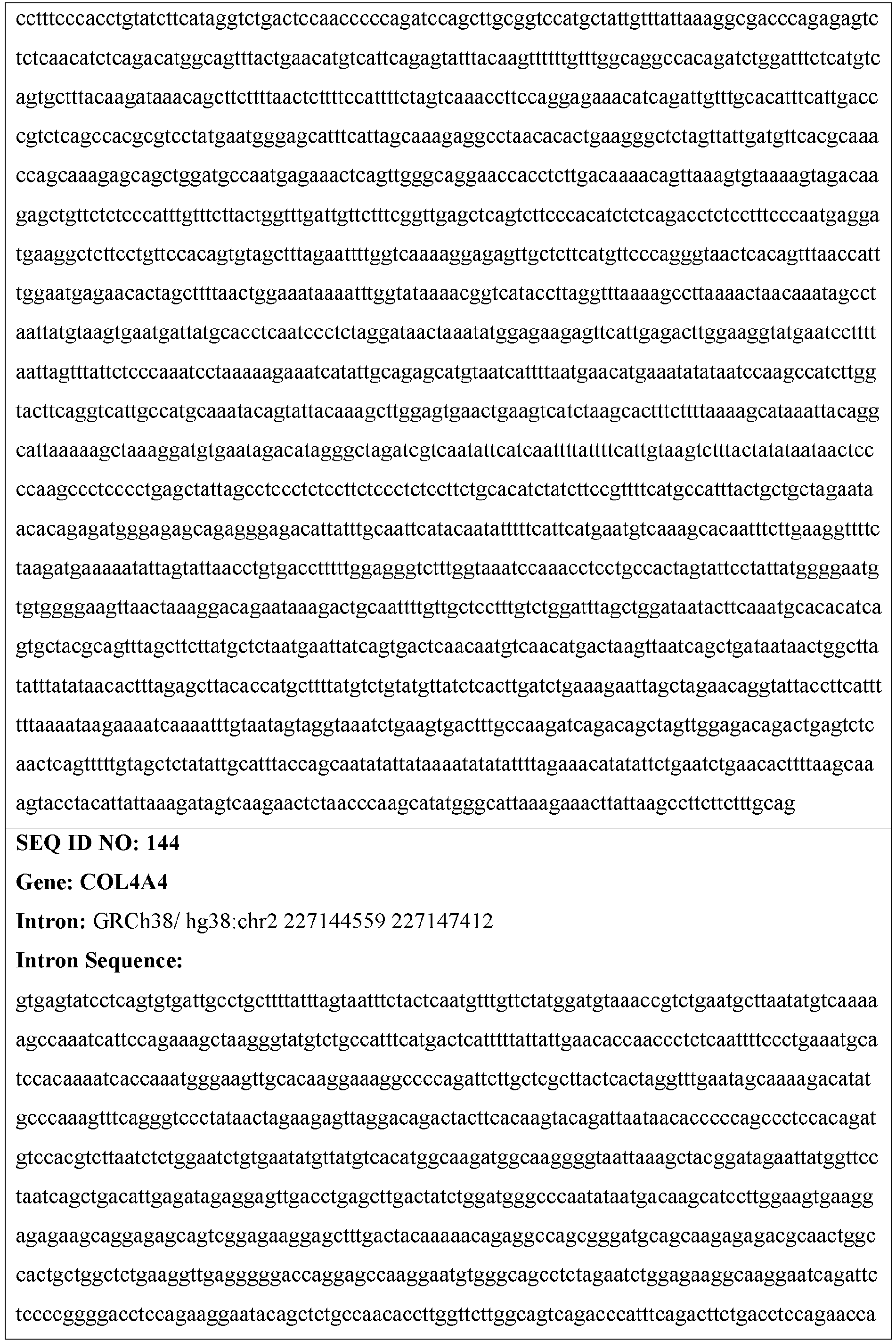

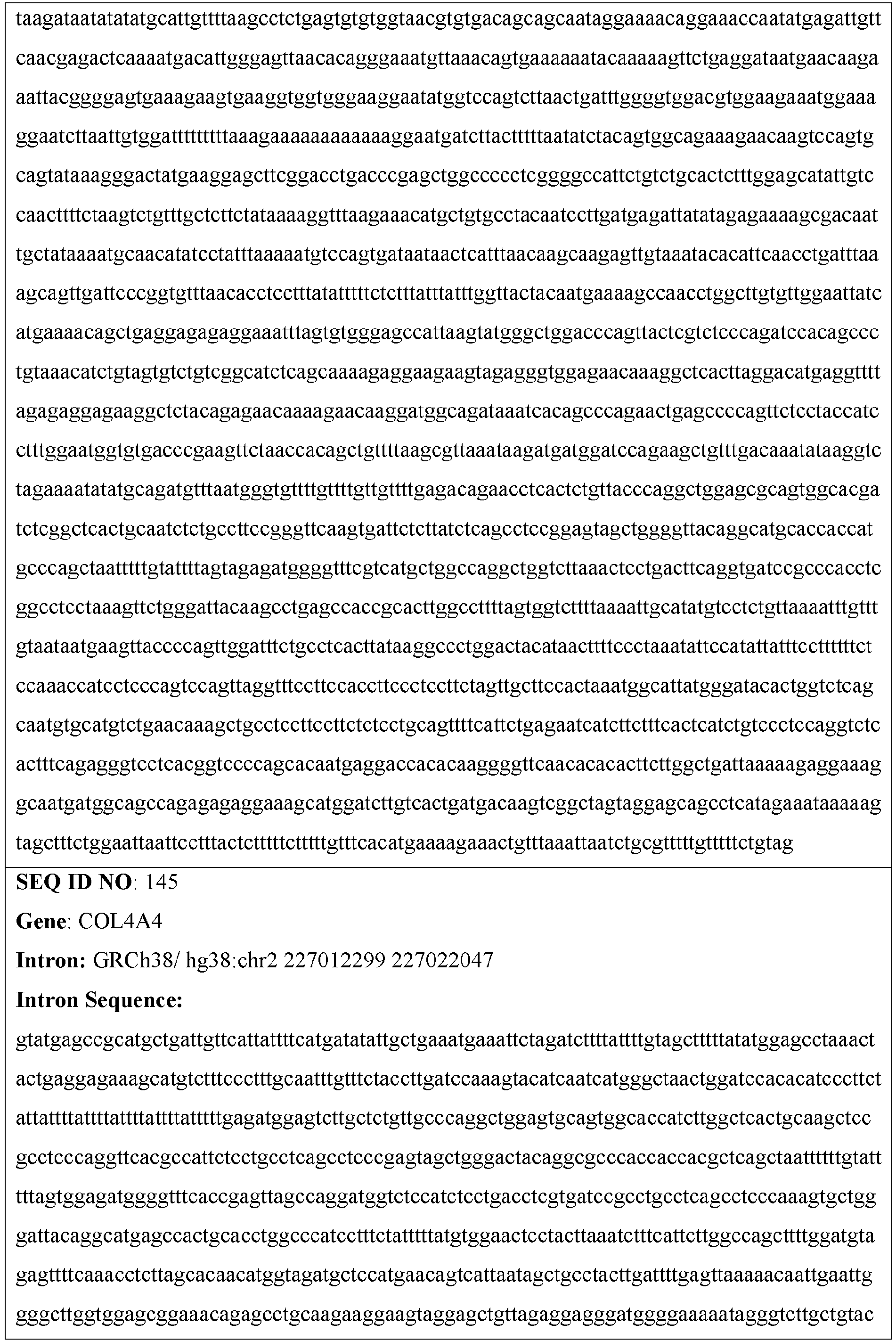

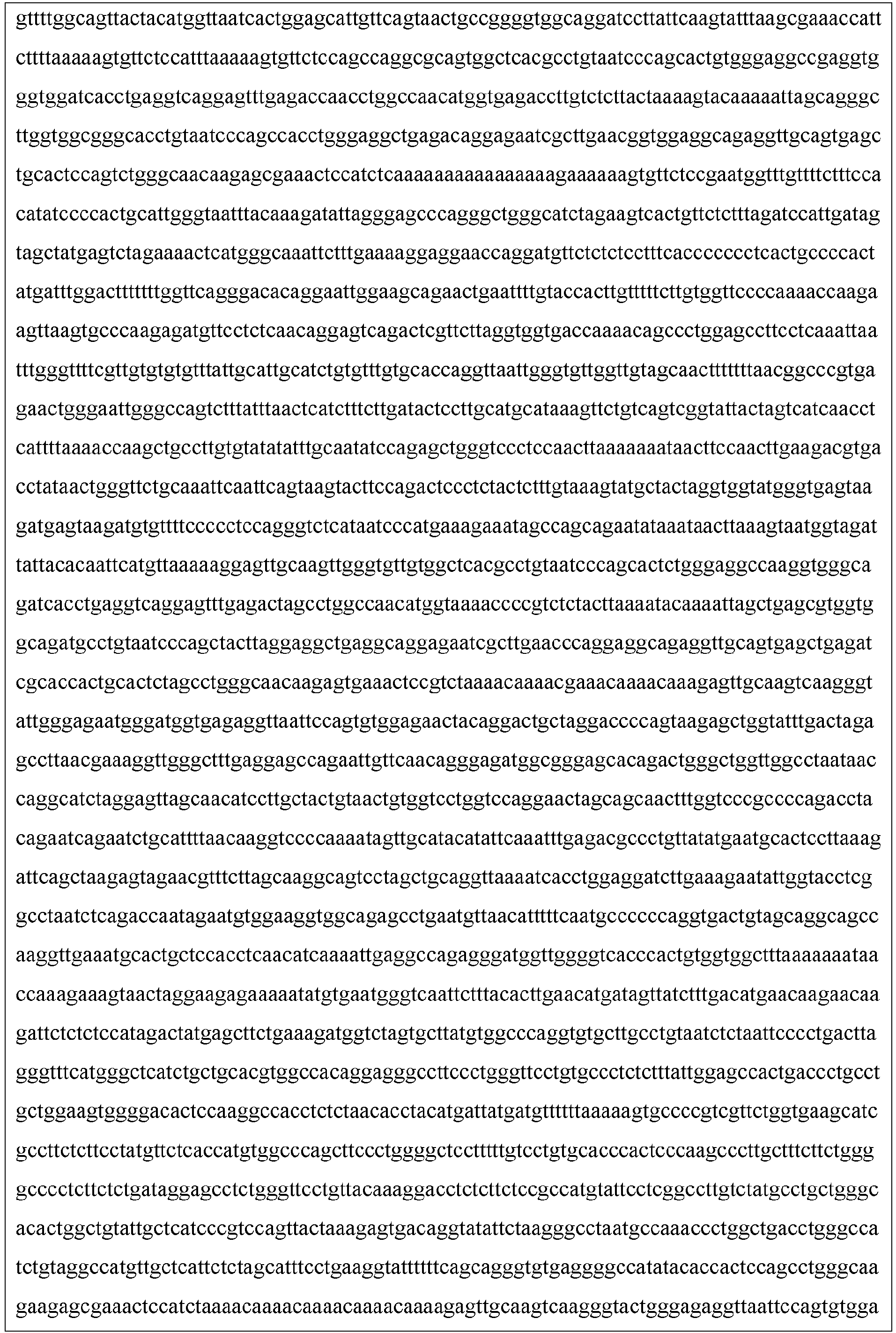

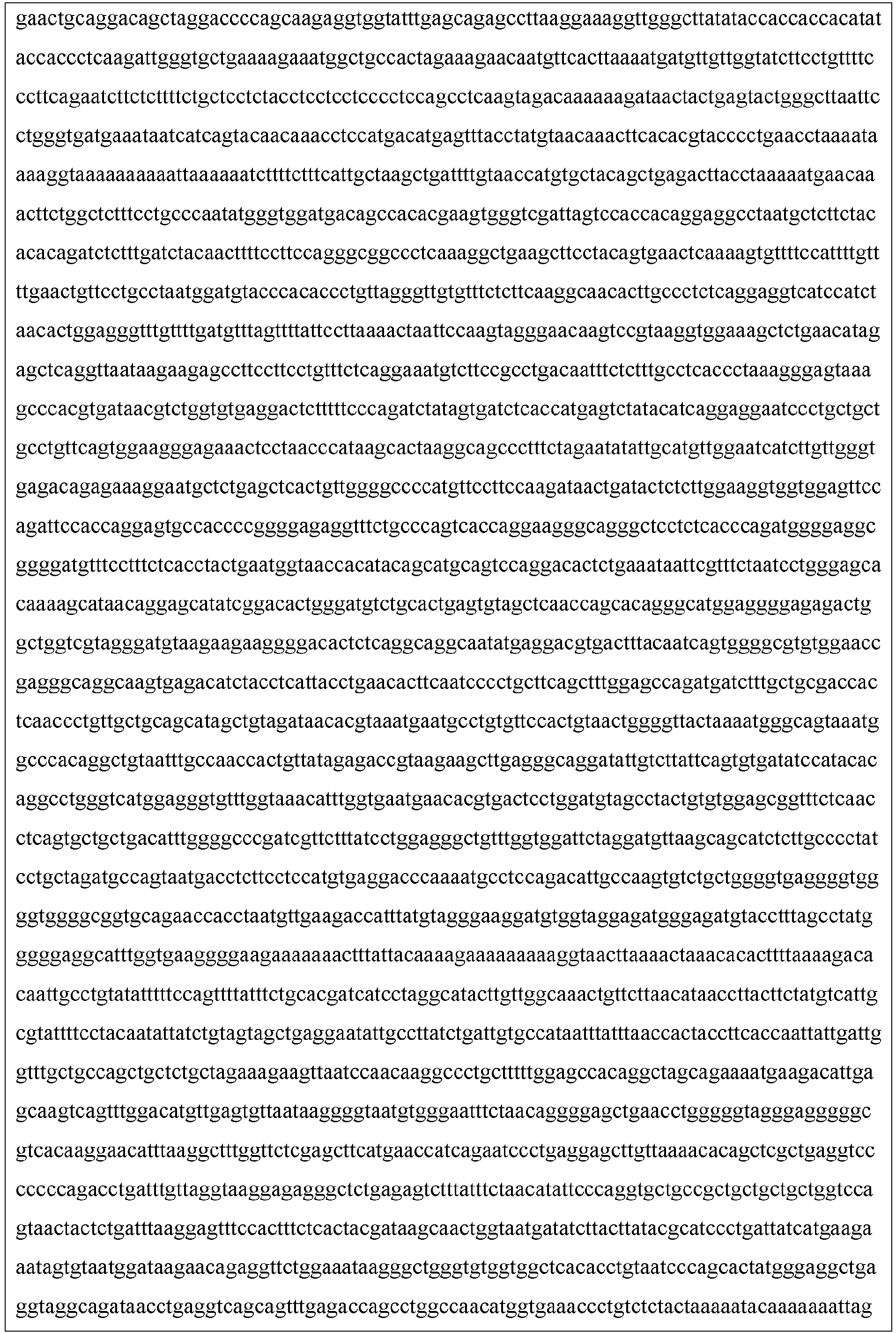

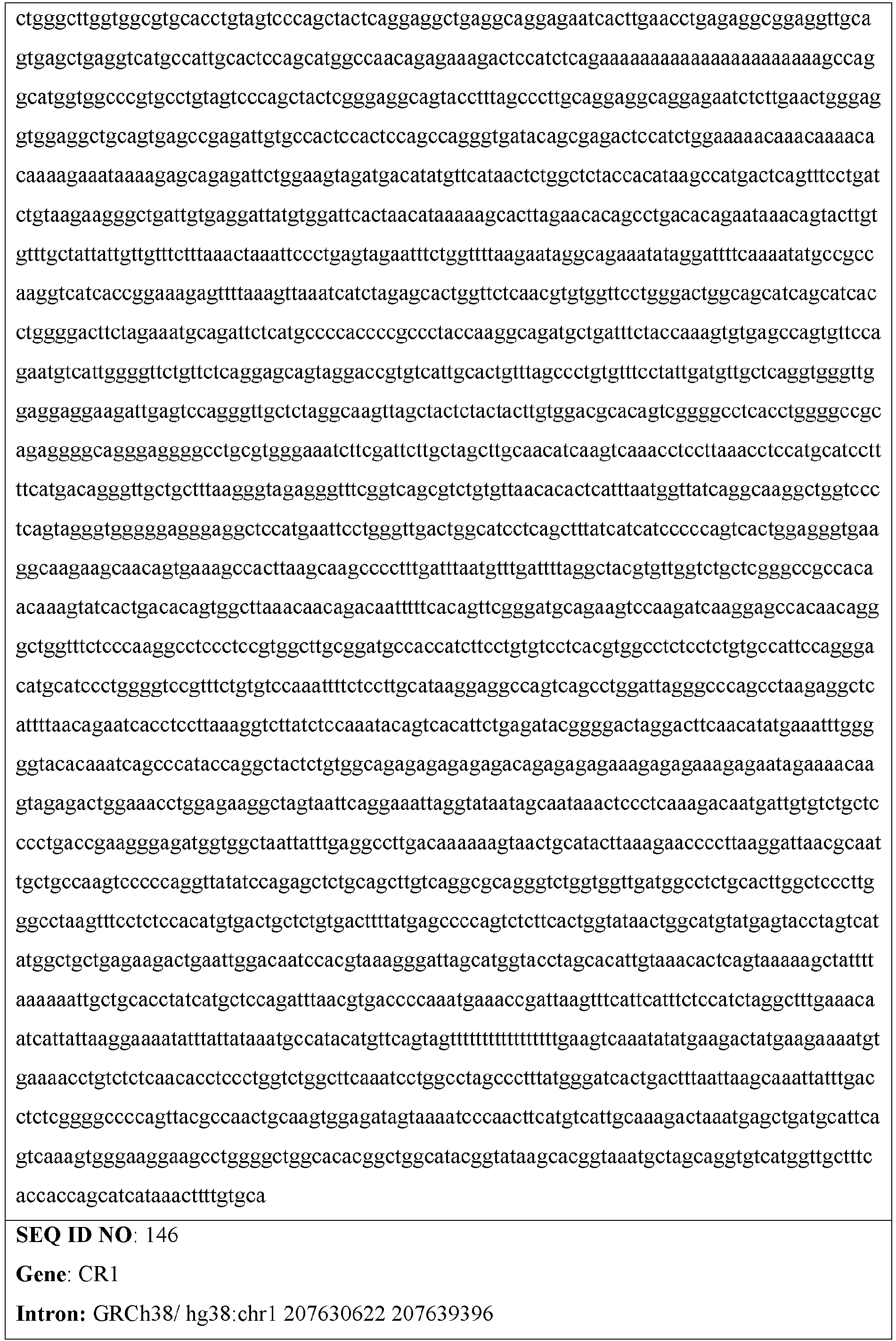

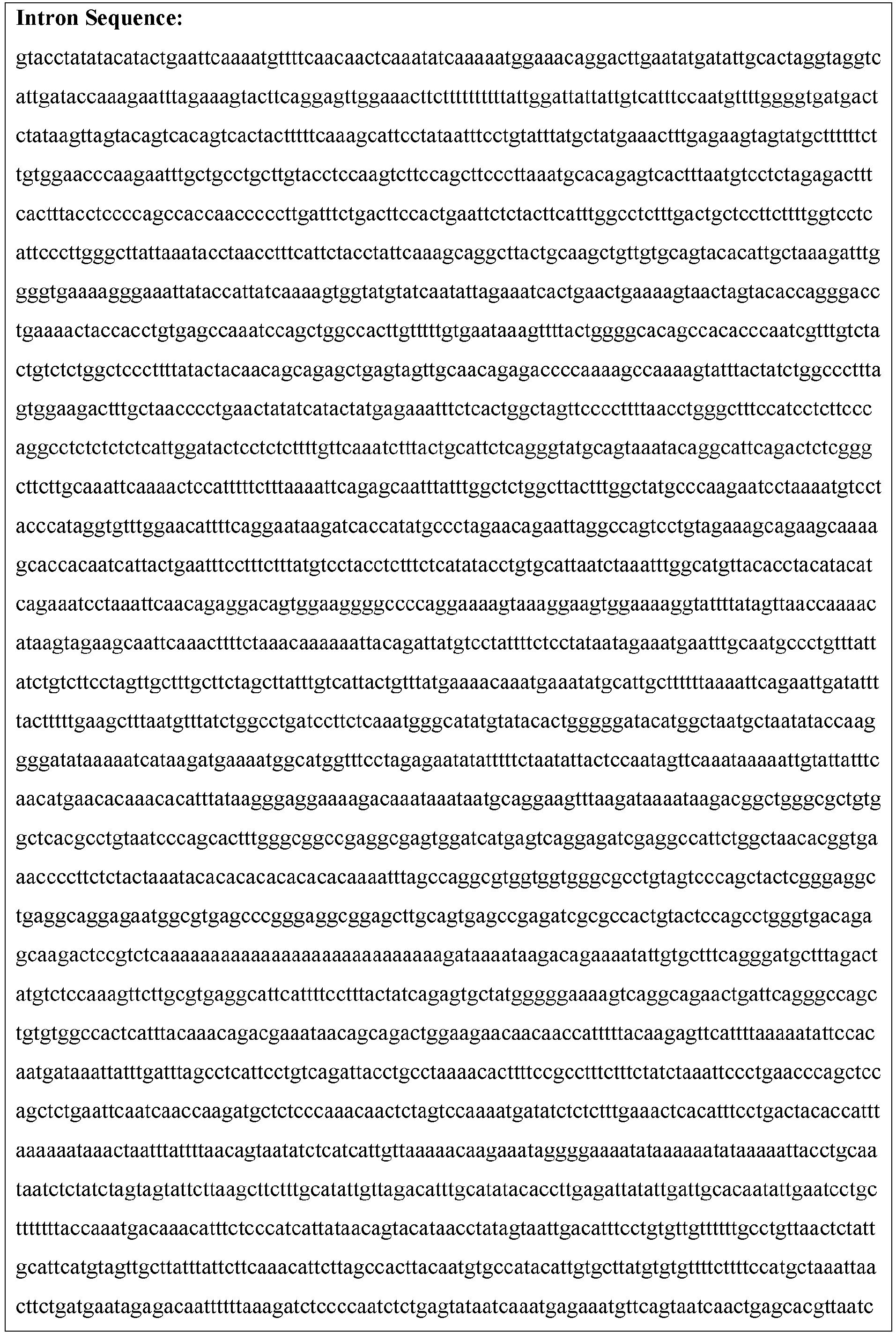

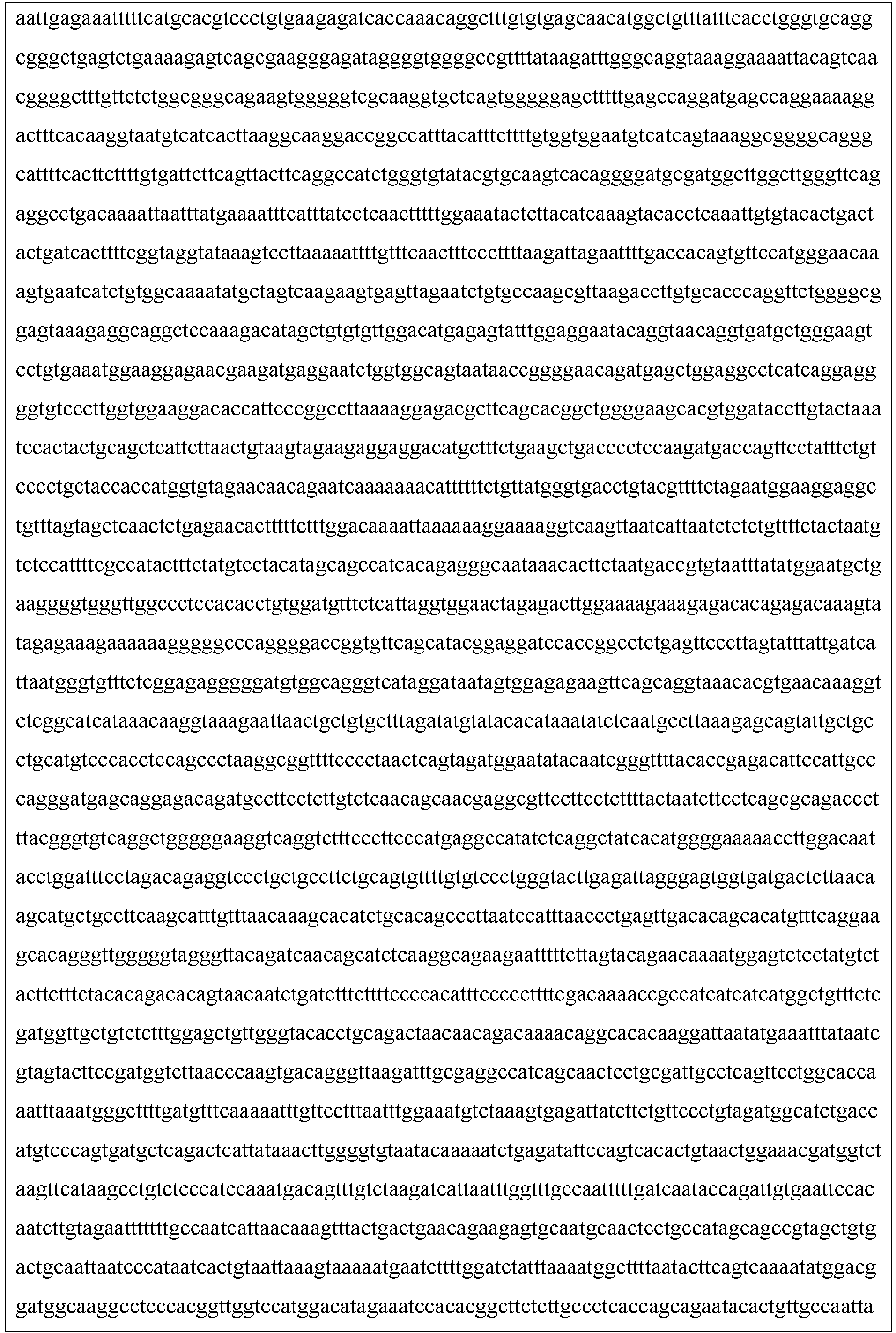

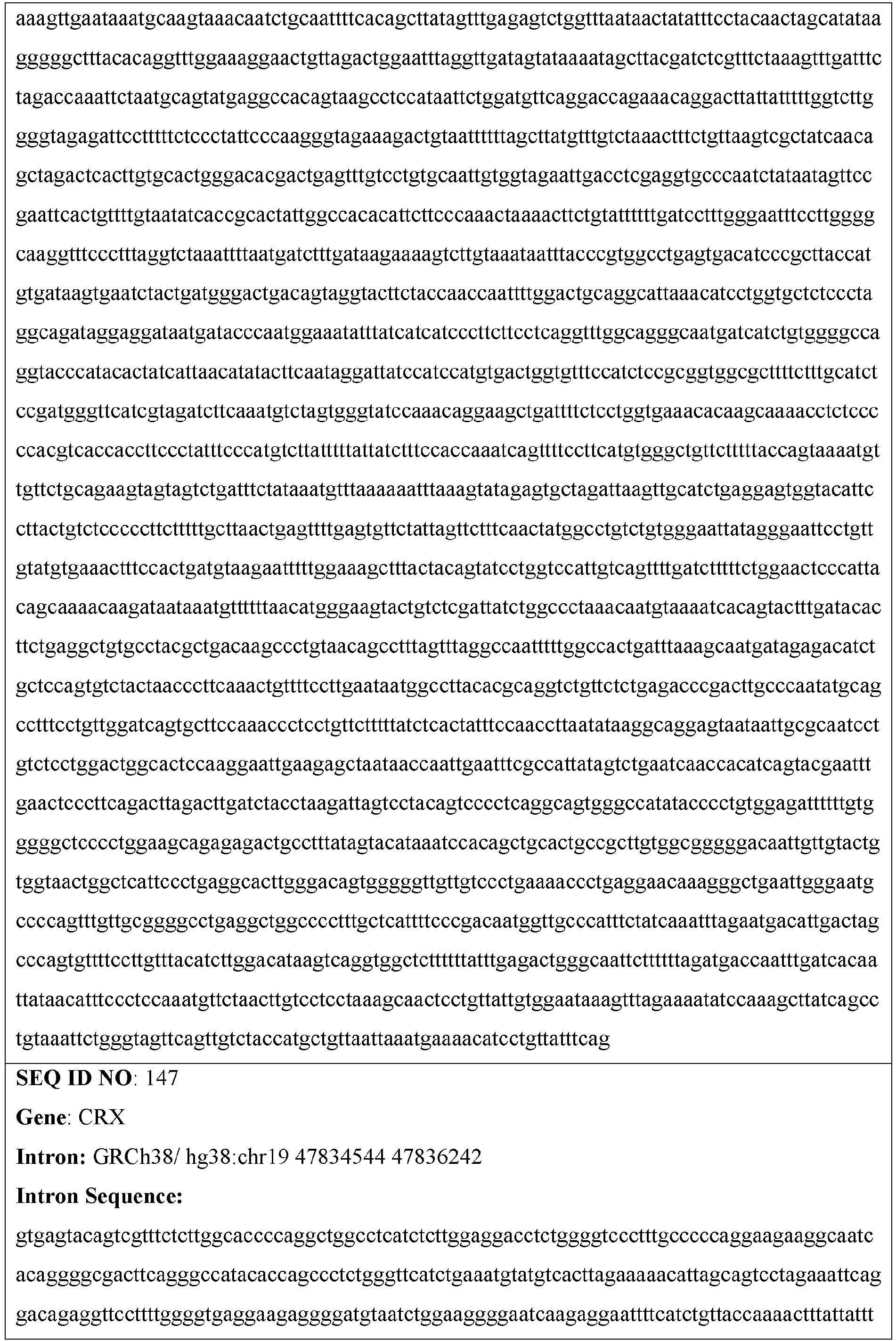

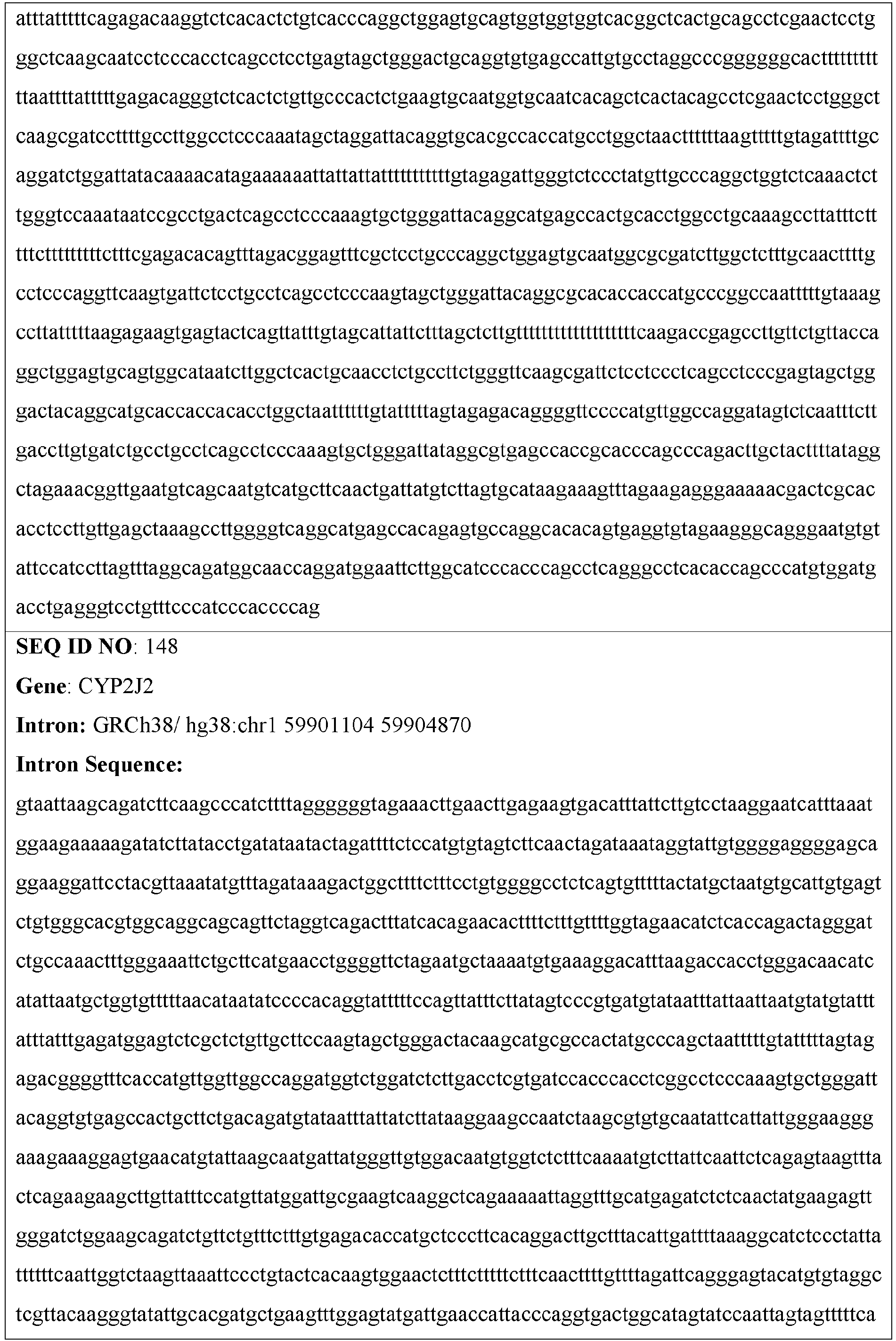

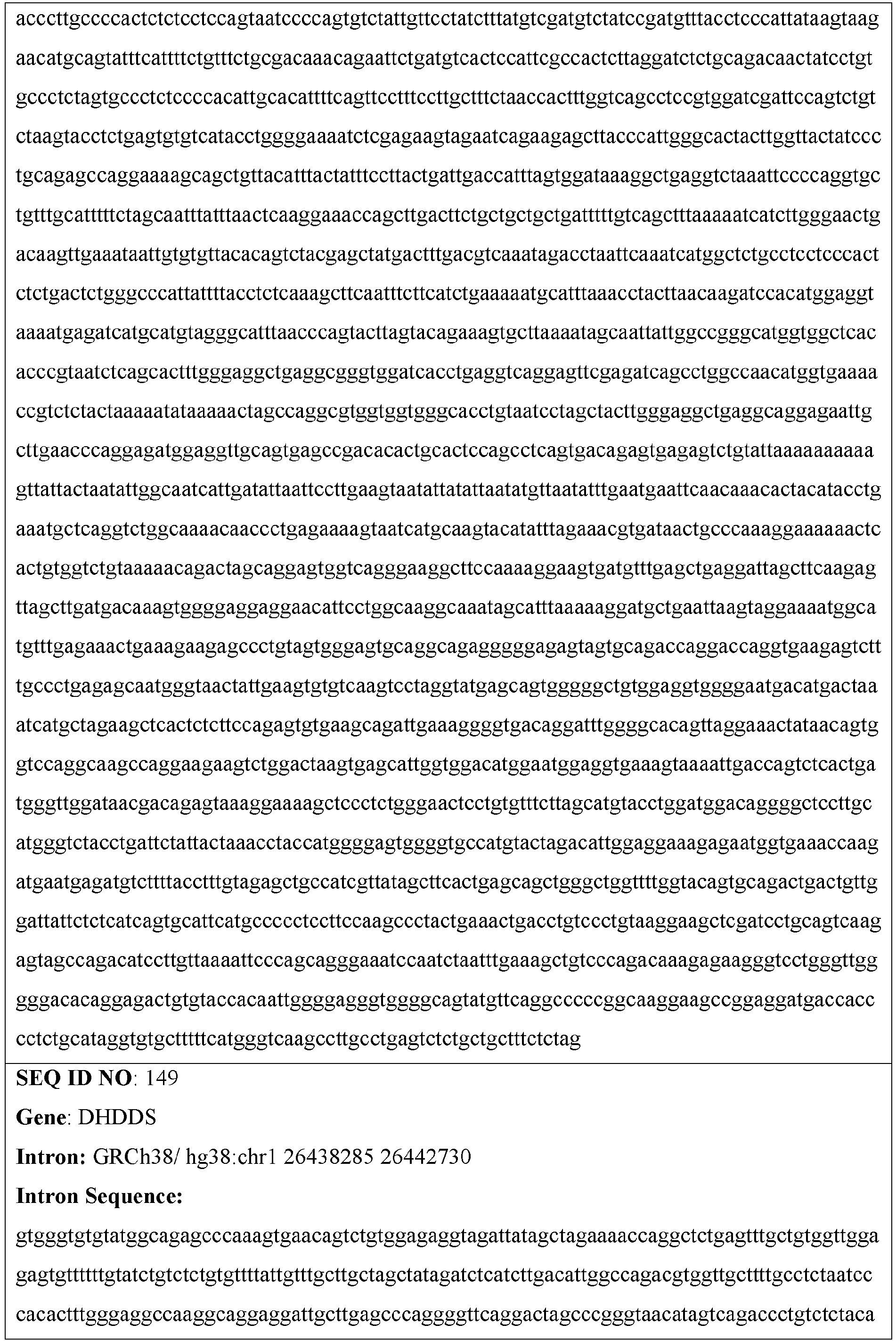

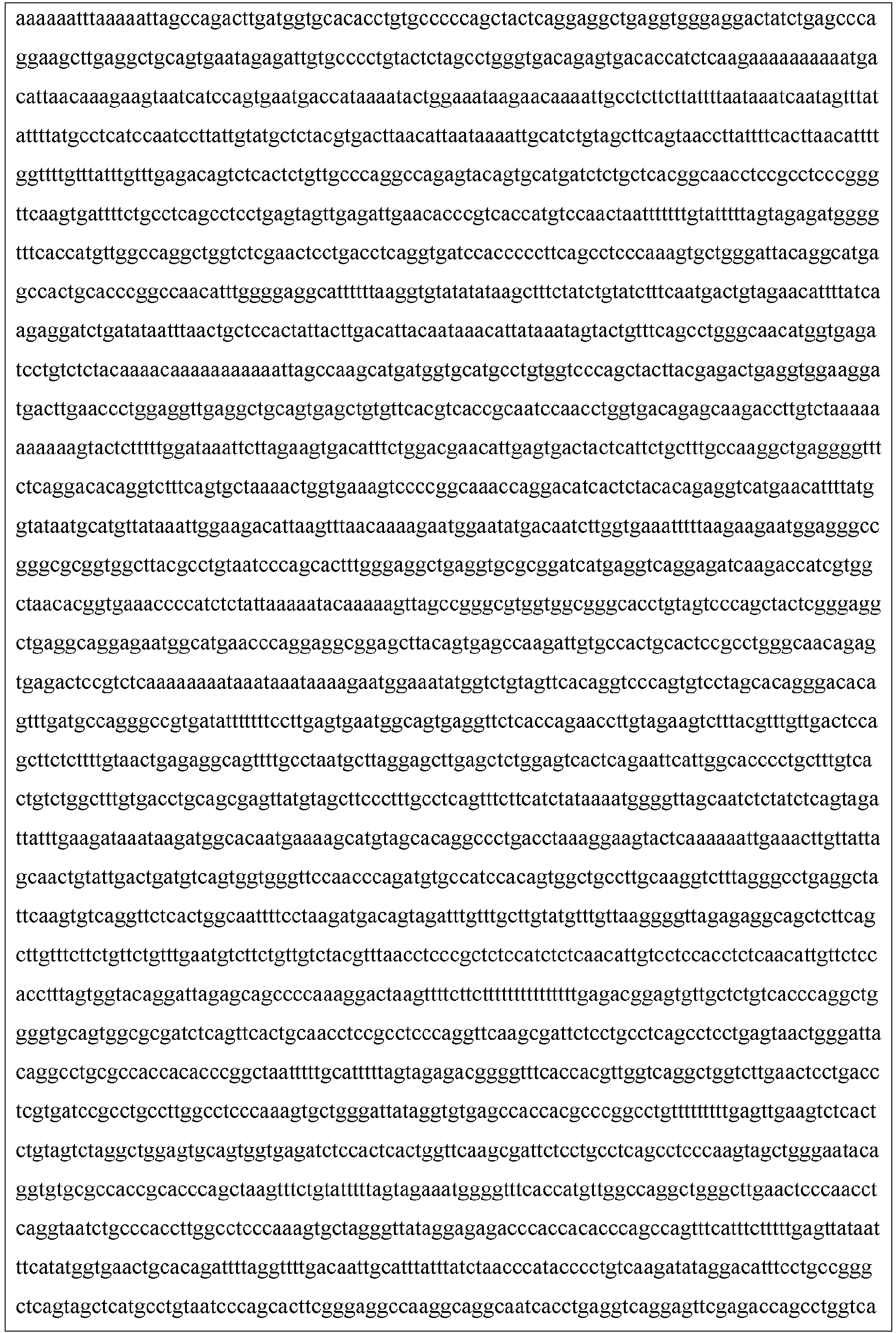

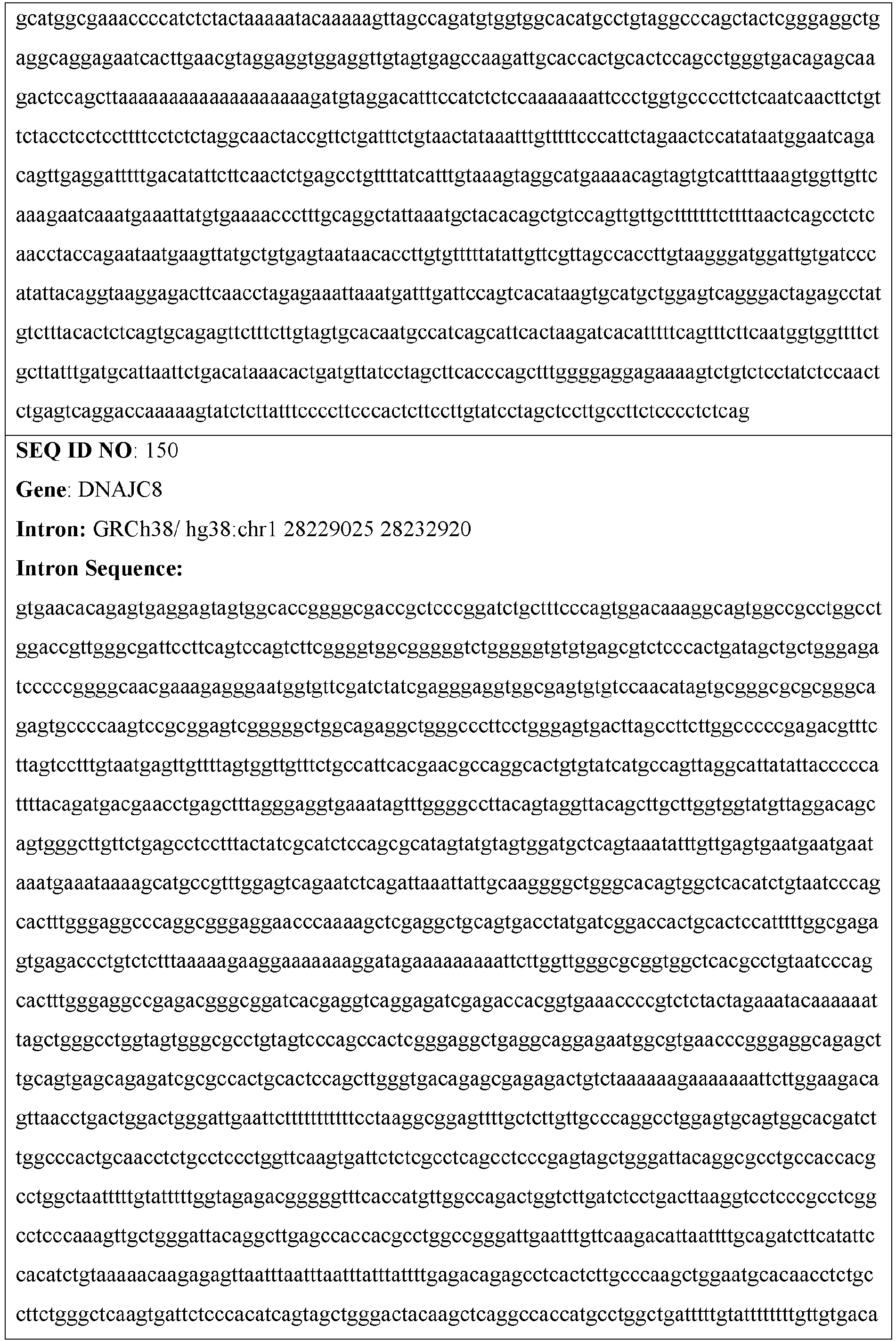

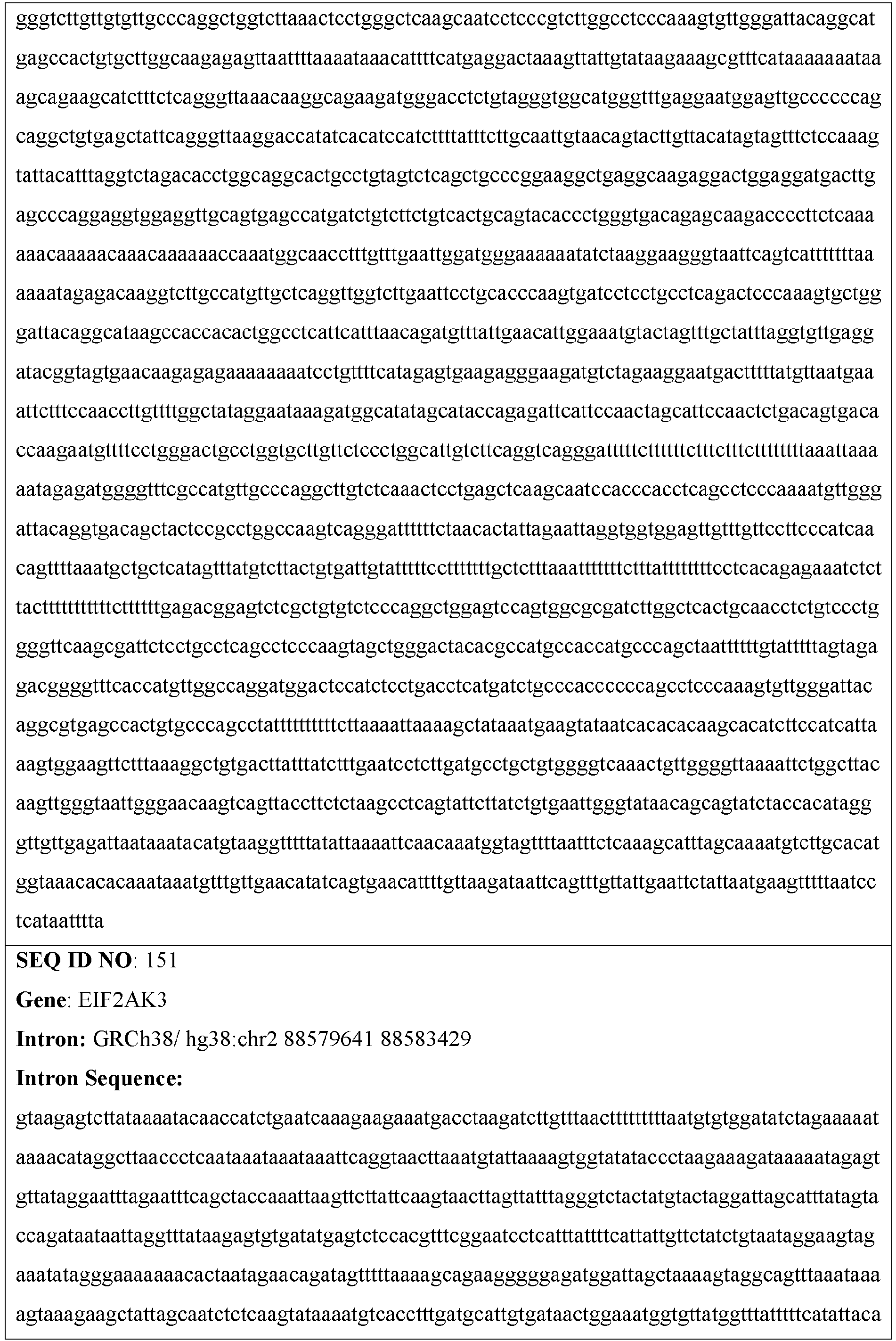

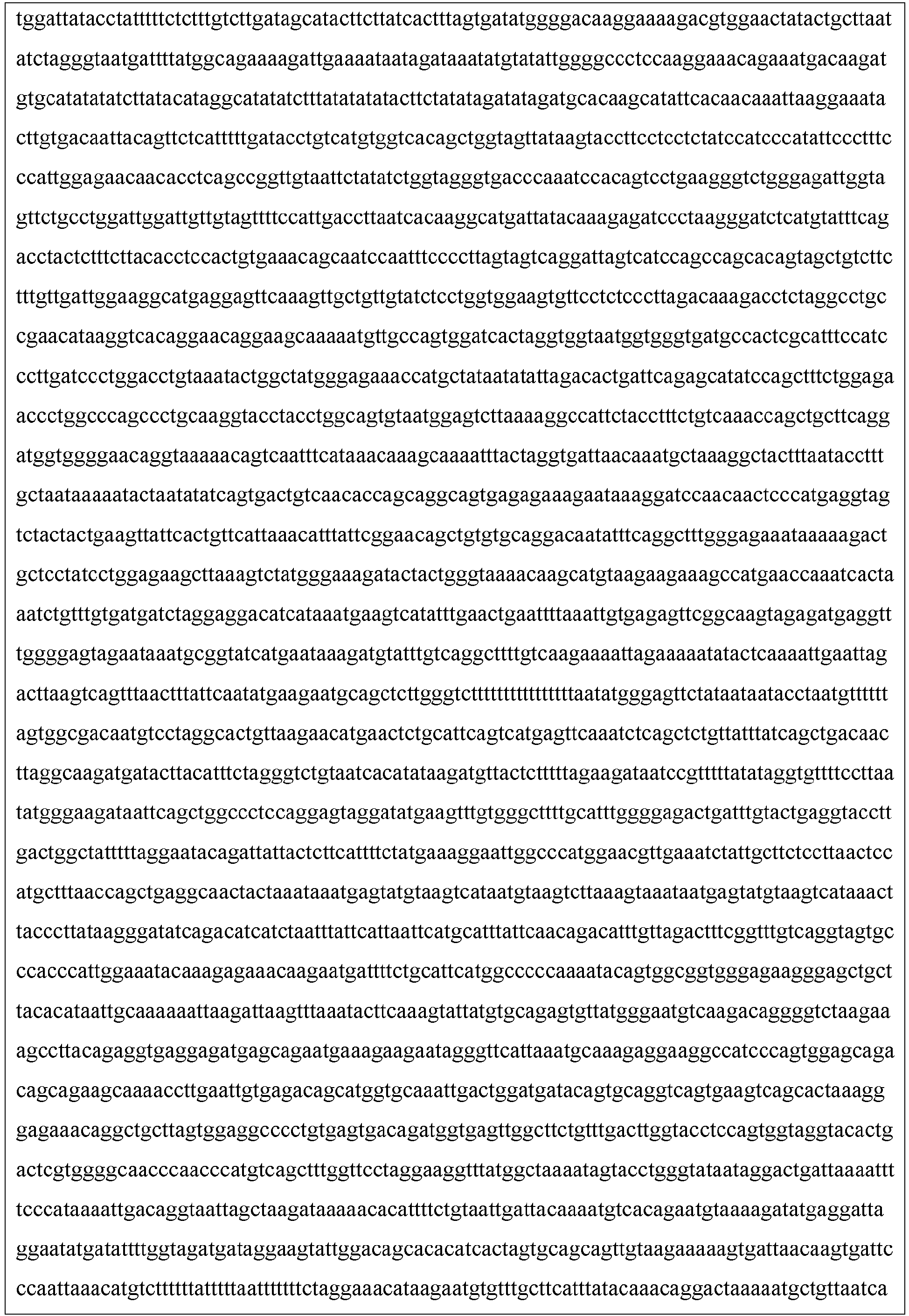

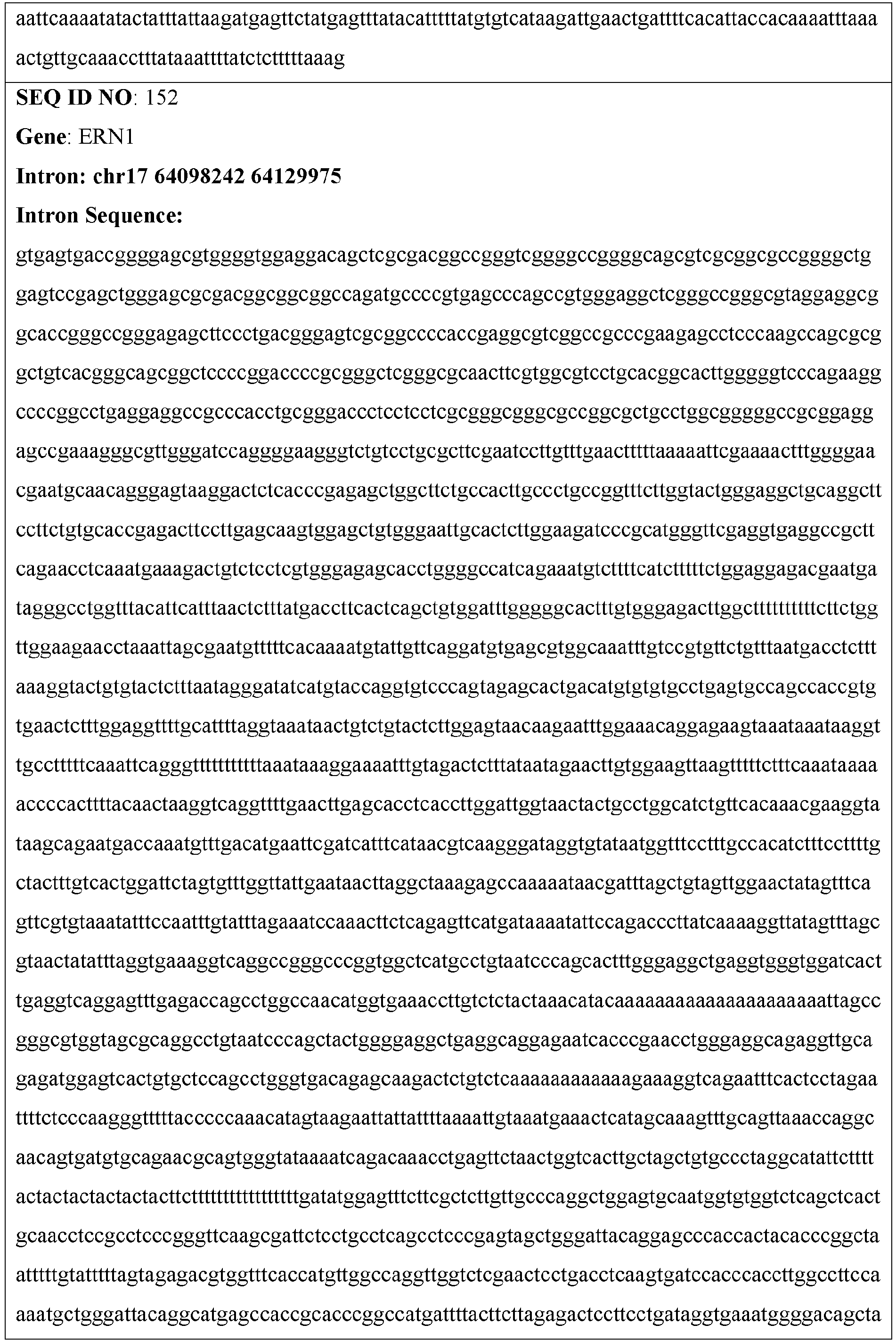

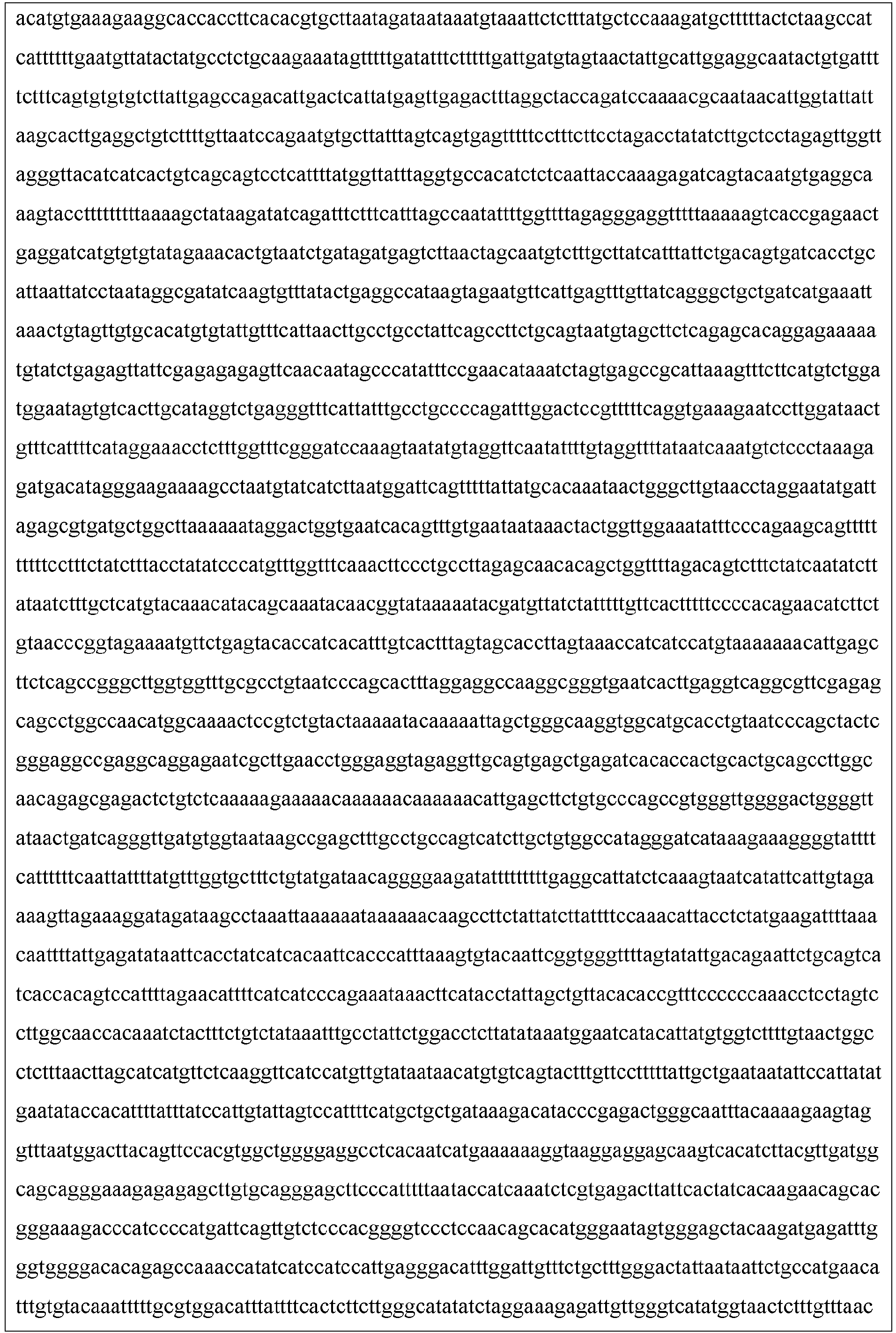

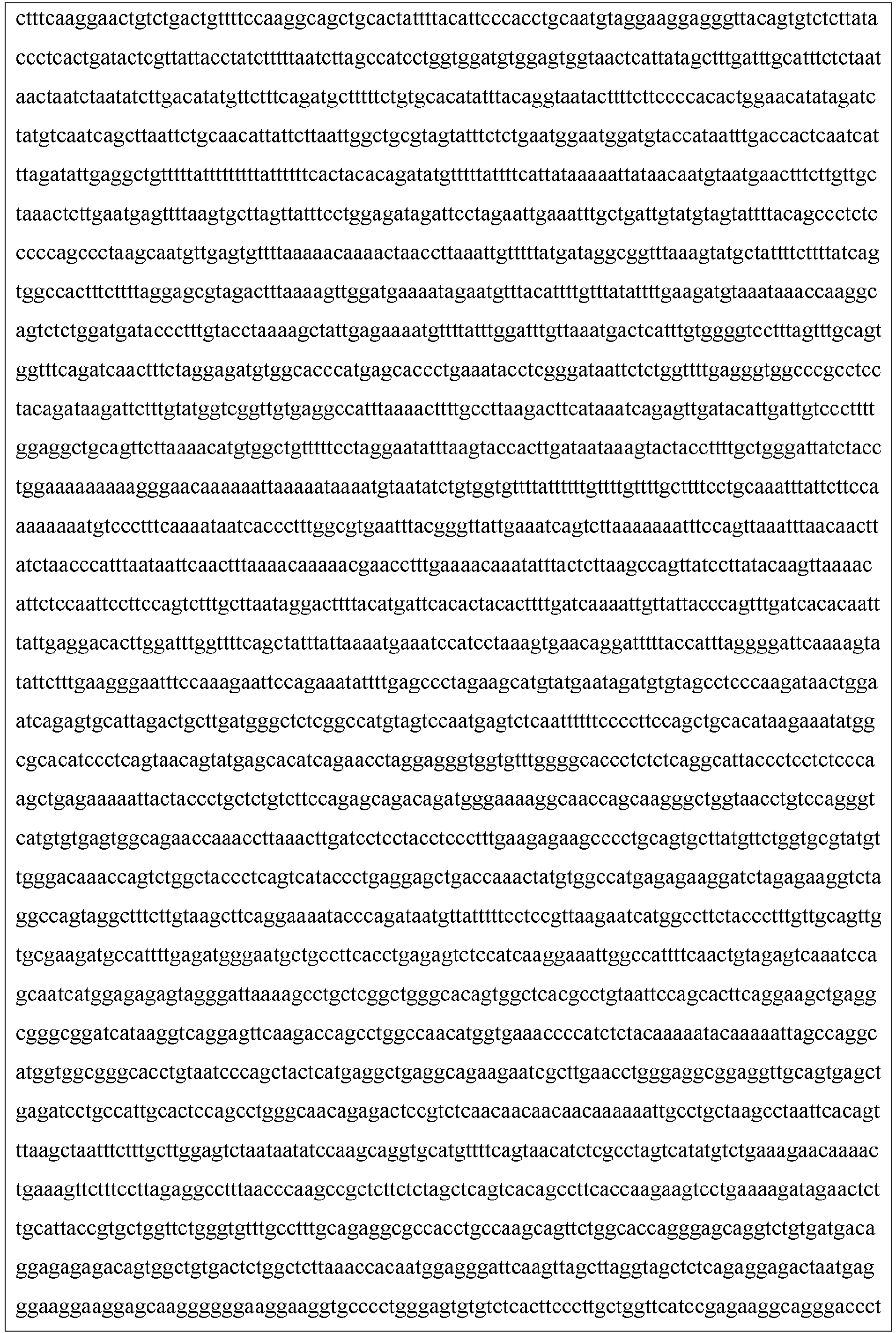

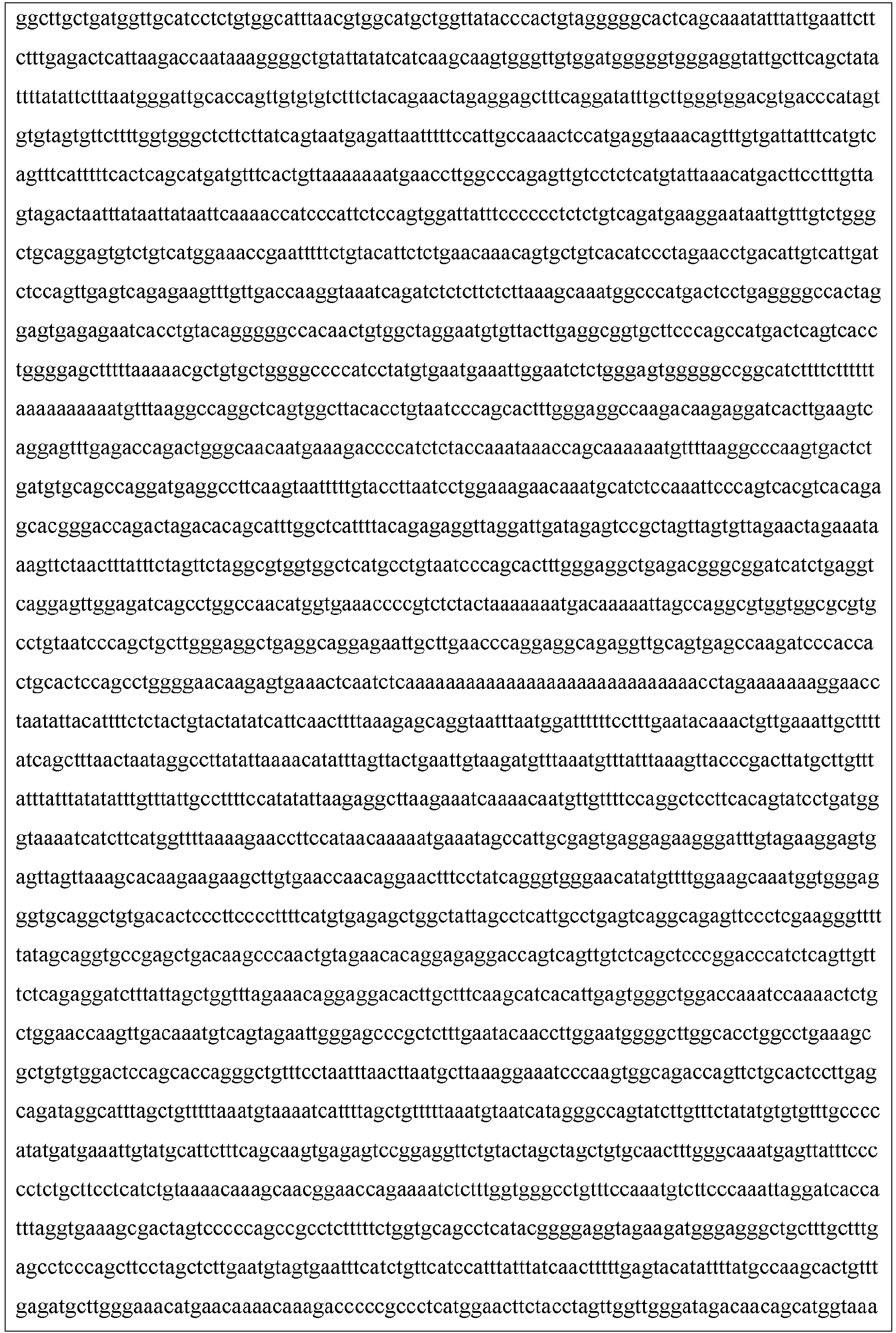

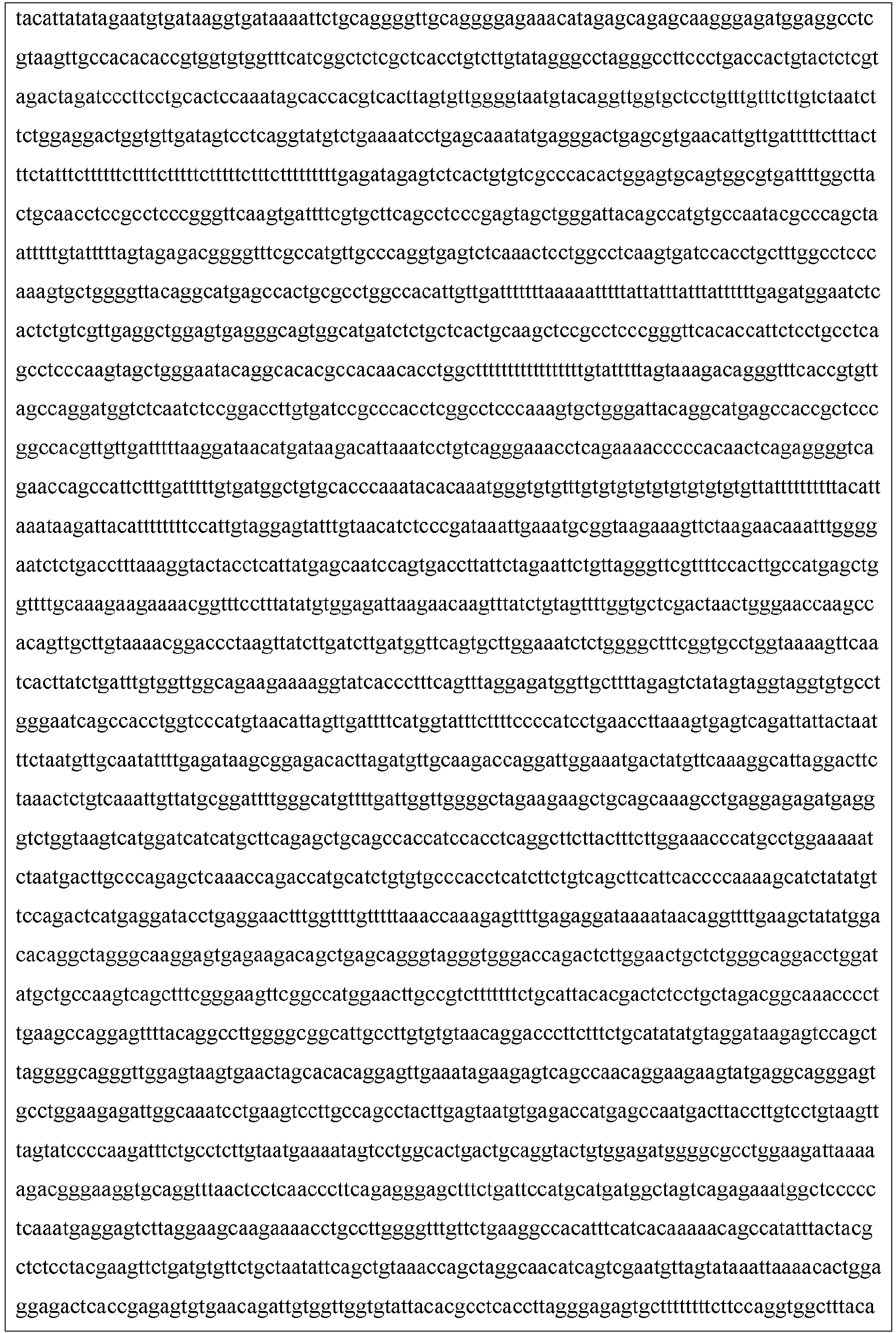

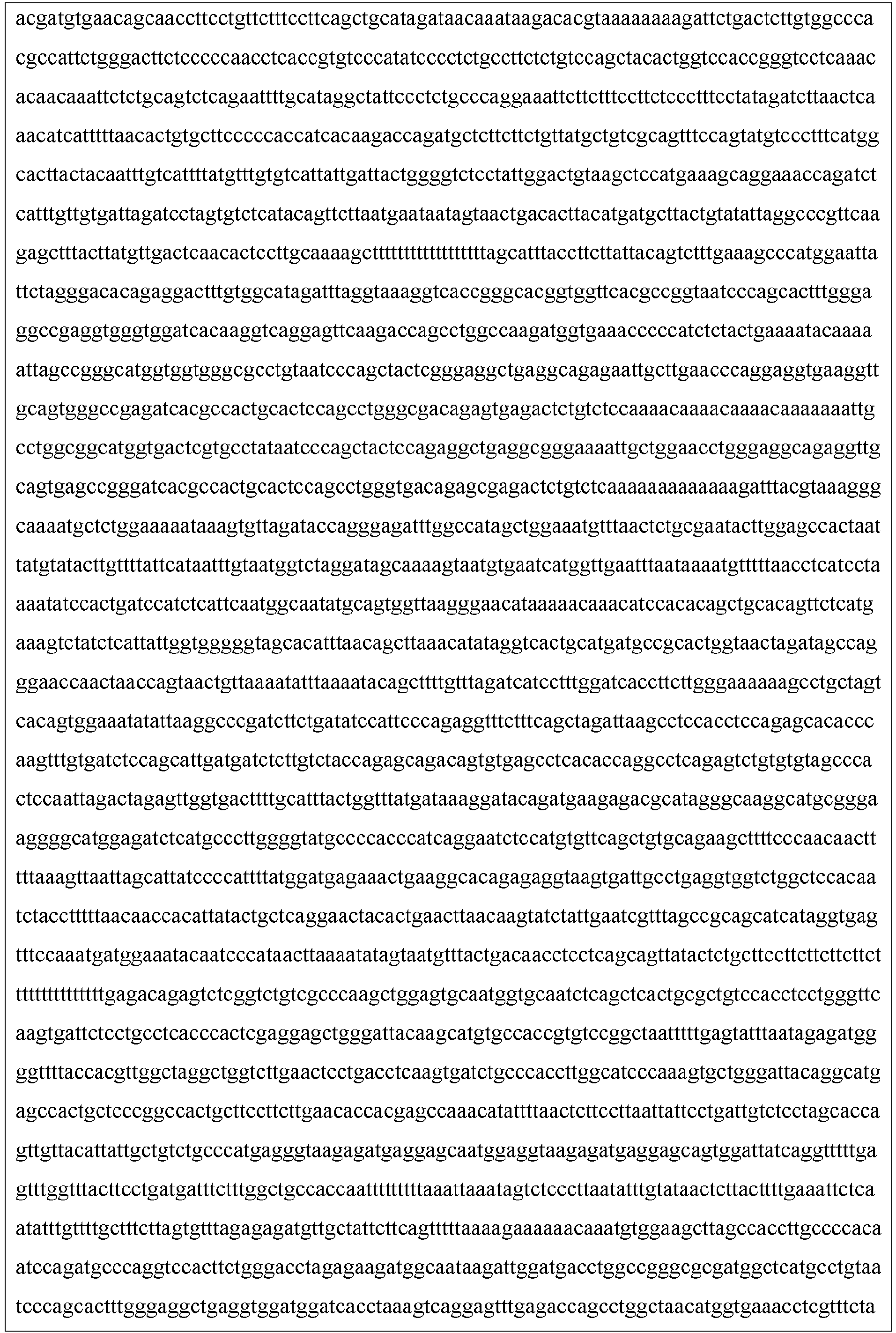

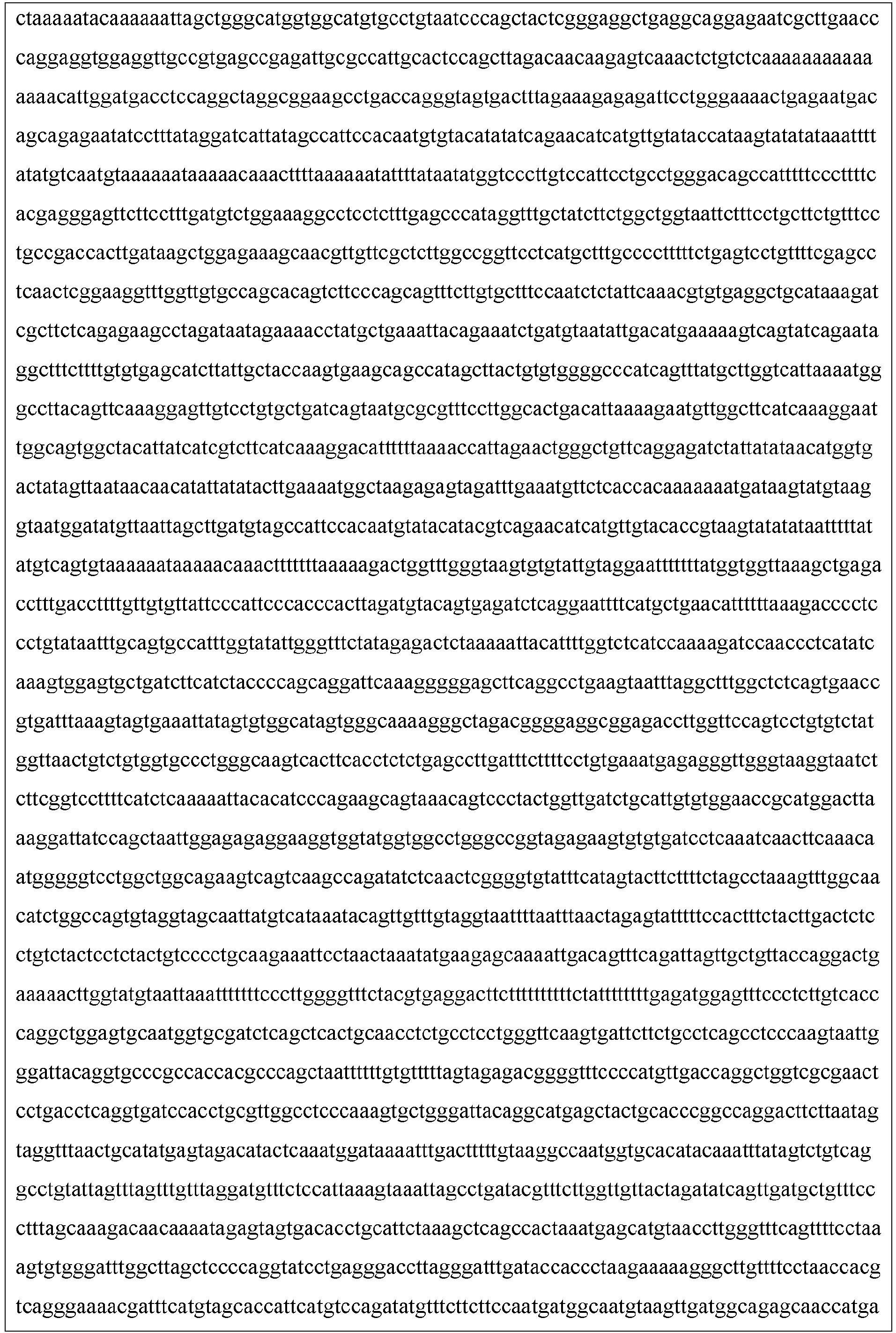

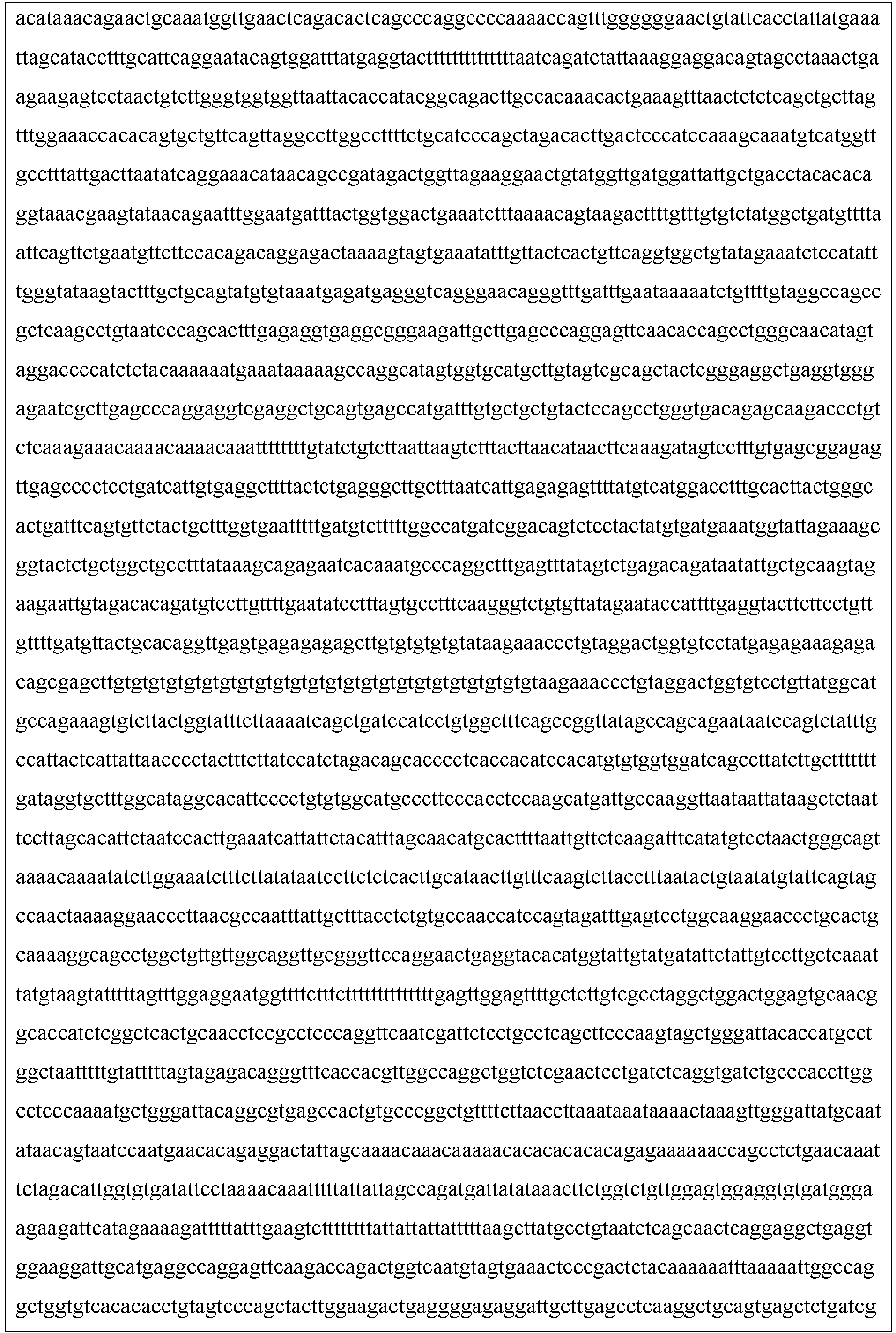

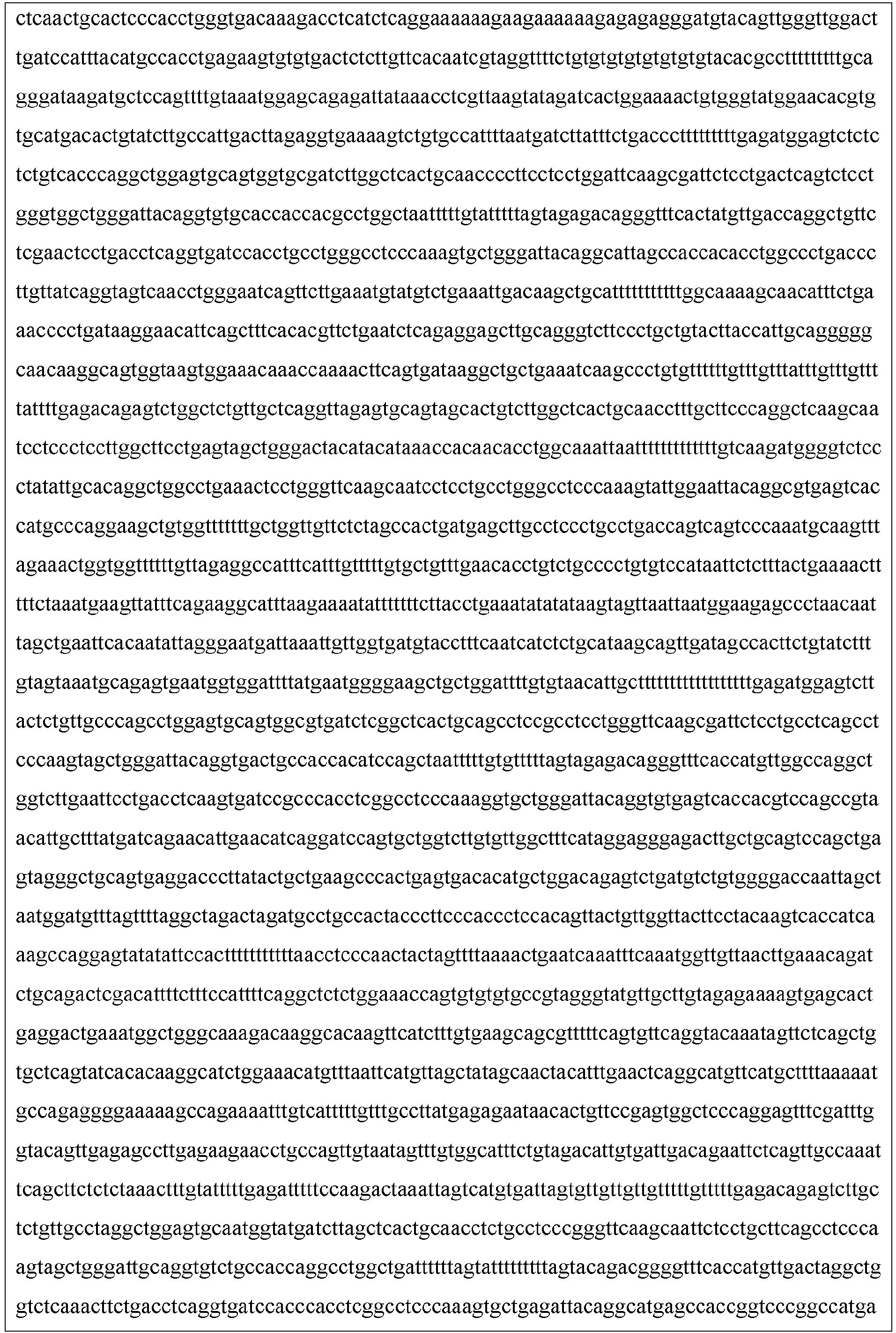

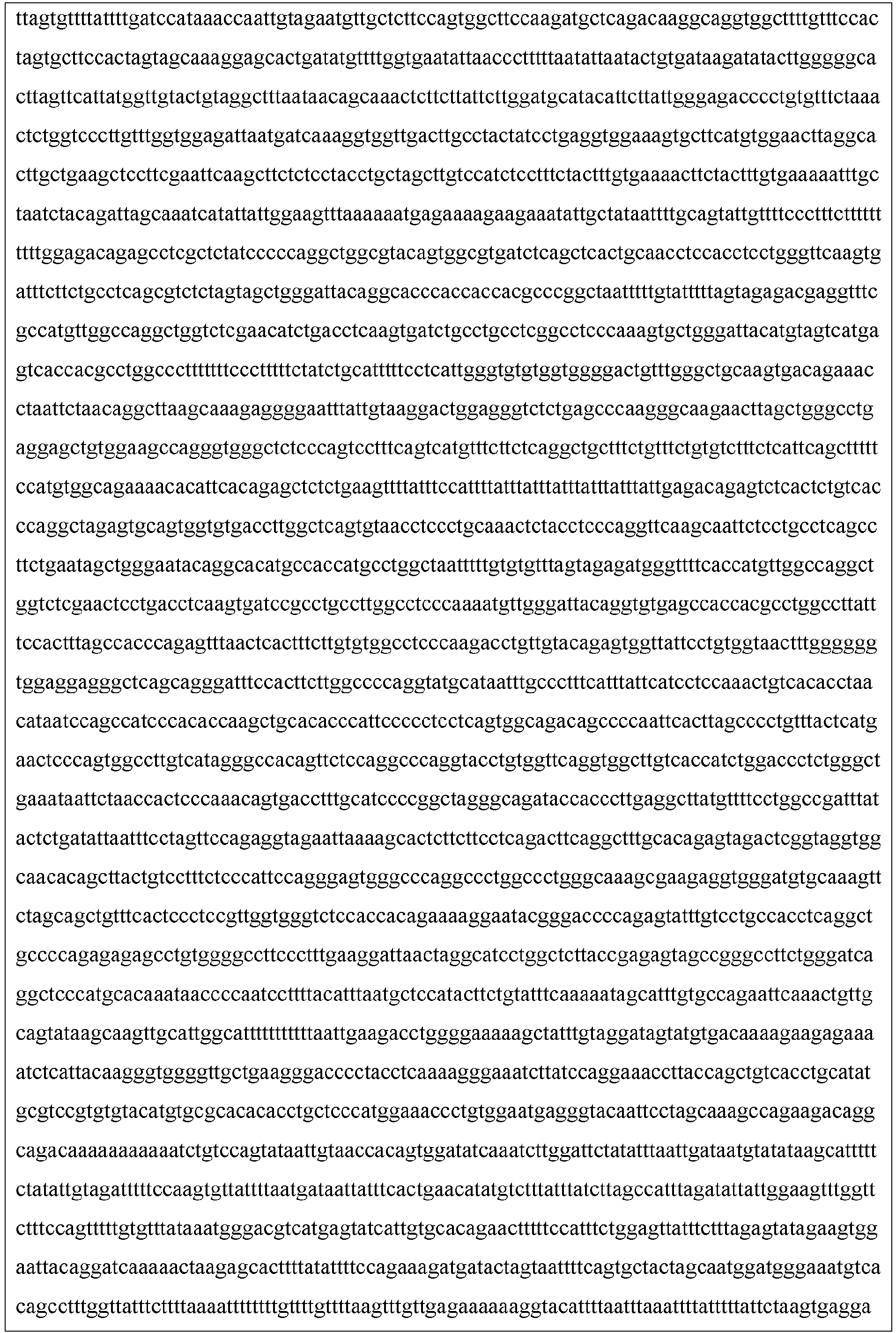

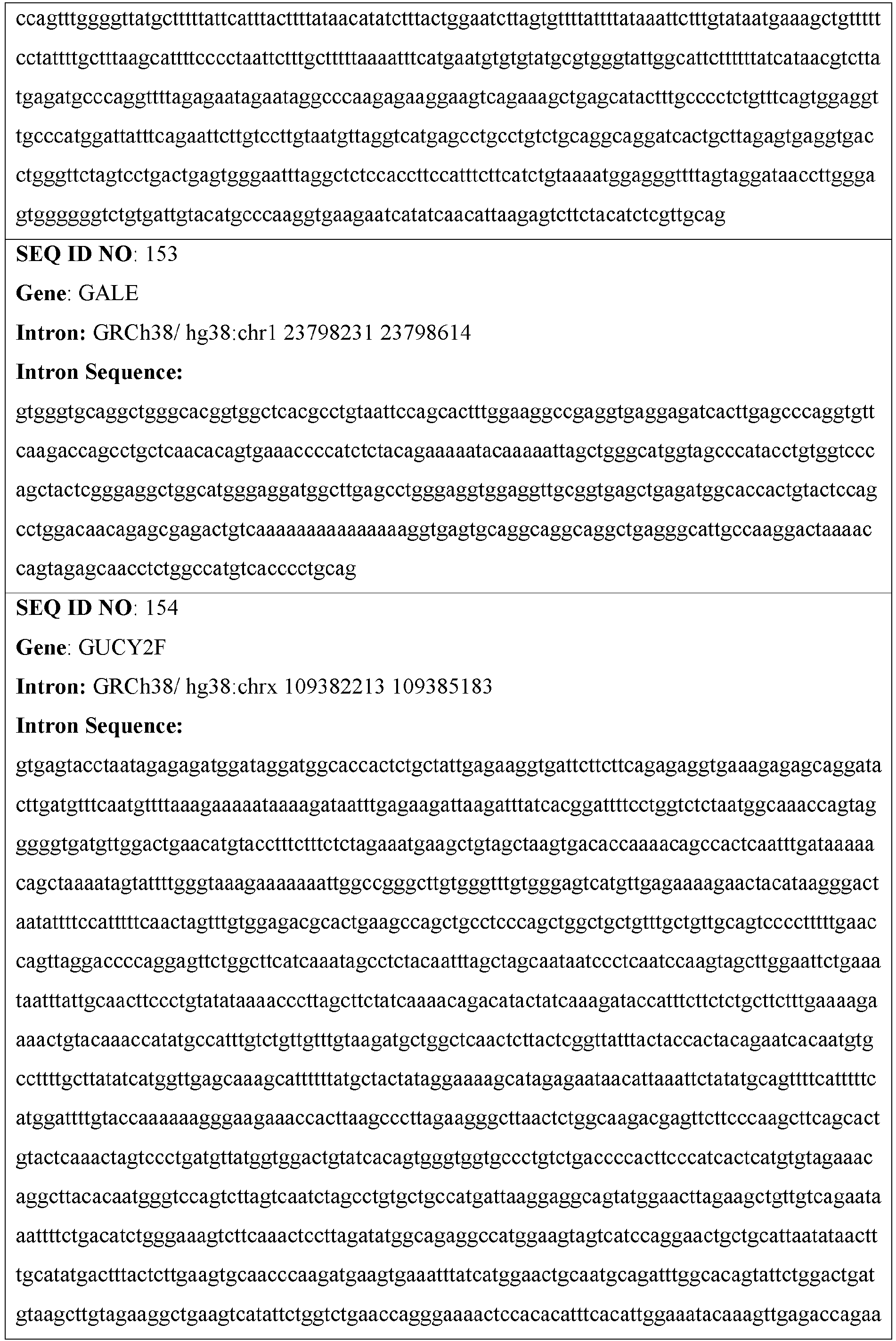

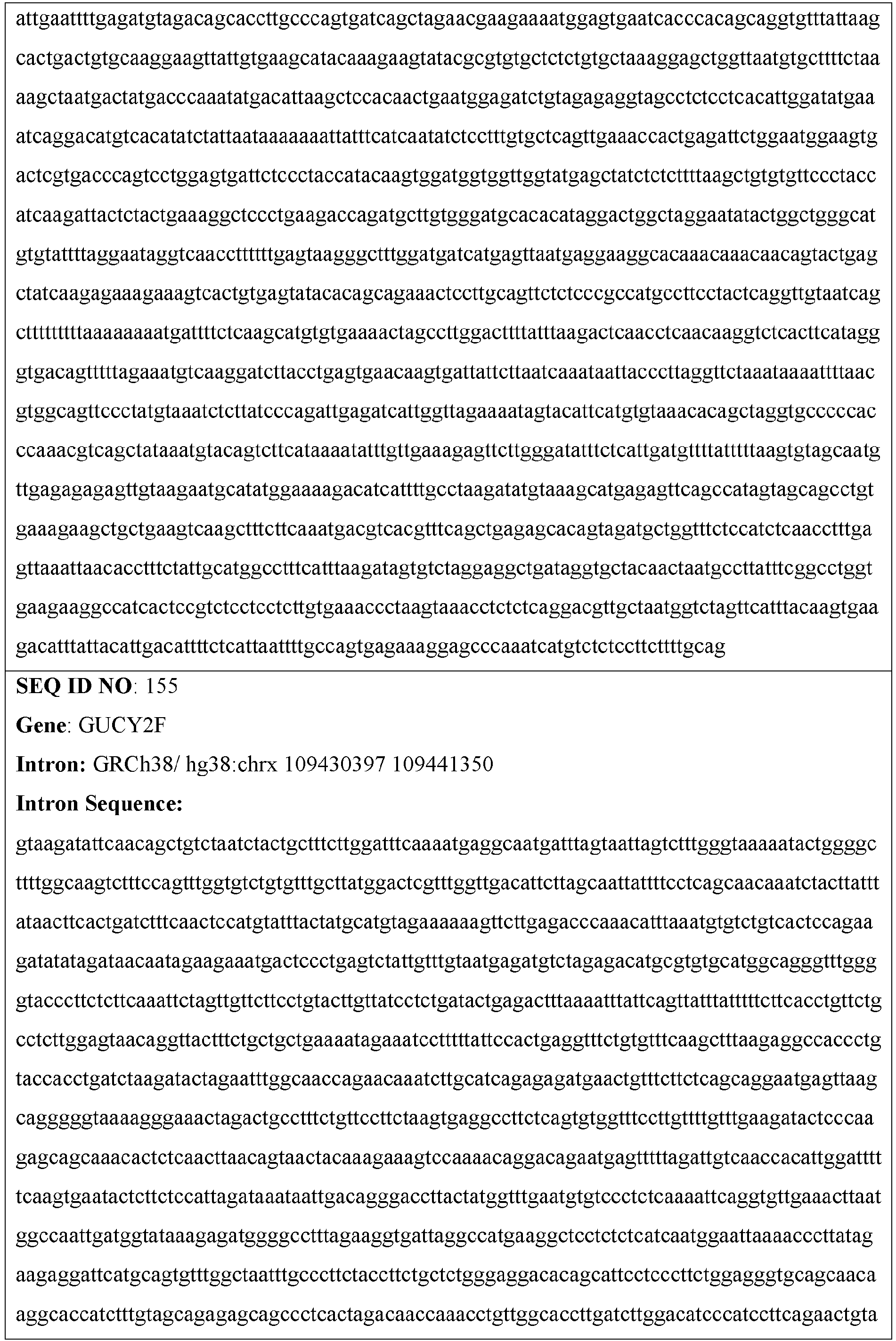

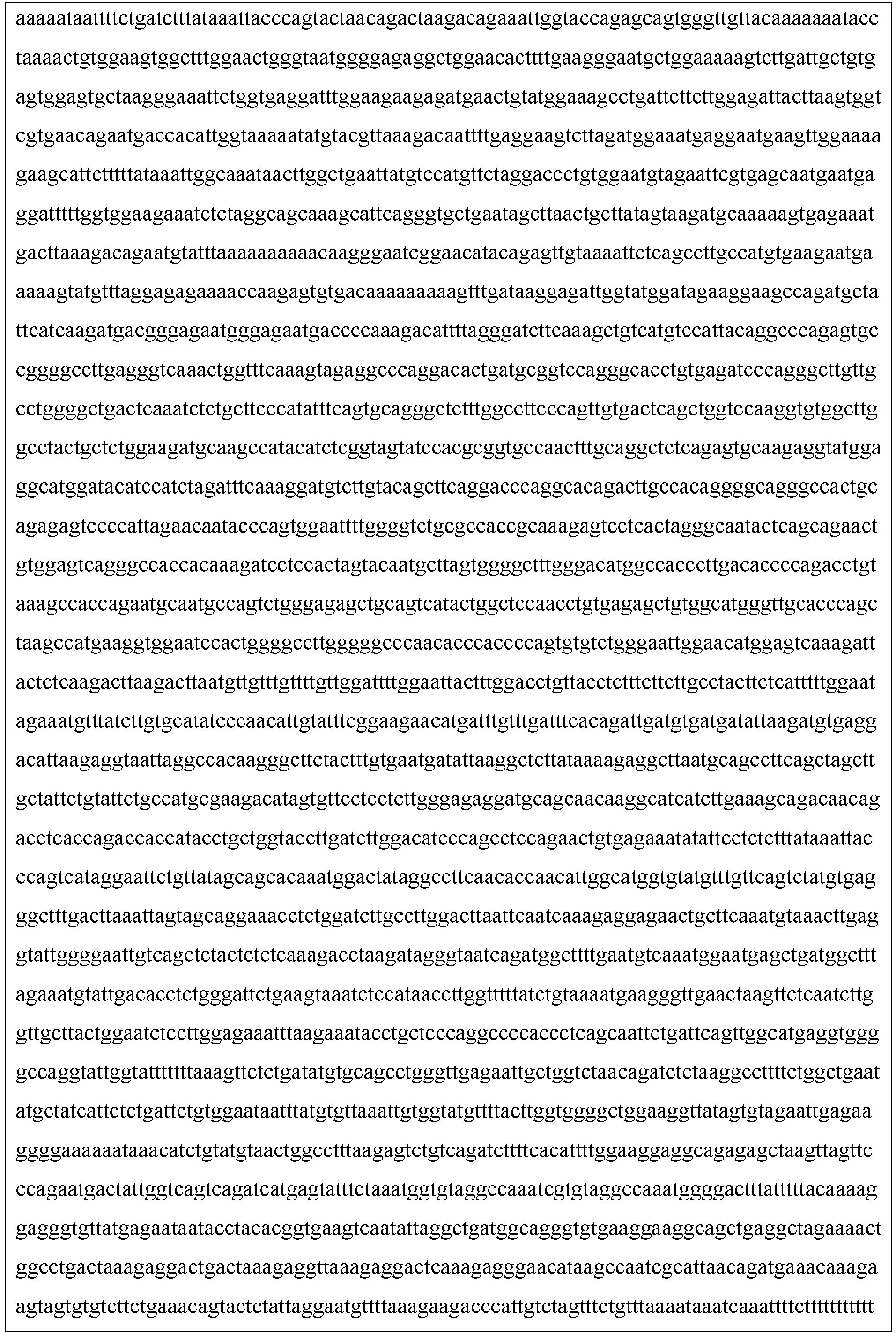

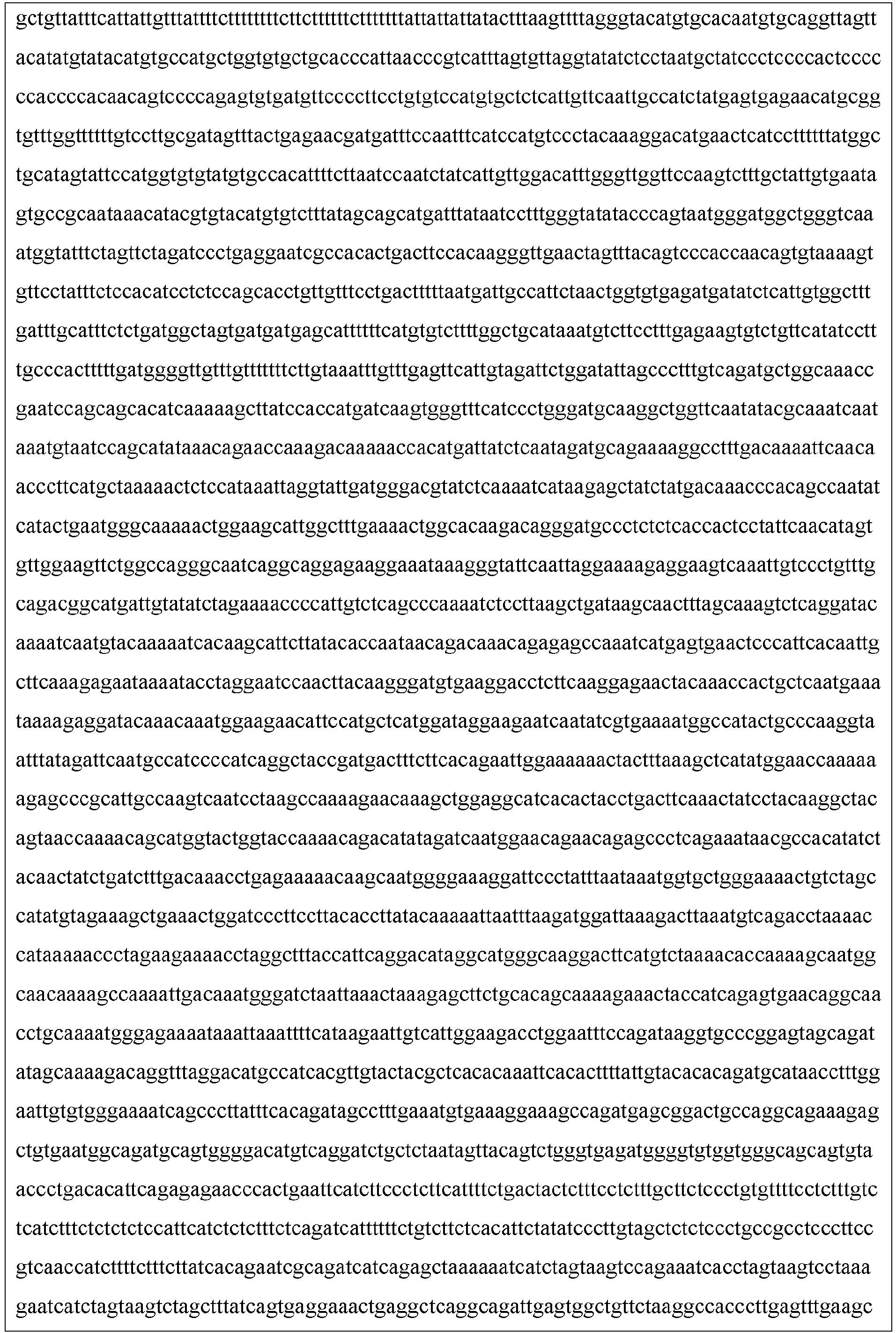

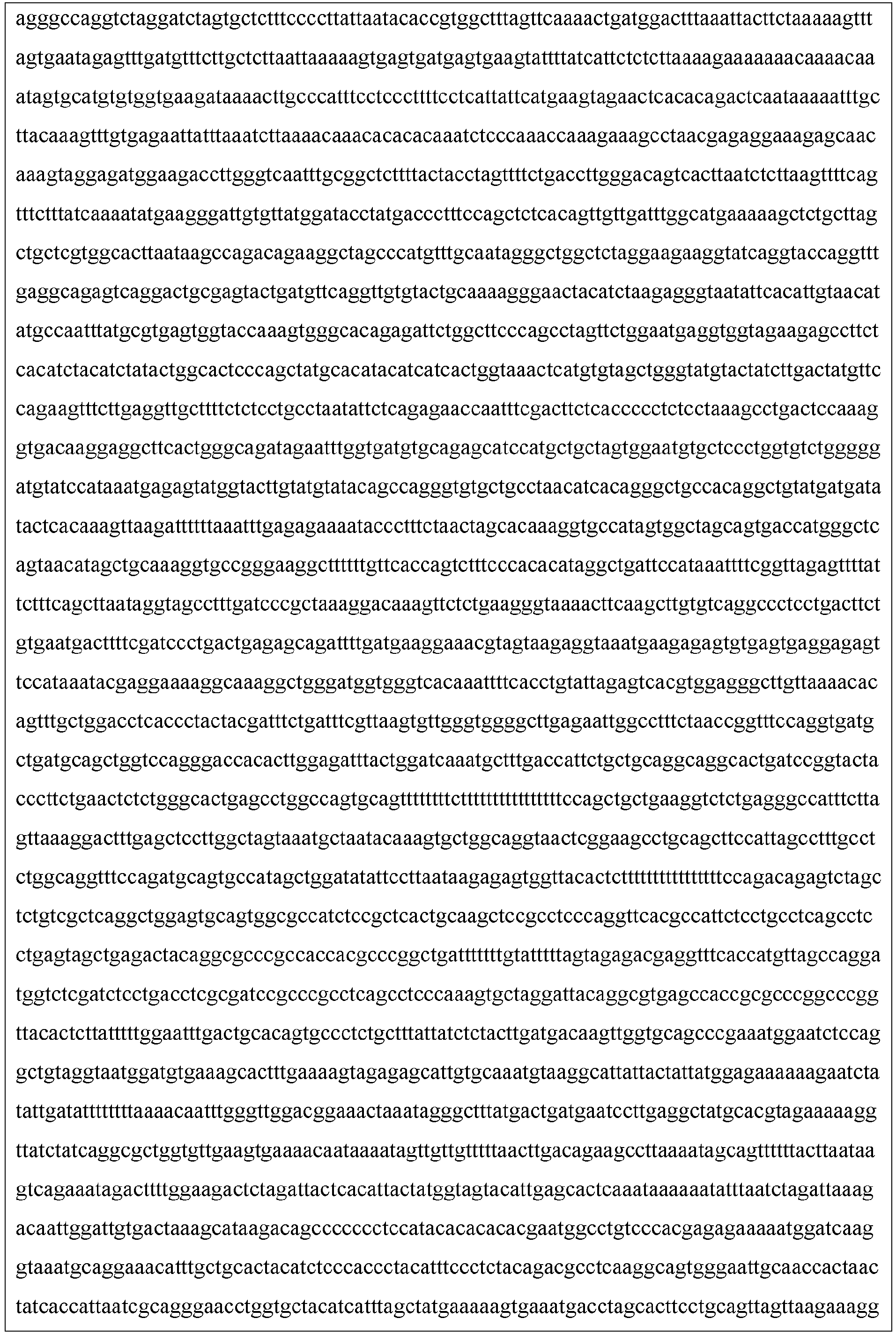

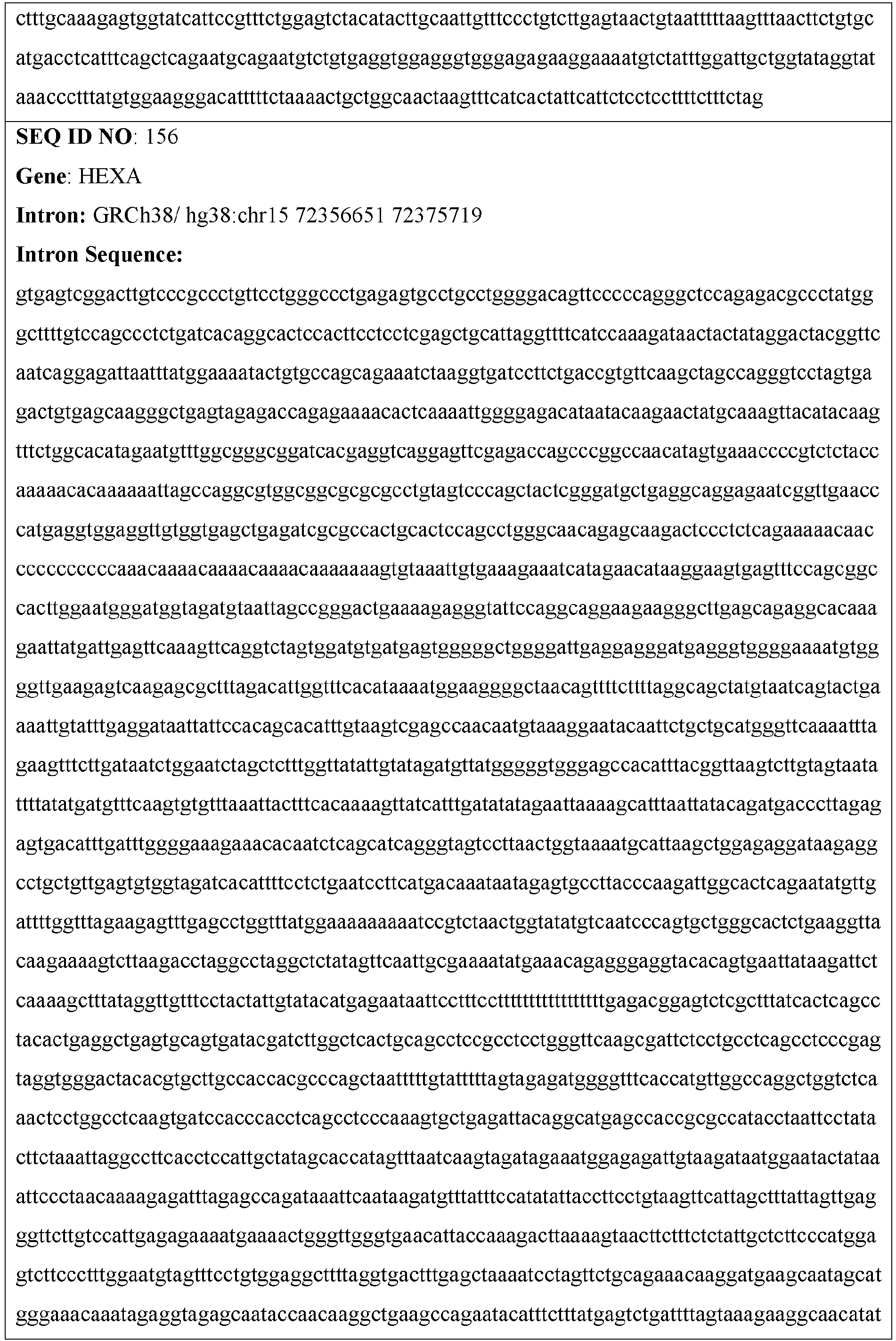

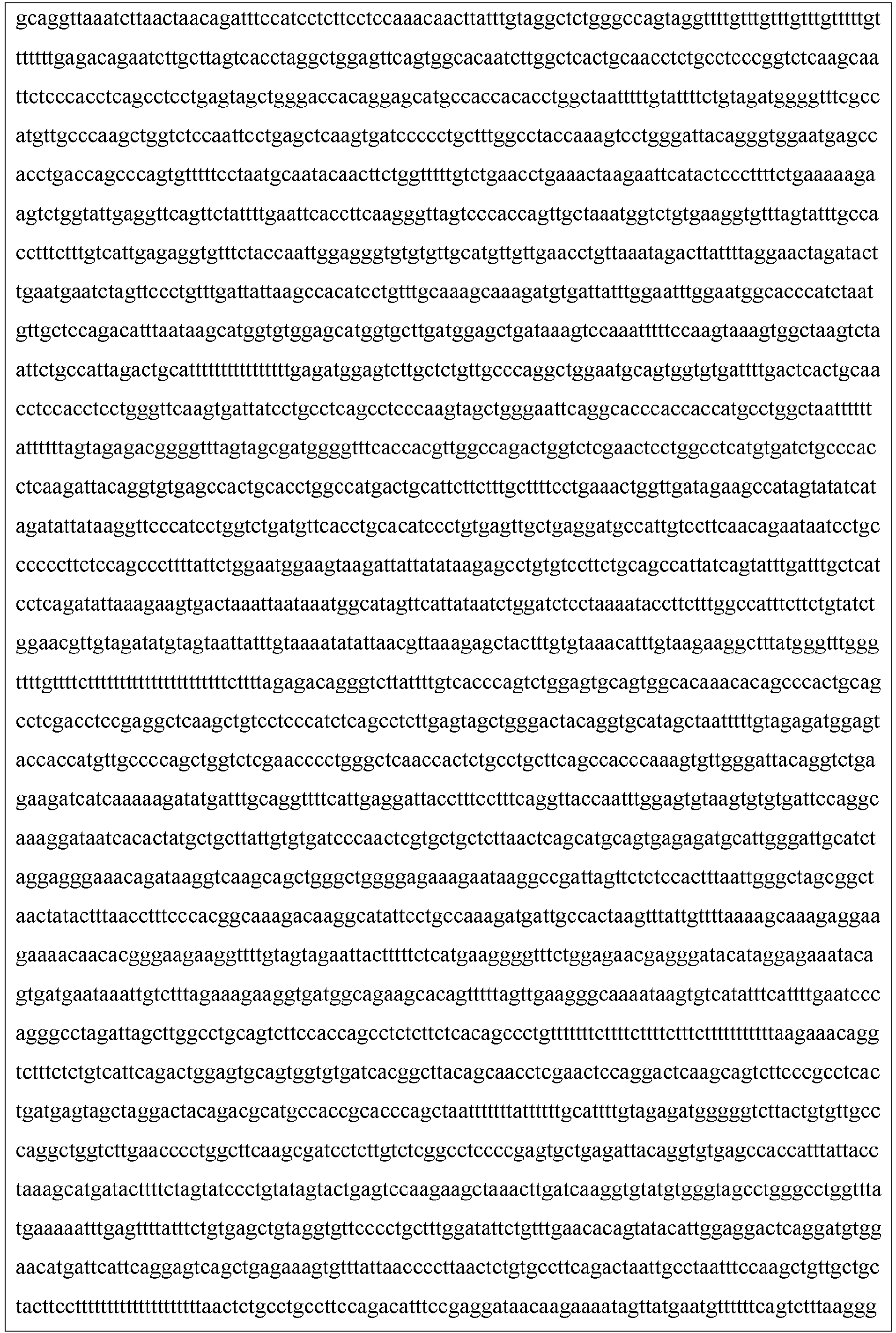

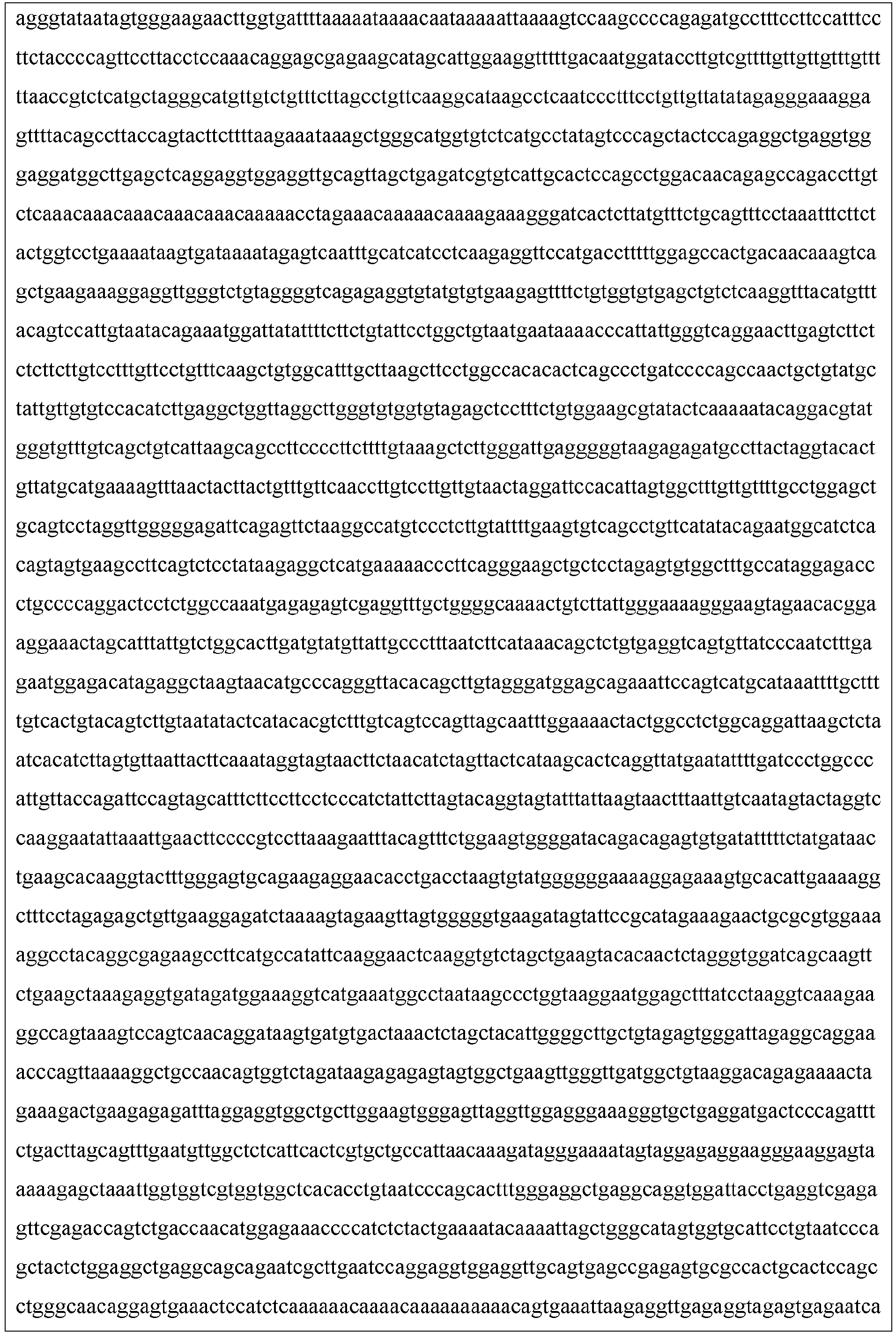

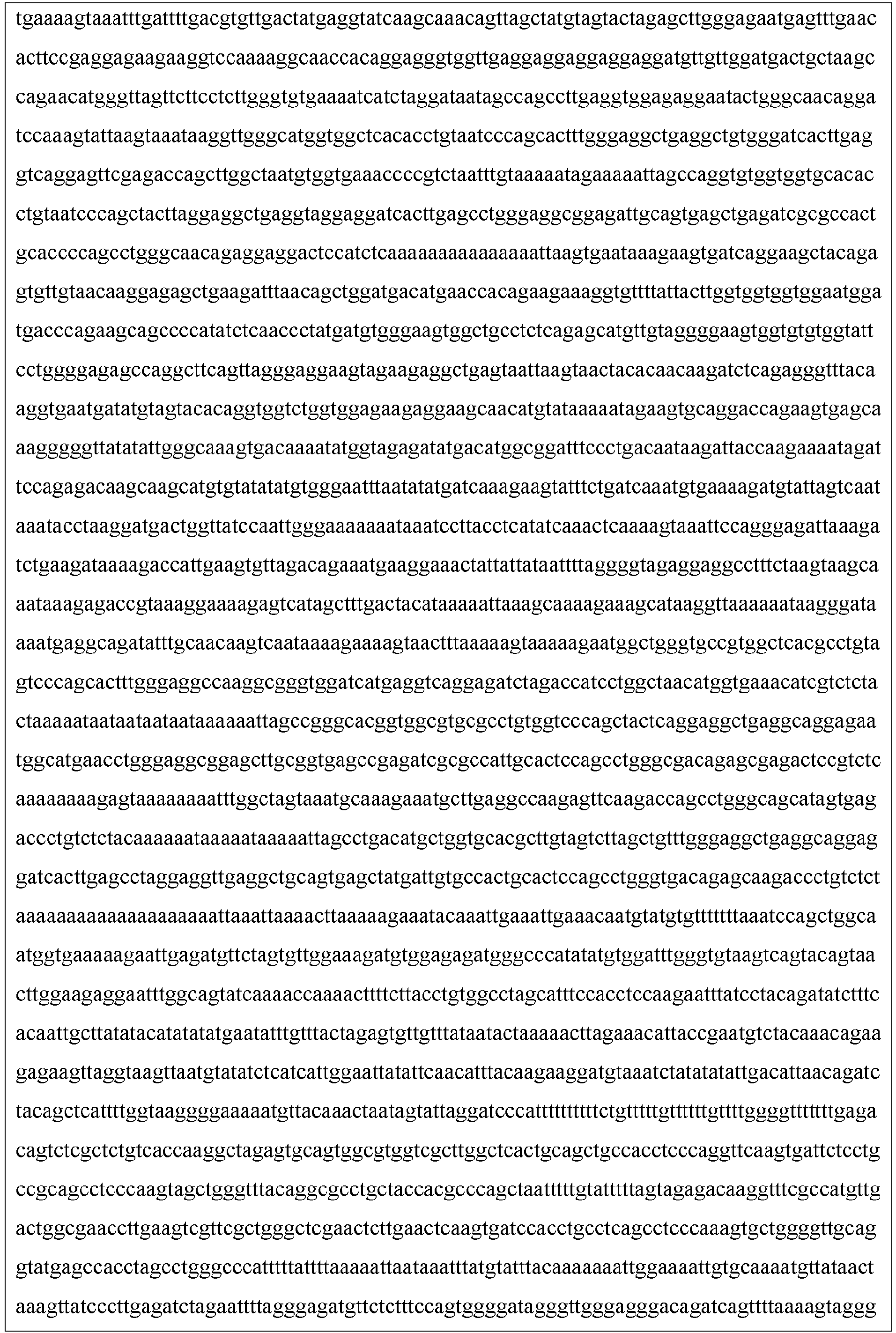

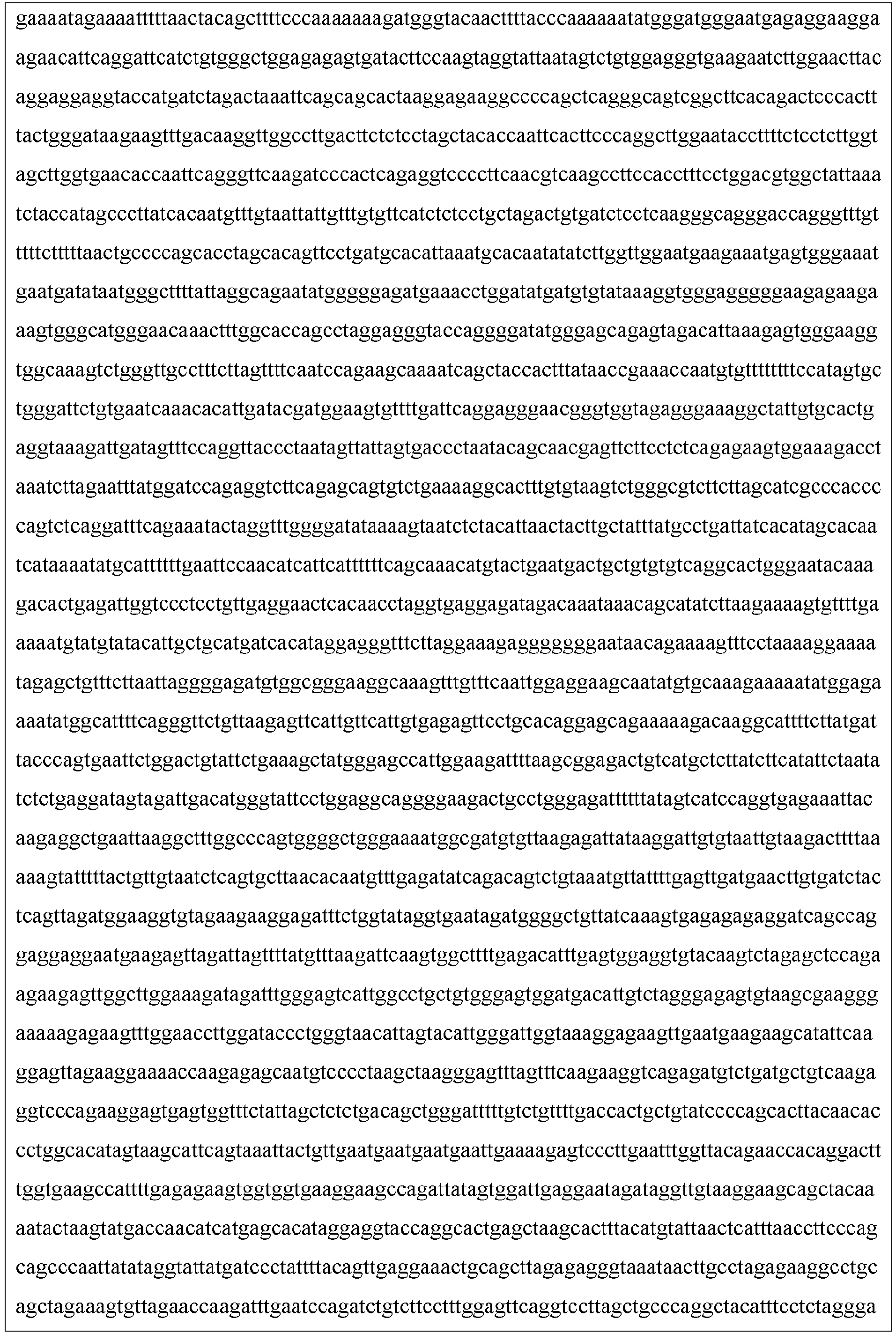

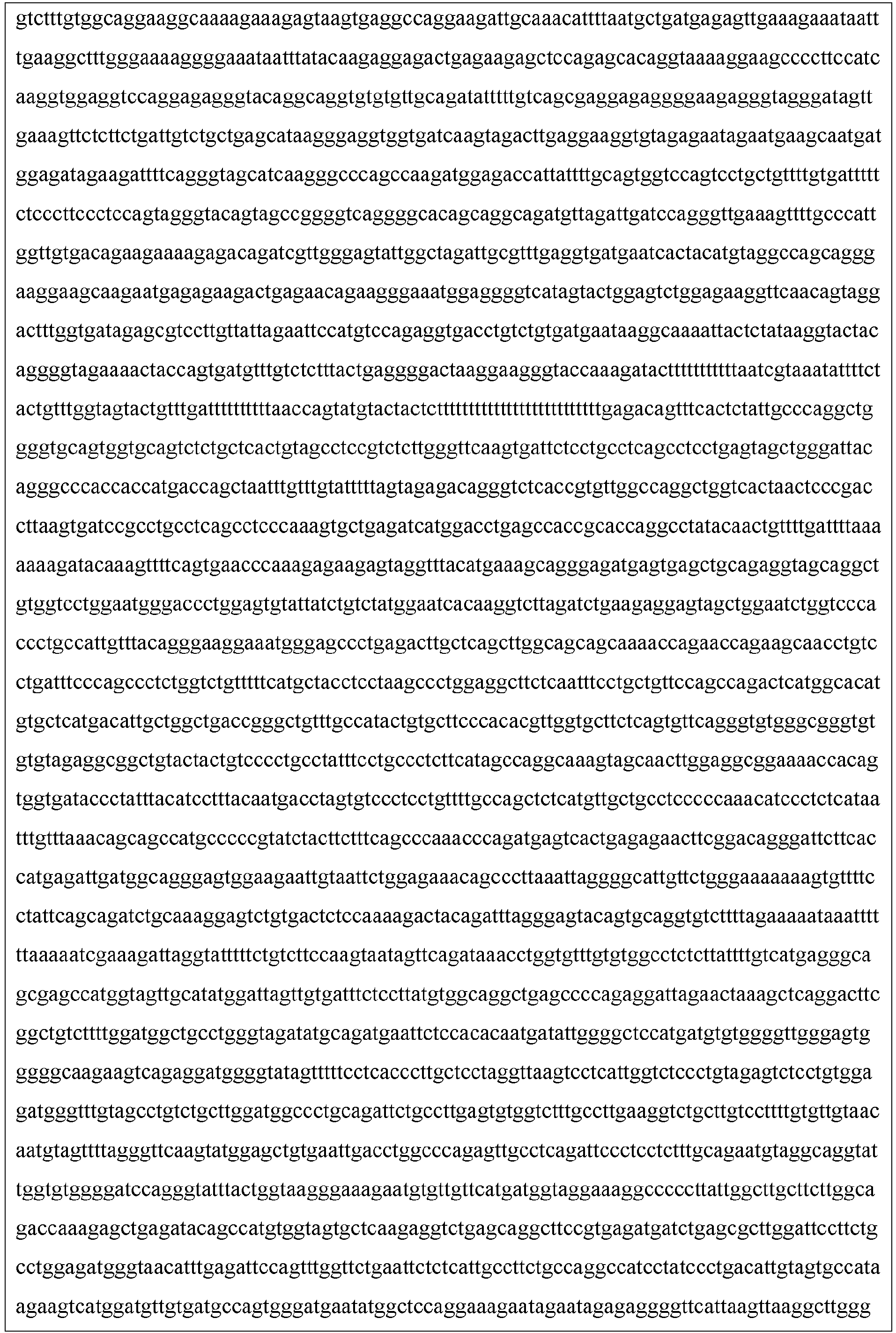

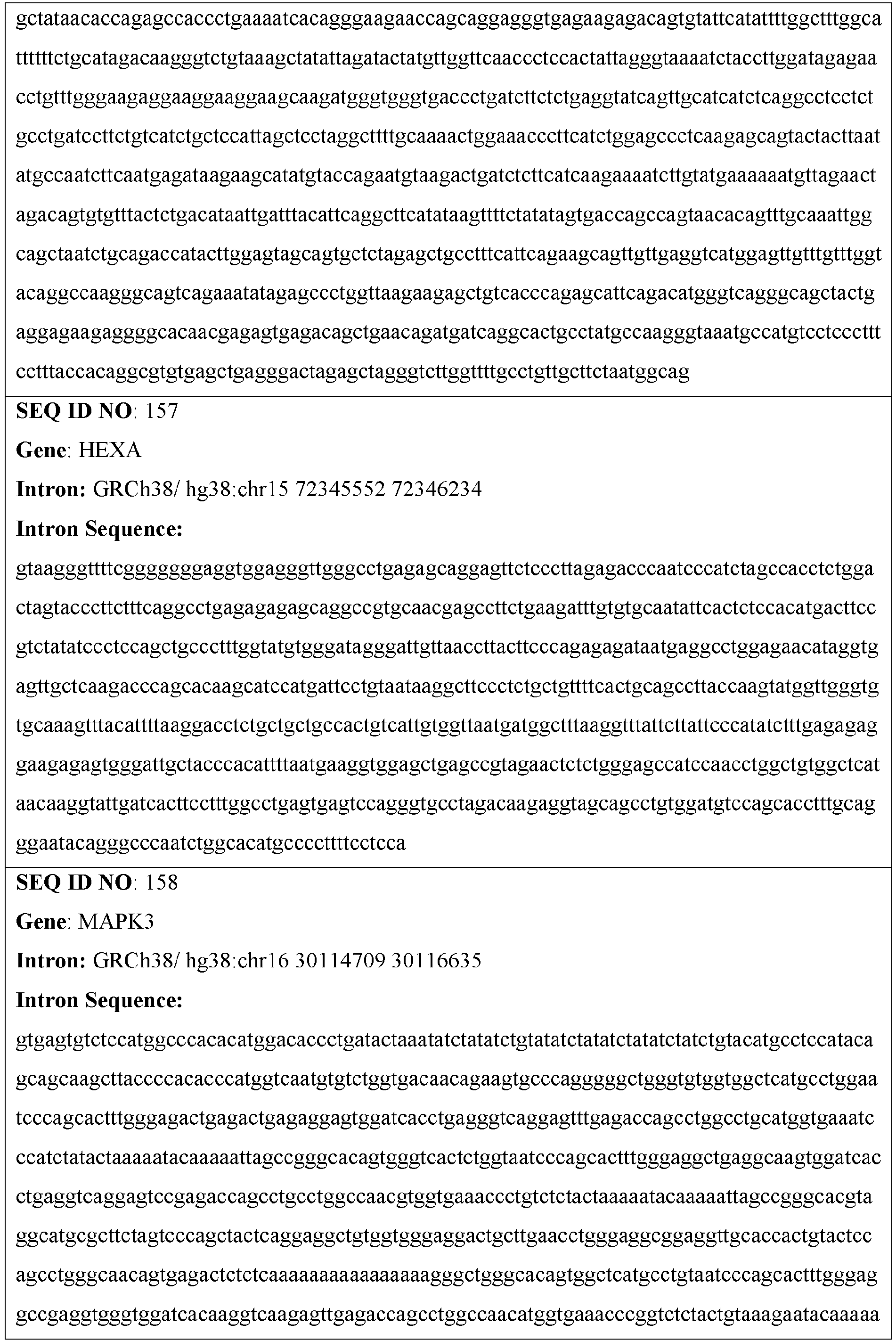

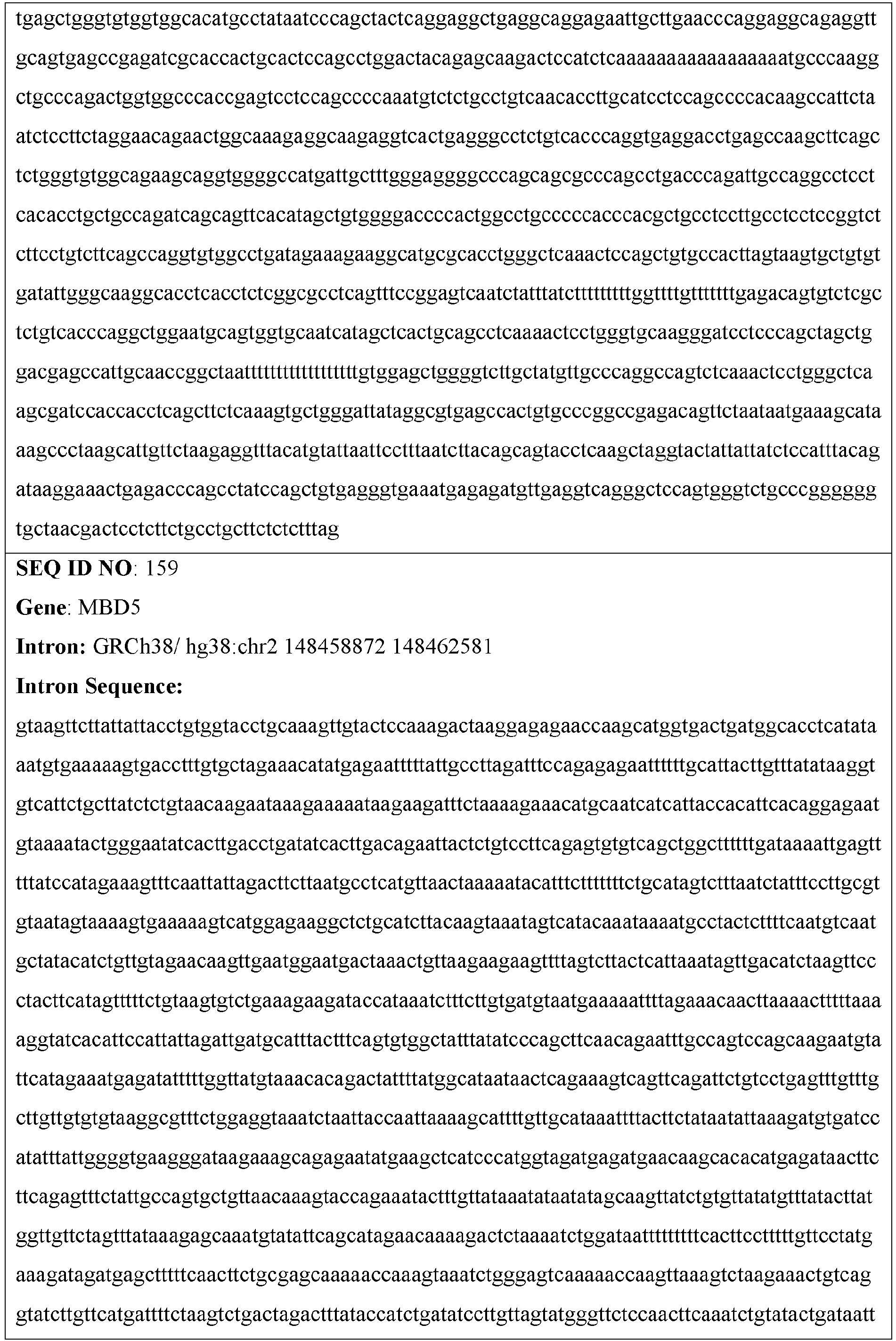

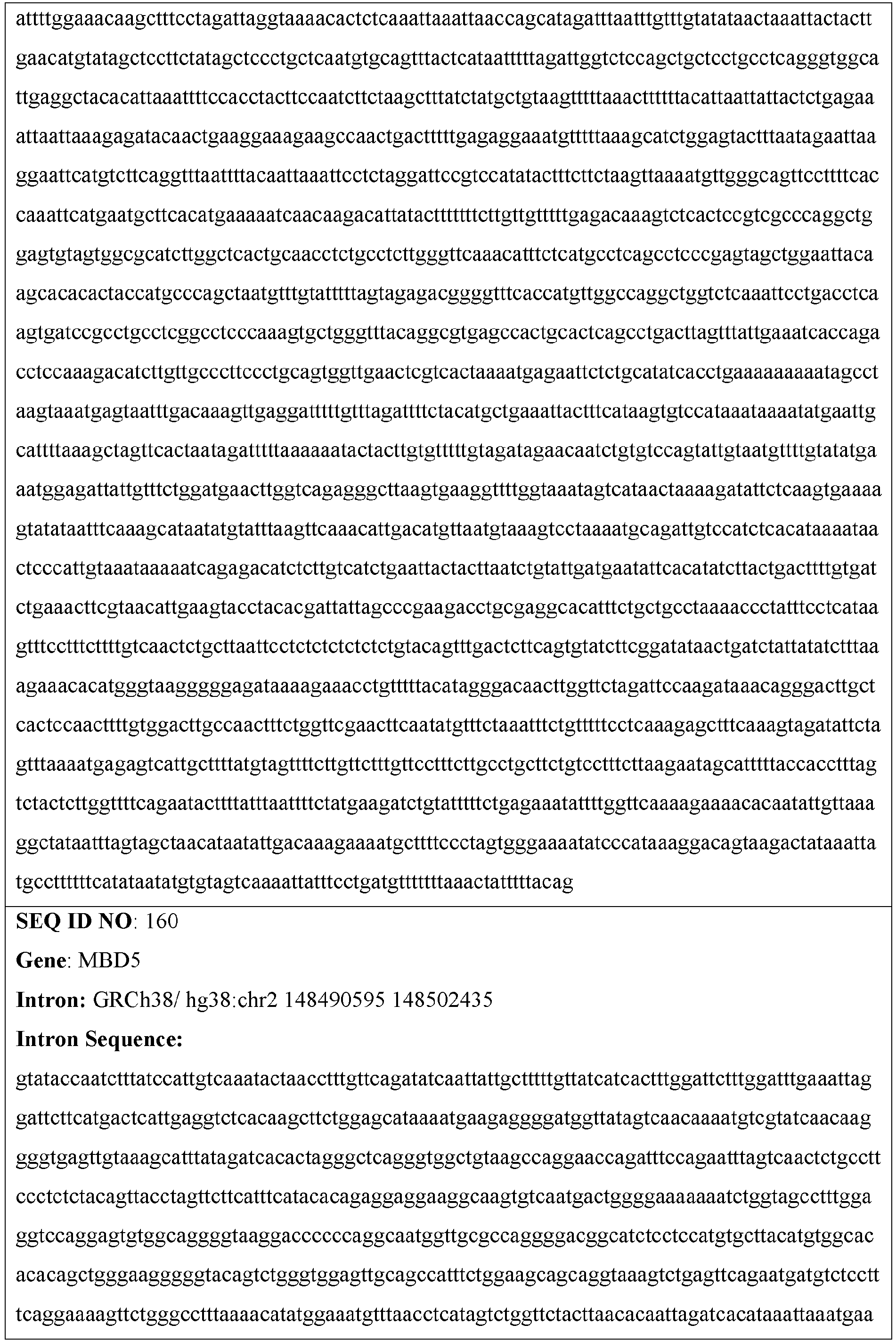

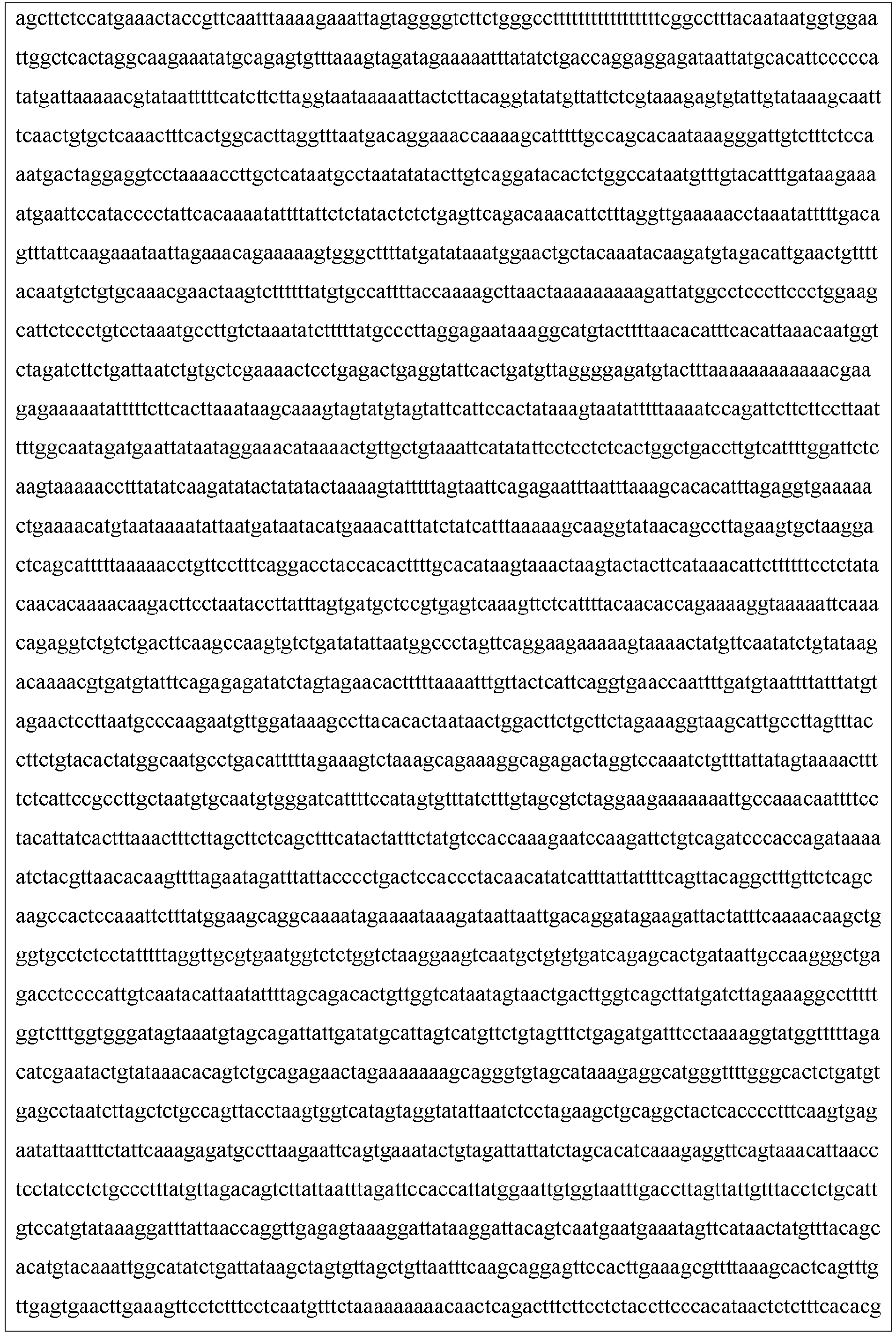

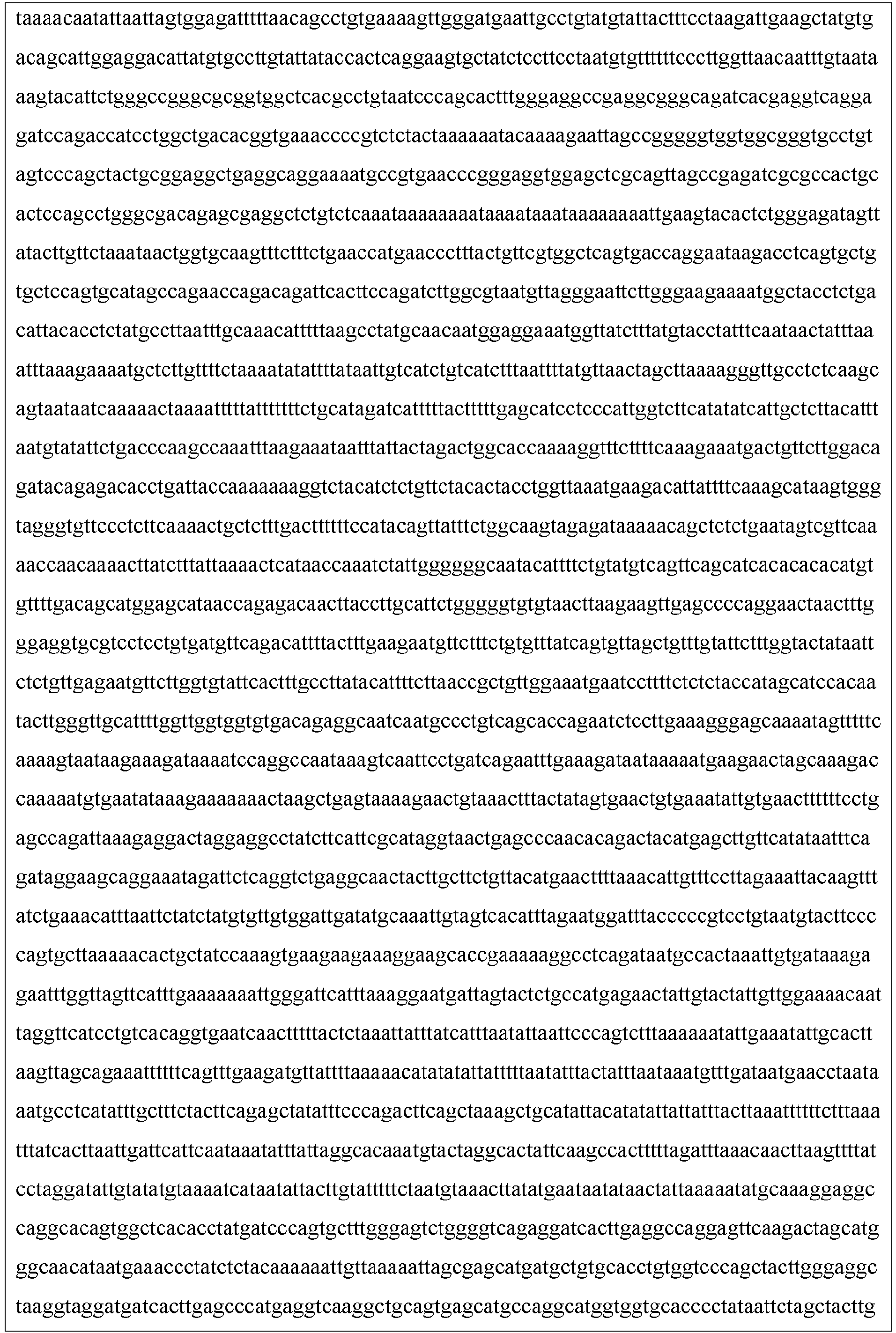

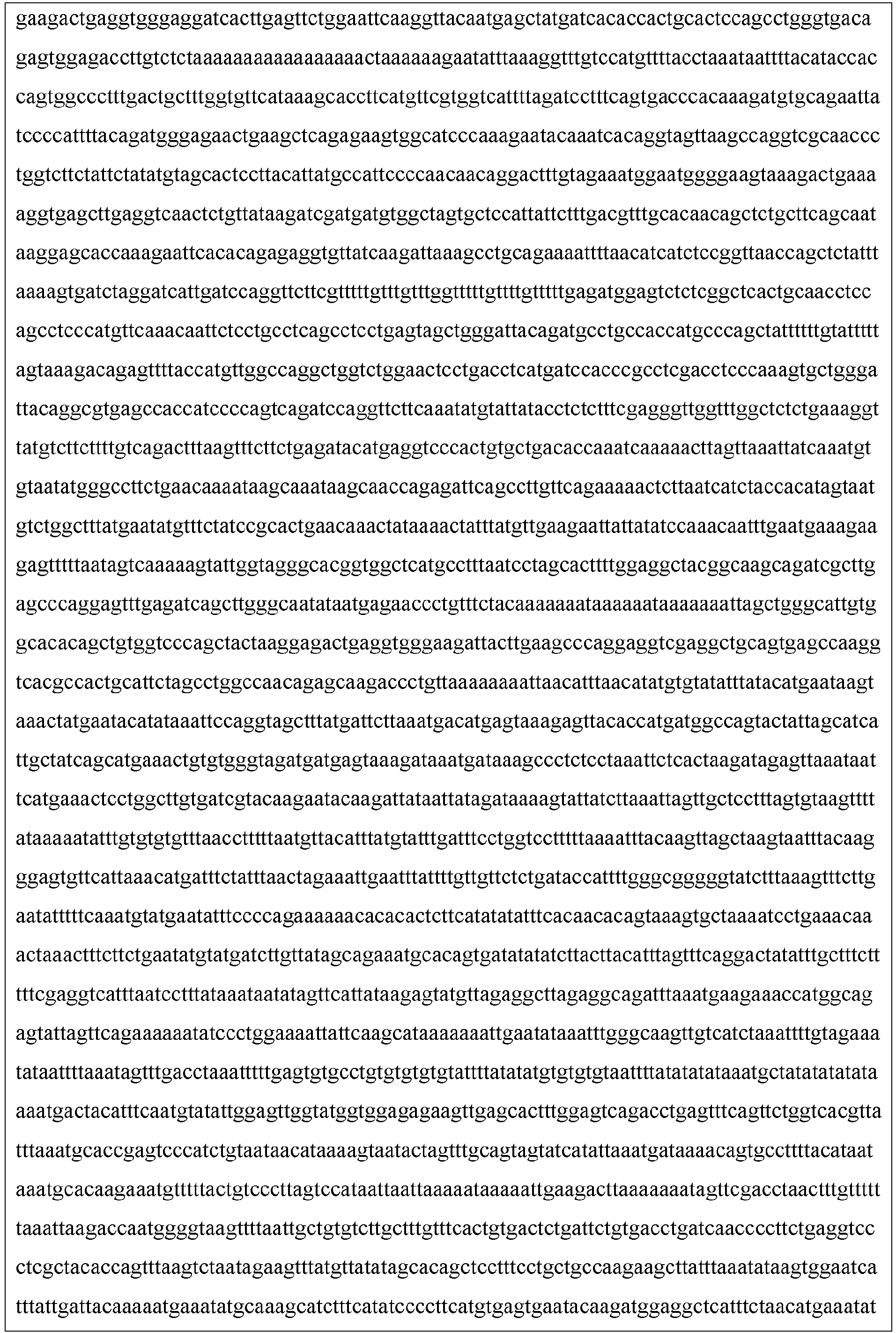

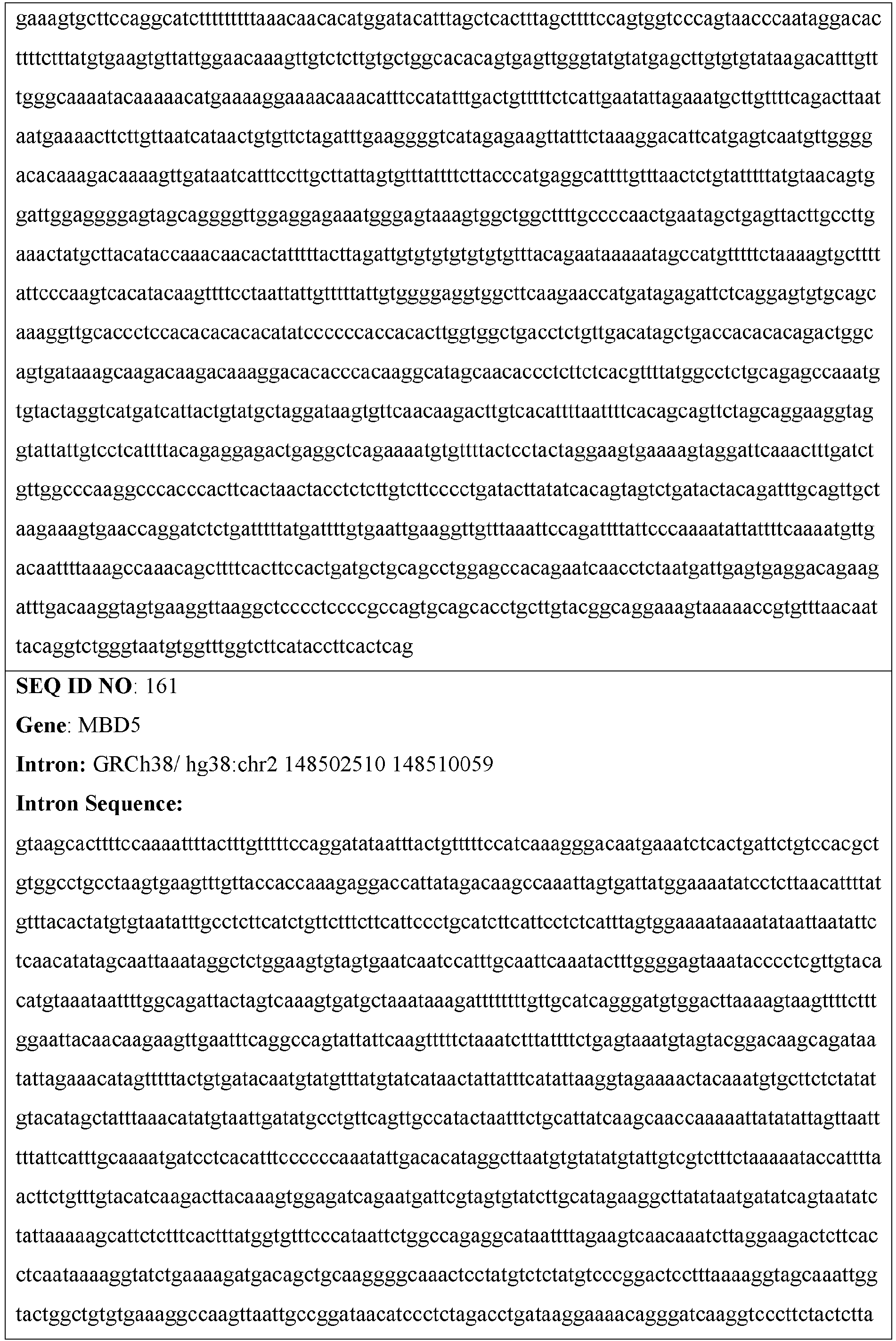

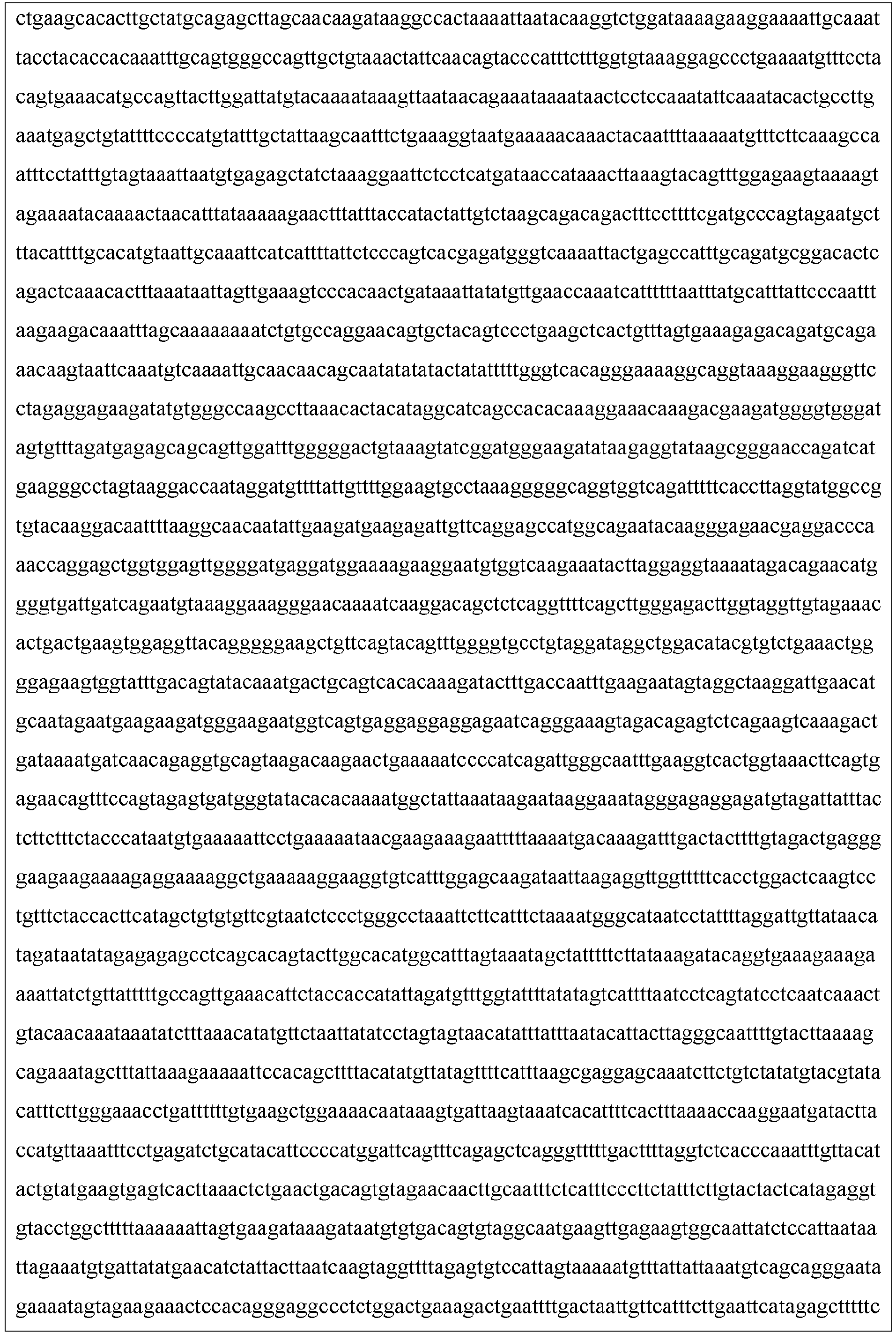

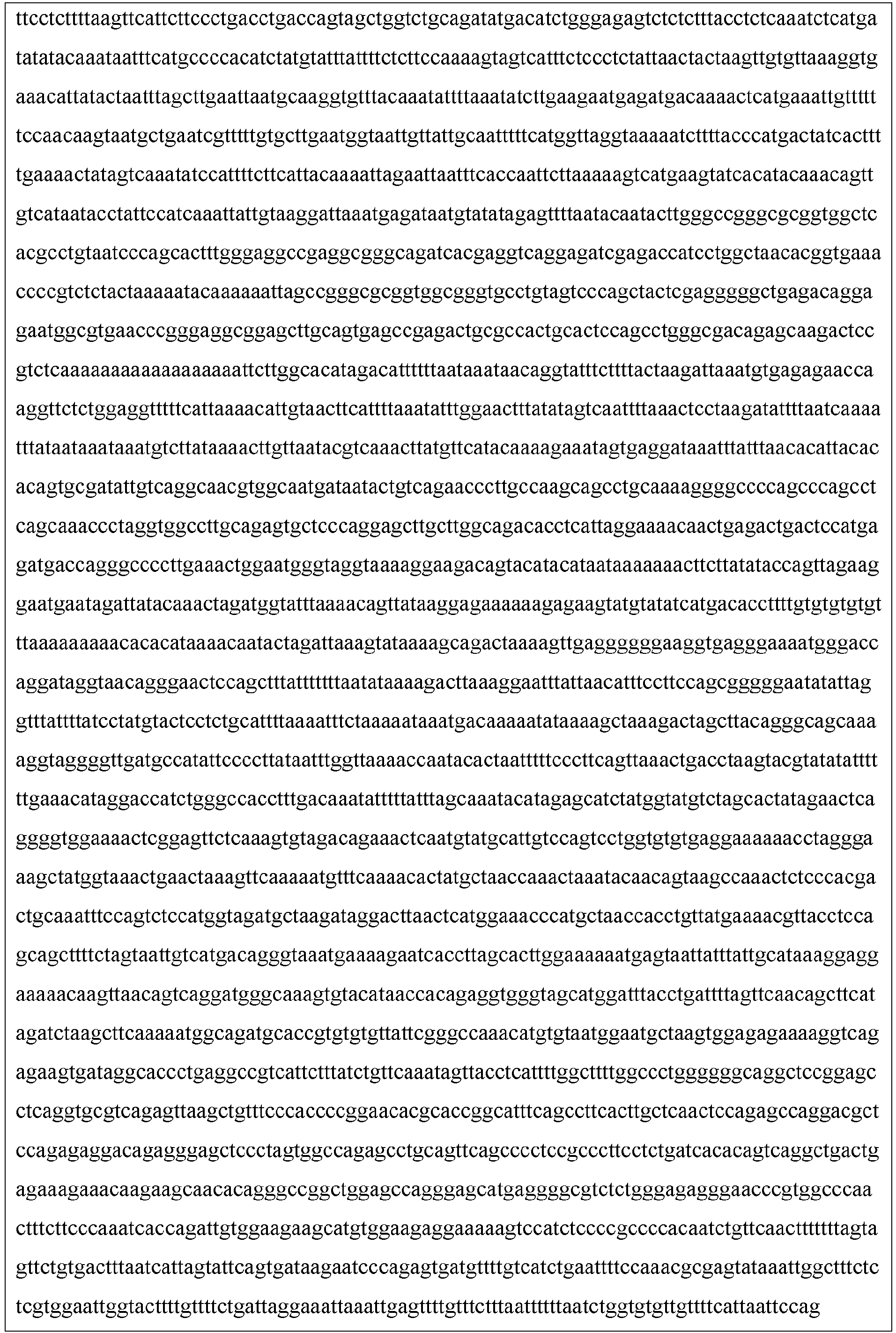

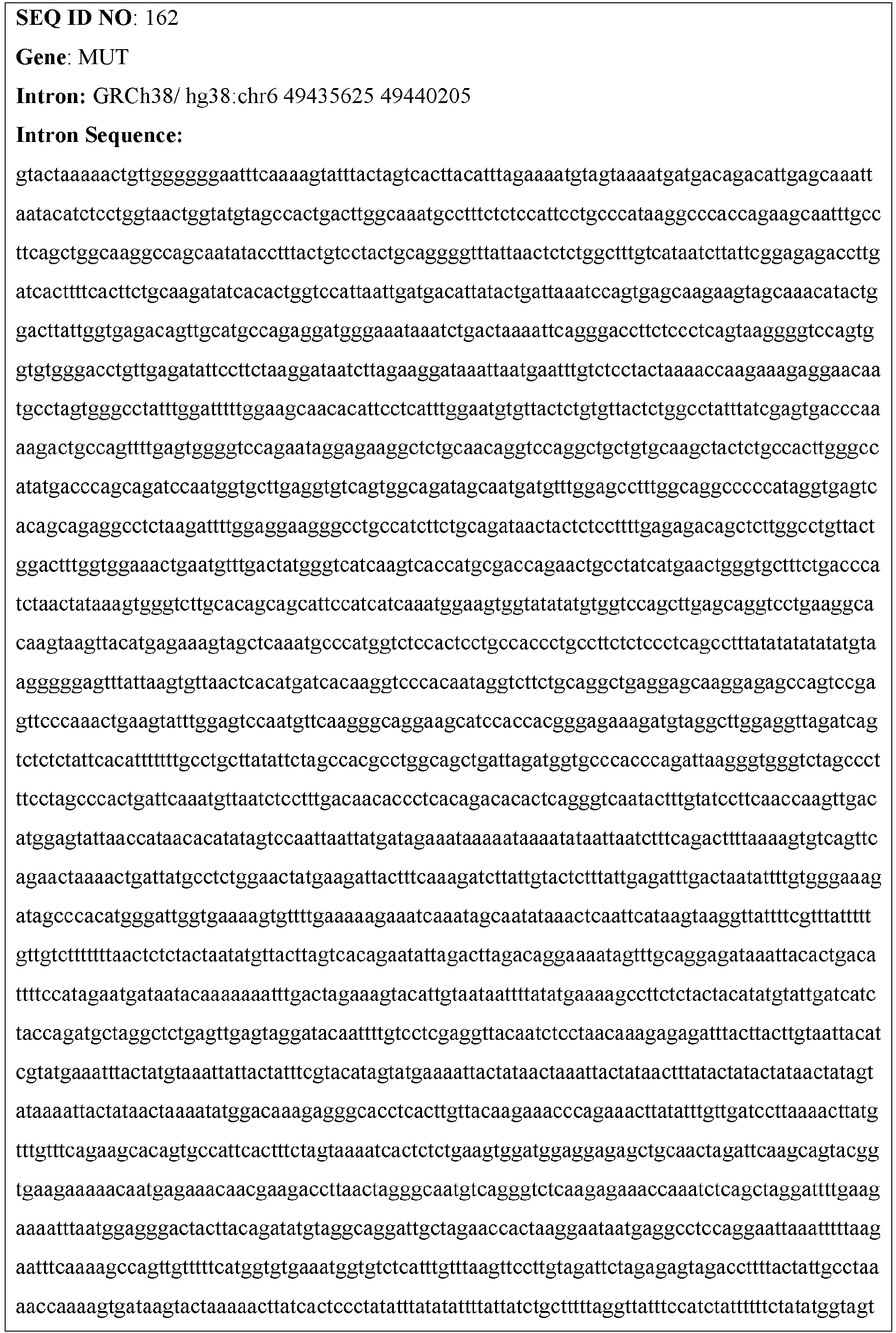

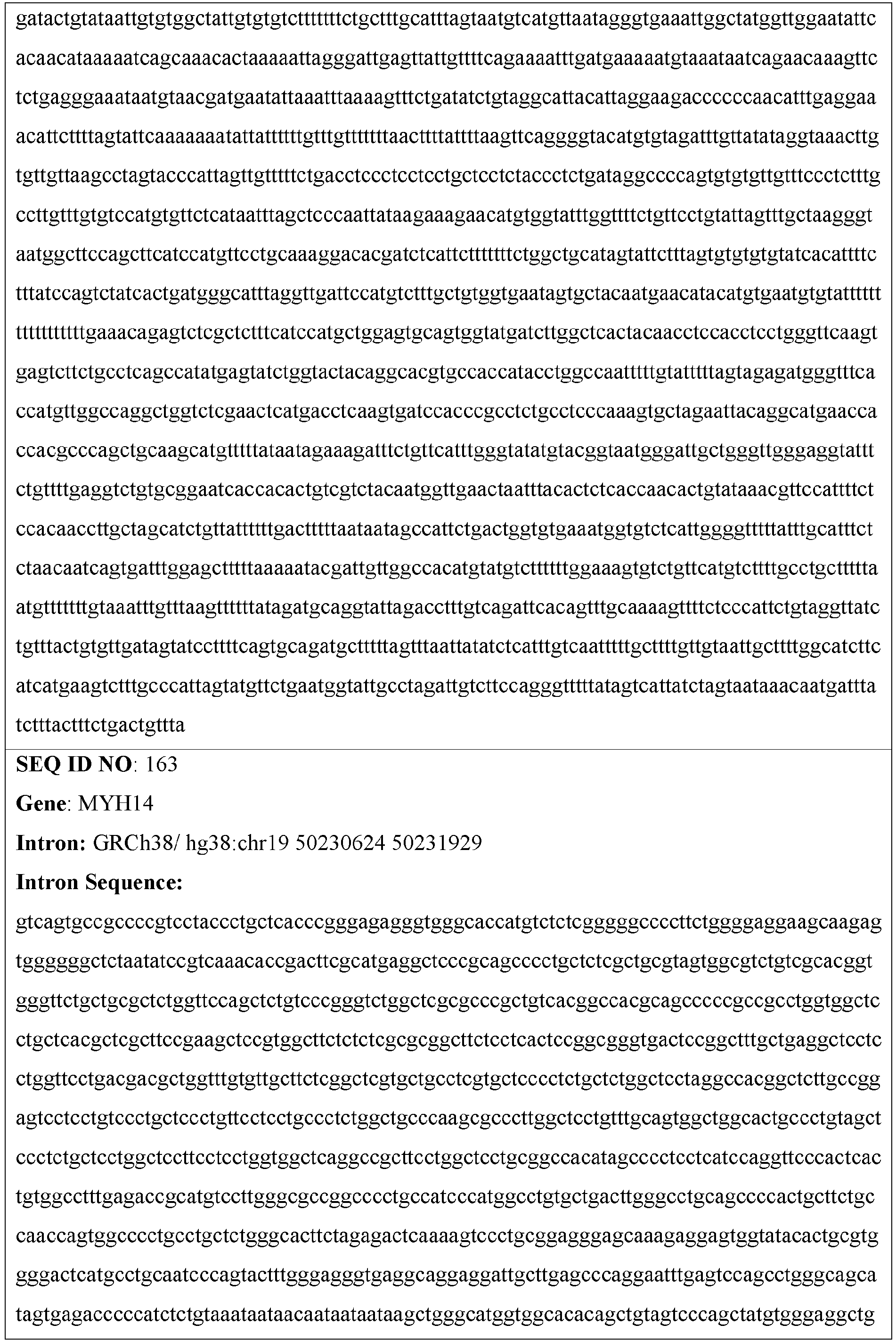

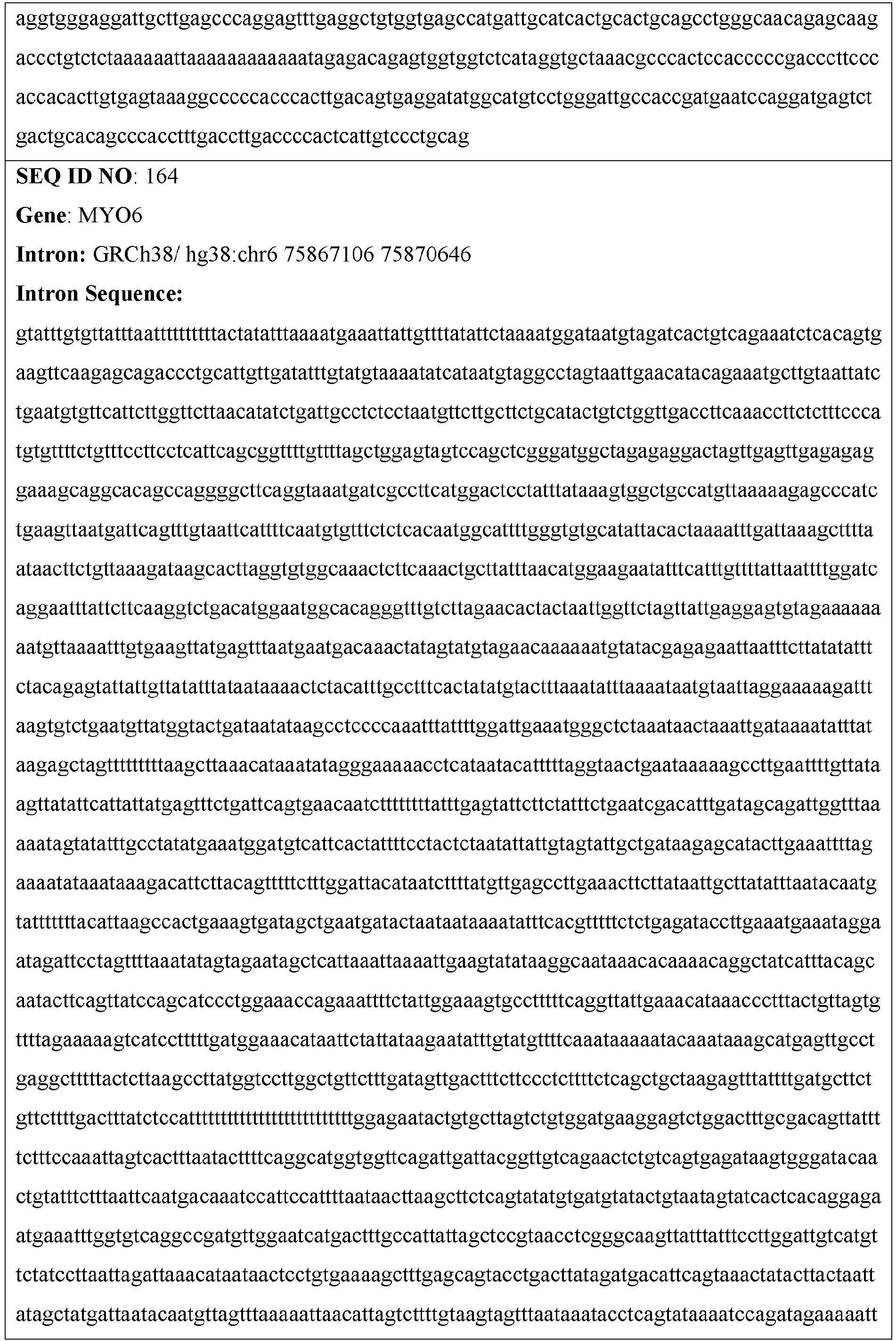

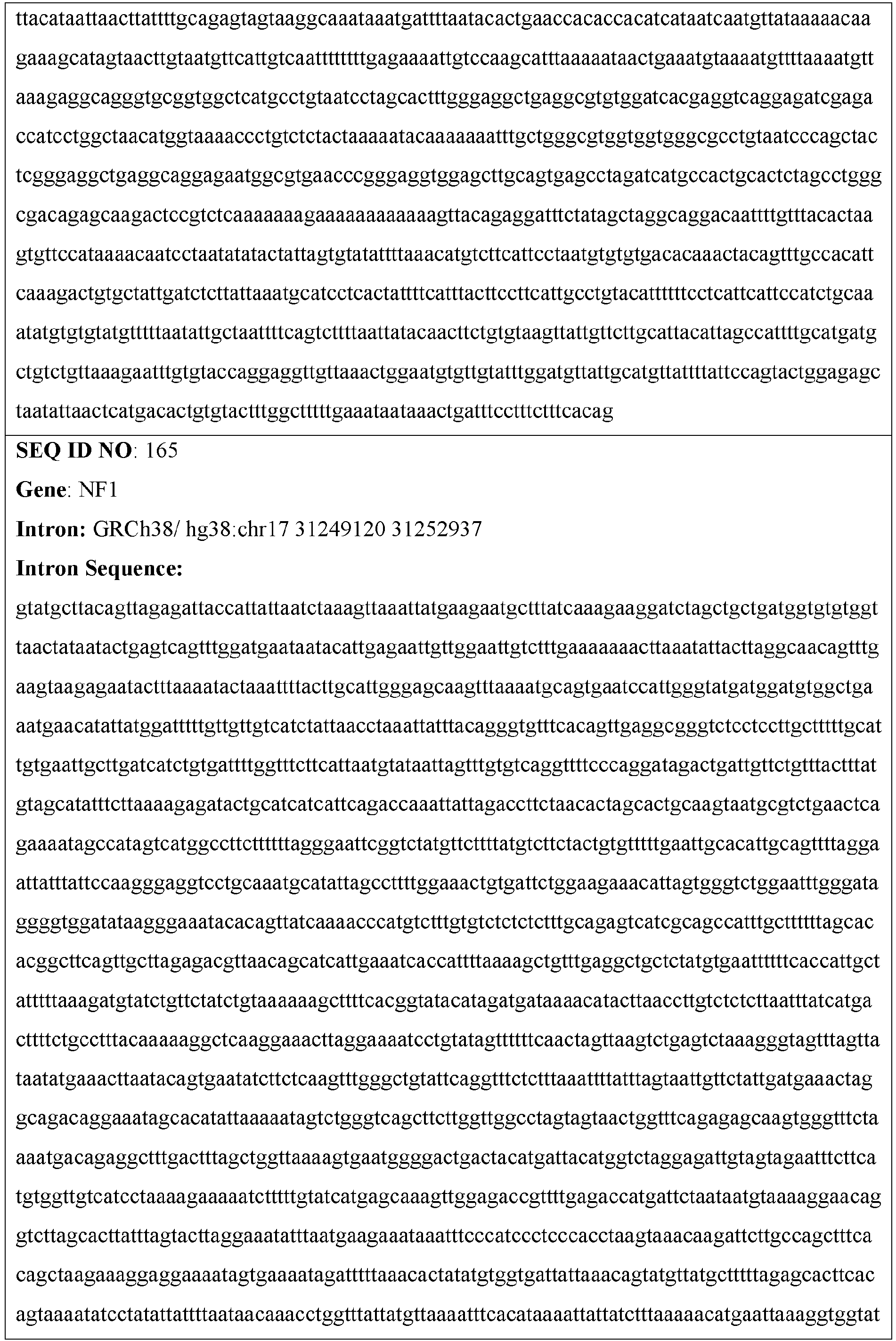

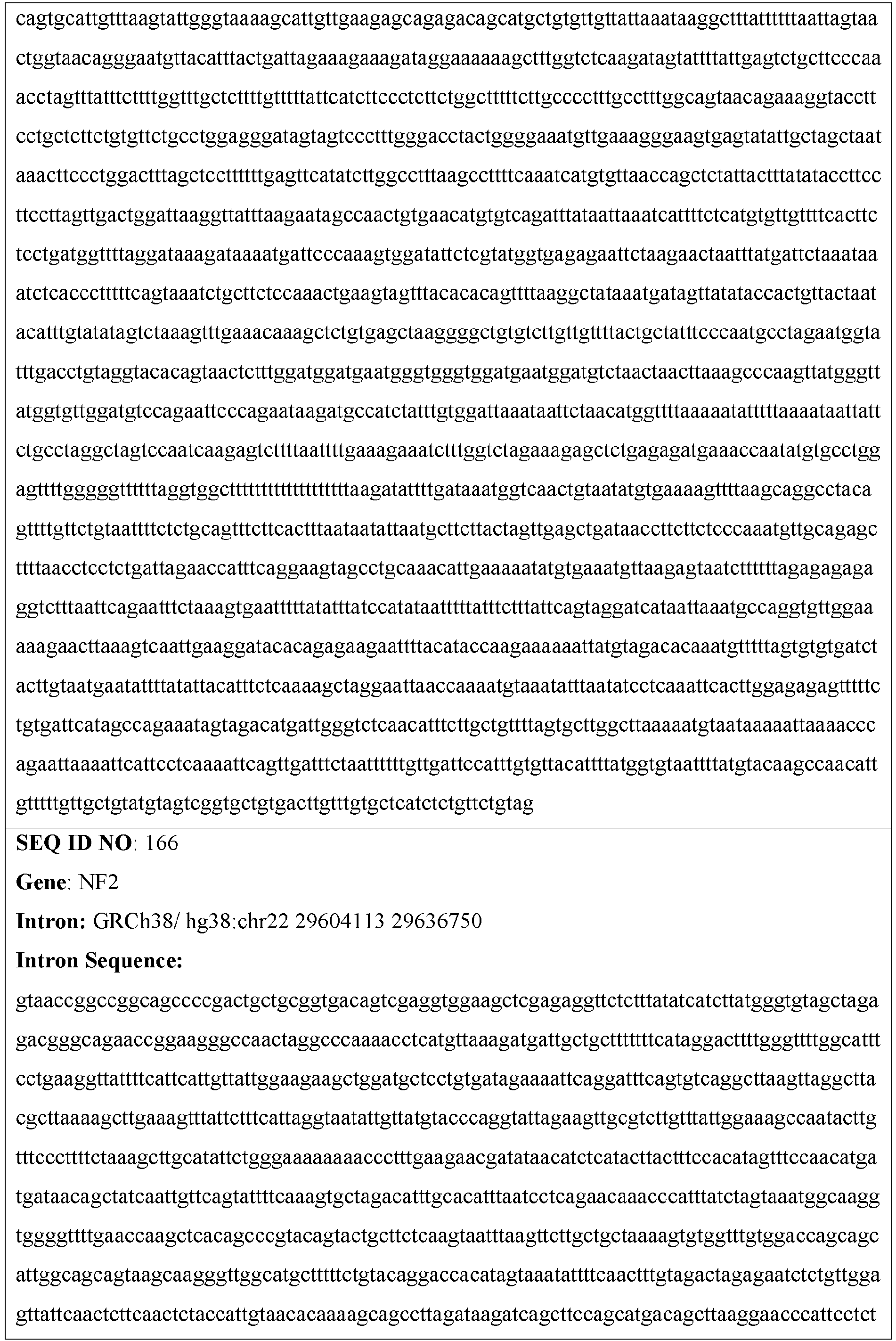

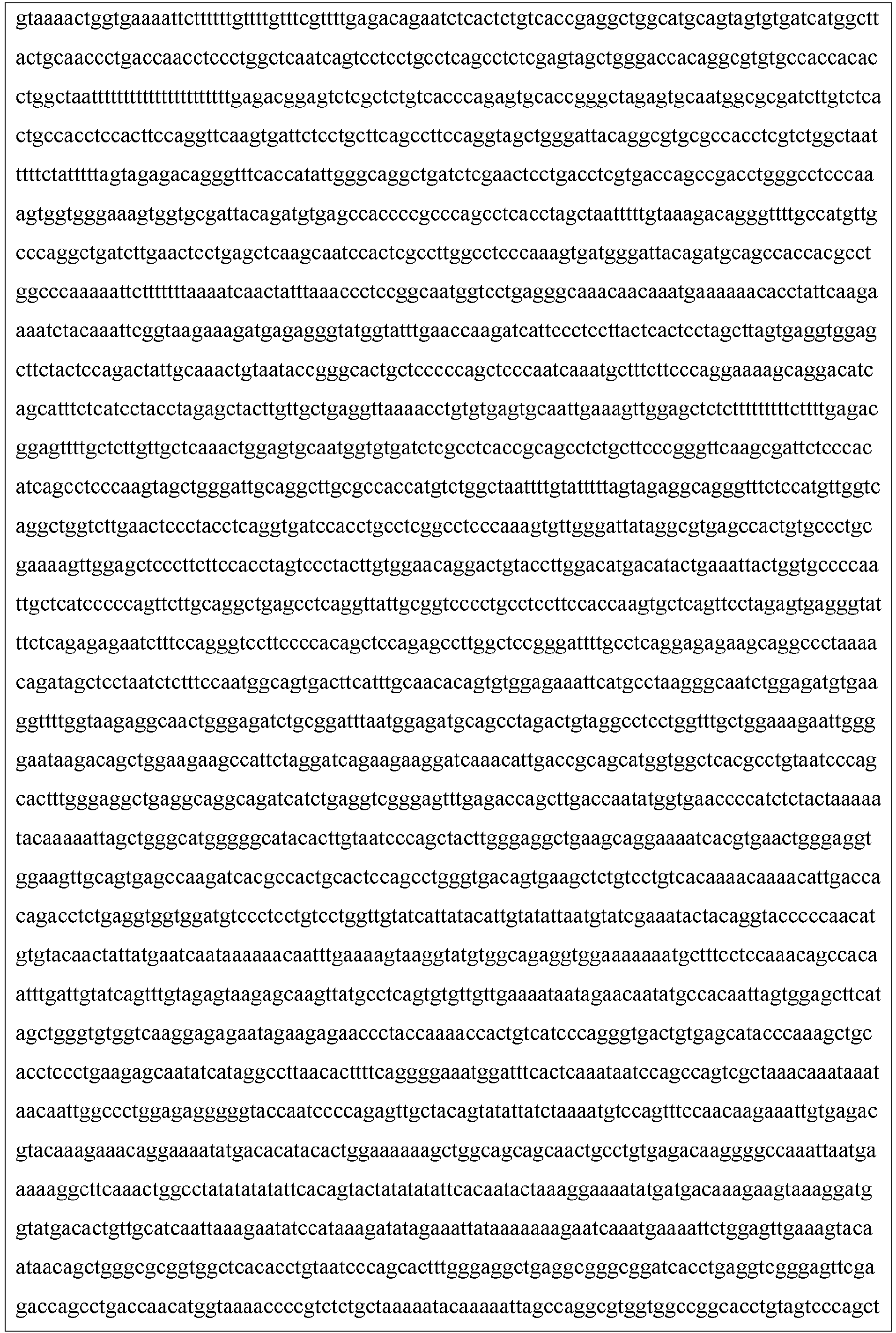

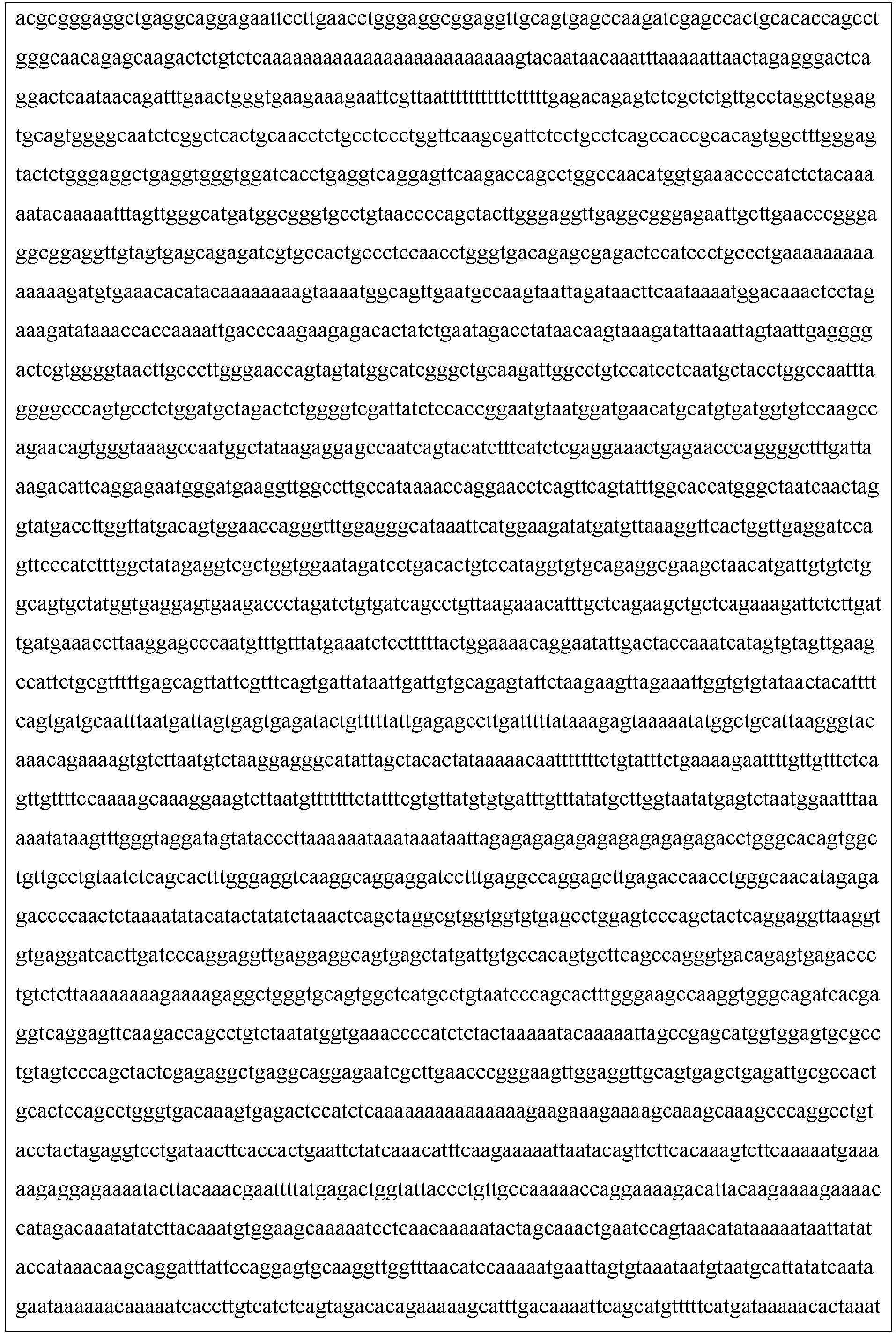

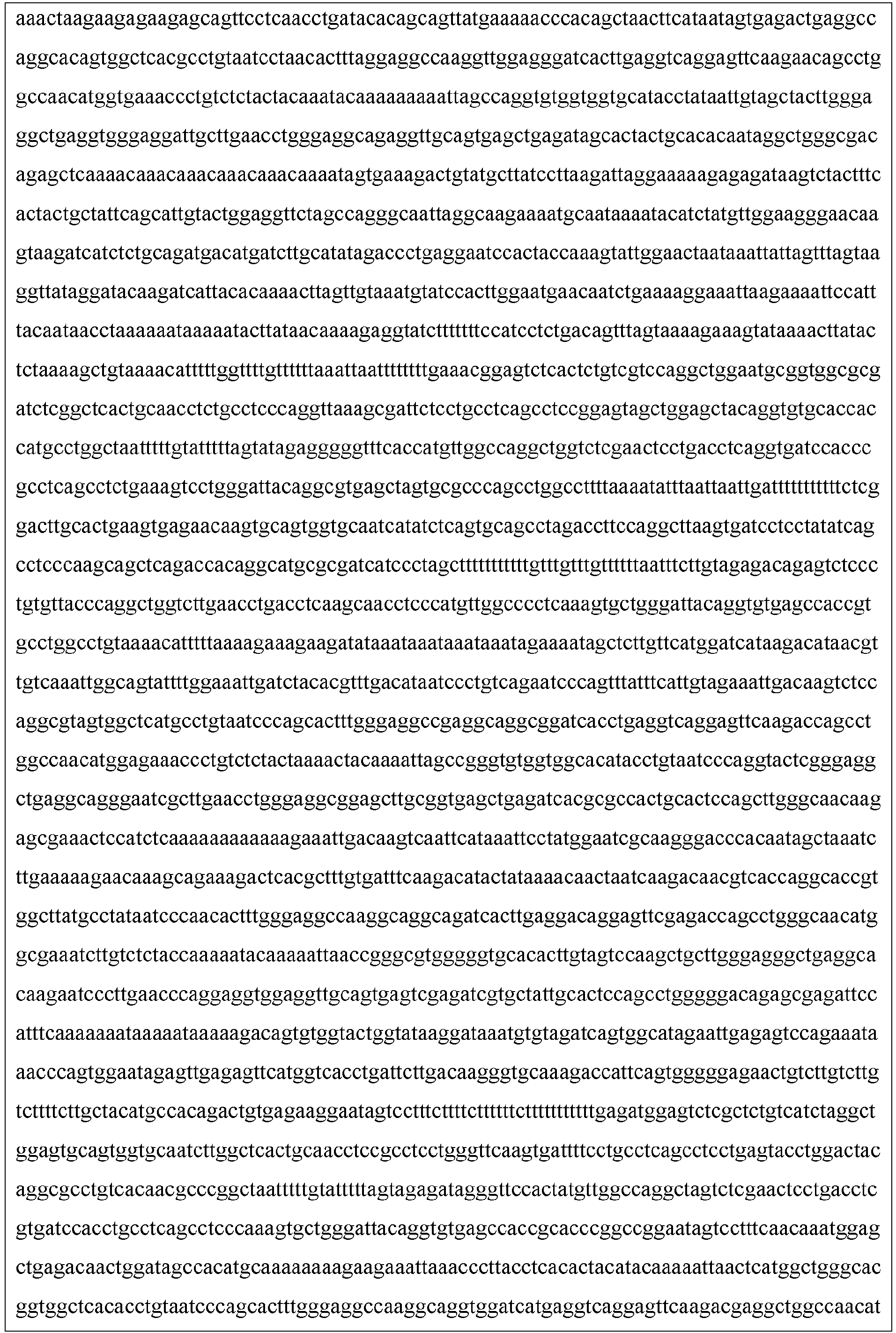

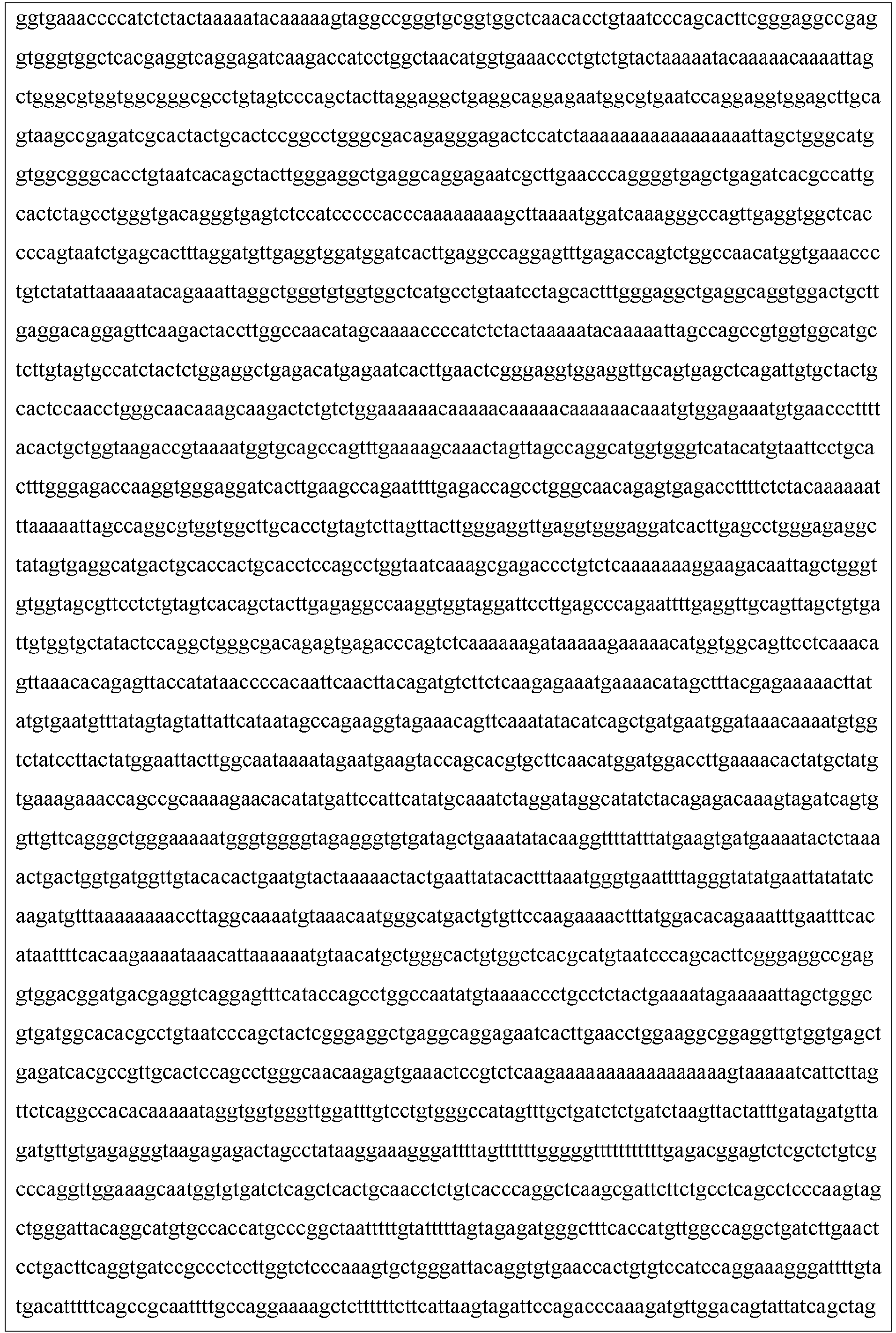

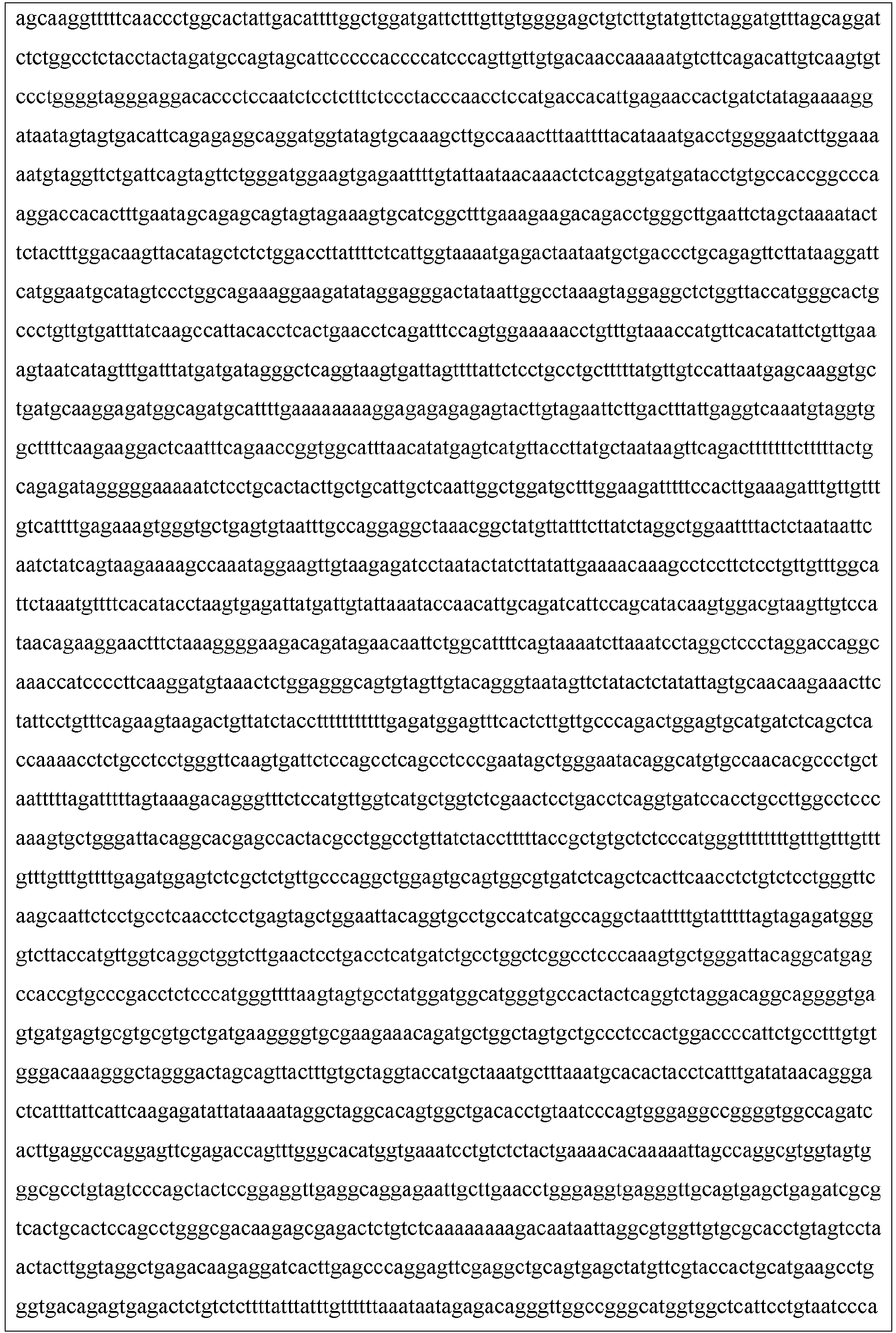

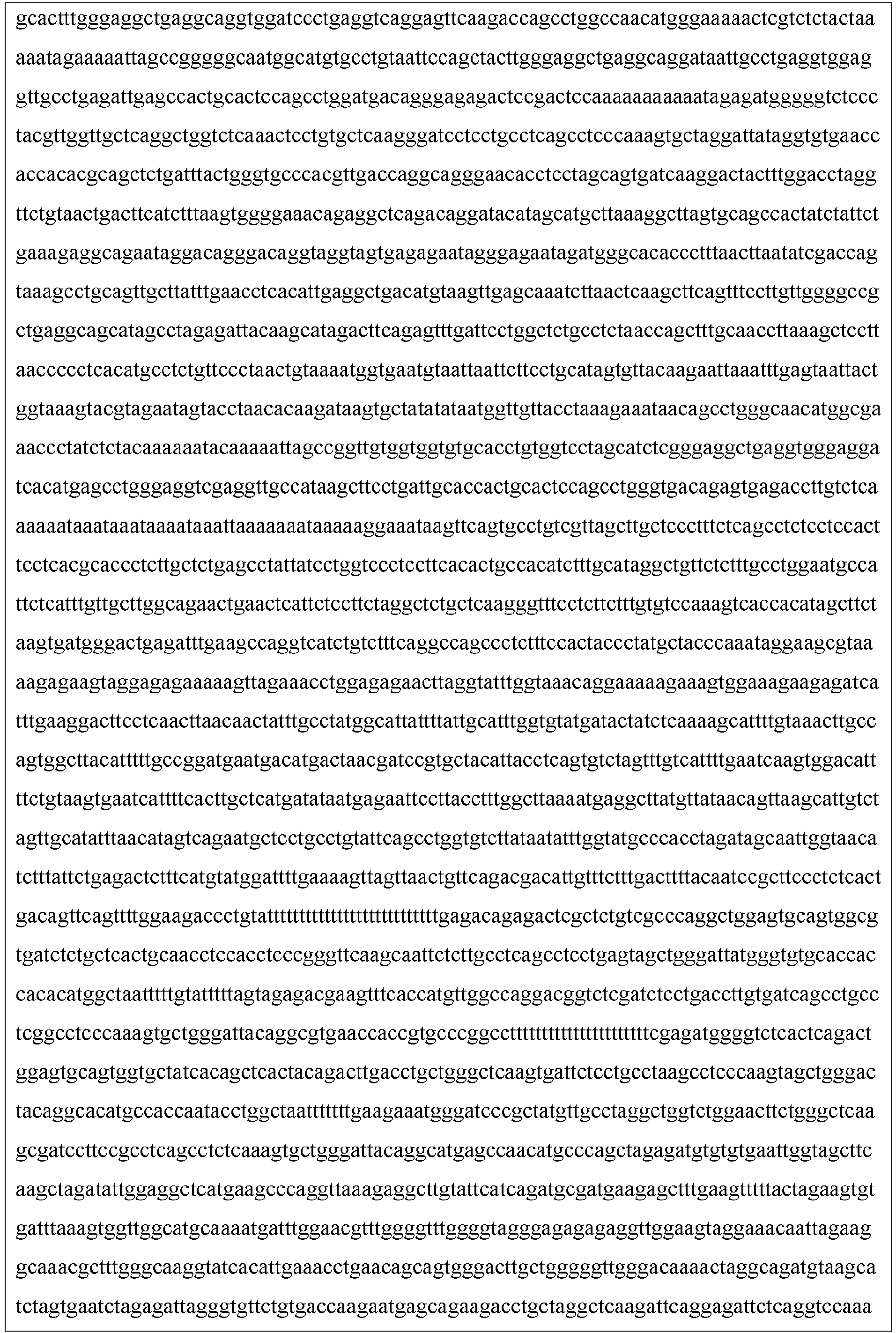

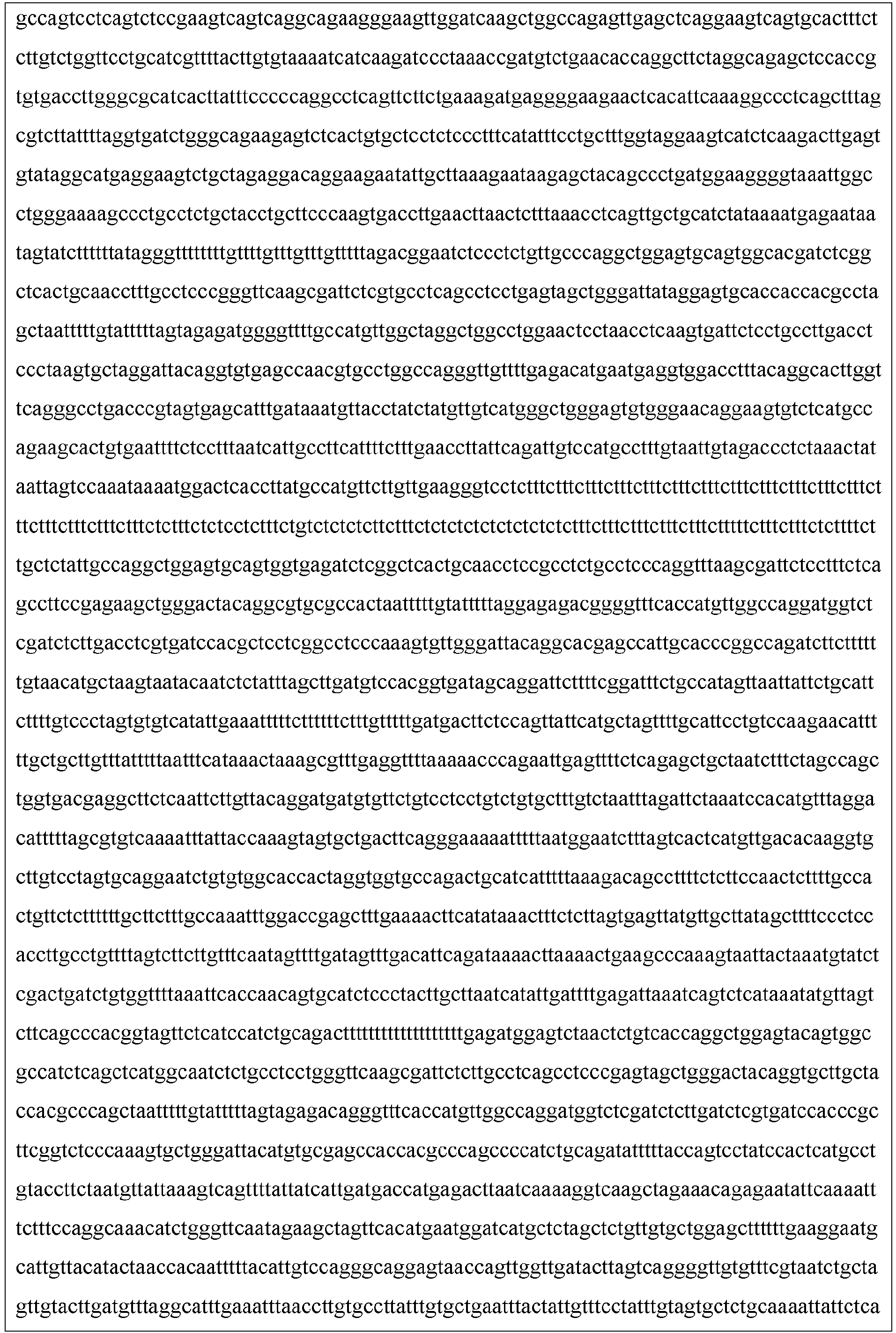

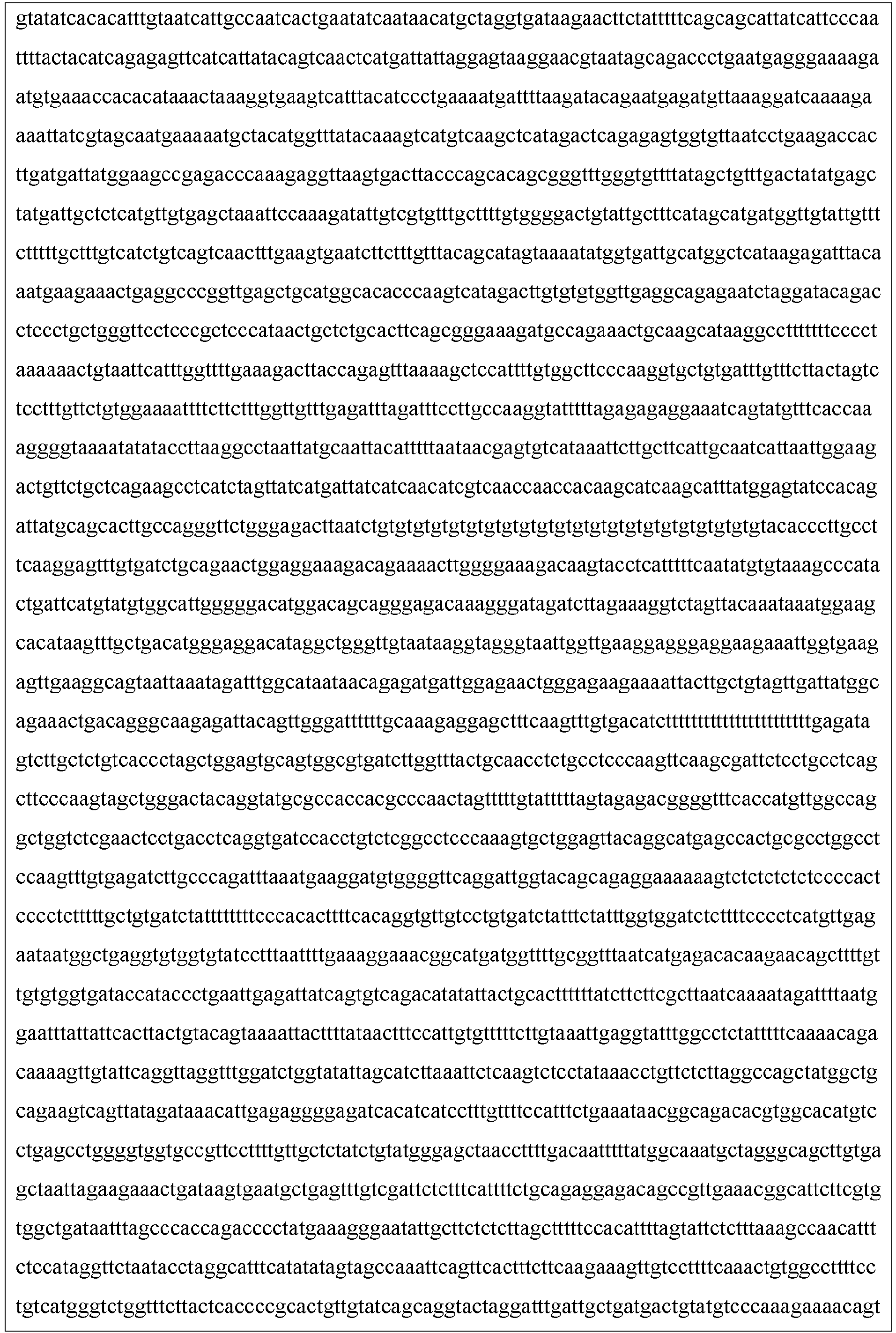

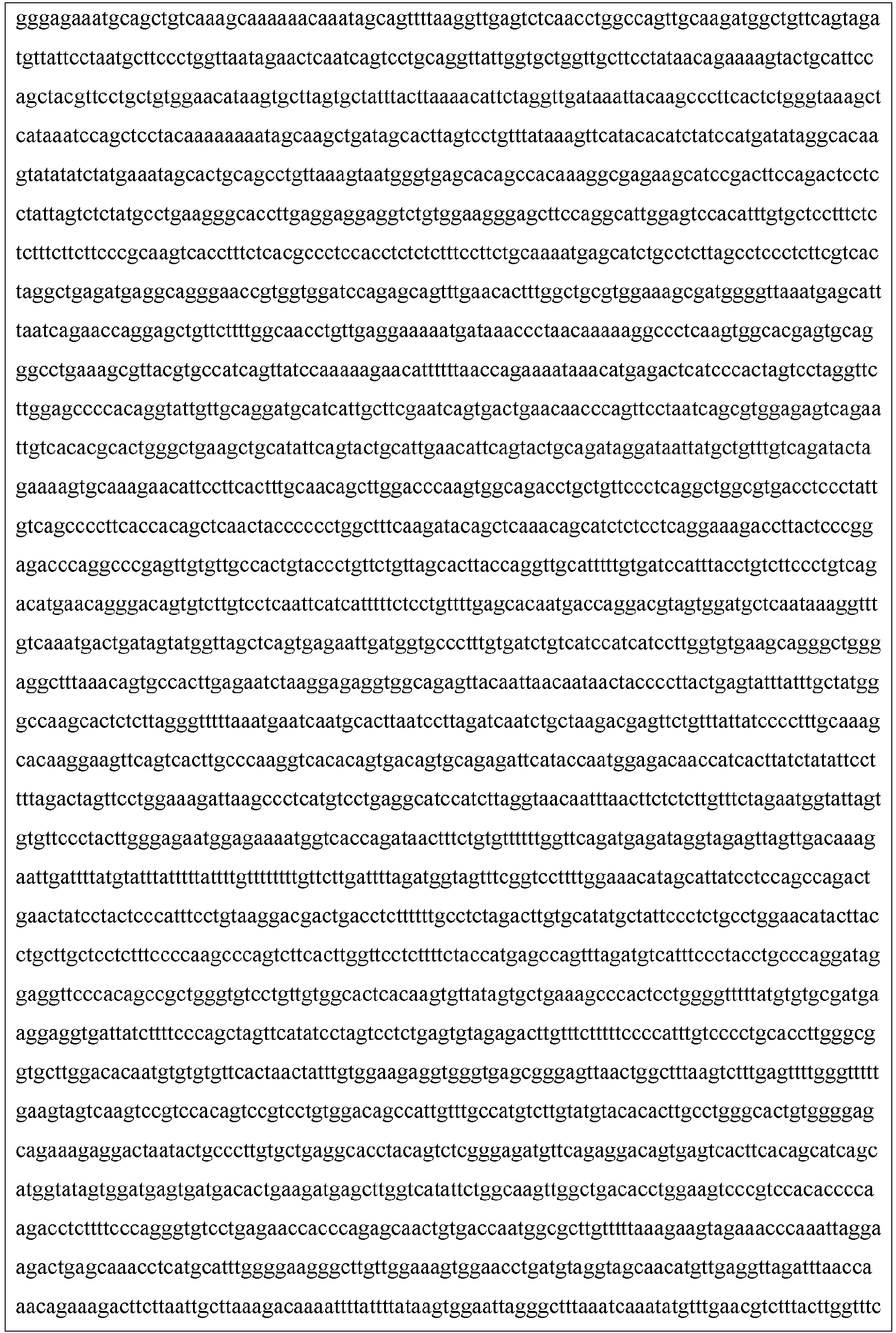

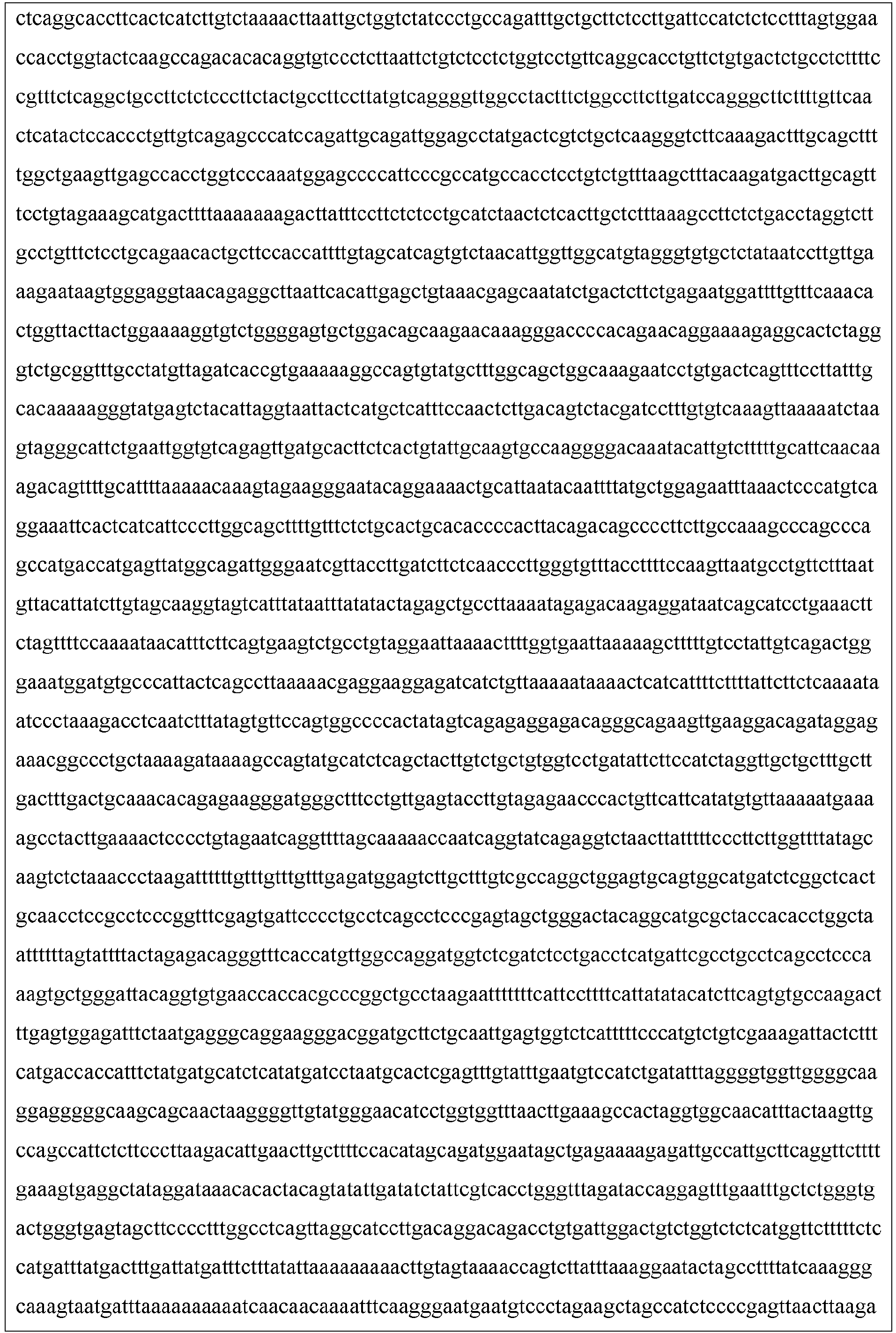

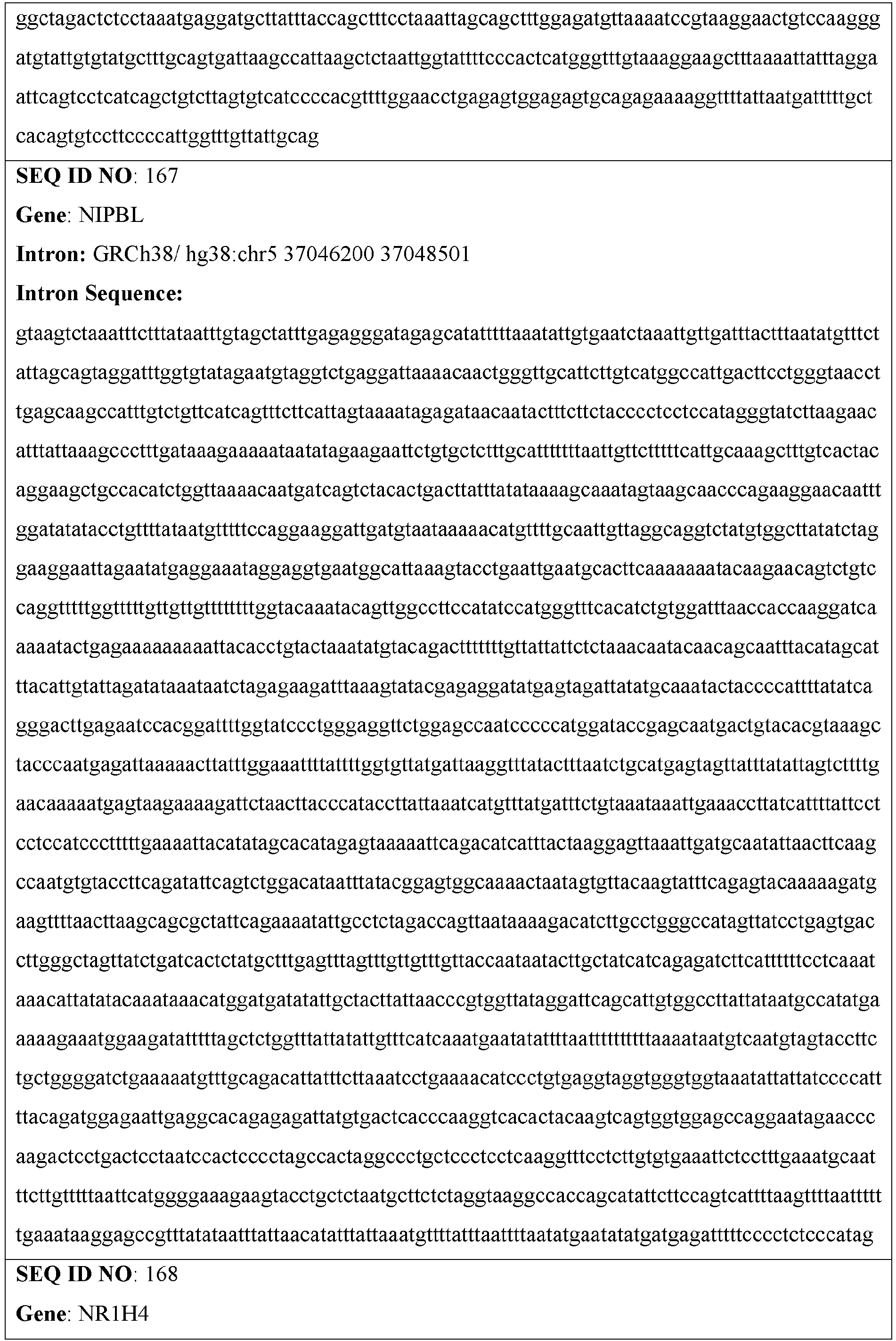

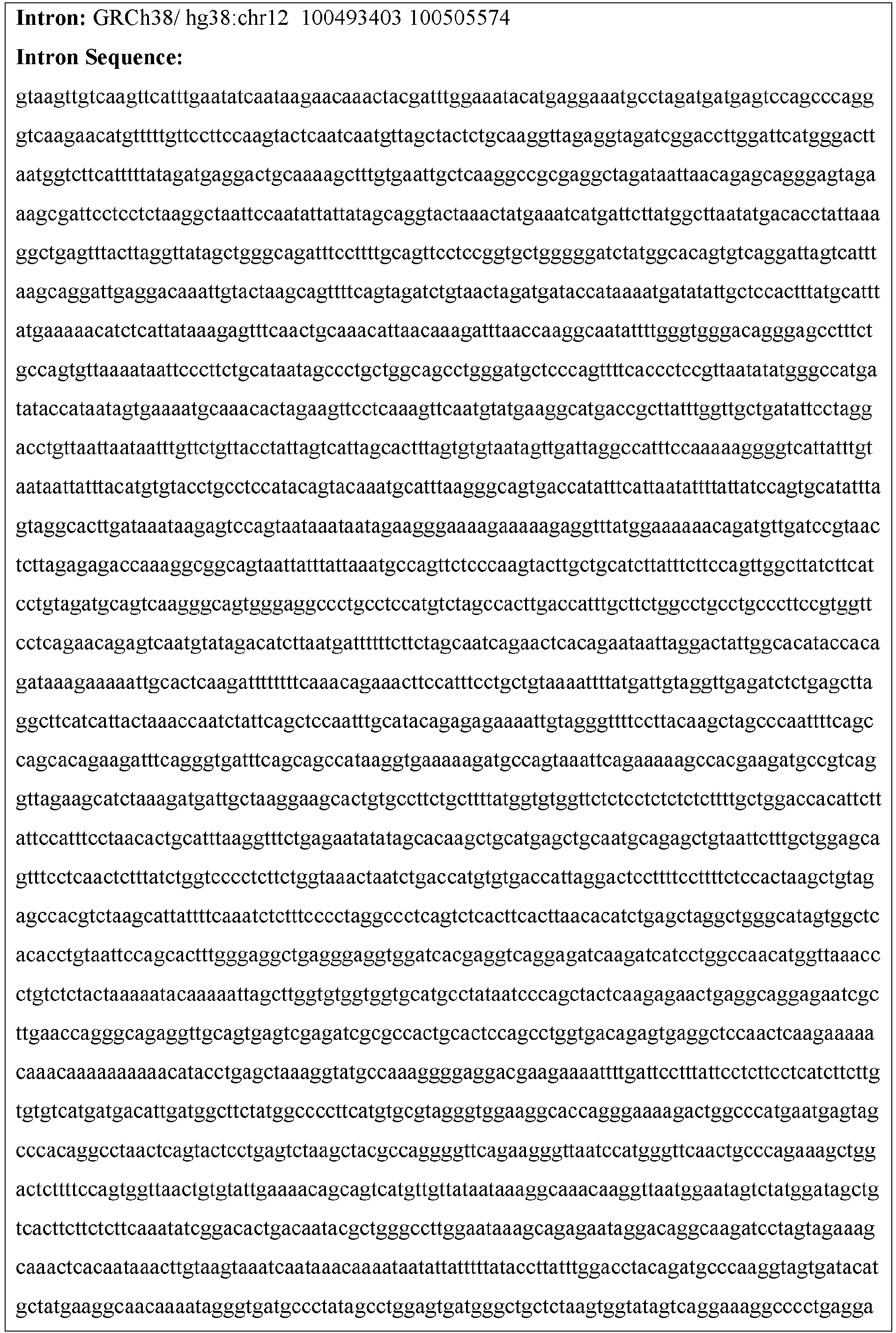

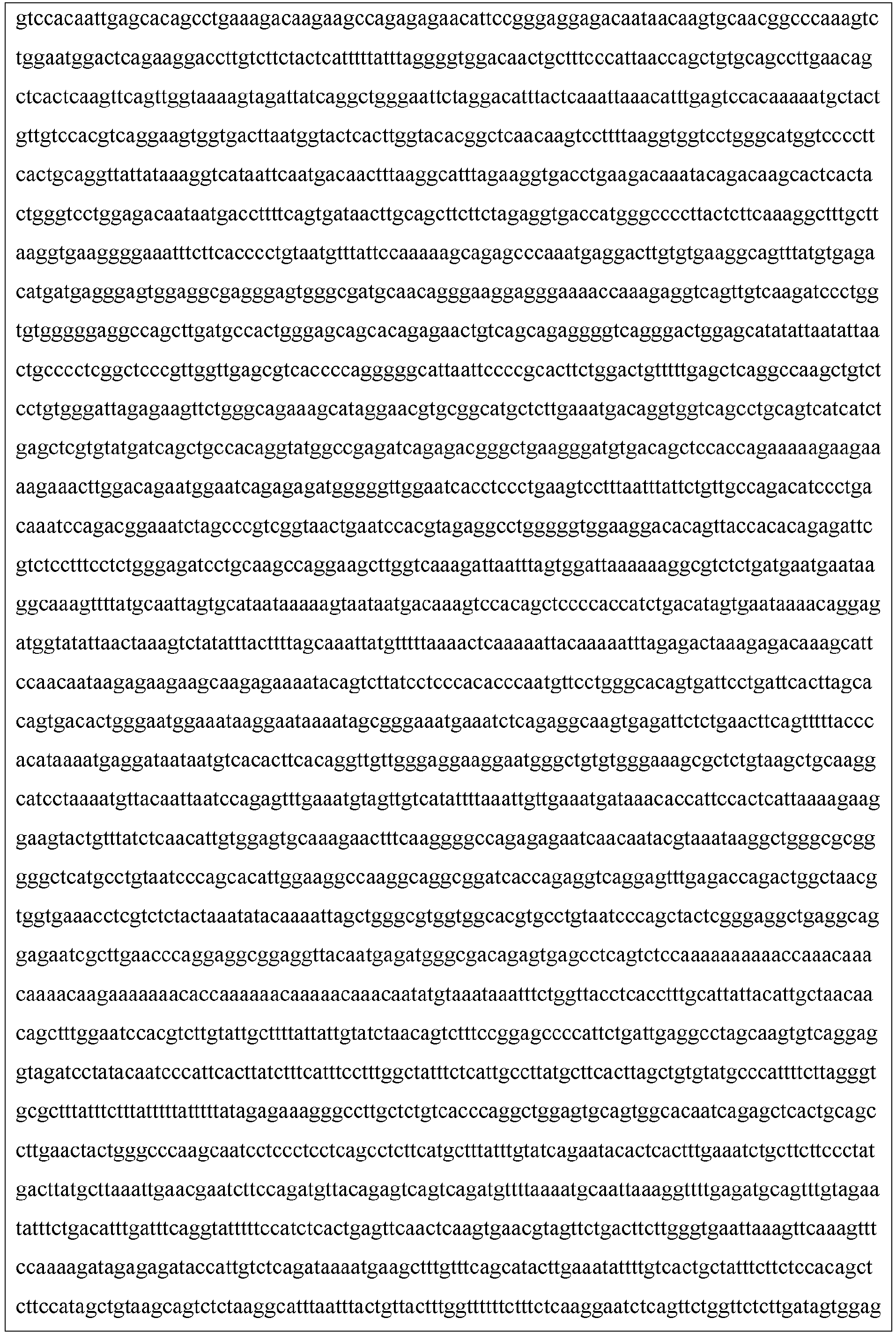

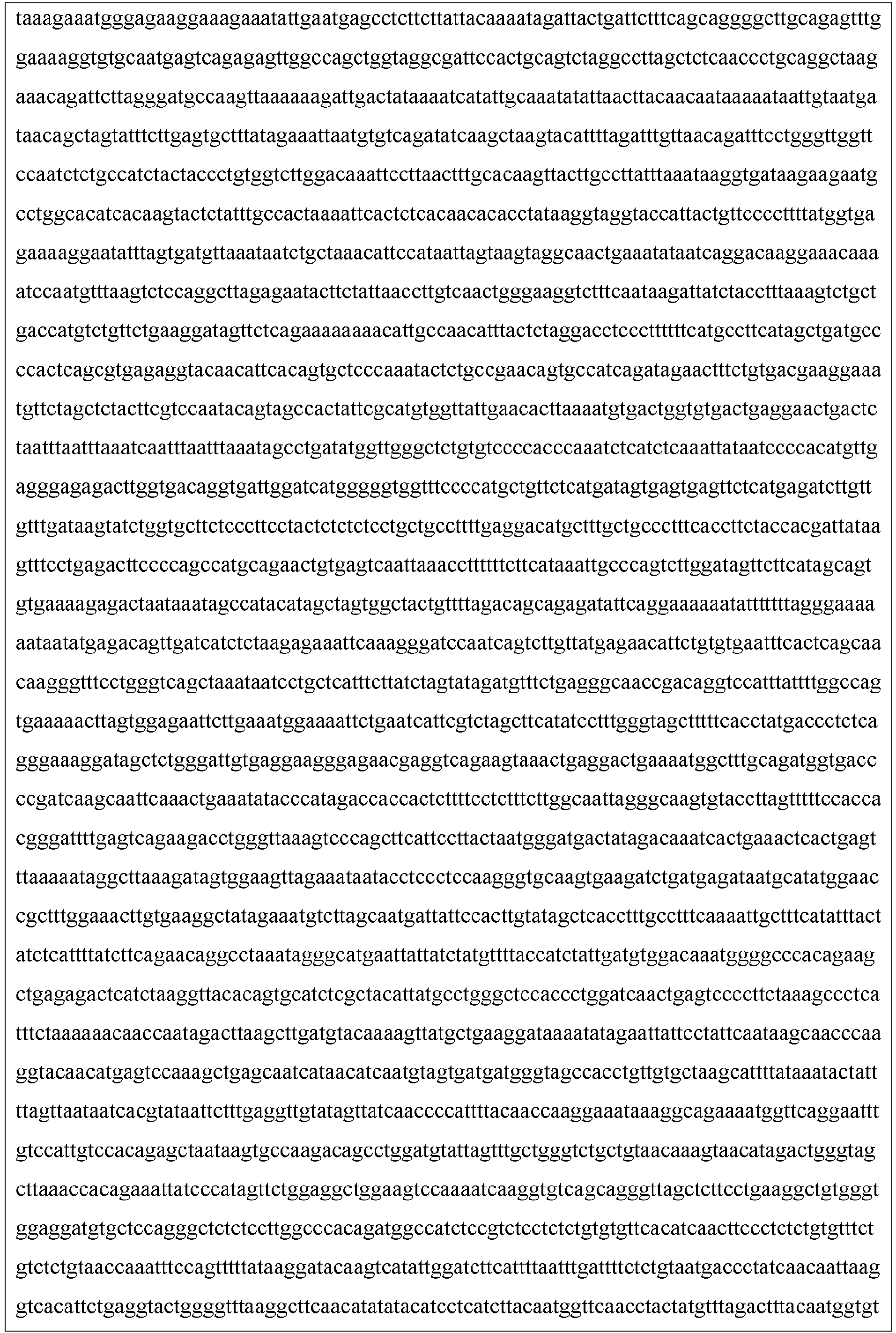

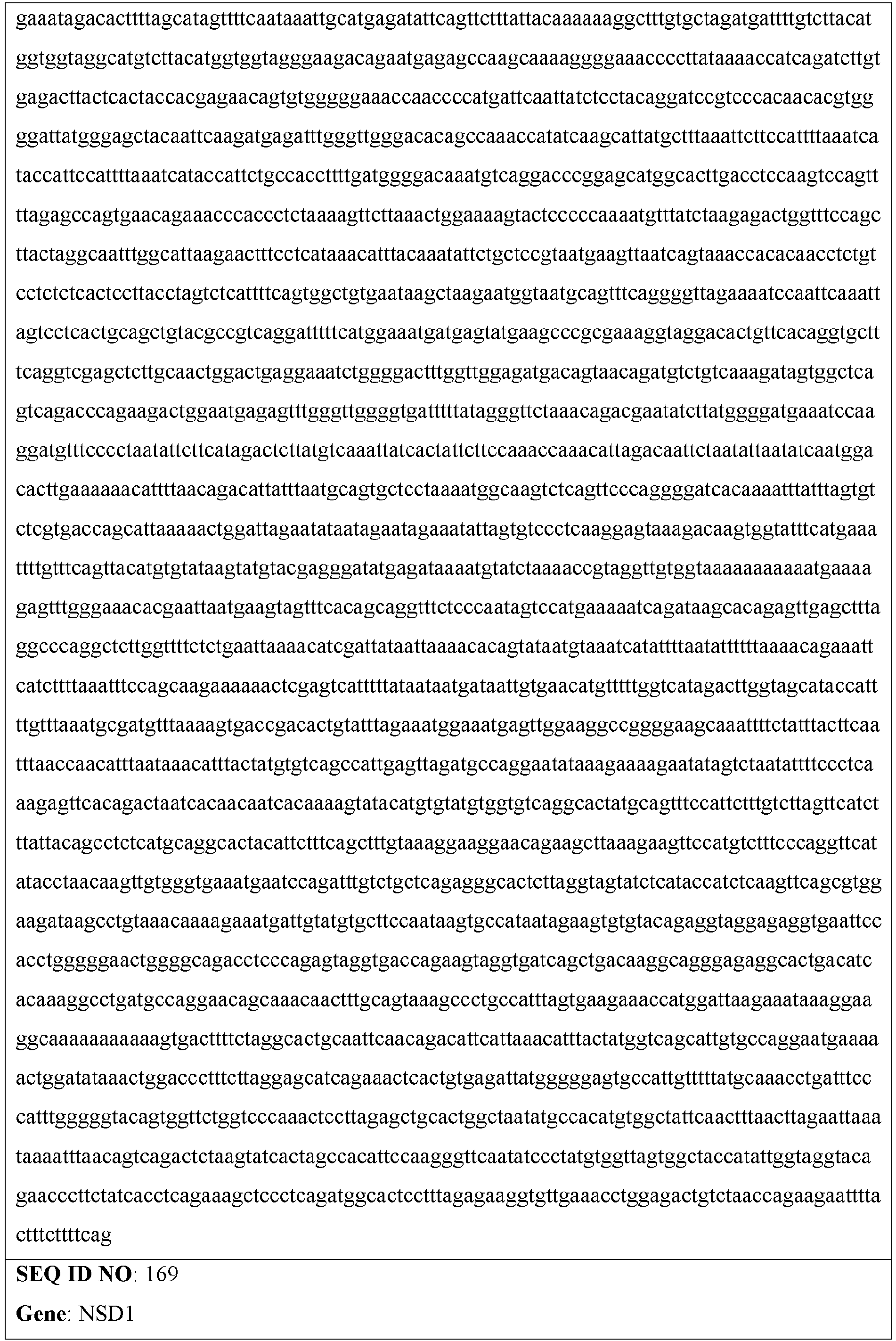

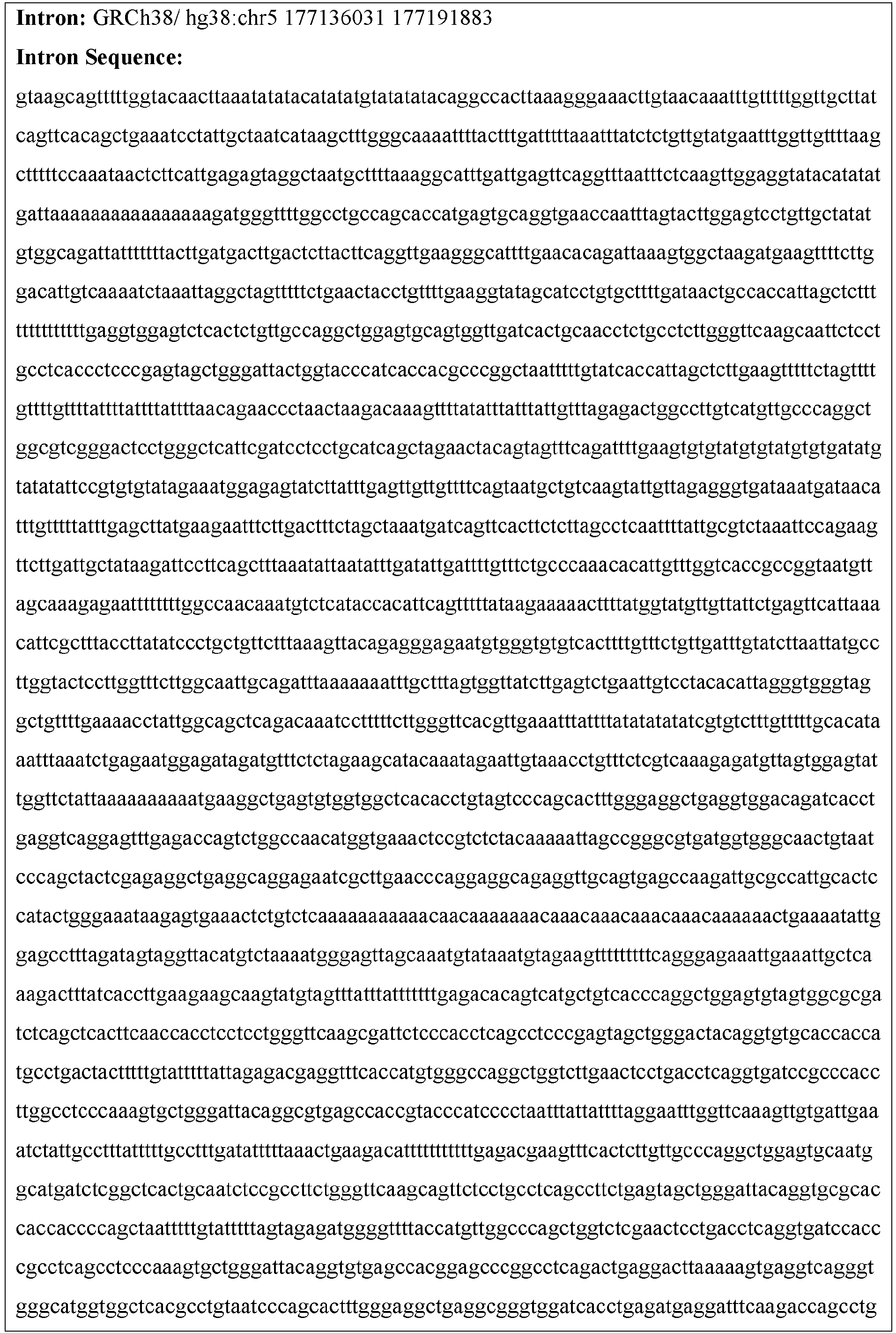

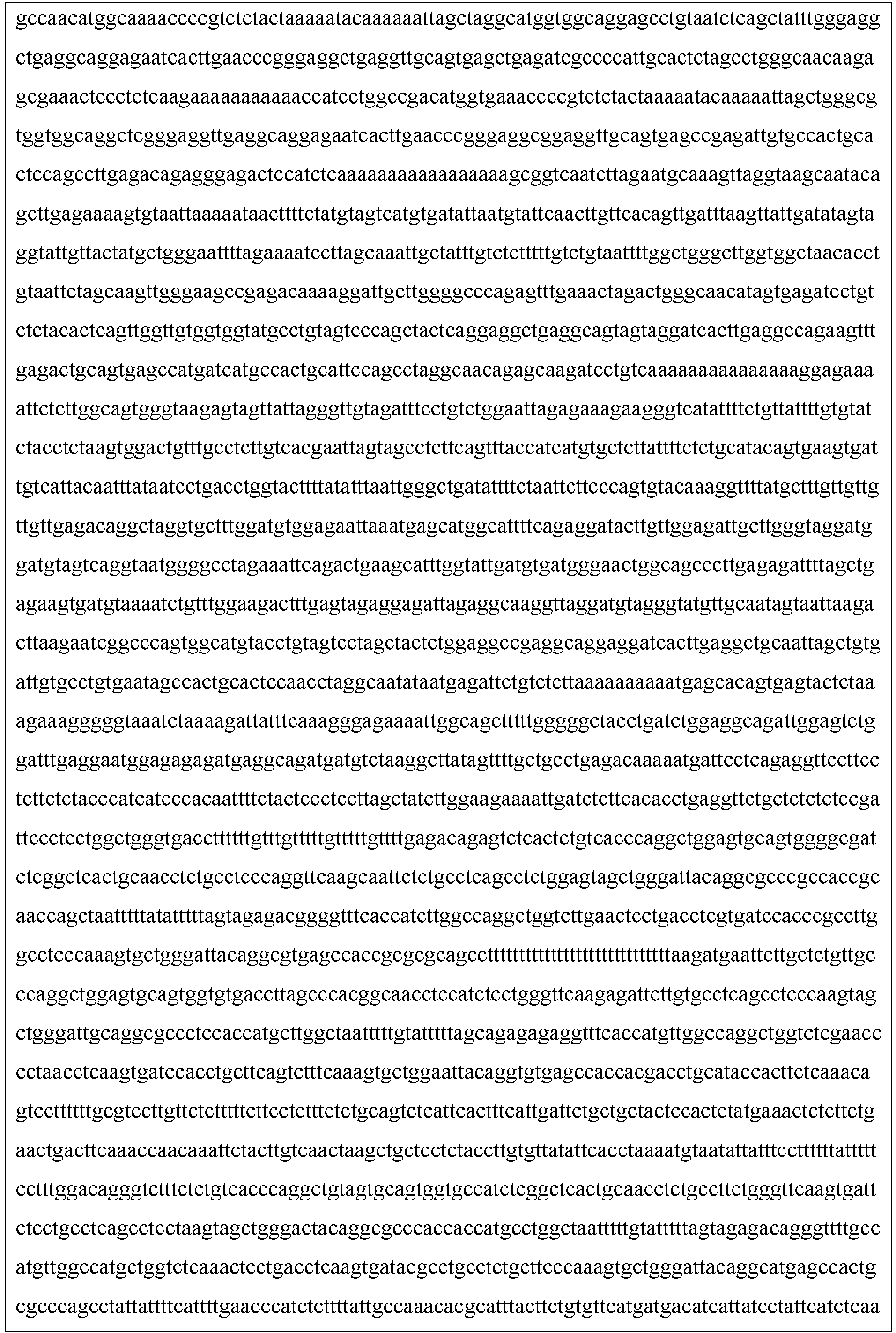

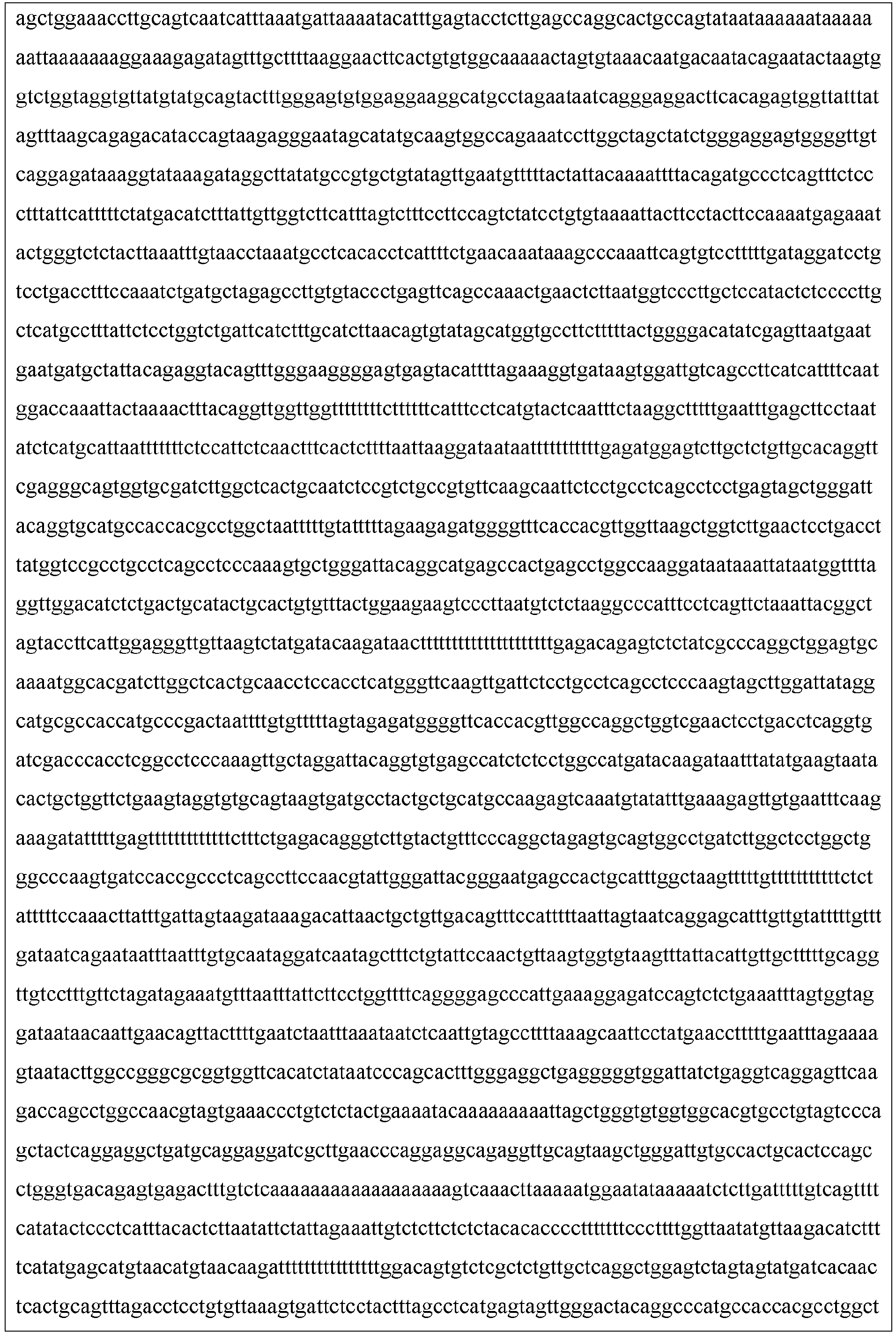

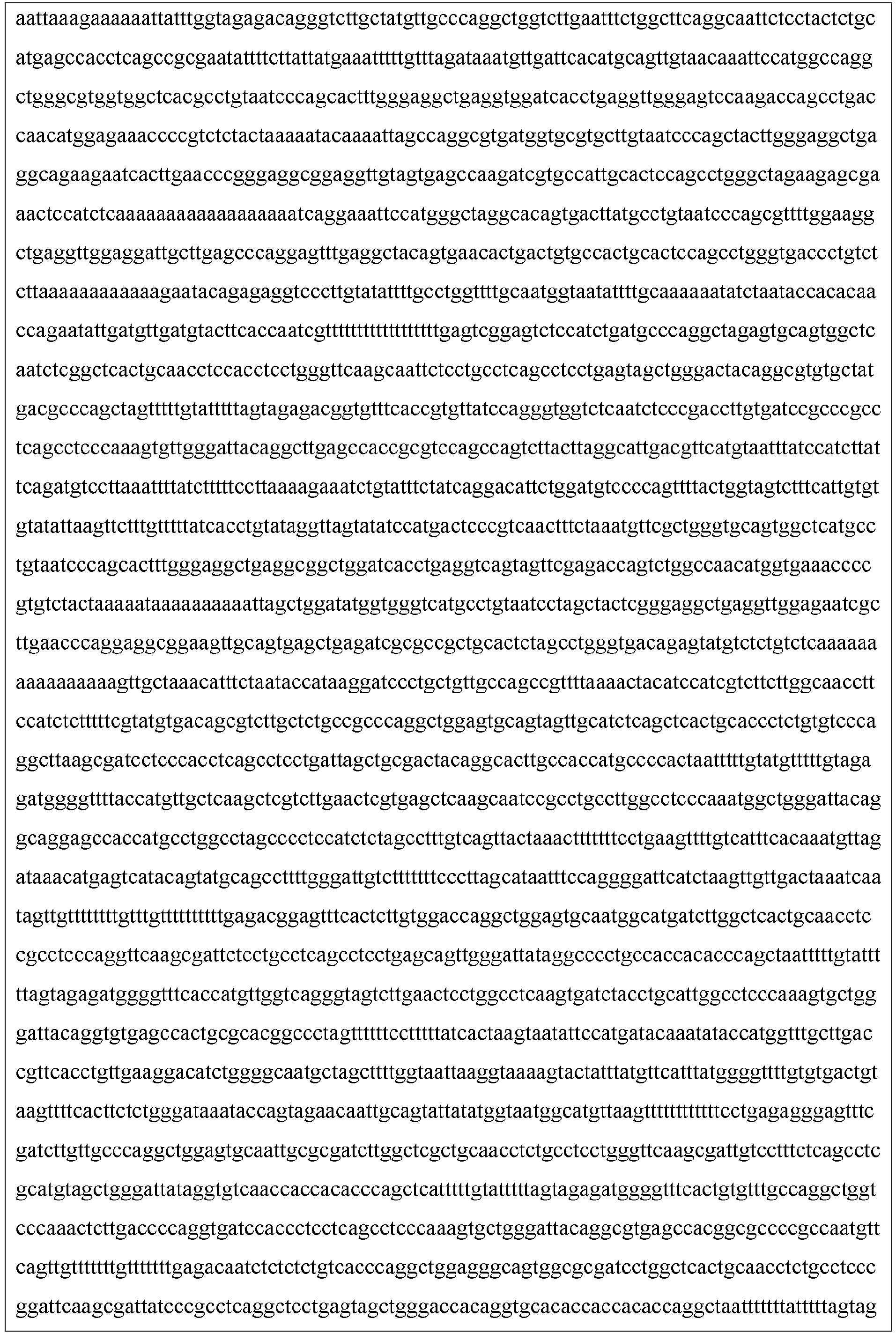

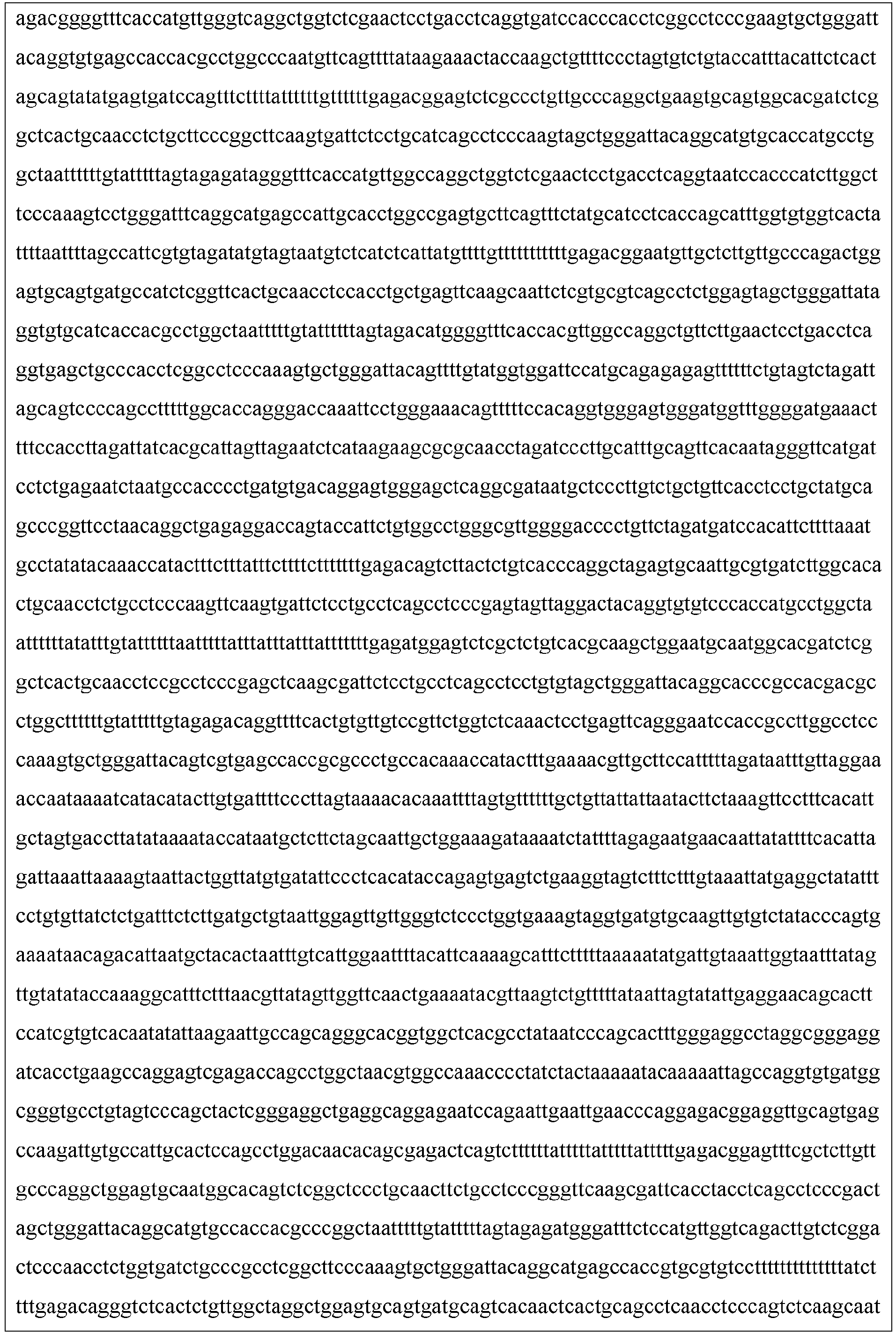

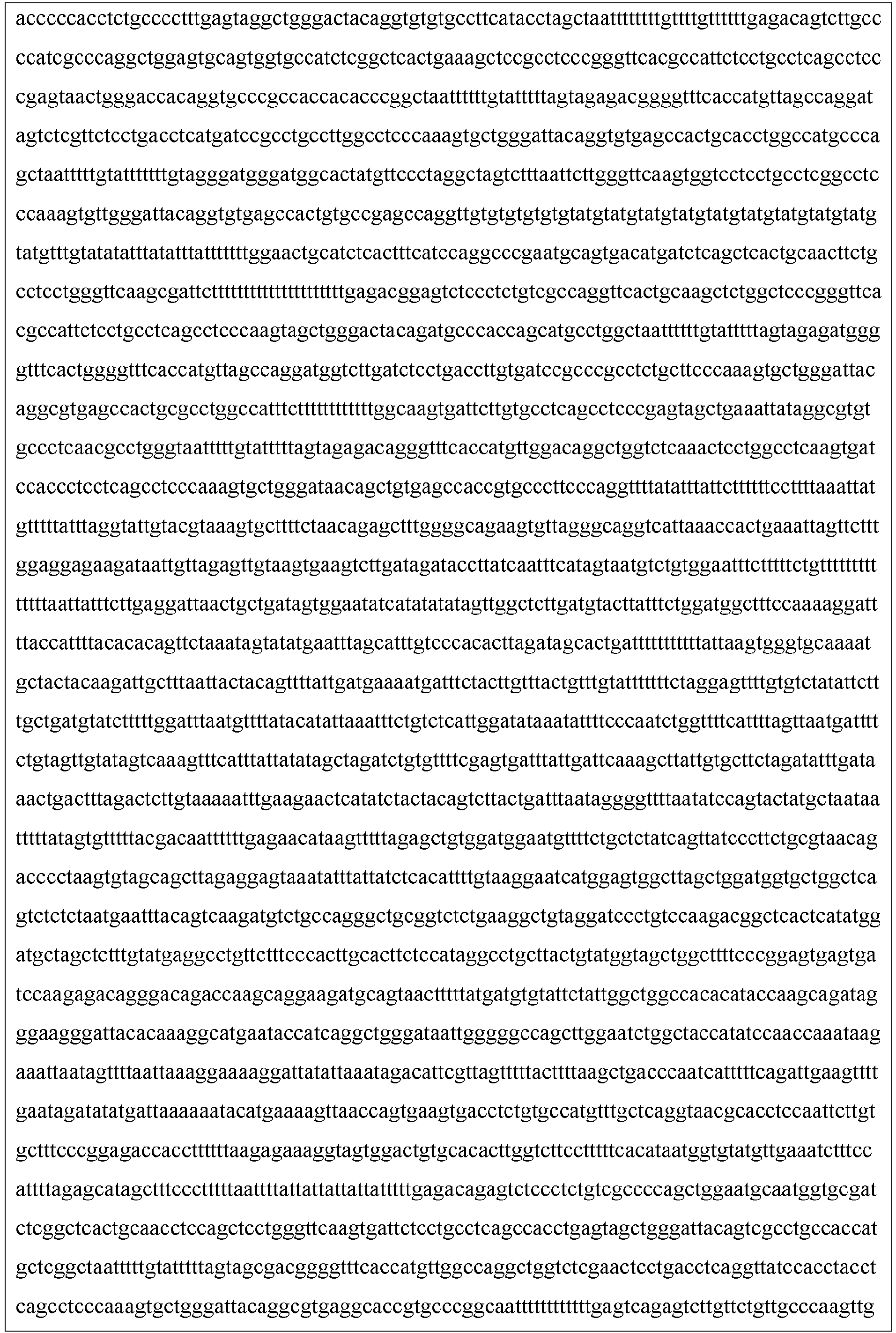

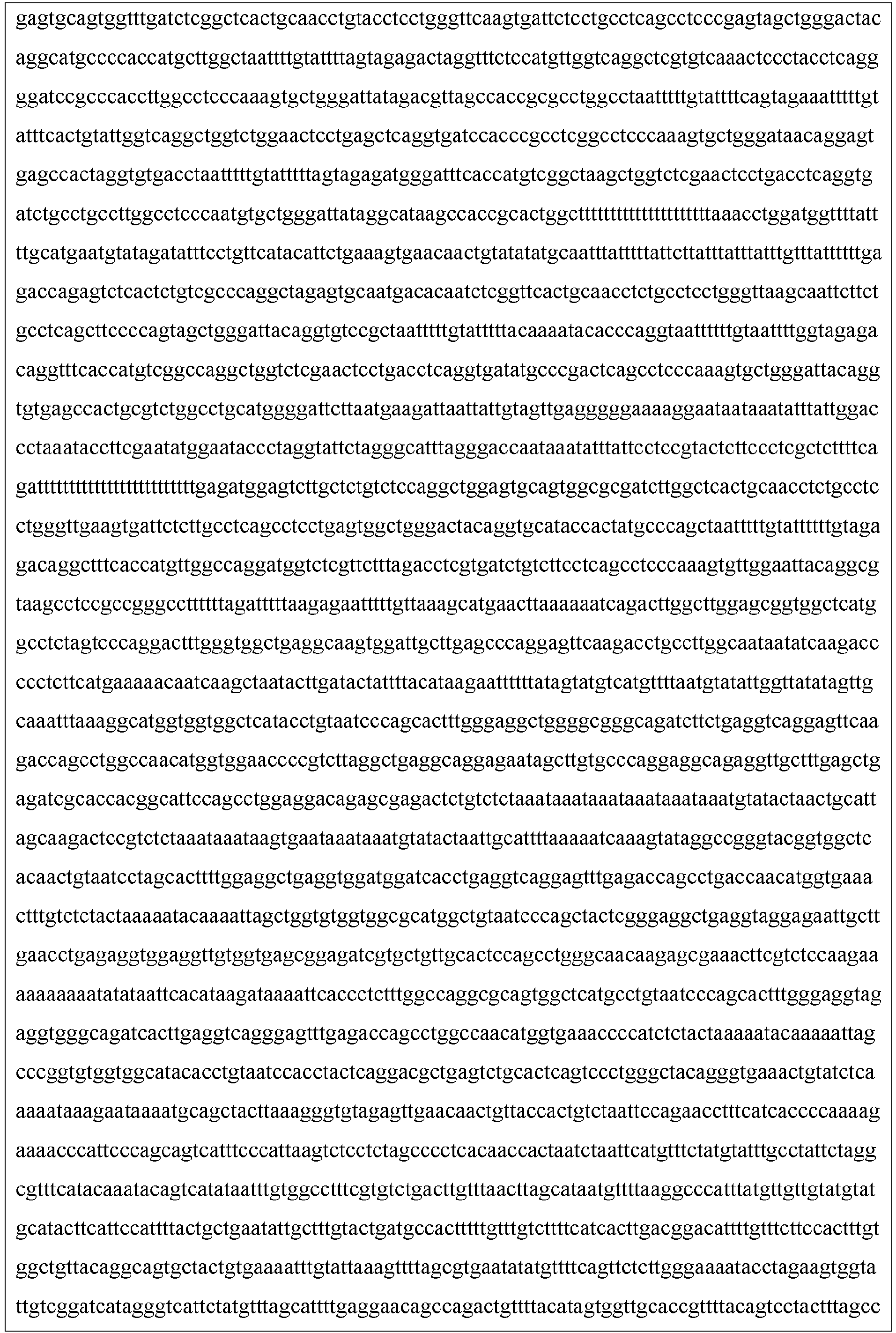

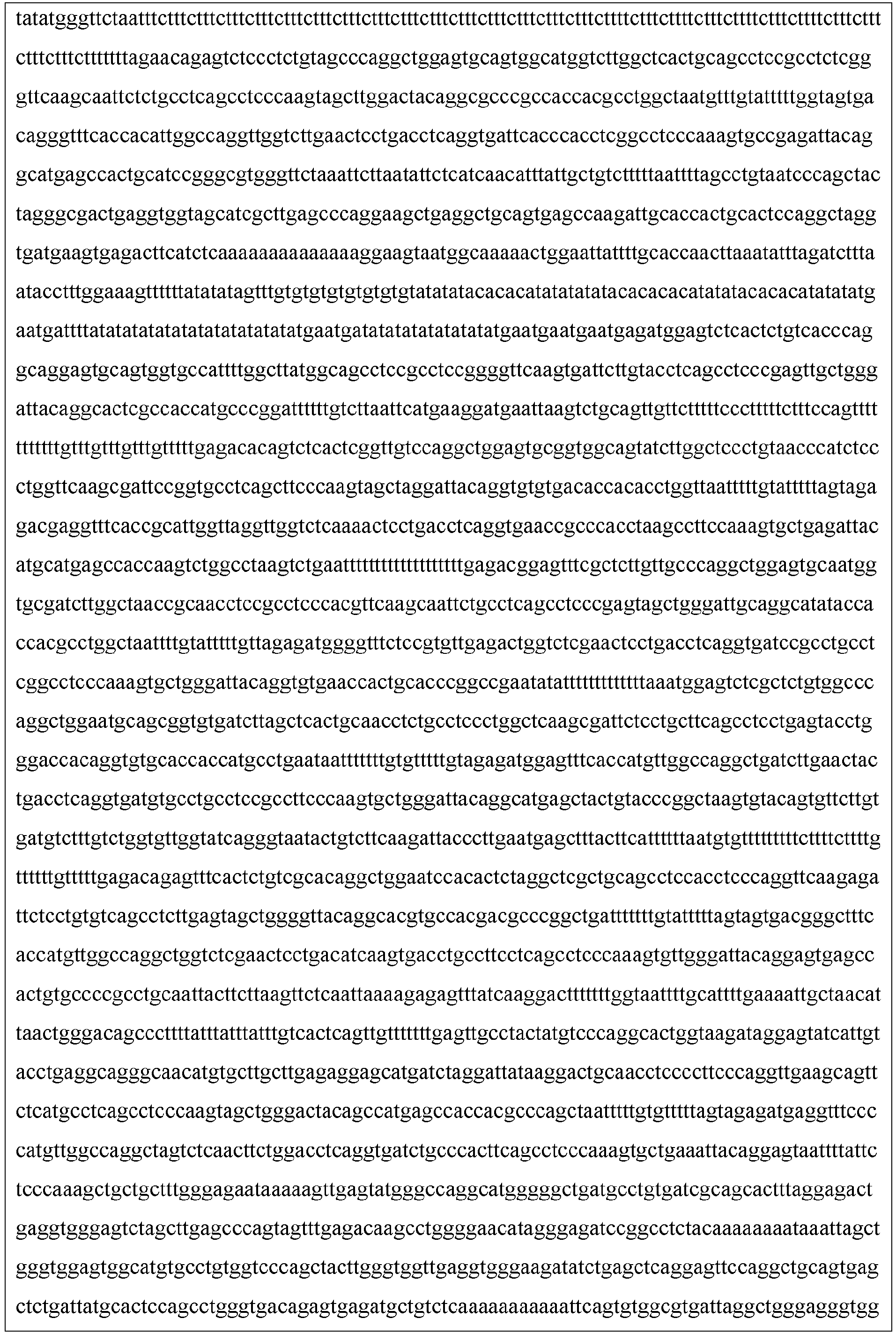

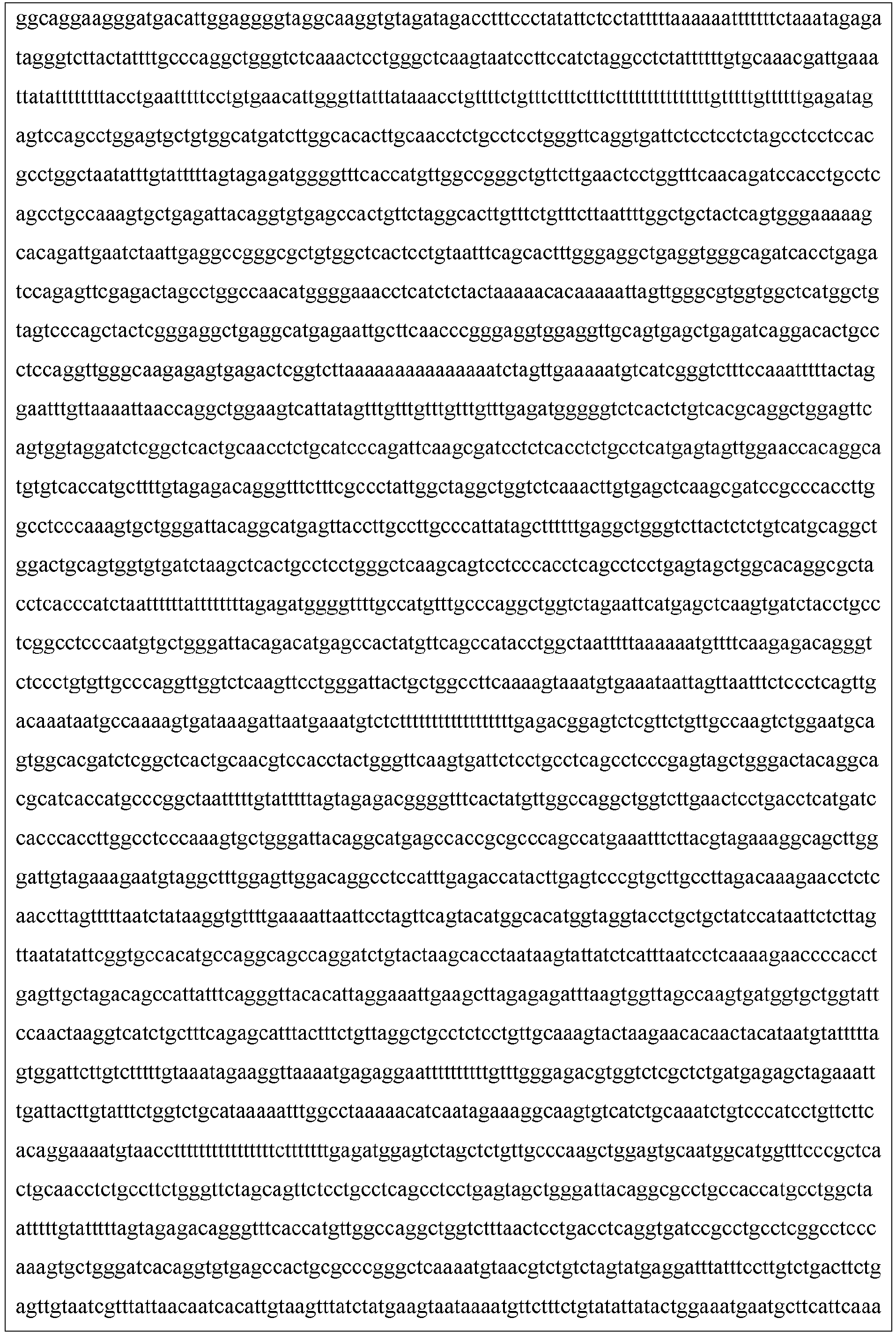

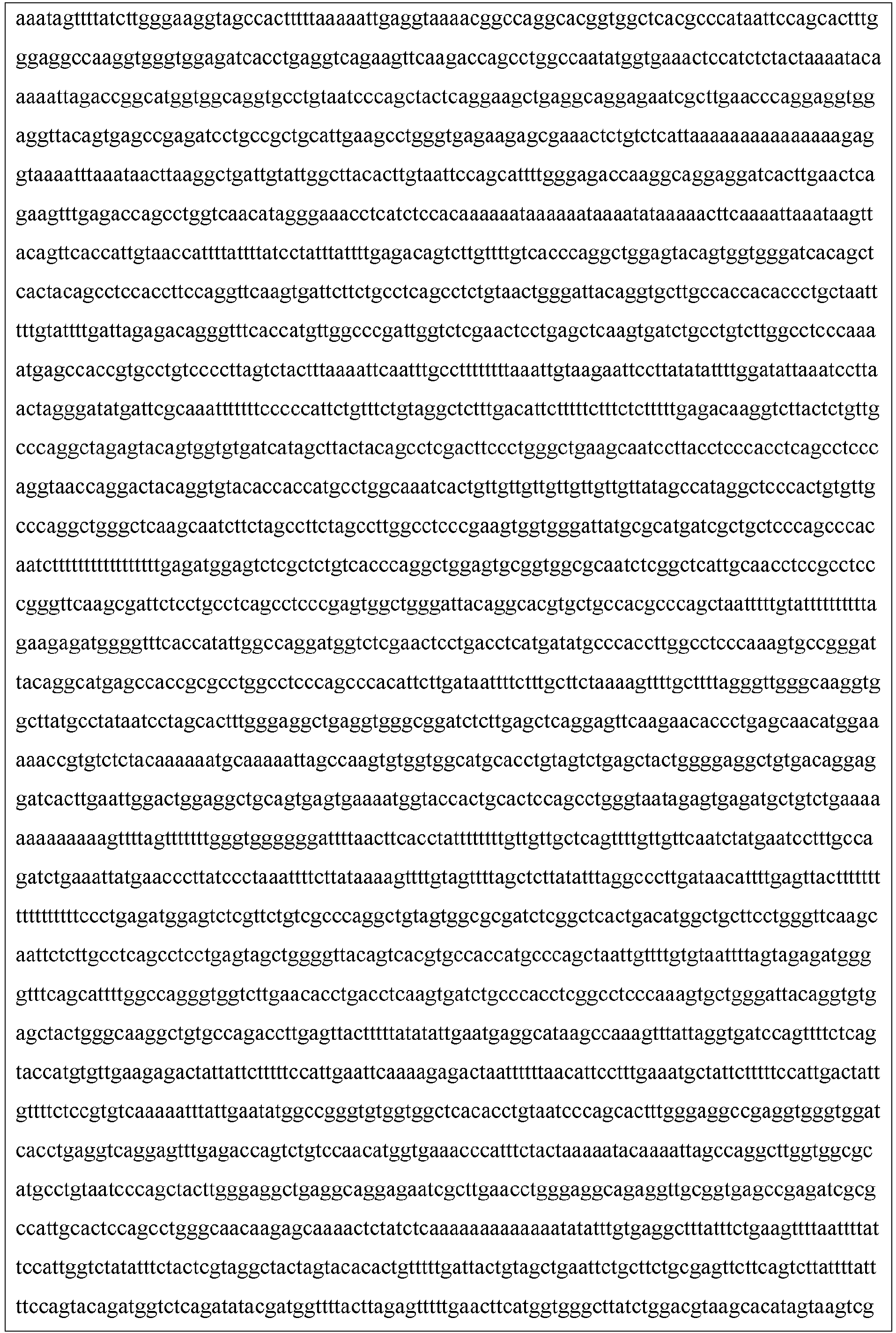

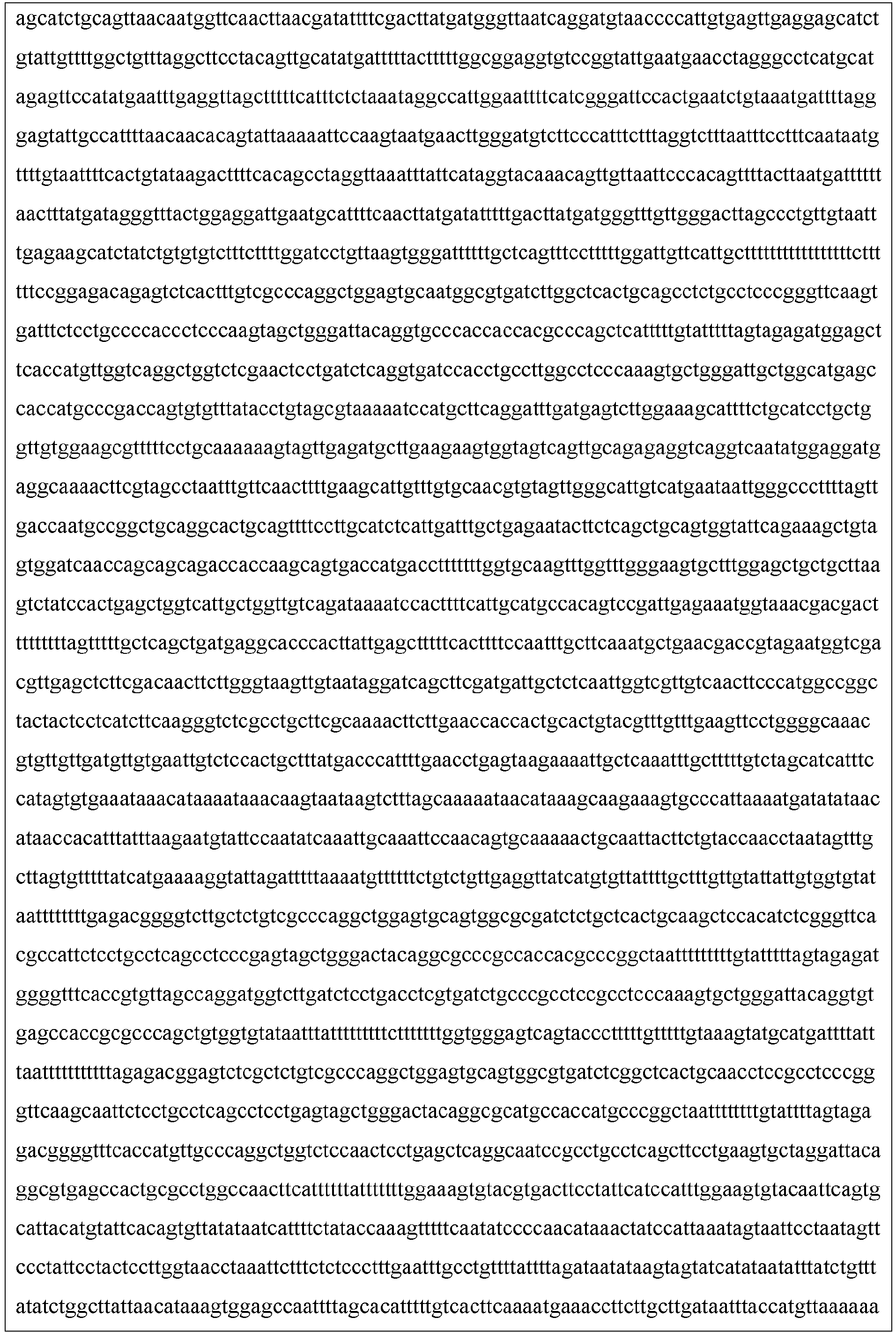

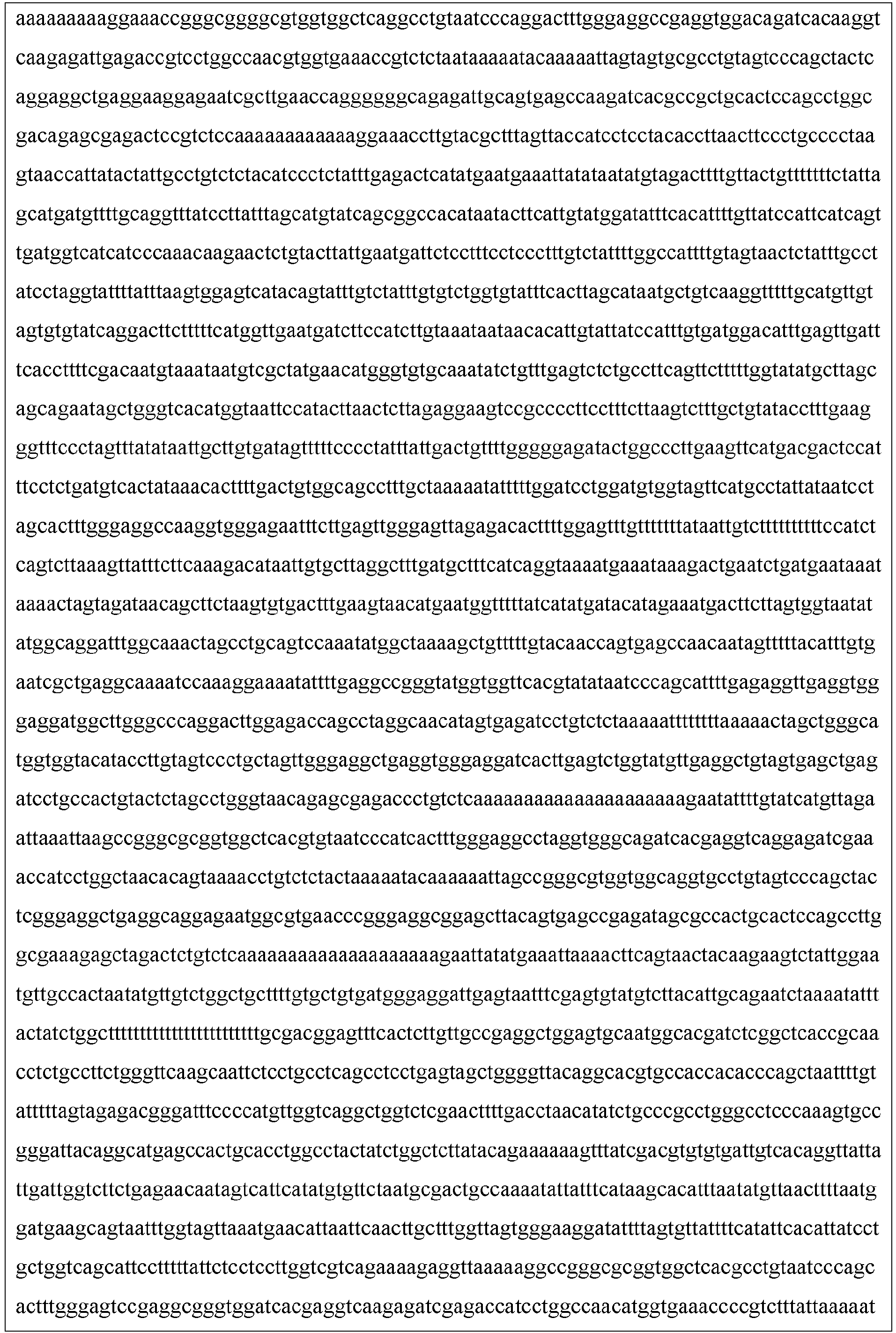

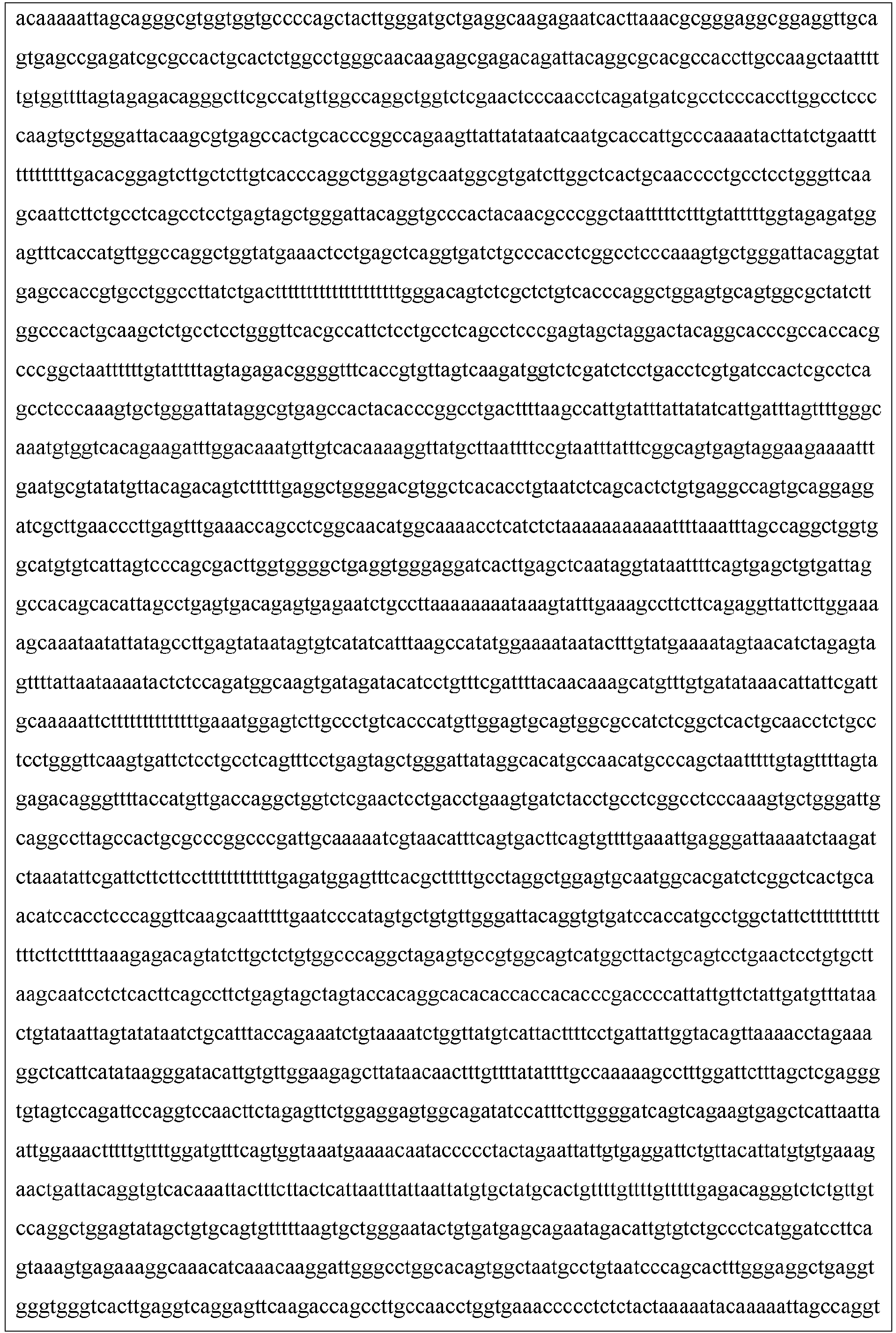

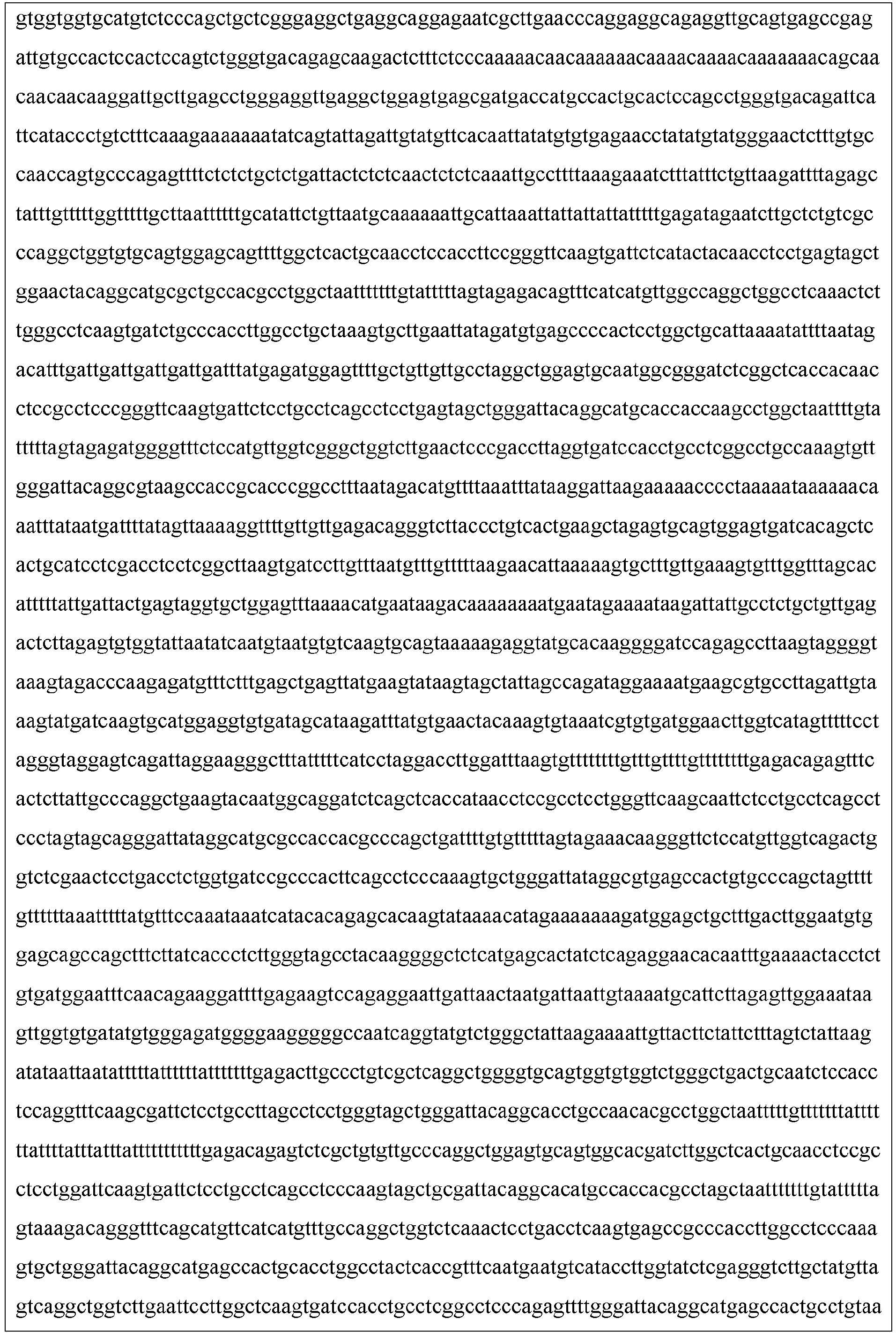

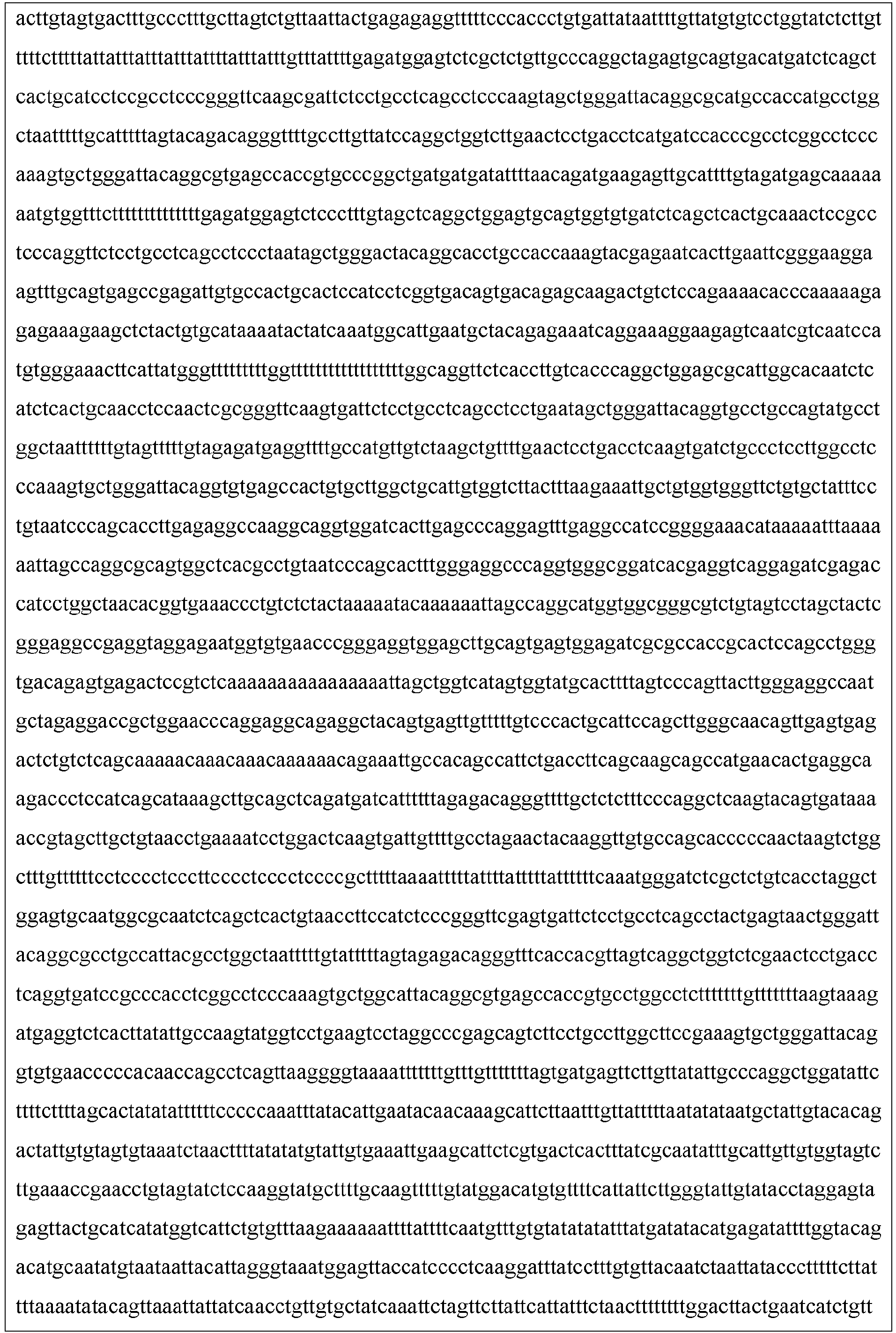

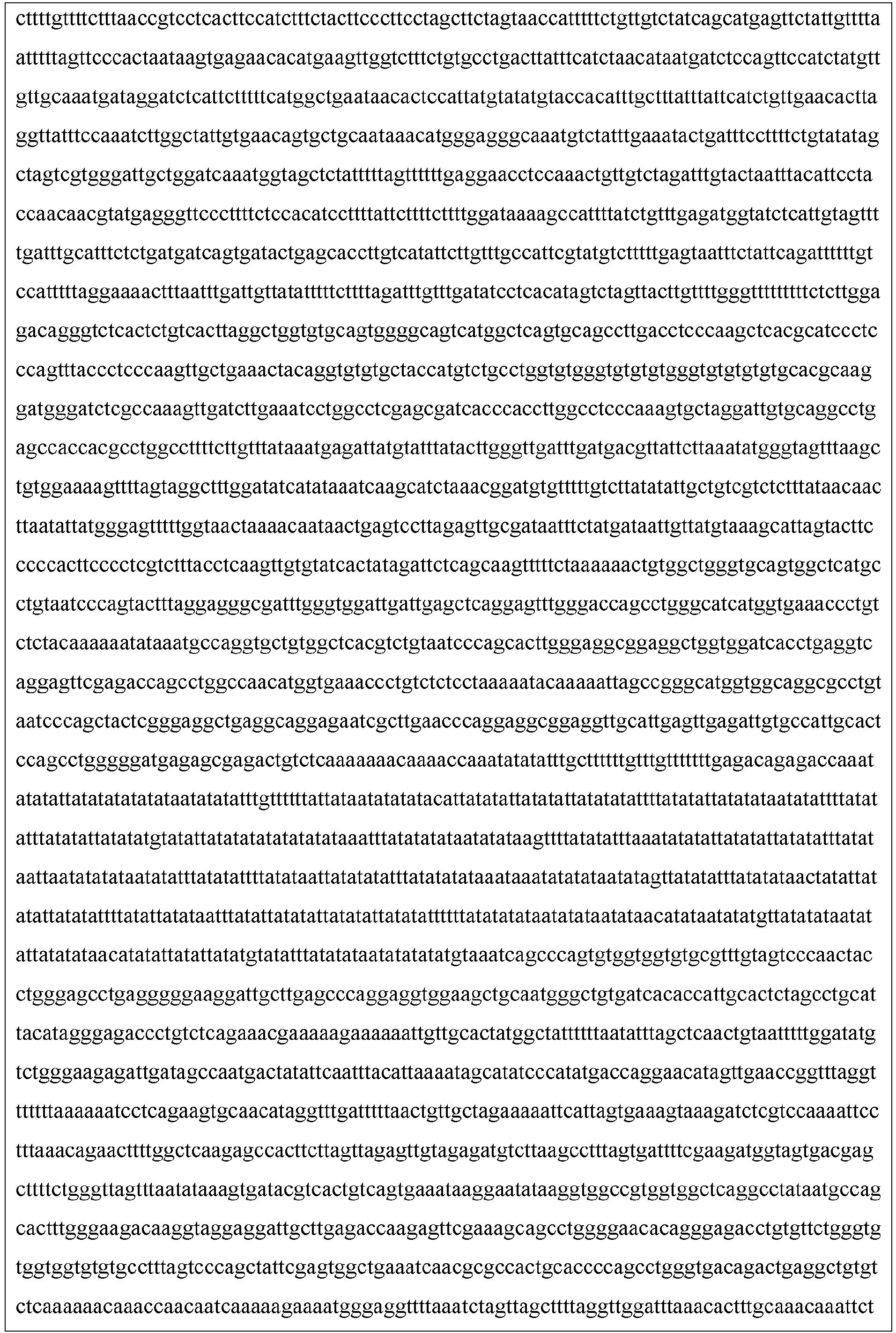

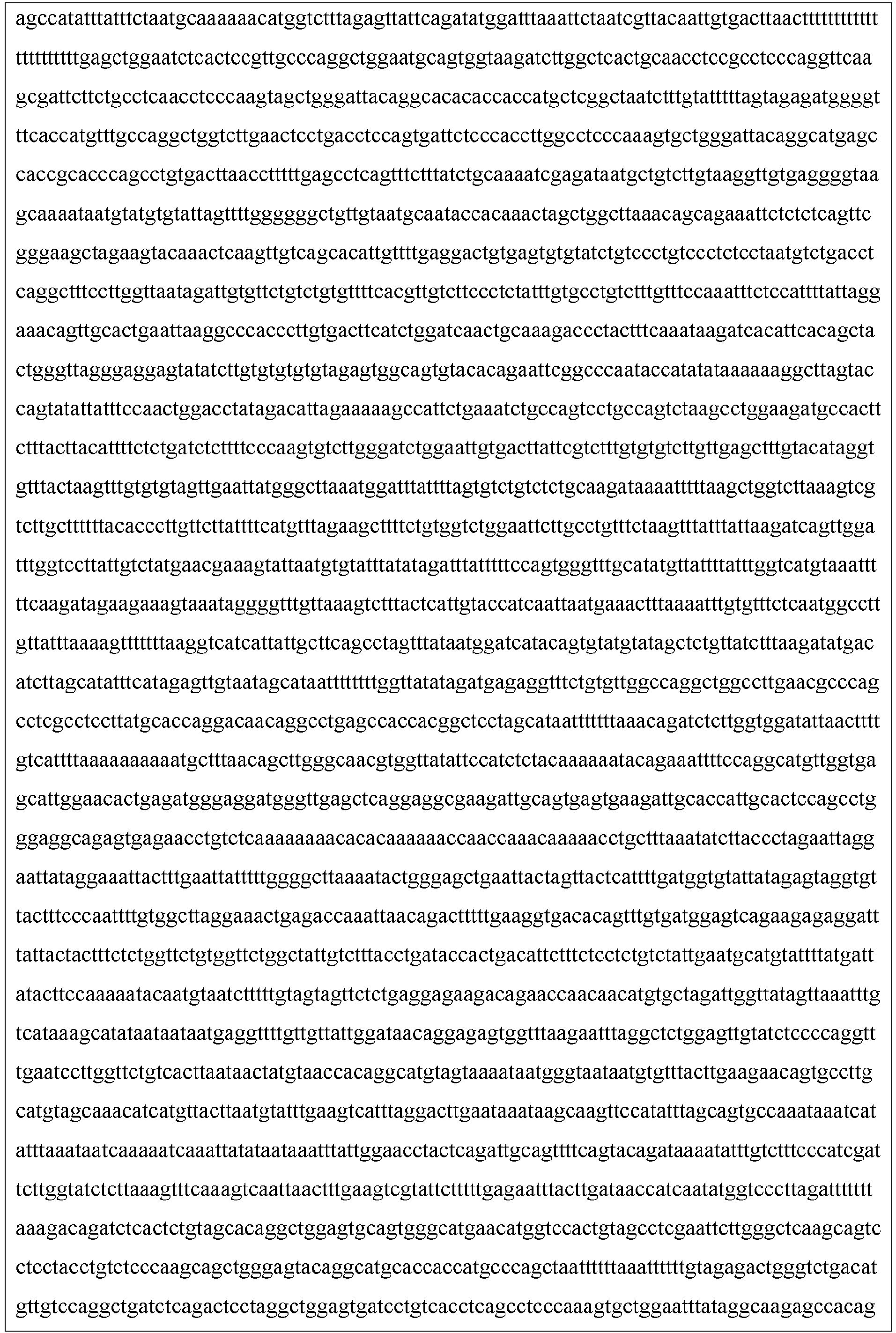

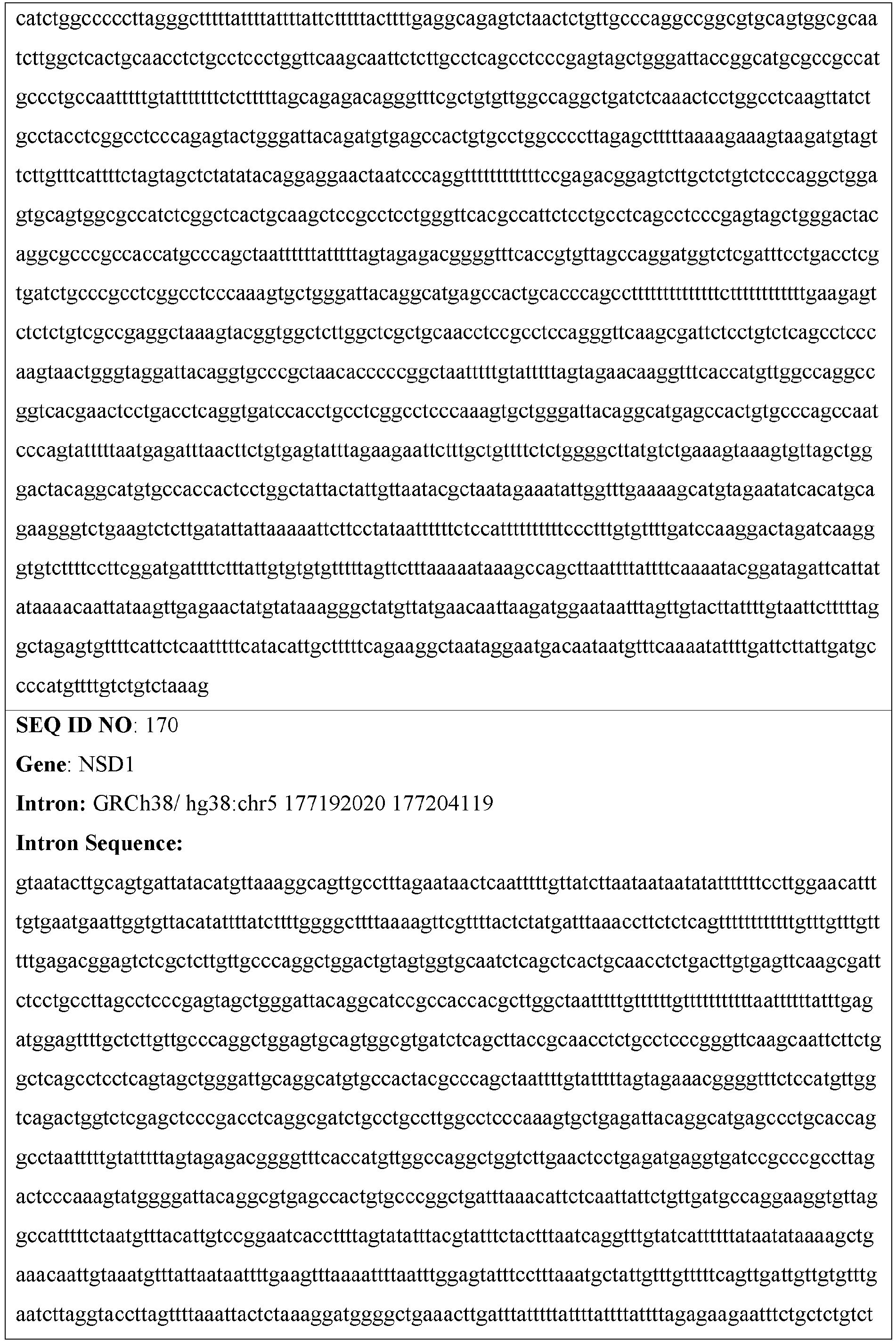

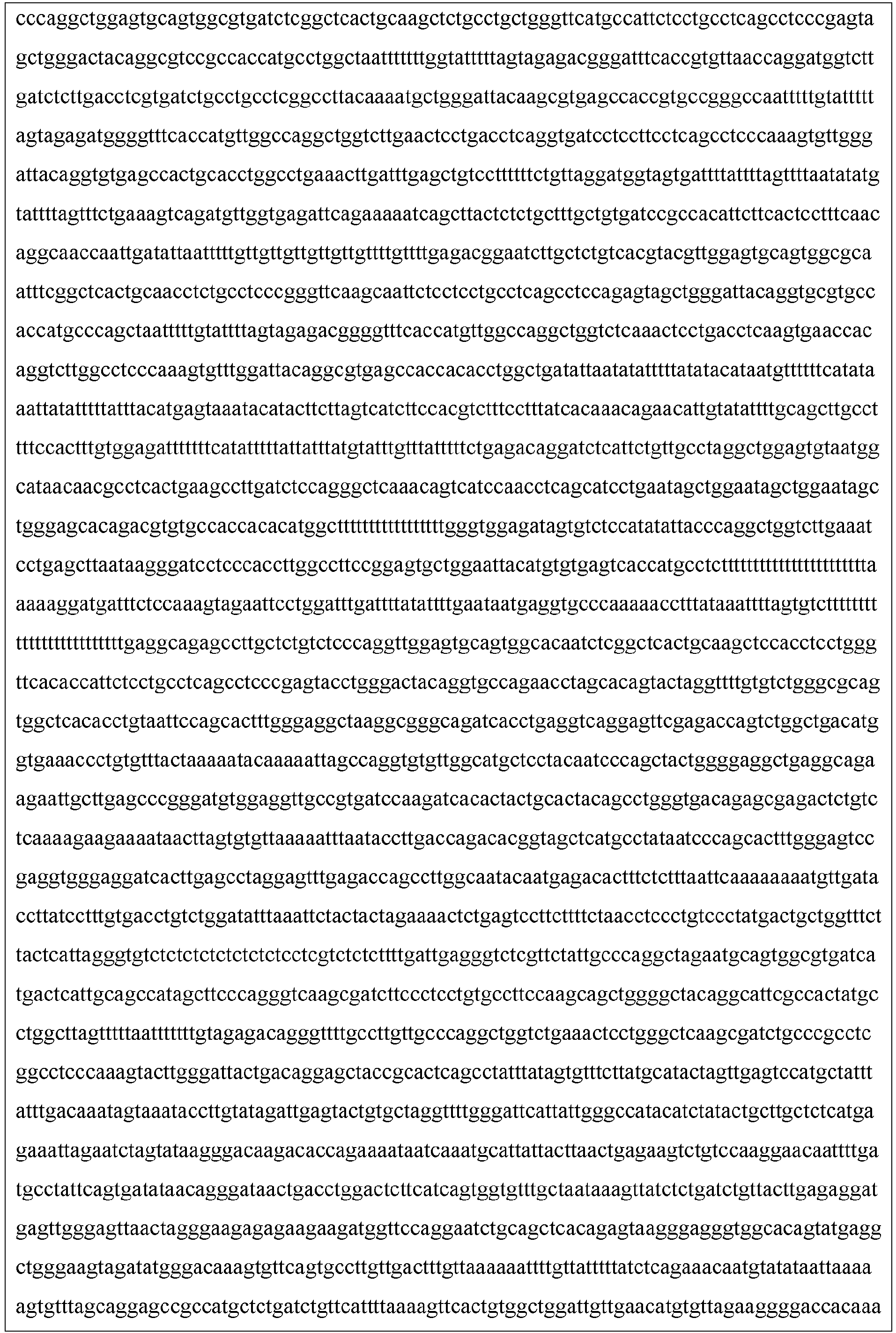

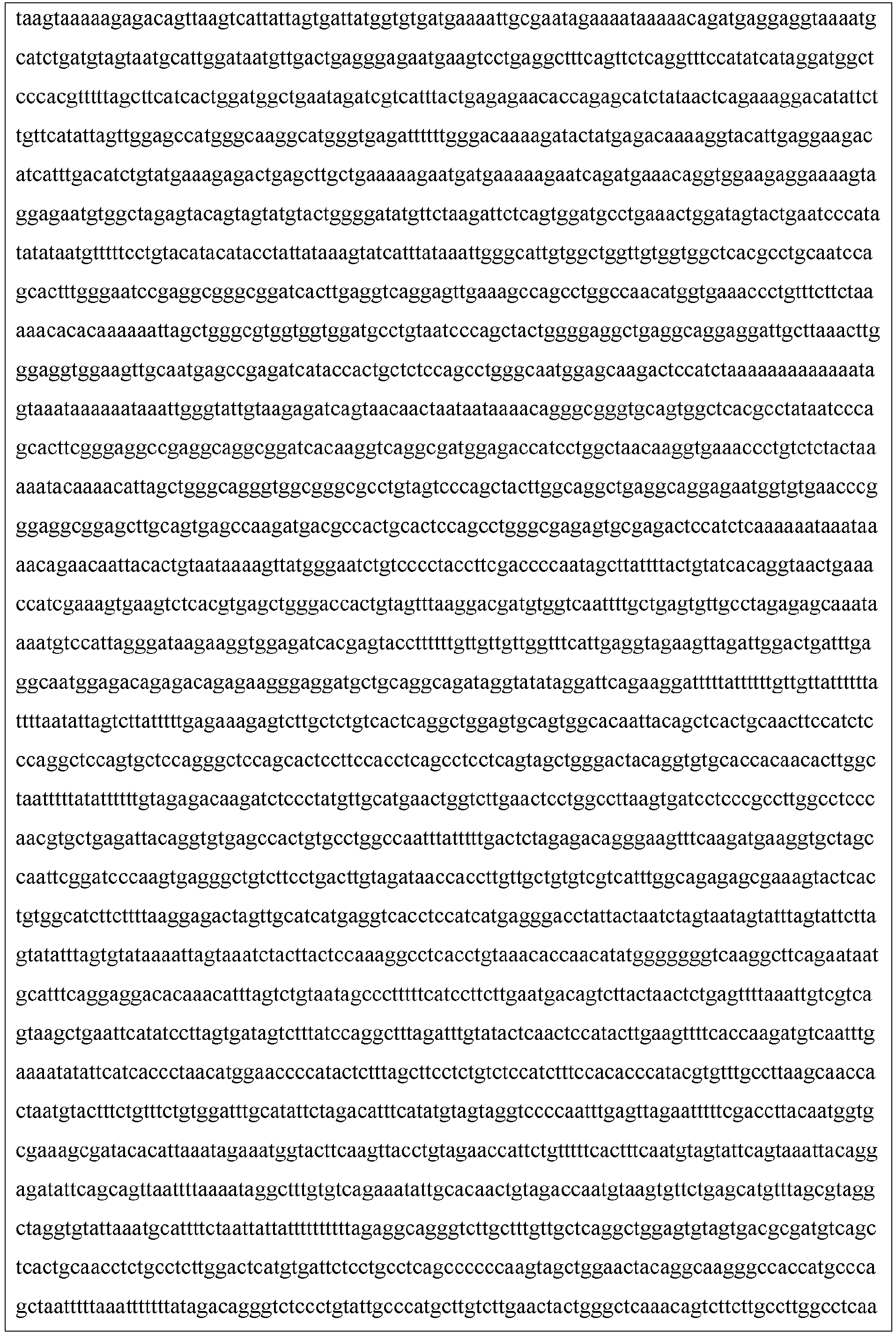

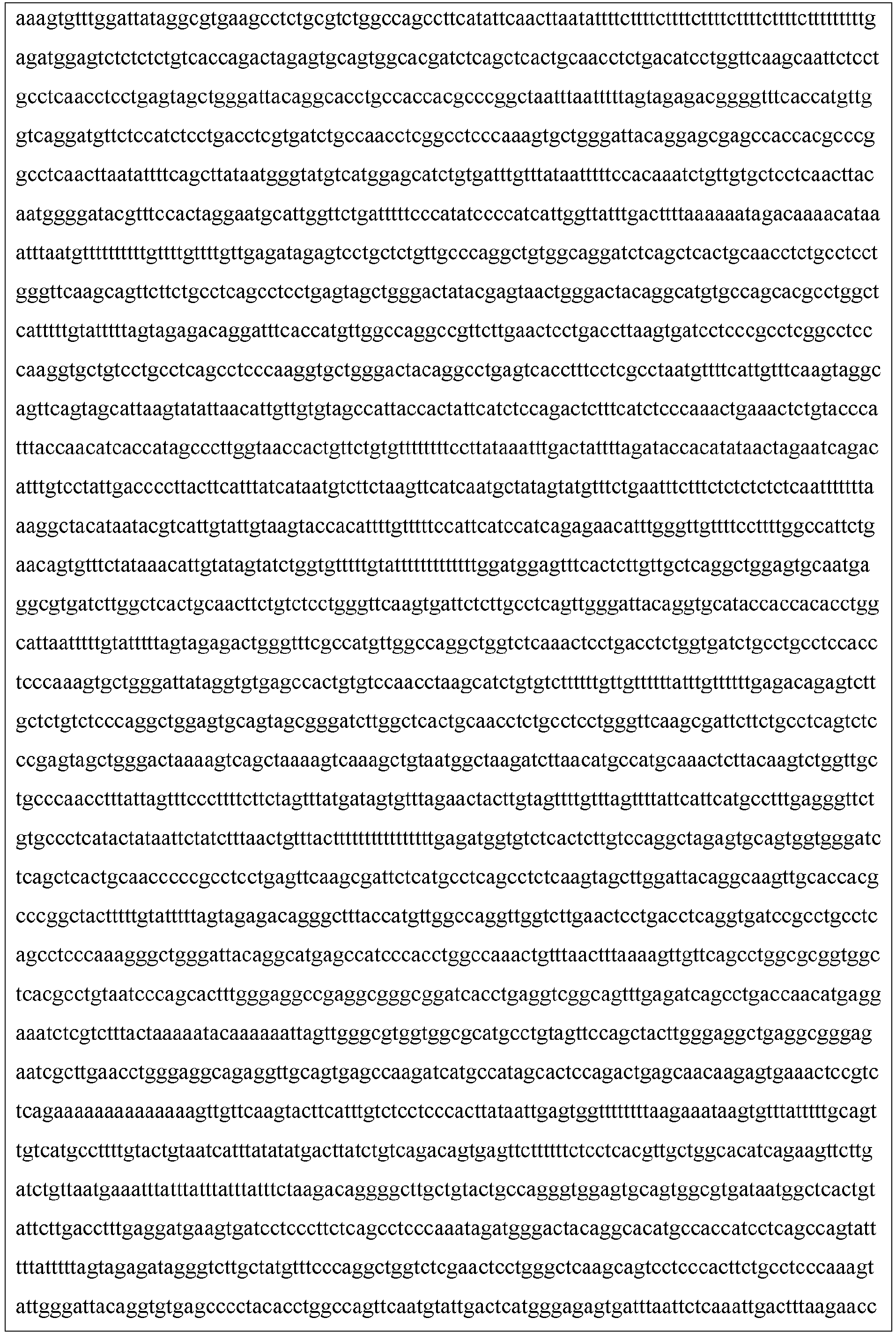

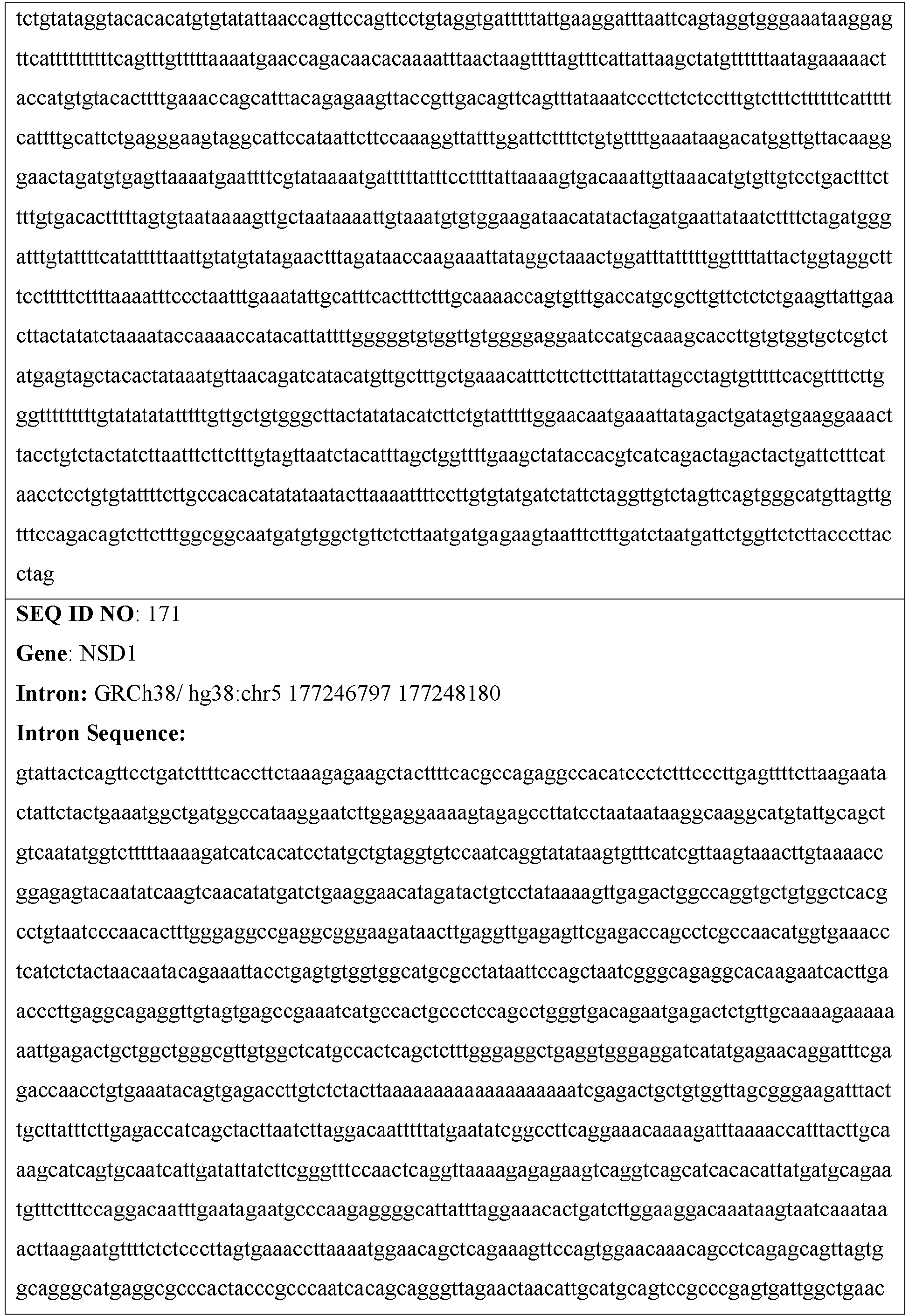

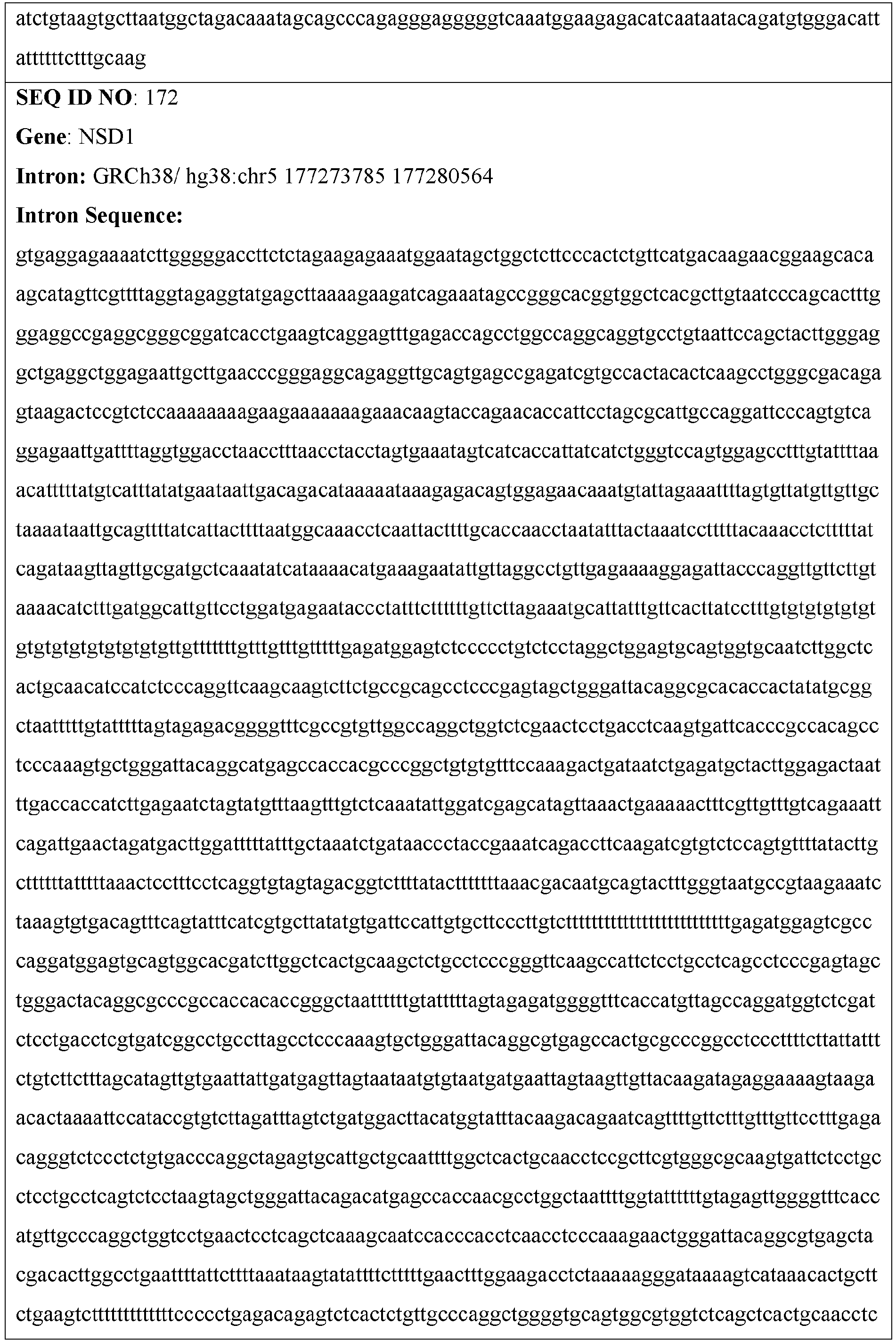

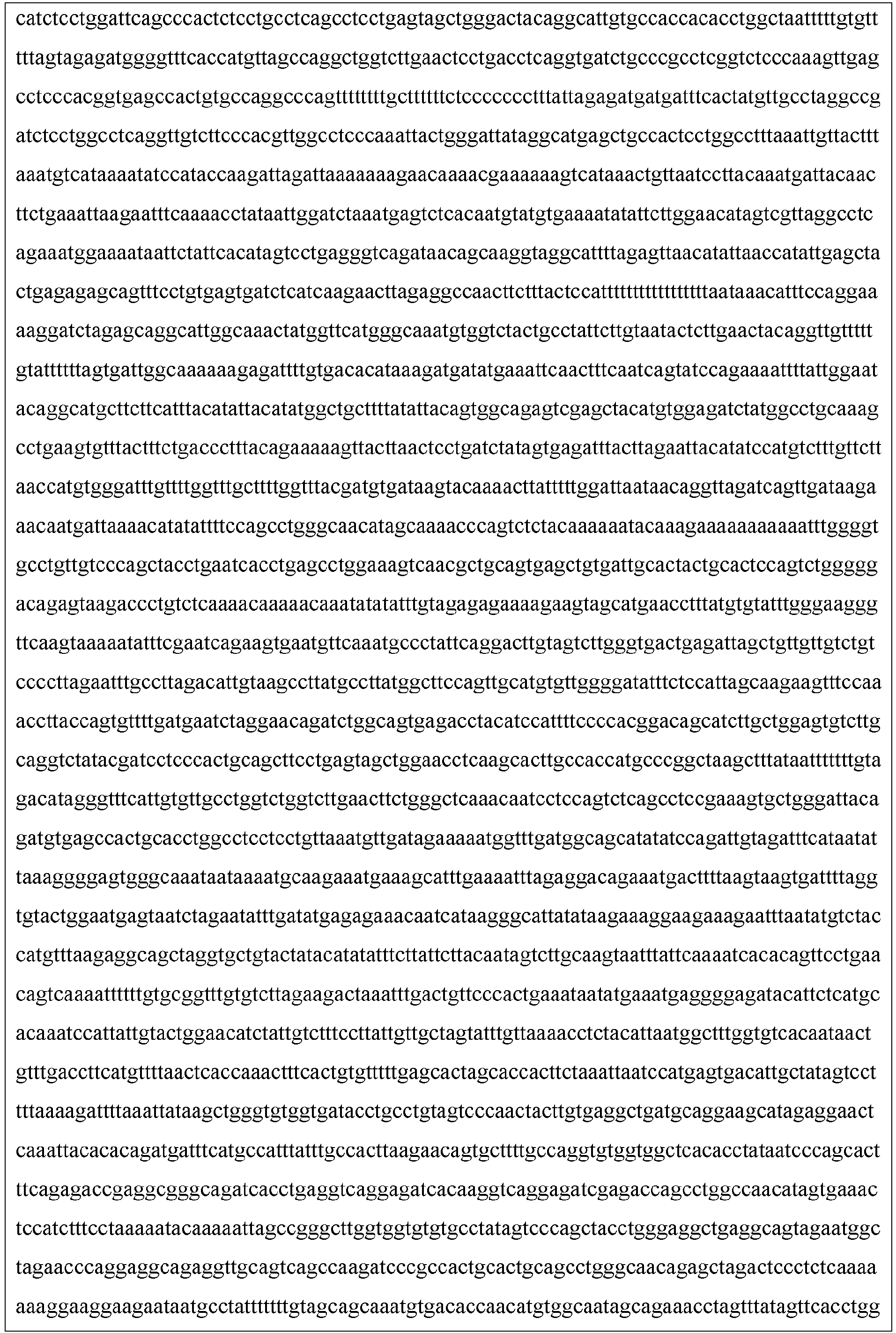

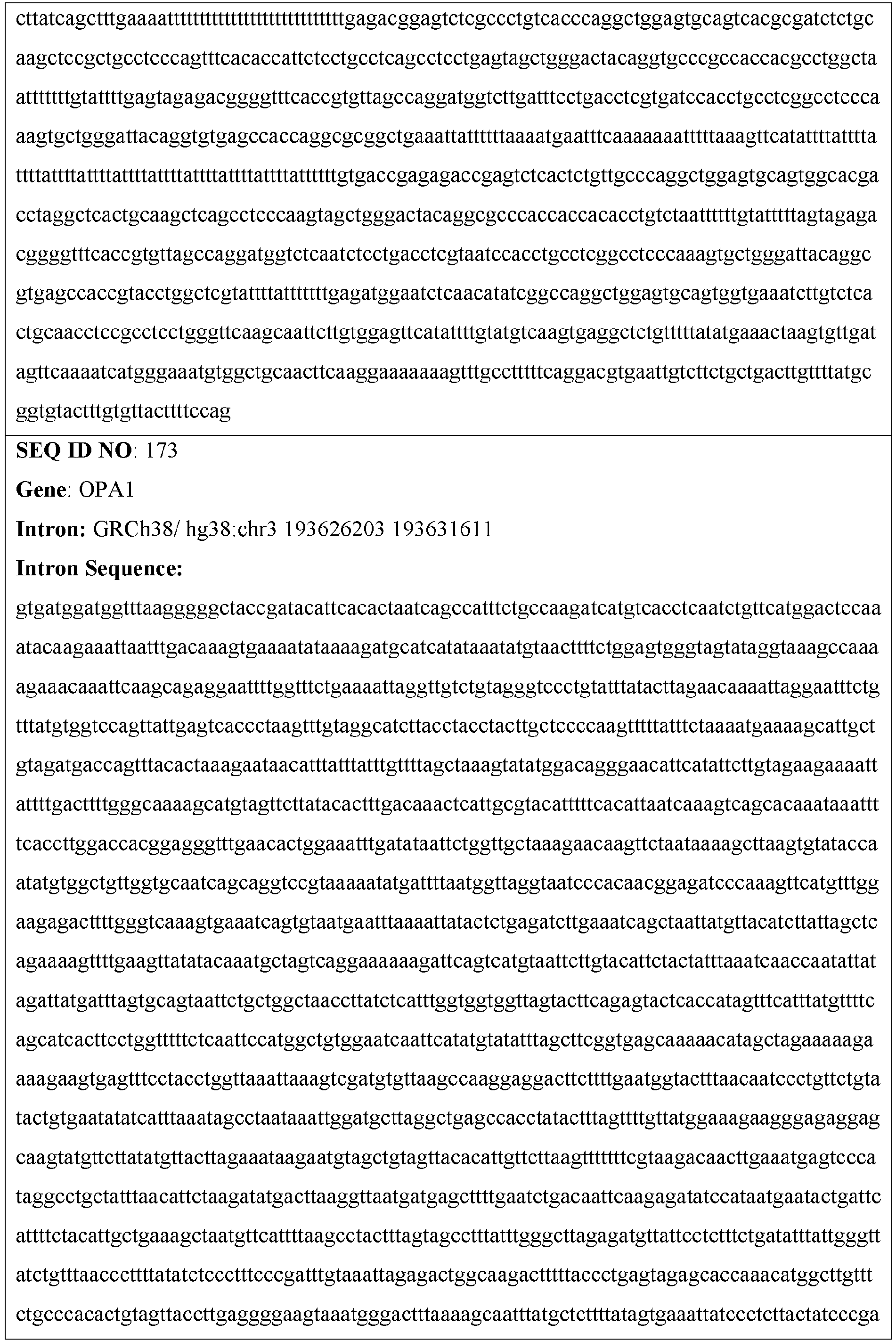

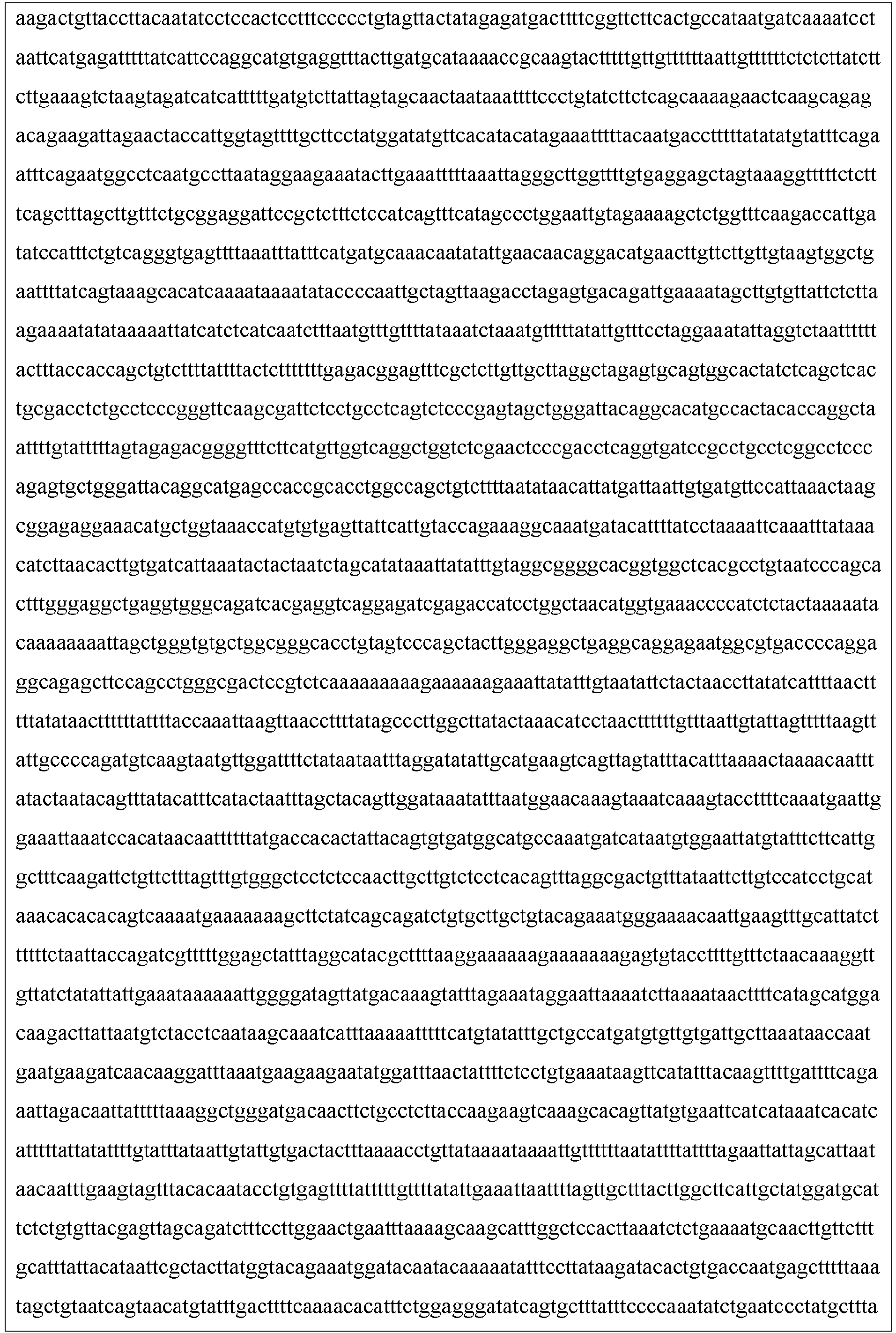

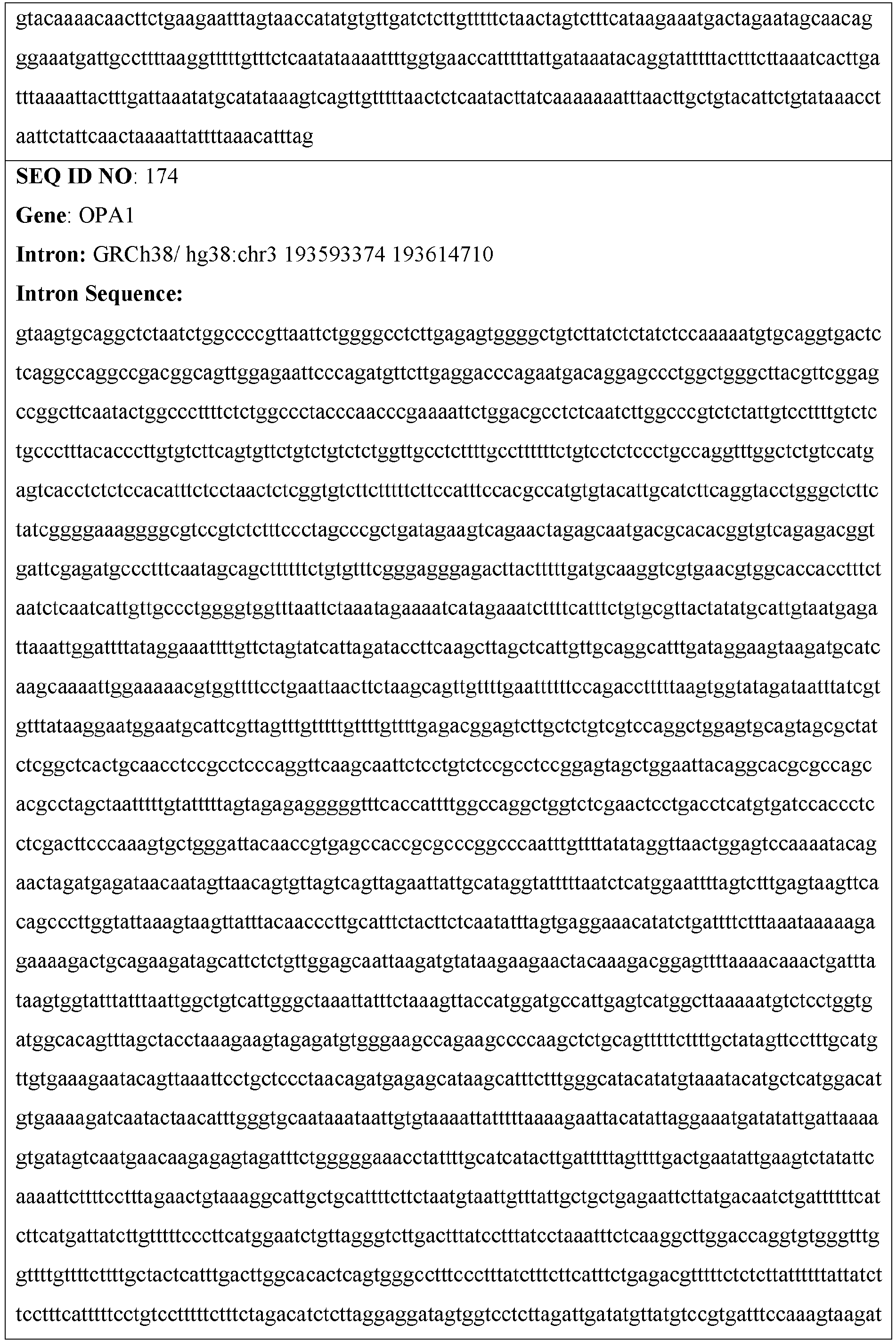

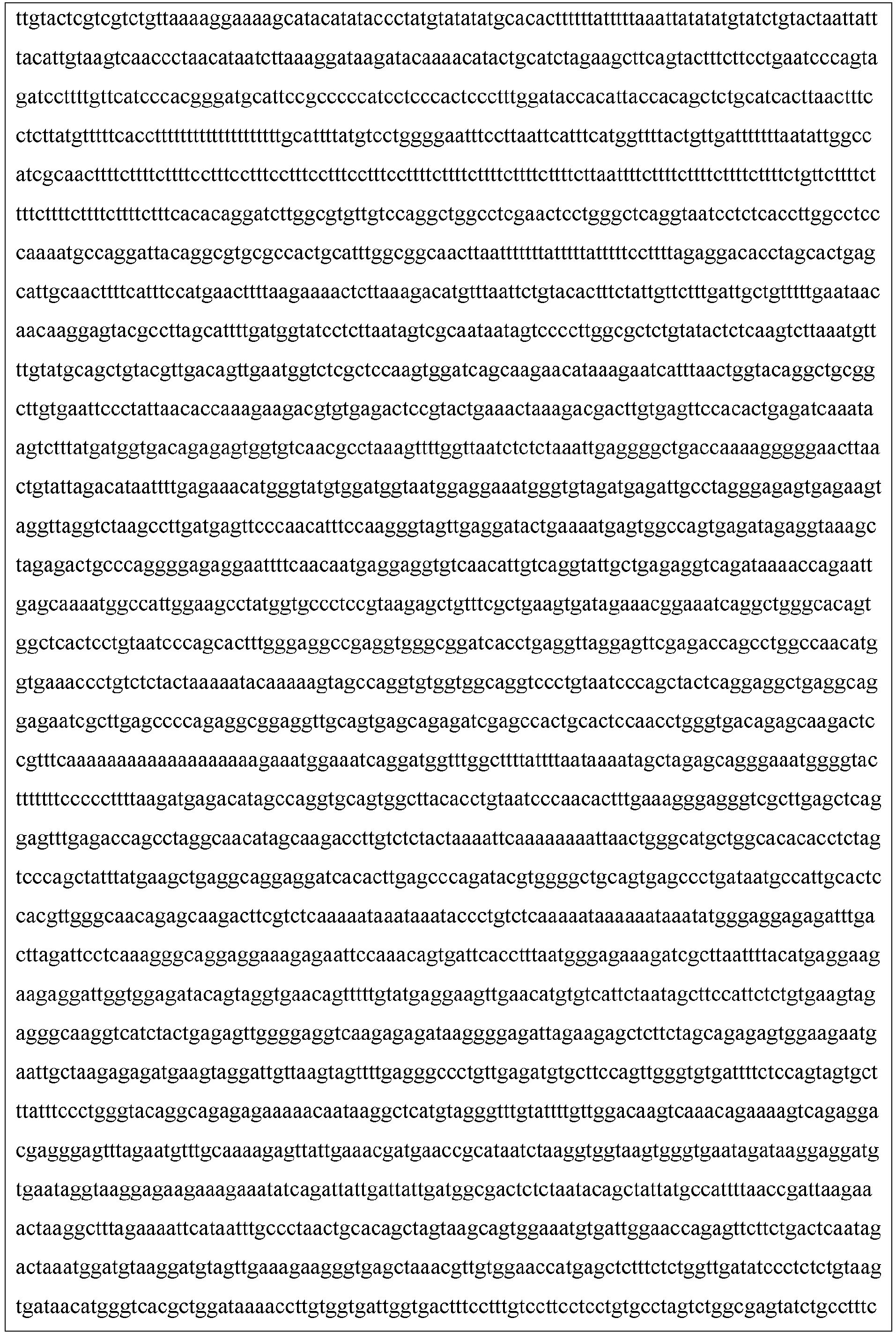

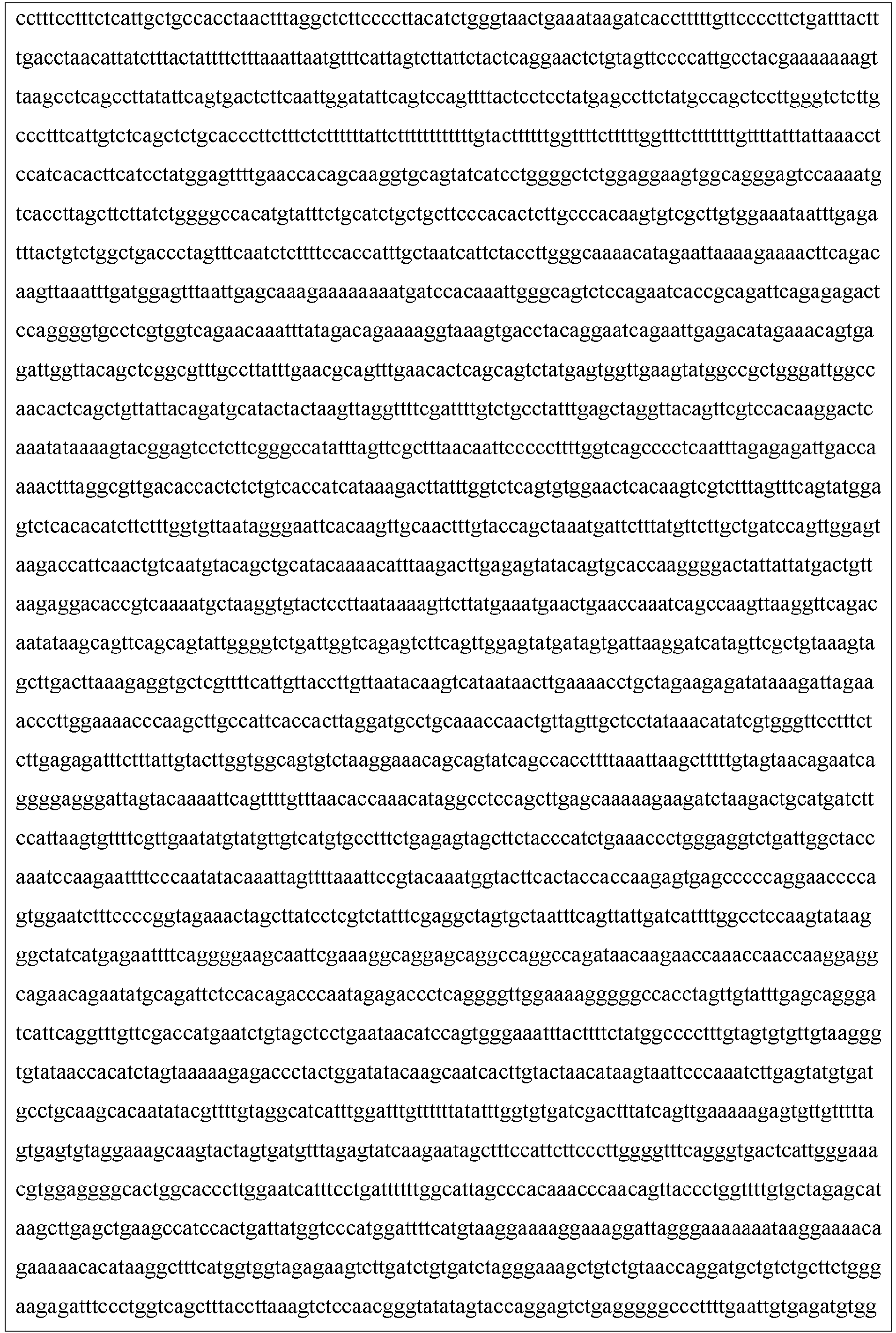

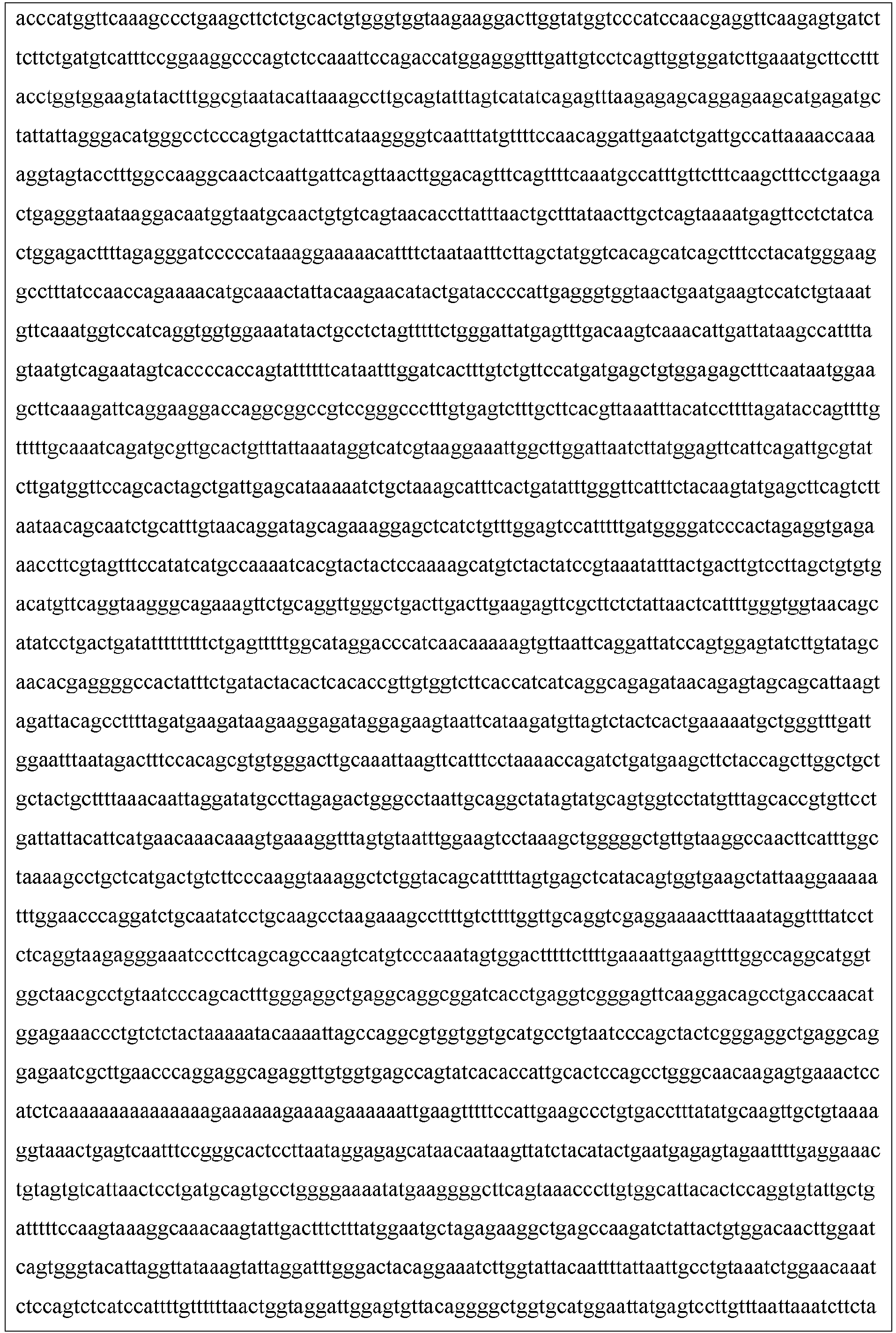

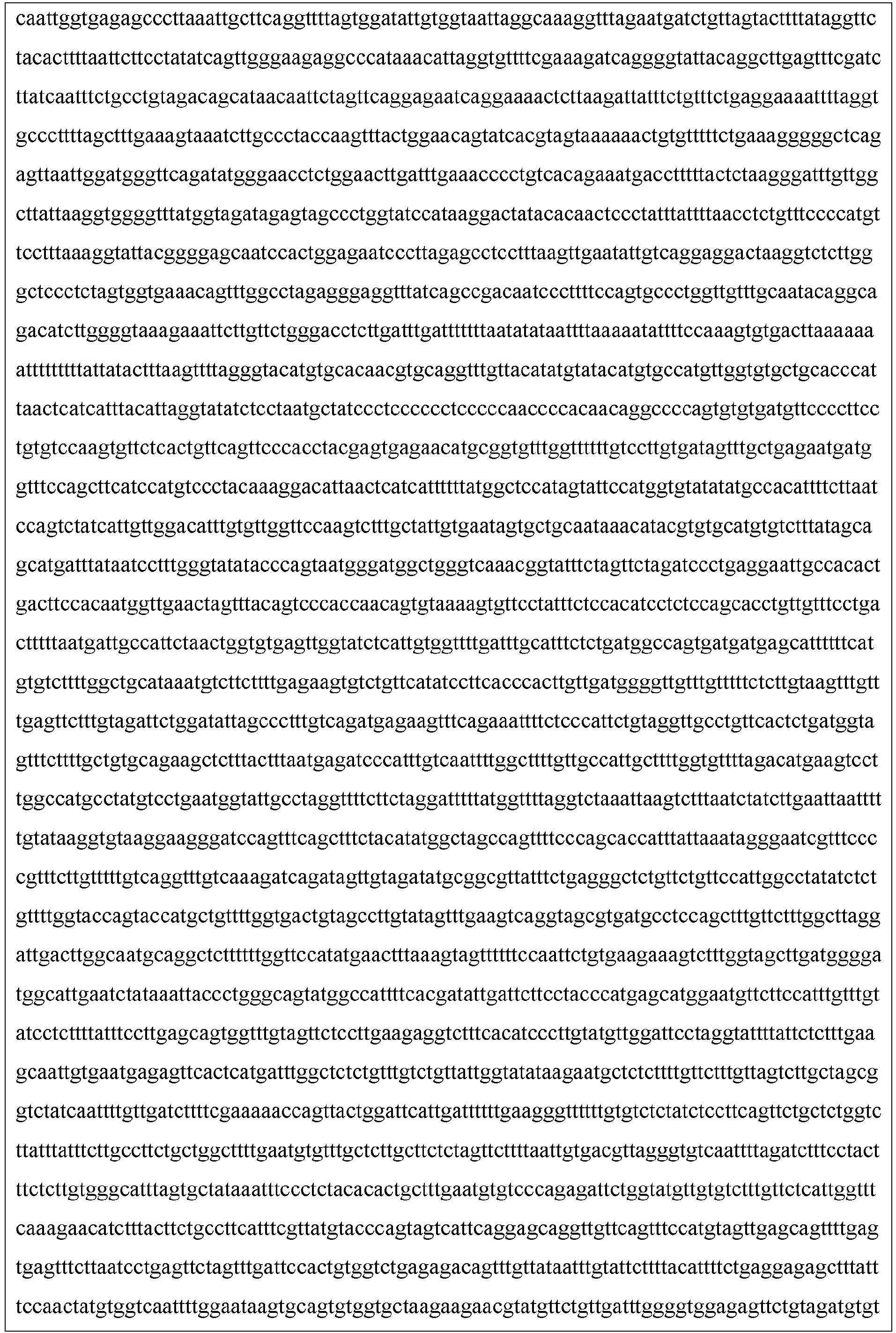

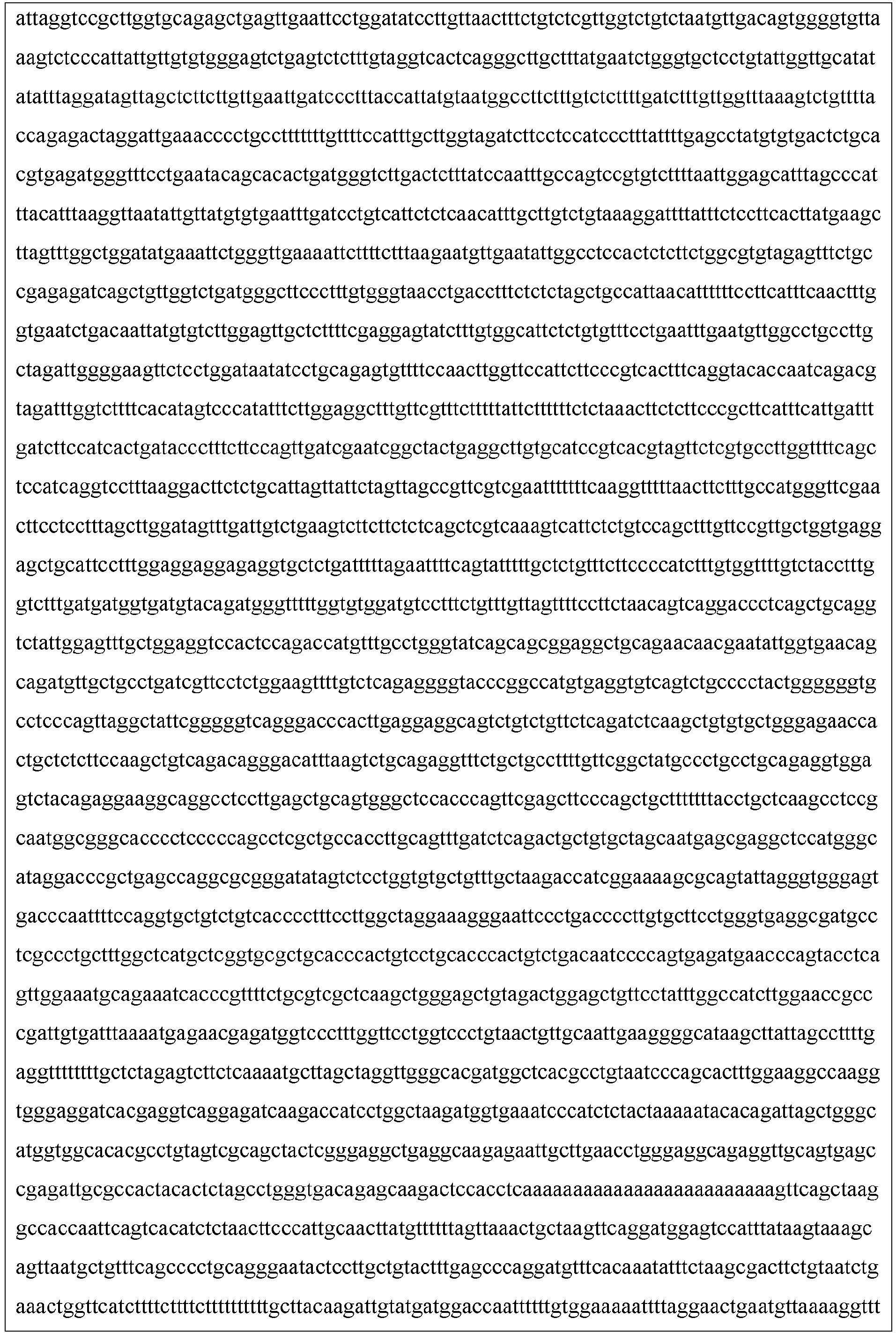

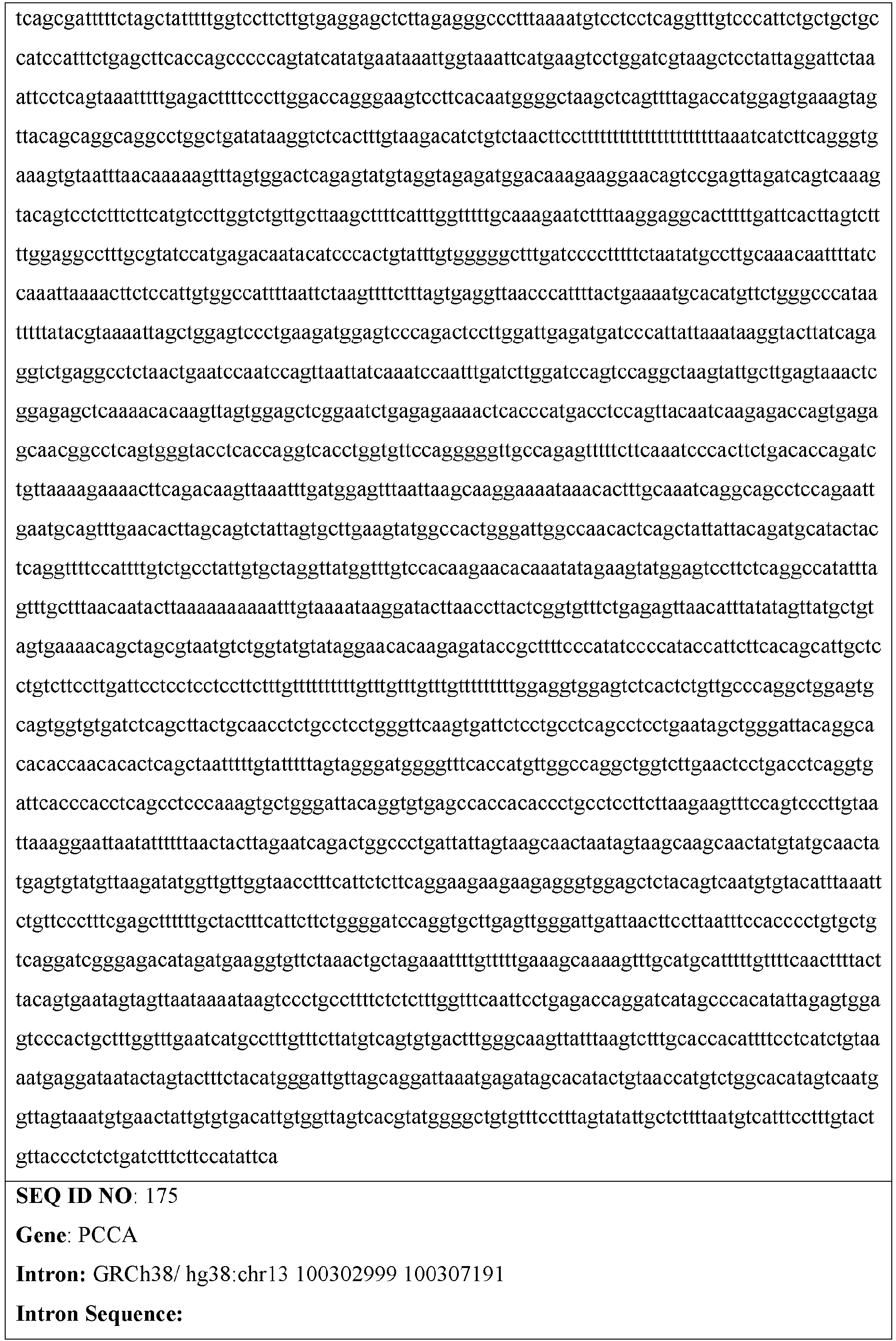

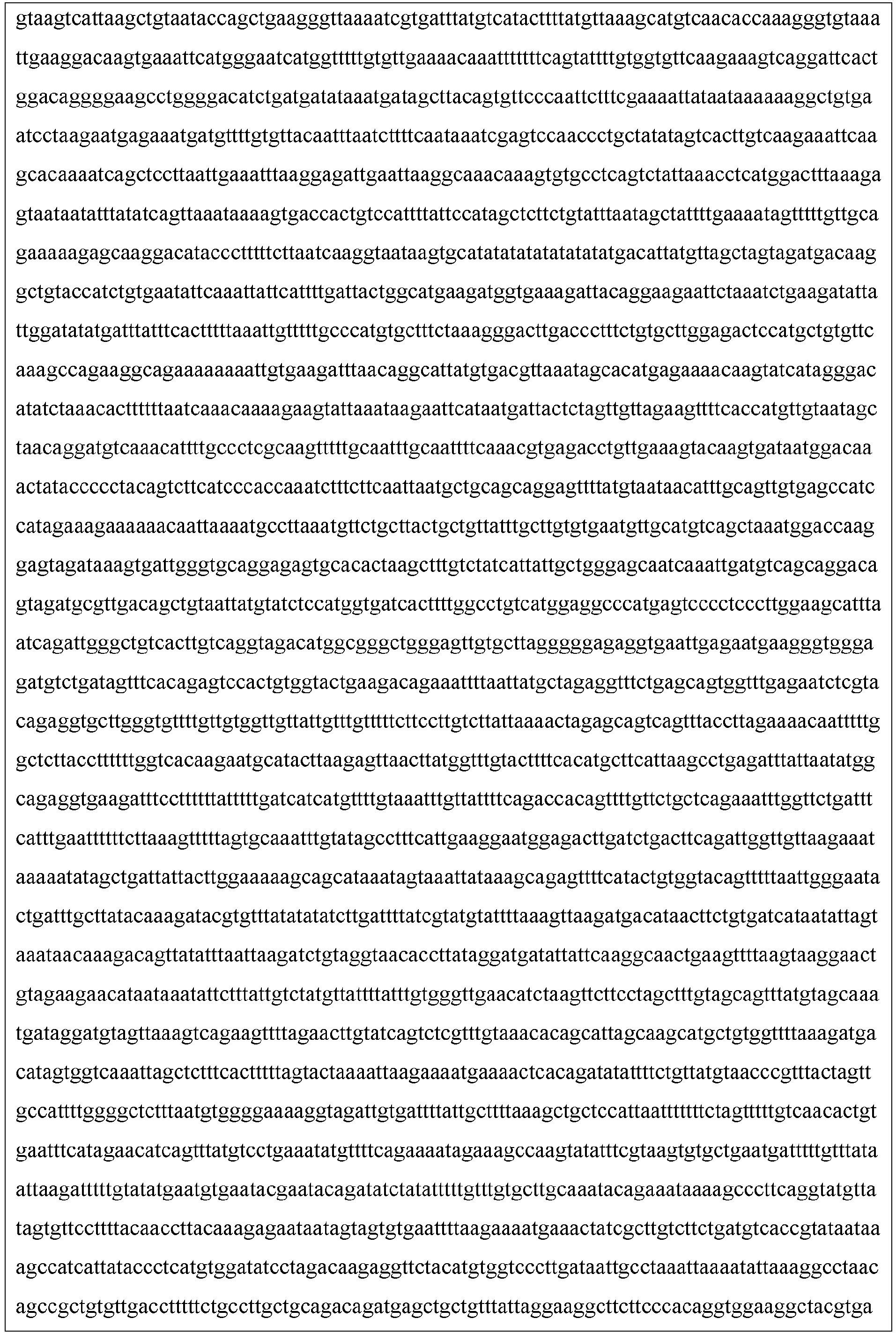

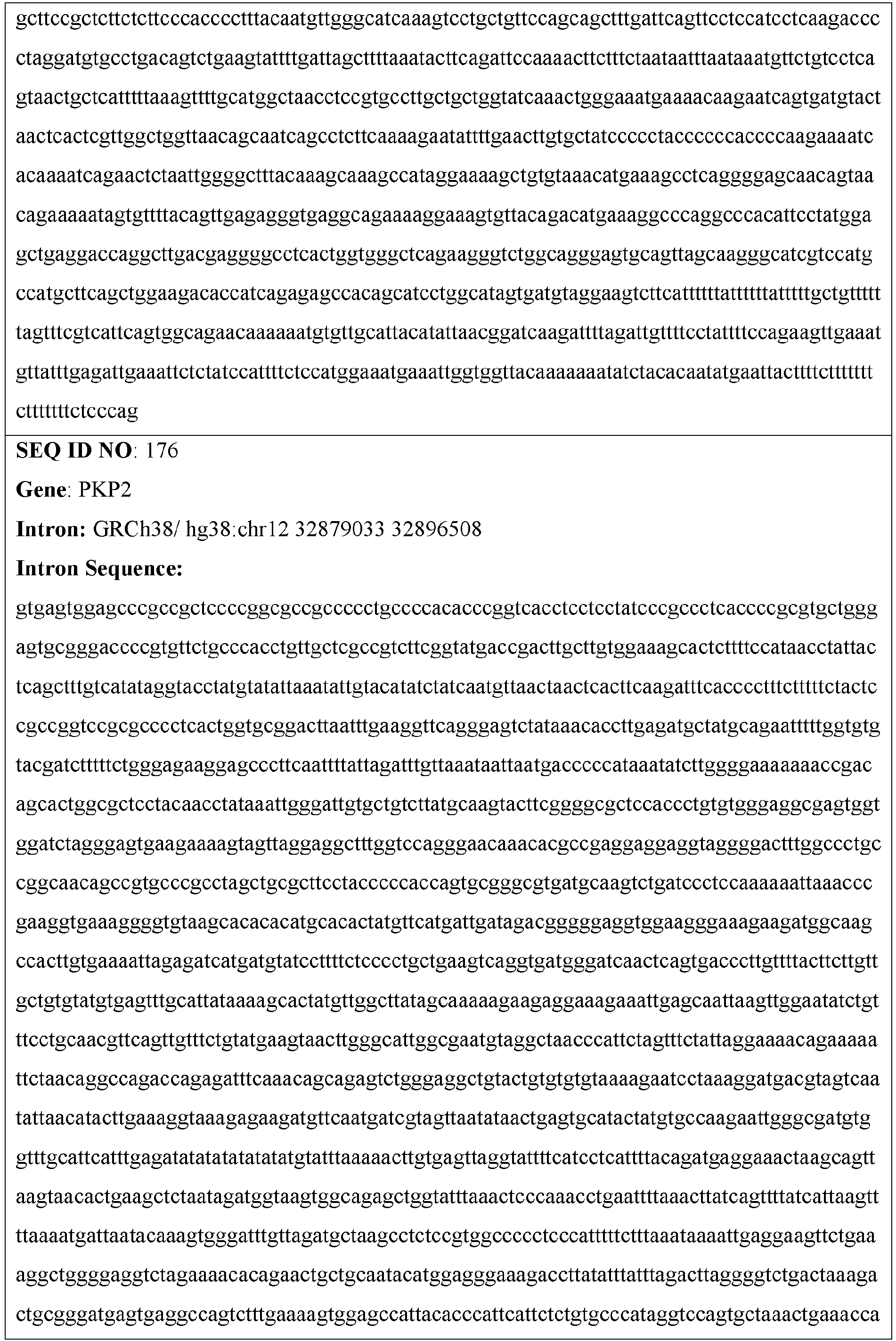

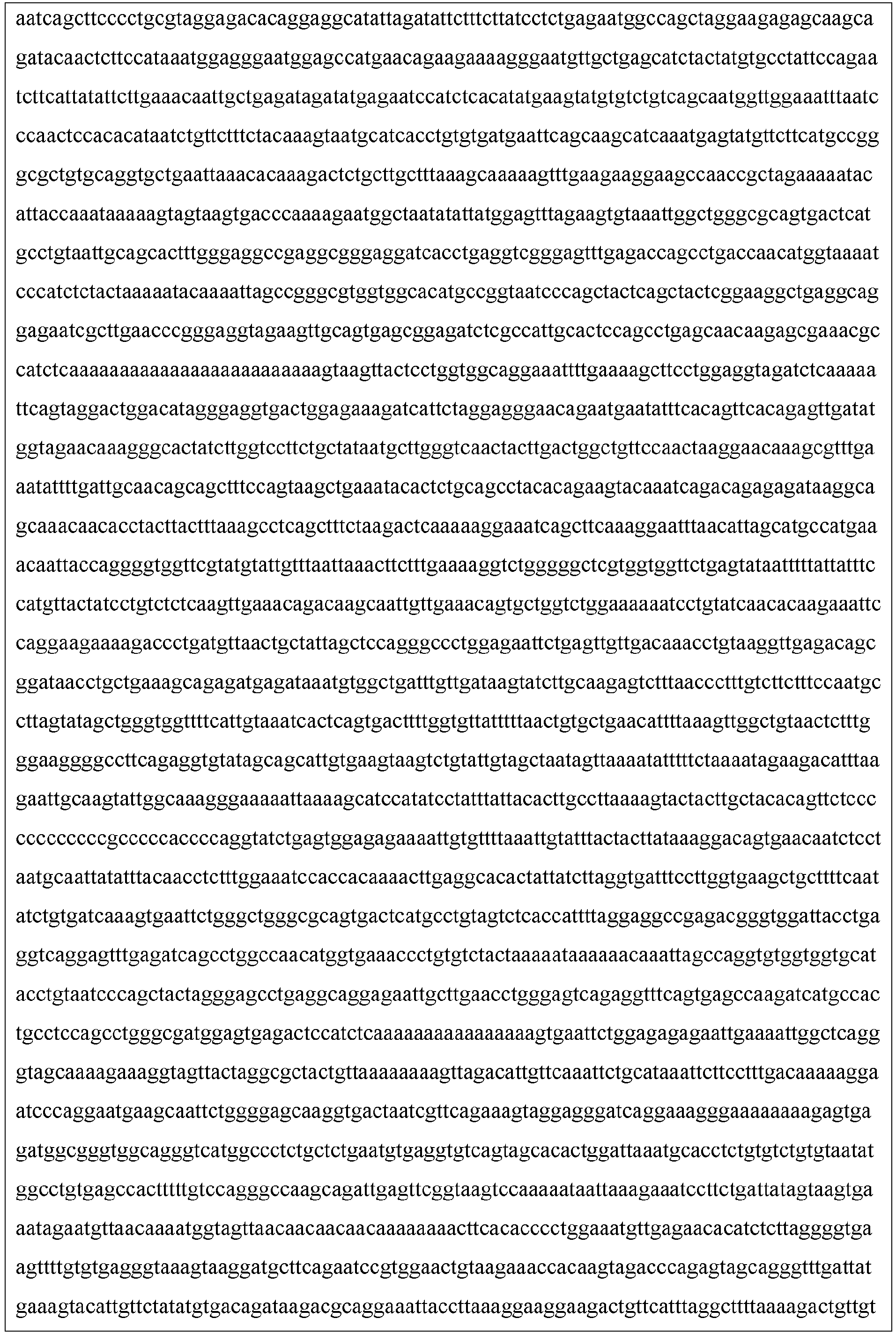

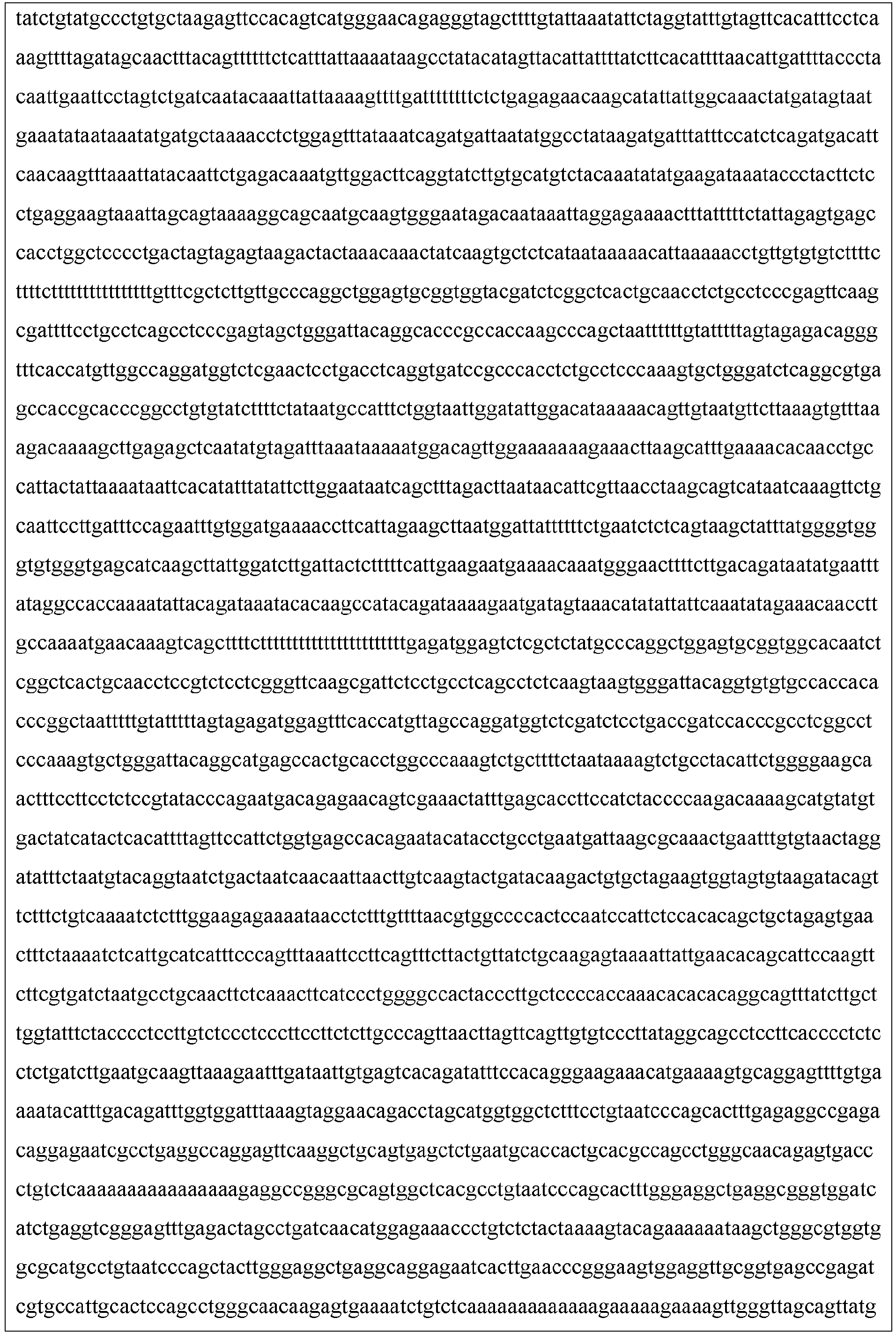

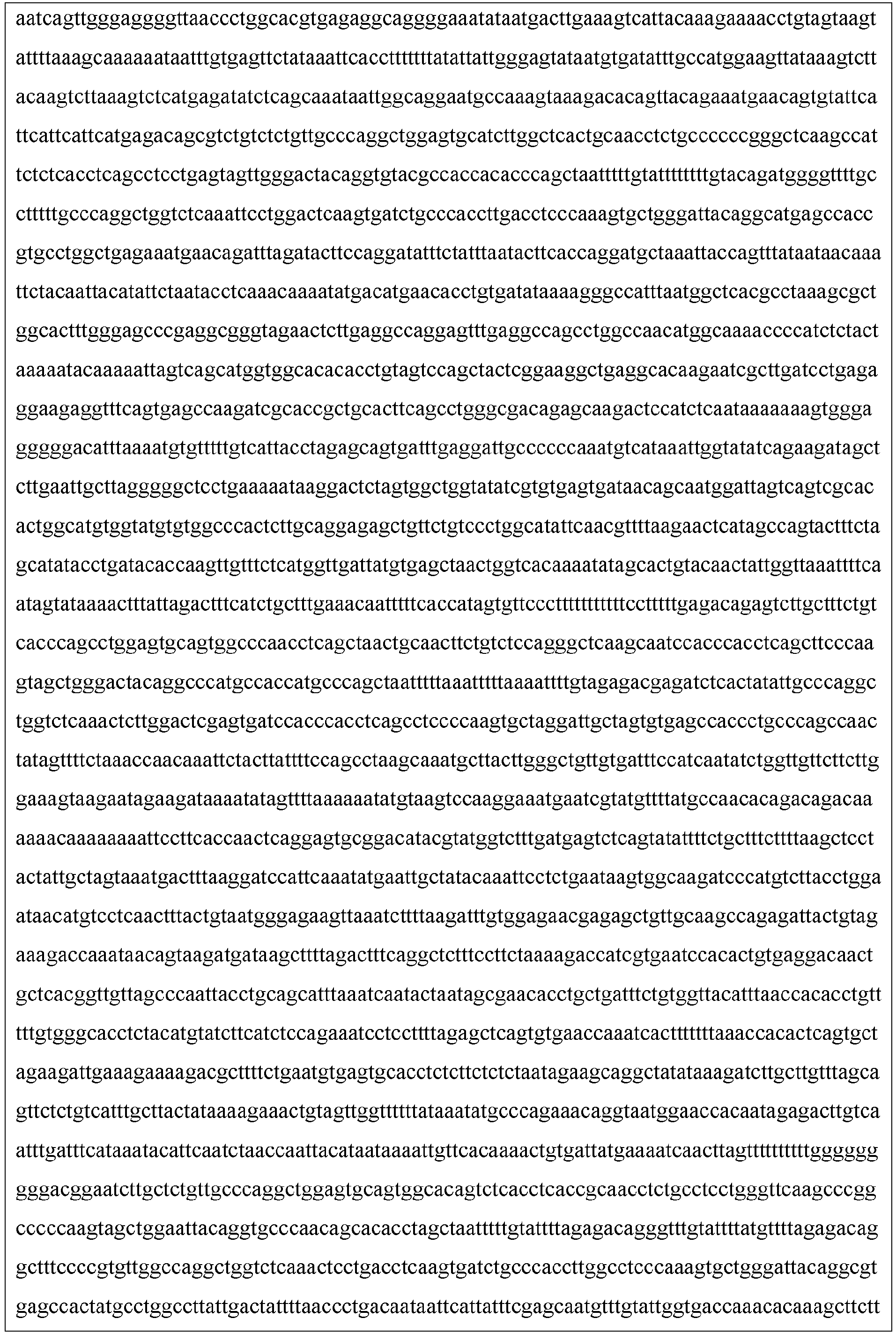

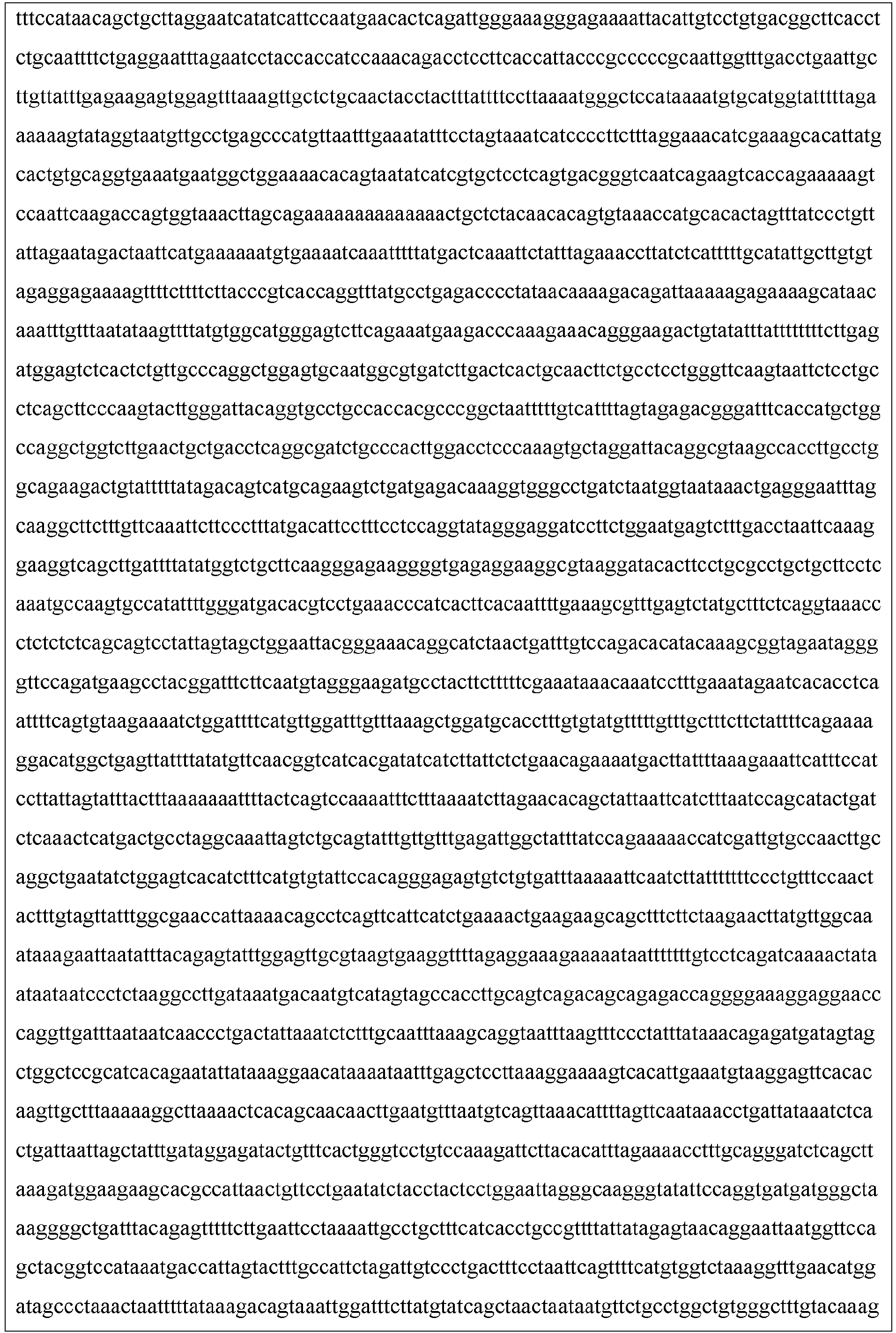

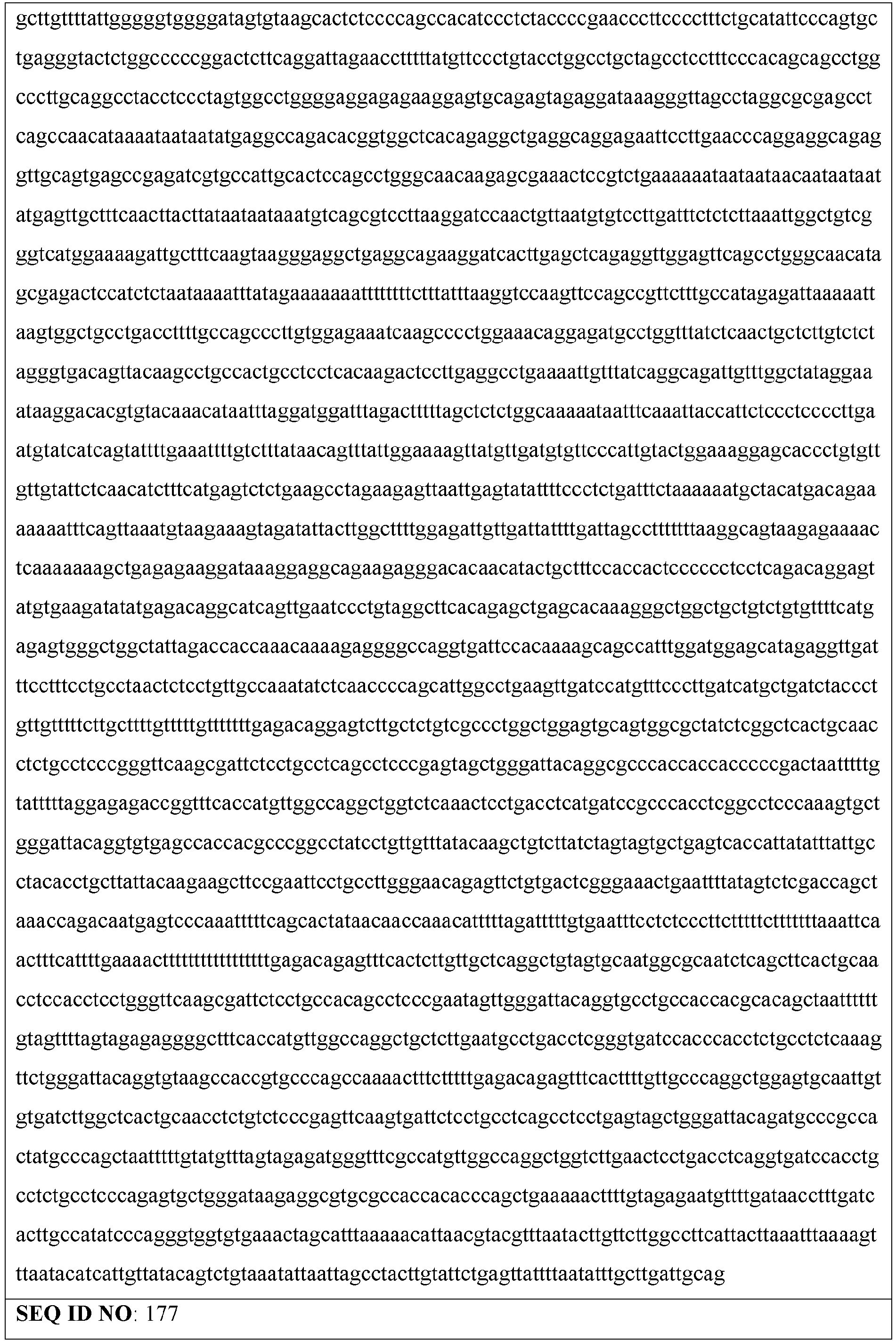

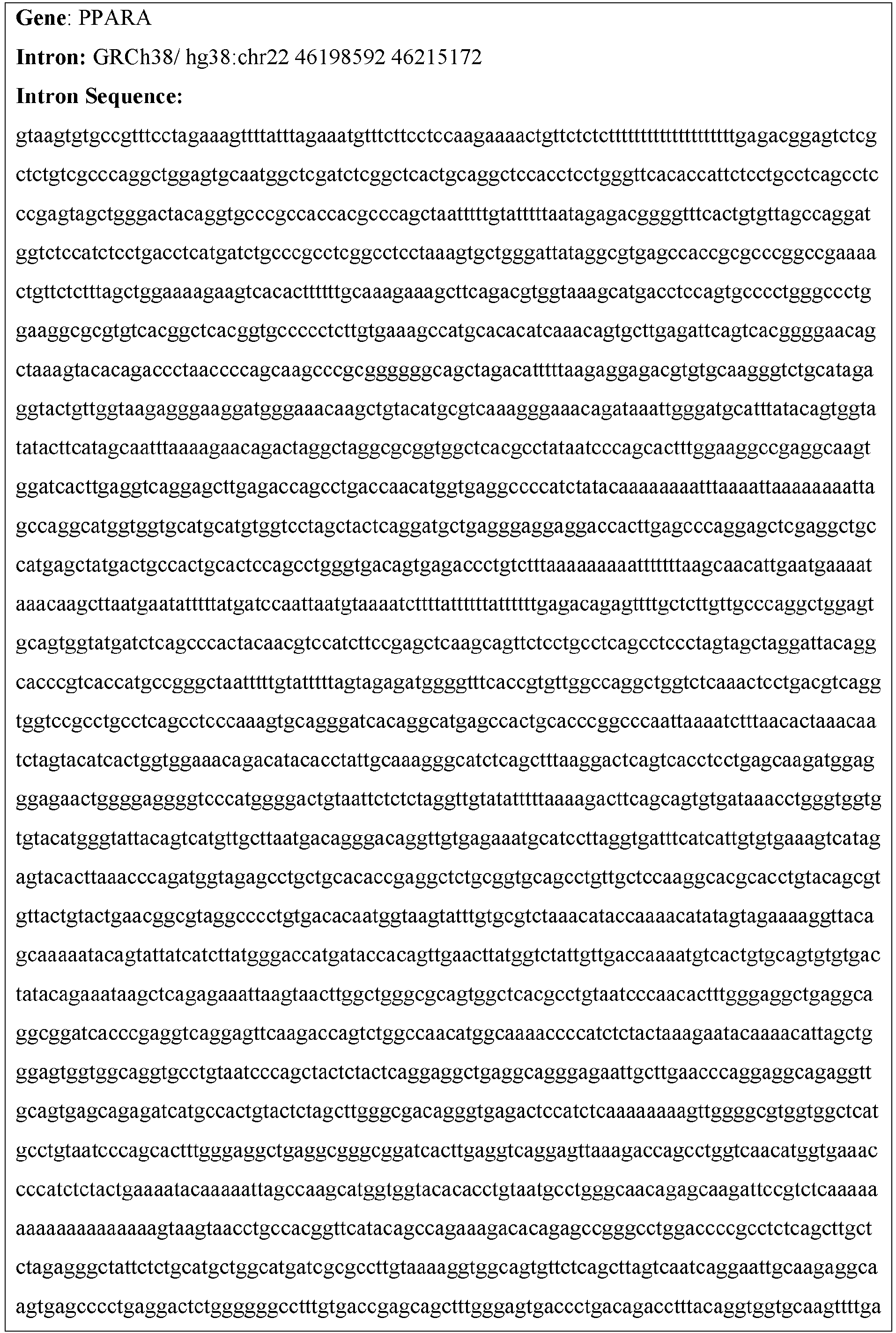

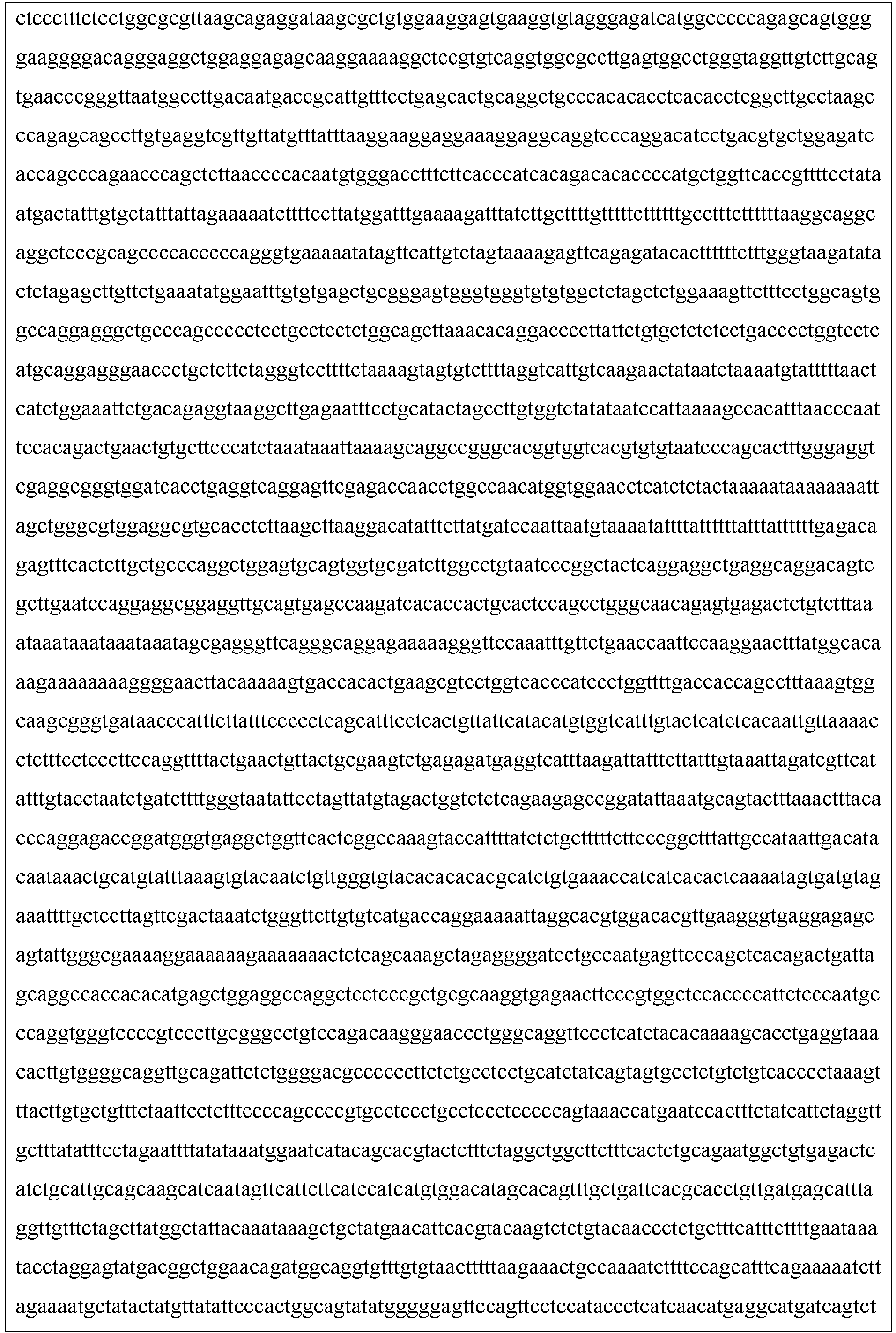

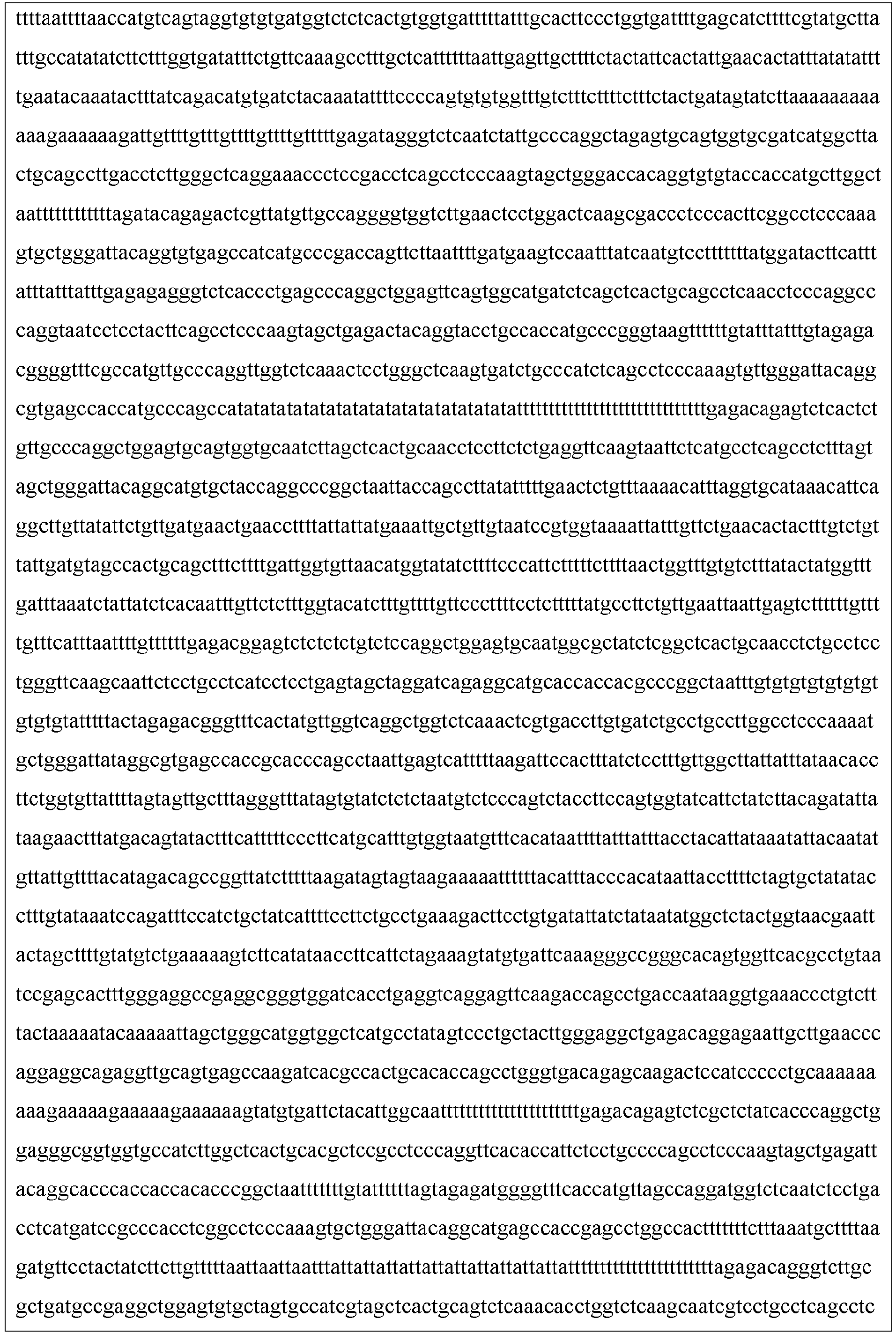

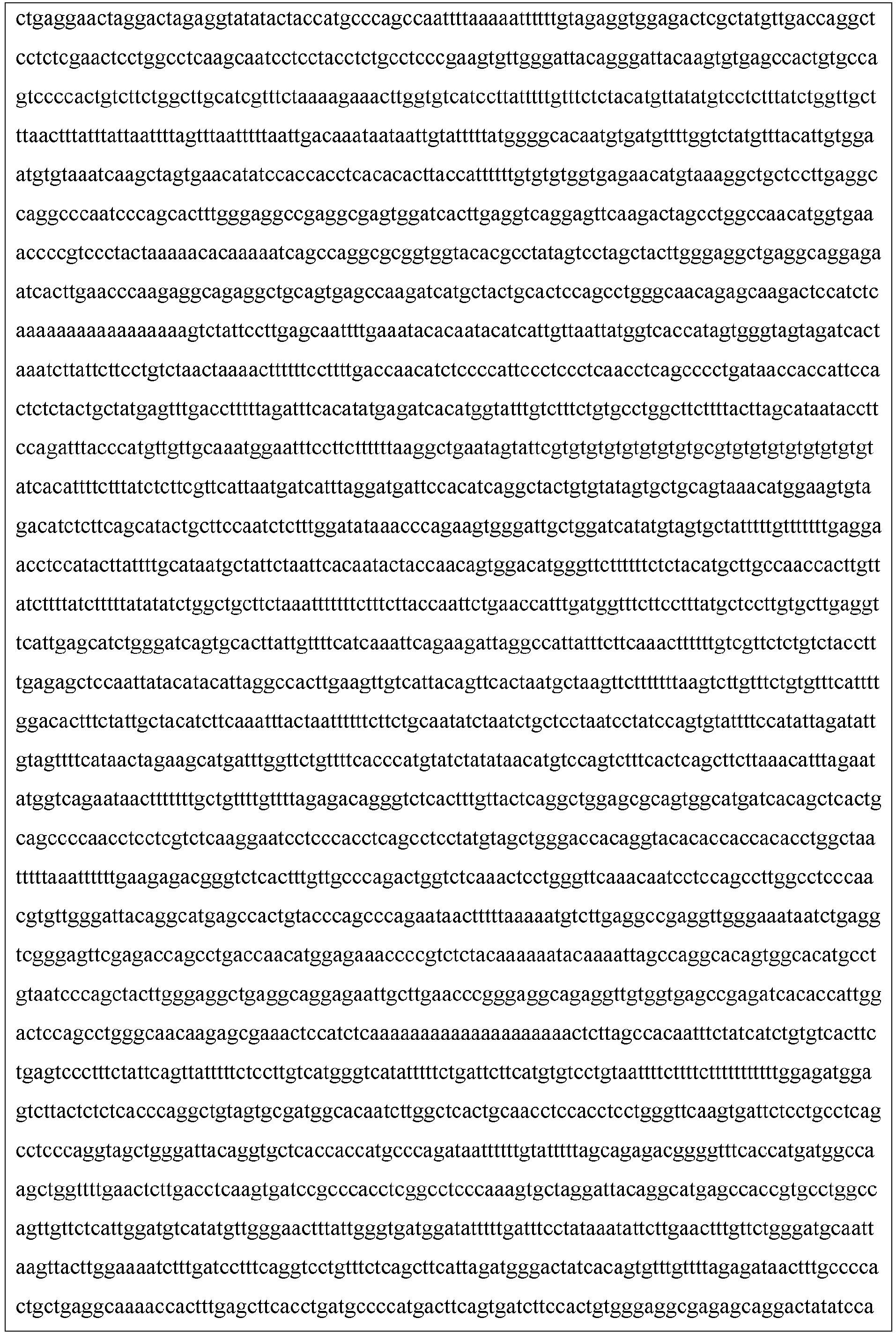

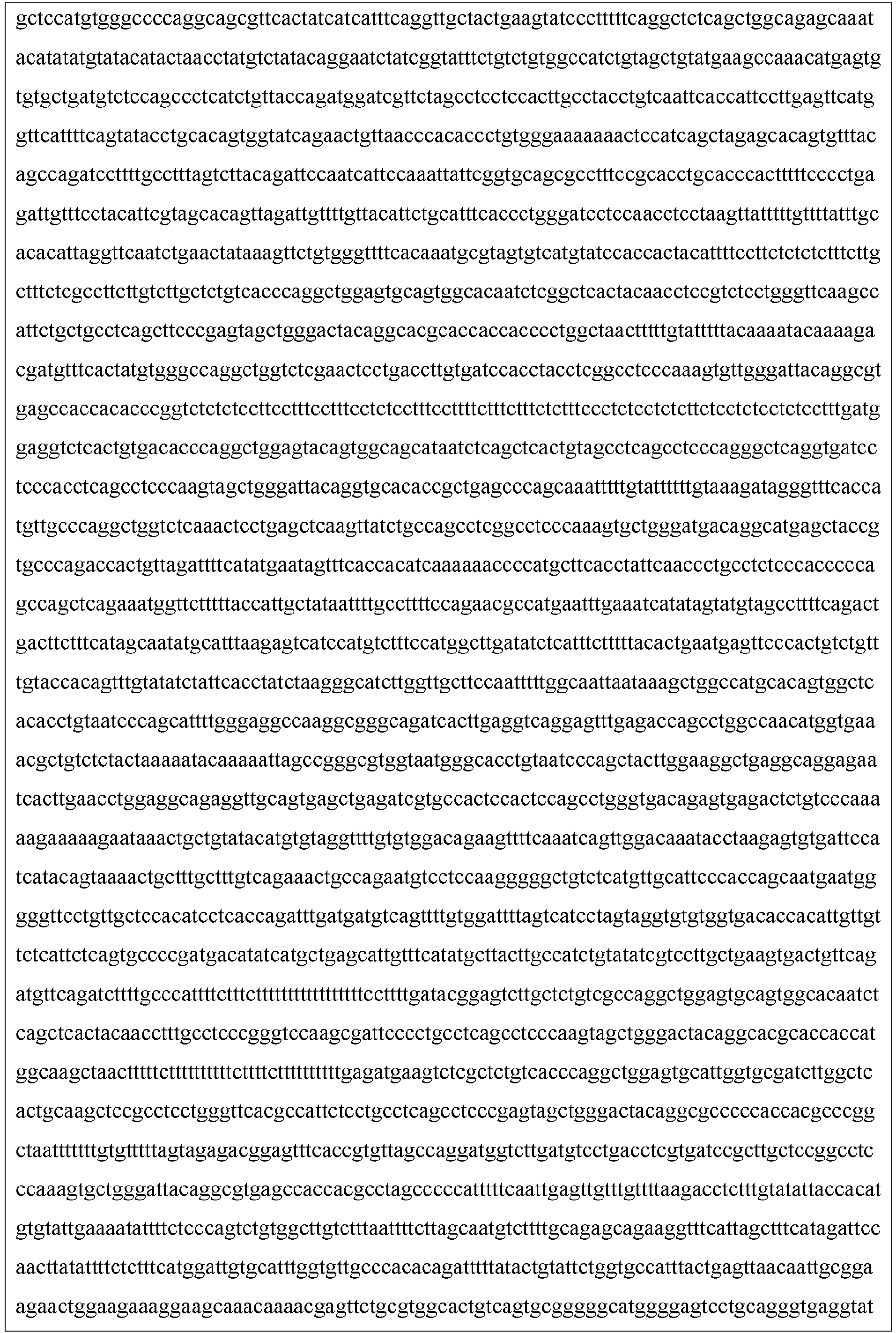

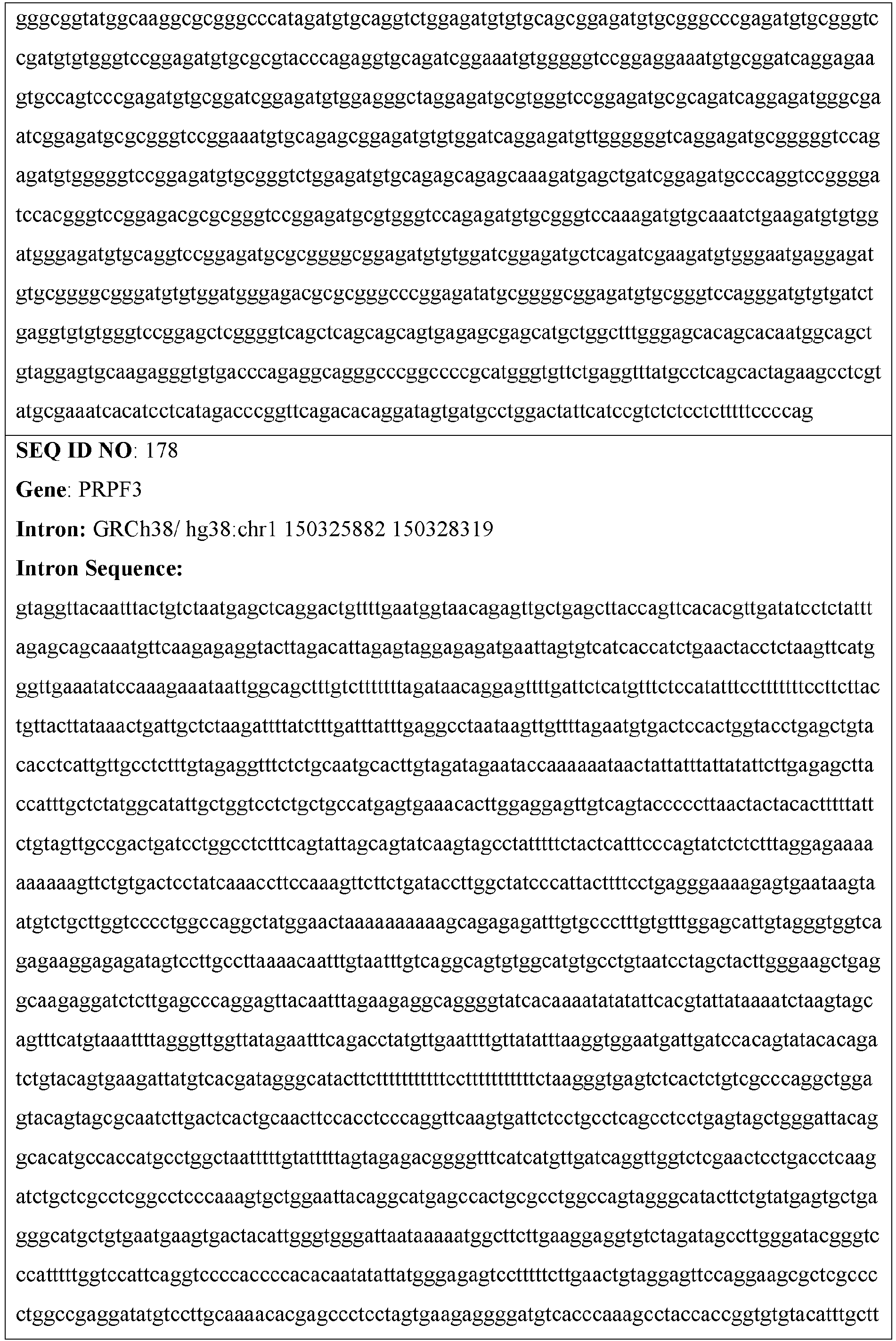

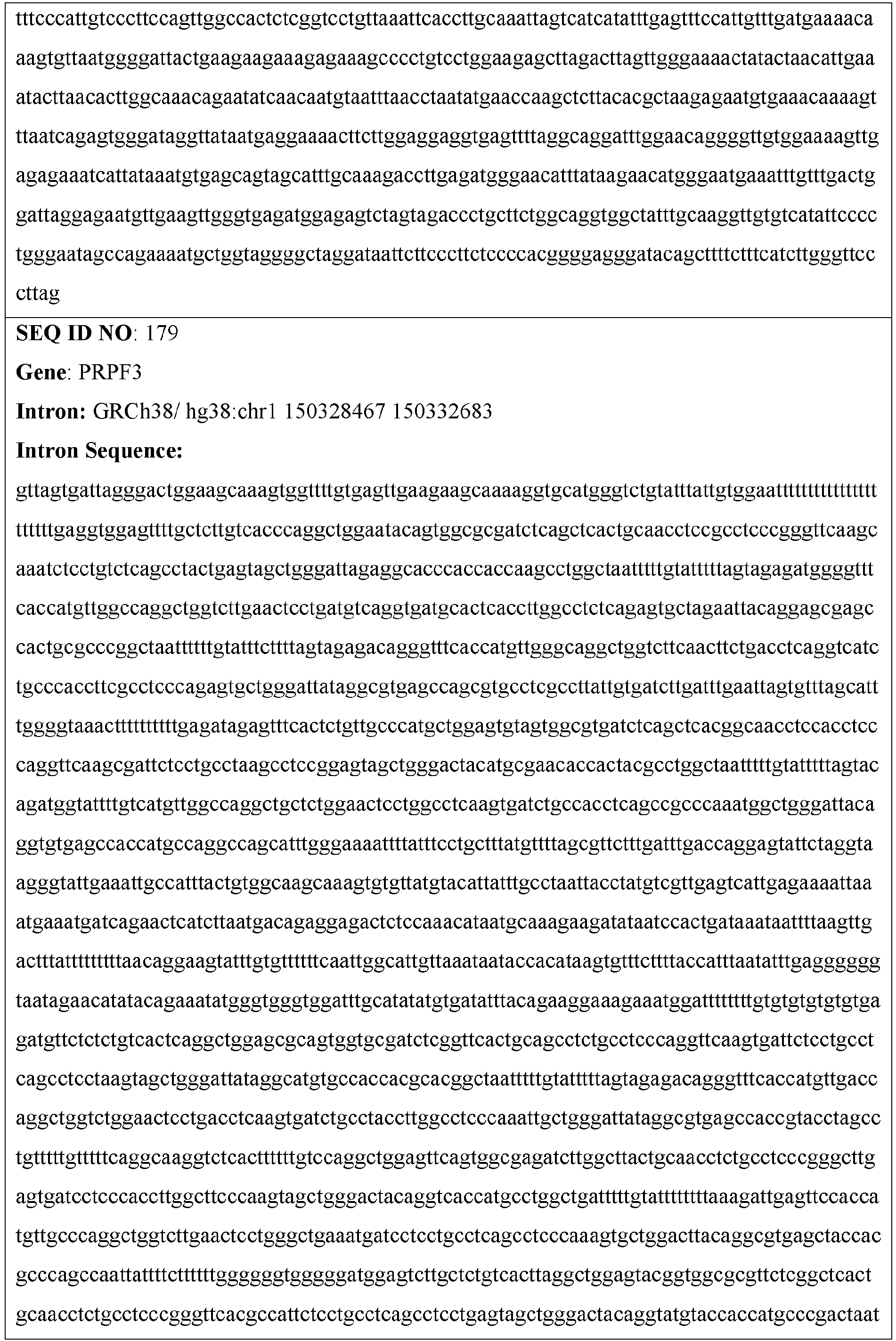

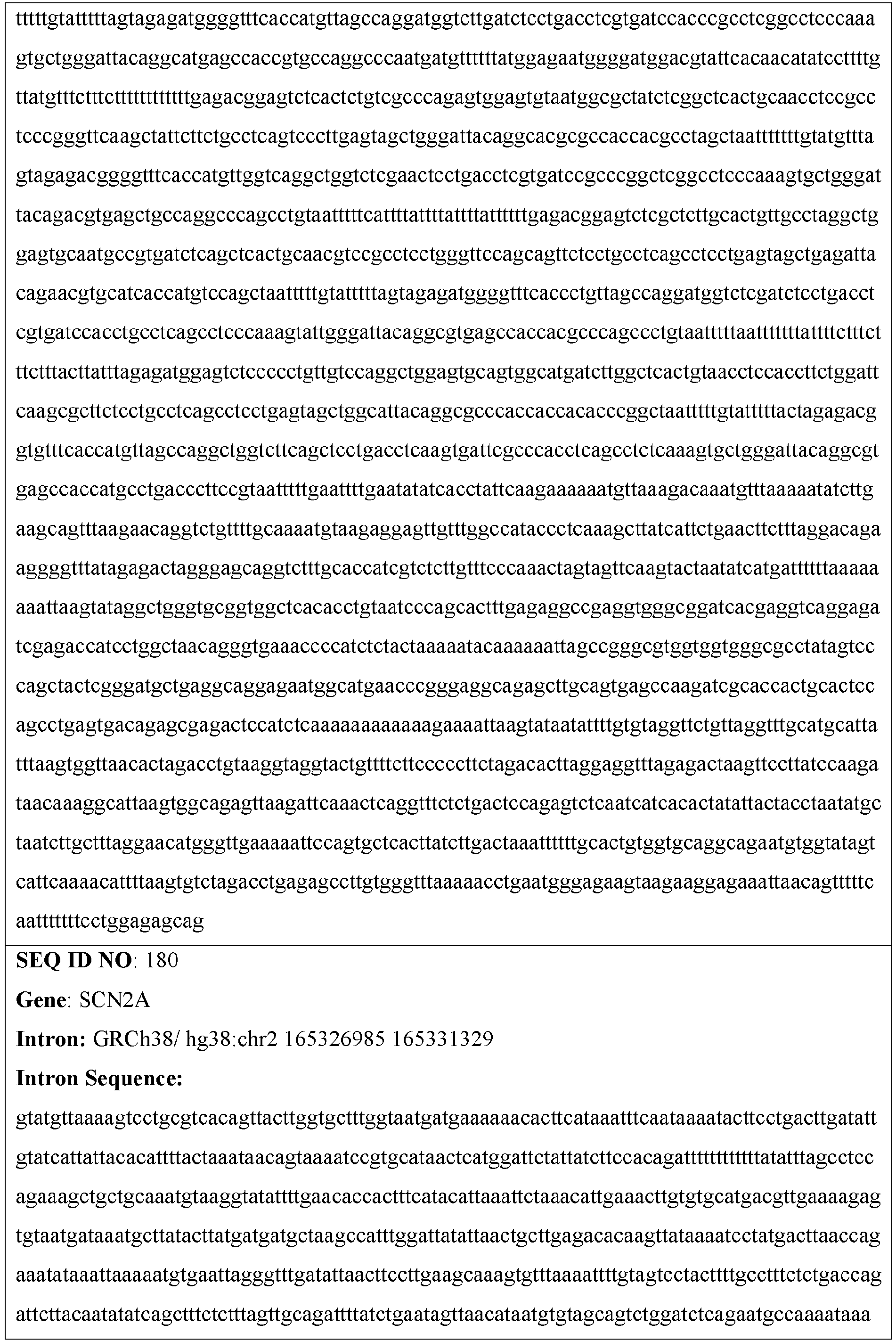

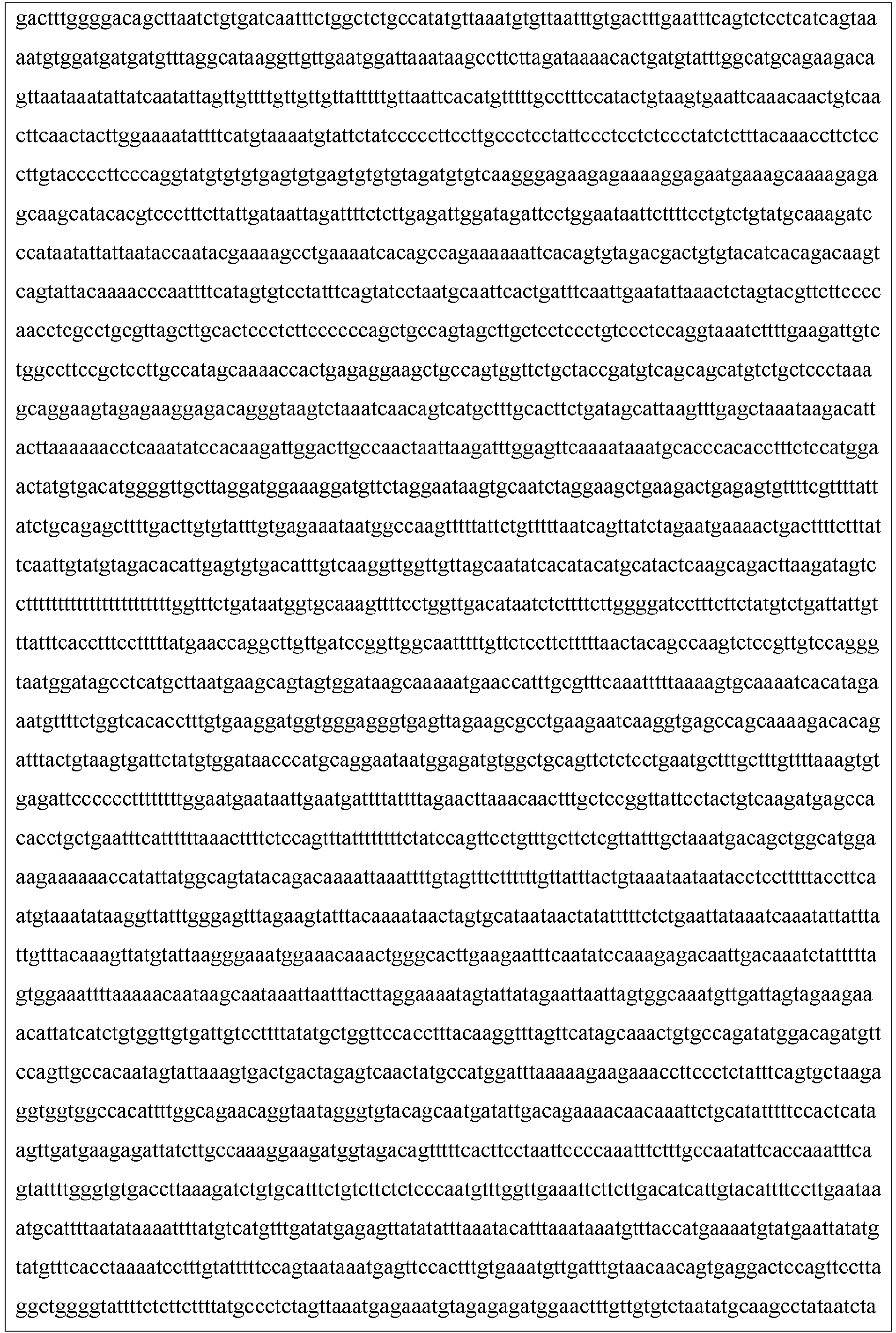

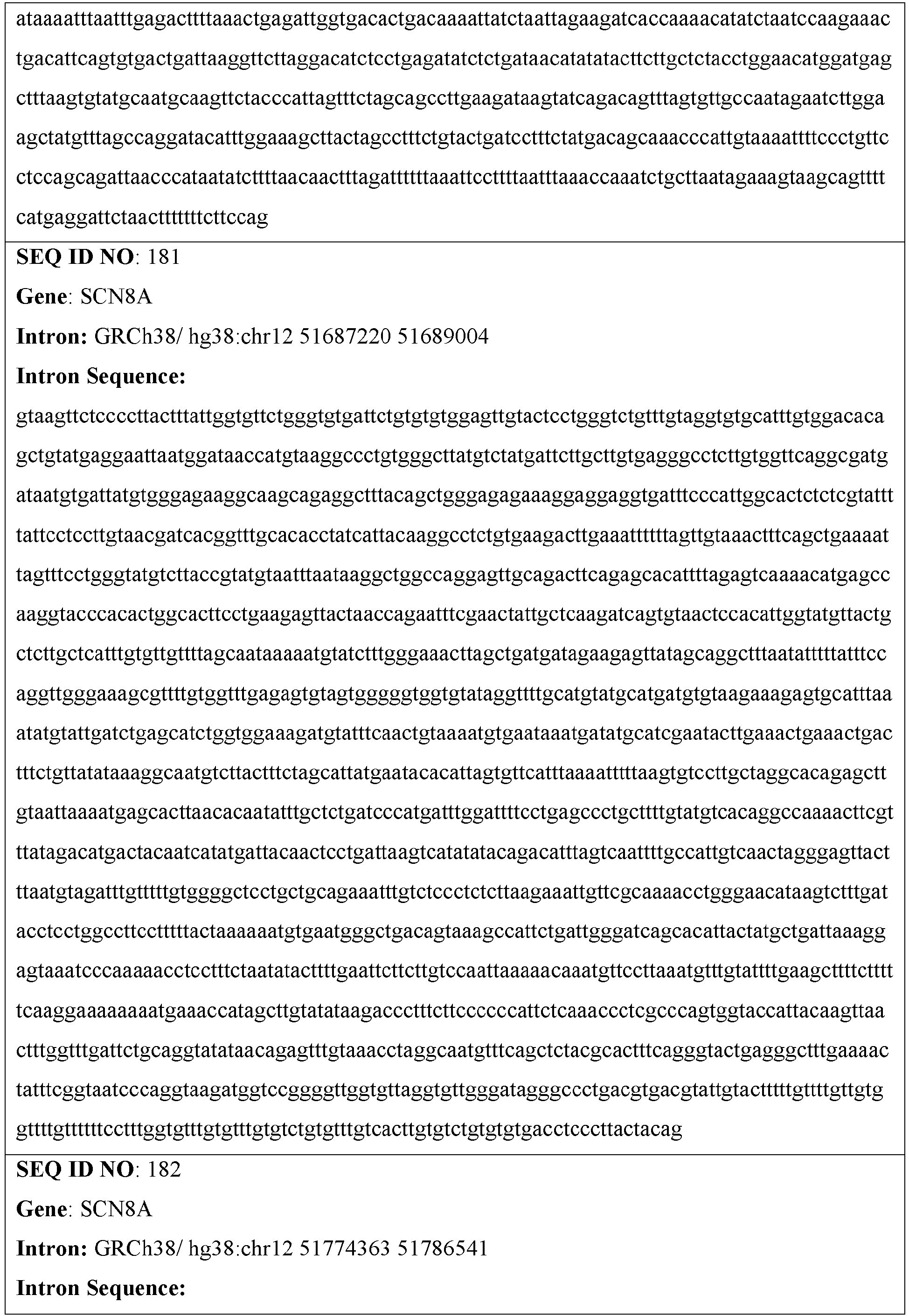

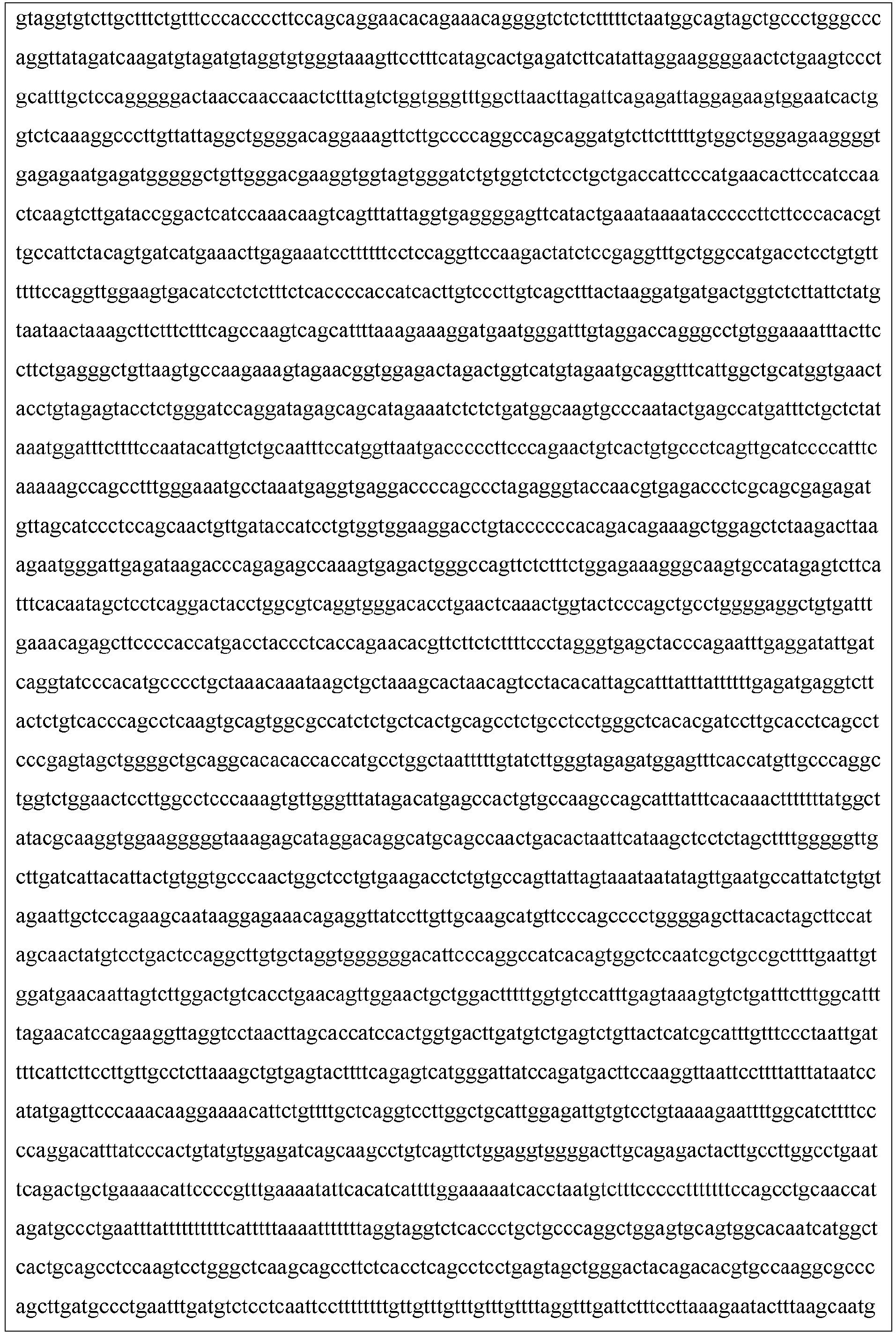

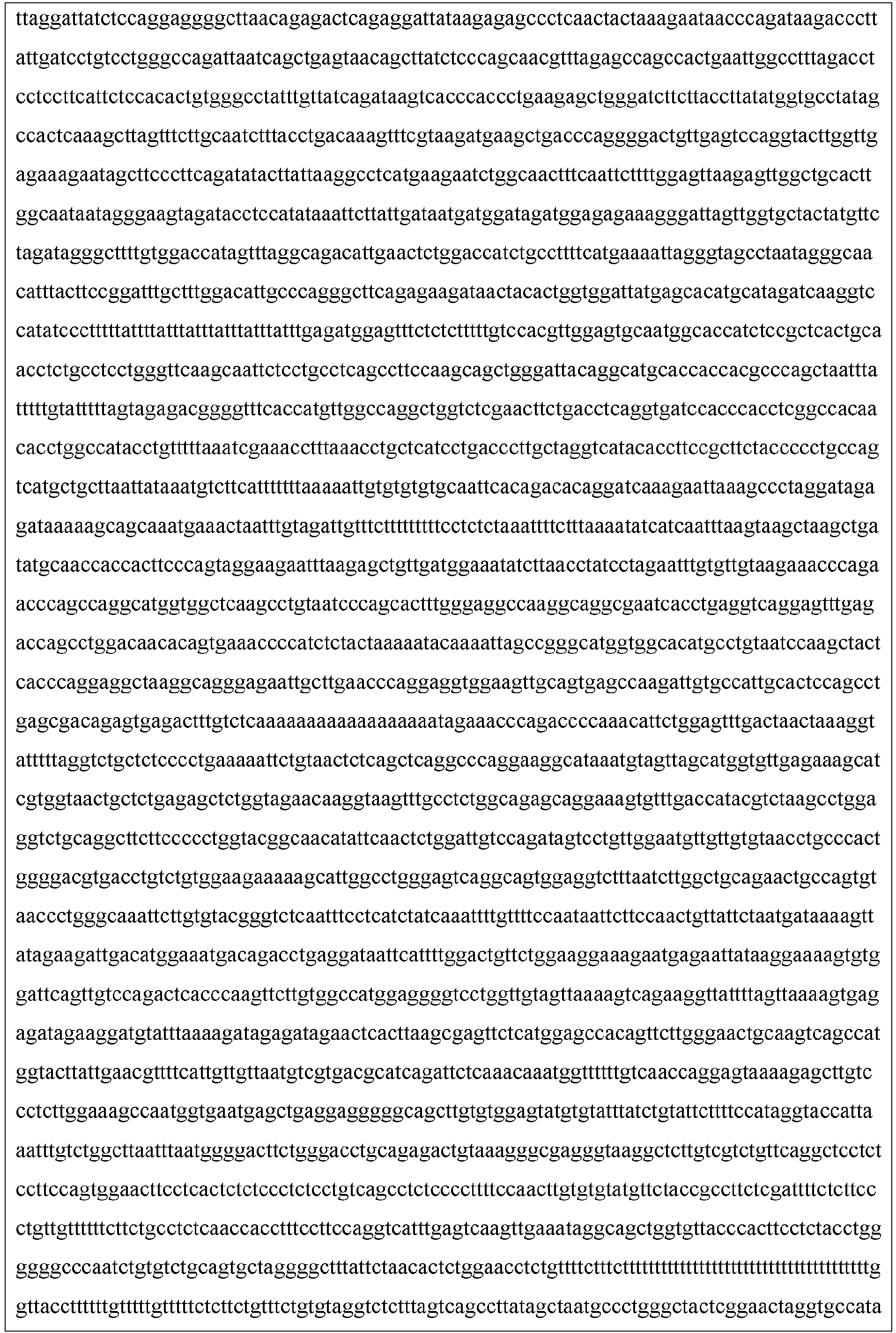

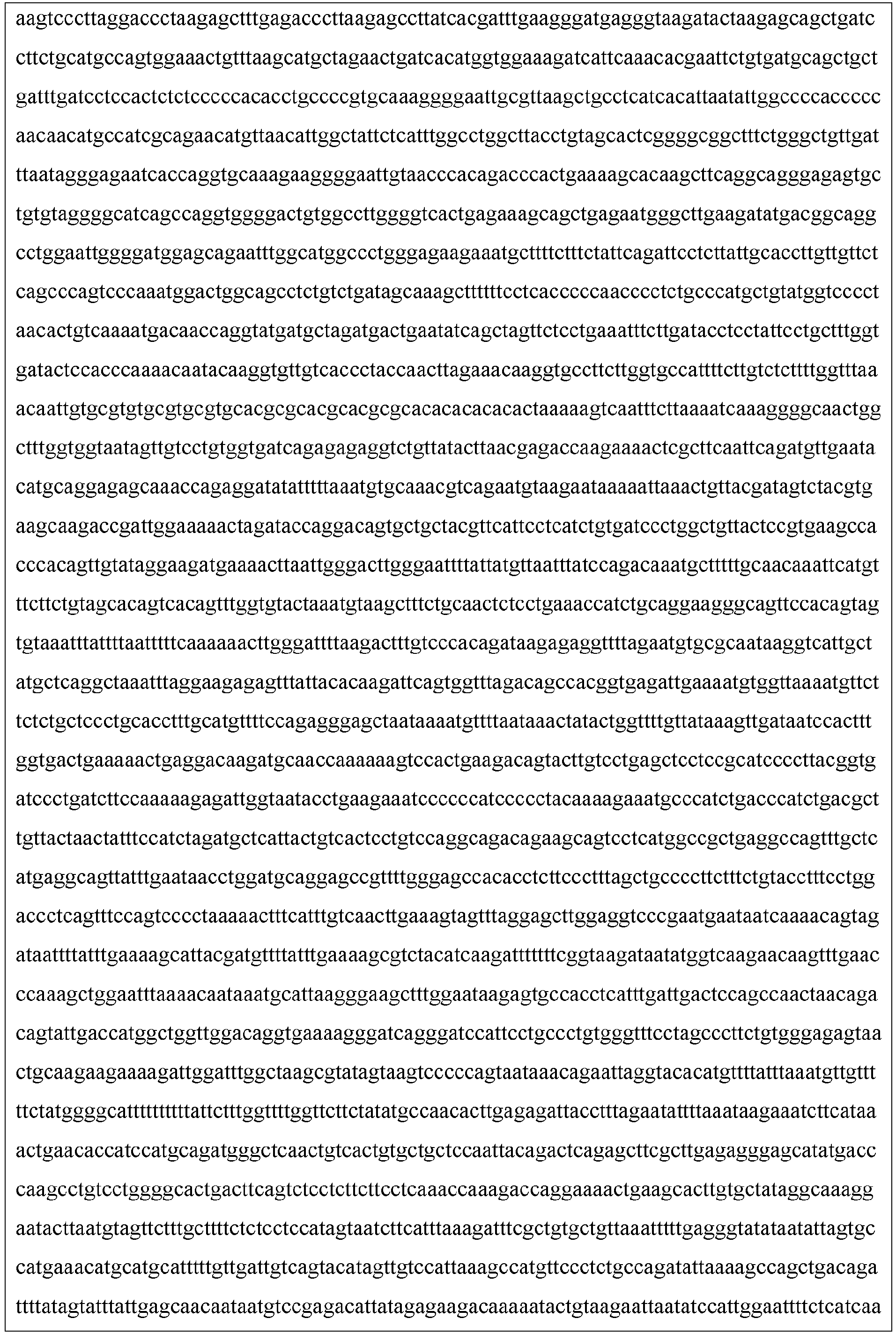

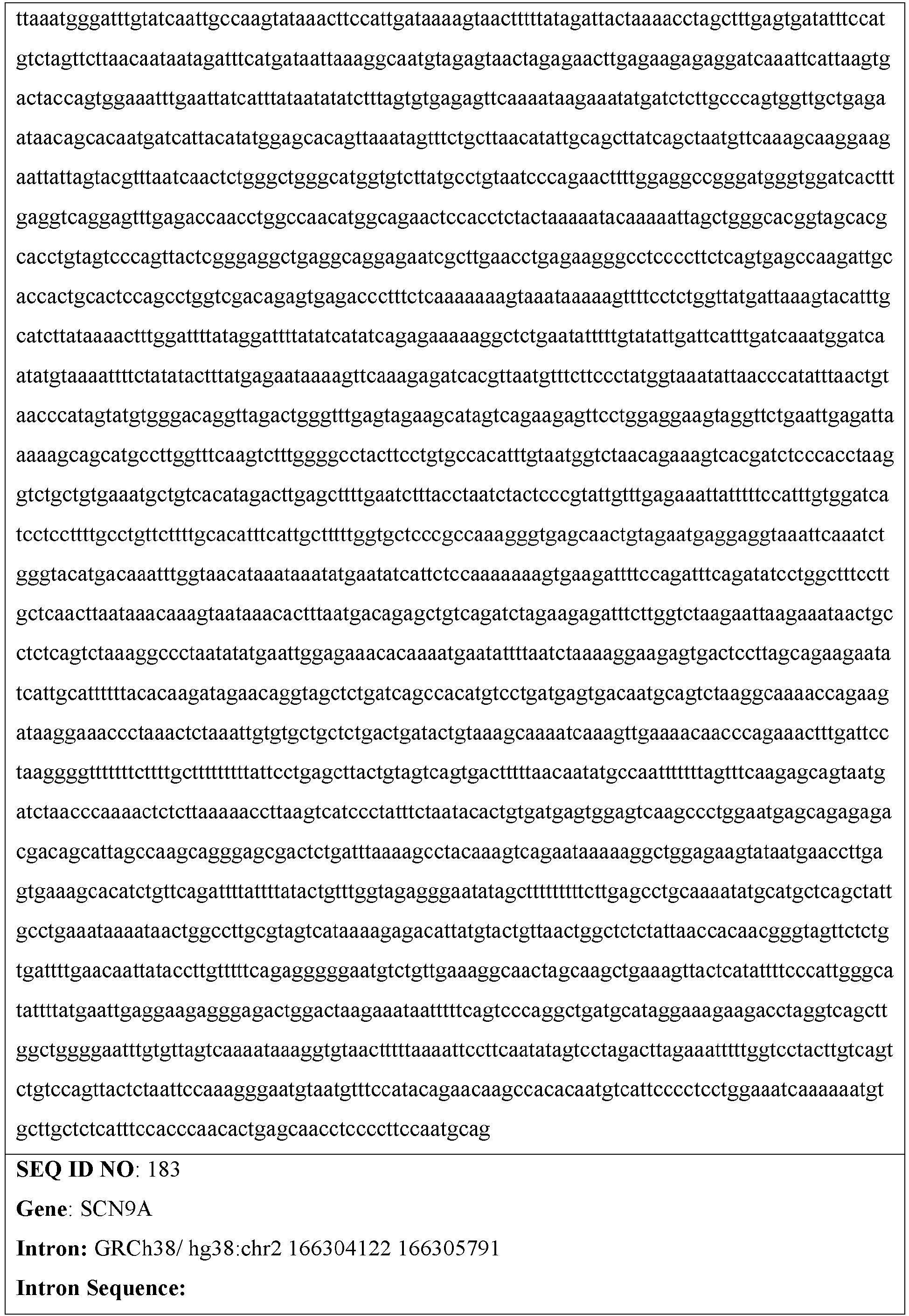

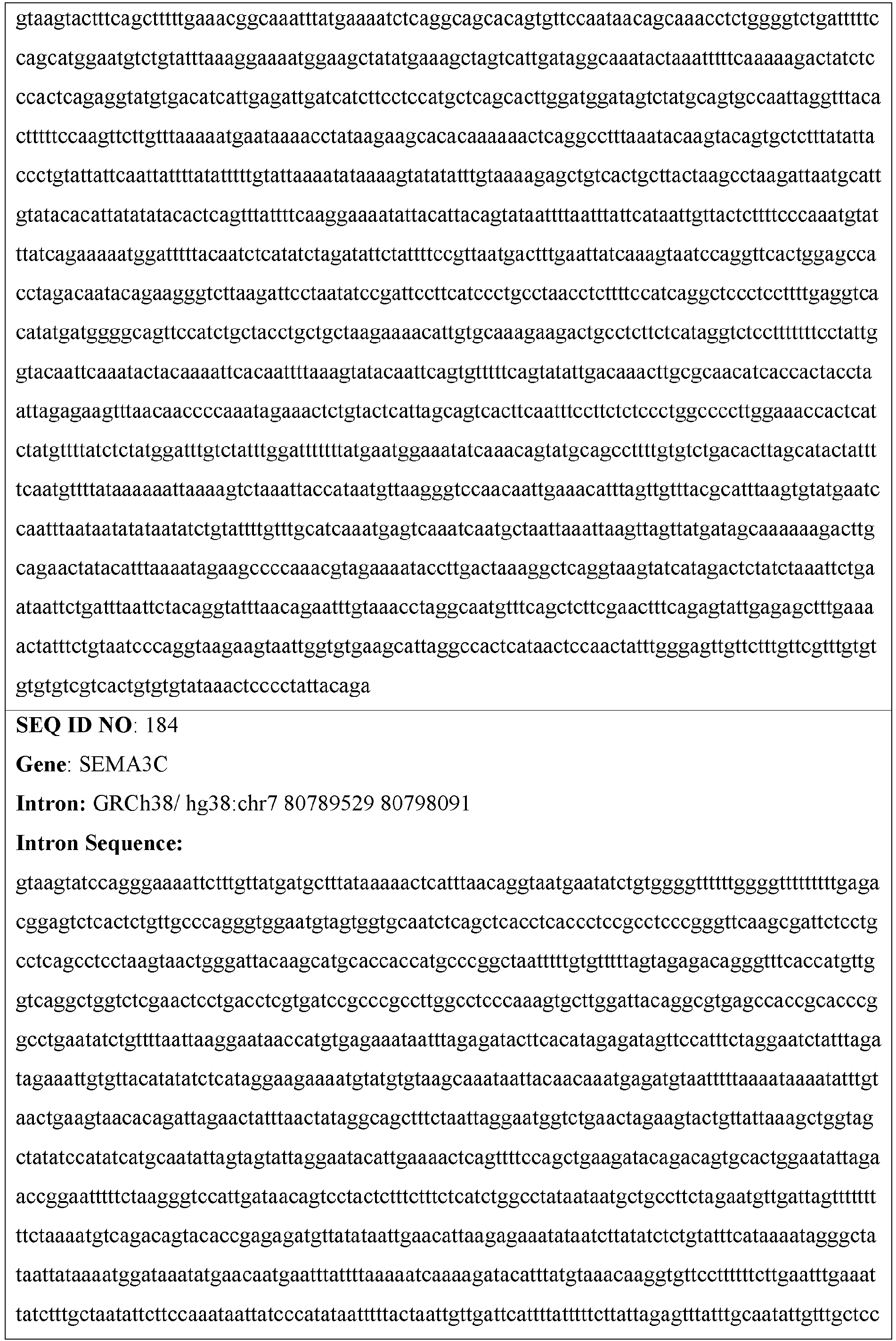

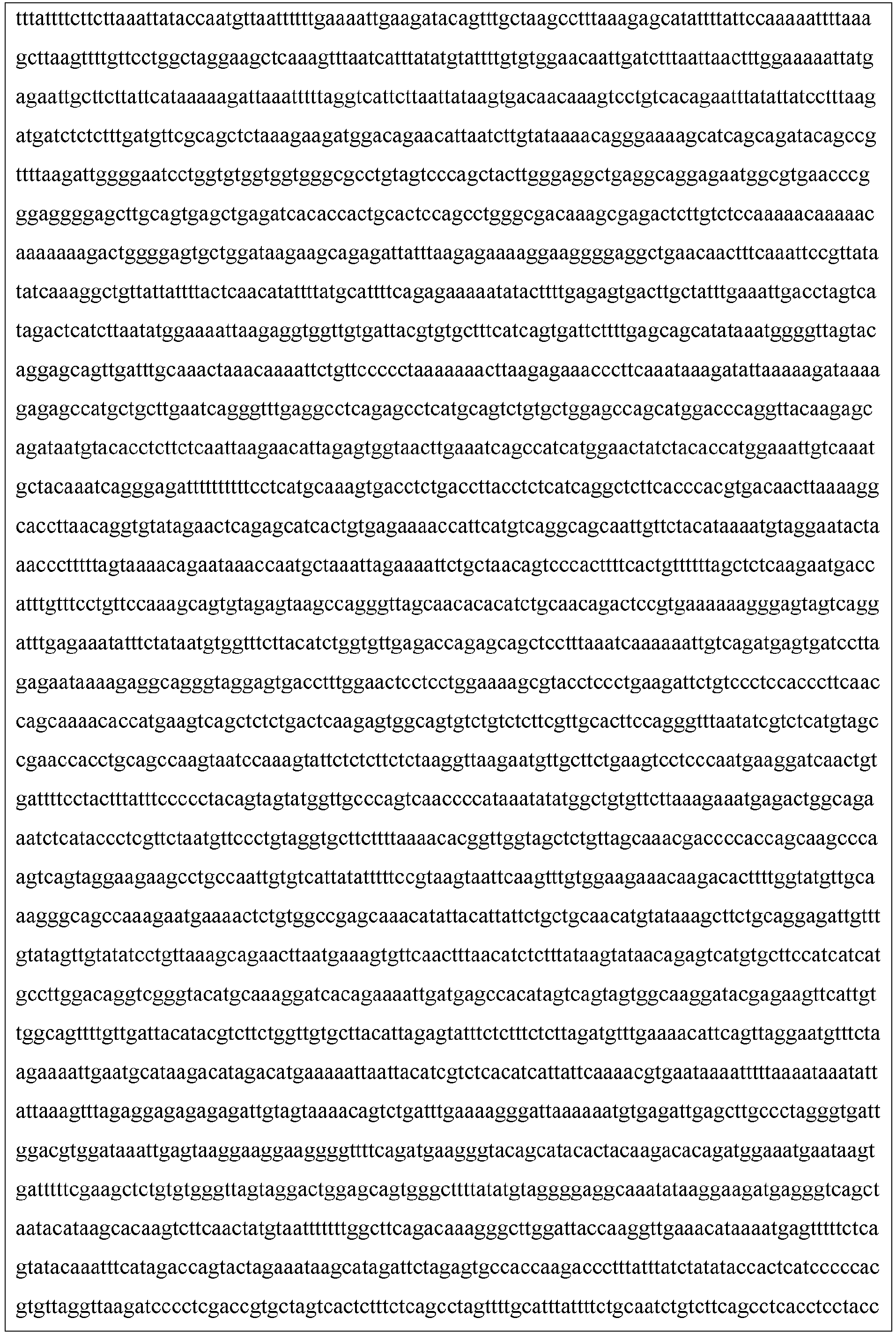

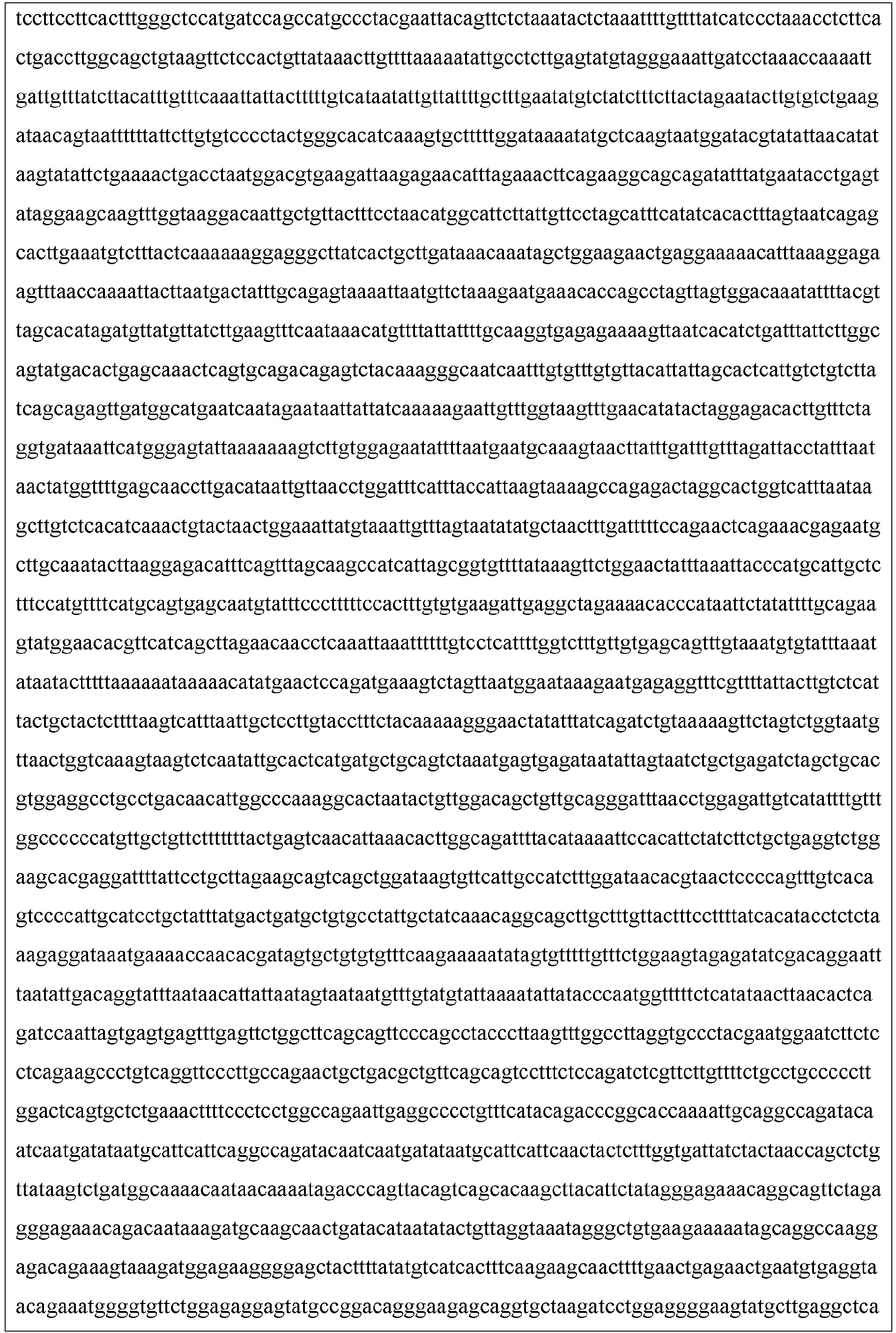

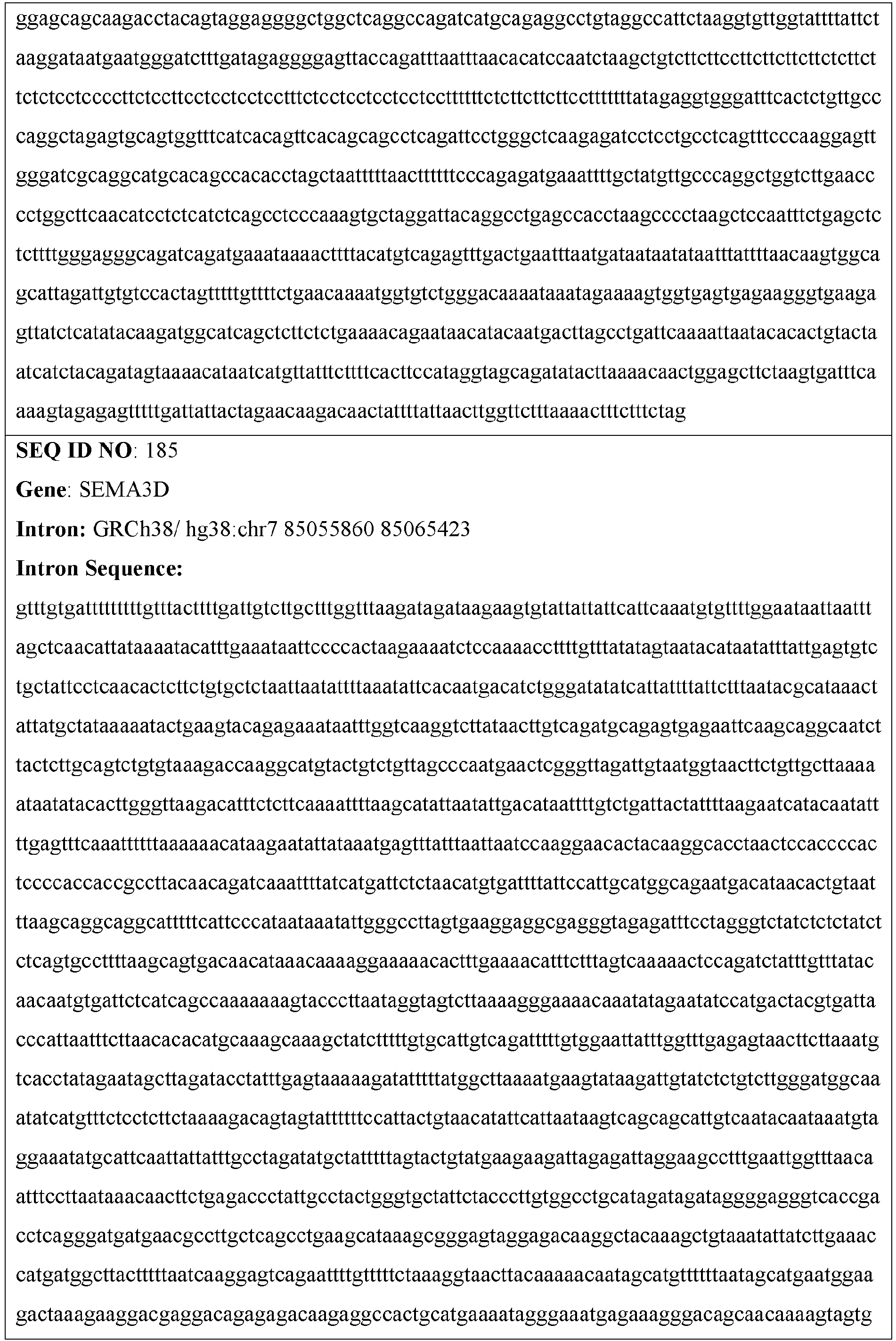

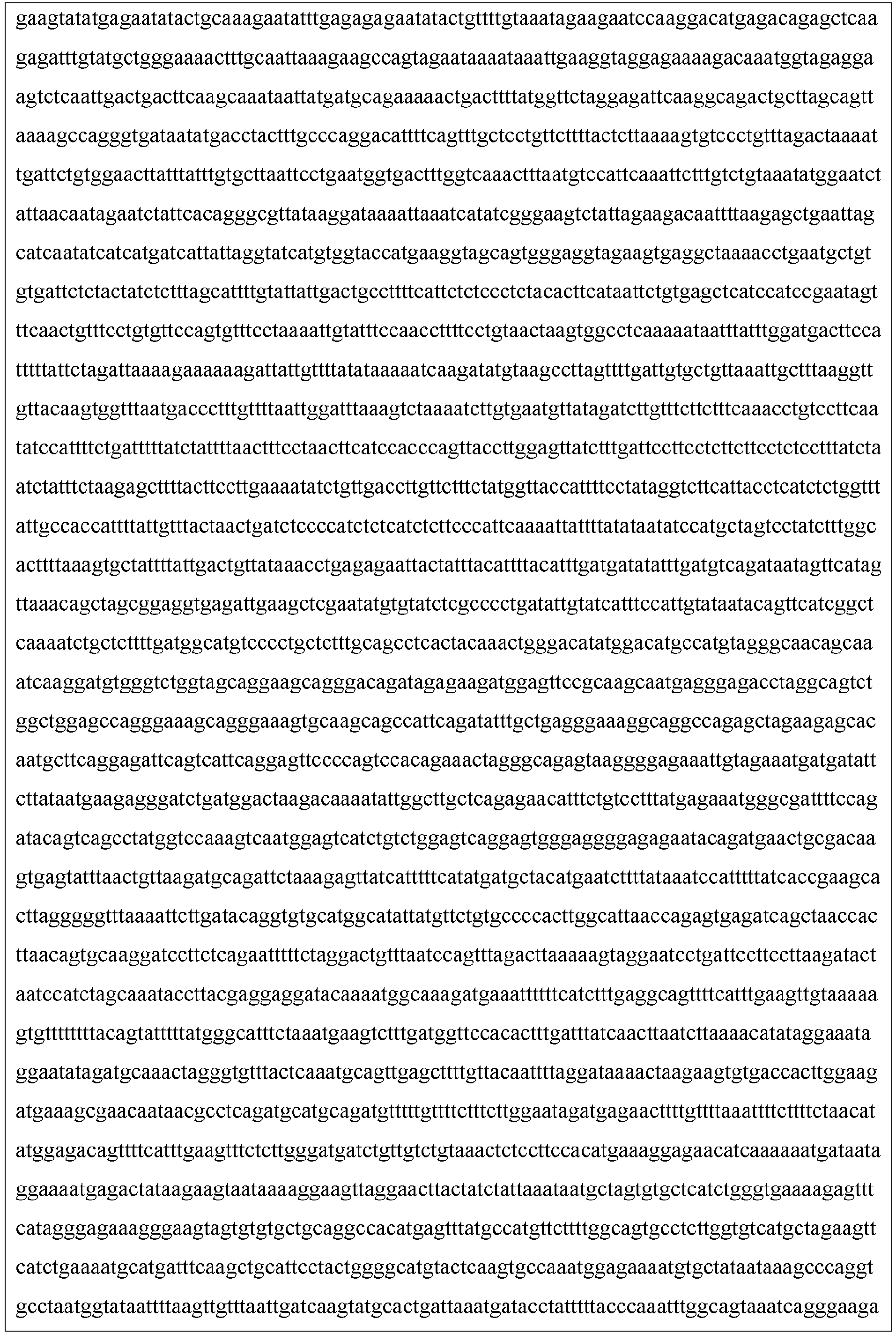

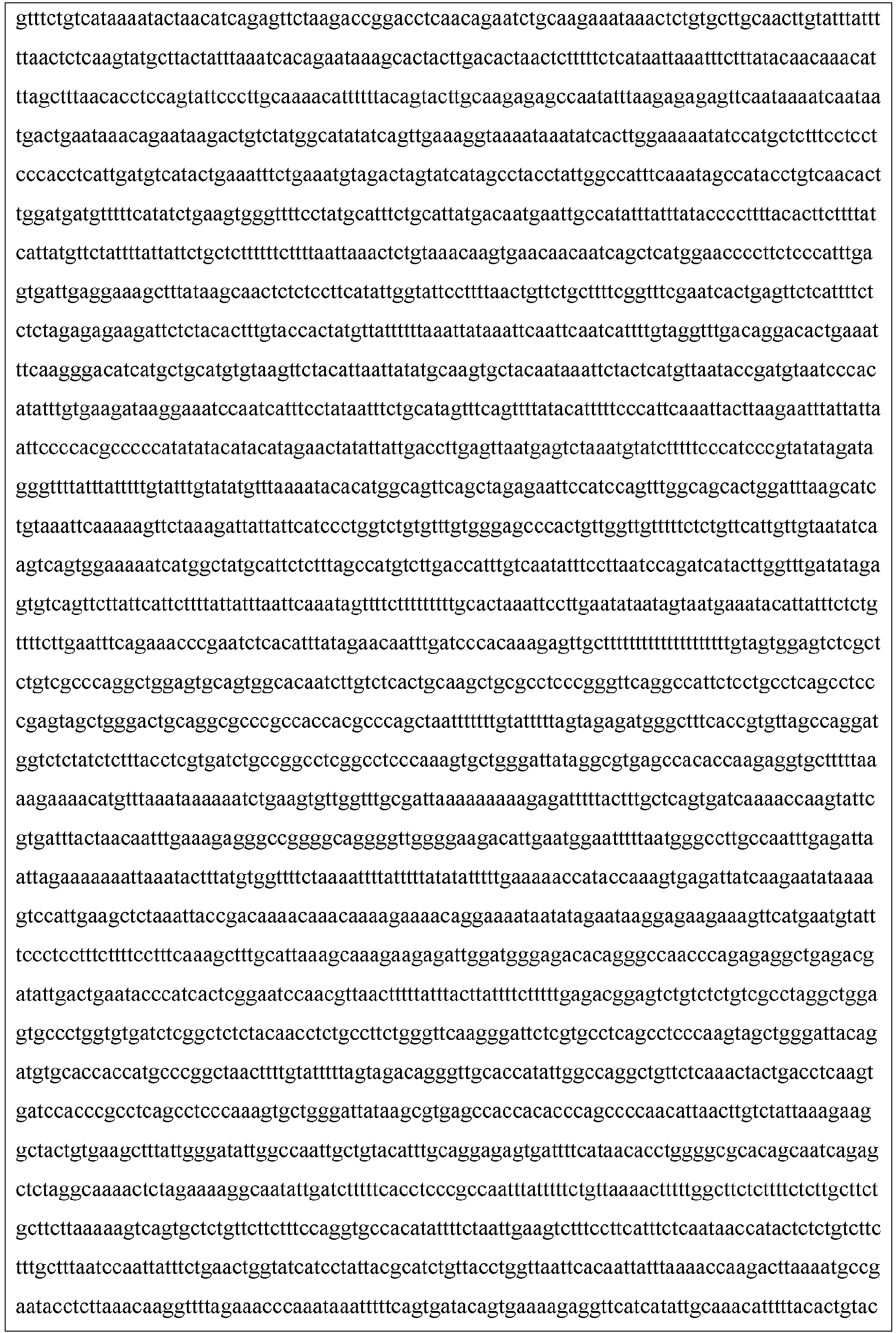

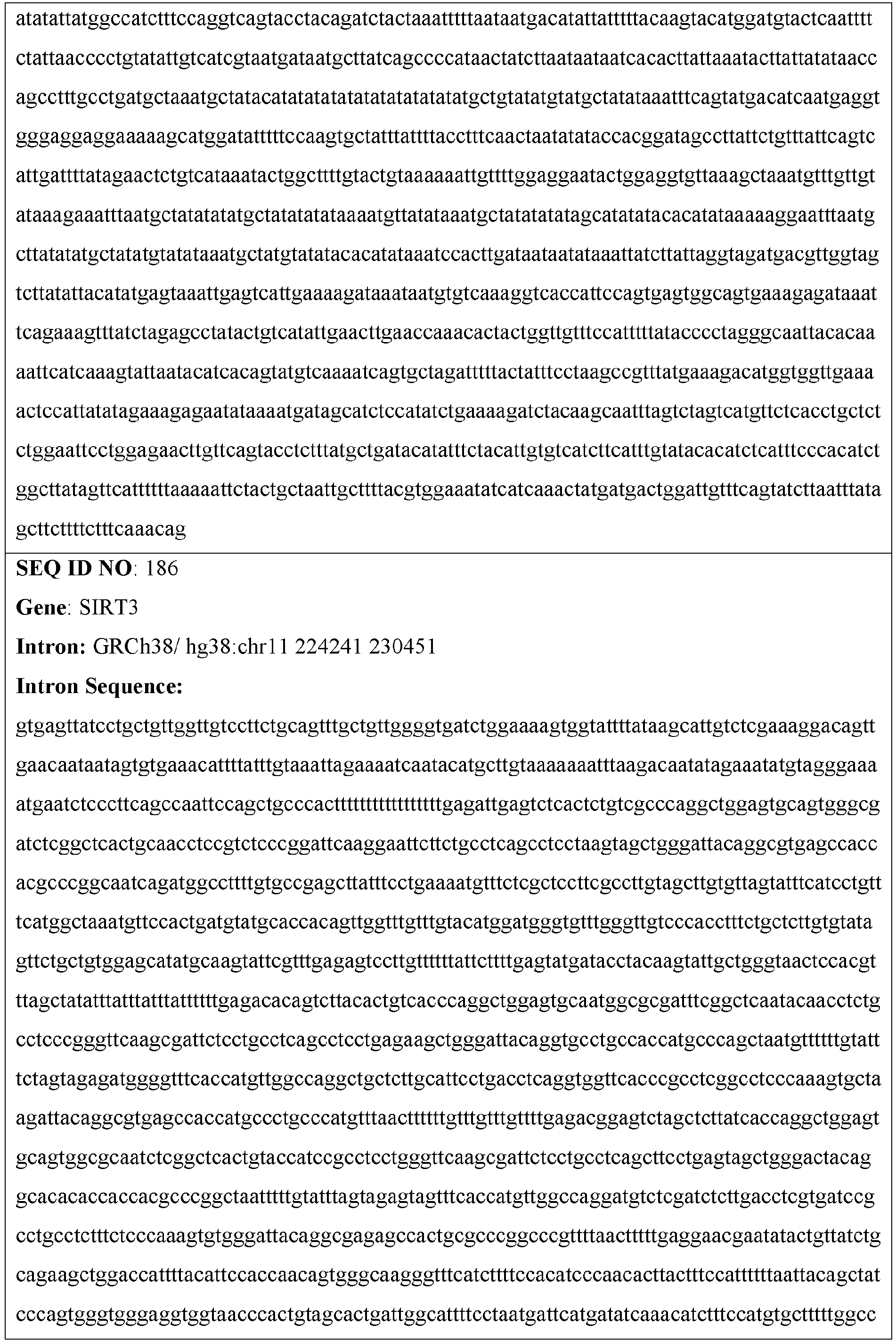

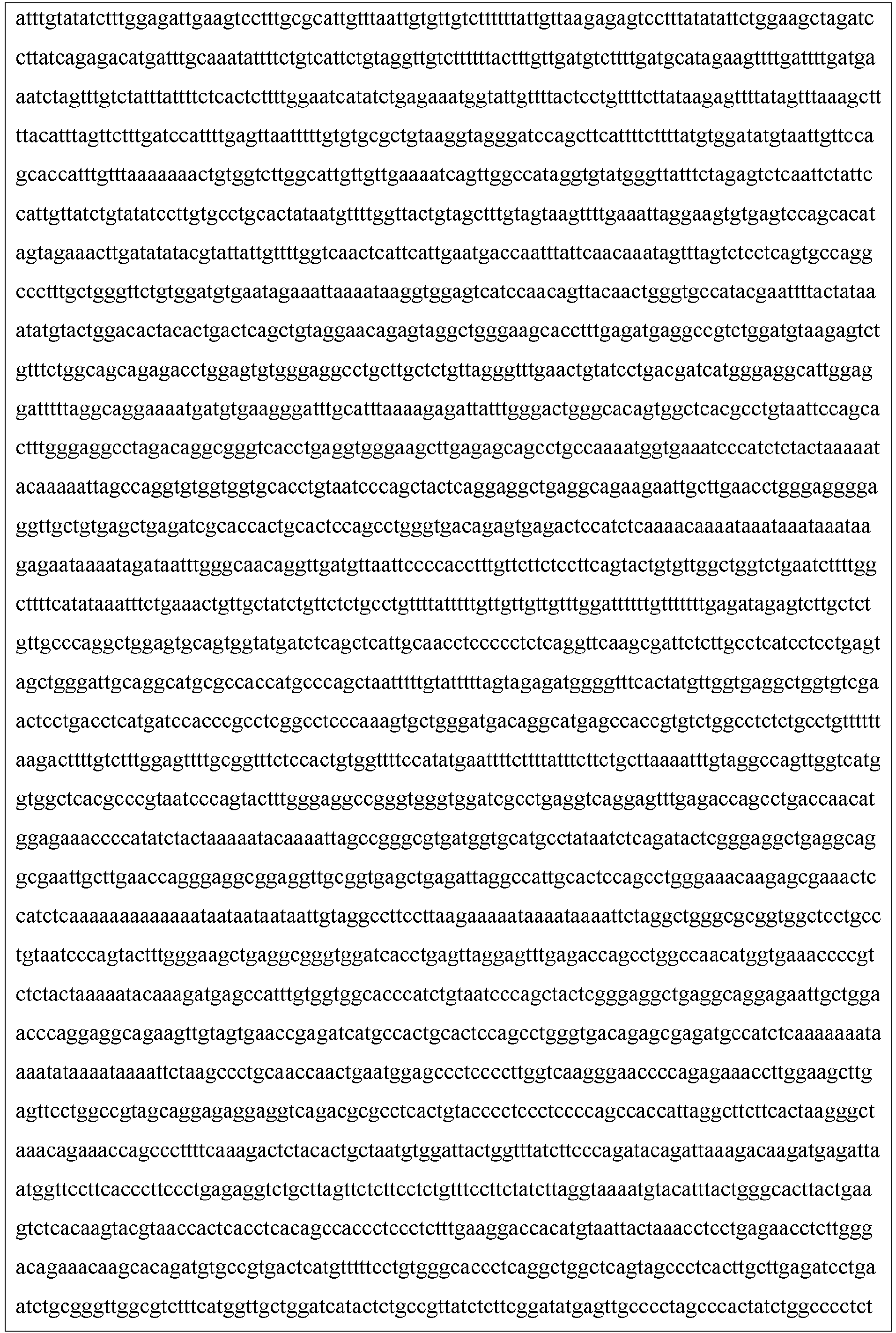

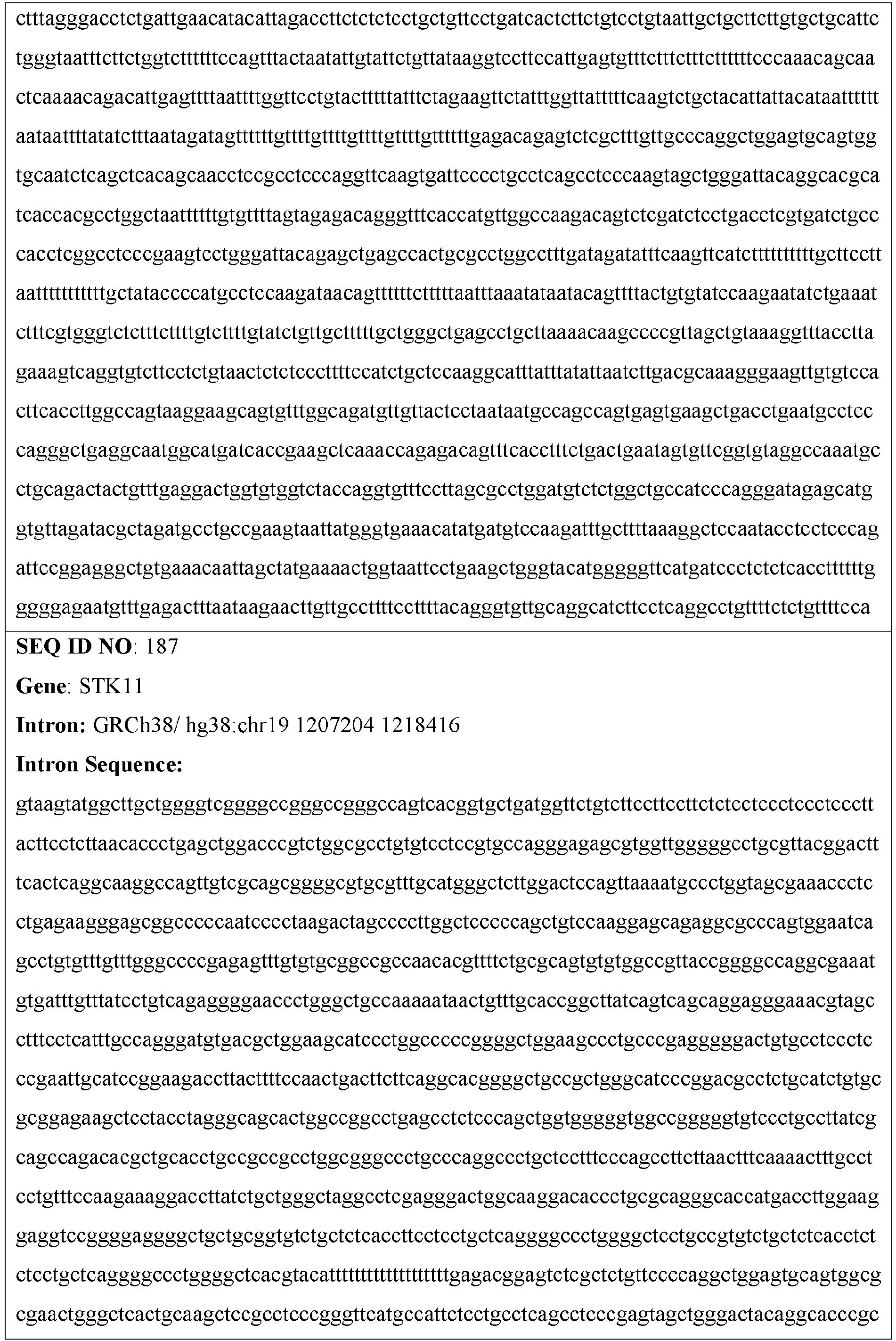

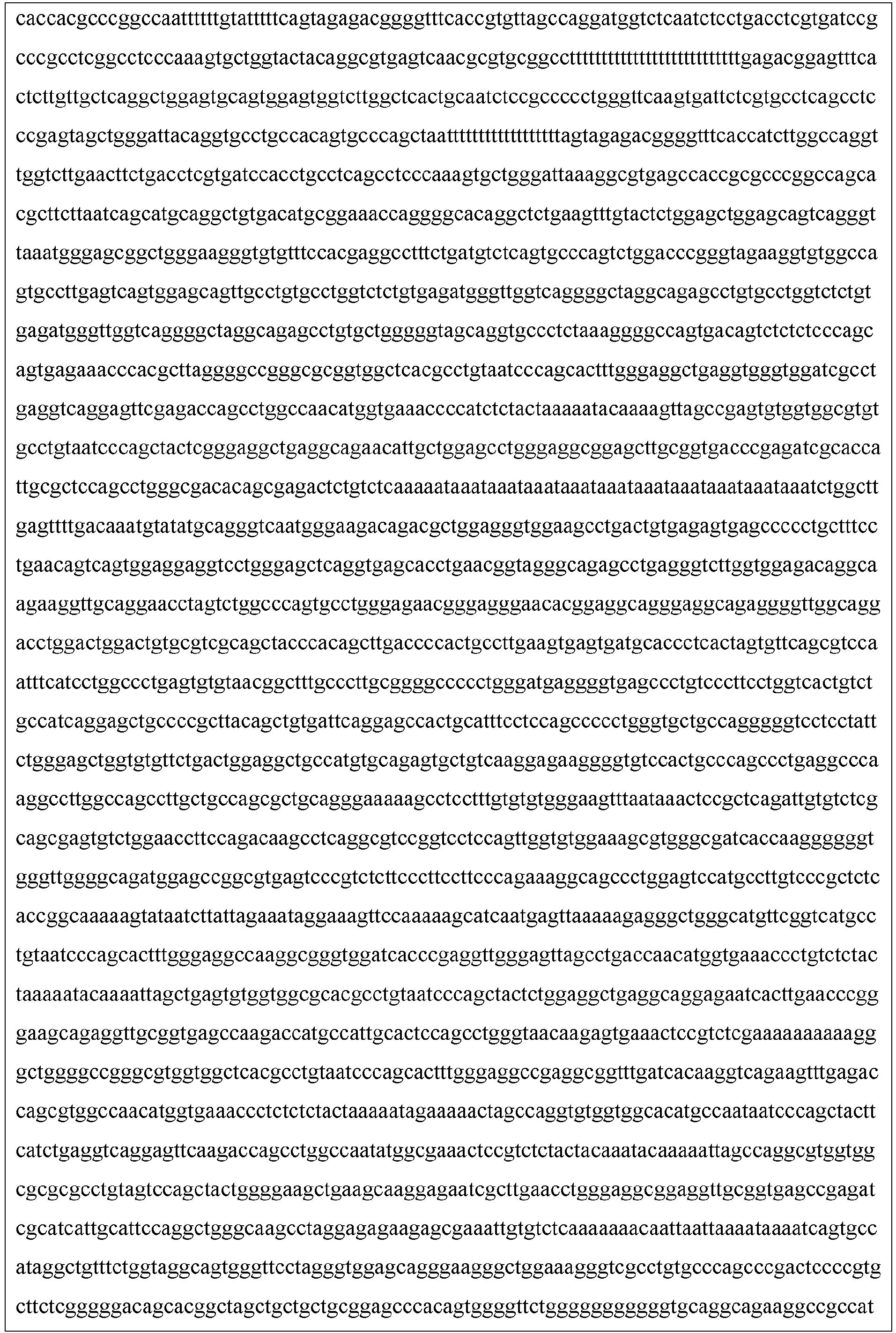

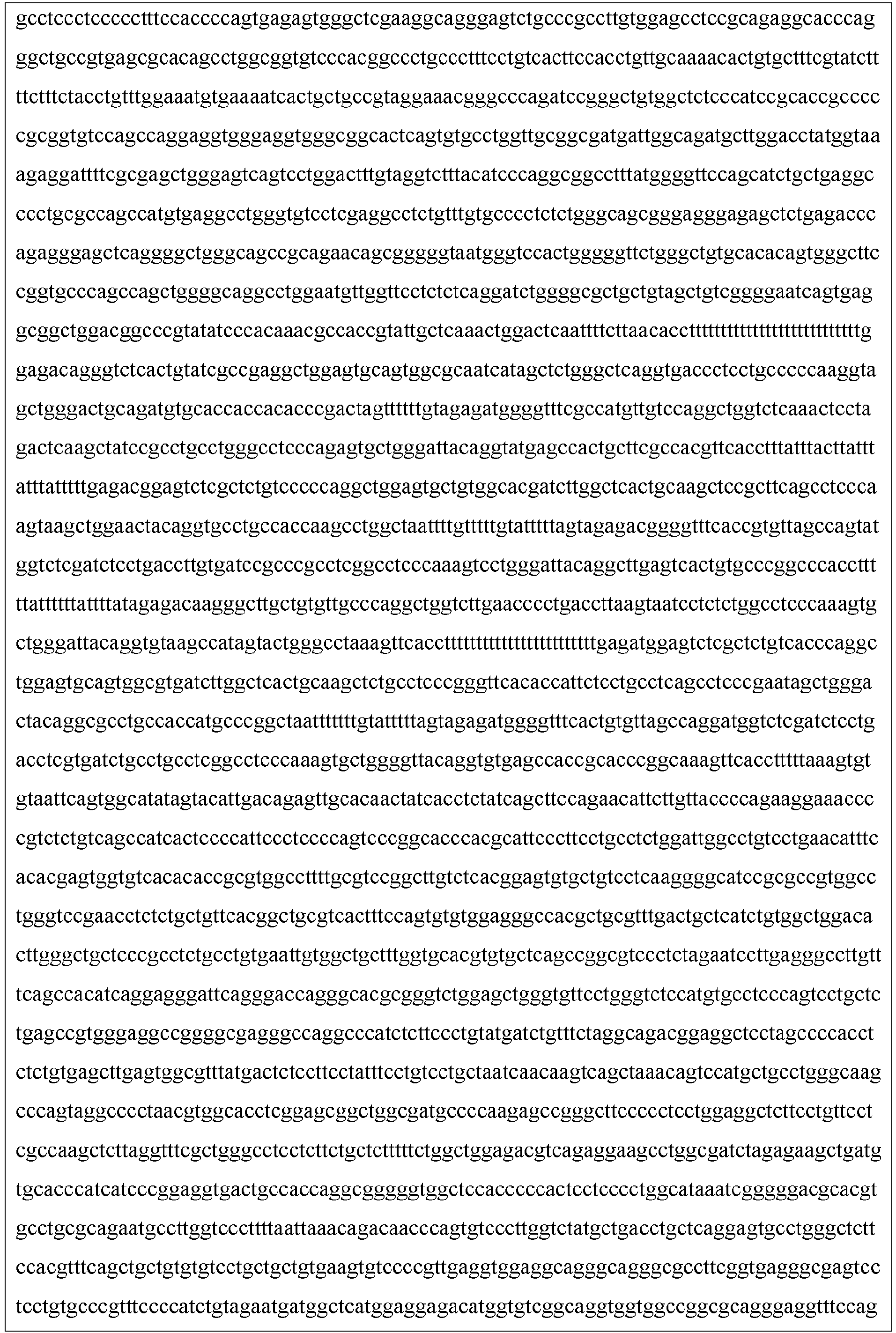

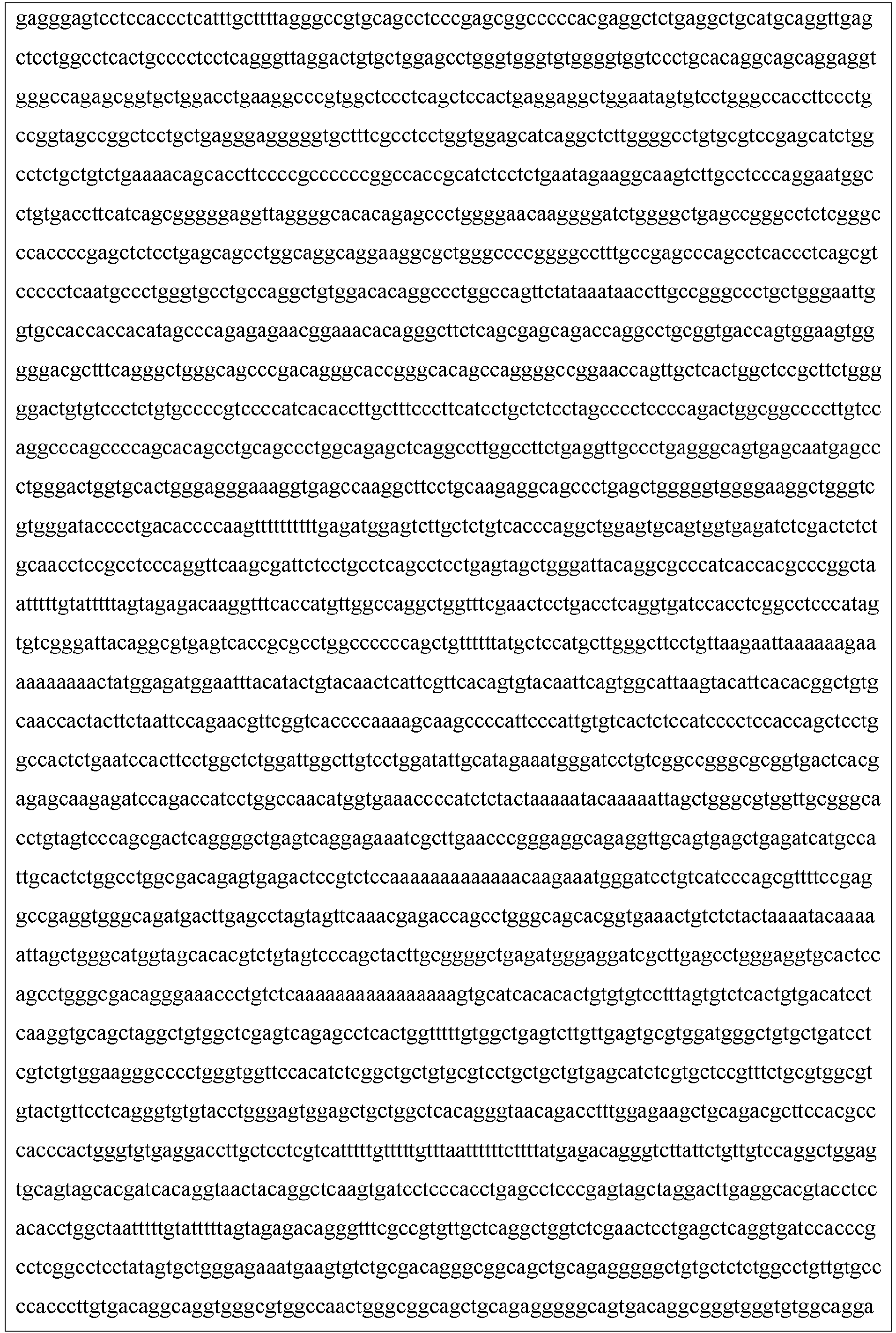

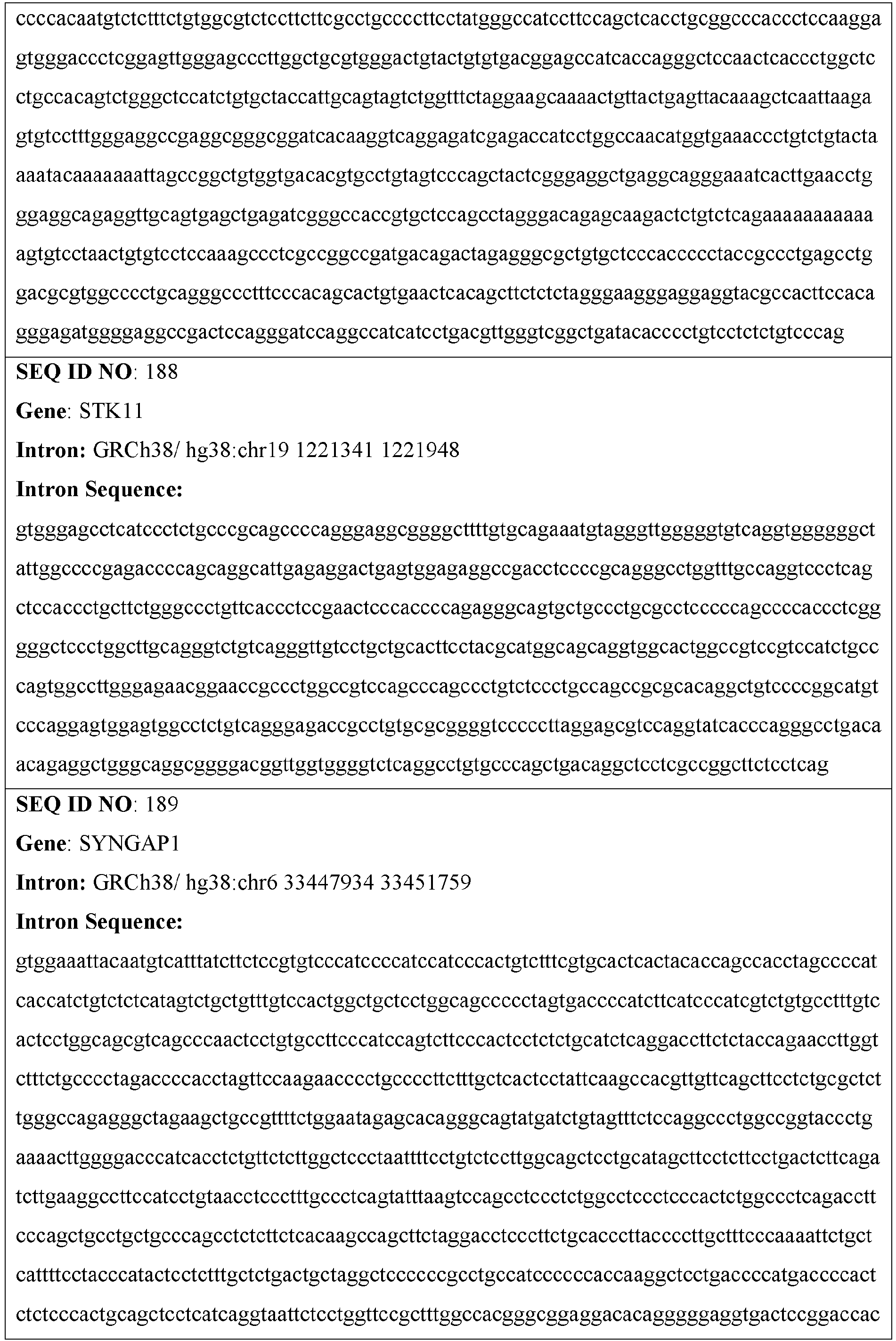

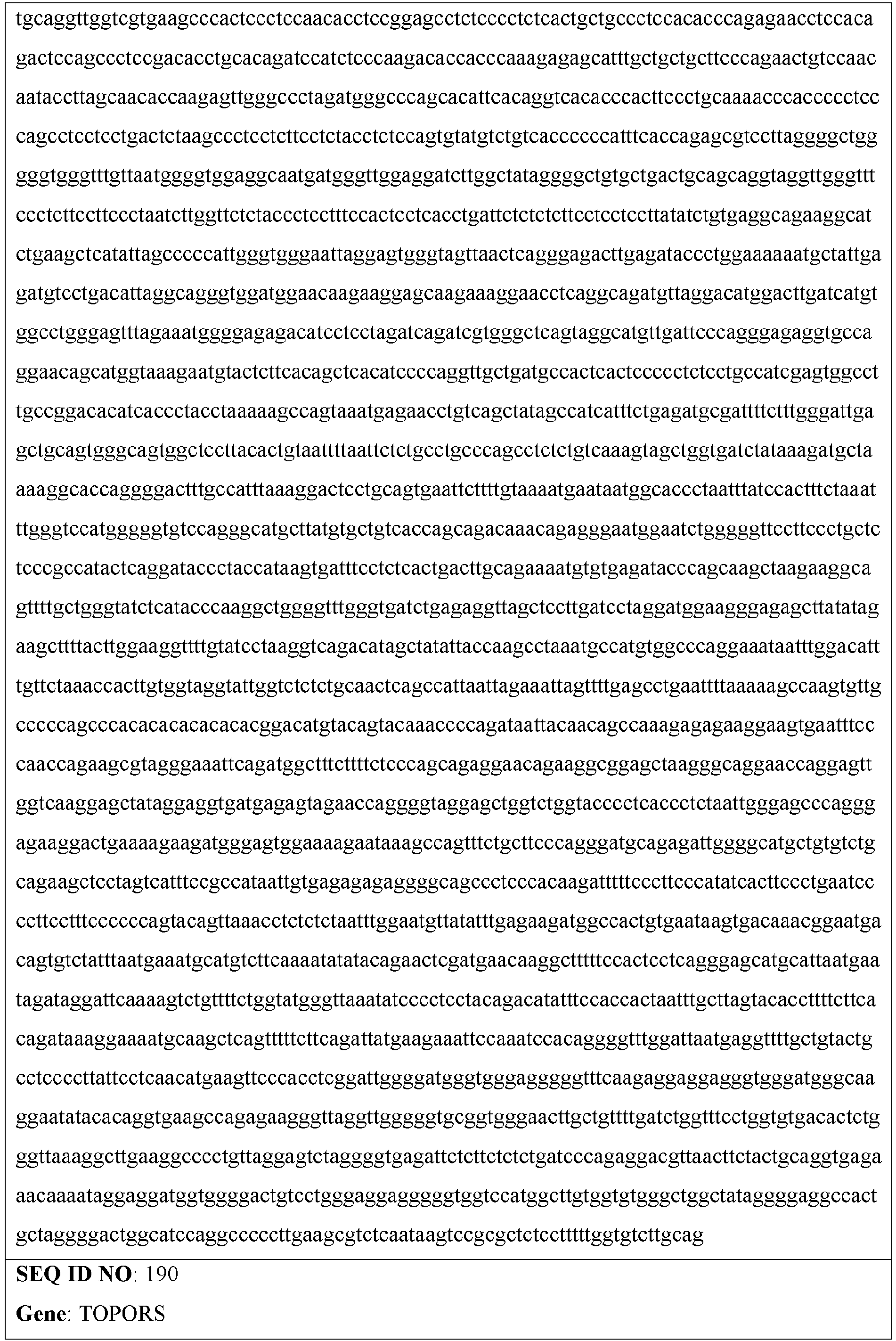

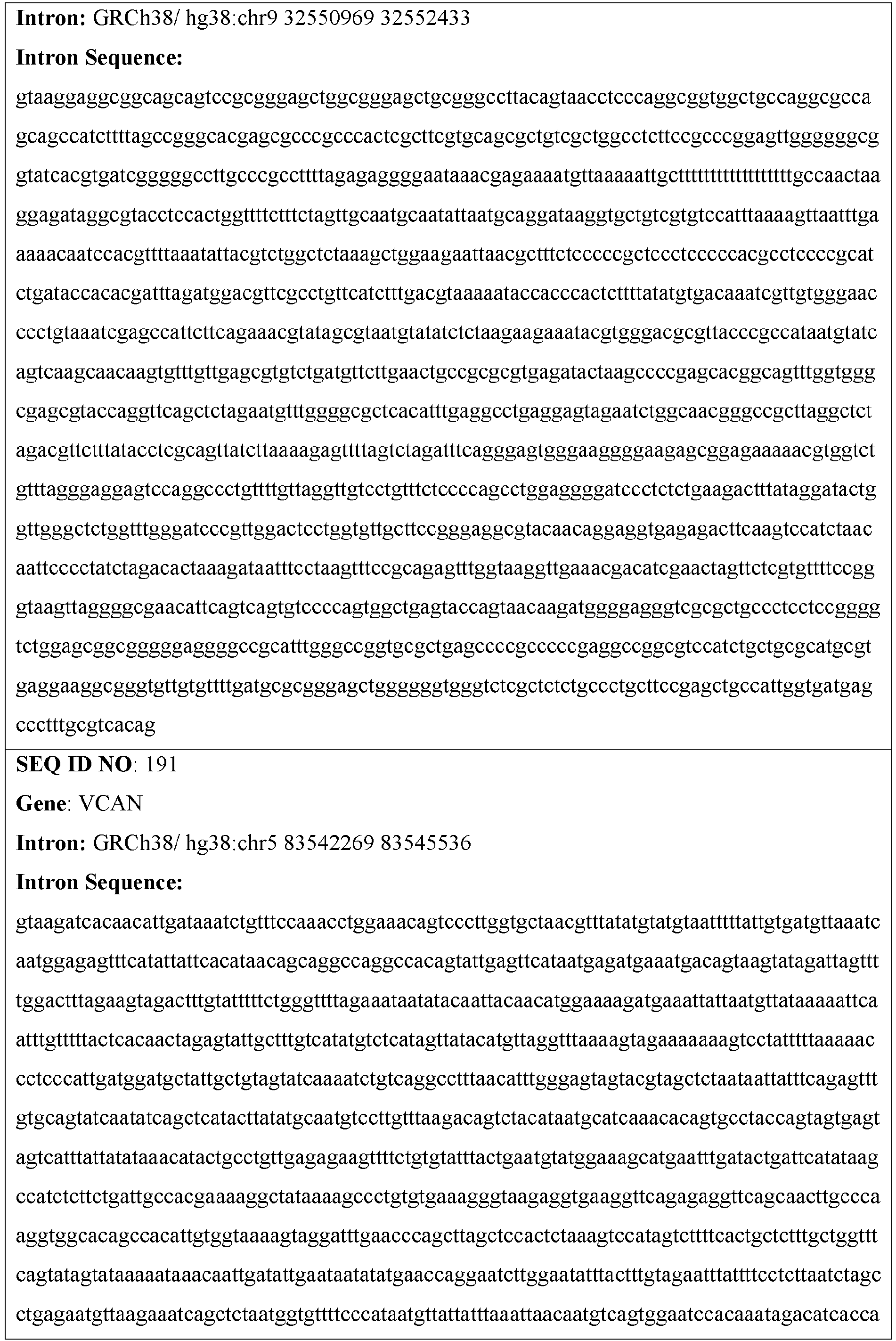

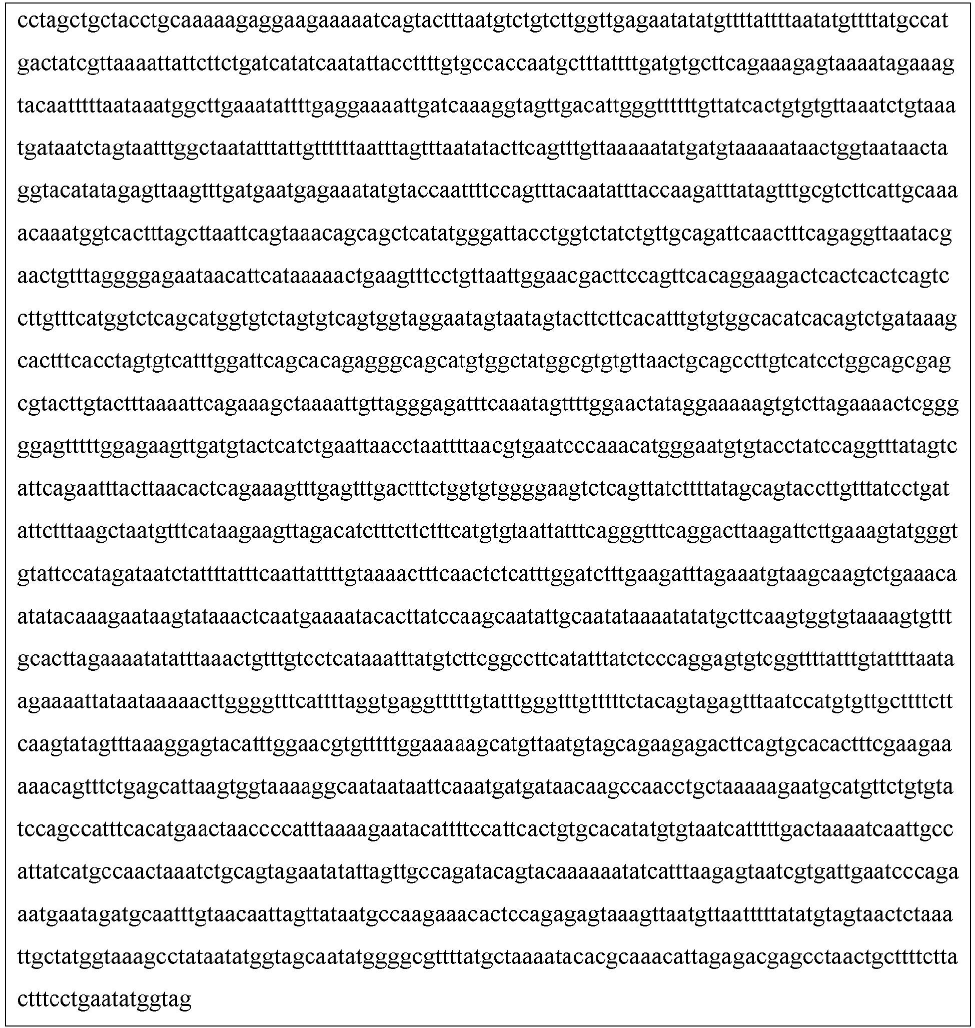

### Example 2: Confirmation of NIE via Cycloheximide Treatment

RT-PCR analysis using cytoplasmic RNA from DMSO-treated or puromycin or cycloheximide-treated human cells and primers in exons can confirm the presence of a band corresponding to an NMD-inducing exon. The identity of the product is confirmed by sequencing. Densitometry analysis of the bands is performed to calculate percent NMD exon inclusion of total transcript. Treatment of cells with cycloheximide or puromycin to inhibit NMD can lead to an increase of the product corresponding to the NMD-inducing exon in the cytoplasmic fraction. **FIGs. 59****,** **62****,** **65****,** **69****,** **72****,** and **75** depict confirmation of exemplary NIE exons in various gene transcripts using cycloheximide or puromycin treatment, respectively.

### Example 3: NMD Exon Region ASO Walk

An ASO walk is performed for NMD exon region targeting sequences immediately upstream of the 3' splice site, across the 3'splice site, the NMD exon, across the 5' splice site, and downstream of the 5' splice site using 2'-MOE ASOs, PS backbone. ASOs are designed to cover these regions by shifting 5 nucleotides at a time. **FIGs. 60****,** **63****,** **66****,** **70****,** **73****,** and **76** depict ASO walk for various exemplary NIE exon regions, respectively.

### Example 4: NMD exon Region ASO Walk Evaluated by RT-PCR

ASO walk sequences can be evaluated by for example RT-PCR. PAGE can be used to show SYBR -safe-stained RT-PCR products of mock-treated (Sham), SMN-control ASO treated (SMN), or treated with a 2'-MOE ASO targeting the NMD exon regions as described herein at 20 µM concentration in human cells by gymnotic uptake. Products corresponding to NMD exon inclusion and full-length are quantified and percent NMD exon inclusion is plotted Full-length products can be normalized to RPL32 internal control and fold-change relative to Sham can be plotted. **FIGs. 71** and **78** depict evaluation via RT-PCR of various exemplary ASO walk along exemplary NIE exon regions, respectively.

### Example 5: NMD exon Region ASO Walk Evaluated by RT-qPCR.

SYBR-green RT-qPCR amplification results normalized to RPL32, can be obtained using the same ASO uptake experiment that can be evaluated by SYBR-safe RT-PCR, and can be plotted as fold change relative to Sham to confirm SYBR-safe RT-PCR results. **FIGs. 61****,** **64****,** **67****,** **68****,** **71****,** **74****,** **77****,** and **78** depict evaluation via RT-qPCR of various exemplary ASO walk along exemplary NIE exon regions, respectively.

### Example 6: Dose-dependent Effect of Selected ASO in CXH-treated Cells.

PAGE can be used to show SYBR-safe-stained RT-PCR products of mock-treated (Sham, RNAiMAX alone), or treated with 2'-MOE ASOs targeting NMD exons at 30 nM, 80 nM, and 200 nM concentrations in mouse or human cells by RNAiMAX transfection. Products corresponding to NMD exon inclusion and full-length are quantified and percent NMD exon inclusion can be plotted . The full-length products can also be normalized to HPRT internal control and fold-change relative to Sham can be plotted.

### Example 7: Intravitreal (IVT) Injection of Selected ASOs.

PAGEs of SYBR-safe-stained RT-PCR products of mice from PBS-injected (1 µL) (-) or ASOs or Cep290 (negative control ASO; Gerard et al, Mol. Ther. Nuc. Ac., 2015) 2'-MOE ASO-injected (1 µL) (+) at 10 mM concentration. Products corresponding to NMD exon inclusion and full-length (are quantified and percent NMD exon inclusion can be plotted Full-length products can be normalized to GAPDH internal control and fold-change of ASO-injected relative to PBS-injected can plotted .

### Example 8: Intracerebroventricular (ICV) Injection of Selected ASOs.

PAGEs of SYBR-safe-stained RT-PCR products of mice from uninjected (-, no ASO control), or 300 µg of Cep290 (negative control ASO; Gerard et al, Mol. Ther. Nuc. Ac., 2015), 2'-MOE ASO-injected brains. Products corresponding to NMD exon inclusion and full-length can be quantified and percent NMD exon inclusion can be plotted. Taqman PCR can be performed using two different probes spanning NMD exon junctions and the products can be normalized to GAPDH internal control and fold-change of ASO-injected relative to Cep290-injected brains can be plotted.

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure. It is intended that the following claims define the scope of the disclosure and that methods and structures within the scope of these claims and their equivalents be covered thereby. Further embodiments of the disclosure are set forth in the following numbered paragraphs:
1. A method of modulating expression of a target protein, by a cell having an mRNA that comprises a non-sense mediated RNA decay-inducing exon (NMD exon) and encodes the target protein, the method comprising contacting a therapeutic agent to the cell, whereby the therapeutic agent modulates splicing of the NMD exon from the mRNA, thereby modulating level of processed mRNA encoding the target protein, and modulating the expression of the target protein in the cell, wherein the target protein is selected from the group consisting of: AKT3, CACNA1A, CBS, CD46, CFH, CHD2, CLN3, COL11A2, COL4A3, COL4A4, COL4A4, CR1, CRX, CYP2J2, DHDDS, DNAJC8, EIF2AK3, ERN1, GALE, GUCY2F, GUCY2F, HEXA, HEXA, MAPK3, MBD5, MBD5, MBD5, MUT, MYH14, MYO6, NF1, NF2, NIPBL, NR1H4, NSD1, NSD1, NSD1, NSD1, OPA1, OPA1, PCCA, PKP2, PPARA, PRPF3, PRPF3, SCN2A, SCN8A, SCN8A, SCN9A, SEMA3C, SEMA3D, SIRT3, STK11, STK11, SYNGAP1, TOPORS, and VCAN proteins.
2. A method of treating a disease or condition in a subject in need thereof by modulating expression of a target protein in a cell of the subject, comprising: contacting the cell of the subject with a therapeutic agent that modulates splicing of a non-sense mediated mRNA decay-inducing exon (NMD exon) from an mRNA in the cell, wherein the mRNA comprises the NMD exon and encodes the target protein, thereby modulating level of processed mRNA encoding the target protein, and modulating expression of the target protein in the cell of the subject, wherein the target protein is selected from the group consisting of: AKT3, CACNA1A, CBS, CD46, CFH, CHD2, CLN3, COL11A2, COL4A3, COL4A4, COL4A4, CR1, CRX, CYP2J2, DHDDS, DNAJC8, EIF2AK3, ERN1, GALE, GUCY2F, GUCY2F, HEXA, HEXA, MAPK3, MBD5, MBD5, MBD5, MUT, MYH14, MYO6, NF1, NF2, NIPBL, NR1H4, NSD1, NSD1, NSD1, NSD1, OPA1, OPA1, PCCA, PKP2, PPARA, PRPF3, PRPF3, SCN2A, SCN8A, SCN8A, SCN9A, SEMA3C, SEMA3D, SIRT3, STK11, STK11, SYNGAP1, TOPORS, and VCAN proteins.
3. The method of paragraph 1 or 2, wherein the therapeutic agent
   (a) binds to a targeted portion of the mRNA encoding the target protein;
   (b) modulates binding of a factor involved in splicing of the NMD exon; or
   (c) a combination of (a) and (b).
4. The method of paragraph 3, wherein the therapeutic agent interferes with binding of the factor involved in splicing of the NMD exon to a region of the targeted portion.
5. The method of paragraph 3, wherein the targeted portion is proximal to the NMD exon.
6. The method of paragraph 5, wherein the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream of 5' end of the NMD exon.
7. The method of paragraph 5, wherein the targeted portion is at least about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides, about 40 nucleotides, about 30 nucleotides, about 20 nucleotides, about 10 nucleotides, about 5 nucleotides, about 4 nucleotides, about 2 nucleotides, about 1 nucleotides upstream of 5' end of the NMD exon.
8. The method of paragraph 5, wherein the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream of 3' end of the NMD exon.
9. The method of paragraph 5, wherein the targeted portion is at least about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides, about 40 nucleotides, about 30 nucleotides, about 20 nucleotides, about 10 nucleotides, about 5 nucleotides, about 4 nucleotides, about 2 nucleotides, about 1 nucleotides downstream of 3' end of the NMD exon.
10. The method of paragraph 5, wherein the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream of genomic site selected from the group consisting of: GRCh38/ hg38: chr1 243564388; GRCh38/ hg38: chr19 13236618; GRCh38/ hg38: chr21 43060012; GRCh38/ hg38: chr1 207775610; GRCh38/ hg38: chr1 196675450; GRCh38/ hg38: chr15 92998149; GRCh38/ hg38: chr16 28479765; GRCh38/ hg38: chr6 33183698; GRCh38/ hg38: chr2 227296487; GRCh38/ hg38: chr2 227144833; GRCh38/ hg38: chr2 227015360; GRCh38/ hg38: chr1 207637688; GRCh38/ hg38: chr19 47835403; GRCh38/ hg38: chr1 59904516; GRCh38/ hg38: chr1 26442335; GRCh38/ hg38: chr1 28230252; GRCh38/ hg38: chr2 88582824; GRCh38/ hg38: chr17 64102804; GRCh38/ hg38: chr1 23798484; GRCh38/ hg38: chrX 109383446; GRCh38/ hg38: chrX 109439175; GRCh38/ hg38: chr15 72362466; GRCh38/ hg38: chr15 72345776; GRCh38/ hg38: chr16 30115645; GRCh38/ hg38: chr2 148460219; GRCh38/ hg38: chr2 148490695; GRCh38/ hg38: chr2 148505761; GRCh38/ hg38: chr6 49436597; GRCh38/ hg38: chr19 50230825; GRCh38/ hg38: chr6 75867431; GRCh38/ hg38: chr17 31249955; GRCh38/ hg38: chr22 29628658; GRCh38/ hg38: chr5 37048127; GRCh38/ hg38: chr12 100499841; GRCh38/ hg38: chr5 177169394; GRCh38/ hg38: chr5 177200761; GRCh38/ hg38: chr5 177247924; GRCh38/ hg38: chr5 177275947; GRCh38/ hg38: chr3 193628509; GRCh38/ hg38: chr3 193603500; GRCh38/ hg38: chr13 100305751; GRCh38/ hg38: chr12 32894778; GRCh38/ hg38: chr22 46203575; GRCh38/ hg38: chr1 150327557; GRCh38/ hg38: chr1 150330401; GRCh38/ hg38: chr2 165327155; GRCh38/ hg38: chr12 51688758; GRCh38/ hg38: chr12 51780202; GRCh38/ hg38: chr2 166304329; GRCh38/ hg38: chr7 80794957; GRCh38/ hg38: chr7 85059541; GRCh38/ hg38: chr11 226081; GRCh38/ hg38: chr19 1216268; GRCh38/ hg38: chr19 1221621; GRCh38/ hg38: chr6 33448789; GRCh38/ hg38: chr9 32551469; and GRCh38/ hg38: chr5 83544965.
11. The method of paragraph 5, wherein the targeted portion is about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides upstream of genomic site selected from the group consisting of: GRCh38/ hg38: chr1 243564388; GRCh38/ hg38: chr19 13236618; GRCh38/ hg38: chr21 43060012; GRCh38/ hg38: chr1 207775610; GRCh38/ hg38: chr1 196675450; GRCh38/ hg38: chr15 92998149; GRCh38/ hg38: chr16 28479765; GRCh38/ hg38: chr6 33183698; GRCh38/ hg38: chr2 227296487; GRCh38/ hg38: chr2 227144833; GRCh38/ hg38: chr2 227015360; GRCh38/ hg38: chr1 207637688; GRCh38/ hg38: chr19 47835403; GRCh38/ hg38: chr1 59904516; GRCh38/ hg38: chr1 26442335; GRCh38/ hg38: chr1 28230252; GRCh38/ hg38: chr2 88582824; GRCh38/ hg38: chr17 64102804; GRCh38/ hg38: chr1 23798484; GRCh38/ hg38: chrX 109383446; GRCh38/ hg38: chrX 109439175; GRCh38/ hg38: chr15 72362466; GRCh38/ hg38: chr15 72345776; GRCh38/ hg38: chr16 30115645; GRCh38/ hg38: chr2 148460219; GRCh38/ hg38: chr2 148490695; GRCh38/ hg38: chr2 148505761; GRCh38/ hg38: chr6 49436597; GRCh38/ hg38: chr19 50230825; GRCh38/ hg38: chr6 75867431; GRCh38/ hg38: chr17 31249955; GRCh38/ hg38: chr22 29628658; GRCh38/ hg38: chr5 37048127; GRCh38/ hg38: chr12 100499841; GRCh38/ hg38: chr5 177169394; GRCh38/ hg38: chr5 177200761; GRCh38/ hg38: chr5 177247924; GRCh38/ hg38: chr5 177275947; GRCh38/ hg38: chr3 193628509; GRCh38/ hg38: chr3 193603500; GRCh38/ hg38: chr13 100305751; GRCh38/ hg38: chr12 32894778; GRCh38/ hg38: chr22 46203575; GRCh38/ hg38: chr1 150327557; GRCh38/ hg38: chr1 150330401; GRCh38/ hg38: chr2 165327155; GRCh38/ hg38: chr12 51688758; GRCh38/ hg38: chr12 51780202; GRCh38/ hg38: chr2 166304329; GRCh38/ hg38: chr7 80794957; GRCh38/ hg38: chr7 85059541; GRCh38/ hg38: chr11 226081; GRCh38/ hg38: chr19 1216268; GRCh38/ hg38: chr19 1221621; GRCh38/ hg38: chr6 33448789; GRCh38/ hg38: chr9 32551469; and GRCh38/ hg38: chr5 83544965.
12. The method of paragraph 5, wherein the targeted portion is at most about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream of genomic site selected from the group consisting of: GRCh38/ hg38: chr1 243564285; GRCh38/ hg38: chr19 13236449; GRCh38/ hg38: chr21 43059730; GRCh38/ hg38: chr1 207775745; GRCh38/ hg38: chr1 196675529; GRCh38/ hg38: chr15 92998261; GRCh38/ hg38: chr16 28479644; GRCh38/ hg38: chr6 33183634; GRCh38/ hg38: chr2 227296526; GRCh38/ hg38: chr2 227144653; GRCh38/ hg38: chr2 227015283; GRCh38/ hg38: chr1 207637848; GRCh38/ hg38: chr19 47835579; GRCh38/ hg38: chr1 59904366; GRCh38/ hg38: chr1 26442372; GRCh38/ hg38: chr1 28230131; GRCh38/ hg38: chr2 88582755; GRCh38/ hg38: chr17 64102673; GRCh38/ hg38: chr1 23798311; GRCh38/ hg38: chrX 109383365; GRCh38/ hg38: chrX 109439038; GRCh38/ hg38: chr15 72362376; GRCh38/ hg38: chr15 72345677; GRCh38/ hg38: chr16 30115595; GRCh38/ hg38: chr2 148460304; GRCh38/ hg38: chr2 148490787; GRCh38/ hg38: chr2 148505830; GRCh38/ hg38: chr6 49436522; GRCh38/ hg38: chr19 50230999; GRCh38/ hg38: chr6 75867523; GRCh38/ hg38: chr17 31250125; GRCh38/ hg38: chr22 29628773; GRCh38/ hg38: chr5 37048354; GRCh38/ hg38: chr12 100500024; GRCh38/ hg38: chr5 177169559; GRCh38/ hg38: chr5 177200783; GRCh38/ hg38: chr5 177248079; GRCh38/ hg38: chr5 177276101; GRCh38/ hg38: chr3 193628616; GRCh38/ hg38: chr3 193603557; GRCh38/ hg38: chr13 100305834; GRCh38/ hg38: chr12 32894516; GRCh38/ hg38: chr22 46203752; GRCh38/ hg38: chr1 150327652; GRCh38/ hg38: chr1 150330498; GRCh38/ hg38: chr2 165327202; GRCh38/ hg38: chr12 51688849; GRCh38/ hg38: chr12 51780271; GRCh38/ hg38: chr2 166304238; GRCh38/ hg38: chr7 80794854; GRCh38/ hg38: chr7 85059498; GRCh38/ hg38: chr11 225673; GRCh38/ hg38: chr19 1216398; GRCh38/ hg38: chr19 1221846; GRCh38/ hg38: chr6 33448868; GRCh38/ hg38: chr9 32551365; and GRCh38/ hg38: chr5 83545070.
13. The method of paragraph 5, wherein the targeted portion is about 1500 nucleotides, about 1000 nucleotides, about 800 nucleotides, about 700 nucleotides, about 600 nucleotides, about 500 nucleotides, about 400 nucleotides, about 300 nucleotides, about 200 nucleotides, about 100 nucleotides, about 80 nucleotides, about 70 nucleotides, about 60 nucleotides, about 50 nucleotides downstream of genomic site selected from the group consisting of: GRCh38/ hg38: chr1 243564285; GRCh38/ hg38: chr19 13236449; GRCh38/ hg38: chr21 43059730; GRCh38/ hg38: chr1 207775745; GRCh38/ hg38: chr1 196675529; GRCh38/ hg38: chr15 92998261; GRCh38/ hg38: chr16 28479644; GRCh38/ hg38: chr6 33183634; GRCh38/ hg38: chr2 227296526; GRCh38/ hg38: chr2 227144653; GRCh38/ hg38: chr2 227015283; GRCh38/ hg38: chr1 207637848; GRCh38/ hg38: chr19 47835579; GRCh38/ hg38: chr1 59904366; GRCh38/ hg38: chr1 26442372; GRCh38/ hg38: chr1 28230131; GRCh38/ hg38: chr2 88582755; GRCh38/ hg38: chr17 64102673; GRCh38/ hg38: chr1 23798311; GRCh38/ hg38: chrX 109383365; GRCh38/ hg38: chrX 109439038; GRCh38/ hg38: chr15 72362376; GRCh38/ hg38: chr15 72345677; GRCh38/ hg38: chr16 30115595; GRCh38/ hg38: chr2 148460304; GRCh38/ hg38: chr2 148490787; GRCh38/ hg38: chr2 148505830; GRCh38/ hg38: chr6 49436522; GRCh38/ hg38: chr19 50230999; GRCh38/ hg38: chr6 75867523; GRCh38/ hg38: chr17 31250125; GRCh38/ hg38: chr22 29628773; GRCh38/ hg38: chr5 37048354; GRCh38/ hg38: chr12 100500024; GRCh38/ hg38: chr5 177169559; GRCh38/ hg38: chr5 177200783; GRCh38/ hg38: chr5 177248079; GRCh38/ hg38: chr5 177276101; GRCh38/ hg38: chr3 193628616; GRCh38/ hg38: chr3 193603557; GRCh38/ hg38: chr13 100305834; GRCh38/ hg38: chr12 32894516; GRCh38/ hg38: chr22 46203752; GRCh38/ hg38: chr1 150327652; GRCh38/ hg38: chr1 150330498; GRCh38/ hg38: chr2 165327202; GRCh38/ hg38: chr12 51688849; GRCh38/ hg38: chr12 51780271; GRCh38/ hg38: chr2 166304238; GRCh38/ hg38: chr7 80794854; GRCh38/ hg38: chr7 85059498; GRCh38/ hg38: chr11 225673; GRCh38/ hg38: chr19 1216398; GRCh38/ hg38: chr19 1221846; GRCh38/ hg38: chr6 33448868; GRCh38/ hg38: chr9 32551365; and GRCh38/ hg38: chr5 83545070.
14. The method of paragraph 3, wherein the targeted portion is located in an intronic region between two canonical exonic regions of the mRNA encoding the target protein, and wherein the intronic region contains the NMD exon.
15. The method of paragraph 3, wherein the targeted portion at least partially overlaps with the NMD exon.
16. The method of paragraph 3, wherein the targeted portion at least partially overlaps with an intron upstream or downstream of the NMD exon.
17. The method of paragraph 3, wherein the targeted portion comprises 5' NMD exon-intron junction or 3' NMD exon-intron junction.
18. The method of paragraph 3, wherein the targeted portion is within the NMD exon.
19. The method of paragraph 3, wherein the targeted portion comprises about 5, 6, 7, 8, 9, 10, 11, 12, 13, 14, 15, 16, 17, 18, 19, 20, 21, 22, 23, 24, 25, 26, 27, 28, 29, 30, or more consecutive nucleotides of the NMD exon.
20. The method of paragraph 1 or 2, wherein the mRNA encoding the target protein comprises a sequence with at least about 80%, 85%, 90%, 95%, 97%, or 100% sequence identity to a sequence selected from the group consisting of SEQ ID NOs: 135-191 .
21. The method of paragraph 1 or 2, wherein the mRNA encoding the target protein is encoded by a genetic sequence with at least about 80%, 85%, 90%, 95%, 97%, or 100% sequence identity to a sequence selected from the group consisting of SEQ ID NOs: 1-5, 12, 19-21, 25, 26, 28, 30, 33, 35, 38, 40, 41, 44, 45, 51, 53, 55-57, and 192-211.
22. The method of paragraph 3, wherein the targeted portion of the mRNA comprises a sequence with at least 80%, 85%, 90%, 95%, 97%, or 100% sequence identity to a region comprising at least 8 contiguous nucleic acids of a sequence selected from the group consisting of SEQ ID NOs: 135-191 .
23. The method of paragraph 1 or 2, wherein the agent is an antisense oligomer (ASO) and wherein the ASO comprises a sequence that is at least about 80%, 85%, 90%, 95%, 97%, or 100% complementary to at least 8 contiguous nucleic acids of a sequence selected from the group consisting of SEQ ID NOs: 135-191.
24. The method of paragraph 3, wherein the targeted portion of the mRNA is within the non-sense mediated RNA decay-inducing exon selected from the group consisting of: GRCh38/ hg38: chr1 243564285 243564388 ; GRCh38/ hg38: chr19 13236449 13236618; GRCh38/ hg38: chr21 43059730 43060012; GRCh38/ hg38: chr1 207775610 207775745; GRCh38/ hg38: chr1 196675450 196675529; GRCh38/ hg38: chr15 92998149 92998261; GRCh38/ hg38: chr16 28479644 28479765; GRCh38/ hg38: chr6 33183634 33183698; GRCh38/ hg38: chr2 227296487 227296526; GRCh38/ hg38: chr2 227144653 227144833; GRCh38/ hg38: chr2 227015283 227015360; GRCh38/ hg38: chr1 207637688 207637848; GRCh38/ hg38: chr19 47835403 47835579; GRCh38/ hg38: chr1 59904366 59904516; GRCh38/ hg38: chr1 26442335 26442372; GRCh38/ hg38: chr1 28230131 28230252 ; GRCh38/ hg38: chr2 88582755 88582824; GRCh38/ hg38: chr17 64102673 64102804; GRCh38/ hg38: chr1 23798311 23798484; GRCh38/ hg38: chrX 109383365 109383446; GRCh38/ hg38: chrX 109439038 109439175; GRCh38/ hg38: chr15 72362376 72362466; GRCh38/ hg38: chr15 72345677 72345776; GRCh38/ hg38: chr16 30115595 30115645; GRCh38/ hg38: chr2 148460219 148460304; GRCh38/ hg38: chr2 148490695 148490787; GRCh38/ hg38: chr2 148505761 148505830; GRCh38/ hg38: chr6 49436522 49436597; GRCh38/ hg38: chr19 50230825 50230999; GRCh38/ hg38: chr6 75867431 75867523; GRCh38/ hg38: chr17 31249955 31250125; GRCh38/ hg38: chr22 29628658 29628773; GRCh38/ hg38: chr5 37048127 37048354; GRCh38/ hg38: chr12 100499841 100500024; GRCh38/ hg38: chr5 177169394 177169559; GRCh38/ hg38: chr5 177200761 177200783; GRCh38/ hg38: chr5 177247924 177248079; GRCh38/ hg38: chr5 177275947 177276101; GRCh38/ hg38: chr3 193628509 193628616; GRCh38/ hg38: chr3 193603500 193603557; GRCh38/ hg38: chr13 100305751 100305834; GRCh38/ hg38: chr12 32894516 32894778; GRCh38/ hg38: chr22 46203575 46203752; GRCh38/ hg38: chr1 150327557 150327652; GRCh38/ hg38: chr1 150330401 150330498; GRCh38/ hg38: chr2 165327155 165327202; GRCh38/ hg38: chr12 51688758 51688849; GRCh38/ hg38: chr12 51780202 51780271; GRCh38/ hg38: chr2 166304238 166304329; GRCh38/ hg38: chr7 80794854 80794957; GRCh38/ hg38: chr7 85059498 85059541; GRCh38/ hg38: chr11 225673 226081; GRCh38/ hg38: chr19 1216268 1216398; GRCh38/ hg38: chr19 1221621 1221846; GRCh38/ hg38: chr6 33448789 33448868; GRCh38/ hg38: chr9 32551365 32551469; and GRCh38/ hg38: chr5 83544965 83545070.
25. The method of paragraph 3, wherein the targeted portion of the mRNA is upstream or downstream of the non-sense mediated RNA decay-inducing exon selected from the group consisting of: GRCh38/ hg38: chr1 243564285 243564388 ; GRCh38/ hg38: chr19 13236449 13236618; GRCh38/ hg38: chr21 43059730 43060012; GRCh38/ hg38: chr1 207775610 207775745; GRCh38/ hg38: chr1 196675450 196675529; GRCh38/ hg38: chr15 92998149 92998261; GRCh38/ hg38: chr16 28479644 28479765; GRCh38/ hg38: chr6 33183634 33183698; GRCh38/ hg38: chr2 227296487 227296526; GRCh38/ hg38: chr2 227144653 227144833; GRCh38/ hg38: chr2 227015283 227015360; GRCh38/ hg38: chr1 207637688 207637848; GRCh38/ hg38: chr19 47835403 47835579; GRCh38/ hg38: chr1 59904366 59904516; GRCh38/ hg38: chr1 26442335 26442372; GRCh38/ hg38: chr1 28230131 28230252 ; GRCh38/ hg38: chr2 88582755 88582824; GRCh38/ hg38: chr17 64102673 64102804; GRCh38/ hg38: chr1 23798311 23798484; GRCh38/ hg38: chrX 109383365 109383446; GRCh38/ hg38: chrX 109439038 109439175; GRCh38/ hg38: chr15 72362376 72362466; GRCh38/ hg38: chr15 72345677 72345776; GRCh38/ hg38: chr16 30115595 30115645; GRCh38/ hg38: chr2 148460219 148460304; GRCh38/ hg38: chr2 148490695 148490787; GRCh38/ hg38: chr2 148505761 148505830; GRCh38/ hg38: chr6 49436522 49436597; GRCh38/ hg38: chr19 50230825 50230999; GRCh38/ hg38: chr6 75867431 75867523; GRCh38/ hg38: chr17 31249955 31250125; GRCh38/ hg38: chr22 29628658 29628773; GRCh38/ hg38: chr5 37048127 37048354; GRCh38/ hg38: chr12 100499841 100500024; GRCh38/ hg38: chr5 177169394 177169559; GRCh38/ hg38: chr5 177200761 177200783; GRCh38/ hg38: chr5 177247924 177248079; GRCh38/ hg38: chr5 177275947 177276101; GRCh38/ hg38: chr3 193628509 193628616; GRCh38/ hg38: chr3 193603500 193603557; GRCh38/ hg38: chr13 100305751 100305834; GRCh38/ hg38: chr12 32894516 32894778; GRCh38/ hg38: chr22 46203575 46203752; GRCh38/ hg38: chr1 150327557 150327652; GRCh38/ hg38: chr1 150330401 150330498; GRCh38/ hg38: chr2 165327155 165327202; GRCh38/ hg38: chr12 51688758 51688849; GRCh38/ hg38: chr12 51780202 51780271; GRCh38/ hg38: chr2 166304238 166304329; GRCh38/ hg38: chr7 80794854 80794957; GRCh38/ hg38: chr7 85059498 85059541; GRCh38/ hg38: chr11 225673 226081; GRCh38/ hg38: chr19 1216268 1216398; GRCh38/ hg38: chr19 1221621 1221846; GRCh38/ hg38: chr6 33448789 33448868; GRCh38/ hg38: chr9 32551365 32551469; and GRCh38/ hg38: chr5 83544965 83545070.
26. The method of paragraph 3, wherein the targeted portion of the mRNA comprises an exon-intron junction of exon selected from the group consisting of: GRCh38/ hg38: chr1 243564285 243564388; GRCh38/ hg38: chr19 13236449 13236618; GRCh38/ hg38: chr21 43059730 43060012; GRCh38/ hg38: chr1 207775610 207775745; GRCh38/ hg38: chr1 196675450 196675529; GRCh38/ hg38: chr15 92998149 92998261; GRCh38/ hg38: chr16 28479644 28479765; GRCh38/ hg38: chr6 33183634 33183698; GRCh38/ hg38: chr2 227296487 227296526; GRCh38/ hg38: chr2 227144653 227144833; GRCh38/ hg38: chr2 227015283 227015360; GRCh38/ hg38: chr1 207637688 207637848; GRCh38/ hg38: chr19 47835403 47835579; GRCh38/ hg38: chr1 59904366 59904516; GRCh38/ hg38: chr1 26442335 26442372; GRCh38/ hg38: chr1 28230131 28230252 ; GRCh38/ hg38: chr2 88582755 88582824; GRCh38/ hg38: chr17 64102673 64102804; GRCh38/ hg38: chr1 23798311 23798484; GRCh38/ hg38: chrX 109383365 109383446; GRCh38/ hg38: chrX 109439038 109439175; GRCh38/ hg38: chr15 72362376 72362466; GRCh38/ hg38: chr15 72345677 72345776; GRCh38/ hg38: chr16 30115595 30115645; GRCh38/ hg38: chr2 148460219 148460304; GRCh38/ hg38: chr2 148490695 148490787; GRCh38/ hg38: chr2 148505761 148505830; GRCh38/ hg38: chr6 49436522 49436597; GRCh38/ hg38: chr19 50230825 50230999; GRCh38/ hg38: chr6 75867431 75867523; GRCh38/ hg38: chr17 31249955 31250125; GRCh38/ hg38: chr22 29628658 29628773; GRCh38/ hg38: chr5 37048127 37048354; GRCh38/ hg38: chr12 100499841 100500024; GRCh38/ hg38: chr5 177169394 177169559; GRCh38/ hg38: chr5 177200761 177200783; GRCh38/ hg38: chr5 177247924 177248079; GRCh38/ hg38: chr5 177275947 177276101; GRCh38/ hg38: chr3 193628509 193628616; GRCh38/ hg38: chr3 193603500 193603557; GRCh38/ hg38: chr13 100305751 100305834; GRCh38/ hg38: chr12 32894516 32894778; GRCh38/ hg38: chr22 46203575 46203752; GRCh38/ hg38: chr1 150327557 150327652; GRCh38/ hg38: chr1 150330401 150330498; GRCh38/ hg38: chr2 165327155 165327202; GRCh38/ hg38: chr12 51688758 51688849; GRCh38/ hg38: chr12 51780202 51780271; GRCh38/ hg38: chr2 166304238 166304329; GRCh38/ hg38: chr7 80794854 80794957; GRCh38/ hg38: chr7 85059498 85059541; GRCh38/ hg38: chr11 225673 226081; GRCh38/ hg38: chr19 1216268 1216398; GRCh38/ hg38: chr19 1221621 1221846; GRCh38/ hg38: chr6 33448789 33448868; GRCh38/ hg38: chr9 32551365 32551469; and GRCh38/ hg38: chr5 83544965 83545070.
27. The method of paragraph 1 or 2, wherein the target protein produced is a full-length protein or a wild-type protein.
28. The method of paragraph 1 or 2, wherein the therapeutic agent promotes exclusion of the NMD exon from the processed mRNA encoding the target protein.
29. The method of paragraph 28, wherein exclusion of the NMD exon from the processed mRNA encoding the target protein in the cell contacted with the therapeutic agent is increased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to exclusion of the NMD exon from the processed mRNA encoding the target protein in a control cell.
30. The method of paragraph 28, wherein the therapeutic agent increases the level of the processed mRNA encoding the target protein in the cell.
31. The method of paragraph 28, wherein the level of the processed mRNA encoding the target protein produced in the cell contacted with the therapeutic agent is increased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the processed mRNA encoding the target protein in a control cell.
32. The method of paragraph 28, wherein the therapeutic agent increases the expression of the target protein in the cell.
33. The method of paragraph 28, wherein a level of the target protein produced in the cell contacted with the therapeutic agent is increased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the target protein produced in a control cell.
34. The method of paragraph 2, wherein the disease or condition is induced by a loss-of-function mutation in the target protein.
35. The method of paragraph 34, wherein the disease or condition is associated with haploinsufficiency of a gene encoding the target protein, and wherein the subject has a first allele encoding a functional target protein, and a second allele from which the target protein is not produced or produced at a reduced level, or a second allele encoding a nonfunctional target protein or a partially functional target protein.
36. The method of paragraph 35, wherein the disease or condition is selected from the group consisting of: Sotos syndrome 1; Beckwith-Wiedemann syndrome; Migraine, familial hemiplegic, 1; Episodic ataxia, type 2; Epileptic encephalopathy, childhood-onset; Wagner syndrome 1; Optic atrophy type 1; Alport syndrome; Arrhythmogenic right ventricular dysplasia 9; Neurofibromatosis type 1; Epileptic encephalopathy, early infantile, 11; Seizures, benign familial infantile, 3; Cognitive impairment with or without cerebellar ataxia; Epileptic encephalopathy, early infantile, 13; Seizures, benign familial infantile, 5; Pathway (CNS); 16p11.2 deletion syndrome?; Mental retardation, autosomal dominant 1; Retinitis pigmentosa 18; Retinitis pigmentosa 31; Deafness, autosomal dominant 13; Cone-rod retinal dystrophy-2; Deafness, autosomal dominant 4A; Peripheral neuropathy, myopathy, hoarseness, and hearing loss ; Deafness, autosomal dominant 22; Neurofibromatosis type 2; Mental retardation, autosomal dominant 5; Epilepsy, generalized, with febrile seizures plus, type 7; and Febrile seizures, familial, 3B.
37. The method of paragraph 34, wherein the disease or condition is associated with an autosomal recessive mutation of a gene encoding the target protein, wherein the subject has a first allele encoding from which:
   (i) the target protein is not produced or produced at a reduced level compared to a wild-type allele; or
   (ii) the target protein produced is nonfunctional or partially functional compared to a wild-type allele, and
   a second allele from which:
   (iii) the target protein is produced at a reduced level compared to a wild-type allele and the target protein produced is at least partially functional compared to a wild-type allele; or
   (iv) the target protein produced is partially functional compared to a wild-type allele.
38. The method of paragraph 37, wherein the disease or condition is selected from the group consisting of: Alport syndrome; Ceroid lipofuscinosis, neuronal, 3; Galactose epimerase deficiency; Homocystinuria, B6-responsive and nonresponsive types; Methyl Malonic Aciduria; Propionic acidemia; Retinitis pigmentosa 59; Tay-Sachs disease; Insensitivity to pain, congenital; and HSAN2D, autosomal recessive.
39. The method of paragraph 34, wherein the therapeutic agent promotes exclusion of the NMD exon from the processed mRNA encoding the target protein and increases the expression of the target protein in the cell.
40. The method of paragraph 1 or 2, wherein the therapeutic agent inhibits exclusion of the NMD exon from the processed mRNA encoding the target protein.
41. The method of paragraph 40, wherein exclusion of the NMD exon from the processed mRNA encoding the target protein in the cell contacted with the therapeutic agent is decreased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to exclusion of the NMD exon from the processed mRNA encoding the target protein in a control cell.
42. The method of paragraph 40, wherein the therapeutic agent decreases the level of the processed mRNA encoding the target protein in the cell.
43. The method of paragraph 40, wherein the level of the processed mRNA encoding the target protein in the cell contacted with the therapeutic agent is decreased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1. 1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the processed mRNA encoding the target protein in a control cell.
44. The method of paragraph 40, wherein the therapeutic agent decreases the expression of the target protein in the cell.
45. The method of paragraph 40, wherein a level of the target protein produced in the cell contacted with the therapeutic agent is decreased by about 1.1 to about 10-fold, about 1.5 to about 10-fold, about 2 to about 10-fold, about 3 to about 10-fold, about 4 to about 10-fold, about 1.1 to about 5-fold, about 1.1 to about 6-fold, about 1.1 to about 7-fold, about 1.1 to about 8-fold, about 1.1 to about 9-fold, about 2 to about 5-fold, about 2 to about 6-fold, about 2 to about 7-fold, about 2 to about 8-fold, about 2 to about 9-fold, about 3 to about 6-fold, about 3 to about 7-fold, about 3 to about 8-fold, about 3 to about 9-fold, about 4 to about 7-fold, about 4 to about 8-fold, about 4 to about 9-fold, at least about 1.1-fold, at least about 1.5-fold, at least about 2-fold, at least about 2.5-fold, at least about 3-fold, at least about 3.5-fold, at least about 4-fold, at least about 5-fold, or at least about 10-fold, compared to a level of the target protein produced in a control cell.
46. The method of paragraph 2, wherein the disease or condition is induced by a gain-of-function mutation in the target protein
47. The method of paragraph 46, wherein the subject has an allele from which the target protein is produced at an increased level, or an allele encoding a mutant target protein that exhibits increased activity in the cell.
48. The method of paragraph 46, wherein the therapeutic agent inhibits exclusion of the NMD exon from the processed mRNA encoding the target protein and decreases the expression of the target protein in the cell.
49. The method of paragraph 40, wherein the target protein comprises SCN8A.
50. The method of paragraph 49, wherein the disease or condition comprises a central nervous system disease.
51. The method of paragraph 50, wherein the disease or condition comprises epilepsy.
52. The method of paragraph 51, wherein the disease or condition comprises Dravet syndrome.
53. The method of paragraph 1 or 2, wherein the therapeutic agent is an antisense oligomer (ASO) and wherein the antisense oligomer comprises a backbone modification comprising a phosphorothioate linkage or a phosphorodiamidate linkage.
54. The method of paragraph 1 or 2, wherein the therapeutic agent is an antisense oligomer (ASO) and wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety.
55. The method of paragraph 1 or 2, wherein the therapeutic agent is an antisense oligomer (ASO) and wherein the antisense oligomer comprises at least one modified sugar moiety.
56. The method of paragraph 55, wherein each sugar moiety is a modified sugar moiety.
57. The method of paragraph 1 or 2, wherein the therapeutic agent is an antisense oligomer (ASO) and wherein the antisense oligomer consists of from 8 to 50 nucleobases, 8 to 40 nucleobases, 8 to 35 nucleobases, 8 to 30 nucleobases, 8 to 25 nucleobases, 8 to 20 nucleobases, 8 to 15 nucleobases, 9 to 50 nucleobases, 9 to 40 nucleobases, 9 to 35 nucleobases, 9 to 30 nucleobases, 9 to 25 nucleobases, 9 to 20 nucleobases, 9 to 15 nucleobases, 10 to 50 nucleobases, 10 to 40 nucleobases, 10 to 35 nucleobases, 10 to 30 nucleobases, 10 to 25 nucleobases, 10 to 20 nucleobases, 10 to 15 nucleobases, 11 to 50 nucleobases, 11 to 40 nucleobases, 11 to 35 nucleobases, 11 to 30 nucleobases, 11 to 25 nucleobases, 11 to 20 nucleobases, 11 to 15 nucleobases, 12 to 50 nucleobases, 12 to 40 nucleobases, 12 to 35 nucleobases, 12 to 30 nucleobases, 12 to 25 nucleobases, 12 to 20 nucleobases, or 12 to 15 nucleobases.
58. The method of paragraph 3, wherein the therapeutic agent is an antisense oligomer (ASO) and wherein the antisense oligomer is at least 80%, at least 85%, at least 90%, at least 95%, at least 98%, at least 99%, or 100%, complementary to the targeted portion of the mRNA.
59. The method of paragraph 1 or 2, wherein the method further comprises assessing mRNA level or expression level of the target protein.
60. The method of paragraph 2, wherein the subject is a human.
61. The method of paragraph 2, wherein the subject is a non-human animal.
62. The method of paragraph 2, wherein the subject is a fetus, an embryo, or a child.
63. The method of paragraph 1 or 2, wherein the cells are ex vivo.
64. The method of paragraph 2, wherein the therapeutic agent is administered by intrathecal injection, intracerebroventricular injection, intraperitoneal injection, intramuscular injection, subcutaneous injection, intravitreal, or intravenous injection of the subject.
65. The method of paragraph 2, wherein the method further comprises administering a second therapeutic agent to the subject.
66. The method of paragraph 65, wherein the second therapeutic agent is a small molecule.
67. The method of paragraph 65, wherein the second therapeutic agent is an antisense oligomer.
68. The method of paragraph 65, wherein the second therapeutic agent corrects intron retention.
69. The method of paragraph 2, wherein the disease or condition is selected from the group consisting of: 16p11.2 deletion syndrome; Alport syndrome; Arrhythmogenic right ventricular dysplasia 9; Ceroid lipofuscinosis, neuronal, 3; Cognitive impairment with or without cerebellar ataxia; Epileptic encephalopathy, early infantile, 13; Seizures, benign familial infantile, 5; Cone-rod retinal dystrophy-2; Cornelia de Lange; Deafness, autosomal dominant 13; Deafness, autosomal dominant 4A; Peripheral neuropathy, myopathy, hoarseness, and hearing loss ; Epilepsy, generalized, with febrile seizures plus, type 7; Febrile seizures, familial, 3B; Insensitivity to pain, congenital; HSAN2D, autosomal recessive; Epileptic encephalopathy, childhood-onset; Epileptic encephalopathy, early infantile, 11; Seizures, benign familial infantile, 3; Galactose epimerase deficiency; Homocystinuria, B6-responsive and nonresponsive types; Mental retardation, autosomal dominant 1; Mental retardation, autosomal dominant 5; Methyl Malonic Aciduria; Migraine, familial hemiplegic, 1; Episodic ataxia, type 2; NASH; Neurofibromatosis type 1; Neurofibromatosis type 2; Optic atrophy type 1; Propionic acidemia; Retinitis pigmentosa 18; Sotos syndrome 1; Beckwith-Wiedemann syndrome; Tay-Sachs disease; and Wagner syndrome 1.

## Claims

1. A pharmaceutical composition comprising an antisense oligomer, and a pharmaceutically acceptable excipient,
wherein the antisense oligomer hybridizes to a targeted portion of a pre-mRNA that comprises a nonsense-mediated mRNA decay-inducing exon (NMD exon) and that encodes a target protein,
wherein the antisense oligomer promotes exclusion of the NMD exon from the pre-mRNA, thereby increasing a level of processed mRNA encoding the target protein and increasing expression of the target protein in the cell comprising the pre-mRNA, and
wherein the target protein is SYNGAP1.

2. A pharmaceutical composition of claim 1 for use as a medicament.

3. A pharmaceutical composition of claim 1 for use in treating a disease or condition in a subject in need thereof, wherein the disease or condition is associated with a deficient amount or activity of the target protein encoded by a gene in the subject.

4. A pharmaceutical composition for use according to claim 3, wherein the disease or condition is associated with haploinsufficiency of the gene encoding the target protein, and wherein the subject has a first allele encoding a functional target protein, and a second allele from which the target protein is not produced or produced at a reduced level, or a second allele encoding a nonfunctional target protein or a partially functional target protein.

5. A pharmaceutical composition for use according to claim 3 or 4, wherein the disease or condition comprises mental retardation, autosomal dominant 5.

6. A pharmaceutical composition of claim 1, or a pharmaceutical composition for use according to any one of claims 2-5, wherein the NMD exon is defined by a pair of genomic coordinates GRCh38/hg38: chr6 33448789 33448868.

7. A pharmaceutical composition of claim 1 or 6, or a pharmaceutical composition for use according to any one of claims 2-6, wherein the targeted portion is in an intronic region between two canonical exonic regions of the pre-mRNA encoding the target protein, and wherein the intronic region contains the NMD exon.

8. A pharmaceutical composition of claim 1 or 6, or a pharmaceutical composition for use according to any one of claims 2-6, wherein the targeted portion at least partially overlaps with the NMD exon.

9. A pharmaceutical composition of claim 1 or 6, or a pharmaceutical composition for use according to any one of claims 2-6, wherein the targeted portion at least partially overlaps with an intron upstream or downstream of the NMD exon.

10. A pharmaceutical composition of claim 1 or 6, or a pharmaceutical composition for use according to any one of claims 2-6, wherein the targeted portion is at most 1500 nucleotides upstream (or 5') of GRCh38/hg38: chr6 33448789, or at most 1500 nucleotides downstream (or 3') of GRCh38/hg38: chr6 33448868.

11. A pharmaceutical composition of claim 1 or 6, or a pharmaceutical composition for use according to any one of claims 2-6, wherein the targeted portion comprises an exon-intron junction of the NMD exon.

12. A pharmaceutical composition of claim 1 or 6, or a pharmaceutical composition for use according to any one of claims 2-6, wherein the targeted portion is within the NMD exon.

13. A pharmaceutical composition of claim 1 or 6, or a pharmaceutical composition for use according to any one of claims 2-6, wherein the targeted portion comprises about 5 or more consecutive nucleotides of the NMD exon.

14. A pharmaceutical composition of any one of claims 1 or 6-13, or a pharmaceutical composition for use according to any one of claims 2-13, wherein the antisense oligomer comprises a sequence that is at least about 80%, 85%, 90%, 95%, 97%, or 100% complementary to at least 8 contiguous nucleotides of SEQ ID NO: 189.

15. A pharmaceutical composition of any one of claims 1 or 6-14, or a pharmaceutical composition for use according to any one of claims 2-14, wherein the antisense oligomer:
(a) comprises a backbone modification comprising a phosphorothioate linkage or a phosphorodiamidate linkage; optionally, wherein the antisense oligomer comprises a phosphorodiamidate morpholino, a locked nucleic acid, a peptide nucleic acid, a 2'-O-methyl, a 2'-Fluoro, or a 2'-O-methoxyethyl moiety;
(b) comprises at least one modified sugar moiety; optionally, wherein each sugar moiety is a modified sugar moiety; and/or
(c) is from 8 to 50 nucleobases, or from 16 to 50 nucleobases in length.
